(19) Europäisches Patentamt
European Patent Office
Office européen des brevets

(11) EP 4 233 893 A2

(12) **EUROPEAN PATENT APPLICATION**

(43) Date of publication:
**30.08.2023 Bulletin 2023/35**

(21) Application number: **23160422.4**

(22) Date of filing: **15.03.2021**

(51) International Patent Classification (IPC):
***A61K 39/00*** (2006.01) ***A61K 47/68*** (2017.01)
***A61P 35/00*** (2006.01) ***C07K 14/725*** (2006.01)
***C07K 16/28*** (2006.01)

(52) Cooperative Patent Classification (CPC):
**C07K 16/2803; A61K 39/001111; A61K 47/6803; A61K 47/6849; A61P 35/00; C07K 14/7051;**
A61K 2039/5156; C07K 2319/03

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **13.03.2020 US 202062989071 P**
**13.03.2020 US 202062989093 P**
**13.03.2020 US 202062989120 P**
**13.03.2020 US 202062989187 P**
**13.03.2020 US 202062989230 P**

(62) Document number(s) of the earlier application(s) in accordance with Art. 76 EPC:
**21713138.2 / 4 117 715**

(71) Applicant: **Janssen Biotech, Inc.**
**Horsham, PA 19044 (US)**

(72) Inventors:
- **DOONAN, Patrick John**
  **Raritan, 08869 (US)**
- **GANESAN, Rajkumar**
  **Raritan, 08869 (US)**
- **DEREBE, Mehabaw Getahun**
  **Raritan, 08869 (US)**
- **VENKATARAMANI, Sathyadevi**
  **Raritan, 08869 (US)**
- **SINGH, Sanjaya**
  **Raritan, 08869 (US)**
- **GREWAL, Iqbal S**
  **Raritan, 08869 (US)**
- **WIEHAGEN, Karla R**
  **Raritan, 08869 (US)**

(74) Representative: **Campabadal Monfa, Gemma**
**Janssen Pharmaceutica NV**
**Patent Law Department**
**Turnhoutseweg 30**
**2340 Beerse (BE)**

Remarks:
This application was filed on 07.03.2023 as a divisional application to the application mentioned under INID code 62.

# (54) MATERIALS AND METHODS FOR BINDING SIGLEC-3/CD33

(57) The invention provides antigen binding domains that bind myeloid cell surface antigen CD33 protein comprising the antigen binding domains that bind CD33, polynucleotides encoding them, vectors, host cells, methods of making and using them.

EP 4 233 893 A2

## Description

## CROSS-REFERENCE TO RELATED APPLICATIONS

The present application claims the priority of U.S. provisional patent application serial number 62/989093 filed on March 13, 2020, U.S. provisional patent application serial number 62/989071 filed on March 13, 2020, U.S. provisional patent application serial number 62/989120 filed on March 13, 2020, U.S. provisional patent application serial number 62/989187 filed on March 13, 2020 and U.S. provisional patent application serial number 62/989230 filed on March 13, 2020.

## TECHNICAL FIELD

The invention provides antigen binding domains that bind myeloid cell surface antigen CD33 protein comprising the antigen binding domains that bind CD33, polynucleotides encoding them, vectors, host cells, methods of making and using them.

## BACKGROUND

Myeloid cell surface antigen CD33, also known as Sialic acid-binding immunoglobulin-like lectin 3 (Siglec-3), is a member of the sialic acid binding family of proteins. CD33 is a type-I single pass transmembrane protein with an N-terminal sialic acid binding domain in its extracellular domain (ECD) and intracellular immunoreceptor tyrosine-based inhibitor motif (ITIM) motifs (Uniprot.org) [4].

In normal cells, CD33 in involved in immune cell maintenance and cell-cell interactions [5]. CD33 protein is expressed in at least the bone marrow, lung, skin, appendix, spleen, lymph nodes, and tonsils. Expression of CD33 mRNA is found in a wide variety of tissues and organs, including but not limited to, bone marrow, the brain, eye, lung, endocrine tissues, salivary glands, esophagus, stomach, colon, rectum, liver, gallbladder, pancreas, kidney, bladder, male and female reproductive tissues, prostate, breast, heart, smooth muscle, skeletal muscle, adipose tissue, skin, thymus, appendix, spleen, lymph nodes, tonsil, granulocytes, and blood (e.g., PBMC, dendritic cells, and lymphocytes). See, e.g., www.proteinatlas.org/ENSG00000105383-CD33/tissue.

CD33 is expressed in 90% of AML patients [6] and is one of the most widely explored surface antigens for AML immunotherapy. Several approaches have been explored to target CD33, including antibody-drug/antibody-toxin conjugates, chimeric antigen receptors (CARs) and T-cell redirector bispecific antibodies [7]. There is a need for additional CD33 binding domains for therapeutic and diagnostic purposes.

Acute Myeloid Leukemia (AML) is a type of leukemia of the myeloid cell lineage characterized by rapid development and progression [1]. AML is a heterogeneous disease with widely varying molecular changes along with different clinical outcomes. Moreover, AML is characterized by the ability of AML blasts, or leukemia cells, to self-renew, continuously proliferate and escape the immune system resulting in disease relapse from minor clones [2]. Accordingly, AML has historically been considered to have one of the lowest long-term survival rates, with an overall 5-year survival rate of adult AML at about 27%. Survival rates have a strong inverse relationship with age [3]. Historically, the primary AML treatment strategies involve chemotherapy, along with stem cell transplantation. In recent years however, there has been aggressive effort to develop immunotherapy for AML exploring key surface antigens. One of the surface antigens highly expressed in AML cells is CD33.

## SUMMARY

In one aspect, the disclosure provides an isolated protein that binds CD33, wherein the isolated protein comprises:

a) a heavy chain complementarity determining region (HCDR) 1, an HCDR2 and an HCDR3 of a heavy chain variable region (VH) of SEQ ID NO: 18;
b) an HCDR1, an HCDR2 and an HCDR3 of a VH of SEQ ID NO: 19;
c) an HCDR1, an HCDR2 and an HCDR3 of a VH of SEQ ID NO: 20;
d) an HCDR1, an HCDR2 and an HCDR3 of a VH of SEQ ID NO: 21;
e) an HCDR1, an HCDR2 and an HCDR3 of a VH of SEQ ID NO: 22;
f) an HCDR1, an HCDR2 and an HCDR3 of a VH of SEQ ID NO: 23;
g) an HCDR1, an HCDR2 and an HCDR3 of a VH of SEQ ID NO: 24;
h) an HCDR1, an HCDR2 and an HCDR3 of a VH of SEQ ID NO: 25;
i) an HCDR1, an HCDR2 and an HCDR3 of a VH of SEQ ID NO: 26; or

j) an HCDR1, an HCDR2 and an HCDR3 of a VH of SEQ ID NO: 27, or a CD33 binding fragment thereof.

In some embodiments, the isolated protein comprises the HCDR1, the HCDR2, the HCDR3 of

a) SEQ ID NOs: 1, 6, and 12, respectively;
b) SEQ ID NOs: 2, 7, and 13, respectively;
c) SEQ ID NOs: 1, 8, and 14, respectively;
d) SEQ ID NOs: 3, 9, and 13, respectively;
e) SEQ ID NOs: 4, 10, and 15, respectively;
f) SEQ ID NOs: 5, 11, and 16, respectively; or
g) SEQ ID NOs: 5, 11, and 17, respectively.

In some embodiments, the isolated protein is a scFv, a $(scFv)_2$, a Fv, a Fab, a $F(ab')_2$, a Fd, a dAb or a VHH. In some embodiments, the isolated protein is the VHH. In some embodiments, the isolated protein is the Fab. In some embodiments, the isolated protein is the scFv. In some embodiments, the isolated protein comprises the VH of any one of SEQ ID NOs: 18, 19, 20, 21, 22, 23, 24, 25, 26, or 27.

In another aspect, the disclosure provides an isolated protein that binds CD33, where the isolated protein comprises a VHH of any one of SEQ ID NOs: 18, 19, 20, 21, 22, 23, 24, 25, 26, or 27.

In another aspect, the disclosure provides an isolated protein that binds CD33, wherein the isolated protein comprises

a) an HCDR1, an HCDR2 and an HCDR3 of a heavy chain variable region (VH) of SEQ ID NO: 52;
b) an HCDR1, an HCDR2 and an HCDR3 of a VH of SEQ ID NO: 53;
c) an HCDR1, an HCDR2 and an HCDR3 of a VH of SEQ ID NO: 54;
d) an HCDR1, an HCDR2 and an HCDR3 of a VH of SEQ ID NO: 55;
e) an HCDR1, an HCDR2 and an HCDR3 of a VH of SEQ ID NO: 56;
f) an HCDR1, an HCDR2 and an HCDR3 of a VH of SEQ ID NO: 57;
g) an HCDR1, an HCDR2 and an HCDR3 of a VH of SEQ ID NO: 58;
h) an HCDR1, an HCDR2 and an HCDR3 of a VH of SEQ ID NO: 59;
i) an HCDR1, an HCDR2 and an HCDR3 of a VH of SEQ ID NO: 60;
j) an HCDR1, an HCDR2 and an HCDR3 of a VH of SEQ ID NO: 61;
k) an HCDR1, an HCDR2 and an HCDR3 of a VH of SEQ ID NO: 262;
l) an HCDR1, an HCDR2 and an HCDR3 of a VH of SEQ ID NO: 263; or
m) an HCDR1, an HCDR2 and an HCDR3 of a VH of SEQ ID NO: 264.

In some embodiments, the isolated protein comprises the HCDR1, the HCDR2, the HCDR3 of

a) SEQ ID NOs: 28, 36, and 45, respectively;
b) SEQ ID NOs: 29, 37, and 46, respectively;
c) SEQ ID NOs: 30, 38, and 47, respectively;
d) SEQ ID NOs: 31, 39, and 48, respectively;
e) SEQ ID NOs: 32, 40, and 49, respectively;
f) SEQ ID NOs: 33, 41, and 50, respectively;
g) SEQ ID NOs: 34, 42, and 51, respectively;
h) SEQ ID NOs: 34, 43, and 51, respectively;
i) SEQ ID NOs: 34, 44, and 51, respectively;
j) SEQ ID NOs: 35, 42, and 51, respectively
k) SEQ ID NOs: 253, 256, and 259, respectively:
l) SEQ ID NOs: 254, 257 and 260, respectively: or
m) SEQ ID NOs: 255, 258 and 261, respectively.

In another aspect, the disclosure provides an isolated protein that binds CD33, wherein the isolated protein comprises the HCDR1, the HCDR2, and the HCDR3 of

a) SEQ ID NOs: 29, 37, and 46, respectively;
b) SEQ ID NOs: 32, 40, and 49, respectively;
c) SEQ ID NOs: 34, 43, and 51, respectively;
d) SEQ ID NOs: 253, 256, and 259, respectively;

e) SEQ ID NOs: 254, 257, and 260, respectively; or
f) SEQ ID NOs: 255, 258, and 261, respectively.

In some embodiments, the isolated protein comprises

a) an HCDR1, an HCDR2 and an HCDR3 of a heavy chain variable region (VH) of SEQ ID NO: 52 and a light chain complementarity determining region (LCDR) 1, an LCDR2 and an LCDR3 of a light chain variable region (VL) of SEQ ID NO: 81;
b) an HCDR1, an HCDR2 and an HCDR3 of a VH of SEQ ID NO: 53 and an LCDR1, an LCDR2 and an LCDR3 of a VL of SEQ ID NO: 82;
c) an HCDR1, an HCDR2 and an HCDR3 of a VH of SEQ ID NO: 54 and an LCDR1, an LCDR2 and an LCDR3 of a VL of SEQ ID NO: 83;
d) an HCDR1, an HCDR2 and an HCDR3 of a VH of SEQ ID NO: 55 and an LCDR1, an LCDR2 and an LCDR3 of a VL of SEQ ID NO: 84;
e) an HCDR1, an HCDR2 and an HCDR3 of a VH of SEQ ID NO: 56 and an LCDR1, an LCDR2 and an LCDR3 of a VL of SEQ ID NO: 85;
f) an HCDR1, an HCDR2 and an HCDR3 of a VH of SEQ ID NO: 57 and an LCDR1, an LCDR2 and an LCDR3 of a VL of SEQ ID NO: 86;
g) an HCDR1, an HCDR2 and an HCDR3 of a VH of SEQ ID NO: 58 and an LCDR1, an LCDR2 and an LCDR3 of a VL of SEQ ID NO: 87;
h) an HCDR1, an HCDR2 and an HCDR3 of a VH of SEQ ID NO: 59 and an LCDR1, an LCDR2 and an LCDR3 of a VL of SEQ ID NO: 87;
i) an HCDR1, an HCDR2 and an HCDR3 of a VH of SEQ ID NO: 60 and an LCDR1, an LCDR2 and an LCDR3 of a VL of SEQ ID NO: 87;
j) an HCDR1, an HCDR2 and an HCDR3 of a VH of SEQ ID NO: 61 and an LCDR1, an LCDR2 and an LCDR3 of a VL of SEQ ID NO: 88;
k) an HCDR1, an HCDR2 and an HCDR3 of a VH of SEQ ID NO: 262 and an LCDR1, an LCDR2 and an LCDR3 of a VL of SEQ ID NO: 271;
l) an HCDR1, an HCDR2 and an HCDR3 of a VH of SEQ ID NO: 263 and an LCDR1, an LCDR2 and an LCDR3 of a VL of SEQ ID NO: 272; or
m) an HCDR1, an HCDR2 and an HCDR3 of a VH of SEQ ID NO: 264 and an LCDR1, an LCDR2 and an LCDR3 of a VL of SEQ ID NO: 273.

In some embodiments, the isolated protein comprises the HCDR1, the HCDR2, the HCDR3, the LCDR1, the LCDR2 and the LCDR3 of

a) SEQ ID NOs: 28, 36, 45, 62, 69, and 75, respectively;
b) SEQ ID NOs: 29, 37, 46, 63, 70, and 76, respectively;
c) SEQ ID NOs: 30, 38, 47, 64, 71, and 77, respectively;
d) SEQ ID NOs: 31, 39, 48, 65, 72, and 78, respectively;
e) SEQ ID NOs: 32, 40, 49, 66, 70, and 76, respectively;
f) SEQ ID NOs: 33, 41, 50, 67, 73, and 79, respectively;
g) SEQ ID NOs: 34, 42, 51, 68, 74, and 80, respectively;
h) SEQ ID NOs: 34, 43, 51, 68, 74, and 80, respectively;
i) SEQ ID NOs: 34, 44, 51, 68, 74, and 80, respectively;
j) SEQ ID NOs: 35, 42, 51, 68, 74, and 80, respectively:
k) SEQ ID NOs: 253, 256, 259, 265, 74 and 269, respectively:
l) SEQ ID NOs: 254, 257, 260, 66, 267 and 76, respectively: or
m) SEQ ID NOs: 255, 258, 261, 266, 268 and 270, respectively.

In some embodiments, the isolated protein is a scFv, a (scFv)z, a Fv, a Fab, a $F(ab')_2$, a Fd, a dAb or a VHH. In some embodiments, the isolated protein is the Fab. In some embodiments, the isolated protein is the VHH. In some embodiments, the isolated protein is the scFv. In some embodiments, the scFv comprises, from the N- to C-terminus, a VH, a first linker (L1) and a VL (VH-L1-VL) or the VL, the L1 and the VH (VL-L1-VH).
In some embodiments, the L1 comprises

a) about 5-50 amino acids;
b) about 5-40 amino acids;

c) about 10-30 amino acids; or
d) about 10-20 amino acids.

In some embodiments, the L1 comprises an amino acid sequence of any one of SEQ ID NOs: 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, or 140. In some embodiments, the L1 comprises the amino acid sequence of SEQ ID NO: 108.

In some embodiments, the isolated protein comprises the VH of SEQ ID NOs: 52, 53, 54, 55, 56, 57, 58, 59, 60, 61, 262, 263, 264, 291, 292, 293, 294, 295, 296, 297, 298, 299, 300, 301, 302, 303, 304, 305, 306, 307, 308, 309, 310, 337, 338, 339, 340, 341, 342, 343, 344, 345, 346, 347, 348, 349, 350, 351, 352, 367, 368, 369, 370, 371, 372, 373, 374, 375, 376, 377, 378, 379, 380, 381, 382, or 382, and the VL of SEQ ID NOs: 81, 82, 83, 84, 85, 86, 87, 88, 271, 272,273, 311, 312, 313, 314, 315, 316, 317, 318, 319, 320, 321, 322, 323, 324, 325, 326, 327, 328, 329, 330, 331, 332, 333, 334, 335, 336, 353, 354, 355, 356, 357, 358, 359, 360, 361, 362, 363, 364, 365, 366, 383, 384, 385, 386, 387, 388, 389, 390, 391, 392, 393, 394, 395, 396, 397 or 398.

In some embodiments, the isolated protein comprises:

a) the VH of SEQ ID NO: 52 and the VL of SEQ ID NO: 81;
b) the VH of SEQ ID NO: 53 and the VL of SEQ ID NO: 82;
c) the VH of SEQ ID NO: 54 and the VL of SEQ ID NO: 83;
d) the VH of SEQ ID NO: 55 and the VL of SEQ ID NO: 84;
e) the VH of SEQ ID NO: 56 and the VL of SEQ ID NO: 85;
f) the VH of SEQ ID NO: 57 and the VL of SEQ ID NO: 86;
g) the VH of SEQ ID NO: 58 and the VL of SEQ ID NO: 87;
h) the VH of SEQ ID NO: 59 and the VL of SEQ ID NO: 87;
i) the VH of SEQ ID NO: 60 and the VL of SEQ ID NO: 87;
j) the VH of SEQ ID NO: 61 and the VL of SEQ ID NO: 88;
k) the VH of SEQ ID NO: 262 and the VL of SEQ ID NO: 271;
1) the VH of SEQ ID NO: 263 and the VL of SEQ ID NO: 272;
m) the VH of SEQ ID NO: 264 and the VL of SEQ ID NO: 273;
n) the VH of SEQ ID NO: 291 and the VL of SEQ ID NO: 271;
o) the VH of SEQ ID NO: 292 and the VL of SEQ ID NO: 271;
p) the VH of SEQ ID NO: 293 and the VL of SEQ ID NO: 271;
q) the VH of SEQ ID NO: 294 and the VL of SEQ ID NO: 271;
r) the VH of SEQ ID NO: 295 and the VL of SEQ ID NO: 271;
s) the VH of SEQ ID NO: 296 and the VL of SEQ ID NO: 271;
t) the VH of SEQ ID NO: 297 and the VL of SEQ ID NO: 271;
u) the VH of SEQ ID NO: 298 and the VL of SEQ ID NO: 271;
v) the VH of SEQ ID NO: 299 and the VL of SEQ ID NO: 271;
w) the VH of SEQ ID NO: 300 and the VL of SEQ ID NO: 271;
x) the VH of SEQ ID NO: 301 and the VL of SEQ ID NO: 271;
y) the VH of SEQ ID NO: 302 and the VL of SEQ ID NO: 271;
z) the VH of SEQ ID NO: 303 and the VL of SEQ ID NO: 271;
aa) the VH of SEQ ID NO: 304 and the VL of SEQ ID NO: 271;
ab) the VH of SEQ ID NO: 305 and the VL of SEQ ID NO: 271;
ac) the VH of SEQ ID NO: 306 and the VL of SEQ ID NO: 271;
ad) the VH of SEQ ID NO: 307 and the VL of SEQ ID NO: 271;
ae) the VH of SEQ ID NO: 308 and the VL of SEQ ID NO: 271;
af) the VH of SEQ ID NO: 309 and the VL of SEQ ID NO: 271;
ag) the VH of SEQ ID NO: 310 and the VL of SEQ ID NO: 271;
ah) the VH of SEQ ID NO: 263 and the VL of SEQ ID NO: 311;
ai) the VH of SEQ ID NO: 263 and the VL of SEQ ID NO: 312.
aj) the VH of SEQ ID NO: 263 and the VL of SEQ ID NO: 313;
ak) the VH of SEQ ID NO: 263 and the VL of SEQ ID NO: 314;
al) the VH of SEQ ID NO: 263 and the VL of SEQ ID NO: 315;
am) the VH of SEQ ID NO: 263 and the VL of SEQ ID NO: 316;
an) the VH of SEQ ID NO: 263 and the VL of SEQ ID NO: 317;
ao) the VH of SEQ ID NO: 263 and the VL of SEQ ID NO: 318;
ap) the VH of SEQ ID NO: 263 and the VL of SEQ ID NO: 319;
aq) the VH of SEQ ID NO: 263 and the VL of SEQ ID NO: 320;

ar) the VH of SEQ ID NO: 263 and the VL of SEQ ID NO: 321;
as) the VH of SEQ ID NO: 263 and the VL of SEQ ID NO: 322;
at) the VH of SEQ ID NO: 263 and the VL of SEQ ID NO: 323;
au) the VH of SEQ ID NO: 263 and the VL of SEQ ID NO: 324;
av) the VH of SEQ ID NO: 263 and the VL of SEQ ID NO: 325;
aw) the VH of SEQ ID NO: 263 and the VL of SEQ ID NO: 326;
ax) the VH of SEQ ID NO: 263 and the VL of SEQ ID NO: 327;
ay) the VH of SEQ ID NO: 263 and the VL of SEQ ID NO: 328;
az) the VH of SEQ ID NO: 263 and the VL of SEQ ID NO: 329;
ba) the VH of SEQ ID NO: 263 and the VL of SEQ ID NO: 330;
bb) the VH of SEQ ID NO: 263 and the VL of SEQ ID NO: 331;
be) the VH of SEQ ID NO: 263 and the VL of SEQ ID NO: 332;
bd) the VH of SEQ ID NO: 263 and the VL of SEQ ID NO: 333;
be) the VH of SEQ ID NO: 263 and the VL of SEQ ID NO: 334;
bf) the VH of SEQ ID NO: 263 and the VL of SEQ ID NO: 335;
bg) the VH of SEQ ID NO: 263 and the VL of SEQ ID NO: 336;
bh) the VH of SEQ ID NO: 337 and the VL of SEQ ID NO: 272;
bi) the VH of SEQ ID NO: 338 and the VL of SEQ ID NO: 272;
bj) the VH of SEQ ID NO: 339 and the VL of SEQ ID NO: 272;
bk) the VH of SEQ ID NO: 340 and the VL of SEQ ID NO: 272;
bl) the VH of SEQ ID NO: 341 and the VL of SEQ ID NO: 272;
bm) the VH of SEQ ID NO: 342 and the VL of SEQ ID NO: 272;
bn) the VH of SEQ ID NO: 343 and the VL of SEQ ID NO: 272;
bo) the VH of SEQ ID NO: 344 and the VL of SEQ ID NO: 272;
bp) the VH of SEQ ID NO: 345 and the VL of SEQ ID NO: 272;
bq) the VH of SEQ ID NO: 346 and the VL of SEQ ID NO: 272;
br) the VH of SEQ ID NO: 347 and the VL of SEQ ID NO: 272;
bs) the VH of SEQ ID NO: 348 and the VL of SEQ ID NO: 272;
bt) the VH of SEQ ID NO: 349 and the VL of SEQ ID NO: 272;
bu) the VH of SEQ ID NO: 350 and the VL of SEQ ID NO: 272;
bv) the VH of SEQ ID NO: 351 and the VL of SEQ ID NO: 272;
bw) the VH of SEQ ID NO: 352 and the VL of SEQ ID NO: 272.
bx) the VH of SEQ ID NO: 56 and the VL of SEQ ID NO: 353;
by) the VH of SEQ ID NO: 56 and the VL of SEQ ID NO: 354;
bz) the VH of SEQ ID NO: 56 and the VL of SEQ ID NO: 355;
ca) the VH of SEQ ID NO: 56 and the VL of SEQ ID NO: 356;
cb) the VH of SEQ ID NO: 56 and the VL of SEQ ID NO: 357;
cc) the VH of SEQ ID NO: 56 and the VL of SEQ ID NO: 358;
cd) the VH of SEQ ID NO: 56 and the VL of SEQ ID NO: 359;
ce) the VH of SEQ ID NO: 56 and the VL of SEQ ID NO: 360;
cf) the VH of SEQ ID NO: 56 and the VL of SEQ ID NO: 361;
cg) the VH of SEQ ID NO: 56 and the VL of SEQ ID NO: 362;
ch) the VH of SEQ ID NO: 56 and the VL of SEQ ID NO: 363;
ci) the VH of SEQ ID NO: 56 and the VL of SEQ ID NO: 364;
cj) the VH of SEQ ID NO: 56 and the VL of SEQ ID NO: 365;
ck) the VH of SEQ ID NO: 56 and the VL of SEQ ID NO: 366;
cl) the VH of SEQ ID NO: 367 and the VL of SEQ ID NO: 85;
cm) the VH of SEQ ID NO: 368 and the VL of SEQ ID NO: 85;
cn) the VH of SEQ ID NO: 369 and the VL of SEQ ID NO: 85;
co) the VH of SEQ ID NO: 370 and the VL of SEQ ID NO: 85;
cp) the VH of SEQ ID NO: 371 and the VL of SEQ ID NO: 85;
cq) the VH of SEQ ID NO: 372 and the VL of SEQ ID NO: 85;
cr) the VH of SEQ ID NO: 373 and the VL of SEQ ID NO: 85;
cs) the VH of SEQ ID NO: 374 and the VL of SEQ ID NO: 85;
ct) the VH of SEQ ID NO: 375 and the VL of SEQ ID NO: 85;
cu) the VH of SEQ ID NO: 376 and the VL of SEQ ID NO: 85;
cv) the VH of SEQ ID NO: 377 and the VL of SEQ ID NO: 85;
cw) the VH of SEQ ID NO: 378 and the VL of SEQ ID NO: 85;

ex) the VH of SEQ ID NO: 379 and the VL of SEQ ID NO: 85;
cy) the VH of SEQ ID NO: 380 and the VL of SEQ ID NO: 85;
cz) the VH of SEQ ID NO: 381 and the VL of SEQ ID NO: 85;
da) the VH of SEQ ID NO: 382 and the VL of SEQ ID NO: 85;
db) the VH of SEQ ID NO: 53 and the VL of SEQ ID NO: 383;
de) the VH of SEQ ID NO: 53 and the VL of SEQ ID NO: 384;
dd) the VH of SEQ ID NO: 53 and the VL of SEQ ID NO: 385;
de) the VH of SEQ ID NO: 53 and the VL of SEQ ID NO: 386;
df) the VH of SEQ ID NO: 53 and the VL of SEQ ID NO: 387;
dg) the VH of SEQ ID NO: 53 and the VL of SEQ ID NO: 388;
dh) the VH of SEQ ID NO: 53 and the VL of SEQ ID NO: 389;
de) the VH of SEQ ID NO: 53 and the VL of SEQ ID NO: 390;
df) the VH of SEQ ID NO: 53 and the VL of SEQ ID NO: 391;
dg) the VH of SEQ ID NO: 53 and the VL of SEQ ID NO: 392;
dh) the VH of SEQ ID NO: 53 and the VL of SEQ ID NO: 393;
di) the VH of SEQ ID NO: 53 and the VL of SEQ ID NO: 394;
dj) the VH of SEQ ID NO: 53 and the VL of SEQ ID NO: 395;
dk) the VH of SEQ ID NO: 53 and the VL of SEQ ID NO: 396;
dl) the VH of SEQ ID NO: 53 and the VL of SEQ ID NO: 397; or
dm) the VH of SEQ ID NO: 53 and the VL of SEQ ID NO: 398.

In another aspect, the disclosure provides an isolated protein that binds CD33, where the isolated protein comprises

a) the VH of SEQ ID NO: 52 and the VL of SEQ ID NO: 81;
b) the VH of SEQ ID NO: 53 and the VL of SEQ ID NO: 82;
c) the VH of SEQ ID NO: 54 and the VL of SEQ ID NO: 83;
d) the VH of SEQ ID NO: 55 and the VL of SEQ ID NO: 84;
e) the VH of SEQ ID NO: 56 and the VL of SEQ ID NO: 85;
f) the VH of SEQ ID NO: 57 and the VL of SEQ ID NO: 86;
g) the VH of SEQ ID NO: 58 and the VL of SEQ ID NO: 87;
h) the VH of SEQ ID NO: 59 and the VL of SEQ ID NO: 87;
i) the VH of SEQ ID NO: 60 and the VL of SEQ ID NO: 87;
j) the VH of SEQ ID NO: 61 and the VL of SEQ ID NO: 88;
k) the VH of SEQ ID NO: 262 and the VL of SEQ ID NO: 271;
l) the VH of SEQ ID NO: 263 and the VL of SEQ ID NO: 272;
m) the VH of SEQ ID NO: 264 and the VL of SEQ ID NO: 273;
n) the VH of SEQ ID NO: 291 and the VL of SEQ ID NO: 271;
o) the VH of SEQ ID NO: 292 and the VL of SEQ ID NO: 271;
p) the VH of SEQ ID NO: 293 and the VL of SEQ ID NO: 271;
q) the VH of SEQ ID NO: 294 and the VL of SEQ ID NO: 271;
r) the VH of SEQ ID NO: 295 and the VL of SEQ ID NO: 271;
s) the VH of SEQ ID NO: 296 and the VL of SEQ ID NO: 271;
t) the VH of SEQ ID NO: 297 and the VL of SEQ ID NO: 271;
u) the VH of SEQ ID NO: 298 and the VL of SEQ ID NO: 271;
v) the VH of SEQ ID NO: 299 and the VL of SEQ ID NO: 271;
w) the VH of SEQ ID NO: 300 and the VL of SEQ ID NO: 271;
x) the VH of SEQ ID NO: 301 and the VL of SEQ ID NO: 271;
y) the VH of SEQ ID NO: 302 and the VL of SEQ ID NO: 271;
z) the VH of SEQ ID NO: 303 and the VL of SEQ ID NO: 271;
aa) the VH of SEQ ID NO: 304 and the VL of SEQ ID NO: 271;
ab) the VH of SEQ ID NO: 305 and the VL of SEQ ID NO: 271;
ac) the VH of SEQ ID NO: 306 and the VL of SEQ ID NO: 271;
ad) the VH of SEQ ID NO: 307 and the VL of SEQ ID NO: 271;
ae) the VH of SEQ ID NO: 308 and the VL of SEQ ID NO: 271;
af) the VH of SEQ ID NO: 309 and the VL of SEQ ID NO: 271;
ag) the VH of SEQ ID NO: 310 and the VL of SEQ ID NO: 271;
ah) the VH of SEQ ID NO: 263 and the VL of SEQ ID NO: 311;

ai) the VH of SEQ ID NO: 263 and the VL of SEQ ID NO: 312.
aj) the VH of SEQ ID NO: 263 and the VL of SEQ ID NO: 313;
ak) the VH of SEQ ID NO: 263 and the VL of SEQ ID NO: 314;
al) the VH of SEQ ID NO: 263 and the VL of SEQ ID NO: 315;
am) the VH of SEQ ID NO: 263 and the VL of SEQ ID NO: 316;
an) the VH of SEQ ID NO: 263 and the VL of SEQ ID NO: 317;
ao) the VH of SEQ ID NO: 263 and the VL of SEQ ID NO: 318;
ap) the VH of SEQ ID NO: 263 and the VL of SEQ ID NO: 319;
aq) the VH of SEQ ID NO: 263 and the VL of SEQ ID NO: 320;
ar) the VH of SEQ ID NO: 263 and the VL of SEQ ID NO: 321;
as) the VH of SEQ ID NO: 263 and the VL of SEQ ID NO: 322;
at) the VH of SEQ ID NO: 263 and the VL of SEQ ID NO: 323;
au) the VH of SEQ ID NO: 263 and the VL of SEQ ID NO: 324;
av) the VH of SEQ ID NO: 263 and the VL of SEQ ID NO: 325;
aw) the VH of SEQ ID NO: 263 and the VL of SEQ ID NO: 326;
ax) the VH of SEQ ID NO: 263 and the VL of SEQ ID NO: 327;
ay) the VH of SEQ ID NO: 263 and the VL of SEQ ID NO: 328;
az) the VH of SEQ ID NO: 263 and the VL of SEQ ID NO: 329;
ba) the VH of SEQ ID NO: 263 and the VL of SEQ ID NO: 330;
bb) the VH of SEQ ID NO: 263 and the VL of SEQ ID NO: 331;
be) the VH of SEQ ID NO: 263 and the VL of SEQ ID NO: 332;
bd) the VH of SEQ ID NO: 263 and the VL of SEQ ID NO: 333;
be) the VH of SEQ ID NO: 263 and the VL of SEQ ID NO: 334;
bf) the VH of SEQ ID NO: 263 and the VL of SEQ ID NO: 335;
bg) the VH of SEQ ID NO: 263 and the VL of SEQ ID NO: 336;
bh) the VH of SEQ ID NO: 337 and the VL of SEQ ID NO: 272;
bi) the VH of SEQ ID NO: 338 and the VL of SEQ ID NO: 272;
bj) the VH of SEQ ID NO: 339 and the VL of SEQ ID NO: 272;
bk) the VH of SEQ ID NO: 340 and the VL of SEQ ID NO: 272;
bl) the VH of SEQ ID NO: 341 and the VL of SEQ ID NO: 272;
bm) the VH of SEQ ID NO: 342 and the VL of SEQ ID NO: 272;
bn) the VH of SEQ ID NO: 343 and the VL of SEQ ID NO: 272;
bo) the VH of SEQ ID NO: 344 and the VL of SEQ ID NO: 272;
bp) the VH of SEQ ID NO: 345 and the VL of SEQ ID NO: 272;
bq) the VH of SEQ ID NO: 346 and the VL of SEQ ID NO: 272;
br) the VH of SEQ ID NO: 347 and the VL of SEQ ID NO: 272;
bs) the VH of SEQ ID NO: 348 and the VL of SEQ ID NO: 272;
bt) the VH of SEQ ID NO: 349 and the VL of SEQ ID NO: 272;
bu) the VH of SEQ ID NO: 350 and the VL of SEQ ID NO: 272;
bv) the VH of SEQ ID NO: 351 and the VL of SEQ ID NO: 272;
bw) the VH of SEQ ID NO: 352 and the VL of SEQ ID NO: 272.
bx) the VH of SEQ ID NO: 56 and the VL of SEQ ID NO: 353;
by) the VH of SEQ ID NO: 56 and the VL of SEQ ID NO: 354;
bz) the VH of SEQ ID NO: 56 and the VL of SEQ ID NO: 355;
ca) the VH of SEQ ID NO: 56 and the VL of SEQ ID NO: 356;
cb) the VH of SEQ ID NO: 56 and the VL of SEQ ID NO: 357;
cc) the VH of SEQ ID NO: 56 and the VL of SEQ ID NO: 358;
cd) the VH of SEQ ID NO: 56 and the VL of SEQ ID NO: 359;
ce) the VH of SEQ ID NO: 56 and the VL of SEQ ID NO: 360;
cf) the VH of SEQ ID NO: 56 and the VL of SEQ ID NO: 361;
cg) the VH of SEQ ID NO: 56 and the VL of SEQ ID NO: 362;
ch) the VH of SEQ ID NO: 56 and the VL of SEQ ID NO: 363;
ci) the VH of SEQ ID NO: 56 and the VL of SEQ ID NO: 364;
cj) the VH of SEQ ID NO: 56 and the VL of SEQ ID NO: 365;
ck) the VH of SEQ ID NO: 56 and the VL of SEQ ID NO: 366;
cl) the VH of SEQ ID NO: 367 and the VL of SEQ ID NO: 85;
cm) the VH of SEQ ID NO: 368 and the VL of SEQ ID NO: 85;
cn) the VH of SEQ ID NO: 369 and the VL of SEQ ID NO: 85;

co) the VH of SEQ ID NO: 370 and the VL of SEQ ID NO: 85;
cp) the VH of SEQ ID NO: 371 and the VL of SEQ ID NO: 85;
cq) the VH of SEQ ID NO: 372 and the VL of SEQ ID NO: 85;
cr) the VH of SEQ ID NO: 373 and the VL of SEQ ID NO: 85;
cs) the VH of SEQ ID NO: 374 and the VL of SEQ ID NO: 85;
ct) the VH of SEQ ID NO: 375 and the VL of SEQ ID NO: 85;
cu) the VH of SEQ ID NO: 376 and the VL of SEQ ID NO: 85;
cv) the VH of SEQ ID NO: 377 and the VL of SEQ ID NO: 85;
cw) the VH of SEQ ID NO: 378 and the VL of SEQ ID NO: 85;
ex) the VH of SEQ ID NO: 379 and the VL of SEQ ID NO: 85;
cy) the VH of SEQ ID NO: 380 and the VL of SEQ ID NO: 85;
cz) the VH of SEQ ID NO: 381 and the VL of SEQ ID NO: 85;
da) the VH of SEQ ID NO: 382 and the VL of SEQ ID NO: 85;
db) the VH of SEQ ID NO: 53 and the VL of SEQ ID NO: 383;
dc) the VH of SEQ ID NO: 53 and the VL of SEQ ID NO: 384;
dd) the VH of SEQ ID NO: 53 and the VL of SEQ ID NO: 385;
de) the VH of SEQ ID NO: 53 and the VL of SEQ ID NO: 386;
df) the VH of SEQ ID NO: 53 and the VL of SEQ ID NO: 387;
dg) the VH of SEQ ID NO: 53 and the VL of SEQ ID NO: 388;
dh) the VH of SEQ ID NO: 53 and the VL of SEQ ID NO: 389;
de) the VH of SEQ ID NO: 53 and the VL of SEQ ID NO: 390;
df) the VH of SEQ ID NO: 53 and the VL of SEQ ID NO: 391;
dg) the VH of SEQ ID NO: 53 and the VL of SEQ ID NO: 392;
dh) the VH of SEQ ID NO: 53 and the VL of SEQ ID NO: 393;
di) the VH of SEQ ID NO: 53 and the VL of SEQ ID NO: 394;
dj) the VH of SEQ ID NO: 53 and the VL of SEQ ID NO: 395;
dk) the VH of SEQ ID NO: 53 and the VL of SEQ ID NO: 396;
dl) the VH of SEQ ID NO: 53 and the VL of SEQ ID NO: 397; or
dm) the VH of SEQ ID NO: 53 and the VL of SEQ ID NO: 398.

In some embodiments, the isolated protein is a scFv, a $(scFv)_2$, a Fv, a Fab, a $F(ab')_2$, a Fd, a dAb or a VHH. In some embodiments, the isolated protein is the Fab. In some embodiments, the isolated protein is the VHH. In some embodiments, the isolated protein is the scFv. In some embodiments, the scFv comprises, from the N- to C-terminus, a VH, a first linker (L1) and a VL (VH-L1-VL) or the VL, the L1 and the VH (VL-L1-VH).

In some embodiments, the L1 comprises

a) about 5-50 amino acids;
b) about 5-40 amino acids;
c) about 10-30 amino acids; or
d) about 10-20 amino acids.

In some embodiments, the L1 comprises an amino acid sequence of any one of SEQ ID NOs: 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, or 140. In some embodiments, the L1 comprises the amino acid sequence of SEQ ID NO: 108.

In some embodiments, the isolated protein comprises the amino acid sequence of any one of SEQ ID NO: 203, 204, 205, 206, 207, 208, 209, 210, 211, 212, 213, 214, 215, 216, 217, 218, 219, 220, 221 274, 275 or 276. In particular, the amino acid sequence of SEQ ID NO: 213, 216, 219, 274, 275 or 276.

In another aspect the disclosure provides an isolated protein that binds myeloid CD33, where the isolated protein comprises:

a) a heavy chain complementarity determining region (HCDR) 1, an HCDR2 and an HCDR3 of a heavy chain variable region (VH) of SEQ ID NO: 95;
b) an HCDR1, an HCDR2 and an HCDR3 of a VH of SEQ ID NO: 96;
c) an HCDR1, an HCDR2 and an HCDR3 of a VH of SEQ ID NO: 97;
d) an HCDR1, an HCDR2 and an HCDR3 of a VH of SEQ ID NO: 170;
e) an HCDR1, an HCDR2 and an HCDR3 of a VH of SEQ ID NO: 171;
f) an HCDR1, an HCDR2 and an HCDR3 of a VH of SEQ ID NO: 175;
g) an HCDR1, an HCDR2 and an HCDR3 of a VH of SEQ ID NO: 179;

h) an HCDR1, an HCDR2 and an HCDR3 of a VH of SEQ ID NO: 181; or
i) an HCDR1, an HCDR2 and an HCDR3 of a VH of SEQ ID NO: 196.

In some embodiments, the isolated protein comprises the HCDR1, the HCDR2, and the HCDR3 of

a) SEQ ID NOs: 89, 90, and 92, respectively;
b) SEQ ID NOs: 89, 90, and 93, respectively;
c) SEQ ID NOs: 33, 91, and 94, respectively
d) SEQ ID NOs: 167, 168, and 169, respectively;
e) SEQ ID NOs: 172, 173, and 174, respectively;
f) SEQ ID NOs: 176, 177, and 178, respectively;
g) SEQ ID NOs: 89, 90, and 180, respectively; or
h) SEQ ID NOs: 89, 90, and 93, respectively.

In another aspect the disclosure provides an isolated protein of claim 32 or 33, wherein the isolated protein comprises

a) an HCDR1, an HCDR2 and an HCDR3 of a heavy chain variable region (VH) of SEQ ID NO: 95 and a light chain complementarity determining region (LCDR) 1, an LCDR2 and an LCDR3 of a light chain variable region (VL) of SEQ ID NO: 105;
b) an HCDR1, an HCDR2 and an HCDR3 of a VH of SEQ ID NO: 96 and an LCDR1, an LCDR2 and an LCDR3 of a VL of SEQ ID NO: 106;
c) an HCDR1, an HCDR2 and an HCDR3 of a VH of SEQ ID NO: 97 and an LCDR1, an LCDR2 and an LCDR3 of a VL of SEQ ID NO: 107;
d) an HCDR1, an HCDR2 and an HCDR3 of a VH of SEQ ID NO: 170 and an LCDR1, an LCDR2 and an LCDR3 of a VL of SEQ ID NO: 185;
e) an HCDR1, an HCDR2 and an HCDR3 of a VH of SEQ ID NO: 171 and an LCDR1, an LCDR2 and an LCDR3 of a VL of SEQ ID NO: 188;
f) an HCDR1, an HCDR2 and an HCDR3 of a VH of SEQ ID NO: 175 and an LCDR1, an LCDR2 and an LCDR3 of a VL of SEQ ID NO: 192;
g) an HCDR1, an HCDR2 and an HCDR3 of a VH of SEQ ID NO: 179 and an LCDR1, an LCDR2 and an LCDR3 of a VL of SEQ ID NO: 196;
h) an HCDR1, an HCDR2 and an HCDR3 of a VH of SEQ ID NO: 181 and an LCDR1, an LCDR2 and an LCDR3 of a VL of SEQ ID NO: 200; or
i) an HCDR1, an HCDR2 and an HCDR3 of a VH of SEQ ID NO: 96 and an LCDR1, an LCDR2 and an LCDR3 of a VL of SEQ ID NO: 202.

In some embodiments, the isolated protein comprises the HCDR1, the HCDR2, the HCDR3, the LCDR1, the LCDR2 and the LCDR3 of

a) SEQ ID NOs: 89, 90, 92, 98, 101, and 104, respectively;
b) SEQ ID NOs: 89, 90, 93, 99, 102, and 104, respectively;
c) SEQ ID NOs: 33, 91, 94, 100, 103, and 104, respectively;
d) SEQ ID NOs: 167, 168, 169, 182, 183, and 184, respectively;
e) SEQ ID NOs: 33, 91, 94, 186, 187, and 104, respectively;
f) SEQ ID NOs: 172, 173, 174, 189, 190, and 191, respectively;
g) SEQ ID NOs: 176, 177, 178, 193, 194, and 195, respectively;
h) SEQ ID NOs: 89, 90, 180, 197, 198, and 199, respectively;
i) SEQ ID NOs: 89, 90, 93, 201, 102, and 104, respectively.

In some embodiments, the isolated protein is a scFv, a $(scFv)_2$, a Fv, a Fab, a $F(ab')_2$, a Fd, a dAb or a VHH.
In some embodiments, the isolated protein is the Fab.
In some embodiments, the isolated protein is the VHH.
In some embodiments, the isolated protein is the scFv.
In some embodiments, the scFv comprises, from the N- to C-terminus, a VH, a first linker (L1) and a VL (VH-L1-VL) or the VL, the L1 and the VH (VL-L1-VH).
In some embodiments, the L1 comprises

a) about 5-50 amino acids;
b) about 5-40 amino acids;
c) about 10-30 amino acids; or
d) about 10-20 amino acids.

In some embodiments, the L1 comprises an amino acid sequence of any one of SEQ ID NOs: 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, or 140.

In some embodiments, the L1 comprises the amino acid sequence of SEQ ID NO: 108.

In some embodiments, the isolated protein comprises the VH of SEQ ID NOs: 95, 96, 97, 170, 171, 175, 179, 181, or 96, and the VL of SEQ ID NOs: 105, 106, 107, 185, 188, 192, 196, 200, or 202. In some embodiments, the isolated protein comprises:

a) the VH of SEQ ID NO: 95 and the VL of SEQ ID NO: 105;
b) the VH of SEQ ID NO: 96 and the VL of SEQ ID NO: 106;
c) the VH of SEQ ID NO: 97 and the VL of SEQ ID NO: 107;
d) the VH of SEQ ID NO: 170 and the VL of SEQ ID NO: 185;
e) the VH of SEQ ID NO: 171 and the VL of SEQ ID NO: 188;
f) the VH of SEQ ID NO: 175 and the VL of SEQ ID NO: 192;
g) the VH of SEQ ID NO: 179 and the VL of SEQ ID NO: 196;
h) the VH of SEQ ID NO: 181 and the VL of SEQ ID NO: 200; or
i) the VH of SEQ ID NO: 96 and the VL of SEQ ID NO: 202.

In another aspect, the disclosure provides an isolated protein that that binds CD33, where the isolated protein comprises:

a) the VH of SEQ ID NO: 95 and the VL of SEQ ID NO: 105;
b) the VH of SEQ ID NO: 96 and the VL of SEQ ID NO: 106;
c) the VH of SEQ ID NO: 97 and the VL of SEQ ID NO: 107;
d) the VH of SEQ ID NO: 170 and the VL of SEQ ID NO: 185;
e) the VH of SEQ ID NO: 171 and the VL of SEQ ID NO: 188;
f) the VH of SEQ ID NO: 175 and the VL of SEQ ID NO: 192;
g) the VH of SEQ ID NO: 179 and the VL of SEQ ID NO: 196;
h) the VH of SEQ ID NO: 181 and the VL of SEQ ID NO: 200; or
i) the VH of SEQ ID NO: 96 and the VL of SEQ ID NO: 202.

In some embodiments, the isolated protein is a scFv, a (scFv)z, a Fv, a Fab, a $F(ab')_2$, a Fd, a dAb or a VHH. In some embodiments, the isolated protein is the Fab. In some embodiments, the isolated protein is the VHH. In some embodiments, the isolated protein is the scFv. In some embodiments, the scFv comprises, from the N- to C-terminus, a VH, a first linker (L1) and a VL (VH-L1-VL) or the VL, the L1 and the VH (VL-L1-VH). In some embodiments, the L1 comprises

a) about 5-50 amino acids;
b) about 5-40 amino acids;
c) about 10-30 amino acids; or
d) about 10-20 amino acids.

In some embodiments, the L1 comprises an amino acid sequence of any one of SEQ ID NOs: 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, or 140. In some embodiments, the L1 comprises the amino acid sequence of SEQ ID NO: 108.

The disclosure provides any isolated protein described above, where the protein is conjugated to a half-life extending moiety. In some embodiments, the half-life extending moiety is an immunoglobulin (1g), a fragment of the Ig, an Ig constant region, a fragment of the Ig constant region, a Fc region, transferrin, albumin, an albumin binding domain or polyethylene glycol. In some embodiments, the isolated protein is a monospecific protein. In some embodiments, the isolated protein is a multispecific protein. In some embodiments, the multispecific protein is a bispecific protein. In some embodiments, the multispecific protein is a trispecific protein. In some embodiments, the multispecific protein further comprises an immunoglobulin (Ig) constant region or a fragment of the Ig constant region thereof. In some embodiments, the fragment of the Ig constant region comprises a Fc region. In some embodiments, the fragment of the Ig constant

region comprises a CH2 domain. In some embodiments, the fragment of the Ig constant region comprises a CH3 domain. In some embodiments, the fragment of the Ig constant region comprises the CH2 domain and the CH3 domain. In some embodiments, the fragment of the Ig constant region comprises at least portion of a hinge, the CH2 domain and the CH3 domain. In some embodiments, the fragment of the Ig constant region comprises a hinge, the CH2 domain and the CH3 domain. In some embodiments, the isolated protein is conjugated to the N-terminus of the Ig constant region or the fragment of the Ig constant region. In some embodiments, the isolated protein is conjugated to the C-terminus of the Ig constant region or the fragment of the Ig constant region. In some embodiments, the isolated protein is conjugated to the Ig constant region or the fragment of the Ig constant region via a second linker (L2). In some embodiments, the L2 comprises the amino acid sequence of any one of SEQ ID NOs: 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, or 140.

In some embodiments, the multispecific protein comprises an antigen binding domain that binds an antigen on a lymphocyte. In some embodiments, the lymphocyte is a T cell. In some embodiments, the T cell is a $CD8^+$ T cell. In some embodiments, the lymphocyte is a natural killer (NK) cell. In some embodiments, the multispecific protein comprises an antigen binding domain that binds CD3, CD3 epsilon (CD3ε), CD8, KI2L4, NKG2E, NKG2D, NKG2F, BTNL3, CD186, BTNL8, PD-1, CD195, TRGV9, or NKG2C. In some embodiments, the multispecific protein comprises an antigen binding domain that binds CD3ε. In some embodiments, the antigen binding domain that binds CD3ε comprises:

a) a heavy chain complementarity determining region 1 (HCDR1) of SEQ ID NO: 141, a HCDR2 of SEQ ID NO: 142, a HCDR3 of SEQ ID NO: 143, a light chain complementarity determining region 1 (LCDR1) of SEQ ID NO: 144, a LCDR2 of SEQ ID NO: 145 and a LCDR3 of SEQ ID NO: 146;
b) the VH of SEQ ID NO: 147 and the VL of SEQ ID NO: 148;
c) the HCDR1 of SEQ ID NO: 149, the HCDR2 of SEQ ID NO: 150, the HCDR3 of SEQ ID NO: 151, the LCDR1 of SEQ ID NO: 152, the LCDR2 of SEQ ID NO: 153 and the LCDR3 of SEQ ID NO: 154; or
d) the VH of SEQ ID NO: 155 and the VL of SEQ ID NO: 156.

In some embodiments, the multispecific protein comprises an antigen binding domain that binds TRGV9. In some embodiments, the antigen binding domain that binds TRGV9 comprises:

a) a heavy chain complementarity determining region 1 (HCDR1) of SEQ ID NO: 236, a HCDR2 of SEQ ID NO: 237, a HCDR3 of SEQ ID NO: 238, a light chain complementarity determining region 1 (LCDR1) of SEQ ID NO: 239, a LCDR2 of SEQ ID NO: 240 and a LCDR3 of SEQ ID NO: 241;
b) a heavy chain complementarity determining region 1 (HCDR1) of SEQ ID NO: 236, a HCDR2 of SEQ ID NO: 237, a HCDR3 of SEQ ID NO: 242, a light chain complementarity determining region 1 (LCDR1) of SEQ ID NO: 239, a LCDR2 of SEQ ID NO: 240 and a LCDR3 of SEQ ID NO: 241;
c) a heavy chain complementarity determining region 1 (HCDR1) of SEQ ID NO: 236, a HCDR2 of SEQ ID NO: 237, a HCDR3 of SEQ ID NO: 243, a light chain complementarity determining region 1 (LCDR1) of SEQ ID NO: 239, a LCDR2 of SEQ ID NO: 240 and a LCDR3 of SEQ ID NO: 241;
d) a heavy chain complementarity determining region 1 (HCDR1) of SEQ ID NO: 236, a HCDR2 of SEQ ID NO: 237, a HCDR3 of SEQ ID NO: 244, a light chain complementarity determining region 1 (LCDR1) of SEQ ID NO: 239, a LCDR2 of SEQ ID NO: 240 and a LCDR3 of SEQ ID NO: 241;
e) the VH of SEQ ID NO: 245 and the VL of SEQ ID NO: 249;
f) the VH of SEQ ID NO: 246 and the VL of SEQ ID NO: 249;
g) the VH of SEQ ID NO: 247 and the VL of SEQ ID NO: 249; or
h) the VH of SEQ ID NO: 248 and the VL of SEQ ID NO: 249.

In some embodiments, the Ig constant region or the fragment of the Ig constant region is an IgG1, an IgG2, an IgG3 or an IgG4 isotype. In some embodiments, the Ig constant region or the fragment of the Ig constant region comprises at least one mutation that results in reduced binding of the protein to a Fcγ receptor (FcγR). In some embodiments, the at least one mutation that results in reduced binding of the protein to the FcγR is selected from the group consisting of F234A/L235A, L234A/L235A, L234A/L235A/D265S, V234A/G237A/ P238S/H268A/V309L/A330S/P331S, F234A/L235A, S228P/F234A/ L235A, N297A, V234A/G237A, K214T/E233P/ L234V/L235A/G236-deleted/A327G/P331A/D365E/L358M, H268Q/V309L/A330S/P331S, S267E/L328F, L234F/L235E/D265A, L234A/L235A/G237A/P238S/H268A/A330S/P331S, S228P/F234A/L235A/G237A/P238S and S228P/F234A/L235A/G236-deleted/G237A/P238S, wherein residue numbering is according to the EU index. In some embodiments, the Ig constant region or the fragment of the Ig constant region comprises at least one mutation that results in enhanced binding of the protein to the FcγR. In some embodiments, the at least one mutation that results in enhanced binding of the protein to the FcγR is selected from the group consisting of S239D/I332E, S298A/E333A/K334A, F243L/R292P/Y300L, F243L/R292P/Y300L/P396L,

F243L/R292P/Y300L/V305I/P396L and G236A/S239D/I332E, wherein residue numbering is according to the EU index. In some embodiments, the FcyR is FcyRI, FcyRIIA, FcyRIIB or FcγRIII, or any combination thereof. In some embodiments, the Ig constant region of the fragment of the Ig constant region comprises at least one mutation that modulates a half-life of the protein. In some embodiments, the at least one mutation that modulates the half-life of the protein is selected from the group consisting of H435A, P257I/N434H, D376V/N434H, M252Y/S254T/T256E/H433K/N434F, T308P/N434A and H435R, wherein residue numbering is according to the EU index. In some embodiments, the protein comprises at least one mutation in a CH3 domain of the Ig constant region. In some embodiments, the at least one mutation in the CH3 domain of the Ig constant region is selected from the group consisting of T350V, L351Y, F405A, Y407V, T366Y, T366W, F405W, T394W, T394S, Y407T, Y407A, T366S/L368A/Y407V, L351Y/F405A/Y407V, T366I/K392M/T394W, F405A/Y407V, T366L/K392M/T394W, L351Y/Y407A, T366A/K409F, L351Y/Y407A, T366V/K409F, T366A/K409F, T350V/L351Y/F405A/Y407V and T350V/T366L/K392L/T394W, wherein residue numbering is according to the EU index.

The disclosure provides any isolated protein described above, where the protein is a chimeric antigen receptor (CAR). In some embodiments, the CAR comprises

a) an extracellular domain comprising any of the isolated proteins described above;
b) a transmembrane domain; and
c) an intracellular signaling domain optionally comprising at least one co-stimulatory domain.

In some embodiments, the CAR further comprises a CD8a-hinge region. In some embodiments,

a) the transmembrane domain comprises a CD8a transmembrane region (CD8a-TM) polypeptide; and
b) the intracellular signaling domain comprises a co-stimulatory domain comprising a TNF receptor superfamily member 9 (CD137) component and a primary signaling domain comprising a T-cell surface glycoprotein CD3 zeta chain (CD3z) component.

In some embodiments,

a) the CD8a-hinge region comprises an amino acid sequence that is at least 90% identical to the amino acid sequence of SEQ ID NO: 157;
b) the transmembrane domain comprises an amino acid sequence that is at least 90% identical to the amino acid sequence of SEQ ID NO: 162; and/or
c) the intracellular signaling domain comprises a co-stimulatory domain having an amino acid sequence that is at least 90% identical to the amino acid sequence of SEQ ID NO: 163 and a primary signaling domain having an amino acid sequence that is at least 90% identical to the amino acid sequence of SEQ ID NO: 164.

In some embodiments, the intracellular signaling domain comprises a polypeptide component selected from the group consisting of a TNF receptor superfamily member 9 (CD137) component, a T-cell surface glycoprotein CD3 zeta chain (CD3z) component, a cluster of differentiation (CD27) component, a cluster of differentiation superfamily member component, or any combinations thereof.

In another aspect, the disclosure provides an isolated multispecific protein comprising a first antigen binding domain that binds CD33 and a second antigen binding domain that binds a lymphocyte antigen. In some embodiments, the lymphocyte antigen is a T cell antigen. In some embodiments, the T cell antigen is a $CD8^+$ T cell antigen. In some embodiments, the lymphocyte antigen is a NK cell antigen. In some embodiments, the lymphocyte antigen is CD3, CD3 epsilon (CD3ε), CD8, K12L4, NKG2E, NKG2D, NKG2F, BTNL3, CD186, BTNL8, PD-1, CD195, TRGV9, or NKG2C. In certain embodiments, the lymphocyte antigen is CD3ε. In some embodiments, the first antigen binding domain that binds CD33 and/or the second antigen binding domain that binds the lymphocyte antigen comprise a scFv, a (scFv)z, a Fv, a Fab, a $F(ab')_2$, a Fd, a dAb or a VHH. In some embodiments, the first antigen binding domain that binds CD33 and/or the second antigen binding domain that binds the lymphocyte antigen comprise the Fab. In some embodiments, the first antigen binding domain that binds CD33 and/or the second antigen binding domain that binds the lymphocyte antigen comprise the VHH. In some embodiments, the first antigen binding domain that binds CD33 and/or the second antigen binding domain that binds the lymphocyte antigen comprise the scFv. In some embodiments, the scFv comprises, from the N- to C-terminus, a VH, a first linker (L1) and a VL (VH-L1-VL) or the VL, the L1 and the VH (VL-L1-VH). In some embodiments, the L1 comprises

a) about 5-50 amino acids;
b) about 5-40 amino acids;
c) about 10-30 amino acids; or
d) about 10-20 amino acids.

In some embodiments, the L1 comprises the amino acid sequence of any one of SEQ ID NOs: 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, or 140. In some embodiments, the L1 comprises the amino acid sequence of SEQ ID NO: 108. In some embodiments, the first antigen binding domain that binds CD33 comprises the HCDR1 of any one of SEQ ID NOs: 1, 2, 3, 4, or 5, the HCDR2 of any one of SEQ ID NOs: 6, 7, 8, 9, 10, or 11, and the HCDR3 of any one of SEQ ID NOs: 12, 13, 14, 15, 16, or 17. In some embodiments, the first antigen binding domain that binds CD33 comprises the HCDR1, the HCDR2, the HCDR3 of

a) SEQ ID NOs: 1, 6, and 12, respectively;
b) SEQ ID NOs: 2, 7, and 13, respectively;
c) SEQ ID NOs: 1, 8, and 14, respectively;
d) SEQ ID NOs: 3, 9, and 13, respectively;
e) SEQ ID NOs: 4, 10, and 15, respectively;
f) SEQ ID NOs: 5, 11, and 16, respectively; or
g) SEQ ID NOs: 5, 11, and 17, respectively.

In some embodiments, the first antigen binding domain that binds CD33 comprises the VH of any one of SEQ ID NOs: 18, 19, 20, 21, 22, 23, 24, 25, 26, or 27. In some embodiments, the first antigen binding domain that binds CD33 comprises the HCDR1 of SEQ ID NOs: 28, 29, 30, 31, 32, 33, 34, or 35, the HCDR2 of SEQ ID NOs: 36, 37, 38, 39, 40, 41, 42, 43, or 44, the HCDR3 of SEQ ID NOs: 45, 46, 47, 48, 49, 50, or 51, the LCDR1 of SEQ ID NOs: 62, 63, 64, 65, 66, 67, or 68, the LCDR2 of SEQ ID NOs: 69, 70, 71, 72, 73, or 74, and the LCDR3 of SEQ ID NOs: 75, 76, 77, 78, 79, or 80. In some embodiments, the first antigen binding domain that binds CD33 comprises the HCDR1, the HCDR2, the HCDR3, the LCDR1, the LCDR2 and the LCDR3 of

a) SEQ ID NOs: 28, 36, 45, 62, 69, and 75, respectively;
b) SEQ ID NOs: 29, 37, 46, 63, 70, and 76, respectively;
c) SEQ ID NOs: 30, 38, 47, 64, 71, and 77, respectively;
d) SEQ ID NOs: 31, 39, 48, 65, 72, and 78, respectively;
e) SEQ ID NOs: 32, 40, 49, 66, 70, and 76, respectively;
f) SEQ ID NOs: 33, 41, 50, 67, 73, and 79, respectively;
g) SEQ ID NOs: 34, 42, 51, 68, 74, and 80, respectively;
h) SEQ ID NOs: 34, 43, 51, 68, 74, and 80, respectively;
i) SEQ ID NOs: 34, 44, 51, 68, 74, and 80, respectively;
j) SEQ ID NOs: 35, 42, 51, 68, 74, and 80, respectively:
k) SEQ ID NOs: 253, 256, 259, 265, 74 and 269, respectively:
l) SEQ ID NOs: 254, 257, 260, 66, 267 and 76, respectively: or
m) SEQ ID NOs: 255, 258, 261, 266, 268 and 270, respectively.

In some embodiments, the antigen binding domain that binds CD33 comprises the VH of SEQ ID NOs: 52, 53, 54, 55, 56, 57, 58, 59, 60, 61, 262, 263 or 264 and the VL of SEQ ID NOs: 81, 82, 83, 84, 85, 86, 87, 88, 271, 272 or 273. In some embodiments, the first antigen binding domain that binds CD33 comprises

a) the VH of SEQ ID NO: 52 and the VL of SEQ ID NO: 81;
b) the VH of SEQ ID NO: 53 and the VL of SEQ ID NO: 82;
c) the VH of SEQ ID NO: 54 and the VL of SEQ ID NO: 83;
d) the VH of SEQ ID NO: 55 and the VL of SEQ ID NO: 84;
e) the VH of SEQ ID NO: 56 and the VL of SEQ ID NO: 85;
f) the VH of SEQ ID NO: 57 and the VL of SEQ ID NO: 86;
g) the VH of SEQ ID NO: 58 and the VL of SEQ ID NO: 87;
h) the VH of SEQ ID NO: 59 and the VL of SEQ ID NO: 87;
i) the VH of SEQ ID NO: 60 and the VL of SEQ ID NO: 87;
j) the VH of SEQ ID NO: 61 and the VL of SEQ ID NO: 88;
k) the VH of SEQ ID NO: 262 and the VL of SEQ ID NO: 271;
l) the VH of SEQ ID NO: 263 and the VL of SEQ ID NO: 272; or
m) the VH of SEQ ID NO: 264 and the VL of SEQ ID NO: 273.

In some embodiments, the first antigen binding domain that binds CD33 comprises the HCDR1 of SEQ ID NOs: 33, 89, 167, 172 or 176, the HCDR2 of SEQ ID NOs: 90, 91, 168, 173, or 177, the HCDR3 of SEQ ID NOs: 92, 93, 94,

169, 174, 178, or 180, the LCDR1 of SEQ ID NOs: 98, 99, 100, 182, 186, 189, 193,197, or 201, the LCDR2 of SEQ ID NOs: 101, 102, 103, 183, 187, 190, 194, or 198, and the LCDR3 of SEQ ID NO: 104, 184, 191, 195, or 199. In some embodiments, the first antigen binding domain that binds CD33 comprises the HCDR1, the HCDR2, the HCDR3, the LCDR1, the LCDR2 and the LCDR3 of

a) SEQ ID NOs: 89, 90, 92, 98, 101, and 104, respectively;
b) SEQ ID NOs: 89, 90, 93, 99, 102, and 104, respectively;
c) SEQ ID NOs: 33, 91, 94, 100, 103, and 104, respectively;
d) SEQ ID NOs: 167, 168, 169, 182, 183, and 184, respectively;
e) SEQ ID NOs: 33, 91, 94, 186, 187, and 104, respectively;
f) SEQ ID NOs: 172, 173, 174, 189, 190, and 191, respectively;
g) SEQ ID NOs: 176, 177, 178, 193, 194, and 195, respectively;
h) SEQ ID NOs: 89, 90, 180, 197, 198, and 199, respectively; or
i) SEQ ID NOs: 89, 90, 93, 201, 102, and 104, respectively.

In some embodiments, the antigen binding domain that binds CD33 comprises the VH of SEQ ID NOs: 95, 96, 97, 170, 171, 175, 179, 181, or 96, and the VL of SEQ ID NOs 105, 106, 107, 185, 188, 192, 196, 200, or 202. In some embodiments, the first antigen binding domain that binds CD33 comprises

a) the VH of SEQ ID NO: 95 and the VL of SEQ ID NO: 105;
b) the VH of SEQ ID NO: 96 and the VL of SEQ ID NO: 106;
c) the VH of SEQ ID NO: 97 and the VL of SEQ ID NO: 107;
d) the VH of SEQ ID NO: 170 and the VL of SEQ ID NO: 185;
e) the VH of SEQ ID NO: 171 and the VL of SEQ ID NO: 188;
f) the VH of SEQ ID NO: 175 and the VL of SEQ ID NO: 192;
g) the VH of SEQ ID NO: 179 and the VL of SEQ ID NO: 196;
h) the VH of SEQ ID NO: 181 and the VL of SEQ ID NO: 200; or
i) the VH of SEQ ID NO: 96 and the VL of SEQ ID NO: 202.

In some embodiments, the second antigen binding domain that binds the lymphocyte antigen comprises

a) the HCDR1 of SEQ ID NO: 141, the HCDR2 of SEQ ID NO: 142, the HCDR3 of SEQ ID NO: 143, the LCDR1 of SEQ ID NO: 144, the LCDR2 of SEQ ID NO: 145 and the LCDR3 of SEQ ID NO: 146;
b) the VH of SEQ ID NO: 147 and the VL of SEQ ID NO: 148;
c) the HCDR1 of SEQ ID NO: 149, the HCDR2 of SEQ ID NO: 150, the HCDR3 of SEQ ID NO: 151, the LCDR1 of SEQ ID NO: 152, the LCDR2 of SEQ ID NO: 153 and the LCDR3 of SEQ ID NO: 154; or
d) the VH of SEQ ID NO: 155 and the VL of SEQ ID NO: 156.

In some embodiments, the second antigen binding domain that binds the lymphocyte antigen comprises:

a) a heavy chain complementarity determining region 1 (HCDR1) of SEQ ID NO: 236, a HCDR2 of SEQ ID NO: 237, a HCDR3 of SEQ ID NO: 238, a light chain complementarity determining region 1 (LCDR1) of SEQ ID NO: 239, a LCDR2 of SEQ ID NO: 240 and a LCDR3 of SEQ ID NO: 241;
b) a heavy chain complementarity determining region 1 (HCDR1) of SEQ ID NO: 236, a HCDR2 of SEQ ID NO: 237, a HCDR3 of SEQ ID NO: 242, a light chain complementarity determining region 1 (LCDR1) of SEQ ID NO: 239, a LCDR2 of SEQ ID NO: 240 and a LCDR3 of SEQ ID NO: 241;
c) a heavy chain complementarity determining region 1 (HCDR1) of SEQ ID NO: 236, a HCDR2 of SEQ ID NO: 237, a HCDR3 of SEQ ID NO: 243, a light chain complementarity determining region 1 (LCDR1) of SEQ ID NO: 239, a LCDR2 of SEQ ID NO: 240 and a LCDR3 of SEQ ID NO: 241;
d) a heavy chain complementarity determining region 1 (HCDR1) of SEQ ID NO: 236, a HCDR2 of SEQ ID NO: 237, a HCDR3 of SEQ ID NO: 244, a light chain complementarity determining region 1 (LCDR1) of SEQ ID NO: 239, a LCDR2 of SEQ ID NO: 240 and a LCDR3 of SEQ ID NO: 241;
e) the VH of SEQ ID NO: 245 and the VL of SEQ ID NO: 249;
f) the VH of SEQ ID NO: 246 and the VL of SEQ ID NO: 249;
g) the VH of SEQ ID NO: 247 and the VL of SEQ ID NO: 249; or
h) the VH of SEQ ID NO: 248 and the VL of SEQ ID NO: 249.

In some embodiments, the first antigen binding domain that binds CD33 is conjugated to a first immunoglobulin

(Ig) constant region or a fragment of the first Ig constant region and/or the second antigen binding domain that binds the lymphocyte antigen is conjugated to a second immunoglobulin (Ig) constant region or a fragment of the second Ig constant region. In certain embodiments, the multispecific protein further comprising a second linker (L2) between the first antigen binding domain that binds CD33 and the first Ig constant region or the fragment of the first Ig constant region and the second antigen binding domain that binds the lymphocyte antigen and the second Ig constant region or the fragment of the second Ig constant region. In some embodiments, the L2 comprises the amino acid sequence of any one of SEQ ID NOs: 108, 109, 110, 111, 112,113, 114, 115, 116, 117, 118, 119, 120, 121, 122, 123, 124, 125, 126, 127, 128, 129, 130, 131, 132, 133, 134, 135, 136, 137, 138, 139, or 140. In some embodiments, the first Ig constant region or the fragment of the first Ig constant region and the second Ig constant region or the fragment of the second Ig constant region is an IgG1, an IgG2, and IgG3 or an IgG4 isotype. In some embodiments, the first Ig constant region or the fragment of the first Ig constant region and the second Ig constant region or the fragment of the second Ig constant region comprises at least one mutation that results in reduced binding of the multispecific protein to a FcγR. In some embodiments, the at least one mutation that results in reduced binding of the multispecific protein to the FcγR is selected from the group consisting of F234A/L235A, L234A/L235A, L234A/L235A/D265S, V234A/G237A/P238S/H268A/V309L/A330S/P331S, F234A/L235A, S228P/F234A/ L235A, N297A, V234A/G237A, K214T/E233P/L234V/L235A/G236-deleted/A327G/P331A/D365E/L358M, H268Q/V309L/A330S/P331S, S267E/L328F, L234F/L235E/D265A, L234A/L235A/G237A/P238S/H268A/A330S/P331S, S228P/F234A/L235A/G237A/P238S and S228P/F234A/L235A/G236-deleted/G237A/P238S, wherein residue numbering is according to the EU index.

In some embodiments, the first Ig constant region or the fragment of the first Ig constant region and the second Ig constant region or the fragment of the second Ig constant region comprises at least one mutation that results in enhanced binding of the multispecific protein to a Fcγ receptor (FcγR). In some embodiments, the at least one mutation that results in enhanced binding of the multispecific protein to the FcγR is selected from the group consisting of S239D/I332E, S298A/E333A/K334A, F243L/R292P/Y300L, F243L/R292P/Y300L/P396L, F243L/R292P/Y300L/V305I/P396L and G236A/S239D/I332E, wherein residue numbering is according to the EU index. In some embodiments, the FcγR is FcγRI, FcγRIIA, FcγRIIB or FcγRIII, or any combination thereof. In some embodiments, the first Ig constant region or the fragment of the first Ig constant region and the second Ig constant region or the fragment of the second Ig constant region comprises at least one mutation that modulates a half-life of the multispecific protein. In some embodiments, the at least one mutation that modulates the half-life of the multispecific protein is selected from the group consisting of H435A, P257I/N434H, D376V/N434H, M252Y/S254T/T256E/H433K/N434F, T308P/N434A and H435R, wherein residue numbering is according to the EU index. In some embodiments, the isolated multispecific protein comprises at least one mutation in a CH3 domain of the first Ig constant region or in a CH3 domain of the fragment of the first Ig constant region and/or at least one mutation in a CH3 domain of the second Ig constant region or in a CH3 domain of the fragment of the second Ig constant region. In some embodiments, the at least one mutation in a CH3 domain of the first Ig constant region or in a CH3 domain of the fragment of the first Ig constant region and/or at least one mutation in a CH3 domain of the second Ig constant region or in a CH3 domain of the fragment of the second Ig constant region is selected from the group consisting of T350V, L351Y, F405A,Y407V, T366Y, T366W, F405W, T394W, T394S, Y407T, Y407A, T366S/L368A/Y407V, L351Y/F405A/Y407V, T366I/K392M/T394W, F405A/Y407V, T366L/K392M/T394W, L351Y/Y407A, T366A/K409F, L351Y/Y407A, T366V/K409F, T366A/K409F, T350V/L351Y/F405A/Y407V and T350V/T366L/K392L/T394W, wherein residue numbering is according to the EU index. In some embodiments, the first Ig constant region or the fragment of the first Ig constant region and the second Ig constant region or the fragment of the second Ig constant region comprise the following mutations:

a) L235A_L235A_D265S_T350V_L351Y_F405A_Y407V in the first Ig constant region and L235A_L235A_D265S_T350V_T366L_K392L_T394W in the second Ig constant region; or
b) L235A_L235A_D265S_T350V_T366L_K392L_T394W in the first Ig constant region and L235A_L235A_D265S_T350V_L351Y_F405A_Y407V in the second Ig constant region.

The disclosure provides an immunoconjugate comprising any of the above isolated proteins conjugated to a therapeutic agent or an imaging agent. The disclosure provides a pharmaceutical composition comprising any of the above isolated proteins and a pharmaceutically acceptable carrier. The disclosure provides a polynucleotide encoding any of the above isolated proteins. The disclosure also provides a vector comprising a polynucleotide encoding any of the above isolated proteins. The disclosure also provides a host cell comprising a vector comprising a polynucleotide encoding any of the above isolated proteins.

In another aspect the disclosure provides a method of producing any of the above isolated proteins, the method comprising culturing the host cell in conditions that the protein is expressed, and recovering the protein produced by the host cell. In another aspect the disclosure provides an immunoconjugate comprising any of the above isolated multispecific proteins conjugated to a therapeutic agent or an imaging agent.

In another aspect the disclosure provides a method of producing any of the above isolated multispecific proteins, comprising culturing any host cell described above in conditions that the multispecific protein is expressed, and recovering the multispecific protein produced by the host cell.
In another aspect the disclosure provides a method of treating a CD33 expressing cancer in a subject, comprising administering a therapeutically effective amount of any of the above isolated proteins, any of the above isolated multispecific proteins, any of the above immunoconjugates, or any of the above pharmaceutical compositions to the subject for a time sufficient to treat the CD33 expressing cancer.
In another aspect the disclosure provides a method of reducing the amount of CD33 expressing cells in a subject, comprising administering any of the above isolated proteins, any of the above isolated multispecific proteins, any of the above immunoconjugates, or any of the above pharmaceutical compositions to the subject for a time sufficient to reduce the amount of CD33 expressing cells. In some embodiments, the CD33 expressing cells are cancerous cells. In some embodiments, the CD33 expressing cells are not cancerous cells.
In another aspect the disclosure provides a method of preventing establishment of a CD33 expressing cancer in a subject, comprising administering any of the above isolated proteins, any of the above isolated multispecific proteins, any of the above immunoconjugates, or any of the above pharmaceutical compositions to the subject to prevent establishment of the CD33 expressing cancer in the subject.
In another aspect the disclosure provides a method of treating a noncancerous condition in a subject at risk of developing a CD33 expressing cancer, comprising administering any of the above isolated proteins, any of the above isolated multispecific proteins, any of the above immunoconjugates, or any of the above pharmaceutical compositions to the subject to the subject to treat the noncancerous condition.
In some embodiments of the above three aspects, the CD33 expressing cancer is a hematologic cancer. In some embodiments, the hematologic cancer is a leukemia, a lymphoma, or a multiple myeloma. In some embodiments, the hematologic cancer is acute myeloid leukemia (AML), myelodysplastic syndrome (MDS), acute lymphocytic leukemia (ALL), diffuse large B-cell lymphoma (DLBCL), chronic myeloid leukemia (CML) or blastic plasmacytoid dendritic cell neoplasm (DPDCN). In some embodiments, the isolated protein or the isolated multispecific protein is administered in combination with a second therapeutic agent. In some embodiments, the second therapeutic agent is surgery, chemotherapy, or radiation, or any combination thereof. The disclosure also provides a method of detecting the presence of a hematologic cancer in a subject, comprising administering any of the above immunoconjugates to a subject suspected to have the hematologic cancer and visualizing the biological structures to which the immunoconjugate is bound, thereby detecting the presence of the hematologic cancer.
The disclosure also provides a kit comprising any of the above isolated proteins, any of the above isolated multispecific proteins, any of the above immunoconjugates, or any of the above pharmaceutical compositions.
The disclosure also provides an anti-idiotypic antibody binding to any of the above isolated proteins.
The disclosure also provides a chimeric antigen receptor (CAR) comprising:

a) an extracellular domain comprising an antigen binding domain that binds CD33;
b) a transmembrane domain; and
c) an intracellular signaling domain optionally comprising at least one co-stimulatory domain.

In some embodiments, the CAR further comprises a CD8a-hinge region.

a) In some embodiments, the transmembrane domain comprises a CD8a transmembrane region (CD8a-TM) polypeptide; and
b) the intracellular signaling domain comprises a co-stimulatory domain comprising a TNF receptor superfamily member 9 (CD137) component and a primary signaling domain comprising a T-cell surface glycoprotein CD3 zeta chain (CD3z) component.

In some embodiments,

a) the CD8a-hinge region comprises an amino acid sequence that is at least 90% identical to the amino acid sequence of SEQ ID NO: 157;
b) the transmembrane domain comprises an amino acid sequence that is at least 90% identical to the amino acid sequence of SEQ ID NO: 162; and/or
c) the intracellular signaling domain comprises a co-stimulatory domain having an amino acid sequence that is at least 90% identical to the amino acid sequence of SEQ ID NO: 163, and a primary signaling domain having an amino acid sequence that is at least 90% identical to the amino acid sequence of SEQ ID NO: 164.

In some embodiments, the antigen binding domain that binds CD33 comprises the HCDR1 of any one of SEQ

ID NOs: 1, 2, 3, 4, or 5, the HCDR2 of any one of SEQ ID NOs: 6, 7, 8, 9, 10, or 11, and the HCDR3 of any one of SEQ ID NOs: 12, 13, 14, 15, 16, or 17.

In some embodiments, the antigen binding domain that binds CD33 comprises the HCDR1, the HCDR2, the HCDR3 of

a) SEQ ID NOs: 1, 6, and 12, respectively;
b) SEQ ID NOs: 2, 7, and 13, respectively;
c) SEQ ID NOs: 1, 8, and 14, respectively;
d) SEQ ID NOs: 3, 9, and 13, respectively;
e) SEQ ID NOs: 4, 10, and 15, respectively;
f) SEQ ID NOs: 5, 11, and 16, respectively; or
g) SEQ ID NOs: 5, 11, and 17, respectively.

In some embodiments, the antigen binding domain that binds CD33 comprises the VH of any one of SEQ ID NOs: 18, 19, 20, 21, 22, 23, 24, 25, 26, or 27. In some embodiments, the antigen binding domain that binds CD33 comprises the HCDR1 of SEQ ID NOs: 28, 29, 30, 31, 32, 33, 34, 35, 253, 254 or 255, the HCDR2 of SEQ ID NOs: 36, 37, 38, 39, 40, 41, 42, 43, 44, 256, 257 or 258, the HCDR3 of SEQ ID NOs: 45, 46, 47, 48, 49, 50, 51, 259, 260 or 261, the LCDR1 of SEQ ID NOs: 62, 63, 64, 65, 66, 67, 68, 265, or 266, the LCDR2 of SEQ ID NOs: 69, 70, 71, 72, 73, 74, 267 or 268, and the LCDR3 of SEQ ID NOs: 75, 76, 77, 78, 79, 80, 269 or 270. In some embodiments, the antigen binding domain that binds CD33 comprises the HCDR1, the HCDR2, the HCDR3, the LCDR1, the LCDR2 and the LCDR3 of

a) SEQ ID NOs: 28, 36, 45, 62, 69, and 75, respectively;
b) SEQ ID NOs: 29, 37, 46, 63, 70, and 76, respectively;
c) SEQ ID NOs: 30, 38, 47, 64, 71, and 77, respectively;
d) SEQ ID NOs: 31, 39, 48, 65, 72, and 78, respectively;
e) SEQ ID NOs: 32, 40, 49, 66, 70, and 76, respectively;
f) SEQ ID NOs: 33, 41, 50, 67, 73, and 79, respectively;
g) SEQ ID NOs: 34, 42, 51, 68, 74, and 80, respectively;
h) SEQ ID NOs: 34, 43, 51, 68, 74, and 80, respectively;
i) SEQ ID NOs: 34, 44, 51, 68, 74, and 80, respectively;
j) SEQ ID NOs: 35, 42, 51, 68, 74, and 80, respectively;
k) SEQ ID NOs: 253, 256, 259, 265, 74 and 269, respectively:
l) SEQ ID NOs: 254, 257, 260, 66, 267 and 76, respectively: or
m) SEQ ID NOs: 255, 258, 261, 266, 268 and 270, respectively.

In some embodiments, the antigen binding domain that binds CD33 comprises the VH of SEQ ID NOs: 52, 53, 54, 55, 56, 57, 58, 59, 60, 61, 262, 263 or 264 and the VL of SEQ ID NOs: 81, 82, 83, 84, 85, 86, 87, 88, 271, 272, 273. In some embodiments, the antigen binding domain that binds CD33 comprises

a) the VH of SEQ ID NO: 52 and the VL of SEQ ID NO: 81;
b) the VH of SEQ ID NO: 53 and the VL of SEQ ID NO: 82;
c) the VH of SEQ ID NO: 54 and the VL of SEQ ID NO: 83;
d) the VH of SEQ ID NO: 55 and the VL of SEQ ID NO: 84;
e) the VH of SEQ ID NO: 56 and the VL of SEQ ID NO: 85;
f) the VH of SEQ ID NO: 57 and the VL of SEQ ID NO: 86;
g) the VH of SEQ ID NO: 58 and the VL of SEQ ID NO: 87;
h) the VH of SEQ ID NO: 59 and the VL of SEQ ID NO: 87;
i) the VH of SEQ ID NO: 60 and the VL of SEQ ID NO: 87;
j) the VH of SEQ ID NO: 61 and the VL of SEQ ID NO: 88
k) the VH of SEQ ID NO: 262 and the VL of SEQ ID NO: 271;
l) the VH of SEQ ID NO: 263 and the VL of SEQ ID NO: 272; or
m) the VH of SEQ ID NO: 264 and the VL of SEQ ID NO: 273.

In some embodiments, the antigen binding domain that binds CD33 comprises the HCDR1 of SEQ ID NOs: 33, 89, 167, 172 or 176, the HCDR2 of SEQ ID NOs: 90, 91, 168, 173, or 177, the HCDR3 of SEQ ID NOs: 92, 93, 94, 169, 174, 178, or 180, the LCDR1 of SEQ ID NOs: 98, 99, 100, 182, 186, 189, 193, 197, or 201, the LCDR2 of SEQ ID NOs: 101, 102, 103, 183, 187, 190, 194, or 198, and the LCDR3 of SEQ ID NO: 104, 184, 191, 195, or 199. In some

embodiments, the antigen binding domain that binds CD33 comprises the HCDR1, the HCDR2, the HCDR3, the LCDR1, the LCDR2 and the LCDR3 of

a) SEQ ID NOs: 89, 90, 92, 98, 101, and 104, respectively;
b) SEQ ID NOs: 89, 90, 93, 99, 102, and 104, respectively;
c) SEQ ID NOs: 33, 91, 94, 100, 103, and 104, respectively;
d) SEQ ID NOs: 167, 168, 169, 182, 183, and 184, respectively;
e) SEQ ID NOs: 33, 91, 94, 186, 187, and 104, respectively;
f) SEQ ID NOs: 172, 173, 174, 189, 190, and 191, respectively;
g) SEQ ID NOs: 176, 177, 178, 193, 194, and 195, respectively;
h) SEQ ID NOs: 89, 90, 180, 197, 198, and 199, respectively; or
i) SEQ ID NOs: 89, 90, 93, 201, 102, and 104, respectively.

In some embodiments, the antigen binding domain that binds CD33 comprises the VH of SEQ ID NOs: 95, 96, 97, 170, 171, 175, 179, 181, or 96, and the VL of SEQ ID NOs 105, 106, 107, 185, 188, 192, 196, 200, or 202. In some embodiments, the antigen binding domain that binds CD33 comprises

a) the VH of SEQ ID NO: 95 and the VL of SEQ ID NO: 105;
b) the VH of SEQ ID NO: 96 and the VL of SEQ ID NO: 106;
c) the VH of SEQ ID NO: 97 and the VL of SEQ ID NO: 107;
d) the VH of SEQ ID NO: 170 and the VL of SEQ ID NO: 185;
e) the VH of SEQ ID NO: 171 and the VL of SEQ ID NO: 188;
f) the VH of SEQ ID NO: 175 and the VL of SEQ ID NO: 192;
g) the VH of SEQ ID NO: 179 and the VL of SEQ ID NO: 196;
h) the VH of SEQ ID NO: 181 and the VL of SEQ ID NO: 200; or
i) the VH of SEQ ID NO: 96 and the VL of SEQ ID NO: 202.

In some embodiments, the antigen binding domain that binds CD33 is a VHH.

In some embodiments, the antigen binding domain that binds CD33 is a scFv.

In some embodiments, the scFv comprises a first linker (L1) between the VL and the VH.

In certain embodiments, the L1 comprises an amino acid sequence of any one of SEQ ID NOs: 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, or 140.

In some embodiments, the L1 comprises the amino acid sequence of SEQ ID NO: 108.

In some embodiments, the extracellular domain of the CAR comprising the antigen binding domain that binds CD33 further comprises a signal polypeptide. In some embodiments, the intracellular signaling domain comprises a polypeptide component selected from the group consisting of a TNF receptor superfamily member 9 (CD137) component, a T-cell surface glycoprotein CD3 zeta chain (CD3z) component, a cluster of differentiation (CD27) component, a cluster of differentiation superfamily member component, and a combination thereof. In some embodiments, the CD137 component comprises the amino acid sequence of SEQ ID NO: 163. In some embodiments, the CD3z component comprises an amino acid sequence of SEQ ID NO: 164. In some embodiments, the intracellular signaling domain comprises an amino acid sequence of SEQ ID NO: 165. In some embodiments, the CD8a-TM polypeptide comprises an amino acid sequence of SEQ ID NO: 162. In some embodiments, the CD8a-hinge region comprises an amino acid sequence of SEQ ID NO: 157.

In some embodiments, the CAR is a multispecific protein. In some embodiments, the multispecific protein is a bispecific protein. In some embodiments, the multispecific protein is a trispecific protein. In some embodiments, the multispecific protein comprises an antigen binding domain that binds an antigen on a lymphocyte. In some embodiments, the lymphocyte is a T cell. In some embodiments, the T cell is a $CD8^+$ T cell. In some embodiments, the lymphocyte is a natural killer (NK) cell. In some embodiments, the multispecific protein comprises an antigen binding domain that binds CD3, CD3 epsilon (CD3ε), CD8, K12L4, NKG2E, NKG2D, NKG2F, BTNL3, CD186, BTNL8, PD-1, CD195, TRGV9, or NKG2C. In some embodiments, the multispecific protein comprises an antigen binding domain that binds CD3ε. In some embodiments, the antigen binding domain that binds CD3ε comprises:

a) a heavy chain complementarity determining region 1 (HCDR1) of SEQ ID NO: 141, a HCDR2 of SEQ ID NO: 142, a HCDR3 of SEQ ID NO: 143, a light chain complementarity determining region 1 (LCDR1) of SEQ ID NO: 144, a LCDR2 of SEQ ID NO: 145 and a LCDR3 of SEQ ID NO: 146;
b) the VH of SEQ ID NO: 147 and the VL of SEQ ID NO: 148;
c) the HCDR1 of SEQ ID NO: 149, the HCDR2 of SEQ ID NO: 150, the HCDR3 of SEQ ID NO: 151, the LCDR1 of

SEQ ID NO: 152, the LCDR2 of SEQ ID NO: 153 and the LCDR3 of SEQ ID NO: 154; or
d) the VH of SEQ ID NO: 155 and the VL of SEQ ID NO: 156.

In some embodiments, the multispecific protein comprises an antigen binding domain that binds TRGV9. In some embodiments, the antigen binding domain that binds TRGV9 comprises:

a) a heavy chain complementarity determining region 1 (HCDR1) of SEQ ID NO: 236, a HCDR2 of SEQ ID NO: 237, a HCDR3 of SEQ ID NO: 238, a light chain complementarity determining region 1 (LCDR1) of SEQ ID NO: 239, a LCDR2 of SEQ ID NO: 240 and a LCDR3 of SEQ ID NO: 241;
b) a heavy chain complementarity determining region 1 (HCDR1) of SEQ ID NO: 236, a HCDR2 of SEQ ID NO: 237, a HCDR3 of SEQ ID NO: 242, a light chain complementarity determining region 1 (LCDR1) of SEQ ID NO: 239, a LCDR2 of SEQ ID NO: 240 and a LCDR3 of SEQ ID NO: 241;
c) a heavy chain complementarity determining region 1 (HCDR1) of SEQ ID NO: 236, a HCDR2 of SEQ ID NO: 237, a HCDR3 of SEQ ID NO: 243, a light chain complementarity determining region 1 (LCDR1) of SEQ ID NO: 239, a LCDR2 of SEQ ID NO: 240 and a LCDR3 of SEQ ID NO: 241;
d) a heavy chain complementarity determining region 1 (HCDR1) of SEQ ID NO: 236, a HCDR2 of SEQ ID NO: 237, a HCDR3 of SEQ ID NO: 244, a light chain complementarity determining region 1 (LCDR1) of SEQ ID NO: 239, a LCDR2 of SEQ ID NO: 240 and a LCDR3 of SEQ ID NO: 241;
e) the VH of SEQ ID NO: 245 and the VL of SEQ ID NO: 249;
f) the VH of SEQ ID NO: 246 and the VL of SEQ ID NO: 249;
g) the VH of SEQ ID NO: 247 and the VL of SEQ ID NO: 249; or
h) the VH of SEQ ID NO: 248 and the VL of SEQ ID NO: 249.

In another aspect, the disclosure provides an isolated lymphocyte expressing any of the CARs above. In some embodiments, the lymphocyte is a T lymphocyte. In some embodiments, the lymphocyte is a natural killer (NK) cell. In another aspect, the disclosure provides an isolated polynucleotide encoding any of the above CARs. The disclosure also provides a vector comprising an isolated polynucleotide encoding any of the above CARs. The disclosure provides a host cell comprising the isolated polynucleotide or the vector. The disclosure also provides a pharmaceutical composition comprising any of the above lymphocytes and a pharmaceutically acceptable excipient.

In another aspect, the disclosure provides a method of treating a subject having a CD33 expressing cancer, the method comprising: administering a therapeutically effective amount of any of the above lymphocytes to a subject in need thereof, whereby the lymphocyte mediates killing of the CD33 expressing cancer in the subject. In some embodiments, the CD33 expressing cancer is a hematologic cancer. In some embodiments, the CD33 expressing cancer is a leukemia, a lymphoma, or a multiple myeloma. In some embodiments, the CD33 expressing cancer is acute myeloid leukemia (AML), myelodysplastic syndrome (MDS), acute lymphocytic leukemia (ALL), diffuse large B-cell lymphoma (DLBCL), chronic myeloid leukemia (CML) or blastic plasmacytoid dendritic cell neoplasm (DPDCN).

In another aspect is provided a method of targeted killing of a cancer cell, the method comprising: contacting the cancer cell with any of the above lymphocytes, whereby the lymphocyte induces targeted killing of the cancer cell. In some embodiments, the cancer cell is a hematologic cancer. In some embodiments, the CD33 expressing cancer is a leukemia, a lymphoma, or a multiple myeloma. In some embodiments, the CD33 expressing cancer is acute myeloid leukemia (AML), myelodysplastic syndrome (MDS), acute lymphocytic leukemia (ALL), diffuse large B-cell lymphoma (DLBCL), chronic myeloid leukemia (CML) or blastic plasmacytoid dendritic cell neoplasm (DPDCN).

In another aspect is provided a method of detecting the presence of a cancer in a subject, comprising:

a) contacting a cell sample obtained from the subject with any of the above CARs, thereby forming a CAR-cell complex, and
b) detecting the CAR-cell complex, wherein detection of the CAR-cell complex is indicative of the presence of the cancer in the subject.

In some embodiments, the cancer cell is a hematologic cancer. In some embodiments, the CD33 expressing cancer is a leukemia, a lymphoma, or a multiple myeloma. In some embodiments, the CD33 expressing cancer is acute myeloid leukemia (AML), myelodysplastic syndrome (MDS), acute lymphocytic leukemia (ALL), diffuse large B-cell lymphoma (DLBCL), chronic myeloid leukemia (CML) or blastic plasmacytoid dendritic cell neoplasm (DPDCN).

In another aspect is provided an immunoconjugate comprising any of the above isolated proteins conjugated to a radioactive agent. In some embodiments, the radioactive agent comprises a radiometal ion. In some embodiments, the radiometal ion is $^{32}P$, $^{47}Sc$, $^{67}Cu$, $^{77}As$, $^{89}Sr$, $^{90}Y$, $^{99}Tc$, $^{105}Rh$, $^{109}Pd$, $^{111}Ag$, $^{131}I$, $^{153}Sm$, $^{159}Gd$, $^{165}Dy$, $^{166}Ho$, $^{169}Er$, $^{177}Lu$, $^{186}Re$, $^{188}Re$, $^{194}Ir$, $^{198}Au$, $^{199}Au$, $^{211}At$, $^{212}Pb$, $^{212}Bi$, $^{213}Bi$, $^{223}Ra$, $^{225}Ac$ , $^{255}Fm$, $^{227}Th$, $^{62}Cu$, $^{64}Cu$, $^{67}Ga$, $^{68}Ga$, $^{86}Y$, $^{89}Zr$, or $^{111}In$. In certain embodiments, the radiometal ion is $^{225}Ac$, $^{111}In$ or $^{89}Zr$. In certain embodiments, the

radiometal ion is $^{225}Ac$. In some embodiments, the radiometal ion of the immunoconjugate is conjugated to a chelating moiety. In some embodiments, the chelating moiety comprises a macrocycle having the structure of formula (I):

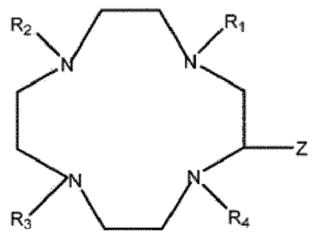

formula (I),

wherein each of $R_1$, $R_2$, $R_3$ and $R_4$ is independently $CHQCO_2X$, wherein

Q is independently hydrogen, $C_1$-$C_4$ alkyl or ($C_1$-$C_2$ alkyl) phenyl, and
X is independently hydrogen, benzyl, $C_1$-$C_4$ alkyl; and
Z is $(CH2)_nY$, wherein
n is 1-10, and
Y is an electrophilic or nucleophilic moiety covalently linked to the second click reaction partner; alternatively, Z is hydrogen; and
each of $R_1$, $R_2$, $R_3$ and $R_4$ is independently $CHQCO_2X$, wherein
Q is independently hydrogen, $C_1$-$C_4$ alkyl or ($C_1$-$C_2$ alkyl) phenyl, and
X is independently hydrogen, benzyl, $C_1$-$C_4$ alkyl, or an electrophilic or nucleophilic moiety covalently linked to the second click reaction partner;
alternatively, the chelant comprises an open chain ligand.

In some embodiments, the chelating moiety comprises a macrocycle having the structure of formula (II):

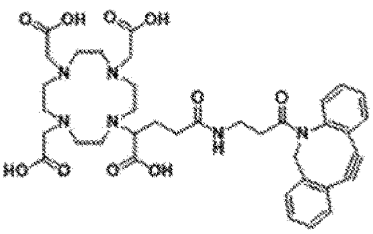

or a structure of formula (III):

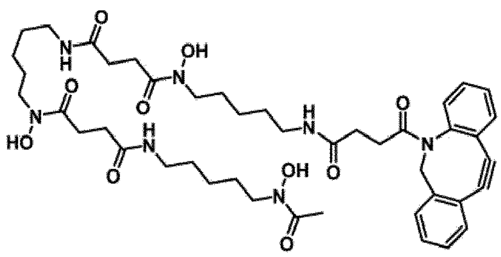

The disclosure also provides an immunoconjugate of formula (IV),

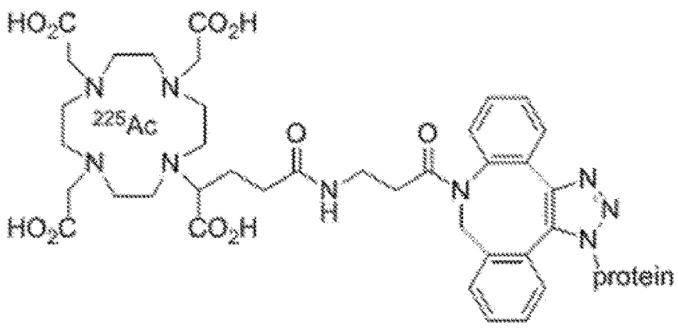

formula (IV)

where the protein is any of the above isolated proteins.

The disclosure also provides an immunoconjugate of formula (V),

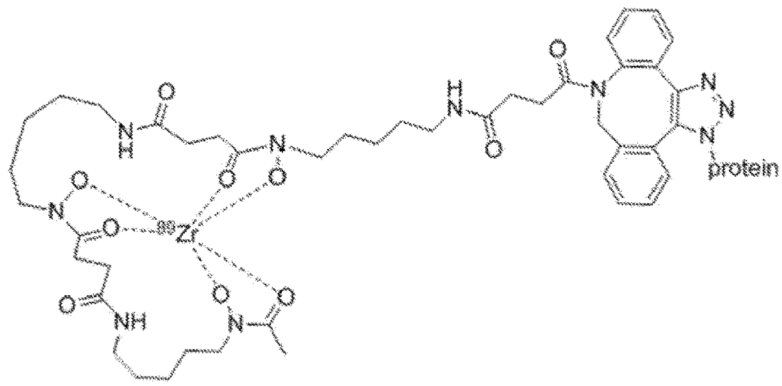

formula (V)

where the protein is any of the above isolated proteins.

The disclosure provides a pharmaceutical composition comprising a means for treating a CD33 expressing cancer in a subject.

The disclosure provides a pharmaceutical composition comprising a means for reducing the amount of CD33 expressing tumor cells in a subject.

The disclosure provides a pharmaceutical composition comprising a means for preventing establishment of a CD33 expressing cancer in a subject.

The disclosure provides a pharmaceutical composition comprising a means for treating a noncancerous condition in a subject at risk of developing a CD33 expressing cancer.

The disclosure provides a method of treating a CD33 expressing cancer in a subject, the method comprising administering to the subject a means for targeting a therapeutic agent to a CD33 expressing cancer cell in the subject.

The disclosure provides a method of reducing the amount of CD33 expressing tumor cells in a subject, the method comprising administering to the subject a means for targeting a therapeutic agent to one or more of the CD33 expressing tumor cells in the subject.

The disclosure provides a pharmaceutical composition comprising a means for preventing establishment of a CD33 expressing cancer in a subject, the method comprising administering to the subject a means for targeting a therapeutic agent to a CD33 expressing cell in the subject.

The disclosure provides a pharmaceutical composition comprising a means for treating a noncancerous condition in a subject at risk of developing a CD33 expressing cancer, the method comprising administering to the subject a means for targeting a therapeutic agent to a CD33 expressing cell in the subject.

## BRIEF DESCRIPTION OF THE DRAWINGS

This patent application file contains at least one drawing executed in color. Copies of this patent application with color drawing(s) will be provided by the Office upon request and payment of the necessary fee.

**FIG. 1** shows representative BIAcore 8K sensorgrams of anti-CD33 vHHs interactions against hu CD33 antigens (FL ECD and IgC2 domain).

**FIGS. 2A** and **2B** show binding of CD33 antibodies to target cell lines. Graphs show the non-linear regression profiles of anti-human CD33 antibodies at 4°C. Median fluorescence intensity (MFI) versus log concentration of antibody are plotted. Binding was performed by incubating the cells with the serial dilutions of the antibody at 4°C followed by detection with PE conjugated Goat F(ab')2 Anti-Human IgG - Fc antibody. The top hit for each cell line is indicated in red, and Lintuzumab binding is shown in blue curves.

**FIGS. 3** show time and temperature dependent binding of different CD33 antibodies to target cell lines. Graphs show the non-linear regression profiles of binding of anti-human CD33 antibodies at 37°C and 4°C. Median fluorescence intensity (MFI) versus log concentrations of antibody are plotted. Binding was performed by incubating the cells with the serial dilutions of the directly conjugated antibodies at 37°C and 4°C.

**FIGS. 4A-4D** show internalization of CD33 antibodies on high, medium and low expression target cell lines. Cells were cultured with serial dilutions of the CD33 antibodies along with Protein A-MMAF for 96hrs. After 96 hours, CellTitre-Glo reagent was added to cells to measure cell viability. Wells with Protein A-MMAF alone were considered as 100% viable. HDLM-2 cells were used as a no expression cell line. A decrease in cell viability indicates antibody internalization. Graphs indicate non-linear regression analysis for cell viability against log concentration of antibody.

**FIG. 5** shows kinetics of CD33 antibody internalization. CD33 antibodies were labeled with pHAb dyes and incubated with the cells at a concentration of 100nM at 37°C for the indicated time points following which the cells were washed and acquired on the flow cytometer. Graphs show MFI values at indicated time points.

**FIG. 6** shows CD33 expression on target cell lines. High, medium and low CD33 expression cell lines were identified using anti-human CD33 antibody from Biolegend. MOLM-13, Kasumi-1, OCI-AML-3 and HDLM2 cells were stained with the antibody (orange histograms) or the mouse IgG isotype control (blue histograms) and fold change over isotype for each cell line was calculated.

**FIG. 7** shows antibody integrity and DAR values post conjugation with pHAb dyes. Antibodies were labeled with pHAb dyes and analyzed on SDS-PAGE. Drug to antibody ratios (DAR) were calculated by measuring the OD at 280 and 530 nm and correcting for the dye absorbance at 280nm.

**FIGS. 8A and 8B** show specificity of pHAb mediated antibody internalization kinetics determination. In FIG. 8A, CD33 antibodies were labeled with pHAb dyes and incubated with the Kasumi-1 or OCI-AML-3 cells at a concentration of 100nM at 4°C for the indicated time points following which the cells were washed and acquired on the flow cytometer. Graphs show MFI values at indicated time points. No internalization was observed at 4C. In FIG. 8B, antibodies were also incubated with HDLM-2 cells at 37°C. No increase in MFI was observed.

**FIG. 9** shows a summary of the binding $EC_{50}$ values of CD33 antibodies to high (MOLM-13), medium (Kasumi-1) and low (OCI-AML-3) cell lines. Binding was carried out at 4°C with serial dilutions of the antibody.

**FIG. 10** shows a summary of CD33 antibody internalization on various target cell lines. For MOLM-13 and OCI-AML-3, $EC_{50}$ values are indicated. For Kasumi-1, % viability at 100nM concentration.

**FIG. 11** shows a schematic demonstrating the binding of an anti-TRGV9/anti-CD33 bispecific antibody to recruit yδ T-cells to a cancer cell that is CD33+ and to induce cancer cell death.

**FIG. 12** shows the results of an assay of the integrity of the VG4 bispecific antibody by an SDS-PAGE (non-reducing) gel.

**FIGS. 13-15** show the results of assays of the binding of anti-CD33 antibody (clone C33B904) to a panel of tumor cell lines were measured by FACS. The EC50 for: MOLM-13 (high receptor density) was 134.3 nM, Kasumi-1 (moderate density) was 82.2 nM and OCI-AML-3 (low surface density) was 16.4 nM.

**FIGS. 16-17** show the results of an experiment demonstrating that the anti-TRGV9/anti-CD33 bispecific antibody mediates yδ T cell cytotoxicity against CD33 expressing Kasumi-3 cells *in vitro.* In the experiment, enriched γδ T cells (Effectors), isolated from PBMCs cultured with Zoledronic acid + IL-2 + IL-15 for 12 days, were co-cultured with CFSE labelled Kasumi-3 cells (Targets) at 1:1 and 5:1 E:T ratios in the presence of various concentrations of the bispecific antibody for 24 hours. Dose response curves show anti-TRGV9/anti-CD33 and anti-TRGV9/anti-NULL bispecific antibodies mediated γδ T cell cytotoxicity against CD33 expressing kasumi-3 cells in a dose dependent manner at 1:1 and 5:1 E:T ratios. Cytotoxicity values represented here were subtracted of basal cytotoxicity value observed in the absence of bispecific antibody. $EC_{50}$ values were calculated as described in methods. Representative data are shown here are from a single experiment.

**FIG. 18** shows the results of experiments demonstrating that the anti-TRGV9/anti-CD33 bispecific antibody mediates γδ T cell cytotoxicity against CD33 expressing Kasumi-3 cells *in vitro.* Healthy donor derived PBMCs (Effectors), cultured with Zoledronic acid + IL-2 + IL-15 for 12 days, were co-cultured with CFSE labelled Kasumi-3 cells (Targets) at 1:1 E:T ratios in the presence of various concentrations of the bispecific antibody for 24 hours.

**FIG. 19** shows in silico analysis of potential Post-Translational Modifications (PTMs) risk. Some potential PTM risk motifs (DG, NG, DP) are highlighted.

**FIG. 20** shows the results of assays to evaluate the thermostability of scFvs generated from NNK library based on C33B1475 (SEQ ID NO: 274). ELISAs were performed on each variant in order to assess thermostability. E. coli supernatants were heat treated at 55C, 60C, 65C and luminescence signals were normalized to untreated room

temperature of the same clone.

**FIG. 21** shows the results of assay to evaluate the thermostability of scFv generated from NNK library based on C33B1516 (SEQ ID NO: 275). ELISAs were performed on each variant in order to assess thermostability. E. coli supernatants were heat treated at 55C, 60C, 65C and luminescence signals were normalized to untreated room temperature of the same clone.

**FIG. 22** shows the results of assay to evaluate the thermostability of scFv generated from NNK library based on C33B1517 (SEQ ID NO: 216). ELISAs were performed on each variant in order to assess thermostability. E. coli supernatants were heat treated at 55C, 60C, 65C and luminescence signals were normalized to untreated room temperature of the same clone.

**FIG. 23** shows the results of assay to evaluate the thermostability of scFv generated from NNK library based on C33B1522 (SEQ ID NO: 213). ELISAs were performed on each variant in order to assess thermostability. E. coli supernatants were heat treated at 55C, 60C, 65C and luminescence signals were normalized to untreated room temperature of the same clone.

**FIG. 24** is a table listing variant VH and VL sequences identified during library screen of NNK library.

## DETAILED DESCRIPTION

The disclosed methods may be understood more readily by reference to the following detailed description taken in connection with the accompanying figures, which form a part of this disclosure. It is to be understood that the disclosed methods are not limited to the specific methods described and/or shown herein, and that the terminology used herein is for the purpose of describing particular embodiments by way of example only and is not intended to be limiting of the claimed methods.

All patents, published patent applications and publications cited herein are incorporated by reference as if set forth fully herein.

When a list is presented, unless stated otherwise, it is to be understood that each individual element of that list, and every combination of that list, is a separate embodiment. For example, a list of embodiments presented as "**A, B, or C**" is to be interpreted as including the embodiments, "A," "B," "C," "A or B," "A or C," "B or C," or "A, B, or C."

As used in this specification and the appended claims, the singular forms "**a,**" "**an,**" and "**the**" include plural referents unless the content clearly dictates otherwise. Thus, for example, reference to "a cell" includes a combination of two or more cells, and the like.

The transitional terms "**comprising,**" "**consisting essentially of,**" and "**consisting of**" are intended to connote their generally accepted meanings in the patent vernacular; that is, (i) "comprising," which is synonymous with "including," "containing," or "characterized by," is inclusive or open-ended and does not exclude additional, unrecited elements or method steps; (ii) "consisting of" excludes any element, step, or ingredient not specified in the claim; and (iii) "consisting essentially of' limits the scope of a claim to the specified materials or steps "and those that do not materially affect the basic and novel characteristic(s)" of the claimed invention. Embodiments described in terms of the phrase "comprising" (or its equivalents) also provide as embodiments those independently described in terms of "consisting of" and "consisting essentially of."

"**About**" means within an acceptable error range for the particular value as determined by one of ordinary skill in the art, which will depend in part on how the value is measured or determined, i.e., the limitations of the measurement system. Unless explicitly stated otherwise within the Examples or elsewhere in the Specification in the context of a particular assay, result or embodiment, "about" means within one standard deviation per the practice in the art, or a range of up to 5%, whichever is larger.

"**Activation**" or "**stimulation**" or "**activated**" or "**stimulated**" refers to induction of a change in the biologic state of a cell resulting in expression of activation markers, cytokine production, proliferation or mediating cytotoxicity of target cells. Cells may be activated by primary stimulatory signals. Co-stimulatory signals can amplify the magnitude of the primary signals and suppress cell death following initial stimulation resulting in a more durable activation state and thus a higher cytotoxic capacity. A "co-stimulatory signal" refers to a signal, which in combination with a primary signal, such as TCR/CD3 ligation, leads to T cell and/or NK cell proliferation and/or upregulation or downregulation of key molecules.

"**Alternative scaffold**" refers to a single chain protein framework that contains a structured core associated with variable domains of high conformational tolerance. The variable domains tolerate variation to be introduced without compromising scaffold integrity, and hence the variable domains can be engineered and selected for binding to a specific antigen.

"**Antibody-dependent cellular cytotoxicity**", "**antibody-dependent cell-mediated cytotoxicity**" or "**ADCC**" refers to the mechanism of inducing cell death that depends upon the interaction of antibody-coated target cells with effector cells possessing lytic activity, such as natural killer cells (NK), monocytes, macrophages and neutrophils via Fc gamma receptors (FcγR) expressed on effector cells.

"**Antibody-dependent cellular phagocytosis**" or "**ADCP**" refers to the mechanism of elimination of antibody-coated target cells by internalization by phagocytic cells, such as macrophages or dendritic cells.

"**Antigen**" refers to any molecule (e.g., protein, peptide, polysaccharide, glycoprotein, glycolipid, nucleic acid, portions thereof, or combinations thereof) capable of being bound by an antigen binding domain or a T-cell receptor that is capable of mediating an immune response. Exemplary immune responses include antibody production and activation of immune cells, such as T cells, B cells or NK cells. Antigens may be expressed by genes, synthetized, or purified from biological samples such as a tissue sample, a tumor sample, a cell or a fluid with other biological components, organisms, subunits of proteins/antigens, killed or inactivated whole cells or lysates.

"**Antigen binding fragment**" or "**antigen binding domain**" refers to a portion of the protein that binds an antigen. Antigen binding fragments may be synthetic, enzymatically obtainable or genetically engineered polypeptides and include portions of an immunoglobulin that bind an antigen, such as VH, the VL, the VH and the VL, Fab, Fab', $F(ab')_2$, Fd and Fv fragments, domain antibodies (dAb) consisting of one VH domain or one VL domain, shark variable IgNAR domains, camelized VH domains, VHH domains, minimal recognition units consisting of the amino acid residues that mimic the CDRs of an antibody, such as FR3-CDR3-FR4 portions, the HCDR1, the HCDR2 and/or the HCDR3 and the LCDR1, the LCDR2 and/or the LCDR3, alternative scaffolds that bind an antigen, and multispecific proteins comprising the antigen binding fragments. Antigen binding fragments (such as VH and VL) may be linked together via a synthetic linker to form various types of single antibody designs where the VH/VL domains may pair intramolecularly, or intermolecularly in those cases when the VH and VL domains are expressed by separate single chains, to form a monovalent antigen binding domain, such as single chain Fv (scFv) or diabody. Antigen binding fragments may also be conjugated to other antibodies, proteins, antigen binding fragments or alternative scaffolds which may be monospecific or multispecific to engineer bispecific and multispecific proteins.

"**Antibodies**" is meant in a broad sense and includes immunoglobulin molecules including monoclonal antibodies including murine, human, humanized and chimeric monoclonal antibodies, antigen binding fragments, multispecific antibodies, such as bispecific, trispecific, tetraspecific etc., dimeric, tetrameric or multimeric antibodies, single chain antibodies, domain antibodies and any other modified configuration of the immunoglobulin molecule that comprises an antigen binding site of the required specificity. "Full length antibodies" are comprised of two heavy chains (HC) and two light chains (LC) inter-connected by disulfide bonds as well as multimers thereof (e.g. IgM). Each heavy chain is comprised of a heavy chain variable region (VH) and a heavy chain constant region (comprised of domains CH1, hinge, CH2 and CH3). Each light chain is comprised of a light chain variable region (VL) and a light chain constant region (CL). The VH and the VL regions may be further subdivided into regions of hypervariability, termed complementarity determining regions (CDR), interspersed with framework regions (FR). Each VH and VL is composed of three CDRs and four FR segments, arranged from amino-to-carboxy-terminus in the following order: FR1, CDR1, FR2, CDR2, FR3, CDR3 and FR4. Immunoglobulins may be assigned to five major classes, IgA, IgD, IgE, IgG and IgM, depending on the heavy chain constant domain amino acid sequence. IgA and IgG are further sub-classified as the isotypes IgA1, IgA2, IgG1, IgG2, IgG3 and IgG4. Antibody light chains of any vertebrate species may be assigned to one of two clearly distinct types, namely kappa ($\kappa$) and lambda ($\lambda$), based on the amino acid sequences of their constant domains.

"**Bispecific**" refers to a molecule (such as an antibody) that specifically binds two distinct antigens or two distinct epitopes within the same antigen. The bispecific molecule may have cross-reactivity to other related antigens, for example to the same antigen from other species (homologs), such as human or monkey, for example *Macaca cynomolgus* (cynomolgus, cyno) or *Pan troglodytes,* or may bind an epitope that is shared between two or more distinct antigens.

"**Cancer**" refers to a broad group of various diseases characterized by the uncontrolled growth of abnormal cells in the body. Unregulated cell division and growth results in the formation of malignant tumors that invade neighboring tissues and may also metastasize to distant parts of the body through the lymphatic system or bloodstream. A "cancer" or "cancer tissue" can include a tumor.

"**Chimeric antigen receptor**" (CAR) as used herein is defined as a cell-surface receptor comprising an extracellular target-binding domain, a transmembrane domain and an intracellular signaling domain, all in a combination that is not naturally found together on a single protein. This includes receptors wherein the extracellular domain and the intracellular signaling domain are not naturally found together on a single receptor protein. CARs are intended primarily for use with lymphocyte such as T cells and natural killer (NK) cells.

"**Complement-dependent cytotoxicity**" or "**CDC**", refers to the mechanism of inducing cell death in which the Fc effector domain of a target-bound protein binds and activates complement component C1q which in turn activates the complement cascade leading to target cell death. Activation of complement may also result in deposition of complement components on the target cell surface that facilitate CDC by binding complement receptors (e.g., CR3) on leukocytes

"**Complementarity determining regions**" (CDR) are antibody regions that bind an antigen. There are three CDRs in the VH (HCDR1, HCDR2, HCDR3) and three CDRs in the VL (LCDR1, LCDR2, LCDR3). CDRs may be defined using various delineations such as Kabat (Wu et al. (1970) J Exp Med 132: 211-50; Kabat et al., Sequences of Proteins of Immunological Interest, 5th Ed. Public Health Service, National Institutes of Health, Bethesda, Md., 1991), Chothia (Chothia et al. (1987) J Mol Biol 196: 901-17), IMGT (Lefranc et al. (2003) Dev Comp Immunol 27: 55-77), AbM (Martin and Thornton J Bmol Biol 263: 800-15, 1996), and Contact, which is based on an analysis of the available complex

crystal structures (MacCallum, R. M., Martin, A. C. R. and Thornton, J. T. "Antibody-antigen interactions: Contact analysis and binding site topography" J. Mol. Biol. 262:732-745). The correspondence between the various delineations and variable region numbering is described (see e.g. Lefranc et al. (2003) Dev Comp Immunol 27: 55-77; Honegger and Pluckthun, J Mol Biol (2001) 309:657-70; International ImMunoGeneTics (IMGT) database; Web resources, www.imgt.org). Available programs such as abYsis by UCL Business PLC may be used to delineate CDRs. The term "CDR", "HCDR1", "HCDR2", "HCDR3", "LCDR1", "LCDR2" and "LCDR3" as used herein includes CDRs defined by any of the methods described supra, Kabat, Chothia, IMGT, AbM, or Contact, unless otherwise explicitly stated in the specification. The CDRs of the sequences with SEQ ID NO. 1 to 17, 28 to 51, 62 to 80, 89 to 94, 98 to 104, 141 to 146, 149 to 154, 167 to 169, 172 to 174, 176 to 178, 180, 182 to 184, 186, 187, 189 to 191, 193 to 195, 197 to 199, 201, 236 to 244, 253 to 261 and 265 to 270 are defined according to Kabat. The CDRs of the sequences with SEQ ID NO. 399 to 434 are defined according to AbM. The CDRs of the sequences with SEQ ID NO. 435 to 470, are defined according to Chothia. The CDRs of the sequences with SEQ ID NO. 471 to 506are defined according to IMGT. The CDRs of the sequences with SEQ ID NO. 507 to 542are defined according to Contact.

"**Decrease,**" "**lower,**" "**lessen,**" "**reduce,**" or "**abate**" refers generally to the ability of a test molecule to mediate a reduced response (*i.e.*, downstream effect) when compared to the response mediated by a control or a vehicle. Exemplary responses are T cell expansion, T cell activation or T-cell mediated tumor cell killing or binding of a protein to its antigen or receptor, enhanced binding to a Fcγ or enhanced Fc effector functions such as enhanced ADCC, CDC and/or ADCP. Decrease may be a statistically significant difference in the measured response between the test molecule and the control (or the vehicle), or a decrease in the measured response, such as a decrease of about 1.1, 12, 1.5, 2, 3, 4, 5, 6, 7, 8, 9, 10, 15, 20 or 30 fold or more, such as 500, 600, 700, 800, 900 or 1000 fold or more (including all integers and decimal points in between and above 1, e.g., 1.5, 1.6, 1.7. 1.8, etc.).

"**Differentiation**" refers to a method of decreasing the potency or proliferation of a cell or moving the cell to a more developmentally restricted state.

"**Encode**" or "**encoding**" refers to the inherent property of specific sequences of nucleotides in a polynucleotide, such as a gene, a cDNA, or an mRNA, to serve as templates for synthesis of other polymers and macromolecules in biological processes having either a defined sequence of nucleotides (e.g., rRNA, tRNA and mRNA) or a defined sequence of amino acids and the biological properties resulting therefrom. Thus, a gene, cDNA, or RNA, encodes a protein if transcription and translation of mRNA corresponding to that gene produces the protein in a cell or other biological system. Both the coding strand, the nucleotide sequence of which is identical to the mRNA sequence, and the noncoding strand, used as the template for transcription of a gene or cDNA, can be referred to as encoding the protein or other product of that gene or cDNA.

"**Enhance,**" "**promote,**" "**increase,**" "**expand**" or "**improve**" refers generally to the ability of a test molecule to mediate a greater response (*i.e.,* downstream effect) when compared to the response mediated by a control or a vehicle. Exemplary responses are T cell expansion, T cell activation or T-cell mediated tumor cell killing or binding of a protein to its antigen or receptor, enhanced binding to a Fcγ or enhanced Fc effector functions such as enhanced ADCC, CDC and/or ADCP. Enhance may be a statistically significant difference in the measured response between the test molecule and control (or vehicle), or an increase in the measured response, such as an increase of about 1.1, 1.2, 1.5, 2, 3, 4, 5, 6, 7, 8, 9, 10, 15, 20 or 30 fold or more, such as 500, 600, 700, 800, 900 or 1000 fold or more (including all integers and decimal points in between and above 1, e.g., 1.5, 1.6, 1.7. 1.8, etc.).

"**Expansion**" refers to the outcome of cell division and cell death.

"**Express**" and "**expression**" refers the to the well-known transcription and translation occurring in cells or *in vitro.* The expression product, *e.g.,* the protein, is thus expressed by the cell or *in vitro* and may be an intracellular, extracellular or a transmembrane protein.

"**Expression vector**" refers to a vector that can be utilized in a biological system or in a reconstituted biological system to direct the translation of a polypeptide encoded by a polynucleotide sequence present in the expression vector.

"**dAb**" or "**dAb fragment**" refers to an antibody fragment composed of a VH domain (Ward et al., Nature 341:544 546 (1989)).

"**Fab**" or "**Fab fragment**" refers to an antibody fragment composed of VH, CH1, VL and CL domains.

"**F(ab')$_2$**" or "**F(ab')$_2$ fragmen**t" refers to an antibody fragment containing two Fab fragments connected by a disulfide bridge in the hinge region.

"**Fd**" or "**Fd fragment**" refers to an antibody fragment composed of VH and CH1 domains.

"**Fv**" or "**Fv fragment**" refers to an antibody fragment composed of the VH and the VL domains from a single arm of the antibody.

"**Full length antibody**" is comprised of two heavy chains (HC) and two light chains (LC) inter-connected by disulfide bonds as well as multimers thereof (e.g. IgM). Each heavy chain is comprised of a heavy chain variable domain (VH) and a heavy chain constant domain, the heavy chain constant domain comprised of subdomains CH1, hinge, CH2 and CH3. Each light chain is comprised of a light chain variable domain (VL) and a light chain constant domain (CL). The VH and the VL may be further subdivided into regions of hypervariability, termed complementarity determining

regions (CDR), interspersed with framework regions (FR). Each VH and VL is composed of three CDRs and four FR segments, arranged from amino-to-carboxy-terminus in the following order: FR1, CDR1, FR2, CDR2, FR3, CDR3 and FR4.

"**Genetic modification**" refers to the introduction of a "foreign" (*i.e.,* extrinsic or extracellular) gene, DNA or RNA sequence to a host cell, so that the host cell will express the introduced gene or sequence to produce a desired substance, typically a protein or enzyme coded by the introduced gene or sequence. The introduced gene or sequence may also be called a "cloned" or "foreign" gene or sequence, may include regulatory or control sequences operably linked to polynucleotide encoding the chimeric antigen receptor, such as start, stop, promoter, signal, secretion, or other sequences used by a cell's genetic machinery. The gene or sequence may include nonfunctional sequences or sequences with no known function. A host cell that receives and expresses introduced DNA or RNA has been "genetically engineered." The DNA or RNA introduced to a host cell can come from any source, including cells of the same genus or species as the host cell, or from a different genus or species.

"**Heterologous**" refers to two or more polynucleotides or two or more polypeptides that are not found in the same relationship to each other in nature.

"**Heterologous polynucleotide**" refers to a non-naturally occurring polynucleotide that encodes two or more neoantigens as described herein.

"**Heterologous polypeptide**" refers to a non-naturally occurring polypeptide comprising two or more neoantigen polypeptides as described herein.

"**Host cell**" refers to any cell that contains a heterologous nucleic acid. An exemplary heterologous nucleic acid is a vector (*e.g.*, an expression vector).

"**Human antibody**" refers to an antibody that is optimized to have minimal immune response when administered to a human subject. Variable regions of human antibody are derived from human immunoglobulin sequences. If human antibody contains a constant region or a portion of the constant region, the constant region is also derived from human immunoglobulin sequences. Human antibody comprises heavy and light chain variable regions that are "derived from" sequences of human origin if the variable regions of the human antibody are obtained from a system that uses human germline immunoglobulin or rearranged immunoglobulin genes. Such exemplary systems are human immunoglobulin gene libraries displayed on phage, and transgenic non-human animals such as mice or rats carrying human immunoglobulin loci. "Human antibody" typically contains amino acid differences when compared to the immunoglobulins expressed in humans due to differences between the systems used to obtain the human antibody and human immunoglobulin loci, introduction of somatic mutations or intentional introduction of substitutions into the frameworks or CDRs, or both. Typically, "human antibody" is at least about 80%, 81%, 82%, 83%, 84%, 85%, 86%, 87%, 88%, 89%, 90%, 91%, 92%, 93%, 94%, 95%, 96%, 97%, 98% or 99% identical in amino acid sequence to an amino acid sequence encoded by human germline immunoglobulin or rearranged immunoglobulin genes. In some cases, "human antibody" may contain consensus framework sequences derived from human framework sequence analyses, for example as described in Knappik et al., (2000) J Mol Biol 296:57-86, or a synthetic HCDR3 incorporated into human immunoglobulin gene libraries displayed on phage, for example as described in Shi et al., (2010) J Mol Biol 397:385-96, and in Int. Patent Publ. No. WO2009/085462. Antibodies in which at least one CDR is derived from a non-human species are not included in the definition of "human antibody".

"**Humanized antibody**" refers to an antibody in which at least one CDR is derived from nonhuman species and at least one framework is derived from human immunoglobulin sequences. Humanized antibody may include substitutions in the frameworks so that the frameworks may not be exact copies of expressed human immunoglobulin or human immunoglobulin germline gene sequences.

"**In combination with**" means that two or more therapeutic agents are be administered to a subject together in a mixture, concurrently as single agents or sequentially as single agents in any order.

"**Intracellular signaling domain**" or "**cytoplasmic signaling domain**" refers to an intracellular portion of a molecule. It is the functional portion of the protein which acts by transmitting information within the cell to regulate cellular activity via defined signaling pathways by generating second messengers or functioning as effectors by responding to such messengers. The intracellular signaling domain generates a signal that promotes an immune effector function of the CAR containing cell, e.g., a CAR-T cell.

"**Isolated**" refers to a homogenous population of molecules (such as synthetic polynucleotides or polypeptides) which have been substantially separated and/or purified away from other components of the system the molecules are produced in, such as a recombinant cell, as well as a protein that has been subjected to at least one purification or isolation step. "Isolated" refers to a molecule that is substantially free of other cellular material and/or chemicals and encompasses molecules that are isolated to a higher purity, such as to 80%, 81%, 82%, 83%, 84%, 85%, 86%, 87%, 88%, 89%, 90%, 91%, 92%, 93%, 94%, 95%, 96%, 97%, 98%, 99% or 100% purity.

"**Modulate**" refers to either enhanced or decreased ability of a test molecule to mediate an enhanced or a reduced response (*i.e.,* downstream effect) when compared to the response mediated by a control or a vehicle.

"**Monoclonal antibody**" refers to an antibody obtained from a substantially homogenous population of antibody

molecules, i.e., the individual antibodies comprising the population are identical except for possible well-known alterations such as removal of C-terminal lysine from the antibody heavy chain or post-translational modifications such as amino acid isomerization or deamidation, methionine oxidation or asparagine or glutamine deamidation. Monoclonal antibodies typically bind one antigenic epitope. A bispecific monoclonal antibody binds two distinct antigenic epitopes. Monoclonal antibodies may have heterogeneous glycosylation within the antibody population. Monoclonal antibody may be monospecific or multispecific such as bispecific, monovalent, bivalent or multivalent.

"**Multispecific**" refers to a molecule, such as an antibody that specifically binds two or more distinct antigens or two or more distinct epitopes within the same antigen. Multispecific molecule may have cross-reactivity to other related antigens, for example to the same antigen from other species (homologs), such as human or monkey, for example *Macaca fascicularis* (cynomolgus, cyno) or *Pan troglodytes,* or may bind an epitope that is shared between two or more distinct antigens.

"**Natural killer cel**l" and "**NK cell**" are used interchangeably and synonymously herein. NK cell refers to a differentiated lymphocyte with a $CD16^+CD56^+$ and/or $CD57^+$ $TCR^-$ phenotype. NK cells are characterized by their ability to bind to and kill cells that fail to express "self' MHC/HLA antigens by the activation of specific cytolytic enzymes, the ability to kill tumor cells or other diseased cells that express a ligand for NK activating receptors, and the ability to release protein molecules called cytokines that stimulate or inhibit the immune response.

"**Operatively linked**" and similar phrases, when used in reference to nucleic acids or amino acids, refers to the operational linkage of nucleic acid sequences or amino acid sequence, respectively, placed in functional relationships with each other. For example, an operatively linked promoter, enhancer elements, open reading frame, 5' and 3' UTR, and terminator sequences result in the accurate production of a nucleic acid molecule (e.g., RNA) and in some instances to the production of a polypeptide (i.e., expression of the open reading frame). Operatively linked peptide refers to a peptide in which the functional domains of the peptide are placed with appropriate distance from each other to impart the intended function of each domain.

"**Pharmaceutical combination**" refers to a combination of two or more active ingredients administered either together or separately.

"**Pharmaceutical composition**" refers to a composition that results from combining an active ingredient and a pharmaceutically acceptable carrier.

"**Pharmaceutically acceptable carrier**" or "excipient" refers to an ingredient in a pharmaceutical composition, other than the active ingredient, which is nontoxic to a subject. Exemplary pharmaceutically acceptable carriers are a buffer, stabilizer or preservative.

"**Polynucleotide**" or "**nucleic acid**" refers to a synthetic molecule comprising a chain of nucleotides covalently linked by a sugar-phosphate backbone or other equivalent covalent chemistry. cDNA is a typical example of a polynucleotide. Polynucleotide may be a DNA or a RNA molecule.

"**Prevent,**" "**preventing,**" "**prevention,**" or "p**rophylaxis**" of a disease or disorder means preventing that a disorder occurs in a subject.

**"Proliferation"** refers to an increase in cell division, either symmetric or asymmetric division of cells.

"**Promoter**" refers to the minimal sequences required to initiate transcription. Promoter may also include enhancers or repressor elements which enhance or suppress transcription, respectively.

"**Protein**" or "**polypeptide**" are used interchangeably herein are refers to a molecule that comprises one or more polypeptides each comprised of at least two amino acid residues linked by a peptide bond. Protein may be a monomer, or may be protein complex of two or more subunits, the subunits being identical or distinct. Small polypeptides of less than 50 amino acids may be referred to as "peptides". Protein may be a heterologous fusion protein, a glycoprotein, or a protein modified by post-translational modifications such as phosphorylation, acetylation, myristoylation, palmitoylation, glycosylation, oxidation, formylation, amidation, citrullination, polyglutamylation, ADP-ribosylation, pegylation or biotinylation. Protein may be recombinantly expressed.

"**Recombinant**" refers to polynucleotides, polypeptides, vectors, viruses and other macromolecules that are prepared, expressed, created or isolated by recombinant means.

"**Regulatory element**" refers to any cis-or trans acting genetic element that controls some aspect of the expression of nucleic acid sequences.

"**Relapsed**" refers to the return of a disease or the signs and symptoms of a disease after a period of improvement after prior treatment with a therapeutic.

"**Refractory**" refers to a disease that does not respond to a treatment. A refractory disease can be resistant to a treatment before or at the beginning of the treatment, or a refractory disease can become resistant during a treatment.

"**Single chain Fv**" or "**scFv**" refers to a fusion protein comprising at least one antibody fragment comprising a light chain variable region (VL) and at least one antibody fragment comprising a heavy chain variable region (VH), wherein the VL and the VH are contiguously linked via a polypeptide linker, and capable of being expressed as a single chain polypeptide. Unless specified, as used herein, a scFv may have the VL and VH variable regions in either order, e.g., with respect to the N- terminal and C-terminal ends of the polypeptide, the scFv may comprise VL-linker-VH or may

comprise VH-linker-VL.

"**Specifically binds,**" "**specific binding,**" "**specifically binding**" or "**binds**" refer to a proteinaceous molecule binding to an antigen or an epitope within the antigen with greater affinity than for other antigens. Typically, the proteinaceous molecule binds to the antigen or the epitope within the antigen with an equilibrium dissociation constant ($K_D$) of about $1\times10^{-7}$ M or less, for example about $5\times10^{-8}$ M or less, about $1\times10^{-8}$ M or less, about $1\times10^{-9}$ M or less, about $1\times10^{-10}$ M or less, about $1\times10^{-11}$ M or less, or about $1\times10^{-12}$ M or less, typically with the $K_D$ that is at least one hundred fold less than its $K_D$ for binding to a non-specific antigen (e.g., BSA, casein).

"**Subject**" includes any human or nonhuman animal. "Nonhuman animal" includes all vertebrates, e.g., mammals and non-mammals, such as nonhuman primates, sheep, dogs, cats, horses, cows, chickens, amphibians, reptiles, etc. The terms "subject" and "patient" can be used interchangeably herein.

"**T cell**" and "**T lymphocyte**" are interchangeable and used synonymously herein. T cell includes thymocytes, naive T lymphocytes, memory T cells, immature T lymphocytes, mature T lymphocytes, resting T lymphocytes, or activated T lymphocytes. A T cell can be a T helper (Th) cell, for example a T helper 1 (Th1) or a T helper 2 (Th2) cell. The T cell can be a helper T cell (HTL; $CD4^+$ T cell) $CD4^+$ T cell, a cytotoxic T cell (CTL; $CD8^+$ T cell), a tumor infiltrating cytotoxic T cell (TIL; $CD8^+$ T cell), $CD4^+CD8^+$ T cell, or any other subset of T cells. Also included are "NKT cells", which refer to a specialized population of T cells that express a semi-invariant $\alpha\beta$ T-cell receptor, but also express a variety of molecular markers that are typically associated with NK cells, such as NK1.1. NKT cells include $NK1.1^+$ and $NK1.1^-$, as well as $CD4^+$, $CD4^-$, $CD8^+$ and $CD8^-$ cells. The TCR on NKT cells is unique in that it recognizes glycolipid antigens presented by the MHC I-like molecule CD Id. NKT cells can have either protective or deleterious effects due to their abilities to produce cytokines that promote either inflammation or immune tolerance. Also included are "gamma-delta T cells ($\gamma\delta$ T cells)," which refer to a specialized population that to a small subset of T cells possessing a distinct TCR on their surface, and unlike the majority of T cells in which the TCR is composed of two glycoprotein chains designated $\alpha$- and $\beta$-TCR chains, the TCR in $\gamma\delta$ T cells is made up of a $\gamma$-chain and a $\delta$-chain . $\gamma\delta$ T cells can play a role in immunosurveillance and immunoregulation, and were found to be an important source of IL-17 and to induce robust $CD8^+$ cytotoxic T cell response. Also included are "regulatory T cells" or "Tregs" which refer to T cells that suppress an abnormal or excessive immune response and play a role in immune tolerance. Tregs are typically transcription factor Foxp3-positive $CD4^+$T cells and can also include transcription factor Foxp3-negative regulatory T cells that are IL-10-producing $CD4^+$T cells.

"**Therapeutically effective amount**" or "**effective amount**" used interchangeably herein, refers to an amount effective, at dosages and for periods of time necessary, to achieve a desired therapeutic result. A therapeutically effective amount may vary according to factors such as the disease state, age, sex, and weight of the individual, and the ability of a therapeutic or a combination of therapeutics to elicit a desired response in the individual. Example indicators of an effective therapeutic or combination of therapeutics that include, for example, improved wellbeing of the patient, reduction of a tumor burden, arrested or slowed growth of a tumor, and/or absence of metastasis of cancer cells to other locations in the body.

"**Transduction**" refers to the introduction of a foreign nucleic acid into a cell using a viral vector.

"**Treat,**" "**treating**" or "**treatment**" of a disease or disorder such as cancer refers to accomplishing one or more of the following: reducing the severity and/or duration of the disorder, inhibiting worsening of symptoms characteristic of the disorder being treated, limiting or preventing recurrence of the disorder in subjects that have previously had the disorder, or limiting or preventing recurrence of symptoms in subjects that were previously symptomatic for the disorder.

"**Tumor cell**" or a "**cancer cell**" refers to a cancerous, pre-cancerous or transformed cell, either *in vivo, ex vivo,* or in tissue culture, that has spontaneous or induced phenotypic changes. These changes do not necessarily involve the uptake of new genetic material. Although transformation may arise from infection with a transforming virus and incorporation of new genomic nucleic acid, uptake of exogenous nucleic acid or it can also arise spontaneously or following exposure to a carcinogen, thereby mutating an endogenous gene. Transformation/cancer is exemplified by morphological changes, immortalization of cells, aberrant growth control, foci formation, proliferation, malignancy, modulation of tumor specific marker levels, invasiveness, tumor growth in suitable animal hosts such as nude mice, and the like, *in vitro, in vivo,* and *ex vivo.*

"**Variant,** "**mutant**" or "**altered**" refers to a polypeptide or a polynucleotide that differs from a reference polypeptide or a reference polynucleotide by one or more modifications, for example one or more substitutions, insertions or deletions.

The numbering of amino acid residues in the antibody constant region throughout the specification is according to the EU index as described in Kabat et al., Sequences of Proteins of Immunological Interest, 5th Ed. Public Health Service, National Institutes of Health, Bethesda, MD. (1991), unless otherwise explicitly stated.

Mutations in the Ig constant regions are referred to as follows: L351Y F405A Y407V refers to L351Y, F405A and Y407V mutations in one immunoglobulin constant region.

L351Y F405A Y407V/T394W refers to L351Y, F405A and Y407V mutations in the first Ig constant region and T394W mutation in the second Ig constant region, which are present in one multimeric protein.

## COMPOSITIONS OF MATTER

### Antigen binding domains that bind CD33

The disclosure provides antigen binding domains that bind CD33, monospecific and multispecific proteins comprising the antigen binding domains that bind CD33, chimeric antigen receptors (CAR) comprising the antigen binding domains that bind CD33, polynucleotides encoding the foregoing, vectors, host cells and methods of making and using the foregoing. The antigen binding domains that bind CD33 identified herein demonstrated improved properties in terms of improved thermostability.

The disclosure provides an isolated protein comprising an antigen binding domain that binds CD33, wherein the antigen binding domain that binds CD33comprises a heavy chain complementarity determining region (HCDR) 1, a HCDR2 and a HCDR3 of a heavy chain variable region (VH) of SEQ ID NO: 18; an HCDR1, an HCDR2 and an HCDR3 of a VH of SEQ ID NO: 19; an HCDR1, an HCDR2 and an HCDR3 of a VH of SEQ ID NO: 20; an HCDR1, an HCDR2 and an HCDR3 of a VH of SEQ ID NO: 21; an HCDR1, an HCDR2 and an HCDR3 of a VH of SEQ ID NO: 22; an HCDR1, an HCDR2 and an HCDR3 of a VH of SEQ ID NO: 23; an HCDR1, an HCDR2 and an HCDR3 of a VH of SEQ ID NO: 24; an HCDR1, an HCDR2 and an HCDR3 of a VH of SEQ ID NO: 25; an HCDR1, an HCDR2 and an HCDR3 of a VH of SEQ ID NO: 26; or an HCDR1, an HCDR2 and an HCDR3 of a VH of SEQ ID NO: 27.

Methods and techniques for identifying CDRs (e.g., HCDR1, HCDR2, HCDR3, LCDR1, LCDR2 and LCDR3) are known in the art and can be used to identify CDRs within any of the heavy chain variable regions and/or any of the light chain variable regions disclosed herein. Conventions that can be used to identify the boundaries of CDRs include, but are not limited to, the Kabat definition, the Chothia definition, the AbM definition, the IMGT definition and the Contact definition. Without wishing to be bound by theory, the Kabat definition is based on sequence variability, the Chothia definition is based on location of structural loop regions, and the AbM definition is a compromise between the Chothia and Kabat approaches.

In some embodiments, the antigen binding domain that binds CD33 comprises the VH of any one of SEQ ID NOs: 18, 19, 20, 21, 22, 23, 24, 25, 26, or 27. In some embodiments, the antigen binding domain that binds CD33 is an scFv. In some embodiments, the antigen binding domain that binds CD33 is an $(scFv)_2$. In some embodiments, the antigen binding domain that binds CD33 is an Fv. In some embodiments, the antigen binding domain that binds CD33 is an Fab. In some embodiments, the antigen binding domain that binds CD33 is an $F(ab')_2$. In some embodiments, the antigen binding domain that binds CD33 is an Fd. In some embodiments, the CD33 antigen binding domain is a dAb. In some embodiments, the CD33 antigen binding domain is a VHH.

The disclosure also provides an isolated protein isolated protein comprises the HCDR1, the HCDR2, the HCDR3 of SEQ ID NOs: 1, 6, and 12, respectively; SEQ ID NOs: 2, 7, and 13, respectively; SEQ ID NOs: 1, 8, and 14, respectively; SEQ ID NOs: 3, 9, and 13, respectively; SEQ ID NOs: 4, 10, and 15, respectively; SEQ ID NOs: 5, 11, and 16, respectively; or SEQ ID NOs: 5, 11, and 17, respectively. In some embodiments, the antigen binding domain that binds CD33 is an scFv. In some embodiments, the antigen binding domain that binds CD33 is an $(scFv)_2$. In some embodiments, the antigen binding domain that binds CD33 is an Fv. In some embodiments, the antigen binding domain that binds CD33 is a Fab. In some embodiments, the antigen binding domain that binds CD33 is an $F(ab')_2$. In some embodiments, the antigen binding domain that binds CD33 is an Fd. In some embodiments, the CD33 antigen binding domain is a dAb. In some embodiments, the CD33 antigen binding domain is a VHH.

The disclosure also provides an isolated protein comprising an antigen binding domain that binds myeloid cell surface antigen CD33, wherein the antigen binding domain that binds CD33 comprises

a) an HCDR1, an HCDR2 and an HCDR3 of a heavy chain variable region (VH) of SEQ ID NO: 52;
b) an HCDR1, an HCDR2 and an HCDR3 of a VH of SEQ ID NO: 53;
c) an HCDR1, an HCDR2 and an HCDR3 of a VH of SEQ ID NO: 54;
d) an HCDR1, an HCDR2 and an HCDR3 of a VH of SEQ ID NO: 55;
e) an HCDR1, an HCDR2 and an HCDR3 of a VH of SEQ ID NO: 56;
f) an HCDR1, an HCDR2 and an HCDR3 of a VH of SEQ ID NO: 57;
g) an HCDR1, an HCDR2 and an HCDR3 of a VH of SEQ ID NO: 58;
h) an HCDR1, an HCDR2 and an HCDR3 of a VH of SEQ ID NO: 59;
i) an HCDR1, an HCDR2 and an HCDR3 of a VH of SEQ ID NO: 60;
j) an HCDR1, an HCDR2 and an HCDR3 of a VH of SEQ ID NO: 61;
k) an HCDR1, an HCDR2 and an HCDR3 of a VH of SEQ ID NO: 262;
l) an HCDR1, an HCDR2 and an HCDR3 of a VH of SEQ ID NO: 263; or
m) an HCDR1, an HCDR2 and an HCDR3 of a VH of SEQ ID NO: 264.

In certain embodiments, the antigen binding domain that binds CD33 comprises an HCDR1, an HCDR2 and

an HCDR3 of a VH of SEQ ID NO: 53.

In other embodiments, the antigen binding domain that binds CD33 comprises an HCDR1, an HCDR2 and an HCDR3 of a VH of SEQ ID NO: 56.

In other embodiments, the the antigen binding domain that binds CD33 comprises an HCDR1, an HCDR2 and an HCDR3 of a VH of SEQ ID NO: 59.

In other embodiments, the antigen binding domain that binds CD33 comprises an HCDR1, an HCDR2 and an HCDR3 of a VH of SEQ ID NO: 262.

In other embodiments, the the antigen binding domain that binds CD33 comprises an HCDR1, an HCDR2 and an HCDR3 of a VH of SEQ ID NO: 263.

In other embodiments, the antigen binding domain that binds CD33 comprises an HCDR1, an HCDR2 and an HCDR3 of a VH of SEQ ID NO: 264.

Exemplary HCDR sequences are described in Tables 4a, 4b, 4c, 4d, 4e and 4f.

In some embodiments, the antigen binding domain that binds CD33 comprises the VH of SEQ ID NOs: 52, 53, 54, 55, 56, 57, 58, 59, 60, 61, 262, 263 or 264.

In certain embodiments, the antigen binding domain that binds CD33 comprises the VH of SEQ ID NO: 53.

In other embodiments, the antigen binding domain that binds CD33 comprises the VH of SEQ ID NO: 56.

In other embodiments, the antigen binding domain that binds CD33 comprises the VH of SEQ ID NO: 59.

In other embodiments, the antigen binding domain that binds CD33 comprises the VH of SEQ ID NO: 262.

In other embodiments, the antigen binding domain that binds CD33 comprises the VH of SEQ ID NO: 263.

In other embodiments, the antigen binding domain that binds CD33 comprises the VH of SEQ ID NO: 264.

In some embodiments, the antigen binding domain that binds CD33 comprises the VL of SEQ ID NOs: 81, 82, 83, 84, 85, 86, 87, 88, 271, 272 or 273.

In certain embodiments, the antigen binding domain that binds CD33 comprises the VL of SEQ ID NO: 82.

In other embodiments, the antigen binding domain that binds CD33 comprises the VL of SEQ ID NO: 85.

In other embodiments, the antigen binding domain that binds CD33 comprises the VL of SEQ ID NO: 87.

In other embodiments, the antigen binding domain that binds CD33 comprises the VL of SEQ ID NO: 271.

In other embodiments, the antigen binding domain that binds CD33 comprises the VL of SEQ ID NO: 272

In other embodiments, the antigen binding domain that binds CD33 comprises the VL of SEQ ID NO: 273.

In some embodiments, the antigen binding domain that binds CD33 comprises the VH of SEQ ID NOs: 52, 53, 54, 55, 56, 57, 58, 59, 60, 61, 262, 263 or 264 and the VL of SEQ ID NOs: 81, 82, 83, 84, 85, 86, 87, 88, 271, 272 or 273.

In certain embodiments, the antigen binding domain that binds CD33 comprises the VH of SEQ ID NO: 53 and the VL of SEQ ID NO: 82.

In other embodiments, the antigen binding domain that binds CD33 comprises the VH of SEQ ID NO: 56 and the VL of SEQ ID NO: 85.

In other embodiments, the antigen binding domain that binds CD33 comprises the VH of SEQ ID NO: 59 and the VL of SEQ ID NO: 87.

In other embodiments, the antigen binding domain that binds CD33 comprises the VH of SEQ ID NO: 262 and the VL of SEQ ID NO: 271.

In other embodiments, the antigen binding domain that binds CD33 comprises the VH of SEQ ID NO: 263 and the VL of SEQ ID NO: 272.

In other embodiments, the antigen binding domain that binds CD33 comprises the VH of SEQ ID NO: 264 and the VL of SEQ ID NO: 273.

In some embodiments, the antigen binding domain that binds CD33 is an scFv. In some embodiments, the antigen binding domain that binds CD33 is an $(scFv)_2$. In some embodiments, the antigen binding domain that binds CD33 is an Fv. In some embodiments, the antigen binding domain that binds CD33 is an Fab. In some embodiments, the antigen binding domain that binds CD33 is an $F(ab')_2$. In some embodiments, the antigen binding domain that binds CD33 is an Fd. In some embodiments, the CD33 antigen binding domain is a dAb. In some embodiments, the CD33 antigen binding domain is a VHH.

The disclosure also provides an isolated protein comprising an HCDR1, an HCDR2, and an HCDR3 of

a) SEQ ID NOs: 28, 36, and 45, respectively;
b) SEQ ID NOs: 29, 37, and 46, respectively;
c) SEQ ID NOs: 30, 38, and 47, respectively;
d) SEQ ID NOs: 31, 39, and 48, respectively;
e) SEQ ID NOs: 32, 40, and 49, respectively;
f) SEQ ID NOs: 33, 41, and 50, respectively;
g) SEQ ID NOs: 34, 42, and 51, respectively;
h) SEQ ID NOs: 34, 43, and 51, respectively;

i) SEQ ID NOs: 34, 44, and 51, respectively; or
j) SEQ ID NOs: 35, 42, and 51, respectively;
k) SEQ ID NOs: 253, 256 and 259, respectively;
l) SEQ ID NOs: 254, 257 and 260, respectively; or
m) SEQ ID NOs: 255, 258 and 261 respectively.

In certain embodiments, the isolated protein comprises an HCDR1, an HCDR2, and an HCDR3 of SEQ ID NOs: 29, 37, and 46, respectively.
In other embodiments, the isolated protein comprises an HCDR1, an HCDR2, and an HCDR3 of SEQ ID NOs: 32, 40, and 49, respectively.
In other embodiments, the isolated protein comprises an HCDR1, an HCDR2, and an HCDR3 of SEQ ID NOs: 34, 43, and 51, respectively.
In other embodiments, the isolated protein comprises an HCDR1, an HCDR2, and an HCDR3 of SEQ ID NOs: 253, 256, and 259, respectively.
In other embodiments, the isolated protein comprises an HCDR1, an HCDR2, and an HCDR3 of SEQ ID NOs: 254, 257, and 260, respectively.
In other embodiments, the isolated protein comprises an HCDR1, an HCDR2, and an HCDR3 of SEQ ID NOs: 255, 258, and 361, respectively.
In some embodiments, the antigen binding domain that binds CD33 comprises the VH of SEQ ID NOs: 52, 53, 54, 55, 56, 57, 58, 59, 60, 61, 262, 263 or 264.
In certain embodiments, the antigen binding domain that binds CD33 comprises the VH of SEQ ID NO: 53.
In other embodiments, the antigen binding domain that binds CD33 comprises the VH of SEQ ID NO: 56.
In other embodiments, the antigen binding domain that binds CD33 comprises the VH of SEQ ID NO: 59.
In other embodiments, the antigen binding domain that binds CD33 comprises the VH of SEQ ID NO: 262.
In other embodiments, the antigen binding domain that binds CD33 comprises the VH of SEQ ID NO: 263.
In other embodiments, the antigen binding domain that binds CD33 comprises the VH of SEQ ID NO: 264.
In some embodiments, the antigen binding domain that binds CD33 comprises the VL of SEQ ID NOs: 81, 82, 83, 84, 85, 86, 87, 88, 271, 272 or 273.
In certain embodiments, the antigen binding domain that binds CD33 comprises the VL of SEQ ID NO: 82.
In other embodiments, the antigen binding domain that binds CD33 comprises the VL of SEQ ID NO: 85.
In other embodiments, the antigen binding domain that binds CD33 comprises the VL of SEQ ID NO: 87.
In other embodiments, the antigen binding domain that binds CD33 comprises the VL of SEQ ID NO: 271.
In other embodiments, the antigen binding domain that binds CD33 comprises the VL of SEQ ID NO: 272
In other embodiments, the antigen binding domain that binds CD33 comprises the VL of SEQ ID NO: 273.
In some embodiments, the antigen binding domain that binds CD33 comprises the VH of SEQ ID NOs: 52, 53, 54, 55, 56, 57, 58, 59, 60, 61, 262, 263 or 264 and the VL of SEQ ID NOs: 81, 82, 83, 84, 85, 86, 87, 88, 271, 272 or 273.
In certain embodiments, the antigen binding domain that binds CD33 comprises the VH of SEQ ID NO: 53 and the VL of SEQ ID NO: 82.
In other embodiments, the antigen binding domain that binds CD33 comprises the VH of SEQ ID NO: 56 and the VL of SEQ ID NO: 85.
In other embodiments, the antigen binding domain that binds CD33 comprises the VH of SEQ ID NO: 59 and the VL of SEQ ID NO: 87.
In other embodiments, the antigen binding domain that binds CD33 comprises the VH of SEQ ID NO: 262 and the VL of SEQ ID NO: 271.
In other embodiments, the antigen binding domain that binds CD33 comprises the VH of SEQ ID NO: 263 and the VL of SEQ ID NO: 272.
In other embodiments, the antigen binding domain that binds CD33 comprises the VH of SEQ ID NO: 264 and the VL of SEQ ID NO: 273.
In some embodiments, the antigen binding domain that binds CD33 is an scFv. In some embodiments, the antigen binding domain that binds CD33 is an $(scFv)_2$. In some embodiments, the antigen binding domain that binds CD33 is an Fv. In some embodiments, the antigen binding domain that binds CD33 is an Fab. In some embodiments, the antigen binding domain that binds CD33 is an $F(ab')_2$. In some embodiments, the antigen binding domain that binds CD33 is an Fd. In some embodiments, the CD33 antigen binding domain is a dAb. In some embodiments, the CD33 antigen binding domain is a VHH.
The disclosure also provides an antigen binding domain that binds myeloid cell surface antigen CD33, wherein the antigen binding domain that binds CD33 comprises a heavy chain complementarity determining region (HCDR) 1, an HCDR2 and an HCDR3 of a heavy chain variable region (VH) of SEQ ID NO: 52 and a light chain complementarity determining region (LCDR) 1, an LCDR2 and an LCDR3 of a light chain variable region (VL) of SEQ ID NO: 81; an

HCDR1, an HCDR2 and an HCDR3 of a VH of SEQ ID NO: 53 and an LCDR1, an LCDR2 and an LCDR3 of a VL of SEQ ID NO: 82; an HCDR1, an HCDR2 and an HCDR3 of a VH of SEQ ID NO: 54 and an LCDR1, an LCDR2 and an LCDR3 of a VL of SEQ ID NO: 83; an HCDR1, an HCDR2 and an HCDR3 of a VH of SEQ ID NO: 55 and an LCDR1, an LCDR2 and an LCDR3 of a VL of SEQ ID NO: 84; an HCDR1, an HCDR2 and an HCDR3 of a VH of SEQ ID NO: 56 and an LCDR1, an LCDR2 and an LCDR3 of a VL of SEQ ID NO: 85; an HCDR1, an HCDR2 and an HCDR3 of a VH of SEQ ID NO: 57 and an LCDR1, an LCDR2 and an LCDR3 of a VL of SEQ ID NO: 86; an HCDR1, an HCDR2 and an HCDR3 of a VH of SEQ ID NO: 58 and an LCDR1, an LCDR2 and an LCDR3 of a VL of SEQ ID NO: 87; an HCDR1, an HCDR2 and an HCDR3 of a VH of SEQ ID NO: 59 and an LCDR1, an LCDR2 and an LCDR3 of a VL of SEQ ID NO: 87; an HCDR1, an HCDR2 and an HCDR3 of a VH of SEQ ID NO: 60 and an LCDR1, an LCDR2 and an LCDR3 of a VL of SEQ ID NO: 87; an HCDR1, an HCDR2 and an HCDR3 of a VH of SEQ ID NO: 61 and an LCDR1, an LCDR2 and an LCDR3 of a VL of SEQ ID NO: 88; an HCDR1, an HCDR2 and an HCDR3 of a VH of SEQ ID NO: 262 and an LCDR1, an LCDR2 and an LCDR3 of a VL of SEQ ID NO: 271; an HCDR1, an HCDR2 and an HCDR3 of a VH of SEQ ID NO: 263 and an LCDR1, an LCDR2 and an LCDR3 of a VL of SEQ ID NO: 272; or an HCDR1, an HCDR2 and an HCDR3 of a VH of SEQ ID NO: 264 and an LCDR1, an LCDR2 and an LCDR3 of a VL of SEQ ID NO: 273.

Exemplary HCDR and LCDR sequences are described in Tables 4a, 4b, 4c, 4d, 4e and 4f.

In certain embodiments, the antigen binding domain that binds CD33 comprises an HCDR1, an HCDR2 and an HCDR3 of a VH of SEQ ID NO: 53 and an LCDR1, an LCDR2 and an LCDR3 of a VL of SEQ ID NO: 82.

In other embodiments, the antigen binding domain that binds CD33 comprises an HCDR1, an HCDR2 and an HCDR3 of a VH of SEQ ID NO: 56 and an LCDR1, an LCDR2 and an LCDR3 of a VL of SEQ ID NO: 85.

In other embodiments, the antigen binding domain that binds CD33 comprises an HCDR1, an HCDR2 and an HCDR3 of a VH of SEQ ID NO: 59 and an LCDR1, an LCDR2 and an LCDR3 of a VL of SEQ ID NO: 87.

In other embodiments, the antigen binding domain that binds CD33 comprises an HCDR1, an HCDR2 and an HCDR3 of a VH of SEQ ID NO: 262 and an LCDR1, an LCDR2 and an LCDR3 of a VL of SEQ ID NO: 271.

In other embodiments, the antigen binding domain that binds CD33 comprises an HCDR1, an HCDR2 and an HCDR3 of a VH of SEQ ID NO: 263 and an LCDR1, an LCDR2 and an LCDR3 of a VL of SEQ ID NO: 272.

In other embodiments, the antigen binding domain that binds CD33 comprises an HCDR1, an HCDR2 and an HCDR3 of a VH of SEQ ID NO: 264 and an LCDR1, an LCDR2 and an LCDR3 of a VL of SEQ ID NO: 273.

In some embodiments, the antigen binding domain that binds CD33 is an scFv. In some embodiments, the antigen binding domain that binds CD33 is an $(scFv)_2$. In some embodiments, the antigen binding domain that binds CD33 is an Fv. In some embodiments, the antigen binding domain that binds CD33 is an Fab. In some embodiments, the antigen binding domain that binds CD33 is an $F(ab')_2$.

In some embodiments, the antigen binding domain that binds CD33 is an Fd. In some embodiments, the CD33 antigen binding domain is a dAb. In some embodiments, the CD33 antigen binding domain is a VHH.

The disclosure also provides an antigen binding domain that binds myeloid cell surface antigen CD33, wherein the antigen binding domain that binds CD33 comprises the HCDR1, the HCDR2, the HCDR3, the LCDR1, the LCDR2 and the LCDR3 of

a) SEQ ID NOs: 28, 36, 45, 62, 69, and 75, respectively;
b) SEQ ID NOs: 29, 37, 46, 63, 70, and 76, respectively;
c) SEQ ID NOs: 30, 38, 47, 64, 71, and 77, respectively;
d) SEQ ID NOs: 31, 39, 48, 65, 72, and 78, respectively;
e) SEQ ID NOs: 32, 40, 49, 66, 70, and 76, respectively;
f) SEQ ID NOs: 33, 41, 50, 67, 73, and 79, respectively;
g) SEQ ID NOs: 34, 42, 51, 68, 74, and 80, respectively;
h) SEQ ID NOs: 34, 43, 51, 68, 74, and 80, respectively;
i) SEQ ID NOs: 34, 44, 51, 68, 74, and 80, respectively;
j) SEQ ID NOs: 35, 42, 51, 68, 74, and 80, respectively;
k) SEQ ID NOs: 253, 256, 259, 265, 74 and 269, respectively;
l) SEQ ID NOs: 254, 257, 260, 66, 267 and 76, respectively;
m) SEQ ID NOs: 255, 258, 261, 266, 268 and 270, respectively.

In certain embodiments, the antigen binding domain that binds CD33 comprises the HCDR1, the HCDR2, the HCDR3, the LCDR1, the LCDR2 and the LCDR3 of SEQ ID NOs: 29, 37, 46, 63, 70, and 76, respectively.

In other embodiments, the antigen binding domain that binds CD33 comprises the HCDR1, the HCDR2, the HCDR3, the LCDR1, the LCDR2 and the LCDR3 of SEQ ID NOs: 32, 40, 49, 66, 70, and 76, respectively.

In other embodiments, the antigen binding domain that binds CD33 comprises the HCDR1, the HCDR2, the HCDR3, the LCDR1, the LCDR2 and the LCDR3 of SEQ ID NOs: 34, 43, 51, 68, 74, and 80, respectively.

In other embodiments, the antigen binding domain that binds CD33 comprises the HCDR1, the HCDR2, the

HCDR3, the LCDR1, the LCDR2 and the LCDR3 of SEQ ID NOs: 253, 256, 259, 265, 74 and 269, respectively.

In other embodiments, the antigen binding domain that binds CD33 comprises the HCDR1, the HCDR2, the HCDR3, the LCDR1, the LCDR2 and the LCDR3 of SEQ ID NOs: 254, 257, 260, 66, 267 and 76, respectively.

In other embodiments, the antigen binding domain that binds CD33 comprises the HCDR1, the HCDR2, the HCDR3, the LCDR1, the LCDR2 and the LCDR3 of SEQ ID NOs: 255, 258, 261, 266, 268 and 270, respectively.

In some embodiments, the antigen binding domain that binds CD33 comprises:

a) the VH of SEQ ID NO: 52 and the VL of SEQ ID NO: 81;
b) the VH of SEQ ID NO: 53 and the VL of SEQ ID NO: 82;
c) the VH of SEQ ID NO: 54 and the VL of SEQ ID NO: 83;
d) the VH of SEQ ID NO: 55 and the VL of SEQ ID NO: 84;
e) the VH of SEQ ID NO: 56 and the VL of SEQ ID NO: 85;
f) the VH of SEQ ID NO: 57 and the VL of SEQ ID NO: 86;
g) the VH of SEQ ID NO: 58 and the VL of SEQ ID NO: 87;
h) the VH of SEQ ID NO: 59 and the VL of SEQ ID NO: 87;
i) the VH of SEQ ID NO: 60 and the VL of SEQ ID NO: 87;
j) the VH of SEQ ID NO: 61 and the VL of SEQ ID NO: 88;
k) the VH of SEQ ID NO: 262 and the VL of SEQ ID NO: 271;
l) the VH of SEQ ID NO: 263 and the VL of SEQ ID NO: 272; or
m) the VH of SEQ ID NO: 264 and the VL of SEQ ID NO: 273.

In certain embodiments, the antigen binding domain that binds CD33 comprises the VH of SEQ ID NO: 53 and the VL of SEQ ID NO: 82.

In other embodiments, the antigen binding domain that binds CD33 comprises the VH of SEQ ID NO: 56 and the VL of SEQ ID NO: 85.

In other embodiments, the antigen binding domain that binds CD33 comprises the VH of SEQ ID NO: 59 and the VL of SEQ ID NO: 87.

In other embodiments, the antigen binding domain that binds CD33 comprises the VH of SEQ ID NO: 262 and the VL of SEQ ID NO: 271.

In other embodiments, the antigen binding domain that binds CD33 comprises the VH of SEQ ID NO: 263 and the VL of SEQ ID NO: 272.

In other embodiments, the antigen binding domain that binds CD33 comprises the VH of SEQ ID NO: 264 and the VL of SEQ ID NO: 273.

In some embodiments, the antigen binding domain that binds CD33 is an scFv. In some embodiments, the antigen binding domain that binds CD33 is an $(scFv)_2$. In some embodiments, the antigen binding domain that binds CD33 is an Fv. In some embodiments, the antigen binding domain that binds CD33 is an Fab. In some embodiments, the antigen binding domain that binds CD33 is an $F(ab')_2$. In some embodiments, the antigen binding domain that binds CD33 is an Fd. In some embodiments, the CD33 antigen binding domain is a dAb. In some embodiments, the CD33 antigen binding domain is a VHH.

The disclosure also provides an isolated protein comprising an antigen binding domain that binds myeloid cell surface antigen CD33, where the antigen binding domain that binds CD33 comprises

a) the VH of SEQ ID NO: 52 and the VL of SEQ ID NO: 81;
b) the VH of SEQ ID NO: 53 and the VL of SEQ ID NO: 82;
c) the VH of SEQ ID NO: 54 and the VL of SEQ ID NO: 83;
d) the VH of SEQ ID NO: 55 and the VL of SEQ ID NO: 84;
e) the VH of SEQ ID NO: 56 and the VL of SEQ ID NO: 85;
f) the VH of SEQ ID NO: 57 and the VL of SEQ ID NO: 86;
g) the VH of SEQ ID NO: 58 and the VL of SEQ ID NO: 87;
h) the VH of SEQ ID NO: 59 and the VL of SEQ ID NO: 87;
i) the VH of SEQ ID NO: 60 and the VL of SEQ ID NO: 87;
j) the VH of SEQ ID NO: 61 and the VL of SEQ ID NO: 88;
k) the VH of SEQ ID NO: 262 and the VL of SEQ ID NO: 271;
l) the VH of SEQ ID NO: 263 and the VL of SEQ ID NO: 272; or
m) the VH of SEQ ID NO: 264 and the VL of SEQ ID NO: 273.

In certain embodiments, the isolated protein comprises the VH of SEQ ID NO: 53 and the VL of SEQ ID NO: 82.

In other embodiments, the isolated protein comprises the VH of SEQ ID NO: 56 and the VL of SEQ ID NO: 85.

In other embodiments, the isolated protein comprises the VH of SEQ ID NO: 59 and the VL of SEQ ID NO: 87.

In other embodiments, the isolated protein comprises the VH of SEQ ID NO: 262 and the VL of SEQ ID NO: 271.

In other embodiments, the isolated protein comprises the VH of SEQ ID NO: 263 and the VL of SEQ ID NO: 272.

In other embodiments, the isolated protein comprises the VH of SEQ ID NO: 264 and the VL of SEQ ID NO: 273.

In some embodiments, the antigen binding domain that binds CD33 is an scFv. In some embodiments, the antigen binding domain that binds CD33 is an $(scFv)_2$. In some embodiments, the antigen binding domain that binds CD33 is an Fv. In some embodiments, the antigen binding domain that binds CD33 is an Fab. In some embodiments, the antigen binding domain that binds CD33 is an $F(ab')_2$. In some embodiments, the antigen binding domain that binds CD33 is an Fd. In some embodiments, the CD33 antigen binding domain is a dAb. In some embodiments, the CD33 antigen binding domain is a VHH.

The disclosure also provides an isolated protein comprising an antigen binding domain that binds myeloid cell surface antigen CD33, where the antigen binding domain that binds CD33 comprises an HCDR1, an HCDR2 and an HCDR3 of a heavy chain variable region (VH) of SEQ ID NO: 95; an HCDR1, an HCDR2 and an HCDR3 of a VH of SEQ ID NO: 96; an HCDR1, an HCDR2 and an HCDR3 of a VH of SEQ ID NO: 97; an HCDR1, an HCDR2 and an HCDR3 of a VH of SEQ ID NO: 170; an HCDR1, an HCDR2 and an HCDR3 of a VH of SEQ ID NO: 171; an HCDR1, an HCDR2 and an HCDR3 of a VH of SEQ ID NO: 175; an HCDR1, an HCDR2 and an HCDR3 of a VH of SEQ ID NO: 179; an HCDR1, an HCDR2 and an HCDR3 of a VH of SEQ ID NO: 181; or an HCDR1, an HCDR2 and an HCDR3 of a VH of SEQ ID NO: 96. In some embodiments, the antigen binding domain that binds CD33 is an scFv. In some embodiments, the antigen binding domain that binds CD33 is an $(scFv)_2$. In some embodiments, the antigen binding domain that binds CD33 is an Fv. In some embodiments, the antigen binding domain that binds CD33 is an Fab. In some embodiments, the antigen binding domain that binds CD33 is an $F(ab')_2$. In some embodiments, the antigen binding domain that binds CD33 is an Fd. In some embodiments, the CD33 antigen binding domain is a dAb. In some embodiments, the CD33 antigen binding domain is a VHH.

The disclosure also provides an isolated protein comprising an antigen binding domain that binds myeloid cell surface antigen CD33, where the antigen binding domain that binds CD33 comprises an HCDR1, an HCDR2, and an HCDR3 of SEQ ID NOs: 89, 90, and 92, respectively; an HCDR1, an HCDR2, and the HCDR3 of SEQ ID NOs: 89, 90, and 93, respectively; an HCDR1, an HCDR2, and an HCDR3 of SEQ ID NOs: 33, 91, and 94, respectively; an HCDR1, an HCDR2, and an HCDR3 of SEQ ID NOs: 167, 168, and 169, respectively; an HCDR1, an HCDR2, and an HCDR3 of SEQ ID NOs: 172, 173, and 174, respectively; an HCDR1, an HCDR2, and an HCDR3 of SEQ ID NOs: 176, 177, and 178, respectively; an HCDR1, an HCDR2, and an HCDR3 of SEQ ID NOs: 89, 90, and 180, respectively; or an HCDR1, an HCDR2, and an HCDR3 of SEQ ID NOs: 89, 90, and 93, respectively. In some embodiments, the antigen binding domain that binds CD33 is an scFv. In some embodiments, the antigen binding domain that binds CD33 is an $(scFv)_2$. In some embodiments, the antigen binding domain that binds CD33 is an Fv. In some embodiments, the antigen binding domain that binds CD33 is an Fab. In some embodiments, the antigen binding domain that binds CD33 is an $F(ab')_2$. In some embodiments, the antigen binding domain that binds CD33 is an Fd. In some embodiments, the CD33 antigen binding domain is a dAb. In some embodiments, the CD33 antigen binding domain is a VHH.

The disclosure also provides an isolated protein comprising an antigen binding domain that binds myeloid cell surface antigen CD33, where the antigen binding domain that binds CD33 comprises a) an HCDR1, an HCDR2 and an HCDR3 of a heavy chain variable region (VH) of SEQ ID NO: 95 and a light chain complementarity determining region (LCDR) 1, an LCDR2 and an LCDR3 of a light chain variable region (VL) of SEQ ID NO: 105; b) an HCDR1, an HCDR2 and an HCDR3 of a VH of SEQ ID NO: 96 and an LCDR1, an LCDR2 and an LCDR3 of a VL of SEQ ID NO: 106; c) an HCDR1, an HCDR2 and an HCDR3 of a VH of SEQ ID NO: 97 and an LCDR1, an LCDR2 and an LCDR3 of a VL of SEQ ID NO: 107; d) an HCDR1, an HCDR2 and an HCDR3 of a VH of SEQ ID NO: 170 and an LCDR1, an LCDR2 and an LCDR3 of a VL of SEQ ID NO: 185; e) an HCDR1, an HCDR2 and an HCDR3 of a VH of SEQ ID NO: 171 and an LCDR1, an LCDR2 and an LCDR3 of a VL of SEQ ID NO: 188; f) an HCDR1, an HCDR2 and an HCDR3 of a VH of SEQ ID NO: 175 and an LCDR1, an LCDR2 and an LCDR3 of a VL of SEQ ID NO: 192; g) an HCDR1, an HCDR2 and an HCDR3 of a VH of SEQ ID NO: 179 and an LCDR1, an LCDR2 and an LCDR3 of a VL of SEQ ID NO: 196; h) an HCDR1, an HCDR2 and an HCDR3 of a VH of SEQ ID NO: 181 and an LCDR1, an LCDR2 and an LCDR3 of a VL of SEQ ID NO: 200; or i) an HCDR1, an HCDR2 and an HCDR3 of a VH of SEQ ID NO: 96 and an LCDR1, an LCDR2 and an LCDR3 of a VL of SEQ ID NO: 202. In some embodiments, the antigen binding domain that binds CD33 is an scFv. In some embodiments, the antigen binding domain that binds CD33 is an $(scFv)_2$. In some embodiments, the antigen binding domain that binds CD33 is an Fv. In some embodiments, the antigen binding domain that binds CD33 is an Fab. In some embodiments, the antigen binding domain that binds CD33 is an $F(ab')_2$. In some embodiments, the antigen binding domain that binds CD33 is an Fd. In some embodiments, the CD33 antigen binding domain is a dAb. In some embodiments, the CD33 antigen binding domain is a VHH.

The disclosure also provides an isolated protein comprising an antigen binding domain that binds myeloid cell surface antigen CD33, where the antigen binding domain that binds CD33 comprises an HCDR1, an HCDR2, an HCDR3, an LCDR1, an LCDR2 and an LCDR3 of

a) SEQ ID NOs: 89, 90, 92, 98, 101, and 104, respectively;
b) SEQ ID NOs: 89, 90, 93, 99, 102, and 104, respectively;
c) SEQ ID NOs: 33, 91, 94, 100, 103, and 104, respectively;
d) SEQ ID NOs: 167, 168, 169, 182, 183, and 184, respectively;
e) SEQ ID NOs: 33, 91, 94, 186, 187, and 104, respectively;
f) SEQ ID NOs: 172, 173, 174, 189, 190, and 191, respectively;
g) SEQ ID NOs: 176, 177, 178, 193, 194, and 195, respectively;
h) SEQ ID NOs: 89, 90, 180, 197, 198, and 199, respectively;
i) SEQ ID NOs: 89, 90, 93, 201, 102, and 104, respectively.

In some embodiments, the antigen binding domain that binds CD33 is an scFv. In some embodiments, the antigen binding domain that binds CD33 is an $(scFv)_2$. In some embodiments, the antigen binding domain that binds CD33 is an Fv. In some embodiments, the antigen binding domain that binds CD33 is an Fab. In some embodiments, the antigen binding domain that binds CD33 is an $F(ab')_2$. In some embodiments, the antigen binding domain that binds CD33 is an Fd. In some embodiments, the CD33 antigen binding domain is a dAb. In some embodiments, the CD33 antigen binding domain is a VHH.

The disclosure also provides an isolated protein comprising an antigen binding domain that binds myeloid cell surface antigen CD33, where the antigen binding domain that binds CD33 comprises a VH of SEQ ID NOs: 95, 96, 97, 170, 171, 175, 179,181, or 96, and a VL of SEQ ID NOs: 105, 106, 107, 185, 188, 192, 196, 200, or 202. In some embodiments, the antigen binding domain that binds CD33 comprises the VH of SEQ ID NO: 95 and the VL of SEQ ID NO: 105; the VH of SEQ ID NO: 96 and the VL of SEQ ID NO: 106; the VH of SEQ ID NO: 97 and the VL of SEQ ID NO: 107; the VH of SEQ ID NO: 170 and the VL of SEQ ID NO: 185; the VH of SEQ ID NO: 171 and the VL of SEQ ID NO: 188; the VH of SEQ ID NO: 175 and the VL of SEQ ID NO: 192; the VH of SEQ ID NO: 179 and the VL of SEQ ID NO: 196; the VH of SEQ ID NO: 181 and the VL of SEQ ID NO: 200; or the VH of SEQ ID NO: 96 and the VL of SEQ ID NO: 202. In some embodiments, the antigen binding domain that binds CD33 is an scFv. In some embodiments, the antigen binding domain that binds CD33 is an $(scFv)_2$. In some embodiments, the antigen binding domain that binds CD33 is an Fv. In some embodiments, the antigen binding domain that binds CD33 is an Fab. In some embodiments, the antigen binding domain that binds CD33 is an $F(ab')_2$. In some embodiments, the antigen binding domain that binds CD33 is an Fd. In some embodiments, the CD33 antigen binding domain is a dAb. In some embodiments, the CD33 antigen binding domain is a VHH.

The disclosure also provides an isolated protein comprising an antigen binding domain that binds myeloid cell surface antigen CD33, where the antigen binding domain that binds CD33 comprises the VH of SEQ ID NO: 95 and the VL of SEQ ID NO: 105; the VH of SEQ ID NO: 96 and the VL of SEQ ID NO: 106; the VH of SEQ ID NO: 97 and the VL of SEQ ID NO: 107; the VH of SEQ ID NO: 170 and the VL of SEQ ID NO: 185; the VH of SEQ ID NO: 171 and the VL of SEQ ID NO: 188; the VH of SEQ ID NO: 175 and the VL of SEQ ID NO: 192; the VH of SEQ ID NO: 179 and the VL of SEQ ID NO: 196; the VH of SEQ ID NO: 181 and the VL of SEQ ID NO: 200; or the VH of SEQ ID NO: 96 and the VL of SEQ ID NO: 202. In some embodiments, the antigen binding domain that binds CD33 is an scFv. In some embodiments, the antigen binding domain that binds CD33 is an $(scFv)_2$. In some embodiments, the antigen binding domain that binds CD33 is an Fv. In some embodiments, the antigen binding domain that binds CD33 is an Fab. In some embodiments, the antigen binding domain that binds CD33 is an $F(ab')_2$. In some embodiments, the antigen binding domain that binds CD33 is an Fd. In some embodiments, the CD33 antigen binding domain is a dAb. In some embodiments, the CD33 antigen binding domain is a VHH.

CD33 binding scFvs

Any of the VH and the VL domains identified herein that bind CD33 may be engineered into scFv format in either VH-linker-VL or VL-linker-VH orientation. Any of the VH and the VL domains identified herein may also be used to generate $sc(Fv)_2$ structures, such as VH-linker-VL-linker-VL-linker-VH, VH-linker-VL-linker-VH-linker-VL. VH-linker-VH-linker-VL-linker-VL. VL-linker-VH-linker-VH-linker-VL. VL-linker-VH-linker-VL-linker-VH or VL-linker-VL-linker-VH-linker-VH.

The VH and the VL domains identified herein may be incorporated into a scFv format and the binding and thermostability of the resulting scFv to CD33 may be assessed using known methods. Binding may be assessed using ProteOn XPR36, Biacore 3000 or KinExA instrumentation, ELISA or competitive binding assays known to those skilled in the art. Binding may be evaluated using purified scFvs or E. coli supernatants or lysed cells containing the expressed scFv. The measured affinity of a test scFv to CD33 may vary if measured under different conditions (e.g., osmolarity, pH). Thus, measurements of affinity and other binding parameters (e.g., $K_D$, $K_{on}$, $K_{off}$) are typically made with standardized conditions and standardized buffers. Thermostability may be evaluated by heating the test scFv at elevated temperatures, such as at 50°C, 55°C or 60°C for a period of time, such as 5 minutes (min), 10 min, 15 min, 20 min, 25 min or 30 min

and measuring binding of the test scFv to CD33. The scFvs retaining comparable binding to CD33 when compared to a non-heated scFv sample are referred to as being thermostable.

In recombinant expression systems, the linker is a peptide linker and may include any naturally occurring amino acid. Exemplary amino acids that may be included into the linker are Gly, Ser Pro, Thr, Glu, Lys, Arg, Ile, Leu, His and The. The linker should have a length that is adequate to link the VH and the VL in such a way that they form the correct conformation relative to one another so that they retain the desired activity, such as binding to CD33.

The linker may be about 5-50 amino acids long. In some embodiments, the linker is about 10-40 amino acids long. In some embodiments, the linker is about 10-35 amino acids long. In some embodiments, the linker is about 10-30 amino acids long. In some embodiments, the linker is about 10-25 amino acids long. In some embodiments, the linker is about 10-20 amino acids long. In some embodiments, the linker is about 15-20 amino acids long. In some embodiments, the linker is 6 amino acids long. In some embodiments, the linker is 7 amino acids long. In some embodiments, the linker is 8 amino acids long. In some embodiments, the linker is 9 amino acids long. In some embodiments, the linker is 10 amino acids long. In some embodiments, the linker is 11 amino acids long. In some embodiments, the linker is 12 amino acids long. In some embodiments, the linker is 13 amino acids long. In some embodiments, the linker is 14 amino acids long. In some embodiments, the linker is 15 amino acids long. In some embodiments, the linker is 16 amino acids long. In some embodiments, the linker is 17 amino acids long. In some embodiments, the linker is 18 amino acids long. In some embodiments, the linker is 19 amino acids long. In some embodiments, the linker is 20 amino acids long. In some embodiments, the linker is 21 amino acids long. In some embodiments, the linker is 22 amino acids long. In some embodiments, the linker is 23 amino acids long. In some embodiments, the linker is 24 amino acids long. In some embodiments, the linker is 25 amino acids long. In some embodiments, the linker is 26 amino acids long. In some embodiments, the linker is 27 amino acids long. In some embodiments, the linker is 28 amino acids long. In some embodiments, the linker is 29 amino acids long. In some embodiments, the linker is 30 amino acids long. In some embodiments, the linker is 31 amino acids long. In some embodiments, the linker is 32 amino acids long. In some embodiments, the linker is 33 amino acids long. In some embodiments, the linker is 34 amino acids long. In some embodiments, the linker is 35 amino acids long. In some embodiments, the linker is 36 amino acids long. In some embodiments, the linker is 37 amino acids long. In some embodiments, the linker is 38 amino acids long. In some embodiments, the linker is 39 amino acids long. In some embodiments, the linker is 40 amino acids long. Exemplary linkers that may be used are Gly rich linkers, Gly and Ser containing linkers, Gly and Ala containing linkers, Ala and Ser containing linkers, and other flexible linkers.

Other linker sequences may include portions of immunoglobulin hinge area, CL or CH1 derived from any immunoglobulin heavy or light chain isotype. Alternatively, a variety of non-proteinaceous polymers, including polyethylene glycol (PEG), polypropylene glycol, polyoxyalkylenes, or copolymers of polyethylene glycol and polypropylene glycol, may find use as linkers. Exemplary linkers that may be used are shown in Table 1. Additional linkers are described for example in International Patent Publication No. WO2019/060695, which is incorporated by reference herein in its entirety.

In some embodiments, the scFv comprises, from the N- to C-terminus, a VH, a first linker (L1) and a VL (VH-L1-VL).

In some embodiments, the scFv comprises, from the N-to C-terminus, the VL, the L1 and the VH (VL-L1-VH).

In some embodiments, the L1 comprises the amino acid sequence of SEQ ID NO: 108.

In some embodiments, the L1 comprises the amino acid sequence of SEQ ID NO: 109.

In some embodiments, the L1 comprises the amino acid sequence of SEQ ID NO: 110.

In some embodiments, the L1 comprises the amino acid sequence of SEQ ID NO: 111.

In some embodiments, the L1 comprises the amino acid sequence of SEQ ID NO: 112.

In some embodiments, the L1 comprises the amino acid sequence of SEQ ID NO: 113.

In some embodiments, the L1 comprises the amino acid sequence of SEQ ID NO: 114.

In some embodiments, the L1 comprises the amino acid sequence of SEQ ID NO: 115.

In some embodiments, the L1 comprises the amino acid sequence of SEQ ID NO: 116.

In some embodiments, the L1 comprises the amino acid sequence of SEQ ID NO: 117.

In some embodiments, the L1 comprises the amino acid sequence of SEQ ID NO: 118.

In some embodiments, the L1 comprises the amino acid sequence of SEQ ID NO: 119.

In some embodiments, the L1 comprises the amino acid sequence of SEQ ID NO: 120.

In some embodiments, the L1 comprises the amino acid sequence of SEQ ID NO: 121.

In some embodiments, the L1 comprises the amino acid sequence of SEQ ID NO: 122.

In some embodiments, the L1 comprises the amino acid sequence of SEQ ID NO: 123.

In some embodiments, the L1 comprises the amino acid sequence of SEQ ID NO: 124.

In some embodiments, the L1 comprises the amino acid sequence of SEQ ID NO: 125.

In some embodiments, the L1 comprises the amino acid sequence of SEQ ID NO: 126.

In some embodiments, the L1 comprises the amino acid sequence of SEQ ID NO: 127.

In some embodiments, the L1 comprises the amino acid sequence of SEQ ID NO: 128.
In some embodiments, the L1 comprises the amino acid sequence of SEQ ID NO: 129.
In some embodiments, the L1 comprises the amino acid sequence of SEQ ID NO: 130.
In some embodiments, the L1 comprises the amino acid sequence of SEQ ID NO: 131.
In some embodiments, the L1 comprises the amino acid sequence of SEQ ID NO: 132.
In some embodiments, the L1 comprises the amino acid sequence of SEQ ID NO: 133.
In some embodiments, the L1 comprises the amino acid sequence of SEQ ID NO: 134.
In some embodiments, the L1 comprises the amino acid sequence of SEQ ID NO: 135.
In some embodiments, the L1 comprises the amino acid sequence of SEQ ID NO: 136.
In some embodiments, the L1 comprises the amino acid sequence of SEQ ID NO: 137.
In some embodiments, the L1 comprises the amino acid sequence of SEQ ID NO: 138.
In some embodiments, the L1 comprises the amino acid sequence of SEQ ID NO: 139.
In some embodiments, the L1 comprises the amino acid sequence of SEQ ID NO: 140.

**Table 1.**

| Linker name | Amino acid sequence | SEQ ID NO: |
|---|---|---|
| Linker 1 | GGSEGKSSGSGSESKSTGGS | 108 |
| Linker 2 | GGGSGGGS | 109 |
| Linker 3 | GGGSGGGSGGGS | 110 |
| Linker 4 | GGGSGGGSGGGSGGGS | 111 |
| Linker 5 | GGGSGGGSGGGSGGGSGGGS | 112 |
| Linker 6 | GGGGSGGGGSGGGGS | 113 |
| Linker 7 | GGGGSGGGGSGGGGSGGGGS | 114 |
| Linker 8 | GGGGSGGGGSGGGGSGGGGSGGGGS | 115 |
| Linker 9 | GSTSGSGKPGSGEGSTKG | 116 |
| Linker 10 | IRPRAIGGSKPRVA | 117 |
| Linker 11 | GKGGSGKGGSGKGGS | 118 |
| Linker 12 | GGKGSGGKGSGGKGS | 119 |
| Linker 13 | GGGKSGGGKSGGGKS | 120 |
| Linker 14 | GKGKSGKGKSGKGKS | 121 |
| Linker 15 | GGGKSGGKGSGKGGS | 122 |
| Linker 16 | GKPGSGKPGSGKPGS | 123 |
| Linker 17 | GKPGSGKPGSGKPGSGKPGS | 124 |
| Linker 18 | GKGKSGKGKSGKGKSGKGKS | 125 |
| Linker 19 | STAGDTHLGGEDFD | 126 |
| Linker 20 | GEGGSGEGGSGEGGS | 127 |
| Linker 21 | GGEGSGGEGSGGEGS | 128 |
| Linker 22 | GEGESGEGESGEGES | 129 |
| Linker 23 | GGGESGGEGSGEGGS | 130 |
| Linker 24 | GEGESGEGESGEGESGEGES | 131 |
| Linker 25 | GSTSGSGKPGSGEGSTKG | 132 |
| Linker 26 | PRGASKSGSASQTGSAPGS | 133 |
| Linker 27 | GTAAAGAGAAGGAAAGAAG | 134 |
| Linker 28 | GTSGSSGSGSGGSGSGGGG | 135 |

(continued)

| Linker name | Amino acid sequence | SEQ ID NO: |
|---|---|---|
| Linker 29 | GKPGSGKPGSGKPGSGKPGS | 136 |
| Linker 30 | GSGS | 137 |
| Linker 31 | APAPAPAPAP | 138 |
| Linker 32 | APAPAPAPAPAPAPAPAPAP | 139 |
| Linker 33 | AEAAAKEAAAKEAAAAKEAAAAKEAAAAKAAA | 140 |

In certain embodiments, the L1 comprises the amino acid sequence of SEQ ID NO: 108 (e.g. consists of the amino acid sequence of SEQ ID NO: 108).

In some embodiments, the scFv comprises a heavy chain complementarity determining region (HCDR) 1, a HCDR2 and a HCDR3 of a heavy chain variable region (VH) of SEQ ID NO: 18; an HCDR1, an HCDR2 and an HCDR3 of a VH of SEQ ID NO: 19; an HCDR1, an HCDR2 and an HCDR3 of a VH of SEQ ID NO: 20; an HCDR1, an HCDR2 and an HCDR3 of a VH of SEQ ID NO: 21; an HCDR1, an HCDR2 and an HCDR3 of a VH of SEQ ID NO: 22; an HCDR1, an HCDR2 and an HCDR3 of a VH of SEQ ID NO: 23; an HCDR1, an HCDR2 and an HCDR3 of a VH of SEQ ID NO: 24; an HCDR1, an HCDR2 and an HCDR3 of a VH of SEQ ID NO: 25; an HCDR1, an HCDR2 and an HCDR3 of a VH of SEQ ID NO: 26; or an HCDR1, an HCDR2 and an HCDR3 of a VH of SEQ ID NO: 27. In some embodiments, the antigen binding domain that binds CD33 comprises the VH of any one of SEQ ID NOs: 18, 19, 20, 21, 22, 23, 24, 25, 26, or 27.

In some embodiments, the scFv comprises an HCDR1, an HCDR2, and an HCDR3 of SEQ ID NOs: 1, 6, and 12, respectively; SEQ ID NOs: 2, 7, and 13, respectively; SEQ ID NOs: 1, 8, and 14, respectively; SEQ ID NOs: 3, 9, and 13, respectively; SEQ ID NOs: 4, 10, and 15, respectively; SEQ ID NOs: 5, 11, and 16, respectively; or SEQ ID NOs: 5, 11, and 17, respectively.

In some embodiments, the scFv comprises

a) an HCDR1, an HCDR2 and an HCDR3 of a heavy chain variable region (VH) of SEQ ID NO: 52;
b) an HCDR1, an HCDR2 and an HCDR3 of a VH of SEQ ID NO: 53;
c) an HCDR1, an HCDR2 and an HCDR3 of a VH of SEQ ID NO: 54;
d) an HCDR1, an HCDR2 and an HCDR3 of a VH of SEQ ID NO: 55;
e) an HCDR1, an HCDR2 and an HCDR3 of a VH of SEQ ID NO: 56;
f) an HCDR1, an HCDR2 and an HCDR3 of a VH of SEQ ID NO: 57;
g) an HCDR1, an HCDR2 and an HCDR3 of a VH of SEQ ID NO: 58;
h) an HCDR1, an HCDR2 and an HCDR3 of a VH of SEQ ID NO: 59;
i) an HCDR1, an HCDR2 and an HCDR3 of a VH of SEQ ID NO: 60;
j) an HCDR1, an HCDR2 and an HCDR3 of a VH of SEQ ID NO: 61;
k) an HCDR1, an HCDR2 and an HCDR3 of a VH of SEQ ID NO: 262;
l) an HCDR1, an HCDR2 and an HCDR3 of a VH of SEQ ID NO: 263; or
m) an HCDR1, an HCDR2 and an HCDR3 of a VH of SEQ ID NO: 264.

In certain embodiments, the scFv comprises an HCDR1, an HCDR2 and an HCDR3 of a VH of SEQ ID NO: 53.
In other embodiments, the scFv comprises an HCDR1, an HCDR2 and an HCDR3 of a VH of SEQ ID NO: 56.
In other embodiments, the scFv comprises an HCDR1, an HCDR2 and an HCDR3 of a VH of SEQ ID NO: 59.
In other embodiments, the scFv comprises an HCDR1, an HCDR2 and an HCDR3 of a VH of SEQ ID NO: 262.
In other embodiments, the scFv comprises an HCDR1, an HCDR2 and an HCDR3 of a VH of SEQ ID NO: 263.
In other embodiments, the scFv comprises an HCDR1, an HCDR2 and an HCDR3 of a VH of SEQ ID NO: 264.
Exemplary HCDR sequences are described in Tables 4a, 4b, 4c, 4d, 4e and 4f.
In some embodiments, the scFv comprises the VH of SEQ ID NOs: 52, 53, 54, 55, 56, 57, 58, 59, 60, 61, 262, 263 or 264.
In certain embodiments, the scFv comprises the VH of SEQ ID NO: 53.
In other embodiments, the scFv comprises the VH of SEQ ID NO: 56.
In other embodiments, the scFv comprises the VH of SEQ ID NO: 59.
In other embodiments, the scFv comprises the VH of SEQ ID NO: 262.
In other embodiments, the scFv comprises the VH of SEQ ID NO: 263.
In other embodiments, the scFv comprises the VH of SEQ ID NO: 264.

In some embodiments, the scFv comprises the VL of SEQ ID NOs: 81, 82, 83, 84, 85, 86, 87, 88, 271, 272 or 273.
In certain embodiments, the scFv comprises the VL of SEQ ID NO: 82.
In other embodiments, the scFv comprises the VL of SEQ ID NO: 85.
In other embodiments, the scFv comprises the VL of SEQ ID NO: 87.
In other embodiments, the scFv comprises the VL of SEQ ID NO: 271.
In other embodiments, the scFv comprises the VL of SEQ ID NO: 272.
In other embodiments, the scFv comprises the VL of SEQ ID NO: 273.
In some embodiments, the scFv comprises the VH of SEQ ID NOs: 52, 53, 54, 55, 56, 57, 58, 59, 60, 61, 262, 263 or 264, and the VL of SEQ ID NOs: 81, 82, 83, 84, 85, 86, 87, 88, 271, 272 or 273. In some embodiments, the scFv comprises an HCDR1, an HCDR2, and an HCDR3 of

a) SEQ ID NOs: 28, 36, and 45, respectively;
b) SEQ ID NOs: 29, 37, and 46, respectively;
c) SEQ ID NOs: 30, 38, and 47, respectively;
d) SEQ ID NOs: 31, 39, and 48, respectively;
e) SEQ ID NOs: 32, 40, and 49, respectively;
f) SEQ ID NOs: 33, 41, and 50, respectively;
g) SEQ ID NOs: 34, 42, and 51, respectively;
h) SEQ ID NOs: 34, 43, and 51, respectively;
i) SEQ ID NOs: 34, 44, and 51, respectively;
j) SEQ ID NOs: 35, 42, and 51, respectively;
k) SEQ ID NOs: 253, 256 and 259, respectively;
l) SEQ ID NOs: 254, 257 and 260, respectively; or
m) SEQ ID NOs: 255, 258 and 261, respectively.

In certain embodiments, the scFv comprises an HCDR1, an HCDR2, and an HCDR3 of SEQ ID NOs: 29, 37, and 46, respectively.
In other embodiments, the scFv comprises an HCDR1, an HCDR2, and an HCDR3 of SEQ ID NOs: 32, 40, and 49, respectively.
In other embodiments, the scFv comprises an HCDR1, an HCDR2, and an HCDR3 of SEQ ID NOs: 34, 43, and 51, respectively.
In other embodiments, the scFv comprises an HCDR1, an HCDR2, and an HCDR3 of SEQ ID NOs: 253, 256, and 259, respectively.
In other embodiments, the scFv comprises an HCDR1, an HCDR2, and an HCDR3 of SEQ ID NOs: 254, 257, and 260, respectively.
In other embodiments, the scFv comprises an HCDR1, an HCDR2, and an HCDR3 of SEQ ID NOs: 255, 258, and 261, respectively.In some embodiments, the scFv comprises an HCDR1, an HCDR2 and an HCDR3 of a heavy chain variable region (VH) of SEQ ID NO: 52 and a light chain complementarity determining region (LCDR) 1, an LCDR2 and an LCDR3 of a light chain variable region (VL) of SEQ ID NO: 81; an HCDR1, an HCDR2 and an HCDR3 of a VH of SEQ ID NO: 53 and an LCDR1, an LCDR2 and an LCDR3 of a VL of SEQ ID NO: 82; an HCDR1, an HCDR2 and an HCDR3 of a VH of SEQ ID NO: 54 and an LCDR1, an LCDR2 and an LCDR3 of a VL of SEQ ID NO: 83; an HCDR1, an HCDR2 and an HCDR3 of a VH of SEQ ID NO: 55 and an LCDR1, an LCDR2 and an LCDR3 of a VL of SEQ ID NO: 84; an HCDR1, an HCDR2 and an HCDR3 of a VH of SEQ ID NO: 56 and an LCDR1, an LCDR2 and an LCDR3 of a VL of SEQ ID NO: 85; an HCDR1, an HCDR2 and an HCDR3 of a VH of SEQ ID NO: 57 and an LCDR1, an LCDR2 and an LCDR3 of a VL of SEQ ID NO: 86; an HCDR1, an HCDR2 and an HCDR3 of a VH of SEQ ID NO: 58 and an LCDR1, an LCDR2 and an LCDR3 of a VL of SEQ ID NO: 87; an HCDR1, an HCDR2 and an HCDR3 of a VH of SEQ ID NO: 59 and an LCDR1, an LCDR2 and an LCDR3 of a VL of SEQ ID NO: 87; an HCDR1, an HCDR2 and an HCDR3 of a VH of SEQ ID NO: 60 and an LCDR1, an LCDR2 and an LCDR3 of a VL of SEQ ID NO: 87; an HCDR1, an HCDR2 and an HCDR3 of a VH of SEQ ID NO: 61 and an LCDR1, an LCDR2 and an LCDR3 of a VL of SEQ ID NO: 88; an HCDR1, an HCDR2 and an HCDR3 of a VH of SEQ ID NO: 262 and an LCDR1, an LCDR2 and an LCDR3 of a VL of SEQ ID NO: 271; an HCDR1, an HCDR2 and an HCDR3 of a VH of SEQ ID NO: 263 and an LCDR1, an LCDR2 and an LCDR3 of a VL of SEQ ID NO: 272; or an HCDR1, an HCDR2 and an HCDR3 of a VH of SEQ ID NO: 264 and an LCDR1, an LCDR2 and an LCDR3 of a VL of SEQ ID NO: 273.
In certain embodiments, the scFv comprises an HCDR1, an HCDR2 and an HCDR3 of a VH of SEQ ID NO: 53 and an LCDR1, an LCDR2 and an LCDR3 of a VL of SEQ ID NO: 82.
In other embodiments, the scFv comprises an HCDR1, an HCDR2 and an HCDR3 of a VH of SEQ ID NO: 56 and an LCDR1, an LCDR2 and an LCDR3 of a VL of SEQ ID NO: 85.
In other embodiments, the scFv comprises an HCDR1, an HCDR2 and an HCDR3 of a VH of SEQ ID NO: 59

and an LCDR1, an LCDR2 and an LCDR3 of a VL of SEQ ID NO: 87.

In other embodiments, the scFv comprises an HCDR1, an HCDR2 and an HCDR3 of a VH of SEQ ID NO: 262 and an LCDR1, an LCDR2 and an LCDR3 of a VL of SEQ ID NO: 271.

In other embodiments, the scFv comprises an HCDR1, an HCDR2 and an HCDR3 of a VH of SEQ ID NO: 263 and an LCDR1, an LCDR2 and an LCDR3 of a VL of SEQ ID NO: 272.

In other embodiments, the scFv comprises an HCDR1, an HCDR2 and an HCDR3 of a VH of SEQ ID NO: 264 and an LCDR1, an LCDR2 and an LCDR3 of a VL of SEQ ID NO: 273.

Exemplary HCDR and LCDR sequences are described in Tables 4a, 4b, 4c, 4d, 4e and 4f.

In some embodiments, the scFv comprises an HCDR1, an HCDR2, an HCDR3, an LCDR1, an LCDR2 and an LCDR3 of

a) SEQ ID NOs: 28, 36, 45, 62, 69, and 75, respectively;
b) SEQ ID NOs: 29, 37, 46, 63, 70, and 76, respectively;
c) SEQ ID NOs: 30, 38, 47, 64, 71, and 77, respectively;
d) SEQ ID NOs: 31, 39, 48, 65, 72, and 78, respectively;
e) SEQ ID NOs: 32, 40, 49, 66, 70, and 76, respectively;
f) SEQ ID NOs: 33, 41, 50, 67, 73, and 79, respectively;
g) SEQ ID NOs: 34, 42, 51, 68, 74, and 80, respectively;
h) SEQ ID NOs: 34, 43, 51, 68, 74, and 80, respectively;
i) SEQ ID NOs: 34, 44, 51, 68, 74, and 80, respectively; or
j) SEQ ID NOs: 35, 42, 51, 68, 74, and 80, respectively;
k) SEQ ID NOs: 253, 256, 259, 265, 74 and 269, respectively;
l) SEQ ID NOs: 254, 257, 260, 66, 267 and 76, respectively;
m) SEQ ID NOs: 255, 258, 261, 266, 268 and 270, respectively.

In certain embodiments, the scFv comprises the HCDR1, the HCDR2, the HCDR3, the LCDR1, the LCDR2 and the LCDR3 of SEQ ID NOs: 29, 37, 46, 63, 70, and 76, respectively.

In other embodiments, the scFv comprises the HCDR1, the HCDR2, the HCDR3, the LCDR1, the LCDR2 and the LCDR3 of SEQ ID NOs: 32, 40, 49, 66, 70, and 76, respectively.

In other embodiments, the scFv comprises the HCDR1, the HCDR2, the HCDR3, the LCDR1, the LCDR2 and the LCDR3 of SEQ ID NOs: 34, 43, 51, 68, 74, and 80, respectively.

In other embodiments, the scFv comprises the HCDR1, the HCDR2, the HCDR3, the LCDR1, the LCDR2 and the LCDR3 of SEQ ID NOs: 253, 256, 259, 265, 74 and 269, respectively.

In other embodiments, the scFv comprises the HCDR1, the HCDR2, the HCDR3, the LCDR1, the LCDR2 and the LCDR3 of SEQ ID NOs: 254, 257, 260, 66, 267 and 76, respectively.

In other embodiments, the scFv comprises the HCDR1, the HCDR2, the HCDR3, the LCDR1, the LCDR2 and the LCDR3 of SEQ ID NOs: 255, 258, 261, 266, 268 and 270, respectively.

In some embodiments, the scFv comprises:

a) the VH of SEQ ID NO: 52 and the VL of SEQ ID NO: 81;
b) the VH of SEQ ID NO: 53 and the VL of SEQ ID NO: 82;
c) the VH of SEQ ID NO: 54 and the VL of SEQ ID NO: 83;
d) the VH of SEQ ID NO: 55 and the VL of SEQ ID NO: 84;
e) the VH of SEQ ID NO: 56 and the VL of SEQ ID NO: 85;
f) the VH of SEQ ID NO: 57 and the VL of SEQ ID NO: 86;
g) the VH of SEQ ID NO: 58 and the VL of SEQ ID NO: 87;
h) the VH of SEQ ID NO: 59 and the VL of SEQ ID NO: 87;
i) the VH of SEQ ID NO: 60 and the VL of SEQ ID NO: 87;
j) the VH of SEQ ID NO: 61 and the VL of SEQ ID NO: 88;
k) the VH of SEQ ID NO: 262 and the VL of SEQ ID NO: 271;
l) the VH of SEQ ID NO: 263 and the VL of SEQ ID NO: 272; or
m) the VH of SEQ ID NO: 264 and the VL of SEQ ID NO: 273.

In certain embodiments, the scFv comprises the VH of SEQ ID NO: 53 and the VL of SEQ ID NO: 82. In certain such embodiments, the scFv comprises a linker sequence of the amino acid sequence of SEQ ID NO: 108. Thus, in some embodiments, the scFv comprises, from the N- to C-terminus, a VH of SEQ ID NO: 53, a first linker (L1) of SEQ ID NO: 108 and a VL of SEQ ID: 82 (VH-L1-VL). In other embodiments, the scFv comprises from the N- to C- terminus, a VL of SEQ ID NO: 82, a first linker of SEQ ID NO: 108 and a VL of SEQ ID NO: 53 (VL-L1-VH).

In other embodiments, the scFv comprises the VH of SEQ ID NO: 56 and the VL of SEQ ID NO: 85. In certain such embodiments, the scFv comprises a linker sequence of the amino acid sequence of SEQ ID NO: 108. Thus, in some embodiments, the scFv comprises, from the N- to C-terminus, a VH of SEQ ID NO: 56, a first linker (L1) of SEQ ID NO: 108 and a VL of SEQ ID: 85 (VH-L1-VL). In other embodiments, the scFv comprises from the N- to C- terminus, a VL of SEQ ID NO: 56, a first linker of SEQ ID NO: 108 and a VL of SEQ ID NO: 85 (VL-L1-VH).

In other embodiments, the scFv comprises the VH of SEQ ID NO: 59 and the VL of SEQ ID NO: 87. In certain such embodiments, the scFv comprises a linker sequence of the amino acid sequence of SEQ ID NO: 108. Thus, in some embodiments, the scFv comprises, from the N- to C-terminus, a VH of SEQ ID NO: 59, a first linker (L1) of SEQ ID NO: 108 and a VL of SEQ ID: 87 (VH-L1-VL). In other embodiments, the scFv comprises from the N- to C- terminus, a VL of SEQ ID NO: 59, a first linker of SEQ ID NO: 108 and a VL of SEQ ID NO: 87 (VL-L1-VH).

In other embodiments, the scFv comprises the VH of SEQ ID NO: 262 and the VL of SEQ ID NO: 271. In certain such embodiments, the scFv comprises a linker sequence of the amino acid sequence of SEQ ID NO: 108. Thus, in some embodiments, the scFv comprises, from the N- to C-terminus, a VH of SEQ ID NO: 262, a first linker (L1) of SEQ ID NO: 108 and a VL of SEQ ID: 271 (VH-L1-VL). In other embodiments, the scFv comprises from the N- to C- terminus, a VL of SEQ ID NO: 271, a first linker of SEQ ID NO: 108 and a VL of SEQ ID NO: 262 (VL-L1-VH).

In other embodiments, the scFv comprises the VH of SEQ ID NO: 263 and the VL of SEQ ID NO: 272. In certain such embodiments, the scFv comprises a linker sequence of the amino acid sequence of SEQ ID NO: 108. Thus, in some embodiments, the scFv comprises, from the N- to C-terminus, a VH of SEQ ID NO: 263, a first linker (L1) of SEQ ID NO: 108 and a VL of SEQ ID: 272 (VH-L1-VL). In other embodiments, the scFv comprises from the N- to C- terminus, a VL of SEQ ID NO: 272, a first linker of SEQ ID NO: 108 and a VL of SEQ ID NO: 263 (VL-L1-VH).

In other embodiments, the scFv comprises the VH of SEQ ID NO: 264 and the VL of SEQ ID NO: 273. In certain such embodiments, the scFv comprises a linker sequence of the amino acid sequence of SEQ ID NO: 108. Thus, in some embodiments, the scFv comprises, from the N- to C-terminus, a VH of SEQ ID NO: 264, a first linker (L1) of SEQ ID NO: 108 and a VL of SEQ ID: 273 (VH-L1-VL). In other embodiments, the scFv comprises from the N- to C- terminus, a VL of SEQ ID NO: 273, a first linker of SEQ ID NO: 108 and a VL of SEQ ID NO: 264 (VL-L1-VH).

In some embodiments, the scFv comprises an HCDR1, an HCDR2 and an HCDR3 of a heavy chain variable region (VH) of SEQ ID NO: 95; an HCDR1, an HCDR2 and an HCDR3 of a VH of SEQ ID NO: 96; an HCDR1, an HCDR2 and an HCDR3 of a VH of SEQ ID NO: 97; an HCDR1, an HCDR2 and an HCDR3 of a VH of SEQ ID NO: 170; an HCDR1, an HCDR2 and an HCDR3 of a VH of SEQ ID NO: 171; an HCDR1, an HCDR2 and an HCDR3 of a VH of SEQ ID NO: 175; an HCDR1, an HCDR2 and an HCDR3 of a VH of SEQ ID NO: 179; an HCDR1, an HCDR2 and an HCDR3 of a VH of SEQ ID NO: 181; or an HCDR1, an HCDR2 and an HCDR3 of a VH of SEQ ID NO: 96.

In some embodiments, the scFv comprises an HCDR1, an HCDR2, and an HCDR3 of SEQ ID NOs: 89, 90, and 92, respectively; an HCDR1, an HCDR2, and the HCDR3 of SEQ ID NOs: 89, 90, and 93, respectively; an HCDR1, an HCDR2, and an HCDR3 of SEQ ID NOs: 33, 91, and 94, respectively; an HCDR1, an HCDR2, and an HCDR3 of SEQ ID NOs: 167, 168, and 169, respectively; an HCDR1, an HCDR2, and an HCDR3 of SEQ ID NOs: 172, 173, and 174, respectively; an HCDR1, an HCDR2, and an HCDR3 of SEQ ID NOs: 176, 177, and 178, respectively; an HCDR1, an HCDR2, and an HCDR3 of SEQ ID NOs: 89, 90, and 180, respectively; or an HCDR1, an HCDR2, and an HCDR3 of SEQ ID NOs: 89, 90, and 93, respectively.

In some embodiments, the scFv comprises an HCDR1, an HCDR2 and an HCDR3 of a heavy chain variable region (VH) of SEQ ID NO: 95 and an LCDR1, an LCDR2 and an LCDR3 of a light chain variable region (VL) of SEQ ID NO: 105; an HCDR1, an HCDR2 and an HCDR3 of a VH of SEQ ID NO: 96 and an LCDR1, an LCDR2 and an LCDR3 of a VL of SEQ ID NO: 106; an HCDR1, an HCDR2 and an HCDR3 of a VH of SEQ ID NO: 97 and an LCDR1, an LCDR2 and an LCDR3 of a VL of SEQ ID NO: 107; an HCDR1, an HCDR2 and an HCDR3 of a VH of SEQ ID NO: 170 and an LCDR1, an LCDR2 and an LCDR3 of a VL of SEQ ID NO: 185; an HCDR1, an HCDR2 and an HCDR3 of a VH of SEQ ID NO: 171 and an LCDR1, an LCDR2 and an LCDR3 of a VL of SEQ ID NO: 188; an HCDR1, an HCDR2 and an HCDR3 of a VH of SEQ ID NO: 175 and an LCDR1, an LCDR2 and an LCDR3 of a VL of SEQ ID NO: 192; an HCDR1, an HCDR2 and an HCDR3 of a VH of SEQ ID NO: 179 and an LCDR1, an LCDR2 and an LCDR3 of a VL of SEQ ID NO: 196; an HCDR1, an HCDR2 and an HCDR3 of a VH of SEQ ID NO: 181 and an LCDR1, an LCDR2 and an LCDR3 of a VL of SEQ ID NO: 200; or an HCDR1, an HCDR2 and an HCDR3 of a VH of SEQ ID NO: 96 and an LCDR1, an LCDR2 and an LCDR3 of a VL of SEQ ID NO: 202.

In some embodiments, the scFv comprises an HCDR1, an HCDR2, an HCDR3, an LCDR1, an LCDR2 and an LCDR3 of

a) SEQ ID NOs: 89, 90, 92, 98, 101, and 104, respectively;
b) SEQ ID NOs: 89, 90, 93, 99, 102, and 104, respectively;
c) SEQ ID NOs: 33, 91, 94, 100, 103, and 104, respectively;
d) SEQ ID NOs: 167, 168, 169, 182, 183, and 184, respectively;
e) SEQ ID NOs: 33, 91, 94, 186, 187, and 104, respectively;

f) SEQ ID NOs: 172, 173, 174, 189, 190, and 191, respectively;
g) SEQ ID NOs: 176, 177, 178, 193, 194, and 195, respectively;
h) SEQ ID NOs: 89, 90, 180, 197, 198, and 199, respectively;
i) SEQ ID NOs: 89, 90, 93, 201, 102, and 104, respectively.

In some embodiments, the scFv comprises a VH of SEQ ID NOs: 95, 96, 97, 170, 171, 175, 179, 181, or 96, and a VL of SEQ ID NOs: 105, 106, 107, 185, 188, 192, 196, 200, or 202. In some embodiments, the scFv comprises the VH of SEQ ID NO: 95 and the VL of SEQ ID NO: 105; the VH of SEQ ID NO: 96 and the VL of SEQ ID NO: 106; the VH of SEQ ID NO: 97 and the VL of SEQ ID NO: 107; the VH of SEQ ID NO: 170 and the VL of SEQ ID NO: 185; the VH of SEQ ID NO: 171 and the VL of SEQ ID NO: 188; the VH of SEQ ID NO: 175 and the VL of SEQ ID NO: 192; the VH of SEQ ID NO: 179 and the VL of SEQ ID NO: 196; the VH of SEQ ID NO: 181 and the VL of SEQ ID NO: 200; or the VH of SEQ ID NO: 96 and the VL of SEQ ID NO: 202.

In some embodiments, the scFv comprises the amino acid sequence of SEQ ID NO: 203.

In some embodiments, the scFv comprises the amino acid sequence of SEQ ID NO: 204.

In some embodiments, the scFv comprises the amino acid sequence of SEQ ID NO: 205.

In some embodiments, the scFv comprises the amino acid sequence of SEQ ID NO: 206.

In some embodiments, the scFv comprises the amino acid sequence of SEQ ID NO: 207.

In some embodiments, the scFv comprises the amino acid sequence of SEQ ID NO: 208.

In some embodiments, the scFv comprises the amino acid sequence of SEQ ID NO: 209.

In some embodiments, the scFv comprises the amino acid sequence of SEQ ID NO: 210.

In some embodiments, the scFv comprises the amino acid sequence of SEQ ID NO: 211.

In some embodiments, the scFv comprises the amino acid sequence of SEQ ID NO: 212.

In some embodiments, the scFv comprises the amino acid sequence of SEQ ID NO: 213.

In some embodiments, the scFv comprises the amino acid sequence of SEQ ID NO: 214.

In some embodiments, the scFv comprises the amino acid sequence of SEQ ID NO: 215.

In some embodiments, the scFv comprises the amino acid sequence of SEQ ID NO: 216.

In some embodiments, the scFv comprises the amino acid sequence of SEQ ID NO: 217.

In some embodiments, the scFv comprises the amino acid sequence of SEQ ID NO: 218.

In some embodiments, the scFv comprises the amino acid sequence of SEQ ID NO: 219.

In some embodiments, the scFv comprises the amino acid sequence of SEQ ID NO: 220.

In some embodiments, the scFv comprises the amino acid sequence of SEQ ID NO: 221.

In some embodiments, the scFv comprises the amino acid sequence of SEQ ID NO: 274.

In some embodiments, the scFv comprises the amino acid sequence of SEQ ID NO: 275.

In some embodiments, the scFv comprises the amino acid sequence of SEQ ID NO: 276.

In certain embodiments, the scFv comprises the amino acid sequence of SEQ ID NO: 213.

In other embodiments, the scFv comprises the amino acid sequence of SEQ ID NO: 216.

In other embodiments, the scFv comprises the amino acid sequence of SEQ ID NO: 219.

In other embodiments, the scFv comprises the amino acid sequence of SEQ ID NO: 274.

In other embodiments, the scFv comprises the amino acid sequence of SEQ ID NO: 275.

In other embodiments, the scFv comprises the amino acid sequence of SEQ ID NO: 276.

**Other antigen binding domains that bind CD33**

Any of the VH and the VL domains identified herein that bind CD33 may also be engineered into Fab, $F(ab')_2$, Fd or Fv format and their binding to CD33 and thermostability may be assessed using the assays described herein. In certain embodiments, the VH and VL domains identified herein are engineered into a Fab.

In some embodiments, the Fab comprises an HCDR1, a HCDR2 and a HCDR3 of a heavy chain variable region (VH) of SEQ ID NO: 18; an HCDR1, an HCDR2 and an HCDR3 of a VH of SEQ ID NO: 19; an HCDR1, an HCDR2 and an HCDR3 of a VH of SEQ ID NO: 20; an HCDR1, an HCDR2 and an HCDR3 of a VH of SEQ ID NO: 21; an HCDR1, an HCDR2 and an HCDR3 of a VH of SEQ ID NO: 22; an HCDR1, an HCDR2 and an HCDR3 of a VH of SEQ ID NO: 23; an HCDR1, an HCDR2 and an HCDR3 of a VH of SEQ ID NO: 24; an HCDR1, an HCDR2 and an HCDR3 of a VH of SEQ ID NO: 25; an HCDR1, an HCDR2 and an HCDR3 of a VH of SEQ ID NO: 26; or an HCDR1, an HCDR2 and an HCDR3 of a VH of SEQ ID NO: 27. In some embodiments, the antigen binding domain that binds CD33 comprises the VH of any one of SEQ ID NOs: 18, 19, 20, 21, 22, 23, 24, 25, 26, or 27.

In some embodiments, the Fab comprises an HCDR1, an HCDR2, and an HCDR3 of SEQ ID NOs: 1, 6, and 12, respectively; SEQ ID NOs: 2, 7, and 13, respectively; SEQ ID NOs: 1, 8, and 14, respectively; SEQ ID NOs: 3, 9, and 13, respectively; SEQ ID NOs: 4, 10, and 15, respectively; SEQ ID NOs: 5, 11, and 16, respectively; or SEQ ID NOs: 5, 11, and 17, respectively.

In some embodiments, the Fab comprises

a) an HCDR1, an HCDR2 and an HCDR3 of a heavy chain variable region (VH) of SEQ ID NO: 52;
b) an HCDR1, an HCDR2 and an HCDR3 of a VH of SEQ ID NO: 53;
c) an HCDR1, an HCDR2 and an HCDR3 of a VH of SEQ ID NO: 54;
d) an HCDR1, an HCDR2 and an HCDR3 of a VH of SEQ ID NO: 55;
e) an HCDR1, an HCDR2 and an HCDR3 of a VH of SEQ ID NO: 56;
f) an HCDR1, an HCDR2 and an HCDR3 of a VH of SEQ ID NO: 57;
g) an HCDR1, an HCDR2 and an HCDR3 of a VH of SEQ ID NO: 58;
h) an HCDR1, an HCDR2 and an HCDR3 of a VH of SEQ ID NO: 59;
i) an HCDR1, an HCDR2 and an HCDR3 of a VH of SEQ ID NO: 60;
j) an HCDR1, an HCDR2 and an HCDR3 of a VH of SEQ ID NO: 61;
k) an HCDR1, an HCDR2 and an HCDR3 of a VH of SEQ ID NO: 262;
l) an HCDR1, an HCDR2 and an HCDR3 of a VH of SEQ ID NO: 263; or
m) an HCDR1, an HCDR2 and an HCDR3 of a VH of SEQ ID NO: 264.

In certain embodiments, the Fab comprises an HCDR1, an HCDR2 and an HCDR3 of a VH of SEQ ID NO: 53.
In other embodiments, the Fab comprises an HCDR1, an HCDR2 and an HCDR3 of a VH of SEQ ID NO: 56.
In other embodiments, the Fab comprises an HCDR1, an HCDR2 and an HCDR3 of a VH of SEQ ID NO: 59.
In other embodiments, the Fab comprises an HCDR1, an HCDR2 and an HCDR3 of a VH of SEQ ID NO: 262.
In other embodiments, the Fab comprises an HCDR1, an HCDR2 and an HCDR3 of a VH of SEQ ID NO: 263.
In other embodiments, the Fab comprises an HCDR1, an HCDR2 and an HCDR3 of a VH of SEQ ID NO: 264.
Exemplary HCDR sequences are described in Tables 4a, 4b, 4c, 4d, 4e and 4f.
In some embodiments, the Fab comprises the VH of SEQ ID NOs: 52, 53, 54, 55, 56, 57, 58, 59, 60, 61, 262, 263 or 264. In some embodiments, the Fab comprises the VL of SEQ ID NOs: 81, 82, 83, 84, 85, 86, 87, 88, 271, 272 or 273. In some embodiments, the Fab comprises the VH of SEQ ID NOs: 52, 53, 54, 55, 56, 57, 58, 59, 60, 61, 262, 263 or 264, and the VL of SEQ ID NOs: 81, 82, 83, 84, 85, 86, 87, or 88, 271, 272 or 273.
In some embodiments, the Fab comprises an HCDR1, an HCDR2, and an HCDR3 of

a) SEQ ID NOs: 28, 36, and 45, respectively;
b) SEQ ID NOs: 29, 37, and 46, respectively;
c) SEQ ID NOs: 30, 38, and 47, respectively;
d) SEQ ID NOs: 31, 39, and 48, respectively;
e) SEQ ID NOs: 32, 40, and 49, respectively;
f) SEQ ID NOs: 33, 41, and 50, respectively;
g) SEQ ID NOs: 34, 42, and 51, respectively;
h) SEQ ID NOs: 34, 43, and 51, respectively;
i) SEQ ID NOs: 34, 44, and 51, respectively; or
j) SEQ ID NOs: 35, 42, and 51, respectively; or
l) SEQ ID NOs: 253, 256, and 259, respectively;
m) SEQ ID NOs: 254, 257, and 260, respectively;
n) SEQ ID NOs: 255, 258, and 261, respectively.

In some embodiments, the Fab comprises an HCDR1, an HCDR2 and an HCDR3 of a heavy chain variable region (VH) of SEQ ID NO: 52 and a light chain complementarity determining region (LCDR) 1, an LCDR2 and an LCDR3 of a light chain variable region (VL) of SEQ ID NO: 81; an HCDR1, an HCDR2 and an HCDR3 of a VH of SEQ ID NO: 53 and an LCDR1, an LCDR2 and an LCDR3 of a VL of SEQ ID NO: 82; an HCDR1, an HCDR2 and an HCDR3 of a VH of SEQ ID NO: 54 and an LCDR1, an LCDR2 and an LCDR3 of a VL of SEQ ID NO: 83; an HCDR1, an HCDR2 and an HCDR3 of a VH of SEQ ID NO: 55 and an LCDR1, an LCDR2 and an LCDR3 of a VL of SEQ ID NO: 84; an HCDR1, an HCDR2 and an HCDR3 of a VH of SEQ ID NO: 56 and an LCDR1, an LCDR2 and an LCDR3 of a VL of SEQ ID NO: 85; an HCDR1, an HCDR2 and an HCDR3 of a VH of SEQ ID NO: 57 and an LCDR1, an LCDR2 and an LCDR3 of a VL of SEQ ID NO: 86; an HCDR1, an HCDR2 and an HCDR3 of a VH of SEQ ID NO: 58 and an LCDR1, an LCDR2 and an LCDR3 of a VL of SEQ ID NO: 87; an HCDR1, an HCDR2 and an HCDR3 of a VH of SEQ ID NO: 59 and an LCDR1, an LCDR2 and an LCDR3 of a VL of SEQ ID NO: 87; an HCDR1, an HCDR2 and an HCDR3 of a VH of SEQ ID NO: 60 and an LCDR1, an LCDR2 and an LCDR3 of a VL of SEQ ID NO: 87; an HCDR1, an HCDR2 and an HCDR3 of a VH of SEQ ID NO: 61 and an LCDR1, an LCDR2 and an LCDR3 of a VL of SEQ ID NO: 88; an HCDR1, an HCDR2 and an HCDR3 of a VH of SEQ ID NO: 262 and an LCDR1, an LCDR2 and an LCDR3 of a VL of SEQ ID NO: 271; an HCDR1, an HCDR2 and an HCDR3 of a VH of SEQ ID NO: 263 and an LCDR1, an LCDR2 and

an LCDR3 of a VL of SEQ ID NO: 272; or an HCDR1, an HCDR2 and an HCDR3 of a VH of SEQ ID NO: 264 and an LCDR1, an LCDR2 and an LCDR3 of a VL of SEQ ID NO: 273.

Exemplary HCDR and LCDR sequences are described in Tables 4a, 4b, 4c, 4d, 4e and 4f.

In certain embodiments, the Fab comprises an HCDR1, an HCDR2 and an HCDR3 of a VH of SEQ ID NO: 53 and an LCDR1, an LCDR2 and an LCDR3 of a VL of SEQ ID NO: 82.

In other embodiments, the Fab comprises an HCDR1, an HCDR2 and an HCDR3 of a VH of SEQ ID NO: 56 and an LCDR1, an LCDR2 and an LCDR3 of a VL of SEQ ID NO: 85.

In other embodiments, the Fab comprises an HCDR1, an HCDR2 and an HCDR3 of a VH of SEQ ID NO: 59 and an LCDR1, an LCDR2 and an LCDR3 of a VL of SEQ ID NO: 87.

In other embodiments, the Fab comprises an HCDR1, an HCDR2 and an HCDR3 of a VH of SEQ ID NO: 262 and an LCDR1, an LCDR2 and an LCDR3 of a VL of SEQ ID NO: 271.

In other embodiments, the Fab comprises an HCDR1, an HCDR2 and an HCDR3 of a VH of SEQ ID NO: 263 and an LCDR1, an LCDR2 and an LCDR3 of a VL of SEQ ID NO: 272.

In other embodiments, the Fab comprises an HCDR1, an HCDR2 and an HCDR3 of a VH of SEQ ID NO: 264 and an LCDR1, an LCDR2 and an LCDR3 of a VL of SEQ ID NO: 273.

In some embodiments, the Fab comprises an HCDR1, an HCDR2, an HCDR3, an LCDR1, an LCDR2 and an LCDR3 of

a) SEQ ID NOs: 28, 36, 45, 62, 69, and 75, respectively;
b) SEQ ID NOs: 29, 37, 46, 63, 70, and 76, respectively;
c) SEQ ID NOs: 30, 38, 47, 64, 71, and 77, respectively;
d) SEQ ID NOs: 31, 39, 48, 65, 72, and 78, respectively;
e) SEQ ID NOs: 32, 40, 49, 66, 70, and 76, respectively;
f) SEQ ID NOs: 33, 41, 50, 67, 73, and 79, respectively;
g) SEQ ID NOs: 34, 42, 51, 68, 74, and 80, respectively;
h) SEQ ID NOs: 34, 43, 51, 68, 74, and 80, respectively;
i) SEQ ID NOs: 34, 44, 51, 68, 74, and 80, respectively;
j) SEQ ID NOs: 35, 42, 51, 68, 74, and 80, respectively;
k) SEQ ID NOs: 253, 256, 259, 265, 74, and 269, respectively;
l) SEQ ID NOs: 254, 257, 260, 66, 267, and 76, respectively; or
m) SEQ ID NOs: 255, 258, 261, 266, 268, and 270, respectively.

In some embodiments, the Fab comprises:

a) the VH of SEQ ID NO: 52 and the VL of SEQ ID NO: 81;
b) the VH of SEQ ID NO: 53 and the VL of SEQ ID NO: 82;
c) the VH of SEQ ID NO: 54 and the VL of SEQ ID NO: 83;
d) the VH of SEQ ID NO: 55 and the VL of SEQ ID NO: 84;
e) the VH of SEQ ID NO: 56 and the VL of SEQ ID NO: 85;
f) the VH of SEQ ID NO: 57 and the VL of SEQ ID NO: 86;
g) the VH of SEQ ID NO: 58 and the VL of SEQ ID NO: 87;
h) the VH of SEQ ID NO: 59 and the VL of SEQ ID NO: 87;
i) the VH of SEQ ID NO: 60 and the VL of SEQ ID NO: 87;
j) the VH of SEQ ID NO: 61 and the VL of SEQ ID NO: 88;
k) the VH of SEQ ID NO: 262 and the VL of SEQ ID NO: 271;
l) the VH of SEQ ID NO: 263 and the VL of SEQ ID NO: 272; or
m) the VH of SEQ ID NO: 264 and the VL of SEQ ID NO: 273.

In some embodiments, the Fab comprises an HCDR1, an HCDR2 and an HCDR3 of a heavy chain variable region (VH) of SEQ ID NO: 95; an HCDR1, an HCDR2 and an HCDR3 of a VH of SEQ ID NO: 96; an HCDR1, an HCDR2 and an HCDR3 of a VH of SEQ ID NO: 97; an HCDR1, an HCDR2 and an HCDR3 of a VH of SEQ ID NO: 170; an HCDR1, an HCDR2 and an HCDR3 of a VH of SEQ ID NO: 171; an HCDR1, an HCDR2 and an HCDR3 of a VH of SEQ ID NO: 175; an HCDR1, an HCDR2 and an HCDR3 of a VH of SEQ ID NO: 179; an HCDR1, an HCDR2 and an HCDR3 of a VH of SEQ ID NO: 181; or an HCDR1, an HCDR2 and an HCDR3 of a VH of SEQ ID NO: 96.

In some embodiments, the Fab comprises an HCDR1, an HCDR2, and an HCDR3 of SEQ ID NOs: 89, 90, and 92, respectively; an HCDR1, an HCDR2, and the HCDR3 of SEQ ID NOs: 89, 90, and 93, respectively; an HCDR1, an HCDR2, and an HCDR3 of SEQ ID NOs: 33, 91, and 94, respectively; an HCDR1, an HCDR2, and an HCDR3 of SEQ ID NOs: 167, 168, and 169, respectively; an HCDR1, an HCDR2, and an HCDR3 of SEQ ID NOs: 172, 173, and

174, respectively; an HCDR1, an HCDR2, and an HCDR3 of SEQ ID NOs: 176, 177, and 178, respectively; an HCDR1, an HCDR2, and an HCDR3 of SEQ ID NOs: 89, 90, and 180, respectively; or an HCDR1, an HCDR2, and an HCDR3 of SEQ ID NOs: 89, 90, and 93, respectively.

In some embodiments, the Fab comprises an HCDR1, an HCDR2 and an HCDR3 of a heavy chain variable region (VH) of SEQ ID NO: 95 and an LCDR1, an LCDR2 and an LCDR3 of a light chain variable region (VL) of SEQ ID NO: 105; an HCDR1, an HCDR2 and an HCDR3 of a VH of SEQ ID NO: 96 and an LCDR1, an LCDR2 and an LCDR3 of a VL of SEQ ID NO: 106; an HCDR1, an HCDR2 and an HCDR3 of a VH of SEQ ID NO: 97 and an LCDR1, an LCDR2 and an LCDR3 of a VL of SEQ ID NO: 107; an HCDR1, an HCDR2 and an HCDR3 of a VH of SEQ ID NO: 170 and an LCDR1, an LCDR2 and an LCDR3 of a VL of SEQ ID NO: 185; an HCDR1, an HCDR2 and an HCDR3 of a VH of SEQ ID NO: 171 and an LCDR1, an LCDR2 and an LCDR3 of a VL of SEQ ID NO: 188; an HCDR1, an HCDR2 and an HCDR3 of a VH of SEQ ID NO: 175 and an LCDR1, an LCDR2 and an LCDR3 of a VL of SEQ ID NO: 192; an HCDR1, an HCDR2 and an HCDR3 of a VH of SEQ ID NO: 179 and an LCDR1, an LCDR2 and an LCDR3 of a VL of SEQ ID NO: 196; an HCDR1, an HCDR2 and an HCDR3 of a VH of SEQ ID NO: 181 and an LCDR1, an LCDR2 and an LCDR3 of a VL of SEQ ID NO: 200; or an HCDR1, an HCDR2 and an HCDR3 of a VH of SEQ ID NO: 96 and an LCDR1, an LCDR2 and an LCDR3 of a VL of SEQ ID NO: 202.

In some embodiments, the Fab comprises an HCDR1, an HCDR2, an HCDR3, an LCDR1, an LCDR2 and an LCDR3 of

a) SEQ ID NOs: 89, 90, 92, 98, 101, and 104, respectively;
b) SEQ ID NOs: 89, 90, 93, 99, 102, and 104, respectively;
c) SEQ ID NOs: 33, 91, 94, 100, 103, and 104, respectively;
d) SEQ ID NOs: 167, 168, 169, 182, 183, and 184, respectively;
e) SEQ ID NOs: 33, 91, 94, 186, 187, and 104, respectively;
f) SEQ ID NOs: 172, 173, 174, 189, 190, and 191, respectively;
g) SEQ ID NOs: 176, 177, 178, 193, 194, and 195, respectively;
h) SEQ ID NOs: 89, 90, 180, 197, 198, and 199, respectively;
i) SEQ ID NOs: 89, 90, 93, 201, 102, and 104, respectively.

In some embodiments, the Fab comprises a VH of SEQ ID NOs: 95, 96, 97, 170, 171, 175, 179, 181, or 96, and a VL of SEQ ID NOs: 105, 106, 107, 185, 188, 192, 196, 200, or 202. In some embodiments, the Fab comprises the VH of SEQ ID NO: 95 and the VL of SEQ ID NO: 105; the VH of SEQ ID NO: 96 and the VL of SEQ ID NO: 106; the VH of SEQ ID NO: 97 and the VL of SEQ ID NO: 107; the VH of SEQ ID NO: 170 and the VL of SEQ ID NO: 185; the VH of SEQ ID NO: 171 and the VL of SEQ ID NO: 188; the VH of SEQ ID NO: 175 and the VL of SEQ ID NO: 192; the VH of SEQ ID NO: 179 and the VL of SEQ ID NO: 196; the VH of SEQ ID NO: 181 and the VL of SEQ ID NO: 200; or the VH of SEQ ID NO: 96 and the VL of SEQ ID NO: 202.

In other embodiments, the VH and VL domains identified herein are engineered into a F(ab')$_2$.

In some embodiments, the F(ab')$_2$ comprises an HCDR1, a HCDR2 and a HCDR3 of a heavy chain variable region (VH) of SEQ ID NO: 18; an HCDR1, an HCDR2 and an HCDR3 of a VH of SEQ ID NO: 19; an HCDR1, an HCDR2 and an HCDR3 of a VH of SEQ ID NO: 20; an HCDR1, an HCDR2 and an HCDR3 of a VH of SEQ ID NO: 21; an HCDR1, an HCDR2 and an HCDR3 of a VH of SEQ ID NO: 22; an HCDR1, an HCDR2 and an HCDR3 of a VH of SEQ ID NO: 23; an HCDR1, an HCDR2 and an HCDR3 of a VH of SEQ ID NO: 24; an HCDR1, an HCDR2 and an HCDR3 of a VH of SEQ ID NO: 25; an HCDR1, an HCDR2 and an HCDR3 of a VH of SEQ ID NO: 26; or an HCDR1, an HCDR2 and an HCDR3 of a VH of SEQ ID NO: 27. In some embodiments, the antigen binding domain that binds CD33 comprises the VH of any one of SEQ ID NOs: 18, 19, 20, 21, 22, 23, 24, 25, 26, or 27.

In some embodiments, the F(ab')$_2$ comprises an HCDR1, an HCDR2, and an HCDR3 of SEQ ID NOs: 1, 6, and 12, respectively; SEQ ID NOs: 2, 7, and 13, respectively; SEQ ID NOs: 1, 8, and 14, respectively; SEQ ID NOs: 3, 9, and 13, respectively; SEQ ID NOs: 4, 10, and 15, respectively; SEQ ID NOs: 5, 11, and 16, respectively; or SEQ ID NOs: 5, 11, and 17, respectively.

In some embodiments, the F(ab')$_2$ comprises

a) an HCDR1, an HCDR2 and an HCDR3 of a heavy chain variable region (VH) of SEQ ID NO: 52;
b) an HCDR1, an HCDR2 and an HCDR3 of a VH of SEQ ID NO: 53;
c) an HCDR1, an HCDR2 and an HCDR3 of a VH of SEQ ID NO: 54;
d) an HCDR1, an HCDR2 and an HCDR3 of a VH of SEQ ID NO: 55;
e) an HCDR1, an HCDR2 and an HCDR3 of a VH of SEQ ID NO: 56;
f) an HCDR1, an HCDR2 and an HCDR3 of a VH of SEQ ID NO: 57;
g) an HCDR1, an HCDR2 and an HCDR3 of a VH of SEQ ID NO: 58;
h) an HCDR1, an HCDR2 and an HCDR3 of a VH of SEQ ID NO: 59;

i) an HCDR1, an HCDR2 and an HCDR3 of a VH of SEQ ID NO: 60;
j) an HCDR1, an HCDR2 and an HCDR3 of a VH of SEQ ID NO: 61;
k) an HCDR1, an HCDR2 and an HCDR3 of a VH of SEQ ID NO: 262;
l) an HCDR1, an HCDR2 and an HCDR3 of a VH of SEQ ID NO: 263; or
m) an HCDR1, an HCDR2 and an HCDR3 of a VH of SEQ ID NO: 264.

In some embodiments, the F(ab')$_2$ comprises the VH of SEQ ID NOs: 52, 53, 54, 55, 56, 57, 58, 59, 60, 61, 262, 263 or 264. In some embodiments, the F(ab')$_2$ comprises the VL of SEQ ID NOs: 81, 82, 83, 84, 85, 86, 87, 88, 271, 272 or 273. In some embodiments, the F(ab')$_2$ comprises the VH of SEQ ID NOs: 52, 53, 54, 55, 56, 57, 58, 59, 60 61, 262, 263 or 264 and the VL of SEQ ID NOs: 81, 82, 83, 84, 85, 86, 87, 88, 271, 272 or 273.
In some embodiments, the F(ab')$_2$ comprises an HCDR1, an HCDR2, and an HCDR3 of

a) SEQ ID NOs: 28, 36, and 45, respectively;
b) SEQ ID NOs: 29, 37, and 46, respectively;
c) SEQ ID NOs: 30, 38, and 47, respectively;
d) SEQ ID NOs: 31, 39, and 48, respectively;
e) SEQ ID NOs: 32, 40, and 49, respectively;
f) SEQ ID NOs: 33, 41, and 50, respectively;
g) SEQ ID NOs: 34, 42, and 51, respectively;
h) SEQ ID NOs: 34, 43, and 51, respectively;
i) SEQ ID NOs: 34, 44, and 51, respectively;
j) SEQ ID NOs: 35, 42, and 51, respectively;
k) SEQ ID NOs: 253, 256 and 259, respectively
l) SEQ ID NOs: 254, 257 and 260, respectively
m) SEQ ID NOs: 255, 258 and 261 respectively.

In some embodiments, the F(ab')$_2$ comprises an HCDR1, an HCDR2 and an HCDR3 of a heavy chain variable region (VH) of SEQ ID NO: 52 and a light chain complementarity determining region (LCDR) 1, an LCDR2 and an LCDR3 of a light chain variable region (VL) of SEQ ID NO: 81; an HCDR1, an HCDR2 and an HCDR3 of a VH of SEQ ID NO: 53 and an LCDR1, an LCDR2 and an LCDR3 of a VL of SEQ ID NO: 82; an HCDR1, an HCDR2 and an HCDR3 of a VH of SEQ ID NO: 54 and an LCDR1, an LCDR2 and an LCDR3 of a VL of SEQ ID NO: 83; an HCDR1, an HCDR2 and an HCDR3 of a VH of SEQ ID NO: 55 and an LCDR1, an LCDR2 and an LCDR3 of a VL of SEQ ID NO: 84; an HCDR1, an HCDR2 and an HCDR3 of a VH of SEQ ID NO: 56 and an LCDR1, an LCDR2 and an LCDR3 of a VL of SEQ ID NO: 85; an HCDR1, an HCDR2 and an HCDR3 of a VH of SEQ ID NO: 57 and an LCDR1, an LCDR2 and an LCDR3 of a VL of SEQ ID NO: 86; an HCDR1, an HCDR2 and an HCDR3 of a VH of SEQ ID NO: 58 and an LCDR1, an LCDR2 and an LCDR3 of a VL of SEQ ID NO: 87; an HCDR1, an HCDR2 and an HCDR3 of a VH of SEQ ID NO: 59 and an LCDR1, an LCDR2 and an LCDR3 of a VL of SEQ ID NO: 87; an HCDR1, an HCDR2 and an HCDR3 of a VH of SEQ ID NO: 60 and an LCDR1, an LCDR2 and an LCDR3 of a VL of SEQ ID NO: 87; an HCDR1, an HCDR2 and an HCDR3 of a VH of SEQ ID NO: 61 and an LCDR1, an LCDR2 and an LCDR3 of a VL of SEQ ID NO: 88 an HCDR1, an HCDR2 and an HCDR3 of a VH of SEQ ID NO: 262 and an LCDR1, an LCDR2 and an LCDR3 of a VL of SEQ ID NO: 271; an HCDR1, an HCDR2 and an HCDR3 of a VH of SEQ ID NO: 263 and an LCDR1, an LCDR2 and an LCDR3 of a VL of SEQ ID NO: 272; or an HCDR1, an HCDR2 and an HCDR3 of a VH of SEQ ID NO: 264 and an LCDR1, an LCDR2 and an LCDR3 of a VL of SEQ ID NO: 273.
In some embodiments, the F(ab')$_2$ comprises an HCDR1, an HCDR2, an HCDR3, an LCDR1, an LCDR2 and an LCDR3 of

a) SEQ ID NOs: 28, 36, 45, 62, 69, and 75, respectively;
b) SEQ ID NOs: 29, 37, 46, 63, 70, and 76, respectively;
c) SEQ ID NOs: 30, 38, 47, 64, 71, and 77, respectively;
d) SEQ ID NOs: 31, 39, 48, 65, 72, and 78, respectively;
e) SEQ ID NOs: 32, 40, 49, 66, 70, and 76, respectively;
f) SEQ ID NOs: 33, 41, 50, 67, 73, and 79, respectively;
g) SEQ ID NOs: 34, 42, 51, 68, 74, and 80, respectively;
h) SEQ ID NOs: 34, 43, 51, 68, 74, and 80, respectively;
i) SEQ ID NOs: 34, 44, 51, 68, 74, and 80, respectively;
j) SEQ ID NOs: 35, 42, 51, 68, 74, and 80, respectively;
k) SEQ ID NOs: 253, 256, 259, 265, 74, and 269, respectively;
l) SEQ ID NOs: 254, 257, 260, 66, 267, and 76, respectively; or

m) SEQ ID NOs: 255, 258, 261, 266, 268, and 270, respectively.

In some embodiments, the F(ab')$_2$ comprises:

a) the VH of SEQ ID NO: 52 and the VL of SEQ ID NO: 81;
b) the VH of SEQ ID NO: 53 and the VL of SEQ ID NO: 82;
c) the VH of SEQ ID NO: 54 and the VL of SEQ ID NO: 83;
d) the VH of SEQ ID NO: 55 and the VL of SEQ ID NO: 84;
e) the VH of SEQ ID NO: 56 and the VL of SEQ ID NO: 85;
f) the VH of SEQ ID NO: 57 and the VL of SEQ ID NO: 86;
g) the VH of SEQ ID NO: 58 and the VL of SEQ ID NO: 87;
h) the VH of SEQ ID NO: 59 and the VL of SEQ ID NO: 87;
i) the VH of SEQ ID NO: 60 and the VL of SEQ ID NO: 87;
j) the VH of SEQ ID NO: 61 and the VL of SEQ ID NO: 88;
k) the VH of SEQ ID NO: 262 and the VL of SEQ ID NO: 271;
l) the VH of SEQ ID NO: 263 and the VL of SEQ ID NO: 272; or
m) the VH of SEQ ID NO: 264 and the VL of SEQ ID NO: 273.

In some embodiments, the F(ab')$_2$ comprises an HCDR1, an HCDR2 and an HCDR3 of a heavy chain variable region (VH) of SEQ ID NO: 95; an HCDR1, an HCDR2 and an HCDR3 of a VH of SEQ ID NO: 96; an HCDR1, an HCDR2 and an HCDR3 of a VH of SEQ ID NO: 97; an HCDR1, an HCDR2 and an HCDR3 of a VH of SEQ ID NO: 170; an HCDR1, an HCDR2 and an HCDR3 of a VH of SEQ ID NO: 171; an HCDR1, an HCDR2 and an HCDR3 of a VH of SEQ ID NO: 175; an HCDR1, an HCDR2 and an HCDR3 of a VH of SEQ ID NO: 179; an HCDR1, an HCDR2 and an HCDR3 of a VH of SEQ ID NO: 181; or an HCDR1, an HCDR2 and an HCDR3 of a VH of SEQ ID NO: 96.

In some embodiments, the F(ab')$_2$ comprises an HCDR1, an HCDR2, and an HCDR3 of SEQ ID NOs: 89, 90, and 92, respectively; an HCDR1, an HCDR2, and the HCDR3 of SEQ ID NOs: 89, 90, and 93, respectively; an HCDR1, an HCDR2, and an HCDR3 of SEQ ID NOs: 33, 91, and 94, respectively; an HCDR1, an HCDR2, and an HCDR3 of SEQ ID NOs: 167, 168, and 169, respectively; an HCDR1, an HCDR2, and an HCDR3 of SEQ ID NOs: 172, 173, and 174, respectively; an HCDR1, an HCDR2, and an HCDR3 of SEQ ID NOs: 176, 177, and 178, respectively; an HCDR1, an HCDR2, and an HCDR3 of SEQ ID NOs: 89, 90, and 180, respectively; or an HCDR1, an HCDR2, and an HCDR3 of SEQ ID NOs: 89, 90, and 93, respectively.

In some embodiments, the F(ab')$_2$ comprises an HCDR1, an HCDR2 and an HCDR3 of a heavy chain variable region (VH) of SEQ ID NO: 95 and an LCDR1, an LCDR2 and an LCDR3 of a light chain variable region (VL) of SEQ ID NO: 105; an HCDR1, an HCDR2 and an HCDR3 of a VH of SEQ ID NO: 96 and an LCDR1, an LCDR2 and an LCDR3 of a VL of SEQ ID NO: 106; an HCDR1, an HCDR2 and an HCDR3 of a VH of SEQ ID NO: 97 and an LCDR1, an LCDR2 and an LCDR3 of a VL of SEQ ID NO: 107; an HCDR1, an HCDR2 and an HCDR3 of a VH of SEQ ID NO: 170 and an LCDR1, an LCDR2 and an LCDR3 of a VL of SEQ ID NO: 185; an HCDR1, an HCDR2 and an HCDR3 of a VH of SEQ ID NO: 171 and an LCDR1, an LCDR2 and an LCDR3 of a VL of SEQ ID NO: 188; an HCDR1, an HCDR2 and an HCDR3 of a VH of SEQ ID NO: 175 and an LCDR1, an LCDR2 and an LCDR3 of a VL of SEQ ID NO: 192; an HCDR1, an HCDR2 and an HCDR3 of a VH of SEQ ID NO: 179 and an LCDR1, an LCDR2 and an LCDR3 of a VL of SEQ ID NO: 196; an HCDR1, an HCDR2 and an HCDR3 of a VH of SEQ ID NO: 181 and an LCDR1, an LCDR2 and an LCDR3 of a VL of SEQ ID NO: 200; or an HCDR1, an HCDR2 and an HCDR3 of a VH of SEQ ID NO: 96 and an LCDR1, an LCDR2 and an LCDR3 of a VL of SEQ ID NO: 202.

In some embodiments, the F(ab')$_2$ comprises an HCDR1, an HCDR2, an HCDR3, an LCDR1, an LCDR2 and an LCDR3 of

a) SEQ ID NOs: 89, 90, 92, 98, 101, and 104, respectively;
b) SEQ ID NOs: 89, 90, 93, 99, 102, and 104, respectively;
c) SEQ ID NOs: 33, 91, 94, 100, 103, and 104, respectively;
d) SEQ ID NOs: 167, 168, 169, 182, 183, and 184, respectively;
e) SEQ ID NOs: 33, 91, 94, 186, 187, and 104, respectively;
f) SEQ ID NOs: 172, 173, 174, 189, 190, and 191, respectively;
g) SEQ ID NOs: 176, 177, 178, 193, 194, and 195, respectively;
h) SEQ ID NOs: 89, 90, 180, 197, 198, and 199, respectively;
i) SEQ ID NOs: 89, 90, 93, 201, 102, and 104, respectively.

In some embodiments, the F(ab')$_2$ comprises a VH of SEQ ID NOs: 95, 96, 97, 170, 171, 175, 179, 181, or 196, and a VL of SEQ ID NOs: 105, 106, 107, 185, 188, 192, 196, 200, or 202. In some embodiments, the F(ab')$_2$

comprises the VH of SEQ ID NO: 95 and the VL of SEQ ID NO: 105; the VH of SEQ ID NO: 96 and the VL of SEQ ID NO: 106; the VH of SEQ ID NO: 97 and the VL of SEQ ID NO: 107; the VH of SEQ ID NO: 170 and the VL of SEQ ID NO: 185; the VH of SEQ ID NO: 171 and the VL of SEQ ID NO: 188; the VH of SEQ ID NO: 175 and the VL of SEQ ID NO: 192; the VH of SEQ ID NO: 179 and the VL of SEQ ID NO: 196; the VH of SEQ ID NO: 181 and the VL of SEQ ID NO: 200; or the VH of SEQ ID NO: 96 and the VL of SEQ ID NO: 202.

In certain embodiments, the VH and VL domains identified herein are engineered into a Fv.

In some embodiments, the Fv comprises a heavy chain complementarity determining region (HCDR) 1, a HCDR2 and a HCDR3 of a heavy chain variable region (VH) of SEQ ID NO: 18; an HCDR1, an HCDR2 and an HCDR3 of a VH of SEQ ID NO: 19; an HCDR1, an HCDR2 and an HCDR3 of a VH of SEQ ID NO: 20; an HCDR1, an HCDR2 and an HCDR3 of a VH of SEQ ID NO: 21; an HCDR1, an HCDR2 and an HCDR3 of a VH of SEQ ID NO: 22; an HCDR1, an HCDR2 and an HCDR3 of a VH of SEQ ID NO: 23; an HCDR1, an HCDR2 and an HCDR3 of a VH of SEQ ID NO: 24; an HCDR1, an HCDR2 and an HCDR3 of a VH of SEQ ID NO: 25; an HCDR1, an HCDR2 and an HCDR3 of a VH of SEQ ID NO: 26; or an HCDR1, an HCDR2 and an HCDR3 of a VH of SEQ ID NO: 27. In some embodiments, the antigen binding domain that binds CD33 comprises the VH of any one of SEQ ID NOs: 18, 19, 20, 21, 22, 23, 24, 25, 26, or 27.

In some embodiments, the Fv comprises an HCDR1, an HCDR2, and an HCDR3 of SEQ ID NOs: 1, 6, and 12, respectively; SEQ ID NOs: 2, 7, and 13, respectively; SEQ ID NOs: 1, 8, and 14, respectively; SEQ ID NOs: 3, 9, and 13, respectively; SEQ ID NOs: 4, 10, and 15, respectively; SEQ ID NOs: 5, 11, and 16, respectively; or SEQ ID NOs: 5, 11, and 17, respectively.

In some embodiments, the Fv comprises

a) an HCDR1, an HCDR2 and an HCDR3 of a heavy chain variable region (VH) of SEQ ID NO: 52;
b) an HCDR1, an HCDR2 and an HCDR3 of a VH of SEQ ID NO: 53;
c) an HCDR1, an HCDR2 and an HCDR3 of a VH of SEQ ID NO: 54;
d) an HCDR1, an HCDR2 and an HCDR3 of a VH of SEQ ID NO: 55;
e) an HCDR1, an HCDR2 and an HCDR3 of a VH of SEQ ID NO: 56;
f) an HCDR1, an HCDR2 and an HCDR3 of a VH of SEQ ID NO: 57;
g) an HCDR1, an HCDR2 and an HCDR3 of a VH of SEQ ID NO: 58;
h) an HCDR1, an HCDR2 and an HCDR3 of a VH of SEQ ID NO: 59;
i) an HCDR1, an HCDR2 and an HCDR3 of a VH of SEQ ID NO: 60;
j) an HCDR1, an HCDR2 and an HCDR3 of a VH of SEQ ID NO: 61;
k) an HCDR1, an HCDR2 and an HCDR3 of a VH of SEQ ID NO: 262;
l) an HCDR1, an HCDR2 and an HCDR3 of a VH of SEQ ID NO: 263; or
m) an HCDR1, an HCDR2 and an HCDR3 of a VH of SEQ ID NO: 264.

In certain embodiments, the Fv comprises an HCDR1, an HCDR2 and an HCDR3 of a VH of SEQ ID NO: 53.

In other embodiments, the Fv comprises an HCDR1, an HCDR2 and an HCDR3 of a VH of SEQ ID NO: 56.

In other embodiments, the Fv comprises an HCDR1, an HCDR2 and an HCDR3 of a VH of SEQ ID NO: 59.

In other embodiments, the Fv comprises an HCDR1, an HCDR2 and an HCDR3 of a VH of SEQ ID NO: 262.

In other embodiments, the Fv comprises an HCDR1, an HCDR2 and an HCDR3 of a VH of SEQ ID NO: 263.

In other embodiments, the Fv comprises an HCDR1, an HCDR2 and an HCDR3 of a VH of SEQ ID NO: 264.

In some embodiments, the Fv comprises the VH of SEQ ID NOs: 52, 53, 54, 55, 56, 57, 58, 59, 60, 61, 262, 263 or 264. In some embodiments, the Fv comprises the VL of SEQ ID NOs: 81, 82, 83, 84, 85, 86, 87, 88, 271, 272, or 273. In some embodiments, the Fv comprises the VH of SEQ ID NOs: 52, 53, 54, 55, 56, 57, 58, 59, 60,r 61, 262, 263 or 264, and the VL of SEQ ID NOs: 81, 82, 83, 84, 85, 86, 87, 88, 271, 272, 273.

In some embodiments, the Fv comprises an HCDR1, an HCDR2, and an HCDR3 of

a) SEQ ID NOs: 28, 36, and 45, respectively;
b) SEQ ID NOs: 29, 37, and 46, respectively;
c) SEQ ID NOs: 30, 38, and 47, respectively;
d) SEQ ID NOs: 31, 39, and 48, respectively;
e) SEQ ID NOs: 32, 40, and 49, respectively;
f) SEQ ID NOs: 33, 41, and 50, respectively;
g) SEQ ID NOs: 34, 42, and 51, respectively;
h) SEQ ID NOs: 34, 43, and 51, respectively;
i) SEQ ID NOs: 34, 44, and 51, respectively;
j) SEQ ID NOs: 35, 42, and 51, respectively;
k) SEQ ID NOs: 253, 256 and 259, respectively

l) SEQ ID NOs: 254, 257 and 260, respectively
m) SEQ ID NOs: 255, 258 and 261 respectively.

In some embodiments, the Fv comprises an HCDR1, an HCDR2 and an HCDR3 of a heavy chain variable region (VH) of SEQ ID NO: 52 and a light chain complementarity determining region (LCDR) 1, an LCDR2 and an LCDR3 of a light chain variable region (VL) of SEQ ID NO: 81; an HCDR1, an HCDR2 and an HCDR3 of a VH of SEQ ID NO: 53 and an LCDR1, an LCDR2 and an LCDR3 of a VL of SEQ ID NO: 82; an HCDR1, an HCDR2 and an HCDR3 of a VH of SEQ ID NO: 54 and an LCDR1, an LCDR2 and an LCDR3 of a VL of SEQ ID NO: 83; an HCDR1, an HCDR2 and an HCDR3 of a VH of SEQ ID NO: 55 and an LCDR1, an LCDR2 and an LCDR3 of a VL of SEQ ID NO: 84; an HCDR1, an HCDR2 and an HCDR3 of a VH of SEQ ID NO: 56 and an LCDR1, an LCDR2 and an LCDR3 of a VL of SEQ ID NO: 85; an HCDR1, an HCDR2 and an HCDR3 of a VH of SEQ ID NO: 57 and an LCDR1, an LCDR2 and an LCDR3 of a VL of SEQ ID NO: 86; an HCDR1, an HCDR2 and an HCDR3 of a VH of SEQ ID NO: 58 and an LCDR1, an LCDR2 and an LCDR3 of a VL of SEQ ID NO: 87; an HCDR1, an HCDR2 and an HCDR3 of a VH of SEQ ID NO: 59 and an LCDR1, an LCDR2 and an LCDR3 of a VL of SEQ ID NO: 87; an HCDR1, an HCDR2 and an HCDR3 of a VH of SEQ ID NO: 60 and an LCDR1, an LCDR2 and an LCDR3 of a VL of SEQ ID NO: 87; an HCDR1, an HCDR2 and an HCDR3 of a VH of SEQ ID NO: 61 and an LCDR1, an LCDR2 and an LCDR3 of a VL of SEQ ID NO: 88; an HCDR1, an HCDR2 and an HCDR3 of a VH of SEQ ID NO: 262 and an LCDR1, an LCDR2 and an LCDR3 of a VL of SEQ ID NO: 271; an HCDR1, an HCDR2 and an HCDR3 of a VH of SEQ ID NO: 263 and an LCDR1, an LCDR2 and an LCDR3 of a VL of SEQ ID NO: 272; or an HCDR1, an HCDR2 and an HCDR3 of a VH of SEQ ID NO: 264 and an LCDR1, an LCDR2 and an LCDR3 of a VL of SEQ ID NO: 273.

In certain embodiment, the Fv comprises an HCDR1, an HCDR2 and an HCDR3 of a VH of SEQ ID NO: 53 and an LCDR1, an LCDR2 and an LCDR3 of a VL of SEQ ID NO: 82.

In other embodiments, the Fv comprises an HCDR1, an HCDR2 and an HCDR3 of a VH of SEQ ID NO: 56 and an LCDR1, an LCDR2 and an LCDR3 of a VL of SEQ ID NO: 85.

In other embodiments, the Fv comprises an HCDR1, an HCDR2 and an HCDR3 of a VH of SEQ ID NO: 59 and an LCDR1, an LCDR2 and an LCDR3 of a VL of SEQ ID NO: 87.

In other embodiments, the Fv comprises an HCDR1, an HCDR2 and an HCDR3 of a VH of SEQ ID NO: 262 and an LCDR1, an LCDR2 and an LCDR3 of a VL of SEQ ID NO: 271.

In other embodiments the Fv comprises an HCDR1, an HCDR2 and an HCDR3 of a VH of SEQ ID NO: 263 and an LCDR1, an LCDR2 and an LCDR3 of a VL of SEQ ID NO: 272.

In other embodiments, the Fv comprises an HCDR1, an HCDR2 and an HCDR3 of a VH of SEQ ID NO: 264 and an LCDR1, an LCDR2 and an LCDR3 of a VL of SEQ ID NO: 273.

In some embodiments, the Fv comprises an HCDR1, an HCDR2, an HCDR3, an LCDR1, an LCDR2 and an LCDR3 of

a) SEQ ID NOs: 28, 36, 45, 62, 69, and 75, respectively;
b) SEQ ID NOs: 29, 37, 46, 63, 70, and 76, respectively;
c) SEQ ID NOs: 30, 38, 47, 64, 71, and 77, respectively;
d) SEQ ID NOs: 31, 39, 48, 65, 72, and 78, respectively;
e) SEQ ID NOs: 32, 40, 49, 66, 70, and 76, respectively;
f) SEQ ID NOs: 33, 41, 50, 67, 73, and 79, respectively;
g) SEQ ID NOs: 34, 42, 51, 68, 74, and 80, respectively;
h) SEQ ID NOs: 34, 43, 51, 68, 74, and 80, respectively;
i) SEQ ID NOs: 34, 44, 51, 68, 74, and 80, respectively; or
j) SEQ ID NOs: 35, 42, 51, 68, 74, and 80, respectively.

In some embodiments, the Fv comprises:

a) the VH of SEQ ID NO: 52 and the VL of SEQ ID NO: 81;
b) the VH of SEQ ID NO: 53 and the VL of SEQ ID NO: 82;
c) the VH of SEQ ID NO: 54 and the VL of SEQ ID NO: 83;
d) the VH of SEQ ID NO: 55 and the VL of SEQ ID NO: 84;
e) the VH of SEQ ID NO: 56 and the VL of SEQ ID NO: 85;
f) the VH of SEQ ID NO: 57 and the VL of SEQ ID NO: 86;
g) the VH of SEQ ID NO: 58 and the VL of SEQ ID NO: 87;
h) the VH of SEQ ID NO: 59 and the VL of SEQ ID NO: 87;
i) the VH of SEQ ID NO: 60 and the VL of SEQ ID NO: 87;
j) the VH of SEQ ID NO: 61 and the VL of SEQ ID NO: 88;

k) the VH of SEQ ID NO: 262 and the VL of SEQ ID NO: 271;
l) the VH of SEQ ID NO: 263 and the VL of SEQ ID NO: 272; or
m) the VH of SEQ ID NO: 264 and the VL of SEQ ID NO: 273.

In some embodiments, the Fv comprises an HCDR1, an HCDR2 and an HCDR3 of a heavy chain variable region (VH) of SEQ ID NO: 95; an HCDR1, an HCDR2 and an HCDR3 of a VH of SEQ ID NO: 96; an HCDR1, an HCDR2 and an HCDR3 of a VH of SEQ ID NO: 97; an HCDR1, an HCDR2 and an HCDR3 of a VH of SEQ ID NO: 170; an HCDR1, an HCDR2 and an HCDR3 of a VH of SEQ ID NO: 171; an HCDR1, an HCDR2 and an HCDR3 of a VH of SEQ ID NO: 175; an HCDR1, an HCDR2 and an HCDR3 of a VH of SEQ ID NO: 179; an HCDR1, an HCDR2 and an HCDR3 of a VH of SEQ ID NO: 181; or an HCDR1, an HCDR2 and an HCDR3 of a VH of SEQ ID NO: 96.

In some embodiments, the Fv comprises an HCDR1, an HCDR2, and an HCDR3 of SEQ ID NOs: 89, 90, and 92, respectively; an HCDR1, an HCDR2, and the HCDR3 of SEQ ID NOs: 89, 90, and 93, respectively; an HCDR1, an HCDR2, and an HCDR3 of SEQ ID NOs: 33, 91, and 94, respectively; an HCDR1, an HCDR2, and an HCDR3 of SEQ ID NOs: 167, 168, and 169, respectively; an HCDR1, an HCDR2, and an HCDR3 of SEQ ID NOs: 172, 173, and 174, respectively; an HCDR1, an HCDR2, and an HCDR3 of SEQ ID NOs: 176, 177, and 178, respectively; an HCDR1, an HCDR2, and an HCDR3 of SEQ ID NOs: 89, 90, and 180, respectively; or an HCDR1, an HCDR2, and an HCDR3 of SEQ ID NOs: 89, 90, and 93, respectively.

In some embodiments, the Fv comprises an HCDR1, an HCDR2 and an HCDR3 of a heavy chain variable region (VH) of SEQ ID NO: 95 and an LCDR1, an LCDR2 and an LCDR3 of a light chain variable region (VL) of SEQ ID NO: 105; an HCDR1, an HCDR2 and an HCDR3 of a VH of SEQ ID NO: 96 and an LCDR1, an LCDR2 and an LCDR3 of a VL of SEQ ID NO: 106; an HCDR1, an HCDR2 and an HCDR3 of a VH of SEQ ID NO: 97 and an LCDR1, an LCDR2 and an LCDR3 of a VL of SEQ ID NO: 107; an HCDR1, an HCDR2 and an HCDR3 of a VH of SEQ ID NO: 170 and an LCDR1, an LCDR2 and an LCDR3 of a VL of SEQ ID NO: 185; an HCDR1, an HCDR2 and an HCDR3 of a VH of SEQ ID NO: 171 and an LCDR1, an LCDR2 and an LCDR3 of a VL of SEQ ID NO: 188; an HCDR1, an HCDR2 and an HCDR3 of a VH of SEQ ID NO: 175 and an LCDR1, an LCDR2 and an LCDR3 of a VL of SEQ ID NO: 192; an HCDR1, an HCDR2 and an HCDR3 of a VH of SEQ ID NO: 179 and an LCDR1, an LCDR2 and an LCDR3 of a VL of SEQ ID NO: 196; an HCDR1, an HCDR2 and an HCDR3 of a VH of SEQ ID NO: 181 and an LCDR1, an LCDR2 and an LCDR3 of a VL of SEQ ID NO: 200; or an HCDR1, an HCDR2 and an HCDR3 of a VH of SEQ ID NO: 96 and an LCDR1, an LCDR2 and an LCDR3 of a VL of SEQ ID NO: 202.

In certain embodiments, the VH and VL domains identified herein are engineered into a Fd.

In some embodiments, the Fd comprises an HCDR1, a HCDR2 and a HCDR3 of a heavy chain variable region (VH) of SEQ ID NO: 18; an HCDR1, an HCDR2 and an HCDR3 of a VH of SEQ ID NO: 19; an HCDR1, an HCDR2 and an HCDR3 of a VH of SEQ ID NO: 20; an HCDR1, an HCDR2 and an HCDR3 of a VH of SEQ ID NO: 21; an HCDR1, an HCDR2 and an HCDR3 of a VH of SEQ ID NO: 22; an HCDR1, an HCDR2 and an HCDR3 of a VH of SEQ ID NO: 23; an HCDR1, an HCDR2 and an HCDR3 of a VH of SEQ ID NO: 24; an HCDR1, an HCDR2 and an HCDR3 of a VH of SEQ ID NO: 25; an HCDR1, an HCDR2 and an HCDR3 of a VH of SEQ ID NO: 26; or an HCDR1, an HCDR2 and an HCDR3 of a VH of SEQ ID NO: 27. In some embodiments, the antigen binding domain that binds CD33 comprises the VH of any one of SEQ ID NOs: 18, 19, 20, 21, 22, 23, 24, 25, 26, or 27.

In some embodiments, the Fd comprises an HCDR1, an HCDR2, and an HCDR3 of SEQ ID NOs: 1, 6, and 12, respectively; SEQ ID NOs: 2, 7, and 13, respectively; SEQ ID NOs: 1, 8, and 14, respectively; SEQ ID NOs: 3, 9, and 13, respectively; SEQ ID NOs: 4, 10, and 15, respectively; SEQ ID NOs: 5, 11, and 16, respectively; or SEQ ID NOs: 5, 11, and 17, respectively.

In some embodiments, the Fd comprises

a) an HCDR1, an HCDR2 and an HCDR3 of a heavy chain variable region (VH) of SEQ ID NO: 52;
b) an HCDR1, an HCDR2 and an HCDR3 of a VH of SEQ ID NO: 53;
c) an HCDR1, an HCDR2 and an HCDR3 of a VH of SEQ ID NO: 54;
d) an HCDR1, an HCDR2 and an HCDR3 of a VH of SEQ ID NO: 55;
e) an HCDR1, an HCDR2 and an HCDR3 of a VH of SEQ ID NO: 56;
f) an HCDR1, an HCDR2 and an HCDR3 of a VH of SEQ ID NO: 57;
g) an HCDR1, an HCDR2 and an HCDR3 of a VH of SEQ ID NO: 58;
h) an HCDR1, an HCDR2 and an HCDR3 of a VH of SEQ ID NO: 59;
i) an HCDR1, an HCDR2 and an HCDR3 of a VH of SEQ ID NO: 60;
j) an HCDR1, an HCDR2 and an HCDR3 of a VH of SEQ ID NO: 61;
k) an HCDR1, an HCDR2 and an HCDR3 of a VH of SEQ ID NO: 262;
l) an HCDR1, an HCDR2 and an HCDR3 of a VH of SEQ ID NO: 263; or
m) an HCDR1, an HCDR2 and an HCDR3 of a VH of SEQ ID NO: 264.

In certain embodiments, the Fd comprises an HCDR1, an HCDR2 and an HCDR3 of a VH of SEQ ID NO: 53.
In other embodiments, the Fd comprises an HCDR1, an HCDR2 and an HCDR3 of a VH of SEQ ID NO: 56.
In other embodiments, the Fd comprises an HCDR1, an HCDR2 and an HCDR3 of a VH of SEQ ID NO: 59.
In other embodiments, the Fd comprises an HCDR1, an HCDR2 and an HCDR3 of a VH of SEQ ID NO: 262.
In other embodiments, the Fd comprises an HCDR1, an HCDR2 and an HCDR3 of a VH of SEQ ID NO: 263.
In other embodiments, the Fd comprises an HCDR1, an HCDR2 and an HCDR3 of a VH of SEQ ID NO: 264.
In some embodiments, the Fd comprises the VH of SEQ ID NOs: 52, 53, 54, 55, 56, 57, 58, 59, 60, 61, 262, 263 or 264.
In some embodiments, the Fd comprises an HCDR1, an HCDR2, and an HCDR3 of

a) SEQ ID NOs: 28, 36, and 45, respectively;
b) SEQ ID NOs: 29, 37, and 46, respectively;
c) SEQ ID NOs: 30, 38, and 47, respectively;
d) SEQ ID NOs: 31, 39, and 48, respectively;
e) SEQ ID NOs: 32, 40, and 49, respectively;
f) SEQ ID NOs: 33, 41, and 50, respectively;
g) SEQ ID NOs: 34, 42, and 51, respectively;
h) SEQ ID NOs: 34, 43, and 51, respectively;
i) SEQ ID NOs: 34, 44, and 51, respectively;
j) SEQ ID NOs: 35, 42, and 51, respectively;
k) SEQ ID NOs: 253, 256 and 259, respectively
l) SEQ ID NOs: 254, 257 and 260, respectively
m) SEQ ID NOs: 255, 258 and 261 respectively.

In some embodiments, the Fd comprises an HCDR1, an HCDR2 and an HCDR3 of a heavy chain variable region (VH) of SEQ ID NO: 52; an HCDR1, an HCDR2 and an HCDR3 of a VH of SEQ ID NO: 53; an HCDR1, an HCDR2 and an HCDR3 of a VH of SEQ ID NO: 54; an HCDR1, an HCDR2 and an HCDR3 of a VH of SEQ ID NO: 55; an HCDR1, an HCDR2 and an HCDR3 of a VH of SEQ ID NO: 56; an HCDR1, an HCDR2 and an HCDR3 of a VH of SEQ ID NO: 57; an HCDR1, an HCDR2 and an HCDR3 of a VH of SEQ ID NO: 58; an HCDR1, an HCDR2 and an HCDR3 of a VH of SEQ ID NO: 59; an HCDR1, an HCDR2 and an HCDR3 of a VH of SEQ ID NO: 60; an HCDR1, an HCDR2 and an HCDR3 of a VH of SEQ ID NO: 61; an HCDR1, an HCDR2 and an HCDR3 of a VH of SEQ ID NO: 262 and an LCDR1, an LCDR2 and an LCDR3 of a VL of SEQ ID NO: 271; an HCDR1, an HCDR2 and an HCDR3 of a VH of SEQ ID NO: 263 and an LCDR1, an LCDR2 and an LCDR3 of a VL of SEQ ID NO: 272; or an HCDR1, an HCDR2 and an HCDR3 of a VH of SEQ ID NO: 264 and an LCDR1, an LCDR2 and an LCDR3 of a VL of SEQ ID NO: 273.
In certain embodiment, the Fd comprises an HCDR1, an HCDR2 and an HCDR3 of a VH of SEQ ID NO: 53 and an LCDR1, an LCDR2 and an LCDR3 of a VL of SEQ ID NO: 82.
In other embodiments, the Fd comprises an HCDR1, an HCDR2 and an HCDR3 of a VH of SEQ ID NO: 56 and an LCDR1, an LCDR2 and an LCDR3 of a VL of SEQ ID NO: 85.
In other embodiments, the Fd comprises an HCDR1, an HCDR2 and an HCDR3 of a VH of SEQ ID NO: 59 and an LCDR1, an LCDR2 and an LCDR3 of a VL of SEQ ID NO: 87.
In other embodiments, the Fd comprises an HCDR1, an HCDR2 and an HCDR3 of a VH of SEQ ID NO: 262 and an LCDR1, an LCDR2 and an LCDR3 of a VL of SEQ ID NO: 271.
In other embodiments the Fd comprises an HCDR1, an HCDR2 and an HCDR3 of a VH of SEQ ID NO: 263 and an LCDR1, an LCDR2 and an LCDR3 of a VL of SEQ ID NO: 272.
In other embodiments, the Fd comprises an HCDR1, an HCDR2 and an HCDR3 of a VH of SEQ ID NO: 264 and an LCDR1, an LCDR2 and an LCDR3 of a VL of SEQ ID NO: 273.
In some embodiments, the Fd comprises:

a) the VH of SEQ ID NO: 52;
b) the VH of SEQ ID NO: 53;
c) the VH of SEQ ID NO: 54;
d) the VH of SEQ ID NO: 55;
e) the VH of SEQ ID NO: 56;
f) the VH of SEQ ID NO: 57;
g) the VH of SEQ ID NO: 58;
h) the VH of SEQ ID NO: 59;
i) the VH of SEQ ID NO: 60;
j) the VH of SEQ ID NO: 61;

k) the VH of SEQ ID NO: 262;
l) the VH of SEQ ID NO: 263; or
m) the VH of SEQ ID NO: 264.

In some embodiments, the Fd comprises an HCDR1, an HCDR2 and an HCDR3 of a heavy chain variable region (VH) of SEQ ID NO: 95; an HCDR1, an HCDR2 and an HCDR3 of a VH of SEQ ID NO: 96; an HCDR1, an HCDR2 and an HCDR3 of a VH of SEQ ID NO: 97; an HCDR1, an HCDR2 and an HCDR3 of a VH of SEQ ID NO: 170; an HCDR1, an HCDR2 and an HCDR3 of a VH of SEQ ID NO: 171; an HCDR1, an HCDR2 and an HCDR3 of a VH of SEQ ID NO: 175; an HCDR1, an HCDR2 and an HCDR3 of a VH of SEQ ID NO: 179; an HCDR1, an HCDR2 and an HCDR3 of a VH of SEQ ID NO: 181; or an HCDR1, an HCDR2 and an HCDR3 of a VH of SEQ ID NO: 96.

In some embodiments, the Fd comprises an HCDR1, an HCDR2, and an HCDR3 of SEQ ID NOs: 89, 90, and 92, respectively; an HCDR1, an HCDR2, and the HCDR3 of SEQ ID NOs: 89, 90, and 93, respectively; an HCDR1, an HCDR2, and an HCDR3 of SEQ ID NOs: 33, 91, and 94, respectively; an HCDR1, an HCDR2, and an HCDR3 of SEQ ID NOs: 167, 168, and 169, respectively; an HCDR1, an HCDR2, and an HCDR3 of SEQ ID NOs: 172, 173, and 174, respectively; an HCDR1, an HCDR2, and an HCDR3 of SEQ ID NOs: 176, 177, and 178, respectively; an HCDR1, an HCDR2, and an HCDR3 of SEQ ID NOs: 89, 90, and 180, respectively; or an HCDR1, an HCDR2, and an HCDR3 of SEQ ID NOs: 89, 90, and 93, respectively.

**Homologous antigen binding domains and antigen binding domains with conservative substitutions**

Variants of the antigen binding domains that bind CD33 are within the scope of the disclosure. For example, variants may comprise 1, 2, 3, 4, 5, 6, 7, 8, 9, 10, 11, 12, 13, 14, 15, 16, 17, 18, 19, 20, 21, 22, 23, 24, 25, 26, 27, 28 or 29 amino acid substitutions in the antigen binding domain that bind CD33 as long as they retain or have improved functional properties when compared to the parent antigen binding domains. For instance, in some embodiments, the variant antigen binding domain that binds CD33 has improved thermostability when compared to the parent antigen binding domain in the same assay. In some embodiments, the variant antigen binding domain that binds CD33 has reduced likelihood of post-translational modification when compared to the parent binding domain. A reduced likelihood of post-translation modification may arise due to the substitution of a motif such as NG, DG or DP. In some embodiments, the sequence identity may be about 80%, 81%, 82%, 83%, 84%, 85%, 86%, 87%, 88%, 89%, 90%, 91%, 92%, 93%, 94%, 95%, 96%, 97%, 98% or 99% to the antigen binding domains that bind CD33 of the disclosure. In some embodiments, the variation is in the framework regions. In some embodiments, variants are generated by conservative substitutions.

For example, the antigen binding domains that bind CD33 may comprise substitutions at residue positions P41, I49, M70, and A88 in the VH (residue numbering according to the hu11B6_VH of SEQ ID NO: 5) and S80, L82, A88 and Y91 in the VL (residue numbering according to the hu11B6 VL of SEQ ID NO: 2). Conservative substitutions may be made at any indicated positions and the resulting variant antigen binding domains that bind CD33 are tested for their desired characteristics in the assays described herein.

Also provided are antigen binding domains that bind CD33 comprising the VH which are at least 80% identical to:

the VH of SEQ ID NO: 18;
the VH of SEQ ID NO: 19;
the VH of SEQ ID NO: 20;
the VH of SEQ ID NO: 21;
the VH of SEQ ID NO: 22;
the VH of SEQ ID NO: 23;
the VH of SEQ ID NO: 24;
the VH of SEQ ID NO: 25;
the VH of SEQ ID NO: 26;
the VH of SEQ ID NO: 27;
the VH of SEQ ID NO: 52;
the VH of SEQ ID NO: 53;
the VH of SEQ ID NO: 54;
the VH of SEQ ID NO: 55;
the VH of SEQ ID NO: 56;
the VH of SEQ ID NO: 57;
the VH of SEQ ID NO: 58;
the VH of SEQ ID NO: 59;
the VH of SEQ ID NO: 60;
the VH of SEQ ID NO: 61;

the VH of SEQ ID NO: 95;
the VH of SEQ ID NO: 96;
the VH of SEQ ID NO: 97;
the VH of SEQ ID NO: 170;
the VH of SEQ ID NO: 171;
the VH of SEQ ID NO: 175;
the VH of SEQ ID NO: 179;
the VH of SEQ ID NO: 181;
the VH of SEQ ID NO: 96
the VH of SEQ ID NO: 262;
the VH of SEQ ID NO: 263; or
the VH of SEQ ID NO: 264.

In certain embodiments, the antigen binding domain that binds to CD33 comprises a VH that is at least 80%, at least 85%, at least 90%, at least 91%, at least 92%, at least 93%, at least 94%, at least 95%, at least 96%, at least 97%, at least 98% or at least 99% identical to the VH of SEQ ID NO: 53.

In other embodiments, the antigen binding domain that binds to CD33 comprises a VH that is least 80%, at least 85%, at least 90%, at least 91%, at least 92%, at least 93%, at least 94%, at least 95%, at least 96%, at least 97%, at least 98% or at least 99% identical to the VH of SEQ ID NO: 56.

In other embodiments, the antigen binding domain that binds to CD33 comprises a VH that is least 80%, at least 85%, at least 90%, at least 91%, at least 92%, at least 93%, at least 94%, at least 95%, at least 96%, at least 97%, at least 98% or at least 99% identical to the VH of SEQ ID NO: 59.

In other embodiments, the antigen binding domain that binds to CD33 comprises a VH that is least 80%, at least 85%, at least 90%, at least 91%, at least 92%, at least 93%, at least 94%, at least 95%, at least 96%, at least 97%, at least 98% or at least 99% identical to the VH of SEQ ID NO: 262.

In other embodiments, the antigen binding domain that binds to CD33 comprises a VH that is least 80%, at least 85%, at least 90%, at least 91%, at least 92%, at least 93%, at least 94%, at least 95%, at least 96%, at least 97%, at least 98% or at least 99% identical to the VH of SEQ ID NO: 263.

In other embodiments, the antigen binding domain that binds to CD33 comprises a VH that is least 80%, at least 85%, at least 90%, at least 91%, at least 92%, at least 93%, at least 94%, at least 95%, at least 96%, at least 97%, at least 98% or at least 99% identical to the VH of SEQ ID NO: 264.

In some embodiments, the identity is 85%. In some embodiments, the identity is 90%. In some embodiments, the identity is 91%. In some embodiments, the identity is 91%. In some embodiments, the identity is 92%. In some embodiments, the identity is 93%. In some embodiments, the identity is 94%. In some embodiments, the identity is 94%. In some embodiments, the identity is 95%. In some embodiments, the identity is 96%. In some embodiments, the identity is 97%. In some embodiments, the identity is 98%. In some embodiments, the identity is 99%.

Also provided are antigen binding domains that bind CD33 comprising the VH and the VL which are at least 80% identical to

the VH of SEQ ID NO: 52 and the VL of SEQ ID NO: 81;
the VH of SEQ ID NO: 53 and the VL of SEQ ID NO: 82;
the VH of SEQ ID NO: 54 and the VL of SEQ ID NO: 83;
the VH of SEQ ID NO: 55 and the VL of SEQ ID NO: 84;
the VH of SEQ ID NO: 56 and the VL of SEQ ID NO: 85;
the VH of SEQ ID NO: 57 and the VL of SEQ ID NO: 86;
the VH of SEQ ID NO: 58 and the VL of SEQ ID NO: 87;
the VH of SEQ ID NO: 59 and the VL of SEQ ID NO: 87;
the VH of SEQ ID NO: 60 and the VL of SEQ ID NO: 87;
the VH of SEQ ID NO: 61 and the VL of SEQ ID NO: 88;
the VH of SEQ ID NO: 95 and the VL of SEQ ID NO: 105;
the VH of SEQ ID NO: 96 and the VL of SEQ ID NO: 106;
the VH of SEQ ID NO: 97 and the VL of SEQ ID NO: 107;
the VH of SEQ ID NO: 170 and the VL of SEQ ID NO: 185;
the VH of SEQ ID NO: 171 and the VL of SEQ ID NO: 188;
the VH of SEQ ID NO: 175 and the VL of SEQ ID NO: 192;
the VH of SEQ ID NO: 179 and the VL of SEQ ID NO: 196;
the VH of SEQ ID NO: 181 and the VL of SEQ ID NO: 200;
the VH of SEQ ID NO: 96 and the VL of SEQ ID NO: 202;

the VH of SEQ ID NO: 262 and the VL of SEQ ID NO: 271;
the VH of SEQ ID NO: 263 and the VL of SEQ ID NO: 272; or
the VH of SEQ ID NO: 264 and the VL of SEQ ID NO: 273.

In certain embodiments, the antigen binding domain that binds to CD33 comprises a VH and VL that are at least 80%, at least 85%, at least 90%, at least 91%, at least 92%, at least 93%, at least 94%, at least 95%, at least 96%, at least 97%, at least 98% or at least 99% identical to the VH of SEQ ID NO: 53 and the VL of SEQ ID NO: 82. For instance, the antigen binding domain that binds to CD33 comprises a VH of SEQ ID NO: 53 and a VL that is at least 80%, at least 85%, at least 90%, at least 91%, at least 92%, at least 93%, at least 94%, at least 95%, at least 96%, at least 97%, at least 98% or at least 99% identical to the VL of SEQ ID NO: 82. For instance, the antigen binding domain that binds to CD33 comprises a VL of SEQ ID NO: 82 and a VH that is at least 80%, at least 85%, at least 90%, at least 91%, at least 92%, at least 93%, at least 94%, at least 95%, at least 96%, at least 97%, at least 98% or at least 99% identical to the VH of SEQ ID NO: 53.

In other embodiments, the antigen binding domain that binds to CD33 comprises a VH that is at least 80%, at least 85%, at least 90%, at least 91%, at least 92%, at least 93%, at least 94%, at least 95%, at least 96%, at least 97%, at least 98% or at least 99% identical to the VH of SEQ ID NO: 56 and the VL of SEQ ID NO: 85. For instance, the antigen binding domain that binds to CD33 comprises a VH of SEQ ID NO: 56 and a VL that is at least 80%, at least 85%, at least 90%, at least 91%, at least 92%, at least 93%, at least 94%, at least 95%, at least 96%, at least 97%, at least 98% or at least 99% identical to the VL of SEQ ID NO: 85. For instance, the antigen binding domain that binds to CD33 comprises a VL of SEQ ID NO: 85 and a VH that is at least 80%, at least 85%, at least 90%, at least 91%, at least 92%, at least 93%, at least 94%, at least 95%, at least 96%, at least 97%, at least 98% or at least 99% identical to the VH of SEQ ID NO: 56.

In other embodiments, the antigen binding domain that binds to CD33 comprises a VH that is at least 80%, at least 85%, at least 90%, at least 91%, at least 92%, at least 93%, at least 94%, at least 95%, at least 96%, at least 97%, at least 98% or at least 99% identical to the VH of SEQ ID NO: 59 and the VL of SEQ ID NO: 87. For instance, the antigen binding domain that binds to CD33 comprises a VH of SEQ ID NO: 59 and a VL that is at least 80%, at least 85%, at least 90%, at least 91%, at least 92%, at least 93%, at least 94%, at least 95%, at least 96%, at least 97%, at least 98% or at least 99% identical to the VL of SEQ ID NO: 87. For instance, the antigen binding domain that binds to CD33 comprises a VL of SEQ ID NO: 87 and a VH that is at least 80%, at least 85%, at least 90%, at least 91%, at least 92%, at least 93%, at least 94%, at least 95%, at least 96%, at least 97%, at least 98% or at least 99% identical to the VH of SEQ ID NO: 59.

In other embodiments, the antigen binding domain that binds to CD33 comprises a VH that is at least 80%, at least 85%, at least 90%, at least 91%, at least 92%, at least 93%, at least 94%, at least 95%, at least 96%, at least 97%, at least 98% or at least 99% identical to the VH of SEQ ID NO: 262 and the VL of SEQ ID NO: 271. For instance, the antigen binding domain that binds to CD33 comprises a VH of SEQ ID NO: 262 and a VL that is at least 80%, at least 85%, at least 90%, at least 91%, at least 92%, at least 93%, at least 94%, at least 95%, at least 96%, at least 97%, at least 98% or at least 99% identical to the VL of SEQ ID NO: 271. For instance, the antigen binding domain that binds to CD33 comprises a VL of SEQ ID NO: 271 and a VH that is at least 80%, at least 85%, at least 90%, at least 91%, at least 92%, at least 93%, at least 94%, at least 95%, at least 96%, at least 97%, at least 98% or at least 99% identical to the VH of SEQ ID NO: 262.

In other embodiments, the antigen binding domain that binds to CD33 comprises a VH that is at least 80%, at least 85%, at least 90%, at least 91%, at least 92%, at least 93%, at least 94%, at least 95%, at least 96%, at least 97%, at least 98% or at least 99% identical to the VH of SEQ ID NO: 263 and the VL of SEQ ID NO: 272. For instance, the antigen binding domain that binds to CD33 comprises a VH of SEQ ID NO: 263 and a VL that is at least 80%, at least 85%, at least 90%, at least 91%, at least 92%, at least 93%, at least 94%, at least 95%, at least 96%, at least 97%, at least 98% or at least 99% identical to the VL of SEQ ID NO: 272. For instance, the antigen binding domain that binds to CD33 comprises a VL of SEQ ID NO: 272 and a VH that is at least 80%, at least 85%, at least 90%, at least 91%, at least 92%, at least 93%, at least 94%, at least 95%, at least 96%, at least 97%, at least 98% or at least 99% identical to the VH of SEQ ID NO: 263.

In other embodiments, the antigen binding domain that binds to CD33 comprises a VH that is at least 80%, at least 85%, at least 90%, at least 91%, at least 92%, at least 93%, at least 94%, at least 95%, at least 96%, at least 97%, at least 98% or at least 99% identical to the VH of SEQ ID NO: 264 and the VL of SEQ ID NO: 273. For instance, the antigen binding domain that binds to CD33 comprises a VH of SEQ ID NO: 264 and a VL that is at least 80%, at least 85%, at least 90%, at least 91%, at least 92%, at least 93%, at least 94%, at least 95%, at least 96%, at least 97%, at least 98% or at least 99% identical to the VL of SEQ ID NO: 273. For instance, the antigen binding domain that binds to CD33 comprises a VL of SEQ ID NO: 273 and a VH that is at least 80%, at least 85%, at least 90%, at least 91%, at least 92%, at least 93%, at least 94%, at least 95%, at least 96%, at least 97%, at least 98% or at least 99% identical to the VH of SEQ ID NO: 264.

The inventors have found that positions in the VH and/or VL domains may be mutated to remove motifs that are liable to post-translational modification. Post-translational modification may increase the likelihood of, for example, deamidation, isomerization and/or fragmentation of the antigen binding domain that binds to CD33. These mutations may also enhance thermostability of an antigen binding domain that binds to CD33.

Examples of sequence motifs that are liable to post-translation modification, for example, NG, DG, and DP.

Accordingly, in some embodiments, the antigen binding domain that binds to CD33 comprises a VH and/or VL domain comprising one or two amino acid substitutions in one or more NG motifs. In some embodiments, the N residue of the NG motif is substituted. In other embodiments, the G residue of the NG motif is substituted. In some embodiments the N and G residues of the NG motif are substituted. In certain such embodiments, the one or two amino acid substitutions in the one or more NG motifs are the only substitutions within the VH and/or VL domain.

In some embodiments, the antigen binding domain that binds to CD33 comprises a VH and/or VL domain comprising one or two amino acid substitutions in one or more DG motifs. In some embodiments, the D residue of the DG motif is substituted. In other embodiments, the G residue of the DG motif is substituted. In some embodiments the D and G residues of the DG motif are substituted. In certain such embodiments, the one or two amino acid substitutions in the one or more DG motifs are the only substitutions within the VH and/or VL domain.

In some embodiments, the antigen binding domain that binds to CD33 comprises a VH and/or VL domain comprising one or two amino acid substitutions in one or more DP motifs. In some embodiments, the D residue of the DP motif is substituted. In other embodiments, the P residue of the DP motif is substituted. In some embodiments the D and P residues of the DP motif are substituted. In certain such embodiments, the one or two amino acid substitutions in the one or more DP motifs are the only substitutions within the VH and/or VL domain.

An exemplary antigen binding domain that binds to CD33 of the invention is a protein (for example, an scFv) comprising the VH of SEQ ID NO: 262 and VL of SEQ ID NO: 271. The inventors have found that mutating the DG motif within the VH of SEQ ID NO: 262 can reduce the likelihood of post-translation modification whilst maintaining CD33 binding properties. These mutations may also enhance thermostability.

An antigen binding domain that binds to CD33 having the VH of SEQ ID NO: 262 and VL of SEQ ID NO: 271 may be mutated to remove a DG motif. The VH of SEQ ID NO: 262 has a DG motif at positions 54-55 (using residue numbering according to EU index).

Thus, in some embodiments, an antigen binding domain that binds to CD33 (for example, an scFv) comprises a VH of SEQ ID NO: 262 in which the D residue at position 54 is substituted (for example, D54G, D54A, D54Y, D54P, D54N, D54S, D54R, D54L, D54V, D54T, D54F or D54W). In other embodiments, an antigen binding domain that binds to CD33 (for example, an scFv) comprises a VH of SEQ ID NO: 262 in which the G residue at position 55 is substituted (for example, G55V, G55P, G55R, G55A, G55L, G55Q, G55T or G55H). In other embodiments, the antigen binding domain that binds to CD33 (for example, an scFv) comprises a VH of SEQ ID NO: 262 in which the D residue at position 54 (for example, D54G, D54A, D54Y, D54P, D54N, D54S, D54R, D54L, D54V, D54T, D54F or D54W) is substituted and the G residue at position 55 is substituted (for example, G55V, G55P, G55R, G55A, G55L, G55Q, G55T or G55H). In some embodiments, the antigen binding domain that binds to CD33 (for example, an scFv) comprises a VH of SEQ ID NO: 291, 292, 293, 294, 295, 296, 297, 298, 299, 300, 301, 302, 303, 304, 305, 306, 307, 308, 309 or 310.

In certain embodiments, the antigen binding domain that binds to CD33 (for example, an scFv) comprises:the VH of SEQ ID NO: 291 and the VL of SEQ ID NO: 271;

the VH of SEQ ID NO: 292 and the VL of SEQ ID NO: 271;
the VH of SEQ ID NO: 293 and the VL of SEQ ID NO: 271;
the VH of SEQ ID NO: 294 and the VL of SEQ ID NO: 271;
the VH of SEQ ID NO: 295 and the VL of SEQ ID NO: 271;
the VH of SEQ ID NO: 296 and the VL of SEQ ID NO: 271;
the VH of SEQ ID NO: 297 and the VL of SEQ ID NO: 271;
the VH of SEQ ID NO: 298 and the VL of SEQ ID NO: 271;
the VH of SEQ ID NO: 299 and the VL of SEQ ID NO: 271;
the VH of SEQ ID NO: 300 and the VL of SEQ ID NO: 271;
the VH of SEQ ID NO: 301 and the VL of SEQ ID NO: 271;
the VH of SEQ ID NO: 302 and the VL of SEQ ID NO: 271;
the VH of SEQ ID NO: 303 and the VL of SEQ ID NO: 271;
the VH of SEQ ID NO: 304 and the VL of SEQ ID NO: 271;
the VH of SEQ ID NO: 305 and the VL of SEQ ID NO: 271;
the VH of SEQ ID NO: 306 and the VL of SEQ ID NO: 271;
the VH of SEQ ID NO: 307 and the VL of SEQ ID NO: 271;
the VH of SEQ ID NO: 308 and the VL of SEQ ID NO: 271;
the VH of SEQ ID NO: 309 and the VL of SEQ ID NO: 271; or

the VH of SEQ ID NO: 310 and the VL of SEQ ID NO: 271.

Another exemplary antigen binding domain that binds to CD33 of the invention is a protein (for example, an scFv) comprising the VH of SEQ ID NO: 263 and VL of SEQ ID NO: 272. The inventors have found that mutating one or more NG motifs within the VH of SEQ ID NO: 263 and VL of SEQ ID NO: 272 can reduce the likelihood of post-translation modification whilst maintaining CD33 binding properties. These mutations may also enhance thermostability.
An antigen binding domain that binds to CD33 having the VH of SEQ ID NO: 263 and VL of SEQ ID NO: 272 may be mutated to remove an NG motif. The VH of SEQ ID NO: 263 has a NG motif at positions 99-100 (using residue numbering according to EU index) and the VL of SEQ ID NO: 272 has a NG motif at positions 97-98 (using residue numbering according to EU index).
Thus, in some embodiments, the antigen binding domain that binds to CD33 (for example, an scFv) comprises a VH of SEQ ID NO: 263 in which the N residue at position 99 is substituted (for example, N99V, N99S, N99E, N99H, N99A, N99G, N99K, N99T, N99F, N99V, N99R, N99L, N99H or N99I). In other embodiments, an antigen binding domain that binds to CD33 (for example, an scFv) comprises a VH of SEQ ID NO: 263 in which the G residue at position 100 is substituted (for example, G100S, G100A). In other embodiments, the antigen binding domain that binds to CD33 (for example, an scFv) comprises a VH of SEQ ID NO: 263 in which the N residue at position 99 is substituted (for example, N99V, N99S, N99E, N99H, N99A, N99G, N99K, N99T, N99F, N99V, N99R, N99L, N99H or N99I) and the G residue at position 100 is substituted (for example, G100S, G100A). In some embodiments, the antigen binding domain that binds to CD33 comprises a VH of SEQ ID NO: 337, 338, 339, 340, 341, 342, 343, 344, 345, 346, 347, 348, 349, 350, 351, or 352.
In some embodiments, an antigen binding domain that binds to CD33 (for example, an scFv) comprises a VL of SEQ ID NO: 272 in which the N residue at position 97 is substituted (for example, N97V, N97R, N97P, N97T, N97G, N97Q, N97S, N97A, N97L, N97Y, N97E, N97F, N97D, N97I or N97H). In other embodiments, an antigen binding domain that binds to CD33 (for example, an scFv) comprises a VL of SEQ ID NO: 272 in which the G residue at position 98 is substituted (for example, G98V, G98P, G98R, G98D, G98E, G98W, G98Y, G98A, G98N, G98K, G98L). In other embodiments, the antigen binding domain that binds to CD33 (for example, an scFv) comprises a VL of SEQ ID NO: 272 in which the N residue at position 97 is substituted and the G residue at position 98 is substituted. In some embodiments, the antigen binding domain that binds to CD33 (for example, an scFv) comprises a VL of SEQ ID NO: 311, 312 313, 314, 315, 316, 317, 318, 319, 320, 321, 322, 323, 324, 325, 326, 327, 328, 329, 330, 331, 332, 333, 334, 335 or 336.
In certain embodiments, the antigen binding domain that binds to CD33 (for example, an scFv) comprises:

the VH of SEQ ID NO: 263 and the VL of SEQ ID NO: 311;
the VH of SEQ ID NO: 263 and the VL of SEQ ID NO: 312.
the VH of SEQ ID NO: 263 and the VL of SEQ ID NO: 313;
the VH of SEQ ID NO: 263 and the VL of SEQ ID NO: 314;
the VH of SEQ ID NO: 263 and the VL of SEQ ID NO: 315;
the VH of SEQ ID NO: 263 and the VL of SEQ ID NO: 316;
the VH of SEQ ID NO: 263 and the VL of SEQ ID NO: 317;
the VH of SEQ ID NO: 263 and the VL of SEQ ID NO: 318;
the VH of SEQ ID NO: 263 and the VL of SEQ ID NO: 319;
the VH of SEQ ID NO: 263 and the VL of SEQ ID NO: 320;
the VH of SEQ ID NO: 263 and the VL of SEQ ID NO: 321;
the VH of SEQ ID NO: 263 and the VL of SEQ ID NO: 322;
the VH of SEQ ID NO: 263 and the VL of SEQ ID NO: 323;
the VH of SEQ ID NO: 263 and the VL of SEQ ID NO: 324;
the VH of SEQ ID NO: 263 and the VL of SEQ ID NO: 325;
the VH of SEQ ID NO: 263 and the VL of SEQ ID NO: 326;
the VH of SEQ ID NO: 263 and the VL of SEQ ID NO: 327;
the VH of SEQ ID NO: 263 and the VL of SEQ ID NO: 328;
the VH of SEQ ID NO: 263 and the VL of SEQ ID NO: 329;
the VH of SEQ ID NO: 263 and the VL of SEQ ID NO: 330;
the VH of SEQ ID NO: 263 and the VL of SEQ ID NO: 331;
the VH of SEQ ID NO: 263 and the VL of SEQ ID NO: 332;
the VH of SEQ ID NO: 263 and the VL of SEQ ID NO: 333;
the VH of SEQ ID NO: 263 and the VL of SEQ ID NO: 334;
the VH of SEQ ID NO: 263 and the VL of SEQ ID NO: 335;
the VH of SEQ ID NO: 263 and the VL of SEQ ID NO: 336;
the VH of SEQ ID NO: 337 and the VL of SEQ ID NO: 272;
the VH of SEQ ID NO: 338 and the VL of SEQ ID NO: 272;

the VH of SEQ ID NO: 339 and the VL of SEQ ID NO: 272;
the VH of SEQ ID NO: 340 and the VL of SEQ ID NO: 272;
the VH of SEQ ID NO: 341 and the VL of SEQ ID NO: 272;
the VH of SEQ ID NO: 342 and the VL of SEQ ID NO: 272;
the VH of SEQ ID NO: 343 and the VL of SEQ ID NO: 272;
the VH of SEQ ID NO: 344 and the VL of SEQ ID NO: 272;
the VH of SEQ ID NO: 345 and the VL of SEQ ID NO: 272;
the VH of SEQ ID NO: 346 and the VL of SEQ ID NO: 272;
the VH of SEQ ID NO: 347 and the VL of SEQ ID NO: 272;
the VH of SEQ ID NO: 348 and the VL of SEQ ID NO: 272;
the VH of SEQ ID NO: 349 and the VL of SEQ ID NO: 272;
the VH of SEQ ID NO: 350 and the VL of SEQ ID NO: 272;
the VH of SEQ ID NO: 351 and the VL of SEQ ID NO: 272; or
the VH of SEQ ID NO: 352 and the VL of SEQ ID NO: 272.

Another exemplary antigen binding domain that binds to CD33 of the invention is a protein (for example, an scFv) comprising the VH of SEQ ID NO: 56 and VL of SEQ ID NO: 85. The inventors have found that mutating one or more NG motifs within the VH of SEQ ID NO: 56 and VL of SEQ ID NO: 85 can reduce the likelihood of post-translation modification whilst maintaining CD33 binding properties. These mutations may also enhance thermostability.

An antigen binding domain that binds to CD33 having the VH of SEQ ID NO: 56 and VL of SEQ ID NO: 85 may be mutated to remove an NG motif. The VH of SEQ ID NO: 56 has a NG motif at positions 99-100 (using residue numbering according to EU index) and the VL of SEQ ID NO: 85 has a NG motif at positions 97-98 (using residue numbering according to EU index).

Thus, in some embodiments, the antigen binding domain that binds to CD33 (for example, an scFv) comprises a VH of SEQ ID NO: 56 in which the N residue at position 99 is substituted (for example, N99R, N99P, N99G, N99V, N99L, N99D, N99E, N99A, N99Q or N99T). In other embodiments, an antigen binding domain that binds to CD33 (for example, an scFv) comprises a VH of SEQ ID NO: 56 in which the G residue at position 100 is substituted (for example, G100S, G100A, G100L, G100P, G100T or G100Q). In other embodiments, the antigen binding domain that binds to CD33 (for example, an scFv) comprises a VH of SEQ ID NO: 56 in which the N residue at position 99 is substituted (for example, N99R, N99P, N99G, N99V, N99L, N99D, N99E, N99A, N99Q or N99T) and the G residue at position 100 is substituted (for example, G100S, G100A, G100L, G100P, G100T or G100Q). In some embodiments, the antigen binding domain that binds to CD33 (for example, an scFv) comprises a VH of SEQ ID NO: 367, 368, 369, 370, 371, 372, 373, 374, 375, 376, 377, 378, 379, 380, 381 or 382.

In some embodiments, an antigen binding domain that binds to CD33 (for example, an scFv) comprises a VL of SEQ ID NO: 85 in which the N residue at position 97 is substituted (for example, N97G, N97D, N97P, N97K, N97E, N97A, N97R, N97Q, N97I, N97V, N97H, N97K, N97W or N97S). In other embodiments, an antigen binding domain that binds to CD33 (for example, an scFv) comprises a VL of SEQ ID NO: 85 in which the G residue at position 98 is substituted. In other embodiments, the antigen binding domain that binds to CD33 (for example, an scFv) comprises a VL of SEQ ID NO: 85 in which the N residue at position 97 is substituted (for example, N97G, N97D, N97P, N97K, N97E, N97A, N97R, N97Q, N971, N97V, N97H, N97K, N97W or N97S) and the G residue at position 98 is substituted. In some embodiments, the antigen binding domain that binds to CD33 (for example, an scFv) comprises a VL of SEQ ID NO: 353, 354, 355, 356, 357, 358, 359, 360, 361, 362, 363, 364, 365, or 366.

In certain embodiments, the antigen binding domain that binds to CD33 (for example, an scFv) comprises:

the VH of SEQ ID NO: 56 and the VL of SEQ ID NO: 353;
the VH of SEQ ID NO: 56 and the VL of SEQ ID NO: 354;
the VH of SEQ ID NO: 56 and the VL of SEQ ID NO: 355;
the VH of SEQ ID NO: 56 and the VL of SEQ ID NO: 356;
the VH of SEQ ID NO: 56 and the VL of SEQ ID NO: 357;
the VH of SEQ ID NO: 56 and the VL of SEQ ID NO: 358;
the VH of SEQ ID NO: 56 and the VL of SEQ ID NO: 359;
the VH of SEQ ID NO: 56 and the VL of SEQ ID NO: 360;
the VH of SEQ ID NO: 56 and the VL of SEQ ID NO: 361;
the VH of SEQ ID NO: 56 and the VL of SEQ ID NO: 362;
the VH of SEQ ID NO: 56 and the VL of SEQ ID NO: 363;
the VH of SEQ ID NO: 56 and the VL of SEQ ID NO: 364;
the VH of SEQ ID NO: 56 and the VL of SEQ ID NO: 365;
the VH of SEQ ID NO: 56 and the VL of SEQ ID NO: 366;

the VH of SEQ ID NO: 367 and the VL of SEQ ID NO: 85;
the VH of SEQ ID NO: 368 and the VL of SEQ ID NO: 85;
the VH of SEQ ID NO: 369 and the VL of SEQ ID NO: 85;
the VH of SEQ ID NO: 370 and the VL of SEQ ID NO: 85;
the VH of SEQ ID NO: 371 and the VL of SEQ ID NO: 85;
the VH of SEQ ID NO: 372 and the VL of SEQ ID NO: 85;
the VH of SEQ ID NO: 373 and the VL of SEQ ID NO: 85;
the VH of SEQ ID NO: 374 and the VL of SEQ ID NO: 85;
the VH of SEQ ID NO: 375 and the VL of SEQ ID NO: 85;
the VH of SEQ ID NO: 376 and the VL of SEQ ID NO: 85;
the VH of SEQ ID NO: 377 and the VL of SEQ ID NO: 85;
the VH of SEQ ID NO: 378 and the VL of SEQ ID NO: 85;
the VH of SEQ ID NO: 379 and the VL of SEQ ID NO: 85;
the VH of SEQ ID NO: 380 and the VL of SEQ ID NO: 85;
the VH of SEQ ID NO: 381 and the VL of SEQ ID NO: 85; or
the VH of SEQ ID NO: 382 and the VL of SEQ ID NO: 85.

Another exemplary antigen binding domain that binds to CD33 of the invention is a protein (for example, an scFv) comprising the VH of SEQ ID NO: 53 and VL of SEQ ID NO: 82. The inventors have found that mutating the NG motif within the VH of SEQ ID NO: 53 and VL of SEQ ID NO: 82 can reduce the likelihood of post-translation modification whilst maintaining CD33 binding properties. These mutations may also enhance thermostability.
An antigen binding domain that binds to CD33 having the VH of SEQ ID NO: 53 and VL of SEQ ID NO: 82 may be mutated to an NG motif. The VL of SEQ ID NO: 82 has a NG motif at positions 97-98 (using residue numbering according to EU index).
In some embodiments, an antigen binding domain that binds to CD33 (for example, an scFv) comprises a VL of SEQ ID NO: 82 in which the N residue at position 97 is substituted (for example, N97S, N97Q, N97V, N97G, N97W, N97T, N97I, N97E, N97A, N97R or N97L). In other embodiments, an antigen binding domain that binds to CD33 (for example, an scFv) comprises a VL of SEQ ID NO: 272 in which the G residue at position 98 is substituted (for example, G98V, G98A, G98P, G98L or G98T). In other embodiments, the antigen binding domain that binds to CD33 (for example, an scFv) comprises a VL of SEQ ID NO: 82 in which the N residue at position 97 is substituted (for example, N97S, N97Q, N97V, N97G, N97W, N97T, N97I, N97E, N97A, N97R or N97L) and the G residue at position 98 is substituted (for example, G98V, G98A, G98P, G98L or G98T). In some embodiments, the antigen binding domain that binds to CD33 (for example, an scFv) comprises a VL of SEQ ID NO: 383, 384, 385, 386, 387, 388, 389, 390, 391, 392, 393, 394, 395, 396, 397 or 398.
In certain embodiments, the antigen binding domain that binds to CD33 (for example, an scFv) comprises:

the VH of SEQ ID NO: 53 and the VL of SEQ ID NO: 383;
the VH of SEQ ID NO: 53 and the VL of SEQ ID NO: 384;
the VH of SEQ ID NO: 53 and the VL of SEQ ID NO: 385;
the VH of SEQ ID NO: 53 and the VL of SEQ ID NO: 386;
the VH of SEQ ID NO: 53 and the VL of SEQ ID NO: 387;
the VH of SEQ ID NO: 53 and the VL of SEQ ID NO: 388;
the VH of SEQ ID NO: 53 and the VL of SEQ ID NO: 389;
the VH of SEQ ID NO: 53 and the VL of SEQ ID NO: 390;
the VH of SEQ ID NO: 53 and the VL of SEQ ID NO: 391;
the VH of SEQ ID NO: 53 and the VL of SEQ ID NO: 392;
the VH of SEQ ID NO: 53 and the VL of SEQ ID NO: 393;
the VH of SEQ ID NO: 53 and the VL of SEQ ID NO: 394;
the VH of SEQ ID NO: 53 and the VL of SEQ ID NO: 395;
the VH of SEQ ID NO: 53 and the VL of SEQ ID NO: 396;
the VH of SEQ ID NO: 53 and the VL of SEQ ID NO: 397; or
the VH of SEQ ID NO: 53 and the VL of SEQ ID NO: 398.

In some embodiments, the antigen binding domain that binds to CD33 (for example, an scFv) comprises a VH domain of SEQ ID NO: 291, 292, 293, 294, 295, 296, 297, 298, 299, 300, 301, 302, 303, 304, 305, 306, 307, 308, 309, 310, 337, 338, 339, 340, 341, 342, 343, 344, 345, 346, 347, 348, 349, 350, 351, 352, 367, 368, 369, 370, 371, 372, 373, 374, 375, 376, 377, 378, 379, 380, 381, 382, or 382.
In some embodiments, the antigen binding domain that binds to CD33 (for example, an scFv) comprises a VL

domain of SEQ ID NO: 311, 312, 313, 314, 315, 316, 317, 318, 319, 320, 321, 322, 323, 324, 325, 326, 327, 328, 329, 330, 331, 332, 333, 334, 335, 336, 353, 354, 355, 356, 357, 358, 359, 360, 361, 362, 363, 364, 365, 366, 383, 384, 385, 386, 387, 388, 389, 390, 391, 392, 393, 394, 395, 396, 397 or 398.

In some embodiments, the antigen binding domain that binds to CD33 (for example, an scFv) comprises a VH domain of SEQ ID NO: 291, 292, 293, 294, 295, 296, 297, 298, 299, 300, 301, 302, 303, 304, 305, 306, 307, 308, 309, 310, 337, 338, 339, 340, 341, 342, 343, 344, 345, 346, 347, 348, 349, 350, 351, 352, 367, 368, 369, 370, 371, 372, 373, 374, 375, 376, 377, 378, 379, 380, 381, 382, or 382 and a VL domain of SEQ ID NO: 311, 312, 313, 314, 315, 316, 317, 318, 319, 320, 321, 322, 323, 324, 325, 326, 327, 328, 329, 330, 331, 332, 333, 334, 335, 336, 353, 354, 355, 356, 357, 358, 359, 360, 361, 362, 363, 364, 365, 366, 383, 384, 385, 386, 387, 388, 389, 390, 391, 392, 393, 394, 395, 396, 397 or 398.

In some embodiments, the identity is 85%. In some embodiments, the identity is 90%. In some embodiments, the identity is 91%. In some embodiments, the identity is 91%. In some embodiments, the identity is 92%. In some embodiments, the identity is 93%. In some embodiments, the identity is 94%. In some embodiments, the identity is 94%. In some embodiments, the identity is 95%. In some embodiments, the identity is 96%. In some embodiments, the identity is 97%. In some embodiments, the identity is 98%. In some embodiments, the identity is 99%.

The percent identity between the two sequences is a function of the number of identical positions shared by the sequences (i.e., % identity = number of identical positions/total number of positions ×100), taking into account the number of gaps, and the length of each gap, which need to be introduced for optimal alignment of the two sequences.

The percent identity between two amino acid sequences may be determined using the algorithm of E. Meyers and W. Miller (*Comput. Appl. Biosci.* 4:11-17 (1988)) which has been incorporated into the ALIGN program (version 2.0), using a PAM120 weight residue table, a gap length penalty of 12 and a gap penalty of 4. In addition, the percent identity between two amino acid sequences may be determined using the Needleman and Wunsch (*J. Mol. Biol.* 48:444-453 (1970)) algorithm which has been incorporated into the GAP program in the GCG software package (available at www_gcg_com), using either a Blossum 62 matrix or a PAM250 matrix, and a gap weight of 16, 14, 12, 10, 8, 6, or 4 and a length weight of 1, 2, 3, 4, 5, or 6.

In some embodiments, variant antigen binding domains that bind CD33 comprise one or two conservative substitutions in any of the CDR regions, while retaining desired functional properties of the parent antigen binding fragments that bind CD33.

"Conservative modifications" refer to amino acid modifications that do not significantly affect or alter the binding characteristics of the antibody containing the amino acid modifications. Conservative modifications include amino acid substitutions, additions and deletions. Conservative amino acid substitutions are those in which the amino acid is replaced with an amino acid residue having a similar side chain. The families of amino acid residues having similar side chains are well defined and include amino acids with acidic side chains (e.g., aspartic acid, glutamic acid), basic side chains (e.g., lysine, arginine, histidine), nonpolar side chains (e.g., alanine, valine, leucine, isoleucine, proline, phenylalanine, methionine), uncharged polar side chains (e.g., glycine, asparagine, glutamine, cysteine, serine, threonine, tyrosine, tryptophan), aromatic side chains (e.g., phenylalanine, tryptophan, histidine, tyrosine), aliphatic side chains (e.g., glycine, alanine, valine, leucine, isoleucine, serine, threonine), amide (e.g., asparagine, glutamine), beta-branched side chains (e.g., threonine, valine, isoleucine) and sulfur-containing side chains (cysteine, methionine). Furthermore, any native residue in the polypeptide may also be substituted with alanine, as has been previously described for alanine scanning mutagenesis (MacLennan et al., (1988) Acta Physiol Scand Suppl 643:55-67; Sasaki et al., (1988) Adv Biophys 35:1-24). Amino acid substitutions to the antibodies of the invention may be made by known methods for example by PCR mutagenesis (U.S. Patent No. 4,683,195). Alternatively, libraries of variants may be generated for example using random (NNK) or non-random codons, for example DVK codons, which encode 11 amino acids (Ala, Cys, Asp, Glu, Gly, Lys, Asn, Arg, Ser, Tyr, Trp). The resulting variants may be tested for their characteristics using assays described herein.

**Methods of generating antigen binding fragment that bind CD33**

Antigen binding domains that bind CD33 provided in the disclosure may be generated using various technologies. For example, the hybridoma method of Kohler and Milstein may be used to identify VH/VL pairs that bind CD33. In the hybridoma method, a mouse or other host animal, such as a hamster, rat or chicken is immunized with human and/or cyno CD33, followed by fusion of spleen cells from immunized animals with myeloma cells using standard methods to form hybridoma cells. Colonies arising from single immortalized hybridoma cells may be screened for production of the antibodies containing the antigen binding domains that bind CD33 with desired properties, such as specificity of binding, cross-reactivity or lack thereof, affinity for the antigen, and any desired functionality.

Antigen binding domains that bind CD33 generated by immunizing non-human animals may be humanized. Exemplary humanization techniques including selection of human acceptor frameworks include CDR grafting (U.S. Patent No. 5,225,539), SDR grafting (U.S. Patent No. 6,818,749), Resurfacing (Padlan, (1991) Mol Immunol 28:489-499), Specificity Determining Residues Resurfacing (U.S. Patent Publ. No. 2010/0261620), human framework adaptation

(U.S. Patent No. 8,748,356) or superhumanization (U.S. Patent No. 7,709,226). In these methods, CDRs or a subset of CDR residues of parental antibodies are transferred onto human frameworks that may be selected based on their overall homology to the parental frameworks, based on similarity in CDR length, or canonical structure identity, or a combination thereof.

Humanized antigen binding domains may be further optimized to improve their selectivity or affinity to a desired antigen by incorporating altered framework support residues to preserve binding affinity (backmutations) by techniques such as those described in Int. Patent Publ. Nos. WO1990/007861 and WO 1992/22653, or by introducing variation at any of the CDRs for example to improve affinity of the antigen binding domain.

Transgenic animals, such as mice, rat or chicken carrying human immunoglobulin (Ig) loci in their genome may be used to generate antigen binding fragments that bind CD33, and are described in for example U.S. Patent No. 6,150,584, Int. Patent Publ. No. WO1999/45962, Int. Patent Publ. Nos. WO2002/066630, WO2002/43478, WO2002/043478 and WO1990/04036. The endogenous immunoglobulin loci in such animal may be disrupted or deleted, and at least one complete or partial human immunoglobulin locus may be inserted into the genome of the animal using homologous or non-homologous recombination, using transchromosomes, or using minigenes. Companies such as Regeneron (www_regeneron_com), Harbour Antibodies (www_harbourantibodies_com), Open Monoclonal Technology, Inc. (OMT) (www_omtinc_net), KyMab (www_kymab_com), Trianni (www.trianni_com) and Ablexis (www_ablexis_com) may be engaged to provide human antibodies directed against a selected antigen using technologies as described above.

Antigen binding domains that bind CD33 may be selected from a phage display library, where the phage is engineered to express human immunoglobulins or portions thereof such as Fabs, single chain antibodies (scFv), or unpaired or paired antibody variable regions. The antigen binding domains that bind CD33 may be isolated for example from phage display library expressing antibody heavy and light chain variable regions as fusion proteins with bacteriophage pIX coat protein as described in Shi et al., (2010) J Mol Biol 397:385-96, and Int. Patent Publ. No. WO09/085462). The libraries may be screened for phage binding to human and/or cyno CD33 and the obtained positive clones may be further characterized, the Fabs isolated from the clone lysates, and converted to scFvs or other configurations of antigen binding fragments.

Preparation of immunogenic antigens and expression and production of antigen binding domains of the disclosure may be performed using any suitable technique, such as recombinant protein production. The immunogenic antigens may be administered to an animal in the form of purified protein, or protein mixtures including whole cells or cell or tissue extracts, or the antigen may be formed *de novo* in the animal's body from nucleic acids encoding said antigen or a portion thereof.

**Conjugation to half-life extending moieties**

The antigen binding domains that bind CD33 of the disclosure may be conjugated to a half-life extending moiety. Exemplary half-life extending moieties are albumin, albumin variants, albumin-binding proteins and/or domains, transferrin and fragments and analogues thereof, immunoglobulins (Ig) or fragments thereof, such as Fc regions. Amino acid sequences of the aforementioned half-life extending moieties are known. Ig or fragments thereof include all isotypes, *i.e.,* IgG1, IgG2, IgG3, IgG4, IgM, IgA and IgE.

Additional half-life extending moieties that may be conjugated to the antigen binding domains that bind CD33 of the disclosure include polyethylene glycol (PEG) molecules, such as PEG5000 or PEG20,000, fatty acids and fatty acid esters of different chain lengths, for example laurate, myristate, stearate, arachidate, behenate, oleate, arachidonate, octanedioic acid, tetradecanedioic acid, octadecanedioic acid, docosanedioic acid, and the like, polylysine, octane, carbohydrates (dextran, cellulose, oligo- or polysaccharides) for desired properties. These moieties may be direct fusions with the antigen binding domains that bind CD33 of the disclosure and may be generated by standard cloning and expression techniques. Alternatively, well known chemical coupling methods may be used to attach the moieties to recombinantly produced antigen binding domains that bind CD33 of the disclosure.

A pegyl moiety may for example be conjugated to the antigen binding domain that bind CD33 of the disclosure by incorporating a cysteine residue to the C-terminus of the antigen binding domain that bind CD33 of the disclosure, or engineering cysteines into residue positions that face away from the CD33 binding site and attaching a pegyl group to the cysteine using well known methods.

In some embodiments, the antigen binding fragment that binds CD33 is conjugated to a half-life extending moiety.

In some embodiments, the half-life extending moiety is an immunoglobulin (Ig), a fragment of the Ig, an Ig constant region, a fragment of the Ig constant region, a Fc region, transferrin, albumin, an albumin binding domain or polyethylene glycol. In some embodiments, the half-life extending moiety is an Ig constant region.

In some embodiments, the half-life extending moiety is the Ig.

In some embodiments, the half-life extending moiety is the fragment of the Ig.

In some embodiments, the half-life extending moiety is the Ig constant region.

In some embodiments, the half-life extending moiety is the fragment of the Ig constant region.

In some embodiments, the half-life extending moiety is the Fc region.

In some embodiments, the half-life extending moiety is albumin.

In some embodiments, the half-life extending moiety is the albumin binding domain.

In some embodiments, the half-life extending moiety is transferrin.

In some embodiments, the half-life extending moiety is polyethylene glycol.

The antigen binding domains that bind CD33 conjugated to a half-life extending moiety may be evaluated for their pharmacokinetic properties utilizing known *in vivo* models.

**Conjugation to immunoglobulin (Ig) constant regions or fragments of the Ig constant regions**

The antigen binding domains that bind CD33 of the disclosure may be conjugated to an Ig constant region or a fragment of the Ig constant region to impart antibody-like properties, including Fc effector functions Clq binding, complement dependent cytotoxicity (CDC), Fc receptor binding, antibody-dependent cell-mediated cytotoxicity (ADCC), phagocytosis or down regulation of cell surface receptors (e.g., B cell receptor; BCR). The Ig constant region or the fragment of the Ig constant region functions also as a half-life extending moiety as discussed herein. The antigen binding domains that bind CD33 of the disclosure may be engineered into conventional full length antibodies using standard methods. The full length antibodies comprising the antigen binding domain that binds CD33 may further be engineered as described herein.

Immunoglobulin heavy chain constant region comprised of subdomains CH1, hinge, CH2 and CH3. The CH1 domain spans residues Al 18-V215, the CH2 domain residues A231-K340 and the CH3 domain residues G341-K447 on the heavy chain, residue numbering according to the EU Index. In some instances G341 is referred as a CH2 domain residue. "Hinge" is generally defined as including E216 and terminating at P230 of human IgG1. Ig Fc region comprises at least the CH2 and the CH3 domains of the Ig constant region, and therefore comprises at least a region from about A231 to K447 of Ig heavy chain constant region.

The invention also provides an antigen binding domain that binds CD33 conjugated to an immunoglobulin (Ig) constant region or a fragment of the Ig constant region. In certain such embodiments, the Ig constant region or a fragment of the Ig constant region is derived from IgG1.

In some embodiments, the Ig constant region is a heavy chain constant region. In certain such embodiments, the Ig constant region is an IgG1 heavy chain constant region.

In some embodiments, the Ig constant region is a light chain constant region.

In some embodiments, the fragment of the Ig constant region comprises a Fc region. In certain such embodiments, the Ig constant region comprises an IgG1 Fc region. In some embodiments, the fragment of the Ig constant region comprises a CH2 domain. In certain such embodiments, the Ig constant region comprises an IgG1 CH2 domain.

In some embodiments, the fragment of the Ig constant region comprises a CH3 domain. In certain such embodiments, the Ig constant region comprises an IgG1 CH3 domain.

In some embodiments, the fragment of the Ig constant region comprises the CH2 domain and the CH3 domain. In certain such embodiments, the Ig constant region comprises an IgG1 CH2 and CH3 domain.

In some embodiments, the fragment of the Ig constant region comprises at least portion of a hinge, the CH2 domain and the CH3 domain. In certain such embodiments, at least portion of a hinge, the CH2 domain and the CH3 domain from IgG1. Portion of the hinge refers to one or more amino acid residues of the Ig hinge.

In some embodiments, the fragment of the Ig constant region comprises the hinge, the CH2 domain and the CH3 domain. In certain such embodiment, the hinge, the CH2 domain and the CH3 domain from IgG1.

In certain embodiments, the fragment of the Ig constant region comprises an amino acid sequence of SEQ ID NO: 278.

The cysteine residue at position 220 of the heavy chain may be mutated to prevent the formation of a cysteine bridge between the constant region and a light chain. Thus, in certain embodiments, the Ig constant region comprises a residue that is not cysteine at position 220. In one embodiment, the Ig constant region comprises a serine at position 220.

In certain embodiments, the fragment of the Ig constant region comprises an amino acid sequence of SEQ ID NO: 279.

In some embodiments, the antigen binding domain that binds CD33 is conjugated to the N-terminus of the Ig constant region or the fragment of the Ig constant region.

In some embodiments, the antigen binding domain that binds CD33 is conjugated to the C-terminus of the Ig constant region or the fragment of the Ig constant region.

In some embodiments, the antigen binding domain that binds CD33 is conjugated to the Ig constant region or the fragment of the Ig constant region via a second linker (L2).

In some embodiments, the L2 comprises the amino acid sequence of SEQ ID NOs: 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, or 140.

The antigen binding domains that binds CD33 of the disclosure conjugated to Ig constant region or the fragment of the Ig constant region may be assessed for their functionality using several known assays. Binding to CD33 may be assessed using methods described herein. Altered properties imparted by the Ig constant domain or the fragment of the Ig constant region such as Fc region may be assayed in Fc receptor binding assays using soluble forms of the receptors, such as the FcγRI, FcγRII, FcγRIII or FcRn receptors, or using cell-based assays measuring for example ADCC, CDC or ADCP.

ADCC may be assessed using an *in vitro* assay using CD33 expressing cells as target cells and NK cells as effector cells. Cytolysis may be detected by the release of label (e.g. radioactive substrates, fluorescent dyes or natural intracellular proteins) from the lysed cells. In an exemplary assay, target cells are used with a ratio of 1 target cell to 4 effector cells. Target cells are pre-labeled with BATDA and combined with effector cells and the test antibody. The samples are incubated for 2 hours and cell lysis measured by measuring released BATDA into the supernatant. Data is normalized to maximal cytotoxicity with 0.67% Triton X-100 (Sigma Aldrich) and minimal control determined by spontaneous release of BATDA from target cells in the absence of any antibody.

ADCP may be evaluated by using monocyte-derived macrophages as effector cells and any CD33 expressing cells as target cells which are engineered to express GFP or other labeled molecule. In an exemplary assay, effector: target cell ratio may be for example 4:1. Effector cells may be incubated with target cells for 4 hours with or without the antibody of the invention. After incubation, cells may be detached using accutase. Macrophages may be identified with anti-CD11b and anti-CD14 antibodies coupled to a fluorescent label, and percent phagocytosis may be determined based on % GFP fluorescence in the $CD11^+CD14^+$ macrophages using standard methods.

CDC of cells may be measured for example by plating Daudi cells at $1\times10^5$ cells/well (50 μL/well) in RPMI-B (RPMI supplemented with 1% BSA), adding 50 μL of test protein to the wells at final concentration between 0-100 μg/mL, incubating the reaction for 15 min at room temperature, adding 11 μL of pooled human serum to the wells, and incubation the reaction for 45 min at 37° C. Percentage (%) lysed cells may be detected as % propidium iodide stained cells in FACS assay using standard methods.

**Proteins comprising the antigen binding domains that bind CD33 of the disclosure**

The antigen binding domains that bind CD33 of the disclosure may be engineered into monospecific or multispecific proteins of various designs using standard methods.

The disclosure also provides a monospecific protein comprising the antigen binding domain that binds CD33 of the disclosure.

In some embodiments, the monospecific protein is an antibody.

The disclosure also provides a multispecific protein comprising the antigen binding domain that binds CD33 of the disclosure.

In some embodiments, the multispecific protein is bispecific.

In some embodiments, the multispecific protein is trispecific.

In some embodiments, the multispecific protein is tetraspecific.

In some embodiments, the multispecific protein is monovalent for binding to CD33.

In some embodiments, the multispecific protein is bivalent for binding to CD33.

The disclosure also provides an isolated multispecific protein comprising a first antigen binding domain that binds CD33 and a second antigen binding domain that binds a lymphocyte antigen.

In some embodiments, the lymphocyte antigen is a T cell antigen.

In some embodiments, the T cell antigen is a $CD8^+$ T cell antigen.

In some embodiments, the lymphocyte antigen is a NK cell antigen.

In some embodiments, the lymphocyte antigen is CD3, CD3 epsilon (CD3ε), CD8, KI2L4, NKG2E, NKG2D, NKG2F, BTNL3, CD186, BTNL8, PD-1, CD195, TRGV9, or NKG2C.

In a certain embodiments, the multispecific protein is a bispecific protein comprising a first antigen binding domain that binds CD33 and second antigen binding domain that binds to TRGV9.TRGV9 is also known as Vy9, and is expressed on γδT cells. As used herein, the term "TRGV9" refers to a polypeptide capable of forming a T cell receptor when expressed on the surface of γδ T cells. TRGV9-expressing γδ T cells are among the first T cells to develop in the human fetus and are the predominant γδ T cell subset in healthy adult peripheral blood cells. The term "TRGV9" includes any TRGV9 variant, isoform, and species homolog, which is naturally expressed by cells (including T cells) or can be expressed on cells transfected with genes or cDNA encoding the polypeptide. Unless noted, preferably the TRGV9 is a human TRGV9. A human TRGV9 amino acid sequence is provided by GenBank Accession Number NG_001336.2. Vy9V82 T lymphocytes, a major γ/δ T cell subset in humans, can recognize phosphoantigens, certain tumor cells, and cells treated with aminobisphosphonates. Vy9V82 T lymphocytes can display cytolytic activity against various tumor cells. Those having ordinary skill appreciate that γ/δ TCR is an heterodimeric TCR complex composed of covalently bound y and δ chains involved in antigen recognition and the non-covalently associated monomorphic proteins CD3δ,

γ, ε, and ζ chains. The Vγ9 TCR is a variant of the TCR γ chain expressed on a subset of γ/δ T cells.

Without wishing to be bound by theory, a bispecific antibody expressing both TRGV9 antigen and a CD33 antigen could recruit γδT cells to the cancerous cells expressing CD33. A multispecific antibody (e.g., a bispecific antibody) can bridge the effector cell (e.g., γδ T cell) and target cells together so as to resulting in cancer cell killing. γδ T-cells may have innate immunity. Without wishing to be bound by theory, γδ T-cells represent only a minor proportion of the peripheral $CD3^+$ T cells (about 1%-5%), but constitute a major subset (about 20%-50%) of T-cells in epithelial tissues. Circulating γδ T-cells mainly express heterodimers of Vγ9 (TRGV9) and Vδ2 (TRVD2) chains whereas tissue γδ T-cells preferentially express Vδ1 chains associated with different Vy chains.

In humans, γδ T-cells are endowed with potent anti-cancer functions (high cytotoxicity and interferon γ secretion). Moreover, γδ T-cells are capable of phagocytosis, a function previously exclusive to innate myeloid lineage cells, and behave as efficient antigen-presenting cells for αβ T-cells and induce adaptive immune response. γδ T-cells may infiltrate cancerous tissues, tumors and cancerous cells. A bispecific antibody expressing both TRGV9 antigen and a CD33 antigen may exhibit less or no significant T cell redirection, less pan activation of T cells, and have increased induction of potent cancer lysis via selective recruitment of γδ T cells. Accordingly, a bispecific antibody expressing both TRGV9 antigen and a CD33 antigen may not lead to severe side effects that might arise by cytokine storm induction.

In some embodiments, the lymphocyte antigen is CD3ε.

In some embodiments, the first antigen binding domain that binds CD33 and/or the second antigen binding domain that binds the lymphocyte antigen (for example, TRGV9) comprise a scFv, a $(scFv)_2$, a Fv, a Fab, a $F(ab')_2$, a Fd, a dAb or a VHH.

In some embodiments, the first antigen binding domain that binds CD33 and/or the second antigen binding domain that binds the lymphocyte antigen (for example, TRGV9) comprise the Fab.

In some embodiments, the first antigen binding domain that binds CD33 and/or the second antigen binding domain that binds the lymphocyte antigen (for example, TRGV9) comprise the $F(ab')_2$.

In some embodiments, the first antigen binding domain that binds CD33 and/or the second antigen binding domain that binds the lymphocyte antigen (for example, TRGV9) comprise the VHH.

In some embodiments, the first antigen binding domain that binds CD33 and/or the second antigen binding domain that binds the lymphocyte antigen (for example, TRGV9) comprise the Fv.

In some embodiments, the first antigen binding domain that binds CD33 and/or the second antigen binding domain that binds the lymphocyte antigen (for example, TRGV9) comprise the Fd.

In some embodiments, the first antigen binding domain that binds CD33 and/or the second antigen binding domain that binds the lymphocyte antigen (for example, TRGV9) comprise the scFv.

In some embodiments, the first antigen binding domain that binds CD33 comprises an scFv and the second binding domain that binds the lymphocyte antigen (for example, TRGV9) comprises a VHH.

In some embodiments, the scFv comprises, from the N- to C-terminus, a VH, a first linker (L1) and a VL (VH-L1-VL) or the VL, the L1 and the VH (VL-L1-VH).

In some embodiments, the L 1 comprises about 5-50 amino acids.

In some embodiments, the L1 comprises about 5-40 amino acids.

In some embodiments, the L1 comprises about 10-30 amino acids.

In some embodiments, the L1 comprises about 10-20 amino acids.

In some embodiments, the L1 comprises the amino acid sequence of SEQ ID NOs: 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, or 140.

In some embodiments, the L1 comprises the amino acid sequence of SEQ ID NO: 108.

In some embodiments, the L1 comprises the amino acid sequence of SEQ ID NO: 109.

In some embodiments, the L1 comprises the amino acid sequence of SEQ ID NO: 110.

In some embodiments, the L1 comprises the amino acid sequence of SEQ ID NO: 111.

In some embodiments, the L1 comprises the amino acid sequence of SEQ ID NO: 112.

In some embodiments, the L1 comprises the amino acid sequence of SEQ ID NO: 113.

In some embodiments, the L1 comprises the amino acid sequence of SEQ ID NO: 114.

In some embodiments, the L1 comprises the amino acid sequence of SEQ ID NO: 115.

In some embodiments, the L1 comprises the amino acid sequence of SEQ ID NO: 116.

In some embodiments, the L1 comprises the amino acid sequence of SEQ ID NO: 117.

In some embodiments, the L1 comprises the amino acid sequence of SEQ ID NO: 118.

In some embodiments, the L1 comprises the amino acid sequence of SEQ ID NO: 119.

In some embodiments, the L1 comprises the amino acid sequence of SEQ ID NO: 120.

In some embodiments, the L1 comprises the amino acid sequence of SEQ ID NO: 121.

In some embodiments, the L1 comprises the amino acid sequence of SEQ ID NO: 122.

In some embodiments, the L1 comprises the amino acid sequence of SEQ ID NO: 123.

In some embodiments, the L1 comprises the amino acid sequence of SEQ ID NO: 124.
In some embodiments, the L1 comprises the amino acid sequence of SEQ ID NO: 125.
In some embodiments, the L1 comprises the amino acid sequence of SEQ ID NO: 126.
In some embodiments, the L1 comprises the amino acid sequence of SEQ ID NO: 127.
In some embodiments, the L1 comprises the amino acid sequence of SEQ ID NO: 128.
In some embodiments, the L1 comprises the amino acid sequence of SEQ ID NO: 129.
In some embodiments, the L1 comprises the amino acid sequence of SEQ ID NO: 130.
In some embodiments, the L1 comprises the amino acid sequence of SEQ ID NO: 131.
In some embodiments, the L1 comprises the amino acid sequence of SEQ ID NO: 132.
In some embodiments, the L1 comprises the amino acid sequence of SEQ ID NO: 133.
In some embodiments, the L1 comprises the amino acid sequence of SEQ ID NO: 134.
In some embodiments, the L1 comprises the amino acid sequence of SEQ ID NO: 135.
In some embodiments, the L1 comprises the amino acid sequence of SEQ ID NO: 136.
In some embodiments, the L1 comprises the amino acid sequence of SEQ ID NO: 137.
In some embodiments, the L1 comprises the amino acid sequence of SEQ ID NO: 138.
In some embodiments, the L1 comprises the amino acid sequence of SEQ ID NO: 139.
In some embodiments, the L1 comprises the amino acid sequence of SEQ ID NO: 140.
In certain embodiments, the L1 comprises the amino acid sequence of SEQ ID NO: 108.
In some embodiments, the first antigen binding domain that binds CD33 comprises the HCDR1 of SEQ ID NOs: 1, 2, 3, 4, 5, 28, 29, 30, 31, 32, 33, 34, 35, 89, 167, 172, 176, 253, 254 or 255, the HCDR2 of SEQ ID NOs: 6, 7, 8, 9, 10, 11, 36, 37, 38, 39, 40, 41, 42, 43, 44, 90, 91, 168, 173, 177, 256, 257 or 258 the HCDR3 of SEQ ID NOs: 12, 13, 14, 15, 16, 17, 45, 46, 47, 48, 49, 50, 51, 92, 93, 94, 169, 174, 178, 180, 259, 260 or 261 the LCDR1 of SEQ ID NOs: 62, 63, 64, 65, 66, 67, 68, 98, 99, 100, 182, 186, 189, 193, 197, 201, 265 or 266 the LCDR2 of SEQ ID NOs: 69, 70, 71, 72, 73, 74, 101, 102, 103, 183, 187, 190, 194, 198, 267 or 268 and the LCDR3 of SEQ ID NOs: 75, 76, 77, 78, 79, 80, 104, 184, 191, 195, 199, 269 or 270.
In some embodiments, the first antigen binding domain that binds CD33 comprises the HCDR1, the HCDR2, the HCDR3, the LCDR1, the LCDR2 and the LCDR3 of

SEQ ID NOs: 28, 36, 45, 62, 69, and 75, respectively;
SEQ ID NOs: 29, 37, 46, 63, 70, and 76, respectively;
SEQ ID NOs: 30, 38, 47, 64, 71, and 77, respectively;
SEQ ID NOs: 31, 39, 48, 65, 72, and 78, respectively;
SEQ ID NOs: 32, 40, 49, 66, 70, and 76, respectively;
SEQ ID NOs: 33, 41, 50, 67, 73, and 79, respectively;
SEQ ID NOs: 34, 42, 51, 68, 74, and 80, respectively;
SEQ ID NOs: 34, 43, 51, 68, 74, and 80, respectively;
SEQ ID NOs: 34, 44, 51, 68, 74, and 80, respectively;
SEQ ID NOs: 35, 42, 51, 68, 74, and 80, respectively;
SEQ ID NOs: 89, 90, 92, 98, 101, and 104, respectively;
SEQ ID NOs: 89, 90, 93, 99, 102, and 104, respectively;
SEQ ID NOs: 33, 91, 94, 100, 103, and 104, respectively;
SEQ ID NOs: 167, 168, 169, 182, 183, and 184, respectively;
SEQ ID NOs: 33, 91, 94, 186, 187, and 104, respectively;
SEQ ID NOs: 172, 173, 174, 189, 190, and 191, respectively;
SEQ ID NOs: 176, 177, 178, 193, 194, and 195, respectively;
SEQ ID NOs: 89, 90, 180, 197, 198, and 199, respectively;
SEQ ID NOs: 89, 90, 93, 201, 102, and 104, respectively;
SEQ ID NOs: 253, 256, 259, 265, 74 and 269 respectively;
SEQ ID NOs: 254, 257, 260, 66, 267 and 76 respectively
SEQ ID NOs: 255, 258, 261, 266, 268 and 270 respectively.

In certain embodiments, the first antigen binding domain that binds CD33 comprises the HCDR1, the HCDR2, the HCDR3, the LCDR1, the LCDR2 and the LCDR3 of SEQ ID NOs: 29, 37, 46, 63, 70, and 76, respectively. In certain such embodiments, the first antigen binding domain is an scFv.
In other embodiments, the first antigen binding domain that binds CD33 comprises the HCDR1, the HCDR2, the HCDR3, the LCDR1, the LCDR2 and the LCDR3 of SEQ ID NOs: 32, 40, 49, 66, 70, and 76, respectively. In certain such embodiments, the first antigen binding domain is an scFv.
In other embodiments, the first antigen binding domain that binds CD33 comprises the HCDR1, the HCDR2,

the HCDR3, the LCDR1, the LCDR2 and the LCDR3 of SEQ ID NOs: 34, 43, 51, 68, 74, and 80, respectively. In certain such embodiments, the first antigen binding domain is an scFv.

In other embodiments, the first antigen binding domain that binds CD33 comprises the HCDR1, the HCDR2, the HCDR3, the LCDR1, the LCDR2 and the LCDR3 of SEQ ID NOs: 253, 256, 259, 265, 74 and 269, respectively. In certain such embodiments, the first antigen binding domain is an scFv.

In other embodiments, the first antigen binding domain that binds CD33 comprises the HCDR1, the HCDR2, the HCDR3, the LCDR1, the LCDR2 and the LCDR3 of SEQ ID NOs: 254, 257, 260, 66, 267 and 76, respectively. In certain such embodiments, the first antigen binding domain is an scFv.

In other embodiments, the first antigen binding domain that binds CD33 comprises the HCDR1, the HCDR2, the HCDR3, the LCDR1, the LCDR2 and the LCDR3 of SEQ ID NOs: 255, 258, 261, 266, 268 and 270, respectively. In certain such embodiments, the first antigen binding domain is an scFv.

In certain embodiments, the first antigen binding domain that binds CD33 is an scFv comprising the HCDR1, the HCDR2, the HCDR3, the LCDR1, the LCDR2 and the LCDR3 of SEQ ID NOs: 29, 37, 46, 63, 70, and 76, respectively and the second antigen binding domain binds to TRGV9 (for example, wherein the second binding domain is a VHH).

In other embodiments, the first antigen binding domain that binds CD33 is an scFv comprising the HCDR1, the HCDR2, the HCDR3, the LCDR1, the LCDR2 and the LCDR3 of SEQ ID NOs: 32, 40, 49, 66, 70, and 76, respectively and the second antigen binding domain binds to TRGV9 (for example, wherein the second binding domain is a VHH).

In other embodiments, the first antigen binding domain that binds CD33 is an scFv comprising the HCDR1, the HCDR2, the HCDR3, the LCDR1, the LCDR2 and the LCDR3 of SEQ ID NOs: 34, 43, 51, 68, 74, and 80, respectively and the second antigen binding domain binds to TRGV9 (for example, wherein the second binding domain is a VHH).

In other embodiments, the first antigen binding domain that binds CD33 is an scFv comprising the HCDR1, the HCDR2, the HCDR3, the LCDR1, the LCDR2 and the LCDR3 of SEQ ID NOs: 253, 256, 259, 265, 74 and 269, respectively and the second antigen binding domain binds to TRGV9 (for example, wherein the second binding domain is a VHH).

In other embodiments, the first antigen binding domain that binds CD33 is an scFv comprising the HCDR1, the HCDR2, the HCDR3, the LCDR1, the LCDR2 and the LCDR3 of SEQ ID NOs: 254, 257, 260, 66, 267 and 76, respectively and the second antigen binding domain binds to TRGV9 (for example, wherein the second binding domain is a VHH).

In other embodiments, the first antigen binding domain that binds CD33 is an scFv comprises the HCDR1, the HCDR2, the HCDR3, the LCDR1, the LCDR2 and the LCDR3 of SEQ ID NOs: 255, 258, 261, 266, 268 and 270, respectively and the second antigen binding domain binds to TRGV9 (for example, wherein the second binding domain is a VHH).

In some embodiments, the first antigen binding domain that binds CD33 comprises the VH of SEQ ID NO: 52 and the VL of SEQ ID NO: 81.

In some embodiments, the first antigen binding domain that binds CD33 comprises the VH of SEQ ID NO: 53 and the VL of SEQ ID NO: 82.

In some embodiments, the first antigen binding domain that binds CD33 comprises the VH of SEQ ID NO: 54 and the VL of SEQ ID NO: 83.

In some embodiments, the first antigen binding domain that binds CD33 comprises the VH of SEQ ID NO: 55 and the VL of SEQ ID NO: 84.

In some embodiments, the first antigen binding domain that binds CD33 comprises the VH of SEQ ID NO: 56 and the VL of SEQ ID NO: 85.

In some embodiments, the first antigen binding domain that binds CD33 comprises the VH of SEQ ID NO: 57 and the VL of SEQ ID NO: 86.

In some embodiments, the first antigen binding domain that binds CD33 comprises the VH of SEQ ID NO: 58 and the VL of SEQ ID NO: 87.

In some embodiments, the first antigen binding domain that binds CD33 comprises the VH of SEQ ID NO: 59 and the VL of SEQ ID NO: 87.

In some embodiments, the first antigen binding domain that binds CD33 comprises the VH of SEQ ID NO: 60 and the VL of SEQ ID NO: 87.

In some embodiments, the first antigen binding domain that binds CD33 comprises the VH of SEQ ID NO: 61 and the VL of SEQ ID NO: 88.

In some embodiments, the first antigen binding domain that binds CD33 comprises the VH of SEQ ID NO: 95 and the VL of SEQ ID NO: 105.

In some embodiments, the first antigen binding domain that binds CD33 comprises the VH of SEQ ID NO: 96 and the VL of SEQ ID NO: 106.

In some embodiments, the first antigen binding domain that binds CD33 comprises the VH of SEQ ID NO: 97 and the VL of SEQ ID NO: 107.

In some embodiments, the first antigen binding domain that binds CD33 comprises the VH of SEQ ID NO: 170 and the VL of SEQ ID NO: 185.

In some embodiments, the first antigen binding domain that binds CD33 comprises the VH of SEQ ID NO: 171 and the VL of SEQ ID NO: 188.

In some embodiments, the first antigen binding domain that binds CD33 comprises the VH of SEQ ID NO: 175 and the VL of SEQ ID NO: 192.

In some embodiments, the first antigen binding domain that binds CD33 comprises the VH of SEQ ID NO: 179 and the VL of SEQ ID NO: 196.

In some embodiments, the first antigen binding domain that binds CD33 comprises the VH of SEQ ID NO: 181 and the VL of SEQ ID NO: 200.

In some embodiments, the first antigen binding domain that binds CD33 comprises the VH of SEQ ID NO: 96 and the VL of SEQ ID NO: 202.

In some embodiments, the first antigen binding domain that binds CD33 comprises the VH of SEQ ID NO: 262 and the VL of SEQ ID NO: 271.

In some embodiments, the first antigen binding domain that binds CD33 comprises the VH of SEQ ID NO: 263 and the VL of SEQ ID NO: 272. In some embodiments, the first antigen binding domain that binds CD33 comprises the VH of SEQ ID NO: 264 and the VL of SEQ ID NO: 273.

In certain embodiments, the first antigen binding domain that binds CD33 comprises the VH of SEQ ID NO: 53 and the VL of SEQ ID NO: 82. In certain such embodiments, the first antigen binding domain is an scFv.

In other embodiments, the first antigen binding domain that binds CD33 comprises the VH of SEQ ID NO: 56 and the VL of SEQ ID NO: 85. In certain such embodiments, the first antigen binding domain is an scFv.

In other embodiments, the first antigen binding domain that binds CD33 comprises the VH of SEQ ID NO: 59 and the VL of SEQ ID NO: 87. In certain such embodiments, the first antigen binding domain is an scFv.

In other embodiments, the first antigen binding domain that binds CD33 comprises the VH of SEQ ID NO: 262 and the VL of SEQ ID NO: 271. In certain such embodiments, the first antigen binding domain is an scFv.

In other embodiments, the first antigen binding domain that binds CD33 comprises the VH of SEQ ID NO: 263 and the VL of SEQ ID NO: 272. In certain such embodiments, the first antigen binding domain is an scFv.

In other embodiments, the first antigen binding domain that binds CD33 comprises the VH of SEQ ID NO: 264 and the VL of SEQ ID NO: 273. In certain such embodiments, the first antigen binding domain is an scFv. In certain embodiments, the first antigen binding domain that binds CD33 is an scFv comprising the VH of SEQ ID NO: 53 and the VL of SEQ ID NO: 82 and the second antigen binding domain binds to TRGV9 (for example, wherein the second binding domain is a VHH).

In other embodiments, the first antigen binding domain that binds CD33 is an scFv comprising the VH of SEQ ID NO: 56 and the VL of SEQ ID NO: 85 and the second antigen binding domain binds to TRGV9 (for example, wherein the second binding domain is a VHH).

In other embodiments, the first antigen binding domain that binds CD33 is an scFv comprising the VH of SEQ ID NO: 59 and the VL of SEQ ID NO: 87 and the second antigen binding binds to TRGV9 (for example, wherein the second binding domain is a VHH).

In other embodiments, the first antigen binding domain that binds CD33 is an scFv comprising the VH of SEQ ID NO: 262 and the VL of SEQ ID NO: 271 and the second antigen binding domain binds to TRGV9 (for example, wherein the second binding domain is a VHH).

In other embodiments, the first antigen binding domain that binds CD33 is an scFv comprising the VH of SEQ ID NO: 263 and the VL of SEQ ID NO: 272 and the second antigen binding domain binds to TRGV9 (for example, wherein the second binding domain is a VHH).

In other embodiments, the first antigen binding domain that binds CD33 is an scFv comprising the VH of SEQ ID NO: 264 and the VL of SEQ ID NO: 273 and the second antigen binding domain binds to TRGV9 (for example, wherein the second binding domain is a VHH).

In some embodiments, the first antigen binding domain that binds CD33 comprises the VH of SEQ ID NOs: 52, 53, 54, 55, 56, 57, 58, 59, 60, 61, 262, 263 or 264 and the VL of SEQ ID NOs: 81, 82, 83, 84, 85, 86, 87, 88, 271, 272 or 273.

In some embodiments, the first antigen binding domain that binds CD33 comprises the VH of SEQ ID NOs: 95, 96, 97, 170, 171, 175, 179, 181, or 96, and the VL of SEQ ID NOs: 105, 106, 107, 185, 188, 192, 196, 200, or 202.

In some embodiments, the first antigen binding domain that binds CD33 comprises:

the VH of SEQ ID NO: 95 and the VL of SEQ ID NO: 105;
the VH of SEQ ID NO: 96 and the VL of SEQ ID NO: 106;
the VH of SEQ ID NO: 97 and the VL of SEQ ID NO: 107;
the VH of SEQ ID NO: 170 and the VL of SEQ ID NO: 185;
the VH of SEQ ID NO: 171 and the VL of SEQ ID NO: 188;
the VH of SEQ ID NO: 175 and the VL of SEQ ID NO: 192;

the VH of SEQ ID NO: 179 and the VL of SEQ ID NO: 196;
the VH of SEQ ID NO: 181 and the VL of SEQ ID NO: 200; or
the VH of SEQ ID NO: 96 and the VL of SEQ ID NO: 202.

In some embodiments, the first antigen binding domain that binds CD33 comprises the amino acid sequence of SEQ ID NOs: 18, 19, 20, 21, 22, 23, 24, 25, 26, or 27.

In some embodiments, the first antigen binding domain that binds CD33 comprises the amino acid sequence of SEQ ID NO: 18.

In some embodiments, the first antigen binding domain that binds CD33 comprises the amino acid sequence of SEQ ID NO: 19.

In some embodiments, the first antigen binding domain that binds CD33 comprises the amino acid sequence of SEQ ID NO: 20.

In some embodiments, the first antigen binding domain that binds CD33 comprises the amino acid sequence of SEQ ID NO: 21.

In some embodiments, the first antigen binding domain that binds CD33 comprises the amino acid sequence of SEQ ID NO: 22.

In some embodiments, the first antigen binding domain that binds CD33 comprises the amino acid sequence of SEQ ID NO: 23.

In some embodiments, the first antigen binding domain that binds CD33 comprises the amino acid sequence of SEQ ID NO: 24.

In some embodiments, the first antigen binding domain that binds CD33 comprises the amino acid sequence of SEQ ID NO: 25.

In some embodiments, the first antigen binding domain that binds CD33 comprises the amino acid sequence of SEQ ID NO: 26.

In some embodiments, the first antigen binding domain that binds CD33 comprises the amino acid sequence of SEQ ID NO: 27.

In some embodiments, the first antigen binding domain that binds CD33 comprises the amino acid sequence of SEQ ID NOs: 52, 53, 54, 55, 56, 57, 58, 59, 60 61, 262, 263 or 264.

In some embodiments, the first antigen binding domain that binds CD33 comprises the amino acid sequence of SEQ ID NO: 52.

In some embodiments, the first antigen binding domain that binds CD33 comprises the amino acid sequence of SEQ ID NO: 53.

In some embodiments, the first antigen binding domain that binds CD33 comprises the amino acid sequence of SEQ ID NO: 54.

In some embodiments, the first antigen binding domain that binds CD33 comprises the amino acid sequence of SEQ ID NO: 55.

In some embodiments, the first antigen binding domain that binds CD33 comprises the amino acid sequence of SEQ ID NO: 56.

In some embodiments, the first antigen binding domain that binds CD33 comprises the amino acid sequence of SEQ ID NO: 57.

In some embodiments, the first antigen binding domain that binds CD33 comprises the amino acid sequence of SEQ ID NO: 58.

In some embodiments, the first antigen binding domain that binds CD33 comprises the amino acid sequence of SEQ ID NO: 59.

In some embodiments, the first antigen binding domain that binds CD33 comprises the amino acid sequence of SEQ ID NO: 60.

In some embodiments, the first antigen binding domain that binds CD33 comprises the amino acid sequence of SEQ ID NO: 61.

In some embodiments, the first antigen binding domain that binds CD33 comprises the amino acid sequence of SEQ ID NO: 262.

In some embodiments, the first antigen binding domain that binds CD33 comprises the amino acid sequence of SEQ ID NO: 263.

In some embodiments, the first antigen binding domain that binds CD33 comprises the amino acid sequence of SEQ ID NO: 264.In certain embodiments, the first antigen binding domain that binds CD33 comprises the amino acid sequence of SEQ ID NO: 53. In certain such embodiments, the first antigen binding domain is an scFv.

In other embodiments, the first antigen binding domain that binds CD33 comprises the amino acid sequence of SEQ ID NO: 56. In certain such embodiments, the first antigen binding domain is an scFv.

In other embodiments, the first antigen binding domain that binds CD33 comprises the amino acid sequence

of SEQ ID NO: 59. In certain such embodiments, the first antigen binding domain is an scFv.

In other embodiments, the first antigen binding domain that binds CD33 comprises the amino acid sequence of SEQ ID NO: 262. In certain such embodiments, the first antigen binding domain is an scFv.

In other embodiments, the first antigen binding domain that binds CD33 comprises the amino acid sequence of SEQ ID NO: 263. In certain such embodiments, the first antigen binding domain is an scFv.

In other embodiments, the first antigen binding domain that binds CD33 comprises the amino acid sequence of SEQ ID NO: 264. In certain such embodiments, the first antigen binding domain is an scFv.

In some embodiments, the first antigen binding domain that binds CD33 comprises the amino acid sequence of SEQ ID NOs: 95, 96, 97, 170, 171, 175, 179, 181, or 96.

In some embodiments, the first antigen binding domain that binds CD33 comprises the amino acid sequence of SEQ ID NO: 95.

In some embodiments, the first antigen binding domain that binds CD33 comprises the amino acid sequence of SEQ ID NO: 96.

In some embodiments, the first antigen binding domain that binds CD33 comprises the amino acid sequence of SEQ ID NO: 97.

In some embodiments, the first antigen binding domain that binds CD33 comprises the amino acid sequence of SEQ ID NO: 170.

In some embodiments, the first antigen binding domain that binds CD33 comprises the amino acid sequence of SEQ ID NO: 171.

In some embodiments, the first antigen binding domain that binds CD33 comprises the amino acid sequence of SEQ ID NO: 175.

In some embodiments, the first antigen binding domain that binds CD33 comprises the amino acid sequence of SEQ ID NO: 179.

In some embodiments, the first antigen binding domain that binds CD33 comprises the amino acid sequence of SEQ ID NO: 181.

In some embodiments, the first antigen binding domain that binds CD33 comprises the amino acid sequence of SEQ ID NO: 96.

In some embodiments, the first antigen binding domain that binds CD33 comprises the amino acid sequence of SEQ ID NOs: 81, 82, 83, 84, 85, 86, 87, 88, 271, 272 or 273.

In some embodiments, the first antigen binding domain that binds CD33 comprises the amino acid sequence of SEQ ID NO: 81.

In some embodiments, the first antigen binding domain that binds CD33 comprises the amino acid sequence of SEQ ID NO: 82.

In some embodiments, the first antigen binding domain that binds CD33 comprises the amino acid sequence of SEQ ID NO: 83.

In some embodiments, the first antigen binding domain that binds CD33 comprises the amino acid sequence of SEQ ID NO: 84.

In some embodiments, the first antigen binding domain that binds CD33 comprises the amino acid sequence of SEQ ID NO: 85.

In some embodiments, the first antigen binding domain that binds CD33 comprises the amino acid sequence of SEQ ID NO: 86.

In some embodiments, the first antigen binding domain that binds CD33 comprises the amino acid sequence of SEQ ID NO: 87.

In some embodiments, the first antigen binding domain that binds CD33 comprises the amino acid sequence of SEQ ID NO: 88.

In some embodiments, the first antigen binding domain that binds CD33 comprises the amino acid sequence of SEQ ID NO: 271.

In some embodiments, the first antigen binding domain that binds CD33 comprises the amino acid sequence of SEQ ID NO: 272.

In some embodiments, the first antigen binding domain that binds CD33 comprises the amino acid sequence of SEQ ID NO: 273.

In certain embodiments, the first antigen binding domain that binds CD33 comprises the amino acid sequence of SEQ ID NO: 82. In certain such embodiments, the first antigen binding domain is an scFv.

In other embodiments, the first antigen binding domain that binds CD33 comprises the amino acid sequence of SEQ ID NO: 85. In certain such embodiments, the first antigen binding domain is an scFv.

In other embodiments, the first antigen binding domain that binds CD33 comprises the amino acid sequence of SEQ ID NO: 87. In certain such embodiments, the first antigen binding domain is an scFv.

In other embodiments, the first antigen binding domain that binds CD33 comprises the amino acid sequence

of SEQ ID NO: 271. In certain such embodiments, the first antigen binding domain is an scFv.

In other embodiments, the first antigen binding domain that binds CD33 comprises the amino acid sequence of SEQ ID NO: 272. In certain such embodiments, the first antigen binding domain is an scFv.

In other embodiments, the first antigen binding domain that binds CD33 comprises the amino acid sequence of SEQ ID NO: 273. In certain such embodiments, the first antigen binding domain is an scFv.

In some embodiments, the first antigen binding domain that binds CD33 comprises the amino acid sequence of SEQ ID NOs: 105, 106, 107, 185, 188, 192, 196, 200, or 202.

In some embodiments, the first antigen binding domain that binds CD33 comprises the amino acid sequence of SEQ ID NO: 105.

In some embodiments, the first antigen binding domain that binds CD33 comprises the amino acid sequence of SEQ ID NO: 106.

In some embodiments, the first antigen binding domain that binds CD33 comprises the amino acid sequence of SEQ ID NO: 107.

In some embodiments, the first antigen binding domain that binds CD33 comprises the amino acid sequence of SEQ ID NO: 185.

In some embodiments, the first antigen binding domain that binds CD33 comprises the amino acid sequence of SEQ ID NO: 188.

In some embodiments, the first antigen binding domain that binds CD33 comprises the amino acid sequence of SEQ ID NO: 192.

In some embodiments, the first antigen binding domain that binds CD33 comprises the amino acid sequence of SEQ ID NO: 196.

In some embodiments, the first antigen binding domain that binds CD33 comprises the amino acid sequence of SEQ ID NO: 200.

In some embodiments, the first antigen binding domain that binds CD33 comprises the amino acid sequence of SEQ ID NO: 202.In some embodiments, the first antigen binding domain that binds CD33 comprises the amino acid sequence of SEQ ID NOs: 203, 204, 205, 206, 207, 208, 209, 210, 211, 212, 213, 214, 215, 216, 217, 218, 219, 220 221, 274, 275 or 276.

In some embodiments, the first antigen binding domain that binds CD33 comprises the amino acid sequence of SEQ ID NO: 203.

In some embodiments, the first antigen binding domain that binds CD33 comprises the amino acid sequence of SEQ ID NO: 204.

In some embodiments, the first antigen binding domain that binds CD33 comprises the amino acid sequence of SEQ ID NO: 205.

In some embodiments, the first antigen binding domain that binds CD33 comprises the amino acid sequence of SEQ ID NO: 206.

In some embodiments, the first antigen binding domain that binds CD33 comprises the amino acid sequence of SEQ ID NO: 207.

In some embodiments, the first antigen binding domain that binds CD33 comprises the amino acid sequence of SEQ ID NO: 208.

In some embodiments, the first antigen binding domain that binds CD33 comprises the amino acid sequence of SEQ ID NO: 209.

In some embodiments, the first antigen binding domain that binds CD33 comprises the amino acid sequence of SEQ ID NO: 210.

In some embodiments, the first antigen binding domain that binds CD33 comprises the amino acid sequence of SEQ ID NO: 211.

In some embodiments, the first antigen binding domain that binds CD33 comprises the amino acid sequence of SEQ ID NO: 212.

In some embodiments, the first antigen binding domain that binds CD33 comprises the amino acid sequence of SEQ ID NO: 213.

In some embodiments, the first antigen binding domain that binds CD33 comprises the amino acid sequence of SEQ ID NO: 214.

In some embodiments, the first antigen binding domain that binds CD33 comprises the amino acid sequence of SEQ ID NO: 215.

In some embodiments, the first antigen binding domain that binds CD33 comprises the amino acid sequence of SEQ ID NO: 216.

In some embodiments, the first antigen binding domain that binds CD33 comprises the amino acid sequence of SEQ ID NO: 217.

In some embodiments, the first antigen binding domain that binds CD33 comprises the amino acid sequence

of SEQ ID NO: 218.

In some embodiments, the first antigen binding domain that binds CD33 comprises the amino acid sequence of SEQ ID NO: 219.

In some embodiments, the first antigen binding domain that binds CD33 comprises the amino acid sequence of SEQ ID NO: 220.

In some embodiments, the first antigen binding domain that binds CD33 comprises the amino acid sequence of SEQ ID NO: 221.

In some embodiments, the first antigen binding domain that binds CD33 comprises the amino acid sequence of SEQ ID NO: 274.

In some embodiments, the first antigen binding domain that binds CD33 comprises the amino acid sequence of SEQ ID NO: 275.

In some embodiments, the first antigen binding domain that binds CD33 comprises the amino acid sequence of SEQ ID NO: 276.

In certain embodiments, the first antigen binding domain that binds CD33 comprises the amino acid sequence of SEQ ID NO: 213.

In other embodiments, the first antigen binding domain that binds CD33 comprises the amino acid sequence of SEQ ID NO: 216.

In other embodiments, the first antigen binding domain that binds CD33 comprises the amino acid sequence of SEQ ID NO: 219.

In other embodiments, the first antigen binding domain that binds CD33 comprises the amino acid sequence of SEQ ID NO: 274.

In other embodiments, the first antigen binding domain that binds CD33 comprises the amino acid sequence of SEQ ID NO: 275.

In other embodiments, the first antigen binding domain that binds CD33 comprises the amino acid sequence of SEQ ID NO: 276.

In certain embodiments, the first antigen binding domain that binds CD33 is an scFv comprising the amino acid sequence of SEQ ID NO: 213 and the second antigen binding domain that binds to TRGV9 (for example, wherein the second binding domain is a VHH).

In other embodiments, the first antigen binding domain that binds CD33 is an scFv comprising the amino acid sequence of SEQ ID NO: 216 and the second antigen binding domain that binds to TRGV9 (for example, wherein the second binding domain is a VHH).

In other embodiments, the first antigen binding domain that binds CD33 is an scFv comprising the amino acid sequence of SEQ ID NO: 219 and the second antigen binding domain that binds to TRGV9 (for example, wherein the second binding domain is a VHH).

In other embodiments, the first antigen binding domain that binds CD33 is an scFv comprising the amino acid sequence of SEQ ID NO: 274 and the second antigen binding domain that binds to TRGV9 (for example, wherein the second binding domain is a VHH).

In other embodiments, the first antigen binding domain that binds CD33 is an scFv comprising the amino acid sequence of SEQ ID NO: 275 and the second antigen binding domain that binds to TRGV9 (for example, wherein the second binding domain is a VHH).

In other embodiments, the first antigen binding domain that binds CD33 is an scFv comprising the amino acid sequence of SEQ ID NO: 276 and the second antigen binding domain that n binds to TRGV9 (for example, wherein the second binding domain is a VHH). In some embodiments, the second antigen binding domain that binds a lymphocyte antigen comprises

the HCDR1 of SEQ ID NO: 141, the HCDR2 of SEQ ID NO: 142, the HCDR3 of SEQ ID NO: 143, the LCDR1 of SEQ ID NO: 144, the LCDR2 of SEQ ID NO: 145 and the LCDR3 of SEQ ID NO: 146;
the VH of SEQ ID NO: 147 and the VL of SEQ ID NO: 148;
the HCDR1 of SEQ ID NO: 149, the HCDR2 of SEQ ID NO: 150, the HCDR3 of SEQ ID NO: 151, the LCDR1 of SEQ ID NO: 152, the LCDR2 of SEQ ID NO: 153 and the LCDR3 of SEQ ID NO: 154; or
the VH of SEQ ID NO: 155 and the VL of SEQ ID NO: 156.

In some embodiments, the second antigen binding domain that binds the lymphocyte antigen comprises:

a heavy chain complementarity determining region 1 (HCDR1) of SEQ ID NO: 236, a HCDR2 of SEQ ID NO: 237, a HCDR3 of SEQ ID NO: 238, a light chain complementarity determining region 1 (LCDR1) of SEQ ID NO: 239, a LCDR2 of SEQ ID NO: 240 and a LCDR3 of SEQ ID NO: 241;
a heavy chain complementarity determining region 1 (HCDR1) of SEQ ID NO: 236, a HCDR2 of SEQ ID NO: 237,

a HCDR3 of SEQ ID NO: 242, a light chain complementarity determining region 1 (LCDR1) of SEQ ID NO: 239, a LCDR2 of SEQ ID NO: 240 and a LCDR3 of SEQ ID NO: 241;
a heavy chain complementarity determining region 1 (HCDR1) of SEQ ID NO: 236, a HCDR2 of SEQ ID NO: 237, a HCDR3 of SEQ ID NO: 243, a light chain complementarity determining region 1 (LCDR1) of SEQ ID NO: 239, a LCDR2 of SEQ ID NO: 240 and a LCDR3 of SEQ ID NO: 241;
a heavy chain complementarity determining region 1 (HCDR1) of SEQ ID NO: 236, a HCDR2 of SEQ ID NO: 237, a HCDR3 of SEQ ID NO: 244, a light chain complementarity determining region 1 (LCDR1) of SEQ ID NO: 239, a LCDR2 of SEQ ID NO: 240 and a LCDR3 of SEQ ID NO: 241;
the VH of SEQ ID NO: 245 and the VL of SEQ ID NO: 249;
the VH of SEQ ID NO: 246 and the VL of SEQ ID NO: 249;
the VH of SEQ ID NO: 247 and the VL of SEQ ID NO: 249; or
the VH of SEQ ID NO: 248 and the VL of SEQ ID NO: 249.

In some embodiments, the first antigen binding domain that binds CD33 is conjugated to a first immunoglobulin (Ig) constant region or a fragment of the first Ig constant region and/or the second antigen binding domain that binds the lymphocyte antigen is conjugated to a second immunoglobulin (Ig) constant region or a fragment of the second Ig constant region.

In some embodiments, the fragment of the first Ig constant region and/or the fragment of the second Ig constant region comprises a Fc region.

In some embodiments, the fragment of the first Ig constant region and/or the fragment of the second Ig constant region comprises a CH2 domain.

In some embodiments, the fragment of the first Ig constant region and/or the fragment of the second Ig constant region comprises a CH3 domain.

In some embodiments, the fragment of the first Ig constant region and/or the fragment of the second Ig constant region comprises the CH2 domain and the CH3 domain.

In some embodiments, the fragment of the first Ig constant region and/or the fragment of the second Ig constant region comprises at least portion of a hinge, the CH2 domain and the CH3 domain.

In some embodiments, the fragment of the Ig constant region comprises the hinge, the CH2 domain and the CH3 domain.

In some embodiments, the multispecific protein further comprises a second linker (L2) between the first antigen binding domain that binds CD33 and the first Ig constant region or the fragment of the first Ig constant region and the second antigen binding domain that binds the lymphocyte antigen and the second Ig constant region or the fragment of the second Ig constant region.

In some embodiments, the L2 comprises the amino acid sequence of SEQ ID NOs: 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, or 140.

In some embodiments, the first Ig constant region or the fragment of the first Ig constant region and the second Ig constant region or the fragment of the second Ig constant region is an IgG1, an IgG2, and IgG3 or an IgG4 isotype.

In some embodiments, the first Ig constant region or the fragment of the first Ig constant region and the second Ig constant region or the fragment of the second Ig constant region is an IgG 1 isotype.

In some embodiments, the first Ig constant region or the fragment of the first Ig constant region and the second Ig constant region or the fragment of the second Ig constant region is an IgG2 isotype.

In some embodiments, the first Ig constant region or the fragment of the first Ig constant region and the second Ig constant region or the fragment of the second Ig constant region is an IgG3 isotype.

In some embodiments, the first Ig constant region or the fragment of the first Ig constant region and the second Ig constant region or the fragment of the second Ig constant region is an IgG4 isotype.

The first Ig constant region or the fragment of the first Ig constant region and the second Ig constant region or the fragment of the second Ig constant region can further be engineered as described herein.

In some embodiments, the first Ig constant region or the fragment of the first Ig constant region and the second Ig constant region or the fragment of the second Ig constant region comprises at least one mutation that results in reduced binding of the multispecific protein to a FcγR. In certain such embodiments, the first Ig constant region or the fragment of the first Ig constant region and the second Ig constant region or the fragment of the second Ig constant region is an IgG1 isotype.

In some embodiments, the at least one mutation that results in reduced binding of the multispecific protein to the FcγR is selected from the group consisting of F234A/L235A, L234A/L235A, L234A/L235A/D265S, V234A/G237A/P238S/H268A/V309L/A330S/P331S, F234A/L235A, S228P/F234A/ L235A, N297A, V234A/G237A, K214T/E233P/L234V/L235A/G236-deleted/A327G/P331A/D365E/L358M, H268Q/V309L/A330S/P331S, S267E/L328F, L234F/L235E/D265A, L234A/L235A/G237A/P238S/H268A/A330S/P331S, S228P/F234A/L235A/G237A/P238S and

S228P/F234A/L235A/G236-deleted/G237A/P238S, wherein residue numbering is according to the EU index.

In certain embodiments, the first Ig constant region or the fragment of the first Ig constant region and the second Ig constant region or the fragment of the second Ig constant region comprise L234A/L235A/D265S mutations, wherein residue numbering is according to the EU index. In certain such embodiments, the first Ig constant region or the fragment of the first Ig constant region and the second Ig constant region or the fragment of the second Ig constant region is an IgG1 isotype.In some embodiments, the first Ig constant region or the fragment of the first Ig constant region and the second Ig constant region or the fragment of the second Ig constant region comprises at least one mutation that results in enhanced binding of the multispecific protein to a Fcy receptor (FcyR).

In some embodiments, the at least one mutation that results in enhanced binding of the multispecific protein to the FcyR is selected from the group consisting of S239D/I332E, S298A/E333A/K334A, F243L/R292P/Y300L, F243L/R292P/Y300L/P396L, F243L/R292P/Y300L/V305I/P396L, M252Y/S254T/T256E and G236A/S239D/I332E, wherein residue numbering is according to the EU index.

In certain embodiments, the first Ig constant region or the fragment of the first Ig constant region and the second Ig constant region or the fragment of the second Ig constant region comprise the M252Y/S254T/T256E mutations, wherein residue numbering is according to the EU index. In certain such embodiments, the first Ig constant region or the fragment of the first Ig constant region and the second Ig constant region or the fragment of the second Ig constant region comprise the L234A/L235A/D265S and the M252Y/S254T/T256E mutations, wherein residue numbering is according to the EU index. In certain embodiments, the first Ig constant region or the fragment of the first Ig constant region and the second Ig constant region or the fragment of the second Ig constant region is an IgG1 isotype.In some embodiments, the FcγR is FcγRI, FcγRIIA, FcγRIIB or FcγRIII, or any combination thereof.

In some embodiments, the first Ig constant region or the fragment of the first Ig constant region and the second Ig constant region or the fragment of the second Ig constant region comprises at least one mutation that modulates a half-life of the multispecific protein.

In some embodiments, the at least one mutation that modulates the half-life of the multispecific protein is selected from the group consisting of H435A, P257I/N434H, D376V/N434H, M252Y/S254T/T256E/H433K/N434F, T308P/N434A and H435R, wherein residue numbering is according to the EU index.

In some embodiments, the first Ig constant region or the fragment of the first Ig constant region and the second Ig constant region or the fragment of the second Ig constant region comprises at least one mutation that modulates binding to protein A. Such modifications may be advantageous for purification purposes during antibody production.

In certain embodiments, the at least one mutation that modulates binding to protein A is H435/Y436F, wherein residue numbering is according to the EU index. In certain such embodiments, the first Ig constant region or the fragment of the first Ig constant region and the second Ig constant region or the fragment of the second Ig constant region comprise the L234A/L235A/D265S, the M252Y/S254T/T256E and the H435/Y436F mutations, wherein residue numbering is according to the EU index. In certain embodiments, the first Ig constant region or the fragment of the first Ig constant region and the second Ig constant region or the fragment of the second Ig constant region is an IgG1 isotypein some embodiments, the multispecific protein comprises at least one mutation in a CH3 domain of the first Ig constant region or in a CH3 domain of the fragment of the first Ig constant region and/or at least one mutation in a CH3 domain of the second Ig constant region or in a CH3 domain of the fragment of the second Ig constant region.

In some embodiments, the at least one mutation in a CH3 domain of the first Ig constant region or in a CH3 domain of the fragment of the first Ig constant region and/or at least one mutation in a CH3 domain of the second Ig constant region or in a CH3 domain of the fragment of the second Ig constant region is selected from the group consisting of T350V, L351Y, F405A, Y407V, T366Y, T366W, F405W, T394W, T394S, Y407T, Y407A, T366S/L368A/Y407V, L351Y/F405A/Y407V, T366I/K392M/T394W, F405A/Y407V, T366L/K392M/T394W, L351Y/Y407A, T366A/K409F, L351Y/Y407A, T366V/K409F, T366A/K409F, T350V/L351Y/F405A/Y407V and T350V/T366L/K392L/T394W, wherein residue numbering is according to the EU index.

In some embodiments, the first Ig constant region or the fragment of the first Ig constant region and the second Ig constant region or the fragment of the second Ig constant region comprise the following mutations

L235A_L235A_D265S_T350V_L351Y_F405A Y407V in the first Ig constant region and
L235A_L235A_D265S_T350V_T366L_K392L_T394W in the second Ig constant region; or
L235A_L235A_D265S_T350V_T366L_K392L_T394W in the first Ig constant region and
L235A_L235A_D265S_T350V_L351Y_F405A_Y407V in the second Ig constant region.

**Generation of multispecific proteins that comprise antigen binding fragments that bind CD33**

The antigen binding fragments that bind CD33 of the disclosure may be engineered into multispecific antibodies which are also encompassed within the scope of the invention.

The antigen binding fragments that bind CD33 may be engineered into full length multispecific antibodies which

are generated using Fab arm exchange, in which substitutions are introduced into two monospecific bivalent antibodies within the Ig constant region CH3 domain which promote Fab arm exchange *in vitro.* In the methods, two monospecific bivalent antibodies are engineered to have certain substitutions at the CH3 domain that promote heterodimer stability; the antibodies are incubated together under reducing conditions sufficient to allow the cysteines in the hinge region to undergo disulfide bond isomerization; thereby generating the bispecific antibody by Fab arm exchange. The incubation conditions may optimally be restored to non-reducing. Exemplary reducing agents that may be used are 2- mercaptoethylamine (2-MEA), dithiothreitol (DTT), dithioerythritol (DTE), glutathione, tris(2-carboxyethyl)phosphine (TCEP), L-cysteine and beta-mercaptoethanol, preferably a reducing agent selected from the group consisting of: 2- mercaptoethylamine, dithiothreitol and tris(2-carboxyethyl)phosphine. For example, incubation for at least 90 min at a temperature of at least 20°C in the presence of at least 25 mM 2-MEA or in the presence of at least 0.5 mM dithiothreitol at a pH of from 5-8, for example at pH of 7.0 or at pH of 7.4 may be used.

CH3 mutations that may be used include technologies such as Knob-in-Hole mutations (Genentech), electrostatically-matched mutations (Chugai, Amgen, NovoNordisk, Oncomed), the Strand Exchange Engineered Domain body (SEEDbody) (EMD Serono), Duobody® mutations (Genmab), and other asymmetric mutations (e.g. Zymeworks).

Knob-in-hole mutations are disclosed for example in WO1996/027011 and include mutations on the interface of CH3 region in which an amino acid with a small side chain (hole) is introduced into the first CH3 region and an amino acid with a large side chain (knob) is introduced into the second CH3 region, resulting in preferential interaction between the first CH3 region and the second CH3 region. Exemplary CH3 region mutations forming a knob and a hole are T366Y/F405A, T366W/F405W, F405W/Y407A, T394W/Y407T, T394S/Y407A, T366W/T394S, F405W/T394S and T366W/T366S_L368A_Y407V.

Heavy chain heterodimer formation may be promoted by using electrostatic interactions by substituting positively charged residues on the first CH3 region and negatively charged residues on the second CH3 region as described in US2010/0015133, US2009/0182127, US2010/028637 or US2011/0123532.

Other asymmetric mutations that can be used to promote heavy chain heterodimerization are

L351Y_F405A_Y407V/T394W, T366I_K392M_T394W/F405A_Y407V, T366L_K392M_T394W/F405A_Y407V, L351Y_Y407A/T366A_K409F, L351Y_Y407A/T366V_K409F, Y407A/T366A_K409F, or T350V_L351Y_F405A_Y407V/T350V_T366L_K392L_T394W as described in US2012/0149876 or US2013/0195849 (Zymeworks).

SEEDbody mutations involve substituting select IgG residues with IgA residues to promote heavy chai heterodimerization as described in US20070287170.

Other exemplary mutations that may be used are R409D_K370E/D399K_E357K, S354C_T366W/Y349C_T366S_L368A_Y407V, Y349C_T366W/S354C_T366S_L368A_Y407V, T366K/L351D, L351K/Y349E, L351K/Y349D, L351K/L368E, L351Y_Y407A/T366A_K409F, L351Y_Y407A/T366V_K409F, K392D/D399K, K392D/ E356K, K253E_D282K_K322D/D239K_E240K_K292D, K392D_K409D/D356K_D399K as described in WO2007/147901, WO 2011/143545, WO2013157954, WO2013096291 and US2018/0118849.

Duobody® mutations (Genmab) are disclosed for example in US9150663 and US2014/0303356 and include mutations F405L/K409R, wild-type/F405L_R409K, T350I K370T F405L/K409R, K370W/K409R, D399AFGHILMNRSTVWY/K409R, T366ADEFGHILMQVY/K409R, L368ADEGHNRSTVQ/K409AGRH, D399FHKRQ/K409AGRH, F405IKLSTVW/K409AGRH and Y407LWQ/K409AGRH.

Additional bispecific or multispecific structures into which the antigen binding domains that bind CD33 can be incorporated include Dual Variable Domain Immunoglobulins (DVD) (Int. Pat. Publ. No. WO2009/134776; DVDs are full length antibodies comprising the heavy chain having a structure VH1-linker-VH2-CH and the light chain having the structure VL1-linker-VL2-CL; linker being optional), structures that include various dimerization domains to connect the two antibody arms with different specificity, such as leucine zipper or collagen dimerization domains (Int. Pat. Publ. No. WO2012/022811, U.S. Pat. No. 5,932,448; U.S. Pat. No. 6,833,441), two or more domain antibodies (dAbs) conjugated together, diabodies, heavy chain only antibodies such as camelid antibodies and engineered camelid antibodies, Dual Targeting (DT)-Ig (GSK/Domantis), Two-in-one Antibody (Genentech), Cross-linked Mabs (Karmanos Cancer Center), mAb2 (F-Star) and CovX-body (CovX/Pfizer), IgG-like Bispecific (InnClone/Eli Lilly), Ts2Ab (MedImmune/AZ) and BsAb (Zymogenetics), HERCULES (Biogen Idec) and TvAb (Roche), ScFv/Fc Fusions (Academic Institution), SCORPION (Emergent BioSolutions/Trubion, Zymogenetics/BMS), Dual Affinity Retargeting Technology (Fc-DART) (MacroGenics) and Dual$(ScFv)_2$-Fab (National Research Center for Antibody Medicine--China), Dual-Action or Bis-Fab (Genentech), Dock-and-Lock (DNL) (ImmunoMedics), Bivalent Bispecific (Biotecnol) and Fab-Fv (UCB-Celltech). ScFv-, diabody-based, and domain antibodies, include but are not limited to, Bispecific T Cell Engager (BiTE) (Micromet), Tandem Diabody (Tandab) (Affimed), Dual Affinity Retargeting Technology (DART) (MacroGenics), Single-chain Diabody (Academic), TCR-like Antibodies (AIT, ReceptorLogics), Human Serum Albumin ScFv Fusion (Merrimack) and COMBODY

(Epigen Biotech), dual targeting nanobodies (Ablynx), dual targeting heavy chain only domain antibodies.

The antigen binding domains that bind CD33 of the disclosure may also be engineered into multispecific proteins which comprise three polypeptide chains. In such designs, at least one antigen binding domain is in the form of a scFv. Exemplary designs include (in which "1" indicates the first antigen binding domain, "2" indicates the second antigen binding domain and "3" indicates the third antigen binding domain:

Design 1: Chain A) scFv1- CH2-CH3; Chain B) VL2-CL; Chain C) VH2-CH1-hinge-CH2-CH3
Design 2: Chain A) scFvl- hinge- CH2-CH3; Chain B) VL2-CL; Chain C) VH2-CH1-hinge-CH2-CH3
Design 3: Chain A) scFvl- CH1-hinge- CH2-CH3; Chain B) VL2-CL; Chain C) VH2-CH1-hinge-CH2-CH3
Design 4: Chain A) CH2-CH3-scFv1; Chain B) VL2-CL; Chain C) VH2-CH1-hinge-CH2-CH3 CH3 engineering may be incorporated to the Designs 1-4, such as mutations

L351Y_F405A_Y407V/T394W, T366I_K392M_T394W/F405A_Y407V, T366L_K392M_T394W/F405A_Y407V, L351Y_Y407A/T366A_K409F, L351Y_Y407A/T366V_K409F, Y407A/T366A_K409F, or T350V_L351Y_F405A_Y407V/T350V_T366L_K392L_T394W as described in US2012/0149876 or US2013/0195849 (Zymeworks).

**Isotypes, allotypes and Fc engineering**

The Ig constant region or the fragment of the Ig constant region, such as the Fc region present in the proteins of the disclosure may be of any allotype or isotype.

In some embodiments, the Ig constant region or the fragment of the Ig constant region is an IgG 1 isotype.

In some embodiments, the Ig constant region or the fragment of the Ig constant region is an IgG2 isotype.

In some embodiments, the Ig constant region or the fragment of the Ig constant region is an IgG3 isotype.

In some embodiments, the Ig constant region or the fragment of the Ig constant region is an IgG4 isotype.

The Ig constant region or the fragment of the Ig constant region may be of any allotype. It is expected that allotype has no influence on properties of the Ig constant region, such as binding or Fc-mediated effector functions. Immunogenicity of therapeutic proteins comprising Ig constant regions of fragments thereof is associated with increased risk of infusion reactions and decreased duration of therapeutic response (Baert et al., (2003) N Engl J Med 348:602-08). The extent to which therapeutic proteins comprising Ig constant regions of fragments thereof induce an immune response in the host may be determined in part by the allotype of the Ig constant region (Stickler et al., (2011) Genes and Immunity 12:213-21). Ig constant region allotype is related to amino acid sequence variations at specific locations in the constant region sequences of the antibody. Table 2 shows select IgG1, IgG2 and IgG4 allotypes.

**Table 2.**

| Allotype | Amino acid residue at position of diversity (residue numbering: EU Index) | | | | | | | |
|---|---|---|---|---|---|---|---|---|
| | IgG2 | | IgG4 | | IgG1 | | | |
| | 189 | 282 | 309 | 422 | 214 | 356 | 358 | 431 |
| G2m(n) | T | M | | | | | | |
| G2m(n-) | P | V | | | | | | |
| G2m(n)/(n-) | T | V | | | | | | |
| nG4m(a) | | | L | R | | | | |
| G1m(17) | | | | | K | E | M | A |
| G1m(17,1) | | | | | K | D | L | A |

C-terminal lysine (CTL) may be removed from the Ig constant region by endogenous circulating carboxypeptidases in the blood stream (Cai et al., (2011) Biotechnol Bioeng 108:404-412). During manufacturing, CTL removal may be controlled to less than the maximum level by control of concentration of extracellular $Zn^{2+}$, EDTA or EDTA - $Fe^{3+}$ as described in U.S. Patent Publ. No. US20140273092. CTL content of proteins may be measured using known methods.

In some embodiments, the antigen binding fragment that binds CD33 conjugated to the Ig constant region has a C-terminal lysine content from about 10% to about 90%. In some embodiments, the C-terminal lysine content is from about 20% to about 80%. In some embodiments, the C-terminal lysine content is from about 40% to about 70%. In some

embodiments, the C-terminal lysine content is from about 55% to about 70%. In some embodiments, the C-terminal lysine content is about 60%.

Fc region mutations may be made to the antigen binding domains that bind CD33 conjugated to the Ig constant region or to the fragment of the Ig constant region to modulate their effector functions such as ADCC, ADCP and/or ADCP and/or pharmacokinetic properties. This may be achieved by introducing mutation(s) into the Fc that modulate binding of the mutated Fc to activating FcyRs (FcyRI, FcyRIIa, FcyRIII), inhibitory FcyRIIb and/or to FcRn.

In some embodiments, the antigen binding domain that binds CD33s conjugated to the Ig constant region or the fragment of the Ig constant region comprises at least one mutation in the Ig constant region or in the fragment of the Ig constant region.

In some embodiments, the at least one mutation is in the Fc region.

In some embodiments, the antigen binding domain that binds CD33 conjugated to the Ig constant region or to the fragment of the Ig constant region comprises at least one, two, three, four, five, six, seven, eight, nine, ten, eleven, twelve, thirteen, fourteen or fifteen mutations in the Fc region.

In some embodiments, the antigen binding domain that binds CD33s conjugated to the Ig constant region or to the fragment of the Ig constant region comprises at least one mutation in the Fc region that modulates binding of the antibody to FcRn.

Fc positions that may be mutated to modulate half-life (e.g. binding to FcRn) include positions 250, 252, 253, 254, 256, 257, 307, 376, 380, 428, 434 and 435. Exemplary mutations that may be made singularly or in combination are mutations T250Q, M252Y, I253A, S254T, T256E, P257I, T307A, D376V, E380A, M428L, H433K, N434S, N434A, N434H, N434F, H435A and H435R. Exemplary singular or combination mutations that may be made to increase the half-life are mutations M428L/N434S, M252Y/S254T/T256E, T250Q/M428L, N434A and T307A/E380A/N434A. Exemplary singular or combination mutations that may be made to reduce the half-life are mutations H435A, P257I/N434H, D376V/N434H, M252Y/S254T/T256E/H433K/N434F, T308P/N434A and H435R.

In some embodiments, the antigen binding domain that binds CD33 conjugated to the Ig constant region or to the fragment of the Ig constant region comprises M252Y/S254T/T256E mutation.

In some embodiments, the antigen binding domain that binds CD33 conjugated to the Ig constant region or to the fragment of the Ig constant region comprises at least one mutation in the Fc region that reduces binding of the protein to an activating Fcy receptor (FcyR) and/or reduces Fc effector functions such as Clq binding, complement dependent cytotoxicity (CDC), antibody-dependent cell-mediated cytotoxicity (ADCC) or phagocytosis (ADCP).

Fc positions that may be mutated to reduce binding of the protein to the activating FcyR and subsequently to reduce effector function include positions 214, 233, 234, 235, 236, 237, 238, 265, 267, 268, 270, 295, 297, 309, 327, 328, 329, 330, 331 and 365. Exemplary mutations that may be made singularly or in combination are mutations K214T, E233P, L234V, L234A, deletion of G236, V234A, F234A, L235A, G237A, P238A, P238S, D265A, S267E, H268A, H268Q, Q268A, N297A, A327Q, P329A, D270A, Q295A, V309L, A327S, L328F, A330S and P331S in IgG1, IgG2, IgG3 or IgG4. Exemplary combination mutations that result in proteins with reduced ADCC are mutations L234A/L235A on IgG1, L234A/L235A/D265S on IgG1, V234A/G237A/ P238S/H268A/V309L/A330S/P331S on IgG2, F234A/L235A on IgG4, S228P/F234A/ L235A on IgG4, N297A on all Ig isotypes, V234A/G237A on IgG2, K214T/E233P/ L234V/L235A/G236-deleted/A327G/P331A/D365E/L358M on IgG1, H268Q/V309L/A330S/P331S on IgG2, S267E/L328F on IgG1, L234F/L235E/D265A on IgG1, L234A/L235A/G237A/P238S/H268A/A330S/P331S on IgG1, S228P/F234A/L235A/G237A/P238S on IgG4, and S228P/F234A/L235A/G236-deleted/G237A/P238S on IgG4. Hybrid IgG2/4 Fc domains may also be used, such as Fc with residues 117-260 from IgG2 and residues 261-447 from IgG4.

Exemplary mutation that result in proteins with reduced CDC is a K322A mutation.

Well-known S228P mutation may be made in IgG4 to enhance IgG4 stability.

In some embodiments, the antigen binding domain that binds CD33 conjugated to the Ig constant region or to the fragment of the Ig constant region comprises at least one mutation selected from the group consisting of K214T, E233P, L234V, L234A, deletion of G236, V234A, F234A, L235A, G237A, P238A, P238S, D265A, S267E, H268A, H268Q, Q268A, N297A, A327Q, P329A, D270A, Q295A, V309L, A327S, L328F, K322, A330S and P331S.

In some embodiments, the antigen binding domain that binds CD33 conjugated to the Ig constant region or to the fragment of the Ig constant region comprises L234A/L235A/D265S mutation.

In some embodiments, the antigen binding domain that binds CD33 conjugated to the Ig constant region or to the fragment of the Ig constant region comprises L234A/L235A mutation.

In some embodiments, the antigen binding domain that binds CD33 conjugated to the Ig constant region or to the fragment of the Ig constant region comprises at least one mutation in the Fc region that enhances binding of the protein to an Fcγ receptor (FcγR) and/or enhances Fc effector functions such as C1q binding, complement dependent cytotoxicity (CDC), antibody-dependent cell-mediated cytotoxicity (ADCC) and/or phagocytosis (ADCP).

Fc positions that may be mutated to increase binding of the protein to the activating FcyR and/or enhance Fc effector functions include positions 236, 239, 243, 256,290,292, 298, 300, 305, 312, 326, 330, 332, 333, 334, 345, 360, 339, 378, 396 or 430 (residue numbering according to the EU index). Exemplary mutations that may be made singularly

or in combination are G236A, S239D, F243L, T256A, K290A, R292P, S298A, Y300L, V305L, K326A, A330K, I332E, E333A, K334A, A339T and P396L. Exemplary combination mutations that result in proteins with increased ADCC or ADCP are a S239D/I332E, S298A/E333A/K334A, F243L/R292P/Y300L, F243L/R292P/Y300L/P396L, F243L/R292P/Y300L/V305I/P396L and G236A/S239D/I332E.

Fc positions that may be mutated to enhance CDC include positions 267, 268, 324, 326, 333, 345 and 430. Exemplary mutations that may be made singularly or in combination are S267E, F1268F, S324T, K326A, K326W, E333A, E345K, E345Q, E345R, E345Y, E430S, E430F and E430T. Exemplary combination mutations that result in proteins with increased CDC are K326A/E333A, K326W/E333A, H268F/S324T, S267E/H268F, S267E/S324T and S267E/H268F/S324T.

The specific mutations described herein are mutations when compared to the IgG1, IgG2 and IgG4 wild-type amino acid sequences of SEQ ID NOs: 222, 223 and 224, respectively.

SEQ ID NO: 222, wild-type IgG1

ASTKGPSVFPLAPSSKSTSGGTAALGCLVKDYFPEPVTVSWNSGALTSGVHTFPAVLQSSGLYSL
SSVVTVPSSSLGTQTYICNVNHKPSNTKVDKKVEPKSCDKTHTCPPCPAPELLGGPSVFLFPPKPK
DTLMISRTPEVTCVVVDVSHEDPEVKFNWYVDGVEVHNAKTKPREEQYNSTYRVVSVLTVLHQ

DWLNGKEYKCKVSNKALPAPIEKTISKAKGQPREPQVYTLPPSRDELTKNQVSLTCLVKGFYPSD
IAVEWESNGQPENNYKTTPPVLDSDGSFFLYSKLTVDKSRWQQGNVFSCSVMHEALHNHYTQK
SLSLSPGK

SEQ ID NO: 223; wild-type IgG2

ASTKGPSVFPLAPCSRSTSESTAALGCLVKDYFPEPVTVSWNSGALTSGVHTFPAVLQSSGLYSLS
SVVTVPSSNFGTQTYTCNVDHKPSNTKVDKTVERKCCVECPPCPAPPVAGPSVFLFPPKPKDTLM
ISRTPEVTCVVVDVSHEDPEVQFNWYVDGVEVHNAKTKPREEQFNSTFRVVSVLTVVHQDWLN
GKEYKCKVSNKGLPAPIEKTISKTKGQPREPQVYTLPPSREEMTKNQVSLTCLVKGFYPSDISVE
WESNGQPENNYKTTPPMLDSDGSFFLYSKLTVDKSRWQQGNVFSCSVMHEALHNHYTQKSLSL
SPGK

SEQ ID NO: 224; wild-type IgG4

ASTKGPSVFPLAPCSRSTSESTAALGCLVKDYFPEPVTVSWNSGALTSGVHTFPAVLQSSGLYSLS
SVVTVPSSSLGTKTYTCNVDHKPSNTKVDKRVESKYGPPCPSCPAPEFLGGPSVFLFPPKPKDTL
MISRTPEVTCVVVDVSQEDPEVQFNWYVDGVEVHNAKTKPREEQFNSTYRVVSVLTVLHQDWL
NGKEYKCKVSNKGLPSSIEKTISKAKGQPREPQVYTLPPSQEEMTKNQVSLTCLVKGFYPSDIAV
EWESNGQPENNYKTTPPVLDSDGSFFLYSRLTVDKSRWQEGNVFSCSVMHEALHNHYTQKSLSL
SLGK

Binding of the antibody to FcyR or FcRn may be assessed on cells engineered to express each receptor using flow cytometry. In an exemplary binding assay, $2\times10^5$ cells per well are seeded in 96-well plate and blocked in BSA Stain Buffer (BD Biosciences, San Jose, USA) for 30 min at 4°C. Cells are incubated with a test antibody on ice for 1.5 hour at 4°C. After being washed twice with BSA stain buffer, the cells are incubated with R-PE labeled anti-human IgG secondary antibody (Jackson Immunoresearch Laboratories) for 45 min at 4°C. The cells are washed twice in stain buffer and then resuspended in 150 μL of Stain Buffer containing 1:200 diluted DRAQ7 live/dead stain (Cell Signaling Technology, Danvers, USA). PE and DRAQ7 signals of the stained cells are detected by Miltenyi MACSQuant flow cytometer (Miltenyi Biotec, Auburn, USA) using B2 and B4 channel respectively. Live cells are gated on DRAQ7 exclusion and the geometric mean fluorescence signals are determined for at least 10,000 live events collected. FlowJo software (Tree Star) is used for analysis. Data is plotted as the logarithm of antibody concentration versus mean fluorescence signals. Nonlinear regression analysis is performed.

**Glycoengineering**

The ability of the antigen binding domain that binds CD33 conjugated to the Ig constant region or to the fragment of the Ig constant region to mediate ADCC can be enhanced by engineering the Ig constant region or the fragment of the Ig constant region oligosaccharide component. Human IgG1 or IgG3 are N-glycosylated at Asn297 with the majority of the glycans in the well-known biantennary G0, G0F, G1, G1F, G2 or G2F forms. Ig constant region containing proteins may be produced by non-engineered CHO cells typically have a glycan fucose content of about at least 85%. The removal of the core fucose from the biantennary complex-type oligosaccharides attached to the antigen binding domain that binds CD33 conjugated to the Ig constant region or to the fragment of the Ig constant region enhances the ADCC of the protein via improved FcγRIIIa binding without altering antigen binding or CDC activity. Such proteins can be achieved using different methods reported to lead to the successful expression of relatively high defucosylated immunoglobulins bearing the biantennary complex-type of Fc oligosaccharides such as control of culture osmolality (Konno et al., Cytotechnology 64(:249-65, 2012), application of a variant CHO line Lec13 as the host cell line (Shields et al., J Biol Chem 277:26733-26740, 2002), application of a variant CHO line EB66 as the host cell line (Olivier et al., MAbs;2(4): 405-415,2010; PMID:20562582), application of a rat hybridoma cell line YB2/0 as the host cell line (Shinkawa et al., J Biol Chem 278:3466-3473, 2003), introduction of small interfering RNA specifically against the a 1,6-fucosyltrasferase (*FUT8*) gene (Mori et al., Biotechnol Bioeng 88:901-908, 2004), or coexpression of β-1,4-*N*-acetylglucosaminyltransferase III and Golgi α-mannosidase II or a potent alpha-mannosidase I inhibitor, kifunensine (Ferrara et al., J Biol Chem 281:5032-5036, 2006, Ferrara et al., Biotechnol Bioeng 93:851-861, 2006; Xhou et al., Biotechnol Bioeng 99:652-65, 2008).

In some embodiments, the antigen binding domain that binds CD33 conjugated to the Ig constant region or to the fragment of the Ig constant region of the disclosure has a biantennary glycan structure with fucose content of about between 1% to about 15%, for example about 15%, 14%, 13%, 12%, 11% 10%, 9%, 8%, 7%, 6%, 5%, 4%, 3%, 2% or 1%. In some embodiments, the antigen binding domain that binds CD33 conjugated to the Ig constant region or to the fragment of the Ig constant region has a glycan structure with fucose content of about 50%, 40%, 45%, 40%, 35%, 30%, 25%, or 20%.

"Fucose content" means the amount of the fucose monosaccharide within the sugar chain at Asn297. The relative amount of fucose is the percentage of fucose-containing structures related to all glycostructures. These may be characterized and quantified by multiple methods, for example: 1) using MALDI-TOF of N-glycosidase F treated sample (e.g. complex, hybrid and oligo- and high-mannose structures) as described in Int Pat. Publ. No. WO2008/077546 2); 2) by enzymatic release of the Asn297 glycans with subsequent derivatization and detection/ quantitation by HPLC (UPLC) with fluorescence detection and/or HPLC-MS (UPLC-MS); 3) intact protein analysis of the native or reduced mAb, with or without treatment of the Asn297 glycans with Endo S or other enzyme that cleaves between the first and the second GlcNAc monosaccharides, leaving the fucose attached to the first GlcNAc; 4) digestion of the mAb to constituent peptides by enzymatic digestion (e.g., trypsin or endopeptidase Lys-C), and subsequent separation, detection and quantitation by HPLC-MS (UPLC-MS); 5) Separation of the mAb oligosaccharides from the mAb protein by specific enzymatic deglycosylation with PNGase F at Asn 297. The oligosaccharides thus released can be labeled with a fluorophore, separated and identified by various complementary techniques which allow: fine characterization of the glycan structures by matrix-assisted laser desorption ionization (MALDI) mass spectrometry by comparison of the experimental masses with the theoretical masses, determination of the degree of sialylation by ion exchange HPLC (GlycoSep C), separation and quantification of the oligosaccharide forms according to hydrophilicity criteria by normal-phase HPLC (GlycoSep N), and separation and quantification of the oligosaccharides by high performance capillary electrophoresis-laser induced fluorescence (HPCE-LIF).

"Low fucose" or "low fucose content" as used herein refers to the antigen binding domain that bind CD33 conjugated to the Ig constant region or to the fragment of the Ig constant region with fucose content of about between 1%-15%.

"Normal fucose" or 'normal fucose content" as used herein refers to the antigen binding domain that bind CD33 conjugated to the Ig constant region or to the fragment of the Ig constant region with fucose content of about over 50%, typically about over 80% or over 85%.

**Anti-idiotypic antibodies**

Anti-idiotypic antibodies are antibodies that specifically bind to the antigen binding domain that binds CD33 of the disclosure.
The invention also provides an anti-idiotypic antibody that specifically binds to the antigen binding domain that binds CD33 of the disclosure.
The invention also provides an anti-idiotypic antibody that specifically binds to the antigen binding domain that binds CD33 comprising

the VH of SEQ ID NO: 52 and the VL of SEQ ID NO: 81;
the VH of SEQ ID NO: 53 and the VL of SEQ ID NO: 82;
the VH of SEQ ID NO: 54 and the VL of SEQ ID NO: 83;
the VH of SEQ ID NO: 55 and the VL of SEQ ID NO: 84;
the VH of SEQ ID NO: 56 and the VL of SEQ ID NO: 85;
the VH of SEQ ID NO: 57 and the VL of SEQ ID NO: 86;
the VH of SEQ ID NO: 58 and the VL of SEQ ID NO: 87;
the VH of SEQ ID NO: 59 and the VL of SEQ ID NO: 87;
the VH of SEQ ID NO: 60 and the VL of SEQ ID NO: 87;
the VH of SEQ ID NO: 61 and the VL of SEQ ID NO: 88;
the VH of SEQ ID NO: 95 and the VL of SEQ ID NO: 105;
the VH of SEQ ID NO: 96 and the VL of SEQ ID NO: 106;
the VH of SEQ ID NO: 97 and the VL of SEQ ID NO: 107;
the VH of SEQ ID NO: 170 and the VL of SEQ ID NO: 185;
the VH of SEQ ID NO: 171 and the VL of SEQ ID NO: 188;
the VH of SEQ ID NO: 175 and the VL of SEQ ID NO: 192;
the VH of SEQ ID NO: 179 and the VL of SEQ ID NO: 196;
the VH of SEQ ID NO: 181 and the VL of SEQ ID NO: 200;
the VH of SEQ ID NO: 96 and the VL of SEQ ID NO: 202;
the VH of SEQ ID NO: 262 and the VL of SEQ ID NO: 271;
the VH of SEQ ID NO: 263 and the VL of SEQ ID NO: 272; or
the VH of SEQ ID NO: 264 and the VL of SEQ ID NO: 273.

In certain embodiments, the anti-idiotypic antibody specifically binds to an antigen binding domain that binds CD33 comprising the VH of SEQ ID NO: 53 and the VL of SEQ ID NO: 82.
In other embodiments, the anti-idiotypic antibody specifically binds to an antigen binding domain that binds CD33 comprising the VH of SEQ ID NO: 56 and the VL of SEQ ID NO: 85.
In other embodiments, the anti-idiotypic antibody specifically binds to an antigen binding domain that binds CD33 comprising the VH of SEQ ID NO: 59 and the VL of SEQ ID NO: 87.
In other embodiments, the anti-idiotypic antibody specifically binds to an antigen binding domain that binds CD33 comprising the VH of SEQ ID NO: 262 and the VL of SEQ ID NO: 271.
In other embodiments, the anti-idiotypic antibody specifically binds to an antigen binding domain that binds CD33 comprising the VH of SEQ ID NO: 263 and the VL of SEQ ID NO: 272.
In other embodiments, the anti-idiotypic antibody specifically binds to an antigen binding domain that binds CD33 comprising the VH of SEQ ID NO: 264 and the VL of SEQ ID NO: 273.
An anti-idiotypic (Id) antibody is an antibody which recognizes the antigenic determinants (e.g. the paratope or CDRs) of the antibody. The Id antibody may be antigen-blocking or non-blocking. The antigen-blocking Id may be used to detect the free antigen binding domain in a sample (e.g. the antigen binding domain that binds CD33 of the disclosure). The non-blocking Id may be used to detect the total antibody (free, partially bond to antigen, or fully bound to antigen) in a sample. An Id antibody may be prepared by immunizing an animal with the antibody to which an anti-Id is being prepared.
An anti-Id antibody may also be used as an immunogen to induce an immune response in yet another animal, producing a so-called anti-anti-Id antibody. An anti-anti-Id may be epitopically identical to the original antigen binding domain which induced the anti-Id. Thus, by using antibodies to the idiotypic determinants of the antigen binding domain, it is possible to identify other clones expressing antigen binding domains of identical specificity. Anti-Id antibodies may

be varied (thereby producing anti-Id antibody variants) and/or derivatized by any suitable technique, such as those described elsewhere herein.

**Immunoconjugates**

The antigen binding domains that bind CD33 of the disclosure, the proteins comprising the antigen binding domains that bind CD33 or the multispecific proteins that comprise the antigen binding domains that bind CD33 (collectively referred herein as to CD33 binding proteins) may be conjugated to a heterologous molecule. CD33 binding protein also includes chimeric antigen receptors (CAR) which comprises the antigen binding domains that bind CD33 of the disclosure.

In some embodiments, the heterologous molecule is a detectable label or a cytotoxic agent.

The invention also provides an antigen binding domain that binds CD33 conjugated to a detectable label.

The invention also provides a protein comprising an antigen binding domain that binds CD33 conjugated to a detectable label.

The invention also provides a multispecific protein comprising an antigen binding domain that binds CD33 conjugated to a detectable label.

The invention also provides a CAR comprising an antigen binding domain that binds CD33 conjugated to a detectable label.

The invention also provides an antigen binding domain that binds CD33 conjugated to a cytotoxic agent.

The invention also provides a protein comprising an antigen binding domain that binds CD33 conjugated to a cytotoxic agent.

The invention also provides a multispecific protein comprising an antigen binding domain that binds CD33 conjugated to a cytotoxic agent.

The invention also provides a CAR comprising an antigen binding domain that binds CD33 conjugated to a cytotoxic agent.

CD33 binding proteins of the disclosure may be used to direct therapeutics to CD33 expressing cells, such as those of a myeloid lineage. Alternatively, CD33 expressing cells may be targeted with a CD33 binding protein of the disclosure coupled to a therapeutic intended to modify cell function once internalized.

In some embodiments, the detectable label is also a cytotoxic agent.

The CD33 binding proteins of the disclosure conjugated to a detectable label may be used to evaluate expression of CD33 on a variety of samples.

Detectable label includes compositions that when conjugated to the CD33 binding proteins of the disclosure renders the latter detectable, via spectroscopic, photochemical, biochemical, immunochemical, or chemical means.

Exemplary detectable labels include radioactive isotopes, magnetic beads, metallic beads, colloidal particles, fluorescent dyes, electron-dense reagents, enzymes (for example, as commonly used in an ELISA), biotin, digoxigenin, haptens, luminescent molecules, chemiluminescent molecules, fluorochromes, fluorophores, fluorescent quenching agents, colored molecules, radioactive isotopes, scintillates, avidin, streptavidin, protein A, protein G, antibodies or fragments thereof, polyhistidine, $Ni^{2+}$, Flag tags, myc tags, heavy metals, enzymes, alkaline phosphatase, peroxidase, luciferase, electron donors/acceptors, acridinium esters, and colorimetric substrates.

Any of the labels disclosed in International Patent Publication No. WO2019/125982, incorporated by reference herein, may be used.

A detectable label may emit a signal spontaneously, such as when the detectable label is a radioactive isotope. In other cases, the detectable label emits a signal as a result of being stimulated by an external field.

Exemplary radioactive isotopes may be $\gamma$-emitting, Auger-emitting, $\beta$-emitting, an alpha-emitting or positron-emitting radioactive isotope. Exemplary radioactive isotopes include $^{3}H$, $^{11}C$, $^{13}C$, $^{15}N$, $^{18}F$, $^{19}F$, $^{55}Co$, $^{57}Co$, $^{60}Co$, $^{61}Cu$, $^{62}Cu$, $^{64}Cu$, $^{67}Cu$, $^{68}Ga$, $^{72}As$, $^{75}Br$, $^{86}Y$, $^{89}Zr$, $^{90}Sr$, $^{94m}Tc$, $^{99m}Tc$, $^{115}In$, $^{123}I$, $^{124}I$, $^{125}I$, $^{131}1$, $^{211}At$, $^{212}Bi$, $^{213}Bi$, $^{223}Ra$, $^{226}Ra$, $^{225}Ac$ and $^{227}Ac$.

In some embodiments, the radioactive isotope is used for therapeutic purposes, such as by enhancing cell killing. Radioactive isotopes used for enhancing cell killing include, but are not limited to, $^{32}P$, $^{47}Sc$, $^{67}Cu$, $^{77}As$, $^{89}Sr$, $^{90}Y$, $^{99}Tc$, $^{105}Rh$, $^{109}Pd$, $^{111}Ag$, $^{131}I$, $^{153}Sm$, $^{159}Gd$, $^{165}Dy$, $^{166}Ho$, $^{169}Er$, $^{186}Re$, $^{188}Re$, $^{194}Ir$, $^{198}Au$, $^{199}Au$, $^{211}At$, $^{212}Pb$, $^{212}Bi$, $^{213}Bi$, $^{223}Ra$, $^{255}Fm$ and $^{227}Th$. Without wishing to be bound by theory, CD33 binding proteins comprising a radioisotope used for therapeutic purposes may be internalized by the target cell and killed by the target cell.

Radioimmunotherapy using a CD33 binding protein conjugated with a radioactive isotope can be beneficial for treating local or diffuse tumors. In some embodiments, the subject is treated by administration of radiolabeled monoclonal antibodies that are directed specifically against tumor-associated antigens or against the tumor microenvironment. Radiolabeled (e.g Ac225) therapy may be highly effective requiring low doses for cell killing, such as in the context of treating hematologic malignancies with cells expressing CD33.

In some embodiments, the radioactive isotope is used for enhancing imaging, such as for testing to indicate

whether the CD33 binding protein is being targeted to tissues of interest. Exemplary isotopes used for enhancing imaging include, but are not limited to, $^{62}Cu$, $^{64}Cu$, $^{67}Ga$, $^{68}Ga$, $^{86}Y$, $^{89}Zr$, and $^{111}In$.

Exemplary metal atoms are metals with an atomic number greater than 20, such as calcium atoms, scandium atoms, titanium atoms, vanadium atoms, chromium atoms, manganese atoms, iron atoms, cobalt atoms, nickel atoms, copper atoms, zinc atoms, gallium atoms, germanium atoms, arsenic atoms, selenium atoms, bromine atoms, krypton atoms, rubidium atoms, strontium atoms, yttrium atoms, zirconium atoms, niobium atoms, molybdenum atoms, technetium atoms, ruthenium atoms, rhodium atoms, palladium atoms, silver atoms, cadmium atoms, indium atoms, tin atoms, antimony atoms, tellurium atoms, iodine atoms, xenon atoms, cesium atoms, barium atoms, lanthanum atoms, hafnium atoms, tantalum atoms, tungsten atoms, rhenium atoms, osmium atoms, iridium atoms, platinum atoms, gold atoms, mercury atoms, thallium atoms, lead atoms, bismuth atoms, francium atoms, radium atoms, actinium atoms, cerium atoms, praseodymium atoms, neodymium atoms, promethium atoms, samarium atoms, europium atoms, gadolinium atoms, terbium atoms, dysprosium atoms, holmium atoms, erbium atoms, thulium atoms, ytterbium atoms, lutetium atoms, thorium atoms, protactinium atoms, uranium atoms, neptunium atoms, plutonium atoms, americium atoms, curium atoms, berkelium atoms, californium atoms, einsteinium atoms, fermium atoms, mendelevium atoms, nobelium atoms, or lawrencium atoms.

In some embodiments, the metal atoms may be alkaline earth metals with an atomic number greater than twenty.

In some embodiments, the metal atoms may be lanthanides.

In some embodiments, the metal atoms may be actinides.

In some embodiments, the metal atoms may be transition metals.

In some embodiments, the metal atoms may be poor metals.

In some embodiments, the metal atoms may be gold atoms, bismuth atoms, tantalum atoms, and gadolinium atoms.

In some embodiments, the metal atoms may be metals with an atomic number of 53 (i.e. iodine) to 83 (i.e. bismuth).

In some embodiments, the metal atoms may be atoms suitable for magnetic resonance imaging.

The metal atoms may be metal ions in the form of +1, +2, or +3 oxidation states, such as $Ba^{2+}$, $Bi^{3+}$, $Cs^{+}$, $Ca^{2+}$, $Cr^{2+}$, $Cr^{3+}$, $Cr^{6+}$, $Co^{2+}$, $Co^{3+}$, $Cu^{+}$, $Cu^{2+}$, $Cu^{3+}$, $Ga^{3+}$, $Gd^{3+}$, $Au^{+}$, $Au^{3+}$, $Fe^{2+}$, $Fe^{3+}$, $F^{3+}$, $Pb^{2+}$, $Mn^{2+}$, $Mn^{3+}$, $Mn^{4+}$, $Mn^{7+}$, $Hg^{2+}$, $Ni^{2+}$, $Ni^{3+}$, $Ag^{+}$, $Sr^{2+}$, $Sn^{2+}$, $Sn^{4+}$, and $Zn^{2+}$. The metal atoms may comprise a metal oxide, such as iron oxide, manganese oxide, or gadolinium oxide.

Suitable dyes include any commercially available dyes such as, for example, 5(6)-carboxyfluorescein, IRDye 680RD maleimide or IRDye 800CW, ruthenium polypyridyl dyes, and the like.

Suitable fluorophores are fluorescein isothiocyanate (FITC), fluorescein thiosemicarbazide, rhodamine, Texas Red, CyDyes (e.g., Cy3, Cy5, Cy5.5), Alexa Fluors (e.g., Alexa488, Alexa555, Alexa594; Alexa647), near infrared (NIR) (700-900 nm) fluorescent dyes, and carbocyanine and aminostyryl dyes.

The antigen binding domain that binds CD33 conjugated to a detectable label may be used as an imaging agent.

The protein comprising an antigen binding domain that binds CD33 conjugated to a detectable label may be used as an imaging agent.

The multispecific protein comprising an antigen binding domain that binds CD33 conjugated to a detectable label may be used as an imaging agent.

The CAR comprising an antigen binding domain that binds CD33 conjugated to a detectable label may be used as an imaging agent.

In some embodiments, the cytotoxic agent is a chemotherapeutic agent, a drug, a growth inhibitory agent, a toxin (e.g., an enzymatically active toxin of bacterial, fungal, plant, or animal origin, or fragments thereof), or a radioactive isotope (i.e., a radioconjugate).

In some embodiments, the cytotoxic agent is daunomycin, doxorubicin, methotrexate, vindesine, bacterial toxins such as diphtheria toxin, ricin, geldanamycin, maytansinoids or calicheamicin. The cytotoxic agent may elicit their cytotoxic and cytostatic effects by mechanisms including tubulin binding, DNA binding, or topoisomerase inhibition.

In some embodiments, the cytotoxic agent is an enzymatically active toxin such as diphtheria A chain, nonbinding active fragments of diphtheria toxin, exotoxin A chain (from *Pseudomonas aeruginosa*), ricin A chain, abrin A chain, modeccin A chain, alpha-sarcin, *Aleurites fordii* proteins, dianthin proteins, *Phytolaca americana* proteins (PAPI, PAPII, and PAP-S), *momordica charantia* inhibitor, curcin, crotin, *sapaonaria officinalis* inhibitor, gelonin, mitogellin, restrictocin, phenomycin, enomycin, and the tricothecenes.

In some embodiments, the cytotoxic agent is a radionuclide, such as $^{212}Bi$, $^{131}I$, $^{131}In$, $^{90}Y$, and $^{186}Re$.

In some embodiments, the cytotoxic agent is dolastatins or dolostatin peptidic analogs and derivatives, auristatin or monomethyl auristatin phenylalanine. Exemplary molecules are disclosed in U.S. Pat No. 5,635,483 and 5,780,588. Dolastatins and auristatins have been shown to interfere with microtubule dynamics, GTP hydrolysis, and nuclear and cellular division (Woyke et al (2001) Antimicrob Agents and Chemother. 45(12):3580-3584) and have anticancer and antifungal activity. The dolastatin or auristatin drug moiety may be attached to the antibody of the invention through the

N (amino) terminus or the C (carboxyl) terminus of the peptidic drug moiety (WO02/088172), or via any cysteine engineered into the antibody.

The CD33 binding proteins of the disclosure may be conjugated to a detectable label using known methods.

In some embodiments, the detectable label is complexed with a chelating agent.

In some embodiments, the detectable label is conjugated to the CD33 binding proteins of the disclosure via a linker.

The detectable label or the cytotoxic moiety may be linked directly, or indirectly, to the CD33 binding proteins of the disclosure using known methods. Suitable linkers are known in the art and include, for example, prosthetic groups, non-phenolic linkers (derivatives of N-succimidyl-benzoates; dodecaborate), chelating moieties of both macrocyclics and acyclic chelators, such as derivatives of 1,4,7,10-tetraazacyclododecane-1,4,7,10,tetraacetic acid (DOTA), derivatives of diethylenetriaminepentaacetic avid (DTPA), derivatives of S-2-(4-Isothiocyanatobenzyl)-1,4,7-triazacyclononane-1,4,7-triacetic acid (NOTA) and derivatives of 1,4,8,11-tetraazacyclodocedan-1,4,8,11-tetraacetic acid (TETA), N-succinimidyl-3-(2-pyridyldithiol) propionate (SPDP), iminothiolane (IT), bifunctional derivatives of imidoesters (such as dimethyl adipimidate HCl), active esters (such as disuccinimidyl suberate), aldehydes (such as glutaraldehyde), bis-azido compounds (such as bis(p-azidobenzoyl)hexanediamine), bis-diazonium derivatives (such as bis-(p-diazoniumbenzoyl)-ethylenediamine), diisocyanates (such as toluene 2,6-diisocyanate), and bis-active fluorine compounds (such as 1,5-difluoro-2,4-dinitrobenzene) and other chelating moieties. Suitable peptide linkers are well known.

In some embodiments, the CD33 binding proteins of the disclosure is removed from the blood via renal clearance.

## Kits

The invention also provides a kit comprising any of the antigen binding domains that bind CD33 described herein.

The invention also provides a kit comprising the protein comprising any of the antigen binding domains that bind CD33 described herein.

The invention also provides a kit comprising any of the multispecific proteins described herein that comprise an antigen binding domain that binds CD33.

The invention also provides a kit comprising the CAR comprising any of the antigen binding domains described herein that bind CD33.

The kit may be used for therapeutic uses and as diagnostic kits.

The kit may be used to detect the presence of CD33in a sample.

In some embodiments, the kit comprises the CD33binding protein of the disclosure and reagents for detecting the CD33binding protein. The kit can include one or more other elements including: instructions for use; other reagents, e.g., a label, a therapeutic agent, or an agent useful for chelating, or otherwise coupling, an antibody to a label or therapeutic agent, or a radioprotective composition; devices or other materials for preparing the antibody for administration; pharmaceutically acceptable carriers; and devices or other materials for administration to a subject.

In some embodiments, the kit comprises the antigen binding domain that binds CD33 in a container and instructions for use of the kit.

In some embodiments, the kit comprises the protein comprising an antigen binding domain that binds CD33in a container and instructions for use of the kit.

In some embodiments, the kit comprises the multispecific protein comprising an antigen binding domain that binds CD33in a container and instructions for use of the kit.

In some embodiments, the antigen binding domain that binds CD33in the kit is labeled.

In some embodiments, the protein comprising an antigen binding domain that binds CD33in the kit is labeled.

In some embodiments, the multispecific protein comprising an antigen binding domain that binds CD33in the kit is labeled.

In some embodiments, the kit comprises the antigen binding domain (for example, an scFv) that binds CD33 comprising

a) the VH of SEQ ID NO: 52 and the VL of SEQ ID NO: 81;
b) the VH of SEQ ID NO: 53 and the VL of SEQ ID NO: 82;
c) the VH of SEQ ID NO: 54 and the VL of SEQ ID NO: 83;
d) the VH of SEQ ID NO: 55 and the VL of SEQ ID NO: 84;
e) the VH of SEQ ID NO: 56 and the VL of SEQ ID NO: 85;
f) the VH of SEQ ID NO: 57 and the VL of SEQ ID NO: 86;
g) the VH of SEQ ID NO: 58 and the VL of SEQ ID NO: 87;
h) the VH of SEQ ID NO: 59 and the VL of SEQ ID NO: 87;
i) the VH of SEQ ID NO: 60 and the VL of SEQ ID NO: 87;
j) the VH of SEQ ID NO: 61 and the VL of SEQ ID NO: 88;

k) the VH of SEQ ID NO: 262 and the VL of SEQ ID NO: 271;
l) the VH of SEQ ID NO: 263 and the VL of SEQ ID NO: 272; or
m) the VH of SEQ ID NO: 264 and the VL of SEQ ID NO: 273.

In certain embodiments, the kit comprises the antigen binding domain (for example, an scFv) that binds CD33 comprising the VH of SEQ ID NO: 53 and the VL of SEQ ID NO: 82.

In other embodiments, the kit comprises the antigen binding domain (for example, an scFv) that binds CD33 comprising the VH of SEQ ID NO: 56 and the VL of SEQ ID NO: 85.

In other embodiments, the kit comprises the antigen binding domain (for example, an scFv) that binds CD33 comprising the VH of SEQ ID NO: 59 and the VL of SEQ ID NO: 87.

In other embodiments, the kit comprises the antigen binding domain (for example, an scFv) that binds CD33 comprising the VH of SEQ ID NO: 262 and the VL of SEQ ID NO: 271.

In other embodiments, the kit comprises the antigen binding domain (for example, an scFv) that binds CD33 comprising the VH of SEQ ID NO: 263 and the VL of SEQ ID NO: 272.

In other embodiments, the kit comprises the antigen binding domain (for example, an scFv) that binds CD33 comprising the VH of SEQ ID NO: 264 and the VL of SEQ ID NO: 273.

In some embodiments, the kit comprises the antigen binding domain that binds CD33 comprising

a) the VH of SEQ ID NO: 95 and the VL of SEQ ID NO: 105;
b) the VH of SEQ ID NO: 96 and the VL of SEQ ID NO: 106;
c) the VH of SEQ ID NO: 97 and the VL of SEQ ID NO: 107;
d) the VH of SEQ ID NO: 170 and the VL of SEQ ID NO: 185;
e) the VH of SEQ ID NO: 171 and the VL of SEQ ID NO: 188;
f) the VH of SEQ ID NO: 175 and the VL of SEQ ID NO: 192;
g) the VH of SEQ ID NO: 179 and the VL of SEQ ID NO: 196;
h) the VH of SEQ ID NO: 181 and the VL of SEQ ID NO: 200; or
i) the VH of SEQ ID NO: 96 and the VL of SEQ ID NO: 202.

In some embodiments, the kit comprises the antigen binding domain that binds CD33 comprising

a) the VH of SEQ ID NO: 18;
b) the VH of SEQ ID NO: 19;
c) the VH of SEQ ID NO: 20;
d) the VH of SEQ ID NO: 21;
e) the VH of SEQ ID NO: 22;
f) the VH of SEQ ID NO: 23;
g) the VH of SEQ ID NO: 24;
h) the VH of SEQ ID NO: 25; or
i) the VH of SEQ ID NO: 26; or
j) the VH of SEQ ID NO: 27.

In some embodiments, the kit comprises the antigen binding domain that binds CD33 comprising SEQ ID NOs: 18, 19, 20, 21, 22, 23, 24, 25, 26, or 27. In some embodiments, the kit comprises the antigen binding domain that binds CD33 comprising SEQ ID NOs: 52, 53, 54, 55, 56, 57, 58, 59, 60, 61, 262, 263 or 264. In some embodiments, the kit comprises the antigen binding domain that binds CD33 comprising SEQ ID NOs: 81, 82, 83, 84, 85, 86, 87, 88, 271, 272 or 273. In some embodiments, the kit comprises the antigen binding domain that binds CD33 comprising SEQ ID NOs: 95, 96, 97, 170, 171, 175, 179, 181, or 96. In some embodiments, the kit comprises the antigen binding domain that binds CD33 comprising SEQ ID NOs: 105, 106, 107, 185, 188, 192, 196, 200, or 202.

In some embodiments, the kit comprises the antigen binding domain that binds CD33 comprising SEQ ID NOs: 203, 204, 205, 206, 207, 208, 209, 210, 211, 212, 213, 214, 215, 216, 217, 218, 219, 220 221, 274, 275 or 276.

In certain embodiments, the kit comprises the antigen binding domain that binds CD33 comprising SEQ ID NO: 213.

In other embodiments, the kit comprises the antigen binding domain that binds CD33 comprising SEQ ID NO: 216.

In other embodiments, the kit comprises the antigen binding domain that binds CD33 comprising SEQ ID NO: 219.

In other embodiments, the kit comprises the antigen binding domain that binds CD33 comprising SEQ ID NO: 274.

In other embodiments, the kit comprises the antigen binding domain that binds CD33 comprising SEQ ID NO: 275.

In other embodiments, the kit comprises the antigen binding domain that binds CD33 comprising SEQ ID NO: 276.

## Methods of detecting CD33

The invention also provides a method of detecting CD33 in a sample, comprising obtaining the sample, contacting the sample with the antigen binding domain that binds CD33 of the disclosure and detecting the bound CD33 in the sample.

In some embodiments, the sample may be derived from urine, blood, serum, plasma, saliva, ascites, circulating cells, synovial fluid, circulating cells, cells that are not tissue associated (*i.e.,* free cells), tissues (*e.g.*, surgically resected tissue, biopsies, including fine needle aspiration), histological preparations, and the like.

The antigen binding domain that binds CD33 of the disclosure may be detected using known methods. Exemplary methods include direct labeling of the antibodies using fluorescent or chemiluminescent labels, or radiolabels, or attaching to the antibodies of the invention a moiety which is readily detectable, such as biotin, enzymes or epitope tags. Exemplary labels and moieties are ruthenium, $^{111}$In-DOTA, $^{111}$In- diethylenetriaminepentaacetic acid (DTPA), horseradish peroxidase, alkaline phosphatase and beta-galactosidase, poly-histidine (HIS tag), acridine dyes, cyanine dyes, fluorone dyes, oxazin dyes, phenanthridine dyes, rhodamine dyes and Alexafluor® dyes.

The antibodies can be directly labeled with any of the radiolabels and radioactive agents disclosed in International Patent Publication No. WO2019/125982, incorporated by reference herein, may be used.

The antigen binding domain that binds CD33 of the disclosure may be used in a variety of assays to detect CD33 in the sample. Exemplary assays are western blot analysis, radioimmunoassay, surface plasmon resonance, immunoprecipitation, equilibrium dialysis, immunodiffusion, electrochemiluminescence (ECL) immunoassay, immunohistochemistry, fluorescence-activated cell sorting (FACS) or ELISA assay.

## Chimeric antigen receptors (CAR) comprising the antigen binding domains that bind CD33 of the disclosure

Any of the antigen binding domains that bind CD33 identified herein may be engineered into a chimeric antigen receptor (CAR) to provide CARs that bind CD33. The disclosure provides for CARs that target CD33, cells comprising such CARs, and methods of treating CD33 producing cancer, such as hematologic cancer, using the CARs described herein.

The CARs of the invention have antigen specificity for CD33. The phrases "have antigen specificity" and "elicit antigen-specific response" as used herein mean that the CAR can specifically bind to and immunologically recognize an antigen, such that binding of the CAR to the CD33 antigen elicits an immune response. Methods of testing the CARs for antigen specificity and for the ability to recognize target cells are known in the art.

The disclosure relates to the use of T cells which have been genetically modified to stably express a desired chimeric antigen receptor. A chimeric antigen receptor (CAR) is an artificially constructed heterologous protein or polypeptide containing an antigen binding domain of an antibody (such as scFv) linked to a T-cell signaling domain. Characteristics of CARs include their ability to redirect T-cell specificity and reactivity toward a selected target in a non-MHC-restricted manner, exploiting the antigen-binding properties of monoclonal antibodies. The non-MHC-restricted antigen recognition gives T cells expressing CARs the ability to recognize antigens independent of antigen processing, thus bypassing a major mechanism of tumor evasion. Moreover, when expressed in T-cells, CARs advantageously do not dimerize with endogenous T cell receptor (TCR) alpha and beta chains.

The CARs described herein provide recombinant polypeptide constructs comprising at least an extracellular antigen binding domain, a transmembrane domain and an intracellular signaling domain (also referred to herein as a cytoplasmic signaling domain) comprising a functional signaling domain derived from a stimulatory molecule as described herein. T cells expressing a CAR are referred to herein as CAR T cells, CAR-T cells or CAR modified T cells, and these terms are used interchangeably herein. The cell can be genetically modified to stably express an antibody binding domain on its surface, conferring novel antigen specificity that is MHC independent.

In some instances, the T cell is genetically modified to stably express a CAR that combines an antigen recognition domain of an antibody with an intracellular domain of the CD3-zeta chain or FcγRI protein into a single chimeric protein. In one embodiment, the stimulatory molecule is the zeta chain associated with the T cell receptor complex.

An "**intracellular signaling domain**," refers to an intracellular portion of a molecule. It is the functional portion of the protein which acts by transmitting information within the cell to regulate cellular activity via defined signaling pathways by generating second messengers or functioning as effectors by responding to such messengers. The intracellular signaling domain generates a signal that promotes an immune effector function of the CAR containing cell, e.g., a CAR-T cell. Examples of immune effector function, e.g., in a CAR-T cell, include cytolytic activity and helper activity,

including the secretion of cytokines.

In one embodiment, the intracellular signaling domain comprises a primary intracellular signaling domain. Exemplary primary intracellular signaling domains include those derived from the molecules responsible for primary stimulation, or antigen dependent simulation. In one embodiment, the intracellular signaling domain comprises a co-stimulatory intracellular domain. Exemplary co-stimulatory intracellular signaling domains include those derived from molecules responsible for co-stimulatory signals, or antigen independent stimulation. F or example, in the case of a CAR-T, a primary intracellular signaling domain comprises a cytoplasmic sequence of a T cell receptor, and a co-stimulatory intracellular signaling domain comprises a cytoplasmic sequence from co-receptor or co-stimulatory molecule.

A primary intracellular signaling domain comprises a signaling motif which is known as an immunoreceptor tyrosine-based activation motif or ITAM. Exemplary ITAM containing primary cytoplasmic signaling sequences include those derived from CD3-zeta, FcRγ, FcR beta, CD3γ, CD3δ, CD3ε, CD5, CD22, CD79a, CD79b, CD66d, DAP10 and DAP12.

The term "zeta" or alternatively "zeta chain", "CD3-zeta" or "TCR-zeta" is defined as the protein provided as GenBank Acc. No. BAG36664.1, or the equivalent residues from a nonhuman species, e.g., murine, rabbit, primate, mouse, rodent, monkey, ape and the like, and a "zeta stimulatory domain" or alternatively a "CD3-zeta stimulatory domain" or a "TCR-zeta stimulatory domain" is defined as the amino acid residues from the cytoplasmic domain of the zeta chain that are sufficient to functionally transmit an initial signal necessary for T cell activation. In one aspect, the cytoplasmic domain of zeta comprises residues 52 through 164 of GenBank Acc. No. BAG36664.1 or the equivalent residues from a non-human species, e.g., mouse, rodent, monkey, ape and the like, that are functional orthologs thereof. In one aspect, the "zeta stimulatory domain" or a "CD3-zeta stimulatory domain" is the sequence provided as SEQ ID NO: 28, or a sequence having at least 50%, at least 55%, at least 60%, at least 65%, at least 70%, at least 75%, at least 80%, at least 85%, at least 90%, at least 95%, at least 98% or at least 99%, sequence identity with SEQ ID NO: 164.

SEQ ID NO: 164 CD3-zeta stimulatory domain

RVKFSRSADAPAYKQGQNQLYNELNLGRREEYDVLDKRRGRDPEMGGKPRRKNPQEGLYNEL
QKDKMAEAYSEIGMKGERRRGKGHDGLYQGLSTATKDTYDALHMQALPPR

The term "co-stimulatory molecule" refers to the cognate binding partner on a T cell that specifically binds with a co-stimulatory ligand, thereby mediating a co-stimulatory response by the T cell, such as, but not limited to, proliferation. Co-stimulatory molecules are cell surface molecules other than antigen receptors or their ligands that are required for an efficient immune response. Co-stimulatory molecules include, but are not limited to an MHC class 1 molecule, BTLA and a Toll ligand receptor, as well as OX40, CD2, CD27, CD28, CDS, ICAM-1, LFA-1 (CD11a/CD18) and 4-1BB (CD137).

A co-stimulatory intracellular signaling domain can be the intracellular portion of a co-stimulatory molecule. A co-stimulatory molecule can be represented in the following protein families: TNF receptor proteins, Immunoglobulin-like proteins, cytokine receptors, integrins, signaling lymphocytic activation molecules (SLAM proteins), and activating NK cell receptors. Exemplary such molecules include CD27, CD28, 4-1BB (CD137), OX40, GITR, CD30, MyD88, CD40, ICOS, BAFFR, HVEM, lymphocyte function-associated antigen-1 (LFA-1), CD2, CD7, LIGHT, NKG2C, SLAMF7, NKp80, CD160, B7-H3, and a ligand that specifically binds with CD83, and the like.

The intracellular signaling domain can comprise the entire intracellular portion, or the entire native intracellular signaling domain, of the molecule from which it is derived, or a functional fragment thereof.

The term "4-1BB" or alternatively "CD137" refers to a member of the TNFR superfamily with an amino acid sequence provided as GenBank Acc. No. AAA62478.2, or the equivalent residues from a non-human species, e.g., mouse, rodent, monkey, ape and the like; and a "4-1BB co-stimulatory domain" is defined as amino acid residues 214-255 of GenBank accession no. AAA62478.2, or the equivalent residues from a non-human species, e.g., mouse, rodent, monkey, ape and the like. In one aspect, the "4-1BB co-stimulatory domain" or "CD137 co-stimulatory domain" is the sequence provided as **SEQ ID NO: 163** (KRGRKKLLYIFKQPFMRPVQTTQEEDGCSCRFPEEEEGGCEL; **SEQ ID NO: 163**) or the equivalent residues from a non-human species, e.g., mouse, rodent, monkey, ape and the like, or a sequence having at least 50%, at least 55%, at least 60%, at least 65%, at least 70%, at least 75%, at least 80%, at least 85%, at least 90%, at least 95%, at least 98% or at least 99% sequence identity with SEQ ID NO: 163.

In one embodiment, a transmembrane domain that is naturally associated with one of the other domains in the CAR is used. In another embodiment, the transmembrane domain can be selected or modified by amino acid substitution to avoid binding of such domains to the transmembrane domains of the same or different surface membrane proteins to minimize interactions with other members of the receptor complex. In one embodiment, the transmembrane domain comprises the CD8α hinge domain.

In some embodiments, the cytoplasmic signaling domain further comprises one or more functional signaling domains derived from at least one co-stimulatory molecule as defined herein. In one embodiment, the co-stimulatory molecule is chosen from 4-1BB (*i.e.,* CD137), CD27, CD3-zeta and/or CD28. CD28 is a T cell marker important in T cell co-stimulation. CD27 is a member of the tumor necrosis factor receptor superfamily and acts as a co-stimulatory immune checkpoint molecule. 4-1BB transmits a potent co-stimulatory signal to T cells, promoting differentiation and enhancing long-term survival of T lymphocytes. CD3-zeta associates with TCRs to produce a signal and contains immunoreceptor tyrosine-based activation motifs (ITAMs). In another embodiment, the co-stimulatory molecule is MyD88 or CD40.

In one embodiment, the CAR comprises an intracellular hinge domain comprising CD8 and an intracellular T cell receptor signaling domain comprising CD28, 4-1BB, and CD3-zeta. In another embodiment, the CAR comprises an intracellular hinge domain and an intracellular T cell receptor signaling domain comprising CD28, 4-1BB, and CD3-zeta, wherein the hinge domain comprises all or part of the extracellular region of CD8, CD4 or CD28; all or part of an antibody constant region; all or part of the FcγRIIIA receptor, an IgG hinge, an IgM hinge, an IgA hinge, an IgD hinge, an IgE hinge, or an Ig hinge. The IgG hinge may be from IgG1, IgG2, IgG3, IgG4, IgM1, IgM2, IgA1, IgA2, IgD, IgE, or a chimera thereof.

CARs described herein provide recombinant polypeptide constructs comprising at least an extracellular antigen binding domain, a transmembrane domain and an intracellular signaling domain (also referred to herein as "a cytoplasmic signaling domain") comprising, e.g., a functional signaling domain derived from a stimulatory molecule as defined below

In one embodiment, the CAR comprises a chimeric fusion protein comprising an extracellular antigen recognition domain, a transmembrane domain and an intracellular signaling domain comprising a functional signaling domain derived from a stimulatory molecule. In one embodiment, the CAR comprises a chimeric fusion protein comprising an extracellular antigen recognition domain, a transmembrane domain and an intracellular signaling domain comprising a functional signaling domain derived from a co-stimulatory molecule and a functional signaling domain derived from a stimulatory molecule. In one embodiment, the CAR comprises a chimeric fusion protein comprising an extracellular antigen recognition domain, a transmembrane domain and an intracellular signaling domain comprising at least two functional signaling domains derived from one or more co-stimulatory molecule(s) and a functional signaling domain derived from a stimulatory molecule.

The CARs of the disclosure can be designed to comprise the CD28 and/or 4-1BB signaling domain by itself or be combined with any other desired cytoplasmic domain(s) useful in the context of the CARs of the invention. In one embodiment, the cytoplasmic domain of the CAR can further comprise the signaling domain of CD3-zeta. For example, the cytoplasmic domain of the CAR can include but is not limited to CD3-zeta, 4-1BB and CD28 signaling modules and combinations thereof. Accordingly, the invention provides CAR T cells and methods of their use for adoptive therapy.

The disclosure further provides variants, e.g., functional variants, of the CARs. The term "functional variant" as used herein refers to a CAR having substantial or significant sequence identity or similarity to a parent CAR, which functional variant retains the biological activity of the CAR for which it is a variant. Functional variants encompass, e.g., those variants of the CAR that retain the ability to recognize target cells to a similar extent, the same extent, or to a higher extent, as the parent CAR. In reference to the parent CAR, the functional variant can, for example, be at least about 30%, about 40%, about 50%, about 60%, about 75%, about 80%, about 85%, about 90%, about 91%, about 92%, about 93%, about 94%, about 95%, about 96%, about 97%, about 98%, about 99% or more identical in amino acid sequence to the parent CAR.

A functional variant can, for example, comprise the amino acid sequence of the parent CAR with at least one conservative amino acid substitution. In one embodiment, the functional variant comprises the amino acid sequence of the parent CAR with at least one non-conservative amino acid substitution. In this case, the non-conservative amino acid substitution may not interfere with or inhibit the biological activity of the functional variant. The non-conservative amino acid substitution may enhance the biological activity of the functional variant such that the biological activity of the functional variant is increased as compared to the parent CAR.

Conservative amino acid substitutions are known in the art, and described herein.

The CAR of the disclosure can consist essentially of the specified amino acid sequence or sequences described herein, such that other components e.g., other amino acids, do not materially change the biological activity of the functional variant.

The CARs of the disclosure (including functional portions and functional variants) can be of any length, i. e., can comprise any number of amino acids, provided that the CARs (or functional portions or functional variants thereof) retain their biological activity, e.g., the ability to specifically bind to an antigen, detect diseased cells (e.g., cancer cells) in a host, or treat or prevent disease in a host, etc. For example, the CARs can be about 50 to about 5000 amino acids long, such as about 50, about 70, about 75, about 100, about 125, about 150, about 175, about 200, about 225, about 250, about 275, about 300, about 325, about 350, about 375, about 400, about 425, about 450, about 475, about 500, about 525, about 550, about 575, about 600, about 625, about 650, about 675, about 700, about 725, about 750, about 775, about 800, about 825, about 850, about 875, about 900, about 925, about 950, about 975, about 1000 or more amino acids in length.

The CARs of the disclosure (including functional portions and functional variants of the invention) may comprise synthetic amino acids in place of one or more naturally-occurring amino acids. Such synthetic amino acids are known in the art, and include, for example, aminocyclohexane carboxylic acid, norleucine, α-amino n-decanoic acid, homoserine, S-acetylaminomethyl-cysteine, trans-3- and trans-4-hydroxyproline, 4-aminophenylalanine, 4-nitrophenylalanine, α-(2-amino-2-norbornane)-carboxylic acid, α,γ-diaminobutyric acid, α,β-diaminopropionic acid, homophenylalanine, 4-chlorophenylalanine, 4-carboxyphenylalanine, β-phenylserine β-hydroxyphenylalanine, phenylglycine, α-naphthylalanine, cyclohexylalanine, cyclohexylglycine, N'-benzyl-N'-methyl-lysine, N',N'-dibenzyl-lysine, 6-hydroxylysine, ornithine, α-aminocyclopentane carboxylic acid, α-aminocyclohexane carboxylic acid, α-aminocycloheptane carboxylic acid, indoline-2-carboxylic acid, 1,2,3,4-tetrahydroisoquinoline-3-carboxylic acid, aminomalonic acid, aminomalonic acid monoamide, and α-tert-butylglycine.

The CARs of the disclosure (including functional portions and functional variants) can be subject to post-translational modifications. They can be glycosylated, esterified, N-acylated, amidated, carboxylated, phosphorylated, esterified, cyclized via, e.g., a disulfide bridge, or converted into an acid addition salt. In some embodiments, they are dimerized or polymerized, or conjugated.

The CARs of the disclosure (including functional portions and functional variants thereof) can be obtained by methods known in the art. Suitable methods of *de novo* synthesizing polypeptides and proteins are described in references, such as Chan et al., Fmoc Solid Phase Peptide Synthesis, Oxford University Press, Oxford, United Kingdom, 2000; Peptide and Protein Drug Analysis, ed. Reid, R., Marcel Dekker, Inc.,2000; and Epitope Mapping, ed. Westwood et al., Oxford University Press, Oxford, United Kingdom, 2001. Also, the CARs can be recombinantly produced using the nucleic acids described herein using standard recombinant methods. Further, some of the CARs (including functional portions and functional variants thereof) can be isolated and/or purified from a source, such as a plant, a bacterium, an insect, a mammal, etc. Methods of isolation and purification are known in the art. Alternatively, the CARs, of the disclosure (including functional portions and functional variants thereof) can be commercially synthesized. In this respect, the CARs, polypeptides, and proteins can be synthetic, recombinant, isolated, and/or purified.

The disclosure also provides an antibody or an antigen binding fragment thereof that binds to an epitope of the CAR of the disclosure. The antibody or the antigen binding fragment thereof may have any level of affinity or avidity for the functional portion of the CAR. In some embodiments, the antibody or the antigen binding fragment may bind CD33 with a range of affinities ($K_D$). In one embodiment the antibody binds to CD33 with the $K_D$ equal to or less than about $10^{-7}$ M, such as but not limited to, 1-9.9 (or any range or value therein, such as 1, 2, 3, 4, 5, 6, 7, 8, or 9)$\times 10^{-8}$ M, $10^{-9}$ M, $10^{-10}$ M, $10^{-11}$ M, $10^{-12}$ M, $10^{-11}$ M, $10^{-14}$ M, $10^{-15}$ M or any range or value therein, as determined by surface plasmon resonance or the Kinexa method, as practiced by those of skill in the art. One exemplary affinity is equal to or less than $1\times 10^{-8}$ M. Another exemplary affinity is equal to or less than $1\times 10^{-9}$ M.

Methods of testing antibodies for their ability to bind to any functional portion of the CARs of the disclosure are known in the art and include any antibody-antigen binding assay, such as, for example, radioimmunoassay (RIA), Western blot, enzyme-linked immunosorbent assay (ELISA), immunoprecipitation, and competitive inhibition assays.

The portion of the CAR comprising an antibody or antibody fragment thereof may exist in a variety of forms where the antigen binding domain is expressed as part of a contiguous polypeptide chain including, for example, a single domain antibody fragment (sdAb), a scFv and a human chimeric or humanized antibody (Harlow et al., 1999, In: Using Antibodies: A Laboratory Manual, Cold Spring Harbor Laboratory Press, N.Y.; Harlow et al., 1989, In: Antibodies: A Laboratory Manual, Cold Spring Harbor, N.Y.; Houston et al., 1988, Proc. Natl. Acad. Sci. USA 85:5879-5883; Bird et al., 1988, Science 242:423-426). In one aspect, the antigen binding domain of a CAR of the disclosure comprises an antibody fragment. In one embodiment, the CAR of the disclosure comprises an antibody fragment that comprises a scFv.

The disclosure also provides a CAR comprising an extracellular antigen-binding domain, a transmembrane domain and an intracellular signaling domain, wherein the extracellular antigen binding domain binds CD33.

The disclosure also provides a CAR comprising

an extracellular domain comprising an antigen binding domain that binds CD33;
a transmembrane domain; and
an intracellular signaling domain optionally comprising at least one co-stimulatory domain.

In some embodiments, the CAR further comprise a CD8a-hinge region.

In some embodiments, the CAR comprises a CD8a transmembrane region (CD8a-TM) polypeptide and the intracellular signaling domain comprising a co-stimulatory domain comprising a TNF receptor superfamily member 9 (CD137) component and a primary signaling domain comprising a T-cell surface glycoprotein CD3 zeta chain (CD3z) component.

In some embodiments, the CAR comprises

the CD8a-hinge region comprising an amino acid sequence that is at least 90% identical to SEQ ID NO: 157;

the transmembrane domain comprising an amino acid sequence that is at least 90% identical to SEQ ID NO: 162; and/or

the intracellular signaling domain comprising a co-stimulatory domain having an amino acid sequence that is at least 90% identical to SEQ ID NO: 163, and a primary signaling domain having an amino acid sequence that is at least 90% identical to SEQ ID NO: 164.

The disclosure also provides a CAR comprising an extracellular antigen binding domain, a transmembrane domain and an intracellular signaling domain, wherein the extracellular antigen binding domain binds CD33 and comprises:

the LCDR1, the LCDR2 and the LCDR3 of the VL of SEQ ID NO: 81 and the HCDR1, the HCDR2 and the HCDR3 of the VH of SEQ ID NO: 52;

the LCDR1, the LCDR2 and the LCDR3 of the VL of SEQ ID NO: 82 and the HCDR1, the HCDR2 and the HCDR3 of the VH of SEQ ID NO: 53;

the LCDR1, the LCDR2 and the LCDR3 of the VL of SEQ ID NO: 83 and the HCDR1, the HCDR2 and the HCDR3 of the VH of SEQ ID NO: 54;

the LCDR1, the LCDR2 and the LCDR3 of the VL of SEQ ID NO: 84 and the HCDR1, the HCDR2 and the HCDR3 of the VH of SEQ ID NO: 55;

the LCDR1, the LCDR2 and the LCDR3 of the VL of SEQ ID NO: 85 and the HCDR1, the HCDR2 and the HCDR3 of the VH of SEQ ID NO: 56;

the LCDR1, the LCDR2 and the LCDR3 of the VL of SEQ ID NO: 86 and the HCDR1, the HCDR2 and the HCDR3 of the VH of SEQ ID NO: 57;

the LCDR1, the LCDR2 and the LCDR3 of the VL of SEQ ID NO: 87 and the HCDR1, the HCDR2 and the HCDR3 of the VH of SEQ ID NO: 58;

the LCDR1, the LCDR2 and the LCDR3 of the VL of SEQ ID NO: 87 and the HCDR1, the HCDR2 and the HCDR3 of the VH of SEQ ID NO: 59;

the LCDR1, the LCDR2 and the LCDR3 of the VL of SEQ ID NO: 87 and the HCDR1, the HCDR2 and the HCDR3 of the VH of SEQ ID NO: 60;

the LCDR1, the LCDR2 and the LCDR3 of the VL of SEQ ID NO: 88 and the HCDR1, the HCDR2 and the HCDR3 of the VH of SEQ ID NO: 61;

the LCDR1, the LCDR2 and the LCDR3 of the VL of SEQ ID NO: 105 and the HCDR1, the HCDR2 and the HCDR3 of the VH of SEQ ID NO: 95;

the LCDR1, the LCDR2 and the LCDR3 of the VL of SEQ ID NO: 106 and the HCDR1, the HCDR2 and the HCDR3 of the VH of SEQ ID NO: 96;

the LCDR1, the LCDR2 and the LCDR3 of the VL of SEQ ID NO: 107 and the HCDR1, the HCDR2 and the HCDR3 of the VH of SEQ ID NO: 97;

the LCDR1, the LCDR2 and the LCDR3 of the VL of SEQ ID NO: 185 and the HCDR1, the HCDR2 and the HCDR3 of the VH of SEQ ID NO: 170;

the LCDR1, the LCDR2 and the LCDR3 of the VL of SEQ ID NO: 188 and the HCDR1, the HCDR2 and the HCDR3 of the VH of SEQ ID NO: 171;

the LCDR1, the LCDR2 and the LCDR3 of the VL of SEQ ID NO: 192 and the HCDR1, the HCDR2 and the HCDR3 of the VH of SEQ ID NO: 175;

the LCDR1, the LCDR2 and the LCDR3 of the VL of SEQ ID NO: 196 and the HCDR1, the HCDR2 and the HCDR3 of the VH of SEQ ID NO: 179;

the LCDR1, the LCDR2 and the LCDR3 of the VL of SEQ ID NO: 200 and the HCDR1, the HCDR2 and the HCDR3 of the VH of SEQ ID NO: 181;

the LCDR1, the LCDR2 and the LCDR3 of the VL of SEQ ID NO: 202 and the HCDR1, the HCDR2 and the HCDR3 of the VH of SEQ ID NO: 96;

the LCDR1, the LCDR2 and the LCDR3 of the VL of SEQ ID NO: 271 and the HCDR1, the HCDR2 and the HCDR3 of the VH of SEQ ID NO: 262;

the LCDR1, the LCDR2 and the LCDR3 of the VL of SEQ ID NO: 272 and the HCDR1, the HCDR2 and the HCDR3 of the VH of SEQ ID NO: 263; or

the LCDR1, the LCDR2 and the LCDR3 of the VL of SEQ ID NO: 273 and the HCDR1, the HCDR2 and the HCDR3 of the VH of SEQ ID NO: 264.

In certain embodiments, the CAR comprises an extracellular antigen binding domain, a transmembrane domain and an intracellular signaling domain, wherein the extracellular antigen binding domain binds CD33 and comprises the LCDR1, the LCDR2 and the LCDR3 of the VL of SEQ ID NO: 82 and the HCDR1, the HCDR2 and the HCDR3 of the

VH of SEQ ID NO: 53.

In certain embodiments, the CAR comprises an extracellular antigen binding domain, a transmembrane domain and an intracellular signaling domain, wherein the extracellular antigen binding domain binds CD33 and comprises the the LCDR1, the LCDR2 and the LCDR3 of the VL of SEQ ID NO: 85 and the HCDR1, the HCDR2 and the HCDR3 of the VH of SEQ ID NO: 56.

In certain embodiments, the CAR comprises an extracellular antigen binding domain, a transmembrane domain and an intracellular signaling domain, wherein the extracellular antigen binding domain binds CD33 and comprises the LCDR1, the LCDR2 and the LCDR3 of the VL of SEQ ID NO: 87 and the HCDR1, the HCDR2 and the HCDR3 of the VH of SEQ ID NO: 59.

In certain embodiments, the CAR comprises an extracellular antigen binding domain, a transmembrane domain and an intracellular signaling domain, wherein the extracellular antigen binding domain binds CD33 and comprises the LCDR1, the LCDR2 and the LCDR3 of the VL of SEQ ID NO: 271 and the HCDR1, the HCDR2 and the HCDR3 of the VH of SEQ ID NO: 262.

In certain embodiments, the CAR comprises an extracellular antigen binding domain, a transmembrane domain and an intracellular signaling domain, wherein the extracellular antigen binding domain binds CD33 and comprises the the LCDR1, the LCDR2 and the LCDR3 of the VL of SEQ ID NO: 272 and the HCDR1, the HCDR2 and the HCDR3 of the VH of SEQ ID NO: 263.

In certain embodiments, the CAR comprises an extracellular antigen binding domain, a transmembrane domain and an intracellular signaling domain, wherein the extracellular antigen binding domain binds CD33 and comprises the LCDR1, the LCDR1, the LCDR2 and the LCDR3 of the VL of SEQ ID NO: 273 and the HCDR1, the HCDR2 and the HCDR3 of the VH of SEQ ID NO: 264.

Exemplary HCDR and LCDR sequences are described in Tables 4a, 4b, 4c, 4d, 4e and 4f.

In some embodiments, the CDRs are defined according to Kabat.

In some embodiments, the CDRs are defined according to Chothia.

In some embodiments, the CDRs are defined according to IMGT.

In some embodiments, the CDRs are defined according to AbM.

In some embodiment, the CDRs are defined according to Contact.

The disclosure also provides a CAR comprising an extracellular antigen binding domain that binds CD33, a transmembrane domain and an intracellular signaling domain, wherein the extracellular antigen binding domain that binds CD33 comprises the HCDR1 of SEQ ID NOs: 28, 29, 30, 31, 32, 33, 34, 35, 253, 254 or 255, the HCDR2 of SEQ ID NOs: 36, 37, 38, 39, 40, 41, 42, 43, 44, 256, 257 or 258, the HCDR3 of SEQ ID NOs: 45, 46, 47, 48, 49, 50, 51, 259, 260 or 261 the LCDR1 of SEQ ID NOs: 62, 63, 64, 65, 66, 67, 68, 265 or 266, the LCDR2 of SEQ ID NOs: 69, 70, 71, 72, 73, 74, 267 or 268. and the LCDR3 of SEQ ID NOs: 75, 76, 77, 78, 79, 80, 269 or 270.

The disclosure also provides a CAR comprising an extracellular antigen binding domain that binds CD33, a transmembrane domain and an intracellular signaling domain, wherein the extracellular antigen binding domain that binds CD33 comprises the HCDR1 of SEQ ID NOs: 33, 89, 167, 172 or 176, the HCDR2 of SEQ ID NOs: 90, 91, 168, 173, or 177, the HCDR3 of SEQ ID NOs: 92, 93, 94, 169, 174, 178, or 180, the LCDR1 of SEQ ID NOs: 98, 99, 100, 182, 186, 189, 193, 197, or 201, the LCDR2 of SEQ ID NOs: 101, 102, 103, 183, 187, 190, 194, or 198, and the LCDR3 of SEQ ID NOs: 104, 184, 191, 195, or 199.

The disclosure also provides a CAR comprising an extracellular antigen binding domain that binds CD33, a transmembrane domain and an intracellular signaling domain, wherein the extracellular antigen binding domain that binds CD33 comprises the HCDR1 of SEQ ID NOs: 28, 29, 30, 31, 32, 33, 34, 35, 253, 254 or 255, or conservative substitutions thereof.

The disclosure also provides a CAR comprising an extracellular antigen binding domain that binds CD33, a transmembrane domain and an intracellular signaling domain, wherein the extracellular antigen binding domain that binds CD33 comprises the HCDR1 of SEQ ID NOs: 33, 89, 167, 172 or 176, or conservative substitutions thereof.

The disclosure also provides a CAR comprising an extracellular antigen binding domain that binds CD33, a transmembrane domain and an intracellular signaling domain, wherein the extracellular antigen binding domain that binds CD33 comprises the HCDR2 of SEQ ID NOs: 36, 37, 38, 39, 40, 41, 42, 43, 44, 256, 257 or 258, or conservative substitutions thereof.

The disclosure also provides a CAR comprising an extracellular antigen binding domain that binds CD33, a transmembrane domain and an intracellular signaling domain, wherein the extracellular antigen binding domain that binds CD33 comprises the HCDR2 of SEQ ID NOs: 90, 91, 168, 173, or 177, or conservative substitutions thereof.

The disclosure also provides a CAR comprising an extracellular antigen binding domain that binds CD33, a transmembrane domain and an intracellular signaling domain, wherein the extracellular antigen binding domain that binds CD33 comprises the HCDR3 of SEQ ID NOs: 45, 46, 47, 48, 49, 50, 51, 259, 260 or 261, or conservative substitutions thereof.

The disclosure also provides a CAR comprising an extracellular antigen binding domain that binds CD33, a

transmembrane domain and an intracellular signaling domain, wherein the extracellular antigen binding domain that binds CD33 comprises the HCDR3 of SEQ ID NOs: 92, 93, 94, 169, 174, 178, or 180, or conservative substitutions thereof.

The disclosure also provides a CAR comprising an extracellular antigen binding domain that binds CD33, a transmembrane domain and an intracellular signaling domain, wherein the extracellular antigen binding domain that binds CD33 comprises the LCDR1 of SEQ ID NOs: 62, 63, 64, 65, 66, 67, 68, 265 or 266 or conservative substitutions thereof.

The disclosure also provides a CAR comprising an extracellular antigen binding domain that binds CD33, a transmembrane domain and an intracellular signaling domain, wherein the extracellular antigen binding domain that binds CD33 comprises the LCDR1 of SEQ ID NOs: 98, 99, 100, 182, 186, 189, 193, 197, or 201, or conservative substitutions thereof.

The disclosure also provides a CAR comprising an extracellular antigen binding domain that binds CD33, a transmembrane domain and an intracellular signaling domain, wherein the extracellular antigen binding domain that binds CD33 comprises the LCDR2 of SEQ ID NOs: 69, 70, 71, 72, 73, 74, 267 or 268, or conservative substitutions thereof.

The disclosure also provides a CAR comprising an extracellular antigen binding domain that binds CD33, a transmembrane domain and an intracellular signaling domain, wherein the extracellular antigen binding domain that binds CD33 comprises the LCDR2 of SEQ ID NOs: 101, 102, 103, 183, 187, 190, 194, or 198, or conservative substitutions thereof.

The disclosure also provides a CAR comprising an extracellular antigen binding domain that binds CD33, a transmembrane domain and an intracellular signaling domain, wherein the extracellular antigen binding domain that binds CD33 comprises the LCDR3 of SEQ ID NOs: 71 or 146, or conservative substitutions thereof.

The disclosure also provides a CAR comprising an extracellular antigen binding domain that binds CD33, a transmembrane domain and an intracellular signaling domain, wherein the extracellular antigen binding domain that binds CD33 comprises the LCDR3 of SEQ ID NOs: 75, 76, 77, 78, 79, 80, 269 or 270, or conservative substitutions thereof.

The disclosure also provides a CAR comprising an extracellular antigen binding domain that binds CD33, a transmembrane domain and an intracellular signaling domain, wherein the extracellular antigen binding domain that binds CD33 comprises the LCDR3 of SEQ ID NOs: 104, 184, 191, 195, or 199, or conservative substitutions thereof.

The disclosure also provides a CAR comprising an extracellular antigen binding domain that binds CD33, a transmembrane domain and an intracellular signaling domain, wherein the extracellular antigen binding domain that binds CD33 comprises the HCDR1, the HCDR2, the HCDR3, the LCDR1, the LCDR2 and the LCDR3 of SEQ ID NOs: 28, 36, 45, 62, 69, and 75, respectively.

The disclosure also provides a CAR comprising an extracellular antigen binding domain that binds CD33, a transmembrane domain and an intracellular signaling domain, wherein the extracellular antigen binding domain that binds CD33 comprises the HCDR1, the HCDR2, the HCDR3, the LCDR1, the LCDR2 and the LCDR3 of SEQ ID NOs: 29, 37, 46, 63, 70, and 76, respectively.

The disclosure also provides a CAR comprising an extracellular antigen binding domain that binds CD33, a transmembrane domain and an intracellular signaling domain, wherein the extracellular antigen binding domain that binds CD33 comprises the HCDR1, the HCDR2, the HCDR3, the LCDR1, the LCDR2 and the LCDR3 of SEQ ID NOs: 30, 38, 47, 64, 71, and 77, respectively.

The disclosure also provides a CAR comprising an extracellular antigen binding domain that binds CD33, a transmembrane domain and an intracellular signaling domain, wherein the extracellular antigen binding domain that binds CD33 comprises the HCDR1, the HCDR2, the HCDR3, the LCDR1, the LCDR2 and the LCDR3 of SEQ ID NOs: 31, 39, 48, 65, 72, and 78, respectively.

The disclosure also provides a CAR comprising an extracellular antigen binding domain that binds CD33, a transmembrane domain and an intracellular signaling domain, wherein the extracellular antigen binding domain that binds CD33 comprises the HCDR1, the HCDR2, the HCDR3, the LCDR1, the LCDR2 and the LCDR3 of SEQ ID NOs: 32, 40, 49, 66, 70, and 76, respectively.

The disclosure also provides a CAR comprising an extracellular antigen binding domain that binds CD33, a transmembrane domain and an intracellular signaling domain, wherein the extracellular antigen binding domain that binds CD33 comprises the HCDR1, the HCDR2, the HCDR3, the LCDR1, the LCDR2 and the LCDR3 of SEQ ID NOs: 33, 41, 50, 67, 73, and 79, respectively.

The disclosure also provides a CAR comprising an extracellular antigen binding domain that binds CD33, a transmembrane domain and an intracellular signaling domain, wherein the extracellular antigen binding domain that binds CD33 comprises the HCDR1, the HCDR2, the HCDR3, the LCDR1, the LCDR2 and the LCDR3 of SEQ ID NOs: 34, 42, 51, 68, 74, and 80, respectively.

The disclosure also provides a CAR comprising an extracellular antigen binding domain that binds CD33, a transmembrane domain and an intracellular signaling domain, wherein the extracellular antigen binding domain that binds CD33 comprises the HCDR1, the HCDR2, the HCDR3, the LCDR1, the LCDR2 and the LCDR3 of SEQ ID NOs: 34, 43, 51, 68, 74, and 80, respectively.

The disclosure also provides a CAR comprising an extracellular antigen binding domain that binds CD33, a transmembrane domain and an intracellular signaling domain, wherein the extracellular antigen binding domain that binds CD33 comprises the HCDR1, the HCDR2, the HCDR3, the LCDR1, the LCDR2 and the LCDR3 of SEQ ID NOs: 34, 44, 51, 68, 74, and 80, respectively.

The disclosure also provides a CAR comprising an extracellular antigen binding domain that binds CD33, a transmembrane domain and an intracellular signaling domain, wherein the extracellular antigen binding domain that binds CD33 comprises the HCDR1, the HCDR2, the HCDR3, the LCDR1, the LCDR2 and the LCDR3 of SEQ ID NOs: 35, 42, 51, 68, 74, and 80, respectively.

The disclosure also provides a CAR comprising an extracellular antigen binding domain that binds CD33, a transmembrane domain and an intracellular signaling domain, wherein the extracellular antigen binding domain that binds CD33 comprises the HCDR1, the HCDR2, the HCDR3, the LCDR1, the LCDR2 and the LCDR3 of SEQ ID NOs: 89, 90, 92, 98, 101, and 104, respectively.

The disclosure also provides a CAR comprising an extracellular antigen binding domain that binds CD33, a transmembrane domain and an intracellular signaling domain, wherein the extracellular antigen binding domain that binds CD33 comprises the HCDR1, the HCDR2, the HCDR3, the LCDR1, the LCDR2 and the LCDR3 of SEQ ID NOs: 89, 90, 93, 99, 102, and 104, respectively.

The disclosure also provides a CAR comprising an extracellular antigen binding domain that binds CD33, a transmembrane domain and an intracellular signaling domain, wherein the extracellular antigen binding domain that binds CD33 comprises the HCDR1, the HCDR2, the HCDR3, the LCDR1, the LCDR2 and the LCDR3 of SEQ ID NOs: 33, 91, 94, 100, 103, and 104, respectively.

The disclosure also provides a CAR comprising an extracellular antigen binding domain that binds CD33, a transmembrane domain and an intracellular signaling domain, wherein the extracellular antigen binding domain that binds CD33 comprises the HCDR1, the HCDR2, the HCDR3, the LCDR1, the LCDR2 and the LCDR3 of SEQ ID NOs: 167, 168, 169, 182, 183, and 184, respectively.

The disclosure also provides a CAR comprising an extracellular antigen binding domain that binds CD33, a transmembrane domain and an intracellular signaling domain, wherein the extracellular antigen binding domain that binds CD33 comprises the HCDR1, the HCDR2, the HCDR3, the LCDR1, the LCDR2 and the LCDR3 of SEQ ID NOs: 33, 91, 94, 186, 187, and 104, respectively.

The disclosure also provides a CAR comprising an extracellular antigen binding domain that binds CD33, a transmembrane domain and an intracellular signaling domain, wherein the extracellular antigen binding domain that binds CD33 comprises the HCDR1, the HCDR2, the HCDR3, the LCDR1, the LCDR2 and the LCDR3 of SEQ ID NOs: 172, 173, 174, 189, 190, and 191, respectively.

The disclosure also provides a CAR comprising an extracellular antigen binding domain that binds CD33, a transmembrane domain and an intracellular signaling domain, wherein the extracellular antigen binding domain that binds CD33 comprises the HCDR1, the HCDR2, the HCDR3, the LCDR1, the LCDR2 and the LCDR3 of SEQ ID NOs: 176, 177, 178, 193, 194, and 195, respectively.

The disclosure also provides a CAR comprising an extracellular antigen binding domain that binds CD33, a transmembrane domain and an intracellular signaling domain, wherein the extracellular antigen binding domain that binds CD33 comprises the HCDR1, the HCDR2, the HCDR3, the LCDR1, the LCDR2 and the LCDR3 of SEQ ID NOs: 89, 90, 180, 197, 198, and 199, respectively.

The disclosure also provides a CAR comprising an extracellular antigen binding domain that binds CD33, a transmembrane domain and an intracellular signaling domain, wherein the extracellular antigen binding domain that binds CD33 comprises the HCDR1, the HCDR2, the HCDR3, the LCDR1, the LCDR2 and the LCDR3 of SEQ ID NOs: 89, 90, 93, 201, 102, and 104, respectively.

The disclosure also provides a CAR comprising an extracellular antigen binding domain that binds CD33, a transmembrane domain and an intracellular signaling domain, wherein the extracellular antigen binding domain that binds CD33 comprises the HCDR1, the HCDR2, the HCDR3, the LCDR1, the LCDR2 and the LCDR3 of SEQ ID NOs: 253, 256, 259, 265, 74, and 269, respectively.

The disclosure also provides a CAR comprising an extracellular antigen binding domain that binds CD33, a transmembrane domain and an intracellular signaling domain, wherein the extracellular antigen binding domain that binds CD33 comprises the HCDR1, the HCDR2, the HCDR3, the LCDR1, the LCDR2 and the LCDR3 of SEQ ID NOs: 254, 257, 260, 66, 267, and 76, respectively.

The disclosure also provides a CAR comprising an extracellular antigen binding domain that binds CD33, a transmembrane domain and an intracellular signaling domain, wherein the extracellular antigen binding domain that binds CD33 comprises the HCDR1, the HCDR2, the HCDR3, the LCDR1, the LCDR2 and the LCDR3 of SEQ ID NOs: 255, 258, 261, 266, 268, and 270, respectively.

In some embodiments, the extracellular antigen binding domain that binds CD33 comprises the VL of SEQ ID NOs: 81, 82, 83, 84, 85, 86, 87, or 88.

In some embodiments, the extracellular antigen binding domain that binds CD33 comprises the VL of SEQ ID NOs: 105, 106, 107, 185, 188, 192, 196, 200, or 202.

In some embodiments, the extracellular antigen binding domain that binds CD33 comprises

a) the VH of SEQ ID NO: 52 and the VL of SEQ ID NO: 81;
b) the VH of SEQ ID NO: 53 and the VL of SEQ ID NO: 82;
c) the VH of SEQ ID NO: 54 and the VL of SEQ ID NO: 83;
d) the VH of SEQ ID NO: 55 and the VL of SEQ ID NO: 84;
e) the VH of SEQ ID NO: 56 and the VL of SEQ ID NO: 85;
f) the VH of SEQ ID NO: 57 and the VL of SEQ ID NO: 86;
g) the VH of SEQ ID NO: 58 and the VL of SEQ ID NO: 87;
h) the VH of SEQ ID NO: 59 and the VL of SEQ ID NO: 87;
i) the VH of SEQ ID NO: 60 and the VL of SEQ ID NO: 87;
j) the VH of SEQ ID NO: 61 and the VL of SEQ ID NO: 88;
k) the VH of SEQ ID NO: 262 and the VL of SEQ ID NO: 271;
l) the VH of SEQ ID NO: 263 and the VL of SEQ ID NO: 272; or
m) the VH of SEQ ID NO: 264 and the VL of SEQ ID NO: 273;

In certain embodiments, the extracellular antigen binding domain that binds CD33 comprises the VH of SEQ ID NO: 53 and the VL of SEQ ID NO: 82.

In other embodiments, the extracellular antigen binding domain that binds CD33 comprises the VH of SEQ ID NO: 56 and the VL of SEQ ID NO: 85.

In other embodiments, the extracellular antigen binding domain that binds CD33 comprises the VH of SEQ ID NO: 59 and the VL of SEQ ID NO: 87.

In other embodiments, the extracellular antigen binding domain that binds CD33 comprises the VH of SEQ ID NO: 262 and the VL of SEQ ID NO: 271.

In other embodiments, the extracellular antigen binding domain that binds CD33 comprises the VH of SEQ ID NO: 263 and the VL of SEQ ID NO: 272.

In other embodiments, the extracellular antigen binding domain that binds CD33 comprises the the VH of SEQ ID NO: 264 and the VL of SEQ ID NO: 273.

In some embodiments, the extracellular antigen binding domain that binds CD33 comprises

a) the VH of SEQ ID NO: 95 and the VL of SEQ ID NO: 105;
b) the VH of SEQ ID NO: 96 and the VL of SEQ ID NO: 106;
c) the VH of SEQ ID NO: 97 and the VL of SEQ ID NO: 107;
d) the VH of SEQ ID NO: 170 and the VL of SEQ ID NO: 185;
e) the VH of SEQ ID NO: 171 and the VL of SEQ ID NO: 188;
f) the VH of SEQ ID NO: 175 and the VL of SEQ ID NO: 192;
g) the VH of SEQ ID NO: 179 and the VL of SEQ ID NO: 196;
h) the VH of SEQ ID NO: 181 and the VL of SEQ ID NO: 200; or
i) the VH of SEQ ID NO: 96 and the VL of SEQ ID NO: 202.

The extracellular antigen-binding domain may also be a variant the CD33 binding domains as described elsewhere (for example, a variant domain as described in Examples 19-20).

In some embodiments, the extracellular antigen-binding domain comprises the VH comprising the amino acid sequence that is at least 50%, at least 55%, at least 60%, at least 65%, at least 70%, at least 75%, at least 80%, at least 85%, at least 90%, at least 95%, at least 98% or at least 99% identical with the polypeptide of SEQ ID NO: 52 and the VL comprising the amino acid sequence that is at least 50%, at least 55%, at least 60%, at least 65%, at least 70%, at least 75%, at least 80%, at least 85%, at least 90%, at least 95%, at least 98% or at least 99% identical with the polypeptide of SEQ ID NO: 81.

In some embodiments, the extracellular antigen-binding domain comprises the VH comprising the amino acid sequence that is at least 50%, at least 55%, at least 60%, at least 65%, at least 70%, at least 75%, at least 80%, at least 85%, at least 90%, at least 95%, at least 98% or at least 99% identical with the polypeptide of SEQ ID NO: 53 and the VL comprising the amino acid sequence that is at least 50%, at least 55%, at least 60%, at least 65%, at least 70%, at least 75%, at least 80%, at least 85%, at least 90%, at least 95%, at least 98% or at least 99% identical with the polypeptide of SEQ ID NO: 82.

In some embodiments, the extracellular antigen-binding domain comprises the VH comprising the amino acid sequence that is at least 50%, at least 55%, at least 60%, at least 65%, at least 70%, at least 75%, at least 80%, at least

85%, at least 90%, at least 95%, at least 98% or at least 99% identical with the polypeptide of SEQ ID NO: 54 and the VL comprising the amino acid sequence that is at least 50%, at least 55%, at least 60%, at least 65%, at least 70%, at least 75%, at least 80%, at least 85%, at least 90%, at least 95%, at least 98% or at least 99% identical with the polypeptide of SEQ ID NO: 83.

In some embodiments, the extracellular antigen-binding domain comprises the VH comprising the amino acid sequence that is at least 50%, at least 55%, at least 60%, at least 65%, at least 70%, at least 75%, at least 80%, at least 85%, at least 90%, at least 95%, at least 98% or at least 99% identical with the polypeptide of SEQ ID NO: 55 and the VL comprising the amino acid sequence that is at least 50%, at least 55%, at least 60%, at least 65%, at least 70%, at least 75%, at least 80%, at least 85%, at least 90%, at least 95%, at least 98% or at least 99% identical with the polypeptide of SEQ ID NO: 84.

In some embodiments, the extracellular antigen-binding domain comprises the VH comprising the amino acid sequence that is at least 50%, at least 55%, at least 60%, at least 65%, at least 70%, at least 75%, at least 80%, at least 85%, at least 90%, at least 95%, at least 98% or at least 99% identical with the polypeptide of SEQ ID NO: 56 and the VL comprising the amino acid sequence that is at least 50%, at least 55%, at least 60%, at least 65%, at least 70%, at least 75%, at least 80%, at least 85%, at least 90%, at least 95%, at least 98% or at least 99% identical with the polypeptide of SEQ ID NO: 85.

In some embodiments, the extracellular antigen-binding domain comprises the VH comprising the amino acid sequence that is at least 50%, at least 55%, at least 60%, at least 65%, at least 70%, at least 75%, at least 80%, at least 85%, at least 90%, at least 95%, at least 98% or at least 99% identical with the polypeptide of SEQ ID NO: 57 and the VL comprising the amino acid sequence that is at least 50%, at least 55%, at least 60%, at least 65%, at least 70%, at least 75%, at least 80%, at least 85%, at least 90%, at least 95%, at least 98% or at least 99% identical with the polypeptide of SEQ ID NO: 86.

In some embodiments, the extracellular antigen-binding domain comprises the VH comprising the amino acid sequence that is at least 50%, at least 55%, at least 60%, at least 65%, at least 70%, at least 75%, at least 80%, at least 85%, at least 90%, at least 95%, at least 98% or at least 99% identical with the polypeptide of SEQ ID NO: 58 and the VL comprising the amino acid sequence that is at least 50%, at least 55%, at least 60%, at least 65%, at least 70%, at least 75%, at least 80%, at least 85%, at least 90%, at least 95%, at least 98% or at least 99% identical with the polypeptide of SEQ ID NO: 87.

In some embodiments, the extracellular antigen-binding domain comprises the VH comprising the amino acid sequence that is at least 50%, at least 55%, at least 60%, at least 65%, at least 70%, at least 75%, at least 80%, at least 85%, at least 90%, at least 95%, at least 98% or at least 99% identical with the polypeptide of SEQ ID NO: 59 and the VL comprising the amino acid sequence that is at least 50%, at least 55%, at least 60%, at least 65%, at least 70%, at least 75%, at least 80%, at least 85%, at least 90%, at least 95%, at least 98% or at least 99% identical with the polypeptide of SEQ ID NO: 87.

In some embodiments, the extracellular antigen-binding domain comprises the VH comprising the amino acid sequence that is at least 50%, at least 55%, at least 60%, at least 65%, at least 70%, at least 75%, at least 80%, at least 85%, at least 90%, at least 95%, at least 98% or at least 99% identical with the polypeptide of SEQ ID NO: 60 and the VL comprising the amino acid sequence that is at least 50%, at least 55%, at least 60%, at least 65%, at least 70%, at least 75%, at least 80%, at least 85%, at least 90%, at least 95%, at least 98% or at least 99% identical with the polypeptide of SEQ ID NO: 87.

In some embodiments, the extracellular antigen-binding domain comprises the VH comprising the amino acid sequence that is at least 50%, at least 55%, at least 60%, at least 65%, at least 70%, at least 75%, at least 80%, at least 85%, at least 90%, at least 95%, at least 98% or at least 99% identical with the polypeptide of SEQ ID NO: 61 and the VL comprising the amino acid sequence that is at least 50%, at least 55%, at least 60%, at least 65%, at least 70%, at least 75%, at least 80%, at least 85%, at least 90%, at least 95%, at least 98% or at least 99% identical with the polypeptide of SEQ ID NO: 88.

In some embodiments, the extracellular antigen-binding domain comprises the VH comprising the amino acid sequence that is at least 50%, at least 55%, at least 60%, at least 65%, at least 70%, at least 75%, at least 80%, at least 85%, at least 90%, at least 95%, at least 98% or at least 99% identical with the polypeptide of SEQ ID NO: 95 and the VL comprising the amino acid sequence that is at least 50%, at least 55%, at least 60%, at least 65%, at least 70%, at least 75%, at least 80%, at least 85%, at least 90%, at least 95%, at least 98% or at least 99% identical with the polypeptide of SEQ ID NO: 105.

In some embodiments, the extracellular antigen-binding domain comprises the VH comprising the amino acid sequence that is at least 50%, at least 55%, at least 60%, at least 65%, at least 70%, at least 75%, at least 80%, at least 85%, at least 90%, at least 95%, at least 98% or at least 99% identical with the polypeptide of SEQ ID NO: 96 and the VL comprising the amino acid sequence that is at least 50%, at least 55%, at least 60%, at least 65%, at least 70%, at least 75%, at least 80%, at least 85%, at least 90%, at least 95%, at least 98% or at least 99% identical with the polypeptide of SEQ ID NO: 106.

In some embodiments, the extracellular antigen-binding domain comprises the VH comprising the amino acid sequence that is at least 50%, at least 55%, at least 60%, at least 65%, at least 70%, at least 75%, at least 80%, at least 85%, at least 90%, at least 95%, at least 98% or at least 99% identical with the polypeptide of SEQ ID NO: 97 and the VL comprising the amino acid sequence that is at least 50%, at least 55%, at least 60%, at least 65%, at least 70%, at least 75%, at least 80%, at least 85%, at least 90%, at least 95%, at least 98% or at least 99% identical with the polypeptide of SEQ ID NO: 107.

In some embodiments, the extracellular antigen-binding domain comprises the VH comprising the amino acid sequence that is at least 50%, at least 55%, at least 60%, at least 65%, at least 70%, at least 75%, at least 80%, at least 85%, at least 90%, at least 95%, at least 98% or at least 99% identical with the polypeptide of SEQ ID NO: 170 and the VL comprising the amino acid sequence that is at least 50%, at least 55%, at least 60%, at least 65%, at least 70%, at least 75%, at least 80%, at least 85%, at least 90%, at least 95%, at least 98% or at least 99% identical with the polypeptide of SEQ ID NO: 185.

In some embodiments, the extracellular antigen-binding domain comprises the VH comprising the amino acid sequence that is at least 50%, at least 55%, at least 60%, at least 65%, at least 70%, at least 75%, at least 80%, at least 85%, at least 90%, at least 95%, at least 98% or at least 99% identical with the polypeptide of SEQ ID NO: 171 and the VL comprising the amino acid sequence that is at least 50%, at least 55%, at least 60%, at least 65%, at least 70%, at least 75%, at least 80%, at least 85%, at least 90%, at least 95%, at least 98% or at least 99% identical with the polypeptide of SEQ ID NO: 188.

In some embodiments, the extracellular antigen-binding domain comprises the VH comprising the amino acid sequence that is at least 50%, at least 55%, at least 60%, at least 65%, at least 70%, at least 75%, at least 80%, at least 85%, at least 90%, at least 95%, at least 98% or at least 99% identical with the polypeptide of SEQ ID NO: 175 and the VL comprising the amino acid sequence that is at least 50%, at least 55%, at least 60%, at least 65%, at least 70%, at least 75%, at least 80%, at least 85%, at least 90%, at least 95%, at least 98% or at least 99% identical with the polypeptide of SEQ ID NO: 192.

In some embodiments, the extracellular antigen-binding domain comprises the VH comprising the amino acid sequence that is at least 50%, at least 55%, at least 60%, at least 65%, at least 70%, at least 75%, at least 80%, at least 85%, at least 90%, at least 95%, at least 98% or at least 99% identical with the polypeptide of SEQ ID NO: 179 and the VL comprising the amino acid sequence that is at least 50%, at least 55%, at least 60%, at least 65%, at least 70%, at least 75%, at least 80%, at least 85%, at least 90%, at least 95%, at least 98% or at least 99% identical with the polypeptide of SEQ ID NO: 196.

In some embodiments, the extracellular antigen-binding domain comprises the VH comprising the amino acid sequence that is at least 50%, at least 55%, at least 60%, at least 65%, at least 70%, at least 75%, at least 80%, at least 85%, at least 90%, at least 95%, at least 98% or at least 99% identical with the polypeptide of SEQ ID NO: 181 and the VL comprising the amino acid sequence that is at least 50%, at least 55%, at least 60%, at least 65%, at least 70%, at least 75%, at least 80%, at least 85%, at least 90%, at least 95%, at least 98% or at least 99% identical with the polypeptide of SEQ ID NO: 200.

In some embodiments, the extracellular antigen-binding domain comprises the VH comprising the amino acid sequence that is at least 50%, at least 55%, at least 60%, at least 65%, at least 70%, at least 75%, at least 80%, at least 85%, at least 90%, at least 95%, at least 98% or at least 99% identical with the polypeptide of SEQ ID NO: 96 and the VL comprising the amino acid sequence that is at least 50%, at least 55%, at least 60%, at least 65%, at least 70%, at least 75%, at least 80%, at least 85%, at least 90%, at least 95%, at least 98% or at least 99% identical with the polypeptide of SEQ ID NO: 202.

In some embodiments, the extracellular antigen-binding domain comprises the VH comprising the amino acid sequence that is at least 50%, at least 55%, at least 60%, at least 65%, at least 70%, at least 75%, at least 80%, at least 85%, at least 90%, at least 95%, at least 98% or at least 99% identical with the polypeptide of SEQ ID NO: 262 and the VL comprising the amino acid sequence that is at least 50%, at least 55%, at least 60%, at least 65%, at least 70%, at least 75%, at least 80%, at least 85%, at least 90%, at least 95%, at least 98% or at least 99% identical with the polypeptide of SEQ ID NO: 271.

In some embodiments, the extracellular antigen-binding domain comprises the VH comprising the amino acid sequence that is at least 50%, at least 55%, at least 60%, at least 65%, at least 70%, at least 75%, at least 80%, at least 85%, at least 90%, at least 95%, at least 98% or at least 99% identical with the polypeptide of SEQ ID NO: 263 and the VL comprising the amino acid sequence that is at least 50%, at least 55%, at least 60%, at least 65%, at least 70%, at least 75%, at least 80%, at least 85%, at least 90%, at least 95%, at least 98% or at least 99% identical with the polypeptide of SEQ ID NO: 272.

In some embodiments, the extracellular antigen-binding domain comprises the VH comprising the amino acid sequence that is at least 50%, at least 55%, at least 60%, at least 65%, at least 70%, at least 75%, at least 80%, at least 85%, at least 90%, at least 95%, at least 98% or at least 99% identical with the polypeptide of SEQ ID NO: 264 and the VL comprising the amino acid sequence that is at least 50%, at least 55%, at least 60%, at least 65%, at least 70%, at

least 75%, at least 80%, at least 85%, at least 90%, at least 95%, at least 98% or at least 99% identical with the polypeptide of SEQ ID NO: 273.

In some embodiments, the extracellular antigen binding domain that binds CD33 comprises a scFv. In some embodiments, the scFv comprises a linker polypeptide between the VL and the VH.

In some embodiments, the linker polypeptide comprises the amino acid sequence of SEQ ID NO: 7. In some embodiments, the linker polypeptide comprises an amino acid sequence having at least 50%, at least 55%, at least 60%, at least 65%, at least 70%, at least 75%, at least 80%, at least 85%, at least 90%, at least 95%, at least 98% or at least 99% sequence identity with SEQ ID NO: 108.

In some embodiments, the linker polypeptide comprises the amino acid sequence of SEQ ID NO: 108 (e.g. wherein the linker polypeptide consists of the amino acid sequence of SEQ ID NO: 108).

In some embodiments, the extracellular antigen binding domain that binds CD33 is a scFv which comprises an amino acid sequence selected from the group consisting of SEQ ID NOs: 203, 204, 205, 206, 207, 208, 209, 210, 211, 212, 213, 214, 215, 216, 217, 218, 219, 220, 221, 274, 275 and 276.

In some embodiments, the extracellular antigen binding domain that binds CD33 is a scFv which comprises an amino acid sequence SEQ ID NO: 203.

In some embodiments, the extracellular antigen binding domain that binds CD33 is a scFv which comprises an amino acid sequence SEQ ID NO: 204.

In some embodiments, the extracellular antigen binding domain that binds CD33 is a scFv which comprises an amino acid sequence SEQ ID NO: 205.

In some embodiments, the extracellular antigen binding domain that binds CD33 is a scFv which comprises an amino acid sequence SEQ ID NO: 206.

In some embodiments, the extracellular antigen binding domain that binds CD33 is a scFv which comprises an amino acid sequence SEQ ID NO: 207.

In some embodiments, the extracellular antigen binding domain that binds CD33 is a scFv which comprises an amino acid sequence SEQ ID NO: 208.

In some embodiments, the extracellular antigen binding domain that binds CD33 is a scFv which comprises an amino acid sequence SEQ ID NO: 209.

In some embodiments, the extracellular antigen binding domain that binds CD33 is a scFv which comprises an amino acid sequence SEQ ID NO: 210.

In some embodiments, the extracellular antigen binding domain that binds CD33 is a scFv which comprises an amino acid sequence SEQ ID NO: 211.

In some embodiments, the extracellular antigen binding domain that binds CD33 is a scFv which comprises an amino acid sequence SEQ ID NO: 212.

In some embodiments, the extracellular antigen binding domain that binds CD33 is a scFv which comprises an amino acid sequence SEQ ID NO: 213.

In some embodiments, the extracellular antigen binding domain that binds CD33 is a scFv which comprises an amino acid sequence SEQ ID NO: 214.

In some embodiments, the extracellular antigen binding domain that binds CD33 is a scFv which comprises an amino acid sequence SEQ ID NO: 215.

In some embodiments, the extracellular antigen binding domain that binds CD33 is a scFv which comprises an amino acid sequence SEQ ID NO: 216.

In some embodiments, the extracellular antigen binding domain that binds CD33 is a scFv which comprises an amino acid sequence SEQ ID NO: 217.

In some embodiments, the extracellular antigen binding domain that binds CD33 is a scFv which comprises an amino acid sequence SEQ ID NO: 218.

In some embodiments, the extracellular antigen binding domain that binds CD33 is a scFv which comprises an amino acid sequence SEQ ID NO: 219.

In some embodiments, the extracellular antigen binding domain that binds CD33 is a scFv which comprises an amino acid sequence SEQ ID NO: 220.

In some embodiments, the extracellular antigen binding domain that binds CD33 is a scFv which comprises an amino acid sequence SEQ ID NO: 221.

In some embodiments, the extracellular antigen binding domain that binds CD33 is a scFv which comprises an amino acid sequence SEQ ID NO: 274.

In some embodiments, the extracellular antigen binding domain that binds CD33 is a scFv which comprises an amino acid sequence SEQ ID NO: 275.

In some embodiments, the extracellular antigen binding domain that binds CD33 is a scFv which comprises an amino acid sequence SEQ ID NO: 276.

In certain embodiments, the extracellular antigen binding domain that binds CD33 is a scFv which comprises

an amino acid sequence SEQ ID NO: 213.

In other embodiments, the extracellular antigen binding domain that binds CD33 is a scFv which comprises an amino acid sequence SEQ ID NO: 216.

In other embodiments, the extracellular antigen binding domain that binds CD33 is a scFv which comprises an amino acid sequence SEQ ID NO: 219.

In other embodiments, the extracellular antigen binding domain that binds CD33 is a scFv which comprises an amino acid sequence SEQ ID NO: 274.

In other embodiments, the extracellular antigen binding domain that binds CD33 is a scFv which comprises an amino acid sequence SEQ ID NO: 275.

In other embodiments, the extracellular antigen binding domain that binds CD33 is a scFv which comprises an amino acid sequence SEQ ID NO: 276.

In some embodiments, the scFv comprises the amino acid sequence that is at least 50%, at least 55%, at least 60%, at least 65%, at least 70%, at least 75%, at least 80%, at least 85%, at least 90%, at least 95%, at least 98% or at least 99% identical to the amino acid sequence of SEQ ID NO: 203.

In some embodiments, the scFv comprises the amino acid sequence that is at least 50%, at least 55%, at least 60%, at least 65%, at least 70%, at least 75%, at least 80%, at least 85%, at least 90%, at least 95%, at least 98% or at least 99% identical to the amino acid sequence of SEQ ID NO: 204.

In some embodiments, the scFv comprises the amino acid sequence that is at least 50%, at least 55%, at least 60%, at least 65%, at least 70%, at least 75%, at least 80%, at least 85%, at least 90%, at least 95%, at least 98% or at least 99% identical to the amino acid sequence of SEQ ID NO: 205.

In some embodiments, the scFv comprises the amino acid sequence that is at least 50%, at least 55%, at least 60%, at least 65%, at least 70%, at least 75%, at least 80%, at least 85%, at least 90%, at least 95%, at least 98% or at least 99% identical to the amino acid sequence of SEQ ID NO: 206.

In some embodiments, the scFv comprises the amino acid sequence that is at least 50%, at least 55%, at least 60%, at least 65%, at least 70%, at least 75%, at least 80%, at least 85%, at least 90%, at least 95%, at least 98% or at least 99% identical to the amino acid sequence of SEQ ID NO: 207.

In some embodiments, the scFv comprises the amino acid sequence that is at least 50%, at least 55%, at least 60%, at least 65%, at least 70%, at least 75%, at least 80%, at least 85%, at least 90%, at least 95%, at least 98% or at least 99% identical to the amino acid sequence of SEQ ID NO: 208.

In some embodiments, the scFv comprises the amino acid sequence that is at least 50%, at least 55%, at least 60%, at least 65%, at least 70%, at least 75%, at least 80%, at least 85%, at least 90%, at least 95%, at least 98% or at least 99% identical to the amino acid sequence of SEQ ID NO: 209.

In some embodiments, the scFv comprises the amino acid sequence that is at least 50%, at least 55%, at least 60%, at least 65%, at least 70%, at least 75%, at least 80%, at least 85%, at least 90%, at least 95%, at least 98% or at least 99% identical to the amino acid sequence of SEQ ID NO: 210.

In some embodiments, the scFv comprises the amino acid sequence that is at least 50%, at least 55%, at least 60%, at least 65%, at least 70%, at least 75%, at least 80%, at least 85%, at least 90%, at least 95%, at least 98% or at least 99% identical to the amino acid sequence of SEQ ID NO: 211.

In some embodiments, the scFv comprises the amino acid sequence that is at least 50%, at least 55%, at least 60%, at least 65%, at least 70%, at least 75%, at least 80%, at least 85%, at least 90%, at least 95%, at least 98% or at least 99% identical to the amino acid sequence of SEQ ID NO: 212.

In some embodiments, the scFv comprises the amino acid sequence that is at least 50%, at least 55%, at least 60%, at least 65%, at least 70%, at least 75%, at least 80%, at least 85%, at least 90%, at least 95%, at least 98% or at least 99% identical to the amino acid sequence of SEQ ID NO: 213.

In some embodiments, the scFv comprises the amino acid sequence that is at least 50%, at least 55%, at least 60%, at least 65%, at least 70%, at least 75%, at least 80%, at least 85%, at least 90%, at least 95%, at least 98% or at least 99% identical to the amino acid sequence of SEQ ID NO: 214.

In some embodiments, the scFv comprises the amino acid sequence that is at least 50%, at least 55%, at least 60%, at least 65%, at least 70%, at least 75%, at least 80%, at least 85%, at least 90%, at least 95%, at least 98% or at least 99% identical to the amino acid sequence of SEQ ID NO: 215.

In some embodiments, the scFv comprises the amino acid sequence that is at least 50%, at least 55%, at least 60%, at least 65%, at least 70%, at least 75%, at least 80%, at least 85%, at least 90%, at least 95%, at least 98% or at least 99% identical to the amino acid sequence of SEQ ID NO: 216.

In some embodiments, the scFv comprises the amino acid sequence that is at least 50%, at least 55%, at least 60%, at least 65%, at least 70%, at least 75%, at least 80%, at least 85%, at least 90%, at least 95%, at least 98% or at least 99% identical to the amino acid sequence of SEQ ID NO: 217.

In some embodiments, the scFv comprises the amino acid sequence that is at least 50%, at least 55%, at least 60%, at least 65%, at least 70%, at least 75%, at least 80%, at least 85%, at least 90%, at least 95%, at least 98% or

at least 99% identical to the amino acid sequence of SEQ ID NO: 218.

In some embodiments, the scFv comprises the amino acid sequence that is at least 50%, at least 55%, at least 60%, at least 65%, at least 70%, at least 75%, at least 80%, at least 85%, at least 90%, at least 95%, at least 98% or at least 99% identical to the amino acid sequence of SEQ ID NO: 219.

In some embodiments, the scFv comprises the amino acid sequence that is at least 50%, at least 55%, at least 60%, at least 65%, at least 70%, at least 75%, at least 80%, at least 85%, at least 90%, at least 95%, at least 98% or at least 99% identical to the amino acid sequence of SEQ ID NO: 220.

In some embodiments, the scFv comprises the amino acid sequence that is at least 50%, at least 55%, at least 60%, at least 65%, at least 70%, at least 75%, at least 80%, at least 85%, at least 90%, at least 95%, at least 98% or at least 99% identical to the amino acid sequence of SEQ ID NO: 221.

In some embodiments, the scFv comprises the amino acid sequence that is at least 50%, at least 55%, at least 60%, at least 65%, at least 70%, at least 75%, at least 80%, at least 85%, at least 90%, at least 95%, at least 98% or at least 99% identical to the amino acid sequence of SEQ ID NO: 274.

In some embodiments, the scFv comprises the amino acid sequence that is at least 50%, at least 55%, at least 60%, at least 65%, at least 70%, at least 75%, at least 80%, at least 85%, at least 90%, at least 95%, at least 98% or at least 99% identical to the amino acid sequence of SEQ ID NO: 275.

In some embodiments, the scFv comprises the amino acid sequence that is at least 50%, at least 55%, at least 60%, at least 65%, at least 70%, at least 75%, at least 80%, at least 85%, at least 90%, at least 95%, at least 98% or at least 99% identical to the amino acid sequence of SEQ ID NO: 276.

The disclosure also provides a CAR comprising an extracellular antigen binding domain that binds CD33, a transmembrane domain and an intracellular signaling domain, wherein the extracellular antigen binding domain that binds CD33 comprises the amino acid sequence selected from the group consisting of SEQ ID NOs: 18, 19, 20, 21, 22, 23, 24, 25, 26, 27, 203, 204, 205, 206, 207, 208, 209, 210, 211, 212, 213, 214, 215, 216, 217, 218, 219, 220, 221, 274, 275 or 276.

In certain embodiments, the CAR comprises an extracellular antigen binding domain that binds CD33, a transmembrane domain and an intracellular signaling domain, wherein the extracellular antigen binding domain that binds CD33 comprises the amino acid sequence selected from the group consisting of SEQ ID NOs: 213, 216, 219, 274, 275 or 276.

The disclosure provides a CAR comprising an extracellular antigen binding domain that comprises an amino acid sequence that is at least 50%, at least 55%, at least 60%, at least 65%, at least 70%, at least 75%, at least 80%, at least 85%, at least 90%, at least 95%, at least 98% or at least 99% identical with the amino acid sequence of SEQ ID NO: 18.

The disclosure also provides a CAR comprising an extracellular antigen binding domain that binds CD33, a transmembrane domain and an intracellular signaling domain, wherein the extracellular antigen binding domain that binds CD33 comprises the amino acid sequence of SEQ ID NO: 18.

The disclosure provides a CAR comprising an extracellular antigen binding domain that comprises an amino acid sequence that is at least 50%, at least 55%, at least 60%, at least 65%, at least 70%, at least 75%, at least 80%, at least 85%, at least 90%, at least 95%, at least 98% or at least 99% identical with the amino acid sequence of SEQ ID NO: 19.

The disclosure also provides a CAR comprising an extracellular antigen binding domain that binds CD33, a transmembrane domain and an intracellular signaling domain, wherein the extracellular antigen binding domain that binds CD33 comprises the amino acid sequence of SEQ ID NO: 19.

The disclosure provides a CAR comprising an extracellular antigen binding domain that comprises an amino acid sequence that is at least 50%, at least 55%, at least 60%, at least 65%, at least 70%, at least 75%, at least 80%, at least 85%, at least 90%, at least 95%, at least 98% or at least 99% identical with the amino acid sequence of SEQ ID NO: 20.

The disclosure also provides a CAR comprising an extracellular antigen binding domain that binds CD33, a transmembrane domain and an intracellular signaling domain, wherein the extracellular antigen binding domain that binds CD33 comprises the amino acid sequence of SEQ ID NO: 20.

The disclosure provides a CAR comprising an extracellular antigen binding domain that comprises an amino acid sequence that is at least 50%, at least 55%, at least 60%, at least 65%, at least 70%, at least 75%, at least 80%, at least 85%, at least 90%, at least 95%, at least 98% or at least 99% identical with the amino acid sequence of SEQ ID NO: 21.

The disclosure also provides a CAR comprising an extracellular antigen binding domain that binds CD33, a transmembrane domain and an intracellular signaling domain, wherein the extracellular antigen binding domain that binds CD33 comprises the amino acid sequence of SEQ ID NO: 21.

The disclosure provides a CAR comprising an extracellular antigen binding domain that comprises an amino acid sequence that is at least 50%, at least 55%, at least 60%, at least 65%, at least 70%, at least 75%, at least 80%,

at least 85%, at least 90%, at least 95%, at least 98% or at least 99% identical with the amino acid sequence of SEQ ID NO: 22.

The disclosure also provides a CAR comprising an extracellular antigen binding domain that binds CD33, a transmembrane domain and an intracellular signaling domain, wherein the extracellular antigen binding domain that binds CD33 comprises the amino acid sequence of SEQ ID NO: 22.

The disclosure provides a CAR comprising an extracellular antigen binding domain that comprises an amino acid sequence that is at least 50%, at least 55%, at least 60%, at least 65%, at least 70%, at least 75%, at least 80%, at least 85%, at least 90%, at least 95%, at least 98% or at least 99% identical with the amino acid sequence of SEQ ID NO: 23.

The disclosure also provides a CAR comprising an extracellular antigen binding domain that binds CD33, a transmembrane domain and an intracellular signaling domain, wherein the extracellular antigen binding domain that binds CD33 comprises the amino acid sequence of SEQ ID NO: 23.

The disclosure provides a CAR comprising an extracellular antigen binding domain that comprises an amino acid sequence that is at least 50%, at least 55%, at least 60%, at least 65%, at least 70%, at least 75%, at least 80%, at least 85%, at least 90%, at least 95%, at least 98% or at least 99% identical with the amino acid sequence of SEQ ID NO: 24.

The disclosure also provides a CAR comprising an extracellular antigen binding domain that binds CD33, a transmembrane domain and an intracellular signaling domain, wherein the extracellular antigen binding domain that binds CD33 comprises the amino acid sequence of SEQ ID NO: 24.

The disclosure provides a CAR comprising an extracellular antigen binding domain that comprises an amino acid sequence that is at least 50%, at least 55%, at least 60%, at least 65%, at least 70%, at least 75%, at least 80%, at least 85%, at least 90%, at least 95%, at least 98% or at least 99% identical with the amino acid sequence of SEQ ID NO: 25.

The disclosure also provides a CAR comprising an extracellular antigen binding domain that binds CD33, a transmembrane domain and an intracellular signaling domain, wherein the extracellular antigen binding domain that binds CD33 comprises the amino acid sequence of SEQ ID NO: 25.

The disclosure provides a CAR comprising an extracellular antigen binding domain that comprises an amino acid sequence that is at least 50%, at least 55%, at least 60%, at least 65%, at least 70%, at least 75%, at least 80%, at least 85%, at least 90%, at least 95%, at least 98% or at least 99% identical with the amino acid sequence of SEQ ID NO: 26.

The disclosure also provides a CAR comprising an extracellular antigen binding domain that binds CD33, a transmembrane domain and an intracellular signaling domain, wherein the extracellular antigen binding domain that binds CD33 comprises the amino acid sequence of SEQ ID NO: 26.

The disclosure provides a CAR comprising an extracellular antigen binding domain that comprises an amino acid sequence that is at least 50%, at least 55%, at least 60%, at least 65%, at least 70%, at least 75%, at least 80%, at least 85%, at least 90%, at least 95%, at least 98% or at least 99% identical with the amino acid sequence of SEQ ID NO: 27.

The disclosure also provides a CAR comprising an extracellular antigen binding domain that binds CD33, a transmembrane domain and an intracellular signaling domain, wherein the extracellular antigen binding domain that binds CD33 comprises the amino acid sequence of SEQ ID NO: 27.

The disclosure provides a CAR comprising an extracellular antigen binding domain that comprises an amino acid sequence that is at least 50%, at least 55%, at least 60%, at least 65%, at least 70%, at least 75%, at least 80%, at least 85%, at least 90%, at least 95%, at least 98% or at least 99% identical with the amino acid sequence of SEQ ID NO: 203.

The disclosure also provides a CAR comprising an extracellular antigen binding domain that binds CD33, a transmembrane domain and an intracellular signaling domain, wherein the extracellular antigen binding domain that binds CD33 comprises the amino acid sequence of SEQ ID NO: 203.

The disclosure provides a CAR comprising an extracellular antigen binding domain that comprises an amino acid sequence that is at least 50%, at least 55%, at least 60%, at least 65%, at least 70%, at least 75%, at least 80%, at least 85%, at least 90%, at least 95%, at least 98% or at least 99% identical with the amino acid sequence of SEQ ID NO: 204.

The disclosure also provides a CAR comprising an extracellular antigen binding domain that binds CD33, a transmembrane domain and an intracellular signaling domain, wherein the extracellular antigen binding domain that binds CD33 comprises the amino acid sequence of SEQ ID NO: 204.

The disclosure provides a CAR comprising an extracellular antigen binding domain that comprises an amino acid sequence that is at least 50%, at least 55%, at least 60%, at least 65%, at least 70%, at least 75%, at least 80%, at least 85%, at least 90%, at least 95%, at least 98% or at least 99% identical with the amino acid sequence of SEQ ID NO: 205.

The disclosure also provides a CAR comprising an extracellular antigen binding domain that binds CD33, a transmembrane domain and an intracellular signaling domain, wherein the extracellular antigen binding domain that binds CD33 comprises the amino acid sequence of SEQ ID NO: 205.

The disclosure provides a CAR comprising an extracellular antigen binding domain that comprises an amino acid sequence that is at least 50%, at least 55%, at least 60%, at least 65%, at least 70%, at least 75%, at least 80%, at least 85%, at least 90%, at least 95%, at least 98% or at least 99% identical with the amino acid sequence of SEQ ID NO: 206.

The disclosure also provides a CAR comprising an extracellular antigen binding domain that binds CD33, a transmembrane domain and an intracellular signaling domain, wherein the extracellular antigen binding domain that binds CD33 comprises the amino acid sequence of SEQ ID NO: 206.

The disclosure provides a CAR comprising an extracellular antigen binding domain that comprises an amino acid sequence that is at least 50%, at least 55%, at least 60%, at least 65%, at least 70%, at least 75%, at least 80%, at least 85%, at least 90%, at least 95%, at least 98% or at least 99% identical with the amino acid sequence of SEQ ID NO: 207.

The disclosure also provides a CAR comprising an extracellular antigen binding domain that binds CD33, a transmembrane domain and an intracellular signaling domain, wherein the extracellular antigen binding domain that binds CD33 comprises the amino acid sequence of SEQ ID NO: 207.

The disclosure provides a CAR comprising an extracellular antigen binding domain that comprises an amino acid sequence that is at least 50%, at least 55%, at least 60%, at least 65%, at least 70%, at least 75%, at least 80%, at least 85%, at least 90%, at least 95%, at least 98% or at least 99% identical with the amino acid sequence of SEQ ID NO: 208.

The disclosure also provides a CAR comprising an extracellular antigen binding domain that binds CD33, a transmembrane domain and an intracellular signaling domain, wherein the extracellular antigen binding domain that binds CD33 comprises the amino acid sequence of SEQ ID NO: 208.

The disclosure provides a CAR comprising an extracellular antigen binding domain that comprises an amino acid sequence that is at least 50%, at least 55%, at least 60%, at least 65%, at least 70%, at least 75%, at least 80%, at least 85%, at least 90%, at least 95%, at least 98% or at least 99% identical with the amino acid sequence of SEQ ID NO: 209.

The disclosure also provides a CAR comprising an extracellular antigen binding domain that binds CD33, a transmembrane domain and an intracellular signaling domain, wherein the extracellular antigen binding domain that binds CD33 comprises the amino acid sequence of SEQ ID NO: 209.

The disclosure provides a CAR comprising an extracellular antigen binding domain that comprises an amino acid sequence that is at least 50%, at least 55%, at least 60%, at least 65%, at least 70%, at least 75%, at least 80%, at least 85%, at least 90%, at least 95%, at least 98% or at least 99% identical with the amino acid sequence of SEQ ID NO: 210.

The disclosure also provides a CAR comprising an extracellular antigen binding domain that binds CD33, a transmembrane domain and an intracellular signaling domain, wherein the extracellular antigen binding domain that binds CD33 comprises the amino acid sequence of SEQ ID NO: 210.

The disclosure provides a CAR comprising an extracellular antigen binding domain that comprises an amino acid sequence that is at least 50%, at least 55%, at least 60%, at least 65%, at least 70%, at least 75%, at least 80%, at least 85%, at least 90%, at least 95%, at least 98% or at least 99% identical with the amino acid sequence of SEQ ID NO: 211.

The disclosure also provides a CAR comprising an extracellular antigen binding domain that binds CD33, a transmembrane domain and an intracellular signaling domain, wherein the extracellular antigen binding domain that binds CD33 comprises the amino acid sequence of SEQ ID NO: 211.

The disclosure provides a CAR comprising an extracellular antigen binding domain that comprises an amino acid sequence that is at least 50%, at least 55%, at least 60%, at least 65%, at least 70%, at least 75%, at least 80%, at least 85%, at least 90%, at least 95%, at least 98% or at least 99% identical with the amino acid sequence of SEQ ID NO: 212.

The disclosure also provides a CAR comprising an extracellular antigen binding domain that binds CD33, a transmembrane domain and an intracellular signaling domain, wherein the extracellular antigen binding domain that binds CD33 comprises the amino acid sequence of SEQ ID NO: 212.

The disclosure provides a CAR comprising an extracellular antigen binding domain that comprises an amino acid sequence that is at least 50%, at least 55%, at least 60%, at least 65%, at least 70%, at least 75%, at least 80%, at least 85%, at least 90%, at least 95%, at least 98% or at least 99% identical with the amino acid sequence of SEQ ID NO: 213.

The disclosure also provides a CAR comprising an extracellular antigen binding domain that binds CD33, a transmembrane domain and an intracellular signaling domain, wherein the extracellular antigen binding domain that

binds CD33 comprises the amino acid sequence of SEQ ID NO: 213.

The disclosure provides a CAR comprising an extracellular antigen binding domain that comprises an amino acid sequence that is at least 50%, at least 55%, at least 60%, at least 65%, at least 70%, at least 75%, at least 80%, at least 85%, at least 90%, at least 95%, at least 98% or at least 99% identical with the amino acid sequence of SEQ ID NO: 214.

The disclosure also provides a CAR comprising an extracellular antigen binding domain that binds CD33, a transmembrane domain and an intracellular signaling domain, wherein the extracellular antigen binding domain that binds CD33 comprises the amino acid sequence of SEQ ID NO: 214.

The disclosure provides a CAR comprising an extracellular antigen binding domain that comprises an amino acid sequence that is at least 50%, at least 55%, at least 60%, at least 65%, at least 70%, at least 75%, at least 80%, at least 85%, at least 90%, at least 95%, at least 98% or at least 99% identical with the amino acid sequence of SEQ ID NO: 215.

The disclosure also provides a CAR comprising an extracellular antigen binding domain that binds CD33, a transmembrane domain and an intracellular signaling domain, wherein the extracellular antigen binding domain that binds CD33 comprises the amino acid sequence of SEQ ID NO: 215.

The disclosure provides a CAR comprising an extracellular antigen binding domain that comprises an amino acid sequence that is at least 50%, at least 55%, at least 60%, at least 65%, at least 70%, at least 75%, at least 80%, at least 85%, at least 90%, at least 95%, at least 98% or at least 99% identical with the amino acid sequence of SEQ ID NO: 216.

The disclosure also provides a CAR comprising an extracellular antigen binding domain that binds CD33, a transmembrane domain and an intracellular signaling domain, wherein the extracellular antigen binding domain that binds CD33 comprises the amino acid sequence of SEQ ID NO: 216.

The disclosure provides a CAR comprising an extracellular antigen binding domain that comprises an amino acid sequence that is at least 50%, at least 55%, at least 60%, at least 65%, at least 70%, at least 75%, at least 80%, at least 85%, at least 90%, at least 95%, at least 98% or at least 99% identical with the amino acid sequence of SEQ ID NO: 217.

The disclosure also provides a CAR comprising an extracellular antigen binding domain that binds CD33, a transmembrane domain and an intracellular signaling domain, wherein the extracellular antigen binding domain that binds CD33 comprises the amino acid sequence of SEQ ID NO: 217.

The disclosure provides a CAR comprising an extracellular antigen binding domain that comprises an amino acid sequence that is at least 50%, at least 55%, at least 60%, at least 65%, at least 70%, at least 75%, at least 80%, at least 85%, at least 90%, at least 95%, at least 98% or at least 99% identical with the amino acid sequence of SEQ ID NO: 218.

The disclosure also provides a CAR comprising an extracellular antigen binding domain that binds CD33, a transmembrane domain and an intracellular signaling domain, wherein the extracellular antigen binding domain that binds CD33 comprises the amino acid sequence of SEQ ID NO: 218.

The disclosure provides a CAR comprising an extracellular antigen binding domain that comprises an amino acid sequence that is at least 50%, at least 55%, at least 60%, at least 65%, at least 70%, at least 75%, at least 80%, at least 85%, at least 90%, at least 95%, at least 98% or at least 99% identical with the amino acid sequence of SEQ ID NO: 219.

The disclosure also provides a CAR comprising an extracellular antigen binding domain that binds CD33, a transmembrane domain and an intracellular signaling domain, wherein the extracellular antigen binding domain that binds CD33 comprises the amino acid sequence of SEQ ID NO: 219.

The disclosure provides a CAR comprising an extracellular antigen binding domain that comprises an amino acid sequence that is at least 50%, at least 55%, at least 60%, at least 65%, at least 70%, at least 75%, at least 80%, at least 85%, at least 90%, at least 95%, at least 98% or at least 99% identical with the amino acid sequence of SEQ ID NO: 220.

The disclosure also provides a CAR comprising an extracellular antigen binding domain that binds CD33, a transmembrane domain and an intracellular signaling domain, wherein the extracellular antigen binding domain that binds CD33 comprises the amino acid sequence of SEQ ID NO: 220.

The disclosure provides a CAR comprising an extracellular antigen binding domain that comprises an amino acid sequence that is at least 50%, at least 55%, at least 60%, at least 65%, at least 70%, at least 75%, at least 80%, at least 85%, at least 90%, at least 95%, at least 98% or at least 99% identical with the amino acid sequence of SEQ ID NO: 221.

The disclosure also provides a CAR comprising an extracellular antigen binding domain that binds CD33, a transmembrane domain and an intracellular signaling domain, wherein the extracellular antigen binding domain that binds CD33 comprises the amino acid sequence of SEQ ID NO: 221.

The disclosure provides a CAR comprising an extracellular antigen binding domain that comprises an amino

acid sequence that is at least 50%, at least 55%, at least 60%, at least 65%, at least 70%, at least 75%, at least 80%, at least 85%, at least 90%, at least 95%, at least 98% or at least 99% identical with the amino acid sequence of SEQ ID NO: 274.

The disclosure also provides a CAR comprising an extracellular antigen binding domain that binds CD33, a transmembrane domain and an intracellular signaling domain, wherein the extracellular antigen binding domain that binds CD33 comprises the amino acid sequence of SEQ ID NO: 274.

The disclosure provides a CAR comprising an extracellular antigen binding domain that comprises an amino acid sequence that is at least 50%, at least 55%, at least 60%, at least 65%, at least 70%, at least 75%, at least 80%, at least 85%, at least 90%, at least 95%, at least 98% or at least 99% identical with the amino acid sequence of SEQ ID NO: 275.

The disclosure also provides a CAR comprising an extracellular antigen binding domain that binds CD33, a transmembrane domain and an intracellular signaling domain, wherein the extracellular antigen binding domain that binds CD33 comprises the amino acid sequence of SEQ ID NO: 275.

The disclosure provides a CAR comprising an extracellular antigen binding domain that comprises an amino acid sequence that is at least 50%, at least 55%, at least 60%, at least 65%, at least 70%, at least 75%, at least 80%, at least 85%, at least 90%, at least 95%, at least 98% or at least 99% identical with the amino acid sequence of SEQ ID NO: 276.

The disclosure also provides a CAR comprising an extracellular antigen binding domain that binds CD33, a transmembrane domain and an intracellular signaling domain, wherein the extracellular antigen binding domain that binds CD33 comprises the amino acid sequence of SEQ ID NO: 276.

In some embodiments, the intracellular signaling domain comprises a polypeptide component selected from the group consisting of a TNF receptor superfamily member 9 (CD137) component, a T-cell surface glycoprotein CD3 zeta chain (CD3z) component, a cluster of differentiation (CD27) component, a cluster of differentiation superfamily member (such as, e.g., CD28 or inducible T-cell co-stimulator (ICOS) component, and a combination thereof.

In some embodiments, the CD137 component comprises an amino acid sequence of SEQ ID NO: 163. In some embodiments, the CD137 component comprises an amino acid sequence that is at least 50%, at least 55%, at least 60%, at least 65%, at least 70%, at least 75%, at least 80%, at least 85%, at least 90%, at least 95%, at least 98 or at least 99% identical to the amino acid sequence of SEQ ID NO: 163.

In some embodiments, the CD3z component comprises an amino acid sequence of SEQ ID NO: 164. In some embodiments, the CD3z component comprises an amino acid sequence that is at least 50%, at least 55%, at least 60%, at least 65%, at least 70%, at least 75%, at least 80%, at least 85%, at least 90%, at least 95%, at least 98 or at least 99% identical to the amino acid sequence of SEQ ID NO: 164.

In some embodiments, the intracellular signaling domain comprises an amino acid sequence of SEQ ID NO: 165. In some embodiments, the intracellular signaling domain comprises an amino acid sequence that is at least 50%, at least 55%, at least 60%, at least 65%, at least 70%, at least 75%, at least 80%, at least 85%, at least 90%, at least 95%, at least 98 or at least 99% identical to the amino acid sequence of SEQ ID NO: 165.

SEQ ID NO: 165

KRGRKKLLYIFKQPFMRPVQTTQEEDGCSCRFPEEEEGGCELRVKFSRSADAPAYKQGQNQLYN
ELNLGRREEYDVLDKRRGRDPEMGGKPRRKNPQEGLYNELQKDKMAEAYSEIGMKGERRRGK
GHDGLYQGLSTATKDTYDALHMQALPPR

In some embodiments, the transmembrane domain comprises a CD8a transmembrane region (CD8a-TM) polypeptide. In some embodiments, the CD8a-TM polypeptide comprises an amino acid sequence of **SEQ ID NO: 162.** (IYIWAPLAGTCGVLLLSLVITLYC; **SEQ ID NO: 162).**

In some embodiments, the CD8a-TM polypeptide comprises an amino acid sequence that is least 50%, at least 55%, at least 60%, at least 65%, at least 70%, at least 75%, at least 80%, at least 85%, at least 90%, at least 95%, at least 98% or at least 99% identical to the amino acid sequence of SEQ ID NO: 26.

In some embodiments, the transmembrane domain comprises at least the transmembrane region(s) of) the α, β or ζ chain of the T-cell receptor, CD28, CD3 epsilon, CD45, CD4, CD5, CD8, CD8α, CD9, CD16, CD22, CD33, CD37, CD40, CD64, CD80, CD86, CD134, CD137, CD154. In some embodiments, the transmembrane domain comprises at least the transmembrane domain of ζ, η or FcεR1γ and -β, MB1 (Igα.), B29 or CD3- γ, ζ, or η. In some embodiments, the transmembrane domain is synthetic, e.g., comprising predominantly hydrophobic residues such as leucine and

valine, a triplet of phenylalanine, or tryptophan.

In some embodiments, the CAR further comprises a hinge region linking the transmembrane domain to the extracellular antigen binding domain that binds CD33. In some embodiments, the hinge region is a CD8a-hinge region. In some embodiments, CD8a-hinge region comprises an amino acid sequence of **SEQ ID NO: 157** (TSTPAPRPPTPAPTIASQPLSLRPEACRPAAGGAVHTRGLDFACD; SEQ ID NO: 157).

In some embodiments, the CD8a-hinge region comprises an amino acid sequence that is at least 50%, at least 55%, at least 60%, at least 65%, at least 70%, at least 75%, at least 80%, at least 85%, at least 90%, at least 95%, at least 98% or at least 99% identical to the amino acid sequence of **SEQ ID NO: 157.** In some embodiments, the hinge region comprises the amino acid sequence EPKSCDKTHTCPPCP (SEQ ID NO: 158), or comprises an amino acid sequence having at least 50%, at least 55%, at least 60%, at least 65%, at least 70%, at least 75%, at least 80%, at least 85%, at least 90%, at least 95%, at least 98% or at least 99% amino acid sequence identity with EPKSCDKTHTCPPCP (SEQ ID NO: 158). In some embodiments, the hinge region comprises the sequence ERKCCVECPPCP (SEQ ID NO: 159) or comprises an amino acid sequence having at least 50%, at least 55%, at least 60%, at least 65%, at least 70%, at least 75%, at least 80%, at least 85%, at least 90%, at least 95%, at least 98% or at least 99% sequence identity with ERKCCVECPPCP (SEQ ID NO: 159). In some embodiments, the hinge region comprises the sequence ELKTPLGDTTHTCPRCP(EPKSCDTPPPCPRCP)$_3$, (SEQ ID NO: 160) or comprises an amino acid sequence having at least 50%, at least 55%, at least 60%, at least 65%, at least 70%, at least 75%, at least 80%, at least 85%, at least 90%, at least 95%, at least 98% or at least 99% sequence identity with ELKTPLGDTTHTCPRCP(EPKSCDTPP-PCPRCP)$_3$ (SEQ ID NO: 160). In some embodiments, the hinge region comprises the sequence ESKYGPPCPSCP (SEQ ID NO: 161), or comprises an amino acid sequence having at least 50%, at least 55%, at least 60%, at least 65%, at least 70%, at least 75%, at least 80%, at least 85%, at least 90%, at least 95%, at least 98% or at least 99% sequence identity with ESKYGPPCPSCP (SEQ ID NO: 161).

## CAR constructs and immunoresponsive cells expressing CARs

The disclosure also provides isolated polynucleotides encoding the CARs described herein.

Unless otherwise specified, a "polynucleotide encoding an amino acid sequence" includes all polynucleotide sequences that are degenerate versions of each other and that encode the same amino acid sequence. The phrase polynucleotide that encodes a protein or a RNA may also include introns to the extent that the polynucleotide encoding the protein may in some variants contain an intron(s) that is spliced into the expressed protein.

The disclosure also provides an expression vector comprising the nucleic acid molecule encoding the CAR of the disclosure.

The term "expression vector" refers to a vector comprising a recombinant polynucleotide comprising expression control sequences operatively linked to a nucleotide sequence to be expressed. An expression vector comprises sufficient cis-acting elements for expression; other elements for expression can be supplied by the host cell or in an *in vitro* expression system. Expression vectors include all those known in the art, including cosmids, plasmids (e.g., naked or contained in liposomes) and viruses (e.g., lentiviruses, retroviruses, adenoviruses, and adeno-associated viruses) that incorporate the recombinant polynucleotide.

The disclosure also provides isolated immunoresponsive cells comprising the CAR of the disclosure. In some embodiments, the isolated immunoresponsive cell is transduced with the CAR, for example, the CAR is constitutively expressed on the surface of the immunoresponsive cell. In certain embodiments, the isolated immunoresponsive cell is further transduced with at least one co-stimulatory ligand such that the immunoresponsive cell expresses the at least one co-stimulatory ligand. In certain embodiments, the at least one co-stimulatory ligand is selected from the group consisting of 4-1BBL, CD48, CD70, CD80, CD86, OX40L, TNFRSF14, and combinations thereof. In certain embodiments, the isolated immunoresponsive cell is further transduced with at least one cytokine such that the immunoresponsive cell secretes the at least one cytokine. In certain embodiments, the at least cytokine is selected from the group consisting of IL-2, IL-3, IL-6, IL-7, IL-11, IL-12, IL-15, IL-17, IL-21, and combinations thereof. In some embodiments, the isolated immunoresponsive cell is selected from the group consisting of a T lymphocyte (T cell), a Natural Killer (NK) cell, a cytotoxic T lymphocyte (CTL), a regulatory T cell (Treg), a human embryonic stem cell, a lymphoid progenitor cell, a T cell-precursor cell, and a pluripotent stem cell from which lymphoid cells may be differentiated.

In some embodiments, the isolated immunoresponsive cell is the T cell.

For purposes herein, the T cell can be any T cell, such as a cultured T cell, e.g., a primary T cell, or a T cell from a cultured T cell line, e.g., Jurkat, SupT1, etc., or a T cell obtained from a mammal. If obtained from a mammal, the T cell can be obtained from numerous sources, including but not limited to bone marrow, blood, lymph node, the thymus, or other tissues or fluids. T cells can also be enriched for or purified. The T cell may be a human T cell. The T cell may be a T cell isolated from a human. The T cell can be any type of T cell and can be of any developmental stage, including but not limited to, $CD4^+/CD8^+$ double positive T cells, $CD8^+$ T cells (e.g., cytotoxic T cells), $CD4^+$ helper T cells, e.g., Th1 and Th2 cells, peripheral blood mononuclear cells (PBMCs), peripheral blood leukocytes (PBLs), tumor

infiltrating cells, memory T cells, naive T cells, and the like. The T cell may be a $CD8^+$ T cell or a $CD4^+$ T cell.

In some embodiments, the isolated immunoresponsive cell is the NK cell.

In some embodiments, the isolated immunoresponsive cell is the CTL.

In some embodiments, the isolated immunoresponsive cell is the Treg.

In some embodiments, the isolated immunoresponsive cell is the human embryonic stem cell. In some embodiments, the isolated immunoresponsive cell is the lymphoid progenitor cell.

In some embodiments, the isolated immunoresponsive cell is the pluripotent stem cell.

In one embodiment, the CAR T cells of the disclosure can be generated by introducing a lentiviral vector comprising a desired CAR, for example, a CAR comprising an extracellular domain comprising an antigen binding domain that binds CD33, CD8α hinge and transmembrane domain, and human 4-1BB and CD3-zeta signaling domains, into the cells. The CAR T cells of the disclosure are able to replicate *in vivo* resulting in long-term persistence that can lead to sustained tumor control.

Embodiments of the invention further provide host cells comprising any of the recombinant expression vectors described herein. As used herein, the term "host cell" refers to any type of cell that can contain the recombinant expression vector. The host cell can be a eukaryotic cell, e.g., plant, animal, or algae, fungi, or can be a prokaryotic cell, e.g., bacteria or protozoa. The host cell can be a cultured cell or a primary cell, i.e., isolated directly from an organism, e.g., a human. The host cell can be an adherent cell or a suspended cell, i.e., a cell that grows in suspension. Suitable host cells are known in the art and include, for instance, DH5α *E.coli* cells, Chinese hamster ovarian cells, monkey VERO cells, COS cells, HEK293 cells, and the like. For purposes of amplifying or replicating the recombinant expression vector, the host cell may be a prokaryotic cell, e.g., a DH5α cell. For purposes of producing a recombinant CAR, polypeptide, or protein, the host cell may be a mammalian cell. The host cell may be a human cell. While the host cell can be of any cell type, can originate from any type of tissue, and can be of any developmental stage, the host cell may be a peripheral blood lymphocyte (PBL). The host cell may be a T cell.

Also provided are a population of cells comprising at least one host cell described herein. The population of cells can be a heterogeneous population comprising the host cell comprising any of the recombinant expression vectors described, in addition to at least one other cell, e.g., a host cell (e.g., a T cell), which does not comprise any of the recombinant expression vectors, or a cell other than a T cell, e.g., a B cell, a macrophage, an erythrocyte, a neutrophil, a hepatocyte, an endothelial cell, an epithelial cell, a muscle cell, a brain cell, etc. Alternatively, the population of cells can be a substantially homogeneous population, in which the population comprises mainly host cells (e.g., consisting essentially of) comprising the recombinant expression vector. The population also can be a clonal population of cells, in which all cells of the population are clones of a single host cell comprising a recombinant expression vector, such that all cells of the population comprise the recombinant expression vector. In one embodiment, the population of cells is a clonal population comprising host cells comprising a recombinant expression vector as described herein.

**Polynucleotides, host cells and vectors**

The disclosure also provides an isolated polynucleotide encoding any of the CD33 binding proteins of the disclosure. The CD33 binding protein includes the antigen binding domains that bind CD33, the proteins comprising the antigen binding domains that bind CD33, the multispecific proteins that comprise the antigen binding domains that bind CD33 and the chimeric antigen receptors (CAR) comprising the antigen binding domains that bind CD33 of the disclosure.

The invention also provides an isolated polynucleotide encoding any of CD33 biding proteins or fragments thereof.

The invention also provides an isolated polynucleotide encoding the VH of SEQ ID NOs: 52, 53, 54, 55, 56, 57, 58, 59, 60, 61, 262, 263, 264, 291, 292, 293, 294, 295, 296, 297, 298, 299, 300, 301, 302, 303, 304, 305, 306, 307, 308, 309, 310, 337, 338, 339, 340, 341, 342, 343, 344, 345, 346, 347, 348, 349, 350, 351, 352, 367, 368, 369, 370, 371, 372, 373, 374, 375, 376, 377, 378, 379, 380, 381, 382, or 382.

In certain embodiments, the isolated polynucleotide encodes the VH of SEQ ID NO: 53.

In other embodiments, the isolated polynucleotide encodes the VH of SEQ ID NO: 56.

In other embodiments, the isolated polynucleotide encodes the VH of SEQ ID NO: 59.

In other embodiments, the isolated polynucleotide encodes the VH of SEQ ID NO: 262.

In other embodiments, the isolated polynucleotide encodes the VH of SEQ ID NO: 263.

In other embodiments, the isolated polynucleotide encodes the VH of SEQ ID NO: 264.

The invention also provides an isolated polynucleotide encoding the VL of SEQ ID NOs: 81, 82, 83, 84, 85, 86, 87,88, 271, 272, 273, 311, 312, 313, 314, 315, 316, 317, 318, 319, 320, 321, 322, 323, 324, 325, 326, 327, 328, 329, 330, 331, 332, 333, 334, 335, 336, 353, 354, 355, 356, 357, 358, 359, 360, 361, 362, 363, 364, 365, 366, 383, 384, 385, 386, 387, 388, 389, 390, 391, 392, 393, 394, 395, 396, 397 or 398.

In certain embodiments, the isolated polynucleotide encodes the VL of SEQ ID NO: 82.

In other embodiments, the isolated polynucleotide encodes the VL of SEQ ID NO: 85.

In other embodiments, the isolated polynucleotide encodes the VL of SEQ ID NO: 87.

In other embodiments, the isolated polynucleotide encodes the VL of SEQ ID NO: 271.

In other embodiments, the isolated polynucleotide encodes the VL of SEQ ID NO: 272.

In other embodiments, the isolated polynucleotide encodes the VL of SEQ ID NO: 273. The invention also provides an isolated polynucleotide encoding the VH of SEQ ID NO: 95, 96, 97, 170, 171, 175, 179, 181, or 96.

The invention also provides an isolated polynucleotide encoding the VL of SEQ ID NO: 105, 106, 107, 185, 188, 192, 196, 200, or 202.

The invention also provides an isolated polynucleotide encoding the VH of SEQ ID NO: 18, 19, 20, 21, 22, 23, 24, 25, 26, or 27.

In some embodiments, the isolated polynucleotide encodes a VH of SEQ ID NOs: 52, 53, 54, 55, 56, 57, 58, 59, 60, 61, 262, 263, 264, 291, 292, 293, 294, 295, 296, 297, 298, 299, 300, 301, 302, 303, 304, 305, 306, 307, 308, 309, 310, 337, 338, 339, 340, 341, 342, 343, 344, 345, 346, 347, 348, 349, 350, 351, 352, 367, 368, 369, 370, 371, 372, 373, 374, 375, 376, 377, 378, 379, 380, 381, 382, or 382 and a VL of SEQ ID NOs: 81, 82, 83, 84, 85, 86, 87, 88, 271, 272, 273, 311, 312, 313, 314, 315, 316, 317, 318, 319, 320, 321, 322, 323, 324, 325, 326, 327, 328, 329, 330, 331, 332, 333, 334, 335, 33δ, 353, 354, 355, 356, 357, 358, 359, 360, 361, 362, 363, 364, 365, 366, 383, 384, 385, 386, 387, 388, 389, 390, 391, 392, 393, 394, 395, 396, 397 or 398.

The invention also provides for an isolated polynucleotide encoding

a) the VH of SEQ ID NO: 52 and the VL of SEQ ID NO: 81;
b) the VH of SEQ ID NO: 53 and the VL of SEQ ID NO: 82;
c) the VH of SEQ ID NO: 54 and the VL of SEQ ID NO: 83;
d) the VH of SEQ ID NO: 55 and the VL of SEQ ID NO: 84;
e) the VH of SEQ ID NO: 56 and the VL of SEQ ID NO: 85;
f) the VH of SEQ ID NO: 57 and the VL of SEQ ID NO: 86;
g) the VH of SEQ ID NO: 58 and the VL of SEQ ID NO: 87;
h) the VH of SEQ ID NO: 59 and the VL of SEQ ID NO: 87;
i) the VH of SEQ ID NO: 60 and the VL of SEQ ID NO: 87;
j) the VH of SEQ ID NO: 61 and the VL of SEQ ID NO: 88;
k) the VH of SEQ ID NO: 262 and the VL of SEQ ID NO: 271;
l) the VH of SEQ ID NO: 263 and the VL of SEQ ID NO: 272 or
m) the VH of SEQ ID NO: 264 and the VL of SEQ ID NO: 273.

In certain embodiments, the isolated polynucleotide encodes the VH of SEQ ID NO: 53 and the VL of SEQ ID NO: 82. In other embodiments, the isolated polynucleotide encodes the VH of SEQ ID NO: 56 and the VL of SEQ ID NO: 85. In other embodiments, the isolated polynucleotide encodes the VH of SEQ ID NO: 59 and the VL of SEQ ID NO: 87. In other embodiments, the isolated polynucleotide encodes the VH of SEQ ID NO: 262 and the VL of SEQ ID NO: 271. In other embodiments, the isolated polynucleotide encodes the VH of SEQ ID NO: 263 and the VL of SEQ ID NO: 272. In other embodiments, the isolated polynucleotide encodes the VH of SEQ ID NO: 264 and the VL of SEQ ID NO: 273.

The invention also provides for an isolated polynucleotide encoding

a) the VH of SEQ ID NO: 95 and the VL of SEQ ID NO: 105;
b) the VH of SEQ ID NO: 96 and the VL of SEQ ID NO: 106;
c) the VH of SEQ ID NO: 97 and the VL of SEQ ID NO: 107;
d) the VH of SEQ ID NO: 170 and the VL of SEQ ID NO: 185;
e) the VH of SEQ ID NO: 171 and the VL of SEQ ID NO: 188;
f) the VH of SEQ ID NO: 175 and the VL of SEQ ID NO: 192;
g) the VH of SEQ ID NO: 179 and the VL of SEQ ID NO: 196;
h) the VH of SEQ ID NO: 181 and the VL of SEQ ID NO: 200; or
i) the VH of SEQ ID NO: 96 and the VL of SEQ ID NO: 202.

The invention also provides an isolated polynucleotide encoding the polypeptide of SEQ ID NOs: 18, 19, 20, 21, 22, 23, 24, 25, 26, 27, 203, 204, 205, 206, 207, 208, 209, 2i0, 211, 212, 213, 214, 215, 216, 217, 218, 219, 220 221, 274, 275 or 276.The invention also provides an isolated polynucleotide encoding the polypeptide of SEQ ID NO: 18.

The invention also provides an isolated polynucleotide encoding the polypeptide of SEQ ID NO: 19.

The invention also provides an isolated polynucleotide encoding the polypeptide of SEQ ID NO: 20.

The invention also provides an isolated polynucleotide encoding the polypeptide of SEQ ID NO: 21.

The invention also provides an isolated polynucleotide encoding the polypeptide of SEQ ID NO: 22.

The invention also provides an isolated polynucleotide encoding the polypeptide of SEQ ID NO: 23.
The invention also provides an isolated polynucleotide encoding the polypeptide of SEQ ID NO: 24.
The invention also provides an isolated polynucleotide encoding the polypeptide of SEQ ID NO: 25.
The invention also provides an isolated polynucleotide encoding the polypeptide of SEQ ID NO: 26.
The invention also provides an isolated polynucleotide encoding the polypeptide of SEQ ID NO: 27.
The invention also provides an isolated polynucleotide encoding the polypeptide of SEQ ID NO: 203.
The invention also provides an isolated polynucleotide encoding the polypeptide of SEQ ID NO: 204.
The invention also provides an isolated polynucleotide encoding the polypeptide of SEQ ID NO: 205.
The invention also provides an isolated polynucleotide encoding the polypeptide of SEQ ID NO: 206.
The invention also provides an isolated polynucleotide encoding the polypeptide of SEQ ID NO: 207.
The invention also provides an isolated polynucleotide encoding the polypeptide of SEQ ID NO: 208.
The invention also provides an isolated polynucleotide encoding the polypeptide of SEQ ID NO: 209.
The invention also provides an isolated polynucleotide encoding the polypeptide of SEQ ID NO: 210.
The invention also provides an isolated polynucleotide encoding the polypeptide of SEQ ID NO: 211.
The invention also provides an isolated polynucleotide encoding the polypeptide of SEQ ID NO: 212.
The invention also provides an isolated polynucleotide encoding the polypeptide of SEQ ID NO: 213.
The invention also provides an isolated polynucleotide encoding the polypeptide of SEQ ID NO: 214.
The invention also provides an isolated polynucleotide encoding the polypeptide of SEQ ID NO: 215.
The invention also provides an isolated polynucleotide encoding the polypeptide of SEQ ID NO: 216.
The invention also provides an isolated polynucleotide encoding the polypeptide of SEQ ID NO: 217.
The invention also provides an isolated polynucleotide encoding the polypeptide of SEQ ID NO: 218.
The invention also provides an isolated polynucleotide encoding the polypeptide of SEQ ID NO: 219.
The invention also provides an isolated polynucleotide encoding the polypeptide of SEQ ID NO: 220.
The invention also provides an isolated polynucleotide encoding the polypeptide of SEQ ID NO: 221.
The invention also provides an isolated polynucleotide encoding the polypeptide of SEQ ID NO: 274.
The invention also provides an isolated polynucleotide encoding the polypeptide of SEQ ID NO: 275.
The invention also provides an isolated polynucleotide encoding the polypeptide of SEQ ID NO: 276.
In certain embodiments, the isolated polynucleotide encodes the polypeptide of SEQ ID NO: 213. In other embodiments, the isolated polynucleotide encodes the polypeptide of SEQ ID NO: 216. In other embodiments, the isolated polynucleotide encodes the polypeptide of SEQ ID NO: 219. In other embodiments, the isolated polynucleotide encodes the polypeptide of SEQ ID NO: 274. In other embodiments, the isolated polynucleotide encodes the polypeptide of SEQ ID NO: 275. In other embodiments, the isolated polynucleotide encodes the polypeptide of SEQ ID NO: 276.
The disclosure also provides an isolated or purified polynucleotide that encodes any of the CAR of the disclosure, or functional portions or functional variants thereof.
The invention also provides an isolated polynucleotide encoding the CAR comprising the amino acid sequence of SEQ ID NOs: 18, 19, 20, 21, 22, 23, 24, 25, 26, 27, 203, 204, 205, 206, 207, 208, 209, 210, 211, 212, 213, 214, 215, 216, 217, 218, 219, 220, 221, 274, 275 or 276.
The invention also provides an isolated polynucleotide encoding the CAR comprising the amino acid sequence of SEQ ID NO: 18.
The invention also provides an isolated polynucleotide encoding the CAR comprising the amino acid sequence of SEQ ID NO: 19.
The invention also provides an isolated polynucleotide encoding the CAR comprising the amino acid sequence of SEQ ID NO: 20.
The invention also provides an isolated polynucleotide encoding the CAR comprising the amino acid sequence of SEQ ID NO: 21.
The invention also provides an isolated polynucleotide encoding the CAR comprising the amino acid sequence of SEQ ID NO: 22.
The invention also provides an isolated polynucleotide encoding the CAR comprising the amino acid sequence of SEQ ID NO: 23.
The invention also provides an isolated polynucleotide encoding the CAR comprising the amino acid sequence of SEQ ID NO: 24.
The invention also provides an isolated polynucleotide encoding the CAR comprising the amino acid sequence of SEQ ID NO: 25.
The invention also provides an isolated polynucleotide encoding the CAR comprising the amino acid sequence of SEQ ID NO: 26.
The invention also provides an isolated polynucleotide encoding the CAR comprising the amino acid sequence of SEQ ID NO: 27.
The invention also provides an isolated polynucleotide encoding the CAR comprising the amino acid sequence

of SEQ ID NO: 203.

The invention also provides an isolated polynucleotide encoding the CAR comprising the amino acid sequence of SEQ ID NO: 204.

The invention also provides an isolated polynucleotide encoding the CAR comprising the amino acid sequence of SEQ ID NO: 205.

The invention also provides an isolated polynucleotide encoding the CAR comprising the amino acid sequence of SEQ ID NO: 206.

The invention also provides an isolated polynucleotide encoding the CAR comprising the amino acid sequence of SEQ ID NO: 207.

The invention also provides an isolated polynucleotide encoding the CAR comprising the amino acid sequence of SEQ ID NO: 208.

The invention also provides an isolated polynucleotide encoding the CAR comprising the amino acid sequence of SEQ ID NO: 209.

The invention also provides an isolated polynucleotide encoding the CAR comprising the amino acid sequence of SEQ ID NO: 210.

The invention also provides an isolated polynucleotide encoding the CAR comprising the amino acid sequence of SEQ ID NO: 211.

The invention also provides an isolated polynucleotide encoding the CAR comprising the amino acid sequence of SEQ ID NO: 212.

The invention also provides an isolated polynucleotide encoding the CAR comprising the amino acid sequence of SEQ ID NO: 213.

The invention also provides an isolated polynucleotide encoding the CAR comprising the amino acid sequence of SEQ ID NO: 214.

The invention also provides an isolated polynucleotide encoding the CAR comprising the amino acid sequence of SEQ ID NO: 215.

The invention also provides an isolated polynucleotide encoding the CAR comprising the amino acid sequence of SEQ ID NO: 216.

The invention also provides an isolated polynucleotide encoding the CAR comprising the amino acid sequence of SEQ ID NO: 217.

The invention also provides an isolated polynucleotide encoding the CAR comprising the amino acid sequence of SEQ ID NO: 218.

The invention also provides an isolated polynucleotide encoding the CAR comprising the amino acid sequence of SEQ ID NO: 219.

The invention also provides an isolated polynucleotide encoding the CAR comprising the amino acid sequence of SEQ ID NO: 220.

The invention also provides an isolated polynucleotide encoding the CAR comprising the amino acid sequence of SEQ ID NO: 221.

The invention also provides an isolated polynucleotide encoding the CAR comprising the amino acid sequence of SEQ ID NO: 274.

The invention also provides an isolated polynucleotide encoding the CAR comprising the amino acid sequence of SEQ ID NO: 275.

The invention also provides an isolated polynucleotide encoding the CAR comprising the amino acid sequence of SEQ ID NO: 276.

Some embodiments of the disclosure also provide an isolated or purified nucleic acid comprising a polynucleotide which is complementary to the polynucleotides encoding the CD33 binding proteins of the disclosure or polynucleotides which hybridize under stringent conditions to the polynucleotides encoding the CD33 binding proteins of the disclosure.

The polynucleotides which hybridize under stringent conditions may hybridize under high stringency conditions. By "high stringency conditions" is meant that the polynucleotide specifically hybridizes to a target sequence (the nucleotide sequence of any of the nucleic acids described herein) in an amount that is detectably stronger than non-specific hybridization. High stringency conditions include conditions which would distinguish a polynucleotide with an exact complementary sequence, or one containing only a few scattered mismatches from a random sequence that happened to have a few small regions (e.g., 3-12 bases) that matched the nucleotide sequence. Such small regions of complementarity are more easily melted than a full-length complement of 14-17 or more bases, and high stringency hybridization makes them easily distinguishable. Relatively high stringency conditions would include, for example, low salt and/or high temperature conditions, such as provided by about 0.02-0.1 M NaCl or the equivalent, at temperatures of about 50-70° C. Such high stringency conditions tolerate little, if any, mismatch between the nucleotide sequence and the template or target strand, and are particularly suitable for detecting expression of any of the CARs described herein. It is generally appreciated that conditions can be rendered more stringent by the addition of increasing amounts of formamide.

The polynucleotide sequences of the disclosure may be operably linked to one or more regulatory elements, such as a promoter or enhancer, that allow expression of the nucleotide sequence in the intended host cell. The polynucleotide may be a cDNA. The promoter bay be a strong, weak, tissue-specific, inducible or developmental-specific promoter. Exemplary promoters that may be used are hypoxanthine phosphoribosyl transferase (HPRT), adenosine deaminase, pyruvate kinase, beta-actin, human myosin, human hemoglobin, human muscle creatine, and others. In addition, many viral promoters function constitutively in eukaryotic cells and are suitable for use with the described embodiments. Such viral promoters include Cytomegalovirus (CMV) immediate early promoter, the early and late promoters of SV40, the Mouse Mammary Tumor Virus (MMTV) promoter, the long terminal repeats (LTRs) of Maloney leukemia virus, Human Immunodeficiency Virus (HIV), Epstein Barr Virus (EBV), Rous Sarcoma Virus (RSV), and other retroviruses, and the thymidine kinase promoter of Herpes Simplex Virus. Inducible promoters such as the metallothionein promoter, tetracycline-inducible promoter, doxycycline-inducible promoter, promoters that contain one or more interferon-stimulated response elements (ISRE) such as protein kinase R 2',5'-oligoadenylate synthetases, Mx genes, ADAR1, and the like may also be sued.

The invention also provides a vector comprising the polynucleotide of the invention. The disclosure also provide an expression vector comprising the polynucleotide of the invention. Such vectors may be plasmid vectors, viral vectors, vectors for baculovirus expression, transposon based vectors or any other vector suitable for introduction of the synthetic polynucleotide of the invention into a given organism or genetic background by any means. Polynucleotides encoding the CD33 binding proteins of the disclosure may be operably linked to control sequences in the expression vector(s) that ensure the expression of the CD33 binding proteins. Such regulatory elements may include a transcriptional promoter, sequences encoding suitable mRNA ribosomal binding sites, and sequences that control the termination of transcription and translation. Expression vectors may also include one or more nontranscribed elements such as an origin of replication, a suitable promoter and enhancer linked to the gene to be expressed, other 5' or 3' flanking nontranscribed sequences, 5' or 3' nontranslated sequences (such as necessary ribosome binding sites), a polyadenylation site, splice donor and acceptor sites, or transcriptional termination sequences. An origin of replication that confers the ability to replicate in a host may also be incorporated.

The expression vectors can comprise naturally-occurring or non-naturally-occurring internucleotide linkages, or both types of linkages. The non-naturally occurring or altered nucleotides or internucleotide linkages do not hinder the transcription or replication of the vector.

Once the vector has been incorporated into the appropriate host, the host is maintained under conditions suitable for high level expression of the CD33 binding proteins of the disclosure encoded by the incorporated polynucleotides. The transcriptional and translational control sequences in expression vectors to be used in transforming vertebrate cells may be provided by viral sources. Exemplary vectors may be constructed as described by Okayama and Berg, 3 *Mol. Cell. Biol.* 280 (1983).

Vectors of the disclosure may also contain one or more Internal Ribosome Entry Site(s) (IRES). Inclusion of an IRES sequence into fusion vectors may be beneficial for enhancing expression of some proteins. In some embodiments, the vector system will include one or more polyadenylation sites (e.g., SV40), which may be upstream or downstream of any of the aforementioned nucleic acid sequences. Vector components may be contiguously linked or arranged in a manner that provides optimal spacing for expressing the gene products (i.e., by the introduction of "spacer" nucleotides between the ORFs) or positioned in another way. Regulatory elements, such as the IRES motif, may also be arranged to provide optimal spacing for expression.

Vectors of the disclosure may be circular or linear. They may be prepared to contain a replication system functional in a prokaryotic or eukaryotic host cell. Replication systems can be derived, e.g., from ColE1, SV40, 2$\mu$ plasmid, $\lambda$, bovine papilloma virus, and the like.

The recombinant expression vectors can be designed for either transient expression, for stable expression, or for both. Also, the recombinant expression vectors can be made for constitutive expression or for inducible expression.

Further, the recombinant expression vectors can be made to include a suicide gene. As used herein, the term "suicide gene" refers to a gene that causes the cell expressing the suicide gene to die. The suicide gene can be a gene that confers sensitivity to an agent, e.g., a drug, upon the cell in which the gene is expressed, and causes the cell to die when the cell is contacted with or exposed to the agent. Suicide genes are known in the art and include, for example, the Herpes Simplex Virus (HSV) thymidine kinase (TK) gene, cytosine deaminase, purine nucleoside phosphorylase, and nitroreductase.

The vectors may also comprise selection markers, which are well known in the art. Selection markers include positive and negative selection marker. Marker genes include biocide resistance, e.g., resistance to antibiotics, heavy metals, etc., complementation in an auxotrophic host to provide prototrophy, and the like. Exemplary marker genes include antibiotic resistance genes (e.g., neomycin resistance gene, a hygromycin resistance gene, a kanamycin resistance gene, a tetracycline resistance gene, a penicillin resistance gene, histidinol resistance gene, histidinol x resistance gene), glutamine synthase genes, HSV-TK, HSV-TK derivatives for ganciclovir selection, or bacterial purine nucleoside

phosphorylase gene for 6-methylpurine selection (Gadi et al., 7 Gene Ther. 1738-1743 (2000)). A nucleic acid sequence encoding a selection marker or the cloning site may be upstream or downstream of a nucleic acid sequence encoding a polypeptide of interest or cloning site.

Exemplary vectors that may be used are Bacterial: pBs, phagescript, PsiX174, pBluescript SK, pBs KS, pNH8a, pNH16a, pNH18a, pNH46a (Stratagene, La Jolla, Calif., USA); pTrc99A, pKK223-3, pKK233-3, pDR540, and pRIT5 (Pharmacia, Uppsala, Sweden). Eukaryotic: pWLneo, pSV2cat, pOG44, PXR1, pSG (Stratagene) pSVK3, pBPV, pMSG and pSVL (Pharmacia), pEE6.4 (Lonza) and pEE12.4 (Lonza). Additional vectors include the pUC series (Fermentas Life Sciences, Glen Burnie, Md.), the pBluescript series (Stratagene, LaJolla, Calif.), the pET series (Novagen, Madison, Wis.), the pGEX series (Pharmacia Biotech, Uppsala, Sweden), and the pEX series (Clontech, Palo Alto, Calif.). Bacteriophage vectors, such as λGT10, λGT11, λEMBL4, and λNM1149, λZapII (Stratagene) can be used. Exemplary plant expression vectors include pBI01, pBI01.2, pBI121, pBI101.3, and pBIN19 (Clontech). Exemplary animal expression vectors include pEUK-Cl, pMAM, and pMAMneo (Clontech). The expression vector may be a viral vector, e.g., a retroviral vector, e.g., a gamma retroviral vector.

In some embodiments, the vector comprises the polynucleotide encoding the VH of SEQ ID NOs: 52, 53, 54, 55, 56, 57, 58, 59, 60, 61, 262, 263, 264, 291, 292, 293, 294, 295, 296, 297, 298, 299, 300, 301, 302, 303, 304, 305, 306, 307, 308, 309, 310, 337, 338, 339, 340, 341, 342, 343, 344, 345, 346, 347, 348, 349, 350, 351, 352, 367, 368, 369, 370, 371, 372, 373, 374, 375, 376, 377, 378, 379, 380, 381, 382, or 382.

In certain embodiments, the vector comprises the polynucleotide encoding the VH of SEQ ID NO: 53. In other embodiments, the vector comprises the polynucleotide encoding the VH of SEQ ID NO: 56. In other embodiments, the vector comprises the polynucleotide encoding the VH of SEQ ID NO: 59. In other embodiments, the vector comprises the polynucleotide encoding the VH of SEQ ID NO: 262. In other embodiments, the vector comprises the polynucleotide encoding the VH of SEQ ID NO: 263. In other embodiments, the vector comprises the polynucleotide encoding the VH of SEQ ID NO: 264.

In some embodiments, the vector comprises the polynucleotide encoding the VL of SEQ ID NOs: 81, 82, 83, 84, 85, 86, 87, 88, 271, 272, 273, 311, 312, 313, 314, 315, 316, 317, 318, 319, 320, 321, 322, 323, 324, 325, 326, 327, 328, 329, 330, 331, 332, 333, 334, 335, 336, 353, 354, 355, 356, 357, 358, 359, 360, 361, 362, 363, 364, 365, 366, 383, 384, 385, 386, 387, 388, 389, 390, 391, 392, 393, 394, 395, 396, 397 or 398.

In certain embodiments, the vector comprises the polynucleotide encoding the VL of SEQ ID NO: 82. In other embodiments, the vector comprises the polynucleotide encoding the VL of SEQ ID NO: 85. In other embodiments, the vector comprises the polynucleotide encoding the VL of SEQ ID NO: 87. In other embodiments, the vector comprises the polynucleotide encoding the VL of SEQ ID NO: 271. In other embodiments, the vector comprises the polynucleotide encoding the VL of SEQ ID NO: 272. In other embodiments, the vector comprises the polynucleotide encoding the VL of SEQ ID NO: 273.

In some embodiments, the vector comprises the polynucleotide encoding the VH of SEQ ID NO: 95, 96, 97, 170, 171, 175, 179, 181, or 96.

In some embodiments, the vector comprises the polynucleotide encoding the VL of SEQ ID NO: 105, 106, 107, 185, 188, 192, 196, 200, or 202.

In some embodiments, the vector comprises the polynucleotide encoding the VH of SEQ ID NO: 18, 19, 20, 21, 22, 23, 24, 25, 26, or 27.

In some embodiments, the vector comprises the polynucleotide encoding the VH of SEQ ID NOs: 52, 53, 54, 55, 56, 57, 58, 59, 60, 61, 262, 263, 264, 291, 292, 293, 294, 295, 296, 297, 298, 299, 300, 301, 302, 303, 304, 305, 306, 307, 308, 309, 310, 337, 338, 339, 340, 341, 342, 343, 344, 345, 346, 347, 348, 349, 350, 351, 352, 367, 368, 369, 370, 371, 372, 373, 374, 375, 376, 377, 378, 379, 380, 381, 382, or 382 and the VL of SEQ ID NOs: 81, 82, 83, 84, 85, 86, 87, 88, 271, 272, 273, 311, 312, 313, 314, 315, 316, 317, 318, 319, 320, 321, 322, 323, 324, 325, 326, 327, 328, 329, 330, 331, 332, 333, 334, 335, 336, 353, 354, 355, 356, 357, 358, 359, 360, 361, 362, 363, 364, 365, 366, 383, 384, 385, 386, 387, 388, 389, 390, 391, 392, 393, 394, 395, 396, 397 or 398.

In some embodiments, the vector comprises the polynucleotide encoding for an isolated polynucleotide encoding

a) the VH of SEQ ID NO: 52 and the VL of SEQ ID NO: 81;
b) the VH of SEQ ID NO: 53 and the VL of SEQ ID NO: 82;
c) the VH of SEQ ID NO: 54 and the VL of SEQ ID NO: 83;
d) the VH of SEQ ID NO: 55 and the VL of SEQ ID NO: 84;
e) the VH of SEQ ID NO: 56 and the VL of SEQ ID NO: 85;
f) the VH of SEQ ID NO: 57 and the VL of SEQ ID NO: 86;
g) the VH of SEQ ID NO: 58 and the VL of SEQ ID NO: 87;
h) the VH of SEQ ID NO: 59 and the VL of SEQ ID NO: 87;
i) the VH of SEQ ID NO: 60 and the VL of SEQ ID NO: 87;

j) the VH of SEQ ID NO: 61 and the VL of SEQ ID NO: 88;
k) the VH of SEQ ID NO: 262 and the VL of SEQ ID NO: 271;
l) the VH of SEQ ID NO: 263 and the VL of SEQ ID NO: 272;
m) the VH of SEQ ID NO: 264 and the VL of SEQ ID NO: 273.

In certain embodiments, the vector comprises the polynucleotide encoding the VH of SEQ ID NO: 53 and the VL of SEQ ID NO: 82. In other embodiments, the vector comprises the polynucleotide encoding the VH of SEQ ID NO: 56 and the VL of SEQ ID NO: 85. In other embodiments, the vector comprises the polynucleotide encoding the VH of SEQ ID NO: 59 and the VL of SEQ ID NO: 87. In other embodiments, the vector comprises the polynucleotide encoding the VH of SEQ ID NO: 262 and the VL of SEQ ID NO: 271. In other embodiments, the vector comprises the polynucleotide encoding the VH of SEQ ID NO: 263 and the VL of SEQ ID NO: 272. In other embodiments, the vector comprises the polynucleotide encoding the VH of SEQ ID NO: 264 and the VL of SEQ ID NO: 273.

In some embodiments, the vector comprises the polynucleotide encoding for an isolated polynucleotide encoding

a) the VH of SEQ ID NO: 95 and the VL of SEQ ID NO: 105;
b) the VH of SEQ ID NO: 96 and the VL of SEQ ID NO: 106;
c) the VH of SEQ ID NO: 97 and the VL of SEQ ID NO: 107;
d) the VH of SEQ ID NO: 170 and the VL of SEQ ID NO: 185;
e) the VH of SEQ ID NO: 171 and the VL of SEQ ID NO: 188;
f) the VH of SEQ ID NO: 175 and the VL of SEQ ID NO: 192;
g) the VH of SEQ ID NO: 179 and the VL of SEQ ID NO: 196;
h) the VH of SEQ ID NO: 181 and the VL of SEQ ID NO: 200; or
i) the VH of SEQ ID NO: 96 and the VL of SEQ ID NO: 202.

In some embodiments, the vector comprises the polynucleotide encoding the polypeptide of SEQ ID NOs: SEQ ID NOs: 18, 19, 20, 21, 22, 23, 24, 25, 26, 27, 203, 204, 205, 206, 207, 208, 209, 210, 211, 212, 213, 214, 215, 216, 217, 218, 219, 220, 221, 274, 275 or 276.

In some embodiments, the vector comprises the polynucleotide encoding the polypeptide of SEQ ID NO: 18.

In some embodiments, the vector comprises the polynucleotide encoding the polypeptide of SEQ ID NO: 19.

In some embodiments, the vector comprises the polynucleotide encoding the polypeptide of SEQ ID NO: 20.

In some embodiments, the vector comprises the polynucleotide encoding the polypeptide of SEQ ID NO: 21.

In some embodiments, the vector comprises the polynucleotide encoding the polypeptide of SEQ ID NO: 22.

In some embodiments, the vector comprises the polynucleotide encoding the polypeptide of SEQ ID NO: 23.

In some embodiments, the vector comprises the polynucleotide encoding the polypeptide of SEQ ID NO: 24.

In some embodiments, the vector comprises the polynucleotide encoding the polypeptide of SEQ ID NO: 25.

In some embodiments, the vector comprises the polynucleotide encoding the polypeptide of SEQ ID NO: 26.

In some embodiments, the vector comprises the polynucleotide encoding the polypeptide of SEQ ID NO: 27.

In some embodiments, the vector comprises the polynucleotide encoding the polypeptide of SEQ ID NO: 203.

In some embodiments, the vector comprises the polynucleotide encoding the polypeptide of SEQ ID NO: 204.

In some embodiments, the vector comprises the polynucleotide encoding the polypeptide of SEQ ID NO: 205.

In some embodiments, the vector comprises the polynucleotide encoding the polypeptide of SEQ ID NO: 206.

In some embodiments, the vector comprises the polynucleotide encoding the polypeptide of SEQ ID NO: 207.

In some embodiments, the vector comprises the polynucleotide encoding the polypeptide of SEQ ID NO: 208.

In some embodiments, the vector comprises the polynucleotide encoding the polypeptide of SEQ ID NO: 209.

In some embodiments, the vector comprises the polynucleotide encoding the polypeptide of SEQ ID NO: 210.

In some embodiments, the vector comprises the polynucleotide encoding the polypeptide of SEQ ID NO: 211.

In some embodiments, the vector comprises the polynucleotide encoding the polypeptide of SEQ ID NO: 212.

In some embodiments, the vector comprises the polynucleotide encoding the polypeptide of SEQ ID NO: 213.

In some embodiments, the vector comprises the polynucleotide encoding the polypeptide of SEQ ID NO: 214.

In some embodiments, the vector comprises the polynucleotide encoding the polypeptide of SEQ ID NO: 215.

In some embodiments, the vector comprises the polynucleotide encoding the polypeptide of SEQ ID NO: 216.

In some embodiments, the vector comprises the polynucleotide encoding the polypeptide of SEQ ID NO: 217.

In some embodiments, the vector comprises the polynucleotide encoding the polypeptide of SEQ ID NO: 218.

In some embodiments, the vector comprises the polynucleotide encoding the polypeptide of SEQ ID NO: 219.

In some embodiments, the vector comprises the polynucleotide encoding the polypeptide of SEQ ID NO: 220.

In some embodiments, the vector comprises the polynucleotide encoding the polypeptide of SEQ ID NO: 221.

In some embodiments, the vector comprises the polynucleotide encoding the polypeptide of SEQ ID NO: 274.

In some embodiments, the vector comprises the polynucleotide encoding the polypeptide of SEQ ID NO: 275.

In some embodiments, the vector comprises the polynucleotide encoding the polypeptide of SEQ ID NO: 276.

In some embodiments, the vector comprises the polynucleotide encoding any of the CAR of the disclosure, or functional portions or functional variants thereof.

In some embodiments, the vector comprises the polynucleotide encoding the CAR comprising the amino acid sequence of SEQ ID NOs: 18, 19, 20, 21, 22, 23, 24, 25, 26, 27, 203, 204, 205, 206, 207, 208, 209, 210, 211, 212, 213, 214, 215, 216, 217, 218, 219, 220, 221, 274, 275 or 276.

In some embodiments, the vector comprises the polynucleotide encoding the CAR comprising the amino acid sequence of SEQ ID NO: 18.

In some embodiments, the vector comprises the polynucleotide encoding the CAR comprising the amino acid sequence of SEQ ID NO: 19.

In some embodiments, the vector comprises the polynucleotide encoding the CAR comprising the amino acid sequence of SEQ ID NO: 20.

In some embodiments, the vector comprises the polynucleotide encoding the CAR comprising the amino acid sequence of SEQ ID NO: 21.

In some embodiments, the vector comprises the polynucleotide encoding the CAR comprising the amino acid sequence of SEQ ID NO: 22.

In some embodiments, the vector comprises the polynucleotide encoding the CAR comprising the amino acid sequence of SEQ ID NO: 23.

In some embodiments, the vector comprises the polynucleotide encoding the CAR comprising the amino acid sequence of SEQ ID NO: 24.

In some embodiments, the vector comprises the polynucleotide encoding the CAR comprising the amino acid sequence of SEQ ID NO: 25.

In some embodiments, the vector comprises the polynucleotide encoding the CAR comprising the amino acid sequence of SEQ ID NO: 26.

In some embodiments, the vector comprises the polynucleotide encoding the CAR comprising the amino acid sequence of SEQ ID NO: 27.

In some embodiments, the vector comprises the polynucleotide encoding the CAR comprising the amino acid sequence of SEQ ID NO: 203.

In some embodiments, the vector comprises the polynucleotide encoding the CAR comprising the amino acid sequence of SEQ ID NO: 204.

In some embodiments, the vector comprises the polynucleotide encoding the CAR comprising the amino acid sequence of SEQ ID NO: 205.

In some embodiments, the vector comprises the polynucleotide encoding the CAR comprising the amino acid sequence of SEQ ID NO: 206.

In some embodiments, the vector comprises the polynucleotide encoding the CAR comprising the amino acid sequence of SEQ ID NO: 207.

In some embodiments, the vector comprises the polynucleotide encoding the CAR comprising the amino acid sequence of SEQ ID NO: 208.

In some embodiments, the vector comprises the polynucleotide encoding the CAR comprising the amino acid sequence of SEQ ID NO: 209.

In some embodiments, the vector comprises the polynucleotide encoding the CAR comprising the amino acid sequence of SEQ ID NO: 210.

In some embodiments, the vector comprises the polynucleotide encoding the CAR comprising the amino acid sequence of SEQ ID NO: 211.

In some embodiments, the vector comprises the polynucleotide encoding the CAR comprising the amino acid sequence of SEQ ID NO: 212.

In some embodiments, the vector comprises the polynucleotide encoding the CAR comprising the amino acid sequence of SEQ ID NO: 213.

In some embodiments, the vector comprises the polynucleotide encoding the CAR comprising the amino acid sequence of SEQ ID NO: 214.

In some embodiments, the vector comprises the polynucleotide encoding the CAR comprising the amino acid sequence of SEQ ID NO: 215.

In some embodiments, the vector comprises the polynucleotide encoding the CAR comprising the amino acid sequence of SEQ ID NO: 216.

In some embodiments, the vector comprises the polynucleotide encoding the CAR comprising the amino acid sequence of SEQ ID NO: 217.

In some embodiments, the vector comprises the polynucleotide encoding the CAR comprising the amino acid

sequence of SEQ ID NO: 218.

In some embodiments, the vector comprises the polynucleotide encoding the CAR comprising the amino acid sequence of SEQ ID NO: 219.

In some embodiments, the vector comprises the polynucleotide encoding the CAR comprising the amino acid sequence of SEQ ID NO: 220.

In some embodiments, the vector comprises the polynucleotide encoding the CAR comprising the amino acid sequence of SEQ ID NO: 221.

In some embodiments, the vector comprises the polynucleotide encoding the CAR comprising the amino acid sequence of SEQ ID NO: 274.

In some embodiments, the vector comprises the polynucleotide encoding the CAR comprising the amino acid sequence of SEQ ID NO: 275.

In some embodiments, the vector comprises the polynucleotide encoding the CAR comprising the amino acid sequence of SEQ ID NO: 276.

The invention also provides for a host cell comprising one or more vectors of the invention. "Host cell" refers to a cell into which a vector has been introduced. It is understood that the term host cell is intended to refer not only to the particular subject cell but to the progeny of such a cell, and also to a stable cell line generated from the particular subject cell. Because certain modifications may occur in succeeding generations due to either mutation or environmental influences, such progeny may not be identical to the parent cell, but are still included within the scope of the term "host cell" as used herein. Such host cells may be eukaryotic cells, prokaryotic cells, plant cells or archeal cells. *Escherichia coli,* bacilli, such as *Bacillus subtilis,* and other enterobacteriaceae, such as Salmonella, Serratia, and various Pseudomonas species are examples of prokaryotic host cells. Other microbes, such as yeast, are also useful for expression. Saccharomyces (e.g., S. cerevisiae) and Pichia are examples of suitable yeast host cells. Exemplary eukaryotic cells may be of mammalian, insect, avian or other animal origins. Mammalian eukaryotic cells include immortalized cell lines such as hybridomas or myeloma cell lines such as SP2/0 (American Type Culture Collection (ATCC), Manassas, VA, CRL-1581), NS0 (European Collection of Cell Cultures (ECACC), Salisbury, Wiltshire, UK, ECACC No. 85110503), FO (ATCC CRL-1646) and Ag653 (ATCC CRL-1580) murine cell lines. An exemplary human myeloma cell line is U266 (ATTC CRL-TIB-196). Other useful cell lines include those derived from Chinese Hamster Ovary (CHO) cells such as CHO-K1 SV (Lonza Biologics, Walkersville, MD), CHO-KI (ATCC CRL-61) or DG44.

The disclosure also provides a method of producing the CD33 binding protein of the disclosure comprising culturing the host cell of the disclosure in conditions that the K2 binding protein is expressed, and recovering the CD33 binding protein produced by the host cell. Methods of making proteins and purifying them are known. Once synthesized (either chemically or recombinantly), the CD33 binding proteins may be purified according to standard procedures, including ammonium sulfate precipitation, affinity columns, column chromatography, high performance liquid chromatography (HPLC) purification, gel electrophoresis, and the like (see generally Scopes, Protein Purification (Springer-Verlag, N.Y., (1982)). A subject protein may be substantially pure, e.g., at least about 80% to 85% pure, at least about 85% to 90% pure, at least about 90% to 95% pure, or at least about 98% to 99%, or more, pure, e.g., free from contaminants such as cell debris, macromolecules, etc. other than the subject protein.

The polynucleotides encoding the CD33 binding proteins of the disclosure may be incorporated into vectors using standard molecular biology methods. Host cell transformation, culture, antibody expression and purification are done using well known methods.

Accordingly, the invention provides a method of producing a polypeptide comprising expressing a nucleotide of the invention that encodes for a polypeptide of the invention. In certain embodiments, the polynucleotide encodes the VH of SEQ ID NO: 53 and the VL of SEQ ID NO: 82. In other embodiments, the polynucleotide encodes the VH of SEQ ID NO: 56 and the VL of SEQ ID NO: 85. In other embodiments, the polynucleotide encodes the VH of SEQ ID NO: 59 and the VL of SEQ ID NO: 87. In other embodiments, the polynucleotide encodes the VH of SEQ ID NO: 262 and the VL of SEQ ID NO: 82. In other embodiments, the polynucleotide encodes the VH of SEQ ID NO: 53 and the VL of SEQ ID NO: 82. In other embodiments, the polynucleotide encodes the VH of SEQ ID NO: 53 and the VL of SEQ ID NO: 82.

In certain embodiments of this method, the polynucleotide encodes a polypeptide comprising the VH of SEQ ID NO: 53 and a polypeptide comprising the VL of SEQ ID NO: 82. In other embodiments, the polynucleotide encodes a polypeptide comprising the VH of SEQ ID NO: 56 and a polypeptide comprising the VL of SEQ ID NO: 85. In other embodiments, the polynucleotide encodes a polypeptide comprising the VH of SEQ ID NO: 59 and a polypeptide comprising the VL of SEQ ID NO: 87. In other embodiments, the polynucleotide encodes a polypeptide comprising the VH of SEQ ID NO: 262 and a polypeptide comprising the VL of SEQ ID NO: 271. In other embodiments, the polynucleotide encodes a polypeptide comprising the VH of SEQ ID NO: 263 and a polypeptide comprising the VL of SEQ ID NO: 272. In other embodiments, the polynucleotide encodes a polypeptide comprising the VH of SEQ ID NO: 264 and a polypeptide comprising the VL of SEQ ID NO: 273.

Where the polypeptide is formed of separate chains which are encoded by different nucleic acids, the invention provides a method of producing a polypeptide comprising expressing nucleotides of the invention that encode for a

polypeptide of the invention. In certain embodiments, a first polynucleotide of the invention encodes a polypeptide comprising the VH of SEQ ID NO: 53 and a second polynucleotide of the invention encodes a polypeptide comprising the VL of SEQ ID NO: 82. In other embodiments, a first polynucleotide of the invention encodes a polypeptide comprising the VH of SEQ ID NO: 56 and a second polynucleotide of the invention encodes a polypeptide comprising the VL of SEQ ID NO: 85. In other embodiments, a first polynucleotide of the invention encodes a polypeptide comprising the VH of SEQ ID NO: 59 and a second polynucleotide of the invention encodes a polypeptide comprising the VL of of SEQ ID NO: 87. In other embodiments, a first polynucleotide of the invention encodes a polypeptide comprising the VH of SEQ ID NO: 262 and a second polynucleotide of the invention encodes a polypeptide comprising the VL of SEQ ID NO: 271. In other embodiments, a first polynucleotide of the invention encodes a polypeptide comprising the VH of SEQ ID NO: 263and a second polynucleotide of the invention encodes a polypeptide comprising the VL of SEQ ID NO: 272. In other embodiments, a first polynucleotide of the invention encodes a polypeptide comprising the VH of SEQ ID NO: 264 and a second polynucleotide of the invention encodes a polypeptide comprising the VL of SEQ ID NO: 273.

Modified nucleotides may be used to generate the polynucleotides of the disclosure. Exemplary modified nucleotides are 5-fluorouracil, 5-bromouracil, 5-chlorouracil, 5-iodouracil, hypoxanthine, xanthine, 4-acetylcytosine, 5-(carboxyhydroxymethyl) uracil, carboxymethylaminomethyl-2-thiouridine, 5-carboxymethylaminomethyluracil, dihydrouracil, $N^6$-substituted adenine, 7-methylguanine, 5-methylaminomethyluracil, 5-methoxyaminomethyl-2-thiouracil, beta-D-mannosylqueosine, 5"-methoxycarboxymethyluracil, 5-methoxyuracil, 2-methylthio-$N^6$-isopentenyladenine, uracil-5-oxyacetic acid (v), wybutoxosine, pseudouracil, queuosine, beta-D-galactosylqueosine, inosine, $N^6$-isopentenyladenine, 1-methylguanine, 1-methylinosine, 2,2-dimethylguanine, 2-methyladenine, 2-methylguanine, 3-methylcytosine, 5-methylcytosine, 2-thiocytosine, 5-methyl-2-thiouracil, 2-thiouracil, 4-thiouracil, 5-methyluracil, uracil-5-oxyacetic acid methylester, 3-(3-amino-3-N-2-carboxypropyl) uracil, and 2,6-diaminopurine.

**Pharmaceutical Compositions/Administration**

The disclosure also provides a pharmaceutical composition comprising the CD33 binding protein of the disclosure and a pharmaceutically acceptable carrier.

The disclosure also provides a pharmaceutical composition comprising the antigen binding domain that binds CD33 of the disclosure and a pharmaceutically acceptable carrier.

The disclosure also provides a pharmaceutical composition comprising the protein comprising the antigen binding domain that binds CD33 of the disclosure and a pharmaceutically acceptable carrier.

The disclosure also provides a pharmaceutical composition comprising the multispecific protein comprising the antigen binding domain that binds CD33 of the disclosure and a pharmaceutically acceptable carrier.

The disclosure also provides a pharmaceutical composition comprising the CAR comprising the antigen binding domain that binds CD33 of the disclosure and a pharmaceutically acceptable carrier. For therapeutic use, the CD33 binding protein of the disclosure may be prepared as pharmaceutical compositions containing an effective amount of the antibody as an active ingredient in a pharmaceutically acceptable carrier. "Carrier" refers to a diluent, adjuvant, excipient, or vehicle with which the antibody of the invention is administered. Such vehicles may be liquids, such as water and oils, including those of petroleum, animal, vegetable or synthetic origin, such as peanut oil, soybean oil, mineral oil, sesame oil and the like. For example, 0.4% saline and 0.3% glycine may be used. These solutions are sterile and generally free of particulate matter. They may be sterilized by conventional, well-known sterilization techniques (e.g., filtration). The compositions may contain pharmaceutically acceptable auxiliary substances as required to approximate physiological conditions such as pH adjusting and buffering agents, stabilizing, thickening, lubricating and coloring agents, etc. The concentration of the antibodies of the invention in such pharmaceutical formulation may vary, from less than about 0.5%, usually to at least about 1% to as much as 15 or 20% by weight and may be selected primarily based on required dose, fluid volumes, viscosities, etc., according to the mode of administration selected. Suitable vehicles and formulations, inclusive of other human proteins, e.g., human serum albumin, are described, for example, in e.g. Remington: The Science and Practice of Pharmacy, 21st Edition, Troy, D.B. ed., Lipincott Williams and Wilkins, Philadelphia, PA 2006, Part 5, Pharmaceutical Manufacturing pp 691-1092, See especially pp. 958-989.

The mode of administration of the CD33 binding protein of the disclosure may be any suitable route such as parenteral administration, e.g., intradermal, intramuscular, intraperitoneal, intravenous or subcutaneous, transmucosal (oral, intranasal, intravaginal, rectal) or other means appreciated by the skilled artisan, as well known in the art.

The CD33 binding protein of the disclosure of the invention may also be administered prophylactically in order to reduce the risk of developing a disease such as cancer.

Thus, a pharmaceutical composition of the invention for intramuscular injection may be prepared to contain 1 ml sterile buffered water, and between about 1 ng to about 100 mg/kg, e.g. about 50 ng to about 30 mg/kg or more preferably, about 5 mg to about 25 mg/kg, of the CD33 binding protein of the disclosure of the invention.

In embodiments of the present disclosure, the CAR-expressing cells may be provided in compositions, e.g., suitable pharmaceutical composition(s) comprising the CAR-expressing cells and a pharmaceutically acceptable carrier.

In one aspect, the present disclosure provides pharmaceutical compositions comprising an effective amount of a lymphocyte expressing one or more of the CARs described and a pharmaceutically acceptable excipient. Pharmaceutical compositions of the present disclosure may comprise a CAR-expressing cell, e.g., a plurality of CAR-expressing cells, as described herein, in combination with one or more pharmaceutically or physiologically acceptable carriers, excipients or diluents. A pharmaceutically acceptable carrier can be an ingredient in a pharmaceutical composition, other than an active ingredient, which is nontoxic to the subject.

A pharmaceutically acceptable carrier can include a buffer, excipient, stabilizer, or preservative. Examples of pharmaceutically acceptable carriers are solvents, dispersion media, coatings, antibacterial and antifungal agents, isotonic and absorption delaying agents, and the like that are physiologically compatible, such as salts, buffers, antioxidants, saccharides, aqueous or non-aqueous carriers, preservatives, wetting agents, surfactants or emulsifying agents, or combinations thereof. The amounts of pharmaceutically acceptable carrier(s) in the pharmaceutical compositions may be determined experimentally based on the activities of the carrier(s) and the desired characteristics of the formulation, such as stability and/or minimal oxidation.

Pharmaceutical compositions may comprise buffers such as acetic acid, citric acid, formic acid, succinic acid, phosphoric acid, carbonic acid, malic acid, aspartic acid, histidine, boric acid, Tris buffers, HEPPSO, HEPES, neutral buffered saline, phosphate buffered saline and the like; carbohydrates such as glucose, mannose, sucrose or dextrans, mannitol; proteins; polypeptides or amino acids such as glycine; antioxidants; chelating agents such as EDTA or glutathione; adjuvants (e.g., aluminum hydroxide); antibacterial and antifungal agents; and preservatives.

Pharmaceutical compositions of the present disclosure can be formulated for a variety of means of parenteral or non-parenteral administration. In one embodiment, the compositions can be formulated for infusion or intravenous administration. Pharmaceutical compositions disclosed herein can be provided, for example, as sterile liquid preparations, e.g., isotonic aqueous solutions, emulsions, suspensions, dispersions, or viscous compositions, which may be buffered to a desirable pH. Formulations suitable for oral administration can include liquid solutions, capsules, sachets, tablets, lozenges, and troches, powders liquid suspensions in an appropriate liquid and emulsions.

The term "pharmaceutically acceptable," as used herein with regard to pharmaceutical compositions, means approved by a regulatory agency of the Federal or a state government or listed in the U.S. Pharmacopeia or other generally recognized pharmacopeia for use in animals and/or in humans.

## METHODS OF TREATMENT AND USES

The disclosure also provides a method of treating a CD33 expressing cancer in a subject, comprising administering a therapeutically effective amount of the antigen binding domain that binds CD33 of the disclosure to the subject in need thereof for a time sufficient to treat the CD33 expressing cancer.

The disclosure also provides a method of treating a CD33 expressing cancer in a subject, comprising administering a therapeutically effective amount of the protein comprising the antigen binding domain that binds CD33 of the disclosure to the subject for a time sufficient to treat the CD33 expressing cancer

The disclosure also provides a method of treating a CD33 expressing cancer in a subject, comprising administering a therapeutically effective amount of the multispecific protein comprising the antigen binding domain that binds CD33 of the disclosure to the subject for a time sufficient to treat the CD33 expressing cancer.

In certain embodiments, the method comprises administering a therapeutically effective amount of a multispecific protein (for example, a bispecific protein) comprising a first antigen binding domain that binds CD33 of the disclosure and a second antigen binding domain that binds TRGV9 to the subject for a time sufficient to treat the CD33 expressing cancer. In certain such embodiments, the first antigen binding domain that binds CD33 is an scFv and, optionally, the second binding domain that binds TRGV9 is a VHH.

In some embodiments, the method comprises administering a therapeutically effective amount of a multispecific protein (for example, a bispecific protein) comprising a first antigen binding domain that binds CD33 and a second antigen binding domain that binds TRGV9 to the subject for a time sufficient to treat the CD33 expressing cancer, wherein the first antigen binding domain that binds CD33 comprises:

the VH of SEQ ID NO: 53 and the VL of SEQ ID NO: 82;
the VH of SEQ ID NO: 56 and the VL of SEQ ID NO: 85;
the VH of SEQ ID NO: 59 and the VL of SEQ ID NO: 87;
the VH of SEQ ID NO: 262 and the VL of SEQ ID NO: 271;
the VH of SEQ ID NO: 263 and the VL of SEQ ID NO: 272; or
the VH of SEQ ID NO: 264 and the VL of SEQ ID NO: 273.

In certain such embodiments, the first antigen binding domain that binds CD33 is an scFv and, optionally, the second binding domain that binds TRGV9 is a VHH.

In some embodiments, the method comprises administering a therapeutically effective amount of a multispecific protein (for example, a bispecific protein) comprising a first antigen binding domain that binds CD33 and a second antigen binding domain that binds TRGV9 to the subject for a time sufficient to treat the CD33 expressing cancer, wherein the first antigen binding domain that binds CD33 comprises the amino acid sequence of SEQ ID NOs: 213, 216, 219, 274, 275 or 276. In certain such embodiments the second binding domain that binds TRGV9 is a VHH.

The disclosure also provides a method of treating a CD33 expressing cancer in a subject, comprising administering a therapeutically effective amount of the CAR comprising the antigen binding domain that binds CD33 of the disclosure to the subject for a time sufficient to treat the CD33 expressing cancer.

The disclosure also provides a method of treating a CD33 expressing cancer in a subject, comprising administering a therapeutically effective amount of the immunoconjugate comprising the antigen binding domain that binds CD33 of the disclosure to the subject for a time sufficient to treat the CD33 expressing cancer.

The disclosure also provides a method of treating a CD33 expressing cancer in a subject, comprising administering a therapeutically effective amount of the pharmaceutical composition comprising the antigen binding domain that binds CD33 of the disclosure to the subject for a time sufficient to treat the CD33 expressing cancer. In certain such embodiments, the pharmaceutical composition comprises a multispecific protein (for example, a bispecific protein) comprising a first antigen binding domain that binds CD33 of the disclosure and a second antigen binding domain that binds TRGV9. In some embodiments the first antigen binding domain that binds CD33 is an scFv and, optionally, the second binding domain that binds TRGV9 is a VHH.

In some embodiments, the method comprises administering a therapeutically effective amount of a pharmaceutical composition comprising a multispecific protein (for example, a bispecific protein), wherein the multispecific protein comprises a first antigen binding domain that binds CD33 and a second antigen binding domain that binds TRGV9 to the subject for a time sufficient to treat the CD33 expressing cancer, wherein the first antigen binding domain that binds CD33 comprises:

the VH of SEQ ID NO: 53 and the VL of SEQ ID NO: 82;
the VH of SEQ ID NO: 56 and the VL of SEQ ID NO: 85;
the VH of SEQ ID NO: 59 and the VL of SEQ ID NO: 87;
the VH of SEQ ID NO: 262 and the VL of SEQ ID NO: 271;
the VH of SEQ ID NO: 263 and the VL of SEQ ID NO: 272; or
the VH of SEQ ID NO: 264 and the VL of SEQ ID NO: 273.

In certain such embodiments, the first antigen binding domain that binds CD33 is an scFv and, optionally, the second binding domain that binds TRGV9 is a VHH.

In some embodiments, the method comprises administering a therapeutically effective amount of a pharmaceutical composition comprising a multispecific protein (for example, a bispecific protein), wherein the multispecific protein comprises a first antigen binding domain that binds CD33 and a second antigen binding domain that binds TRGV9 to the subject for a time sufficient to treat the CD33 expressing cancer, wherein the first antigen binding domain that binds CD33 comprise the amino acid sequence of SEQ ID NOs: 213, 216, 219, 274, 275 or 276. In certain such embodiments the second binding domain that binds TRGV9 is a VHH.

The disclosure also provides a method of administering a genetically modified T cell expressing a CAR for the treatment of a subject having cancer or at risk of having cancer using lymphocyte infusion.

In at least one embodiment, autologous lymphocyte infusion is used in the treatment. Autologous PBMCs are collected from a subject in need of treatment and T cells are activated and expanded using the methods described herein and known in the art and then infused back into the subject.

In one aspect, the disclosure relates generally to the treatment of a subject at risk of developing cancer. The invention also includes treating a malignancy or an autoimmune disease in which chemotherapy and/or immunotherapy results in significant immunosuppression in a subject, thereby increasing the risk of the subject developing cancer.

The disclosure also provides a method of treating a noncancerous condition in a subject at risk of developing a cancerous condition, comprising administering the antigen binding domain that bind CD33 of the disclosure to the subject to treat the noncancerous condition.

The disclosure also provides a method of treating a noncancerous condition in a subject at risk of developing a cancerous condition, comprising administering the protein comprising the antigen binding domain that bind CD33 of the disclosure to the subject to treat the noncancerous condition.

The disclosure also provides a method of treating a noncancerous condition in a subject at risk of developing a cancerous condition, comprising administering the multispecific protein comprising the antigen binding domain that bind CD33 of the disclosure to the subject to treat the noncancerous condition.

The disclosure also provides a method of treating a noncancerous condition in a subject at risk of developing a cancerous condition, comprising administering the immunoconjugate of the disclosure to the subject to treat the

noncancerous condition.

The disclosure also provides a method of treating a noncancerous condition in a subject at risk of developing a cancerous condition, comprising administering the pharmaceutical composition of the disclosure to the subject to treat the noncancerous condition.

The disclosure also provides a method of treating a noncancerous condition in a subject at risk of developing a cancerous condition, comprising administering the CAR of the disclosure to the subject to treat the noncancerous condition.

The disclosure also provides a method of preventing CD33 expressing cancer in a subject, comprising administering a therapeutically effective amount of any antigen binding domain that binds CD33 of the disclosure to the subject for a time sufficient to prevent the CD33 expressing cancer.

The disclosure also provides a method of preventing a CD33 expressing cancer in a subject, comprising administering a therapeutically effective amount of the protein comprising any antigen binding domain that binds CD33 of the disclosure to the subject for a time sufficient to prevent the CD33 expressing cancer

The disclosure also provides a method of preventing a CD33 expressing cancer in a subject, comprising administering a therapeutically effective amount of the multispecific protein comprising the antigen binding domain that binds CD33 of the disclosure to the subject for a time sufficient to prevent the CD33 expressing cancer.

The disclosure also provides a method of preventing a CD33 expressing cancer in a subject, comprising administering a therapeutically effective amount of the CAR comprising the antigen binding domain that binds CD33 of the disclosure to the subject for a time sufficient to prevent the CD33 expressing cancer.

The disclosure also provides a method of preventing a CD33 expressing cancer in a subject, comprising administering a therapeutically effective amount of the immunoconjugate comprising the antigen binding domain that binds CD33 of the disclosure to the subject for a time sufficient to prevent the CD33 expressing cancer.

The disclosure also provides a method of preventing a CD33 expressing cancer in a subject, comprising administering a therapeutically effective amount of the pharmaceutical composition comprising any antigen binding domain that binds CD33 of the disclosure to the subject for a time sufficient to prevent the CD33 expressing cancer.

The disclosure also provides a method of reducing the amount of CD33 expressing tumor cells in a subject, comprising administering any antigen binding domain that binds CD33 of the disclosure to the subject for a time sufficient to reduce the amount of CD33 expressing tumor cells.

The disclosure also provides a method of reducing the amount of CD33 expressing tumor cells in a subject, comprising administering the protein comprising the antigen binding domain that binds CD33 of the disclosure to the subject for a time sufficient to reduce the amount of CD33 expressing tumor cells.

The disclosure also provides a method of reducing the amount of CD33 expressing tumor cells in a subject, comprising administering the multispecific protein comprising the antigen binding domain that binds CD33 of the disclosure to the subject for a time sufficient to reduce the amount of CD33 expressing tumor cells.

The disclosure also provides a method of reducing the amount of CD33 expressing tumor cells in a subject, comprising administering the CAR that comprises any antigen binding domain that binds CD33 of the disclosure to the subject for a time sufficient to reduce the amount of CD33 expressing tumor cells.

The disclosure also provides a method of reducing the amount of CD33 expressing tumor cells in a subject, comprising administering the immunoconjugate of the disclosure to the subject for a time sufficient to reduce the amount of CD33 expressing tumor cells.

The disclosure also provides a method of reducing the amount of CD33 expressing tumor cells in a subject, comprising administering the pharmaceutical composition of the disclosure to the subject for a time sufficient to reduce the amount of CD33 expressing tumor cells.

In some embodiments, the CD33 expressing cancer is hematologic cancer.

In some embodiments, the CD33 expressing cancer is leukemia.

In some embodiments, the CD33 expressing cancer is lymphoma.

In some embodiments, the CD33 expressing cancer is multiple myeloma.

In some embodiments, the CD33 expressing cancer is acute myeloid leukemia (AML).

In some embodiments, the CD33 expressing cancer is myelodysplastic syndrome (MDS).

In some embodiments, the CD33 expressing cancer is acute lymphocytic leukemia (ALL).

In some embodiments, the CD33 expressing cancer is diffuse large B-cell lymphoma (DLBCL). In some embodiments, the CD33 expressing cancer is chronic myeloid leukemia (CML).

In some embodiments, the CD33 expressing cancer is blastic plasmacytoid dendritic cell neoplasm (DPDCN).

In certain embodiments, the CD33 expressing cancer is acute myeloid leukemia (AML).

In some embodiments, the cancer is relapsed, refractory, malignant, or any combination thereof.

The disclosure also provides a method of treating hematologic cancer (e.g., leukemia (in particular, AML), lymphoma or multiple myeloma) in a subject, comprising administering a therapeutically effective amount of the multispecific protein comprising the antigen binding domain that binds CD33 to the subject for a time sufficient to treat the

hematologic cancer, wherein the antigen binding domain that bind CD33 comprises:

the VH of SEQ ID NO: 52 and the VL of SEQ ID NO: 81;
the VH of SEQ ID NO: 53 and the VL of SEQ ID NO: 82;
the VH of SEQ ID NO: 54 and the VL of SEQ ID NO: 83;
the VH of SEQ ID NO: 55 and the VL of SEQ ID NO: 84;
the VH of SEQ ID NO: 56 and the VL of SEQ ID NO: 85;
the VH of SEQ ID NO: 57 and the VL of SEQ ID NO: 86;
the VH of SEQ ID NO: 58 and the VL of SEQ ID NO: 87;
the VH of SEQ ID NO: 59 and the VL of SEQ ID NO: 87;
the VH of SEQ ID NO: 60 and the VL of SEQ ID NO: 87;
the VH of SEQ ID NO: 61 and the VL of SEQ ID NO: 88;
the VH of SEQ ID NO: 262 and the VL of SEQ ID NO: 271;
the VH of SEQ ID NO: 263 and the VL of SEQ ID NO: 272; or
the VH of SEQ ID NO: 264 and the VL of SEQ ID NO: 273.

In certain embodiments, the antigen binding domain that binds CD33 comprises the VH of SEQ ID NO: 53 and the VL of SEQ ID NO: 82. In other embodiments, the antigen binding domain that binds CD33 comprises the VH of SEQ ID NO: 56 and the VL of SEQ ID NO: 85. In other embodiments, the antigen binding domain that binds CD33 comprises the VH of SEQ ID NO: 59 and the VL of SEQ ID NO: 87. In other embodiments, the antigen binding domain that binds CD33 comprises the VH of SEQ ID NO: 262 and the VL of SEQ ID NO: 271. In other embodiments, the antigen binding domain that binds CD33 comprises the VH of SEQ ID NO: 263 and the VL of SEQ ID NO: 272. In other embodiments, the antigen binding domain that binds CD33 comprises the VH of SEQ ID NO: 264 and the VL of SEQ ID NO: 273.

The disclosure also provides a method of treating hematologic cancer (e.g., leukemia (in particular, AML), lymphoma or multiple myeloma) in a subject, comprising administering a therapeutically effective amount of the multispecific protein comprising the antigen binding domain that binds CD33 to the subject for a time sufficient to treat the hematologic cancer, wherein the antigen binding domain that bind CD33 comprises:

the VH of SEQ ID NO: 95 and the VL of SEQ ID NO: 105;
the VH of SEQ ID NO: 96 and the VL of SEQ ID NO: 106;
the VH of SEQ ID NO: 97 and the VL of SEQ ID NO: 107;
the VH of SEQ ID NO: 170 and the VL of SEQ ID NO: 185;
the VH of SEQ ID NO: 171 and the VL of SEQ ID NO: 188;
the VH of SEQ ID NO: 175 and the VL of SEQ ID NO: 192;
the VH of SEQ ID NO: 179 and the VL of SEQ ID NO: 196;
the VH of SEQ ID NO: 181 and the VL of SEQ ID NO: 200; or
the VH of SEQ ID NO: 96 and the VL of SEQ ID NO: 202.

The disclosure also provides a method of treating hematologic cancer (e.g., leukemia (in particular, AML), lymphoma or multiple myeloma) in a subject, comprising administering a therapeutically effective amount of the multispecific protein comprising the antigen binding domain that binds CD33 to the subject for a time sufficient to treat the hematologic cancer, wherein the antigen binding domain that bind CD33 comprises:

the VH of SEQ ID NO: 18;
the VH of SEQ ID NO: 19;
the VH of SEQ ID NO: 20;
the VH of SEQ ID NO: 21;
the VH of SEQ ID NO: 22;
the VH of SEQ ID NO: 23;
the VH of SEQ ID NO: 24;
the VH of SEQ ID NO: 25;
the VH of SEQ ID NO: 26; or
the VH of SEQ ID NO: 27.

The disclosure also provides a method of treating hematologic cancer (e.g., leukemia (in particular, AML), lymphoma or multiple myeloma) in a subject, comprising administering a therapeutically effective amount of the multispecific protein comprising the antigen binding domain that binds CD33 to the subject for a time sufficient to treat the hematologic cancer, wherein the antigen binding domain that binds CD33 comprises the amino acid sequence of SEQ

ID NOs: 18, 19, 20, 21, 22, 23, 24, 25, 26, 27, 203, 204, 205, 206, 207, 208, 209, 210, 211, 212, 213, 214, 215, 216, 217, 218, 219, 220, 221, 274, 275 or 276.

In certain embodiments, the antigen binding domain that binds CD33 comprises the amino acid sequence of SEQ ID NOs: 213, 216, 219, 274, 275 or 276.

In one aspect, the present disclosure provides methods of preventing cancer, the methods comprising administering an amount of a lymphocyte expressing one or more of the CARs described to a subject in need thereof.

In one aspect, the present disclosure provides methods of treating a subject having cancer, the methods comprising administering a therapeutically effective amount of a lymphocyte expressing one or more of the CARs described to a subject in need thereof, whereby the lymphocyte induces or modulates killing of cancer cells in the subject.

In another aspect, the present disclosure provides methods of reducing tumor burden in a subject having cancer, the methods comprising administering a therapeutically effective amount of a lymphocyte expressing one or more of the CARs described herein to a subject in need thereof, whereby the lymphocyte induces killing of hematologic cancer cells in the subject. In another aspect, the present disclosure provides methods of increasing survival of a subject having cancer, the methods comprising administering a therapeutically effective amount of a lymphocyte expressing one or more of the CARs described to a subject in need thereof, whereby the survival of the subject is lengthened. Generally, the lymphocytes expressing the CAR(s) induce killing of hematologic cancer cells in the subject and result in reduction or eradication of the hematologic tumors/cancer cells in the subject. A non-limiting list of hematologic cancers, inclusive of metastatic lesions, that can be targeted, includes leukemia, lymphoma, multiple myeloma. In some embodiments, the hematologic cancer is acute myeloid leukemia (AML), myelodysplastic syndrome (MDS), acute lymphocytic leukemia (ALL), diffuse large B-cell lymphoma (DLBCL), chronic myeloid leukemia (CML) or blastic plasmacytoid dendritic cell neoplasm (DPDCN).

In one aspect, methods of treating a subject having cancer are provided that comprise administering a therapeutically effective amount of a lymphocyte expressing a CAR, the CAR having an extracellular antigen-binding domain that binds the CD33 antigen, to a subject in need thereof, whereby the lymphocyte induces killing of cancer cells in the subject.

In one aspect, a method of targeted killing of a cancer cell is disclosed, the method comprising contacting the cancer cell with a lymphocyte expressing one or more of the CARs described, whereby the lymphocyte induces killing of the cancer cell. A non-limiting list of cancer cells, inclusive of hematologic cancer cells, that can be targeted includes leukemia, lymphoma, multiple myeloma. In some embodiments, the hematologic cancer is acute myeloid leukemia (AML), myelodysplastic syndrome (MDS), acute lymphocytic leukemia (ALL), diffuse large B-cell lymphoma (DLBCL), chronic myeloid leukemia (CML) or blastic plasmacytoid dendritic cell neoplasm (DPDCN).

When a therapeutically effective amount is indicated, the precise amount of the CARs or CAR-Ts of the present disclosure to be administered can be determined by a physician with consideration of individual differences in age, weight, tumor size, extent of infection or metastasis, and condition of the subject. It can generally be stated that a pharmaceutical composition comprising the T cells described herein may be administered at a dosage of about $10^4$ to about $10^{10}$ cells/kg body weight, in some instances about $10^5$ to about $10^6$ cells/kg body weight, including all integer values within those ranges. In some embodiments, a pharmaceutical composition comprising the T cells described herein may be administered at a dosage of about $10^6$ cells/kg body weight. T cell compositions may also be administered multiple times at these dosages. The cells can be administered by using infusion techniques that are commonly known in immunotherapy (see, e.g., Rosenberg et al., New Eng. J. of Med. 319:1676, 1988).

Delivery systems useful in the context of the CAR-T of the invention may include time-released, delayed release, and sustained release delivery systems such that the delivery of the T cell compositions occurs prior to, and with sufficient time to cause, sensitization of the site to be treated. The composition can be used in conjunction with other therapeutic agents or therapies. Such systems can avoid repeated administrations of the composition, thereby increasing convenience to the subject and the physician, and may be particularly suitable for certain composition embodiments of the invention.

Many types of release delivery systems are available and known to those of ordinary skill in the art. They include polymer base systems such as poly(lactide-glycolide), copolyoxalates, polyesteramides, polyorthoesters, polycaprolactones, polyhydroxybutyric acid, and polyanhydrides. Microcapsules of the foregoing polymers containing drugs are described in, for example, U.S. Pat. No. 5,075,109. Delivery systems also include non-polymer systems that are lipids including sterols such as cholesterol, cholesterol esters, and fatty acids or neutral fats such as mono-di- and tri-glycerides; sylastic systems; peptide based systems; hydrogel release systems; wax coatings; compressed tablets using conventional binders and excipients; partially fused implants; and the like. Specific examples include, but are not limited to: (a) erosional systems in which the active composition is contained in a form within a matrix such as those described in U.S. Patent Nos. 4,452,775; 4,667,014; 4,748,034; and 5,239,660 and (b) diffusional systems in which an active component permeates at a controlled rate from a polymer such as described in U.S. Patent Nos. 3,854,480 and 3,832,253. In addition, pump-based hardware delivery systems can be used, some of which are adapted for implantation.

In certain aspects, it may be desirable to administer activated T cells to a subject and then subsequently redraw

blood (or have an apheresis performed), activate the T cells according to the present disclosure, and reinfuse the subject with these activated and expanded T cells. This process can be carried out multiple times every few weeks. In certain aspects, T cells can be activated from blood draws of from 10 cc to 400 cc. In certain aspects, T cells are activated from blood draws of 20 cc, 30 cc, 40 cc, 50 cc, 60 cc, 70 cc, 80 cc, 90 cc, or 100 cc.

The administration of the CAR-T cells and compositions may be carried out in any manner, e.g., by parenteral or nonparenteral administration, including by aerosol inhalation, injection, infusions, ingestion, transfusion, implantation or transplantation. For example, the CAR-T cells and compositions described herein may be administered to a patient trans-arterially, intradermally, subcutaneously, intratumorally, intramedullary, intranodally, intramuscularly, by intravenous (i.v.) injection, or intraperitoneally. In one aspect, the compositions of the present disclosure are administered by i.v. injection. In one aspect, the compositions of the present disclosure are administered to a subject by intradermal or subcutaneous injection. The compositions of T cells may be injected, for instance, directly into a tumor, lymph node, tissue, organ, or site of infection.

Administration can be autologous or non-autologous. For example, immunoresponsive cells expressing a CD33-specific CAR can be obtained from one subject, and administered to the same subject or a different, compatible subject. Peripheral blood derived T cells of the present disclosure, or expanded T cells (e.g., *in vivo, ex vivo* or *in vitro* derived) can be administered via, e.g., intravenous injection, localized injection, systemic injection, catheter administration, or parenteral administration.

In particular embodiments, subjects may undergo leukapheresis, wherein leukocytes are collected, enriched, or depleted *ex vivo* to select and/or isolate the cells of interest, e.g., T cells. These T cell isolates may be expanded by methods known in the art and treated such that one or more CAR constructs of the present disclosure may be introduced, thereby creating a CAR-T cell. Subjects in need thereof may subsequently undergo standard treatment with high dose chemotherapy followed by peripheral blood stem cell transplantation. In certain aspects, following or concurrent with the transplant, subjects receive an infusion of the expanded CAR-T cells. In one aspect, expanded cells are administered before or following surgery.

The dosage administered to a patient having a malignancy is sufficient to alleviate or at least partially arrest the disease being treated ("therapeutically effective amount"). The dosage of the above treatments to be administered to a subject will vary with the precise nature of the condition being treated and the recipient of the treatment. The scaling of dosages for human administration can be performed according to practices generally accepted in the art.

The CAR T cells of the invention can undergo *in vivo* T cell expansion and can establish CD33-specific memory cells that persist at high levels for an extended amount of time in blood and bone marrow. In some instances, the CAR T cells of the invention infused into a subject can eliminate cancer cells, e.g., hematologic cancer cells, *in vivo* in subjects with advanced chemotherapy-resistant cancer.

In one embodiment, a CAR of the present disclosure is introduced into T cells, e.g., using *in vitro* transcription, and the subject (e.g., human) receives an initial administration of CAR-T cells of the disclosure, and one or more subsequent administrations of the CAR-T cells, wherein the one or more subsequent administrations are administered less than 15 days, e.g., 14, 13, 12, 11, 10, 9, 8, 7, 6, 5, 4, 3, or 2 days after the previous administration. In one embodiment, more than one administration of the CAR-T cells are administered to the subject (e.g., human) per week, e.g., 2, 3, or 4 administrations of the CAR-T cells are administered per week. In one embodiment, the subject receives more than one administration of the CAR-T cells per week (e.g., 2, 3 or 4 administrations per week) (also referred to herein as a cycle), followed by a week of no CAR-T cell administrations, and then one or more additional administration of the CAR-T cells (e.g., more than one administration of the CAR-T cells per week) is administered to the subject. In another embodiment, the subject receives more than one cycle of CAR-T cells, and the time between each cycle is less than 10, 9, 8, 7, 6, 5, 4, or 3 days. In one embodiment, the CAR-T cells are administered every other day for 3 administrations per week. In one embodiment, the CAR-T cells are administered for at least two, three, four, five, six, seven, eight or more weeks.

In one embodiment, administration may be repeated after one day, two days, three days, four days, five days, six days, one week, two weeks, three weeks, one month, five weeks, six weeks, seven weeks, two months, three months, four months, five months, six months or longer. Repeated courses of treatment are also possible, as is chronic administration. The repeated administration may be at the same dose or at a different dose.

The CAR-T cells may be administered in the methods of the invention by maintenance therapy, such as, e.g., once a week for a period of 6 months or more.

In one embodiment, CAR-T cells are generated using lentiviral viral vectors, such as lentivirus. CAR-T cells generated with such viral vectors will generally have stable CAR expression.

In one embodiment, CAR-T cells transiently express CAR vectors for 4, 5, 6, 7, 8, 9, 10, 11, 12, 13, 14, 15 days after transduction. Transient expression of CARs can be affected by RNA CAR vector delivery. In one embodiment, the CAR RNA is transduced into the T cell by electroporation.

If a patient is at high risk of generating an anti-CAR antibody response during the course of transient CAR therapy (such as those generated by RNA transductions), CAR-T infusion breaks should not last more than ten to

fourteen days.

### Combination therapies

The CD33 binding proteins of the disclosure may be administered in combination with at least one additional therapeutics.

In some embodiments the at least one additional therapeutic is surgery, chemotherapy, or radiation, or any combination thereof.

In some embodiments, the delivery of one treatment is still occurring when the delivery of the second begins, so that there is overlap in terms of administration. This is sometimes referred to herein as "simultaneous" or "concurrent delivery". In other embodiments, the delivery of one treatment ends before the delivery of the other treatment begins. In some embodiments of either case, the treatment is more effective because of combined administration. For example, the second treatment is more effective, e.g., an equivalent effect is seen with less of the second treatment, or the second treatment reduces symptoms to a greater extent, than would be seen if the second treatment were administered in the absence of the first treatment, or the analogous situation is seen with the first treatment. In some embodiments, delivery is such that the reduction in a symptom, or other parameter related to the disorder is greater than what would be observed with one treatment delivered in the absence of the other. The effect of the two treatments can be partially additive, wholly additive, or greater than additive. The delivery can be such that an effect of the first treatment delivered is still detectable when the second is delivered.

In one embodiment, other therapeutic agents such as factors may be administered before, after, or at the same time (simultaneous with) as the CD33 binding proteins such as CAR-T cells, including, but not limited to, interleukins, as well as colony stimulating factors, such as G-, M- and GM-CSF, and interferons.

The CD33 binding proteins such as CAR-expressing cells described herein and the at least one additional therapeutic agent can be administered simultaneously, in the same or in separate compositions, or sequentially. For sequential administration, the CD33 binding proteins such as CAR-expressing cells described herein can be administered first, and the additional agent can be administered second, or the order of administration can be reversed.

In further embodiments, the CD33 binding proteins such as CAR-expressing cells described herein may be used in a treatment regimen in combination with surgery, radiation, chemotherapy, immunosuppressive agents, antibodies, or other immunoablative agents. In one embodiment, the subject can be administered an agent which enhances the activity of a CAR-expressing cell. For example, in one embodiment, the agent can be an agent which inhibits an inhibitory molecule.

The following examples are provided to further describe some of the embodiments disclosed herein. The examples are intended to illustrate, not to limit, the disclosed embodiments.

## EXAMPLES

The present invention is also described and demonstrated by way of the following examples. However, the use of these and other examples anywhere in the specification is illustrative only and in no way limits the scope and meaning of the invention or of any exemplified term. Likewise, the invention is not limited to any particular preferred embodiments described here. Indeed, many modifications and variations of the invention may be apparent to those skilled in the art upon reading this specification, and such variations can be made without departing from the invention in spirit or in scope. The invention is therefore to be limited only by the terms of the appended claims along with the full scope of equivalents to which those claims are entitled.

### Example 1: Antigen Generation

Expression constructs encoding the human CD33 extracellular domain (ECD) or its sub-domains were designed based on the sequence of myeloid cell surface antigen CD33 (Uniprot accession # P20138) and its domain annotation with either 6X His-tag sequence or as a fusion protein to a C34S variant of human serum albumin (HSA) with a 6X His-tag sequence at the C-terminus. Similar expression constructs encoding CD33 (ECD) or its sub-domains from cynomolgus monkey (*Macaca fascicularis*) were designed based on NCBI Accession # XP 005590138.1. The amino acid sequences of the generated antigens are shown in Table 3.

Human and cyno CD33 full-length ECD or sub-domain expression constructs were transiently transfected into HEK293 derived cells, Expi293 (Gibco/Thermo Fisher Scientific) using Expifectamine according to manufacturer protocol. Cells were incubated 5 days at 37°C with 8% $CO_2$ on an orbital shaker before harvesting. The expressed cells were removed by centrifugation and the soluble CD33 proteins with his-tags were purified from the media using immobilized metal affinity chromatography using Ni Sepharose 6 Fast Flow resin (GE Healthcare) and subsequently buffer-exchanged into 1X Dubelcco's Phosphate Saline buffer pH 7.2 without calcium or magnesium using Zeba™ Spin Desalting Columns,

7K MWCO, 10 mL; Thermo Scientific Catalog number: 89893 per the manufacturer's specifications.

**Table 3.** Amino acid sequences of the CD33 antigens.

| Protein AA ID | Description | Amino Acid Sequence |
|---|---|---|
| C33W1 | HSA N-terminal Fusion; CynoCD33ECD-HSA (C34S)-6xHis | DPRVRLEVQESVTVQEGLCVLVPCTFFHPVPYHTRNSPVHGYWFR<br>EGAIVSLDSPVATNKLDQEVQEETQGRFRLLGDPSRNNCSLSIVDA<br>RRRDNGSYFFRMEKGSTKYSYKSTQLSVHVTDLTHRPQILIPGALD<br>PDHSKNLTCSVPWACEQGTPPIFSWMSAAPTSLGLRTTHSSVLIITP<br>RPQDHGTNLTCQVKFPGAGVTTERTIQLNVSYASQNPRTDIFLGDG<br>SGKQGVVQGSGSGSENLYFQGVRSSSDAHKSEVAHRFKDLGEENF<br>KALVLIAFAQYLQQSPFEDHVKLVNEVTEFAKTCVADESAENCDK<br>SLHTLFGDKLCTVATLRETYGEMADCCAKQEPERNECFLQHKDD<br>NPNLPRLVRPEVDVMCTAFHDNEETFLKKYLYEIARRHPYFYAPE<br>LLFFAKRYKAAFTECCQAADKAACLLPKLDELRDEGKASSAKQRL<br>KCASLQKFGERAFKAWAVARLSQRFPKAEFAEVSKLVTDLTKVH<br>TECCHGDLLECADDRADLAKYICENQDSISSKLKECCEKPLLEKSH<br>CIAEVENDEMPADLPSLAADFVESKDVCKNYAEAKDVFLGMFLY<br>EYARRHPDYSVVLLLRLAKTYETTLEKCCAAADPHECYAKVFDEF<br>KPLVEEPQNLIKQNCELFEQLGEYKFQNALLVRYTKKVPQVSTPTL<br>VEVSRNLGKVGSKCCKHPEAKRMPCAEDYLSVVLNQLCVLHEKT<br>PVSDRVTKCCTESLVNRRPCFSALEVDETYVPKEFNAETFTFHADI<br>CTLSEKERQIKKQTALVELVKHKPKATKEQLKAVMDDFAAFVEK<br>CCKADDKETCFAEEGKKLVAASQAALGLGGGSHHHHHH<br><br>(SEQ ID NO: 225) |
| C33W2 | HSA N-terminal Fusion; Human CD33ECD-Tev-HSA (C34S)-6xHis | DPNFWLQVQESVTVQEGLCVLVPCTFFHPIPYYDKNSPVHGYWFR<br>EGAIISRDSPVATNKLDQEVQEETQGRFRLLGDPSRNNCSLSIVDA<br>RRRDNGSYFFRMERGSTKYSYKSPQLSVHVTDLTHRPKILIPGTLE<br>PGHSKNLTCSVSWACEQGTPPIFSWLSAAPTSLGPRTTHSSVLIITP<br>RPQDHGTNLTCQVKFAGAGVTTERTIQLNVTYVPQNPTTGIFPGD<br>GSGKQETRAGVVHGSGSGSENLYFQGVRSSSDAHKSEVAHRFKD<br>LGEENFKALVLIAFAQYLQQSPFEDHVKLVNEVTEFAKTCVADES<br>AENCDKSLHTLFGDKLCTVATLRETYGEMADCCAKQEPERNECFL<br>QHKDDNPNLPRLVRPEVDVMCTAFHDNEETFLKKYLYEIARRHPY<br>FYAPELLFFAKRYKAAFTECCQAADKAACLLPKLDELRDEGKASS<br>AKQRLKCASLQKFGERAFKAWAVARLSQRFPKAEFAEVSKLVTD<br>LTKVHTECCHGDLLECADDRADLAKYICENQDSISSKLKECCEKPL<br>LEKSHCIAEVENDEMPADLPSLAADFVESKDVCKNYAEAKDVFLG<br>MFLYEYARRHPDYSVVLLLRLAKTYETTLEKCCAAADPHECYAK<br>VFDEFKPLVEEPQNLIKQNCELFEQLGEYKFQNALLVRYTKKVPQ<br>VSTPTLVEVSRNLGKVGSKCCKHPEAKRMPCAEDYLSVVLNQLC<br>VLHEKTPVSDRVTKCCTESLVNRRPCFSALEVDETYVPKEFNAETF<br>TFHADICTLSEKERQIKKQTALVELVKHKPKATKEQLKAVMDDFA<br>AFVEKCCKADDKETCFAEEGKKLVAASQAALGLGGGSHHHHHH<br><br>(SEQ ID NO: 226) |
| C33W3 | HSA N-terminal Fusion; hCD33 Ig-like V-type domain | DPNFWLQVQESVTVQEGLCVLVPCTFFHPIPYYDKNSPVHGYWFR<br>EGAIISRDSPVATNKLDQEVQEETQGRFRLLGDPSRNNCSLSIVDA<br>RRRDNGSYFFRMERGSTKYSYKSPQLSVHVTDLTHRDAHKSEVA<br>HRFKDLGEENFKALVLIAFAQYLQQSPFEDHVKLVNEVTEFAKTC |

(continued)

| Protein AA ID | Description | Amino Acid Sequence |
|---|---|---|
| | (Uniprot P20138, V136-H259) fused to HSA and 6xHis Tag at C-terminus | VADESAENCDKSLHTLFGDKLCTVATLRETYGEMADCCAKQEPE RNECFLQHKDDNPNLPRLVRPEVDVMCTAFHDNEETFLKKYLYEI ARRHPYFYAPELLFFAKRYKAAFTECCQAADKAACLLPKLDELRD EGKASSAKQRLKCASLQKFGERAFKAWAVARLSQRFPKAEFAEV SKLVTDLTKVHTECCHGDLLECADDRADLAKYICENQDSISSKLK ECCEKPLLEKSHCIAEVENDEMPADLPSLAADFVESKDVCKNYAE AKDVFLGMFLYEYARRHPDYSVVLLLRLAKTYETTLEKCCAAAD PHECYAKVFDEFKPLVEEPQNLIKQNCELFEQLGEYKFQNALLVR YTKKVPQVSTPTLVEVSRNLGKVGSKCCKHPEAKRMPCAEDYLS VVLNQLCVLHEKTPVSDRVTKCCTESLVNRRPCFSALEVDETYVP KEFNAETFTFHADICTLSEKERQIKKQTALVELVKHKPKATKEQLK AVMDDFAAFVEKCCKADDKETCFAEEGKKLVAASQAALGLHHH HHH<br><br>(SEQ ID NO: 227) |
| C33W4 | HSA N-terminal Fusion; hCD33 Ig-like C2-type domain (Uniprot P20138, V136-H259) fused to HSA and 6xHis Tag at C-terminus | VHVTDLTHRPKILIPGTLEPGHSKNLTCSVSWACEQGTPPIFSWLSA APTSLGPRTTHSSVLIITPRPQDHGTNLTCQVKFAGAGVTTERTIQL NVTYVPQNPTTGIFPGDGSGKQETRAGVVHDAHKSEVAHRFKDL GEENFKALVLIAFAQYLQQSPFEDHVKLVNEVTEFAKTCVADESA ENCDKSLHTLFGDKLCTVATLRETYGEMADCCAKQEPERNECFLQ HKDDNPNLPRLVRPEVDVMCTAFHDNEETFLKKYLYEIARRHPYF YAPELLFFAKRYKAAFTECCQAADKAACLLPKLDELRDEGKASSA KQRLKCASLQKFGERAFKAWAVARLSQRFPKAEFAEVSKLVTDL TKVHTECCHGDLLECADDRADLAKYICENQDSISSKLKECCEKPLL EKSHCIAEVENDEMPADLPSLAADFVESKDVCKNYAEAKDVFLG MFLYEYARRHPDYSVVLLLRLAKTYETTLEKCCAAADPHECYAK VFDEFKPLVEEPQNLIKQNCELFEQLGEYKFQNALLVRYTKKVPQ VSTPTLVEVSRNLGKVGSKCCKHPEAKRMPCAEDYLSVVLNQLC VLHEKTPVSDRVTKCCTESLVNRRPCFSALEVDETYVPKEFNAETF TFHADICTLSEKERQIKKQTALVELVKHKPKATKEQLKAVMDDFA AFVEKCCKADDKETCFAEEGKKLVAASQAALGLHHHHHH<br><br>(SEQ ID NO: 228) |
| C33W8 | hCD33_Ig-like V-Type domain | DPNFWLQVQESVTVQEGLCVLVPCTFFHPIPYYDKNSPVHGYWFR EGAIISRDSPVATNKLDQEVQEETQGRFRLLGDPSRNNCSLSIVDA RRRDNGSYFFRMERGSTKYSYKSPQLSVHVTDLTHRGSHHHHHH<br><br>(SEQ ID NO: 229) |
| C33W9 | hCD33 Ig-like C2-type domain | VHVTDLTHRPKILIPGTLEPGHSKNLTCSVSWACEQGTPPIFSWLSA APTSLGPRTTHSSVLIITPRPQDHGTNLTCQVKFAGAGVTTERTIQL NVTYVPQNPTTGIFPGDGSGKQETRAGVVHGSHHHHHH<br><br>(SEQ ID NO: 230) |
| C33W10 | MuIgV_ Hu IgC2 chimera CD33-6xHis_v1 | QDLEFQLVAPESVTVEEGLCVHVPCSVFYPSIKLTLGPVTGSWLRK GVSLHEDSPVATSDPRQLVQKATQGRFQLLGDPQKHDCSLFIRDA QKNDTGMYFFRVVREPFVRYSYKKSQLSLHVTSLSRTPKILIPGTL EPGHSKNLTCSVSWACEQGTPPIFSWLSAAPTSLGPRTTHSSVLIITP RPQDHGTNLTCQVKFAGAGVTTERTIQLNVTYVPQNPTTGIFPGD GSGKQETRAGVVHGSHHHHHH<br><br>(SEQ ID NO: 231) |

(continued)

| Protein AA ID | Description | Amino Acid Sequence |
|---|---|---|
| C33W12 | MuIgV_cyno IgC2 chimera CD33-6xHis_v1 | QDLEFQLVAPESVTVEEGLCVHVPCSVFYPSIKLTLGPVTGSWLRK GVSLHEDSPVATSDPRQLVQKATQGRFQLLGDPQKHDCSLFIRDA QKNDTGMYFFRVVREPFVRYSYKKSQLSLHVTSLSRTPQILIPGAL DPDHSKNLTCSVPWACEQGTPPIFSWMSAAPTSLGLRTTHSSVLIIT PRPQDHGTNLTCQVKFPGAGVTTERTIQLNVSYASQNPRTDIFLGD GSGRKARKQGVVQGSHHHHHH<br><br>(SEQ ID NO: 232) |
| C33W49 | Human CD33 ECD (D18-H259) from Uniprot P20138 with C-terminal 6xHis tag | DPNFWLQVQESVTVQEGLCVLVPCTFFHPIPYYDKNSPVHGYWFR EGAIISRDSPVATNKLDQEVQEETQGRFRLLGDPSRNNCSLSIVDA RRRDNGSYFFRMERGSTKYSYKSPQLSVHVTDLTHRPKILIPGTLE PGHSKNLTCSVSWACEQGTPPIFSWLSAAPTSLGPRTTHSSVLIITP RPQDHGTNLTCQVKFAGAGVTTERTIQLNVTYVPQNPTTGIFPGD GSGKQETRAGVVHGSHHHHHH<br><br>(SEQ ID NO: 233) |
| C33W50 | Cyno CD33 ECD (D37-Q274) from XP 005590138.1 with C-terminal 6xHis tag | DPRVRLEVQESVTVQEGLCVLVPCTFFHPVPYHTRNSPVHGYWFR EGAIVSLDSPVATNKLDQEVQEETQGRFRLLGDPSRNNCSLSIVDA RRRDNGSYFFRMEKGSTKYSYKSTQLSVHVTDLTHRPQILIPGALD PDHSKNLTCSVPWACEQGTPPIFSWMSAAPTSLGLRTTHSSVLIITP RPQDHGTNLTCQVKFPGAGVTTERTIQLNVSYASQNPRTDIFLGDG SGKQGVVQGSHHHHHH<br><br>(SEQ ID NO: 234) |
| C33W51 | Human CD33 ECD (D18-G241) from Uniprot P20138 with 6xHis tag | DPNFWLQVQESVTVQEGLCVLVPCTFFHPIPYYDKNSPVHGYWFR EGAIISRDSPVATNKLDQEVQEETQGRFRLLGDPSRNNCSLSIVDA RRRDNGSYFFRMERGSTKYSYKSPQLSVHVTDLTHRPKILIPGTLE PGHSKNLTCSVSWACEQGTPPIFSWLSAAPTSLGPRTTHSSVLIITP RPQDHGTNLTCQVKFAGAGVTTERTIQLNVTYVPQNPTTGHHHH HH<br><br>(SEQ ID NO: 235) |

**Example 2. Generation of anti-CD33 antibodies**

Antibodies were generated using either the Open Monoclonal Technologies (OMT) or the Ablexis Transgenic mice technologies as follows. Both the AlivaMab and OMT transgenic mice were engineered to produce human/mouse immunoglobulins. AlivaMab or OMT transgenic mice were immunized with recombinant human CD33 protein (a selection of antigens from Table-1). Lymphocytes were extracted from secondary lymphoid organs and either fused with FO mouse myeloma cell line for hybridoma generation or subjected to single cell sorting via FACS. Hybridoma supernatants were screened by MSD electrochemiluminescence for binding to over-expressing human CD33 ECD HEK cells. The samples identified from the screening were further assayed with FACS for binding to over-expressing human CD33 ECD HEK cells (positive signal) compared to parental HEK cells (negative signal). Confirmed cell binders were light chain isotyped using ELISA. Single cell sorting supernatants were screened by MSD electrochemiluminescence for binding to recombinant human CD33 protein. Several hits with the desired binding profile were selected and sequenced.

V region cloning was performed as follows. Both RNA purified by the Qiagen® RNeasy Plus Mini Kit and B cell lysate were used to perform cDNA synthesis using the Smarter cDNA synthesis kit (Clontech, Mount View, CA). To facilitate cDNA synthesis, oligodT was used to prime reverse transcription of all messenger RNAs, followed by "5' capping" with a Smarter IIA oligonucleotide. Subsequent amplification of the VH and VL fragments was performed using a 2-step PCR amplification using 5' primers targeting the Smarter IIA cap and 3' primers targeting consensus regions in CH1. Briefly, each 50 μl PCR reaction consists of 20 μM of forward and reverse primer mixes, 25 μl of PrimeStar Max DNA polymerase premix (Clontech), 2 μl of unpurified cDNA, and 21 μl of double-distilled H2O. The cycling program

starts at 94 °C for 3 min, followed by 35 cycles (94 °C for 30 Sec, 55°C for 1 min, 68 °C for 1 min), and ends at 72 °C for 7 min. A second round of PCR was performed using VL and VH second round primers that contained 15bp complementary extensions that "overlap" respective regions in their respective Lonza mother vector (VH and VL). Second round PCR was performed with the following program: 94 °C for 3 min; 35 cycles (94 °C for 30 Sec, 55°C for 1 min, 68 °C for 1 min), and ends at 72 °C for 7 min. In-Fusion® HD Cloning Kit (Clonetech, U.S.A.) was used for directional cloning of VL gene into Lonza huIgK or Lambda vector and VH gene into Lonza huIgGl vector. To facilitate In-Fusion® HD Cloning, PCR products were treated with Cloning Enhancer before performing In-Fusion® HD Cloning. The cloning and transformation were performed according to manufacturer's protocol (Clonetech, U.S.A.). Mini-prep DNAs were subjected to Sanger sequencing to confirm that complete V-gene fragments were obtained.

Table 4 shows the amino acid sequences of the heavy and light chains of each of the antibodies generated by the above methods.

**Table 4.**

| Antibody Name | Amino acid sequence of Heavy and Light Chains (CDRs according to the Kabat system in bold and underlined) |
|---|---|
| C33B1474 | VH:<br>QVQLVESGGGLVQPGGSLRLSCAASGFTFS**SFGMS**WVRQAPGKGLEWVA**NIKRDGSEKYYVDSVKG**RFTISRDNAKNSLFLQMSSLRAEDTAVYYCVR**DYGYFDF**WGQGTLVTVSS (SEQ ID NO: 52)<br>VL:<br>DIQMTQSPSSLSASVGDRVTITC**QASQAISNYLN**WYQQKPGKAPKLLIY**DASNLET**GVPSRFSGSGSGTDFTFTISSLQPEDFSTYFC**QHYDNLPYT**FGQGTKLEIK (SEQ ID NO: 81) |
| C33B1522 | VH:<br>QVQLQESGPGLVKPSETLSLTCTVSGGSIS**SYYWG**WIRQPPGKGLEWIG**YIYYSGSTNYNPSLKS**RVTISVDTSKNQFSLKLSSVTAADTAVYYCAR**MWEILGFDP**WGQGTLVTVS (SEQ ID NO: 53)<br>VL:<br>QSVLTQPPSASGTPGQRVTISC**SGSSSNIGSNPVN**WYQQLPGTAPKLLIY**SNNQRPS**GVPDRFSGSKSGTSASLAISGLQSEDEADYFC**AAWDDSLNGPV**FGGGTKLTVL (SEQ ID NO: 82) |
| C33B1607 | VH:<br>EVQLQQSGPELVKPGSSVKISCKASGYTFT**DYNIN**WVKQSHGKNLEWIG**TINPNNGVTFYNQRFKG**QATLTVDKSSSTAYMELRSLTSEDSAVYYCSR**DGYDTYYAMDY**WGQGTTLTVSS (SEQ ID NO: 54)<br>VL:<br>DIVMTQSHKFMSTSVGDRVSITC**KASQDVRTAEA**WYQQKPGQSPKLLIY**SASNRYT**GVPDRFTGSGSGTDFTFTISSVQAEDLAVYYC**QQYYTTPRT**FGGGTKLEIK (SEQ ID NO: 83) |
| C33B1601 | VH:<br>EVQLVESGGGLVRPGGSLKLSCAASGFTFS**NYAMS**WVRQTPEKRLEWVA**GISNSGYSLYYPDTLKG**RFTISRDNARNTLYLQMISLRSEDTAMYYCAR**DGGSYPYAMDY**WGHGTSVTVSS (SEQ ID NO: 55)<br>VL:<br>DIVMTQSQKFMSTSVGDRVSVTC**KASQNVGTYVA**WYQQKPGHSPKALIY**SASYRYS**GVPDRFTGSGSGTDFTLTISNVQSEDLADYFC**QQYKSYPLT**FGAGTKLELK (SEQ ID NO: 84) |

(continued)

| Antibody Name | Amino acid sequence of Heavy and Light Chains (CDRs according to the Kabat system in bold and underlined) |
|---|---|
| C33B1517 | VH:<br>QVQLQESGPGLVKPSETLSLTCSVSGASIR**NYYWS**WIRQTAGKGLEWLG**HIYSTGNIHYNPSLKS**RVTMSVDTSNNQFSLKLRSVTAADTAVYYCAR**DNGAALFDY**WGQGTLVTVSS (SEQ ID NO: 56)<br>VL:<br>QSVLTQPPSASGTPGQRVTISC**SGSSSNIGSNIVN**WYQQFPGTAPKLLIY**SNNQRPS**GVPDRVSGSKSGTSASLAISGLQSEDEADYYC**AAWDDSLNGPV**FGPGTKVTVL (SEQ ID NO: 85) |
| C33B1495 | VH:<br>EVQLVESGGGLVQPGGSLRLSCTASGFTFS**SYWMS**WVRQAPGKGLDWVA**NIKRDGSEKHYVDSVKG**RFTISRDNAKNSLYLQMNSLRAEDSAVYYCTR**DYGYFDY**WGQGTLVTVSS (SEQ ID NO: 57)<br>VL:<br>DIQLTQSPSFLSASVGDRVTITC**RASQGISSYLA**WYQQKPGKAPKVLIY**AASTLQS**GVPSRFSGSGSGTEFTLTISSLQPEDFATYFC**QQLNSYPVT**FGGGTKVEIK (SEQ ID NO: 86) |
| C33B1478 | VH:<br>EVQLVESGGGLVQPGGSLKVSCEASGFTFS**VSAIH**WVRQASGKGLEWIG**RIRSKGNSYATAYDVSVKG**RFTISRDDSKNTAYLQMDSLKTEDTAVYYCTR**HNDKWNYYGLDV**WGQGTTVTVSS (SEQ ID NO: 58)<br>VL:<br>DIVMTQSPLSLPVTPGEPASISC**KSSQSLLFSNGYKFLD**WYLQRPGQSPQLLIY**LGSYRAS**GVPDRFSGSGSGTDFTLKISRVEAEDVGLYYC**MQALQTPPT**FGGGTKVEIK (SEQ ID NO: 87) |
| C33B1477 | VH:<br>EVQLVESGGGLVQPGGSLKVSCEASGFTFS**VSAIH**WVRQASGKGLEWIG**RIRSKGNSYATAYAASVKG**RFTISRDDSKNTAYLQMDSLKTEDTAVYYCTR**HNDKWNYYGLDV**WGQGTTVTVSS (SEQ ID NO: 59)<br>VL:<br>DIVMTQSPLSLPVTPGEPASISC**KSSQSLLFSNGYKFLD**WYLQRPGQSPQLLIY**LGSYRAS**GVPDRFSGSGSGTDFTLKISRVEAEDVGLYYC**MQALQTPPT**FGGGTKVEIK (SEQ ID NO: 87) |
| C33B1476 | VH:<br>EVQLVESGGGLVQPGGSLKVSCEASGFTFS**VSAIH**WVRQASGKGLEWIG**RIRSKGNSYATAYNVSVKG**RFTISRDDSKNTAYLQMDSLKTEDTAVYYCTR**HNDKWNYYGLDV**WGQGTTVTVSS (SEQ ID NO: 60)<br>VL:<br>DIVMTQSPLSLPVTPGEPASISC**KSSQSLLFSNGYKFLD**WYLQRPGQSPQLLIY**LGSYRAS**GVPDRFSGSGSGTDFTLKISRVEAEDVGLYYC**MQALQTPPT**FGGGTKVEIK (SEQ ID NO: 87) |

(continued)

| Antibody Name | Amino acid sequence of Heavy and Light Chains (CDRs according to the Kabat system in bold and underlined) |
|---|---|
| C33B1484 | VH:<br>EVQLVESGGGLVQPGGSLKVSCEASGFTFS**VFAIH**WVRQASGKGLEWIG**RIRSKGNSYATAYDVSVKG**RFTISRDDSKNTAYLQMDSLKTEDTAVYYCTR**HNDKWNYYGLDV**WGQGTTVTVSS (SEQ ID NO: 61)<br>VL:<br>DIVMTQSPLSLPVTPGEPASISC**KSSQSLLFSNGYKFLD**WYLQRPGQSPQLLIY**LGSYRAS**GVPDRFSGSGSGTDFTLKISRVEAEDVGLYYC**MQALQTPPT**FGQGTKVEIK (SEQ ID NO: 88) |
| C33B933 | VH:<br>QVQLVESGGGVVQPGRSLRLSCAASGFTFS**SYGMH**WVRQSPGKGLEWVA**VISYDGSNKYYADSVKG**RFTISRDNSKSTLYLQMNSLRAEDTAVYYCAK**DFRSLDWLPPDSTSYDGMDV**WGQGTTVTVSS (SEQ ID NO: 95)<br>VL:<br>SYELTQPPSVSVSPGQTASITCSGHKLGDKYASWYQQKPGQSPVVVIYKDSKRPSGIPERFSGSNFGNTATLTISGTQAMDEADYYCQAWDSSTVVFGGGTKLTVL (SEQ ID NO: 105) |
| C33B919 | VH:<br>QVQLVESGGGVVQPGRSLRLSCAASGFTFS**SYGMH**WVRQAPGKGLEWVA**VISYDGSNKYYADSVKG**RFTISRDNSKNTLYLQMNSLRAEDTAVYYCAK**DFRSFDWLPPDSTSYYGMDV**WGQGTTVTVSS (SEQ ID NO: 96)<br>VL:<br>SYELTQPPSVSVSPGQTASITCSGDKLGDKYVSWYQQKPGQSPVVVIHQDRKRPSGIPERFSGSNFGNTATLTISGTQAMDEADYYCQAWDSSTVVFGGGTKLTVL (SEQ ID NO: 106) |
| C33B931 | VH:<br>EVQLVESGGGFVQPGGSLRLSCAASGFTFS**SYWMS**WVRQAPGKGLEWVA**NIKQHGSEKYYVDSVKG**RFTISRDNAKNSLYLQMNSLRAEDTAVYYCAR**DRDLGYFDY**WGQGTLVTVSS (SEQ ID NO: 97)<br>VL:<br>SYELTQPPSVSVSPGQTASITC**SGDKLGSKFAS**WYQQKPGQSPVLVIY**QDSKRPS**GIPERFSGSNSGNTATLTISGTQAMDEADYYC**QAWDSSTVV**FGGGTKLTVL (SEQ ID NO: 107) |
| C33B836 | VH:<br>QVQLVQSGSELRKPGASVKVSCKASGYTFT**NYAMN**WVRQAPGQGLEWMG**WINTNTGNPTYAQGFTG**RFVFSLDTSVSSAYLQISSLKAEDTAMYYCAT**DRDRGTDY**WGQGTLVTVSS (SEQ ID NO: 170)<br>VL:<br>QSALTQPASVSGSPGQSITISC**TGTSSDVGDYNYVS**WYQQHPGKVPKLMIY**DVSNRPS**GVSNRFSGSMSGNTASLTISGLQAEDEADYYC**SSYSSSSALEV**FGGGTKLTVL (SEQ ID NO: 185) |

(continued)

| Antibody Name | Amino acid sequence of Heavy and Light Chains (CDRs according to the Kabat system in bold and underlined) |
|---|---|
| C33B782 | VH:<br>EVQLVESGGGLVQPGGSLRLSCAASGFTFS**SYWMS**WVRQAPGKGLEWVA**NIKQHGSEKYYVDSVKG**RFTISRDNAKNSLYLQMNSLRAEDTAVYYCAR**DRDLGYFDY**WGQGTLVTVSS (SEQ ID NO: 171)<br>VL:<br>SYELTQPPSVSVSPGQTASITC**SGNKLGAKFAS**WYQQKPGQSPVLVIY**QDNKRPS**GIPERFSGSNSGNTATLTISGTQAVDEADYYC**QAWDSSTVV**FGGGTKLTVL (SEQ ID NO: 188) |
| C33B806 | VH:<br>QLQLQESGPGLVKPSETLSLTCTVSGGSIS**SSSYYWG**WIRQPPGKGLDWIG**SINYSGSTYYNPSLKS**RVTISVDTSKIQFSLKLRSVTAADTAVYYCAR**LDGYESPFDY**WGQGTLVTVSS (SEQ ID NO: 175)<br>VL:<br>DIQMTQSPSSVSASVGDRVTITC**RASQGISSWLA**WYQQKPGKAPKLLIY**AASSLQS**GVPSRFSGSGSGTDFTLTISSLQPEDFATYYC**QQANSFPFT**FGPGTKVDIK (SEQ ID NO: 192) |
| C33B904 | VH:<br>EVQLVESGGGLVQPGRSLRLSCAASGFTFD**DYAMH**WVRQAPGKGLEWVS**GIGWSGGSIVYADSVKG**RFTISRDNAKNSLYLQMNSLRAEDTALYYCAK**DSPYGDFFDY**WGQGTLVTVSS (SEQ ID NO: 179)<br>VL:<br>DIVMTQSPDSLAVSLGERATINC**KSSQTVFYSSNNKNYLA**WYQQKPGQPPKLLIS**WASTRKS**GVPDRFSGSGSGTDFTLTVSSLQAEDVAVYYC**QHYYSTPYT**FGQGTKLEIK (SEQ ID NO: 196) |
| C33B937 | VH:<br>QVQLVESGGGVVQPGRSLRLSCVASGFTFS**SYGMH**WVRQAPGKGLEWVA**VISYDGSNKYYADSVKG**RFTISRDNSKNTLYLQMNSLRAEGTAVYYCAK**DFRSFDWLPPDSASYHGMDV**WGQGTTVTVSS (SEQ ID NO: 181)<br>VL:<br>SYELTQPPSVSVSPGQTASITC**SGDKLGDKYAS**WYQQKPGQSPVLVIY**QDGKRPS**GIPERFSGSNFGNKATLTISGTQAMDEADYYC**QAWDRNTVV**FGGGTKLTVL (SEQ ID NO: 200) |
| C33B125 | VH:<br>QVQLVESGGGVVQPGRSLRLSCAASGFTFS**SYGMH**WVRQAPGKGLEWVA**VISYDGSNKYYADSVKG**RFTISRDNSKNTLYLQMNSLRAEDTAVYYCAK**DFRSFDWLPPDSTSYYGMDV**WGQGTTVTVSS (SEQ ID NO: 96)<br>VL:<br>SYELTQPPSVSVSPGQTASITC**SGDKLGDKYVC**WYQQKPGQSPVVVIH**QDRKRPS**GIPERFSGSNFGNTATLTISGTQAMDEADYYC**QAWDSSTVV**FGGGTKLTVL (SEQ ID NO: 202) |

(continued)

| Antibody Name | Amino acid sequence of Heavy and Light Chains (CDRs according to the Kabat system in bold and underlined) |
|---|---|
| C33B1475 | VH:<br>EVQLVESGGGLVQPGGSLRLSCVVSGFTFS**SYWMT**WVRQAPGKGLEWVA**NIKQDGSERYYVDSVKG**RFTISRDSAKNSLYLQMNSLRAEDTAVYYCAR**EVGYNWNQGGYFDY**WGQGTLVTVSS (SEQ ID NO: 262)<br>VL:<br>DIVMTQSPLSLPVTPGEPASISC**RSSQSLLHSDGYNYLD**WYLQKSGQSPQLLIY**LGSYRAS**GVPDRFSGSGSGTDFTLKISRVEAEDVGIYYC**MQVLQTPWT**FGQGTKVEIK (SEQ ID NO: 271) |
| C33B1516 | VH:<br>QVQLQESGPGLVKPSETLSLTCSVSGGSIR**NYYWS**WIRQSAGKELEWFG**HIFSTGHINYDSSLKS**RVTMSVDTSNNQFSLKLRSVTAADTAVYYCAR**DNGAALFDF**WGQGTLVTVSS (SEQ ID NO: 263)<br>VL:<br>QSVLTQPPSASGTPGQRVTISC**SGSSSNIGSNIVN**WYQQFPGTAPKLLLY**SDNQRPS**GVPDRFSGSKSGTSASLAISGLQSEDEADYYC**AAWDDSLNGPV**FGTGTKVTVL (SEQ ID NO: 272) |
| C33B1481 | VH:<br>QVQLVESGGGLVQPGGSLRLSCAASGITFS**NYWMS**WVRQAPGKGLEWVA**SIKRDGSDKYYVDSVKG**RFTISRDNAKNSLSLQMHSLRAEDTAVYYCAK**GEFDY**WGQGTLVTVSS (SEQ ID NO: 264)<br>VL:<br>DVVMTQSPLSLPVTLGQPASISC**RSSQSLVYSDGNTYLN**WFQQRPGQSPRRLIY**KVSTRDS**GVPDRFSGSGSGTDFTLKISRVEAEDVAVYYC**LQGTHWPWT**FGQGTKVEIK (SEQ ID NO: 273) |

Table 4a shows the amino acid sequences of the CDR sequences of the C33B1522 antibody (an antibody having the VH of SEQ ID NO: 53 and the VL of SEQ ID NO: 82) as defined according to the AbM, Kabat, Chothia, IMGT and Contact systems.

**Table 4a.**

| | HC1 | HC2 | HC3 | LC1 | LC2 | LC3 |
|---|---|---|---|---|---|---|
| AbM | **GGSISSYYWG** (SEQ ID NO: 399) | **YIYYSGSTN** (SEQ ID NO: 405) | **MWEILGFDP** (SEQ ID NO: 411) | **SGSSSNIGSNPVN** (SEQ ID NO: 417) | **SNNQRPS** (SEQ ID NO: 423) | **AAWDDSLNGPV** (SEQ ID NO: 429) |
| Kabat | **SYYWG** (SEQ ID NO: 29) | **YIYYSGSTNYNPSLKS** (SEQ ID NO: 37) | **MWEILGFDP** (SEQ ID NO: 46) | **SGSSSNIGSNPVN** (SEQ ID NO: 63) | **SNNQRPS** (SEQ ID NO: 70) | **AAWDDSLNGPV** (SEQ ID NO: 76) |
| Chothia | **GGSISSY** (SEQ ID NO: 435) | **YYSGS** (SEQ ID NO: 441) | **MWEILGFD** (SEQ ID NO: 447) | **SSSNIGSNP** (SEQ ID NO: 453) | **SNN** (SEQ ID NO: 459) | **WDDSLNGP** (SEQ ID NO: 465) |
| IMGT | **GGSISSYY** (SEQ ID NO: 471) | **IYYSGST** (SEQ ID NO: 477) | **ARMWEILGFDP** (SEQ ID NO: 483) | **SSNIGSNP** (SEQ ID NO: 489) | **SNN** (SEQ ID NO: 495) | **AAWDDSLNGPV** (SEQ ID NO: 501) |
| Contact | **SSYYWG** (SEQ ID NO: 507) | **WIGYIYYSGSTN** (SEQ ID NO: 513) | **ARMWEILGFD** (SEQ ID NO: 519) | **IGSNPVNWY** (SEQ ID NO: 525) | **LLIYSNNQRP** (SEQ ID NO: 531) | **AAWDDSLNGP** (SEQ ID NO: 537) |

Table 4b shows the amino acid sequences of the CDR sequences of the C33B1477 antibody (an antibody having the VH of SEQ ID NO: 59 and the VL of SEQ ID NO: 87) as defined according to the AbM, Kabat, Chothia, IMGT and Contact systems.

**Table 4b.**

| | HC1 | HC2 | HC3 | LC1 | LC2 | LC3 |
|---|---|---|---|---|---|---|
| AbM | **GFTFSVSAIH** (SEQ ID NO: 400) | **RIRSKGNSYATA** (SEQ ID NO: 406) | **HNDKWNYYGLDV** (SEQ ID NO: 412) | **KSSQSLLFSNGYKFLD** (SEQ ID NO: 418) | **LGSYRAS** (SEQ ID NO: 424) | **MQALQTPPT** (SEQ ID NO: 430) |
| Kabat | **VSAIH** (SEQ ID NO: 34) | **RIRSKGNSYATAYAASVKG** (SEQ ID NO: 43) | **HNDKWNYYGLDV** (SEQ ID NO: 51) | **KSSQSLLFSNGYKFLD** (SEQ ID NO: 68) | **LGSYRAS** (SEQ ID NO: 74) | **MQALQTPPT** (SEQ ID NO: 80) |
| Chothia | **GFTFSVS** (SEQ ID NO: 436) | **RSKGNSYA** (SEQ ID NO: 442) | **HNDKWNYYGLD** (SEQ ID NO: 448) | **SQSLLFSNGYKF** (SEQ ID NO: 454) | **LGS** (SEQ ID NO: 460) | **ALQTPP** (SEQ ID NO: 466) |
| IMGT | **GFTFSVSA** (SEQ ID NO: 472) | **IRSKGNSYAT** (SEQ ID NO: 478) | **TRHNDKWNYYGLDV** (SEQ ID NO: 484) | **QSLLFSNGYKF** (SEQ ID NO: 490) | **LGS** (SEQ ID NO: 496) | **MQALQTPPT** (SEQ ID NO: 502) |
| Contact | **SVSAIH** (SEQ ID NO: 508) | **WIGRIRSKGNSYATA** (SEQ ID NO: 514) | **TRHNDKWNYYGLD** (SEQ ID NO: 520) | **LFSNGYKFLDWY** (SEQ ID NO: 526) | **LLIYLGSYRA** (SEQ ID NO: 532) | **MQALQTPP** (SEQ ID NO: 538) |

Table 4c shows the amino acid sequences of the CDR sequences of the C33B1475 antibody (an antibody having the VH of SEQ ID NO: 262 and the VL of SEQ ID NO: 271) as defined according to the AbM, Kabat, Chothia, IMGT and Contact systems.

**Table 4c.**

| | HC1 | HC2 | HC3 | LC1 | LC2 | LC3 |
|---|---|---|---|---|---|---|
| AbM | **GFTFSSYWMT** (SEQ ID NO: 401) | **NIKQDGSERY** (SEQ ID NO: 407) | **EVGYNWNQGGYFDY** (SEQ ID NO: 413) | **RSSQSLLHSDGYNYLD** (SEQ ID NO: 419) | **LGSYRAS** (SEQ ID NO: 425) | **MQVLQTPWT** (SEQ ID NO: 431) |
| Kabat | **SYWMT** (SEQ ID NO: 253) | **NIKQDGSERYYVDSVKG** (SEQ ID NO: 256) | **EVGYNWNQGGYFDY** (SEQ ID NO: 259) | **RSSQSLLHSDGYNYLD** (SEQ ID NO: 265) | **LGSYRAS** (SEQ ID NO: 74) | **MQVLQTPWT** (SEQ ID NO: 269) |
| Chothia | **GFTFSSY** (SEQ ID NO: 437) | **KQDGSE** (SEQ ID NO: 443) | **EVGYNWNQGGYFD** (SEQ ID NO: 449) | **SQSLLHSDGYNY** (SEQ ID NO: 455) | **LGS** (SEQ ID NO: 461) | **VLQTPW** (SEQ ID NO: 467) |
| IMGT | **GFTFSSYW** (SEQ ID NO: 473) | **IKQDGSER** (SEQ ID NO: 479) | **AREVGYNWNQGGYFDY** (SEQ ID NO: 485) | **QSLLHSDGYNY** (SEQ ID NO: 491) | **LGS** (SEQ ID NO: 497) | **MQVLQTPWT** (SEQ ID NO: 503) |
| Contact | **SSYWMT** (SEQ ID NO: 509) | **WVANIKQDGSERY** (SEQ ID NO: 515) | **AREVGYNWNQGGYFD** (SEQ ID NO: 521) | **LHSDGYNYLDWY** (SEQ ID NO: 527) | **LLIYLGSYRA** (SEQ ID NO: 533) | **MQVLQTPW** (SEQ ID NO: 539) |

Table 4d shows the amino acid sequences of the CDR sequences of the C33B1517 antibody (an antibody having the VH of SEQ ID NO: 56 and the VL of SEQ ID NO: 85) as defined according to the AbM, Kabat, Chothia, IMGT and Contact systems.

**Table 4d.**

| | HC1 | HC2 | HC3 | LC1 | LC2 | LC3 |
|---|---|---|---|---|---|---|
| AbM | **GASIR NYYWS** (SEQ ID NO: 402) | **HIYSTGNIH** (SEQ ID NO: 408) | **DNGAALFD Y** (SEQ ID NO: 414) | **SGSSSNIGSNI VN** (SEQ ID NO: 420) | **SNNQRPS** (SEQ ID NO: 426) | **AAWDDSLN GPV** (SEQ ID NO: 432) |
| Kabat | **NYYWS** (SEQ ID NO: 32) | **HIYSTGNIHYN PSLKS** (SEQ ID NO: 40) | **DNGAALFD Y** (SEQ ID NO: 49) | **SGSSSNIGSNI VN** (SEQ ID NO: 66) | **SNNQRPS** (SEQ ID NO: 70) | **AAWDDSLN GPV** (SEQ ID NO: 76) |
| Chothia | **GASIR NY** (SEQ ID NO: 438) | **YSTGN** (SEQ ID NO: 444) | **DNGAALFD** (SEQ ID NO: 450) | **SSSNIGSNI** (SEQ ID NO: 456) | **SNN** (SEQ ID NO: 462) | **WDDSLNGP** (SEQ ID NO: 468) |
| IMGT | **GASIR NYY** (SEQ ID NO: 474) | **IYSTGNI** (SEQ ID NO: 480) | **ARDNGAAL FDY** (SEQ ID NO: 486) | **SSNIGSNI** (SEQ ID NO: 492) | **SNN** (SEQ ID NO: 498) | **AAWDDSLN GPV** (SEQ ID NO: 504) |
| Contact | **RNYY WS** (SEQ ID NO: 510) | **WLGHIYSTGN IH** (SEQ ID NO: 516) | **ARDNGAAL FD** (SEQ ID NO: 522) | **IGSNIVNWY** (SEQ ID NO: 528) | **LLIYSNNQ RP** (SEQ ID NO: 534) | **AAWDDSLN GP** (SEQ ID NO: 540) |

Table 4c shows the amino acid sequences of the CDR sequences of the C33B1516 antibody (an antibody having the VH of SEQ ID NO: 263 and the VL of SEQ ID NO: 272) as defined according to the AbM, Kabat, Chothia, IMGT and Contact systems.

**Table 4e.**

| | HC1 | HC2 | HC3 | LC1 | LC2 | LC3 |
|---|---|---|---|---|---|---|
| AbM | **GGSIR NYYWS** (SEQ ID NO: 403) | **HIFSTGHIN** (SEQ ID NO: 409) | **DNGAALFD F** (SEQ ID NO: 415) | **SGSSSNIGSN IVN** (SEQ ID NO: 421) | **SDNQRPS** (SEQ ID NO: 427) | **AAWDDSLN GPV**(SEQ ID NO: 433) |
| Kabat | **NYYWS** (SEQ ID NO: 254) | **HIFSTGHINYD SSLKS** (SEQ ID NO: 257) | **DNGAALFD F** (SEQ ID NO: 260) | **SGSSSNIGSN IVN** (SEQ ID NO: 66) | **SDNQRPS** (SEQ ID NO: 267) | **AAWDDSLN GPV** (SEQ ID NO: 76) |
| Chothia | **GGSIR NY** (SEQ ID NO: 439) | **FSTGH** (SEQ ID NO: 445 ) | **DNGAALFD** (SEQ ID NO: 451) | **SSSNIGSNI** (SEQ ID NO: 457) | **SDN** (SEQ ID NO: 463) | **WDDSLNGP** (SEQ ID NO: 469) |
| IMGT | **GGSIR NYY** (SEQ ID NO: 475) | **IFSTGHI** (SEQ ID NO: 481) | **ARDNGAAL FDF** (SEQ ID NO: 487) | **SSNIGSNI** (SEQ ID NO: 493) | **SDN** (SEQ ID NO: 499) | **AAWDDSLN GPV** (SEQ ID NO: 505) |
| Contact | **RNYY WS** (SEQ ID NO: 511) | **WFGHIFSTGH IN** (SEQ ID NO: 517) | **ARDNGAAL FD** (SEQ ID NO: 523) | **IGSNIVNWY** (SEQ ID NO: 529) | **LLLYSDN QRP** (SEQ ID NO: 535) | **AAWDDSLN GP** (SEQ ID NO: 541) |

Table 4f shows the amino acid sequences of the CDR sequences of the C33B1481 antibody (an antibody having the VH of SEQ ID NO: 264 and the VL of SEQ ID NO: 273) as defined according to the AbM, Kabat, Chothia, IMGT and Contact systems.

**Table 4f.**

| | HC1 | HC2 | HC3 | LC1 | LC2 | LC3 |
|---|---|---|---|---|---|---|
| AbM | **GITFSNYWMS** (SEQ ID NO: 404) | **SIKRDGSDKY** (SEQ ID NO: 410) | **GEFDY** (SEQ ID NO: 416) | **RSSQSLVYSDGNTYLN**(SEQ ID NO: 422) | **KVSTRDS** (SEQ ID NO: 428) | **LQGTHWPWT** (SEQ ID NO: 434) |
| Kabat | **NYWMS** (SEQ ID NO: 255) | **SIKRDGSDKYYVDSVKG** (SEQ ID NO: 258) | **GEFDY** (SEQ ID NO: 261) | **RSSQSLVYSDGNTYLN** (SEQ ID NO: 266) | **KVSTRDS** (SEQ ID NO: 268) | **LQGTHWPWT** (SEQ ID NO: 270) |
| Chothia | **GITFSNY** (SEQ ID NO: 440) | **KRDGSD** (SEQ ID NO: 446) | **GEFD** (SEQ ID NO: 452) | **SQSLVYSDGNTY** (SEQ ID NO: 458) | **KVS** (SEQ ID NO: 464) | **GTHWPW** (SEQ ID NO: 470) |
| IMGT | **GITFSNYW** (SEQ ID NO: 476) | **IKRDGSDK** (SEQ ID NO: 482) | **AKGEFDY** (SEQ ID NO: 488) | **QSLVYSDGNTY** (SEQ ID NO: 494) | **KVS** (SEQ ID NO: 500) | **LQGTHWPWT**(SEQ ID NO: 506) |
| Contact | **SNYWMS** (SEQ ID NO: 512) | **WVASIKRDGSDKY** (SEQ ID NO: 518) | **AKGEFD** (SEQ ID NO: 524) | **VYSDGNTYLNWF** (SEQ ID NO: 530) | **RLIYKVSTRD** (SEQ ID NO: 536) | **LQGTHWPW** (SEQ ID NO: 542) |

Antibodies were generated in llamas as follows. Three llamas were injected subcutaneously with hCD33-IgC2 antigen from Table 3 for three injections (Days 0, 14 and 28), with the first injection in Complete Freund's Adjuvant and subsequent injections in Incomplete Freund's Adjuvant). The first injection was followed by three injections of hCD33-FL ECD antigen from Table 3 (days 61, 76 and 91). PBMCs for single B cell sorting were acquired on days 14, 35 and 86 and frozen. Frozen PBMCs were thawed and stained with recombinant antigen baits, either biotinylated or conjugated with Alexa Fluor 647. The antigen baits were recombinant human CD33-ECD and recombinant human CD33-IgC domain. Biotinylated bait was detected using PE-labelled Streptavidin (SA). Antigen-specific cells (positive for both antigen baits) were bulk sorted directly into RLT lysis buffer (Qiagen) using BD FACSAria Fusion sorter. The cell lysate was frozen at -80 °C and shipped to Genewiz for further processing.

Llama vHH V- region cloning and mono-Fc fusion construct design were performed as follows. B-cells were lysed in RealTime Ready Cell Lysis Buffer (Roche). RNA were then purified with the RNeasy Plus® Mini Kit (Qiagen 74134). Fresh RNA was used directly for cDNA synthesis using the SuperScript® cDNA kit (Invitrogen 18091-050). To facilitate cDNA synthesis, oligodT was used to prime reverse transcription of all messenger RNAs. Subsequent amplification of the VHH fragments was performed using a 2-step PCR amplification using Llama SPs and 3' primers targeting consensus regions in CH2. Briefly, each 50 μl PCR reaction consisted of 20 μM of forward and reverse primer mixes, 25 μl of PrimeStar Max DNA polymerase premix (Clontech), 2 μl of unpurified cDNA, and 21 μl of double-distilled $H_2O$. The cycling program started at 94 °C for 3 min, followed by 35 cycles (94 °C for 30 Sec, 55°C for 1 min, 68 °C for 1 min), and ended at 72 °C for 7 min.

VHH DNA from first round PCR were gel-purified. Second round VHH PCR was performed with second round primers containing 15bp complementary extensions that "overlap" respective regions in their respective Lonza mother vector. Second round PCR was performed with the following program: 94 °C for 3 min; 35 cycles (94 °C for 30 Sec, 55°C for 1 min, 68 °C for 1 min), and ending at 72 °C for 7 min. An In-Fusion® HD Cloning Kit (Clonetech, U.S.A.) was used for directional cloning of VHH gene into Lonza vector with mono-Fc. To facilitate In-Fusion® HD Cloning, PCR products were treated with Cloning Enhancer before In-Fusion HD Cloning. Cloning and transformation were performed according to manufacturer's protocol (Clonetech, U.S.A.). Mini-prep DNAs were subjected to Sanger sequencing to confirm that complete V-gene fragments were obtained. Table 5 shows the amino acid sequences of the heavy chain of the llama antibodies generated by the above method.

**Table 5.**

| Antibody Name | Amino acid sequence of Heavy Chain (CDR sequences underlined in bold) |
|---|---|
| C33B1785 | VH:<br>QVQLVESGGGSVQPGGSLRLSCAVFGFSLS**DYAIG**WFRQAPGKEREKVS**CISESDGRTYLSDSVKS**RFTISRDNGKNTVYLQMNNLKPDDTGVYYCAT**VDQAISESSYHCEKGFGS**WGQGTQVTVSS (SEQ ID NO: 18) |
| C33B1784 | VH:<br>EVQLVESGGGLVQAGGSLRLSCAASGRTFS**TYVMG**WFRQAPGQERELVA**AIVSGRNPTYADSVKS**RFTISRDNAKNTIYLHMNSLQPEDTAVYYCHM**YHYSSQY**WGQGTQVTVSS (SEQ ID NO: 19) |
| C33B1787 | VH:<br>EVQLVESGGGLVQAGGSLRLSCAASGFTFD**DYAIG**WFRQAPGKEREGVS**CISSSDGSTYYVDSVKG**RFTISSDNAKNTVYLQMNSLKPEDTAVYYCTA**LIIMYGSGSERAAAACRYKYEY**WGQGTQVTVSS (SEQ ID NO: 20) |
| C33B1786 | VH:<br>EVQVVESGGGVVQSGGSLTLSCEASESILS**FSTMD**WFRQAPGKEREGVS**CIRPRYSTTTHADSIKG**RFKMYSDNAKNTIYLHMNSLQPEDTAVYYCHM**YHYSSQY**WGQGTQVTVSS (SEQ ID NO: 21) |
| C33B1781 | VH:<br>EVQVVESGGGLVQGGGSLRLSCAASGSIFS**INAMG**WYHQAPGKQRELVA**RITGGGATDYVDSVKG**RFTISLDSAKSTVYLQMNSLKPEDTAVYHCYA**DLVTRVGSDLLYDDY**WGQGTQVTVSS (SEQ ID NO: 22) |

(continued)

| Antibody Name | Amino acid sequence of Heavy Chain (CDR sequences underlined in bold) |
|---|---|
| C33B1780 | VH:<br>EVQLVESGGGLVQPGGSLRLSCAVFGFSLS**DYAIG**WFRQAPGKEREKVS**CISESDGRTYLSDSVKS**RFTISRDNGKNTVYLQMNNLKPDDTGVYYCAT**VDQAISESSYHCEKGFGS**WGQGTQVTVSS (SEQ ID NO: 23) |
| C33B1783 | VH:<br>EVQLVESGGGLVQAGGSLRLSCAASGFTFD**DYAVG**WFRQVPGKEREGVS**CISSSDGATYYADSVKG**RFTISSDNAKNTVYLQMNSLKPEDTAVYYCTA**IIVRGSGWERAAAACRYEYSY**WGQGTQVTVSS (SEQ ID NO: 24) |
| C33B1782 | VH:<br>EVQLVESGGGLVQAGGSLRLSCAASGFTFD**DYAVG**WFRQAPGKEREGIS**CISSSDGATYYADSVKG**RFTISTDNAKNTAFLQMNSLKPEDTAVYYCTA**VIVRGTGWERAAAACRYEYAY**WGQGTQVTVSS (SEQ ID NO: 25) |
| C33B1789 | VH:<br>QVQLVESGGGLVQAGGSLRLSCAVFGFSLS**DYAIG**WFRQAPGKEREKVS**CISESDGRTYLSDSVKS**RFTISRDNGKNTVYLQMNNLKPDDTGVYYCAT**VDQAISESSYHCEKGFGS**WGQGTQVTVSS (SEQ ID NO: 26) |
| C33B1788 | VH:<br>QVQLVESGGGLVQPGGSLRLSCAVFGFSLS**DYAIG**WFRQAPGKEREKVS**CISESDGRTYLSDSVKS**RFTISRDNGKNTVYLQMNNLKPDDTGVYYCAT**VDQAISESSYHCEKGFGS**WGQGTQVTVSS (SEQ ID NO: 27) |

The anti-CD33 antibodies and vHH-monoFc fusion proteins were expressed in ExpiCHO-S™ cells (ThermoFisher Scientific; Waltham, MA, Cat # A29127) by transient transfection with purified plasmid DNA encoding the proteins following the manufacturer's recommendations. Briefly, ExpiCHO-S™ cells were maintained in suspension in ExpiCHO™ expression medium (ThermoFisher Scientific, Cat # A29100) in an orbital shaking incubator set at 37°C, 8% $CO_2$ and 125 RPM. The cells were passaged and diluted prior to transfection to $6.0 \times 10^6$ cells per ml, maintaining cell viability at 99.0% or better. Transient transfections were done using the ExpiFectamine™ CHO transfection kit (ThermoFisher Scientific, Cat # A29131). For each ml of diluted cells to be transfected, 0.5 microgram of scFv Fc fusion encoding DNA and 0.5 microgram of pAd VAntage DNA (Promega, Cat# E1711) was used and diluted into OptiPRO™ SFM complexation medium. ExpiFectamine™ CHO reagent was used at a 1:4 ratio (v/v, DNA:reagent) and diluted into OptiPRO™. The diluted DNA and transfection reagent were combined for one minute, allowing DNA/lipid complex formation, and then added to the cells. After overnight incubation, ExpiCHO™ feed and ExpiFectamine™ CHO enhancers were added to the cells as per the manufacturer's Standard protocol. Cells were incubated with orbital shaking (125 rpm) at 37°C for seven days prior to harvesting the culture broth. The culture supernatant from the transiently transfected ExpiCHO-S™ cells was clarified by centrifugation (30 min, 3000rcf) followed by filtration (0.2μm PES membrane, Corning; Corning, NY).

Protein Purification was performed as follows. The filtered cell culture supernatant was loaded onto a pre-equilibrated (1xDPBS, pH 7.2) MabSelect Sure Protein A column (GE Healthcare) using an AKTAXpress chromatography system. After loading, the column was washed with 10 column volumes of 1xDPBS, pH7.2. The protein was eluted with 10 column volumes of 0.1 M sodium (Na)-Acetate, pH 3.5. Protein fractions were neutralized immediately by the addition of 2.5 M Tris HCl, pH 7.5 to 20% (v/v) of the elution fraction volume. Peak fractions were pooled and filtered (0.2 μm). The quality of the purified protein was assessed by analytical size exclusion HPLC (Agilent HPLC system).

**Example 3. Characterization of anti-CD33 antibodies and Llama vHHs by Surface Plasmon Resonance (SPR; Biacore)**

Affinities ($K_D$) for the interaction of anti-CD33 antibodies human CD33 (full length ECD and IgC2 domain) were obtained by SPR. The anti-CD33 antibodies were captured using an anti-human Fc antibody and the antigens were injected in solution. The data is shown in Table 6 below.

**Table 6.**

| Protein ID # | binding to hu CD33 (FL ECD) $K_D$ (nM) | binding to hu CD33 (IgC2) $K_D$ (nM) |
|---|---|---|
| C33B1474 | 2.47 | 3.99 |
| C33B1517 | 4.44 | no binding |
| C33B919 | 0.5 | no binding |
| C33B1522 | >10 | no binding |
| C33B1607 | 1.49 | no binding |
| C33B933 | 0.5 | Not measured |
| C33B125 | 0.1 | >10 |
| C33B931 | 0.4 | Not measured |
| C33B937 | 0.3 | 56 |
| C33B1601 | 1.1 | 6.8 |

The binding affinity of anti-CD33 antibodies and Llama vHHs to the recombinant human CD33 (FL ECD or IgC2 domain) was determined by surface plasmon resonance (SPR) using Biacore 8K instrument. The Fc fused scFvs or mono-Fc fused vHHs were captured using a goat anti-Fc antibody-modified GLC chip and titrated with 5-fold serial dilutions of CD33 antigen spanning concentrations of 40nM-1.6nM. The association and dissociation were monitored for 2 and 30 minutes, respectively, using a flow rate of 50 μL/min. Raw binding data was referenced by subtracting the analyte binding signals from blanks and analyzed using a 1:1 Langmuir binding model using the Biacore software to obtain the kinetics which were used to calculate the binding affinity.

Affinities ($K_D$) for the interaction of anti-CD33 Llama vHHs with human CD33 (FL ECD or IgC2 domain). The anti-CD33 Llama vHHs on a mono-Fc backbone were captured using an anti-human Fc antibody and the antigens were injected in solution. The data are shown in Table 7 below. Representative Biacore 8K sensorgrams of anti-CD33 vHHs interactions against hu CD33 antigens (FL ECD and IgC2 domain) are also shown in **FIG. 1.**

**Table 7.**

| **vHH Protein ID** | binding to hu CD33 (FL ECD) **KD (M)** | binding to hu CD33 (IgC2) **KD (M)** |
|---|---|---|
| C33B1783 | 8.59E-11 | no/low binding |
| C33B1782 | 2.06E-10 | no/low binding |
| C33B1786 | 3.57E-10 | no/low binding |
| C33B1785 | 6.61E-10 | 3.31E-09 |
| C33B1789 | 6.70E-10 | 3.03E-09 |
| C33B1788 | 7.07E-10 | 4.34E-09 |
| C33B1787 | 7.92E-10 | no/low binding |
| C33B1780 | 8.47E-10 | 3.38E-09 |
| C33B1781 | 2.21E-09 | 5.59E-11 |
| C33B1784 | low nM binder; low response at this cone | no/low binding |

The thermal stability of anti-CD33 scFv-Fc fusion antibodies and Llama VHHs was determined by nanoDSF on the Prometheus® NT.Plex instrument (NanoTemper Technologies). Measurements were made by loading samples into Standard Capillaries (NanoTemper Technologies) from a 384 well sample plate. Duplicate or triplicate runs were performed. Thermal unfolding was monitored in a 1.0°C/minute thermal ramp from 20°C to 95°C. Thermal inflection temperatures (midpoint Tms) and onset of aggregation temperature (Tagg) were determined automatically by PR. ThermControl Software (NanoTemper Technologies) and further analyzed using the PR. Stability Analysis Software (NanoTemper Technologies). The data are shown in Table 8 below.

**Table 8. Thermal stability data for anti-CD33 scFvs and vHH antibodies (Llama vHHs on mono-Fc backbone) as obtained using a nanoDSF instrument.**

| vHH Protein ID | Tm1 ($^0C$) | Tm2 ($^0C$) | Tm3 ($^0C$) |
|---|---|---|---|
| C33B1780 | 50.2 | 72.1 | |
| C33B1781 | 52.6 | 53 | |
| C33B1782 | 50.3 | 66.5 | |
| C33B1783 | 51.4 | 64.7 | |
| C33B1784 | 48.4 | 58.5 | |
| C33B1785 | 49.9 | 63 | |
| C33B1786 | 48.0 | 63.6 | |
| C33B1787 | 50.4 | 59.3 | |
| C33B1788 | 50.2 | 63.5 | 75.7 |
| C33B1789 | 50.2 | 63.8 | 81.5 |

**Table 8a:** Thermal stability data for anti-CD33 scFvs and vHHs antibodies as obtained using a nanoDSF instrument. An IgG1 Fc backbone of SEQ ID NO: 277 was used. scFvs were attached directly to this IgG1 backbone without the use of a linker sequence.

**scFvs on human IgG1 Fc backbone**

| scFv-Fc fusion protein ID | scFv clone ID | Protein Description | Parental mAb | Average Tm1-ScFv (($^0$C) | Average Tm2 ($^0$C) | Average Tm3 ($^0$C) | Average Tagg ($^0$C) | Average Onset ($^0$C) |
|---|---|---|---|---|---|---|---|---|
| C33B1689 | C33B1617 | C33B1617 LH Fc | C33B937 | 58 | 67.7 | 80.6 | 71.8 | 53.6 |
| C33B1699 | C33B1617 | C33B1617 HL Fc | C33B937 | 56.4 | 67.8 | 80.7 | 77.9 | 51.7 |
| C33B1690 | C33B1618 | C33B1618 LH Fc | C33B933 | 63.1 | | 80.7 | 77.7 | 52.5 |
| C33B1700 | C33B1618 | C33B1618 HL Fc | C33B933 | 63.7 | | 80.5 | 79.6 | 52.2 |
| C33B1691 | C33B1619 | C33B1619 LH Fc | C33B931 | 61.2 | 68.3 | 80.1 | 78.5 | 56.6 |
| C33B1701 | C33B1619 | C33B1619 HL Fc | C33B931 | 61.6 | 68.4 | 80.6 | 78.7 | 56.5 |
| C33B1698 | C33B1620 | C33B1620 LH Fc | C33B919 | 63.5 | 68.8 | 81.3 | 77.6 | 59.2 |
| C33B1702 | C33B1620 | C33B1620 HL Fc | C33B919 | 64.9 | | 80.4 | 79.2 | 55.5 |
| C33B1693 | C33B1646 | C33B1646 LH Fc | C33B1607 | 66.5 | | 81.2 | 66.2 | 60.5 |
| C33B1703 | C33B1646 | C33B1646 HL Fc | C33B1607 | 64.7 | 81 | | 64.1 | 58.9 |
| C33B1694 | C33B1651 | C33B1651 LH Fc | C33B1601 | 65.2 | | 81.2 | 63.4 | 56.9 |
| C33B1704 | C33B1651 | C33B1651 HL Fc | C33B1601 | 64.6 | 81.6 | | 70.7 | 56.2 |
| C33B1696 | C33B1659 | C33B1659 LH Fc | C33B1517 | 68.1 | | 81.1 | 76.3 | 56.1 |
| C33B 1706 | C33B1659 | C33B1659 HL Fc | C33B1517 | 68.1 | | 80.9 | 79.8 | 60.7 |

(continued)

| scFv-Fc fusion protein ID | scFv clone ID | Protein Description | Parental mAb | Average Tm1-ScFv (($^0$C) | Average Tm2 ($^0$C) | Average Tm3 ($^0$C) | Average Tagg ($^0$C) | Average Onset ($^0$C) |
|---|---|---|---|---|---|---|---|---|
| C33B1697 | C33B1680 | C33B1680 LH Fc | C33B1474 | 56.7 | 67.1 | 80.6 | 69 | 48.2 |
| C33B1707 | C33B1680 | C33B1680 HL Fc | C33B1474 | 54.6 | 67.2 | 80.2 | 78.7 | 46.2 |
| C33B1692 | C33B1682 | C33B1682 LH Fc | C33B125 | 65 | | 81 | 77.7 | 55.4 |
| C33B1708 | C33B1682 | C33B1682 HL Fc | C33B125 | 63.9 | | 80.2 | 80.5 | 58.8 |

**Example 4: Construction and expression of CD33 scFv CARs**

CD33 CAR constructs comprising the following scFv sequences are constructed:

| |
|---|
| QSALTQPASVSGSPGQSITISCTGTSSDVGDYNYVSWYQQHPGKVPKLMIYDVSNRPSGVSNRFSGSMSGNTASLTISGLQAEDEADYYCSSYSSSALEVFGGGTKLTVLGGSEGKSSGSGSESKSTGGSQVQLVQSGSELRKPGASVKVSCKASGYTFTNYAMNWVRQAPGQGLEWMGWINTNTGNPTYAQGFTGRFVFSLDTSVSSAYLQISSLKAEDTAMYYCATDRDRGTDYWGQGTLVTVSS (SEQ ID NO: 203) |
| SYELTQPPSVSVSPGQTASITCSGNKLGAKFASWYQQKPGQSPVLVIYQDNKRPSGIPERFSGSNSGNTATLTISGTQAVDEADYYCQAWDSSTVVFGGGTKLTVLGGSEGKSSGSGSESKSTGGSEVQLVESGGGLVQPGGSLRLSCAASGFTFSSYWMSWVRQAPGKGLEWVANIKQHGSEKYYVDSVKGRFTISRDNAKNSLYLQMNSLRAEDTAVYYCARDRDLGYFDYWGQGTLVTVSS (SEQ ID NO: 204) |
| DIQMTQSPSSVSASVGDRVTITCRASQGISSWLAWYQQKPGKAPKLLIYAASSLQSGVPSRFSGSGSGTDFTLTISSLQPEDFATYYCQQANSFPFTFGPGTKVDIKGGSEGKSSGSGSESKSTGGSQLQLQESGPGLVKPSETLSLTCTVSGGSISSSSYYWGWIRQPPGKGLDWIGSINYSGSTYYNPSLKSRVTISVDTSKIQFSLKLRSVTAADTAVYYCARLDGYESPFDYWGQGTLVTVSS (SEQ ID NO: 205) |
| DIVMTQSPDSLAVSLGERATINCKSSQTVFYSSNNKNYLAWYQQKPGQPPKLLISWASTRKSGVPDRFSGSGSGTDFTLTVSSLQAEDVAVYYCQHYYSTPYTFGQGTKLEIKGGSEGKSSGSGSESKSTGGSEVQLVESGGGLVQPGRSLRLSCAASGFTFDDYAMHWVRQAPGKGLEWVSGIGWSGGSIVYADSVKGRFTISRDNAKNSLYLQMNSLRAEDTALYYCAKDSPYGDFFDYWGQGTLVTVSS (SEQ ID NO: 206) |
| SYELTQPPSVSVSPGQTASITCSGHKLGDKYASWYQQKPGQSPVVVIYKDSKRPSGIPERFSGSNFGNTATLTISGTQAMDEADYYCQAWDSSTVVFGGGTKLTVLGGSEGKSSGSGSESKSTGGSQVQLVESGGGVVQPGRSLRLSCAASGFTFSSYGMHWVRQSPGKGLEWVAVISYDGSNKYYADSVKGRFTISRDNSKSTLYLQMNSLRAEDTAVYYCAKDFRSLDWLPPDSTSYDGMDVWGQGTTVTVSS (SEQ ID NO: 207) |

(continued)

| |
|---|
| SYELTQPPSVSVSPGQTASITCSGDKLGDKYASWYQQKPGQSPVLVIYQDGKRPSGIPERFSGSNFGNKATLTISGTQAMDEADYYCQAWDRNTVVFGGGTKLTVLGGSEGKSSGSGSESKSTGGSQVQLVESGGGVVQPGRSLRLSCVASGFTFSSYGMHWVRQAPGKGLEWVAVISYDGSNKYYADSVKGRFTISRDNSKNTLYLQMNSLRAEGTAVYYCAKDFRSFDWLPPDSASYHGMDVWGQGTTVTVSS (SEQ ID NO: 208) |
| SYELTQPPSVSVSPGQTASITCSGDKLGDKYVSWYQQKPGQSPVVVIHQDRKRPSGIPERFSGSNFGNTATLTISGTQAMDEADYYCQAWDSSTVVFGGGTKLTVLGGSEGKSSGSGSESKSTGGSQVQLVESGGGVVQPGRSLRLSCAASGFTFSSYGMHWVRQAPGKGLEWVAVISYDGSNKYYADSVKGRFTISRDNSKNTLYLQMNSLRAEDTAVYYCAKDFRSFDWLPPDSTSYYGMDVWGQGTTVTVSS (SEQ ID NO: 209) |
| SYELTQPPSVSVSPGQTASITCSGDKLGDKYVCWYQQKPGQSPVVVIHQDRKRPSGIPERFSGSNFGNTATLTISGTQAMDEADYYCQAWDSSTVVFGGGTKLTVLGGSEGKSSGSGSESKSTGGSQVQLVESGGGVVQPGRSLRLSCAASGFTFSSYGMHWVRQAPGKGLEWVAVISYDGSNKYYADSVKGRFTISRDNSKNTLYLQMNSLRAEDTAVYYCAKDFRSFDWLPPDSTSYYGMDVWGQGTTVTVSS (SEQ ID NO: 210) |
| SYELTQPPSVSVSPGQTASITCSGDKLGSKFASWYQQKPGQSPVLVIYQDSKRPSGIPERFSGSNSGNTATLTISGTQAMDEADYYCQAWDSSTVVFGGGTKLTVLGGSEGKSSGSGSESKSTGGSEVQLVESGGGFVQPGGSLRLSCAASGFTFSSYWMSWVRQAPGKGLEWVANIKQHGSEKYYVDSVKGRFTISRDNAKNSLYLQMNSLRAEDTAVYYCARDRDLGYFDYWGQGTLVTVSS (SEQ ID NO: 211) |
| DIQMTQSPSSLSASVGDRVTITCQASQAISNYLNWYQQKPGKAPKLLIYDASNLETGVPSRFSGSGSGTDFTFTISSLQPEDFSTYFCQHYDNLPYTFGQGTKLEIKGGSEGKSSGSGSESKSTGGSQV |

| |
|---|
| QLVESGGGLVQPGGSLRLSCAASGFTFSSFGMSWVRQAPGKGLEWVANIKRDGSEKYYVDSVKGRFTISRDNAKNSLFLQMSSLRAEDTAVYYCVRDYGYFDFWGQGTLVTVSS (SEQ ID NO: 212) |

(continued)

| |
|---|
| QSVLTQPPSASGTPGQRVTISCSGSSSNIGSNPVNWYQQLPGTAPKLLIYSNNQRPSGVPDRFSG SKSGTSASLAISGLQSEDEADYFCAAWDDSLNGPVFGGGTKLTVLGGSEGKSSGSGSESKSTGG SQVQLQESGPGLVKPSETLSLTCTVSGGSISSYYWGWIRQPPGKGLEWIGYIYYSGSTNYNPSL KSRVTISVDTSKNQFSLKLSSVTAADTAVYYCARMWEILGFDPWGQGTLVTVS (SEQ ID NO: 213) |
| DIVMTQSHKFMSTSVGDRVSITCKASQDVRTAEAWYQQKPGQSPKLLIYSASNRYTGVPDRFT GSGSGTDFTFTISSVQAEDLAVYYCQQYYTTPRTFGGGTKLEIKGGSEGKSSGSGSESKSTGGSE VQLQQSGPELVKPGSSVKISCKASGYTFTDYNINWVKQSHGKNLEWIGTINPNNGVTFYNQRF KGQATLTVDKSSSTAYMELRSLTSEDSAVYYCSRDGYDTYYAMDYWGQGTTLTVSS (SEQ ID NO: 214) |
| DIVMTQSQKFMSTSVGDRVSVTCKASQNVGTYVAWYQQKPGHSPKALIYSASYRYSGVPDRF TGSGSGTDFTLTISNVQSEDLADYFCQQYKSYPLTFGAGTKLELKGGSEGKSSGSGSESKSTGG SEVQLVESGGGLVRPGGSLKLSCAASGFTFSNYAMSWVRQTPEKRLEWVAGISNSGYSLYYPD TLKGRFTISRDNARNTLYLQMISLRSEDTAMYYCARDGGSYPYAMDYWGHGTSVTVSS (SEQ ID NO: 215) |
| QSVLTQPPSASGTPGQRVTISCSGSSSNIGSNIVNWYQQFPGTAPKLLIYSNNQRPSGVPDRVSG SKSGTSASLAISGLQSEDEADYYCAAWDDSLNGPVFGPGTKVTVLGGSEGKSSGSGSESKSTG GSQVQLQESGPGLVKPSETLSLTCSVSGASIRNYYWSWIRQTAGKGLEWLGHIYSTGNIHYNPS LKSRVTMSVDTSNNQFSLKLRSVTAADTAVYYCARDNGAALFDYWGQGTLVTVSS (SEQ ID NO: 216) |
| DIQLTQSPSFLSASVGDRVTITCRASQGISSYLAWYQQKPGKAPKVLIYAASTLQSGVPSRFSGS GSGTEFTLTISSLQPEDFATYFCQQLNSYPVTFGGGTKVEIKGGSEGKSSGSGSESKSTGGSEVQ LVESGGGLVQPGGSLRLSCTASGFTFSSYWMSWVRQAPGKGLDWVANIKRDGSEKHYVDSV KGRFTISRDNAKNSLYLQMNSLRAEDSAVYYCTRDYGYFDYWGQGTLVTVSS (SEQ ID NO: 217) |

| |
|---|
| DIVMTQSPLSLPVTPGEPASISCKSSQSLLFSNGYKFLDWYLQRPGQSPQLLIYLGSYRASGVPD RFSGSGSGTDFTLKISRVEAEDVGLYYCMQALQTPPTFGGGTKVEIKGGSEGKSSGSGSESKST GGSEVQLVESGGGLVQPGGSLKVSCEASGFTFSVSAIHWVRQASGKGLEWIGRIRSKGNSYAT AYDVSVKGRFTISRDDSKNTAYLQMDSLKTEDTAVYYCTRHNDKWNYYGLDVWGQGTTVT VSS (SEQ ID NO: 218) |

(continued)

| |
|---|
| DIVMTQSPLSLPVTPGEPASISCKSSQSLLFSNGYKFLDWYLQRPGQSPQLLIYLGSYRASGVPDRFSGSGSGTDFTLKISRVEAEDVGLYYCMQALQTPPTFGGGTKVEIKGGSEGKSSGSGSESKSTGGSEVQLVESGGGLVQPGGSLKVSCEASGFTFSVSAIHWVRQASGKGLEWIGRIRSKGNSYATAYAASVKGRFTISRDDSKNTAYLQMDSLKTEDTAVYYCTRHNDKWNYYGLDVWGQGTTVTVSS (SEQ ID NO: 219) |
| DIVMTQSPLSLPVTPGEPASISCKSSQSLLFSNGYKFLDWYLQRPGQSPQLLIYLGSYRASGVPDRFSGSGSGTDFTLKISRVEAEDVGLYYCMQALQTPPTFGGGTKVEIKGGSEGKSSGSGSESKSTGGSEVQLVESGGGLVQPGGSLKVSCEASGFTFSVSAIHWVRQASGKGLEWIGRIRSKGNSYATAYNVSVKGRFTISRDDSKNTAYLQMDSLKTEDTAVYYCTRHNDKWNYYGLDVWGQGTTVTVSS (SEQ ID NO: 220) |
| DIVMTQSPLSLPVTPGEPASISCKSSQSLLFSNGYKFLDWYLQRPGQSPQLLIYLGSYRASGVPDRFSGSGSGTDFTLKISRVEAEDVGLYYCMQALQTPPTFGQGTKVEIKGGSEGKSSGSGSESKSTGGSEVQLVESGGGLVQPGGSLKVSCEASGFTFSVFAIHWVRQASGKGLEWIGRIRSKGNSYATAYDVSVKGRFTISRDDSKNTAYLQMDSLKTEDTAVYYCTRHNDKWNYYGLDVWGQGTTVTVSS (SEQ ID NO: 221) |
| DIVMTQSPLSLPVTPGEPASISCRSSQSLLHSDGYNYLDWYLQKSGQSPQLLIYLGSYRASGVPDRFSGSGSGTDFTLKISRVEAEDVGIYYCMQVLQTPWTFGQGTKVEIKGGSEGKSSGSGSESKSTGGSEVQLVESGGGLVQPGGSLRLSCVVSGFTFSSYWMTWVRQAPGKGLEWVANIKQDGSERYYVDSVKGRFTISRDSAKNSLYLQMNSLRAEDTAVYYCAREVGYNWNQGGYFDYWGQGTLVTVSS (SEQ ID NO: 274) |
| QSVLTQPPSASGTPGQRVTISCSGSSSNIGSNIVNWYQQFPGTAPKLLLYSDNQRPSGVPDRFSGSKSGTSASLAISGLQSEDEADYYCAAWDDSLNGPVFGTGTKVTVLGGSEGKSSGSGSESKSTGGSQVQLQESGPGLVKPSETLSLTCSVSGGSIRNYYWSWIRQSAGKELEWFGHIFSTGHINYDSSLKSRVTMSVDTSNNQFSLKLRSVTAADTAVYYCARDNGAALFDFWGQGTLVTVSS (SEQ ID NO: 275) |

(continued)

| |
|---|
| DVVMTQSPLSLPVTLGQPASISCRSSQSLVYSDGNTYLNWFQQRPGQSPRRLIYKVSTRDSGVP DRFSGSGSGTDFTLKISRVEAEDVAVYYCLQGTHWPWTFGQGTKVEIKGGSEGKSSGSGSESK STGGSQVQLVESGGGLVQPGGSLRLSCAASGITFSNYWMSWVRQAPGKGLEWVASIKRDGSD KYYVDSVKGRFTISRDNAKNSLSLQMHSLRAEDTAVYYCAKGEFDYWGQGTLVTVSS (SEQ ID NO: 276) |

Dynabeads Human T-Expander CD3/CD28 stimulated T cells are subjected to electroporation, then washed three times with OPTI-MEM (Invitrogen), and resuspended in OPTI-MEM at the final concentration of 50E6/ml. Subsequently, 0.1 ml of the cells (5E6) are mixed with 10ug of IVT CAR encoding RNA and electroporated in a 2-mm Gap cuvette (Harvard Apparatus BTX, Holliston, MA, USA) using BTX ECM830 (Harvard Apparatus BTX, Holliston, MA, USA) by pressing the "pulse" button one time. (Settings: 500 Volts, 750 μsec Pulse Length and single(1) pulse, 100mSec interval.) Post electroporation, the T cells are transferred to a 6-well plate and immediately put back into a 37°C incubator. Primary human T cells are electroporated with no mRNA (MOCK) or 10μg of mRNA expressing either the CD33 scFv CAR or irrelevant control CAR. 24 hours post-electroporation CAR surface expression is measured by flow cytometry following staining with 2 μg/ml biotinylated L-protein and streptavidin-conjugated PE, or biotinylated CD33 (1 μg/ml) and streptavidin-conjugated PE.

Twenty-four hours post electroporation, the T cells are counted. 1E5 T cells are collected for each. The cells are washed with FACS buffer twice using 200 μL/well of FACS buffer for microtiter plates, with the supernatant discarded. All wells are stained with 100 μl staining buffer containing Protein L (Genscript, Cat. No. M000971:500;2ug/ml), and incubated for at least 30 minutes at 4°C while being protected from light. The cells are washed by adding FACS Buffer twice, using 150 μL/well for microtiter plates with FACS buffer. Centrifugation at 400x g is performed for 4 minutes at room temperature. The supernatant is then discarded. All wells are stained with 100 μl Streptavidin-R-Phycoerythrin (SA-PE;1:250) and Live/dead Fixable Near-IR Dead Cell Stain dye (1: 1000), incubated for at least 30 minutes at 4°C while being protected from light. The cells are then ready for flow cytometry analysis.

Observation of protein L staining is observed for the CD33 CARs, whereas only the background staining (~5.5%) is seen in the control T cells that were T cells without mRNA electroporation. CAR expression on primary human T cells also can be detected via J&J internal biotin-labeled recombinant KLK2 protein followed by SA-PE. As shown, T cells efficiently express CD33 CARs as measured by flow cytometry, whereas only the background staining is seen in the control T cells that are T cells without mRNA electroporation or undisclosed control CAR (non-CD33 specific).

**Example 5: Tumor cell killing by CD33 CAR-T cells**

Co-culture for CellTrace Violet (CTV, Thermo Fisher Scientific Catalog number: C34557) based cytotoxicity assay using flow cytometer is performed as follows.

The T cells are prepared as follows. Twenty-four hours post EP, T cells are counted and resuspended at the concentration needed for the most concentrated/desired E:T. The T cells are added at 100 μl/well of assay ($2\times10^6$ cells/ml; plated 100 μl in a 10:1 E:T ratio, i.e., 2E5 T cells per 2E4 target cells). A stock of the 10:1 E:T concentration is made, with two-fold serial dilutions made with complete T cell media (Optimizer w/CTS, 5% Human Serum, 1% GlutaMax) to 0.3125:1. The T cells are plated (100ul/well) in triplicate using a 96 well round bottom tissue culture treated plate.

CTV labeled target cells are prepared as follows. 20 μL DMSO is added to a vial of CTV staining solution. This stock solution is diluted into 20 mL of PBS (warmed to 37°C) for a 5 μM staining solution. 10E6 tumor cells are collected, washed with PBS twice and resuspended in 4E6/ml (2.5ml). An equal volume (2.5ml) of CTV staining solution is added. The cells are incubated for 20 minutes in a 37°C incubator. 40 ml PRMI+20% FBS are added to the cells to absorb any unbound dye. The cells are incubated for 5 minutes. The cells re centrifuged for 5 minutes at 400 x g. The cell pellet is resuspended in pre-warmed RPMI+10%FBS medium. In the meantime, T cells are seeded at the desired E/T ratio described above. A CD33+ tumor cell lines and a CD33- tumor cell line are recounted, and then the cells are resuspended in 2E5/ml and 100ul in duplicate. The cells are co-incubated with labelled tumor cell lines in a flat-bottom 96-well plate.

A cytotoxicity assay is performed as follows using a flow cytometer. After 20 hours of co-culture, all of the cells are transferred to a U-bottom 96-well plate and washed. After 20 hours of co-culture all of the cells are collected from a flat-bottom 96-well plate and transferred to a U-bottom 96-well plate, and then washed. 30 μl of 0.25% trypsin is added to all the wells and incubated for 5 minutes in a 37°C incubator. After 5 minutes, all of the tumor cells are collected to a U-bottom 96-well plate. The cells are centrifuged and washed for 5 minutes at 400 x g twice. The cell pellet is then resuspended in diluted (1:1000) LIVE/DEAD™ Fixable Near-IR staining dye (100 μl). The cells are incubated for 30 mins

at 4 °C, and washed with FACS buffer twice by centrifuging the cells for 5 minutes at 400 x g. After washing, all of the cells are fixed for 10 minutes using 100 μl of BD Cytofix™ Fixation Buffer (50ul FACS buffer +50ulFixation Buffer). The cells are centrifuged and washed for 5 minutes at 400 x g once. The cell pellet is resuspended in FACS buffer. Stained samples are analyzed by multicolor flow cytometry after the end of the incubation period. The percentage of cytotoxic activity is calculated using the following equation:

% specific death = % Near IR+CTV+(dead) cells - % spontaneous Near IR +CTV+ / (100% - % spontaneous Near IR +CTV+ (dead) cells) x 100%.

Twenty-four hours after transient transfection, target cells (CD33 positive Vcap and CD33 negative DU145 cells) are labeled with Cell Trace Violet (CTV) fluorescent dye and then co-cultured with CD33 CAR-T cells. Mock T cells serve as negative effector controls. Cells are co-cultured for 20 hours at the effector-to-target cell (E/T) ratios ranging from 0.3125:1 to 10: 1. The percent killing is measured as the ratio of the absolute number of live (viability dye negative) target (CTV positive) cells remaining in the co-culture relative to the number of live target cells cultured without CAR-T cells. As shown, CD33 CAR T cells specifically and efficiently lyse the CD33(+) human cancer cell lines, but not CD33(-) cells at E/T ratios of 10:1 to 0.3125:1, whereas only the background cytotoxicity is seen in the T cells that were Mock or CD33 CAR.

CD33 CAR-T cells are also tested for real-time cytotoxicity using xCELLigence as a real-time cell analysis system as a potency assay for immune cell-mediated cytolysis of target cells.

50 μL of target cancer cell culturing media is added to each well of the 96-well E-Plates (ACEA Biosciences), and the background impedance is measured and displayed as a Cell Index. Then, adherent target cells CD33(+) and CD33(-) are dissociated and seeded at a density of 5E4 (VCap), 5E3 cells/well of the E-Plate in a volume of 100 μE, and allowed to passively adhere on the electrode surface. Post seeding, the E-Plate is kept at ambient temperature inside a laminar flow hood for 30 minutes and then transferred to the RTCA MP instrument inside a cell culture incubator. Data recording is initiated immediately at 15-minute intervals for the entire duration (96 hours) of the experiment.

At the time treatment is applied (24 hours post cancer cells seeding), data acquisition is paused, 50 μL of media is removed from each well, and effector CAR-T cells are added at different effector to target (E:T) ratios in a volume of 50 μL. CD33(+) CAR-T and undisclosed control CAR (non-CD33 specific) T cells are resuspended. Two-fold dilutions are then performed in duplicate in a 96-well plate (from 5:1 to 0.156:1 E/T ratio). Target plus Mock effector controls (no RNA electroporation T cells) are also added to the target cells.

Target cells CD33(+) and CD33(-) are incubated with Mock, 10μg mRNA electroporated (24 hours post transfection) into CD33(+) and CD33(-) CAR-T cells at different E/T ratios for approximately 72 hours. Normalized cell index (CI) plots for CD33(+) and CD33(-) are generated. When seeded alone, target cells adhered to the plate and proliferated, increasing the CI readout.

**Example 6: Cytokine production by CD33 CAR-T cells**

IFN-γ produced by cytotoxic T cells allows for exertion of immune surveillance of tumors, which can directly inhibit proliferation and induce apoptosis of some malignancies *in vivo* and *in vitro.* To determine whether CD33 CAR-T cells are able to recognize and be activated by CD33 (+) tumor cells, the supernatant was collected from xCELLigence-based killing assay, as described in Example 5. After about 70 hours co-culture, the supernatant was collected and assayed by ELISA according to the directions provided with the ELISA kit (Human IFN-γ ELISA MAX™ Deluxe, BioLegend, Cat#:430106).

IFN-γ production of antigen-stimulated CAR-T cells occurs. CD33 CAR and control CAR-T cells secreted IFN-γ during co-culture with CD33-expressing cells in an E:T ratio-dependent manner, but not during co-culture with CD33-negative cells. Undisclosed control CAR secreted much higher amount of IFN-γ due to the much higher antigen expression level than CD33.

**Example 7: Tumor cell killing by CD33 CAR-T cells**

CD33 CAR-T cells are evaluated in the real-time IncuCyte tumor killing assay for antigen-dependent cytotoxicity. CD33 CAR-T cells are co-incubated with target cells for 88 hours at effector:target ratio of 1:1 or 0.5:1 which was calculated based on CAR expression data. Target cells are identified that are stably expressing a red nuclear dye as measured by IncuCyte imaging system in a real-time fashion. The following calculation is performed: Tumor cell growth inhibition (%) = (Initial Viable Target Cell Number-Current Viable Target Cell Number)/Initial Viable Cell Number* 100 (%).

**Example 8: Cytokine production by CD33 CAR-T cells**

Supernatant is collected from overnight (approximately 20 hours) co-culture of CD33 CAR-T cells with cells at 1:1 of E/T ratio and was analyzed using 13-plex Milliplex Human High Sensitivity T cell kit (HSTCMAG28SPMX13). CD33 CAR modified T cells secreted cytokines during co-culture with CD33-expressing cells, but minimal for un-transduced T cells (UTD).

Supernatant was collected from overnight (approximately 20 hours) co-culture of CD33 CAR-T cells with cells at 1:1 of E/T ratio. CD33 CAR-T cells secreted IFN-$\gamma$ during co-culture with CD33-expressing cells, but not during co-culture with CD33-negative cells. CD3/28 beads stimulated T cells and T cells only were used as positive and negative controls, respectively. IFN-$\gamma$ release by CD33 CAR-T cells. Mean IFN-$\gamma$ concentration $\pm$ SD (pg/ml) from duplicate cultures is shown. Different thermally stabilized CAR-T cells produced different amount of IFN- $\gamma$ when co-culture with CD33 (+) cells.

**Example 9: Proliferation of CD33 CAR-T cells**

CD33 CAR-T cells are evaluated in a proliferation assay. T-cell proliferation is an important *in vitro* parameter of *in vivo* immune function. To further evaluate the function of CD33 CAR-T cells, the CD33 CAR -T cells are labeled with CTV to assess T cells proliferation.

CD33 CAR-T and un-transduced (UTD) T cells are labelled with CellTrace Violet (CTV; 5$\mu$M) and co-cultured with CD33 (+) and CD33 (-) cells. Five days post co-culture, cells are harvested and stained with CD3, CD25, NearIR live/dead Dye and CD33 CAR. Flow cytometric analysis is performed on a Fortessa flow cytometer with Flowjo software. Lymphocytes are identified by live CD3, and the frequencies of CAR-T cells with CTV dye dilution and activation marker CD25 are determined. By gating on CD33 CAR+ T cells, as shown, the CD33 (+) cells but not CD33 (-) cells promote the all CAR constructs. CD3/28 beads stimulated T cells and T cells only are used as positive and negative controls, respectively. T cells only without any stimulation do not proliferate and CD3/28 beads stimulated T cells displayed equivalent proliferation pattern. CD33 CAR-T cells proliferated more robustly than CD3/28 beads positive control after 5 days of co-culture with cells. Different CAR constructs engineered T cells have different proliferation activity and displayed different CAR-T cells counts. The CAR-T cells counts are based on mean absolute cell count +/- SEM from three technical replicates.

The protocol is performed as follows. The tumor cells are collected, washed twice with PBS, and resuspended in 10E6/ml in PBS containing 100ug/ml Mitomycin C (MMC) for 1.5 hours in a 37°C incubator so as to block tumor cells proliferation. 20 $\mu$L of DMSO is added to a vial of CTV staining solution. 5 $\mu$l of the solution is diluted into 5mL (1:1000) PBS (warmed to 37°C) to provide a 5 $\mu$M staining solution. The 2E6 T cells are counted, collected, washed with PBS twice, and resuspended in 4E6/ml (0.5ml). An equal volume (0.5 ml) of CTV staining solution is added. The cells are incubated for 20 minutes at 37°C. Then, 4 ml PRMI+20% FBS is added to the cells to absorb any unbound dye. The cells are incubated for 5 minutes, and centrifuged for 5 minutes at 400 x g. The cell pellet is resuspended in pre-warmed RPMI+10%FBS medium. The T cells are counted, and 1E5 cells (100ul) are seeded in 96-wells flat bottom-plate.

In the meantime, MMC-treated tumor cells are collected and counted after 1.5 hours, and then resuspended at 1E6/ml. 1E5 of the cells (100 $\mu$l) are cocultured with T cells in a 96-well plate. T cells alone, and T cells added 3:1 CD3/28 beads to cells ratio, are used as negative and positive controls, respectively.

After 5 days of co-culture, all of the cells are collected from each well. The cells are centrifuged and washed for 5 minutes at 400 x g twice, then stained CD33 CAR, CD3, CD8 and CD25, live/dead (Near-IR) in 96-well U bottom plate. After washing, all cells are fixed for 10 minutes using 100 $\mu$l BD Cytofix™ Fixation Buffer (50ul FACS buffer +50ulFixation Buffer). The stained samples are analyzed by multicolor flow cytometry after the end of the incubation period.

Data analysis is performed as follows. A CTV histogram is prepared. The CTV undiluted gate is set to encompass the far-right peak (CTV bright) of T cells cultured alone, and the CTV diluted gate to capture the rest of the population. This is applied to all samples.

**Example 10: Characterization of CAR-T cells transduced with CAR17, CAR18, CAR19 or CAR20.**

The generated CAR-T cells are evaluated in the JNL reporter assay for antigen-dependent activity. Briefly, Jurkat cells containing the luciferase gene driven by the signaling-responsive NFAT promoter (termed JNL cells) were transduced with CAR17 (KL2B413 HL), CAR18 (KL2B413 LH), CAR19 (KL2B359 HL) or CAR20 (KL2B359 LH) constructs. Expression of each CAR is determined by biotinylated CD33 followed by streptavidin-conjugated PE.

Binding between the CD33 CAR construct and its cognate cellular antigen (CD33 on target cells) leads to luciferase expression in the JNL cells. To that end, JNL cells transduced with the test CAR constructs or untransduced JNL cells (UTD) were co-cultured with target tumor cell lines and luciferase activity was measured as luminescence

intensity. Constructs were considered active when the luminescence intensity exceeded 1.5-fold the level of UTD cells in the presence of antigen-expressing cells. No antigen-dependent activation was found for the tested CAR constructs.

**CAR-T cells mediate tumor cell killing in antigen-dependent manner**

CAR-T cells are transduced with CAR17, CAR18, CAR19 and CAR20 are co-incubated with CD33 positive cells and CD33 negative cells for 96 hours at effector-to-target (ET) ratio of 1:1 or 0.5:1 which was calculated based on CAR expression on T cells. Target cells were stably expressing a red nuclear dye, which was measured by IncuCyte imaging system in a real-time fashion. Tumor cell growth inhibition (TGI) (%) = (Initial Viable Target Cell Number-Current Viable Target Cell Number)/Initial Viable Cell Number* 100 (%). Tested CAR-T cells achieved approximately 100% TGI whereas the untransduced control did not demonstrate any TGI. No TGI was observed with the tested CAR-T cells in CD33 negative cells.

**CAR-T cells produce cytokines upon antigen stimulation**

IFN-$\gamma$ produced by cytotoxic T cells is critical for exerting immune surveillance of tumors, which can directly inhibit proliferation and induce apoptosis of some malignancies *in vivo* and *in vitro.* To determine whether CD33 CARmodified human T cells were able to recognize and become activated by CD33 positive tumor cells, primary T cells transduced with indicated CAR clones and control untransduced T cells (UTD) were co-cultured with target cells lines and supernatant were collected for IFN-$\gamma$ concentration measurement. CAR-T cells transduced with CD33 CARs cells secreted IFN-$\gamma$ during co-culture with LNCaP cells recombinantly express CD33 cells and also during co-culture with very low CD33-expressing cells but not CD33-negative cells.

**CAR-T cells are activated and upregulate markers of degranulation in antigen-dependent manner**

Tumor cells can be recognized and killed by cytotoxic lymphocytes, such as CD8+ T lymphocytes and natural killer (NK) cells mainly through the immune secretion of lytic granules that kill the target tumor cells. This process involves the fusion of the granule membrane with the cytoplasmic membrane of the immune effector cell, resulting in surface exposure of lysosomal-associated proteins that are typically present on the lipid bilayer surrounding lytic granules, such as CD107a. Therefore, membrane expression of CD107a constitutes a marker of immune cell activation and cytotoxic degranulation.

The degranulation assay is performed as described below. Target cells ($5 \times 10^4$) were co-cultured with an equal number of effector cells in 0.1ml per well in a 96-well plate. Control wells contained T cells alone. Anti-CD107a (5$\mu$l per well) are added in addition to 1$\mu$l/sample of monensin (BD Biosciences) and incubated for 4 hours at 37°C. Cells are washed two times with PBS, stained for expression of the CD33 CAR, CD3, and CD8 and analyzed on a flow cytometer BD Fortessa.

**CD33 CAR-T cells proliferate in antigen-dependent manner**

CAR-T cells are evaluated for their proliferation using T-cell proliferation assay protocol described in Example 9. CD33 CAR-T and untransduced (UTD) T cells were labelled with CellTrace Violet(CTV; 5$\mu$M) and co-cultured with CD33 positive and CD33 negative cells. Five days post co-culture, cells were harvested and stained with CD3, CD25, NearIR live/dead Dye and CD33 CAR. Flow cytometric analysis was performed on a Fortessa flow cytometer with Flowjo software. Lymphocytes were identified by live CD3, and the frequencies of CAR-T cells with CTV dye dilution and activation marker CD25 were determined. By gating on CD3+ T cells, the CD33 positive cells but not CD33 negative cells promoted proliferation of each tested CAR-T cell line. T cells only without any stimulation did not proliferate and CD3/28 beads stimulated T cells displayed equivalent proliferation pattern. CD33 CAR+ T cells proliferated more robustly than CD3/28 beads positive control after 5 days of coculture with cells. Different tested CAR-T cells had different proliferation activity and displayed different CAR-T cells counts. The percentage of proliferating T cells and CD25 expressing T cells was based on mean absolute cell count +/- SEM from duplicate.

**Example 11: Generation of bispecific CD33xCD3 antibodies**

The VH/VL regions of the anti-CD33 antibodies generated in the Examples above and the VH/VL regions of the anti-CD3 antibodies CD3B219 and CD3B376 are engineered into bispecific format and expressed as IgG1. CD3B219 and CD3B376 are engineered as Fabs and the CD33 VH/VL regions are engineered as scFvs in both orientation into the bispecific antibodies, yielding CD33 biding arm in a format scFv-hinge-CH2-CH3 and a CD3 binding arm in a format of heavy chain: VH-CH1-linker-CH2-CH3 and light chain: VL-CL. Alternatively, the VH/VL regions of the anti-CD3 antibodies are engineered as scFvs and the VH/VL regions of the anti-CD33 antibodies are engineered as Fabs. The linker that is used in the scFv is the linker of **SEQ ID NO: 7.** CD3B219 has been described in U.S. Patent No. 9,850,310.

T350V L351Y F405A Y407V CH3 mutations are engineered into one heavy chain and T350V T366L K392L T394W CH3 mutations are engineered into the other heavy chain. In addition, both CD33 and CD3 binding arms are engineered to contain Fc effector silencing mutations L235A_L235A_D265S.

The engineered chains are expressed and the resulting bispecific antibodies purified using standard methods. The bispecific antibodies are characterized for their binding to CD33 and CD3, and their *in vitro* and *in vivo* cytotoxicity as described herein. **Table 10** shows the amino acid sequences of the anti-CD3 antibodies CD3B219 and CD3B376.

**Table 10.**

| Antibody | Region | Amino acid sequence | SEQ ID NO: |
|---|---|---|---|
| CD3B219 | HCDR1 | TYAMN | 108 |
| | HCDR2 | RIRSKYNNYATYYAASVKG | 109 |
| | HCDR3 | HGNFGNSYVSWFAY | 110 |
| | LICDR1 | RSSTGAVTTSNYAN | 111 |
| | LCDR2 | GTNKRAP | 112 |
| | LCDR3 | ALWYSNLWV | 113 |
| | VH (CD3B219VH) | EVQLVESGGGLVQPGGSLRLSCAASGFTFNTY AMNWVRQAPGKGLEWVARIRSKYNNYATY YAASVKGRFTISRDDSKNSLYLQMNSLKTED TAVYYCARHGNFGNSYVSWFAYWGQGTLVT VSS | 114 |
| | VL (CD3B219VL) | QTVVTQEPSLTVSPGGTVTLTCRSSTGAVTTS NYANWVQQKPGQAPRGLIGGTNKRAPGTPA RFSGSLLGGKAALTLSGVQPEDEAEYYCALW YSNLWVFGGGTKLTVL | 115 |
| CD3B376 | HCDR1 | NNNAAWS | 116 |
| | HCDR2 | RTYYRSKWLYDYAVSVKS | 117 |
| | HCDR3 | GYSSSFDY | 118 |
| | LICDR1 | TGTSSNIGTYKFVS | 119 |
| | LCDR2 | EVSKRPS | 120 |
| | LCDR3 | VSYAGSGTLL | 121 |
| | VH (CD3H219) | QVQLQQSGPRLVRPSQTLSLTCAISGDSVFNN NAAWSWIRQSPSRGLEWLGRTYYRSKWLYD YAVSVKSRITVNPDTSRNQFTLQLNSVTPEDT ALYYCARGYSSSFDYWGQGTLVTVSS | 122 |
| | VL (CD3L150) | QSALTQPASVSGSPGQSITISCTGTSSNIGTYKF VSWYQQHPDKAPKVLLYEVSKRPSGVSSRFS GSKSGNTASLTISGLQAEDQADYHCVSYAGS GTLLFGGGTKLTVL | 123 |

**Example 12: Generated bispecific CD33xCD3 antibodies are cytotoxic *in vitro***

T-cell-mediated cytotoxicity assay is used to evaluate the cytotoxicity potential of the generated bispecific antibodies *in vitro,* using live-time lapse imaging on the Incucyte platform. The bispecific antibodies are tested in CD33 positive cell line cells, in the presence of isolated pan human CD3+ T cells from healthy donors at a (Effector:Target) effector:target ratio (E:T ratio) of 3:1. Cell death by apoptosis is monitored by measuring the fluorescence signal from a dye which is stably expressed by target cells. The bispecific antibodies promote a dose-dependent reduction of viable

cells with increasing time and hence induce T cell mediated death of the tumor cells. Percent of tumor cell inhibition %= (Number of initial plated tumor cells - Current number of viable tumor cells) / (Number of initial plated tumor cells) *100%

**Example 13: Generation and characterization of ADC**

Antibodies or antigen binding domains that bind CD33 are to be labeled with various radiometals such as In-111, Zr-89, Lu-177, or Ac-225 and in vivo tumor biodistribution or tumor efficacy is evaluated in established subcutaneous (SC) human prostate models including LNCaP, VCaP or C4-2B in male NOD.Cg-Prkdc$^{scid}$ Il2rg$^{tm1Wjl}$/SzJ (NSG, The Jackson Laboratory, Bar Harbor, ME) or in Balb-c or SHO Nude mice. Various doses of antibodies or antigen binding domains that bind CD33 or isotype control antibodies are labeled with different amounts of radioactivity (10-1000 nCi) and tumor uptake or efficacy is measured.

**Example 14: Testing Anti-CD33 antibodies conjugated with drugs for internalization**

The following tests were performed to characterize the degree of internalization of anti-CD33 antibodies and to determine a therapeutic effect of anti-CD33 antibodies conjugated to drugs. Without wishing to be bound by theory, internalization of an anti-CD33 antibody is suggestive of increased therapeutic benefit of the anti-CD33 antibody when conjugated to a radioactive isotope, e.g. In-111, Zr-89, Lu-177, or Ac-225.

**Materials and Methods**

Cell culture was performed as follows. MOLM-13, Kasumi-1, OCI-AML-3 and HDLM2 cells were cultured in RPMI 1640 (Gibco) media with 10 % FBS (HiMedia). Cells were stained with anti-CD33 antibody (clone WM53, Biolegend) at saturating concentration to determine receptor density on each cell line. Receptor density was determined using BD Quantibrite PE beads (BD Biosciences).

MOLM-13, Kasumi-1, OCI-AML-3 and HDLM-2 cells were stained for viability by fixable violet dead cell stain (L34955, ThermoFisher) according to the manufacturer's protocol. Cells were harvested, washed once with PBS and then stained with the fixable viability dye at room temperature for 30min. Following incubation, cells were washed twice with FACS buffer (PBS+2% FBS). Cells were then used for binding assay with test antibodies. 100,000 cells were stained with the test antibodies in a 100ul staining volume. Antibodies for staining were diluted in FACS buffer and incubated with the cells for 40 mins on ice, followed by washing 2X with FACS buffer. Test antibodies were then detected using the PE conjugated Goat F(ab')2 Anti-Human IgG - Fc (ab98596; Abcam) for 40 minutes on ice. Following washing, data was acquired on the Novocyte flow cytometer (ACEA). Cells were gated on FSC/SSC, followed by live cell gating and doublet discrimination. Data was analyzed using Flow Jo (BD).

Median fluorescence intensity values were used to plot a 4-parameter non-linear regression analysis curve after subtracting the MFI of the secondary only sample.

For kinetics binding studies at 37°C, cells were processed as above for staining with the test antibodies. Test antibodies were conjugated to Alexa Fluor 647 (AF647) dye using the AF647 labeling kit (A20186, ThermoFisher) using the manufacturer's protocol. Post antibody addition, cells were incubated at 37°C in a thermomixer (Eppendorf) for the indicated time points. Samples were then fixed using Cytofix (BD Biosciences) and acquired on the Novocyte (ACEA Biosciences). Data was analyzed as above.

Antibody Internalization assays were performed as follows:
A Protein A-MMAF assay was performed as follows. Protein A-MMAF was purchased from Levena Biopharma. MOLM-13, Kasumi-1, OCI-AML3 and HDLM2 cells were seeded as 4000 cells per well of a 96 well flat bottom dark well plate with transparent bottom (Coming) in a 50ul volume. Antibody stocks were prepared as 4X conc. (400nM) and serially diluted. 25 $\mu$l of each antibody dilution was added to the cells. Protein A-MMAF stock solution was also prepared at 4X concentration (400nM), and 25 $\mu$l was added to each well. Wells with cells and Protein A-MMAF, but no antibody, were considered as 100% viable cells. Cells were incubated with antibodies and Protein A-MMAF for 96 hours. 100ul of CellTitre-Glo (Promega) was added to the cells at the end of the incubation period and luminescence was read in a Tecan plate reader. The % viability was calculated as below and used to plot a 4-parameter non-linear regression curve using Graph Pad Prism.

$$\% \text{ Viability} = \frac{\text{Cells} + \text{MMAF} + \text{Ab}}{\text{Cells} + \text{MMAF}} \text{ X100}$$

A pHAb dye-based internalization assay was performed as follows. pHAb amine reactive dyes were purchased from Promega. 400ug of each test antibody was conjugated to pHAb dyes. Briefly reconstituted the amine reactive pHAb

dye (Promega, #G9845) in 12.5uL DMSO + 12.5uL water (total volume 25uL) for a final concentration of 10mg/mL (11.3mM). Conjugation reactions were run in a 10 fold molar excess of the dye.

Reaction mix contained antibody (500ug in a 250ul volume+3ul of pHAb dye+30ul of 0.5M sodium borate buffer+ water to make the final reaction volume to 300ul. Reactions were incubated at 37C for 2hrs with agitation followed by quenching by addition of 1M Tris pH 7.5 to a final concentration of 10mM. Dye labeled samples were exchanged into PBS with Zeba columns - 2mL, 40kDa cutoff (Thermo 87768). Antibody concentration and DAR values were calculated as follows:

Measured concentrations by NanoDrop

- $$\text{pHAb concentration (uM)} = (\text{Absorbance} / (\text{EC_pHAb} * \text{path length})) \times 10^6,$$

  where:

  - Absorbance = A532
  - EC_pHAb= 75,000

- $$\text{Ab concentration (uM)} = (\text{Absorbance} / (\text{EC-Ab} * \text{path length})) \times 10^6,$$

  where:

  - $$\text{Absorbance} = \text{A280-(0.256*A532)}$$
    - 0.256 is correction factor for residual pHAb absorbance at A280
  - EC_Ab= 210,000

- $$\text{Dye:Ab} = \text{conc. pHAb (uM)} / \text{conc. Ab (uM)}$$

To analyze the kinetics of pHAb dye labeled CD33 antibodies, MOLM-13, Kasumi-1, OCI-AML-3 and HDLM2 cells were incubated with 100nM of pHAb conjugated test antibody for 45 minutes on ice. Antibody dilutions were prepared in FACS buffer. Post labeling, cells were washed with chilled FACS buffer and incubated at 37°C for the indicated time points. At the end of the incubation period, cells were acquired on the Novocyte flow cytometer. An unstained sample was included for every time to account for background fluorescence.

**Results**

Binding was performed by incubating the cells with the serial dilutions of the CD33 antibody at 4°C followed by detection with PE conjugated Goat F(ab')2 Anti-Human IgG - Fc antibody. The data showing binding of CD33 antibodies to target cell lines is shown in **FIGS. 2A** and **2B** as graphs which show the non-linear regression profiles of anti-human CD33 antibodies at 4°C. Median fluorescence intensity (MFI) versus log concentration of antibody are plotted. The top hit for each cell line is indicated in red, and Lintuzumab binding is shown in blue curves.

Time and temperature dependent binding of CD33 antibodies to target cell lines was assayed. Binding was performed by incubating the cells with the serial dilutions of the directly conjugated antibodies at 37°C and 4°C. The data is shown in **FIGS. 3A** and **3B.** The graphs in these figures show the non-linear regression profiles of binding of anti-human CD33 antibodies at 37°C and 4°C. Median fluorescence intensity (MFI) versus log concentrations of antibody are plotted.

Internalization of CD33 antibodies on high, medium and low expression target cell lines was assayed. Cells were cultured with serial dilutions of the CD33 antibodies along with Protein A-MMAF for 96hrs. After 96 hours, CellTitre-Glo reagent was added to cells to measure cell viability. Wells with Protein A-MMAF alone were considered as 100%

viable. HDLM-2 cells were used as a no expression cell line. The results are shown in **FIGS. 4A-4D.** A decrease in cell viability indicates antibody internalization. Graphs indicate non-linear regression analysis for cell viability against log concentration of antibody.

The kinetics of CD33 antibody internalization were assessed. CD33 antibodies were labeled with pHAb dyes and incubated with the cells at a concentration of 100nM at 37°C for the indicated time points following which the cells were washed and acquired on the flow cytometer. The results are shown in **FIG. 5,** in which the graphs show MFI values at indicated time points.

CD33 expression on target cell lines was assayed. High, medium and low CD33 expression cell lines were identified using anti-human CD33 antibody from Biolegend. MOLM-13, Kasumi-1, OCI-AML-3 and HDLM2 cells were stained with the antibody (orange histograms) or the mouse IgG isotype control (blue histograms) and fold change over isotype for each cell line was calculated. The data are shown in **FIG. 6.**

Antibody integrity and DAR values post conjugation with pHAb dyes were assessed. Antibodies were labeled with pHAb dyes and analyzed on SDS-PAGE. Drug to antibody ratios (DAR) were calculated by measuring the OD at 280 and 530 nm and correcting for the dye absorbance at 280nm. The data are shown in **FIG. 7.**

The specificity of pHAb mediated antibody internalization kinetics was determined. CD33 antibodies were labeled with pHAb dyes and incubated with the Kasumi-1 or OCI-AML-3 cells at a concentration of 100nM at 4°C for the indicated time points following which the cells were washed and acquired on the flow cytometer. The data are shown in **FIG. 8A.** Graphs show MFI values at indicated time points. No internalization was observed at 4 °C. Antibodies were also incubated with HDLM-2 cells at 37°C. The data are shown in **FIG. 8B.** No increase in MFI was observed.

The binding $EC_{50}$ values of CD33 antibodies to high (MOLM-13), medium (Kasumi-1) and low (OCI-AML-3) cell lines were assayed. Binding was carried out at 4°C with serial dilutions of the antibody. The data are shown in **FIG. 9.**

CD33 antibody internalization on various target cell lines was assayed, with the data shown in **FIG. 10.** For MOLM-13 and OCI-AML-3, $EC_{50}$ values are indicated. For Kasumi-1, % viability at 100nM concentration is indicated.

## Example 15: Effect of CD33 Antibodies on CD33 Cell Surface Levels

An assay is performed to test the effect of anti-CD33 and CD33 bispecific antibodies on the cell surface level of CD33 on monocytes, macrophages, dendritic cells, neutrophils, T cells, and/or microglia. In the assay, anti-CD33 antibodies are evaluated for their ability to reduce cell surface levels of CD33 on the histiocytic lymphoma cell line U937, as well as CD33-expressing CHO cells, human primary monocytes, human primary macrophages derived from peripheral blood monocytes, human primary dendritic cells derived from peripheral blood monocytes, human microglial cells derived from peripheral blood monocytes, and human primary T cells.

Human microglial cells are prepared from peripheral blood monocytes by cell culture. Monocytes from peripheral human blood samples are isolated using monocyte isolation antibodies, differentiated into dendritic cells, and cultured for five days. Cells are cultured in media containing 10% fetal calf scrum at 37° C in 5% $CO_2$. Non-adherent cells are collected and used for phagocytosis experiments. To generate human macrophages, monocytes from peripheral human blood samples are isolated and differentiated into macrophages or into dendritic cells.

Cell samples are plated at 200,000 cells per ml in 2 ml of RPMI supplemented with 10% Hyclone FBS, 2 mM glutamine, penicillin, streptomycin, and non-essential amino acids. CD33 antibodies or control isotypes are added at 1.0 $\mu$g/ml, and incubated for 24 hours at 37° C. with 5% $CO_2$.

To assess receptor dynamics, antibodies are allowed to bind cells for one hour, and then are washed out. Surface levels of CD33 are determined at least 24 hours later. Cell surface receptor expression is detected by FACS analysis. Cells are incubated with an anti-CD33 antibody conjugated with FITC and with a control surface marker. The cells are washed 2× in FACS buffer (PBS+2% FBS, 2 mM EDTA) and flow cytometry is performed. For analysis, the data is calculated as a percent of receptor expression in the absence of antibody using WI values for the respective fluorophores.

## Example 16: Assay of anti-CD33 antibodies and CD33 bispecific antibodies for induction or inhibition of signaling molecule phosphorylation

Various cells (e.g., J774, RAW 264.7, BMM cells, human primary monocytes, macrophages, dendritic cells, T cells Microglia or osteoclasts) are cultured individually. The cells are removed from culture dishes and counted. The cells are then incubated with (i) an anti-CD33 antibody, (ii) an anti-CD33 bispecific antibody, or (iii) an isotype-matched control antibody. The cells are then lysed and centrifuged to remove any insoluble materials. Immunoprecipitation reactions are performed on each supernatant with one or more different antibodies against each of DAP 12, ERK, AKT, protein A-agarose, and protein G-agarose. The beads are extensively washed with RIPA buffer. The proteins are separated by SDS-polyacrylamide gel electrophoresis (SDS-PAGE) and then transferred to nitrocellulose membranes by Western blotting. The beads are then incubated with antibodies that specifically recognize phosphorylated tyrosine or

phosphorylated form of DAP12, ERK, Syk, LCK, FYN, C-Cbl, VAV, or AKT. The beads are visualized with an enhanced chemiluminescence (ECL) system so as to quantify the degree of phosphorylation of DAP12, ERK, Syk, LCK, FYN, C-Cbl, VAV, and AKT.

**Example 17: *In vivo* Analysis of the Anti-Cancer Effect of CD33 Antibodies and/or Bispecific Antibodies**

Groups of 10 mice at 8 weeks (+/-2 weeks) of age, either regular mice or mice that overexpress the human CD33 gene, are challenged subcutaneously with tumor cells (e.g. $1\times10^5$ to $1\times10^6$ MC38, Lewis Lung, or B16 cells) suspended in 1.00 μl PBS. The mice are anesthetized with isoflurane prior to implant. Starting at day 2, groups of mice are injected i.p. every 3 days for 4 doses with 200 μg of each of antagonistic anti-CD33 antibodies. Tumor growth is monitored with a caliper biweekly to measure tumor growth starting at day 4. The endpoint of the experiment is a tumor volume of 2000 $mm^3$ or 60 days. Tumor growth and % survival are the outcome measures. If the anti-CD33 has an anti-cancer effect, one or more of the following is observed: reduced tumor take and growth rate, reduced number of tumor infiltrating immune suppressor macrophages, and increased effector T cell influx into the tumor.

**Example 18: Anti-TRGV9 and Anti-CD33 Bispecific Antibodies**

γ/δ T cell stimulation and expansion was performed. Expansion of Vγ9-Vδ2 T cells was carried out by treating PBMCs in complete RPMI media containing rhIL-2 (1000 IU/mL), rhIL-15 (10 ng/mL) and Zoledronic acid (5 μM) for 14 days.

De novo sequencing of the anti-Vy9 monoclonal antibodies was performed as follows. The mouse IgG1 anti-human T cell receptor anti-TRGV9 clone 7A5 was obtained from Abeam (cat # ab171109). Sample preparation and LC-MS/MS analysis were performed by Lake Pharma (San Carlos, CA). The sample was reduced and alkylated, divided into seven aliquots, and proteolytically digested with Trypsin/LysC, Chymotrypsin, LysC, Pepsin, and AspN, Elastase, and Proteinase K enzymes. Resulting peptides were desalted using a ZipTip C18 Pipette Tips and separated on-line using reverse phase chromatography. Mass spectrometry was performed on Thermo Q-Exactive spectrometer using HCD fragmentation. MS data sets were analyzed using PEAKS software by matching *de novo* sequence tags to an IMGT-based antibody sequences database. Gaps in the sequence were assigned using Contig sequence assembly of *de novo* identified peptides. All CDRs and hyper-mutations were confirmed by inspecting the MS/MS spectra.

The sequences of four monoclonal antibodies generated according to the above, as well as their CDR sequences, are shown in Tables 11-15 below.

**Table 11: CDR Sequences of anti-TRGV9 mAb (LP7A5_1)**

| **Antibody** | **HCDR1** | **SEQ ID NO:** | **HCDR2** | **SEQ ID NO:** | **HCDR3** | **SEQ ID NO:** |
|---|---|---|---|---|---|---|
| LP7A5_1 | DHYIN | 236 | QIYPGDGNTYYNQKFKG | 237 | NYGDYTIDF | 238 |
| **Antibody** | **LCDR1** | **SEQ ID NO:** | **LCDR2** | **SEQ ID NO:** | **LCDR3** | **SEQ ID NO:** |
| LP7A5_1 | KSSQSLLYSSNQKNYLA | 239 | WASTRES | 240 | QQYYRYHT | 241 |

**Table 12: CDR Sequences of anti-TRGV9 mAb.**

| **Antibody** | **HCDR1** | **SEQ ID NO:** | **HCDR2** | **SEQ ID NO:** | **HCDR3** | **SEQ ID NO:** |
|---|---|---|---|---|---|---|
| LP7A5_2 | DHYIN | 236 | QIYPGDGNTYYNQKFKG | 237 | NMGMYTIDF | 242 |
| **Antibody** | **LCDR1** | **SEQ ID NO:** | **LCDR2** | **SEQ ID NO:** | **LCDR3** | **SEQ ID NO:** |
| LP7A5_2 | KSSQSLLYSSNQKNYLA | 239 | WASTRES | 240 | QQYYRYHT | 241 |

**Table 13: CDR Sequences of anti-TRGV9 mAb.**

| **Antibody** | **HCDR1** | **SEQ ID NO:** | **HCDR2** | **SEQ ID NO:** | **HCDR3** | **SEQ ID NO:** |
|---|---|---|---|---|---|---|
| LP7A5_3 | DHYIN | 236 | QIYPGDGNTYYNQKFKG | 237 | NMGMYTLDF | 243 |
| **Antibody** | **LCDR1** | **SEQ ID NO:** | **LCDR2** | **SEQ ID NO:** | **LCDR3** | **SEQ ID NO:** |
| LP7A5_3 | KSSQSLLYSSNQKNYLA | 239 | WASTRES | 240 | QQYYRYHT | 241 |

**Table 14: CDR Sequences of anti-TRGV9 mAb.**

| **Antibody** | **HCDR1** | **SEQ ID NO:** | **HCDR2** | **SEQ ID NO:** | **HCDR3** | **SEQ ID NO:** |
|---|---|---|---|---|---|---|
| LP7A5_4 | DHYIN | 236 | QIYPGDGNTYYNQKFKG | 237 | NYGDYTLDF | 244 |
| **Antibody** | **LCDR1** | **SEQ ID NO:** | **LCDR2** | **SEQ ID NO:** | **LCDR3** | **SEQ ID NO:** |
| LP7A5_4 | KSSQSLLYSSNQKNYLA | 239 | WASTRES | 240 | QQYYRYHT | 241 |

**Table 15: Heavy chain and light chain sequences of anti-TRGV9 mAb**

| **mAb** | **Heavy Chain Amino Acid Sequence (CDR sequences bold and underlined)** | **SEQ ID NO:** |
|---|---|---|
| LP7A5_1 | EVQLQQSGAELARPGASVKLSCKASGFTFT**DHYIN**WVKQRTGQGLEWIG**QIYPGDGNTYYNQKFKG**KATLTADKSSSTAYMQLSSLTSEDSAVYFCAP**NYGDYTIDF**WGQGTSVTVSS | 245 |
| LP7A5_2 | EVQLQQSGAELARPGASVKLSCKASGFTFT**DHYIN**WVKQRTGQGLEWIG**QIYPGDGNTYYNQKFKG**KATLTADKSSSTAYMQLSSLTSEDSAVYFCAP**NMGMYTIDF**WGQGTSVTVSS | 246 |
| LP7A5_3 | EVQLQQSGAELARPGASVKLSCKASGFTFT**DHYIN**WVKQRTGQGLEWIG**QIYPGDGNTYYNQKFKG**KATLTADKSSSTAYMQLSSLTSEDSAVYFCAP**NMGMYTLDF**WGQGTSVTVSS | 247 |
| LP7A5_4 | EVQLQQSGAELARPGASVKLSCKASGFTFT**DHYIN**WVKQRTGQGLEWIG**QIYPGDGNTYYNQKFKG**KATLTADKSSSTAYMQLSSLTSEDSAVYFCAP**NYGDYTLDF**WGQGTSVTVSS | 248 |
| | **Light Chain Amino Acid Sequence** | **SEQ ID NO:** |
| LP7A5_1 | DIVMSQSPSSLAVSVGEKVTMSC**KSSQSLLYSSNQKNYLA**WYQQKPGQSPKLLIY**WASTRES**GVPDRFTGSGSGTDFTLTISSVKAEDLAVYYC**QQYYRYHT**FGTGTKLEIK | 249 |

Preparation of the Vγ9 × CD33 bispecific antibody was performed as follows. The variable region sequence of 7A5 (anti-TRGV9) and C33B904 (anti-CD33 antibody) was used to generate a bispecific antibody to be tested for T cell re-directed killing of acute myeloid leukemia (AML) cells. The bispecific antibodies VG4 (anti-TRGV9 × CD33) and VG3 (anti-TRGV9 × Null) were produced as full-length antibodies in the knob-into-hole format as human IgG4. Nucleic acid sequences encoding variable regions were sub-cloned into a custom mammalian expression vectors containing constant region of human IgG4 expression cassettes using standard PCR restriction enzyme based standard cloning

techniques, and sequenced verified. The bispecific antibodies were expressed by transient transfection in a Chinese hamster ovary (CHO) cell line. The sequences of the bispecific antibodies expressed in the CHO cells are shown in Table 16 below.

**Table 16: Sequences of antibodies expressed in CHO cells**

| mAb ID | 'Knob' arm and 'hole' arm amino acid sequence | SEQ ID NO: |
|---|---|---|
| ANTI-TRGV9 ('hole' arm) | MAWVWTLLFLMAAAQSIQADIVMSQSPSSLAVSVGEKVTMSCKSSQSLLYS<br>SNQKNYLAWYQQKPGQSPKLLIYWASTRESGVPDRFTGSGSGTDFTLTISSV<br>KAEDLAVYYCQQYYRYHTFGTGTKLEIKRTVAAPSVFIFPPSDEQLKSGTAS<br>VVCLLNNFYPREAKVQWKVDNALQSGNSQESVTEQDSKDSTYSLSSTLTLS<br>KADYEKHKVYACEVTHQGLSSPVTKSFNRGECGGSEGKSSGSGSESKSTEG<br>KSSGSGSESKSTGGSEVQLQQSGAELARPGASVKLSCKASGFTFTDHYINWV<br>KQRTGQGLEWIGQIYPGDGNTYYNQKFKGKATLTADKSSSTAYMQLSSLTS<br>EDSAVYFCAPNYGDYTIDFWGQGTSVTVSSASTKGPSVFPLAPCSRSTSEST<br>AALGCLVKDYFPEPVTVSWNSGALTSGVHTFPAVLQSSGLYSLSSVVTVPSS<br>SLGTKTYTCNVDHKPSNTKVDKRVESKYGPPCPPCPAPEAAGGPSVFLFPPK<br>PKDTLMISRTPEVTCVVVDVSQEDPEVQFNWYVDGVEVHNAKTKPREEQF<br>NSTYRVVSVLTVLHQDWLNGKEYKCKVSNKGLPSSIEKTISKAKGQPREPQ<br>VYTLPPSQEEMTKNQVSLSCAVKGFYPSDIAVEWESNGQPENNYKTTPPVL<br>DSDGSFFLVSRLTVDKSRWQEGNVFSCSVMHEALHNRFTQKSLSLSLGK | 250 |
| anti-CD33 ('knob' arm) | MAWVWTLLFLMAAAQSIQADIVMTQSPDSLAVSLGERATINCKSSQTVFYS<br>SNNKNYLAWYQQKPGQPPKLLISWASTRKSGVPDRFSGSGSGTDFTLTVSSL<br>QAEDVAVYYCQHYYSTPYTFGQGTKLEIKRTVAAPSVFIFPPSDEQLKSGTA<br>SVVCLLNNFYPREAKVQWKVDNALQSGNSQESVTEQDSKDSTYSLSSTLTL<br>SKADYEKHKVYACEVTHQGLSSPVTKSFNRGECGGSEGKSSGSGSESKSTE<br>GKSSGSGSESKSTGGSEVQLVESGGGLVQPGRSLRLSCAASGFTFDDYAMH<br>WVRQAPGKGLEWVSGIGWSGGSIVYADSVKGRFTISRDNAKNSLYLQMNS<br>LRAEDTALYYCAKDSPYGDFFDYWGQGTLVTVSSASTKGPSVFPLAPCSRS<br>TSESTAALGCLVKDYFPEPVTVSWNSGALTSGVHTFPAVLQSSGLYSLSSVV<br>TVPSSSLGTKTYTCNVDHKPSNTKVDKRVESKYGPPCPPCPAPEAAGGPSVF<br>LFPPKPKDTLMISRTPEVTCVVVDVSQEDPEVQFNWYVDGVEVHNAKTKPR<br>EEQFNSTYRVVSVLTVLHQDWLNGKEYKCKVSNKGLPSSIEKTISKAKGQP<br>REPQVYTLPPSQEEMTKNQVSLWCLVKGFYPSDIAVEWESNGQPENNYKTT<br>PPVLDSDGSFFLYSRLTVDKSRWQEGNVFSCSVMHEALHNHYTQKSLSLSL<br>GK | 251 |
| Anti-RSV ('knob' arm) | MAWVWTLLFLMAAAQSIQAEIVLTQSPGTLSLSPGERATLSCRASQSVSSSY<br>LAWYQQKPGQAPRLLIYGASSRATGIPDRFSGSGSGTDFTLTISRLEPEDFAV<br>YYCQQDYGFPWTFGQGTKVEIKRTVAAPSVFIFPPSDEQLKSGTASVVCLLN<br>NFYPREAKVQWKVDNALQSGNSQESVTEQDSKDSTYSLSSTLTLSKADYEK<br>HKVYACEVTHQGLSSPVTKSFNRGECGGSEGKSSGSGSESKSTEGKSSGSGS<br>ESKSTGGSEVQLVQSGAEVKKPGESLKISCKGSGYSFTSYWISWVRQMPGK<br>GLEWMGIIDPSDSDTRYSPSFQGQVTISADKSISTAYLQWSSLKASDTAMYY<br>CARGDGSTDLDYWGQGTLVTVSSASTKGPSVFPLAPCSRSTSESTAALGCLV<br>KDYFPEPVTVSWNSGALTSGVHTFPAVLQSSGLYSLSSVVTVPSSSLGTKTY<br>TCNVDHKPSNTKVDKRVESKYGPPCPPCPAPEAAGGPSVFLFPPKPKDTLMI<br>SRTPEVTCVVVDVSQEDPEVQFNWYVDGVEVHNAKTKPREEQFNSTYRVV<br>SVLTVLHQDWLNGKEYKCKVSNKGLPSSIEKTISKAKGQPREPQVYTLPPSQ<br>EEMTKNQVSLWCLVKGFYPSDIAVEWESNGQPENNYKTTPPVLDSDGSFFL<br>YSRLTVDKSRWQEGNVFSCSVMHEALHNHYTQKSLSLSLGK | 252 |

The antibodies were initially purified by Mab Select SuRe Protein A column (GE Healthcare). The column was equilibrated with PBS pH 7.2 and loaded with fermentation supernatant at a flow rate of 2 mL/min. After loading, the column was washed with 4 column volumes of PBS followed by elution in 30 mM sodium acetate, pH 3.5. Fractions containing protein peaks as monitored by absorbance at 280 nm were pooled and neutralized to pH 5.0 by adding 1%

3 M sodium acetate pH 9.0. The bispecific mAbs were further purified on a preparative Superdex 200 10/300 GL (GE healthcare) size exclusion chromatography (SEC) column equilibrated with PBS buffer. The integrity of the sample was assessed by endotoxin measurement and SDS-PAGE under reducing and non-reducing conditions. A representative gel for VG68 is shown in FIG. 12. The final protein concentrations were 1.0 mg/ml for anti-TRGV9/anti-CD33 and 1.0 mg/mL for ANTI-TRGV9/Null. The final EU levels of ANTI-TRGV9/anti-CD33 and ANTI-TRGV9/Null based on these were <3.0 EU/mg.

The binding activity of anti-CD33 antibody on target cell lines was assessed. The binding of anti-CD33 clone C33B904 to a panel CD33+ cell lines were measured by FACS. The EC50 and EC90 were calculated for MOLM-13 (FIG. 13), Kasumi-1 (FIG. 14) and OCI-AML-3 (FIG. 16).

Characterization of Vγ9+ (γδ) T cells and Pan T-cells was performed as follows. Zoledronic acid was used to selectively expand $V\gamma 9^+$ γδ T cells from whole PBMCs. The PBMCs were isolated from whole fresh PBMCs using the EasySep™Human γδ T cell isolation kit (Stem cell Technologies; Vancouver, CA) according to the manufacturer's instructions. Isolated PBMCs were cultured in RPMI-10 (RPMI supplemented with 10%FBS, 1x Pen/Strep) medium with recombinant human IL-2 (rhIL-2) to a final concentration of 1000IU/mL, recombinant human IL-15 (rhIL-15) to a final concentration of 10ng/mL, and Zoledronic acid to a final concentration of 5μM for 14 days.

Evaluation of binding and cytotoxic properties of the anti-TRGV9/anti-CD33 bispecific antibody using Kasumi-3 cells and human γδ T cells was performed as follows. Enriched γδ T cells (Effectors), isolated from PBMCs cultured with Zoledronic acid + IL-2 + IL-15 for 12 days, were co-cultured with CFSE labelled Kasumi-3 cells (Targets) at 1:1 and 5:1 E:T ratios in the presence of various concentrations of the bispecific antibody for 24 hours. Dose response curves show anti-TRGV9/anti-CD33 and anti-TRGV9/anti-NULL bispecific mediated γδ T cell cytotoxicity against CD33 expressing kasumi-3 cells in a dose dependent manner at 1:1 (Fig.6) and 5:1 (Fig.7) E:T ratios. Cytotoxicity values represented here were subtracted of basal cytotoxicity value observed in the absence of bispecific antibody. $EC_{50}$ values were calculated as described in methods. Representative data shown from a single experiment are provided in **FIGS. 16-17,** which show that the anti-TRGV9/anti-CD33 bispecific antibody mediates γδ T cell cytotoxicity against CD33 expressing Kasumi-3 cells *in vitro.*

Healthy donor derived PBMCs (Effectors), cultured with Zoledronic acid + IL-2 + IL-15 for 12 days, were co-cultured with CFSE labelled MOLM-13 cells (Targets) at 1:1 E:T ratios in the presence of various concentrations of the bispecific antibody for 24 hours. **FIG. 18** shows that the anti-TRGV9/anti-CD33 bispecific antibody mediates γδ T cell cytotoxicity (from whole PBMCs) against CD33 expressing MOLM-13 cells *in vitro.* Dose response curves in **FIG. 18** show anti-TRGV9/anti-CD33 and anti-TRGV9/anti-NULL bispecific mediated γδ T cell cytotoxicity against CD33 expressing kasumi-3 cells in a dose dependent manner. Cytotoxicity values represented there were subtracted of basal cytotoxicity value observed in the absence of bispecific antibody. $EC_{50}$ values were calculated as described in methods. Representative data shown here are from a single experiment.

### Example 19: *In silico* analysis and NKK library design

*In silico* analysis of the VH and VL domains identified herein was performed to evaluate the potential for these VH and VL domains to undergo post-translational modifications (PTMs). An NNK library was generated at certain PTM sites and screened to determine the effects of mutation of amino acids in a potential PTM.

Potential PTM risks were identified using internal sequence analysis tool focused on identifying motifs including NG, DG, and DP. NNK saturation mutagenesis libraries were designed for the first and second positions of each motif. The NNK library utilized codons (N=A, G, C, T; K=G, T) such that all 20 amino acids are represented in the library. Libraries were cloned into an *E.coli* expression plasmid by infusion cloning. Libraries were plated and colonies were isolated to represent mutated PTMs. **FIG. 19** shows *in silico* analysis of PTMs, with PTMs selected for NNK library in boxes.

### Example 20: Testing thermostability of scFv variants generated from NNK library

The thermostability of parental and mutated scFv clones generated in Example 19 was evaluated. Cultures of parental clone and mutagenesis libraries were inoculated and grown overnight. Expression was induced and supernatants were analyzed by ELISA. CD33 was plated on 96 well plates and a binding ELISA was performed. Supernatants were split into 4 separate plates for heating. The assay was performed on untreated RT, 55° C, 60° C, 65° C to assess thermal stability. Luminescence signals were normalized to untreated room temperature of the same clone.

**FIG. 20** shows the thermostability of scFvs generated from NNK library based on C33B1475 (SEQ ID NO: 277). **FIG. 21** shows the thermostability of scFv generated from NNK library based on C33B1516 (SEQ ID NO: 278). **FIG. 22** shows the thermostability of scFv generated from NNK library based on C33B1517SEQ (SEQ ID NO: 216). **FIG. 23** shows the thermostability of scFv generated from NNK library based on C33B1522 (SEQ ID NO: 213). **FIG. 24** is a table with preferred residues identified during library screen of NNK library.

These figures illustrate that removal of potential PTM sites by mutation did not impair binding to CD33 or reduce

thermostability. Unexpected improvements in stability were seen in several scFv clones.

**Numbered embodiments**

The invention also provides the following numbered embodiments:

List of numbered embodiments (A)

1) An isolated protein that binds CD33, the isolated protein comprising

a) an HCDR1, an HCDR2 and an HCDR3 of a heavy chain variable region (VH) of SEQ ID NO: 18;
b) an HCDR1, an HCDR2 and an HCDR3 of a VH of SEQ ID NO: 19;
c) an HCDR1, an HCDR2 and an HCDR3 of a VH of SEQ ID NO: 20;
d) an HCDR1, an HCDR2 and an HCDR3 of a VH of SEQ ID NO: 21;
e) an HCDR1, an HCDR2 and an HCDR3 of a VH of SEQ ID NO: 22;
f) an HCDR1, an HCDR2 and an HCDR3 of a VH of SEQ ID NO: 23;
g) an HCDR1, an HCDR2 and an HCDR3 of a VH of SEQ ID NO: 24;
h) an HCDR1, an HCDR2 and an HCDR3 of a VH of SEQ ID NO: 25;
i) an HCDR1, an HCDR2 and an HCDR3 of a VH of SEQ ID NO: 26; or
j) an HCDR1, an HCDR2 and an HCDR3 of a VH of SEQ ID NO: 27,

or a CD33 binding fragment thereof.
2) The isolated protein of embodiment 1, comprising the HCDR1, the HCDR2, the HCDR3 of

a) SEQ ID NOs: 1, 6, and 12, respectively;
b) SEQ ID NOs: 2, 7, and 13, respectively;
c) SEQ ID NOs: 1, 8, and 14, respectively;
d) SEQ ID NOs: 3, 9, and 13, respectively;
e) SEQ ID NOs: 4, 10, and 15, respectively;
f) SEQ ID NOs: 5, 11, and 16, respectively; or
g) SEQ ID NOs: 5, 11, and 17, respectively.

3) The isolated protein of embodiments 1 or 2, wherein isolated protein is a scFv, a $(scFv)_2$, a Fv, a Fab, a $F(ab')_2$, a Fd, a dAb or a VHH.
4) The isolated protein of embodiment 3, wherein the isolated protein is the VHH.
5) The isolated protein of embodiment 3, wherein the isolated protein is the Fab.
6) The isolated protein of embodiment 3, wherein the isolated protein is the scFv.
7) The isolated protein of any of embodiments 1-6, wherein the isolated protein comprises the VH of any one of SEQ ID NOs: 18, 19, 20, 21, 22, 23, 24, 25, 26, or 27.
8) An isolated protein that binds CD33, wherein the isolated protein comprises a VHH of any one of SEQ ID NOs: 18, 19, 20, 21, 22, 23, 24, 25, 26, or 27, or a CD33 binding fragment thereof.
9) An isolated protein that binds CD33, wherein the isolated protein comprises

a) an HCDR1, an HCDR2 and an HCDR3 of a heavy chain variable region (VH) of SEQ ID NO: 52;
b) an HCDR1, an HCDR2 and an HCDR3 of a VH of SEQ ID NO: 53;
c) an HCDR1, an HCDR2 and an HCDR3 of a VH of SEQ ID NO: 54;
d) an HCDR1, an HCDR2 and an HCDR3 of a VH of SEQ ID NO: 55;
e) an HCDR1, an HCDR2 and an HCDR3 of a VH of SEQ ID NO: 56;
f) an HCDR1, an HCDR2 and an HCDR3 of a VH of SEQ ID NO: 57;
g) an HCDR1, an HCDR2 and an HCDR3 of a VH of SEQ ID NO: 58;
h) an HCDR1, an HCDR2 and an HCDR3 of a VH of SEQ ID NO: 59;
i) an HCDR1, an HCDR2 and an HCDR3 of a VH of SEQ ID NO: 60; or
j) an HCDR1, an HCDR2 and an HCDR3 of a VH of SEQ ID NO: 61.

10) The isolated protein of embodiment 9, comprising the HCDR1, the HCDR2, and the HCDR3 of

a) SEQ ID NOs: 28, 36, and 45, respectively;
b) SEQ ID NOs: 29, 37, and 46, respectively;
c) SEQ ID NOs: 30, 38, and 47, respectively;
d) SEQ ID NOs: 31, 39, and 48, respectively;
e) SEQ ID NOs: 32, 40, and 49, respectively;
f) SEQ ID NOs: 33, 41, and 50, respectively;
g) SEQ ID NOs: 34, 42, and 51, respectively;
h) SEQ ID NOs: 34, 43, and 51, respectively;
i) SEQ ID NOs: 34, 44, and 51, respectively; or
j) SEQ ID NOs: 35, 42, and 51, respectively.

11) The isolated protein of embodiment 9 or 10, wherein the isolated protein comprises

a) an HCDR1, an HCDR2 and an HCDR3 of a heavy chain variable region (VH) of SEQ ID NO: 52 and a light chain complementarity determining region (LCDR) 1, an LCDR2 and an LCDR3 of a light chain variable region (VL) of SEQ ID NO: 81;
b) an HCDR1, an HCDR2 and an HCDR3 of a VH of SEQ ID NO: 53 and an LCDR1, an LCDR2 and an LCDR3 of a VL of SEQ ID NO: 82;
c) an HCDR1, an HCDR2 and an HCDR3 of a VH of SEQ ID NO: 54 and an LCDR1, an LCDR2 and an LCDR3 of a VL of SEQ ID NO: 83;
d) an HCDR1, an HCDR2 and an HCDR3 of a VH of SEQ ID NO: 55 and an LCDR1, an LCDR2 and an LCDR3 of a VL of SEQ ID NO: 84;
e) an HCDR1, an HCDR2 and an HCDR3 of a VH of SEQ ID NO: 56 and an LCDR1, an LCDR2 and an LCDR3 of a VL of SEQ ID NO: 85;
f) an HCDR1, an HCDR2 and an HCDR3 of a VH of SEQ ID NO: 57 and an LCDR1, an LCDR2 and an LCDR3 of a VL of SEQ ID NO: 86;
g) an HCDR1, an HCDR2 and an HCDR3 of a VH of SEQ ID NO: 58 and an LCDR1, an LCDR2 and an LCDR3 of a VL of SEQ ID NO: 87;
h) an HCDR1, an HCDR2 and an HCDR3 of a VH of SEQ ID NO: 59 and an LCDR1, an LCDR2 and an LCDR3 of a VL of SEQ ID NO: 87;
i) an HCDR1, an HCDR2 and an HCDR3 of a VH of SEQ ID NO: 60 and an LCDR1, an LCDR2 and an LCDR3 of a VL of SEQ ID NO: 87; or
j) an HCDR1, an HCDR2 and an HCDR3 of a VH of SEQ ID NO: 61 and an LCDR1, an LCDR2 and an LCDR3 of a VL of SEQ ID NO: 88.

12) The isolated protein of embodiment 11, comprising the HCDR1, the HCDR2, the HCDR3, the LCDR1, the LCDR2 and the LCDR3 of

a) SEQ ID NOs: 28, 36, 45, 62, 69, and 75, respectively;
b) SEQ ID NOs: 29, 37, 46, 63, 70, and 76, respectively;
c) SEQ ID NOs: 30, 38, 47, 64, 71, and 77, respectively;
d) SEQ ID NOs: 31, 39, 48, 65, 72, and 78, respectively;
e) SEQ ID NOs: 32, 40, 49, 66, 70, and 76, respectively;
f) SEQ ID NOs: 33, 41, 50, 67, 73, and 79, respectively;
g) SEQ ID NOs: 34, 42, 51, 68, 74, and 80, respectively;
h) SEQ ID NOs: 34, 43, 51, 68, 74, and 80, respectively;
i) SEQ ID NOs: 34, 44, 51, 68, 74, and 80, respectively; or
j) SEQ ID NOs: 35, 42, 51, 68, 74, and 80, respectively.

13) The isolated protein of any one of embodiments 9-12, wherein the isolated protein is a scFv, a $(scFv)_2$, a Fv, a Fab, a $F(ab')_2$, a Fd, a dAb or a VHH.
14) The isolated protein of embodiment 13, wherein the isolated protein is the Fab.
15) The isolated protein of embodiment 13, wherein the isolated protein is the VHH.
16) The isolated protein of embodiment 13, wherein the isolated protein is the scFv.
17) The isolated protein of embodiment 16, wherein the scFv comprises, from the N- to C-terminus, a VH, a first linker (L1) and a VL (VH-L1-VL) or the VL, the L1 and the VH (VL-L1-VH).
18) The isolated protein of embodiment 17, wherein the L1 comprises

a) about 5-50 amino acids;
b) about 5-40 amino acids;
c) about 10-30 amino acids; or
d) about 10-20 amino acids.

19) The isolated protein of embodiment 18, wherein the L1 comprises an amino acid sequence of any one of SEQ ID NOs: 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, or 140.
20) The isolated protein of embodiment 19, wherein the L1 comprises the amino acid sequence of SEQ ID NO: 108.
21) The isolated protein of any one of embodiments 9-20, wherein the isolated protein comprises the VH of SEQ ID NOs: 52, 53, 54, 55, 56, 57, 58, 59, 60, or 61, and the VL of SEQ ID NOs: 81, 82, 83, 84, 85, 86, 87, or 88.
22) The isolated protein of embodiment 21, wherein the isolated protein comprises:

a) the VH of SEQ ID NO: 52 and the VL of SEQ ID NO: 81;
b) the VH of SEQ ID NO: 53 and the VL of SEQ ID NO: 82;
c) the VH of SEQ ID NO: 54 and the VL of SEQ ID NO: 83;
d) the VH of SEQ ID NO: 55 and the VL of SEQ ID NO: 84;
e) the VH of SEQ ID NO: 56 and the VL of SEQ ID NO: 85;
f) the VH of SEQ ID NO: 57 and the VL of SEQ ID NO: 86;
g) the VH of SEQ ID NO: 58 and the VL of SEQ ID NO: 87;
h) the VH of SEQ ID NO: 59 and the VL of SEQ ID NO: 87;
i) the VH of SEQ ID NO: 60 and the VL of SEQ ID NO: 87; or
j) the VH of SEQ ID NO: 61 and the VL of SEQ ID NO: 88.

23) An isolated protein that binds CD33, wherein the isolated protein comprises

a) the VH of SEQ ID NO: 52 and the VL of SEQ ID NO: 81;
b) the VH of SEQ ID NO: 53 and the VL of SEQ ID NO: 82;
c) the VH of SEQ ID NO: 54 and the VL of SEQ ID NO: 83;
d) the VH of SEQ ID NO: 55 and the VL of SEQ ID NO: 84;
e) the VH of SEQ ID NO: 56 and the VL of SEQ ID NO: 85;
f) the VH of SEQ ID NO: 57 and the VL of SEQ ID NO: 86;
g) the VH of SEQ ID NO: 58 and the VL of SEQ ID NO: 87;
h) the VH of SEQ ID NO: 59 and the VL of SEQ ID NO: 87;
i) the VH of SEQ ID NO: 60 and the VL of SEQ ID NO: 87; or
j) the VH of SEQ ID NO: 61 and the VL of SEQ ID NO: 88.

24) The isolated protein of embodiment 23, wherein the isolated protein is a scFv, a $(scFv)_2$, a Fv, a Fab, a $F(ab')_2$, a Fd, a dAb or a VHH.
25) The isolated protein of embodiment 24, wherein the isolated protein is the Fab.
26) The isolated protein of embodiment 24, wherein the isolated protein is the VHH.
27) The isolated protein of embodiment 24, wherein the isolated protein is the scFv.
28) The isolated protein of embodiment 27, wherein the scFv comprises, from the N- to C-terminus, a VH, a first linker (L1) and a VL (VH-L1-VL) or the VL, the L1 and the VH (VL-L1-VH).
29) The isolated protein of embodiment 28, wherein the L1 comprises

a) about 5-50 amino acids;
b) about 5-40 amino acids;
c) about 10-30 amino acids; or
d) about 10-20 amino acids.

30) The isolated protein of embodiment 29, wherein the L1 comprises an amino acid sequence of any one of SEQ ID NOs: 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, or 140.
31) The isolated protein of embodiment 30, wherein the L1 comprises the amino acid sequence of SEQ ID NO: 108.
32) An isolated protein that binds CD33, wherein the isolated protein comprises

a) an HCDR1, an HCDR2 and an HCDR3 of a heavy chain variable region (VH) of SEQ ID NO: 95;

b) an HCDR1, an HCDR2 and an HCDR3 of a VH of SEQ ID NO: 96;
c) an HCDR1, an HCDR2 and an HCDR3 of a VH of SEQ ID NO: 97;
d) an HCDR1, an HCDR2 and an HCDR3 of a VH of SEQ ID NO: 170;
e) an HCDR1, an HCDR2 and an HCDR3 of a VH of SEQ ID NO: 171;
f) an HCDR1, an HCDR2 and an HCDR3 of a VH of SEQ ID NO: 175;
g) an HCDR1, an HCDR2 and an HCDR3 of a VH of SEQ ID NO: 179;
h) an HCDR1, an HCDR2 and an HCDR3 of a VH of SEQ ID NO: 181;
i) an HCDR1, an HCDR2 and an HCDR3 of a VH of SEQ ID NO: 96.

33) The isolated protein of embodiment 32, comprising the HCDR1, the HCDR2, and the HCDR3 of

a) SEQ ID NOs: 89, 90, and 92, respectively;
b) SEQ ID NOs: 89, 90, and 93, respectively;
c) SEQ ID NOs: 33, 91, and 94, respectively
d) SEQ ID NOs: 167, 168, and 169, respectively;
e) SEQ ID NOs: 172, 173, and 174, respectively;
f) SEQ ID NOs: 176, 177, and 178, respectively;
g) SEQ ID NOs: 89, 90, and 180, respectively; or
h) SEQ ID NOs: 89, 90, and 93, respectively.

34) The isolated protein of embodiment 32 or 33, wherein the isolated protein comprises

a) an HCDR1, an HCDR2 and an HCDR3 of a heavy chain variable region (VH) of SEQ ID NO: 95 and a light chain complementarity determining region (LCDR) 1, an LCDR2 and an LCDR3 of a light chain variable region (VL) of SEQ ID NO: 105;
b) an HCDR1, an HCDR2 and an HCDR3 of a VH of SEQ ID NO: 96 and an LCDR1, an LCDR2 and an LCDR3 of a VL of SEQ ID NO: 106;
c) an HCDR1, an HCDR2 and an HCDR3 of a VH of SEQ ID NO: 97 and an LCDR1, an LCDR2 and an LCDR3 of a VL of SEQ ID NO: 107;
d) an HCDR1, an HCDR2 and an HCDR3 of a VH of SEQ ID NO: 170 and an LCDR1, an LCDR2 and an LCDR3 of a VL of SEQ ID NO: 185;
e) an HCDR1, an HCDR2 and an HCDR3 of a VH of SEQ ID NO: 171 and an LCDR1, an LCDR2 and an LCDR3 of a VL of SEQ ID NO: 188;
f) an HCDR1, an HCDR2 and an HCDR3 of a VH of SEQ ID NO: 175 and an LCDR1, an LCDR2 and an LCDR3 of a VL of SEQ ID NO: 192;
g) an HCDR1, an HCDR2 and an HCDR3 of a VH of SEQ ID NO: 179 and an LCDR1, an LCDR2 and an LCDR3 of a VL of SEQ ID NO: 196;
h) an HCDR1, an HCDR2 and an HCDR3 of a VH of SEQ ID NO: 181 and an LCDR1, an LCDR2 and an LCDR3 of a VL of SEQ ID NO: 200; or
i) an HCDR1, an HCDR2 and an HCDR3 of a VH of SEQ ID NO: 96 and an LCDR1, an LCDR2 and an LCDR3 of a VL of SEQ ID NO: 202.

35) The isolated protein of embodiment 34, comprising the HCDR1, the HCDR2, the HCDR3, the LCDR1, the LCDR2 and the LCDR3 of

a) SEQ ID NOs: 89, 90, 92, 98, 101, and 104, respectively;
b) SEQ ID NOs: 89, 90, 93, 99, 102, and 104, respectively;
c) SEQ ID NOs: 33, 91, 94, 100, 103, and 104, respectively;
d) SEQ ID NOs: 167, 168, 169, 182, 183, and 184, respectively;
e) SEQ ID NOs: 33, 91, 94, 186, 187, and 104, respectively;
f) SEQ ID NOs: 172, 173, 174, 189, 190, and 191, respectively;
g) SEQ ID NOs: 176, 177, 178, 193, 194, and 195, respectively;
h) SEQ ID NOs: 89, 90, 180, 197, 198, and 199, respectively;
i) SEQ ID NOs: 89, 90, 93, 201, 102, and 104, respectively.

36) The isolated protein of any one of embodiments 32-35, wherein the isolated protein is a scFv, a $(scFv)_2$, a Fv, a Fab, a $F(ab')_2$, a Fd, a dAb or a VHH.
37) The isolated protein of embodiment 36, wherein the isolated protein is the Fab.

38) The isolated protein of embodiment 36, wherein the isolated protein is the VHH.
39) The isolated protein of embodiment 36, wherein the isolated protein is the scFv.
40) The isolated protein of embodiment 39, wherein the scFv comprises, from the N- to C-terminus, a VH, a first linker (L1) and a VL (VH-L1-VL) or the VL, the L1 and the VH (VL-L1-VH).
41) The isolated protein of embodiment 40, wherein the L1 comprises

a) about 5-50 amino acids;
b) about 5-40 amino acids;
c) about 10-30 amino acids; or
d) about 10-20 amino acids.

42) The isolated protein of embodiment 41, wherein the L1 comprises an amino acid sequence of any one of SEQ ID NOs: 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, or 140.
43) The isolated protein of embodiment 42, wherein the L1 comprises the amino acid sequence of SEQ ID NO: 108.
44) The isolated protein of any one of embodiments 32-43, wherein the antigen binding domain that binds CD33 comprises the VH of SEQ ID NOs: 95, 96, 97, 170, 171, 175, 179, 181, or 96, and the VL of SEQ ID NOs: 105, 106, 107, 185, 188, 192, 196, 200, or 202.
45) The isolated protein of embodiment 44, wherein the antigen binding domain that binds CD33 comprises:

a) the VH of SEQ ID NO: 95 and the VL of SEQ ID NO: 105;
b) the VH of SEQ ID NO: 96 and the VL of SEQ ID NO: 106;
c) the VH of SEQ ID NO: 97 and the VL of SEQ ID NO: 107;
d) the VH of SEQ ID NO: 170 and the VL of SEQ ID NO: 185;
e) the VH of SEQ ID NO: 171 and the VL of SEQ ID NO: 188;
f) the VH of SEQ ID NO: 175 and the VL of SEQ ID NO: 192;
g) the VH of SEQ ID NO: 179 and the VL of SEQ ID NO: 196;
h) the VH of SEQ ID NO: 181 and the VL of SEQ ID NO: 200; or
i) the VH of SEQ ID NO: 96 and the VL of SEQ ID NO: 202.

46) An isolated protein that binds CD33, wherein the isolated protein comprises

a) the VH of SEQ ID NO: 95 and the VL of SEQ ID NO: 105;
b) the VH of SEQ ID NO: 96 and the VL of SEQ ID NO: 106;
c) the VH of SEQ ID NO: 97 and the VL of SEQ ID NO: 107;
d) the VH of SEQ ID NO: 170 and the VL of SEQ ID NO: 185;
e) the VH of SEQ ID NO: 171 and the VL of SEQ ID NO: 188;
f) the VH of SEQ ID NO: 175 and the VL of SEQ ID NO: 192;
g) the VH of SEQ ID NO: 179 and the VL of SEQ ID NO: 196;
h) the VH of SEQ ID NO: 181 and the VL of SEQ ID NO: 200; or
i) the VH of SEQ ID NO: 96 and the VL of SEQ ID NO: 202.

47) The isolated protein of embodiment 46, wherein the isolated protein is a scFv, a $(scFv)_2$, a Fv, a Fab, a $F(ab')_2$, a Fd, a dAb or a VHH.
48) The isolated protein of embodiment 47, wherein the isolated protein is the Fab.
49) The isolated protein of embodiment 47, wherein the isolated protein is the VHH.
50) The isolated protein of embodiment 47, wherein the isolated protein is the scFv.
51) The isolated protein of embodiment 50, wherein the scFv comprises, from the N- to C-terminus, a VH, a first linker (L1) and a VL (VH-L1-VL) or the VL, the L1 and the VH (VL-L1-VH).
52) The isolated protein of embodiment 51, wherein the L1 comprises

a) about 5-50 amino acids;
b) about 5-40 amino acids;
c) about 10-30 amino acids; or
d) about 10-20 amino acids.

53) The isolated protein of embodiment 52, wherein the L1 comprises an amino acid sequence of any one of SEQ ID NOs: 108, 109, 110, 111, 112, 113, 114, 115, 116, 117, 118, 119, 120, 121, 122, 123, 124, 125, 126, 127, 128,

129, 130, 131, 132, 133, 134, 135, 136, 137, 138, 139, or 140.
54) The isolated protein of embodiment 53, wherein the L1 comprises the amino acid sequence of SEQ ID NO: 108.
55) The isolated protein of any one of embodiments 1-54, wherein the protein is conjugated to a half-life extending moiety.
56) The isolated protein of embodiment 55, wherein the half-life extending moiety is an immunoglobulin (Ig), a fragment of the Ig, an Ig constant region, a fragment of the Ig constant region, a Fc region, transferrin, albumin, an albumin binding domain or polyethylene glycol.
57) The isolated protein of any one of embodiments 1-56, wherein the isolated protein is a monospecific protein.
58) The isolated protein of any one of embodiments 1-57, wherein the isolated protein is a multispecific protein.
59) The isolated protein of embodiment 58, wherein the multispecific protein is a bispecific protein.
60) The isolated protein of embodiment 59, wherein the multispecific protein is a trispecific protein.
61) The isolated protein of any one of embodiments 1-60, further comprising an immunoglobulin (Ig) constant region or a fragment of the Ig constant region thereof.
62) The isolated protein of embodiment 61, wherein the fragment of the Ig constant region comprises a Fc region.
63) The isolated protein of embodiment 61, wherein the fragment of the Ig constant region comprises a CH2 domain.
64) The isolated protein of embodiment 61, wherein the fragment of the Ig constant region comprises a CH3 domain.
65) The isolated protein of embodiment 61, wherein the fragment of the Ig constant region comprises the CH2 domain and the CH3 domain.
66) The isolated protein of embodiment 61, wherein the fragment of the Ig constant region comprises at least portion of a hinge, the CH2 domain and the CH3 domain.
67) The isolated protein of embodiment 61, wherein the fragment of the Ig constant region comprises a hinge, the CH2 domain and the CH3 domain.
68) The isolated protein of any one of embodiments 61-67, comprising an antigen binding domain that binds CD33 that is conjugated to the N-terminus of the Ig constant region or the fragment of the Ig constant region.
69) The isolated protein of any one of embodiments 61-67, comprising an antigen binding domain that binds CD33 that is conjugated to the C-terminus of the Ig constant region or the fragment of the Ig constant region.
70) The isolated protein of any one of embodiments 61-69, comprising an antigen binding domain that binds CD33 that is conjugated to the Ig constant region or the fragment of the Ig constant region via a second linker (L2).
71) The isolated protein of embodiment 70, wherein the L2 comprises the amino acid sequence of any one of SEQ ID NOs:108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, or 140.
72) The isolated protein of any one of embodiments 58-71, wherein the multispecific protein comprises an antigen binding domain that binds an antigen on a lymphocyte.
73) The isolated protein of embodiment 72, wherein the lymphocyte is a T cell.
74) The isolated protein of embodiment 72, wherein the T cell is a $CD8^+$ T cell
75) The isolated protein of embodiment 72, wherein the lymphocyte is a natural killer (NK) cell.
76) The isolated protein of any one of embodiments 58-75, wherein the multispecific protein comprises an antigen binding domain that binds CD3, CD3 epsilon (CD3ε), CD8, KI2L4, NKG2E, NKG2D, NKG2F, BTNL3, CD186, BTNL8, PD-1, CD195, TRGV9, or NKG2C.
77) The isolated protein of embodiment 76, wherein the multispecific protein comprises an antigen binding domain that binds CD3ε.
78) The isolated protein of embodiment 77, wherein the antigen binding domain that binds CD3ε comprises:

a) a heavy chain complementarity determining region 1 (HCDR1) of SEQ ID NO: 141, a HCDR2 of SEQ ID NO: 142, a HCDR3 of SEQ ID NO: 143, a light chain complementarity determining region 1 (LCDR1) of SEQ ID NO: 144, a LCDR2 of SEQ ID NO: 145 and a LCDR3 of SEQ ID NO: 146;
b) the VH of SEQ ID NO: 147 and the VL of SEQ ID NO: 148;
c) the HCDR1 of SEQ ID NO: 149, the HCDR2 of SEQ ID NO: 150, the HCDR3 of SEQ ID NO: 151, the LCDR1 of SEQ ID NO: 152, the LCDR2 of SEQ ID NO: 153 and the LCDR3 of SEQ ID NO: 154; or
d) the VH of SEQ ID NO: 155 and the VL of SEQ ID NO: 156.

79) The isolated protein of embodiment 76, wherein the multispecific protein comprises an antigen binding domain that binds TRGV9.
80) The isolated protein of embodiment 79, wherein the antigen binding domain that binds TRGV9 comprises:

a) a heavy chain complementarity determining region 1 (HCDR1) of SEQ ID NO: 236, a HCDR2 of SEQ ID NO: 237, a HCDR3 of SEQ ID NO: 238, a light chain complementarity determining region 1 (LCDR1) of SEQ ID NO: 239, a LCDR2 of SEQ ID NO: 240 and a LCDR3 of SEQ ID NO: 241;

b) a heavy chain complementarity determining region 1 (HCDR1) of SEQ ID NO: 236, a HCDR2 of SEQ ID NO: 237, a HCDR3 of SEQ ID NO: 242, a light chain complementarity determining region 1 (LCDR1) of SEQ ID NO: 239, a LCDR2 of SEQ ID NO: 240 and a LCDR3 of SEQ ID NO: 241;
c) a heavy chain complementarity determining region 1 (HCDR1) of SEQ ID NO: 236, a HCDR2 of SEQ ID NO: 237, a HCDR3 of SEQ ID NO: 243, a light chain complementarity determining region 1 (LCDR1) of SEQ ID NO: 239, a LCDR2 of SEQ ID NO: 240 and a LCDR3 of SEQ ID NO: 241;
d) a heavy chain complementarity determining region 1 (HCDR1) of SEQ ID NO: 236, a HCDR2 of SEQ ID NO: 237, a HCDR3 of SEQ ID NO: 244, a light chain complementarity determining region 1 (LCDR1) of SEQ ID NO: 239, a LCDR2 of SEQ ID NO: 240 and a LCDR3 of SEQ ID NO: 241;
e) the VH of SEQ ID NO: 245 and the VL of SEQ ID NO: 249;
f) the VH of SEQ ID NO: 246 and the VL of SEQ ID NO: 249;
g) the VH of SEQ ID NO: 247 and the VL of SEQ ID NO: 249; or
h) the VH of SEQ ID NO: 248 and the VL of SEQ ID NO: 249.

81) The isolated protein of any one of embodiments 61-80, wherein the Ig constant region or the fragment of the Ig constant region is an IgG1, an IgG2, an IgG3 or an IgG4 isotype.
82) The isolated protein of any one of embodiments 61-81, wherein the Ig constant region or the fragment of the Ig constant region comprises at least one mutation that results in reduced binding of the protein to a Fcy receptor (FcγR).
83) The isolated protein of embodiment 82, wherein the at least one mutation that results in reduced binding of the protein to the FcyR is selected from the group consisting of F234A/L235A, L234A/L235A, L234A/L235A/D265S, V234A/G237A/ P238S/H268A/V309L/A330S/P331S, F234A/L235A, S228P/F234A/ L235A, N297A, V234A/G237A, K214T/E233P/ L234V/L235A/G236-deleted/A327G/P331A/D365E/L358M, H268Q/V309L/A330S/P331S, S267E/L328F, L234F/L235E/D265A, L234A/L235A/G237A/P238S/H268A/A330S/P331S, S228P/F234A/L235A/G237A/P238S and S228P/F234A/L235A/G236-deleted/G237A/P238S, wherein residue numbering is according to the EU index.
84) The isolated protein of any one of embodiments 61-81, wherein the Ig constant region or the fragment of the Ig constant region comprises at least one mutation that results in enhanced binding of the protein to the FcyR.
85) The isolated protein of embodiment 84, wherein the at least one mutation that results in enhanced binding of the protein to the FcyR is selected from the group consisting of S239D/I332E, S298A/E333A/K334A, F243L/R292P/Y300L, F243L/R292P/Y300L/P396L, F243L/R292P/Y300L/V305I/P396L and G236A/S239D/I332E, wherein residue numbering is according to the EU index.
86) The isolated protein of any one of embodiments 82-85, wherein the FcyR is FcyRI, FcyRIIA, FcyRIIB or FcyRIII, or any combination thereof.
87) The isolated protein of any one of embodiments 61-86, wherein the Ig constant region of the fragment of the Ig constant region comprises at least one mutation that modulates a half-life of the protein.
88) The isolated protein of embodiment 87, wherein the at least one mutation that modulates the half-life of the protein is selected from the group consisting of H435A, P257I/N434H, D376V/N434H, M252Y/S254T/T256E/H433K/N434F, T308P/N434A and H435R, wherein residue numbering is according to the EU index.
89) The isolated protein of any one of the embodiments 61-88, wherein the protein comprises at least one mutation in a CH3 domain of the Ig constant region.
90) The isolated protein of embodiment 89, wherein the at least one mutation in the CH3 domain of the Ig constant region is selected from the group consisting of T350V, L351Y, F405A, Y407V, T366Y, T366W, F405W, T394W, T394S, Y407T, Y407A, T366S/L368A/Y407V, L351Y/F405A/Y407V, T366I/K392M/T394W, F405A/Y407V, T366L/K392M/T394W, L351Y/Y407A, T366A/K409F, L351Y/Y407A, T366V/K409F, T366A/K409F, T350V/L351Y/F405A/Y407V and T350V/T366L/K392L/T394W, wherein residue numbering is according to the EU index.
91) The isolated protein of any one of embodiments 1-54, wherein the protein is a chimeric antigen receptor (CAR).
92) The isolated protein of embodiment 91, wherein the CAR comprises

a) an extracellular domain comprising the antigen binding domain that binds CD33 of any one of embodiments 1-54;
b) a transmembrane domain; and
c) an intracellular signaling domain optionally comprising at least one co-stimulatory domain.

93) The isolated protein of embodiment 91 or 92, wherein the CAR further comprises a CD8a-hinge region.
94) The isolated protein of any one of embodiments 91-93, wherein

a) the transmembrane domain comprises a CD8a transmembrane region (CD8a-TM) polypeptide; and
b) the intracellular signaling domain comprises a co-stimulatory domain comprising a TNF receptor superfamily member 9 (CD137) component and a primary signaling domain comprising a T-cell surface glycoprotein CD3 zeta chain (CD3z) component.

95) The isolated protein of any one of embodiments 91-93, wherein

a) the CD8a-hinge region comprises an amino acid sequence that is at least 90% identical to the amino acid sequence of SEQ ID NO: 157;
b) the transmembrane domain comprises an amino acid sequence that is at least 90% identical to the amino acid sequence of SEQ ID NO: 162; and/or
c) the intracellular signaling domain comprises a co-stimulatory domain having an amino acid sequence that is at least 90% identical to the amino acid sequence of SEQ ID NO: 163 and a primary signaling domain having an amino acid sequence that is at least 90% identical to the amino acid sequence of SEQ ID NO: 164.

96) The isolated protein of embodiment 95, wherein the intracellular signaling domain comprises a polypeptide component selected from the group consisting of a TNF receptor superfamily member 9 (CD137) component, a T-cell surface glycoprotein CD3 zeta chain (CD3z) component, a cluster of differentiation (CD27) component, a cluster of differentiation superfamily member component, or any combinations thereof.
97) A pharmaceutical composition comprising the isolated protein of any one of embodiments 1-96 and a pharmaceutically acceptable carrier.
98) A polynucleotide encoding the isolated protein of any one of embodiments 1-96.
99) A vector comprising the polynucleotide of embodiment 98.
100) A host cell comprising the vector of embodiment 99.
101) A method of producing the isolated protein of any one of embodiments 1-96, comprising culturing the host cell of embodiment 100 in conditions that the protein is expressed, and recovering the protein produced by the host cell.
102) An anti-idiotypic antibody binding to the isolated protein of any one of embodiments 1-96.
103) A kit comprising the isolated protein of any one of embodiments 1-96.
104) A pharmaceutical composition comprising a means for treating a CD33 expressing cancer in a subject.
105) A pharmaceutical composition comprising a means for reducing the amount of CD33 expressing tumor cells in a subject.
106) A pharmaceutical composition comprising a means for preventing establishment of a CD33 expressing cancer in a subject.
107) A pharmaceutical composition comprising a means for treating a noncancerous condition in a subject at risk of developing a CD33 expressing cancer.

List of numbered embodiments (B)

1) An isolated multispecific protein comprising a first antigen binding domain that binds CD33 and a second antigen binding domain that binds a lymphocyte antigen.
2) The isolated multispecific protein of embodiment 1, wherein the lymphocyte antigen is a T cell antigen.
3) The isolated multispecific protein of embodiment 1, wherein the T cell antigen is a $CD8^+$ T cell antigen.
4) The isolated multispecific protein of embodiment 1, wherein the lymphocyte antigen is a NK cell antigen.
5) The isolated multispecific protein of any one of embodiments 1-4, wherein the lymphocyte antigen is CD3, CD3 epsilon (CD3ε), CD8, KI2L4, NKG2E, NKG2D, NKG2F, BTNL3, CD186, BTNL8, PD-1, CD195, TRGV9, or NKG2C.
6) The isolated multispecific protein of embodiment 5, wherein the lymphocyte antigen is CD3ε.
7) The isolated multispecific protein of any one of embodiments 1-6, wherein the first antigen binding domain that binds CD33 and/or the second antigen binding domain that binds the lymphocyte antigen comprise a scFv, a (scFv)z, a Fv, a Fab, a $F(ab')_2$, a Fd, a dAb or a VHH.
8) The isolated multispecific protein of embodiment 7, wherein the first antigen binding domain that binds CD33 and/or the second antigen binding domain that binds the lymphocyte antigen comprise the Fab.
9) The isolated multispecific protein of embodiment 7, wherein the first antigen binding domain that binds CD33 and/or the second antigen binding domain that binds the lymphocyte antigen comprise the VHH.
10) The isolated multispecific protein of embodiment 7, wherein the first antigen binding domain that binds CD33 and/or the second antigen binding domain that binds the lymphocyte antigen comprise the scFv.
11) The isolated multispecific protein of embodiment 10, wherein the scFv comprises, from the N- to C-terminus, a

VH, a first linker (L1) and a VL (VH-L1-VL) or the VL, the L1 and the VH (VL-L1-VH).
12) The isolated multispecific protein of embodiment 11, wherein the L1 comprises

a) about 5-50 amino acids;
b) about 5-40 amino acids;
c) about 10-30 amino acids; or
d) about 10-20 amino acids.

13) The isolated multispecific protein of embodiment 12, wherein the L1 comprises the amino acid sequence of any one of SEQ ID NOs: 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, or 140.
14) The isolated multispecific protein of embodiment 13, wherein the L1 comprises the amino acid sequence of SEQ ID NO: 108.
15) The isolated multispecific of any one of embodiments 1-14, wherein the first antigen binding domain that binds CD33 comprises the HCDR1 of any one of SEQ ID NOs: 1, 2, 3, 4, or 5, the HCDR2 of any one of SEQ ID NOs: 6, 7, 8, 9, 10, or 11, and the HCDR3 of any one of SEQ ID NOs: 12, 13, 14, 15, 16, or 17.
16) The isolated multispecific protein of any one of 1-15, wherein the first antigen binding domain that binds CD33 comprises the HCDR1, the HCDR2, the HCDR3 of

a) SEQ ID NOs: 1, 6, and 12, respectively;
b) SEQ ID NOs: 2, 7, and 13, respectively;
c) SEQ ID NOs: 1, 8, and 14, respectively;
d) SEQ ID NOs: 3, 9, and 13, respectively;
e) SEQ ID NOs: 4, 10, and 15, respectively;
f) SEQ ID NOs: 5, 11, and 16, respectively; or
g) SEQ ID NOs: 5, 11, and 17, respectively,
or a CD33 binding fragment thereof.

17) The isolated multispecific protein of any one of embodiments 1-16, wherein the first antigen binding domain that binds CD33 comprises the VH of any one of SEQ ID NOs: 18, 19, 20, 21, 22, 23, 24, 25, 26, or 27.
18) The isolated multispecific protein of any one of embodiments 1-14, wherein the first antigen binding domain that binds CD33 comprises the HCDR1 of SEQ ID NOs: 28, 29, 30, 31, 32, 33, 34, or 35, the HCDR2 of SEQ ID NOs: 36, 37, 38, 39, 40, 41, 42, 43, or 44, the HCDR3 of SEQ ID NOs: 45, 46, 47, 48, 49, 50, or 51, the LCDR1 of SEQ ID NOs: 62, 63, 64, 65, 66, 67, or 68, the LCDR2 of SEQ ID NOs: 69, 70, 71, 72, 73, or 74, and the LCDR3 of SEQ ID NOs: 75, 76, 77, 78, 79, or 80.
19) The isolated multispecific protein of any one of embodiments 1-14 and 18, wherein the first antigen binding domain that binds CD33 comprises the HCDR1, the HCDR2, the HCDR3, the LCDR1, the LCDR2 and the LCDR3 of

a) SEQ ID NOs: 28, 36, 45, 62, 69, and 75, respectively;
b) SEQ ID NOs: 29, 37, 46, 63, 70, and 76, respectively;
c) SEQ ID NOs: 30, 38, 47, 64, 71, and 77, respectively;
d) SEQ ID NOs: 31, 39, 48, 65, 72, and 78, respectively;
e) SEQ ID NOs: 32, 40, 49, 66, 70, and 76, respectively;
f) SEQ ID NOs: 33, 41, 50, 67, 73, and 79, respectively;
g) SEQ ID NOs: 34, 42, 51, 68, 74, and 80, respectively;
h) SEQ ID NOs: 34, 43, 51, 68, 74, and 80, respectively;
i) SEQ ID NOs: 34, 44, 51, 68, 74, and 80, respectively; or
j) SEQ ID NOs: 35, 42, 51, 68, 74, and 80, respectively.

20) The isolated multispecific protein of any one of embodiments 1-14, 18, and 19, wherein the antigen binding domain that binds CD33 comprises the VH of SEQ ID NOs: 52, 53, 54, 55, 56, 57, 58, 59, 60, or 61 and the VL of SEQ ID NOs: 81, 82, 83, 84, 85, 86, 87, or 88.
21) The isolated multispecific protein of any one of embodiments 1-14 and 18-20, wherein the first antigen binding domain that binds CD33 comprises

a) the VH of SEQ ID NO: 52 and the VL of SEQ ID NO: 81;
b) the VH of SEQ ID NO: 53 and the VL of SEQ ID NO: 82;
c) the VH of SEQ ID NO: 54 and the VL of SEQ ID NO: 83;

d) the VH of SEQ ID NO: 55 and the VL of SEQ ID NO: 84;
e) the VH of SEQ ID NO: 56 and the VL of SEQ ID NO: 85;
f) the VH of SEQ ID NO: 57 and the VL of SEQ ID NO: 86;
g) the VH of SEQ ID NO: 58 and the VL of SEQ ID NO: 87;
h) the VH of SEQ ID NO: 59 and the VL of SEQ ID NO: 87;
i) the VH of SEQ ID NO: 60 and the VL of SEQ ID NO: 87; or
j) the VH of SEQ ID NO: 61 and the VL of SEQ ID NO: 88.

22) The isolated multispecific protein of any one of embodiments 1-14, wherein the first antigen binding domain that binds CD33 comprises the HCDR1 of SEQ ID NOs: 33, 89, 167, 172 or 176, the HCDR2 of SEQ ID NOs: 90, 91, 168, 173, or 177, the HCDR3 of SEQ ID NOs: 92, 93, 94, 169, 174, 178, or 180, the LCDR1 of SEQ ID NOs: 98, 99, 100, 182, 186, 189, 193, 197, or 201, the LCDR2 of SEQ ID NOs: 101, 102, 103, 183, 187, 190, 194, or 198, and the LCDR3 of SEQ ID NO: 104, 184, 191, 195, or 199.
23) The isolated multispecific protein of any one of embodiments 1-14 and 22, wherein the first antigen binding domain that binds CD33 comprises the HCDR1, the HCDR2, the HCDR3, the LCDR1, the LCDR2 and the LCDR3 of

a) SEQ ID NOs: 89, 90, 92, 98, 101, and 104, respectively;
b) SEQ ID NOs: 89, 90, 93, 99, 102, and 104, respectively;
c) SEQ ID NOs: 33, 91, 94, 100, 103, and 104, respectively;
d) SEQ ID NOs: 167, 168, 169, 182, 183, and 184, respectively;
e) SEQ ID NOs: 33, 91, 94, 186, 187, and 104, respectively;
f) SEQ ID NOs: 172, 173, 174, 189, 190, and 191, respectively;
g) SEQ ID NOs: 176, 177, 178, 193, 194, and 195, respectively;
h) SEQ ID NOs: 89, 90, 180, 197, 198, and 199, respectively; or
i) SEQ ID NOs: 89, 90, 93, 201, 102, and 104, respectively.

24) The isolated multispecific protein of any one of embodiments 1-14, 22 and 23, wherein the antigen binding domain that binds CD33 comprises the VH of SEQ ID NOs: 95, 96, 97, 170, 171, 175, 179, 181, or 96, and the VL of SEQ ID NOs 105, 106, 107, 185, 188, 192, 196, 200, or 202.
25) The isolated multispecific protein of any one of embodiments 1-14 and 22-24, wherein the first antigen binding domain that binds CD33 comprises

a) the VH of SEQ ID NO: 95 and the VL of SEQ ID NO: 105;
b) the VH of SEQ ID NO: 96 and the VL of SEQ ID NO: 106;
c) the VH of SEQ ID NO: 97 and the VL of SEQ ID NO: 107;
d) the VH of SEQ ID NO: 170 and the VL of SEQ ID NO: 185;
e) the VH of SEQ ID NO: 171 and the VL of SEQ ID NO: 188;
f) the VH of SEQ ID NO: 175 and the VL of SEQ ID NO: 192;
g) the VH of SEQ ID NO: 179 and the VL of SEQ ID NO: 196;
h) the VH of SEQ ID NO: 181 and the VL of SEQ ID NO: 200; or
i) the VH of SEQ ID NO: 96 and the VL of SEQ ID NO: 202.

26) The isolated multispecific protein of any one of embodiments 1-25, wherein the second antigen binding domain that binds the lymphocyte antigen comprises

a) the HCDR1 of SEQ ID NO: 141, the HCDR2 of SEQ ID NO: 142, the HCDR3 of SEQ ID NO: 143, the LCDR1 of SEQ ID NO: 144, the LCDR2 of SEQ ID NO: 145 and the LCDR3 of SEQ ID NO: 146;
b) the VH of SEQ ID NO: 147 and the VL of SEQ ID NO: 148;
c) the HCDR1 of SEQ ID NO: 149, the HCDR2 of SEQ ID NO: 150, the HCDR3 of SEQ ID NO: 151, the LCDR1 of SEQ ID NO: 152, the LCDR2 of SEQ ID NO: 153 and the LCDR3 of SEQ ID NO: 154; or
d) the VH of SEQ ID NO: 155 and the VL of SEQ ID NO: 156.

27) The isolated multispecific protein of any one of embodiments 1-25, wherein the second antigen binding domain that binds the lymphocyte antigen comprises

a) a heavy chain complementarity determining region 1 (HCDR1) of SEQ ID NO: 236, a HCDR2 of SEQ ID NO: 237, a HCDR3 of SEQ ID NO: 238, a light chain complementarity determining region 1 (LCDR1) of SEQ ID NO: 239, a LCDR2 of SEQ ID NO: 240 and a LCDR3 of SEQ ID NO: 241;

b) a heavy chain complementarity determining region 1 (HCDR1) of SEQ ID NO: 236, a HCDR2 of SEQ ID NO: 237, a HCDR3 of SEQ ID NO: 242, a light chain complementarity determining region 1 (LCDR1) of SEQ ID NO: 239, a LCDR2 of SEQ ID NO: 240 and a LCDR3 of SEQ ID NO: 241;
c) a heavy chain complementarity determining region 1 (HCDR1) of SEQ ID NO: 236, a HCDR2 of SEQ ID NO: 237, a HCDR3 of SEQ ID NO: 243, a light chain complementarity determining region 1 (LCDR1) of SEQ ID NO: 239, a LCDR2 of SEQ ID NO: 240 and a LCDR3 of SEQ ID NO: 241;
d) a heavy chain complementarity determining region 1 (HCDR1) of SEQ ID NO: 236, a HCDR2 of SEQ ID NO: 237, a HCDR3 of SEQ ID NO: 244, a light chain complementarity determining region 1 (LCDR1) of SEQ ID NO: 239, a LCDR2 of SEQ ID NO: 240 and a LCDR3 of SEQ ID NO: 241;
e) the VH of SEQ ID NO: 245 and the VL of SEQ ID NO: 249;
f) the VH of SEQ ID NO: 246 and the VL of SEQ ID NO: 249;
g) the VH of SEQ ID NO: 247 and the VL of SEQ ID NO: 249; or
h) the VH of SEQ ID NO: 248 and the VL of SEQ ID NO: 249.

28) The isolated multispecific protein of any one of embodiments 1-27, wherein the first antigen binding domain that binds CD33 is conjugated to a first immunoglobulin (Ig) constant region or a fragment of the first Ig constant region and/or the second antigen binding domain that binds the lymphocyte antigen is conjugated to a second immunoglobulin (Ig) constant region or a fragment of the second Ig constant region.
29) The isolated multispecific protein of embodiment 28, further comprising a second linker (L2) between the first antigen binding domain that binds CD33 and the first Ig constant region or the fragment of the first Ig constant region and the second antigen binding domain that binds the lymphocyte antigen and the second Ig constant region or the fragment of the second Ig constant region.
30) The isolated multispecific protein of embodiment 29 wherein the L2 comprises the amino acid sequence of any one of SEQ ID NOs: 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, or 140.
31) The isolated multispecific protein of any one of embodiments 27-29, wherein the first Ig constant region or the fragment of the first Ig constant region and the second Ig constant region or the fragment of the second Ig constant region is an IgG1, an IgG2, and IgG3 or an IgG4 isotype.
32) The isolated multispecific protein of embodiment 28-31, wherein the first Ig constant region or the fragment of the first Ig constant region and the second Ig constant region or the fragment of the second Ig constant region comprises at least one mutation that results in reduced binding of the multispecific protein to a FcyR.
33) The isolated multispecific protein of embodiment 32, wherein the at least one mutation that results in reduced binding of the multispecific protein to the FcyR is selected from the group consisting of F234A/L235A, L234A/L235A, L234A/L235A/D265S, V234A/G237A/ P238S/H268A/V309L/A330S/P331S, F234A/L235A, S228P/F234A/ L235A, N297A, V234A/G237A, K214T/E233P/ L234V/L235A/G236-deleted/A327G/P331A/D365E/L358M, H268Q/V309L/A330S/P331S, S267E/L328F, L234F/L235E/D265A, L234A/L235A/G237A/P238S/H268A/A330S/P331S, S228P/F234A/L235A/G237A/P238S and S228P/F234A/L235A/G236-deleted/G237A/P238S, wherein residue numbering is according to the EU index.
34) The isolated multispecific protein of any one of embodiments 28-31, wherein the first Ig constant region or the fragment of the first Ig constant region and the second Ig constant region or the fragment of the second Ig constant region comprises at least one mutation that results in enhanced binding of the multispecific protein to a Fcγ receptor (FcyR).
35) The isolated multispecific protein of embodiment 34, wherein the at least one mutation that results in enhanced binding of the multispecific protein to the FcγR is selected from the group consisting of S239D/I332E, S298A/E333A/K334A, F243L/R292P/Y300L, F243L/R292P/Y300L/P396L, F243L/R292P/Y300L/V305I/P396L and G236A/S239D/I332E, wherein residue numbering is according to the EU index.
36) The isolated multispecific protein of any one of embodiments 32-35, wherein the FcyR is FcyRI, FcγRIIA, FcyRIIB or FcyRIII, or any combination thereof.
37) The isolated multispecific protein of any one of embodiments 28-36, wherein the first Ig constant region or the fragment of the first Ig constant region and the second Ig constant region or the fragment of the second Ig constant region comprises at least one mutation that modulates a half-life of the multispecific protein.
38) The isolated multispecific protein of embodiment 37, wherein the at least one mutation that modulates the half-life of the multispecific protein is selected from the group consisting of H435A, P257I/N434H, D376V/N434H, M252Y/S254T/T256E/H433K/N434F, T308P/N434A and H435R, wherein residue numbering is according to the EU index.
39) The isolated multispecific protein of any one of the embodiments 28-38, comprising at least one mutation in a CH3 domain of the first Ig constant region or in a CH3 domain of the fragment of the first Ig constant region and/or at least one mutation in a CH3 domain of the second Ig constant region or in a CH3 domain of the fragment of the

second Ig constant region.

40) The isolated multispecific protein of embodiment 39, wherein the at least one mutation in a CH3 domain of the first Ig constant region or in a CH3 domain of the fragment of the first Ig constant region and/or at least one mutation in a CH3 domain of the second Ig constant region or in a CH3 domain of the fragment of the second Ig constant region is selected from the group consisting of T350V, L351Y, F405A,Y407V, T366Y, T366W, F405W, T394W, T394S, Y407T, Y407A, T366S/L368A/Y407V, L351Y/F405A/Y407V, T366I/K392M/T394W, F405A/Y407V, T366L/K392M/T394W, L351Y/Y407A, T366A/K409F, L351Y/Y407A, T366V/K409F, T366A/K409F, T350V/L351Y/F405A/Y407V and T350V/T366L/K392L/T394W, wherein residue numbering is according to the EU index.

41) The isolated multispecific protein of any one of embodiments 28-40, wherein the first Ig constant region or the fragment of the first Ig constant region and the second Ig constant region or the fragment of the second Ig constant region comprise the following mutations:

a) L235A_L235A_D265S_T350V_L351Y_F405A Y407V in the first Ig constant region and L235A_L235A_D265S _T350V_T366L_K392L_T394W in the second Ig constant region; or
b) L235A_L235A_D265S_T350V_T366L_K392L_T394W in the first Ig constant region and L235A_L235A_D265S_T350V_L351Y_F405A_Y407V in the second Ig constant region.

41) A kit comprising the isolated multispecific protein of any one of embodiments 1-41.

42) A pharmaceutical composition comprising a means for treating a CD33 expressing cancer in a subject.

43) A pharmaceutical composition comprising a means for reducing the amount of CD33 expressing tumor cells in a subject.

44) A pharmaceutical composition comprising a means for preventing establishment of a CD33 expressing cancer in a subject.

45) A pharmaceutical composition comprising a means for treating a noncancerous condition in a subject at risk of developing a CD33 expressing cancer.

List of numbered embodiments (C):

1) An immunoconjugate comprising an isolated protein conjugated to an agent, wherein the isolated protein binds CD33, wherein the isolated protein comprises

a) an HCDR1, an HCDR2 and an HCDR3 of a heavy chain variable region (VH) of SEQ ID NO: 18;
b) an HCDR1, an HCDR2 and an HCDR3 of a VH of SEQ ID NO: 19;
c) an HCDR1, an HCDR2 and an HCDR3 of a VH of SEQ ID NO: 20;
d) an HCDR1, an HCDR2 and an HCDR3 of a VH of SEQ ID NO: 21;
e) an HCDR1, an HCDR2 and an HCDR3 of a VH of SEQ ID NO: 22;
f) an HCDR1, an HCDR2 and an HCDR3 of a VH of SEQ ID NO: 23;
g) an HCDR1, an HCDR2 and an HCDR3 of a VH of SEQ ID NO: 24;
h) an HCDR1, an HCDR2 and an HCDR3 of a VH of SEQ ID NO: 25;
i) an HCDR1, an HCDR2 and an HCDR3 of a VH of SEQ ID NO: 26; or
j) an HCDR1, an HCDR2 and an HCDR3 of a VH of SEQ ID NO: 27,

or a CD33 binding fragment thereof.

2) The immunoconjugate of embodiment 1, wherein the isolated protein comprises the HCDR1, the HCDR2, the HCDR3 of

a) SEQ ID NOs: 1, 6, and 12, respectively;
b) SEQ ID NOs: 2, 7, and 13, respectively;
c) SEQ ID NOs: 1, 8, and 14, respectively;
d) SEQ ID NOs: 3, 9, and 13, respectively;
e) SEQ ID NOs: 4, 10, and 15, respectively;
f) SEQ ID NOs: 5, 11, and 16, respectively; or
g) SEQ ID NOs: 5, 11, and 17, respectively.

3) The immunoconjugate of embodiments 1 or 2, wherein the antigen binding domain that binds CD33 is a scFv, a

$(scFv)_2$, a Fv, a Fab, a $F(ab')_2$, a Fd, a dAb or a VHH.
4) The immunoconjugate of embodiment 3, wherein the antigen binding domain that binds CD33 is the VHH.
5) The immunoconjugate of embodiment 3, wherein the antigen binding domain that binds CD33 is the Fab.
6) The immunoconjugate of embodiment 3, wherein the antigen binding domain that binds CD33 is the scFv.
7) The immunoconjugate of any of embodiments 1-6, wherein the antigen binding domain that binds CD33 comprises the VH of any one of SEQ ID NOs: 18, 19, 20, 21, 22, 23, 24, 25, 26, or 27.
8) An immunoconjugate comprising an isolated protein conjugated to an agent, wherein the isolated protein binds CD33, wherein the isolated protein comprises a VHH of any one of SEQ ID NOs: 18, 19, 20, 21, 22, 23, 24, 25, 26, or 27.
9) An immunoconjugate comprising an isolated protein conjugated to an agent, wherein the isolated protein binds CD33, and wherein the isolated protein comprises

a) an HCDR1, an HCDR2 and an HCDR3 of a heavy chain variable region (VH) of SEQ ID NO: 52;
b) an HCDR1, an HCDR2 and an HCDR3 of a VH of SEQ ID NO: 53;
c) an HCDR1, an HCDR2 and an HCDR3 of a VH of SEQ ID NO: 54;
d) an HCDR1, an HCDR2 and an HCDR3 of a VH of SEQ ID NO: 55;
e) an HCDR1, an HCDR2 and an HCDR3 of a VH of SEQ ID NO: 56;
f) an HCDR1, an HCDR2 and an HCDR3 of a VH of SEQ ID NO: 57;
g) an HCDR1, an HCDR2 and an HCDR3 of a VH of SEQ ID NO: 58;
h) an HCDR1, an HCDR2 and an HCDR3 of a VH of SEQ ID NO: 59;
i) an HCDR1, an HCDR2 and an HCDR3 of a VH of SEQ ID NO: 60; or
j) an HCDR1, an HCDR2 and an HCDR3 of a VH of SEQ ID NO: 61.

10) The immunoconjugate of embodiment 9, wherein the isolated protein comprises the HCDR1, the HCDR2, and the HCDR3 of

a) SEQ ID NOs: 28, 36, and 45, respectively;
b) SEQ ID NOs: 29, 37, and 46, respectively;
c) SEQ ID NOs: 30, 38, and 47, respectively;
d) SEQ ID NOs: 31, 39, and 48, respectively;
e) SEQ ID NOs: 32, 40, and 49, respectively;
f) SEQ ID NOs: 33, 41, and 50, respectively;
g) SEQ ID NOs: 34, 42, and 51, respectively;
h) SEQ ID NOs: 34, 43, and 51, respectively;
i) SEQ ID NOs: 34, 44, and 51, respectively; or
j) SEQ ID NOs: 35, 42, and 51, respectively.

11) The immunoconjugate of embodiment 9 or 10, wherein the isolated protein comprises

a) an HCDR1, an HCDR2 and an HCDR3 of a heavy chain variable region (VH) of SEQ ID NO: 52 and a light chain complementarity determining region (LCDR) 1, an LCDR2 and an LCDR3 of a light chain variable region (VL) of SEQ ID NO: 81;
b) an HCDR1, an HCDR2 and an HCDR3 of a VH of SEQ ID NO: 53 and an LCDR1, an LCDR2 and an LCDR3 of a VL of SEQ ID NO: 82;
c) an HCDR1, an HCDR2 and an HCDR3 of a VH of SEQ ID NO: 54 and an LCDR1, an LCDR2 and an LCDR3 of a VL of SEQ ID NO: 83;
d) an HCDR1, an HCDR2 and an HCDR3 of a VH of SEQ ID NO: 55 and an LCDR1, an LCDR2 and an LCDR3 of a VL of SEQ ID NO: 84;
e) an HCDR1, an HCDR2 and an HCDR3 of a VH of SEQ ID NO: 56 and an LCDR1, an LCDR2 and an LCDR3 of a VL of SEQ ID NO: 85;
f) an HCDR1, an HCDR2 and an HCDR3 of a VH of SEQ ID NO: 57 and an LCDR1, an LCDR2 and an LCDR3 of a VL of SEQ ID NO: 86;
g) an HCDR1, an HCDR2 and an HCDR3 of a VH of SEQ ID NO: 58 and an LCDR1, an LCDR2 and an LCDR3 of a VL of SEQ ID NO: 87;
h) an HCDR1, an HCDR2 and an HCDR3 of a VH of SEQ ID NO: 59 and an LCDR1, an LCDR2 and an LCDR3 of a VL of SEQ ID NO: 87;
i) an HCDR1, an HCDR2 and an HCDR3 of a VH of SEQ ID NO: 60 and an LCDR1, an LCDR2 and an LCDR3 of a VL of SEQ ID NO: 87; or
j) an HCDR1, an HCDR2 and an HCDR3 of a VH of SEQ ID NO: 61 and an LCDR1, an LCDR2 and an LCDR3

of a VL of SEQ ID NO: 88.

12) The immunoconjugate of embodiment 11, wherein the isolated protein comprises the HCDR1, the HCDR2, the HCDR3, the LCDR1, the LCDR2 and the LCDR3 of

a) SEQ ID NOs: 28, 36, 45, 62, 69, and 75, respectively;
b) SEQ ID NOs: 29, 37, 46, 63, 70, and 76, respectively;
c) SEQ ID NOs: 30, 38, 47, 64, 71, and 77, respectively;
d) SEQ ID NOs: 31, 39, 48, 65, 72, and 78, respectively;
e) SEQ ID NOs: 32, 40, 49, 66, 70, and 76, respectively;
f) SEQ ID NOs: 33, 41, 50, 67, 73, and 79, respectively;
g) SEQ ID NOs: 34, 42, 51, 68, 74, and 80, respectively;
h) SEQ ID NOs: 34, 43, 51, 68, 74, and 80, respectively;
i) SEQ ID NOs: 34, 44, 51, 68, 74, and 80, respectively; or
j) SEQ ID NOs: 35, 42, 51, 68, 74, and 80, respectively.

13) The immunoconjugate of any one of embodiments 9-12, wherein the isolated protein is a scFv, a $(scFv)_2$, a Fv, a Fab, a $F(ab')_2$, a Fd, a dAb or a VHH.
14) The immunoconjugate of embodiment 13, wherein the isolated protein is the Fab.
15) The immunoconjugate of embodiment 13, wherein the isolated protein is the VHH.
16) The immunoconjugate of embodiment 13, wherein the isolated protein is the scFv.
17) The immunoconjugate of embodiment 16, wherein the scFv comprises, from the N- to C-terminus, a VH, a first linker (L1) and a VL (VH-L1-VL) or the VL, the L1 and the VH (VL-L1-VH).
18) The immunoconjugate of embodiment 17, wherein the L1 comprises

a) about 5-50 amino acids;
b) about 5-40 amino acids;
c) about 10-30 amino acids; or
d) about 10-20 amino acids.

19) The immunoconjugate of embodiment 18, wherein the L1 comprises an amino acid sequence of any one of SEQ ID NOs: 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, or 140.
20) The immunoconjugate of embodiment 19, wherein the L1 comprises the amino acid sequence of SEQ ID NO: 108.
21) The immunoconjugate of any one of embodiments 9-20, wherein the isolated protein comprises the VH of SEQ ID NOs: 52, 53, 54, 55, 56, 57, 58, 59, 60, or 61, and the VL of SEQ ID NOs: 81, 82, 83, 84, 85, 86, 87, or 88.
22) The immunoconjugate of embodiment 21, wherein the isolated protein comprises:

a) the VH of SEQ ID NO: 52 and the VL of SEQ ID NO: 81;
b) the VH of SEQ ID NO: 53 and the VL of SEQ ID NO: 82;
c) the VH of SEQ ID NO: 54 and the VL of SEQ ID NO: 83;
d) the VH of SEQ ID NO: 55 and the VL of SEQ ID NO: 84;
e) the VH of SEQ ID NO: 56 and the VL of SEQ ID NO: 85;
f) the VH of SEQ ID NO: 57 and the VL of SEQ ID NO: 86;
g) the VH of SEQ ID NO: 58 and the VL of SEQ ID NO: 87;
h) the VH of SEQ ID NO: 59 and the VL of SEQ ID NO: 87;
i) the VH of SEQ ID NO: 60 and the VL of SEQ ID NO: 87; or
j) the VH of SEQ ID NO: 61 and the VL of SEQ ID NO: 88.

23) An immunoconjugate comprising an isolated protein conjugated to an agent, wherein the isolated protein binds CD33, and wherein the isolated protein comprises

a) the VH of SEQ ID NO: 52 and the VL of SEQ ID NO: 81;
b) the VH of SEQ ID NO: 53 and the VL of SEQ ID NO: 82;
c) the VH of SEQ ID NO: 54 and the VL of SEQ ID NO: 83;
d) the VH of SEQ ID NO: 55 and the VL of SEQ ID NO: 84;
e) the VH of SEQ ID NO: 56 and the VL of SEQ ID NO: 85;
f) the VH of SEQ ID NO: 57 and the VL of SEQ ID NO: 86;

g) the VH of SEQ ID NO: 58 and the VL of SEQ ID NO: 87;
h) the VH of SEQ ID NO: 59 and the VL of SEQ ID NO: 87;
i) the VH of SEQ ID NO: 60 and the VL of SEQ ID NO: 87; or
j) the VH of SEQ ID NO: 61 and the VL of SEQ ID NO: 88.

24) The immunoconjugate of embodiment 23, wherein the isolated protein is a scFv, a $(scFv)_2$, a Fv, a Fab, a $F(ab')_2$, a Fd, a dAb or a VHH.
25) The immunoconjugate of embodiment 24, wherein the isolated protein is the Fab.
26) The immunoconjugate of embodiment 24, wherein the isolated protein is the VHH.
27) The immunoconjugate of embodiment 24, wherein the isolated protein is the scFv.
28) The immunoconjugate of embodiment 27, wherein the scFv comprises, from the N- to C-terminus, a VH, a first linker (L1) and a VL (VH-L1-VL) or the VL, the L1 and the VH (VL-L1-VH).
29) The immunoconjugate of embodiment 28, wherein the L1 comprises

a) about 5-50 amino acids;
b) about 5-40 amino acids;
c) about 10-30 amino acids; or
d) about 10-20 amino acids.

30) The immunoconjugate of embodiment 29, wherein the L1 comprises an amino acid sequence of any one of SEQ ID NOs: 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, or 140.
31) The immunoconjugate of embodiment 30, wherein the L1 comprises the amino acid sequence of SEQ ID NO: 108.
32) An immunoconjugate comprising an isolated protein conjugated to an agent, wherein the isolated protein binds CD33, and wherein the isolated protein comprises

a) an HCDR1, an HCDR2 and an HCDR3 of a heavy chain variable region (VH) of SEQ ID NO: 95;
b) an HCDR1, an HCDR2 and an HCDR3 of a VH of SEQ ID NO: 96;
c) an HCDR1, an HCDR2 and an HCDR3 of a VH of SEQ ID NO: 97;
d) an HCDR1, an HCDR2 and an HCDR3 of a VH of SEQ ID NO: 170;
e) an HCDR1, an HCDR2 and an HCDR3 of a VH of SEQ ID NO: 171;
f) an HCDR1, an HCDR2 and an HCDR3 of a VH of SEQ ID NO: 175;
g) an HCDR1, an HCDR2 and an HCDR3 of a VH of SEQ ID NO: 179;
h) an HCDR1, an HCDR2 and an HCDR3 of a VH of SEQ ID NO: 181;
i) an HCDR1, an HCDR2 and an HCDR3 of a VH of SEQ ID NO: 96.

33) The immunoconjugate of embodiment 32, comprising the HCDR1, the HCDR2, and the HCDR3 of

a) SEQ ID NOs: 89, 90, and 92, respectively;
b) SEQ ID NOs: 89, 90, and 93, respectively;
c) SEQ ID NOs: 33, 91, and 94, respectively
d) SEQ ID NOs: 167, 168, and 169, respectively;
e) SEQ ID NOs: 172, 173, and 174, respectively;
f) SEQ ID NOs: 176, 177, and 178, respectively;
g) SEQ ID NOs: 89, 90, and 180, respectively; or
h) SEQ ID NOs: 89, 90, and 93, respectively.

34) The immunoconjugate of embodiment 32 or 33, wherein the isolated protein comprises

a) an HCDR1, an HCDR2 and an HCDR3 of a heavy chain variable region (VH) of SEQ ID NO: 95 and a light chain complementarity determining region (LCDR) 1, an LCDR2 and an LCDR3 of a light chain variable region (VL) of SEQ ID NO: 105;
b) an HCDR1, an HCDR2 and an HCDR3 of a VH of SEQ ID NO: 96 and an LCDR1, an LCDR2 and an LCDR3 of a VL of SEQ ID NO: 106;
c) an HCDR1, an HCDR2 and an HCDR3 of a VH of SEQ ID NO: 97 and an LCDR1, an LCDR2 and an LCDR3 of a VL of SEQ ID NO: 107;
d) an HCDR1, an HCDR2 and an HCDR3 of a VH of SEQ ID NO: 170 and an LCDR1, an LCDR2 and an LCDR3 of a VL of SEQ ID NO: 185;

e) an HCDR1, an HCDR2 and an HCDR3 of a VH of SEQ ID NO: 171 and an LCDR1, an LCDR2 and an LCDR3 of a VL of SEQ ID NO: 188;
f) an HCDR1, an HCDR2 and an HCDR3 of a VH of SEQ ID NO: 175 and an LCDR1, an LCDR2 and an LCDR3 of a VL of SEQ ID NO: 192;
g) an HCDR1, an HCDR2 and an HCDR3 of a VH of SEQ ID NO: 179 and an LCDR1, an LCDR2 and an LCDR3 of a VL of SEQ ID NO: 196;
h) an HCDR1, an HCDR2 and an HCDR3 of a VH of SEQ ID NO: 181 and an LCDR1, an LCDR2 and an LCDR3 of a VL of SEQ ID NO: 200; or
i) an HCDR1, an HCDR2 and an HCDR3 of a VH of SEQ ID NO: 96 and an LCDR1, an LCDR2 and an LCDR3 of a VL of SEQ ID NO: 202.

35) The immunoconjugate of embodiment 34, comprising the HCDR1, the HCDR2, the HCDR3, the LCDR1, the LCDR2 and the LCDR3 of

a) SEQ ID NOs: 89, 90, 92, 98, 101, and 104, respectively;
b) SEQ ID NOs: 89, 90, 93, 99, 102, and 104, respectively;
c) SEQ ID NOs: 33, 91, 94, 100, 103, and 104, respectively;
d) SEQ ID NOs: 167, 168, 169, 182, 183, and 184, respectively;
e) SEQ ID NOs: 33, 91, 94, 186, 187, and 104, respectively;
f) SEQ ID NOs: 172, 173, 174, 189, 190, and 191, respectively;
g) SEQ ID NOs: 176, 177, 178, 193, 194, and 195, respectively;
h) SEQ ID NOs: 89, 90, 180, 197, 198, and 199, respectively;
i) SEQ ID NOs: 89, 90, 93, 201, 102, and 104, respectively.

36) The immunoconjugate of any one of embodiments 32-35, wherein the isolated protein is a scFv, a (scFv)z, a Fv, a Fab, a $F(ab')_2$, a Fd, a dAb or a VHH.
37) The immunoconjugate of embodiment 36, wherein the isolated protein is the Fab.
38) The immunoconjugate of embodiment 36, wherein the isolated protein is the VHH.
39) The immunoconjugate of embodiment 36, wherein the isolated protein is the scFv.
40) The immunoconjugate of embodiment 39, wherein the scFv comprises, from the N- to C-terminus, a VH, a first linker (L1) and a VL (VH-L1-VL) or the VL, the L1 and the VH (VL-L1-VH).
41) The immunoconjugate of embodiment 40, wherein the L1 comprises

a) about 5-50 amino acids;
b) about 5-40 amino acids;
c) about 10-30 amino acids; or
d) about 10-20 amino acids.

42) The immunoconjugate of embodiment 41, wherein the L1 comprises an amino acid sequence of any one of SEQ ID NOs: 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, or 140.
43) The immunoconjugate of embodiment 42, wherein the L1 comprises the amino acid sequence of SEQ ID NO: 108.
44) The immunoconjugate of any one of embodiments 32-43, wherein the isolated protein comprises the VH of SEQ ID NOs: 95, 96, 97, 170, 171, 175, 179, 181, or 96, and the VL of SEQ ID NOs: 105, 106, 107, 185, 188, 192, 196, 200, or 202.
45) The immunoconjugate of embodiment 44, wherein the isolated protein comprises:

a) the VH of SEQ ID NO: 95 and the VL of SEQ ID NO: 105;
b) the VH of SEQ ID NO: 96 and the VL of SEQ ID NO: 106;
c) the VH of SEQ ID NO: 97 and the VL of SEQ ID NO: 107;
d) the VH of SEQ ID NO: 170 and the VL of SEQ ID NO: 185;
e) the VH of SEQ ID NO: 171 and the VL of SEQ ID NO: 188;
f) the VH of SEQ ID NO: 175 and the VL of SEQ ID NO: 192;
g) the VH of SEQ ID NO: 179 and the VL of SEQ ID NO: 196;
h) the VH of SEQ ID NO: 181 and the VL of SEQ ID NO: 200; or
i) the VH of SEQ ID NO: 96 and the VL of SEQ ID NO: 202.

46) An immunoconjugate comprising an isolated protein conjugated to an agent, wherein the isolated protein binds

CD33, and wherein the isolated protein comprises

a) the VH of SEQ ID NO: 95 and the VL of SEQ ID NO: 105;
b) the VH of SEQ ID NO: 96 and the VL of SEQ ID NO: 106;
c) the VH of SEQ ID NO: 97 and the VL of SEQ ID NO: 107;
d) the VH of SEQ ID NO: 170 and the VL of SEQ ID NO: 185;
e) the VH of SEQ ID NO: 171 and the VL of SEQ ID NO: 188;
f) the VH of SEQ ID NO: 175 and the VL of SEQ ID NO: 192;
g) the VH of SEQ ID NO: 179 and the VL of SEQ ID NO: 196;
h) the VH of SEQ ID NO: 181 and the VL of SEQ ID NO: 200; or
i) the VH of SEQ ID NO: 96 and the VL of SEQ ID NO: 202.

47) The immunoconjugate of embodiment 46, wherein the isolated protein is a scFv, a $(scFv)_2$, a Fv, a Fab, a $F(ab')_2$, a Fd, a dAb or a VHH.
48) The immunoconjugate of embodiment 47, wherein the isolated protein is the Fab.
49) The immunoconjugate of embodiment 47, wherein the isolated protein is the VHH.
50) The immunoconjugate of embodiment 47, wherein the isolated protein is the scFv.
51) The immunoconjugate of embodiment 50, wherein the scFv comprises, from the N- to C-terminus, a VH, a first linker (L1) and a VL (VH-L1-VL) or the VL, the L1 and the VH (VL-L1-VH).
52) The immunoconjugate of embodiment 51, wherein the L1 comprises

a) about 5-50 amino acids;
b) about 5-40 amino acids;
c) about 10-30 amino acids; or
d) about 10-20 amino acids.

53) The immunoconjugate of embodiment 52, wherein the L1 comprises an amino acid sequence of any one of SEQ ID NOs: 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, or 140.
54) The immunoconjugate of embodiment 53, wherein the L1 comprises the amino acid sequence of SEQ ID NO: 108.
55) The immunoconjugate of any one of embodiments 1-54, wherein the isolated protein is conjugated to a half-life extending moiety.
56) The immunoconjugate of embodiment 55, wherein the half-life extending moiety is an immunoglobulin (Ig), a fragment of the Ig, an Ig constant region, a fragment of the Ig constant region, a Fc region, transferrin, albumin, an albumin binding domain or polyethylene glycol.
57) The immunoconjugate of any one of embodiments 1-56, wherein the isolated protein is a monospecific protein.
58) The immunoconjugate of any one of embodiments 1-57, wherein the isolated protein is a multispecific protein.
59) The immunoconjugate of embodiment 58, wherein the multispecific protein is a bispecific protein.
60) The immunoconjugate of embodiment 59, wherein the multispecific protein is a trispecific protein.
61) The immunoconjugate of any one of embodiments 1-60, further comprising an immunoglobulin (Ig) constant region or a fragment of the Ig constant region thereof.
62) The immunoconjugate of embodiment 61, wherein the fragment of the Ig constant region comprises a Fc region.
63) The immunoconjugate of embodiment 61, wherein the fragment of the Ig constant region comprises a CH2 domain.
64) The immunoconjugate of embodiment 61, wherein the fragment of the Ig constant region comprises a CH3 domain.
65) The immunoconjugate of embodiment 61, wherein the fragment of the Ig constant region comprises the CH2 domain and the CH3 domain.
66) The immunoconjugate of embodiment 61, wherein the fragment of the Ig constant region comprises at least portion of a hinge, the CH2 domain and the CH3 domain.
67) The immunoconjugate of embodiment 61, wherein the fragment of the Ig constant region comprises a hinge, the CH2 domain and the CH3 domain.
68) The immunoconjugate of any one of embodiments 61-67, wherein the isolated protein is conjugated to the N-terminus of the Ig constant region or the fragment of the Ig constant region.
69) The immunoconjugate of any one of embodiments 61-67, wherein the isolated protein is conjugated to the C-terminus of the Ig constant region or the fragment of the Ig constant region.
70) The immunoconjugate of any one of embodiments 61-69, wherein the isolated protein is conjugated to the Ig constant region or the fragment of the Ig constant region via a second linker (L2).

71) The immunoconjugate of embodiment 70, wherein the L2 comprises the amino acid sequence of any one of SEQ ID NOs:108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, or 140.
72) The immunoconjugate of any one of embodiments 58-71, wherein the multispecific protein comprises an antigen binding domain that binds an antigen on a lymphocyte.
73) The immunoconjugate of embodiment 72, wherein the lymphocyte is a T cell.
74) The immunoconjugate of embodiment 72, wherein the T cell is a $CD8^+$ T cell
75) The immunoconjugate of embodiment 72, wherein the lymphocyte is a natural killer (NK) cell.
76) The immunoconjugate of any one of embodiments 58-75, wherein the multispecific protein comprises an antigen binding domain that binds CD3, CD3 epsilon (CD3ε), CD8, KI2L4, NKG2E, NKG2D, NKG2F, BTNL3, CD186, BTNL8, PD-1, CD195, TRGV9, or NKG2C.
77) The immunoconjugate of embodiment 76, wherein the multispecific protein comprises an antigen binding domain that binds CD3ε.
78) The immunoconjugate of embodiment 77, wherein the antigen binding domain that binds CD3ε comprises:

a) a heavy chain complementarity determining region 1 (HCDR1) of SEQ ID NO: 141, a HCDR2 of SEQ ID NO: 142, a HCDR3 of SEQ ID NO: 143, a light chain complementarity determining region 1 (LCDR1) of SEQ ID NO: 144, a LCDR2 of SEQ ID NO: 145 and a LCDR3 of SEQ ID NO: 146;
b) the VH of SEQ ID NO: 147 and the VL of SEQ ID NO: 148;
c) the HCDR1 of SEQ ID NO: 149, the HCDR2 of SEQ ID NO: 150, the HCDR3 of SEQ ID NO: 151, the LCDR1 of SEQ ID NO: 152, the LCDR2 of SEQ ID NO: 153 and the LCDR3 of SEQ ID NO: 154; or
d) the VH of SEQ ID NO: 155 and the VL of SEQ ID NO: 156.

79) The immunoconjugate of embodiment 76, wherein the multispecific protein comprises an antigen binding domain that binds TRGV9.
80) The immunoconjugate of embodiment 77, wherein the antigen binding domain that binds TRGV9 comprises:

a) a heavy chain complementarity determining region 1 (HCDR1) of SEQ ID NO: 236, a HCDR2 of SEQ ID NO: 237, a HCDR3 of SEQ ID NO: 238, a light chain complementarity determining region 1 (LCDR1) of SEQ ID NO: 239, a LCDR2 of SEQ ID NO: 240 and a LCDR3 of SEQ ID NO: 241;
b) a heavy chain complementarity determining region 1 (HCDR1) of SEQ ID NO: 236, a HCDR2 of SEQ ID NO: 237, a HCDR3 of SEQ ID NO: 242, a light chain complementarity determining region 1 (LCDR1) of SEQ ID NO: 239, a LCDR2 of SEQ ID NO: 240 and a LCDR3 of SEQ ID NO: 241;
c) a heavy chain complementarity determining region 1 (HCDR1) of SEQ ID NO: 236, a HCDR2 of SEQ ID NO: 237, a HCDR3 of SEQ ID NO: 243, a light chain complementarity determining region 1 (LCDR1) of SEQ ID NO: 239, a LCDR2 of SEQ ID NO: 240 and a LCDR3 of SEQ ID NO: 241;
d) a heavy chain complementarity determining region 1 (HCDR1) of SEQ ID NO: 236, a HCDR2 of SEQ ID NO: 237, a HCDR3 of SEQ ID NO: 244, a light chain complementarity determining region 1 (LCDR1) of SEQ ID NO: 239, a LCDR2 of SEQ ID NO: 240 and a LCDR3 of SEQ ID NO: 241;
e) the VH of SEQ ID NO: 245 and the VL of SEQ ID NO: 249;
f) the VH of SEQ ID NO: 246 and the VL of SEQ ID NO: 249;
g) the VH of SEQ ID NO: 247 and the VL of SEQ ID NO: 249; or
h) the VH of SEQ ID NO: 248 and the VL of SEQ ID NO: 249.

81) The immunoconjugate of any one of embodiments 61-80, wherein the Ig constant region or the fragment of the Ig constant region is an IgG1, an IgG2, an IgG3 or an IgG4 isotype.
82) The immunoconjugate of any one of embodiments 61-81, wherein the Ig constant region or the fragment of the Ig constant region comprises at least one mutation that results in reduced binding of the protein to a Fcγ receptor (FcγR).
83) The immunoconjugate of embodiment 82, wherein the at least one mutation that results in reduced binding of the protein to the FcγR is selected from the group consisting of F234A/L235A, L234A/L235A, L234A/L235A/D265S, V234A/G237A/ P238S/H268A/V309L/A330S/P331S, F234A/L235A, S228P/F234A/ L235A, N297A, V234A/G237A, K214T/E233P/ L234V/L235A/G236-deleted/A327G/P331A/D365E/L358M, H268Q/V309L/A330S/P331S, S267E/L328F, L234F/L235E/D265A, L234A/L235A/G237A/P238S/H268A/A330S/P331S, S228P/F234A/L235A/G237A/P238S and S228P/F234A/L235A/G236-deleted/G237A/P238S, wherein residue numbering is according to the EU index.
84) The immunoconjugate of any one of embodiments 61-81, wherein the Ig constant region or the fragment of the Ig constant region comprises at least one mutation that results in enhanced binding of the protein to the FcγR.

85) The immunoconjugate of embodiment 84, wherein the at least one mutation that results in enhanced binding of the protein to the FcyR is selected from the group consisting of S239D/I332E, S298A/E333A/K334A, F243L/R292P/Y300L, F243L/R292P/Y300L/P396L, F243L/R292P/Y300L/V3051/P396L and G236A/S239D/I332E, wherein residue numbering is according to the EU index.

86) The immunoconjugate of any one of embodiments 80-85, wherein the FcyR is FcyRI, FcyRIIA, FcyRIIB or FcγRIII, or any combination thereof.

87) The immunoconjugate of any one of embodiments 61-86, wherein the Ig constant region of the fragment of the Ig constant region comprises at least one mutation that modulates a half-life of the protein.

88) The immunoconjugate of embodiment 87, wherein the at least one mutation that modulates the half-life of the protein is selected from the group consisting of H435A, P257I/N434H, D376V/N434H, M252Y/S254T/T256E/H433K/N434F, T308P/N434A and H435R, wherein residue numbering is according to the EU index.

89) The immunoconjugate of any one of the embodiments 61-88, wherein the isolated protein comprises at least one mutation in a CH3 domain of the Ig constant region.

90) The immunoconjugate of embodiment 89, wherein the at least one mutation in the CH3 domain of the Ig constant region is selected from the group consisting of T350V, L351Y, F405A, Y407V, T366Y, T366W, F405W, T394W, T394S, Y407T, Y407A, T366S/L368A/Y407V, L351Y/F405A/Y407V, T366I/K392M/T394W, F405A/Y407V, T366L/K392M/T394W, L351Y/Y407A, T366A/K409F, L351Y/Y407A, T366V/K409F, T366A/K409F, T350V/L351Y/F405A/Y407V and T350V/T366L/K392L/T394W, wherein residue numbering is according to the EU index.

91) The immunoconjugate of any one of embodiments 1-54, wherein the isolated protein is a chimeric antigen receptor (CAR).

92) The immunoconjugate of embodiment 91, wherein the CAR comprises

a) an extracellular domain comprising the antigen binding domain that binds CD33 as defined in any one of embodiments 1-54;
b) a transmembrane domain; and
c) an intracellular signaling domain optionally comprising at least one co-stimulatory domain.

93) The immunoconjugate of embodiment 91 or 92, wherein the CAR further comprises a CD8a-hinge region.

94) The immunoconjugate of any one of embodiments 91-93, wherein

a) the transmembrane domain comprises a CD8a transmembrane region (CD8a-TM) polypeptide; and
b) the intracellular signaling domain comprises a co-stimulatory domain comprising a TNF receptor superfamily member 9 (CD137) component and a primary signaling domain comprising a T-cell surface glycoprotein CD3 zeta chain (CD3z) component.

95) The immunoconjugate of any one of embodiments 91-93, wherein

a) the CD8a-hinge region comprises an amino acid sequence that is at least 90% identical to the amino acid sequence of SEQ ID NO: 157;
b) the transmembrane domain comprises an amino acid sequence that is at least 90% identical to the amino acid sequence of SEQ ID NO: 162; and/or
c) the intracellular signaling domain comprises a co-stimulatory domain having an amino acid sequence that is at least 90% identical to the amino acid sequence of SEQ ID NO: 163 and a primary signaling domain having an amino acid sequence that is at least 90% identical to the amino acid sequence of SEQ ID NO: 164.

96) The immunoconjugate of embodiment 95, wherein the intracellular signaling domain comprises a polypeptide component selected from the group consisting of a TNF receptor superfamily member 9 (CD137) component, a T-cell surface glycoprotein CD3 zeta chain (CD3z) component, a cluster of differentiation (CD27) component, a cluster of differentiation superfamily member component, or any combinations thereof.

97) The immunoconjugate of any one of embodiments 1-96, wherein the agent is a therapeutic agent or an imaging agent.

98) A pharmaceutical composition comprising the immunoconjugate of any one of embodiments 1-96 and a pharmaceutically acceptable carrier.

99) A polynucleotide encoding the isolated protein of the immunoconjugate of any one of embodiments 1-96.

100) A vector comprising the polynucleotide of embodiment 99.

101) A host cell comprising the vector of embodiment 100.

102) A method of producing the immunoconjugate of any one of embodiments 1-96, comprising culturing the host cell of embodiment 101 in conditions that the protein is expressed, recovering the protein produced by the host cell, and conjugating the protein produced by the host cell with the agent.

103) An immunoconjugate comprising an isolated multispecific protein conjugated to an agent, wherein the isolated multispecific protein comprises a first antigen binding domain that binds CD33 and a second antigen binding domain that binds a lymphocyte antigen.

104) The immunoconjugate of embodiment 103, wherein the lymphocyte antigen is a T cell antigen.

105) The immunoconjugate protein of embodiment 103, wherein the T cell antigen is a $CD8^+$ T cell antigen.

106) The immunoconjugate of embodiment 103, wherein the lymphocyte antigen is a NK cell antigen.

107) The immunoconjugate of any one of embodiments 103-106, wherein the lymphocyte antigen is CD3, CD3 epsilon (CD3ε), CD8, KI2L4, NKG2E, NKG2D, NKG2F, BTNL3, CD186, BTNL8, PD-1, CD195, TRGV9, orNKG2C.

108) The immunoconjugate of embodiment 107, wherein the lymphocyte antigen is CD3ε.

109) The immunoconjugate of any one of embodiments 103-108, wherein the first antigen binding domain that binds CD33 and/or the second antigen binding domain that binds the lymphocyte antigen comprise a scFv, a $(scFv)_2$, a Fv, a Fab, a $F(ab')_2$, a Fd, a dAb or a VHH.

110) The immunoconjugate of embodiment 109, wherein the first antigen binding domain that binds CD33 and/or the second antigen binding domain that binds the lymphocyte antigen comprise the Fab.

111) The immunoconjugate of embodiment 109, wherein the first antigen binding domain that binds CD33 and/or the second antigen binding domain that binds the lymphocyte antigen comprise the VHH.

112) The immunoconjugate of embodiment 110, wherein the first antigen binding domain that binds CD33 and/or the second antigen binding domain that binds the lymphocyte antigen comprise the scFv.

113) The immunoconjugate of embodiment 112, wherein the scFv comprises, from the N- to C-terminus, a VH, a first linker (L1) and a VL (VH-L1-VL) or the VL, the L1 and the VH (VL-L1-VH).

114) The immunoconjugate of embodiment 113, wherein the L1 comprises

a) about 5-50 amino acids;
b) about 5-40 amino acids;
c) about 10-30 amino acids; or
d) about 10-20 amino acids.

115) The immunoconjugate of embodiment 114, wherein the L1 comprises the amino acid sequence of any one of SEQ ID NOs: 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, or 140.

116) The immunoconjugate of embodiment 115, wherein the L1 comprises the amino acid sequence of SEQ ID NO: 108.

117) The immunoconjugate of any one of embodiments 103-116, wherein the first antigen binding domain that binds CD33 comprises the HCDR1 of any one of SEQ ID NOs: 1, 2, 3, 4, or 5, the HCDR2 of any one of SEQ ID NOs: 6, 7, 8, 9, 10, or 11, and the HCDR3 of any one of SEQ ID NOs: 12, 13, 14, 15, 16, or 17.

118) The immunoconjugate of any one of 103-117, wherein the first antigen binding domain that binds CD33 comprises the HCDR1, the HCDR1, the HCDR3 of

a) SEQ ID NOs: 1, 6, and 12, respectively;
b) SEQ ID NOs: 2, 7, and 13, respectively;
c) SEQ ID NOs: 1, 8, and 14, respectively;
d) SEQ ID NOs: 3, 9, and 13, respectively;
e) SEQ ID NOs: 4, 10, and 15, respectively;
f) SEQ ID NOs: 5, 11, and 16, respectively; or
g) SEQ ID NOs: 5, 11, and 17, respectively.

119) The immunoconjugate of any one of embodiments 103-118, wherein the first antigen binding domain that binds CD33 comprises the VH of any one of SEQ ID NOs: 18, 19, 20, 21, 22, 23, 24, 25, 26, or 27.

120) The immunoconjugate of any one of embodiments 103-116, wherein the first antigen binding domain that binds CD33 comprises the HCDR1 of SEQ ID NOs: 28, 29, 30, 31, 32, 33, 34, or 35, the HCDR2 of SEQ ID NOs: 36, 37, 38, 39, 40, 41, 42, 43, or 44, the HCDR3 of SEQ ID NOs: 45, 46, 47, 48, 49, 50, or 51, the LCDR1 of SEQ ID NOs: 62, 63, 64, 65, 66, 67, or 68, the LCDR2 of SEQ ID NOs: 69, 70, 71, 72, 73, or 74, and the LCDR3 of SEQ ID NOs: 75, 76, 77, 78, 79, or 80.

121) The immunoconjugate of any one of embodiments 103-116 and 120, wherein the first antigen binding domain

that binds CD33 comprises the HCDR1, the HCDR2, the HCDR3, the LCDR1, the LCDR2 and the LCDR3 of

a) SEQ ID NOs: 28, 36, 45, 62, 69, and 75, respectively;
b) SEQ ID NOs: 29, 37, 46, 63, 70, and 76, respectively;
c) SEQ ID NOs: 30, 38, 47, 64, 71, and 77, respectively;
d) SEQ ID NOs: 31, 39, 48, 65, 72, and 78, respectively;
e) SEQ ID NOs: 32, 40, 49, 66, 70, and 76, respectively;
f) SEQ ID NOs: 33, 41, 50, 67, 73, and 79, respectively;
g) SEQ ID NOs: 34, 42, 51, 68, 74, and 80, respectively;
h) SEQ ID NOs: 34, 43, 51, 68, 74, and 80, respectively;
i) SEQ ID NOs: 34, 44, 51, 68, 74, and 80, respectively; or
j) SEQ ID NOs: 35, 42, 51, 68, 74, and 80, respectively.

122) The immunoconjugate of any one of embodiments 103-116, 120, and 121, wherein the antigen binding domain that binds CD33 comprises the VH of SEQ ID NOs: 52, 53, 54, 55, 56, 57, 58, 59, 60, or 61 and the VL of SEQ ID NOs: 81, 82, 83, 84, 85, 86, 87, or 88.
123) The immunoconjugate of any one of embodiments 103-116 and 120-122, wherein the first antigen binding domain that binds CD33 comprises

a) the VH of SEQ ID NO: 52 and the VL of SEQ ID NO: 81;
b) the VH of SEQ ID NO: 53 and the VL of SEQ ID NO: 82;
c) the VH of SEQ ID NO: 54 and the VL of SEQ ID NO: 83;
d) the VH of SEQ ID NO: 55 and the VL of SEQ ID NO: 84;
e) the VH of SEQ ID NO: 56 and the VL of SEQ ID NO: 85;
f) the VH of SEQ ID NO: 57 and the VL of SEQ ID NO: 86;
g) the VH of SEQ ID NO: 58 and the VL of SEQ ID NO: 87;
h) the VH of SEQ ID NO: 59 and the VL of SEQ ID NO: 87;
i) the VH of SEQ ID NO: 60 and the VL of SEQ ID NO: 87; or
j) the VH of SEQ ID NO: 61 and the VL of SEQ ID NO: 88.

124) The immunoconjugate of any one of embodiments 103-116, wherein the first antigen binding domain that binds CD33 comprises the HCDR1 of SEQ ID NOs: 33, 89, 167, 172 or 176, the HCDR2 of SEQ ID NOs: 90, 91, 168, 173, or 177, the HCDR3 of SEQ ID NOs: 92, 93, 94, 169, 174, 178, or 180, the LCDR1 of SEQ ID NOs: 98, 99, 100, 182, 186, 189, 193, 197, or 201, the LCDR2 of SEQ ID NOs: 101, 102, 103, 183, 187, 190, 194, or 198, and the LCDR3 of SEQ ID NO: 104, 184, 191, 195, or 199.
125) The immunoconjugate of any one of embodiments 103-116 and 124, wherein the first antigen binding domain that binds CD33 comprises the HCDR1, the HCDR2, the HCDR3, the LCDR1, the LCDR2 and the LCDR3 of

a) SEQ ID NOs: 89, 90, 92, 98, 101, and 104, respectively;
b) SEQ ID NOs: 89, 90, 93, 99, 102, and 104, respectively;
c) SEQ ID NOs: 33, 91, 94, 100, 103, and 104, respectively;
d) SEQ ID NOs: 167, 168, 169, 182, 183, and 184, respectively;
e) SEQ ID NOs: 33, 91, 94, 186, 187, and 104, respectively;
f) SEQ ID NOs: 172, 173, 174, 189, 190, and 191, respectively;
g) SEQ ID NOs: 176, 177, 178, 193, 194, and 195, respectively;
h) SEQ ID NOs: 89, 90, 180, 197, 198, and 199, respectively; or
i) SEQ ID NOs: 89, 90, 93, 201, 102, and 104, respectively.

126) The immunoconjugate of any one of embodiments 103-116, 124 and 125, wherein the antigen binding domain that binds CD33 comprises the VH of SEQ ID NOs: 95, 96, 97, 170, 171, 175, or 179 and the VL of SEQ ID NOs 105, 106, 107, 185, 188, 192, 196, 200, or 202.
127) The immunoconjugate of any one of embodiments 103-116 and 124-126, wherein the first antigen binding domain that binds CD33 comprises

a) the VH of SEQ ID NO: 95 and the VL of SEQ ID NO: 105;
b) the VH of SEQ ID NO: 96 and the VL of SEQ ID NO: 106;
c) the VH of SEQ ID NO: 97 and the VL of SEQ ID NO: 107;
d) the VH of SEQ ID NO: 170 and the VL of SEQ ID NO: 185;

e) the VH of SEQ ID NO: 171 and the VL of SEQ ID NO: 188;
f) the VH of SEQ ID NO: 175 and the VL of SEQ ID NO: 192;
g) the VH of SEQ ID NO: 179 and the VL of SEQ ID NO: 196;
h) the VH of SEQ ID NO: 181 and the VL of SEQ ID NO: 200; or
i) the VH of SEQ ID NO: 96 and the VL of SEQ ID NO: 202.

128) The immunoconjugate of any one of embodiments 103-127, wherein the second antigen binding domain that binds the lymphocyte antigen comprises

a) the HCDR1 of SEQ ID NO: 141, the HCDR2 of SEQ ID NO: 142, the HCDR3 of SEQ ID NO: 143, the LCDR1 of SEQ ID NO: 144, the LCDR2 of SEQ ID NO: 145 and the LCDR3 of SEQ ID NO: 146;
b) the VH of SEQ ID NO: 147 and the VL of SEQ ID NO: 148;
c) the HCDR1 of SEQ ID NO: 149, the HCDR2 of SEQ ID NO: 150, the HCDR3 of SEQ ID NO: 151, the LCDR1 of SEQ ID NO: 152, the LCDR2 of SEQ ID NO: 153 and the LCDR3 of SEQ ID NO: 154; or
d) the VH of SEQ ID NO: 155 and the VL of SEQ ID NO: 156.

129) The immunoconjugate of any one of embodiments 101-128, wherein the second antigen binding domain that binds the lymphocyte antigen comprises

a) a heavy chain complementarity determining region 1 (HCDR1) of SEQ ID NO: 236, a HCDR2 of SEQ ID NO: 237, a HCDR3 of SEQ ID NO: 238, a light chain complementarity determining region 1 (LCDR1) of SEQ ID NO: 239, a LCDR2 of SEQ ID NO: 240 and a LCDR3 of SEQ ID NO: 241;
b) a heavy chain complementarity determining region 1 (HCDR1) of SEQ ID NO: 236, a HCDR2 of SEQ ID NO: 237, a HCDR3 of SEQ ID NO: 242, a light chain complementarity determining region 1 (LCDR1) of SEQ ID NO: 239, a LCDR2 of SEQ ID NO: 240 and a LCDR3 of SEQ ID NO: 241;
c) a heavy chain complementarity determining region 1 (HCDR1) of SEQ ID NO: 236, a HCDR2 of SEQ ID NO: 237, a HCDR3 of SEQ ID NO: 243, a light chain complementarity determining region 1 (LCDR1) of SEQ ID NO: 239, a LCDR2 of SEQ ID NO: 240 and a LCDR3 of SEQ ID NO: 241;
d) a heavy chain complementarity determining region 1 (HCDR1) of SEQ ID NO: 236, a HCDR2 of SEQ ID NO: 237, a HCDR3 of SEQ ID NO: 244, a light chain complementarity determining region 1 (LCDR1) of SEQ ID NO: 239, a LCDR2 of SEQ ID NO: 240 and a LCDR3 of SEQ ID NO: 241;
e) the VH of SEQ ID NO: 245 and the VL of SEQ ID NO: 249;
f) the VH of SEQ ID NO: 246 and the VL of SEQ ID NO: 249;
g) the VH of SEQ ID NO: 247 and the VL of SEQ ID NO: 249; or
h) the VH of SEQ ID NO: 248 and the VL of SEQ ID NO: 249.

130) The immunoconjugate of any one of embodiments 103-129, wherein the first antigen binding domain that binds CD33 is conjugated to a first immunoglobulin (Ig) constant region or a fragment of the first Ig constant region and/or the second antigen binding domain that binds the lymphocyte antigen is conjugated to a second immunoglobulin (Ig) constant region or a fragment of the second Ig constant region.
131) The immunoconjugate of embodiment 130, further comprising a second linker (L2) between the first antigen binding domain that binds CD33 and the first Ig constant region or the fragment of the first Ig constant region and the second antigen binding domain that binds the lymphocyte antigen and the second Ig constant region or the fragment of the second Ig constant region.
132) The immunoconjugate of embodiment 131, wherein the L2 comprises the amino acid sequence of any one of SEQ ID NOs: 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, or 140.
133) The immunoconjugate of any one of embodiments 130-132, wherein the first Ig constant region or the fragment of the first Ig constant region and the second Ig constant region or the fragment of the second Ig constant region is an IgG1, an IgG2, and IgG3 or an IgG4 isotype.
134) The immunoconjugate of any one of embodiments 130-133, wherein the first Ig constant region or the fragment of the first Ig constant region and the second Ig constant region or the fragment of the second Ig constant region comprises at least one mutation that results in reduced binding of the multispecific protein to a FcγR.
135) The immunoconjugate of embodiment 134, wherein the at least one mutation that results in reduced binding of the multispecific protein to the FcγR is selected from the group consisting of F234A/L235A, L234A/L235A, L234A/L235A/D265S, V234A/G237A/ P238S/H268A/V309L/A330S/P331S, F234A/L235A, S228P/F234A/ L235A, N297A, V234A/G237A, K214T/E233P/ L234V/L235A/G236-deleted/A327G/P331A/D365E/L358M, H268Q/V309L/A330S/P331S, S267E/L328F, L234F/L235E/D265A,

L234A/L235A/G237A/P238S/H268A/A330S/P331S, S228P/F234A/L235A/G237A/P238S and S228P/F234A/L235A/G236-deleted/G237A/P238S, wherein residue numbering is according to the EU index.

136) The immunoconjugate of any one of embodiments 130-133, wherein the first Ig constant region or the fragment of the first Ig constant region and the second Ig constant region or the fragment of the second Ig constant region comprises at least one mutation that results in enhanced binding of the multispecific protein to a Fcγ receptor (FcγR).

137) The immunoconjugate of embodiment 136, wherein the at least one mutation that results in enhanced binding of the multispecific protein to the FcγR is selected from the group consisting of S239D/I332E, S298A/E333A/K334A, F243L/R292P/Y300L, F243L/R292P/Y300L/P396L, F243L/R292P/Y300L/V305I/P396L and G236A/S239D/I332E, wherein residue numbering is according to the EU index.

138) The immunoconjugate of any one of embodiments 134-137, wherein the FcγR is FcγRI, FcγRIIA, FcγRIIB or FcγRIII, or any combination thereof.

139) The immunoconjugate of any one of embodiments 130-138, wherein the first Ig constant region or the fragment of the first Ig constant region and the second Ig constant region or the fragment of the second Ig constant region comprises at least one mutation that modulates a half-life of the multispecific protein.

140) The immunoconjugate of embodiment 139, wherein the at least one mutation that modulates the half-life of the multispecific protein is selected from the group consisting of H435A, P257I/N434H, D376V/N434H, M252Y/S254T/T256E/H433K/N434F, T308P/N434A and H435R, wherein residue numbering is according to the EU index.

141) The immunoconjugate of any one of the embodiments 130-140, wherein the protein comprises at least one mutation in a CH3 domain of the first Ig constant region or in a CH3 domain of the fragment of the first Ig constant region and/or at least one mutation in a CH3 domain of the second Ig constant region or in a CH3 domain of the fragment of the second Ig constant region.

142) The immunoconjugate of embodiment 141, wherein the at least one mutation in a CH3 domain of the first Ig constant region or in a CH3 domain of the fragment of the first Ig constant region and/or at least one mutation in a CH3 domain of the second Ig constant region or in a CH3 domain of the fragment of the second Ig constant region is selected from the group consisting of T350V, L351Y, F405A,Y407V, T366Y, T366W, F405W, T394W, T394S, Y407T, Y407A, T366S/L368A/Y407V, L351Y/F405A/Y407V, T366I/K392M/T394W, F405A/Y407V, T366L/K392M/T394W, L351Y/Y407A, T366A/K409F, L351Y/Y407A, T366V/K409F, T366A/K409F, T350V/L351Y/F405A/Y407V and T350V/T366L/K392L/T394W, wherein residue numbering is according to the EU index.

143) The immunoconjugate of any one of embodiments 130-142, wherein the first Ig constant region or the fragment of the first Ig constant region and the second Ig constant region or the fragment of the second Ig constant region comprise the following mutations:

a) L235A_L235A_D265S_T350V_L351Y_F405A Y407V in the first Ig constant region and L235A_L235A_D265S_T350V_T366L_K392L_T394W in the second Ig constant region; or

b) L235A_L235A_D265S_T350V_T366L_K392L_T394W in the first Ig constant region and L235A_L235A_D265S_T350V_L351Y_F405A_Y407V in the second Ig constant region.

144) The immunoconjugate of any one of embodiments 103-143, wherein the agent is a therapeutic agent or an imaging agent.

145) A pharmaceutical composition comprising the immunoconjugate of any one of embodiments 103-143 and a pharmaceutically acceptable carrier.

146) A polynucleotide encoding the isolated multispecific protein of the immunoconjugate of any one of embodiments 103-143.

147) A vector comprising the polynucleotide of embodiment 146.

148) A host cell comprising the vector of embodiment 147.

149) A method of producing the immunoconjugate of any one of embodiments 103-143, comprising culturing the host cell of embodiment 148 in conditions that the multispecific protein is expressed, recovering the multispecific protein produced by the host cell, and conjugating the multispecific protein to an agent.

150) The immunoconjugate of any one of embodiments 1-96 and 103-143, wherein the agent is a radioactive agent.

151) The immunoconjugate of embodiment 150, wherein the radioactive agent comprises a radiometal ion.

152) The immunoconjugate of embodiment 151, wherein the radiometal ion is $^{32}$P, $^{47}$Sc, $^{67}$Cu, $^{77}$As, $^{89}$Sr, $^{90}$Y, $^{99}$Tc, $^{105}$Rh, $^{109}$Pd, $^{111}$Ag, $^{131}$I, $^{153}$Sm, $^{159}$Gd, $^{165}$Dy, $^{166}$Ho, $^{169}$Er, $^{177}$Lu, $^{186}$Re, $^{188}$Re, $^{194}$Ir, $^{198}$Au, $^{199}$Au, $^{211}$At, $^{212}$Pb, $^{212}$Bi, $^{213}$Bi, $^{223}$Ra, $^{225}$Ac, $^{255}$Fm, $^{227}$Th, $^{62}$Cu, $^{64}$Cu, $^{67}$Ga, $^{68}$Ga, $^{86}$Y, $^{89}$Zr, or $^{111}$In.

153) The immunoconjugate of embodiment 151, wherein the radiometal ion is $^{225}$Ac, $^{111}$In or $^{89}$Zr.

154) The immunoconjugate of embodiment 151, wherein the radiometal ion is $^{225}$Ac.

155) The immunoconjugate of any one of embodiments 151 to 154, wherein the radiometal ion is conjugated to a

chelating moiety.

156) The immunoconjugate of embodiment 155, wherein the chelating moiety comprises a macrocycle having the structure of formula (I):

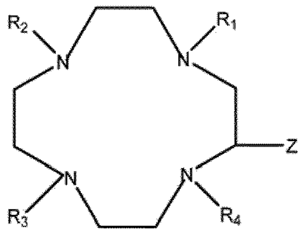

formula (I),

wherein each of $R_1$, $R_2$, $R_3$ and $R_4$ is independently $CHQCO_2X$, wherein
Q is independently hydrogen, $C_1$-$C_4$ alkyl or ($C_1$-$C_2$ alkyl) phenyl, and
X is independently hydrogen, benzyl, $C_1$-$C_4$ alkyl; and
Z is $(CH2)_nY$, wherein
n is 1-10, and
Y is an electrophilic or nucleophilic moiety covalently linked to the second click reaction partner; alternatively,
Z is hydrogen; and
each of $R_1$, $R_2$, $R_3$ and $R_4$ is independently $CHQCO_2X$, wherein
Q is independently hydrogen, $C_1$-$C_4$ alkyl or ($C_1$-$C_2$ alkyl) phenyl, and
X is independently hydrogen, benzyl, $C_1$-$C_4$ alkyl, or an electrophilic or nucleophilic moiety covalently linked to the second click reaction partner;
alternatively, the chelant comprises an open chain ligand.

157) The immunoconjugate of embodiment 155, wherein the chelating moiety comprises a macrocycle having the structure of formula (II):

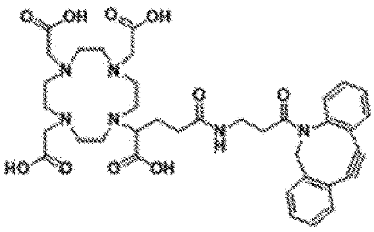

or a structure of formula (III):

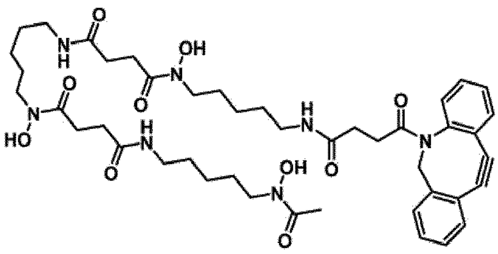

158) An immunoconjugate of formula (IV),

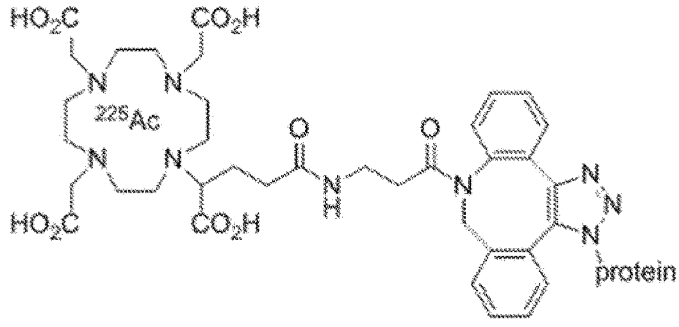

formula (IV)

wherein the protein is the isolated protein as defined in any one of embodiments 1-96.

159) An immunoconjugate of formula (V),

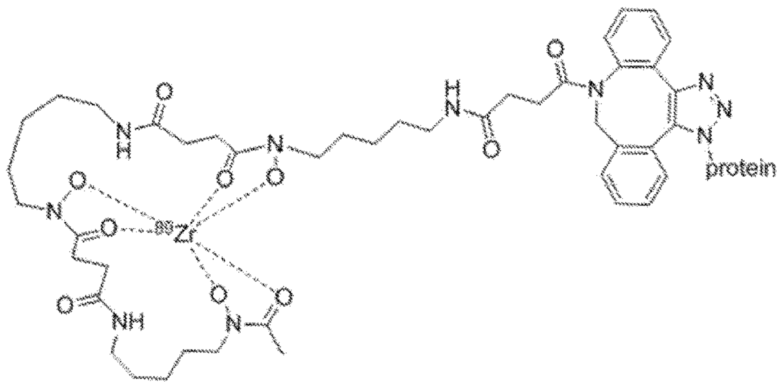

formula (V)

wherein the protein is the isolated protein as defined in any one of embodiments 1-96.

160) A pharmaceutical composition comprising a means for treating a CD33 expressing cancer in a subject.

161) A pharmaceutical composition comprising a means for reducing the amount of CD33 expressing tumor cells in a subject.

162) A pharmaceutical composition comprising a means for preventing establishment of a CD33 expressing cancer in a subject.

163) A pharmaceutical composition comprising a means for treating a noncancerous condition in a subject at risk of developing a CD33 expressing cancer.

List of numbered embodiments (D):

1) A method of treating a CD33 expressing cancer in a subject, comprising administering a therapeutically effective amount of an isolated protein to the subject for a time sufficient to treat the CD33 expressing cancer, wherein the isolated protein binds CD33, and wherein the isolated protein comprises

a) an HCDR1, an HCDR2 and an HCDR3 of a heavy chain variable region (VH) of SEQ ID NO: 18;
b) an HCDR1, an HCDR2 and an HCDR3 of a VH of SEQ ID NO: 19;
c) an HCDR1, an HCDR2 and an HCDR3 of a VH of SEQ ID NO: 20;
d) an HCDR1, an HCDR2 and an HCDR3 of a VH of SEQ ID NO: 21;
e) an HCDR1, an HCDR2 and an HCDR3 of a VH of SEQ ID NO: 22;
f) an HCDR1, an HCDR2 and an HCDR3 of a VH of SEQ ID NO: 23;
g) an HCDR1, an HCDR2 and an HCDR3 of a VH of SEQ ID NO: 24;
h) an HCDR1, an HCDR2 and an HCDR3 of a VH of SEQ ID NO: 25;
i) an HCDR1, an HCDR2 and an HCDR3 of a VH of SEQ ID NO: 26; or
j) an HCDR1, an HCDR2 and an HCDR3 of a VH of SEQ ID NO: 27,

or a CD33 binding fragment thereof.

2) The method of embodiment 1, wherein the isolated protein comprises the HCDR1, the HCDR2, the HCDR3 of

a) SEQ ID NOs: 1, 6, and 12, respectively;
b) SEQ ID NOs: 2, 7, and 13, respectively;
c) SEQ ID NOs: 1, 8, and 14, respectively;
d) SEQ ID NOs: 3, 9, and 13, respectively;
e) SEQ ID NOs: 4, 10, and 15, respectively;
f) SEQ ID NOs: 5, 11, and 16, respectively; or
g) SEQ ID NOs: 5, 11, and 17, respectively.

3) The method of embodiments 1 or 2, wherein the isolated protein is a scFv, a $(scFv)_2$, a Fv, a Fab, a $F(ab')_2$, a Fd, a dAb or a VHH.

4) The method of embodiment 3, wherein the isolated protein is the VHH.

5) The method of embodiment 3, wherein the isolated protein is the Fab.

6) The method of embodiment 3, wherein the isolated protein is the scFv.

7) The method of any of embodiments 1-6, wherein the isolated protein comprises the VH of any one of SEQ ID NOs: 18, 19, 20, 21, 22, 23, 24, 25, 26, or 27.

8) A method of treating a CD33 expressing cancer in a subject, comprising administering a therapeutically effective amount of an isolated protein to the subject for a time sufficient to treat the CD33 expressing cancer, wherein the isolated protein binds CD33, and wherein the isolated protein comprises a VHH of any one of SEQ ID NOs: 18, 19, 20, 21, 22, 23, 24, 25, 26, or 27.

9) A method of treating a CD33 expressing cancer in a subject, comprising administering a therapeutically effective amount of an isolated protein to the subject for a time sufficient to treat the CD33 expressing cancer, wherein the isolated protein binds CD33, wherein the antigen binding domain that binds CD33 comprises

a) an HCDR1, an HCDR2 and an HCDR3 of a heavy chain variable region (VH) of SEQ ID NO: 52;
b) an HCDR1, an HCDR2 and an HCDR3 of a VH of SEQ ID NO: 53;
c) an HCDR1, an HCDR2 and an HCDR3 of a VH of SEQ ID NO: 54;
d) an HCDR1, an HCDR2 and an HCDR3 of a VH of SEQ ID NO: 55;
e) an HCDR1, an HCDR2 and an HCDR3 of a VH of SEQ ID NO: 56;
f) an HCDR1, an HCDR2 and an HCDR3 of a VH of SEQ ID NO: 57;
g) an HCDR1, an HCDR2 and an HCDR3 of a VH of SEQ ID NO: 58;
h) an HCDR1, an HCDR2 and an HCDR3 of a VH of SEQ ID NO: 59;
i) an HCDR1, an HCDR2 and an HCDR3 of a VH of SEQ ID NO: 60; or
j) an HCDR1, an HCDR2 and an HCDR3 of a VH of SEQ ID NO: 61,

or a CD33 binding fragment thereof.

10) The method of embodiment 9, wherein the isolated protein comprises the HCDR1, the HCDR2, and the HCDR3 of

a) SEQ ID NOs: 28, 36, and 45, respectively;
b) SEQ ID NOs: 29, 37, and 46, respectively;
c) SEQ ID NOs: 30, 38, and 47, respectively;
d) SEQ ID NOs: 31, 39, and 48, respectively;
e) SEQ ID NOs: 32, 40, and 49, respectively;
f) SEQ ID NOs: 33, 41, and 50, respectively;
g) SEQ ID NOs: 34, 42, and 51, respectively;
h) SEQ ID NOs: 34, 43, and 51, respectively;
i) SEQ ID NOs: 34, 44, and 51, respectively; or
j) SEQ ID NOs: 35, 42, and 51, respectively.

11) The method of embodiment 9 or 10, wherein the isolated protein comprises

a) an HCDR1, an HCDR2 and an HCDR3 of a heavy chain variable region (VH) of SEQ ID NO: 52 and a light chain complementarity determining region (LCDR) 1, an LCDR2 and an LCDR3 of a light chain variable region (VL) of SEQ ID NO: 81;
b) an HCDR1, an HCDR2 and an HCDR3 of a VH of SEQ ID NO: 53 and an LCDR1, an LCDR2 and an LCDR3 of a VL of SEQ ID NO: 82;

c) an HCDR1, an HCDR2 and an HCDR3 of a VH of SEQ ID NO: 54 and an LCDR1, an LCDR2 and an LCDR3 of a VL of SEQ ID NO: 83;
d) an HCDR1, an HCDR2 and an HCDR3 of a VH of SEQ ID NO: 55 and an LCDR1, an LCDR2 and an LCDR3 of a VL of SEQ ID NO: 84;
e) an HCDR1, an HCDR2 and an HCDR3 of a VH of SEQ ID NO: 56 and an LCDR1, an LCDR2 and an LCDR3 of a VL of SEQ ID NO: 85;
f) an HCDR1, an HCDR2 and an HCDR3 of a VH of SEQ ID NO: 57 and an LCDR1, an LCDR2 and an LCDR3 of a VL of SEQ ID NO: 86;
g) an HCDR1, an HCDR2 and an HCDR3 of a VH of SEQ ID NO: 58 and an LCDR1, an LCDR2 and an LCDR3 of a VL of SEQ ID NO: 87;
h) an HCDR1, an HCDR2 and an HCDR3 of a VH of SEQ ID NO: 59 and an LCDR1, an LCDR2 and an LCDR3 of a VL of SEQ ID NO: 87;
i) an HCDR1, an HCDR2 and an HCDR3 of a VH of SEQ ID NO: 60 and an LCDR1, an LCDR2 and an LCDR3 of a VL of SEQ ID NO: 87; or
j) an HCDR1, an HCDR2 and an HCDR3 of a VH of SEQ ID NO: 61 and an LCDR1, an LCDR2 and an LCDR3 of a VL of SEQ ID NO: 88.

12) The method of embodiment 11, comprising the HCDR1, the HCDR2, the HCDR3, the LCDR1, the LCDR2 and the LCDR3 of

a) SEQ ID NOs: 28, 36, 45, 62, 69, and 75, respectively;
b) SEQ ID NOs: 29, 37, 46, 63, 70, and 76, respectively;
c) SEQ ID NOs: 30, 38, 47, 64, 71, and 77, respectively;
d) SEQ ID NOs: 31, 39, 48, 65, 72, and 78, respectively;
e) SEQ ID NOs: 32, 40, 49, 66, 70, and 76, respectively;
f) SEQ ID NOs: 33, 41, 50, 67, 73, and 79, respectively;
g) SEQ ID NOs: 34, 42, 51, 68, 74, and 80, respectively;
h) SEQ ID NOs: 34, 43, 51, 68, 74, and 80, respectively;
i) SEQ ID NOs: 34, 44, 51, 68, 74, and 80, respectively; or
j) SEQ ID NOs: 35, 42, 51, 68, 74, and 80, respectively.

13) The method of any one of embodiments 9-12, wherein the isolated protein is a scFv, a $(scFv)_2$, a Fv, a Fab, a $F(ab')_2$, a Fd, a dAb or a VHH.
14) The method of embodiment 13, wherein the isolated protein is the Fab.
15) The method of embodiment 13, wherein the isolated protein is the VHH.
16) The method of embodiment 13, wherein the isolated protein is the scFv.
17) The method of embodiment 16, wherein the scFv comprises, from the N- to C-terminus, a VH, a first linker (L1) and a VL (VH-L1-VL) or the VL, the L1 and the VH (VL-L1-VH).
18) The method of embodiment 17, wherein the L1 comprises

a) about 5-50 amino acids;
b) about 5-40 amino acids;
c) about 10-30 amino acids; or
d) about 10-20 amino acids.

19) The method of embodiment 18, wherein the L1 comprises an amino acid sequence of any one of SEQ ID NOs: 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, or 140.
20) The method of embodiment 19, wherein the L1 comprises the amino acid sequence of SEQ ID NO: 108.
21) The method of any one of embodiments 9-20, wherein the antigen binding domain that binds CD33 comprises the VH of SEQ ID NOs: 52, 53, 54, 55, 56, 57, 58, 59, 60, or 61, and the VL of SEQ ID NOs: 81, 82, 83, 84, 85, 86, 87, or 88.
22) The method of embodiment 21, wherein the isolated protein comprises:

a) the VH of SEQ ID NO: 52 and the VL of SEQ ID NO: 81;
b) the VH of SEQ ID NO: 53 and the VL of SEQ ID NO: 82;
c) the VH of SEQ ID NO: 54 and the VL of SEQ ID NO: 83;
d) the VH of SEQ ID NO: 55 and the VL of SEQ ID NO: 84;

e) the VH of SEQ ID NO: 56 and the VL of SEQ ID NO: 85;
f) the VH of SEQ ID NO: 57 and the VL of SEQ ID NO: 86;
g) the VH of SEQ ID NO: 58 and the VL of SEQ ID NO: 87;
h) the VH of SEQ ID NO: 59 and the VL of SEQ ID NO: 87;
i) the VH of SEQ ID NO: 60 and the VL of SEQ ID NO: 87; or
j) the VH of SEQ ID NO: 61 and the VL of SEQ ID NO: 88.

23) A method of treating a CD33 expressing cancer in a subject, comprising administering a therapeutically effective amount of an isolated protein to the subject for a time sufficient to treat the CD33 expressing cancer, wherein isolated protein binds CD33, and wherein the isolated protein comprises

a) the VH of SEQ ID NO: 52 and the VL of SEQ ID NO: 81;
b) the VH of SEQ ID NO: 53 and the VL of SEQ ID NO: 82;
c) the VH of SEQ ID NO: 54 and the VL of SEQ ID NO: 83;
d) the VH of SEQ ID NO: 55 and the VL of SEQ ID NO: 84;
e) the VH of SEQ ID NO: 56 and the VL of SEQ ID NO: 85;
f) the VH of SEQ ID NO: 57 and the VL of SEQ ID NO: 86;
g) the VH of SEQ ID NO: 58 and the VL of SEQ ID NO: 87;
h) the VH of SEQ ID NO: 59 and the VL of SEQ ID NO: 87;
i) the VH of SEQ ID NO: 60 and the VL of SEQ ID NO: 87; or
j) the VH of SEQ ID NO: 61 and the VL of SEQ ID NO: 88.

24) The method of embodiment 23, wherein the isolated protein is a scFv, a $(scFv)_2$, a Fv, a Fab, a $F(ab')_2$, a Fd, a dAb or a VHH.
25) The method of embodiment 24, wherein the isolated protein is the Fab.
26) The method of embodiment 24, wherein the isolated protein is the VHH.
27) The method of embodiment 24, wherein the isolated protein is the scFv.
28) The method of embodiment 27, wherein the scFv comprises, from the N- to C-terminus, a VH, a first linker (L1) and a VL (VH-L1-VL) or the VL, the L1 and the VH (VL-L1-VH).
29) The method of embodiment 28, wherein the L1 comprises

a) about 5-50 amino acids;
b) about 5-40 amino acids;
c) about 10-30 amino acids; or
d) about 10-20 amino acids.

30) The method of embodiment 29, wherein the L1 comprises an amino acid sequence of any one of SEQ ID NOs: 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, or 140.
31) The method of embodiment 30, wherein the L1 comprises the amino acid sequence of SEQ ID NO: 108.
32) A method of treating a CD33 expressing cancer in a subject, comprising administering a therapeutically effective amount of an isolated protein to the subject for a time sufficient to treat the CD33 expressing cancer, wherein the isolated protein binds CD33, and wherein isolated protein comprises

a) an HCDR1, an HCDR2 and an HCDR3 of a heavy chain variable region (VH) of SEQ ID NO: 95;
b) an HCDR1, an HCDR2 and an HCDR3 of a VH of SEQ ID NO: 96;
c) an HCDR1, an HCDR2 and an HCDR3 of a VH of SEQ ID NO: 97;
d) an HCDR1, an HCDR2 and an HCDR3 of a VH of SEQ ID NO: 170;
e) an HCDR1, an HCDR2 and an HCDR3 of a VH of SEQ ID NO: 171;
f) an HCDR1, an HCDR2 and an HCDR3 of a VH of SEQ ID NO: 175;
g) an HCDR1, an HCDR2 and an HCDR3 of a VH of SEQ ID NO: 179;
h) an HCDR1, an HCDR2 and an HCDR3 of a VH of SEQ ID NO: 181;
i) an HCDR1, an HCDR2 and an HCDR3 of a VH of SEQ ID NO: 96.

33) The method of embodiment 32, comprising the HCDR1, the HCDR2, and the HCDR3 of

a) SEQ ID NOs: 89, 90, and 92, respectively;
b) SEQ ID NOs: 89, 90, and 93, respectively;

c) SEQ ID NOs: 33, 91, and 94, respectively
d) SEQ ID NOs: 167, 168, and 169, respectively;
e) SEQ ID NOs: 172, 173, and 174, respectively;
f) SEQ ID NOs: 176, 177, and 178, respectively;
g) SEQ ID NOs: 89, 90, and 180, respectively; or
h) SEQ ID NOs: 89, 90, and 93, respectively.

34) The method of embodiment 32 or 33, wherein the isolated protein comprises

a) an HCDR1, an HCDR2 and an HCDR3 of a heavy chain variable region (VH) of SEQ ID NO: 95 and a light chain complementarity determining region (LCDR) 1, an LCDR2 and an LCDR3 of a light chain variable region (VL) of SEQ ID NO: 105;
b) an HCDR1, an HCDR2 and an HCDR3 of a VH of SEQ ID NO: 96 and an LCDR1, an LCDR2 and an LCDR3 of a VL of SEQ ID NO: 106;
c) an HCDR1, an HCDR2 and an HCDR3 of a VH of SEQ ID NO: 97 and an LCDR1, an LCDR2 and an LCDR3 of a VL of SEQ ID NO: 107;
d) an HCDR1, an HCDR2 and an HCDR3 of a VH of SEQ ID NO: 170 and an LCDR1, an LCDR2 and an LCDR3 of a VL of SEQ ID NO: 185;
e) an HCDR1, an HCDR2 and an HCDR3 of a VH of SEQ ID NO: 171 and an LCDR1, an LCDR2 and an LCDR3 of a VL of SEQ ID NO: 188;
f) an HCDR1, an HCDR2 and an HCDR3 of a VH of SEQ ID NO: 175 and an LCDR1, an LCDR2 and an LCDR3 of a VL of SEQ ID NO: 192;
g) an HCDR1, an HCDR2 and an HCDR3 of a VH of SEQ ID NO: 179 and an LCDR1, an LCDR2 and an LCDR3 of a VL of SEQ ID NO: 196;
h) an HCDR1, an HCDR2 and an HCDR3 of a VH of SEQ ID NO: 181 and an LCDR1, an LCDR2 and an LCDR3 of a VL of SEQ ID NO: 200; or
i) an HCDR1, an HCDR2 and an HCDR3 of a VH of SEQ ID NO: 96 and an LCDR1, an LCDR2 and an LCDR3 of a VL of SEQ ID NO: 202.

35) The method of embodiment 34, wherein the isolated protein comprises the HCDR1, the HCDR2, the HCDR3, the LCDR1, the LCDR2 and the LCDR3 of

a) SEQ ID NOs: 89, 90, 92, 98, 101, and 104, respectively;
b) SEQ ID NOs: 89, 90, 93, 99, 102, and 104, respectively;
c) SEQ ID NOs: 33, 91, 94, 100, 103, and 104, respectively;
d) SEQ ID NOs: 167, 168, 169, 182, 183, and 184, respectively;
e) SEQ ID NOs: 33, 91, 94, 186, 187, and 104, respectively;
f) SEQ ID NOs: 172, 173, 174, 189, 190, and 191, respectively;
g) SEQ ID NOs: 176, 177, 178, 193, 194, and 195, respectively;
h) SEQ ID NOs: 89, 90,180, 197, 198, and 199, respectively;
i) SEQ ID NOs: 89, 90, 93, 201, 102, and 104, respectively.

36) The method of any one of embodiments 32-35, wherein the isolated protein is a scFv, a (scFv)z, a Fv, a Fab, a $F(ab')_2$, a Fd, a dAb or a VHH.
37) The method of embodiment 36, wherein the isolated protein is the Fab.
38) The method of embodiment 36, wherein the isolated protein is the VHH.
39) The method of embodiment 36, wherein the isolated protein is the scFv.
40) The method of embodiment 39, wherein the scFv comprises, from the N- to C-terminus, a VH, a first linker (L1) and a VL (VH-L1-VL) or the VL, the L1 and the VH (VL-L1-VH).
41) The method of embodiment 40, wherein the L1 comprises

a) about 5-50 amino acids;
b) about 5-40 amino acids;
c) about 10-30 amino acids; or
d) about 10-20 amino acids.

42) The method of embodiment 41, wherein the L1 comprises an amino acid sequence of any one of SEQ ID NOs: 108, 109, 110, 111, 112, 113, 114, 115, 116, 117, 118, 119, 120, 121, 122, 123, 124, 125, 126, 127, 128, 129, 130,

131, 132, 133, 134, 135, 136, 137, 138, 139, or 140.
43) The method of embodiment 42, wherein the L1 comprises the amino acid sequence of SEQ ID NO: 108.
44) The method of any one of embodiments 32-43, wherein the isolated protein comprises the VH of SEQ ID NOs: 95, 96, 97, 170, 171, 175, 179, 181, or 96, and the VL of SEQ ID NOs: 105, 106, 107, 185, 188, 192, 196, 200, or 202.
45) The method of embodiment 44, wherein the isolated protein comprises:

a) the VH of SEQ ID NO: 95 and the VL of SEQ ID NO: 105;
b) the VH of SEQ ID NO: 96 and the VL of SEQ ID NO: 106;
c) the VH of SEQ ID NO: 97 and the VL of SEQ ID NO: 107;
d) the VH of SEQ ID NO: 170 and the VL of SEQ ID NO: 185;
e) the VH of SEQ ID NO: 171 and the VL of SEQ ID NO: 188;
f) the VH of SEQ ID NO: 175 and the VL of SEQ ID NO: 192;
g) the VH of SEQ ID NO: 179 and the VL of SEQ ID NO: 196;
h) the VH of SEQ ID NO: 181 and the VL of SEQ ID NO: 200; or
i) the VH of SEQ ID NO: 96 and the VL of SEQ ID NO: 202.

46) A method of treating a CD33 expressing cancer in a subject, comprising administering a therapeutically effective amount of an isolated protein to the subject for a time sufficient to treat the CD33 expressing cancer, wherein the isolated protein binds CD33, wherein the isolated protein comprises

a) the VH of SEQ ID NO: 95 and the VL of SEQ ID NO: 105;
b) the VH of SEQ ID NO: 96 and the VL of SEQ ID NO: 106;
c) the VH of SEQ ID NO: 97 and the VL of SEQ ID NO: 107;
d) the VH of SEQ ID NO: 170 and the VL of SEQ ID NO: 185;
e) the VH of SEQ ID NO: 171 and the VL of SEQ ID NO: 188;
f) the VH of SEQ ID NO: 175 and the VL of SEQ ID NO: 192;
g) the VH of SEQ ID NO: 179 and the VL of SEQ ID NO: 196;
h) the VH of SEQ ID NO: 181 and the VL of SEQ ID NO: 200; or
i) the VH of SEQ ID NO: 96 and the VL of SEQ ID NO: 202.

47) The method of embodiment 46, wherein the isolated protein is a scFv, a $(scFv)_2$, a Fv, a Fab, a $F(ab')_2$, a Fd, a dAb or a VHH.
48) The method of embodiment 47, wherein the isolated protein is the Fab.
49) The method of embodiment 47, wherein the isolated protein is the VHH.
50) The method of embodiment 47, wherein the isolated protein is the scFv.
51) The method of embodiment 50, wherein the scFv comprises, from the N- to C-terminus, a VH, a first linker (L1) and a VL (VH-L1-VL) or the VL, the L1 and the VH (VL-L1-VH).
52) The method of embodiment 51, wherein the L1 comprises

a) about 5-50 amino acids;
b) about 5-40 amino acids;
c) about 10-30 amino acids; or
d) about 10-20 amino acids.

53) The method of embodiment 52, wherein the L1 comprises an amino acid sequence of any one of SEQ ID NOs: 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, or 140.
54) The method of embodiment 53, wherein the L1 comprises the amino acid sequence of SEQ ID NO: 108.
55) The method of any one of embodiments 1-54, wherein the isolated protein is conjugated to a half-life extending moiety.
56) The method of embodiment 55, wherein the half-life extending moiety is an immunoglobulin (Ig), a fragment of the Ig, an Ig constant region, a fragment of the Ig constant region, a Fc region, transferrin, albumin, an albumin binding domain or polyethylene glycol.
57) The method of any one of embodiments 1-56, wherein the isolated protein is a monospecific protein.
58) The method of any one of embodiments 1-57, wherein the isolated protein is a multispecific protein.
59) The method of embodiment 58, wherein the multispecific protein is a bispecific protein.
60) The method of embodiment 59, wherein the multispecific protein is a trispecific protein.
61) The method of any one of embodiments 1-60, further comprising an immunoglobulin (Ig) constant region or a

fragment of the Ig constant region thereof.

62) The method of embodiment 61, wherein the fragment of the Ig constant region comprises a Fc region.

63) The method of embodiment 61, wherein the fragment of the Ig constant region comprises a CH2 domain.

64) The method of embodiment 61, wherein the fragment of the Ig constant region comprises a CH3 domain.

65) The method of embodiment 61, wherein the fragment of the Ig constant region comprises the CH2 domain and the CH3 domain.

66) The method of embodiment 61, wherein the fragment of the Ig constant region comprises at least portion of a hinge, the CH2 domain and the CH3 domain.

67) The method of embodiment 61, wherein the fragment of the Ig constant region comprises a hinge, the CH2 domain and the CH3 domain.

68) The method of any one of embodiments 61-67, wherein the isolated protein is conjugated to the N-terminus of the Ig constant region or the fragment of the Ig constant region.

69) The method of any one of embodiments 61-67, wherein the isolated protein is conjugated to the C-terminus of the Ig constant region or the fragment of the Ig constant region.

70) The method of any one of embodiments 61-69, wherein the isolated protein is conjugated to the Ig constant region or the fragment of the Ig constant region via a second linker (L2).

71) The method of embodiment 70, wherein the L2 comprises the amino acid sequence of any one of SEQ ID NOs:108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, or 140.

72) The method of any one of embodiments 58-71, wherein the multispecific protein comprises an antigen binding domain that binds an antigen on a lymphocyte.

73) The method of embodiment 72, wherein the lymphocyte is a T cell.

74) The method of embodiment 72, wherein the T cell is a $CD8^+$ T cell

75) The method of embodiment 72, wherein the lymphocyte is a natural killer (NK) cell.

76) The method of any one of embodiments 58-75, wherein the multispecific protein comprises an antigen binding domain that binds CD3, CD3 epsilon (CD3ε), CD8, KI2L4, NKG2E, NKG2D, NKG2F, BTNL3, CD186, BTNL8, PD-1, CD195, TRGV9, or NKG2C.

77) The method of embodiment 76, wherein the multispecific protein comprises an antigen binding domain that binds CD3ε.

78) The method of embodiment 77, wherein the antigen binding domain that binds CD3ε comprises:

a) a heavy chain complementarity determining region 1 (HCDR1) of SEQ ID NO: 141, a HCDR2 of SEQ ID NO: 142, a HCDR3 of SEQ ID NO: 143, a light chain complementarity determining region 1 (LCDR1) of SEQ ID NO: 144, a LCDR2 of SEQ ID NO: 145 and a LCDR3 of SEQ ID NO: 146;
b) the VH of SEQ ID NO: 147 and the VL of SEQ ID NO: 148;
c) the HCDR1 of SEQ ID NO: 149, the HCDR2 of SEQ ID NO: 150, the HCDR3 of SEQ ID NO: 151, the LCDR1 of SEQ ID NO: 152, the LCDR2 of SEQ ID NO: 153 and the LCDR3 of SEQ ID NO: 154; or
d) the VH of SEQ ID NO: 155 and the VL of SEQ ID NO: 156.

79) The isolated protein of embodiment 76, wherein the multispecific protein comprises an antigen binding domain that binds TRGV9.

80) The isolated protein of embodiment 79, wherein the antigen binding domain that binds TRGV9 comprises:

a) a heavy chain complementarity determining region 1 (HCDR1) of SEQ ID NO: 236, a HCDR2 of SEQ ID NO: 237, a HCDR3 of SEQ ID NO: 238, a light chain complementarity determining region 1 (LCDR1) of SEQ ID NO: 239, a LCDR2 of SEQ ID NO: 240 and a LCDR3 of SEQ ID NO: 241;
b) a heavy chain complementarity determining region 1 (HCDR1) of SEQ ID NO: 236, a HCDR2 of SEQ ID NO: 237, a HCDR3 of SEQ ID NO: 242, a light chain complementarity determining region 1 (LCDR1) of SEQ ID NO: 239, a LCDR2 of SEQ ID NO: 240 and a LCDR3 of SEQ ID NO: 241;
c) a heavy chain complementarity determining region 1 (HCDR1) of SEQ ID NO: 236, a HCDR2 of SEQ ID NO: 237, a HCDR3 of SEQ ID NO: 243, a light chain complementarity determining region 1 (LCDR1) of SEQ ID NO: 239, a LCDR2 of SEQ ID NO: 240 and a LCDR3 of SEQ ID NO: 241;
d) a heavy chain complementarity determining region 1 (HCDR1) of SEQ ID NO: 236, a HCDR2 of SEQ ID NO: 237, a HCDR3 of SEQ ID NO: 244, a light chain complementarity determining region 1 (LCDR1) of SEQ ID NO: 239, a LCDR2 of SEQ ID NO: 240 and a LCDR3 of SEQ ID NO: 241;
e) the VH of SEQ ID NO: 245 and the VL of SEQ ID NO: 249;
f) the VH of SEQ ID NO: 246 and the VL of SEQ ID NO: 249;
g) the VH of SEQ ID NO: 247 and the VL of SEQ ID NO: 249; or

h) the VH of SEQ ID NO: 248 and the VL of SEQ ID NO: 249.

81) The method of any one of embodiments 61-80, wherein the Ig constant region or the fragment of the Ig constant region is an IgG1, an IgG2, an IgG3 or an IgG4 isotype.
82) The method of any one of embodiments 61-81, wherein the Ig constant region or the fragment of the Ig constant region comprises at least one mutation that results in reduced binding of the protein to a Fcγ receptor (FcγR).
83) The method of embodiment 82, wherein the at least one mutation that results in reduced binding of the protein to the FcγR is selected from the group consisting of F234A/L235A, L234A/L235A, L234A/L235A/D265S, V234A/G237A/ P238S/H268A/V309L/A330S/P331S, F234A/L235A, S228P/F234A/ L235A, N297A, V234A/G237A, K214T/E233P/ L234V/L235A/G236-deleted/A327G/P331A/D365E/L358M, H268Q/V309L/A330S/P331S, S267E/L328F, L234F/L235E/D265A, L234A/L235A/G237A/P238S/H268A/A330S/P331S, S228P/F234A/L235A/G237A/P238S and S228P/F234A/L235A/G236-deleted/G237A/P238S, wherein residue numbering is according to the EU index.
84) The method of any one of embodiments 61-81, wherein the Ig constant region or the fragment of the Ig constant region comprises at least one mutation that results in enhanced binding of the protein to the FcγR.
85) The method of embodiment 84, wherein the at least one mutation that results in enhanced binding of the protein to the FcγR is selected from the group consisting of S239D/I332E, S298A/E333A/K334A, F243L/R292P/Y300L, F243L/R292P/Y300L/P396L, F243L/R292P/Y300L/V305I/P396L and G236A/S239D/I332E, wherein residue numbering is according to the EU index.
86) The method of any one of embodiments 82-85, wherein the FcγR is FcγRI, FcγRIIA, FcγRIIB or FcγRIII, or any combination thereof.
87) The method of any one of embodiments 61-86, wherein the Ig constant region of the fragment of the Ig constant region comprises at least one mutation that modulates a half-life of the protein.
88) The method of embodiment 87, wherein the at least one mutation that modulates the half-life of the protein is selected from the group consisting of H435A, P257I/N434H, D376V/N434H, M252Y/S254T/T256E/H433K/N434F, T308P/N434A and H435R, wherein residue numbering is according to the EU index.
89) The method of any one of the embodiments 61-88, wherein the isolated protein comprises at least one mutation in a CH3 domain of the Ig constant region.
90) The method of embodiment 89, wherein the at least one mutation in the CH3 domain of the Ig constant region is selected from the group consisting of T350V, L351Y, F405A, Y407V, T366Y, T366W, F405W, T394W, T394S, Y407T, Y407A, T366S/L368A/Y407V, L351Y/F405A/Y407V, T366I/K392M/T394W, F405A/Y407V, T366L/K392M/T394W, L351Y/Y407A, T366A/K409F, L351Y/Y407A, T366V/K409F, T366A/K409F, T350V/L351Y/F405A/Y407V and T350V/T366L/K392L/T394W, wherein residue numbering is according to the EU index.
91) The method of any one of embodiments 1-54, wherein the isolated protein is a chimeric antigen receptor (CAR).
92) The method of embodiment 91, wherein the CAR comprises

a) an extracellular domain comprising the antigen binding domain that binds CD33 as defined in any one of embodiments 1-54;
b) a transmembrane domain; and
c) an intracellular signaling domain optionally comprising at least one co-stimulatory domain.

93) The method of embodiment 91 or 92, wherein the CAR further comprises a CD8a-hinge region.
94) The method of any one of embodiments 91-93, wherein

a) the transmembrane domain comprises a CD8a transmembrane region (CD8a-TM) polypeptide; and
b) the intracellular signaling domain comprises a co-stimulatory domain comprising a TNF receptor superfamily member 9 (CD137) component and a primary signaling domain comprising a T-cell surface glycoprotein CD3 zeta chain (CD3z) component.

95) The method of any one of embodiments 91-93, wherein

a) the CD8a-hinge region comprises an amino acid sequence that is at least 90% identical to the amino acid sequence of SEQ ID NO: 157;
b) the transmembrane domain comprises an amino acid sequence that is at least 90% identical to the amino acid sequence of SEQ ID NO: 162; and/or
c) the intracellular signaling domain comprises a co-stimulatory domain having an amino acid sequence that is at least 90% identical to the amino acid sequence of SEQ ID NO: 163 and a primary signaling domain having

an amino acid sequence that is at least 90% identical to the amino acid sequence of SEQ ID NO: 164.

96) The method of embodiment 95, wherein the intracellular signaling domain comprises a polypeptide component selected from the group consisting of a TNF receptor superfamily member 9 (CD137) component, a T-cell surface glycoprotein CD3 zeta chain (CD3z) component, a cluster of differentiation (CD27) component, a cluster of differentiation superfamily member component, or any combinations thereof.
97) A method of treating a CD33 expressing cancer in a subject, comprising administering a therapeutically effective amount of an isolated multispecific protein to the subject for a time sufficient to treat the CD33 expressing cancer, wherein the isolated multispecific protein comprising a first antigen binding domain that binds CD33 and a second antigen binding domain that binds a lymphocyte antigen.
98) The method of embodiment 97, wherein the lymphocyte antigen is a T cell antigen.
99) The method of embodiment 97, wherein the T cell antigen is a $CD8^+$ T cell antigen.
100) The method of embodiment 97, wherein the lymphocyte antigen is a NK cell antigen.
101) The method of any one of embodiments 97-100, wherein the lymphocyte antigen is CD3, CD3 epsilon (CD3ε), CD8, KI2L4, NKG2E, NKG2D, NKG2F, BTNL3, CD186, BTNL8, PD-1, CD195, TRGV9, or NKG2C.
102) The method of embodiment 101, wherein the lymphocyte antigen is CD3ε.
103) The method of any one of embodiments 97-102, wherein the first antigen binding domain that binds CD33 and/or the second antigen binding domain that binds the lymphocyte antigen comprise a scFv, a (scFv)z, a Fv, a Fab, a $F(ab')_2$, a Fd, a dAb or a VHH.
104) The method of embodiment 103, wherein the first antigen binding domain that binds CD33 and/or the second antigen binding domain that binds the lymphocyte antigen comprise the Fab.
105) The method of embodiment 103, wherein the first antigen binding domain that binds CD33 and/or the second antigen binding domain that binds the lymphocyte antigen comprise the VHH.
106) The method of embodiment 103, wherein the first antigen binding domain that binds CD33 and/or the second antigen binding domain that binds the lymphocyte antigen comprise the scFv.
107) The method of embodiment 106, wherein the scFv comprises, from the N- to C-terminus, a VH, a first linker (L1) and a VL (VH-L1-VL) or the VL, the L1 and the VH (VL-L1-VH).
108) The method of embodiment 107, wherein the L1 comprises

a) about 5-50 amino acids;
b) about 5-40 amino acids;
c) about 10-30 amino acids; or
d) about 10-20 amino acids.

109) The method of embodiment 108, wherein the L1 comprises the amino acid sequence of any one of SEQ ID NOs: 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, or 140.
110) The method of embodiment 109, wherein the L1 comprises the amino acid sequence of SEQ ID NO: 108.
111) The method of any one of embodiments 97-110, wherein the first antigen binding domain that binds CD33 comprises the HCDR1 of any one of SEQ ID NOs: 1, 2, 3, 4, or 5, the HCDR2 of any one of SEQ ID NOs: 6, 7, 8, 9, 10, or 11, and the HCDR3 of any one of SEQ ID NOs: 12, 13, 14, 15, 16, or 17.
112) The method of any one of 97-111, wherein the first antigen binding domain that binds CD33 comprises the HCDR1, the HCDR2, the HCDR3 of

a) SEQ ID NOs: 1, 6, and 12, respectively;
b) SEQ ID NOs: 2, 7, and 13, respectively;
c) SEQ ID NOs: 1, 8, and 14, respectively;
d) SEQ ID NOs: 3, 9, and 13, respectively;
e) SEQ ID NOs: 4, 10, and 15, respectively;
f) SEQ ID NOs: 5, 11, and 16, respectively; or
g) SEQ ID NOs: 5, 11, and 17, respectively.

113) The method of any one of embodiments 97-112, wherein the first antigen binding domain that binds CD33 comprises the VH of any one of SEQ ID NOs: 18, 19, 20, 21, 22, 23, 24, 25, 26, or 27.
114) The method of any one of embodiments 97-110, wherein the first antigen binding domain that binds CD33 comprises the HCDR1 of SEQ ID NOs: 28, 29, 30, 31, 32, 33, 34, or 35, the HCDR2 of SEQ ID NOs: 36, 37, 38, 39, 40, 41, 42, 43, or 44, the HCDR3 of SEQ ID NOs: 45, 46, 47, 48, 49, 50, or 51, the LCDR1 of SEQ ID NOs: 62, 63, 64, 65, 66, 67, or 68, the LCDR2 of SEQ ID NOs: 69, 70, 71, 72, 73, or 74, and the LCDR3 of SEQ ID NOs: 75,

76, 77, 78, 79, or 80.

115) The method of any one of embodiments 97-110 and 114, wherein the first antigen binding domain that binds CD33 comprises the HCDR1, the HCDR2, the HCDR3, the LCDR1, the LCDR2 and the LCDR3 of

a) SEQ ID NOs: 28, 36, 45, 62, 69, and 75, respectively;
b) SEQ ID NOs: 29, 37, 46, 63, 70, and 76, respectively;
c) SEQ ID NOs: 30, 38, 47, 64, 71, and 77, respectively;
d) SEQ ID NOs: 31, 39, 48, 65, 72, and 78, respectively;
e) SEQ ID NOs: 32, 40, 49, 66, 70, and 76, respectively;
f) SEQ ID NOs: 33, 41, 50, 67, 73, and 79, respectively;
g) SEQ ID NOs: 34, 42, 51, 68, 74, and 80, respectively;
h) SEQ ID NOs: 34, 43, 51, 68, 74, and 80, respectively;
i) SEQ ID NOs: 34, 44, 51, 68, 74, and 80, respectively; or
j) SEQ ID NOs: 35, 42, 51, 68, 74, and 80, respectively.

116) The method of any one of embodiments 97-110, 114, and 115, wherein the antigen binding domain that binds CD33 comprises the VH of SEQ ID NOs: 52, 53, 54, 55, 56, 57, 58, 59, 60, or 61 and the VL of SEQ ID NOs: 81, 82, 83, 84, 85, 86, 87, or 88.

117) The method of any one of embodiments 97-110 and 114-116, wherein the first antigen binding domain that binds CD33 comprises

a) the VH of SEQ ID NO: 52 and the VL of SEQ ID NO: 81;
b) the VH of SEQ ID NO: 53 and the VL of SEQ ID NO: 82;
c) the VH of SEQ ID NO: 54 and the VL of SEQ ID NO: 83;
d) the VH of SEQ ID NO: 55 and the VL of SEQ ID NO: 84;
e) the VH of SEQ ID NO: 56 and the VL of SEQ ID NO: 85;
f) the VH of SEQ ID NO: 57 and the VL of SEQ ID NO: 86;
g) the VH of SEQ ID NO: 58 and the VL of SEQ ID NO: 87;
h) the VH of SEQ ID NO: 59 and the VL of SEQ ID NO: 87;
i) the VH of SEQ ID NO: 60 and the VL of SEQ ID NO: 87; or
j) the VH of SEQ ID NO: 61 and the VL of SEQ ID NO: 88.

118) The method of any one of embodiments 97-110, wherein the first antigen binding domain that binds CD33 comprises the HCDR1 of SEQ ID NOs: 33, 89, 167, 172 or 176, the HCDR2 of SEQ ID NOs: 90, 91, 168, 173, or 177, the HCDR3 of SEQ ID NOs: 92, 93, 94, 169, 174, 178, or 180, the LCDR1 of SEQ ID NOs: 98, 99, 100, 182, 186, 189, 193, 197, or 201, the LCDR2 of SEQ ID NOs: 101, 102, 103, 183, 187, 190, 194, or 198, and the LCDR3 of SEQ ID NO: 104, 184, 191, 195, or 199.

119) The method of any one of embodiments 97-110 and 118, wherein the first antigen binding domain that binds CD33 comprises the HCDR1, the HCDR2, the HCDR3, the LCDR1, the LCDR2 and the LCDR3 of

a) SEQ ID NOs: 89, 90, 92, 98, 101, and 104, respectively;
b) SEQ ID NOs: 89, 90, 93, 99, 102, and 104, respectively;
c) SEQ ID NOs: 33, 91, 94, 100, 103, and 104, respectively;
d) SEQ ID NOs: 167, 168, 169, 182, 183, and 184, respectively;
e) SEQ ID NOs: 33, 91, 94, 186, 187, and 104, respectively;
f) SEQ ID NOs: 172, 173, 174, 189, 190, and 191, respectively;
g) SEQ ID NOs: 176, 177, 178, 193, 194, and 195, respectively;
h) SEQ ID NOs: 89, 90, 180, 197, 198, and 199, respectively; or
i) SEQ ID NOs: 89, 90, 93, 201, 102, and 104, respectively.

120) The method of any one of embodiments 97-110, 118 and 119, wherein the antigen binding domain that binds CD33 comprises the VH of SEQ ID NOs: 95, 96, 97, 170, 171, 175, 179, 181, or 96, and the VL of SEQ ID NOs 105, 106, 107, 185, 188, 192, 196, 200, or 202.

121) The method of any one of embodiments 97-110 and 118-120, wherein the first antigen binding domain that binds CD33 comprises

a) the VH of SEQ ID NO: 95 and the VL of SEQ ID NO: 105;
b) the VH of SEQ ID NO: 96 and the VL of SEQ ID NO: 106;

c) the VH of SEQ ID NO: 97 and the VL of SEQ ID NO: 107;
d) the VH of SEQ ID NO: 170 and the VL of SEQ ID NO: 185;
e) the VH of SEQ ID NO: 171 and the VL of SEQ ID NO: 188;
f) the VH of SEQ ID NO: 175 and the VL of SEQ ID NO: 192;
g) the VH of SEQ ID NO: 179 and the VL of SEQ ID NO: 196;
h) the VH of SEQ ID NO: 181 and the VL of SEQ ID NO: 200; or
i) the VH of SEQ ID NO: 96 and the VL of SEQ ID NO: 202.

122) The method of any one of embodiments 97-121, wherein the second antigen binding domain that binds the lymphocyte antigen comprises

a) the HCDR1 of SEQ ID NO: 141, the HCDR2 of SEQ ID NO: 142, the HCDR3 of SEQ ID NO: 143, the LCDR1 of SEQ ID NO: 144, the LCDR2 of SEQ ID NO: 145 and the LCDR3 of SEQ ID NO: 146;
b) the VH of SEQ ID NO: 147 and the VL of SEQ ID NO: 148;
c) the HCDR1 of SEQ ID NO: 149, the HCDR2 of SEQ ID NO: 150, the HCDR3 of SEQ ID NO: 151, the LCDR1 of SEQ ID NO: 152, the LCDR2 of SEQ ID NO: 153 and the LCDR3 of SEQ ID NO: 154; or
d) the VH of SEQ ID NO: 155 and the VL of SEQ ID NO: 156.

123) The method of any one of embodiments 95-119, wherein the antigen binding domain that binds TRGV9 comprises:

a) a heavy chain complementarity determining region 1 (HCDR1) of SEQ ID NO: 236, a HCDR2 of SEQ ID NO: 237, a HCDR3 of SEQ ID NO: 238, a light chain complementarity determining region 1 (LCDR1) of SEQ ID NO: 239, a LCDR2 of SEQ ID NO: 240 and a LCDR3 of SEQ ID NO: 241;
b) a heavy chain complementarity determining region 1 (HCDR1) of SEQ ID NO: 236, a HCDR2 of SEQ ID NO: 237, a HCDR3 of SEQ ID NO: 242, a light chain complementarity determining region 1 (LCDR1) of SEQ ID NO: 239, a LCDR2 of SEQ ID NO: 240 and a LCDR3 of SEQ ID NO: 241;
c) a heavy chain complementarity determining region 1 (HCDR1) of SEQ ID NO: 236, a HCDR2 of SEQ ID NO: 237, a HCDR3 of SEQ ID NO: 243, a light chain complementarity determining region 1 (LCDR1) of SEQ ID NO: 239, a LCDR2 of SEQ ID NO: 240 and a LCDR3 of SEQ ID NO: 241;
d) a heavy chain complementarity determining region 1 (HCDR1) of SEQ ID NO: 236, a HCDR2 of SEQ ID NO: 237, a HCDR3 of SEQ ID NO: 244, a light chain complementarity determining region 1 (LCDR1) of SEQ ID NO: 239, a LCDR2 of SEQ ID NO: 240 and a LCDR3 of SEQ ID NO: 241;
e) the VH of SEQ ID NO: 245 and the VL of SEQ ID NO: 249;
f) the VH of SEQ ID NO: 246 and the VL of SEQ ID NO: 249;
g) the VH of SEQ ID NO: 247 and the VL of SEQ ID NO: 249; or
h) the VH of SEQ ID NO: 248 and the VL of SEQ ID NO: 249.

124) The method of any one of embodiments 97-123, wherein the first antigen binding domain that binds CD33 is conjugated to a first immunoglobulin (Ig) constant region or a fragment of the first Ig constant region and/or the second antigen binding domain that binds the lymphocyte antigen is conjugated to a second immunoglobulin (Ig) constant region or a fragment of the second Ig constant region.
125) The method of embodiment 124, further comprising a second linker (L2) between the first antigen binding domain that binds CD33 and the first Ig constant region or the fragment of the first Ig constant region and the second antigen binding domain that binds the lymphocyte antigen and the second Ig constant region or the fragment of the second Ig constant region.
126) The method of embodiment 125, wherein the L2 comprises the amino acid sequence of any one of SEQ ID NOs: 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, or 140.
127) The method of any one of embodiments 124-136, wherein the first Ig constant region or the fragment of the first Ig constant region and the second Ig constant region or the fragment of the second Ig constant region is an IgG1, an IgG2, and IgG3 or an IgG4 isotype.
128) The method of any one of embodiments 124-127, wherein the first Ig constant region or the fragment of the first Ig constant region and the second Ig constant region or the fragment of the second Ig constant region comprises at least one mutation that results in reduced binding of the multispecific protein to a FcγR.
129) The method of embodiment 128, wherein the at least one mutation that results in reduced binding of the multispecific protein to the FcγR is selected from the group consisting of F234A/L235A, L234A/L235A, L234A/L235A/D265S, V234A/G237A/ P238S/H268A/V309L/A330S/P331S, F234A/L235A, S228P/F234A/ L235A,

N297A, V234A/G237A, K214T/E233P/ L234V/L235A/G236-deleted/A327G/P331A/D365E/L358M, H268Q/V309L/A330S/P331S, S267E/L328F, L234F/L235E/D265A, L234A/L235A/G237A/P238S/H268A/A330S/P331S, S228P/F234A/L235A/G237A/P238S and S228P/F234A/L235A/G236-deleted/G237A/P238S, wherein residue numbering is according to the EU index.
130) The method of any one of embodiments 124-127, wherein the first Ig constant region or the fragment of the first Ig constant region and the second Ig constant region or the fragment of the second Ig constant region comprises at least one mutation that results in enhanced binding of the multispecific protein to a Fcy receptor (FcyR).
131) The method of embodiment 130, wherein the at least one mutation that results in enhanced binding of the multispecific protein to the FcyR is selected from the group consisting of S239D/1332E, S298A/E333A/K334A, F243L/R292P/Y300L, F243L/R292P/Y300L/P396L, F243L/R292P/Y300L/V305I/P396L and G236A/S239D/1332E, wherein residue numbering is according to the EU index.
132) The method of any one of embodiments 128-131, wherein the FcyR is FcyRI, FcyRIIA, FcyRIIB or FcγRIII, or any combination thereof.
133) The method of any one of embodiments 124-132, wherein the first Ig constant region or the fragment of the first Ig constant region and the second Ig constant region or the fragment of the second Ig constant region comprises at least one mutation that modulates a half-life of the multispecific protein.
134) The method of embodiment 133, wherein the at least one mutation that modulates the half-life of the multispecific protein is selected from the group consisting of H435A, P257I/N434H, D376V/N434H, M252Y/S254T/T256E/H433K/N434F, T308P/N434A and H435R, wherein residue numbering is according to the EU index.
135) The method of any one of the embodiments 124-134, comprising at least one mutation in a CH3 domain of the first Ig constant region or in a CH3 domain of the fragment of the first Ig constant region and/or at least one mutation in a CH3 domain of the second Ig constant region or in a CH3 domain of the fragment of the second Ig constant region.
136) The method of embodiment 135, wherein the at least one mutation in a CH3 domain of the first Ig constant region or in a CH3 domain of the fragment of the first Ig constant region and/or at least one mutation in a CH3 domain of the second Ig constant region or in a CH3 domain of the fragment of the second Ig constant region is selected from the group consisting of T350V, L351Y, F405A,Y407V, T366Y, T366W, F405W, T394W, T394S, Y407T, Y407A, T366S/L368A/Y407V, L351Y/F405A/Y407V, T366I/K392M/T394W, F405A/Y407V, T366L/K392M/T394W, L351Y/Y407A, T366A/K409F, L351Y/Y407A, T366V/K409F, T366A/K409F, T350V/L351Y/F405A/Y407V and T350V/T366L/K392L/T394W, wherein residue numbering is according to the EU index.
137) The method of any one of embodiments 124-136, wherein the first Ig constant region or the fragment of the first Ig constant region and the second Ig constant region or the fragment of the second Ig constant region comprise the following mutations:

a) L235A_L235A_D265S_T350V_L351Y_F405A_Y407V in the first Ig constant region and L235A_L235A_D265S_D265S_T350V_T366L_K392L_T394W in the second Ig constant region; or
b) L235A_L235A_D265S_T350V_T366L_K392L_T394W in the first Ig constant region and L235A_L235A_D265S_T350V_L351Y_F405A_Y407V in the second Ig constant region.

138) A method of treating a CD33 expressing cancer in a subject, comprising administering a therapeutically effective amount of an immunoconjugate to the subject for a time sufficient to treat the CD33 expressing cancer, wherein the immunoconjugate comprises an isolated protein as defined in any one of embodiments 1-137 conjugated to an agent.
139) The method of embodiment 138, wherein the agent is a radioactive agent.
140) The immunoconjugate of embodiment 139, wherein the radioactive agent comprises a radiometal ion.
141) The immunoconjugate of embodiment 140, wherein the radiometal ion is $^{32}$P, $^{47}$Sc, $^{67}$Cu, $^{77}$As, $^{89}$Sr, $^{90}$Y, $^{99}$Tc, $^{105}$Rh, $^{109}$Pd, $^{111}$Ag, $^{131}$I, $^{153}$Sm, $^{159}$Gd, $^{165}$Dy, $^{166}$Ho, $^{169}$Er, $^{177}$Lu, $^{186}$Re, $^{188}$Re, $^{194}$Ir, $^{198}$Au, $^{199}$Au, $^{211}$At, $^{212}$Pb, $^{212}$Bi, $^{213}$Bi, $^{223}$Ra, $^{225}$Ac, $^{255}$Fm, $^{227}$Th, $^{62}$Cu, $^{64}$Cu, $^{67}$Ga, $^{68}$Ga, $^{86}$Y, $^{89}$Zr, or $^{111}$In.
142) The immunoconjugate of embodiment 140, wherein the radiometal ion is $^{225}$Ac, $^{111}$In or $^{89}$Zr.
143) The immunoconjugate of embodiment 140, wherein the radiometal ion is $^{225}$Ac.
144) The immunoconjugate of any one of embodiments 140 to 143, wherein the radiometal ion is conjugated to a chelating moiety.
145) The immunoconjugate of embodiment 144, wherein the chelating moiety comprises a macrocycle having the structure of formula (I):

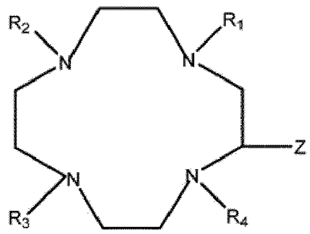

formula (I),

wherein each of $R_1$, $R_2$, $R_3$ and $R_4$ is independently CHQCOzX, wherein
Q is independently hydrogen, $C_1$-$C_4$ alkyl or ($C_1$-$C_2$ alkyl) phenyl, and
X is independently hydrogen, benzyl, $C_1$-$C_4$ alkyl; and
Z is $(CH2)_nY$, wherein
n is 1-10, and
Y is an electrophilic or nucleophilic moiety covalently linked to the second click reaction partner; alternatively, Z is hydrogen; and
each of $R_1$, $R_2$, $R_3$ and $R_4$ is independently CHQCOzX, wherein
Q is independently hydrogen, $C_1$-$C_4$ alkyl or ($C_1$-$C_2$ alkyl) phenyl, and
X is independently hydrogen, benzyl, $C_1$-$C_4$ alkyl, or an electrophilic or nucleophilic moiety covalently linked to the second click reaction partner;
alternatively, the chelant comprises an open chain ligand.

146) The immunoconjugate of embodiment 144, wherein the chelating moiety comprises a macrocycle having the structure of formula (II):

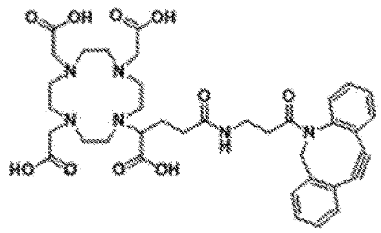

or a structure of formula (III):

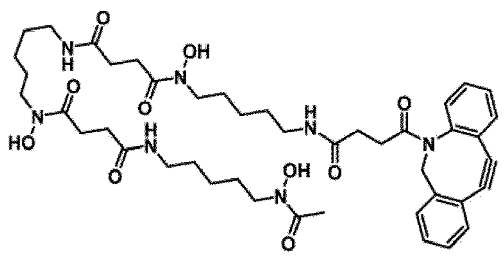

147) An immunoconjugate of formula (IV),

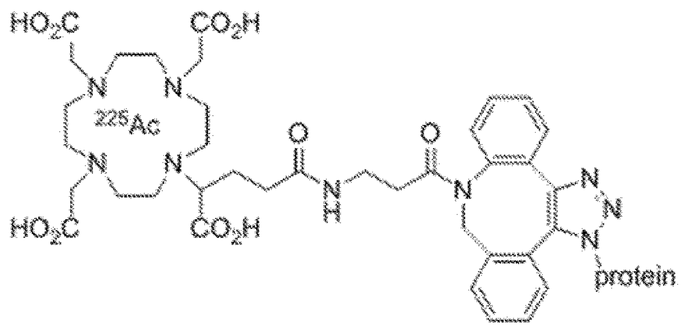

formula (IV)

wherein the protein is the isolated protein as defined in any one of embodiments 1-96.

148) An immunoconjugate of formula (V),

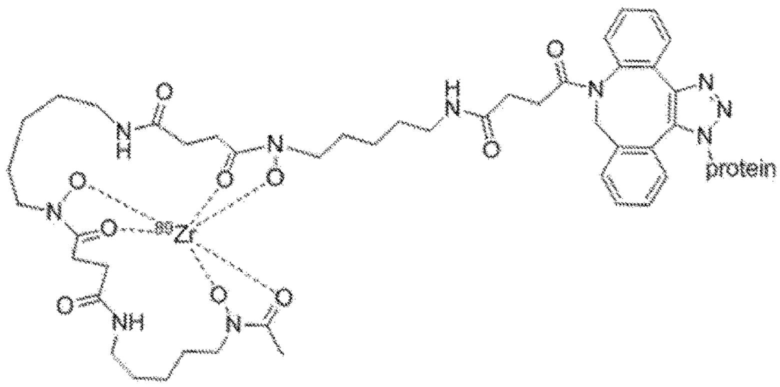

formula (V)

wherein the protein is the isolated protein as defined in any one of embodiments 1-96.

149) The method of any one of embodiments 1 to 148, wherein the method is effective to reduce the amount of CD33 expressing cells in a subject, wherein the CD33 expressing cells are cancerous cells or wherein the CD33 expressing cells are non-cancerous cells.

150) The method of any one of embodiments 1 to 149, wherein the method is effective to prevent establishment of a CD33 expressing cancer in the subject.

151) The method of any one of embodiments 148-150, wherein the CD33 expressing cancer is a hematologic cancer.

152) The method of embodiment 151, wherein the hematologic cancer is a leukemia, a lymphoma, or a multiple myeloma.

153) The method of embodiment 151, wherein the hematologic cancer is acute myeloid leukemia (AML), myelodysplastic syndrome (MDS), acute lymphocytic leukemia (ALL), diffuse large B-cell lymphoma (DLBCL), chronic myeloid leukemia (CML) or blastic plasmacytoid dendritic cell neoplasm (DPDCN).

154) The method of any one of embodiments 148-153, wherein the isolated protein or the isolated multispecific protein is administered in combination with a second therapeutic agent.

155) The method of embodiment 154, wherein the second therapeutic agent is surgery, chemotherapy, androgen deprivation therapy or radiation, or any combination thereof.

156) A method of treating a CD33 expressing cancer in a subject, the method comprising administering to the subject a means for targeting a therapeutic agent to a CD33 expressing cancer cell in the subject.

157) A method of reducing the amount of CD33 expressing tumor cells in a subject, the method comprising administering to the subject a means for targeting a therapeutic agent to one or more of the CD33 expressing tumor cells in the subject.

158) A pharmaceutical composition comprising a means for preventing establishment of a CD33 expressing cancer in a subject, the method comprising administering to the subject a means for targeting a therapeutic agent to a CD33 expressing cell in the subject.

159) A pharmaceutical composition comprising a means for treating a noncancerous condition in a subject at risk of developing a CD33 expressing cancer, the method comprising administering to the subject a means for targeting a therapeutic agent to a CD33 expressing cell in the subject.

List of numbered embodiments (E):

1) A chimeric antigen receptor (CAR) comprising:

a) an extracellular domain comprising an antigen binding domain that binds CD33;
b) a transmembrane domain; and
c) an intracellular signaling domain optionally comprising at least one co-stimulatory domain.

2) The CAR of embodiment 1, further comprising a CD8a-hinge region.
3) The CAR of embodiment 1 or 2, wherein

a) the transmembrane domain comprises a CD8a transmembrane region (CD8a-TM) polypeptide; and
b) the intracellular signaling domain comprises a co-stimulatory domain comprising a TNF receptor superfamily member 9 (CD137) component and a primary signaling domain comprising a T-cell surface glycoprotein CD3 zeta chain (CD3z) component.

4) The CAR of embodiment 2 or 3, wherein

a) the CD8a-hinge region comprises an amino acid sequence that is at least 90% identical to the amino acid sequence of SEQ ID NO: 157;
b) the transmembrane domain comprises an amino acid sequence that is at least 90% identical to the amino acid sequence of SEQ ID NO: 162; and/or
c) the intracellular signaling domain comprises a co-stimulatory domain having an amino acid sequence that is at least 90% identical to the amino acid sequence of SEQ ID NO: 163, and a primary signaling domain having an amino acid sequence that is at least 90% identical to the amino acid sequence of SEQ ID NO: 164.

5) The CAR of any one of embodiments 1-4, wherein the antigen binding domain that binds CD33 comprises the HCDR1 of any one of SEQ ID NOs: 1, 2, 3, 4, or 5, the HCDR2 of any one of SEQ ID NOs: 6, 7, 8, 9, 10, or 11, and the HCDR3 of any one of SEQ ID NOs: 12, 13, 14, 15, 16, or 17.
6) The CAR of any one of 1-5, wherein the antigen binding domain that binds CD33 comprises the HCDR1, the HCDR1, the HCDR3 of

a) SEQ ID NOs: 1, 6, and 12, respectively;
b) SEQ ID NOs: 2, 7, and 13, respectively;
c) SEQ ID NOs: 1, 8, and 14, respectively;
d) SEQ ID NOs: 3, 9, and 13, respectively;
e) SEQ ID NOs: 4, 10, and 15, respectively;
f) SEQ ID NOs: 5, 11, and 16, respectively; or
g) SEQ ID NOs: 5, 11, and 17, respectively.

7) The CAR of any one of embodiments 1-6, wherein the antigen binding domain that binds CD33 comprises the VH of any one of SEQ ID NOs: 18, 19, 20, 21, 22, 23, 24, 25, 26, or 27.
8) The CAR of any one of embodiments 1-4, wherein the antigen binding domain that binds CD33 comprises the HCDR1 of SEQ ID NOs: 28, 29, 30, 31, 32, 33, 34, or 35, the HCDR2 of SEQ ID NOs: 36, 37, 38, 39, 40, 41, 42, 43, or 44, the HCDR3 of SEQ ID NOs: 45, 46, 47, 48, 49, 50, or 51, the LCDR1 of SEQ ID NOs: 62, 63, 64, 65, 66, 67, or 68, the LCDR2 of SEQ ID NOs: 69, 70, 71, 72, 73, or 74, and the LCDR3 of SEQ ID NOs: 75, 76, 77, 78, 79, or 80.
9) The CAR of any one of embodiments 1-4 and 8, wherein the antigen binding domain that binds CD33 comprises the HCDR1, the HCDR2, the HCDR3, the LCDR1, the LCDR2 and the LCDR3 of

a) SEQ ID NOs: 28, 36, 45, 62, 69, and 75, respectively;
b) SEQ ID NOs: 29, 37, 46, 63, 70, and 76, respectively;
c) SEQ ID NOs: 30, 38, 47, 64, 71, and 77, respectively;
d) SEQ ID NOs: 31, 39, 48, 65, 72, and 78, respectively;
e) SEQ ID NOs: 32, 40, 49, 66, 70, and 76, respectively;
f) SEQ ID NOs: 33, 41, 50, 67, 73, and 79, respectively;
g) SEQ ID NOs: 34, 42, 51, 68, 74, and 80, respectively;

h) SEQ ID NOs: 34, 43, 51, 68, 74, and 80, respectively;
i) SEQ ID NOs: 34, 44, 51, 68, 74, and 80, respectively; or
j) SEQ ID NOs: 35, 42, 51, 68, 74, and 80, respectively.

10) The CAR of any one of embodiments 1-4, 8, and 9, wherein the antigen binding domain that binds CD33 comprises the VH of SEQ ID NOs: 52, 53, 54, 55, 56, 57, 58, 59, 60, or 61 and the VL of SEQ ID NOs: 81, 82, 83, 84, 85, 86, 87, or 88.
11) The CAR of any one of embodiments 1-4 and 8-10, wherein the antigen binding domain that binds CD33 comprises

a) the VH of SEQ ID NO: 52 and the VL of SEQ ID NO: 81;
b) the VH of SEQ ID NO: 53 and the VL of SEQ ID NO: 82;
c) the VH of SEQ ID NO: 54 and the VL of SEQ ID NO: 83;
d) the VH of SEQ ID NO: 55 and the VL of SEQ ID NO: 84;
e) the VH of SEQ ID NO: 56 and the VL of SEQ ID NO: 85;
f) the VH of SEQ ID NO: 57 and the VL of SEQ ID NO: 86;
g) the VH of SEQ ID NO: 58 and the VL of SEQ ID NO: 87;
h) the VH of SEQ ID NO: 59 and the VL of SEQ ID NO: 87;
i) the VH of SEQ ID NO: 60 and the VL of SEQ ID NO: 87; or
j) the VH of SEQ ID NO: 61 and the VL of SEQ ID NO: 88.

12) The CAR of any one of embodiments 1-4, wherein the antigen binding domain that binds CD33 comprises the HCDR1 of SEQ ID NOs: 33, 89, 167, 172 or 176, the HCDR2 of SEQ ID NOs: 90, 91, 168, 173, or 177, the HCDR3 of SEQ ID NOs: 92, 93, 94, 169, 174, 178, or 180, the LCDR1 of SEQ ID NOs: 98, 99, 100, 182, 186, 189, 193, 197, the LCDR2 of SEQ ID NOs: 101, 102, 103, 183, 187, 190, 194, or 198, and the LCDR3 of SEQ ID NO: 104, 184, 191, 195, or 199.
13) The CAR of any one of embodiments 1-4 and 12, wherein the antigen binding domain that binds CD33 comprises the HCDR1, the HCDR2, the HCDR3, the LCDR1, the LCDR2 and the LCDR3 of

a) SEQ ID NOs: 89, 90, 92, 98, 101, and 104, respectively;
b) SEQ ID NOs: 89, 90, 93, 99, 102, and 104, respectively;
c) SEQ ID NOs: 33, 91, 94, 100, 103, and 104, respectively;
d) SEQ ID NOs: 167, 168, 169, 182, 183, and 184, respectively;
e) SEQ ID NOs: 33, 91, 94, 186, 187, and 104, respectively;
f) SEQ ID NOs: 172, 173, 174, 189, 190, and 191, respectively;
g) SEQ ID NOs: 176, 177, 178, 193, 194, and 195, respectively;
h) SEQ ID NOs: 89, 90, 180, 197, 198, and 199, respectively; or
i) SEQ ID NOs: 89, 90, 93, 201, 102, and 104, respectively.

14) The CAR of any one of embodiments 1-4, 12 and 13, wherein the antigen binding domain that binds CD33 comprises the VH of SEQ ID NOs: 95, 96, 97, 170, 171, 175, 179, or 181 and the VL of SEQ ID NOs 105, 106, 107, 185, 188, 192, 196, 200, or 202.
15) The CAR of any one of embodiments 1-4 and 12-14, wherein the antigen binding domain that binds CD33 comprises

a) the VH of SEQ ID NO: 95 and the VL of SEQ ID NO: 105;
b) the VH of SEQ ID NO: 96 and the VL of SEQ ID NO: 106;
c) the VH of SEQ ID NO: 97 and the VL of SEQ ID NO: 107;
d) the VH of SEQ ID NO: 170 and the VL of SEQ ID NO: 185;
e) the VH of SEQ ID NO: 171 and the VL of SEQ ID NO: 188;
f) the VH of SEQ ID NO: 175 and the VL of SEQ ID NO: 192;
g) the VH of SEQ ID NO: 179 and the VL of SEQ ID NO: 196;
h) the VH of SEQ ID NO: 181 and the VL of SEQ ID NO: 200; or
i) the VH of SEQ ID NO: 96 and the VL of SEQ ID NO: 202.

16) The CAR of any one of embodiments 1-15, wherein the antigen binding domain that binds CD33 is a VHH.
17) The CAR of any one of embodiments 1-16, wherein the antigen binding domain that binds CD33 is a scFv.
18) The CAR of embodiment 17, wherein the scFv comprises a first linker (L1) between the VL and the VH.
19) The CAR of embodiment 18, wherein the L1 comprises an amino acid sequence of any one of SEQ IDNOs:

108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, or 140.

20) The CAR of embodiment 19, wherein the L1 comprises the amino acid sequence of SEQ ID NO: 108.

21) The CAR of any of embodiments 1-20, wherein the extracellular domain comprising the antigen binding domain that binds CD33 further comprises a signal polypeptide.

22) The CAR of embodiment 21, wherein the signal polypeptide comprises the amino acid sequence of SEQ ID NO: 166.

23) The CAR of any one of embodiments 1-22, wherein the intracellular signaling domain comprises a polypeptide component selected from the group consisting of a TNF receptor superfamily member 9 (CD137) component, a T-cell surface glycoprotein CD3 zeta chain (CD3z) component, a cluster of differentiation (CD27) component, a cluster of differentiation superfamily member component, and a combination thereof.

24) The CAR of any one of embodiments 3-23, wherein the CD137 component comprises the amino acid sequence of SEQ ID NO: 163.

25) The CAR of any one of embodiments 3-24, wherein the CD3z component comprises an amino acid sequence of SEQ ID NO: 164.

26) The CAR of any one of embodiments 3-25, wherein the intracellular signaling domain comprises an amino acid sequence of SEQ ID NO: 165.

27) The CAR of any one of embodiments 3-26, wherein the CD8a-TM polypeptide comprises an amino acid sequence of SEQ ID NO: 162.

28) The CAR of any one of embodiments 3-27, wherein the CD8a-hinge region comprises an amino acid sequence of SEQ ID NO: 157.

29) The CAR of any one of embodiments 1-28, wherein the CAR is a multispecific protein.

30) The CAR of embodiment 29, wherein the multispecific protein is a bispecific protein.

31) The CAR of embodiment 30, wherein the multispecific protein is a trispecific protein.

32) The CAR of any one of embodiments 29-31, wherein the multispecific protein comprises an antigen binding domain that binds an antigen on a lymphocyte.

33) The CAR of embodiment 32, wherein the lymphocyte is a T cell.

34) The CAR of embodiment 32, wherein the T cell is a $CD8^+$ T cell

35) The CAR of embodiment 32, wherein the lymphocyte is a natural killer (NK) cell.

36) The CAR of any one of embodiments 29-35, wherein the multispecific protein comprises an antigen binding domain that binds CD3, CD3 epsilon (CD3ε), CD8, KI2L4, NKG2E, NKG2D, NKG2F, BTNL3, CD186, BTNL8, PD-1, CD195, TRGV9, orNKG2C.

37) The CAR of embodiment 36, wherein the multispecific protein comprises an antigen binding domain that binds CD3ε.

38) The CAR of embodiment 37, wherein the antigen binding domain that binds CD3ε comprises:

a) a heavy chain complementarity determining region 1 (HCDR1) of SEQ ID NO: 141, a HCDR2 of SEQ ID NO: 142, a HCDR3 of SEQ ID NO: 143, a light chain complementarity determining region 1 (LCDR1) of SEQ ID NO: 144, a LCDR2 of SEQ ID NO: 145 and a LCDR3 of SEQ ID NO: 146;

b) the VH of SEQ ID NO: 147 and the VL of SEQ ID NO: 148;

c) the HCDR1 of SEQ ID NO: 149, the HCDR2 of SEQ ID NO: 150, the HCDR3 of SEQ ID NO: 151, the LCDR1 of SEQ ID NO: 152, the LCDR2 of SEQ ID NO: 153 and the LCDR3 of SEQ ID NO: 154; or

d) the VH of SEQ ID NO: 155 and the VL of SEQ ID NO: 156.

39) The CAR of embodiment 36, wherein the multispecific protein comprises an antigen binding domain that binds TRGV9.

40) The CAR of embodiment 39, wherein the antigen binding domain that binds TRGV9 comprises:

a) a heavy chain complementarity determining region 1 (HCDR1) of SEQ ID NO: 236, a HCDR2 of SEQ ID NO: 237, a HCDR3 of SEQ ID NO: 238, a light chain complementarity determining region 1 (LCDR1) of SEQ ID NO: 239, a LCDR2 of SEQ ID NO: 240 and a LCDR3 of SEQ ID NO: 241;

b) a heavy chain complementarity determining region 1 (HCDR1) of SEQ ID NO: 236, a HCDR2 of SEQ ID NO: 237, a HCDR3 of SEQ ID NO: 242, a light chain complementarity determining region 1 (LCDR1) of SEQ ID NO: 239, a LCDR2 of SEQ ID NO: 240 and a LCDR3 of SEQ ID NO: 241;

c) a heavy chain complementarity determining region 1 (HCDR1) of SEQ ID NO: 236, a HCDR2 of SEQ ID NO: 237, a HCDR3 of SEQ ID NO: 243, a light chain complementarity determining region 1 (LCDR1) of SEQ ID NO: 239, a LCDR2 of SEQ ID NO: 240 and a LCDR3 of SEQ ID NO: 241;

d) a heavy chain complementarity determining region 1 (HCDR1) of SEQ ID NO: 236, a HCDR2 of SEQ ID NO:

237, a HCDR3 of SEQ ID NO: 244, a light chain complementarity determining region 1 (LCDR1) of SEQ ID NO: 239, a LCDR2 of SEQ ID NO: 240 and a LCDR3 of SEQ ID NO: 241;
e) the VH of SEQ ID NO: 245 and the VL of SEQ ID NO: 249;
f) the VH of SEQ ID NO: 246 and the VL of SEQ ID NO: 249;
g) the VH of SEQ ID NO: 247 and the VL of SEQ ID NO: 249; or
h) the VH of SEQ ID NO: 248 and the VL of SEQ ID NO: 249.

41) An isolated lymphocyte expressing the CAR of any one of embodiments 1-40.
42) The isolated lymphocyte of embodiment 41, wherein the lymphocyte is a T lymphocyte.
43) The isolated lymphocyte of embodiment 41, wherein the lymphocyte is a natural killer (NK) cell.
44) An isolated polynucleotide encoding the CAR of any one of embodiments 1-40.
45) A vector comprising the polynucleotide of embodiment 44.
46) A host cell comprising the polynucleotide of embodiment 44 or the vector of embodiment 45.
47) A pharmaceutical composition comprising the lymphocyte of any one of embodiments 41-43 and a pharmaceutically acceptable excipient.
48) A method of treating a subject having a CD33 expressing cancer, the method comprising: administering a therapeutically effective amount of the lymphocyte of any one of embodiments 41-43 to a subject in need thereof, whereby the lymphocyte mediates killing of the CD33 expressing cancer in the subject.
49) The method of embodiment 48, wherein the CD33 expressing cancer is a hematologic cancer.
50) The method of embodiment 49, wherein the CD33 expressing cancer is a leukemia, a lymphoma, or a multiple myeloma.
51) The method of embodiment 50, wherein the CD33 expressing cancer is acute myeloid leukemia (AML), myelodysplastic syndrome (MDS), acute lymphocytic leukemia (ALL), diffuse large B-cell lymphoma (DLBCL), chronic myeloid leukemia (CML) or blastic plasmacytoid dendritic cell neoplasm (DPDCN).
52) A method of targeted killing of a cancer cell, the method comprising: contacting the cancer cell with the lymphocyte of any one of embodiments 41-43, whereby the lymphocyte induces targeted killing of the cancer cell.
53) The method of embodiment 52, wherein the cancer cell is a hematologic cancer.
54) The method of embodiment 53, wherein the CD33 expressing cancer is a leukemia, a lymphoma, or a multiple myeloma.
55) The method of embodiment 54, wherein the CD33 expressing cancer is acute myeloid leukemia (AML), myelodysplastic syndrome (MDS), acute lymphocytic leukemia (ALL), diffuse large B-cell lymphoma (DLBCL), chronic myeloid leukemia (CML) or blastic plasmacytoid dendritic cell neoplasm (DPDCN).
56) A method of detecting the presence of a cancer in a subject, comprising:

a) contacting a cell sample obtained from the subject with the CAR of any one of embodiments 1 - 40, thereby forming a CAR-cell complex, and
b) detecting the CAR-cell complex, wherein detection of the CAR-cell complex is indicative of the presence of the cancer in the subject.

57) The method of embodiment 56, wherein the cancer cell is a hematologic cancer.
58) The method of embodiment 57, wherein the CD33 expressing cancer is a leukemia, a lymphoma, or a multiple myeloma.
59) The method of embodiment 58, wherein the CD33 expressing cancer is acute myeloid leukemia (AML), myelodysplastic syndrome (MDS), acute lymphocytic leukemia (ALL), diffuse large B-cell lymphoma (DLBCL), chronic myeloid leukemia (CML) or blastic plasmacytoid dendritic cell neoplasm (DPDCN).
60) A kit comprising the CAR of any one of embodiments 1-40.
61) A pharmaceutical composition comprising a means for treating a CD33 expressing cancer in a subject.
62) A pharmaceutical composition comprising a means for reducing the amount of CD33 expressing tumor cells in a subject.
63) A pharmaceutical composition comprising a means for preventing establishment of a CD33 expressing cancer in a subject.
64) A pharmaceutical composition comprising a means for treating a noncancerous condition in a subject at risk of developing a CD33 expressing cancer.
65) A method of treating a CD33 expressing cancer in a subject, the method comprising administering to the subject a means for targeting a therapeutic agent to a CD33 expressing cancer cell in the subject.
66) A method of reducing the amount of CD33 expressing tumor cells in a subject, the method comprising administering to the subject a means for targeting a therapeutic agent to one or more of the CD33 expressing tumor cells in the subject.

67) A pharmaceutical composition comprising a means for preventing establishment of a CD33 expressing cancer in a subject, the method comprising administering to the subject a means for targeting a therapeutic agent to a CD33 expressing cell in the subject.
68) A pharmaceutical composition comprising a means for treating a noncancerous condition in a subject at risk of developing a CD33 expressing cancer, the method comprising administering to the subject a means for targeting a therapeutic agent to a CD33 expressing cell in the subject.

List of numbered embodiments (F):

1) An isolated protein that binds CD33, wherein the isolated protein comprises

a) an HCDR1, an HCDR2 and an HCDR3 of a heavy chain variable region (VH) of SEQ ID NO: 53;
b) an HCDR1, an HCDR2 and an HCDR3 of a VH of SEQ ID NO: 56;
c) an HCDR1, an HCDR2 and an HCDR3 of a VH of SEQ ID NO: 59;
d) an HCDR1, an HCDR2 and an HCDR3 of a VH of SEQ ID NO: 262;
e) an HCDR1, an HCDR2 and an HCDR3 of a VH of SEQ ID NO: 263; or
f) an HCDR1, an HCDR2 and an HCDR3 of a VH of SEQ ID NO: 264.

2) The isolated protein of claim 1, comprising the HCDR1, the HCDR2, and the HCDR3 of

a) SEQ ID NOs: 29, 37, and 46, respectively;
b) SEQ ID NOs: 32, 40, and 49, respectively;
c) SEQ ID NOs: 34, 43, and 51, respectively;
d) SEQ ID NOs: 253, 256, and 259, respectively;
e) SEQ ID NOs: 254, 257, and 260, respectively; or
f) SEQ ID NOs: 255, 258, and 261, respectively.

3) An isolated protein that binds CD33, wherein the isolated protein comprises the HCDR1, the HCDR2, and the HCDR3 of

a) SEQ ID NOs: 29, 37, and 46, respectively;
b) SEQ ID NOs: 32, 40, and 49, respectively;
c) SEQ ID NOs: 34, 43, and 51, respectively;
d) SEQ ID NOs: 253, 256, and 259, respectively;
e) SEQ ID NOs: 254, 257, and 260, respectively; or
f) SEQ ID NOs: 255, 258, and 261, respectively.

4) The isolated protein of claim 1 or 2, wherein the isolated protein comprises

a) an HCDR1, an HCDR2 and an HCDR3 of a VH of SEQ ID NO: 53 and an LCDR1, an LCDR2 and an LCDR3 of a VL of SEQ ID NO: 82;
b) an HCDR1, an HCDR2 and an HCDR3 of a VH of SEQ ID NO: 56 and an LCDR1, an LCDR2 and an LCDR3 of a VL of SEQ ID NO: 85;
c) an HCDR1, an HCDR2 and an HCDR3 of a VH of SEQ ID NO: 59 and an LCDR1, an LCDR2 and an LCDR3 of a VL of SEQ ID NO: 87;
d) an HCDR1, an HCDR2 and an HCDR3 of a VH of SEQ ID NO: 262 and an LCDR1, an LCDR2 and an LCDR3 of a VL of SEQ ID NO: 271;
e) an HCDR1, an HCDR2 and an HCDR3 of a VH of SEQ ID NO: 263 and an LCDR1, an LCDR2 and an LCDR3 of a VL of SEQ ID NO: 272; or
f) an HCDR1, an HCDR2 and an HCDR3 of a VH of SEQ ID NO: 264 and an LCDR1, an LCDR2 and an LCDR3 of a VL of SEQ ID NO: 273;.

5) The isolated protein of claim 3 or 4, comprising the HCDR1, the HCDR2, the HCDR3, the LCDR1, the LCDR2 and the LCDR3 of

a) SEQ ID NOs: 29, 37, 46, 63, 70, and 76, respectively;

b) SEQ ID NOs: 32, 40, 49, 66, 70, and 76, respectively;
c) SEQ ID NOs: 34, 43, 51, 68, 74, and 80, respectively;
d) SEQ ID NOs: 253, 256, 259, 265, 74 and 269, respectively
e) SEQ ID NOs: 254, 257, 260, 66, 267 and 76, respectively; or
f) SEQ ID NOs: 255, 258, 261, 266, 268 and 270, respectively.

6) The isolated protein of any one of claims 1-5, wherein the isolated protein comprises the VH of SEQ ID NOs: 53, 56, 59, 262, 263, 264, 291, 292, 293, 294, 295, 296, 297, 298, 299, 300, 301, 302, 303, 304, 305, 306, 307, 308, 309, 310, 337, 338, 339, 340, 341, 342, 343, 344, 345, 346, 347, 348, 349, 350, 351, 352, 367, 368, 369, 370, 371, 372, 373, 374, 375, 376, 377, 378, 379, 380, 381, 382, or 382, and the VL of SEQ ID NOs: 82, 85, 87, 271, 272, 273, 311, 312, 313, 314, 315, 316, 317, 318, 319, 320, 321, 322, 323, 324, 325, 326, 327, 328, 329, 330, 331, 332, 333, 334, 335, 336, 353, 354, 355, 356, 357, 358, 359, 360, 361, 362, 363, 364, 365, 366, 383, 384, 385, 386, 387, 388, 389, 390, 391, 392, 393, 394, 395, 396, 397 or 398.

7) The isolated protein of claim 6, wherein the isolated protein comprises:

a) the VH of SEQ ID NO: 53 and the VL of SEQ ID NO: 82;
b) the VH of SEQ ID NO: 56 and the VL of SEQ ID NO: 85;
c) the VH of SEQ ID NO: 59 and the VL of SEQ ID NO: 87;
d) the VH of SEQ ID NO: 262 and the VL of SEQ ID NO: 271;
e) the VH of SEQ ID NO: 263 and the VL of SEQ ID NO: 272;
f) the VH of SEQ ID NO: 264 and the VL of SEQ ID NO: 273;
g) the VH of SEQ ID NO: 291 and the VL of SEQ ID NO: 271;
h) the VH of SEQ ID NO: 292 and the VL of SEQ ID NO: 271;
i) the VH of SEQ ID NO: 293 and the VL of SEQ ID NO: 271;
j) the VH of SEQ ID NO: 294 and the VL of SEQ ID NO: 271;
k) the VH of SEQ ID NO: 295 and the VL of SEQ ID NO: 271;
l) the VH of SEQ ID NO: 296 and the VL of SEQ ID NO: 271;
m) the VH of SEQ ID NO: 297 and the VL of SEQ ID NO: 271;
n) the VH of SEQ ID NO: 298 and the VL of SEQ ID NO: 271;
o) the VH of SEQ ID NO: 299 and the VL of SEQ ID NO: 271;
p) the VH of SEQ ID NO: 300 and the VL of SEQ ID NO: 271;
q) the VH of SEQ ID NO: 301 and the VL of SEQ ID NO: 271;
r) the VH of SEQ ID NO: 302 and the VL of SEQ ID NO: 271;
s) the VH of SEQ ID NO: 303 and the VL of SEQ ID NO: 271;
t) the VH of SEQ ID NO: 304 and the VL of SEQ ID NO: 271;
u) the VH of SEQ ID NO: 305 and the VL of SEQ ID NO: 271;
v) the VH of SEQ ID NO: 306 and the VL of SEQ ID NO: 271;
w) the VH of SEQ ID NO: 307 and the VL of SEQ ID NO: 271;
x) the VH of SEQ ID NO: 308 and the VL of SEQ ID NO: 271;
y) the VH of SEQ ID NO: 309 and the VL of SEQ ID NO: 271;
z) the VH of SEQ ID NO: 310 and the VL of SEQ ID NO: 271;
aa) the VH of SEQ ID NO: 263 and the VL of SEQ ID NO: 311;
ab) the VH of SEQ ID NO: 263 and the VL of SEQ ID NO: 312.
ac) the VH of SEQ ID NO: 263 and the VL of SEQ ID NO: 313;
ad) the VH of SEQ ID NO: 263 and the VL of SEQ ID NO: 314;
ae) the VH of SEQ ID NO: 263 and the VL of SEQ ID NO: 315;
af) the VH of SEQ ID NO: 263 and the VL of SEQ ID NO: 316;
ag) the VH of SEQ ID NO: 263 and the VL of SEQ ID NO: 317;
ah) the VH of SEQ ID NO: 263 and the VL of SEQ ID NO: 318;
ai) the VH of SEQ ID NO: 263 and the VL of SEQ ID NO: 319;
aj) the VH of SEQ ID NO: 263 and the VL of SEQ ID NO: 320;
ak) the VH of SEQ ID NO: 263 and the VL of SEQ ID NO: 321;
al) the VH of SEQ ID NO: 263 and the VL of SEQ ID NO: 322;
am)the VH of SEQ ID NO: 263 and the VL of SEQ ID NO: 323;
an) the VH of SEQ ID NO: 263 and the VL of SEQ ID NO: 324;
ao) the VH of SEQ ID NO: 263 and the VL of SEQ ID NO: 325;
ap) the VH of SEQ ID NO: 263 and the VL of SEQ ID NO: 326;
aq) the VH of SEQ ID NO: 263 and the VL of SEQ ID NO: 327;

ar) the VH of SEQ ID NO: 263 and the VL of SEQ ID NO: 328;
as) the VH of SEQ ID NO: 263 and the VL of SEQ ID NO: 329;
at) the VH of SEQ ID NO: 263 and the VL of SEQ ID NO: 330;
au) the VH of SEQ ID NO: 263 and the VL of SEQ ID NO: 331;
av) the VH of SEQ ID NO: 263 and the VL of SEQ ID NO: 332;
aw) the VH of SEQ ID NO: 263 and the VL of SEQ ID NO: 333;
ax) the VH of SEQ ID NO: 263 and the VL of SEQ ID NO: 334;
ay) the VH of SEQ ID NO: 263 and the VL of SEQ ID NO: 335;
az) the VH of SEQ ID NO: 263 and the VL of SEQ ID NO: 336;
ba) the VH of SEQ ID NO: 337 and the VL of SEQ ID NO: 272;
bb) the VH of SEQ ID NO: 338 and the VL of SEQ ID NO: 272;
be) the VH of SEQ ID NO: 339 and the VL of SEQ ID NO: 272;
bd) the VH of SEQ ID NO: 340 and the VL of SEQ ID NO: 272;
be) the VH of SEQ ID NO: 341 and the VL of SEQ ID NO: 272;
bf) the VH of SEQ ID NO: 342 and the VL of SEQ ID NO: 272;
bg) the VH of SEQ ID NO: 343 and the VL of SEQ ID NO: 272;
bh) the VH of SEQ ID NO: 344 and the VL of SEQ ID NO: 272;
bi) the VH of SEQ ID NO: 345 and the VL of SEQ ID NO: 272;
bj) the VH of SEQ ID NO: 346 and the VL of SEQ ID NO: 272;
bk) the VH of SEQ ID NO: 347 and the VL of SEQ ID NO: 272;
bl) the VH of SEQ ID NO: 348 and the VL of SEQ ID NO: 272;
bm)the VH of SEQ ID NO: 349 and the VL of SEQ ID NO: 272;
bn) the VH of SEQ ID NO: 350 and the VL of SEQ ID NO: 272;
bo) the VH of SEQ ID NO: 351 and the VL of SEQ ID NO: 272;
bp) the VH of SEQ ID NO: 352 and the VL of SEQ ID NO: 272.
bq) the VH of SEQ ID NO: 56 and the VL of SEQ ID NO: 353;
br) the VH of SEQ ID NO: 56 and the VL of SEQ ID NO: 354;
bs) the VH of SEQ ID NO: 56 and the VL of SEQ ID NO: 355;
bt) the VH of SEQ ID NO: 56 and the VL of SEQ ID NO: 356;
bu) the VH of SEQ ID NO: 56 and the VL of SEQ ID NO: 357;
bv) the VH of SEQ ID NO: 56 and the VL of SEQ ID NO: 358;
bw)the VH of SEQ ID NO: 56 and the VL of SEQ ID NO: 359;
bx) the VH of SEQ ID NO: 56 and the VL of SEQ ID NO: 360;
by) the VH of SEQ ID NO: 56 and the VL of SEQ ID NO: 361;
bz) the VH of SEQ ID NO: 56 and the VL of SEQ ID NO: 362;
ca) the VH of SEQ ID NO: 56 and the VL of SEQ ID NO: 363;
cb) the VH of SEQ ID NO: 56 and the VL of SEQ ID NO: 364;
cc) the VH of SEQ ID NO: 56 and the VL of SEQ ID NO: 365;
cd) the VH of SEQ ID NO: 56 and the VL of SEQ ID NO: 366;
ce) the VH of SEQ ID NO: 367 and the VL of SEQ ID NO: 85;
cf) the VH of SEQ ID NO: 368 and the VL of SEQ ID NO: 85;
cg) the VH of SEQ ID NO: 369 and the VL of SEQ ID NO: 85;
ch) the VH of SEQ ID NO: 370 and the VL of SEQ ID NO: 85;
ci) the VH of SEQ ID NO: 371 and the VL of SEQ ID NO: 85;
cj) the VH of SEQ ID NO: 372 and the VL of SEQ ID NO: 85;
ck) the VH of SEQ ID NO: 373 and the VL of SEQ ID NO: 85;
cl) the VH of SEQ ID NO: 374 and the VL of SEQ ID NO: 85;
cm)the VH of SEQ ID NO: 375 and the VL of SEQ ID NO: 85;
cn) the VH of SEQ ID NO: 376 and the VL of SEQ ID NO: 85;
co) the VH of SEQ ID NO: 377 and the VL of SEQ ID NO: 85;
cp) the VH of SEQ ID NO: 378 and the VL of SEQ ID NO: 85;
cq) the VH of SEQ ID NO: 379 and the VL of SEQ ID NO: 85;
cr) the VH of SEQ ID NO: 380 and the VL of SEQ ID NO: 85;
cs) the VH of SEQ ID NO: 381 and the VL of SEQ ID NO: 85;
ct) the VH of SEQ ID NO: 382 and the VL of SEQ ID NO: 85;
cu) the VH of SEQ ID NO: 53 and the VL of SEQ ID NO: 383;
cv) the VH of SEQ ID NO: 53 and the VL of SEQ ID NO: 384;
cw) the VH of SEQ ID NO: 53 and the VL of SEQ ID NO: 385;

cx) the VH of SEQ ID NO: 53 and the VL of SEQ ID NO: 386;
cy) the VH of SEQ ID NO: 53 and the VL of SEQ ID NO: 387;
cz) the VH of SEQ ID NO: 53 and the VL of SEQ ID NO: 388;
da) the VH of SEQ ID NO: 53 and the VL of SEQ ID NO: 389;
db) the VH of SEQ ID NO: 53 and the VL of SEQ ID NO: 390;
dc) the VH of SEQ ID NO: 53 and the VL of SEQ ID NO: 391;
dd) the VH of SEQ ID NO: 53 and the VL of SEQ ID NO: 392;
de) the VH of SEQ ID NO: 53 and the VL of SEQ ID NO: 393;
df) the VH of SEQ ID NO: 53 and the VL of SEQ ID NO: 394;
dg) the VH of SEQ ID NO: 53 and the VL of SEQ ID NO: 395;
dh) the VH of SEQ ID NO: 53 and the VL of SEQ ID NO: 396;
di) the VH of SEQ ID NO: 53 and the VL of SEQ ID NO: 397; or
dj) the VH of SEQ ID NO: 53 and the VL of SEQ ID NO: 398.

8) An isolated protein that binds CD33, wherein the isolated protein comprises

a) the VH of SEQ ID NO: 53 and the VL of SEQ ID NO: 82;
b) the VH of SEQ ID NO: 56 and the VL of SEQ ID NO: 85;
c) the VH of SEQ ID NO: 59 and the VL of SEQ ID NO: 87;
d) the VH of SEQ ID NO: 262 and the VL of SEQ ID NO: 271;
e) the VH of SEQ ID NO: 263 and the VL of SEQ ID NO: 272;
f) the VH of SEQ ID NO: 264 and the VL of SEQ ID NO: 273;
g) the VH of SEQ ID NO: 291 and the VL of SEQ ID NO: 271;
h) the VH of SEQ ID NO: 292 and the VL of SEQ ID NO: 271;
i) the VH of SEQ ID NO: 293 and the VL of SEQ ID NO: 271;
j) the VH of SEQ ID NO: 294 and the VL of SEQ ID NO: 271;
k) the VH of SEQ ID NO: 295 and the VL of SEQ ID NO: 271;
l) the VH of SEQ ID NO: 296 and the VL of SEQ ID NO: 271;
m) the VH of SEQ ID NO: 297 and the VL of SEQ ID NO: 271;
n) the VH of SEQ ID NO: 298 and the VL of SEQ ID NO: 271;
o) the VH of SEQ ID NO: 299 and the VL of SEQ ID NO: 271;
p) the VH of SEQ ID NO: 300 and the VL of SEQ ID NO: 271;
q) the VH of SEQ ID NO: 301 and the VL of SEQ ID NO: 271;
r) the VH of SEQ ID NO: 302 and the VL of SEQ ID NO: 271;
s) the VH of SEQ ID NO: 303 and the VL of SEQ ID NO: 271;
t) the VH of SEQ ID NO: 304 and the VL of SEQ ID NO: 271;
u) the VH of SEQ ID NO: 305 and the VL of SEQ ID NO: 271;
v) the VH of SEQ ID NO: 306 and the VL of SEQ ID NO: 271;
w) the VH of SEQ ID NO: 307 and the VL of SEQ ID NO: 271;
x) the VH of SEQ ID NO: 308 and the VL of SEQ ID NO: 271;
y) the VH of SEQ ID NO: 309 and the VL of SEQ ID NO: 271;
z) the VH of SEQ ID NO: 310 and the VL of SEQ ID NO: 271;
aa) the VH of SEQ ID NO: 263 and the VL of SEQ ID NO: 311;
ab) the VH of SEQ ID NO: 263 and the VL of SEQ ID NO: 312.
ac) the VH of SEQ ID NO: 263 and the VL of SEQ ID NO: 313;
ad) the VH of SEQ ID NO: 263 and the VL of SEQ ID NO: 314;
ae) the VH of SEQ ID NO: 263 and the VL of SEQ ID NO: 315;
af) the VH of SEQ ID NO: 263 and the VL of SEQ ID NO: 316;
ag) the VH of SEQ ID NO: 263 and the VL of SEQ ID NO: 317;
ah) the VH of SEQ ID NO: 263 and the VL of SEQ ID NO: 318;
ai) the VH of SEQ ID NO: 263 and the VL of SEQ ID NO: 319;
aj) the VH of SEQ ID NO: 263 and the VL of SEQ ID NO: 320;
ak) the VH of SEQ ID NO: 263 and the VL of SEQ ID NO: 321;
al) the VH of SEQ ID NO: 263 and the VL of SEQ ID NO: 322;
am)the VH of SEQ ID NO: 263 and the VL of SEQ ID NO: 323;
an) the VH of SEQ ID NO: 263 and the VL of SEQ ID NO: 324;
ao) the VH of SEQ ID NO: 263 and the VL of SEQ ID NO: 325;
ap) the VH of SEQ ID NO: 263 and the VL of SEQ ID NO: 326;

aq) the VH of SEQ ID NO: 263 and the VL of SEQ ID NO: 327;
ar) the VH of SEQ ID NO: 263 and the VL of SEQ ID NO: 328;
as) the VH of SEQ ID NO: 263 and the VL of SEQ ID NO: 329;
at) the VH of SEQ ID NO: 263 and the VL of SEQ ID NO: 330;
au) the VH of SEQ ID NO: 263 and the VL of SEQ ID NO: 331;
av) the VH of SEQ ID NO: 263 and the VL of SEQ ID NO: 332;
aw) the VH of SEQ ID NO: 263 and the VL of SEQ ID NO: 333;
ax) the VH of SEQ ID NO: 263 and the VL of SEQ ID NO: 334;
ay) the VH of SEQ ID NO: 263 and the VL of SEQ ID NO: 335;
az) the VH of SEQ ID NO: 263 and the VL of SEQ ID NO: 336;
ba) the VH of SEQ ID NO: 337 and the VL of SEQ ID NO: 272;
bb) the VH of SEQ ID NO: 338 and the VL of SEQ ID NO: 272;
be) the VH of SEQ ID NO: 339 and the VL of SEQ ID NO: 272;
bd) the VH of SEQ ID NO: 340 and the VL of SEQ ID NO: 272;
be) the VH of SEQ ID NO: 341 and the VL of SEQ ID NO: 272;
bf) the VH of SEQ ID NO: 342 and the VL of SEQ ID NO: 272;
bg) the VH of SEQ ID NO: 343 and the VL of SEQ ID NO: 272;
bh) the VH of SEQ ID NO: 344 and the VL of SEQ ID NO: 272;
bi) the VH of SEQ ID NO: 345 and the VL of SEQ ID NO: 272;
bj) the VH of SEQ ID NO: 346 and the VL of SEQ ID NO: 272;
bk) the VH of SEQ ID NO: 347 and the VL of SEQ ID NO: 272;
bl) the VH of SEQ ID NO: 348 and the VL of SEQ ID NO: 272;
bm)the VH of SEQ ID NO: 349 and the VL of SEQ ID NO: 272;
bn) the VH of SEQ ID NO: 350 and the VL of SEQ ID NO: 272;
bo) the VH of SEQ ID NO: 351 and the VL of SEQ ID NO: 272;
bp) the VH of SEQ ID NO: 352 and the VL of SEQ ID NO: 272.
bq) the VH of SEQ ID NO: 56 and the VL of SEQ ID NO: 353;
br) the VH of SEQ ID NO: 56 and the VL of SEQ ID NO: 354;
bs) the VH of SEQ ID NO: 56 and the VL of SEQ ID NO: 355;
bt) the VH of SEQ ID NO: 56 and the VL of SEQ ID NO: 356;
bu) the VH of SEQ ID NO: 56 and the VL of SEQ ID NO: 357;
bv) the VH of SEQ ID NO: 56 and the VL of SEQ ID NO: 358;
bw)the VH of SEQ ID NO: 56 and the VL of SEQ ID NO: 359;
bx) the VH of SEQ ID NO: 56 and the VL of SEQ ID NO: 360;
by) the VH of SEQ ID NO: 56 and the VL of SEQ ID NO: 361;
bz) the VH of SEQ ID NO: 56 and the VL of SEQ ID NO: 362;
ca) the VH of SEQ ID NO: 56 and the VL of SEQ ID NO: 363;
cb) the VH of SEQ ID NO: 56 and the VL of SEQ ID NO: 364;
cc) the VH of SEQ ID NO: 56 and the VL of SEQ ID NO: 365;
cd) the VH of SEQ ID NO: 56 and the VL of SEQ ID NO: 366;
ce) the VH of SEQ ID NO: 367 and the VL of SEQ ID NO: 85;
cf) the VH of SEQ ID NO: 368 and the VL of SEQ ID NO: 85;
cg) the VH of SEQ ID NO: 369 and the VL of SEQ ID NO: 85;
ch) the VH of SEQ ID NO: 370 and the VL of SEQ ID NO: 85;
ci) the VH of SEQ ID NO: 371 and the VL of SEQ ID NO: 85;
cj) the VH of SEQ ID NO: 372 and the VL of SEQ ID NO: 85;
ck) the VH of SEQ ID NO: 373 and the VL of SEQ ID NO: 85;
cl) the VH of SEQ ID NO: 374 and the VL of SEQ ID NO: 85;
cm)the VH of SEQ ID NO: 375 and the VL of SEQ ID NO: 85;
cn) the VH of SEQ ID NO: 376 and the VL of SEQ ID NO: 85;
co) the VH of SEQ ID NO: 377 and the VL of SEQ ID NO: 85;
cp) the VH of SEQ ID NO: 378 and the VL of SEQ ID NO: 85;
cq) the VH of SEQ ID NO: 379 and the VL of SEQ ID NO: 85;
cr) the VH of SEQ ID NO: 380 and the VL of SEQ ID NO: 85;
cs) the VH of SEQ ID NO: 381 and the VL of SEQ ID NO: 85;
ct) the VH of SEQ ID NO: 382 and the VL of SEQ ID NO: 85;
cu) the VH of SEQ ID NO: 53 and the VL of SEQ ID NO: 383;
cv) the VH of SEQ ID NO: 53 and the VL of SEQ ID NO: 384;

cw) the VH of SEQ ID NO: 53 and the VL of SEQ ID NO: 385;
ex) the VH of SEQ ID NO: 53 and the VL of SEQ ID NO: 386;
cy) the VH of SEQ ID NO: 53 and the VL of SEQ ID NO: 387;
cz) the VH of SEQ ID NO: 53 and the VL of SEQ ID NO: 388;
da) the VH of SEQ ID NO: 53 and the VL of SEQ ID NO: 389;
db) the VH of SEQ ID NO: 53 and the VL of SEQ ID NO: 390;
de) the VH of SEQ ID NO: 53 and the VL of SEQ ID NO: 391;
dd) the VH of SEQ ID NO: 53 and the VL of SEQ ID NO: 392;
de) the VH of SEQ ID NO: 53 and the VL of SEQ ID NO: 393;
df) the VH of SEQ ID NO: 53 and the VL of SEQ ID NO: 394;
dg) the VH of SEQ ID NO: 53 and the VL of SEQ ID NO: 395;
dh) the VH of SEQ ID NO: 53 and the VL of SEQ ID NO: 396;
di) the VH of SEQ ID NO: 53 and the VL of SEQ ID NO: 397; or
dj) the VH of SEQ ID NO: 53 and the VL of SEQ ID NO: 398.

9) The isolated protein of any one of claims 1-8, wherein the isolated protein is a scFv, a $(scFv)_2$, a Fv, a Fab, a $F(ab')_2$, a Fd, a dAb or a VHH.
10) The isolated protein of claim 9, wherein the isolated protein is the Fab.
11) The isolated protein of claim 9, wherein the isolated protein is the scFv.
12) The isolated protein of claim 11, wherein the scFv comprises, from the N- to C-terminus, a VH, a first linker (L1) and a VL (VH-L1-VL) or the VL, the L1 and the VH (VL-L1-VH).
13) The isolated protein of claim 12, wherein the L1 comprises

a) about 5-50 amino acids;
b) about 5-40 amino acids;
c) about 10-30 amino acids; or
d) about 10-20 amino acids.

14) The isolated protein of claim 13, wherein the L1 comprises an amino acid sequence of any one of SEQ ID NOs: 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, or 140.
15) The isolated protein of claim 14, wherein the L1 comprises the amino acid sequence of SEQ ID NO: 108.
16) The isolated protein of claim 15, wherein the isolated protein the amino acid sequence of SEQ ID NO: 213, 216, 219, 274, 275 or 276.
17) The isolated protein of any one of claims 1-16, wherein the protein is conjugated to a half-life extending moiety.
18) The isolated protein of claim 17, wherein the half-life extending moiety is an immunoglobulin (Ig), a fragment of the Ig, an Ig constant region, a fragment of the Ig constant region, a Fc region, transferrin, albumin, an albumin binding domain or polyethylene glycol.
19) The isolated protein of any one of claims 1-18, wherein the isolated protein is a monospecific protein.
20) The isolated protein of any one of claims 1-19, wherein the isolated protein is a multispecific protein.
21) The isolated protein of claim 20, wherein the multispecific protein is a bispecific protein.
22) The isolated protein of claim 20, wherein the multispecific protein is a trispecific protein.
23) The isolated protein of any one of claims 1-22, further comprising an immunoglobulin (Ig) constant region or a fragment of the Ig constant region thereof.
24) The isolated protein of claim 23, wherein the fragment of the Ig constant region comprises a Fc region.
25) The isolated protein of claim 23, wherein the fragment of the Ig constant region comprises a CH2 domain.
26) The isolated protein of claim 23, wherein the fragment of the Ig constant region comprises a CH3 domain.
27) The isolated protein of claim 23, wherein the fragment of the Ig constant region comprises the CH2 domain and the CH3 domain.
28) The isolated protein of claim 23, wherein the fragment of the Ig constant region comprises at least portion of a hinge, the CH2 domain and the CH3 domain.
29) The isolated protein of claim 23, wherein the fragment of the Ig constant region comprises a hinge, the CH2 domain and the CH3 domain.
30) The isolated protein of any one of claims 23-29, comprising an antigen binding domain that binds CD33 that is conjugated to the N-terminus of the Ig constant region or the fragment of the Ig constant region.
31) The isolated protein of any one of claims 23-29, comprising an antigen binding domain that binds CD33 that is conjugated to the C-terminus of the Ig constant region or the fragment of the Ig constant region.
32) The isolated protein of any one of claims 23-31, comprising an antigen binding domain that binds CD33 that is

conjugated to the Ig constant region or the fragment of the Ig constant region via a second linker (L2).

33) The isolated protein of claim 32, wherein the L2 comprises the amino acid sequence of any one of SEQ ID NOs:108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, or 140.

34) The isolated protein of any one of claims 20-33, wherein the multispecific protein comprises an antigen binding domain that binds an antigen on a lymphocyte.

35) The isolated protein of claim 34, wherein the lymphocyte is a T cell.

36) The isolated protein of claim 35, wherein the T cell is a $CD8^+$ T cell

37) The isolated protein of claim 34, wherein the lymphocyte is a natural killer (NK) cell.

38) The isolated protein of any one of claims 20-37, wherein the multispecific protein comprises an antigen binding domain that binds CD3, CD3 epsilon (CD3ε), CD8, KI2L4, NKG2E, NKG2D, NKG2F, BTNL3, CD186, BTNL8, PD-1, CD195, TRGV9, orNKG2C.

39) The isolated protein of claim 38, wherein the multispecific protein comprises an antigen binding domain that binds CD3ε.

40) The isolated protein of claim 38, wherein the multispecific protein comprises an antigen binding domain that binds TRGV9.

41) The isolated protein of any one of claims 34-40, wherein a first antigen binding domain that binds CD33 and/or a second antigen binding domain that binds the lymphocyte antigen comprise a scFv, a (scFv)2, a Fv, a Fab, a F(ab')2, a Fd, a dAb or a VHH.

42) The isolated protein of claim 41, wherein

a) the first antigen binding domain that binds CD33 and/or the second antigen binding domain that binds the lymphocyte antigen comprise the Fab;
b) the first antigen binding domain that binds CD33 and/or the second antigen binding domain that binds the lymphocyte antigen comprise the VHH;
c) the first antigen binding domain that binds CD33 and/or the second antigen binding domain that binds the lymphocyte antigen comprise the scFv; or
d) the first antigen binding domain that binds CD33 comprises the scFv and the second antigen binding domain that binds the lymphocyte antigen comprises the VHH.

43) The isolated protein of any one of claims 23-42, wherein the Ig constant region or the fragment of the Ig constant region is an IgG1, an IgG2, an IgG3 or an IgG4 isotype, wherein optionally the Ig constant region or the fragment of the Ig constant region is an IgG1.

44) The isolated protein of claim 43, wherein the fragment of the Ig constant region has the sequence of SEQ ID NO: 277 or 278.

45) The isolated protein of any one of claims 23-43, wherein the Ig constant region or the fragment of the Ig constant region comprises at least one mutation that results in reduced binding of the protein to a Fcγ receptor (FcγR).

46) The isolated protein of claim 45, wherein the at least one mutation that results in reduced binding of the protein to the FcγR is selected from the group consisting of F234A/L235A, L234A/L235A, L234A/L235A/D265S, V234A/G237A/ P238S/H268A/V309L/A330S/P331S, F234A/L235A, S228P/F234A/ L235A, N297A, V234A/G237A, K214T/E233P/ L234V/L235A/G236-deleted/A327G/P331A/D365E/L358M, H268Q/V309L/A330S/P331S, S267E/L328F, L234F/L235E/D265A, L234A/L235A/G237A/P238S/H268A/A330S/P331S, S228P/F234A/L235A/G237A/P238S and S228P/F234A/L235A/G236-deleted/G237A/P238S, wherein residue numbering is according to the EU index.

47) The isolated protein of any one of claims 23-43, wherein the Ig constant region or the fragment of the Ig constant region comprises at least one mutation that results in enhanced binding of the protein to the FcγR.

48) The isolated protein of claim 47, wherein the at least one mutation that results in enhanced binding of the protein to the FcγR is selected from the group consisting of S239D/I332E, S298A/E333A/K334A, F243L/R292P/Y300L, F243L/R292P/Y300L/P396L, F243L/R292P/Y300L/V305I/P396L and G236A/S239D/I332E, wherein residue numbering is according to the EU index.

49) The isolated protein of any one of claims 45-48, wherein the FcγR is FcγRI, FcγRIIA, FcγRIIB or FcγRIII, or any combination thereof.

50) The isolated protein of any one of claims 23-49, wherein the Ig constant region of the fragment of the Ig constant region comprises at least one mutation that modulates a half-life of the protein.

51) The isolated protein of claim 50, wherein the at least one mutation that modulates the half-life of the protein is selected from the group consisting of H435A, P257I/N434H, D376V/N434H, M252Y/S254T/T256E/H433K/N434F, T308P/N434A and H435R, wherein residue numbering is according to the EU index.

52) The isolated protein of any one of the claims 23-51, wherein the protein comprises at least one mutation in a

CH3 domain of the Ig constant region.

53) The isolated protein of claim 52, wherein the at least one mutation in the CH3 domain of the Ig constant region is selected from the group consisting of T350V, L351Y, F405A, Y407V, T366Y, T366W, F405W, T394W, T394S, Y407T, Y407A, T366S/L368A/Y407V, L351Y/F405A/Y407V, T366I/K392M/T394W, F405A/Y407V, T366L/K392M/T394W, L351Y/Y407A, T366A/K409F, L351Y/Y407A, T366V/K409F, T366A/K409F, T350V/L351Y/F405A/Y407V and T350V/T366L/K392L/T394W, wherein residue numbering is according to the EU index.

54) An isolated multispecific protein comprising a first antigen binding domain that binds CD33 and a second antigen binding domain that binds a lymphocyte antigen, wherein the first antigen binding domain that binds CD33 comprises the binding domain as defined in any one of claims 1-16.

55) The isolated multispecific protein of claim 54, wherein the lymphocyte antigen is a T cell antigen.

56) The isolated multispecific protein of claim 55, wherein the T cell antigen is a CD8+ T cell antigen.

57) The isolated multispecific protein of claim 54, wherein the lymphocyte antigen is a NK cell antigen.

58) The isolated multispecific protein of any one of claims 54-57, wherein the lymphocyte antigen is CD3, CD3 epsilon (CD3ε), CD8, KI2L4, NKG2E, NKG2D, NKG2F, BTNL3, CD186, BTNL8, PD-1, CD195, TRGV9, orNKG2C.

59) The isolated multispecific protein of claim 58, wherein the lymphocyte antigen is CD3s.

60) The isolated multispecific protein of any one of claims 54-59, wherein the first antigen binding domain that binds CD33 and/or the second antigen binding domain that binds the lymphocyte antigen comprise a scFv, a (scFv)2, a Fv, a Fab, a F(ab')2, a Fd, a dAb or a VHH.

61) The isolated multispecific protein of claim 60, wherein the first antigen binding domain that binds CD33 and/or the second antigen binding domain that binds the lymphocyte antigen comprise the Fab.

62) The isolated multispecific protein of claim 60, wherein the first antigen binding domain that binds CD33 and/or the second antigen binding domain that binds the lymphocyte antigen comprise the VHH.

63) The isolated multispecific protein of claim 60, wherein the first antigen binding domain that binds CD33 and/or the second antigen binding domain that binds the lymphocyte antigen comprise the scFv.

64) The isolated multispecific protein of claim 60 or 63, wherein the scFv comprises, from the N- to C-terminus, a VH, a first linker (L1) and a VL (VH-L1-VL) or the VL, the L1 and the VH (VL-L1-VH).

65) The isolated multispecific protein of claim 64, wherein the L1 comprises

about 5-50 amino acids;
about 5-40 amino acids;
about 10-30 amino acids; or
about 10-20 amino acids.

66) The isolated multispecific protein of claim 65, wherein the L1 comprises the amino acid sequence of any one of SEQ ID NOs: 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, or 140.

67) The isolated multispecific protein of claim 66, wherein the L1 comprises the amino acid sequence of SEQ ID NO: 108.

68) The isolated multispecific protein of any one of claims 54-67, wherein the first antigen binding domain that binds CD33 is conjugated to a first immunoglobulin (Ig) constant region or a fragment of the first Ig constant region and/or the second antigen binding domain that binds the lymphocyte antigen is conjugated to a second immunoglobulin (Ig) constant region or a fragment of the second Ig constant region.

69) The isolated multispecific protein of claim 68, further comprising a second linker (L2) between the first antigen binding domain that binds CD33 and the first Ig constant region or the fragment of the first Ig constant region and the second antigen binding domain that binds the lymphocyte antigen and the second Ig constant region or the fragment of the second Ig constant region.

70) The isolated multispecific protein of claim 69 wherein the L2 comprises the amino acid sequence of any one of SEQ ID NOs: 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, or 140.

71) The isolated multispecific protein of any one of claims 68-70, wherein the first Ig constant region or the fragment of the first Ig constant region and the second Ig constant region or the fragment of the second Ig constant region is an IgG1, an IgG2, and IgG3 or an IgG4 isotype.

72) The isolated multispecific protein of claim 68-71, wherein the first Ig constant region or the fragment of the first Ig constant region and the second Ig constant region or the fragment of the second Ig constant region comprises at least one mutation that results in reduced binding of the multispecific protein to a FcyR.

73) The isolated multispecific protein of claim 72, wherein the at least one mutation that results in reduced binding of the multispecific protein to the FcyR is selected from the group consisting of F234A/L235A, L234A/L235A,

L234A/L235A/D265S, V234A/G237A/ P238S/H268A/V309L/A330S/P331S, F234A/L235A, S228P/F234A/ L235A, N297A, V234A/G237A, K214T/E233P/ L234V/L235A/G236-deleted/A327G/P331A/D365E/L358M, H268Q/V309L/A330S/P331S, S267E/L328F, L234F/L235E/D265A, L234A/L235A/G237A/P238S/H268A/A330S/P331S, S228P/F234A/L235A/G237A/P238S and S228P/F234A/L235A/G236-deleted/G237A/P238S, wherein residue numbering is according to the EU index.

74) The isolated multispecific protein of any one of claims 68-71, wherein the first Ig constant region or the fragment of the first Ig constant region and the second Ig constant region or the fragment of the second Ig constant region comprises at least one mutation that results in enhanced binding of the multispecific protein to a Fcy receptor (FcyR).

75) The isolated multispecific protein of claim 74, wherein the at least one mutation that results in enhanced binding of the multispecific protein to the FcyR is selected from the group consisting of S239D/I332E, S298A/E333A/K334A, F243L/R292P/Y300L, F243L/R292P/Y300L/P396L, F243L/R292P/Y300L/V305I/P396L and G236A/S239D/I332E, wherein residue numbering is according to the EU index.

76) The isolated multispecific protein of any one of claims 72-75, wherein the FcyR is FcyRI, FcyRIIA, FcyRIIB or FcyRIII, or any combination thereof.

77) The isolated multispecific protein of any one of claims 67-76, wherein the first Ig constant region or the fragment of the first Ig constant region and the second Ig constant region or the fragment of the second Ig constant region comprises at least one mutation that modulates a half-life of the multispecific protein.

78) The isolated multispecific protein of claim 77, wherein the at least one mutation that modulates the half-life of the multispecific protein is selected from the group consisting of H435A, P257I/N434H, D376V/N434H, M252Y/S254T/T256E/H433K/N434F, T308P/N434A and H435R, wherein residue numbering is according to the EU index.

79) The isolated multispecific protein of any one of the claims 68-78, comprising at least one mutation in a CH3 domain of the first Ig constant region or in a CH3 domain of the fragment of the first Ig constant region and/or at least one mutation in a CH3 domain of the second Ig constant region or in a CH3 domain of the fragment of the second Ig constant region.

80) The isolated multispecific protein of claim 79, wherein the at least one mutation in a CH3 domain of the first Ig constant region or in a CH3 domain of the fragment of the first Ig constant region and/or at least one mutation in a CH3 domain of the second Ig constant region or in a CH3 domain of the fragment of the second Ig constant region is selected from the group consisting of T350V, L351Y, F405A,Y407V, T366Y, T366W, F405W, T394W, T394S, Y407T, Y407A, T366S/L368A/Y407V, L351Y/F405A/Y407V, T366I/K392M/T394W, F405A/Y407V, T366L/K392M/T394W, L351Y/Y407A, T366A/K409F, L351Y/Y407A, T366V/K409F, T366A/K409F, T350V/L351Y/F405A/Y407V and T350V/T366L/K392L/T394W, wherein residue numbering is according to the EU index.

81) The isolated multispecific protein of any one of claims 68-80, wherein the first Ig constant region or the fragment of the first Ig constant region and the second Ig constant region or the fragment of the second Ig constant region comprise the following mutations:

L235A_L235A_D265S_T350V_L35IY_F405A_Y407V in the first Ig constant region and
L235A_L235A_D265S_T350V_T366L_K392L_T394W in the second Ig constant region; or
L235A_L235A_D265S_T350V_T366L_K392L_T394W in the first Ig constant region and
L235A_L235A_D265S_T350V_L351Y_F405A_Y407V in the second Ig constant region.

82) The isolated protein of any one of claims 1-16, wherein the protein is a chimeric antigen receptor (CAR).

83) The isolated protein of claim 82, wherein the CAR comprises

a) an extracellular domain comprising the antigen binding domain that binds CD33 of any one of claims 1-16;
b) a transmembrane domain; and
c) an intracellular signaling domain optionally comprising at least one co-stimulatory domain.

84) The isolated protein of claim 82 or 83, wherein the CAR further comprises a CD8a-hinge region.

85) The isolated protein of any one of claims 82-84, wherein

a) the transmembrane domain comprises a CD8a transmembrane region (CD8a-TM) polypeptide; and
b) the intracellular signaling domain comprises a co-stimulatory domain comprising a TNF receptor superfamily member 9 (CD137) component and a primary signaling domain comprising a T-cell surface glycoprotein CD3 zeta chain (CD3z) component.

86) The isolated protein of any one of claims 82-84, wherein

a) the CD8a-hinge region comprises an amino acid sequence that is at least 90% identical to the amino acid sequence of SEQ ID NO: 157;
b) the transmembrane domain comprises an amino acid sequence that is at least 90% identical to the amino acid sequence of SEQ ID NO: 162; and/or
c) the intracellular signaling domain comprises a co-stimulatory domain having an amino acid sequence that is at least 90% identical to the amino acid sequence of SEQ ID NO: 163 and a primary signaling domain having an amino acid sequence that is at least 90% identical to the amino acid sequence of SEQ ID NO: 164.

87) The isolated protein of claim 86, wherein the intracellular signaling domain comprises a polypeptide component selected from the group consisting of a TNF receptor superfamily member 9 (CD 137) component, a T-cell surface glycoprotein CD3 zeta chain (CD3z) component, a cluster of differentiation (CD27) component, a cluster of differentiation superfamily member component, or any combinations thereof.
88) A chimeric antigen receptor (CAR) comprising:

a) an extracellular domain comprising an antigen binding domain that binds CD33;
b) a transmembrane domain; and
c) an intracellular signaling domain optionally comprising at least one co-stimulatory domain,

wherein the antigen binding domain that binds CD33 comprises the binding domain as defined in any one of claims 1-16, 20-22 or 34-40.
89) The CAR of claim 88, further comprising a CD8a-hinge region.
90) The CAR of claim 88 or 89, wherein

a) the transmembrane domain comprises a CD8a transmembrane region (CD8a-TM) polypeptide; and
b) the intracellular signaling domain comprises a co-stimulatory domain comprising a TNF receptor superfamily member 9 (CD137) component and a primary signaling domain comprising a T-cell surface glycoprotein CD3 zeta chain (CD3z) component.

91) The CAR of claim 89 or 90, wherein

d) the CD8a-hinge region comprises an amino acid sequence that is at least 90% identical to the amino acid sequence of SEQ ID NO: 157;
e) the transmembrane domain comprises an amino acid sequence that is at least 90% identical to the amino acid sequence of SEQ ID NO: 162; and/or
f) the intracellular signaling domain comprises a co-stimulatory domain having an amino acid sequence that is at least 90% identical to the amino acid sequence of SEQ ID NO: 163, and a primary signaling domain having an amino acid sequence that is at least 90% identical to the amino acid sequence of SEQ ID NO: 164.

92) The CAR of any one of claims 88-91, wherein the extracellular domain comprising the antigen binding domain that binds CD33 further comprises a signal polypeptide.
93) The CAR of claim 92, wherein the signal polypeptide comprises the amino acid sequence of SEQ ID NO: 166.
94) The CAR of any one of claims 88-93, wherein the intracellular signaling domain comprises a polypeptide component selected from the group consisting of a TNF receptor superfamily member 9 (CD137) component, a T-cell surface glycoprotein CD3 zeta chain (CD3z) component, a cluster of differentiation (CD27) component, a cluster of differentiation superfamily member component, and a combination thereof.
95) The CAR of any one of claims 90-94, wherein the CD 137 component comprises the amino acid sequence of SEQ ID NO: 163.
96) The CAR of any one of claims 90-95, wherein the CD3z component comprises an amino acid sequence of SEQ ID NO: 164.
97) The CAR of any one of claims 90-96, wherein the intracellular signaling domain comprises an amino acid sequence of SEQ ID NO: 165.
98) The CAR of any one of claims 90-97, wherein the CD8a-TM polypeptide comprises an amino acid sequence of SEQ ID NO: 162.
99) The CAR of any one of claims 90-98, wherein the CD8a-hinge region comprises an amino acid sequence of SEQ ID NO: 157.
100) An isolated lymphocyte expressing the CAR of any one of claims 90-99.

101) The isolated lymphocyte of claim 100, wherein the lymphocyte is a T lymphocyte.
102) The isolated lymphocyte of claim 100, wherein the lymphocyte is a natural killer (NK) cell.
103) An immunoconjugate comprising the isolated protein of any one of claims 1-53, or isolated multispecific protein of any one of claims 54-81, conjugated to an agent.
104) The immunoconjugate of claim 103, wherein the agent is a therapeutic agent or an imaging agent.
105) The immunoconjugate of claim 103, wherein the agent is a radioactive agent.
106) The immunoconjugate of claim 105, wherein the radioactive agent comprises a radiometal ion.
107) The immunoconjugate of claim 106, wherein the radiometal ion is $^{32}P$, $^{47}Sc$, $^{67}Cu$, $^{77}As$, $^{89}Sr$, $^{90}Y$, $^{99}Tc$, $^{105}Rh$, $^{109}Pd$, $^{111}Ag$ $^{131}I$, $^{153}Sm$, $^{159}Gd$, $^{165}Dy$, $^{166}Ho$, $^{169}Er$, $^{177}Lu$, $^{186}Re$, $^{188}Re$, $^{194}Ir$, $^{198}Au$, $^{199}Au$, $^{211}At$, $^{212}Pb$, $^{212}Bi$, $^{213}Bi$, $^{223}Ra$, $^{225}Ac$, $^{255}Fm$, $^{227}Th$, $^{62}Cu$, $^{64}Cu$, $^{67}Ga$, $^{68}Ga$, $^{86}Y$, $^{89}Zr$, or $^{111}In$.
108) The immunoconjugate of claim 107, wherein the radiometal ion is $^{225}Ac$, $^{111}In$ or $^{89}Zr$.
109) The immunoconjugate of claim 107, wherein the radiometal ion is $^{225}Ac$.
110) The immunoconjugate of any one of claims 106 to 109, wherein the radiometal ion is conjugated to a chelating moiety.
111) The immunoconjugate of claim 110, wherein the chelating moiety comprises a macrocycle having the structure of formula (I):

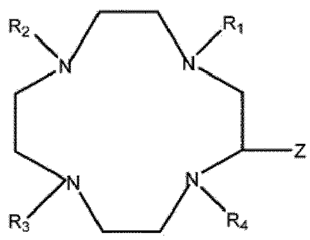

formula (I),

wherein each of $R_1$, $R_2$, $R_3$ and $R_4$ is independently $CHQCO_2X$, wherein Q is independently hydrogen, $C_1$-$C_4$ alkyl or ($C_1$-$C_2$ alkyl) phenyl, and
X is independently hydrogen, benzyl, $C_1$-$C_4$ alkyl; and
Z is $(CH2)_nY$, wherein
n is 1-10, and
Y is an electrophilic or nucleophilic moiety covalently linked to the second click reaction partner;

alternatively, Z is hydrogen; and
each of $R_1$, $R_2$, $R_3$ and $R_4$ is independently CHQCOzX, wherein Q is independently hydrogen, $C_1$-$C_4$ alkyl or ($C_1$-$C_2$ alkyl) phenyl, and
X is independently hydrogen, benzyl, $C_1$-$C_4$ alkyl, or an electrophilic or nucleophilic moiety covalently linked to the second click reaction partner;
alternatively, the chelant comprises an open chain ligand.

112) The immunoconjugate of claim 110, wherein the chelating moiety comprises a macrocycle having the structure of formula (II):

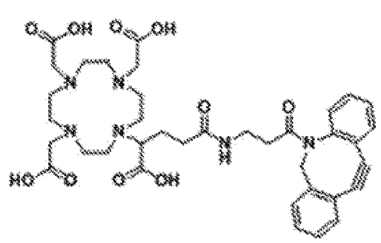

or a structure of formula (III):

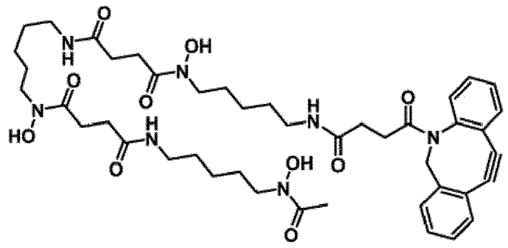

113) An immunoconjugate of formula (IV),

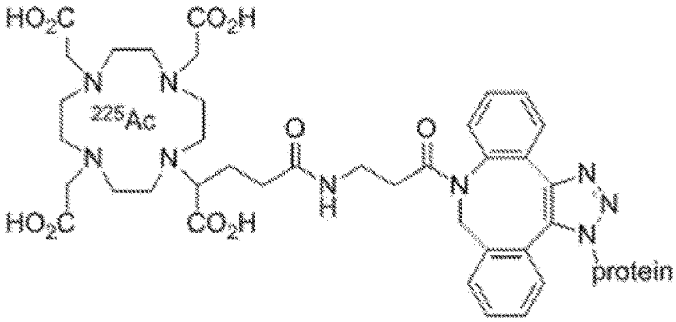

formula (IV)

wherein the protein is the isolated protein as defined in any one of claims 1-53, 82-87 or the isolated multispecific protein as defined in any one of claims 54-81.

114) An immunoconjugate of formula (V),

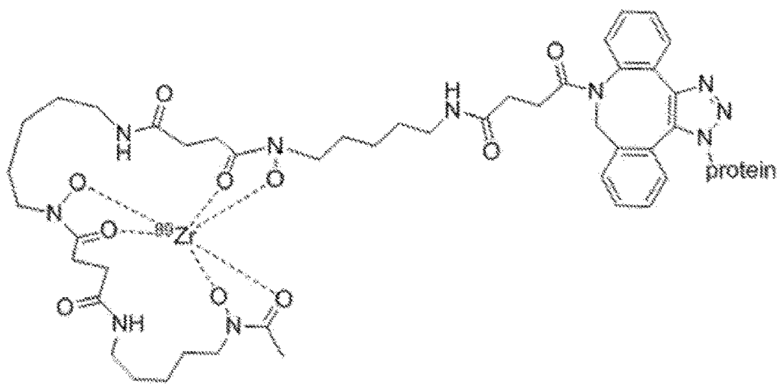

formula (V)

wherein the protein is the isolated protein as defined in any one of claims 1-53, 82-87 or the isolated multispecific protein as defined in any one of claims 54-81.

115) A pharmaceutical composition comprising the isolated protein of any one of claims 1-53, 82-87, the isolated multispecific protein of any one of claims 54-81, the CAR of any one of claims 88-99, the lymphocyte of any one of claims 100-102 or immunoconjugate of any one of claims 103-114, and a pharmaceutically acceptable carrier.

116) A polynucleotide encoding the isolated protein of any one of claims 1-53, 82-87, the isolated multispecific protein of any one of claims 54-81, the CAR of any one of claims 88-99, or immunoconjugate of any one of claims 103-114.

117) A vector comprising the polynucleotide of claim 116.

118) A host cell comprising the vector of claim 117 or polynucleotide of claim 116.

119) A method of producing the isolated protein of any one of claims 1-53, 82-87, or the isolated multispecific protein of any one of claims 54-81, comprising culturing the host cell of claim 118 in conditions that the protein is expressed, and recovering the protein produced by the host cell.

120) A method of producing the immunoconjugate of any one of claims 103-114, comprising culturing the host cell of claim 118 in conditions that the protein is expressed, recovering the protein produced by the host cell, and conjugating the protein produced by the host cell with the agent.

121) An anti-idiotypic antibody binding to the isolated protein of any one of claims 1-53 or 82-87.
122) A kit comprising the isolated protein of any one of claims 1-53, 82-87, or the isolated multispecific protein of any one of claims 54-81 or CAR of any one of claims 88-99.
123) A method of treating a CD33 expressing cancer in a subject, comprising administering a therapeutically effective amount of the isolated protein of any one of claims 1-53, 82-87, the isolated multispecific protein of any one of claims 54-81, or immunoconjugate of any one of claims 103-114, to the subject for a time sufficient to treat the CD33 expressing cancer.
124) The method of claim 123, wherein the method is effective to reduce the amount of CD33 expressing cells in a subject, wherein the CD33 expressing cells are cancerous cells or wherein the CD33 expressing cells are non-cancerous cells.
125) The method of claim 123 or 124, wherein the method is effective to prevent establishment of a CD33 expressing cancer in the subject.
126) A method of treating a subject having a CD33 expressing cancer, the method comprising: administering a therapeutically effective amount of the lymphocyte of any one of claims 100-102 to a subject in need thereof, whereby the lymphocyte mediates killing of the CD33 expressing cancer in the subject.
127) A method of targeted killing of a cancer cell, the method comprising: contacting the cancer cell with the lymphocyte of any one of claims 100-102, whereby the lymphocyte induces targeted killing of the cancer cell.
128) A method of detecting the presence of a cancer in a subject, comprising:

a) contacting a cell sample obtained from the subject with the CAR of any one of claims 88-99, thereby forming a CAR-cell complex, and
b) detecting the CAR-cell complex, wherein detection of the CAR-cell complex is indicative of the presence of the cancer in the subject.

129) The method of any one of claims 123-128, wherein the CD33 expressing cancer is a hematologic cancer.
130) The method of claim 129, wherein the hematologic cancer is a leukemia, a lymphoma, or a multiple myeloma.
131) The method of claim 130, wherein the hematologic cancer is acute myeloid leukemia (AML), myelodysplastic syndrome (MDS), acute lymphocytic leukemia (ALL), diffuse large B-cell lymphoma (DLBCL), chronic myeloid leukemia (CML) or blastic plasmacytoid dendritic cell neoplasm (DPDCN).
132) The method of claim 131, wherein the hematologic cancer is acute myeloid leukemia (AML).
133) The method of any one of claims 123-132, wherein the isolated protein, isolated multispecific protein or the immunoconjugate, is administered in combination with a second therapeutic agent.
134) The method of claim 133, wherein the second therapeutic agent is surgery, chemotherapy, androgen deprivation therapy or radiation, or any combination thereof.

**Sequences**

SEQ ID NO: 1 CDR1 (C33B1785; C33B1787; C33B1780; C33B1789; C33B1788)
DYAIG

SEQ ID NO: 2 CDR1 (C33B1784)
TYVMG

SEQ ID NO: 3 CDR1 (C33B1786)
FSTMD

SEQ ID NO: 4 CDR1 (C33B1781)
INAMG

SEQ ID NO: 5 CDR1 (C33B1783; C33B1782)
DYAVG

SEQ ID NO: 6 CDR2 (C33B1785; C33B1780; C33B1789; C33B1788)
CISESDGRTYLSDSVKS

SEQ ID NO: 7 CDR2 (C33B1784)

AIVSGRNPTYADSVKS

SEQ ID NO: 8 CDR2 (C33B1787)
CISSSDGSTYYVDSVKG

SEQ ID NO: 9 CDR2 (C33B1786)
CIRPRYSTTTHADSIKG

SEQ ID NO: 10 CDR2 (C33B1781)
RITGGGATDYVDSVKG

SEQ ID NO: 11 CDR2 (C33B1783; C33B1782)
CISSSDGATYYADSVKG

SEQ ID NO: 12 CDR3 (C33B1785; C33B1780; C33B1789; C33B1788)
VDQAISES SYHCEKGFGS

SEQ ID NO: 13 CDR3 (C33B1784; C33B1786)
YHYSSQY

SEQ ID NO: 14 CDR3 (C33B 1787)
LIIMYGSGSERAAAACRYKYEY

SEQ ID NO: 15 CDR3 (G33B1781)
DLVTRVGSDLLYDDY

SEQ ID NO: 16 CDR3 (C33B1783)
IIVRGSGWERAAAACRYEYSY

SEQ ID NO: 17 CDR3 (C33B1782)
VIVRGTGWERAAAACRYEYAY

SEQ ID NO: 18 VHH sequence (C33B1785)

QVQLVESGGGSVQPGGSLRLSCAVFGFSLSDYAIGWFRQAPGKEREKVSCISESDGRTYLSDSVK SRFTISRDNGKNTVYLQMNNLKPDDTGVYYCATVDQAISESSYHCEKGFGSWGQGTQVTVSS

SEQ ID NO: 19 VHH sequence (C33B1784)

EVQLVESGGGLVQAGGSLRLSCAASGRTFSTYVMGWFRQAPGQERELVAAIVSGRNPTYADSV KSRFTISRDNAKNTIYLHMNSLQPEDTAVYYCHMYHYSSQYWGQGTQVTVSS

SEQ ID NO: 20 VHH sequence (C33B1787)

EVQLVESGGGLVQAGGSLRLSCAASGFTFDDYAIGWFRQAPGKEREGVSCISSSDGSTYYVDSV KGRFTISSDNAKNTVYLQMNSLKPEDTAVYYCTALIIMYGSGSERAAAACRYKYEYWGQGTQV TVSS

SEQ ID NO: 21 VHH sequence (C33B1786)

EVQVVESGGGVVQSGGSLTLSCEASESILSFSTMDWFRQAPGKEREGVSCIRPRYSTTTHADSIKG RFKMYSDNAKNTIYLHMNSLQPEDTAVYYCHMYHYSSQYWGQGTQVTVSS

SEQ ID NO: 22 VHH sequence (C33B1781)

EVQVVESGGGLVQGGGSLRLSCAASGSIFSINAMGWYHQAPGKQRELVARITGGGATDYVDSV KGRFTISLDSAKSTVYLQMNSLKPEDTAVYHCYADLVTRVGSDLLYDDYWGQGTQVTVSS

SEQ ID NO: 23 VHH sequence (C33B1780)

EVQLVESGGGLVQPGGSLRLSCAVFGFSLSDYAIGWFRQAPGKEREKVSCISESDGRTYLSDSVK SRFTISRDNGKNTVYLQMNNLKPDDTGVYYCATVDQAISESSYHCEKGFGSWGQGTQVTVSS

SEQ ID NO: 24 VHH sequence (C33B1783)

EVQLVESGGGLVQAGGSLRLSCAASGFTFDDYAVGWFRQVPGKEREGVSCISSSDGATYYADSV KGRFTISSDNAKNTVYLQMNSLKPEDTAVYYCTAIIVRGSGWERAAAACRYEYSYWGQGTQVT VSS

SEQ ID NO: 25 VHH sequence (C33B1782)

EVQLVESGGGLVQAGGSLRLSCAASGFTFDDYAVGWFRQAPGKEREGISCISSSDGATYYADSV KGRFTISTDNAKNTAFLQMNSLKPEDTAVYYCTAVIVRGTGWERAAAACRYEYAYWGQGTQV TVSS

SEQ ID NO: 26 VHH sequence (C33B1789)

QVQLVESGGGLVQAGGSLRLSCAVFGFSLSDYAIGWFRQAPGKEREKVSCISESDGRTYLSDSVK SRFTISRDNGKNTVYLQMNNLKPDDTGVYYCATVDQAISESSYHCEKGFGSWGQGTQVTVSS

SEQ ID NO: 27 VHH sequence (C33B1788)

QVQLVESGGGLVQPGGSLRLSCAVFGFSLSDYAIGWFRQAPGKEREKVSCISESDGRTYLSDSVK SRFTISRDNGKNTVYLQMNNLKPDDTGVYYCATVDQAISESSYHCEKGFGSWGQGTQVTVSS

SEQ ID NO: 28 CDR1 of VH (C33B1474)
SFGMS

SEQ ID NO: 29 CDR1 of VH (C33B1522)
SYYWG

SEQ ID NO: 30 CDR1 of VH (C33B1607)
DYNIN

SEQ ID NO: 31 CDR1 of VH (C33B1601)
NYAMS

SEQ ID NO: 32 CDR1 of VH (C33B1517)
NYYWS

SEQ ID NO: 33 CDR1 of VH (C33B1495; C33B931; C33B782)
SYWMS

SEQ ID NO: 34 CDR1 of VH IC33B1478; C33B1477; C33B1476)
VSAIH

SEQ ID NO: 35 CDR1 of VH (C33B1484)
VFAIH

SEQ ID NO: 36 CDR2 of VH (C33B1474)
NIKRDGSEKYYVDSVKG

SEQ ID NO: 37 CDR2 of VH (C33B1522)
YIYYSGSTNYNPSLKS

SEQ ID NO: 38 CDR2 of VH (C33B1607)
TINPNNGVTFYNQRFKG

SEQ ID NO: 39 CDR2 of VH (C33B1601)
GISNSGYSLYYPDTLKG

SEQ ID NO: 40 CDR2 of VH (C33B1517)
HIYSTGNIHYNPSLKS

SEQ ID NO: 41 CDR2 of VH (C33B1495)
NIKRDGSEKHYVDSVKG

SEQ ID NO: 42 CDR2 of VH (C33B1478; C33B1484)
RIRSKGNSYATAYDVSVKG

SEQ ID NO: 43 CDR2 of VH (C33B1477)
RIRSKGNSYATAYAASVKG

SEQ ID NO: 44 CDR2 of VH (C33B1476)
RIRSKGNSYATAYNVSVKG

SEO ID NO: 45 CDR3 of VH (C33B1474)
DYGYFDF

SEQ ID NO: 46 CDR3 of VH (C33B1522)
MWEILGFDP

SEQ ID NO: 47 CDR3 of VH (C33B1607)
DGYDTYYAMDY

SEO ID NO: 48 CDR3 of VH (C33B 160 1)
DGGSYPYAMDY

SEQ ID NO: 49 CDR3 of VH (C33B1517)
DNGAALFDY

SEQ ID NO: 50 CDR3 of VH (C33B1495)
DYGYFDY

SEQ ID NO: 51 CDR3 of VH (C33B1478; C33B1477: C33B1476: C33B1484)
HNDKWNYYGLDV

SEQ ID NO: 52 VH sequence (C33B1474)

QVQLVESGGGLVQPGGSLRLSCAASGFTFSSFGMSWVRQAPGKGLEWVANIKRDGSEKYYVDS VKGRFTISRDNAKNSLFLQMSSLRAEDTAVYYCVRDYGYFDFWGQGTLVTVSS

SEQ ID NO: 53 VH sequence (C33B1522)

QVQLQESGPGLVKPSETLSLTCTVSGGSISSYYWGWIRQPPGKGLEWIGYIYYSGSTNYNPSLKSR VTISVDTSKNQFSLKLSSVTAADTAVYYCARMWEILGFDPWGQGTLVTVS

SEQ ID NO: 54 VH sequence (C33B1607)

EVQLQQSGPELVKPGSSVKISCKASGYTFTDYNINWVKQSHGKNLEWIGTINPNNGVTFYNQRF KGQATLTVDKSSSTAYMELRSLTSEDSAVYYCSRDGYDTYYAMDYWGQGTTLTVSS

SEQ ID NO: 55 VH sequence (C33B1607)

EVQLVESGGGLVRPGGSLKLSCAASGFTFSNYAMSWVRQTPEKRLEWVAGISNSGYSLYYPDTL KGRFTISRDNARNTLYLQMISLRSEDTAMYYCARDGGSYPYAMDYWGHGTSVTVSS

SEQ ID NO: 56 VH sequence (C33BI517)

QVQLQESGPGLVKPSETLSLTCSVSGASIRNYYWSWIRQTAGKGLEWLGHIYSTGNIHYNPSLKS RVTMSVDTSNNQFSLKLRSVTAADTAVYYCARDNGAALFDYWGQGTLVTVSS

SEQ ID NO: 57 VH sequence (C3 3B 1495)

EVQLVESGGGLVQPGGSLRLSCTASGFTFSSYWMSWVRQAPGKGLDWVANIKRDGSEKHYVDS VKGRFTISRDNAKNSLYLQMNSLRAEDSAVYYCTRDYGYFDYWGQGTLVTVSS

SEQ ID NO: 58 VH sequence (C33B1478)

EVQLVESGGGLVQPGGSLKVSCEASGFTFSVSAIHWVRQASGKGLEWIGRIRSKGNSYATAYDV SVKGRFTISRDDSKNTAYLQMDSLKTEDTAVYYCTRHNDKWNYYGLDVWGQGTTVTVSS

SEQ ID NO: 59 VH sequence (C33B1477)

EVQLVESGGGLVQPGGSLKVSCEASGFTFSVSAIHWVRQASGKGLEWIGRIRSKGNSYATAYAA SVKGRFTISRDDSKNTAYLQMDSLKTEDTAVYYCTRHNDKWNYYGLDVWGQGTTVTVSS

SEQ ID NO: 60 VH sequence (C33B1476)

EVQLVESGGGLVQPGGSLKVSCEASGFTFSVSAIHWVRQASGKGLEWIGRIRSKGNSYATAYNV SVKGRFTISRDDSKNTAYLQMDSLKTEDTAVYYCTRHNDKWNYYGLDVWGQGTTVTVSS

SEQ ID NO: 61 VH sequence (C33B1484)

EVQLVESGGGLVQPGGSLKVSCEASGFTFSVFAIHWVRQASGKGLEWIGRIRSKGNSYATAYDV SVKGRFTISRDDSKNTAYLQMDSLKTEDTAVYYCTRHNDKWNYYGLDVWGQGTTVTVSS

SEQ ID NO: 62 CDR1 of VL (C33B1474)
QASQAISNYLN

SEQ ID NO: 63 CDR1 of VL (C33B1522)
SGSSSNIGSNPVN

SEQ ID NO: 64 CDR1 of VL (C33B1607)
KASQDVRTAEA

SEQ ID NO: 65 CDR1 of VL (C33B1601)
KASQNVGTYVA

SEQ ID NO: 66 CDR1 of VL (C33B1517; C33B1516)
SGSSSNIGSNIVN

SEQ ID NO: 67 CDR1 of VL (C33B1495)
RASQGISSYLA

SEQ ID NO: 68 CDR1 of VL (C33B1478; C33B1477; C33B1476; C33B1484)
KSSQSLLFSNGYKFLD

SEQ ID NO: 69 CDR2 of VL (C33B1474)
DASNLET

SEQ ID NO: 70 CDR2 of VL (C33B1522; C33B1517)
SNNQRPS

SEQ ID NO: 71 CDR2 of VL (C33B1607)
SASNRYT

SEQ ID NO: 72 CDR2 of VL (C33B1601)
SASYRYS

SEQ ID NO: 73 CDR2 of VL (C33B1495)
AASTLQS

SEQ ID NO: 74 CDR2 of VL (C33B1478; C33B1477, C33B1476; C33B1484; C33B1475)
LGSYRAS

SEQ ID NO: 75 CDR3 of VL (C33B1474)
QHYDNLPYT

SEQ ID NO: 76 CDR3 of VL (C33B1522; C33B1517; C33B1516)
AAWDDSLNGPV

SEQ ID NO: 77 CDR3 of VL (C33B1607)
QQYYTTPRT

SEO ID NO: 78 CDR3 of VL (C33B1601)
QQYKSYPLT

SEQ ID NO: 79 CDR3 of VL (C33B1495)
QQLNSYPVT

SEQ ID NO: 80 CDR3 of VL (C33B1478; C33B1477; C33B1476; C33B1484)
MQALQTPPT

SEQ ID NO: 81 VL sequence (C33B1474)

DIQMTQSPSSLSASVGDRVTITCQASQAISNYLNWYQQKPGKAPKLLIYDASNLETGVPSRFSGS GSGTDFTFTISSLQPEDFSTYFCQHYDNLPYTFGQGTKLEIK

SEQ ID NO: 82 VL sequence (C33B1522)

QSVLTQPPSASGTPGQRVTISCSGSSSNIGSNPVNWYQQLPGTAPKLLIYSNNQRPSGVPDRFSGS KSGTSASLAISGLQSEDEADYFCAAWDDSLNGPVFGGGTKLTVL

SEQ ID NO: 83 VL sequence (C33B1607)

DIVMTQSHKFMSTSVGDRVSITCKASQDVRTAEAWYQQKPGQSPKLLIYSASNRYTGVPDRFTG SGSGTDFTFTISSVQAEDLAVYYCQQYYTTPRTFGGGTKLEIK

SEQ ID NO: 84 VL sequence (C33B1601)

DIVMTQSQKFMSTSVGDRVSVTCKASQNVGTYVAWYQQKPGHSPKALIYSASYRYSGVPDRFT GSGSGTDFTLTISNVQSEDLADYFCQQYKSYPLTFGAGTKLELK

SEQ ID NO: 85 VL sequence (C33B1517)

QSVLTQPPSASGTPGQRVTISCSGSSSNIGSNIVNWYQQFPGTAPKLLIYSNNQRPSGVPDRVSGS KSGTSASLAISGLQSEDEADYYCAAWDDSLNGPVFGPGTKVTVL

SEQ ID NO: 86 VL sequence (C33B1495)

DIQLTQSPSFLSASVGDRVTITCRASQGISSYLAWYQQKPGKAPKVLIYAASTLQSGVPSRFSGSG SGTEFTLTISSLQPEDFATYFCQQLNSYPVTFGGGTKVEIK

SEQ ID NO: 87 VL sequence (C33B1478; C33B1477; C33B1476)

DIVMTQSPLSLPVTPGEPASISCKSSQSLLFSNGYKFLDWYLQRPGQSPQLLIYLGSYRASGVPDR FSGSGSGTDFTLKISRVEAEDVGLYYCMQALQTPPTFGGGTKVEIK

SEQ ID NO: 88 VL sequence (C33B1484)

DIVMTQSPLSLPVTPGEPASISCKSSQSLLFSNGYKFLDWYLQRPGQSPQLLIYLGSYRASGVPDR FSGSGSGTDFTLKISRVEAEDVGLYYCMQALQTPPTFGQGTKVEIK

SEQ ID NO: 89 CDR1 of VH (C33B933; C33B919; C33B937; C33B125)
SYGMH

SEQ ID NO: 90 CDR2 of VH (C33B933; C33B919, C33B937, C33B125)
VISYDGSNKYYADSVKG

SEQ ID NO: 91 CDR2 of VH (C33B931; C33B782)
NIKQHGSEKYYVDSVKG

SEQ ID NO: 92 CDR3 of VH (C33B933)
DFRSLDWLPPDSTSYDGMDV

SEQ ID NO: 93 CDR3 of VH (C33B933)
DFRSFDWLPPDSTSYYGMDV

SEQ ID NO: 94 CDR3 of VH (C33B931)
DRDLGYFDY

SEQ ID NO: 95 VH sequence (C33B933)

QVQLVESGGGVVQPGRSLRLSCAASGFTFSSYGMHWVRQSPGKGLEWVAVISYDGSNKYYADSVKGRFTISRDNSKSTLYLQMNSLRAEDTAVYYCAKDFRSLDWLPPDSTSYDGMDVWGQGTTVTVSS

SEQ ID NO: 96 VH sequence (C33B919; C33B125)

QVQLVESGGGVVQPGRSLRLSCAASGFTFSSYGMHWVRQAPGKGLEWVAVISYDGSNKYYADSVKGRFTISRDNSKNTLYLQMNSLRAEDTAVYYCAKDFRSFDWLPPDSTSYYGMDVWGQGTTVTVSS

SEQ ID NO: 97 VH sequence (C33B931)

EVQLVESGGGFVQPGGSLRLSCAASGFTFSSYWMSWVRQAPGKGLEWVANIKQHGSEKYYVDSVKGRFTISRDNAKNSLYLQMNSLRAEDTAVYYCARDRDLGYFDYWGQGTLVTVSS

SEQ ID NO: 98 CDR1 of VL (C33B933)
SGHKLGDKYAS

SEQ ID NO: 99 CDR1 of VL (C33B919)
SGDKLGDKYVS

SEQ ID NO: 100 CDR1 of VL (C33B931)
SGDKLGSKFAS

SEQ ID NO: 101 CDR2 of VL (C33B933)
KDSKRPS

SEQ ID NO: 102 CDR2 of VL (C33B919; C33B125)
QDRKRPS

SEQ ID NO: 103 CDR2 of VL (C33B931)
QDSKRPS

SEQ ID NO: 104 CDR3 of VL (C33B933; C33B919; C33B931; C33B782; C33B125)
QAWDSSTW

SEQ ID NO: 105 VL sequence (C33B933)

SYELTQPPSVSVSPGQTASITCSGHKLGDKYASWYQQKPGQSPVVVIYKDSKRPSGIPERFSGSNFGNTATLTISGTQAMDEADYYCQAWDSSTVVFGGGTKLTVL

SEQ ID NO: 106 VL sequence (C33B919)

SYELTQPPSVSVSPGQTASITCSGDKLGDKYVSWYQQKPGQSPVVVIHQDRKRPSGIPERFSGSNF
GNTATLTISGTQAMDEADYYCQAWDSSTVVFGGGTKLTVL

SEQ ID NO: 107 VL sequence (C33B931)

SYELTQPPSVSVSPGQTASITCSGDKLGSKFASWYQQKPGQSPVLVIYQDSKRPSGIPERFSGSNS
GNTATLTISGTQAMDEADYYCQAWDSSTVVFGGGTKLTVL

SEQ ID NO: 108 Linker sequence
GGSEGKSSGSGSESKSTGGS

SEQ ID NO: 109 Linker sequence
GGGSGGGS

SEQ ID NO: 110 Linker sequence
GGGSGGGSGGGS

SEQ ID NO: 111 Linker sequence
GGGSGGGSGGGSGGGS

SEQ ID NO: 112 Linker sequence
GGGSGGGSGGGSGGGSGGGS

SEQ ID NO: 113 Linker sequence
GGGGSGGGGSGGGGS

SEQ ID NO: 114 Linker sequence
GGGGSGGGGSGGGGSGGGGS

SEQ ID NO: 115 Linker sequence
GGGGSGGGGSGGGGSGGGGSGGGGS

SEQ ID NO: 116 Linker sequence
GSTSGSGKPGSGEGSTKG

SEQ ID NO: 117 Linker sequence
IRPRAIGGSKPRVA

SEQ ID NO: 118 Linker sequence
GKGGSGKGGSGKGGS

SEQ ID NO: 119 Linker sequence
GGKGSGGKGSGGKGS

SEQ ID NO: 120 Linker sequence
GGGKSGGGKSGGGKS

SEQ ID NO: 121 Linker sequence
GKGKSGKGKSGKGKS

SEQ ID NO: 122 Linker sequence
GGGKSGGKGSGKGGS

SEQ ID NO: 123 Linker sequence
GKPGSGKPGSGKPGS

SEQ ID NO: 124 Linker sequence
GKPGSGKPGSGKPGSGKPGS

SEQ ID NO: 125 Linker sequence
GKGKSGKGKSGKGKSGKGKS

SEQ ID NO: 126 Linker sequence
STAGDTHLGGEDFD

SEQ ID NO: 127 Linker sequence
GEGGSGEGGSGEGGS

SEQ ID NO: 128 Linker sequence
GGEGSGGEGSGGEGS

SEQ ID NO: 129 Linker sequence
GEGESGEGESGEGES

SEQ ID NO: 130 Linker sequence
GGGESGGEGSGEGGS

SEQ ID NO: 131 Linker sequence
GEGESGEGESGEGESGEGES

SEQ ID NO: 132 Linker sequence
GSTSGSGKPGSGEGSTKG

SEQ ID NO: 133 Linker sequence
PRGASKSGSASQTGSAPGS

SEQ ID NO: 134 Linker sequence
GTAAAGAGAAGGAAAGAAG

SEQ ID NO: 135 Linker sequence
GTSGSSGSGSGGSGSGGGG

SEQ ID NO: 136 Linker sequence
GKPGSGKPGSGKPGSGKPGS

SEQ ID NO: 137 Linker sequence
GSGS

SEQ ID NO: 138 Linker sequence
APAPAPAPAP

SEQ ID NO: 139 Linker sequence
APAPAPAPAPAPAPAPAPAP

SEQ ID NO: 140 Linker sequence
AEAAAKEAAAKEAAAAKEAAAAKEAAAAKAAA

SEQ ID NO: 141 HCDR1 of antigen binding domain that binds CD3ε (CD3B219)
TYAMN

SEQ ID NO: 142 HCDR2 of antigen binding domain that binds CD3ε (CD3B219)
RIRSKYNNYATYYAASVKG

SEQ ID NO: 143 HCDR3 of antigen binding domain that binds CD3s (CD3B219)

HGNFGNSYVSWFAY

SEQ ID NO: 144 LCDR1 of antigen binding domain that binds CD3ε (CD3B219)
RSSTGAVTTSNYAN

SEQ ID NO: 145 LCDR2 of antigen binding domain that binds CD3ε (CD3B219)
GTNKRAP

SEQ ID NO: 146 LCDR3 of antigen binding domain that binds CD3ε (CD3B219)
ALWYSNLWV

SEQ ID NO: 147 VH of antigen binding domain that binds CD3ε (CD3B219)

EVQLVESGGGLVQPGGSLRLSCAASGFTFNTYAMNWVRQAPGKGLEWVARIRSKYNNYATYY
AASVKGRFTISRDDSKNSLYLQMNSLKTEDTAVYYCARHGNFGNSYVSWFAYWGQGTLVTVSS

SEQ ID NO: 148 VL of antigen binding domain that binds CD3ε (CD3B219)

QTVVTQEPSLTVSPGGTVTLTCRSSTGAVTTSNYANWVQQKPGQAPRGLIGGTNKRAPGTPARF
SGSLLGGKAALTLSGVQPEDEAEYYCALWYSNLWVFGGGTKLTVL

SEO ID NO: 149 HCDR1 of antigen binding domain that binds CD3ε (CD3B376)
NNNAAWS

SEQ ID NO: 150 HCDR2 of antigen binding domain that binds CD3s (CD3B376)
RTYYRSKWLYDYAVSVKS

SEQ ID NO: 151 HCDR3 of antigen binding domain that binds CD3ε (CD3B376)
GYSSSFDY

SEQ ID NO: 152 LCDR1 of antigen binding domain that binds CD3ε (CD3B376)
TGTSSNIGTYKFVS

SEO ID NO: 153 LCDR2 of antigen binding domain that binds CD3ε (CD3B376)
EVSKRPS

SEQ ID NO: 154 LCDR3 of antigen binding domain that binds CD3ε (CD3B376)
VSYAGSGTLL

SEQ ID NO: 155 VH of antigen binding domain that binds CD3ε (CD3B376)

QVQLQQSGPRLVRPSQTLSLTCAISGDSVFNNNAAWSWIRQSPSRGLEWLGRTYYRSKWLYDY
AVSVKSRITVNPDTSRNQFTLQLNSVTPEDTALYYCARGYSSSFDYWGQGTLVTVSS

SEQ ID NO: 156 VL of antigen binding domain that binds CD3ε (CD3B376)

QSALTQPASVSGSPGQSITISCTGTSSNIGTYKFVSWYQQHPDKAPKVLLYEVSKRPSGVSSRFSG
SKSGNTASLTISGLQAEDQADYHCVSYAGSGTLLFGGGTKLTVL

SEQ ID NO: 157 CD8a-hinge region of CAR comprising antigen binding domain that binds CD33
TSTPAPRPPTPAPTIASQPLSLRPEACRPAAGGAVHTRGLDFACD

SEQ ID NO: 158 hinge region of CAR comprising antigen binding domain that binds CD33
EPKSCDKTHTCPPCP

SEQ ID NO: 159 hinge region of CAR comprising antigen binding domain that binds CD33
ERKCCVECPPCP

SEQ ID NO: 160 hinge region of CAR comprising antigen binding domain that binds CD33
ELKTPLGDTTHTCPRCP(EPKSCDTPPPCPRCP)$_3$

SEQ ID NO: 161 hinge region of CAR comprising antigen binding domain that binds CD33
ESKYGPPCPSCP

SEQ ID NO: 162 CD8a transmembrane region of CAR comprising antigen binding domain that binds CD33
IYIWAPLAGTCGVLLLSLVITLYC

SEQ ID NO: 163 CD137 co-stimulatory domain of CAR comprising antigen binding domain that binds CD33
KRGRKKLLYIFKQPFMRPVQTTQEEDGCSCRFPEEEEGGCEL

SEQ ID NO: 164 CD137 co-stimulatory domain of CAR comprising antigen binding domain that binds CD33

RVKFSRSADAPAYKQGQNQLYNELNLGRREEYDVLDKRRGRDPEMGGKPRRKNPQEGLYNEL
QKDKMAEAYSEIGMKGERRRGKGHDGLYQGLSTATKDTYDALHMQALPPR

SEQ ID NO: 165 Intracellular signaling domain of CAR comprising antigen binding domain that binds CD33

KRGRKKLLYIFKQPFMRPVQTTQEEDGCSCRFPEEEEGGCELRVKFSRSADAPAYKQGQNQLYN
ELNLGRREEYDVLDKRRGRDPEMGGKPRRKNPQEGLYNELQKDKMAEAYSEIGMKGERRRGK
GHDGLYQGLSTATKDTYDALHMQALPPR

SEQ ID NO: 166 hinge region of CAR comprising antigen binding domain that binds CD33
ESKYGPPCPSCP

SEQ ID NO: 167 CDR1 of VH (C33B836)
NYAMN

SEQ ID NO: 168 CDR2 of VH (C33B836)
WINTNTGNPTYAQGFTG

SEQ ID NO: 169 CDR3 of VH (C33B836)
DRDRGTDY

SEQ ID NO: 170 VH sequence (C33B836)

QVQLVQSGSELRKPGASVKVSCKASGYTFTNYAMNWVRQAPGQGLEWMGWINTNTGNPTYA
QGFTGRFVFSLDTSVSSAYLQISSLKAEDTAMYYCATDRDRGTDYWGQGTLVTVSS

SEQ ID NO: 171 VH sequence (C33B782)

EVQLVESGGGLVQPGGSLRLSCAASGFTFSSYWMSWVRQAPGKGLEWVANIKQHGSEKYYVDS
VKGRFTISRDNAKNSLYLQMNSLRAEDTAVYYCARDRDLGYFDYWGQGTLVTVSS

SEQ ID NO: 172 CDR1 of VH (C33B806)
SSSYYWG

SEQ ID NO: 173 CDR2 of VH (C33B806)

SINYSGSTYYNPSLKS

SEQ ID NO: 174 CDR3 of VH (C33B806)
LDGYESPFDY

SEQ ID NO: 175 VH sequence (C33B806)

QLQLQESGPGLVKPSETLSLTCTVSGGSISSSSYYWGWIRQPPGKGLDWIGSINYSGSTYYNPSLK SRVTISVDTSKIQFSLKLRSVTAADTAVYYCARLDGYESPFDYWGQGTLVTVSS

SEQ ID NO: 176 CDR1 of VH (C33B904)
DYAMH

SEQ ID NO: 177 CDR2 of VH (C33B904)
GIGWSGGSIVYADSVKG

SEQ ID NO: 178 CDR3 of VH (C33B904)
DSPYGDFFDY

SEQ ID NO: 179 VH sequence (C33B904)

EVQLVESGGGLVQPGRSLRLSCAASGFTFDDYAMHWVRQAPGKGLEWVSGIGWSGGSIVYADS VKGRFTISRDNAKNSLYLQMNSLRAEDTALYYCAKDSPYGDFFDYWGQGTLVTVSS

SEQ ID NO: 180 CDR3 of VH (C33B937)
DFRSFDWLPPDSASYHGMDV

SEQ ID NO: 181 VH sequence (C33B937)

QVQLVESGGGVVQPGRSLRLSCVASGFTFSSYGMHWVRQAPGKGLEWVAVISYDGSNKYYAD SVKGRFTISRDNSKNTLYLQMNSLRAEGTAVYYCAKDFRSFDWLPPDSASYHGMDVWGQGTTV TVSS

SEQ ID NO: 182 CDR1 of VL (C33B836)
TGTSSDVGDYNYVS

SEQ ID NO: 183 CDR2 of VL (C33B836)
DVSNRPS

SEQ ID NO: 184 CDR3 of VL (C33B836)
SSYSSSSALEV

SEQ ID NO: 185 VL sequence (C33B836)

QSALTQPASVSGSPGQSITISCTGTSSDVGDYNYVSWYQQHPGKVPKLMIYDVSNRPSGVSNRFS GSMSGNTASLTISGLQAEDEADYYCSSYSSSSALEVFGGGTKLTVL

SEQ ID NO: 186 CDR1 of VL (C33B782)
SGNKLGAKFAS

SEQ ID NO: 187 CDR2 of VL (C33B782)
QDNKRPS

SEQ ID NO: 188 VL sequence (C33B782)

SYELTQPPSVSVSPGQTASITCSGNKLGAKFASWYQQKPGQSPVLVIYQDNKRPSGIPERFSGSNSGNTATLTISGTQAVDEADYYCQAWDSSTVVFGGGTKLTVL

SEQ ID NO: 189 CDR1 of VL (C33B806)
RASQGISSWLA

SEQ ID NO: 190 CDR2 of VL (C33B806)
AASSLQS

SEQ ID NO: 191 CDR3 of VL (C33B806)
QQANSFPFT

SEQ ID NO: 192 VL sequence (C33B806)

DIQMTQSPSSVSASVGDRVTITCRASQGISSWLAWYQQKPGKAPKLLIYAASSLQSGVPSRFSGSGSGTDFTLTISSLQPEDFATYYCQQANSFPFTFGPGTKVDIK

SEQ ID NO: 193 CDR1 of VL (C33B904)
KSSQTVFYSSNNKNYLA

SEQ ID NO: 194 CDR2 of VL (C33B904)
WASTRKS

SEQ ID NO: 195 CDR3 of VL (C33B904)
QHYYSTPYT

SEQ ID NO: 196 VL sequence (C33B904)

DIVMTQSPDSLAVSLGERATINCKSSQTVFYSSNNKNYLAWYQQKPGQPPKLLISWASTRKSGVPDRFSGSGSGTDFTLTVSSLQAEDVAVYYCQHYYSTPYTFGQGTKLEIK

SEQ ID NO: 197 CDR1 of VL (C33B937)
SGDKLGDKYAS

SEQ ID NO: 198 CDR2 of VL (C33B937)
QDGKRPS

SEQ ID NO: 199 CDR3 of VL (C33B937)
QAWDRNTW

SEQ ID NO: 200 VL sequence (C33B937)

SYELTQPPSVSVSPGQTASITCSGDKLGDKYASWYQQKPGQSPVLVIYQDGKRPSGIPERFSGSNFGNKATLTISGTQAMDEADYYCQAWDRNTVVFGGGTKLTVL

SEQ ID NO: 201 CDR1 of VL (C33B125)
SGDKLGDKYVC

SEQ ID NO: 202 VL sequence (C33B125)

SYELTQPPSVSVSPGQTASITCSGDKLGDKYVCWYQQKPGQSPVVVIHQDRKRPSGIPERFSGSNF GNTATLTISGTQAMDEADYYCQAWDSSTVVFGGGTKLTVL

SEQ ID NO: 203 scFv (C33B836)

QSALTQPASVSGSPGQSITISCTGTSSDVGDYNYVSWYQQHPGKVPKLMIYDVSNRPSGVSNRFS GSMSGNTASLTISGLQAEDEADYYCSSYSSSSALEVFGGGTKLTVLGGSEGKSSGSGSESKSTGG SQVQLVQSGSELRKPGASVKVSCKASGYTFTNYAMNWVRQAPGQGLEWMGWINTNTGNPTYA QGFTGRFVFSLDTSVSSAYLQISSLKAEDTAMYYCATDRDRGTDYWGQGTLVTVSS

SEQ ID NO: 204 scFv (C33B782)

SYELTQPPSVSVSPGQTASITCSGNKLGAKFASWYQQKPGQSPVLVIYQDNKRPSGIPERFSGSNS GNTATLTISGTQAVDEADYYCQAWDSSTVVFGGGTKLTVLGGSEGKSSGSGSESKSTGGSEVQL VESGGGLVQPGGSLRLSCAASGFTFSSYWMSWVRQAPGKGLEWVANIKQHGSEKYYVDSVKG RFTISRDNAKNSLYLQMNSLRAEDTAVYYCARDRDLGYFDYWGQGTLVTVSS

SEQ ID NO: 205 scFv (C33B806)

DIQMTQSPSSVSASVGDRVTITCRASQGISSWLAWYQQKPGKAPKLLIYAASSLQSGVPSRFSGS GSGTDFTLTISSLQPEDFATYYCQQANSFPFTFGPGTKVDIKGGSEGKSSGSGSESKSTGGSQLQL QESGPGLVKPSETLSLTCTVSGGSISSSSYYWGWIRQPPGKGLDWIGSINYSGSTYYNPSLKSRVTI SVDTSKIQFSLKLRSVTAADTAVYYCARLDGYESPFDYWGQGTLVTVSS

SEQ ID NO: 206 scFv (C33B904)

DIVMTQSPDSLAVSLGERATINCKSSQTVFYSSNNKNYLAWYQQKPGQPPKLLISWASTRKSGVP DRFSGSGSGTDFTLTVSSLQAEDVAVYYCQHYYSTPYTFGQGTKLEIKGGSEGKSSGSGSESKST GGSEVQLVESGGGLVQPGRSLRLSCAASGFTFDDYAMHWVRQAPGKGLEWVSGIGWSGGSIVY ADSVKGRFTISRDNAKNSLYLQMNSLRAEDTALYYCAKDSPYGDFFDYWGQGTLVTVSS

SEQ ID NO: 207 scFv (C33B933)

SYELTQPPSVSVSPGQTASITCSGHKLGDKYASWYQQKPGQSPVVVIYKDSKRPSGIPERFSGSNF GNTATLTISGTQAMDEADYYCQAWDSSTVVFGGGTKLTVLGGSEGKSSGSGSESKSTGGSQVQL VESGGGVVQPGRSLRLSCAASGFTFSSYGMHWVRQSPGKGLEWVAVISYDGSNKYYADSVKGR FTISRDNSKSTLYLQMNSLRAEDTAVYYCAKDFRSLDWLPPDSTSYDGMDVWGQGTTVTVSS

SEQ ID NO: 208 scFv (C33B937)

SYELTQPPSVSVSPGQTASITCSGDKLGDKYASWYQQKPGQSPVLVIYQDGKRPSGIPERFSGSNF GNKATLTISGTQAMDEADYYCQAWDRNTVVFGGGTKLTVLGGSEGKSSGSGSESKSTGGSQVQ LVESGGGVVQPGRSLRLSCVASGFTFSSYGMHWVRQAPGKGLEWVAVISYDGSNKYYADSVK GRFTISRDNSKNTLYLQMNSLRAEGTAVYYCAKDFRSFDWLPPDSASYHGMDVWGQGTTVTVS S

SEQ ID NO: 209 scFv (C33B919)

SYELTQPPSVSVSPGQTASITCSGDKLGDKYVSWYQQKPGQSPVVVIHQDRKRPSGIPERFSGSNF GNTATLTISGTQAMDEADYYCQAWDSSTVVFGGGTKLTVLGGSEGKSSGSGSESKSTGGSQVQL VESGGGVVQPGRSLRLSCAASGFTFSSYGMHWVRQAPGKGLEWVAVISYDGSNKYYADSVKG RFTISRDNSKNTLYLQMNSLRAEDTAVYYCAKDFRSFDWLPPDSTSYYGMDVWGQGTTVTVSS

SEQ ID NO: 210 scFv (C33B125)

SYELTQPPSVSVSPGQTASITCSGDKLGDKYVCWYQQKPGQSPVVVIHQDRKRPSGIPERFSGSNF GNTATLTISGTQAMDEADYYCQAWDSSTVVFGGGTKLTVLGGSEGKSSGSGSESKSTGGSQVQL VESGGGVVQPGRSLRLSCAASGFTFSSYGMHWVRQAPGKGLEWVAVISYDGSNKYYADSVKG RFTISRDNSKNTLYLQMNSLRAEDTAVYYCAKDFRSFDWLPPDSTSYYGMDVWGQGTTVTVSS

SEQ ID NO: 211 scFv (C33B931)

SYELTQPPSVSVSPGQTASITCSGDKLGSKFASWYQQKPGQSPVLVIYQDSKRPSGIPERFSGSNS GNTATLTISGTQAMDEADYYCQAWDSSTVVFGGGTKLTVLGGSEGKSSGSGSESKSTGGSEVQL VESGGGFVQPGGSLRLSCAASGFTFSSYWMSWVRQAPGKGLEWVANIKQHGSEKYYVDSVKG RFTISRDNAKNSLYLQMNSLRAEDTAVYYCARDRDLGYFDYWGQGTLVTVSS

SEQ ID NO: 212 scFv (C33B1474)

DIQMTQSPSSLSASVGDRVTITCQASQAISNYLNWYQQKPGKAPKLLIYDASNLETGVPSRFSGS GSGTDFTFTISSLQPEDFSTYFCQHYDNLPYTFGQGTKLEIKGGSEGKSSGSGSESKSTGGSQVQL VESGGGLVQPGGSLRLSCAASGFTFSSFGMSWVRQAPGKGLEWVANIKRDGSEKYYVDSVKGR FTISRDNAKNSLFLQMSSLRAEDTAVYYCVRDYGYFDFWGQGTLVTVSS

SEQ ID NO: 213 scPv (C33B1522)

QSVLTQPPSASGTPGQRVTISCSGSSSNIGSNPVNWYQQLPGTAPKLLIYSNNQRPSGVPDRFSGS KSGTSASLAISGLQSEDEADYFCAAWDDSLNGPVFGGGTKLTVLGGSEGKSSGSGSESKSTGGSQ VQLQESGPGLVKPSETLSLTCTVSGGSISSYYWGWIRQPPGKGLEWIGYIYYSGSTNYNPSLKSRV TISVDTSKNQFSLKLSSVTAADTAVYYCARMWEILGFDPWGQGTLVTVS

SEQ ID NO: 214 scFv (C33B1607)

DIVMTQSHKFMSTSVGDRVSITCKASQDVRTAEAWYQQKPGQSPKLLIYSASNRYTGVPDRFTGSGSGTDFTFTISSVQAEDLAVYYCQQYYTTPRTFGGGTKLEIKGGSEGKSSGSGSESKSTGGSEVQLQQSGPELVKPGSSVKISCKASGYTFTDYNINWVKQSHGKNLEWIGTINPNNGVTFYNQRFKGQATLTVDKSSSTAYMELRSLTSEDSAVYYCSRDGYDTYYAMDYWGQGTTLTVSS

SEQ ID NO: 215 scFv (C33B1601)

DIVMTQSQKFMSTSVGDRVSVTCKASQNVGTYVAWYQQKPGHSPKALIYSASYRYSGVPDRFTGSGSGTDFTLTISNVQSEDLADYFCQQYKSYPLTFGAGTKLELKGGSEGKSSGSGSESKSTGGSEVQLVESGGGLVRPGGSLKLSCAASGFTFSNYAMSWVRQTPEKRLEWVAGISNSGYSLYYPDTLKGRFTISRDNARNTLYLQMISLRSEDTAMYYCARDGGSYPYAMDYWGHGTSVTVSS

SEQ ID NO: 216 scFv (C33B1517)

QSVLTQPPSASGTPGQRVTISCSGSSSNIGSNIVNWYQQFPGTAPKLLIYSNNQRPSGVPDRVSGSKSGTSASLAISGLQSEDEADYYCAAWDDSLNGPVFGPGTKVTVLGGSEGKSSGSGSESKSTGGSQVQLQESGPGLVKPSETLSLTCSVSGASIRNYYWSWIRQTAGKGLEWLGHIYSTGNIHYNPSLKSRVTMSVDTSNNQFSLKLRSVTAADTAVYYCARDNGAALFDYWGQGTLVTVSS

SEQ ID NO: 217 scFv (C33B1495)

DIQLTQSPSFLSASVGDRVTITCRASQGISSYLAWYQQKPGKAPKVLIYAASTLQSGVPSRFSGSGSGTEFTLTISSLQPEDFATYFCQQLNSYPVTFGGGTKVEIKGGSEGKSSGSGSESKSTGGSEVQLVESGGGLVQPGGSLRLSCTASGFTFSSYWMSWVRQAPGKGLDWVANIKRDGSEKHYVDSVKGRFTISRDNAKNSLYLQMNSLRAEDSAVYYCTRDYGYFDYWGQGTLVTVSS

SEO ID NO: 218 scFv (C33B1478)

DIVMTQSPLSLPVTPGEPASISCKSSQSLLFSNGYKFLDWYLQRPGQSPQLLIYLGSYRASGVPDRFSGSGSGTDFTLKISRVEAEDVGLYYCMQALQTPPTFGGGTKVEIKGGSEGKSSGSGSESKSTGGSEVQLVESGGGLVQPGGSLKVSCEASGFTFSVSAIHWVRQASGKGLEWIGRIRSKGNSYATAYDVSVKGRFTISRDDSKNTAYLQMDSLKTEDTAVYYCTRHNDKWNYYGLDVWGQGTTVTVSS

SEQ ID NO: 219 scFv (C33B1477)

DIVMTQSPLSLPVTPGEPASISCKSSQSLLFSNGYKFLDWYLQRPGQSPQLLIYLGSYRASGVPDR
FSGSGSGTDFTLKISRVEAEDVGLYYCMQALQTPPTFGGGTKVEIKGGSEGKSSGSGSESKSTGG
SEVQLVESGGGLVQPGGSLKVSCEASGFTFSVSAIHWVRQASGKGLEWIGRIRSKGNSYATAYA
ASVKGRFTISRDDSKNTAYLQMDSLKTEDTAVYYCTRHNDKWNYYGLDVWGQGTTVTVSS

SEQ ID NO: 220 scFv (C33B1476)

DIVMTQSPLSLPVTPGEPASISCKSSQSLLFSNGYKFLDWYLQRPGQSPQLLIYLGSYRASGVPDR
FSGSGSGTDFTLKISRVEAEDVGLYYCMQALQTPPTFGGGTKVEIKGGSEGKSSGSGSESKSTGG
SEVQLVESGGGLVQPGGSLKVSCEASGFTFSVSAIHWVRQASGKGLEWIGRIRSKGNSYATAYN
VSVKGRFTISRDDSKNTAYLQMDSLKTEDTAVYYCTRHNDKWNYYGLDVWGQGTTVTVSS

SEQ ID NO: 221 scFv (C33B1484)

DIVMTQSPLSLPVTPGEPASISCKSSQSLLFSNGYKFLDWYLQRPGQSPQLLIYLGSYRASGVPDR
FSGSGSGTDFTLKISRVEAEDVGLYYCMQALQTPPTFGQGTKVEIKGGSEGKSSGSGSESKSTGG
SEVQLVESGGGLVQPGGSLKVSCEASGFTFSVFAIHWVRQASGKGLEWIGRIRSKGNSYATAYD
VSVKGRFTISRDDSKNTAYLQMDSLKTEDTAVYYCTRHNDKWNYYGLDVWGQGTTVTVSS

SEQ ID NO: 222 wild-type IgG1

ASTKGPSVFPLAPSSKSTSGGTAALGCLVKDYFPEPVTVSWNSGALTSGVHTFPAVLQSSGLYSL
SSVVTVPSSSLGTQTYICNVNHKPSNTKVDKKVEPKSCDKTHTCPPCPAPELLGGPSVFLFPPKPK
DTLMISRTPEVTCVVVDVSHEDPEVKFNWYVDGVEVHNAKTKPREEQYNSTYRVVSVLTVLHQ
DWLNGKEYKCKVSNKALPAPIEKTISKAKGQPREPQVYTLPPSRDELTKNQVSLTCLVKGFYPSD
IAVEWESNGQPENNYKTTPPVLDSDGSFFLYSKLTVDKSRWQQGNVFSCSVMHEALHNHYTQK
SLSLSPGK

SEQ ID NO: 223 wild-type IgG2

ASTKGPSVFPLAPCSRSTSESTAALGCLVKDYFPEPVTVSWNSGALTSGVHTFPAVLQSSGLYSLS
SVVTVPSSNFGTQTYTCNVDHKPSNTKVDKTVERKCCVECPPCPAPPVAGPSVFLFPPKPKDTLM
ISRTPEVTCVVVDVSHEDPEVQFNWYVDGVEVHNAKTKPREEQFNSTFRVVSVLTVVHQDWLN
GKEYKCKVSNKGLPAPIEKTISKTKGQPREPQVYTLPPSREEMTKNQVSLTCLVKGFYPSDISVE
WESNGQPENNYKTTPPMLDSDGSFFLYSKLTVDKSRWQQGNVFSCSVMHEALHNHYTQKSLSL
SPGK

SEQ ID NO: 224 wild-type IgG4

ASTKGPSVFPLAPCSRSTSESTAALGCLVKDYFPEPVTVSWNSGALTSGVHTFPAVLQSSGLYSLS SVVTVPSSSLGTKTYTCNVDHKPSNTKVDKRVESKYGPPCPSCPAPEFLGGPSVFLFPPKPKDTL MISRTPEVTCVVVDVSQEDPEVQFNWYVDGVEVHNAKTKPREEQFNSTYRVVSVLTVLHQDWL NGKEYKCKVSNKGLPSSIEKTISKAKGQPREPQVYTLPPSQEEMTKNQVSLTCLVKGFYPSDIAV EWESNGQPENNYKTTPPVLDSDGSFFLYSRLTVDKSRWQEGNVFSCSVMHEALHNHYTQKSLSL SLGK

SEQ ID NO: 225 CD33 antigen: HSA N-terminal Fusion: CynoCD33ECD-HSA(C34S)-6xHis

DPRVRLEVQESVTVQEGLCVLVPCTFFHPVPYHTRNSPVHGYWFREGAIVSLDSPVATNKLDQE VQEETQGRFRLLGDPSRNNCSLSIVDARRRDNGSYFFRMEKGSTKYSYKSTQLSVHVTDLTHRP

QILIPGALDPDHSKNLTCSVPWACEQGTPPIFSWMSAAPTSLGLRTTHSSVLIITPRPQDHGTNLTC QVKFPGAGVTTERTIQLNVSYASQNPRTDIFLGDGSGKQGVVQGSGSGSENLYFQGVRSSSDAH KSEVAHRFKDLGEENFKALVLIAFAQYLQQSPFEDHVKLVNEVTEFAKTCVADESAENCDKSLH TLFGDKLCTVATLRETYGEMADCCAKQEPERNECFLQHKDDNPNLPRLVRPEVDVMCTAFHDN EETFLKKYLYEIARRHPYFYAPELLFFAKRYKAAFTECCQAADKAACLLPKLDELRDEGKASSA KQRLKCASLQKFGERAFKAWAVARLSQRFPKAEFAEVSKLVTDLTKVHTECCHGDLLECADDR ADLAKYICENQDSISSKLKECCEKPLLEKSHCIAEVENDEMPADLPSLAADFVESKDVCKNYAEA KDVFLGMFLYEYARRHPDYSVVLLLRLAKTYETTLEKCCAAADPHECYAKVFDEFKPLVEEPQ NLIKQNCELFEQLGEYKFQNALLVRYTKKVPQVSTPTLVEVSRNLGKVGSKCCKHPEAKRMPCA EDYLSVVLNQLCVLHEKTPVSDRVTKCCTESLVNRRPCFSALEVDETYVPKEFNAETFTFHADIC TLSEKERQIKKQTALVELVKHKPKATKEQLKAVMDDFAAFVEKCCKADDKETCFAEEGKKLVA ASQAALGLGGGSHHHHHH

SEQ ID NO: 226 CD33 antigen: HSA N-terminal Fusion: Human CD33ECD-Tev-HSA(C34S)-6xHis

DPNFWLQVQESVTVQEGLCVLVPCTFFHPIPYYDKNSPVHGYWFREGAIISRDSPVATNKLDQEV
QEETQGRFRLLGDPSRNNCSLSIVDARRRDNGSYFFRMERGSTKYSYKSPQLSVHVTDLTHRPKI
LIPGTLEPGHSKNLTCSVSWACEQGTPPIFSWLSAAPTSLGPRTTHSSVLIITPRPQDHGTNLTCQV
KFAGAGVTTERTIQLNVTYVPQNPTTGIFPGDGSGKQETRAGVVHGSGSGSENLYFQGVRSSSDA
HKSEVAHRFKDLGEENFKALVLIAFAQYLQQSPFEDHVKLVNEVTEFAKTCVADESAENCDKSL
HTLFGDKLCTVATLRETYGEMADCCAKQEPERNECFLQHKDDNPNLPRLVRPEVDVMCTAFHD
NEETFLKKYLYEIARRHPYFYAPELLFFAKRYKAAFTECCQAADKAACLLPKLDELRDEGKASS
AKQRLKCASLQKFGERAFKAWAVARLSQRFPKAEFAEVSKLVTDLTKVHTECCHGDLLECADD
RADLAKYICENQDSISSKLKECCEKPLLEKSHCIAEVENDEMPADLPSLAADFVESKDVCKNYAE
AKDVFLGMFLYEYARRHPDYSVVLLLRLAKTYETTLEKCCAAADPHECYAKVFDEFKPLVEEP
QNLIKQNCELFEQLGEYKFQNALLVRYTKKVPQVSTPTLVEVSRNLGKVGSKCCKHPEAKRMPC
AEDYLSVVLNQLCVLHEKTPVSDRVTKCCTESLVNRRPCFSALEVDETYVPKEFNAETFTFHADI
CTLSEKERQIKKQTALVELVKHKPKATKEQLKAVMDDFAAFVEKCCKADDKETCFAEEGKKLV
AASQAALGLGGGSHHHHHH

SEQ ID NO: 227 CD33 antigen: HSA N-terminal Fusion: hCD33 Ig-like V-type domain (Uniprot P20138, V136-H259) fused to HSA and 6xHis Tag at C-terminus

DPNFWLQVQESVTVQEGLCVLVPCTFFHPIPYYDKNSPVHGYWFREGAIISRDSPVATNKLDQEV
QEETQGRFRLLGDPSRNNCSLSIVDARRRDNGSYFFRMERGSTKYSYKSPQLSVHVTDLTHRDA
HKSEVAHRFKDLGEENFKALVLIAFAQYLQQSPFEDHVKLVNEVTEFAKTCVADESAENCDKSL

HTLFGDKLCTVATLRETYGEMADCCAKQEPERNECFLQHKDDNPNLPRLVRPEVDVMCTAFHD
NEETFLKKYLYEIARRHPYFYAPELLFFAKRYKAAFTECCQAADKAACLLPKLDELRDEGKASS
AKQRLKCASLQKFGERAFKAWAVARLSQRFPKAEFAEVSKLVTDLTKVHTECCHGDLLECADD
RADLAKYICENQDSISSKLKECCEKPLLEKSHCIAEVENDEMPADLPSLAADFVESKDVCKNYAE
AKDVFLGMFLYEYARRHPDYSVVLLLRLAKTYETTLEKCCAAADPHECYAKVFDEFKPLVEEP
QNLIKQNCELFEQLGEYKFQNALLVRYTKKVPQVSTPTLVEVSRNLGKVGSKCCKHPEAKRMPC
AEDYLSVVLNQLCVLHEKTPVSDRVTKCCTESLVNRRPCFSALEVDETYVPKEFNAETFTFHADI
CTLSEKERQIKKQTALVELVKHKPKATKEQLKAVMDDFAAFVEKCCKADDKETCFAEEGKKLV
AASQAALGLHHHHHH

SEQ ID NO: 228 CD33 antigen: HSA N-terminal Fusion: hCD33 Ig-like C2-type domain (Uniprot P20138, V136-H259) fused to HSA and 6xHis Tag at C-terminus

VHVTDLTHRPKILIPGTLEPGHSKNLTCSVSWACEQGTPPIFSWLSAAPTSLGPRTTHSSVLIITPRP
QDHGTNLTCQVKFAGAGVTTERTIQLNVTYVPQNPTTGIFPGDGSGKQETRAGVVHDAHKSEV
AHRFKDLGEENFKALVLIAFAQYLQQSPFEDHVKLVNEVTEFAKTCVADESAENCDKSLHTLFG
DKLCTVATLRETYGEMADCCAKQEPERNECFLQHKDDNPNLPRLVRPEVDVMCTAFHDNEETF
LKKYLYEIARRHPYFYAPELLFFAKRYKAAFTECCQAADKAACLLPKLDELRDEGKASSAKQRL
KCASLQKFGERAFKAWAVARLSQRFPKAEFAEVSKLVTDLTKVHTECCHGDLLECADDRADLA
KYICENQDSISSKLKECCEKPLLEKSHCIAEVENDEMPADLPSLAADFVESKDVCKNYAEAKDVF
LGMFLYEYARRHPDYSVVLLLRLAKTYETTLEKCCAAADPHECYAKVFDEFKPLVEEPQNLIKQ
NCELFEQLGEYKFQNALLVRYTKKVPQVSTPTLVEVSRNLGKVGSKCCKHPEAKRMPCAEDYL
SVVLNQLCVLHEKTPVSDRVTKCCTESLVNRRPCFSALEVDETYVPKEFNAETFTFHADICTLSE
KERQIKKQTALVELVKHKPKATKEQLKAVMDDFAAFVEKCCKADDKETCFAEEGKKLVAASQ
AALGLHHHHHH

SEQ ID NO: 229 CD33 antigen: hCD33_Ig-like V-Type domain

DPNFWLQVQESVTVQEGLCVLVPCTFFHPIPYYDKNSPVHGYWFREGAIISRDSPVATNKLDQEV
QEETQGRFRLLGDPSRNNCSLSIVDARRRDNGSYFFRMERGSTKYSYKSPQLSVHVTDLTHRGSH
HHHHH

SEQ ID NO: 230 CD33 antigen: hCD33 Ig-like C2-type domain

VHVTDLTHRPKILIPGTLEPGHSKNLTCSVSWACEQGTPPIFSWLSAAPTSLGPRTTHSSVLIITPRP
QDHGTNLTCQVKFAGAGVTTERTIQLNVTYVPQNPTTGIFPGDGSGKQETRAGVVHGSHHHHH
H

SEQ ID NO: 231 CD33 antigen: MuIgV Hu IgC2 chimera CD33-6xHis _v1

QDLEFQLVAPESVTVEEGLCVHVPCSVFYPSIKLTLGPVTGSWLRKGVSLHEDSPVATSDPRQLV
QKATQGRFQLLGDPQKHDCSLFIRDAQKNDTGMYFFRVVREPFVRYSYKKSQLSLHVTSLSRTP
KILIPGTLEPGHSKNLTCSVSWACEQGTPPIFSWLSAAPTSLGPRTTHSSVLIITPRPQDHGTNLTCQ
VKFAGAGVTTERTIQLNVTYVPQNPTTGIFPGDGSGKQETRAGVVHGSHHHHHH

SEQ ID NO: 232 CD33 antigen: MuIgV cvno IgC2 chimera CD33-6xHis_v1

QDLEFQLVAPESVTVEEGLCVHVPCSVFYPSIKLTLGPVTGSWLRKGVSLHEDSPVATSDPRQLV
QKATQGRFQLLGDPQKHDCSLFIRDAQKNDTGMYFFRVVREPFVRYSYKKSQLSLHVTSLSRTP
QILIPGALDPDHSKNLTCSVPWACEQGTPPIFSWMSAAPTSLGLRTTHSSVLIITPRPQDHGTNLTC
QVKFPGAGVTTERTIQLNVSYASQNPRTDIFLGDGSGRKARKQGVVQGSHHHHHH

SEQ ID NO: 233 CD33 antigen: Human CD33 ECD (D18-H259) from Uniprot P20138 with C-terminal 6xHis tag

DPNFWLQVQESVTVQEGLCVLVPCTFFHPIPYYDKNSPVHGYWFREGAIISRDSPVATNKLDQEV QEETQGRFRLLGDPSRNNCSLSIVDARRRDNGSYFFRMERGSTKYSYKSPQLSVHVTDLTHRPKI LIPGTLEPGHSKNLTCSVSWACEQGTPPIFSWLSAAPTSLGPRTTHSSVLIITPRPQDHGTNLTCQV KFAGAGVTTERTIQLNVTYVPQNPTTGIFPGDGSGKQETRAGVVHGSHHHHHH

SEQ ID NO: 234 CD33 antigen: Cyno CD33 ECD (D37-Q274) from XP 005590138.1 with C-terminal 6xHis tag

DPRVRLEVQESVTVQEGLCVLVPCTFFHPVPYHTRNSPVHGYWFREGAIVSLDSPVATNKLDQE VQEETQGRFRLLGDPSRNNCSLSIVDARRRDNGSYFFRMEKGSTKYSYKSTQLSVHVTDLTHRP QILIPGALDPDHSKNLTCSVPWACEQGTPPIFSWMSAAPTSLGLRTTHSSVLIITPRPQDHGTNLTC QVKFPGAGVTTERTIQLNVSYASQNPRTDIFLGDGSGKQGVVQGSHHHHHH

SEQ ID NO: 235 CD33 antigen: Human CD33 ECD (D18-G241) from Uniprot P20138 with 6xHis tag

DPNFWLQVQESVTVQEGLCVLVPCTFFHPIPYYDKNSPVHGYWFREGAIISRDSPVATNKLDQEV QEETQGRFRLLGDPSRNNCSLSIVDARRRDNGSYFFRMERGSTKYSYKSPQLSVHVTDLTHRPKI LIPGTLEPGHSKNLTCSVSWACEQGTPPIFSWLSAAPTSLGPRTTHSSVLIITPRPQDHGTNLTCQV KFAGAGVTTERTIQLNVTYVPQNPTTGHHHHHH

SEQ ID NO: 236 HCDR1 sequence of anti-TRGV9 mAbs LP7A5_1. LP7A5_2, LP7A5_3, LP7A5_4
DHYIN

SEQ ID NO: 237 HCDR2 sequence of anti-TRGV9 mAbs LP7A5_1, LP7A5_2, LP7A5_3, LP7A5 4
QIYPGDGNTYYNQKFKG

SEQ ID NO: 238 HCDR3 sequence of anti-TRGV9 mAb LP7A5_1
NYGDYTIDF

SEQ ID NO: 239 LCDR1 sequence of anti-TRGV9 mAbs LP7A5_1, LP7A5 2, LP7A5_3, LP7A5_4
KSSQSLLYSSNQKNYLA

SEQ ID NO: 240 LCDR2 sequence of anti-TRGV9 mAbs LP7A5_1, LP7A5_2, LP7A5_3, LP7A5_4
WASTRES

SEQ ID NO: 241 LCDR3 sequence of anti-TRGV9 mAbs LP7A5_1, LP7A5 2, LP7A5 3, LP7A5 4
QQYYRYHT

SEQ ID NO: 242 HCDR3 sequence of anti-TRGV9 mAb LP7A5_2
NMGMYTIDF

SEQ ID NO: 243 HCDR3 sequence of anti-TRGV9 mAb LP7A5 3
NMGMYTLDF

SEQ ID NO: 244 HCDR3 sequence of anti-TRGV9 mAb_LP7A5_4
NYGDYTLDF

SEQ ID NO: 245 Heavy Chain Sequence of LP7A5_1

EVQLQQSGAELARPGASVKLSCKASGFTFTDHYINWVKQRTGQGLEWIGQIYPGDGNTYYNQK FKGKATLTADKSSSTAYMQLSSLTSEDSAVYFCAPNYGDYTIDFWGQGTSVTVSS

SEQ ID NO: 246 Heavy Chain Sequence of LP7A5_2

EVQLQQSGAELARPGASVKLSCKASGFTFTDHYINWVKQRTGQGLEWIGQIYPGDGNTYYNQK FKGKATLTADKSSSTAYMQLSSLTSEDSAVYFCAPNMGMYTIDFWGQGTSVTVSS

SEQ ID NO: 247 Heavy Chain Sequence of LP7A5_3

EVQLQQSGAELARPGASVKLSCKASGFTFTDHYINWVKQRTGQGLEWIGQIYPGDGNTYYNQK FKGKATLTADKSSSTAYMQLSSLTSEDSAVYFCAPNMGMYTLDFWGQGTSVTVSS

SEQ ID NO: 248 Heavy Chain Sequence of LP7A54

EVQLQQSGAELARPGASVKLSCKASGFTFTDHYINWVKQRTGQGLEWIGQIYPGDGNTYYNQK FKGKATLTADKSSSTAYMQLSSLTSEDSAVYFCAPNMGMYTLDFWGQGTSVTVSS

SEQ ID NO: 249 Light Chain Sequence of LP7A5_1

DIVMSQSPSSLAVSVGEKVTMSCKSSQSLLYSSNQKNYLAWYQQKPGQSPKLLIYWASTRESGV PDRFTGSGSGTDFTLTISSVKAEDLAVYYCQQYYRYHTFGTGTKLEIK

SEQ ID NO: 250 Anti-TRGV9 hole arm sequence

MAWVWTLLFLMAAAQSIQADIVMSQSPSSLAVSVGEKVTMSCKSSQSLLYSSNQKNYLAWYQ QKPGQSPKLLIYWASTRESGVPDRFTGSGSGTDFTLTISSVKAEDLAVYYCQQYYRYHTFGTGTK LEIKRTVAAPSVFIFPPSDEQLKSGTASVVCLLNNFYPREAKVQWKVDNALQSGNSQESVTEQDS KDSTYSLSSTLTLSKADYEKHKVYACEVTHQGLSSPVTKSFNRGECGGSEGKSSGSGSESKSTEG KSSGSGSESKSTGGSEVQLQQSGAELARPGASVKLSCKASGFTFTDHYINWVKQRTGQGLEWIG QIYPGDGNTYYNQKFKGKATLTADKSSSTAYMQLSSLTSEDSAVYFCAPNYGDYTIDFWGQGTS VTVSSASTKGPSVFPLAPCSRSTSESTAALGCLVKDYFPEPVTVSWNSGALTSGVHTFPAVLQSSG LYSLSSVVTVPSSSLGTKTYTCNVDHKPSNTKVDKRVESKYGPPCPPCPAPEAAGGPSVFLFPPKP KDTLMISRTPEVTCVVVDVSQEDPEVQFNWYVDGVEVHNAKTKPREEQFNSTYRVVSVLTVLH QDWLNGKEYKCKVSNKGLPSSIEKTISKAKGQPREPQVYTLPPSQEEMTKNQVSLSCAVKGFYP SDIAVEWESNGQPENNYKTTPPVLDSDGSFFLVSRLTVDKSRWQEGNVFSCSVMHEALHNRFTQ KSLSLSLGK

SEQ ID NO: 251 Anti-CD33 knob arm sequence

MAWVWTLLFLMAAAQSIQADIVMTQSPDSLAVSLGERATINCKSSQTVFYSSNNKNYLAWYQQ
KPGQPPKLLISWASTRKSGVPDRFSGSGSGTDFTLTVSSLQAEDVAVYYCQHYYSTPYTFGQGTK
LEIKRTVAAPSVFIFPPSDEQLKSGTASVVCLLNNFYPREAKVQWKVDNALQSGNSQESVTEQDS
KDSTYSLSSTLTLSKADYEKHKVYACEVTHQGLSSPVTKSFNRGECGGSEGKSSGSGSESKSTEG
KSSGSGSESKSTGGSEVQLVESGGGLVQPGRSLRLSCAASGFTFDDYAMHWVRQAPGKGLEWV
SGIGWSGGSIVYADSVKGRFTISRDNAKNSLYLQMNSLRAEDTALYYCAKDSPYGDFFDYWGQ
GTLVTVSSASTKGPSVFPLAPCSRSTSESTAALGCLVKDYFPEPVTVSWNSGALTSGVHTFPAVL
QSSGLYSLSSVVTVPSSSLGTKTYTCNVDHKPSNTKVDKRVESKYGPPCPPCPAPEAAGGPSVFL
FPPKPKDTLMISRTPEVTCVVVDVSQEDPEVQFNWYVDGVEVHNAKTKPREEQFNSTYRVVSVL
TVLHQDWLNGKEYKCKVSNKGLPSSIEKTISKAKGQPREPQVYTLPPSQEEMTKNQVSLWCLVK
GFYPSDIAVEWESNGQPENNYKTTPPVLDSDGSFFLYSRLTVDKSRWQEGNVFSCSVMHEALHN
HYTQKSLSLSLGK

SEQ ID NO: 252 Anti-RSV knob arm sequence

MAWVWTLLFLMAAAQSIQAEIVLTQSPGTLSLSPGERATLSCRASQSVSSSYLAWYQQKPGQAP
RLLIYGASSRATGIPDRFSGSGSGTDFTLTISRLEPEDFAVYYCQQDYGFPWTFGQGTKVEIKRTV
AAPSVFIFPPSDEQLKSGTASVVCLLNNFYPREAKVQWKVDNALQSGNSQESVTEQDSKDSTYSL
SSTLTLSKADYEKHKVYACEVTHQGLSSPVTKSFNRGECGGSEGKSSGSGSESKSTEGKSSGSGS
ESKSTGGSEVQLVQSGAEVKKPGESLKISCKGSGYSFTSYWISWVRQMPGKGLEWMGIIDPSDSD
TRYSPSFQGQVTISADKSISTAYLQWSSLKASDTAMYYCARGDGSTDLDYWGQGTLVTVSSAST
KGPSVFPLAPCSRSTSESTAALGCLVKDYFPEPVTVSWNSGALTSGVHTFPAVLQSSGLYSLSSVV
TVPSSSLGTKTYTCNVDHKPSNTKVDKRVESKYGPPCPPCPAPEAAGGPSVFLFPPKPKDTLMISR
TPEVTCVVVDVSQEDPEVQFNWYVDGVEVHNAKTKPREEQFNSTYRVVSVLTVLHQDWLNGK
EYKCKVSNKGLPSSIEKTISKAKGQPREPQVYTLPPSQEEMTKNQVSLWCLVKGFYPSDIAVEWE
SNGQPENNYKTTPPVLDSDGSFFLYSRLTVDKSRWQEGNVFSCSVMHEALHNHYTQKSLSLSLG
K

SEO ID NO: 253 CDR1 of VH (C33B1475)
SYWMT

SEQ ID NO: 254 CDR1 of VH (C33B1516)
NYYWS

SEQ ID NO: 255 CDR1 of VH (C33B1481)
NYWMS

SEQ ID NO: 256 CDR2 of VH (C33B1475)
NIKODGSERYYVDSVKG

SEQ ID NO: 257 CDR2 of VH (C33B1516)
HIFSTGHINYDSSLKS

SEQ ID NO: 258 CDR2 of VH (C33B1481)

SIKRDGSDKYYVDSVKG

SEQ ID NO: 259 CDR3 of VH (C33B1475)
EVGYNWNQGGYFDY

SEQ ID NO: 260 CDR3 of VH (C33B1516)
DNGAALFDF

SEQ ID NO: 261 CDR3 of VH (C33B1481)
GEFDY

SEQ ID NO: 262 VH sequence (C33B1475)

EVQLVESGGGLVQPGGSLRLSCVVSGFTFSSYWMTWVRQAPGKGLEWVANIKQDGSERYYVDS
VKGRFTISRDSAKNSLYLQMNSLRAEDTAVYYCAREVGYNWNQGGYFDYWGQGTLVTVSS

SEQ ID NO: 263 VH sequence (C33B 1516)

QVQLQESGPGLVKPSETLSLTCSVSGGSIRNYYWSWIRQSAGKELEWFGHIFSTGHINYDSSLKSR
VTMSVDTSNNQFSLKLRSVTAADTAVYYCARDNGAALFDFWGQGTLVTVSS

SEQ ID NO: 264 VH sequence (C33B1481)

QVQLVESGGGLVQPGGSLRLSCAASGITFSNYWMSWVRQAPGKGLEWVASIKRDGSDKYYVDS
VKGRFTISRDNAKNSLSLQMHSLRAEDTAVYYCAKGEFDYWGQGTLVTVSS

SEQ ID NO: 265 CDR1 of VL (C33B1475)
RSSOSLLHSDGYNYLD

SEQ ID NO: 266 CDR1 of VL (C33B1481)
RSSQSLVYSDGNTYLN

SEQ ID NO: 267 CDR2 of VL (C33B1516)
SDNQRPS

SEQ ID NO: 268 CDR2 of VL (C33B1481)
KVSTRDS

SEQ ID NO: 269 CDR3 of VL (C33B1475)
MQVLQTPWT

SEQ ID NO: 270 CDR3 of VL (C33B1481)
LQGTHWPWT

SEQ ID NO: 271 VL sequence (C33B1475)

DIVMTQSPLSLPVTPGEPASISCRSSQSLLHSDGYNYLDWYLQKSGQSPQLLIYLGSYRASGVPDR
FSGSGSGTDFTLKISRVEAEDVGIYYCMQVLQTPWTFGQGTKVEIK

SEQ ID NO: 272 VL sequence (C33B1516)

QSVLTQPPSASGTPGQRVTISCSGSSSNIGSNIVNWYQQFPGTAPKLLLYSDNQRPSGVPDRFSGS
KSGTSASLAISGLQSEDEADYYCAAWDDSLNGPVFGTGTKVTVL

SEQ ID NO: 273 VL sequence (C33B1481)

DVVMTQSPLSLPVTLGQPASISCRSSQSLVYSDGNTYLNWFQQRPGQSPRRLIYKVSTRDSGVPD
RFSGSGSGTDFTLKISRVEAEDVAVYYCLQGTHWPWTFGQGTKVEIK

SEQ ID NO: 274 scFv (C33B1475)

DIVMTQSPLSLPVTPGEPASISCRSSQSLLHSDGYNYLDWYLQKSGQSPQLLIYLGSYRASGVPDR
FSGSGSGTDFTLKISRVEAEDVGIYYCMQVLQTPWTFGQGTKVEIKGGSEGKSSGSGSESKSTGG
SEVQLVESGGGLVQPGGSLRLSCVVSGFTFSSYWMTWVRQAPGKGLEWVANIKQDGSERYYVD
SVKGRFTISRDSAKNSLYLQMNSLRAEDTAVYYCAREVGYNWNQGGYFDYWGQGTLVTVSS

SEQ ID NO: 275 scFv (C33B1516)

QSVLTQPPSASGTPGQRVTISCSGSSSNIGSNIVNWYQQFPGTAPKLLLYSDNQRPSGVPDRFSGS
KSGTSASLAISGLQSEDEADYYCAAWDDSLNGPVFGTGTKVTVLGGSEGKSSGSGSESKSTGGS
QVQLQESGPGLVKPSETLSLTCSVSGGSIRNYYWSWIRQSAGKELEWFGHIFSTGHINYDSSLKSR
VTMSVDTSNNQFSLKLRSVTAADTAVYYCARDNGAALFDFWGQGTLVTVSS

SEQ ID NO: 276 scFv (C33B1481)

DVVMTQSPLSLPVTLGQPASISCRSSQSLVYSDGNTYLNWFQQRPGQSPRRLIYKVSTRDSGVPD
RFSGSGSGTDFTLKISRVEAEDVAVYYCLQGTHWPWTFGQGTKVEIKGGSEGKSSGSGSESKSTG
GSQVQLVESGGGLVQPGGSLRLSCAASGITFSNYWMSWVRQAPGKGLEWVASIKRDGSDKYYV
DSVKGRFTISRDNAKNSLSLQMHSLRAEDTAVYYCAKGEFDYWGQGTLVTVSS

SEQ ID NO: 277 huIgG1_Glm(17)-hinge-FC_C220S|IgG1| (C33B1475; C33B1516; C33B1481; C33B1477; C33B1517; C33B1522)

EPKSSDKTHTCPPCPAPELLGGPSVFLFPPKPKDTLMISRTPEVTCVVVDVSHEDPEVKFNWYVD
GVEVHNAKTKPREEQYNSTYRVVSVLTVLHQDWLNGKEYKCKVSNKALPAPIEKTISKAKGQP
REPQVYTLPPSREEMTKNQVSLTCLVKGFYPSDIAVEWESNGQPENNYKTTPPVLDSDGSFFLYS
KLTVDKSRWQQGNVFSCSVMHEALHNHYTQKSLSLSPGK

SEQ ID NO 278: huIgG1 G1m(17)

EPKSCDKTHTCPPCPAPELLGGPSVFLFPPKPKDTLMISRTPEVTCVVVDVSHEDPEVKFNWYVD
GVEVHNAKTKPREEQYNSTYRVVSVLTVLHQDWLNGKEYKCKVSNKALPAPIEKTISKAKGQP
REPQVYTLPPSREEMTKNQVSLTCLVKGFYPSDIAVEWESNGQPENNYKTTPPVLDSDGSFFLYS
KLTVDKSRWQQGNVFSCSVMHEALHNHYTQKSLSLSPGK

SEQ ID NO: 279 Fab HC (C33B1475)

QVQLVESGGGLVQPGGSLRLSCAASGITFSNYWMSWVRQAPGKGLEWVASIKRDGSDKYYVDSVKGRFTISRDNAKNSLSLQMHSLRAEDTAVYYCAKGEFDYWGQGTLVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKV

SEQ ID NO: 280 Fab HC (C33B1516)

QVQLQESGPGLVKPSETLSLTCSVSGGSIRNYYWSWIRQSAGKELEWFGHIFSTGHINYDSSLKSRVTMSVDTSNNQFSLKLRSVTAADTAVYYCARDNGAALFDFWGQGTLVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKV

SEQ ID NO: 281 Fab HC (C33B1481)

QVQLVESGGGLVQPGGSLRLSCAASGITFSNYWMSWVRQAPGKGLEWVASIKRDGSDKYYVDSVKGRFTISRDNAKNSLSLQMHSLRAEDTAVYYCAKGEFDYWGQGTLVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKV

SEQ ID NO: 282 Fab HC (C33B1477)

EVQLVESGGGLVQPGGSLKVSCEASGFTFSVSAIHWVRQASGKGLEWIGRIRSKGNSYATAYAASVKGRFTISRDDSKNTAYLQMDSLKTEDTAVYYCTRHNDKWNYYGLDVWGQGTTVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKV

SEQ ID NO: 283 Fab HC (C33B1517)

QVQLQESGPGLVKPSETLSLTCSVSGASIRNYYWSWIRQTAGKGLEWLGHIYSTGNIHYNPSLKSRVTMSVDTSNNQFSLKLRSVTAADTAVYYCARDNGAALFDYWGQGTLVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKV

SEQ ID NO: 284 Fab HC (C33B1522)

QVQLQESGPGLVKPSETLSLTCTVSGGSISSYYWGWIRQPPGKGLEWIGYIYYSGSTNYNPSLKSRVTISVDTSKNQFSLKLSSVTAADTAVYYCARMWEILGFDPWGQGTLVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKV

SEQ ID NO: 285 Fab LC (C33B1475)

DIVMTQSPLSLPVTPGEPASISCRSSQSLLHSDGYNYLDWYLQKSGQSPQLLIYLGSYRASGVPDR FSGSGSGTDFTLKISRVEAEDVGIYYCMQVLQTPWTFGQGTKVEIKRTVAAPSVFIFPPSDEQLKS GTASVVCLLNNFYPREAKVQWKVDNALQSGNSQESVTEQDSKDSTYSLSSTLTLSKADYEKHK VYACEVTHQGLSSPVTKSFNRGEC

SEQ ID NO: 286 Fab LC (C33B1516)

QSVLTQPPSASGTPGQRVTISCSGSSSNIGSNIVNWYQQFPGTAPKLLLYSDNQRPSGVPDRFSGS KSGTSASLAISGLQSEDEADYYCAAWDDSLNGPVFGTGTKVTVLGQPKAAPSVTLFPPSSEELQA NKATLVCLISDFYPGAVTVAWKADSSPVKAGVETTTPSKQSNNKYAASSYLSLTPEQWKSHRSY SCQVTHEGSTVEKTVAPTECS

SEQ ID NO: 287 Fab LC (C33B1481)

DVVMTQSPLSLPVTLGQPASISCRSSQSLVYSDGNTYLNWFQQRPGQSPRRLIYKVSTRDSGVPD RFSGSGSGTDFTLKISRVEAEDVAVYYCLQGTHWPWTFGQGTKVEIKRTVAAPSVFIFPPSDEQL KSGTASVVCLLNNFYPREAKVQWKVDNALQSGNSQESVTEQDSKDSTYSLSSTLTLSKADYEKH KVYACEVTHQGLSSPVTKSFNRGEC

SEQ ID NO: 288 Fab LC (C33B1477)

DIVMTQSPLSLPVTPGEPASISCKSSQSLLFSNGYKFLDWYLQRPGQSPQLLIYLGSYRASGVPDR FSGSGSGTDFTLKISRVEAEDVGLYYCMQALQTPPTFGGGTKVEIKRTVAAPSVFIFPPSDEQLKS GTASVVCLLNNFYPREAKVQWKVDNALQSGNSQESVTEQDSKDSTYSLSSTLTLSKADYEKHK VYACEVTHQGLSSPVTKSFNRGEC

SEQ ID NO: 289 Fab LC (C33B1517)

QSVLTQPPSASGTPGQRVTISCSGSSSNIGSNIVNWYQQFPGTAPKLLIYSNNQRPSGVPDRVSGS KSGTSASLAISGLQSEDEADYYCAAWDDSLNGPVFGPGTKVTVLGQPKAAPSVTLFPPSSEELQA NKATLVCLISDFYPGAVTVAWKADSSPVKAGVETTTPSKQSNNKYAASSYLSLTPEQWKSHRSY SCQVTHEGSTVEKTVAPTECS

SEQ ID NO: 290 Fab LC (C33B1522)

QSVLTQPPSASGTPGQRVTISCSGSSSNIGSNPVNWYQQLPGTAPKLLIYSNNQRPSGVPDRFSGS KSGTSASLAISGLQSEDEADYFCAAWDDSLNGPVFGGGTKLTVLGQPKAAPSVTLFPPSSEELQA NKATLVCLISDFYPGAVTVAWKADSSPVKAGVETTTPSKQSNNKYAASSYLSLTPEQWKSHRSY SCQVTHEGSTVEKTVAPTECS

SEQ ID NO: 291 (C33B1475VH1-2)

EVQLVESGGGLVQPGGSLRLSCVVSGFTFSSYWMTWVRQAPGKGLEWVANIKQGGSERYYVDS VKGRFTISRDSAKNSLYLQMNSLRAEDTAVYYCAREVGYNWNQGGYFDYWGQGTLVTVSS

SEQ ID NO: 292 (C33B1475VH1-3)

EVQLVESGGGLVQPGGSLRLSCVVSGFTFSSYWMTWVRQAPGKGLEWVANIKQAGSERYYVDS VKGRFTISRDSAKNSLYLQMNSLRAEDTAVYYCAREVGYNWNQGGYFDYWGQGTLVTVSS

SEQ ID NO: 293 (C33B1475VH1-5)

EVQLVESGGGLVQPGGSLRLSCVVSGFTFSSYWMTWVRQAPGKGLEWVANIKQYGSERYYVDS VKGRFTISRDSAKNSLYLQMNSLRAEDTAVYYCAREVGYNWNQGGYFDYWGQGTLVTVSS

SEQ ID NO: 294 (C33B1475VH1-12)

EVQLVESGGGLVQPGGSLRLSCVVSGFTFSSYWMTWVRQAPGKGLEWVANIKQPGSERYYVDS VKGRFTISRDSAKNSLYLQMNSLRAEDTAVYYCAREVGYNWNQGGYFDYWGQGTLVTVSS

SEQ ID NO: 295 (C33B1475VH1-14)

EVQLVESGGGLVQPGGSLRLSCVVSGFTFSSYWMTWVRQAPGKGLEWVANIKQNGSERYYVDS VKGRFTISRDSAKNSLYLQMNSLRAEDTAVYYCAREVGYNWNQGGYFDYWGQGTLVTVSS

SEQ ID NO: 296 (C33B1475VH1-22)

EVQLVESGGGLVQPGGSLRLSCVVSGFTFSSYWMTWVRQAPGKGLEWVANIKQSGSERYYVDS VKGRFTISRDSAKNSLYLQMNSLRAEDTAVYYCAREVGYNWNQGGYFDYWGQGTLVTVSS

SEQ ID NO: 297 (C33B1475VH1-27)

EVQLVESGGGLVQPGGSLRLSCVVSGFTFSSYWMTWVRQAPGKGLEWVANIKQRGSERYYVDS VKGRFTISRDSAKNSLYLQMNSLRAEDTAVYYCAREVGYNWNQGGYFDYWGQGTLVTVSS

SEQ ID NO: 298 (C33B1475VH1-32)

EVQLVESGGGLVQPGGSLRLSCVVSGFTFSSYWMTWVRQAPGKGLEWVANIKQLGSERYYVDS VKGRFTISRDSAKNSLYLQMNSLRAEDTAVYYCAREVGYNWNQGGYFDYWGQGTLVTVSS

SEQ ID NO: 299 (C33B1475VH1-36)

EVQLVESGGGLVQPGGSLRLSCVVSGFTFSSYWMTWVRQAPGKGLEWVANIKQVGSERYYVDSVKGRFTISRDSAKNSLYLQMNSLRAEDTAVYYCAREVGYNWNQGGYFDYWGQGTLVTVSS

SEO ID NO: 300 (C33B1475VH1-46)

EVQLVESGGGLVQPGGSLRLSCVVSGFTFSSYWMTWVRQAPGKGLEWVANIKQTGSERYYVDSVKGRFTISRDSAKNSLYLQMNSLRAEDTAVYYCAREVGYNWNQGGYFDYWGQGTLVTVSS

SEQ ID NO: 301 (C33B1475VH1-63)

EVQLVESGGGLVQPGGSLRLSCVVSGFTFSSYWMTWVRQAPGKGLEWVANIKQFGSERYYVDSVKGRFTISRDSAKNSLYLQMNSLRAEDTAVYYCAREVGYNWNQGGYFDYWGQGTLVTVSS

SEQ ID NO: 302 (C33B1475VH1-89)

EVQLVESGGGLVQPGGSLRLSCVVSGFTFSSYWMTWVRQAPGKGLEWVANIKQWGSERYYVDSVKGRFTISRDSAKNSLYLQMNSLRAEDTAVYYCAREVGYNWNQGGYFDYWGQGTLVTVSS

SEQ ID NO: 303 (C33B1475VH2-12)

EVQLVESGGGLVQPGGSLRLSCVVSGFTFSSYWMTWVRQAPGKGLEWVANIKQDVSERYYVDSVKGRFTISRDSAKNSLYLQMNSLRAEDTAVYYCAREVGYNWNQGGYFDYWGQGTLVTVSS

SEQ ID NO: 304 (C33B1475VH2-16)

EVQLVESGGGLVQPGGSLRLSCVVSGFTFSSYWMTWVRQAPGKGLEWVANIKQDPSERYYVDSVKGRFTISRDSAKNSLYLQMNSLRAEDTAVYYCAREVGYNWNQGGYFDYWGQGTLVTVSS

SEQ ID NO: 305 (C33B1475VH2-20)

EVQLVESGGGLVQPGGSLRLSCVVSGFTFSSYWMTWVRQAPGKGLEWVANIKQDRSERYYVDSVKGRFTISRDSAKNSLYLQMNSLRAEDTAVYYCAREVGYNWNQGGYFDYWGQGTLVTVSS

SEQ ID NO: 306 (C33B1475VH2-22)

EVQLVESGGGLVQPGGSLRLSCVVSGFTFSSYWMTWVRQAPGKGLEWVANIKQDASERYYVDSVKGRFTISRDSAKNSLYLQMNSLRAEDTAVYYCAREVGYNWNQGGYFDYWGQGTLVTVSS

SEQ ID NO: 307 (C33B1475VH2-24)

EVQLVESGGGLVQPGGSLRLSCVVSGFTFSSYWMTWVRQAPGKGLEWVANIKQDLSERYYVDSVKGRFTISRDSAKNSLYLQMNSLRAEDTAVYYCAREVGYNWNQGGYFDYWGQGTLVTVSS

SEQ ID NO: 308 (C33B1475VH2-35)

EVQLVESGGGLVQPGGSLRLSCVVSGFTFSSYWMTWVRQAPGKGLEWVANIKQDQSERYYVDSVKGRFTISRDSAKNSLYLQMNSLRAEDTAVYYCAREVGYNWNQGGYFDYWGQGTLVTVSS

SEQ ID NO: 309 (C33B1475VH2-58)

EVQLVESGGGLVQPGGSLRLSCVVSGFTFSSYWMTWVRQAPGKGLEWVANIKQDTSERYYVDSVKGRFTISRDSAKNSLYLQMNSLRAEDTAVYYCAREVGYNWNQGGYFDYWGQGTLVTVSS

SEQ ID NO: 310 (C33B1475VH2-86)

EVQLVESGGGLVQPGGSLRLSCVVSGFTFSSYWMTWVRQAPGKGLEWVANIKQDHSERYYVDSVKGRFTISRDSAKNSLYLQMNSLRAEDTAVYYCAREVGYNWNQGGYFDYWGQGTLVTVSS

SEQ ID NO: 311 (C33B1516 VL1-1)

QSVLTQPPSASGTPGQRVTISCSGSSSNIGSNIVNWYQQFPGTAPKLLLYSDNQRPSGVPDRFSGSKSGTSASLAISGLQSEDEADYYCAAWDDSLVGPVFGTGTKVTVL

SEQ ID NO: 312 (C33B1516 VL1-2)

QSVLTQPPSASGTPGQRVTISCSGSSSNIGSNIVNWYQQFPGTAPKLLLYSDNQRPSGVPDRFSGSKSGTSASLAISGLQSEDEADYYCAAWDDSLRGPVFGTGTKVTVL

SEQ ID NO: 313 (C33B1516 VL1-4)

QSVLTQPPSASGTPGQRVTISCSGSSSNIGSNIVNWYQQFPGTAPKLLLYSDNQRPSGVPDRFSGSKSGTSASLAISGLQSEDEADYYCAAWDDSLPGPVFGTGTKVTVL

SEQ ID NO: 314 (C33B1516 VL1-10)

QSVLTQPPSASGTPGQRVTISCSGSSSNIGSNIVNWYQQFPGTAPKLLLYSDNQRPSGVPDRFSGSKSGTSASLAISGLQSEDEADYYCAAWDDSLTGPVFGTGTKVTVL

SEQ ID NO: 315(C33B1516 VL1-11)

QSVLTQPPSASGTPGQRVTISCSGSSSNIGSNIVNWYQQFPGTAPKLLLYSDNQRPSGVPDRFSGSKSGTSASLAISGLQSEDEADYYCAAWDDSLGGPVFGTGTKVTVL

SEQ ID NO: 316 (C33B1516 VL1-14)

QSVLTQPPSASGTPGQRVTISCSGSSSNIGSNIVNWYQQFPGTAPKLLLYSDNQRPSGVPDRFSGSKSGTSASLAISGLQSEDEADYYCAAWDDSLQGPVFGTGTKVTVL

SEQ ID NO: 317 (C33B1516 VL1-16)

QSVLTQPPSASGTPGQRVTISCSGSSSNIGSNIVNWYQQFPGTAPKLLLYSDNQRPSGVPDRFSGSKSGTSASLAISGLQSEDEADYYCAAWDDSLSGPVFGTGTKVTVL

SEQ ID NO: 328 (C33B1516 VL1-20)

QSVLTQPPSASGTPGQRVTISCSGSSSNIGSNIVNWYQQFPGTAPKLLLYSDNQRPSGVPDRFSGS KSGTSASLAISGLQSEDEADYYCAAWDDSLAGPVFGTGTKVTVL

SEQ ID NO: 319 (C33B1516 VL1-22)

QSVLTQPPSASGTPGQRVTISCSGSSSNIGSNIVNWYQQFPGTAPKLLLYSDNQRPSGVPDRFSGS KSGTSASLAISGLQSEDEADYYCAAWDDSLLGPVFGTGTKVTVL

SEQ ID NO: 320 (C33B1516 VL1-23)

QSVLTQPPSASGTPGQRVTISCSGSSSNIGSNIVNWYQQFPGTAPKLLLYSDNQRPSGVPDRFSGS KSGTSASLAISGLQSEDEADYYCAAWDDSLYGPVFGTGTKVTVL

SEQ ID NO: 321 (C33B1516 VL1-30)

QSVLTQPPSASGTPGQRVTISCSGSSSNIGSNIVNWYQQFPGTAPKLLLYSDNQRPSGVPDRFSGS KSGTSASLAISGLQSEDEADYYCAAWDDSLEGPVFGTGTKVTVL

SEQ ID NO: 322 (C33B1516VL1-39)

QSVLTQPPSASGTPGQRVTISCSGSSSNIGSNIVNWYQQFPGTAPKLLLYSDNQRPSGVPDRFSGS KSGTSASLAISGLQSEDEADYYCAAWDDSLFGPVFGTGTKVTVL

SEQ ID NO: 323 (C33B1516VL1-41)

QSVLTQPPSASGTPGQRVTISCSGSSSNIGSNIVNWYQQFPGTAPKLLLYSDNQRPSGVPDRFSGS KSGTSASLAISGLQSEDEADYYCAAWDDSLDGPVFGTGTKVTVL

SEQ ID NO: 324 (C33B1516VL1-63)

QSVLTQPPSASGTPGQRVTISCSGSSSNIGSNIVNWYQQFPGTAPKLLLYSDNQRPSGVPDRFSGS KSGTSASLAISGLQSEDEADYYCAAWDDSLIGPVFGTGTKVTVL

SEQ ID NO: 325 (C33B1516VL1-90)

QSVLTQPPSASGTPGQRVTISCSGSSSNIGSNIVNWYQQFPGTAPKLLLYSDNQRPSGVPDRFSGS KSGTSASLAISGLQSEDEADYYCAAWDDSLHGPVFGTGTKVTVL

SEQ ID NO: 326 (C33B1516 VL2-3)

QSVLTQPPSASGTPGQRVTISCSGSSSNIGSNIVNWYQQFPGTAPKLLLYSDNQRPSGVPDRFSGS KSGTSASLAISGLQSEDEADYYCAAWDDSLNVPVFGTGTKVTVL

SEQ ID NO: 327 (C33B1516 VL2-4)

QSVLTQPPSASGTPGQRVTISCSGSSSNIGSNIVNWYQQFPGTAPKLLLYSDNQRPSGVPDRFSGS KSGTSASLAISGLQSEDEADYYCAAWDDSLNPPVFGTGTKVTVL

SEQ ID NO: 328 (C33B1516 VL2-7)

QSVLTQPPSASGTPGQRVTISCSGSSSNIGSNIVNWYQQFPGTAPKLLLYSDNQRPSGVPDRFSGS KSGTSASLAISGLQSEDEADYYCAAWDDSLNRPVFGTGTKVTVL

SEQ ID NO: 329 (C33B1516 VL2-8)

QSVLTQPPSASGTPGQRVTISCSGSSSNIGSNIVNWYQQFPGTAPKLLLYSDNQRPSGVPDRFSGS KSGTSASLAISGLQSEDEADYYCAAWDDSLNDPVFGTGTKVTVL

SEQ ID NO: 330 (C33B1516 VL2-10)

QSVLTQPPSASGTPGQRVTISCSGSSSNIGSNIVNWYQQFPGTAPKLLLYSDNQRPSGVPDRFSGS KSGTSASLAISGLQSEDEADYYCAAWDDSLNEPVFGTGTKVTVL

SEQ ID NO: 331 (C33B1516 VL2-11)

QSVLTQPPSASGTPGQRVTISCSGSSSNIGSNIVNWYQQFPGTAPKLLLYSDNQRPSGVPDRFSGS KSGTSASLAISGLQSEDEADYYCAAWDDSLNWPVFGTGTKVTVL

SEQ ID NO: 332 (C33B1516 VL2-29)

QSVLTQPPSASGTPGQRVTISCSGSSSNIGSNIVNWYQQFPGTAPKLLLYSDNQRPSGVPDRFSGS KSGTSASLAISGLQSEDEADYYCAAWDDSLNYPVFGTGTKVTVL

SEQ ID NO: 333 (C33B1516 VL2-35)

QSVLTQPPSASGTPGQRVTISCSGSSSNIGSNIVNWYQQFPGTAPKLLLYSDNQRPSGVPDRFSGS KSGTSASLAISGLQSEDEADYYCAAWDDSLNAPVFGTGTKVTVL

SEQ ID NO: 334 (C33B1516VL2-37)

QSVLTQPPSASGTPGQRVTISCSGSSSNIGSNIVNWYQQFPGTAPKLLLYSDNQRPSGVPDRFSGS KSGTSASLAISGLQSEDEADYYCAAWDDSLNNPVFGTGTKVTVL

SEQ ID NO: 335 (C33B1516VL2-38)

QSVLTQPPSASGTPGQRVTISCSGSSSNIGSNIVNWYQQFPGTAPKLLLYSDNQRPSGVPDRFSGS KSGTSASLAISGLQSEDEADYYCAAWDDSLNKPVFGTGTKVTVL

SEQ ID NO: 336 (C33B1516VL2-90)

QSVLTQPPSASGTPGQRVTISCSGSSSNIGSNIVNWYQQFPGTAPKLLLYSDNQRPSGVPDRFSGS KSGTSASLAISGLQSEDEADYYCAAWDDSLNLPVFGTGTKVTVL

SEQ ID NO: 337 (C33B1516VH1-1)

QVQLQESGPGLVKPSETLSLTCSVSGGSIRNYYWSWIRQSAGKELEWFGHIFSTGHINYDSSLKSR VTMSVDTSNNQFSLKLRSVTAADTAVYYCARDVGAALFDFWGQGTLVTVSS

SEQ ID NO: 338 (C33B1516VH1-3)

QVQLQESGPGLVKPSETLSLTCSVSGGSIRNYYWSWIRQSAGKELEWFGHIFSTGHINYDSSLKSR VTMSVDTSNNQFSLKLRSVTAADTAVYYCARDSGAALFDFWGQGTLVTVSS

SEQ ID NO: 339 (C33B1516VH1-4)

QVQLQESGPGLVKPSETLSLTCSVSGGSIRNYYWSWIRQSAGKELEWFGHIFSTGHINYDSSLKSR VTMSVDTSNNQFSLKLRSVTAADTAVYYCARDEGAALFDFWGQGTLVTVSS

SEQ ID NO: 340 (C33B1516VH1-6)

QVQLQESGPGLVKPSETLSLTCSVSGGSIRNYYWSWIRQSAGKELEWFGHIFSTGHINYDSSLKSR VTMSVDTSNNQFSLKLRSVTAADTAVYYCARDHGAALFDFWGQGTLVTVSS

SEQ ID NO: 341 (C33B1516VH1-7)

QVQLQESGPGLVKPSETLSLTCSVSGGSIRNYYWSWIRQSAGKELEWFGHIFSTGHINYDSSLKSR VTMSVDTSNNQFSLKLRSVTAADTAVYYCARDAGAALFDFWGQGTLVTVSS

SEQ ID NO: 342 (C33B1516VH1-8)

QVQLQESGPGLVKPSETLSLTCSVSGGSIRNYYWSWIRQSAGKELEWFGHIFSTGHINYDSSLKSR VTMSVDTSNNQFSLKLRSVTAADTAVYYCARDGGAALFDFWGQGTLVTVSS

SEQ ID NO: 343 (C33B1516VH1-10)

QVQLQESGPGLVKPSETLSLTCSVSGGSIRNYYWSWIRQSAGKELEWFGHIFSTGHINYDSSLKSR VTMSVDTSNNQFSLKLRSVTAADTAVYYCARDKGAALFDFWGQGTLVTVSS

SEQ ID NO: 344 (C33B1516VH1-13)

QVQLQESGPGLVKPSETLSLTCSVSGGSIRNYYWSWIRQSAGKELEWFGHIFSTGHINYDSSLKSR VTMSVDTSNNQFSLKLRSVTAADTAVYYCARDTGAALFDFWGQGTLVTVSS

SEQ ID NO: 345 (C33B1516VH1-171

QVQLQESGPGLVKPSETLSLTCSVSGGSIRNYYWSWIRQSAGKELEWFGHIFSTGHINYDSSLKSR VTMSVDTSNNQFSLKLRSVTAADTAVYYCARDFGAALFDFWGQGTLVTVSS

SEQ ID NO: 346 (C33B1516VH1-20)

QVQLQESGPGLVKPSETLSLTCSVSGGSIRNYYWSWIRQSAGKELEWFGHIFSTGHINYDSSLKSR VTMSVDTSNNQFSLKLRSVTAADTAVYYCARDVGAALFDFWGQGTLVTVSS

SEQ ID NO: 347 (C33B1516VH1-21)

QVQLQESGPGLVKPSETLSLTCSVSGGSIRNYYWSWIRQSAGKELEWFGHIFSTGHINYDSSLKSRVTMSVDTSNNQFSLKLRSVTAADTAVYYCARDRGAALFDFWGQGTLVTVSS

SEQ ID NO: 348 (C33B1516VH1-27)

QVQLQESGPGLVKPSETLSLTCSVSGGSIRNYYWSWIRQSAGKELEWFGHIFSTGHINYDSSLKSRVTMSVDTSNNQFSLKLRSVTAADTAVYYCARDLGAALFDFWGQGTLVTVSS

SEQ ID NO: 349 (C33B1516VH1-38)

QVQLQESGPGLVKPSETLSLTCSVSGGSIRNYYWSWIRQSAGKELEWFGHIFSTGHINYDSSLKSRVTMSVDTSNNQFSLKLRSVTAADTAVYYCARDHGAALFDFWGQGTLVTVSS

SEQ ID NO: 350 (C33B1516VH1-56)

QVQLQESGPGLVKPSETLSLTCSVSGGSIRNYYWSWIRQSAGKELEWFGHIFSTGHINYDSSLKSRVTMSVDTSNNQFSLKLRSVTAADTAVYYCARDIGAALFDFWGQGTLVTVSS

SEQ ID NO: 351 (C33B1516VH2-8)

QVQLQESGPGLVKPSETLSLTCSVSGGSIRNYYWSWIRQSAGKELEWFGHIFSTGHINYDSSLKSRVTMSVDTSNNQFSLKLRSVTAADTAVYYCARDNSAALFDFWGQGTLVTVSS

SEQ ID NO: 352 (C33B1516VH2-9)

QVQLQESGPGLVKPSETLSLTCSVSGGSIRNYYWSWIRQSAGKELEWFGHIFSTGHINYDSSLKSRVTMSVDTSNNQFSLKLRSVTAADTAVYYCARDNAAALFDFWGQGTLVTVSS

SEQ ID NO: 353 (C33B1517VL1-1)

QSVLTQPPSASGTPGQRVTISCSGSSSNIGSNIVNWYQQFPGTAPKLLIYSNNQRPSGVPDRVSGSKSGTSASLAISGLQSEDEADYYCAAWDDSLGGPVFGPGTKVTVL

SEQ ID NO: 354 (C33B1517VL1-7)

QSVLTQPPSASGTPGQRVTISCSGSSSNIGSNIVNWYQQFPGTAPKLLIYSNNQRPSGVPDRVSGSKSGTSASLAISGLQSEDEADYYCAAWDDSLDGPVFGPGTKVTVL

SEQ ID NO: 355 (C33B1517VL1-8)

QSVLTQPPSASGTPGQRVTISCSGSSSNIGSNIVNWYQQFPGTAPKLLIYSNNQRPSGVPDRVSGSKSGTSASLAISGLQSEDEADYYCAAWDDSLPGPVFGPGTKVTVL

SEQ ID NO: 356 (C33B1517VL1-12)

QSVLTQPPSASGTPGQRVTISCSGSSSNIGSNIVNWYQQFPGTAPKLLIYSNNQRPSGVPDRVSGS KSGTSASLAISGLQSEDEADYYCAAWDDSLKGPVFGPGTKVTVL

SEQ ID NO: 357 (C33B1517VL1-13)

QSVLTQPPSASGTPGQRVTISCSGSSSNIGSNIVNWYQQFPGTAPKLLIYSNNQRPSGVPDRVSGS KSGTSASLAISGLQSEDEADYYCAAWDDSLEGPVFGPGTKVTVL

SEQ ID NO: 358 (C33B1517VL1-16)

QSVLTQPPSASGTPGQRVTISCSGSSSNIGSNIVNWYQQFPGTAPKLLIYSNNQRPSGVPDRVSGS KSGTSASLAISGLQSEDEADYYCAAWDDSLAGPVFGPGTKVTVL

SEO ID NO: 359 (C33B1517VL1-22)

QSVLTQPPSASGTPGQRVTISCSGSSSNIGSNIVNWYQQFPGTAPKLLIYSNNQRPSGVPDRVSGS KSGTSASLAISGLQSEDEADYYCAAWDDSLRGPVFGPGTKVTVL

SEQ ID NO: 360 (C33B1517VL1-24)

QSVLTQPPSASGTPGQRVTISCSGSSSNIGSNIVNWYQQFPGTAPKLLIYSNNQRPSGVPDRVSGS KSGTSASLAISGLQSEDEADYYCAAWDDSLQGPVFGPGTKVTVL

SEQ ID NO: 361 (C33B1517VL1-27)

QSVLTQPPSASGTPGQRVTISCSGSSSNIGSNIVNWYQQFPGTAPKLLIYSNNQRPSGVPDRVSGS KSGTSASLAISGLQSEDEADYYCAAWDDSLIGPVFGPGTKVTVL

SEQ ID NO: 362 (C33B1517VL1-35)

QSVLTQPPSASGTPGQRVTISCSGSSSNIGSNIVNWYQQFPGTAPKLLIYSNNQRPSGVPDRVSGS KSGTSASLAISGLQSEDEADYYCAAWDDSLVGPVFGPGTKVTVL

SEQ ID NO: 363 (C33B1517VL1-49)

QSVLTQPPSASGTPGQRVTISCSGSSSNIGSNIVNWYQQFPGTAPKLLIYSNNQRPSGVPDRVSGS KSGTSASLAISGLQSEDEADYYCAAWDDSLHGPVFGPGTKVTVL

SEQ ID NO: 364 (C33B1517VL1-66)

QSVLTQPPSASGTPGQRVTISCSGSSSNIGSNIVNWYQQFPGTAPKLLIYSNNQRPSGVPDRVSGS KSGTSASLAISGLQSEDEADYYCAAWDDSLKGPVFGPGTKVTVL

SEQ ID NO: 365 (C33B1517VL1-69)

QSVLTQPPSASGTPGQRVTISCSGSSSNIGSNIVNWYQQFPGTAPKLLIYSNNQRPSGVPDRVSGS KSGTSASLAISGLQSEDEADYYCAAWDDSLWGPVFGPGTKVTVL

SEQ ID NO: 366 (C33B1517VL1-71)

QSVLTQPPSASGTPGQRVTISCSGSSSNIGSNIVNWYQQFPGTAPKLLIYSNNQRPSGVPDRVSGS KSGTSASLAISGLQSEDEADYYCAAWDDSLSGPVFGPGTKVTVL

SEQ ID NO: 367 (C33B1517VH1-1)

QVQLQESGPGLVKPSETLSLTCSVSGASIRNYYWSWIRQTAGKGLEWLGHIYSTGNIHYNPSLKS RVTMSVDTSNNQFSLKLRSVTAADTAVYYCARDRGAALFDYWGQGTLVTVSS

SEQ ID NO: 368 (C33B1517VH1-3)

QVQLQESGPGLVKPSETLSLTCSVSGASIRNYYWSWIRQTAGKGLEWLGHIYSTGNIHYNPSLKS RVTMSVDTSNNQFSLKLRSVTAADTAVYYCARDPGAALFDYWGQGTLVTVSS

SEQ ID NO: 369 (C33B1517VH1-4)

QVQLQESGPGLVKPSETLSLTCSVSGASIRNYYWSWIRQTAGKGLEWLGHIYSTGNIHYNPSLKS RVTMSVDTSNNQFSLKLRSVTAADTAVYYCARDGGAALFDYWGQGTLVTVSS

SEQ ID NO: 370 (C33B1517VH1-6)

QVQLQESGPGLVKPSETLSLTCSVSGASIRNYYWSWIRQTAGKGLEWLGHIYSTGNIHYNPSLKS RVTMSVDTSNNQFSLKLRSVTAADTAVYYCARDVGAALFDYWGQGTLVTVSS

SEQ ID NO: 371 (C33B1517VH1-8)

QVQLQESGPGLVKPSETLSLTCSVSGASIRNYYWSWIRQTAGKGLEWLGHIYSTGNIHYNPSLKS RVTMSVDTSNNQFSLKLRSVTAADTAVYYCARDLGAALFDYWGQGTLVTVSS

SEQ ID NO: 372 (C33B1517VH1-11)

QVQLQESGPGLVKPSETLSLTCSVSGASIRNYYWSWIRQTAGKGLEWLGHIYSTGNIHYNPSLKS RVTMSVDTSNNQFSLKLRSVTAADTAVYYCARDDGAALFDYWGQGTLVTVSS

SEQ ID NO: 373 (C33B1517VH1-17)

QVQLQESGPGLVKPSETLSLTCSVSGASIRNYYWSWIRQTAGKGLEWLGHIYSTGNIHYNPSLKS RVTMSVDTSNNQFSLKLRSVTAADTAVYYCARDEGAALFDYWGQGTLVTVSS

SEQ ID NO: 374 (C33B1517VH1-33)

QVQLQESGPGLVKPSETLSLTCSVSGASIRNYYWSWIRQTAGKGLEWLGHIYSTGNIHYNPSLKS RVTMSVDTSNNQFSLKLRSVTAADTAVYYCARDAGAALFDYWGQGTLVTVSS

SEQ ID NO: 375 (C33B1517VH1-60)

QVQLQESGPGLVKPSETLSLTCSVSGASIRNYYWSWIRQTAGKGLEWLGHIYSTGNIHYNPSLKS RVTMSVDTSNNQFSLKLRSVTAADTAVYYCARDQGAALFDYWGQGTLVTVSS

SEQ ID NO: 376 (C33B1517VH1-71)

QVQLQESGPGLVKPSETLSLTCSVSGASIRNYYWSWIRQTAGKGLEWLGHIYSTGNIHYNPSLKS RVTMSVDTSNNQFSLKLRSVTAADTAVYYCARDTGAALFDYWGQGTLVTVSS

SEQ ID NO: 377 (C33B1517VH2-7)

QVQLQESGPGLVKPSETLSLTCSVSGASIRNYYWSWIRQTAGKGLEWLGHIYSTGNIHYNPSLKS RVTMSVDTSNNQFSLKLRSVTAADTAVYYCARDNSAALFDYWGQGTLVTVSS

SEQ ID NO: 378 (C33B1517VH2-30)

QVQLQESGPGLVKPSETLSLTCSVSGASIRNYYWSWIRQTAGKGLEWLGHIYSTGNIHYNPSLKS RVTMSVDTSNNQFSLKLRSVTAADTAVYYCARDNAAALFDYWGQGTLVTVSS

SEQ ID NO: 379 (C33B1517VH2-31)

QVQLQESGPGLVKPSETLSLTCSVSGASIRNYYWSWIRQTAGKGLEWLGHIYSTGNIHYNPSLKS RVTMSVDTSNNQFSLKLRSVTAADTAVYYCARDNLAALFDYWGQGTLVTVSS

SEQ ID NO: 380 (C33B1517VH2-60)

QVQLQESGPGLVKPSETLSLTCSVSGASIRNYYWSWIRQTAGKGLEWLGHIYSTGNIHYNPSLKS RVTMSVDTSNNQFSLKLRSVTAADTAVYYCARDNPAALFDYWGQGTLVTVSS

SEQ ID NO: 381 (C33B1517VH2-68)

QVQLQESGPGLVKPSETLSLTCSVSGASIRNYYWSWIRQTAGKGLEWLGHIYSTGNIHYNPSLKS RVTMSVDTSNNQFSLKLRSVTAADTAVYYCARDNTAALFDYWGQGTLVTVSS

SEQ ID NO: 382 (C33B1517VH2-76)

QVQLQESGPGLVKPSETLSLTCSVSGASIRNYYWSWIRQTAGKGLEWLGHIYSTGNIHYNPSLKS RVTMSVDTSNNQFSLKLRSVTAADTAVYYCARDNQAALFDYWGQGTLVTVSS

SEQ ID NO: 383 (C33B1522VL1-2)

QSVLTQPPSASGTPGQRVTISCSGSSSNIGSNPVNWYQQLPGTAPKLLIYSNNQRPSGVPDRFSGS KSGTSASLAISGLQSEDEADYFCAAWDDSLSGPVFGGGTKLTVL

SEQ ID NO: 384 (C33B1522VL1-3)

QSVLTQPPSASGTPGQRVTISCSGSSSNIGSNPVNWYQQLPGTAPKLLIYSNNQRPSGVPDRFSGS KSGTSASLAISGLQSEDEADYFCAAWDDSLQGPVFGGGTKLTVL

SEQ ID NO: 385 (C33B1522VL1-4)

QSVLTQPPSASGTPGQRVTISCSGSSSNIGSNPVNWYQQLPGTAPKLLIYSNNQRPSGVPDRFSGS KSGTSASLAISGLQSEDEADYFCAAWDDSLVGPVFGGGTKLTVL

SEQ ID NO: 386 (C33B1522VL1-5)

QSVLTQPPSASGTPGQRVTISCSGSSSNIGSNPVNWYQQLPGTAPKLLIYSNNQRPSGVPDRFSGS KSGTSASLAISGLQSEDEADYFCAAWDDSLGGPVFGGGTKLTVL

SEQ ID NO: 387 (C33B1522VL1-7)

QSVLTQPPSASGTPGQRVTISCSGSSSNIGSNPVNWYQQLPGTAPKLLIYSNNQRPSGVPDRFSGS KSGTSASLAISGLQSEDEADYFCAAWDDSLWGPVFGGGTKLTVL

SEQ ID NO: 388 (C33B1522VL1-12)

QSVLTQPPSASGTPGQRVTISCSGSSSNIGSNPVNWYQQLPGTAPKLLIYSNNQRPSGVPDRFSGS KSGTSASLAISGLQSEDEADYFCAAWDDSLTGPVFGGGTKLTVL

SEQ ID NO: 389 (C33B1522VL1-15)

QSVLTQPPSASGTPGQRVTISCSGSSSNIGSNPVNWYQQLPGTAPKLLIYSNNQRPSGVPDRFSGS KSGTSASLAISGLQSEDEADYFCAAWDDSLIGPVFGGGTKLTVL

SEQ ID NO: 390 (C33B1522VL1-27)

QSVLTQPPSASGTPGQRVTISCSGSSSNIGSNPVNWYQQLPGTAPKLLIYSNNQRPSGVPDRFSGS KSGTSASLAISGLQSEDEADYFCAAWDDSLEGPVFGGGTKLTVL

SEQ ID NO: 391 (C33B1522VL1-29)

QSVLTQPPSASGTPGQRVTISCSGSSSNIGSNPVNWYQQLPGTAPKLLIYSNNQRPSGVPDRFSGS KSGTSASLAISGLQSEDEADYFCAAWDDSLAGPVFGGGTKLTVL

SEQ ID NO: 392 (C33B1522VL1-32)

QSVLTQPPSASGTPGQRVTISCSGSSSNIGSNPVNWYQQLPGTAPKLLIYSNNQRPSGVPDRFSGS KSGTSASLAISGLQSEDEADYFCAAWDDSLRGPVFGGGTKLTVL

SEQ ID NO: 393 (C33B1522VL1-47)

QSVLTQPPSASGTPGQRVTISCSGSSSNIGSNPVNWYQQLPGTAPKLLIYSNNQRPSGVPDRFSGS KSGTSASLAISGLQSEDEADYFCAAWDDSLLGPVFGGGTKLTVL

SEQ ID NO: 394 (C33B1522VL2-82)

QSVLTQPPSASGTPGQRVTISCSGSSSNIGSNPVNWYQQLPGTAPKLLIYSNNQRPSGVPDRFSGS KSGTSASLAISGLQSEDEADYFCAAWDDSLNVPVFGGGTKLTVL

SEQ ID NO: 395 (C33B1522VL2-1)

QSVLTQPPSASGTPGQRVTISCSGSSSNIGSNPVNWYQQLPGTAPKLLIYSNNQRPSGVPDRFSGS KSGTSASLAISGLQSEDEADYFCAAWDDSLNAPVFGGGTKLTVL

SEO ID NO: 396 (C33B1522VL2-2)

QSVLTQPPSASGTPGQRVTISCSGSSSNIGSNPVNWYQQLPGTAPKLLIYSNNQRPSGVPDRFSGS KSGTSASLAISGLQSEDEADYFCAAWDDSLNPPVFGGGTKLTVL

SEQ ID NO: 397 (C33B1522VL2-8)

QSVLTQPPSASGTPGQRVTISCSGSSSNIGSNPVNWYQQLPGTAPKLLIYSNNQRPSGVPDRFSGS KSGTSASLAISGLQSEDEADYFCAAWDDSLNLPVFGGGTKLTVL

SEQ ID NO: 398 (C33B1522VL2-17)

QSVLTQPPSASGTPGQRVTISCSGSSSNIGSNPVNWYQQLPGTAPKLLIYSNNQRPSGVPDRFSGS KSGTSASLAISGLQSEDEADYFCAAWDDSLNTPVFGGGTKLTVL

SEQ ID NO: 399 HC AbM CDR1 (C33B1522)
GGSISSYYWG

SEQ ID NO: 400 HC AbM CDR1 (C33B14771
GFTFSVSAIH

SEQ ID NO: 401 HC AbM CDR1 (C33B1475)
GFTFSSYWMT

SEQ ID NO: 402 HC AbM CDR1 (C33B1517)
GASIRNYYWS

SEQ ID NO: 403 HC AbM CDR1 (G33B1516)
GGSIRNYYWS

SEQ ID NO: 404 HC AbM CDR1 (C33B1481)
GITFSNYWMS

SEQ ID NO: 405 HC AbM CDR2 (C33B1522)
YIYYSGSTN

SEQ ID NO: 406 HC AbM CDR2 (C33B1477)
RIRSKGNSYATA

SEQ ID NO: 407 HC AbM CDR2 (C33B1475)
NIKODGSERY

SEQ ID NO: 408 HC AbM CDR2 (C33B1517)
HIYSTGNIH

SEQ ID NO: 409 HC AbM CDR2 (C33B1516)
HIFSTGHIN

SEQ ID NO: 410 HC AbM CDR2 (C33B1481)
SIKRDGSDKY

SEQ ID NO: 411 HC AbM CDR3 (C33B1522)
MWEILGFDP

SEQ ID NO: 412 HC AbM CDR3 (C33B1477)
HNDKWNYYGLDV

SEO ID NO: 413 HC AbM CDR3 (C33B1475)
EVGYNWNOGGYFDY

SEQ ID NO: 414 HC AbM CDR3 (C33B1517)
DNGAALFDY

SEQ ID NO: 415 HC AbM CDR3 (C33B1516)
DNGAALFDF

SEQ ID NO: 416 HC AbM CDR3 (C33B1481)
GEFDY

SEQ ID NO: 417 LC AbM CDR1 (C33B1522)
SGSSSNIGSNPVN

SEQ ID NO: 418 LC AbM CDR1 (C33B1477)
KSSOSLLFSNGYKFLD

SEQ ID NO: 419 LC AbM CDR1 (C33B1475)
RSSQSLLHSDGYNYLD

SEQ ID NO: 420 LC AbM CDR1 (C33B1517)
SGSSSNIGSNIVN

SEQ ID NO: 421 LC AbM CDR1 (C33B1516)
SGSSSNIGSNIVN

SEQ ID NO: 422 LC AbM CDR1 (C33B1481)
RSSQSLVYSDGNTYLN

SEQ ID NO: 423 LC AbM CDR2 (C33B1522)
SNNQRPS

SEQ ID NO: 424 LC AbM CDR2 (C33B1477)
LGSYRAS

SEQ ID NO: 425 LC AbM CDR2 (C33B1475)
LGSYRAS

SEQ ID NO: 426 LC AbM CDR2 (C33B1517)
SNNORPS

SEQ ID NO: 427 LC AbM CDR2 (C33B1516)
SDNQRPS

SEQ ID NO: 428 LC AbM CDR2 (C33B1481)

KVSTRDS

SEQ ID NO: 429 LC AbM CDR3 (C33B1522)
AAWDDSLNGPV

SEQ ID NO: 430 LC AbM CDR3 (C33B1477)
MQALQTPPT

SEQ ID NO: 431 LC AbM CDR3 (C33B1475)
MOVLOTPWT

SEQ ID NO: 432 LC AbM CDR3 (C33B 1517)
AAWDDSLNGPV

SEQ ID NO: 433 LC AbM CDR3 (C33B1516)
AAWDDSLNGPV

SEQ ID NO: 434 LC AbM CDR3 (C33B1481)
LQGTHWPWT

SEQ ID NO: 435 HC CHOTHIA CDR1 (C33B1522)
GGSISSY

SEQ ID NO: 436 HC CHOTHIA CDR1 (C33B1477)
GFTFSVS

SEQ ID NO: 437 HC CHOTHIA CDR1 (C33B1475)
GFTFSSY

SEQ ID NO: 438 HC CHOTHIA CDR1 (C33B1517)
GASIRNY

SEQ ID NO: 439 HC CHOTHIA CDR1 (C33B1516)
GGSIRNY

SEQ ID NO: 440 HC CHOTHIA CDR1 (C33B1481)
GITFSNY

SEQ ID NO: 441 HC CHOTHIA CDR2 (C33B1522)
YYSGS

SEQ ID NO: 442 HC CHOTHIA CDR2 (C33B1477)
RSKGNSYA

SEQ ID NO: 443 HC CHOTHIA CDR2 (C33B1475)
KODGSE

SEQ ID NO: 444 HC CHOTHIA CDR2 (C33B1517)
YSTGN

SEQ ID NO: 445 HC CHOTHIA CDR2 (C33B1516)
FSTGH

SEQ ID NO: 446 HC CHOTHIA CDR2 (C33B1481)
KRDGSD

SEQ ID NO: 447 HC CHOTHIA CDR3 (C33B1522)
MWEILGFD

SEQ ID NO: 448 HC CHOTHIA CDR3 (C33B1477)
HNDKWNYYGLD

SEQ ID NO: 449 HC CHOTHIA CDR3 (C33B1475)
EVGYNWNQGGYFD

SEQ ID NO: 450 HC CHOTHIA CDR3 (C33B1517)
DNGAALFD

SEQ ID NO: 451 HC CHOTHIA CDR3 (C33B1516)
DNGAALFD

SEQ ID NO: 452 HC CHOTHIA CDR3 (C33B1481)
GEFD

SEQ ID NO: 453 LC CHOTHIA CDR1 (C33B1522)
SSSNIGSNP

SEQ ID NO: 454 LC CHOTHIA CDR1 (C33B1477)
SQSLLFSNGYKF

SEQ ID NO: 455 LC CHOTHIA CDR1 (C33B1475)
SOSLLHSDGYNY

SEQ ID NO: 456 LC CHOTHIA CDR1 (C33B1517)
SSSNIGSNI

SEQ ID NO: 457 LC CHOTHIA CDR1 (C33B1516)
SSSNIGSNI

SEQ ID NO: 458 LC CHOTHIA CDR1 (C33B1481)
SQSLVYSDGNTY

SEQ ID NO: 459 LC CHOTHIA CDR2 (C33B1522)
SNN

SEQ ID NO: 460 LC CHOTHIA CDR2 (C33B1477)
LGS

SEQ ID NO: 461 LC CHOTHIA CDR2 (C33B1475)
LGS

SEQ ID NO: 462 LC CHOTHIA CDR2 (C33B1517)
SNN

SEQ ID NO: 463 LC CHOTHIA CDR2 (C33B1516)
SDN

SEQ ID NO: 464 LC CHOTHIA CDR2 (C33B 148 1)
KVS

SEQ ID NO: 465 LC CHOTHIA CDR3 (C33B1522)
WDDSLNGP

SEQ ID NO: 466 LC CHOTHIA CDR3 (C33B1477)
AL TPP

SEQ ID NO: 467 LC CHOTHIA CDR3 (C33B1475)

VLOTPW

SEQ ID NO: 468 LC CHOTHIA CDR3 (C33B1517)
WDDSLNGP

SEQ ID NO: 469 LC CHOTHIA CDR3 (C33B1516)
WDDSLNGP

SEQ ID NO: 470 LC CHOTHIA CDR3 (C33B1481)
GTHWPW

SEQ ID NO: 471 HC IMGT CDR1 (C33B1522)
GGSISSYY

SEQ ID NO: 472 HC IMGT CDR1 (C33B1477)
GFTFSVSA

SEQ ID NO: 473 HC IMGT CDR1 (C33B1475)
GFTFSSYW

SEQ ID NO: 474 HC IMGT CDR1 (C33B1517)
GASIRNYY

SEQ ID NO: 475 HC IMGT CDR1 (C33B1516)
GGSIRNYY

SEQ ID NO: 476 HC IMGT CDR1 (C33B1481)
GITFSNYW

SEQ ID NO: 477 HC IMGT CDR2 (C33B1522)
IYYSGST

SEQ ID NO: 478 HC IMGT CDR2 (C33B1477)
IRSKGNSYAT

SEQ ID NO: 479 HC IMGT CDR2 (C33B1475)
IKODGSER

SEQ ID NO: 480 HC IMGT CDR2 (C33B1517)
IYSTGNI

SEQ ID NO: 481 HCIMGTCDR2 (C33B1516)
IFSTGHI

SEQ ID NO: 482 HC IMGT CDR2 (C33B1481)
IKRDGSDK

SEQ ID NO: 483 HC IMGT CDR3 (C33B1522)
ARMWEILGFDP

SEQ ID NO: 484 HC IMGT CDR3 (C33B1477)
TRHNDKWNYYGLDV

SEQ ID NO: 485 HC IMGT CDR3 (C33B1475)
AREVGYNWNOGGYFDY

SEQ ID NO: 486 HC IMGT CDR3 (C33B1517)
ARDNGAALFDY

SEQ ID NO: 487 HC IMGT CDR3 (C33B1516)
ARDNGAALFDF

SEQ ID NO: 488 HC IMGT CDR3 (C33B1481)
AKGEFDY

SEQ ID NO: 489 LC IMGT CDR1 (C33B1522)
SSNIGSNP

SEQ ID NO: 490 LC IMGT CDR1 (C33B1477)
QSLLFSNGYKF

SEQ ID NO: 491 LC IMGT CDR1 (C33BI475)
QSLLHSDGYNY

SEQ ID NO: 492 LC IMGT CDR1 (C33B1517)
SSNIGSNI

SEQ ID NO: 493 LC IMGT CDR1 (C33B1516)
SSNIGSNI

SEQ ID NO: 494 LC IMGT CDR1 (C33B1481)
QSLVYSDGNTY

SEQ ID NO: 495 LC IMGT CDR2 (C33B1522)
SNN

SEQ ID NO: 496 LC IMGT CDR2 (C33B1477)
LGS

SEQ ID NO: 497 LC IMGT CDR2 (C33B14751
LGS

SEQ ID NO: 498 LC IMGT CDR2 (C33B1517)
SNN

SEQ ID NO: 499 LC IMGT CDR2 (C33B1516)
SDN

SEQ ID NO: 500 LC IMGT CDR2 (C33B1481)
KVS

SEQ ID NO: 501 LC IMGT CDR3 (C33B1522)
AAWDDSLNGPV

SEQ ID NO: 502 LC IMGT CDR3 (C33B1477)
MOALOTPPT

SEQ ID NO: 503 LC IMGT CDR3 (C33B1475)
MOVLOTPWT

SEQ ID NO: 504 LC IMGT CDR3 (C33B1517)
AAWDDSLNGPV

SEQ ID NO: 505 LC IMGT CDR3 (C33B1516)
AAWDDSLNGPV

SEQ ID NO: 506 LC IMGT CDR3 (C33B1481)

LQGTHWPWT

SEQ ID NO: 507 HC CONTACT CDR1 (C33B1522)
SSYYWG

SEQ ID NO: 508 HC CONTACT CDR1 (C33B1477)
SVSAIH

SEQ ID NO: 509 HC CONTACT CDR1 (C33B1475)
SSYWMT

SEQ ID NO: 510 HC CONTACT CDR1 (C33B1517)
RNYYWS

SEQ ID NO: 511 HC CONTACT CDR1 (C33B1516)
RNYYWS

SEQ ID NO: 512 HC CONTACT CDR1 (C33B1481)
SNYWMS

SEQ ID NO: 513 HC CONTACT CDR2 (C33B1522)
WIGYIYYSGSTN

SEQ ID NO: 514 HC CONTACT CDR2 (C33B1477)
WIGRIRSKGNSYATA

SEQ ID NO: 515 HC CONTACT CDR2 (C33B1475)
WVANIKODGSERY

SEQ ID NO: 516 HC CONTACT CDR2 (C33B1517)
WLGHIYSTGNIH

SEQ ID NO: 517 HC CONTACT CDR2 (C33B1516)
WFGHIFSTGHIN

SEQ ID NO: 518 HC CONTACT CDR2 (C33B1481)
WVASIKRDGSDKY

SEQ ID NO: 519 HC CONTACT CDR3 (C33B1522)
ARMWEILGFD

SEQ ID NO: 520 HC CONTACT CDR3 (C33B1477)
TRHNDKWNYYGLD

SEQ ID NO: 521 HC CONTACT CDR3 (C33B1475)
AREVGYNWNOGGYFD

SEQ ID NO: 522 HC CONTACT CDR3 (C33B1517)
ARDNGAALFD

SEQ ID NO: 523 HC CONTACT CDR3 (C33B1516)
ARDNGAALFD

SEQ ID NO: 524 HC CONTACT CDR3 (C33B1481)
AKGEFD

SEQ ID NO: 525 LC CONTACT CDR1 (C33B1522)
IGSNPVNWY

SEQ ID NO: 526 LC CONTACT CDR1 (C33B1477)
LFSNGYKFLDWY

SEQ ID NO: 527 LC CONTACT CDR1 (C33B1475)
LHSDGYNYLDWY

SEQ ID NO: 528 LC CONTACT CDR1 (C33B1517)
IGSNIVNWY

SEQ ID NO: 529 LC CONTACT CDR1 (C33B1516)
IGSNIVNWY

SEQ ID NO: 530 LC CONTACT CDR1 (C33B14811
VYSDGNTYLNWF

SEQ ID NO: 531 LC CONTACT CDR2 (C33B1522)
LLIYSNNORP

SEQ ID NO: 532 LC CONTACT CDR2 (C33B1477)
LLIYLGSYRA

SEQ ID NO: 533 LC CONTACT CDR2 (C33B1475)
LLIYLGSYRA

SEQ ID NO: 534 LC CONTACT CDR2 (C33B1517)
LLIYSNNORP

SEQ ID NO: 535 LC CONTACT CDR2 (C33B1516)
LLLYSDNQRP

SEQ ID NO: 536 LC CONTACT CDR2 (C33B1481)
RLIYKVSTRD

SEQ ID NO: 537 LC CONTACT CDR3 (C33B1522)
AAWDDSLNGP

SEQ ID NO: 538 LC CONTACT CDR3 (C33B1477)
MOALOTPP

SEQ ID NO: 539 LC CONTACT CDR3 (C33B1475)
MOVLOTPW

SEQ ID NO: 540 LC CONTACT CDR3 (C33B1517)
AAWDDSLNGP

SEQ ID NO: 541 LC CONTACT CDR3 (C33B1516)
AAWDDSLNGP

SEQ ID NO: 542 LC CONTACT CDR3 (C33B1481)
LOGTHWPW

References

1. www_lls_org/sites/default/files/file_assets/PS32 AML_Booklet_2019 _FINAL.pdf

2. D H Wiseman, B F Greystoke & T C P Somervaille, The variety of leukemic stem cells in myeloid malignancy, ,

Oncogene volume 33, pages3091-3098(2014)

3. American Cancer Society: Cancer Facts and Figures 2014. Atlanta, Ga: American Cancer Society, 2014. Available onlineExit Disclaimer

4. www.uniprot.org/uniprot/P20138

5. Engagement of p75/AIRM1 or CD33 inhibits the proliferation of normal or leukemic myeloid cells, Vitale C., Romagnani C., Falco M., Ponte M., Vitale M., Moretta A., Bacigalupo A., Moretta L., Mingari M.C., Proc. Natl. Acad. Sci. U.S.A. 96:15091-15096(1999)

6. Ehninger A. Et al (2015) Blood Cancer Journal (2014) 4, e218

7. Felix S. Lichtenegger, et al., Recent developments in immunotherapy of acute myeloid leukemia, J Hematol Oncol. 2017; 10: 142

The present invention is not to be limited in scope by the specific embodiments described herein. Indeed, various modifications of the invention in addition to those described herein will become apparent to those skilled in the art from the foregoing description and the accompanying figures. Such modifications are intended to fall within the scope of the appended claims. It is further to be understood that all values are approximate, and are provided for description.

Patents, patent applications, publications, product descriptions, and protocols are cited throughout this application, the disclosures of which are incorporated herein by reference in their entireties for all purposes.

**Claims**

1. An isolated protein that binds CD33, wherein the isolated protein comprises the HCDR1, the HCDR2, the HCDR3, the LCDR1, the LCDR2 and the LCDR3 of SEQ ID NOs: 29, 37, 46, 63, 70, and 76, respectively.

2. The isolated protein of claim 1, wherein the isolated protein comprises the VH of SEQ ID NOs: 53, and the VL of SEQ ID NOs: 82.

3. The isolated protein of any one of claims 1-2, wherein the isolated protein is a Fab, a scFv, a $(scFv)_2$, a Fv, a $F(ab')_2$, a Fd, a dAb, or a VHH.

4. The isolated protein of claim 3, wherein the isolated protein is a scFv comprising, from the N- to C-terminus, (i) the VH, a first linker (L1), and the VL (VH-L1-VL), or (ii) the VL, the L1, and the VH (VL-L1-VH); and optionally wherein the L1 comprises:

   a) an amino acid sequence of any one of SEQ ID NOs: 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, or 140; and/or
   b) about 5-50 amino acids; about 5-40 amino acids; about 10-30 amino acids; or about 10-20 amino acids.

5. The isolated protein of claim 4, wherein the isolated protein is a scFv comprising the amino acid sequence of SEQ ID NO: 213.

6. The isolated protein of any one of claims 1-4, wherein the protein is conjugated to a half-life-extending or -modulating moiety selected from: an immunoglobulin (Ig); a fragment of the Ig; an Ig constant region; a fragment of the Ig constant region; a Fc region; a fragment of the Ig constant region comprising a Fc region; a fragment of the Ig constant region comprising a CH2 domain; a fragment of the Ig constant region comprising a CH3 domain; a fragment of the Ig constant region comprising a CH2 domain and a CH3 domain; a fragment of the Ig constant region comprising at least a portion of a hinge, a CH2 domain and a CH3 domain; a fragment of the Ig constant region comprising a hinge, a CH2 domain and a CH3 domain; transferrin; albumin; an albumin binding domain; or polyethylene glycol.

7. The isolated protein of claim 6, comprising an antigen binding domain that binds CD33, wherein:

   a) said antigen binding domain is conjugated to the N-terminus of the Ig constant region or the fragment of the Ig constant region;
   b) said antigen binding domain is conjugated to the C-terminus of the Ig constant region or the fragment of the Ig constant region; or
   c) said antigen binding domain is conjugated to the Ig constant region or the fragment of the Ig constant region via a second linker (L2) comprising the amino acid sequence of any one of SEQ ID NOs: 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, or 140.

8. The isolated protein of any one of claims 1-7, wherein the isolated protein is a monospecific protein, a multispecific protein, a bispecific protein, or a trispecific protein; optionally wherein the multispecific protein, the bispecific protein, or the trispecific protein, each independently, comprises an antigen binding domain that binds an antigen on: a lymphocyte, a T cell, a $CD8^+$ T cell, or a natural killer (NK) cell; optionally wherein the multispecific protein, the bispecific protein, or the trispecific protein, each independently, comprises an antigen binding domain that binds

TRGV9, CD3 epsilon (CD3ε), CD3, CD8, KI2L4, NKG2E, NKG2D, NKG2F, BTNL3, CD186, BTNL8, PD-1, CD195, or NKG2C.

**9.** The isolated protein of claim 8, wherein a first antigen binding domain that binds CD33 and/or a second antigen binding domain that binds the lymphocyte antigen comprise a Fab, a scFv, a VHH, a (scFv)2, a Fv, a F(ab')2, a Fd, or a dAb; or wherein the first antigen binding domain that binds CD33 comprises the scFv and the second antigen binding domain that binds the lymphocyte antigen comprises the VHH.

**10.** The isolated protein of any one of claims 6-9, wherein the Ig constant region or the fragment of the Ig constant region is an IgG1, an IgG2, an IgG3 or an IgG4 isotype, wherein optionally the Ig constant region or the fragment of the Ig constant region is an IgG1; optionally wherein the fragment of the Ig constant region has the sequence of SEQ ID NO: 277 or 278.

**11.** A pharmaceutical composition comprising the isolated protein of any one of claims 1-10, and a pharmaceutically acceptable carrier.

**12.** A polynucleotide encoding the isolated protein of any one of claims 1-10.

**13.** A vector comprising the polynucleotide of claim 12.

**14.** A host cell comprising the vector of claim 13 or polynucleotide of claim 12.

**15.** The isolated protein of any one of claims 1-10, for use in a method of treating a CD33 expressing cancer in a subject,wherein the CD33 expressing cancer is a hematologic cancer selected from a leukemia, a lymphoma, a multiple myeloma, acute myeloid leukemia (AML), myelodysplastic syndrome (MDS), acute lymphocytic leukemia (ALL), diffuse large B-cell lymphoma (DLBCL), chronic myeloid leukemia (CML), or blastic plasmacytoid dendritic cell neoplasm (DPDCN).

# FIG. 1

Ligand CL002405648

Analyte CD33-hu-domain

Sensogram Ch 1, Fc 2-1

Temperature 25

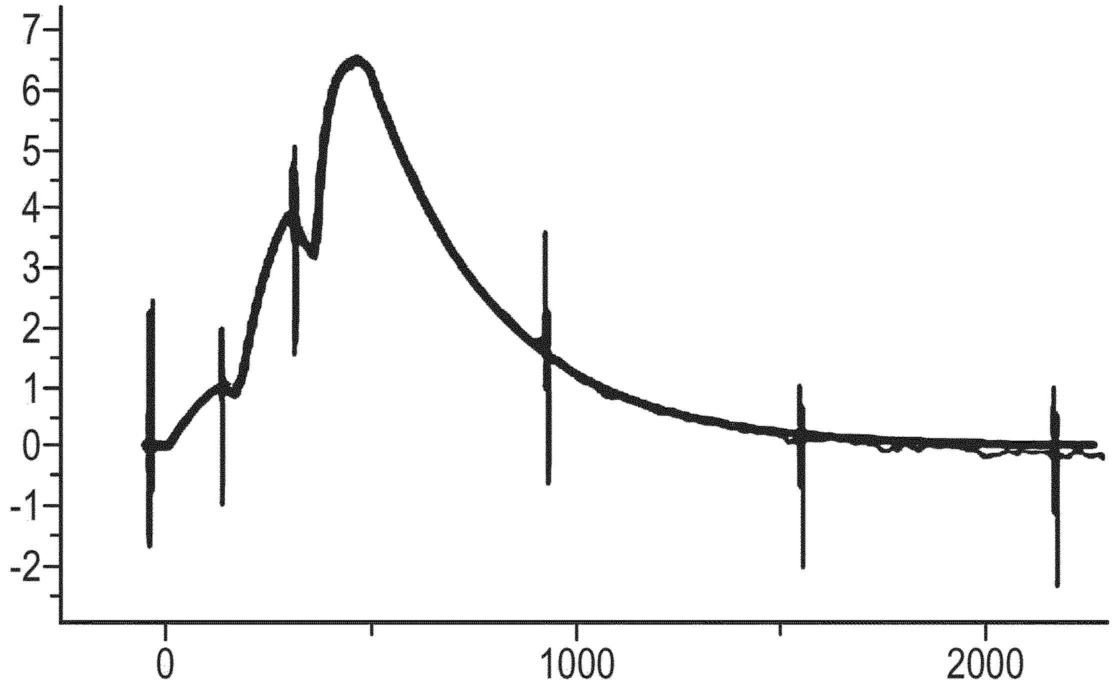


Model Group 1
1:1 binding

ka{1/Ms} 9.62e+05 kd{1/s} 3.25e+03

Rmax{RU} 7.1 KD {M} 3.38e-09

# FIG. 1(contd)

Ligand CL002405784

Analyte CD33-hu-domain

Sensogram Ch 6, Fc 2-1

Temperature 25

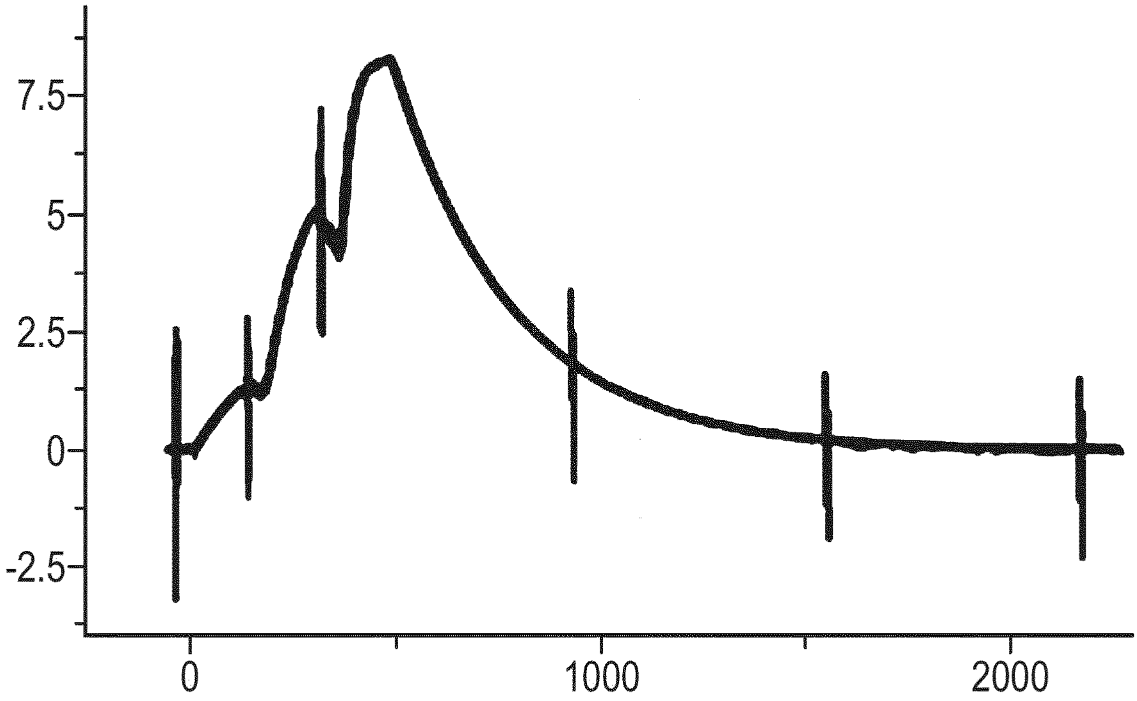


Model Group 6
1:1 binding

ka{1/Ms} 1.03e+06 kd{1/s} 3.40e-03

Rmax{RU} 9.0 KD {M} 3.31e-09

# FIG. 1(contd)

Ligand CL002405648
Analyte CD33-hu-FL
Sensogram Ch 1, Fc 2-1
Temperature 25

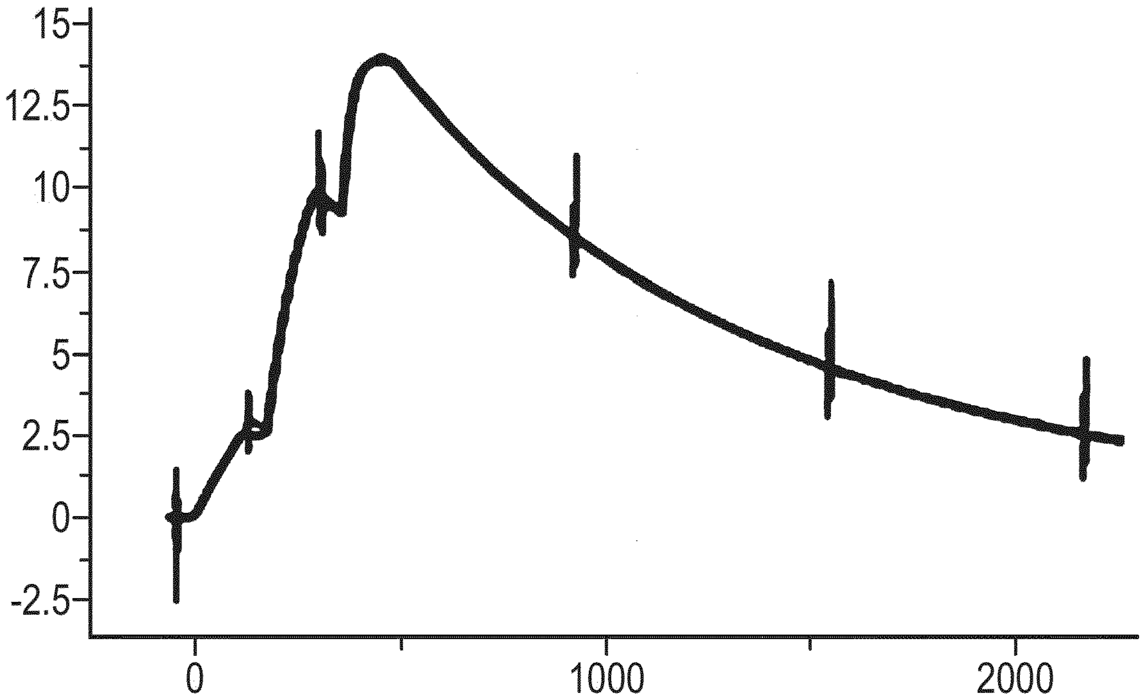


Model Group 9
1:1 binding

ka{1/Ms} 1.38e+06 kd{1/s} 1.17e-03

Rmax{RU} 14.2 KD {M} 8.47e-10

# FIG. 1(contd)

Ligand CL002405784

Analyte CD33-hu-FL

Sensogram Ch 6, Fc 2-1

Temperature 25

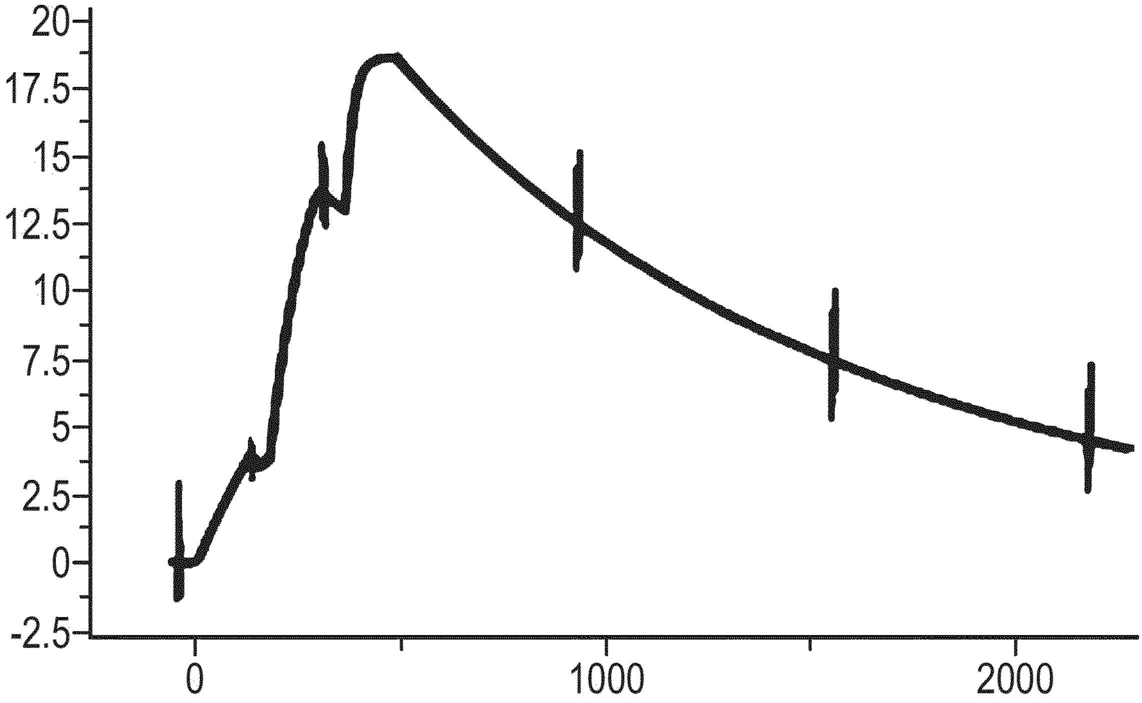


Model Group 19
1:1 binding

ka{1/Ms} 1.40e+06 kd{1/s} 9.28e-04

Rmax{RU} 18.9 KD {M} 6.61e-10

FIG. 2

A

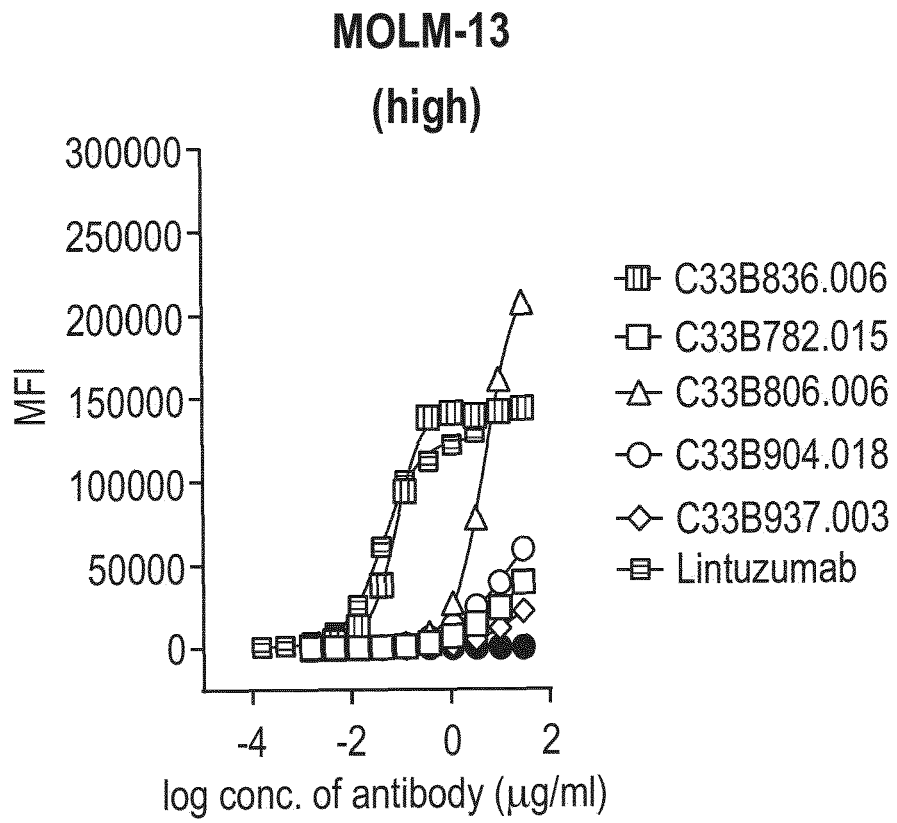
MOLM-13
(high)
300000
250000
200000
150000
100000
50000
0
MFI
-4
-2
0
2
log conc. of antibody (µg/ml)
C33B836.006
C33B782.015
C33B806.006
C33B904.018
C33B937.003
Lintuzumab

B

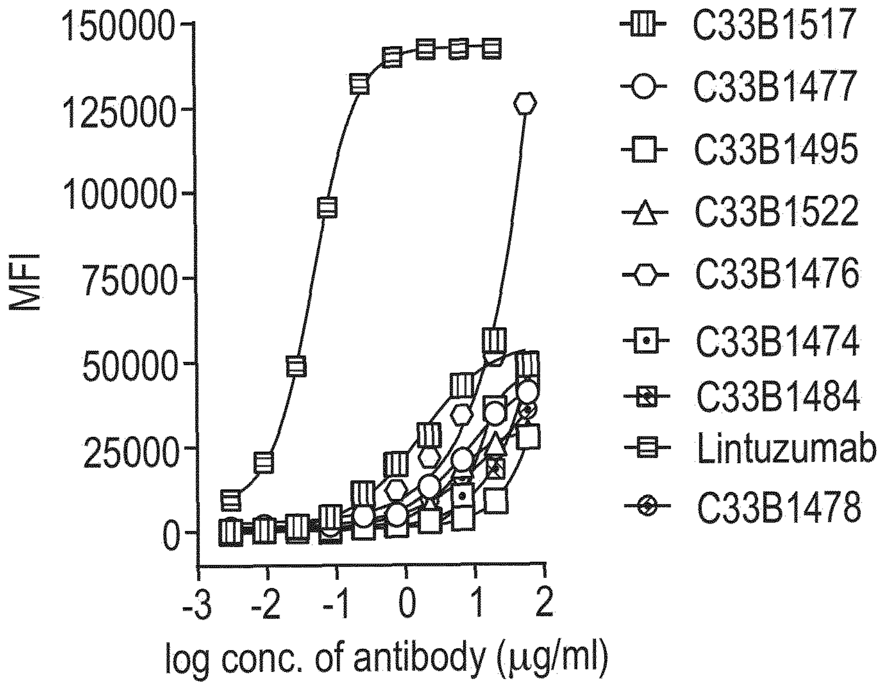
150000
125000
100000
75000
50000
25000
0
MFI
-3
-2
-1
0
1
2
log conc. of antibody (µg/ml)
C33B1517
C33B1477
C33B1495
C33B1522
C33B1476
C33B1474
C33B1484
Lintuzumab
C33B1478

# FIG. 2(contd)
## Kasumi-1
## (medium)

A
(contd)

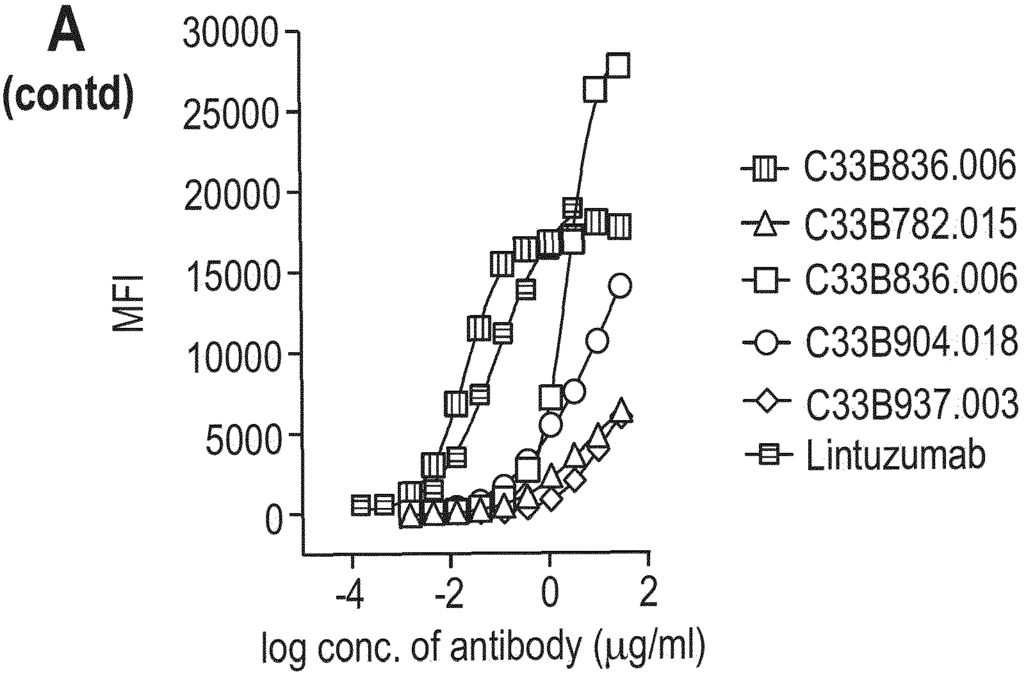


B
(contd)

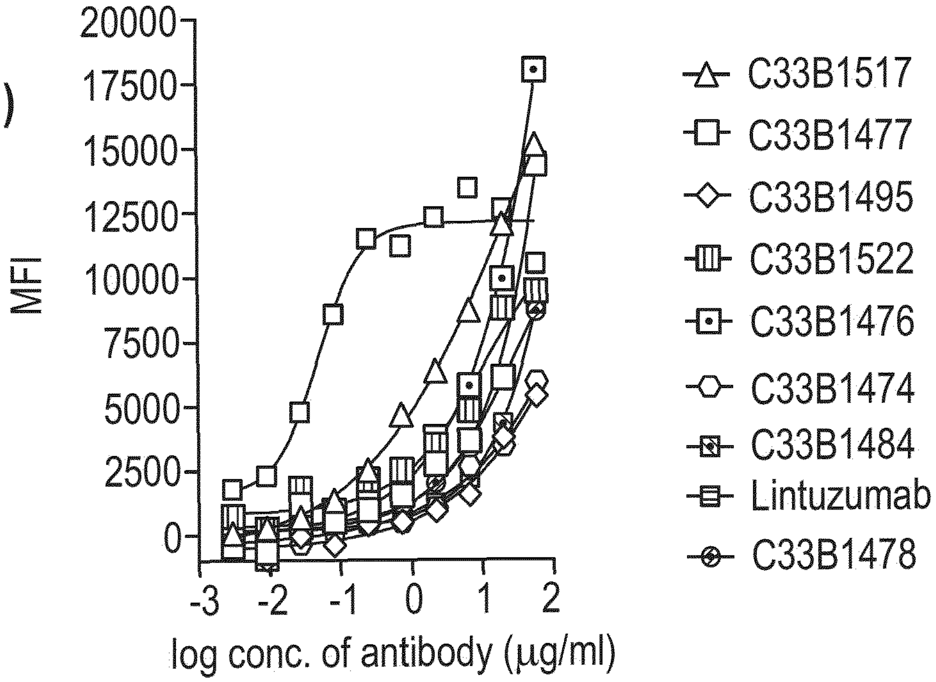

# FIG. 2(contd)

OCI-AML-3
(low)

A
(contd)

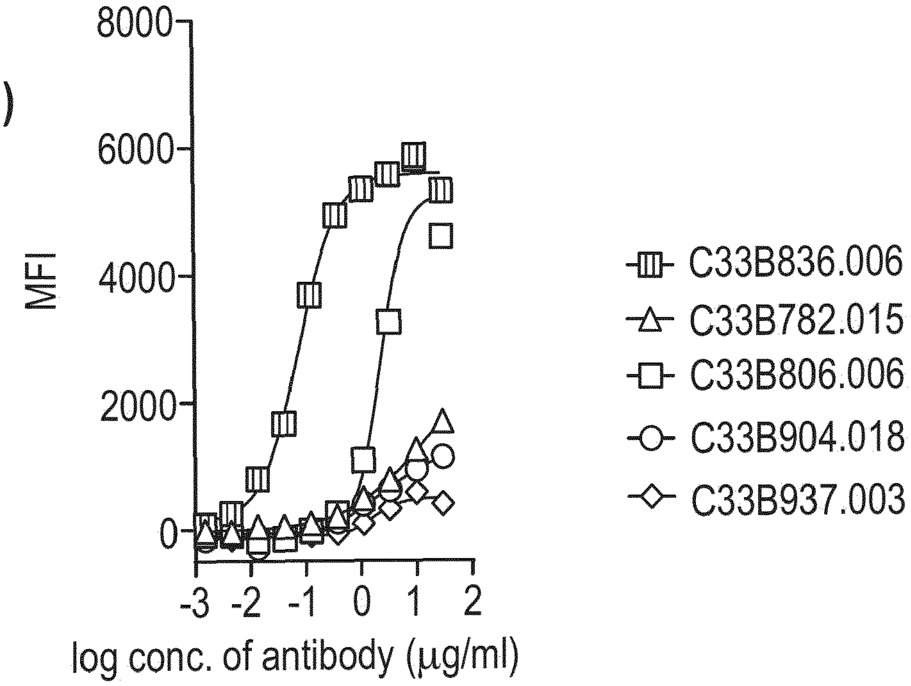


B
(contd)

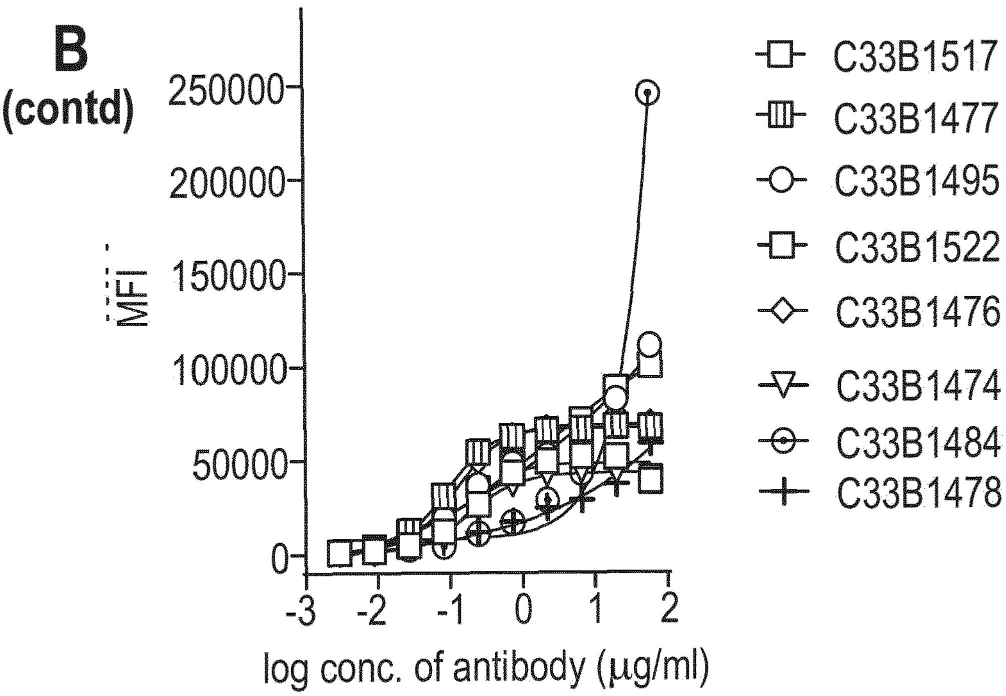

# FIG. 2(contd)

A
(contd)

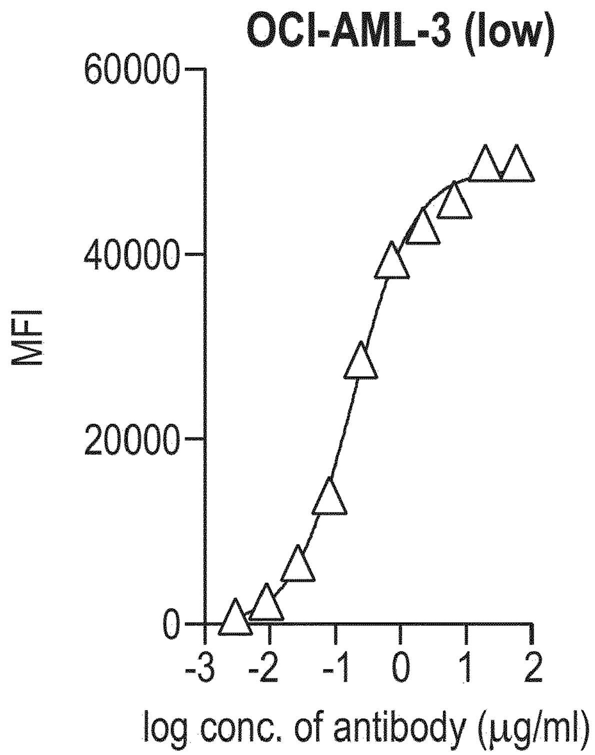


B
(contd)

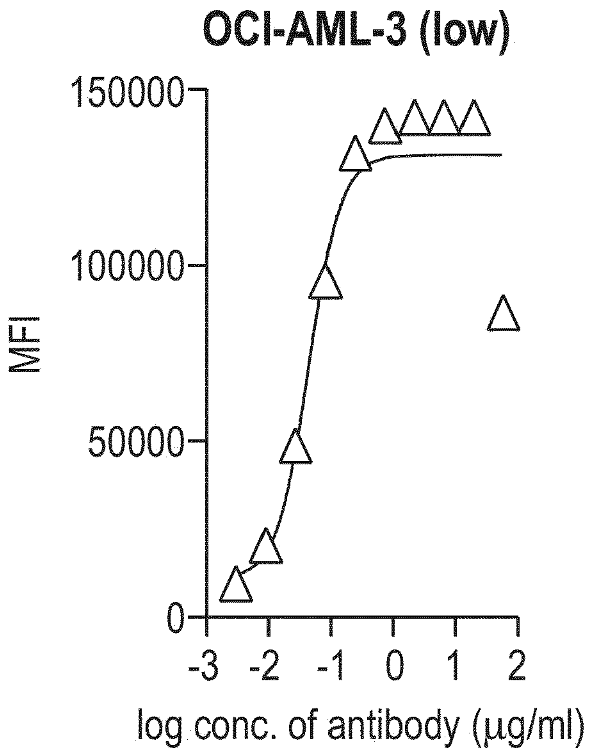

FIG. 3
C33B806.006

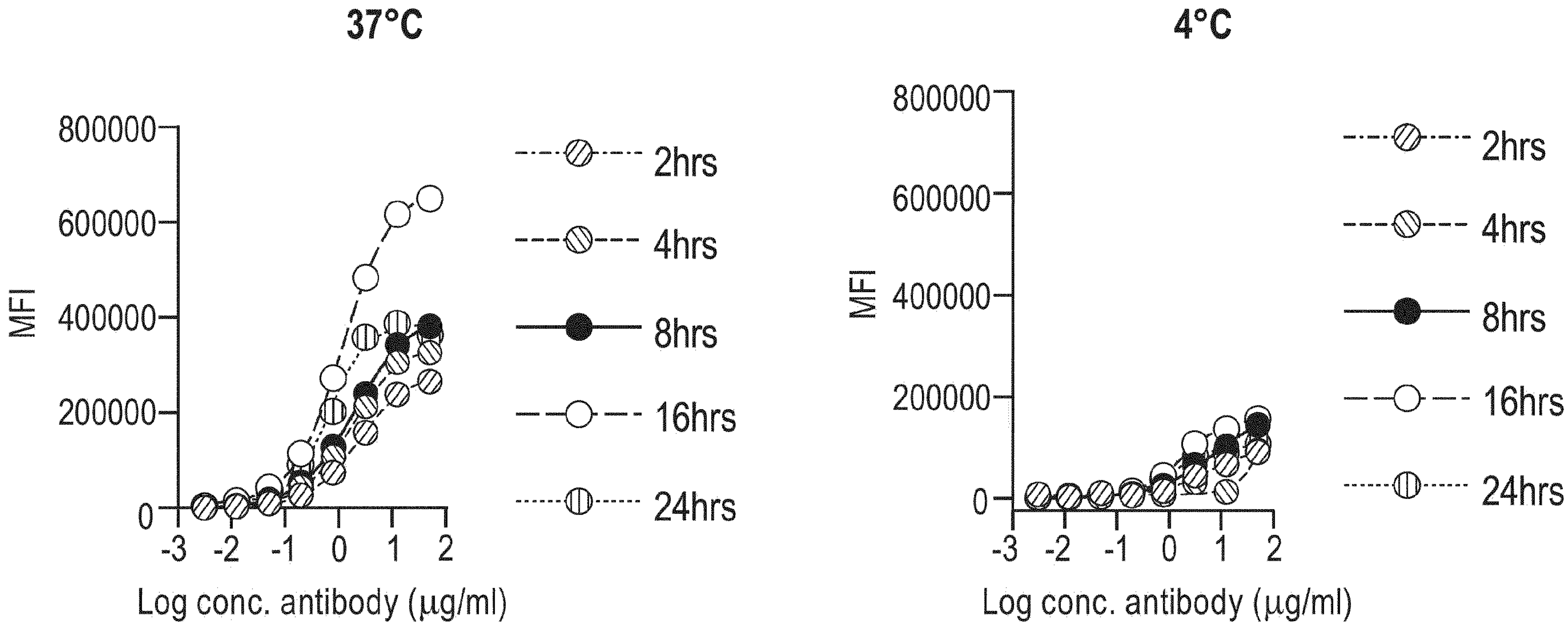

FIG. 3(contd)
C33B782.015

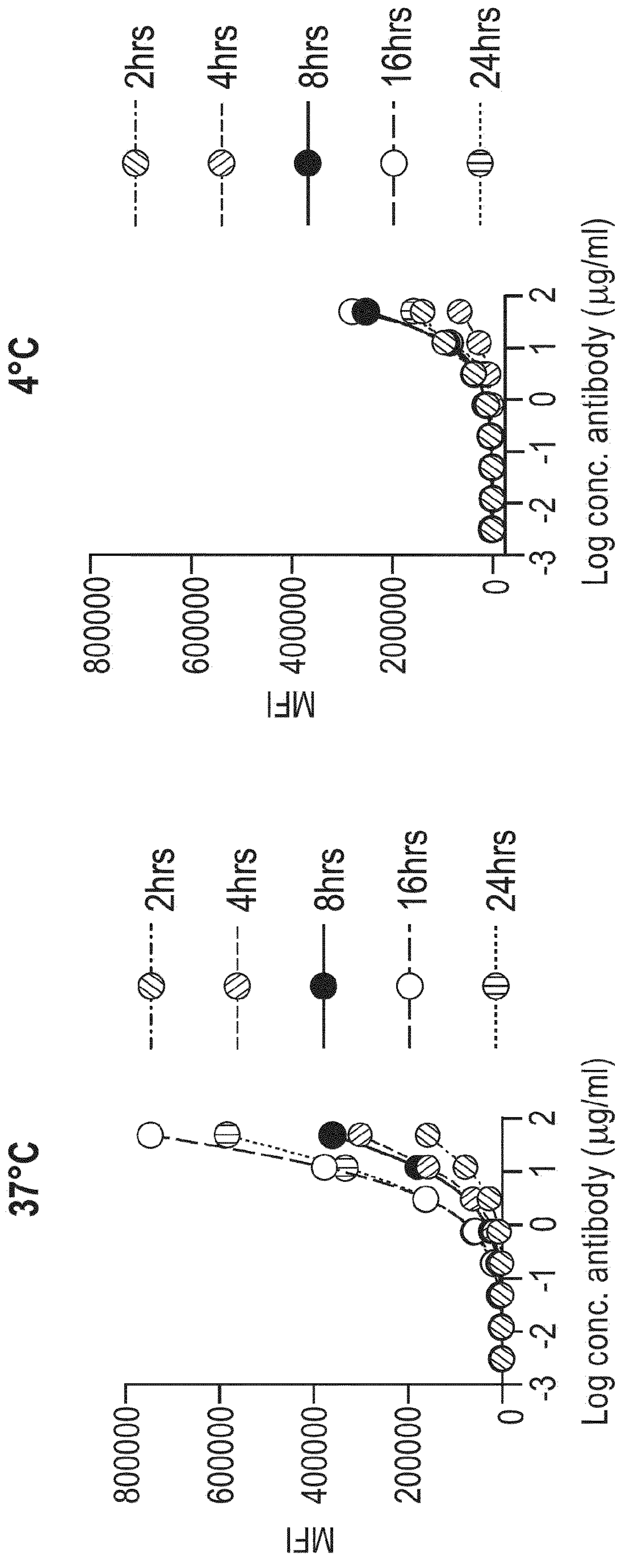

FIG. 3(contd)
C33B904.01

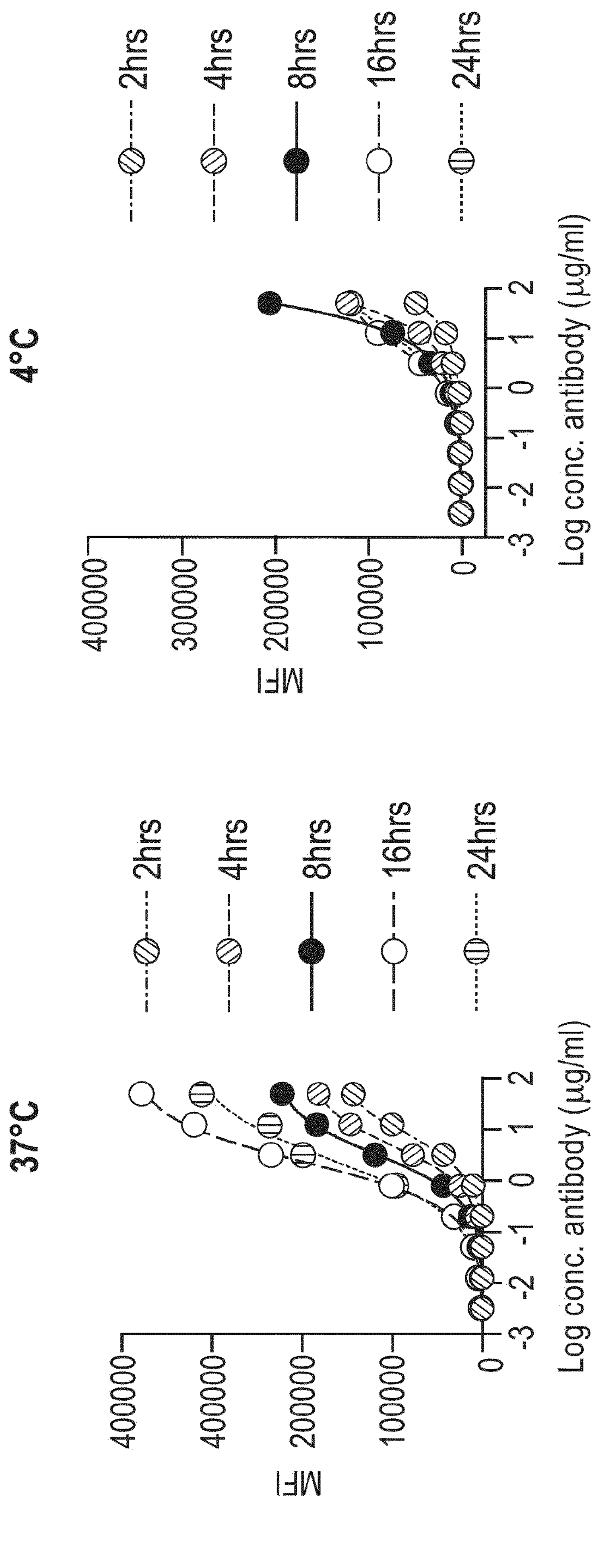

FIG. 3(contd)
C33B937.003

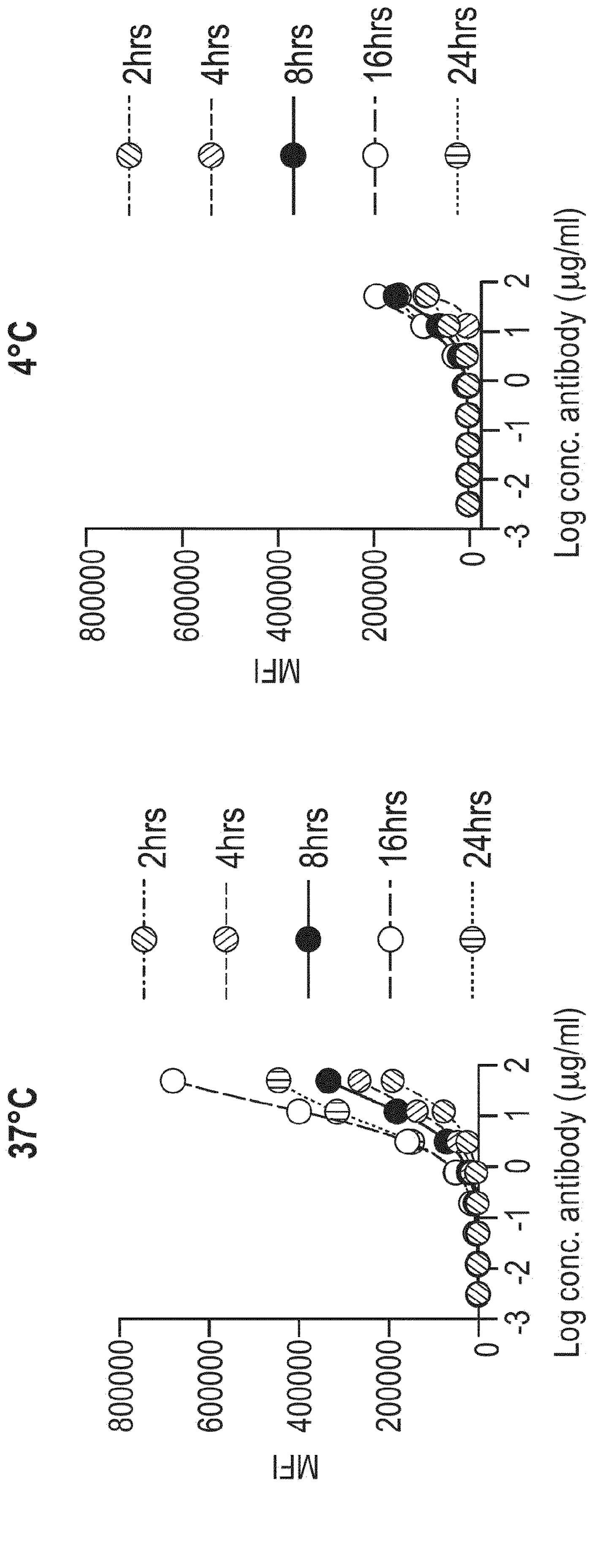

**FIG. 3(contd)**
**C33B836.006**

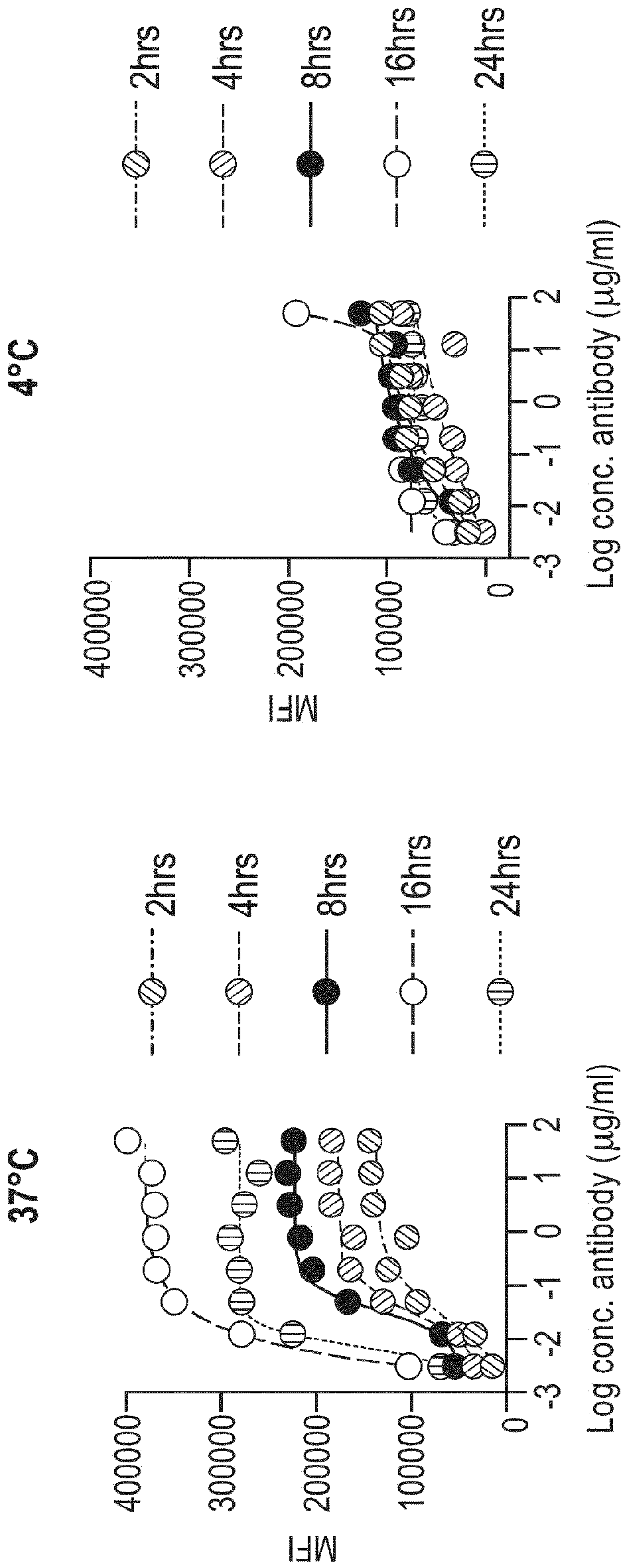

FIG. 3(contd)

isotype

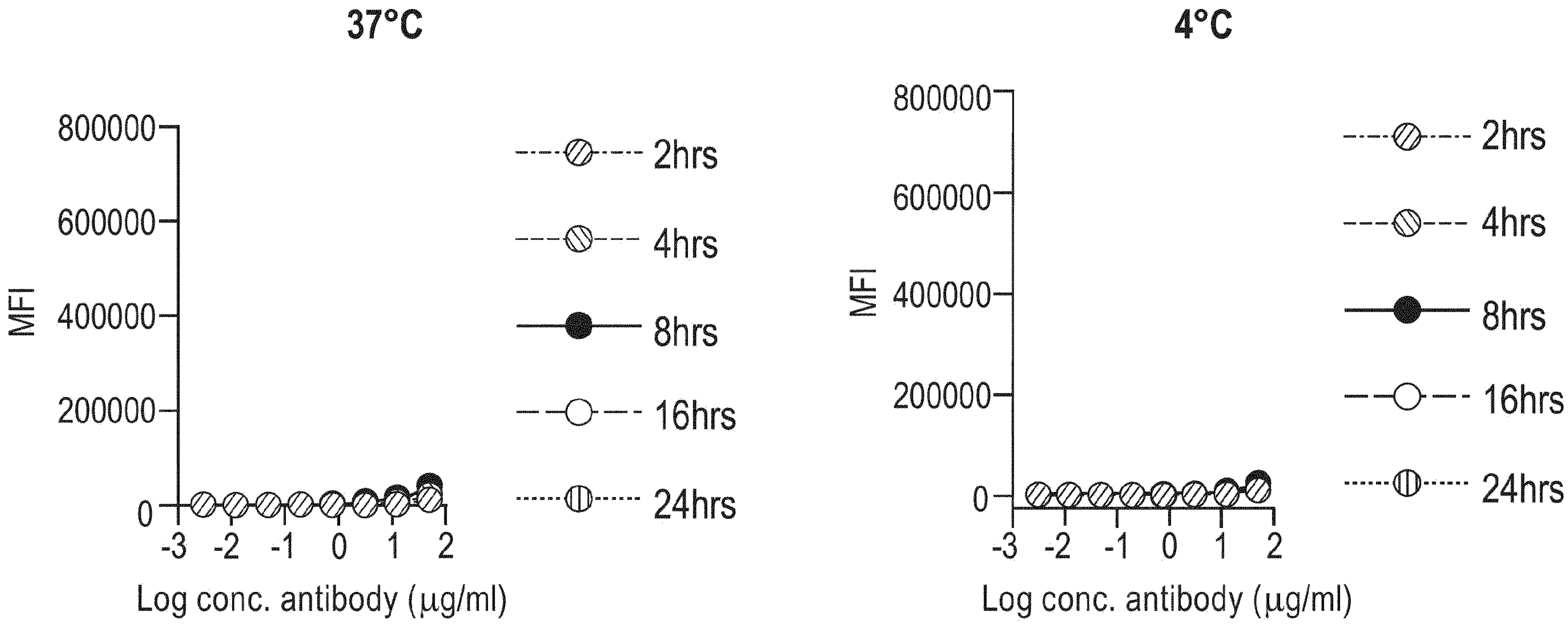

# FIG. 3(contd)

sCD33B1474

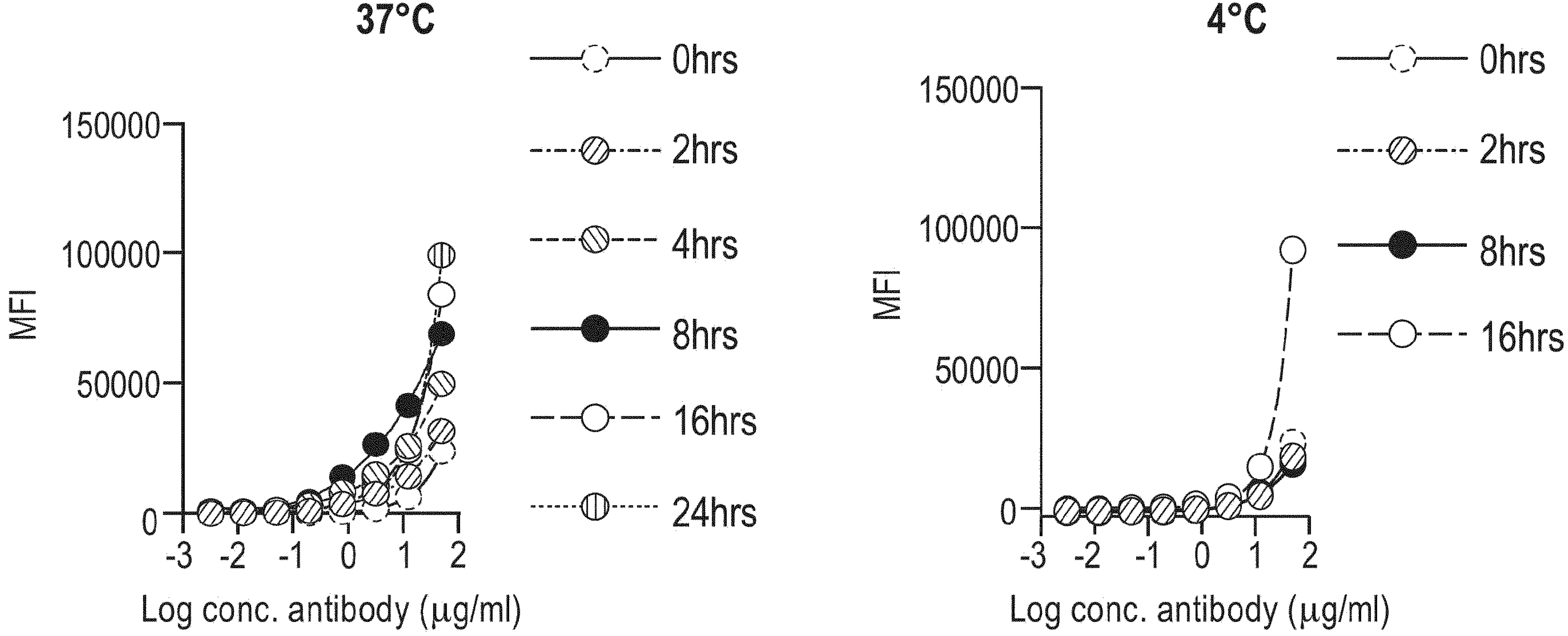

FIG. 3(contd)
sCD33B1478

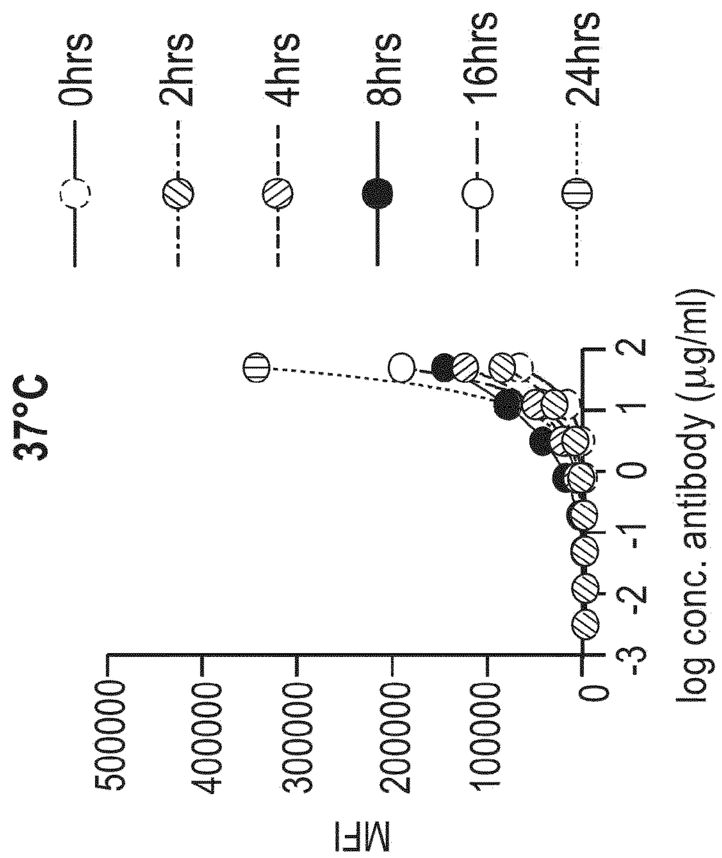


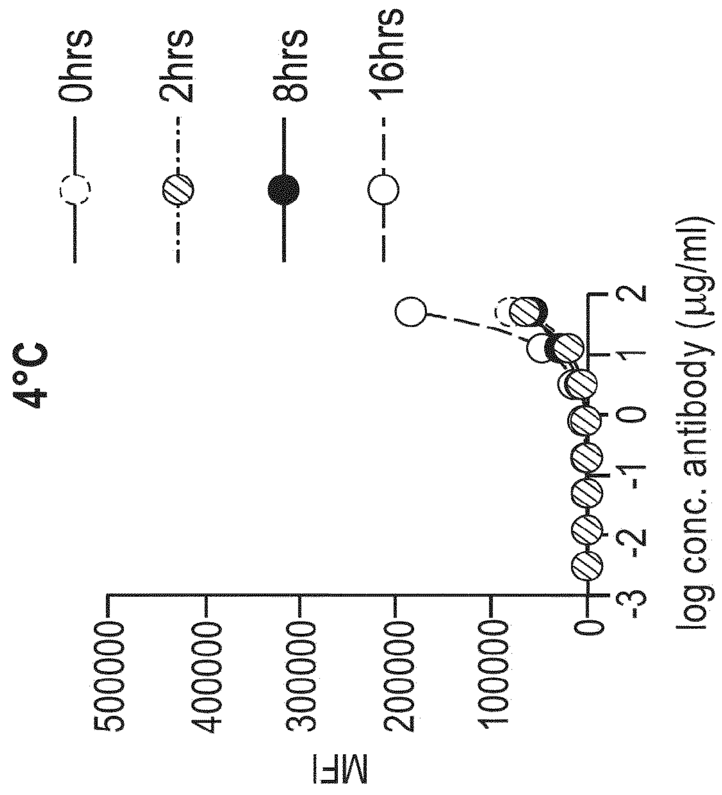

FIG. 3(contd)
sCD33B1517

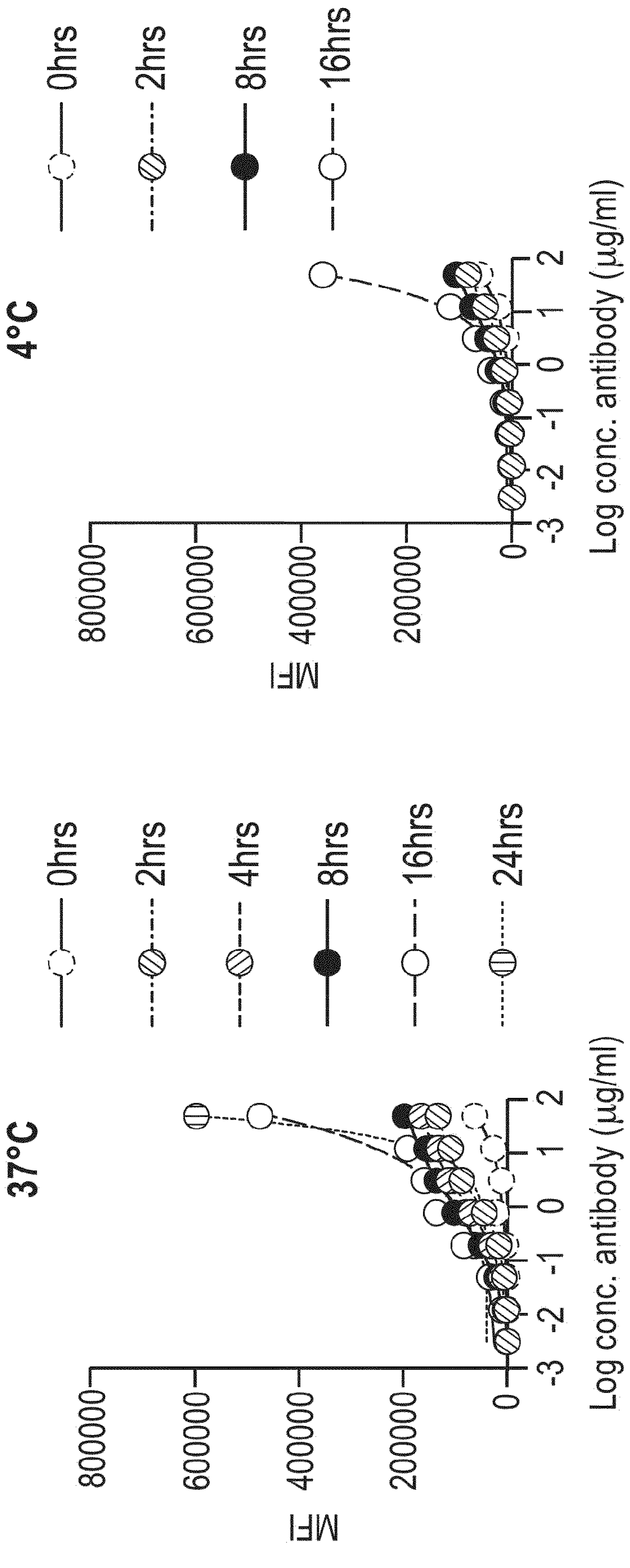

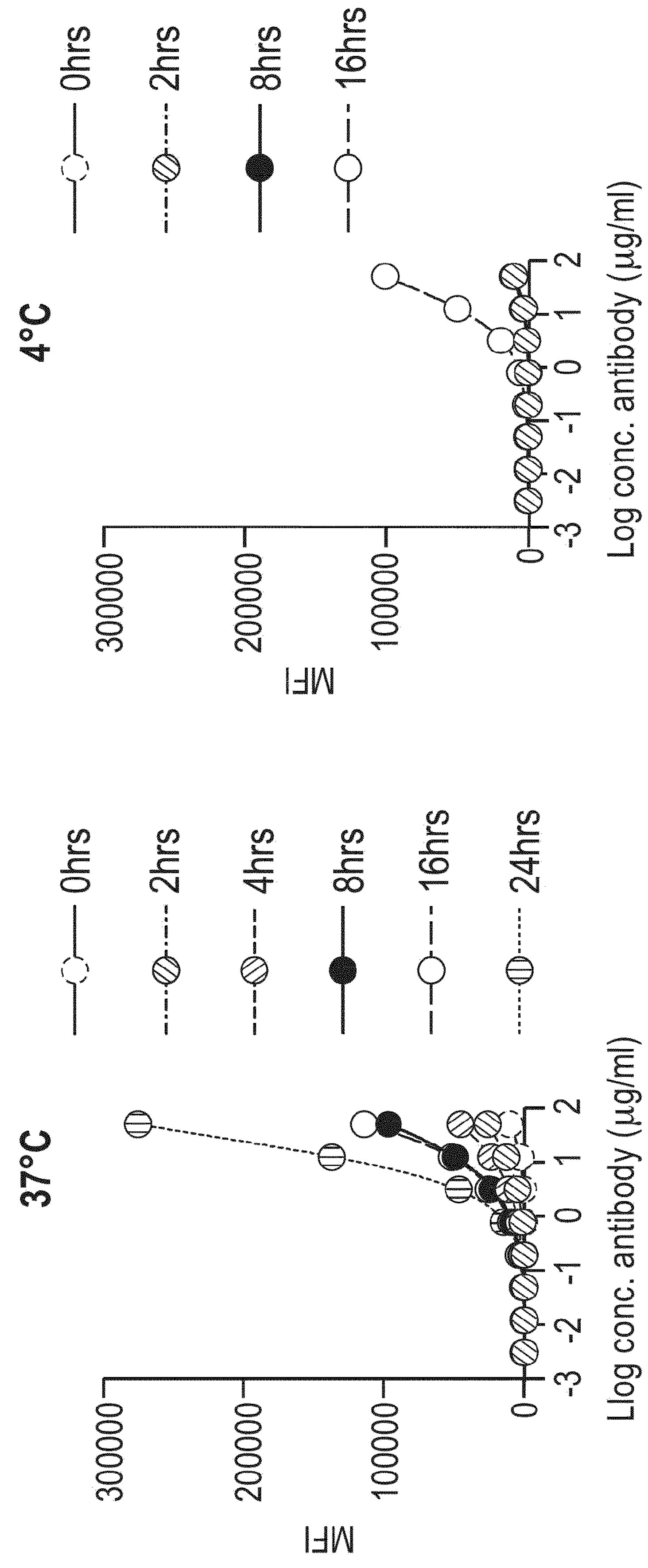


FIG. 3(contd)
sCD33B1476

FIG. 3(contd)
sCD33B1484

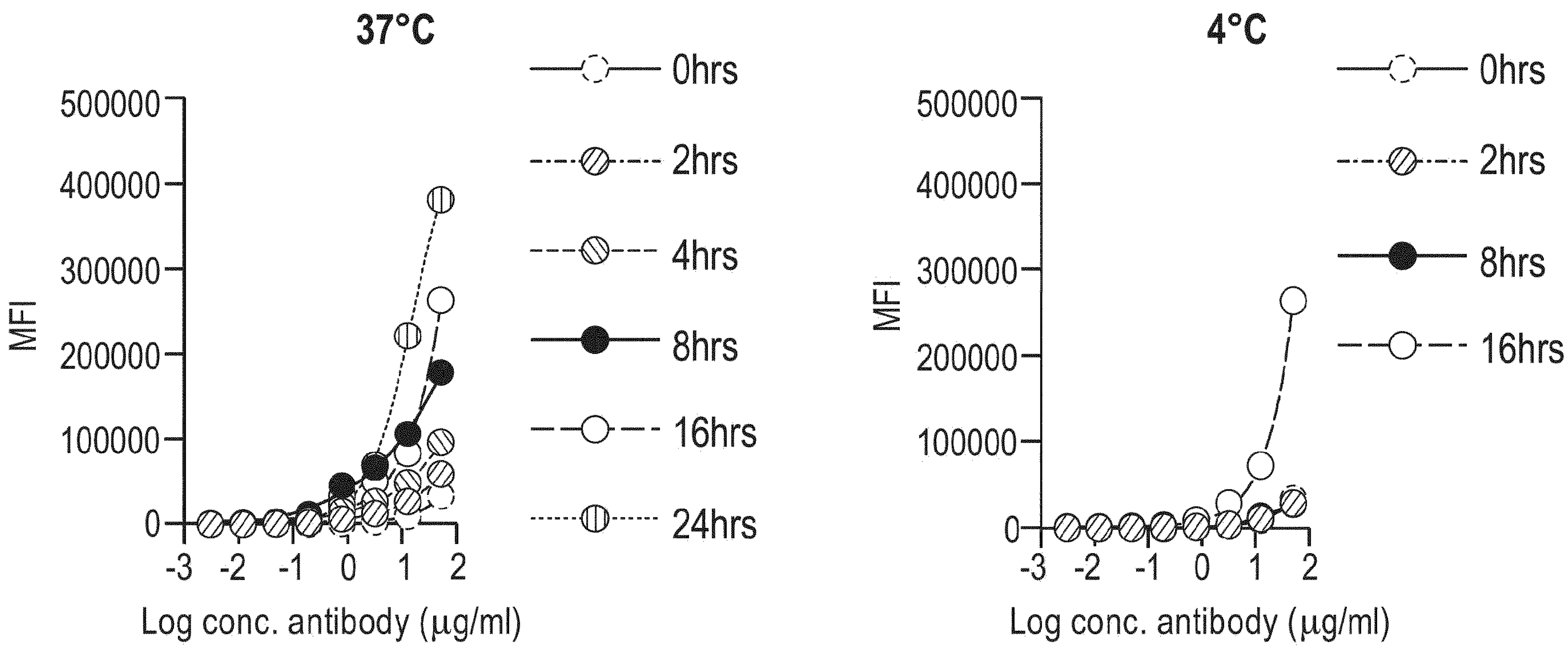

FIG. 3(contd)
sCD33B1522

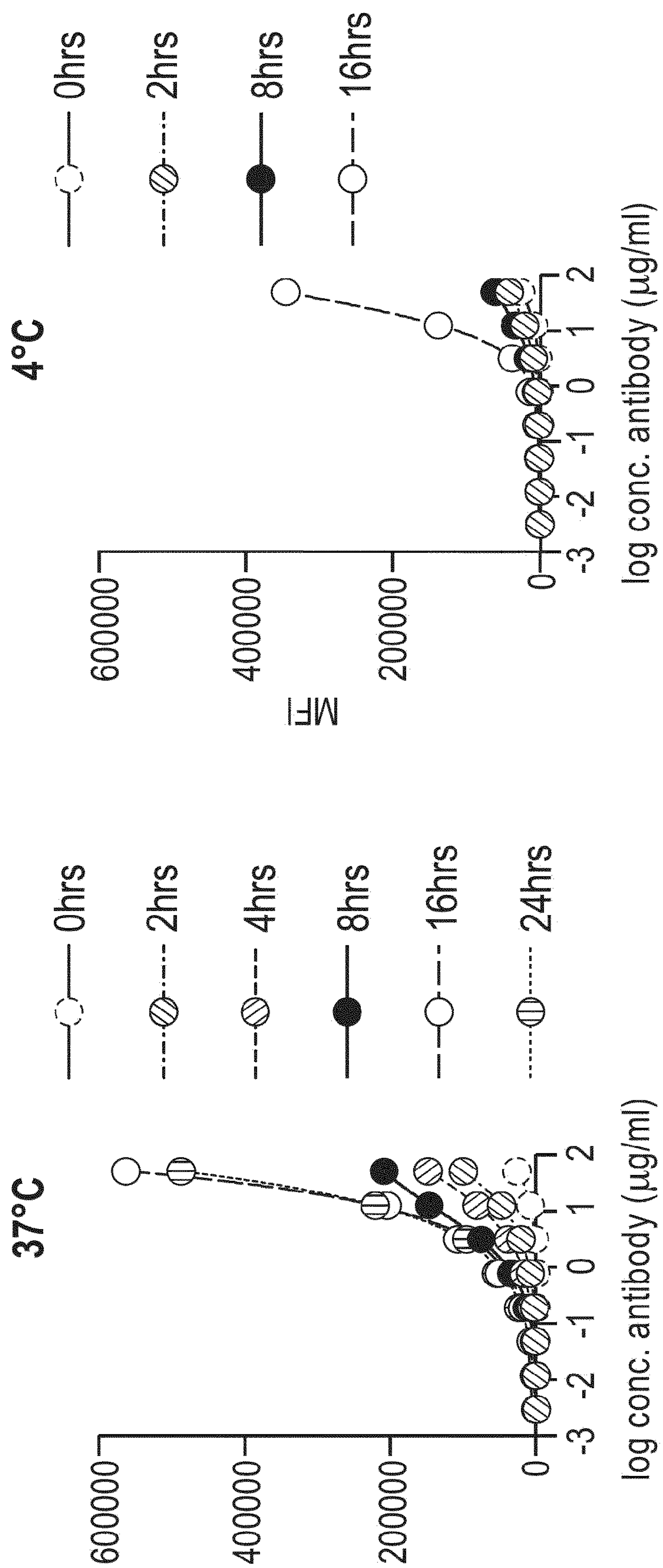

FIG. 3(contd)

sCD33B1477

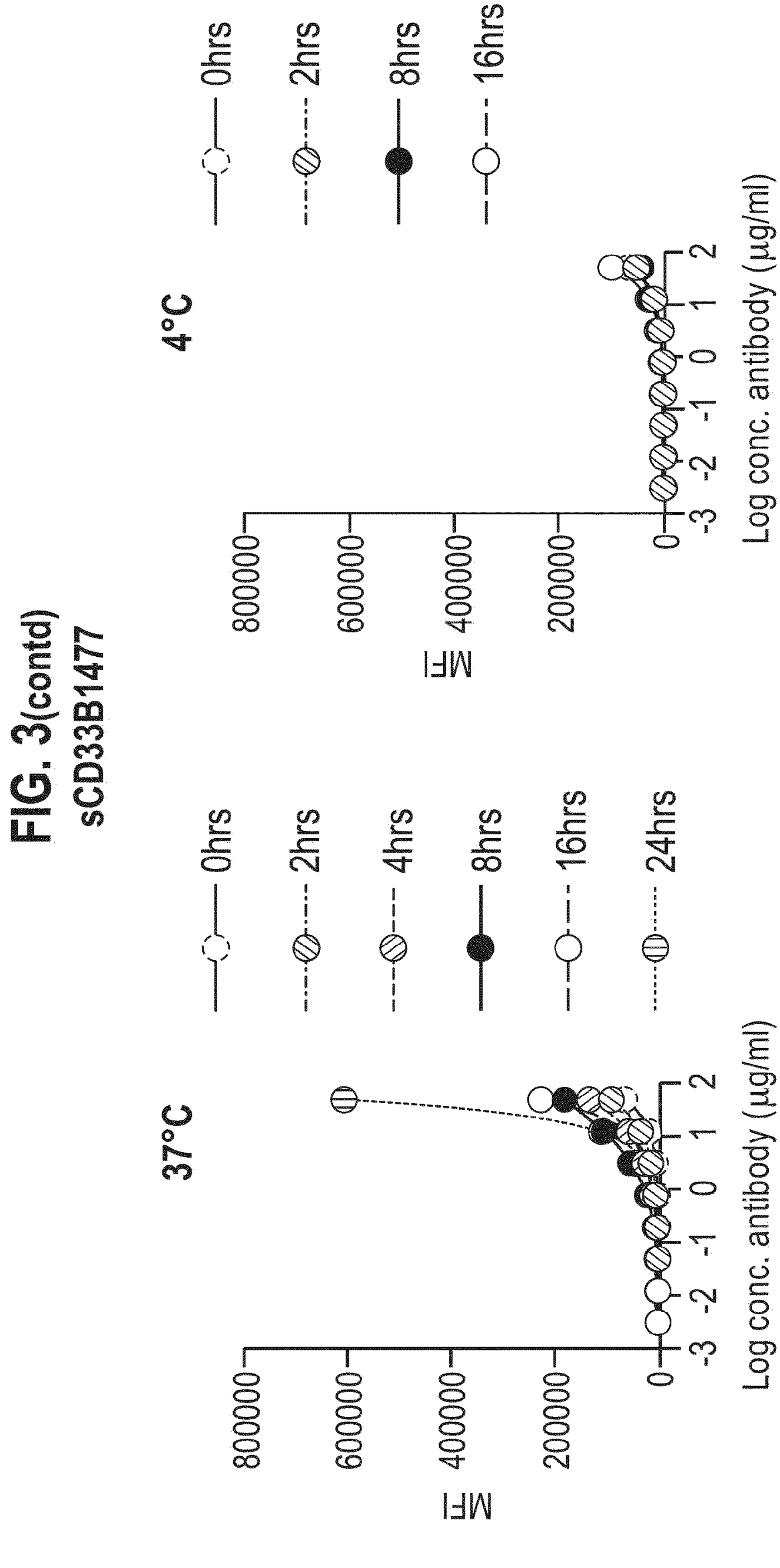

FIG. 3(contd)

sCD33B1495

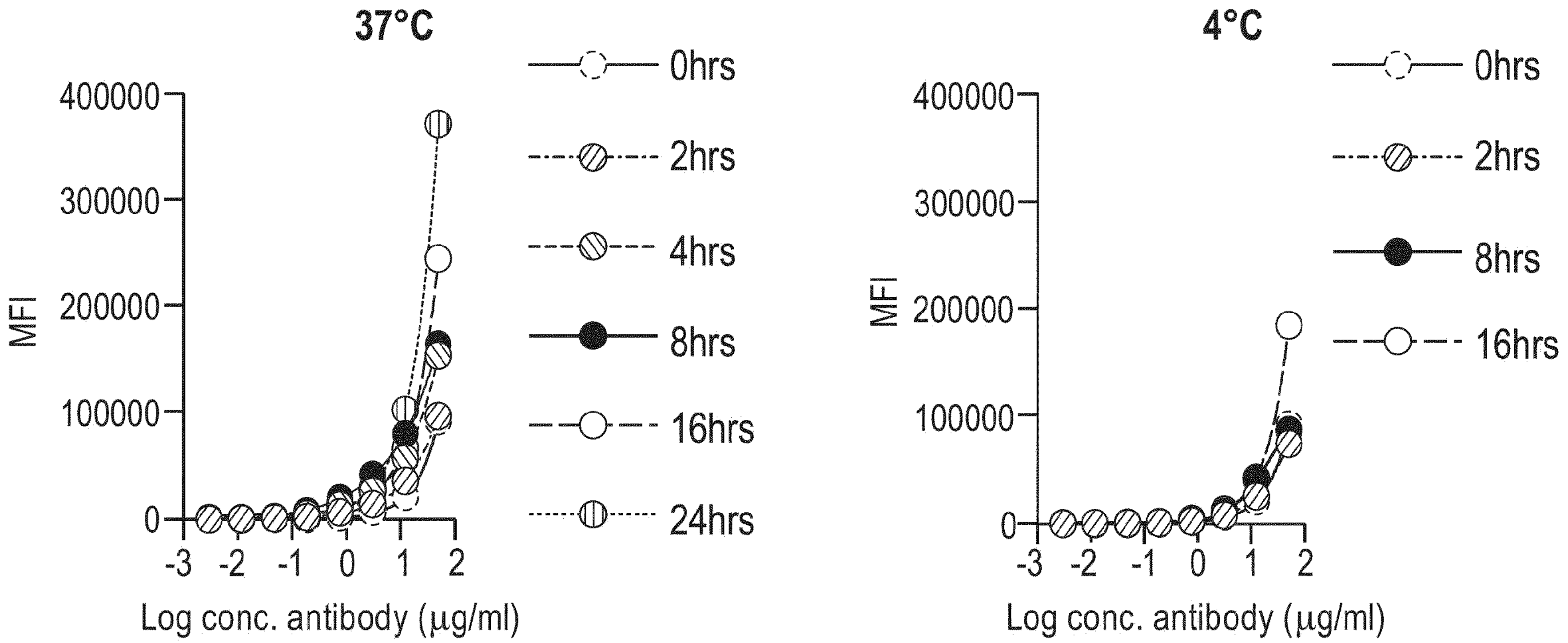

FIG. 3(contd)
Lintuzumab

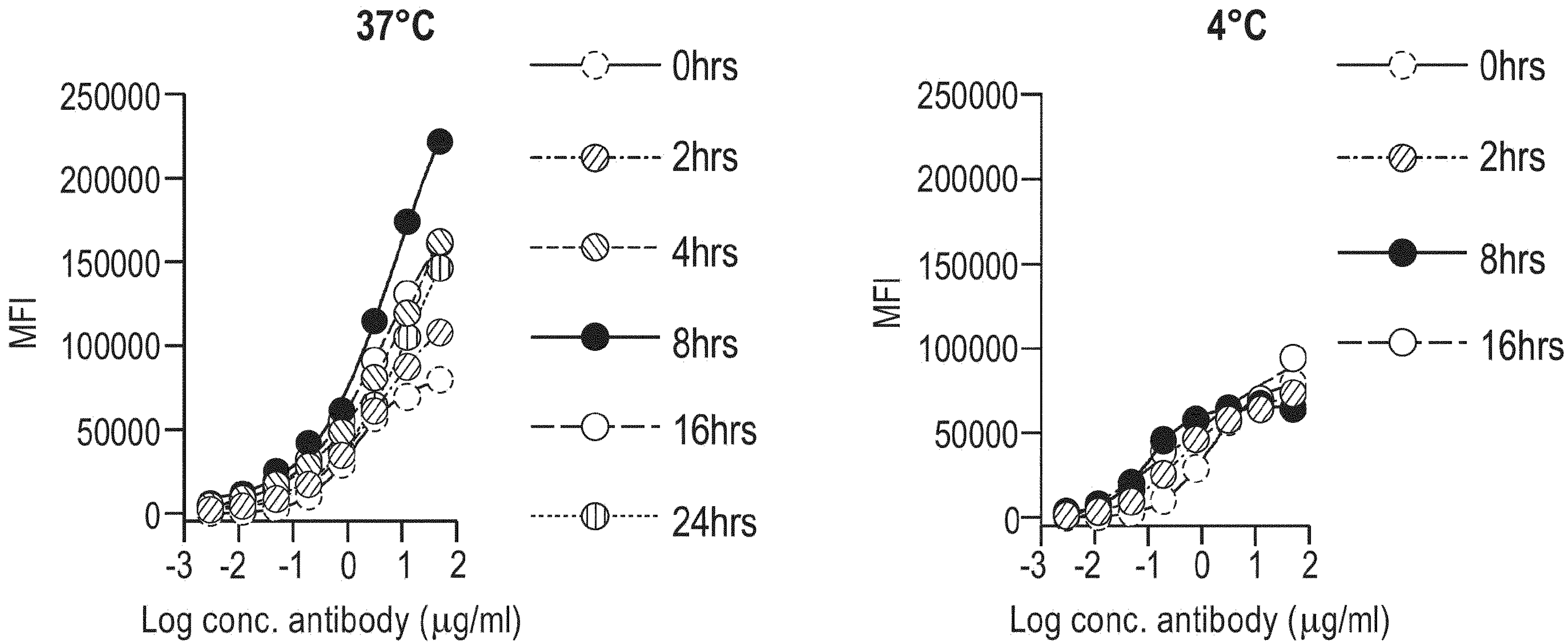

# FIG. 4A

MOLM-13

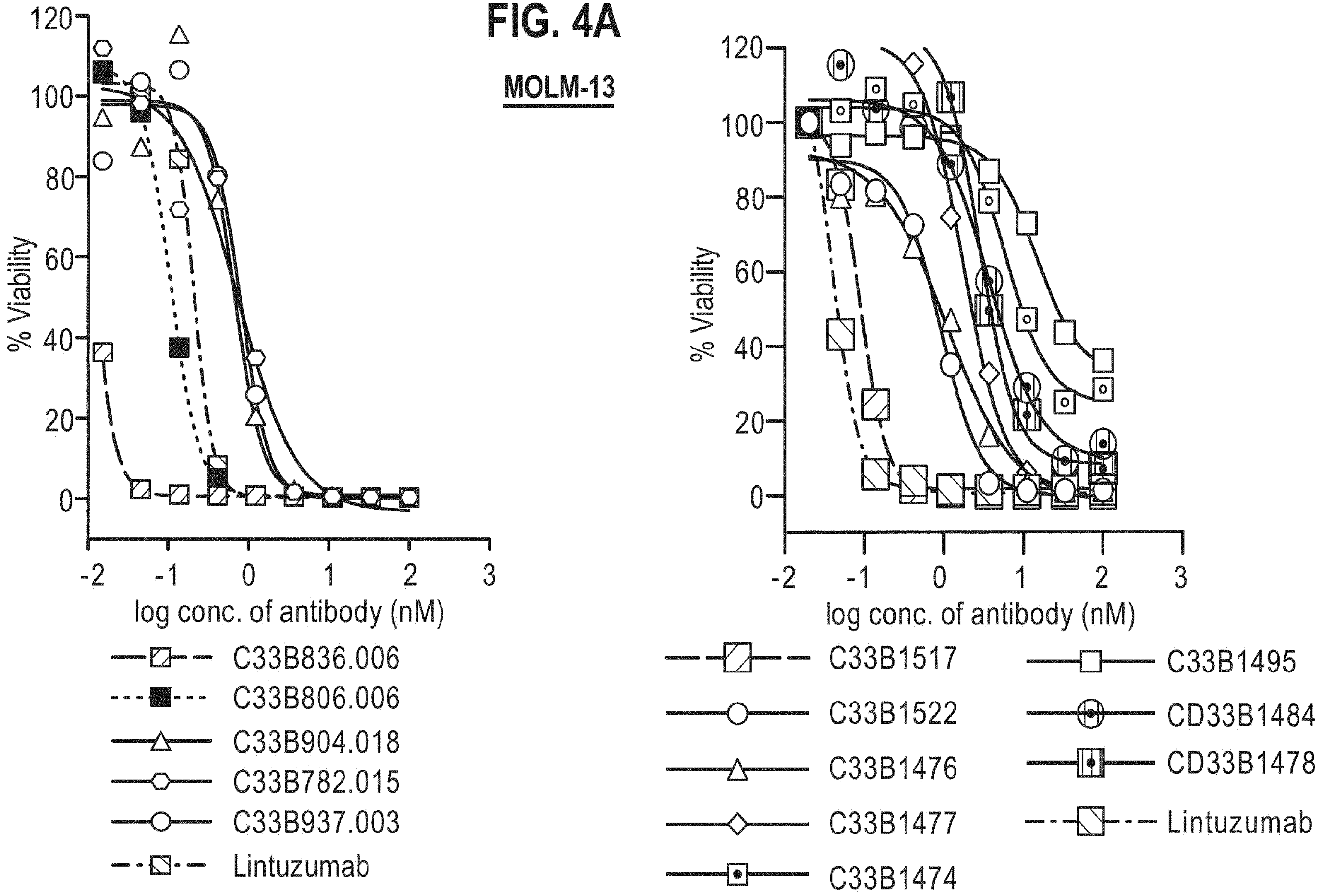

FIG. 4B Kasumi-1

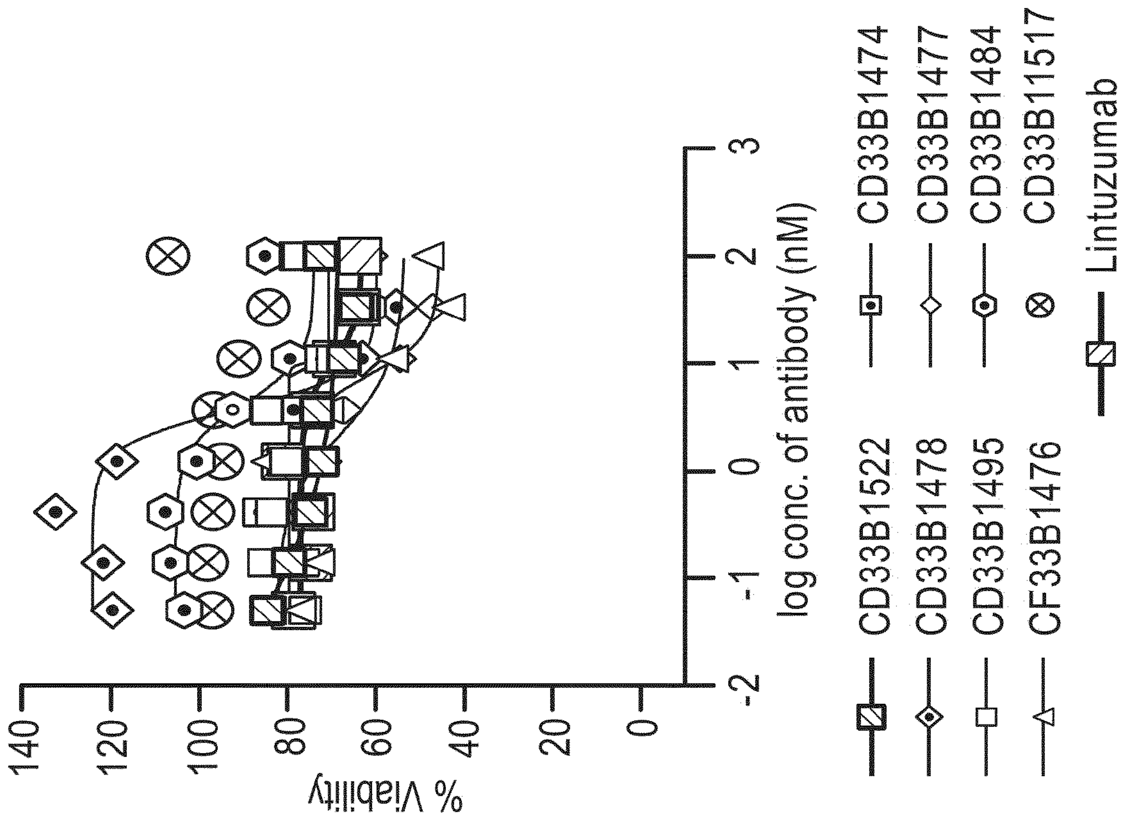


FIG. 4C OCI-AML-3

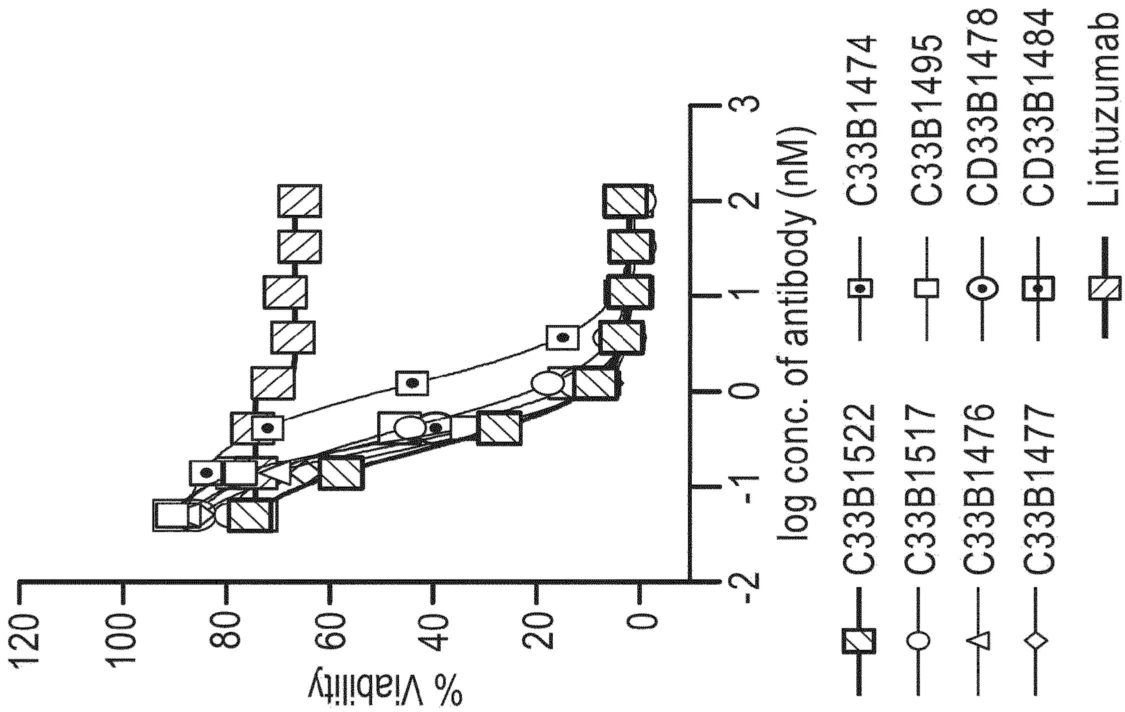

FIG. 4D

HDLM-2

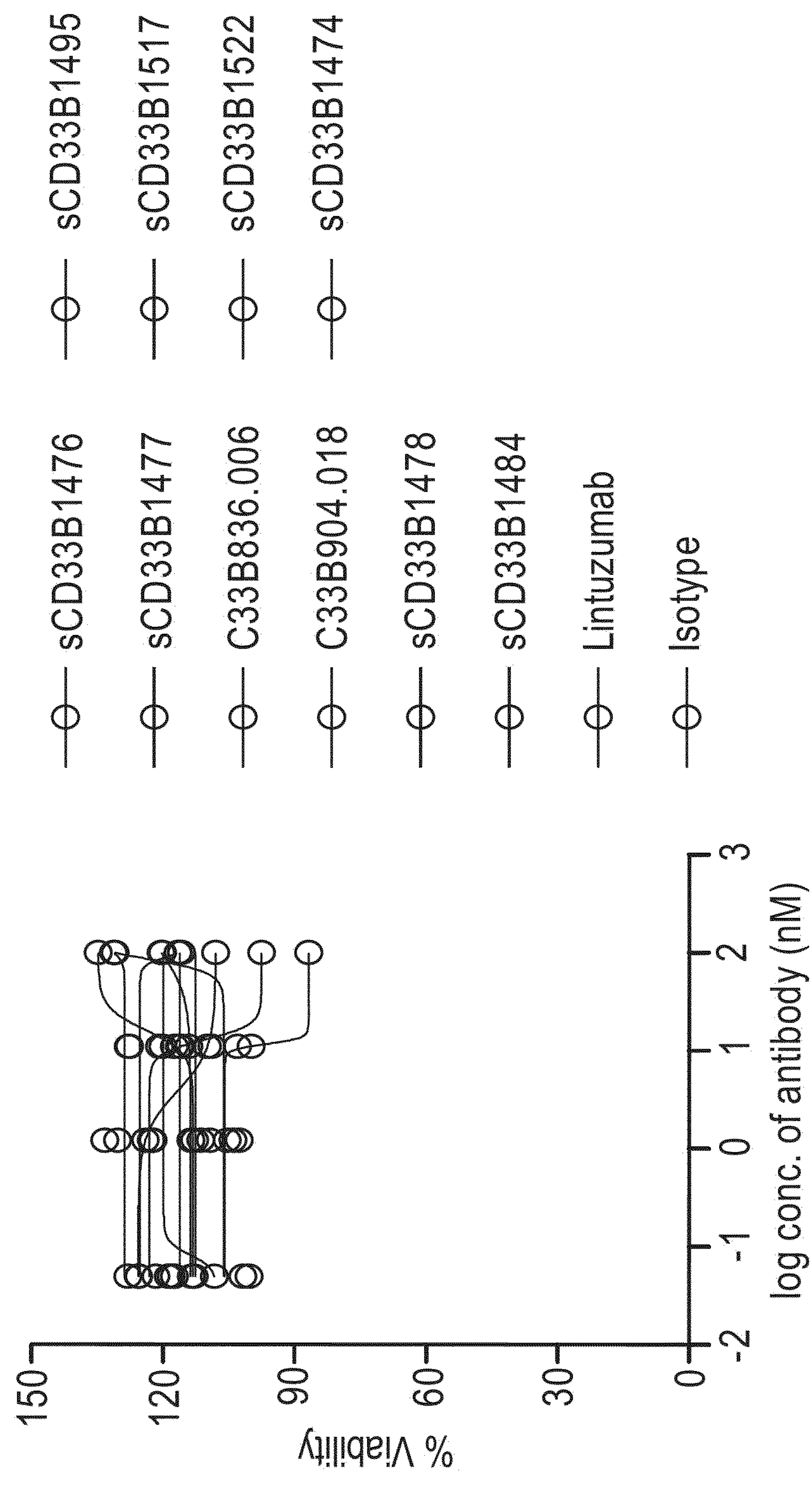
% Viability
150
120
90
60
30
0
-2
-1
0
1
2
3
log conc. of antibody (nM)
sCD33B1476
sCD33B1477
C33B836.006
C33B904.018
sCD33B1478
sCD33B1484
Lintuzumab
Isotype
sCD33B1495
sCD33B1517
sCD33B1522
sCD33B1474

# FIG. 5

**MOLM-13**

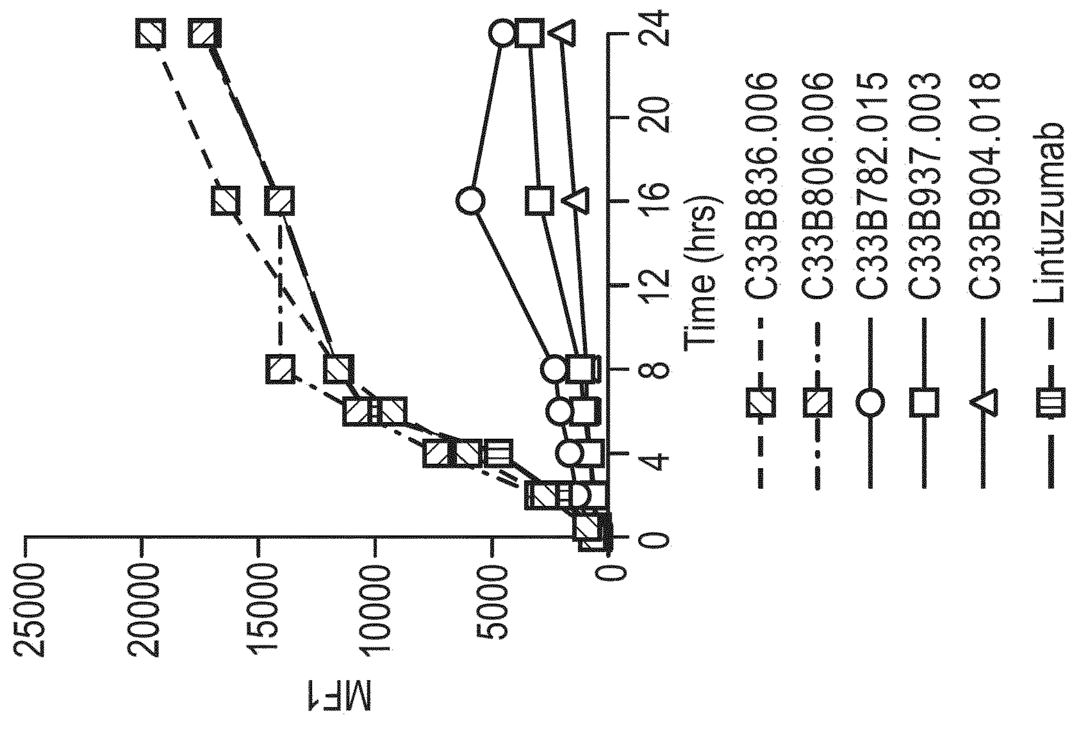
MF1
25000
20000
15000
10000
5000
0
0
4
8
12
16
20
24
Time (hrs)
C33B836.006
C33B806.006
C33B782.015
C33B937.003
C33B904.018
Lintuzumab

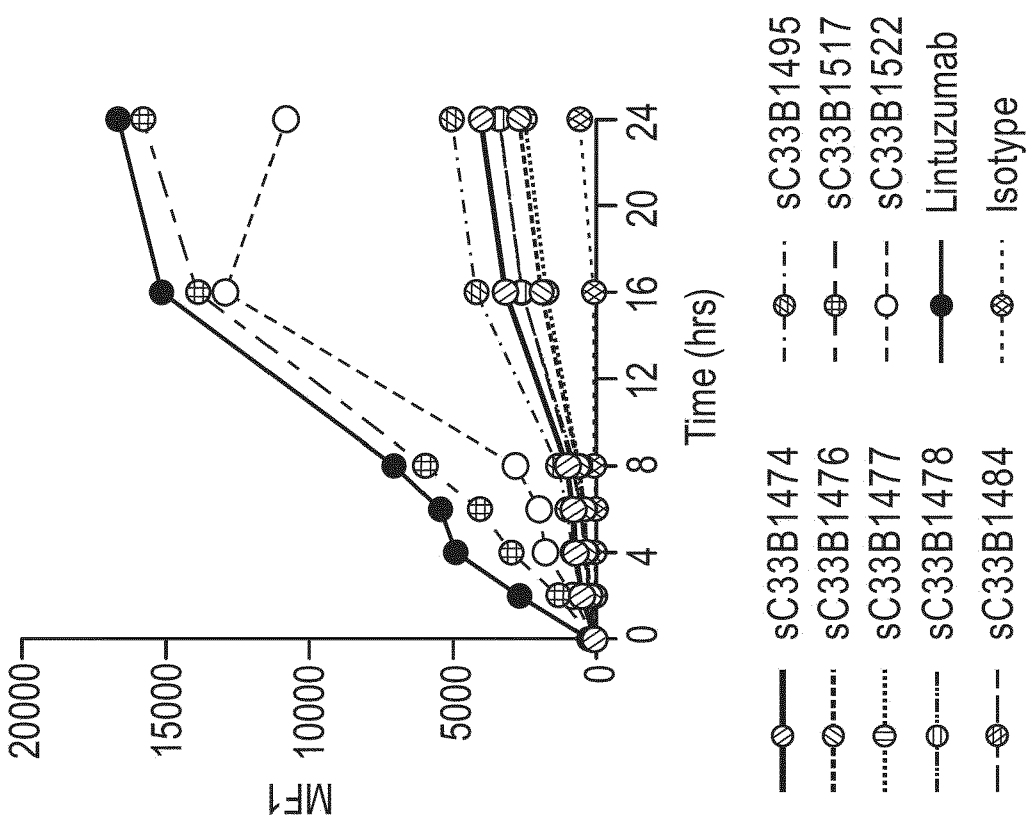
MF1
20000
15000
10000
5000
0
0
4
8
12
16
20
24
Time (hrs)
sC33B1474
sC33B1476
sC33B1477
sC33B1478
sC33B1484
sC33B1495
sC33B1517
sC33B1522
Lintuzumab
Isotype

FIG. 5(contd)

Kasumi-1

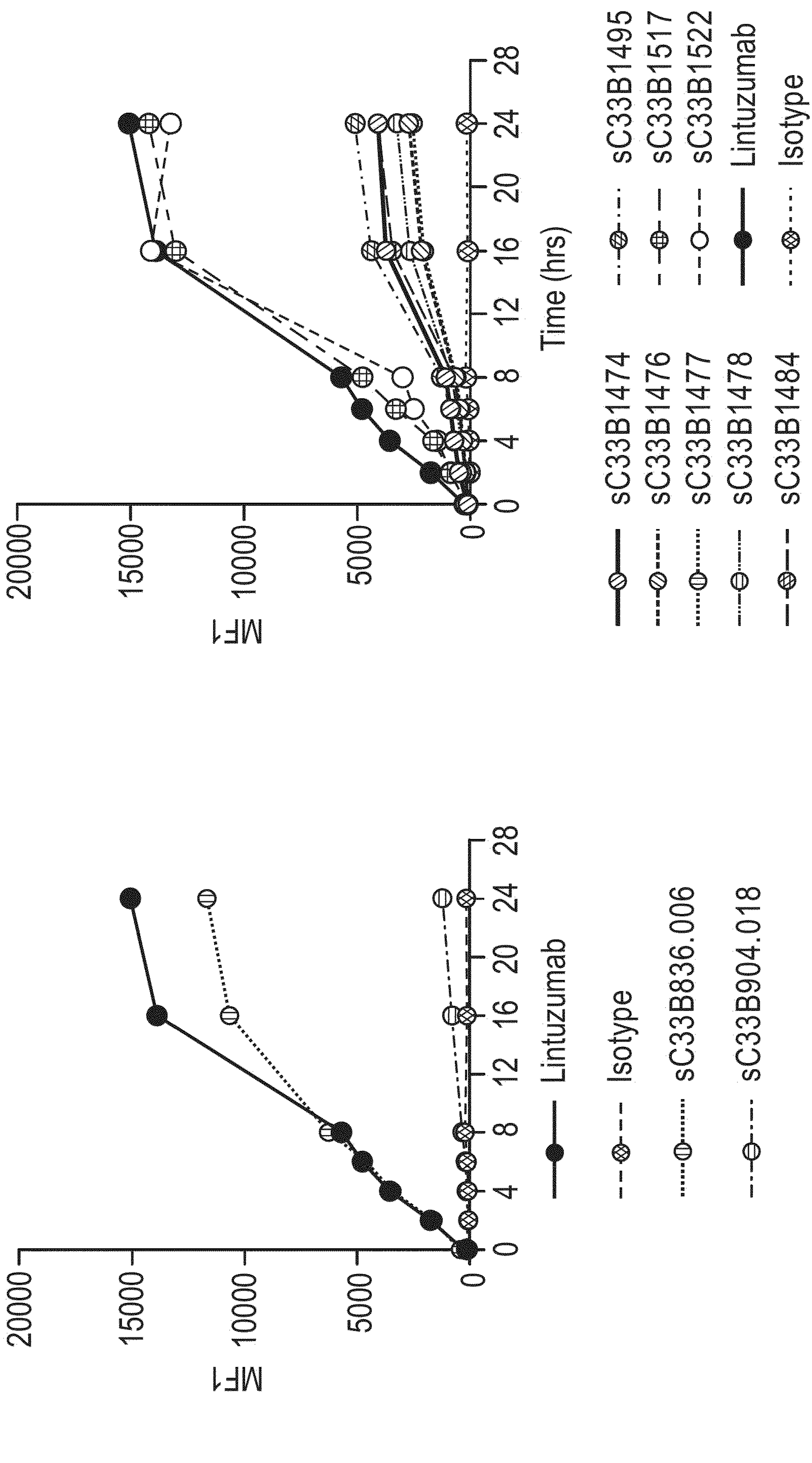

FIG. 5(contd)

OCI-AML-3

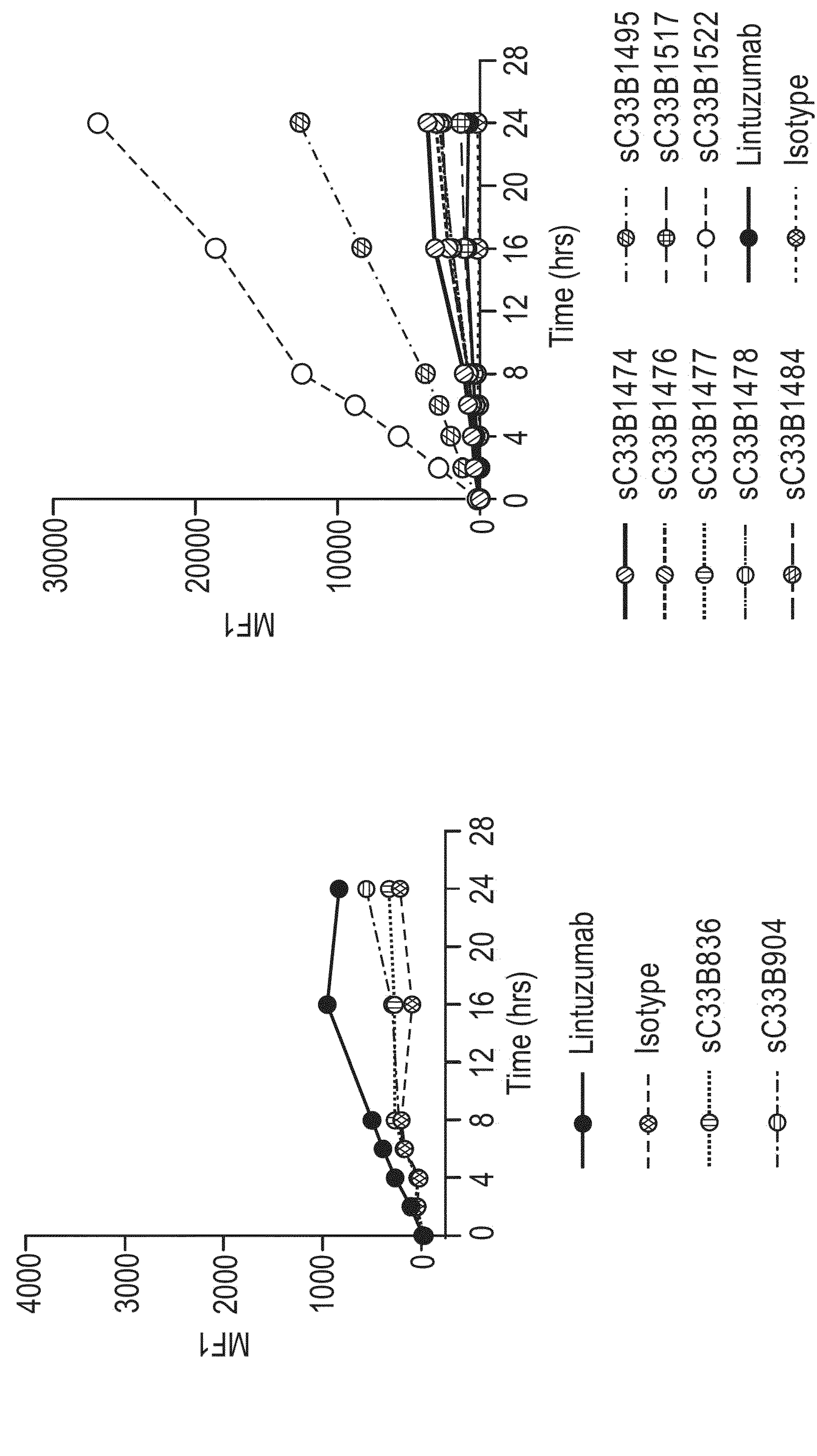

FIG. 6

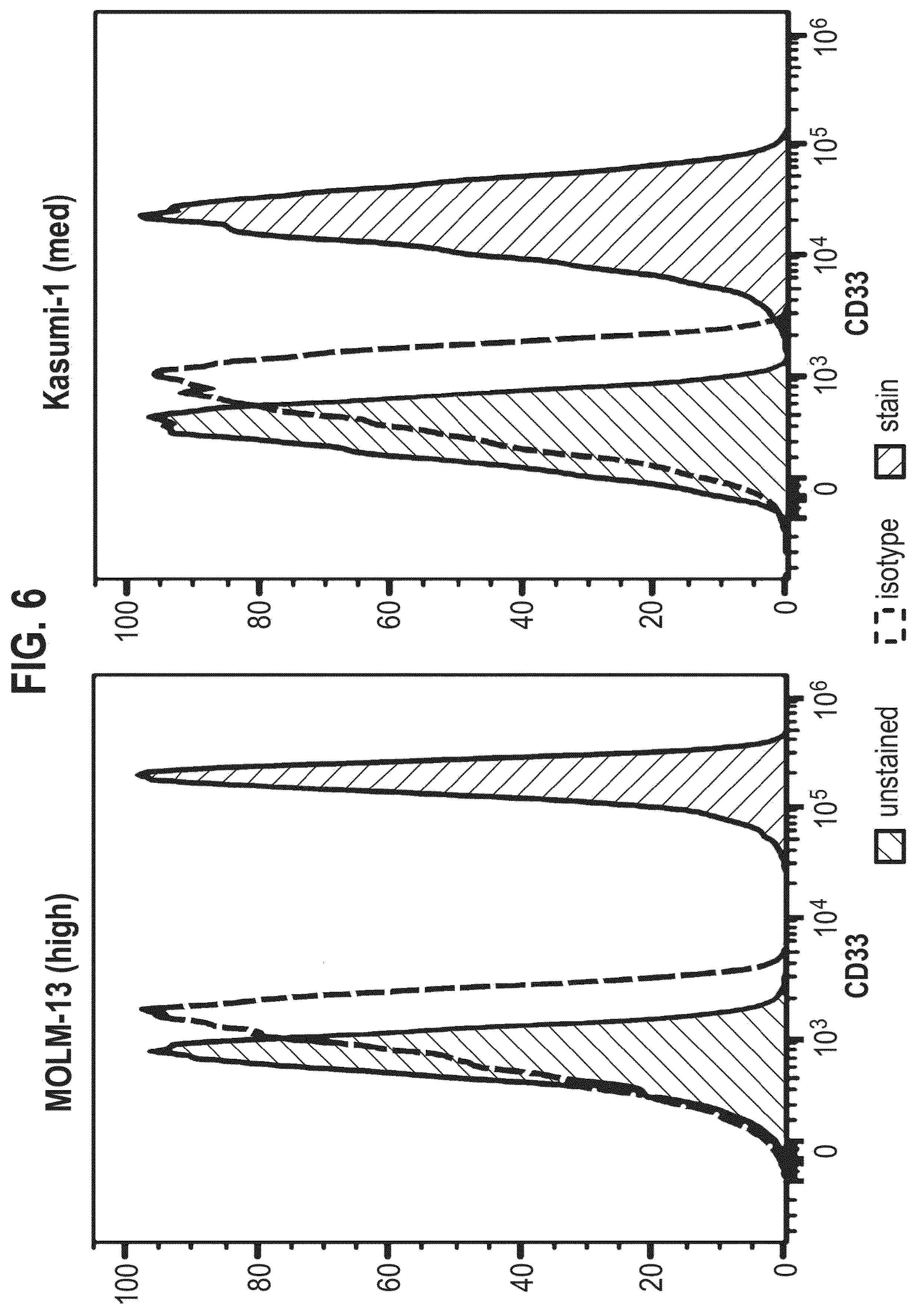
MOLM-13 (high)
Kasumi-1 (med)
100
80
60
40
20
0
0
$10^3$
$10^4$
$10^5$
$10^6$
CD33
unstained
isotype
stain

FIG. 6(contd)

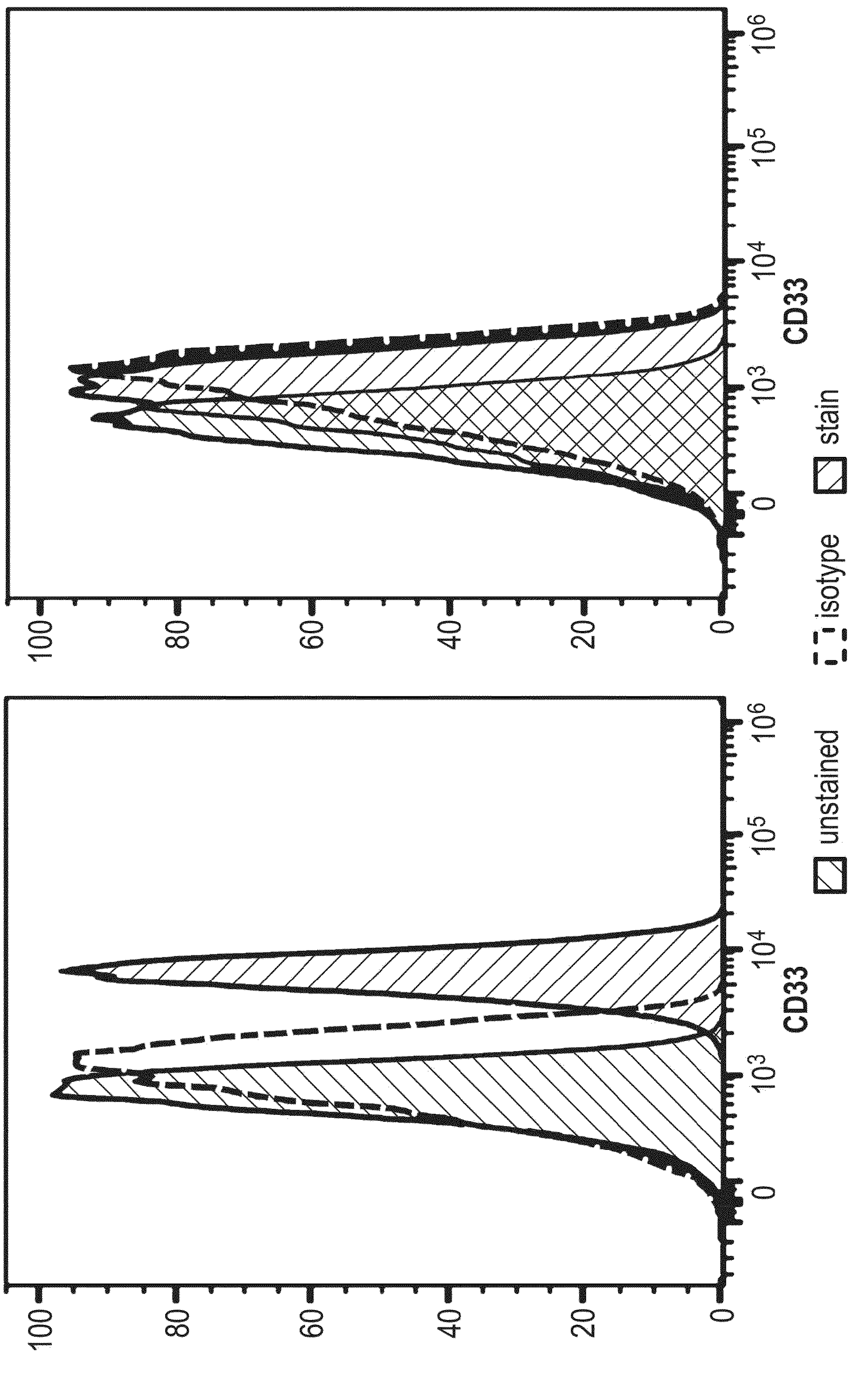
OCI-AML-3 (low)
HDLM-2 (negative)
100
80
60
40
20
0
0
$10^3$
$10^4$
$10^5$
$10^6$
CD33
unstained
isotype
stain

FIG. 6(contd)

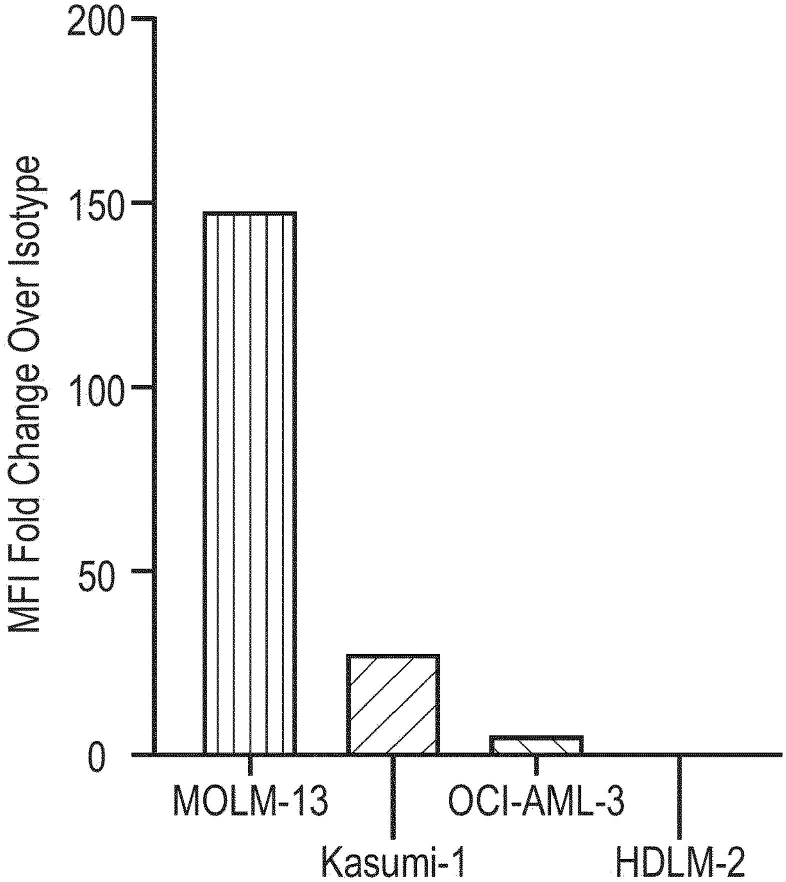
MFI Fold Change Over Isotype
200
150
100
50
0
MOLM-13
Kasumi-1
OCI-AML-3
HDLM-2

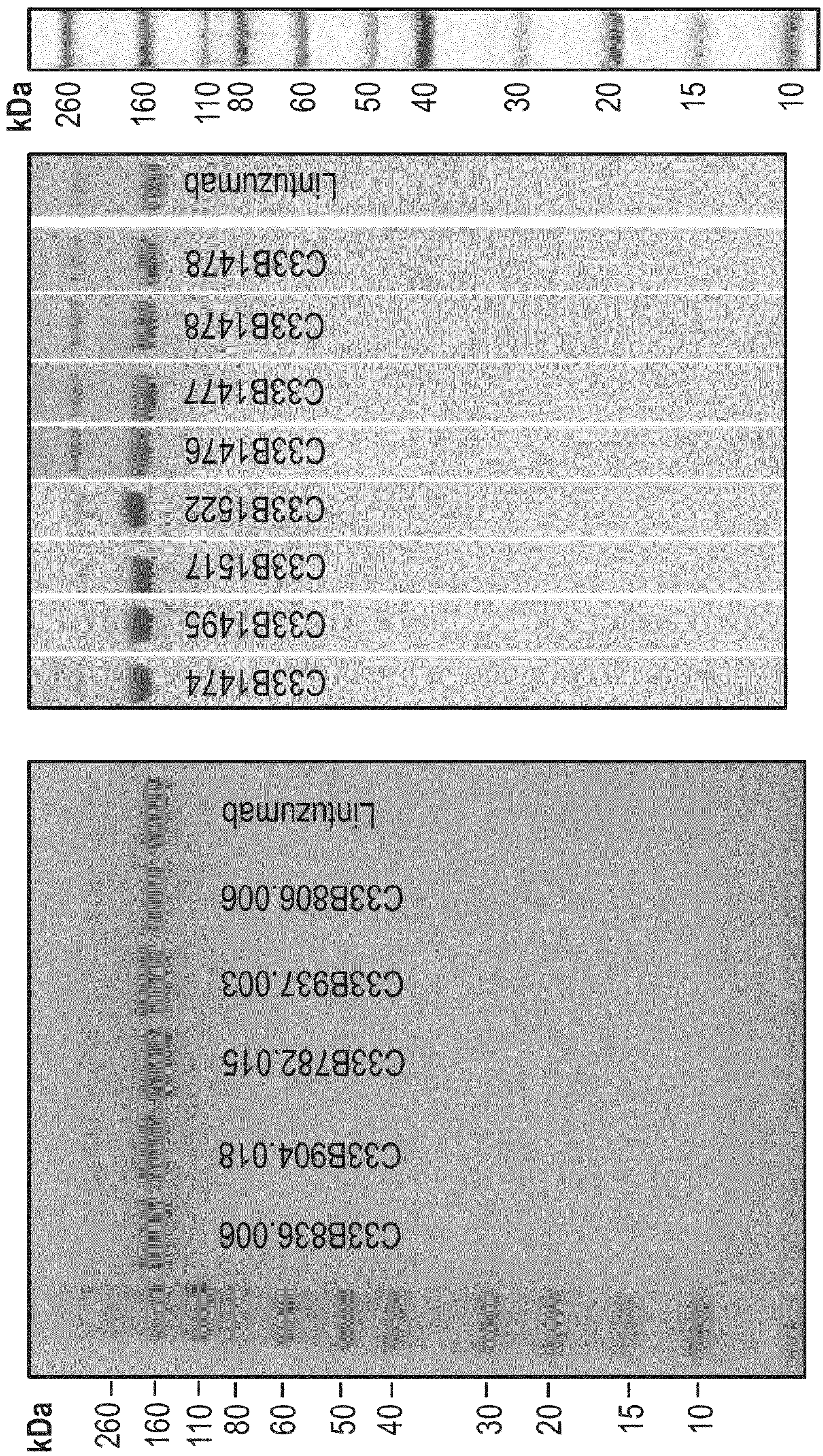
kDa
260
160
110
80
60
50
40
30
20
15
10
Lintuzumab
C33B1478
C33B1478
C33B1477
C33B1476
C33B1522
C33B1517
C33B1495
C33B1474
Lintuzumab
C33B806.006
C33B937.003
C33B782.015
C33B904.018
C33B836.006

FIG. 7

# FIG. 7(contd)

| Antibody | DAR |
|---|---|
| sC331474 | 4.5 |
| sC331476 | 2.7 |
| sC331477 | 2.6 |
| sC331478 | 3.7 |
| sC331484 | 5.2 |
| sC331495 | 4.3 |
| sC331517 | 2.1 |
| sC331522 | 2.1 |
| C33B836.006 | 3.2 |
| C33B782.015 | 2.8 |
| C33B904.018 | 3.5 |
| C33B806.006 | 2.4 |
| C33B937.003 | 3.2 |
| Lintuzumab | 3.6 |

FIG. 8A

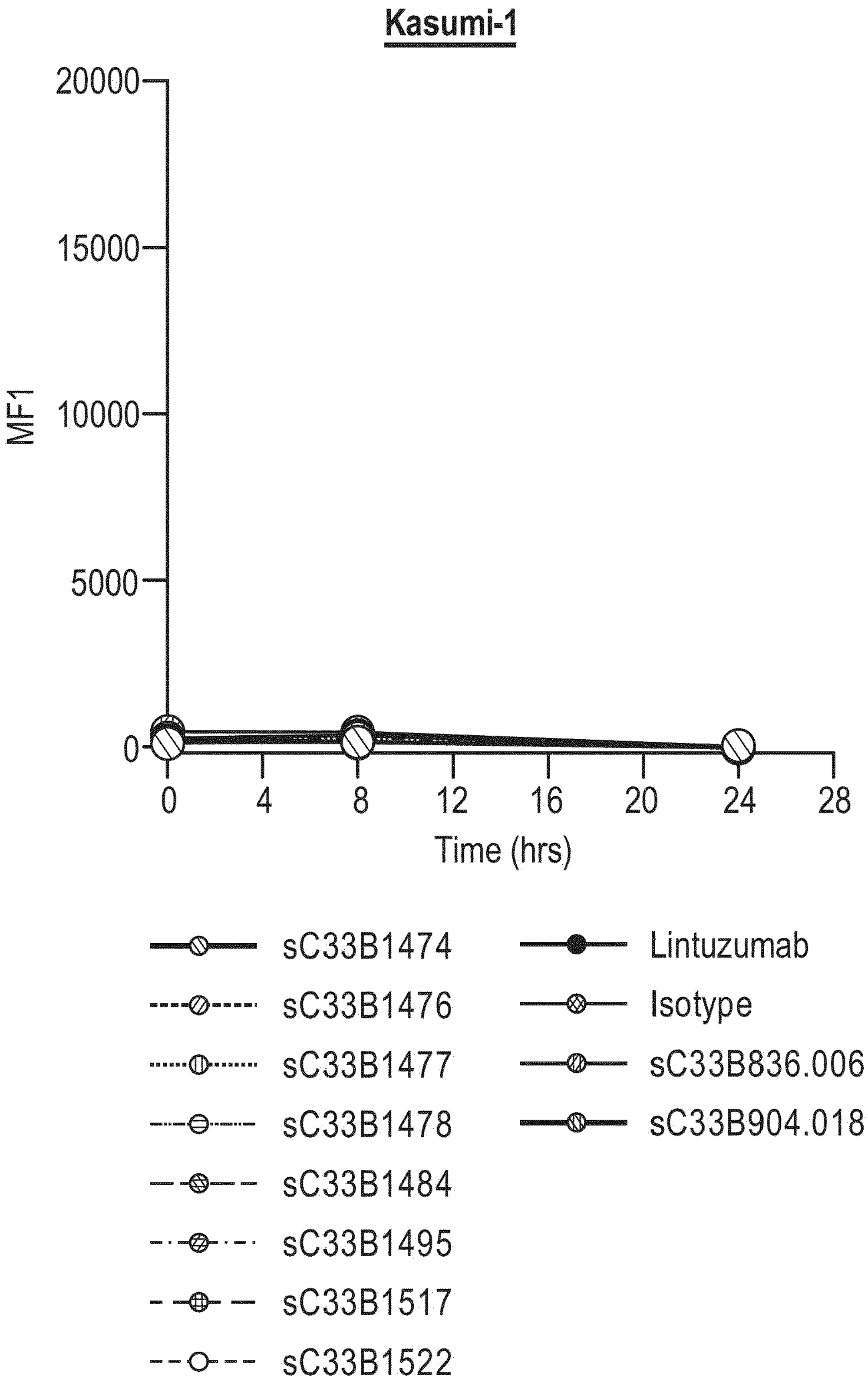
Kasumi-1
20000
15000
10000
5000
0
MF1
0
4
8
12
16
20
24
28
Time (hrs)
sC33B1474
sC33B1476
sC33B1477
sC33B1478
sC33B1484
sC33B1495
sC33B1517
sC33B1522
Lintuzumab
Isotype
sC33B836.006
sC33B904.018

# FIG. 8A(contd)

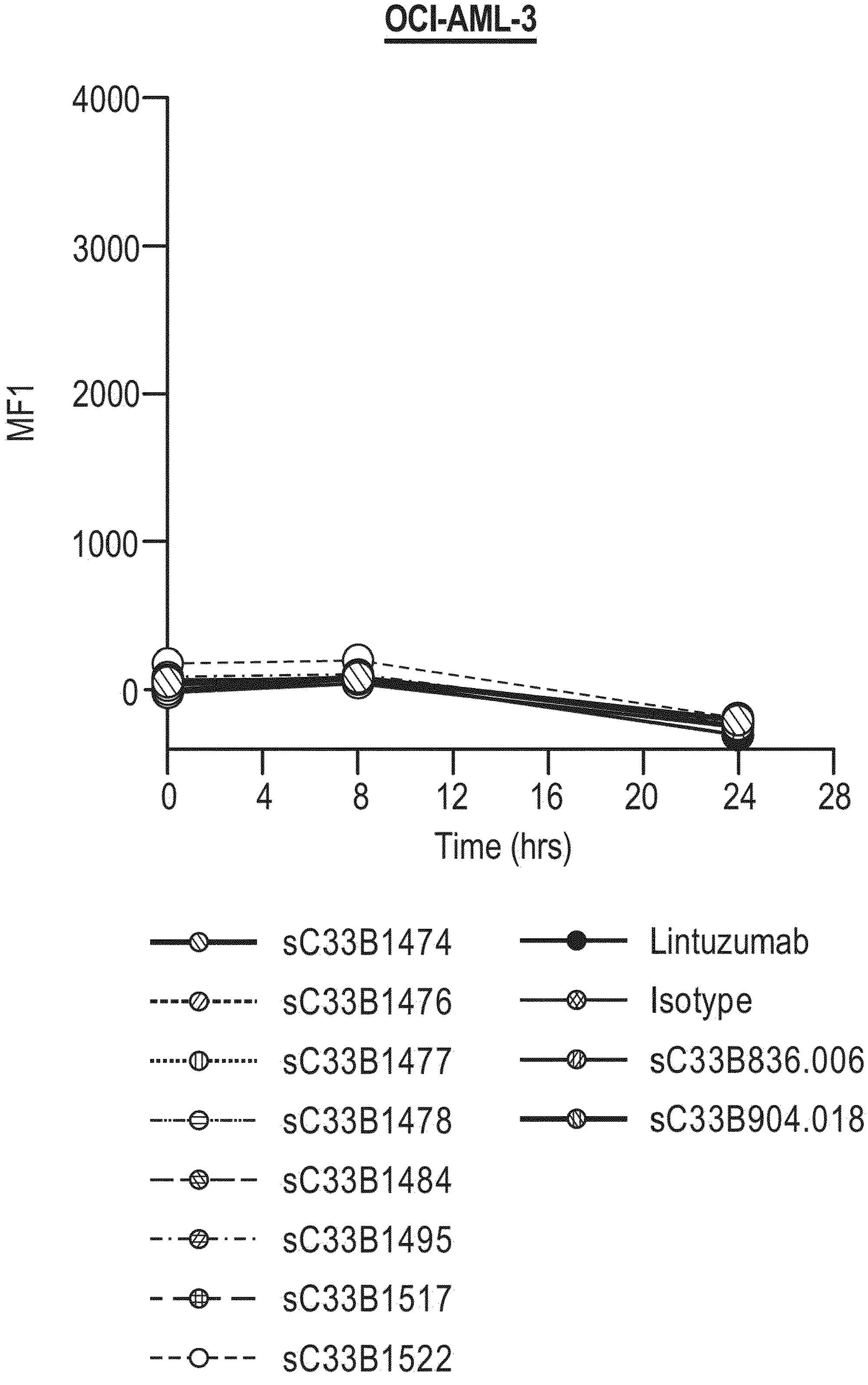
OCI-AML-3
4000
3000
2000
1000
0
MF1
0
4
8
12
16
20
24
28
Time (hrs)
sC33B1474
sC33B1476
sC33B1477
sC33B1478
sC33B1484
sC33B1495
sC33B1517
sC33B1522
Lintuzumab
Isotype
sC33B836.006
sC33B904.018

# FIG. 8B

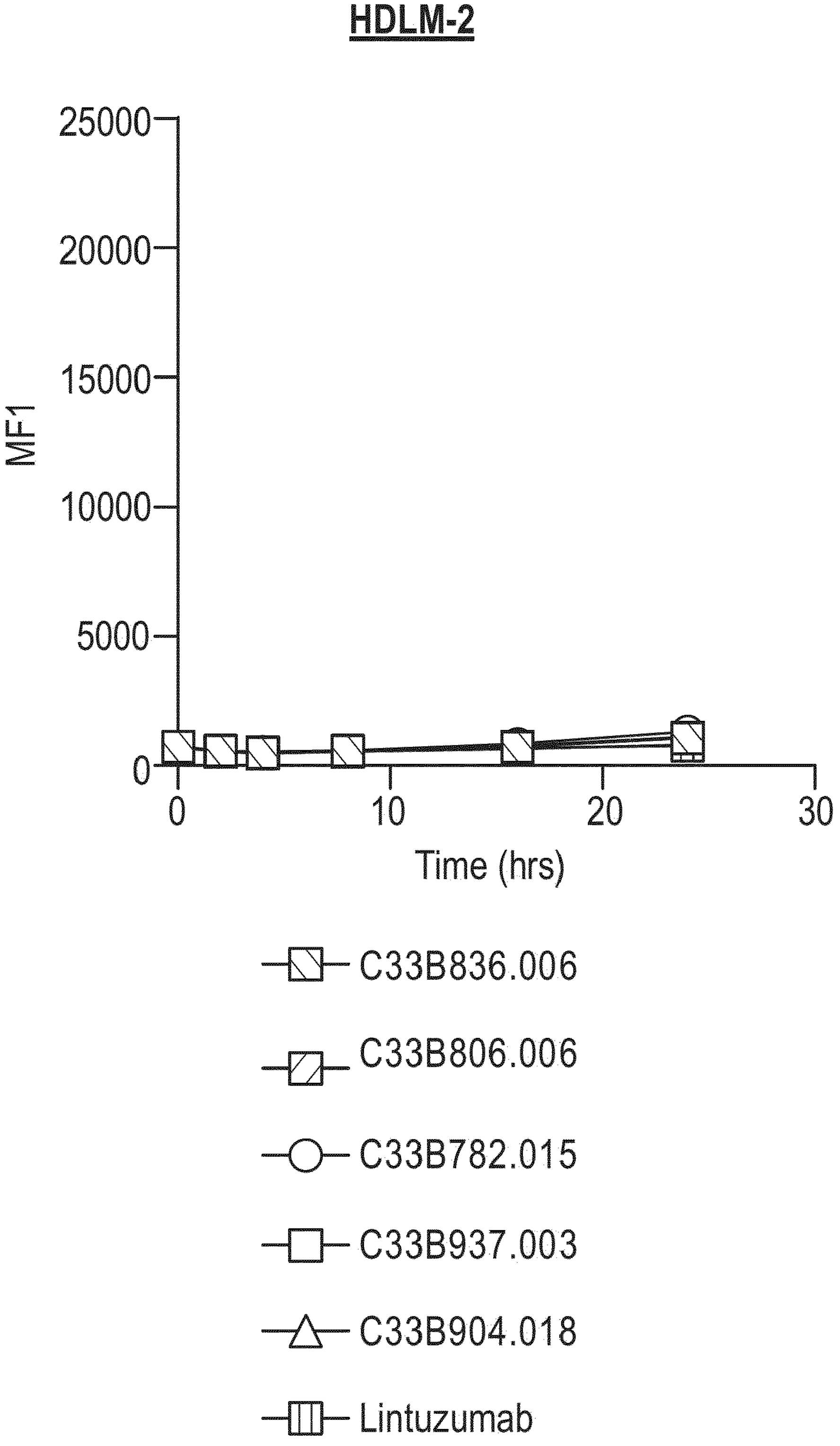
HDLM-2
MF1
25000
20000
15000
10000
5000
0
0
10
20
30
Time (hrs)
C33B836.006
C33B806.006
C33B782.015
C33B937.003
C33B904.018
Lintuzumab

# FIG. 8B(contd)

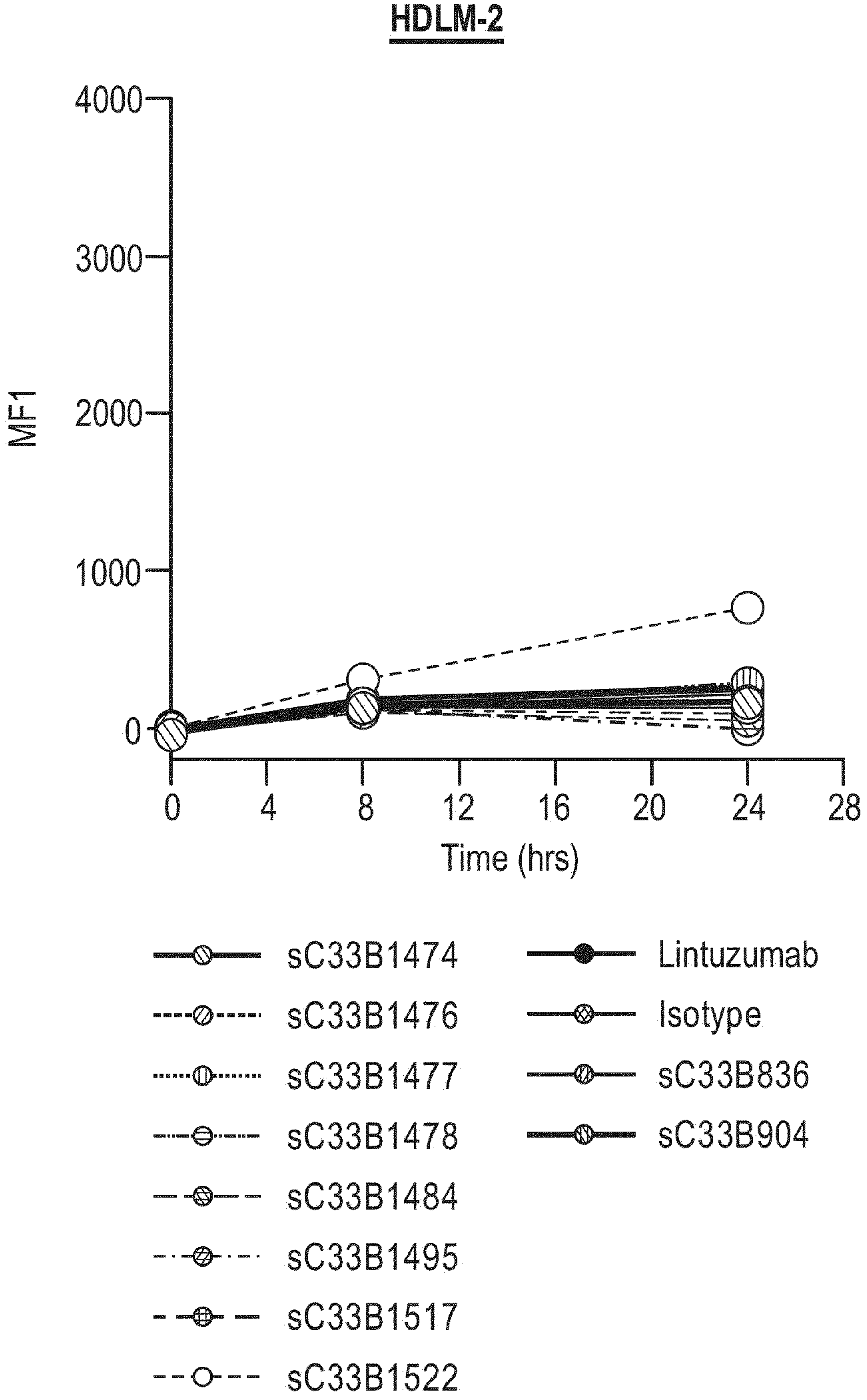
HDLM-2
4000
3000
2000
1000
0
MF1
0
4
8
12
16
20
24
28
Time (hrs)
sC33B1474
sC33B1476
sC33B1477
sC33B1478
sC33B1484
sC33B1495
sC33B1517
sC33B1522
Lintuzumab
Isotype
sC33B836
sC33B904

FIG. 9

| | MOLM-13 (high) | Kasumi-1 (med) | OCI-AML-3 (low) |
|---|---|---|---|
| Antibody | $EC_{50}$ (nM) | $EC_{50}$ (nM) | $EC_{50}$ (nM) |
| C33B836.006 | 0.5 | 17.06 | 0.46 |
| C33B806.006 | 35.6 | 0.13 | 15.3 |
| C33B904.018 | ND | ND | ND |
| C33B937.003 | ND | ND | ND |
| C33B782.015 | ND | ND | ND |
| C33B1517 | 10.6 | ND | 1.4 |
| C33B1522 | 32.66 | 73.4 | ND |
| C33B1477 | 56 | ND | 0.6 |
| C33B1474 | 78.4 | ND | 1.26 |
| C33B1478 | ND | ND | ND |
| C33B1476 | ND | ND | 0.8 |
| C33B1495 | ND | ND | ND |
| C33B1484 | ND | ND | ND |
| Lintuzumab | 0.31 | 0.34 | 0.9 |

FIG. 10

| | MOLM-13 (high) | Kasumi-1 (med) | OCI-AML-3 (low) |
|---|---|---|---|
| Antibody | $EC_{50}$ (nM) | % viability | $EC_{50}$ (nM) |
| ***C33B836.006*** | ***~0.002*** | Not tested | Not tested |
| C33B806.006 | 0.106 | Not tested | Not tested |
| C33B904.018 | 0.744 | Not tested | Not tested |
| C33B937.003 | 0.840 | Not tested | Not tested |
| C33B782.015 | 1.062 | Not tested | Not tested |
| ***C33B1517*** | ***0.09*** | 106 | 0.45 |
| C33B1522 | 0.9 | 73.4 | 0.24 |
| C33B1476 | 1.09 | 48.1 | 0.27 |
| C33B1477 | 1.76 | 60.9 | 0.24 |
| C33B1478 | 2.5 | 64.6 | 0.34 |
| C33B1484 | 3.5 | 85.01 | 0.39 |
| C33B1474 | 5.93 | 78.1 | 1.18 |
| C33B1495 | 14.44 | 77.4 | 0.30 |
| Lintuzumab | 0.04 | 63.5 | 0.9 |

FIG. 11

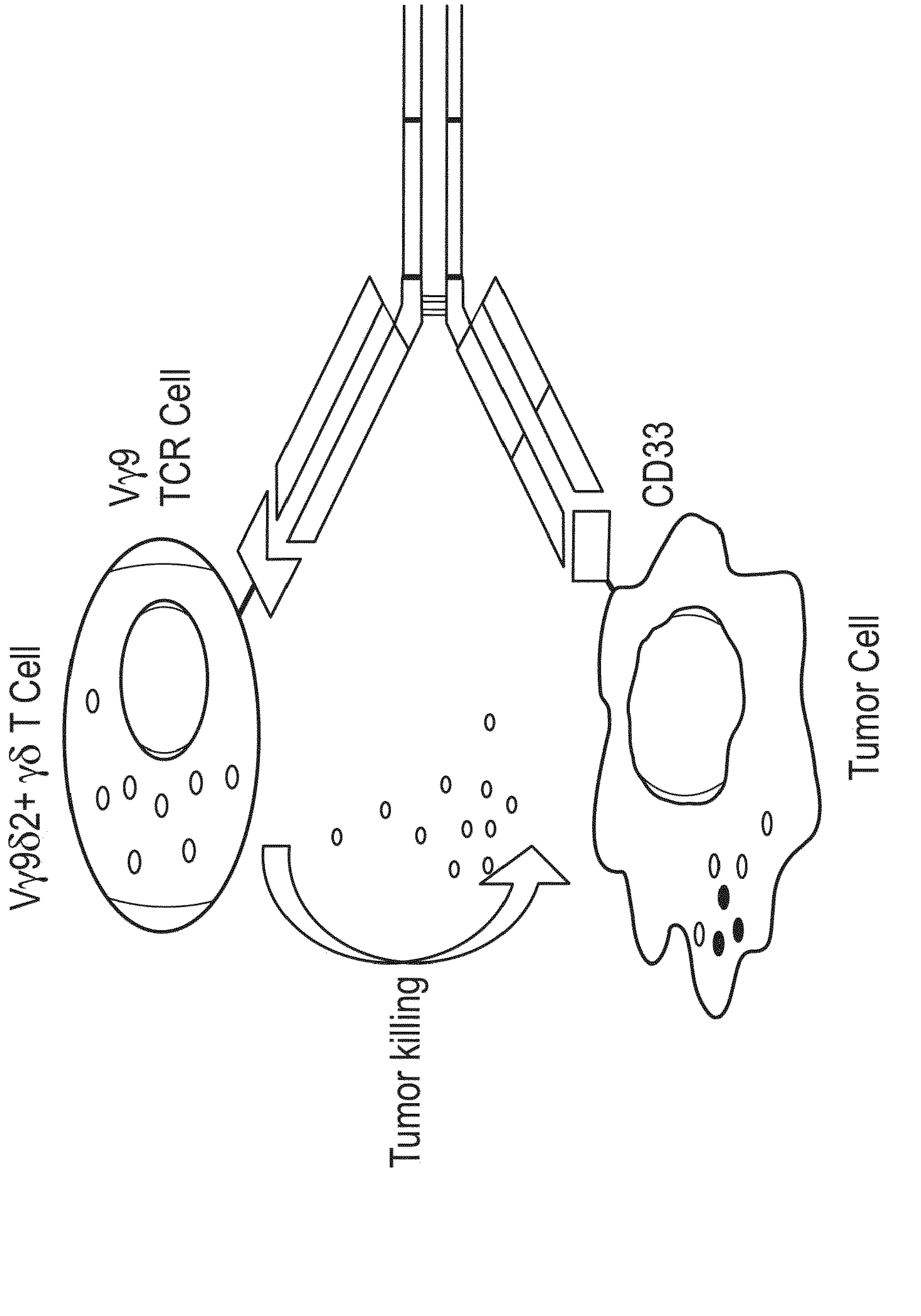
Vγ9δ2+ γδ T Cell
Vγ9
TCR Cell
CD33
Tumor killing
Tumor Cell

FIG. 12

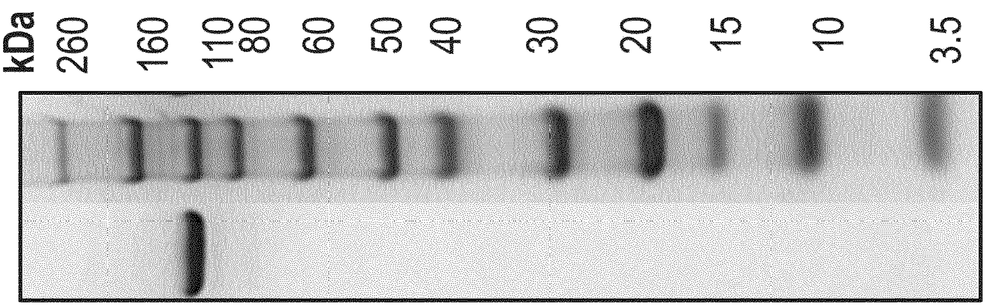
kDa
260
160
110
80
60
50
40
30
20
15
10
3.5

FIG. 13

MOLM-13
(high)

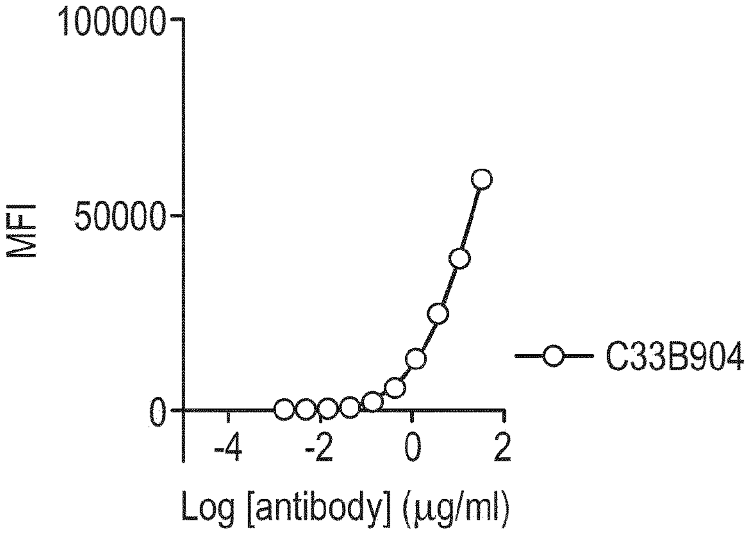


| Antibody | $EC_{50}$ (nM) | $EC_{90}$ (nM) |
|---|---|---|
| C33B904 | ~134.33 | 3748 |

FIG. 14

Kasumi-1
(med)

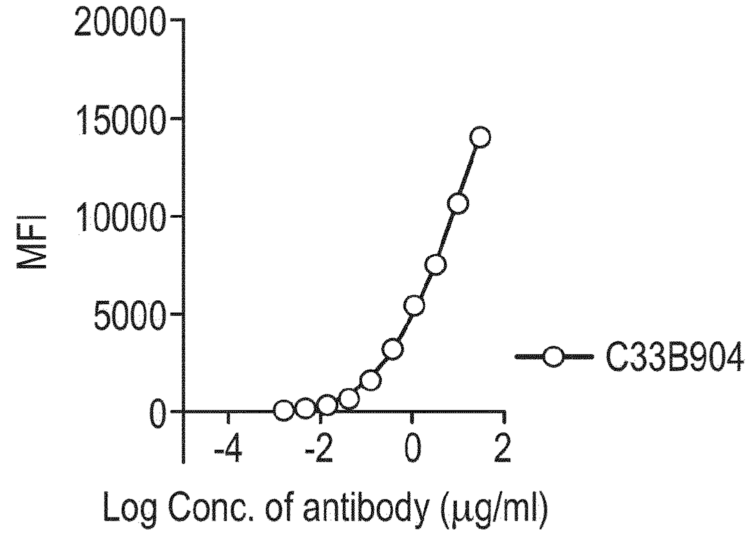


| Antibody | $EC_{50}$ (nM) | $EC_{90}$ (nM) |
|---|---|---|
| C33B904 | 82.2 | 6660 |

# FIG. 15

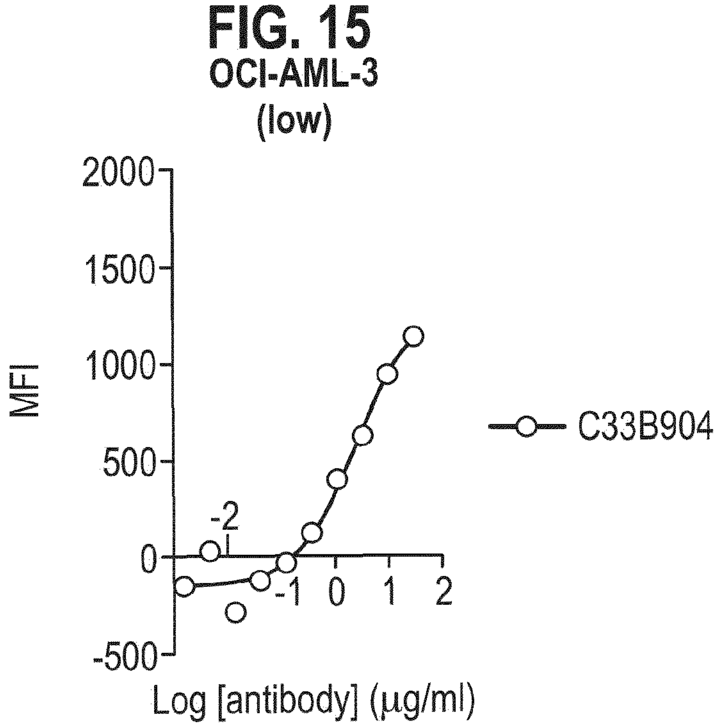


| Antibody | $EC_{50}$ (nM) | $EC_{90}$ (nM) |
|---|---|---|
| C33B904 | 16.4 | 274 |

# FIG. 16

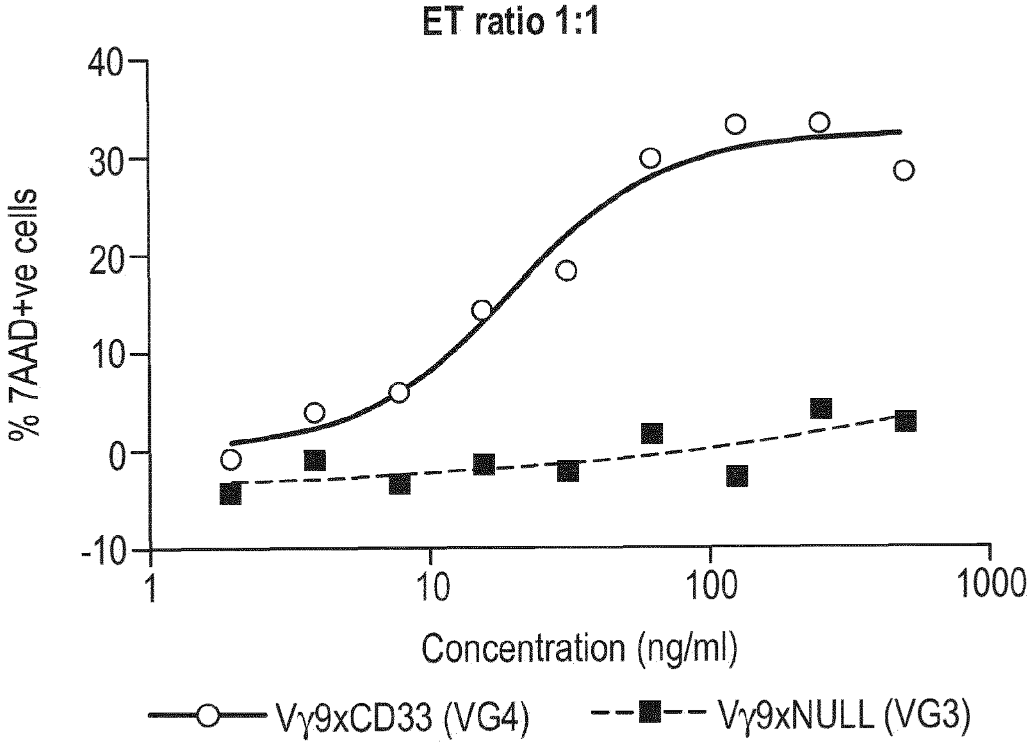

# FIG. 17

ET ratio 5:1

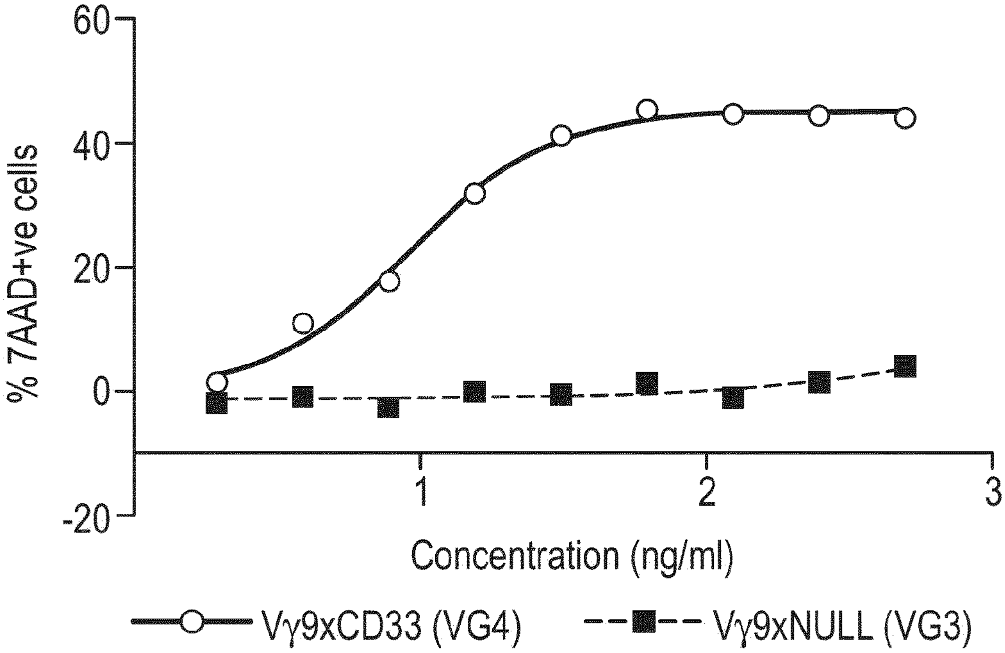


# FIG. 18

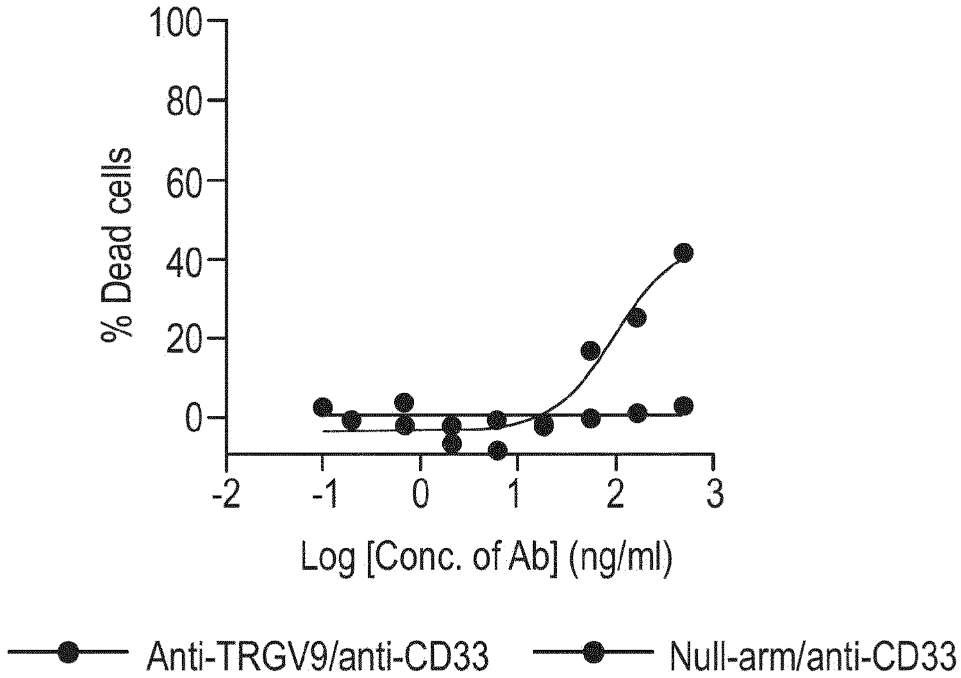

# FIG. 19

| | Significant HC Liabilities | | | | | | | Significant LC Liabilities | | | | | | |
|---|---|---|---|---|---|---|---|---|---|---|---|---|---|---|
| | HCFW1 | HCDR1 | HCFW2 | HCDR2 | HCFW3 | HCDR3 | HCFW4 | LCFW1 | LCDR1 | LCFW2 | LCDR2 | LCFW3 | LCDR3 | LCFW4 |
| JL2 | FR1 | CDR1 | FR2 | DG | NS DT DT | CDR3 | FR4 | FR1 | DG | FR2 | CDR1 | FR3 | CDR3 | FR4 |
| JL3 | FR1 | CDR1 | FR2 | CDR2 | NN DT DT | NG | FR4 | FR1 | CDR1 | FR2 | CDR1 | FR3 | NG DS | FR4 |
| JL5 | FR1 | CDR1 | FR2 | CDR2 | NN DT | NG | FR4 | FR1 | CDR1 | FR2 | NN | FR3 | NG DS | FR4 |
| JL6 | FR1 | CDR1 | FR2 | CDR2 | DT | DP | FR4 | FR1 | CDR1 | FR2 | NN | FR3 | DP DS | FR4 |

# FIG. 20

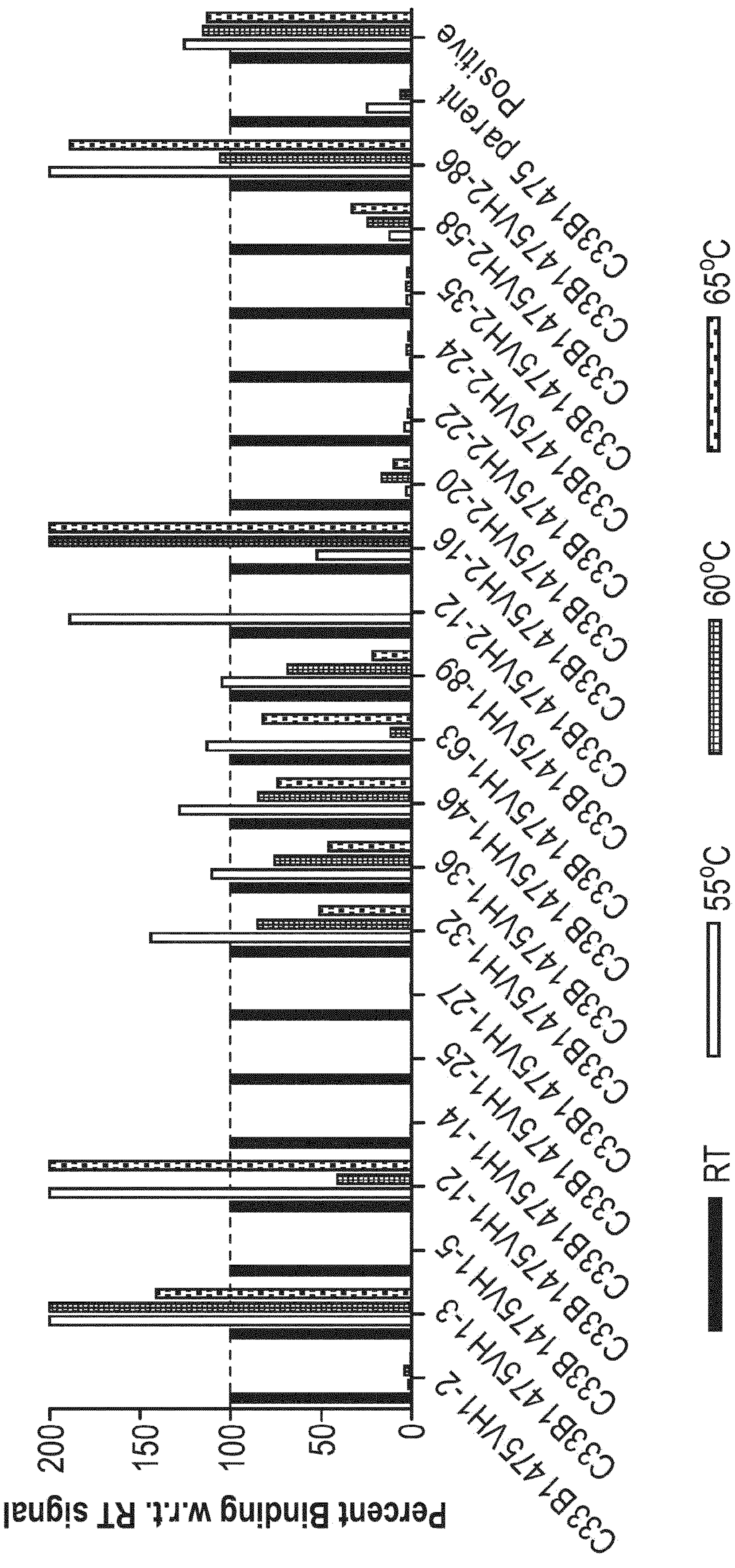
Percent Binding w.r.t. RT signal
200
150
100
50
0
C33B1475VH1-2
C33B1475VH1-3
C33B1475VH1-5
C33B1475VH1-12
C33B1475VH1-14
C33B1475VH1-25
C33B1475VH1-27
C33B1475VH1-32
C33B1475VH1-36
C33B1475VH1-46
C33B1475VH1-63
C33B1475VH1-89
C33B1475VH2-12
C33B1475VH2-16
C33B1475VH2-20
C33B1475VH2-22
C33B1475VH2-24
C33B1475VH2-35
C33B1475VH2-58
C33B1475VH2-86
C33B1475 parent
Positive
RT
55°C
60°C
65°C

# FIG. 21

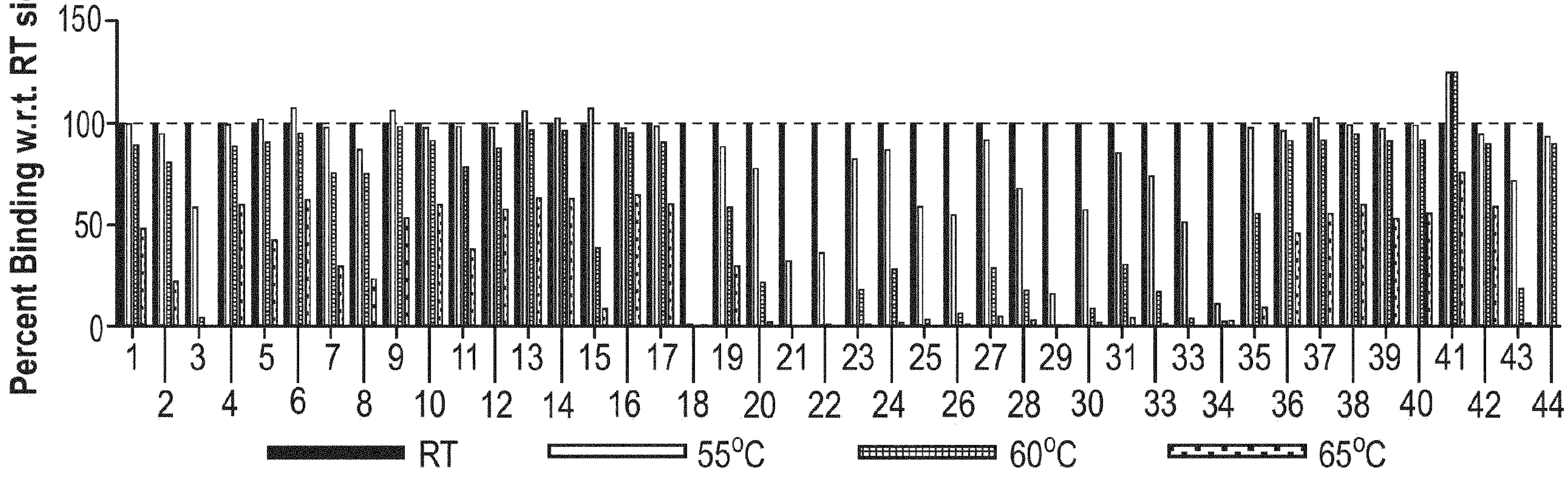


| | | | | | | | | | |
|---|---|---|---|---|---|---|---|---|---|
| **1** | C33B1516VL1-1 | **2** | 3B1516VL1-10 | **3** | C33B1516VL1-11 | **4** | C33B1516VL1-14 | **5** | C33B1516VL1-16 |
| **6** | C33B1516VL1-2 | **7** | C33B1516VL1-20 | **8** | C33B1516VL1-22 | **9** | C33B1516VL1-23 | **10** | C33B1516VL1-30 |
| **11** | C33B1516VL1-4 | **12** | C33B1516VL2-10 | **13** | C33B1516VL2-11 | **14** | C33B1516VL2-29 | **15** | C33B1516VL2-3 |
| **16** | C33B1516VL2-35 | **17** | C33B1516VL2-4 | **18** | C33B1516VL2-7 | **19** | C33B1516VL2-8 | **20** | C33B1516VH1-1 |
| **21** | C33B1516VH1-10 | **22** | C33B1516VH1-13 | **23** | C33B1516VH1-17 | **24** | C33B1516VH1-20 | **25** | C33B1516VH1-21 |
| **26** | C33B1516VH1-27 | **27** | C33B1516VH1-3 | **28** | C33B1516VH1-38 | **29** | C33B1516VH1-4 | **30** | C33B1516VH1-56 |
| **31** | C33B1516VH1-6 | **32** | C33B1516VH1-7 | **33** | C33B1516VH1-8 | **34** | C33B1516VH2-8 | **35** | C33B1516VH2-9 |
| **36** | C33B1516VL1-39 | **37** | C33B1516VL1-41 | **38** | C33B1516VL1-63 | **39** | C33B1516VL1-90 | **40** | C33B1516VL2-37 |
| **41** | C33B1516VL2-38 | **42** | C33B1516VL2-90 | **43** | C33B1516 Parent | **44** | Positive | | **KEY** |

FIG. 22

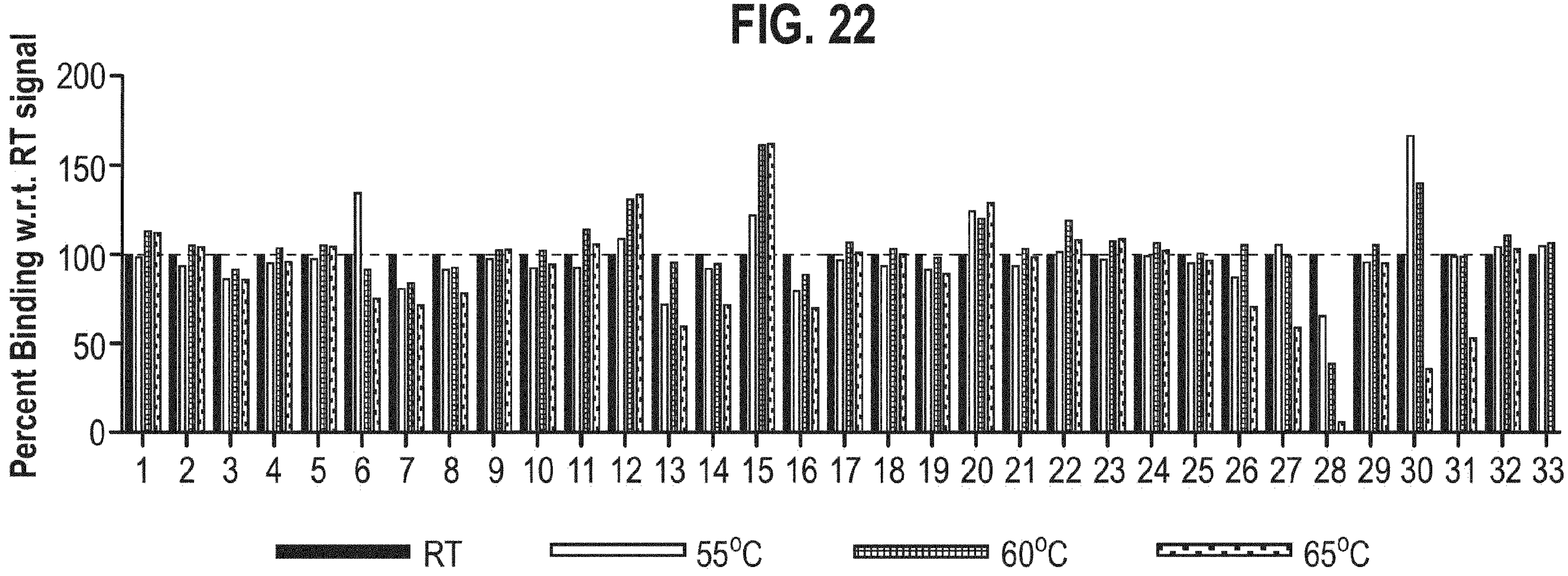


| | | | | | | | | | |
|---|---|---|---|---|---|---|---|---|---|
| **1** | C33B1517VL1-1 | **2** | C33B1517VL1-7 | **3** | C33B1517VL1-12 | **4** | C33B1517VL1-8 | **5** | C33B1517VL1-13 |
| **6** | C33B1517VL1-16 | **7** | C33B1517VL1-22 | **8** | C33B1517VL1-35 | **9** | C33B1517VL1-24 | **10** | C33B1517VL1-27 |
| **11** | C33B1517VL1-49 | **12** | C33B1517VL1-66 | **13** | C33B1517VL1-69 | **14** | C33B1517VL1-71 | **15** | C33B1517VH1-1 |
| **16** | C33B1517VH1-3 | **17** | C33B1517VH1-6 | **18** | C33B1517VH1-4 | **19** | C33B1517VH1-8 | **20** | C33B1517VH1-11 |
| **21** | C33B1517VH1-17 | **22** | C33B1517VH1-27 | **23** | C33B1517VH1-33 | **24** | C33B1517VH1-60 | **25** | C33B1517VH1-71 |
| **26** | C33B1517VH2-7 | **27** | C33B1517VH2-30 | **28** | C33B1517VH2-31 | **29** | C33B1517VH2-60 | **30** | C33B1517VH2-68 |
| **31** | C33B1517VH2-76 | **32** | Positive | **33** | C33B1517 Parent | **KEY** | | | |

FIG. 23

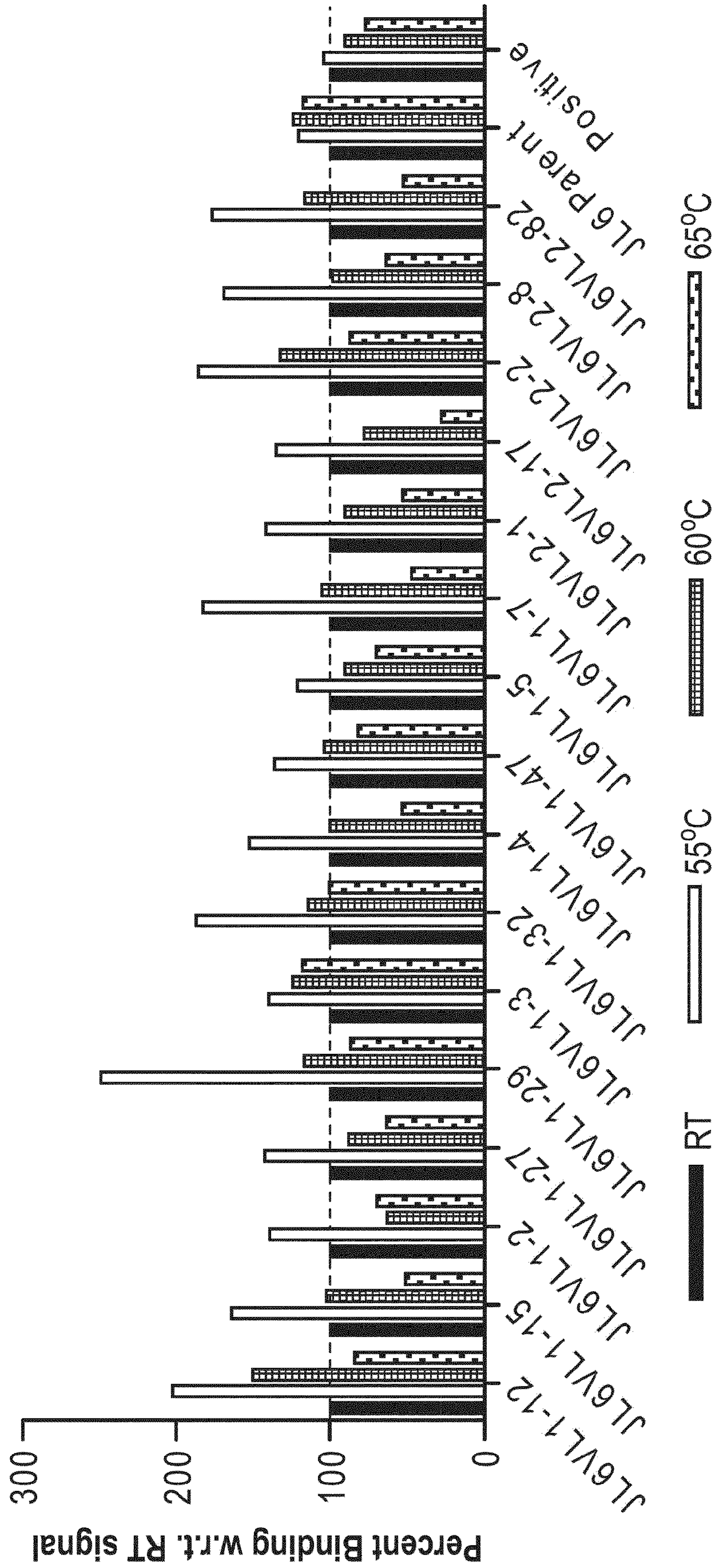

Percent Binding w.r.t. RT signal
0
100
200
300
JL6VL1-12
JL6VL1-15
JL6VL1-2
JL6VL1-27
JL6VL1-29
JL6VL1-3
JL6VL1-32
JL6VL1-4
JL6VL1-47
JL6VL1-5
JL6VL1-7
JL6VL2-1
JL6VL2-17
JL6VL2-2
JL6VL2-8
JL6VL2-82
JL6 Parent
Positive
RT
55°C
60°C
65°C

# FIG. 24

## JL2

| Clone ID | AA |
|---|---|
| C33B1475VH1-2 | G |
| C33B1475VH1-3 | A |
| C33B1475VH1-5 | Y |
| C33B1475VH1-12 | P |
| C33B1475VH1-14 | N |
| C33B1475VH1-22 | S |
| C33B1475VH1-27 | R |
| C33B1475VH1-32 | L |
| C33B1475VH1-36 | V |
| C33B1475VH1-46 | T |
| C33B1475VH1-63 | F |
| C33B1475VH1-89 | W |
| C33B1475VH2-12 | V |
| C33B1475VH2-16 | P |
| C33B1475VH2-20 | R |
| C33B1475VH2-22 | A |
| C33B1475VH2-24 | L |
| C33B1475VH2-35 | Q |
| C33B1475VH2-58 | T |
| C33B1475VH2-86 | H |

## JL3

| Clone ID | AA | Clone ID | AA |
|---|---|---|---|
| C33B1516 VL1-1 | V | C33B1516VH1-1 | V |
| C33B1516 VL1-2 | R | C33B1516VH1-3 | S |
| C33B1516 VL1-4 | P | C33B1516VH1-4 | E |
| C33B1516 VL1-10 | T | C33B1516VH1-6 | H |
| C33B1516 VL1-11 | G | C33B1516VH1-7 | A |
| C33B1516 VL1-14 | Q | C33B1516VH1-8 | G |
| C33B1516 VL1-16 | S | C33B1516VH1-10 | K |
| C33B1516 VL1-20 | A | C33B1516VH1-13 | T |
| C33B1516 VL1-22 | L | C33B1516VH1-17 | F |
| C33B1516 VL1-23 | Y | C33B1516VH1-20 | V |
| C33B1516 VL1-30 | E | C33B1516VH1-21 | R |
| C33B1516VL1-39 | F | C33B1516VH1-27 | L |
| C33B1516VL1-41 | D | C33B1516VH1-38 | H |
| C33B1516VL1-63 | I | C33B1516VH1-56 | I |
| C33B1516VL1-90 | H | C33B1516VH2-8 | S |
| C33B1516 VL2-3 | V | C33B1516VH2-9 | A |
| C33B1516 VL2-4 | P | | |
| C33B1516 VL2-7 | R | | |
| C33B1516 VL2-8 | D | | |
| C33B1516 VL2-10 | E | | |
| C33B1516 VL2-11 | W | | |
| C33B1516 VL2-29 | Y | | |
| C33B1516 VL2-35 | A | | |
| C33B1516VL2-37 | N | | |
| C33B1516VL2-38 | K | | |
| C33B1516VL2-90 | L | | |

# FIG. 24(contd)

## JL5

| Clone ID | AA | Clone ID | AA |
|---|---|---|---|
| C33B1517VL1-1 | G | C33B1517VH1-1 | R |
| C33B1517VL1-7 | D | C33B1517VH1-3 | P |
| C33B1517VL1-8 | P | C33B1517VH1-4 | G |
| C33B1517VL1-12 | K | C33B1517VH1-6 | V |
| C33B1517VL1-13 | E | C33B1517VH1-8 | L |
| C33B1517VL1-16 | A | C33B1517VH1-11 | D |
| C33B1517VL1-22 | R | C33B1517VH1-17 | E |
| C33B1517VL1-24 | Q | C33B1517VH1-33 | A |
| C33B1517VL1-27 | I | C33B1517VH1-60 | Q |
| C33B1517VL1-35 | V | C33B1517VH1-71 | T |
| C33B1517VL1-49 | H | C33B1517VH2-7 | S |
| C33B1517VL1-66 | K | C33B1517VH2-30 | A |
| C33B1517VL1-69 | W | C33B1517VH2-31 | L |
| C33B1517VL1-71 | S | C33B1517VH2-60 | P |
| | | C33B1517VH2-68 | T |
| | | C33B1517VH2-76 | Q |

## JL6

| Clone ID | AA |
|---|---|
| C33B1522VL1-2 | S |
| C33B1522VL1-3 | Q |
| C33B1522VL1-4 | V |
| C33B1522VL1-5 | G |
| C33B1522VL1-7 | W |
| C33B1522VL1-12 | T |
| C33B1522VL1-15 | I |
| C33B1522VL1-27 | E |
| C33B1522VL1-29 | A |
| C33B1522VL1-32 | R |
| C33B1522VL1-47 | L |
| C33B1522VL2-82 | V |
| C33B1522VL2-1 | A |
| C33B1522VL2-2 | P |
| C33B1522VL2-8 | L |
| C33B1522VL2-17 | T |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 62989093
- US 62989071
- US 62989120
- US 62989187
- US 62989230
- WO 2009085462 A
- WO 2019060695 A
- US 4683195 A
- US 5225539 A
- US 6818749 B
- US 20100261620 A
- US 8748356 B
- US 7709226 B
- WO 1990007861 A
- WO 199222653 A
- US 6150584 A
- WO 199945962 A
- WO 2002066630 A
- WO 200243478 A
- WO 2002043478 A
- WO 199004036 A
- WO 09085462 A
- WO 1996027011 A
- US 20100015133 A
- US 20090182127 A
- US 2010028637 A
- US 20110123532 A
- US 20120149876 A
- US 20130195849 A
- US 20070287170 A
- WO 2007147901 A
- WO 2011143545 A
- WO 2013157954 A
- WO 2013096291 A
- US 20180118849 A
- US 9150663 B
- US 20140303356 A
- WO 2009134776 A
- WO 2012022811 A
- US 5932448 A
- US 6833441 B
- US 20140273092 A
- WO 2008077546 A
- WO 2019125982 A
- US 5635483 A
- US 5780588 A
- WO 02088172 A
- US 5075109 A
- US 4452775 A
- US 4667014 A
- US 4748034 A
- US 5239660 A
- US 3854480 A
- US 3832253 A
- US 9850310 B


### Non-patent literature cited in the description


- **WU et al.** *J Exp Med,* 1970, vol. 132, 211-50
- **KABAT et al.** Sequences of Proteins of Immunological Interest. Public Health Service, National Institutes of Health, 1991
- **CHOTHIA et al.** *J Mol Biol,* 1987, vol. 196, 901-17
- **LEFRANC et al.** *Dev Comp Immunol,* 2003, vol. 27, 55-77
- **MARTIN ; THORNTON.** *J Bmol Biol,* 1996, vol. 263, 800-15
- **MACCALLUM, R. M. ; MARTIN, A. C. R. ; THORNTON, J. T.** Antibody-antigen interactions: Contact analysis and binding site topography. *J. Mol. Biol.,* vol. 262, 732-745
- **HONEGGER ; PLUCKTHUN.** *J Mol Biol,* 2001, vol. 309, 657-70
- **WARD et al.** *Nature,* 1989, vol. 341, 544-546
- **KNAPPIK et al.** *J Mol Biol,* 2000, vol. 296, 57-86
- **SHI et al.** *J Mol Biol,* 2010, vol. 397, 385-96
- **MACLENNAN et al.** *Acta Physiol Scand Suppl,* 1988, vol. 643, 55-67
- **SASAKI et al.** *Adv Biophys,* 1988, vol. 35, 1-24
- **PADLAN.** *Mol Immunol,* 1991, vol. 28, 489-499
- **BAERT et al.** *N Engl J Med,* 2003, vol. 348, 602-08
- **STICKLER et al.** *Genes and Immunity,* 2011, vol. 12, 213-21
- **CAI et al.** *Biotechnol Bioeng,* 2011, vol. 108, 404-412

- **KONNO et al.** *Cytotechnology,* 2012, vol. 64, 249-65
- **SHIELDS et al.** *J Biol Chem,* 2002, vol. 277, 26733-26740
- **OLIVIER et al.** *MAbs,* 2010, vol. 2 (4), 405-415
- **SHINKAWA et al.** *J Biol Chem,* 2003, vol. 278, 3466-3473
- **MORI et al.** *Biotechnol Bioeng,* 2004, vol. 88, 901-908
- **FERRARA et al.** *J Biol Chem,* 2006, vol. 281, 5032-5036
- **FERRARA et al.** *Biotechnol Bioeng,* 2006, vol. 93, 851-861
- **XHOU et al.** *Biotechnol Bioeng,* 2008, vol. 99, 652-65
- **WOYKE et al.** *Antimicrob Agents and Chemother.,* 2001, vol. 45 (12), 3580-3584
- **CHAN et al.** Fmoc Solid Phase Peptide Synthesis. Oxford University Press, 2000
- Peptide and Protein Drug Analysis. Marcel Dekker, Inc, 2000
- Epitope Mapping. Oxford University Press, 2001
- **HARLOW et al.** Using Antibodies: A Laboratory Manual. Cold Spring Harbor Laboratory Press, 1999
- **HARLOW et al.** Antibodies: A Laboratory Manual. 1989
- **HOUSTON et al.** *Proc. Natl. Acad. Sci. USA,* 1988, vol. 85, 5879-5883
- **BIRD et al.** *Science,* 1988, vol. 242, 423-426
- **GADI et al.** *Gene Ther.,* 2000, vol. 7, 1738-1743
- **SCOPES.** Protein Purification. Springer- Verlag, 1982
- Pharmaceutical Manufacturing. Remington: The Science and Practice of Pharmacy. Lipincott Williams and Wilkins, 2006, 691-1092, 958-989
- **ROSENBERG et al.** *New Eng. J. of Med.,* 1988, vol. 319, 1676
- **D H WISEMAN ; B F GREYSTOKE ; T C P SOMERVAILLE.** The variety of leukemic stem cells in myeloid malignancy. *Oncogene,* 2014, vol. 33, 3091-3098
- American Cancer Society: Cancer Facts and Figures 2014. American Cancer Society, 2014
- **VITALE C. ; ROMAGNANI C. ; FALCO M. ; PONTE M. ; VITALE M. ; MORETTA A. ; BACIGALUPO A. ; MORETTA L. ; MINGARI M.C.** Engagement of p75/AIRM1 or CD33 inhibits the proliferation of normal or leukemic myeloid cells. *Proc. Natl. Acad. Sci. U.S.A.,* 1999, vol. 96, 15091-15096
- **EHNINGER A. et al.** *Blood Cancer Journal,* 2014, vol. 4, e218
- **FELIX S. LICHTENEGGER et al.** Recent developments in immunotherapy of acute myeloid leukemia. *J Hematol Oncol.,* 2017, vol. 10, 142

SEQUENCE LISTING

<110> JANSSEN BIOTECH, INC.

<120> MATERIALS AND METHODS FOR SIALIC ACID BINDING IG-LIKE LECTIN BINDING

<141> 2021-03-12

<150> US 62/989,071
<151> 2020-03-13

<150> US 62/989,093
<151> 2020-03-13

<150> US 62/989,120
<151> 2020-03-13

<150> US 62/989,230
<151> 2020-03-13

<150> US 62/989,187
<151> 2020-03-13

<160> 542

<170> SeqWin2010, version 1.0

<210> 1
<211> 5
<212> PRT
<213> Artificial Sequence

<220>
<223> Description of Artificial Sequence: Synthetic peptide

<400> 1
Asp Tyr Ala Ile Gly
1               5

<210> 2
<211> 5
<212> PRT
<213> Artificial Sequence

<220>
<223> Description of Artificial Sequence: Synthetic peptide

<400> 2
Thr Tyr Val Met Gly
1               5

<210> 3
<211> 5

<212> PRT
<213> Artificial Sequence

<220>
<223> Description of Artificial Sequence: Synthetic peptide

<400> 3
Phe Ser Thr Met Asp
1 5

<210> 4
<211> 5
<212> PRT
<213> Artificial Sequence

<220>
<223> Description of Artificial Sequence: Synthetic peptide

<400> 4
Ile Asn Ala Met Gly
1 5

<210> 5
<211> 5
<212> PRT
<213> Artificial Sequence

<220>
<223> Description of Artificial Sequence: Synthetic peptide

<400> 5
Asp Tyr Ala Val Gly
1 5

<210> 6
<211> 17
<212> PRT
<213> Artificial Sequence

<220>
<223> Description of Artificial Sequence: Synthetic peptide

<400> 6
Cys Ile Ser Glu Ser Asp Gly Arg Thr Tyr Leu Ser Asp Ser Val Lys
1 5 10 15

Ser

<210> 7
<211> 16
<212> PRT
<213> Artificial Sequence

<220>
<223> Description of Artificial Sequence: Synthetic peptide

<400> 7
Ala Ile Val Ser Gly Arg Asn Pro Thr Tyr Ala Asp Ser Val Lys Ser
1 5 10 15

<210> 8
<211> 17
<212> PRT
<213> Artificial Sequence

<220>
<223> Description of Artificial Sequence: Synthetic peptide

<400> 8
Cys Ile Ser Ser Ser Asp Gly Ser Thr Tyr Tyr Val Asp Ser Val Lys
1 5 10 15

Gly

<210> 9
<211> 17
<212> PRT
<213> Artificial Sequence

<220>
<223> Description of Artificial Sequence: Synthetic peptide

<400> 9
Cys Ile Arg Pro Arg Tyr Ser Thr Thr Thr His Ala Asp Ser Ile Lys
1 5 10 15

Gly

<210> 10
<211> 16
<212> PRT
<213> Artificial Sequence

<220>
<223> Description of Artificial Sequence: Synthetic peptide

<400> 10
Arg Ile Thr Gly Gly Gly Ala Thr Asp Tyr Val Asp Ser Val Lys Gly
1 5 10 15

<210> 11
<211> 17
<212> PRT
<213> Artificial Sequence

<220>
<223> Description of Artificial Sequence: Synthetic peptide

<400> 11
```
Cys Ile Ser Ser Ser Asp Gly Ala Thr Tyr Tyr Ala Asp Ser Val Lys
1               5                   10                  15

Gly
```

<210> 12
<211> 18
<212> PRT
<213> Artificial Sequence

<220>
<223> Description of Artificial Sequence: Synthetic peptide

<400> 12
```
Val Asp Gln Ala Ile Ser Glu Ser Ser Tyr His Cys Glu Lys Gly Phe
1               5                   10                  15

Gly Ser
```

<210> 13
<211> 7
<212> PRT
<213> Artificial Sequence

<220>
<223> Description of Artificial Sequence: Synthetic peptide

<400> 13
```
Tyr His Tyr Ser Ser Gln Tyr
1               5
```

<210> 14
<211> 22
<212> PRT
<213> Artificial Sequence

<220>
<223> Description of Artificial Sequence: Synthetic peptide

<400> 14
```
Leu Ile Ile Met Tyr Gly Ser Gly Ser Glu Arg Ala Ala Ala Ala Cys
1               5                   10                  15

Arg Tyr Lys Tyr Glu Tyr
            20
```

<210> 15
<211> 15

<212> PRT
<213> Artificial Sequence

<220>
<223> Description of Artificial Sequence: Synthetic peptide

<400> 15
Asp Leu Val Thr Arg Val Gly Ser Asp Leu Leu Tyr Asp Asp Tyr
1 5 10 15

<210> 16
<211> 21
<212> PRT
<213> Artificial Sequence

<220>
<223> Description of Artificial Sequence: Synthetic peptide

<400> 16
Ile Ile Val Arg Gly Ser Gly Trp Glu Arg Ala Ala Ala Ala Cys Arg
1 5 10 15

Tyr Glu Tyr Ser Tyr
20

<210> 17
<211> 21
<212> PRT
<213> Artificial Sequence

<220>
<223> Description of Artificial Sequence: Synthetic peptide

<400> 17
Val Ile Val Arg Gly Thr Gly Trp Glu Arg Ala Ala Ala Ala Cys Arg
1 5 10 15

Tyr Glu Tyr Ala Tyr
20

<210> 18
<211> 127
<212> PRT
<213> Artificial Sequence

<220>
<223> Description of Artificial Sequence: Synthetic peptide

<400> 18
Gln Val Gln Leu Val Glu Ser Gly Gly Gly Ser Val Gln Pro Gly Gly
1 5 10 15

Ser Leu Arg Leu Ser Cys Ala Val Phe Gly Phe Ser Leu Ser Asp Tyr
20 25 30

```
Ala Ile Gly Trp Phe Arg Gln Ala Pro Gly Lys Glu Arg Glu Lys Val
        35                  40                  45

Ser Cys Ile Ser Glu Ser Asp Gly Arg Thr Tyr Leu Ser Asp Ser Val
    50                  55                  60

Lys Ser Arg Phe Thr Ile Ser Arg Asp Asn Gly Lys Asn Thr Val Tyr
65                  70                  75                  80

Leu Gln Met Asn Asn Leu Lys Pro Asp Asp Thr Gly Val Tyr Tyr Cys
                85                  90                  95

Ala Thr Val Asp Gln Ala Ile Ser Glu Ser Ser Tyr His Cys Glu Lys
            100                 105                 110

Gly Phe Gly Ser Trp Gly Gln Gly Thr Gln Val Thr Val Ser Ser
        115                 120                 125

<210>   19
<211>   115
<212>   PRT
<213>   Artificial Sequence

<220>
<223>   Description of Artificial Sequence: Synthetic peptide

<400>   19
Glu Val Gln Leu Val Glu Ser Gly Gly Gly Leu Val Gln Ala Gly Gly
1               5                   10                  15

Ser Leu Arg Leu Ser Cys Ala Ala Ser Gly Arg Thr Phe Ser Thr Tyr
            20                  25                  30

Val Met Gly Trp Phe Arg Gln Ala Pro Gly Gln Glu Arg Glu Leu Val
        35                  40                  45

Ala Ala Ile Val Ser Gly Arg Asn Pro Thr Tyr Ala Asp Ser Val Lys
    50                  55                  60

Ser Arg Phe Thr Ile Ser Arg Asp Asn Ala Lys Asn Thr Ile Tyr Leu
65                  70                  75                  80

His Met Asn Ser Leu Gln Pro Glu Asp Thr Ala Val Tyr Tyr Cys His
                85                  90                  95

Met Tyr His Tyr Ser Ser Gln Tyr Trp Gly Gln Gly Thr Gln Val Thr
            100                 105                 110

Val Ser Ser
        115

<210>   20
<211>   131
```

<212> PRT
<213> Artificial Sequence

<220>
<223> Description of Artificial Sequence: Synthetic peptide

<400> 20
Glu Val Gln Leu Val Glu Ser Gly Gly Gly Leu Val Gln Ala Gly Gly
1 5 10 15

Ser Leu Arg Leu Ser Cys Ala Ala Ser Gly Phe Thr Phe Asp Asp Tyr
20 25 30

Ala Ile Gly Trp Phe Arg Gln Ala Pro Gly Lys Glu Arg Glu Gly Val
35 40 45

Ser Cys Ile Ser Ser Ser Asp Gly Ser Thr Tyr Tyr Val Asp Ser Val
50 55 60

Lys Gly Arg Phe Thr Ile Ser Ser Asp Asn Ala Lys Asn Thr Val Tyr
65 70 75 80

Leu Gln Met Asn Ser Leu Lys Pro Glu Asp Thr Ala Val Tyr Tyr Cys
85 90 95

Thr Ala Leu Ile Ile Met Tyr Gly Ser Gly Ser Glu Arg Ala Ala Ala
100 105 110

Ala Cys Arg Tyr Lys Tyr Glu Tyr Trp Gly Gln Gly Thr Gln Val Thr
115 120 125

Val Ser Ser
130

<210> 21
<211> 116
<212> PRT
<213> Artificial Sequence

<220>
<223> Description of Artificial Sequence: Synthetic peptide

<400> 21
Glu Val Gln Val Val Glu Ser Gly Gly Gly Val Val Gln Ser Gly Gly
1 5 10 15

Ser Leu Thr Leu Ser Cys Glu Ala Ser Glu Ser Ile Leu Ser Phe Ser
20 25 30

Thr Met Asp Trp Phe Arg Gln Ala Pro Gly Lys Glu Arg Glu Gly Val
35 40 45

Ser Cys Ile Arg Pro Arg Tyr Ser Thr Thr Thr His Ala Asp Ser Ile
50 55 60

```
Lys Gly Arg Phe Lys Met Tyr Ser Asp Asn Ala Lys Asn Thr Ile Tyr
65                  70                  75                  80

Leu His Met Asn Ser Leu Gln Pro Glu Asp Thr Ala Val Tyr Tyr Cys
                85                  90                  95

His Met Tyr His Tyr Ser Ser Gln Tyr Trp Gly Gln Gly Thr Gln Val
            100                 105                 110

Thr Val Ser Ser
        115

<210>   22
<211>   123
<212>   PRT
<213>   Artificial Sequence

<220>
<223>   Description of Artificial Sequence: Synthetic peptide

<400>   22
Glu Val Gln Val Val Glu Ser Gly Gly Gly Leu Val Gln Gly Gly Gly
1               5                   10                  15

Ser Leu Arg Leu Ser Cys Ala Ala Ser Gly Ser Ile Phe Ser Ile Asn
            20                  25                  30

Ala Met Gly Trp Tyr His Gln Ala Pro Gly Lys Gln Arg Glu Leu Val
        35                  40                  45

Ala Arg Ile Thr Gly Gly Gly Ala Thr Asp Tyr Val Asp Ser Val Lys
    50                  55                  60

Gly Arg Phe Thr Ile Ser Leu Asp Ser Ala Lys Ser Thr Val Tyr Leu
65                  70                  75                  80

Gln Met Asn Ser Leu Lys Pro Glu Asp Thr Ala Val Tyr His Cys Tyr
                85                  90                  95

Ala Asp Leu Val Thr Arg Val Gly Ser Asp Leu Leu Tyr Asp Asp Tyr
            100                 105                 110

Trp Gly Gln Gly Thr Gln Val Thr Val Ser Ser
        115                 120

<210>   23
<211>   127
<212>   PRT
<213>   Artificial Sequence

<220>
<223>   Description of Artificial Sequence: Synthetic peptide
```

<400> 23
Glu Val Gln Leu Val Glu Ser Gly Gly Gly Leu Val Gln Pro Gly Gly
1 5 10 15

Ser Leu Arg Leu Ser Cys Ala Val Phe Gly Phe Ser Leu Ser Asp Tyr
20 25 30

Ala Ile Gly Trp Phe Arg Gln Ala Pro Gly Lys Glu Arg Glu Lys Val
35 40 45

Ser Cys Ile Ser Glu Ser Asp Gly Arg Thr Tyr Leu Ser Asp Ser Val
50 55 60

Lys Ser Arg Phe Thr Ile Ser Arg Asp Asn Gly Lys Asn Thr Val Tyr
65 70 75 80

Leu Gln Met Asn Asn Leu Lys Pro Asp Asp Thr Gly Val Tyr Tyr Cys
85 90 95

Ala Thr Val Asp Gln Ala Ile Ser Glu Ser Ser Tyr His Cys Glu Lys
100 105 110

Gly Phe Gly Ser Trp Gly Gln Gly Thr Gln Val Thr Val Ser Ser
115 120 125

<210> 24
<211> 130
<212> PRT
<213> Artificial Sequence

<220>
<223> Description of Artificial Sequence: Synthetic peptide

<400> 24
Glu Val Gln Leu Val Glu Ser Gly Gly Gly Leu Val Gln Ala Gly Gly
1 5 10 15

Ser Leu Arg Leu Ser Cys Ala Ala Ser Gly Phe Thr Phe Asp Asp Tyr
20 25 30

Ala Val Gly Trp Phe Arg Gln Val Pro Gly Lys Glu Arg Glu Gly Val
35 40 45

Ser Cys Ile Ser Ser Ser Asp Gly Ala Thr Tyr Tyr Ala Asp Ser Val
50 55 60

Lys Gly Arg Phe Thr Ile Ser Ser Asp Asn Ala Lys Asn Thr Val Tyr
65 70 75 80

Leu Gln Met Asn Ser Leu Lys Pro Glu Asp Thr Ala Val Tyr Tyr Cys
85 90 95

Thr Ala Ile Ile Val Arg Gly Ser Gly Trp Glu Arg Ala Ala Ala Ala
100 105 110

```
Cys Arg Tyr Glu Tyr Ser Tyr Trp Gly Gln Gly Thr Gln Val Thr Val
        115                 120                 125

Ser Ser
    130

<210>   25
<211>   130
<212>   PRT
<213>   Artificial Sequence

<220>
<223>   Description of Artificial Sequence: Synthetic peptide

<400>   25
Glu Val Gln Leu Val Glu Ser Gly Gly Gly Leu Val Gln Ala Gly Gly
1               5                   10                  15

Ser Leu Arg Leu Ser Cys Ala Ala Ser Gly Phe Thr Phe Asp Asp Tyr
            20                  25                  30

Ala Val Gly Trp Phe Arg Gln Ala Pro Gly Lys Glu Arg Glu Gly Ile
        35                  40                  45

Ser Cys Ile Ser Ser Ser Asp Gly Ala Thr Tyr Tyr Ala Asp Ser Val
    50                  55                  60

Lys Gly Arg Phe Thr Ile Ser Thr Asp Asn Ala Lys Asn Thr Ala Phe
65                  70                  75                  80

Leu Gln Met Asn Ser Leu Lys Pro Glu Asp Thr Ala Val Tyr Tyr Cys
                85                  90                  95

Thr Ala Val Ile Val Arg Gly Thr Gly Trp Glu Arg Ala Ala Ala Ala
            100                 105                 110

Cys Arg Tyr Glu Tyr Ala Tyr Trp Gly Gln Gly Thr Gln Val Thr Val
        115                 120                 125

Ser Ser
    130

<210>   26
<211>   127
<212>   PRT
<213>   Artificial Sequence

<220>
<223>   Description of Artificial Sequence: Synthetic peptide

<400>   26
Gln Val Gln Leu Val Glu Ser Gly Gly Gly Leu Val Gln Ala Gly Gly
1               5                   10                  15
```

```
Ser Leu Arg Leu Ser Cys Ala Val Phe Gly Phe Ser Leu Ser Asp Tyr
            20                  25                  30

Ala Ile Gly Trp Phe Arg Gln Ala Pro Gly Lys Glu Arg Glu Lys Val
        35                  40                  45

Ser Cys Ile Ser Glu Ser Asp Gly Arg Thr Tyr Leu Ser Asp Ser Val
    50                  55                  60

Lys Ser Arg Phe Thr Ile Ser Arg Asp Asn Gly Lys Asn Thr Val Tyr
65                  70                  75                  80

Leu Gln Met Asn Asn Leu Lys Pro Asp Asp Thr Gly Val Tyr Tyr Cys
                85                  90                  95

Ala Thr Val Asp Gln Ala Ile Ser Glu Ser Ser Tyr His Cys Glu Lys
            100                 105                 110

Gly Phe Gly Ser Trp Gly Gln Gly Thr Gln Val Thr Val Ser Ser
        115                 120                 125

<210>   27
<211>   127
<212>   PRT
<213>   Artificial Sequence

<220>
<223>   Description of Artificial Sequence: Synthetic peptide

<400>   27
Gln Val Gln Leu Val Glu Ser Gly Gly Gly Leu Val Gln Pro Gly Gly
1               5                   10                  15

Ser Leu Arg Leu Ser Cys Ala Val Phe Gly Phe Ser Leu Ser Asp Tyr
            20                  25                  30

Ala Ile Gly Trp Phe Arg Gln Ala Pro Gly Lys Glu Arg Glu Lys Val
        35                  40                  45

Ser Cys Ile Ser Glu Ser Asp Gly Arg Thr Tyr Leu Ser Asp Ser Val
    50                  55                  60

Lys Ser Arg Phe Thr Ile Ser Arg Asp Asn Gly Lys Asn Thr Val Tyr
65                  70                  75                  80

Leu Gln Met Asn Asn Leu Lys Pro Asp Asp Thr Gly Val Tyr Tyr Cys
                85                  90                  95

Ala Thr Val Asp Gln Ala Ile Ser Glu Ser Ser Tyr His Cys Glu Lys
            100                 105                 110

Gly Phe Gly Ser Trp Gly Gln Gly Thr Gln Val Thr Val Ser Ser
        115                 120                 125
```

<210> 28
<211> 5
<212> PRT
<213> Artificial Sequence

<220>
<223> Description of Artificial Sequence: Synthetic peptide

<400> 28
Ser Phe Gly Met Ser
1 5

<210> 29
<211> 5
<212> PRT
<213> Artificial Sequence

<220>
<223> Description of Artificial Sequence: Synthetic peptide

<400> 29
Ser Tyr Tyr Trp Gly
1 5

<210> 30
<211> 5
<212> PRT
<213> Artificial Sequence

<220>
<223> Description of Artificial Sequence: Synthetic peptide

<400> 30
Asp Tyr Asn Ile Asn
1 5

<210> 31
<211> 5
<212> PRT
<213> Artificial Sequence

<220>
<223> Description of Artificial Sequence: Synthetic peptide

<400> 31
Asn Tyr Ala Met Ser
1 5

<210> 32
<211> 5
<212> PRT
<213> Artificial Sequence

<220>
<223> Description of Artificial Sequence: Synthetic peptide

<400> 32
Asn Tyr Tyr Trp Ser
1 5

<210> 33
<211> 5
<212> PRT
<213> Artificial Sequence

<220>
<223> Description of Artificial Sequence: Synthetic peptide

<400> 33
Ser Tyr Trp Met Ser
1 5

<210> 34
<211> 5
<212> PRT
<213> Artificial Sequence

<220>
<223> Description of Artificial Sequence: Synthetic peptide

<400> 34
Val Ser Ala Ile His
1 5

<210> 35
<211> 5
<212> PRT
<213> Artificial Sequence

<220>
<223> Description of Artificial Sequence: Synthetic peptide

<400> 35
Val Phe Ala Ile His
1 5

<210> 36
<211> 17
<212> PRT
<213> Artificial Sequence

<220>
<223> Description of Artificial Sequence: Synthetic peptide

<400> 36
Asn Ile Lys Arg Asp Gly Ser Glu Lys Tyr Tyr Val Asp Ser Val Lys
1 5 10 15

Gly

<210> 37
<211> 16
<212> PRT
<213> Artificial Sequence

<220>
<223> Description of Artificial Sequence: Synthetic peptide

<400> 37
Tyr Ile Tyr Tyr Ser Gly Ser Thr Asn Tyr Asn Pro Ser Leu Lys Ser
1 5 10 15

<210> 38
<211> 17
<212> PRT
<213> Artificial Sequence

<220>
<223> Description of Artificial Sequence: Synthetic peptide

<400> 38
Thr Ile Asn Pro Asn Asn Gly Val Thr Phe Tyr Asn Gln Arg Phe Lys
1 5 10 15

Gly

<210> 39
<211> 17
<212> PRT
<213> Artificial Sequence

<220>
<223> Description of Artificial Sequence: Synthetic peptide

<400> 39
Gly Ile Ser Asn Ser Gly Tyr Ser Leu Tyr Tyr Pro Asp Thr Leu Lys
1 5 10 15

Gly

<210> 40
<211> 16
<212> PRT
<213> Artificial Sequence

<220>
<223> Description of Artificial Sequence: Synthetic peptide

<400> 40
His Ile Tyr Ser Thr Gly Asn Ile His Tyr Asn Pro Ser Leu Lys Ser
1 5 10 15

<210> 41
<211> 17
<212> PRT
<213> Artificial Sequence

<220>
<223> Description of Artificial Sequence: Synthetic peptide

<400> 41
Asn Ile Lys Arg Asp Gly Ser Glu Lys His Tyr Val Asp Ser Val Lys
1 5 10 15

Gly

<210> 42
<211> 19
<212> PRT
<213> Artificial Sequence

<220>
<223> Description of Artificial Sequence: Synthetic peptide

<400> 42
Arg Ile Arg Ser Lys Gly Asn Ser Tyr Ala Thr Ala Tyr Asp Val Ser
1 5 10 15

Val Lys Gly

<210> 43
<211> 19
<212> PRT
<213> Artificial Sequence

<220>
<223> Description of Artificial Sequence: Synthetic peptide

<400> 43
Arg Ile Arg Ser Lys Gly Asn Ser Tyr Ala Thr Ala Tyr Ala Ala Ser
1 5 10 15

Val Lys Gly

<210> 44
<211> 19
<212> PRT
<213> Artificial Sequence

<220>
<223> Description of Artificial Sequence: Synthetic peptide

<400> 44
Arg Ile Arg Ser Lys Gly Asn Ser Tyr Ala Thr Ala Tyr Asn Val Ser
1 5 10 15

Val Lys Gly

<210> 45
<211> 7
<212> PRT
<213> Artificial Sequence

<220>
<223> Description of Artificial Sequence: Synthetic peptide

<400> 45
Asp Tyr Gly Tyr Phe Asp Phe
1 5

<210> 46
<211> 9
<212> PRT
<213> Artificial Sequence

<220>
<223> Description of Artificial Sequence: Synthetic peptide

<400> 46
Met Trp Glu Ile Leu Gly Phe Asp Pro
1 5

<210> 47
<211> 11
<212> PRT
<213> Artificial Sequence

<220>
<223> Description of Artificial Sequence: Synthetic peptide

<400> 47
Asp Gly Tyr Asp Thr Tyr Tyr Ala Met Asp Tyr
1 5 10

<210> 48
<211> 11
<212> PRT
<213> Artificial Sequence

<220>
<223> Description of Artificial Sequence: Synthetic peptide

<400> 48
Asp Gly Gly Ser Tyr Pro Tyr Ala Met Asp Tyr
1 5 10

<210> 49
<211> 9
<212> PRT
<213> Artificial Sequence

<220>
<223> Description of Artificial Sequence: Synthetic peptide

<400> 49
Asp Asn Gly Ala Ala Leu Phe Asp Tyr
1 5

<210> 50
<211> 7
<212> PRT
<213> Artificial Sequence

<220>
<223> Description of Artificial Sequence: Synthetic peptide

<400> 50
Asp Tyr Gly Tyr Phe Asp Tyr
1 5

<210> 51
<211> 12
<212> PRT
<213> Artificial Sequence

<220>
<223> Description of Artificial Sequence: Synthetic peptide

<400> 51
His Asn Asp Lys Trp Asn Tyr Tyr Gly Leu Asp Val
1 5 10

<210> 52
<211> 116
<212> PRT
<213> Artificial Sequence

<220>
<223> Description of Artificial Sequence: Synthetic peptide

<400> 52
Gln Val Gln Leu Val Glu Ser Gly Gly Gly Leu Val Gln Pro Gly Gly
1 5 10 15

Ser Leu Arg Leu Ser Cys Ala Ala Ser Gly Phe Thr Phe Ser Ser Phe
20 25 30

```
Gly Met Ser Trp Val Arg Gln Ala Pro Gly Lys Gly Leu Glu Trp Val
        35                  40                  45

Ala Asn Ile Lys Arg Asp Gly Ser Glu Lys Tyr Tyr Val Asp Ser Val
    50                  55                  60

Lys Gly Arg Phe Thr Ile Ser Arg Asp Asn Ala Lys Asn Ser Leu Phe
65                  70                  75                  80

Leu Gln Met Ser Ser Leu Arg Ala Glu Asp Thr Ala Val Tyr Tyr Cys
                85                  90                  95

Val Arg Asp Tyr Gly Tyr Phe Asp Phe Trp Gly Gln Gly Thr Leu Val
            100                 105                 110

Thr Val Ser Ser
        115

<210>   53
<211>   116
<212>   PRT
<213>   Artificial Sequence

<220>
<223>   Description of Artificial Sequence: Synthetic peptide

<400>   53
Gln Val Gln Leu Gln Glu Ser Gly Pro Gly Leu Val Lys Pro Ser Glu
1               5                   10                  15

Thr Leu Ser Leu Thr Cys Thr Val Ser Gly Gly Ser Ile Ser Ser Tyr
            20                  25                  30

Tyr Trp Gly Trp Ile Arg Gln Pro Pro Gly Lys Gly Leu Glu Trp Ile
        35                  40                  45

Gly Tyr Ile Tyr Tyr Ser Gly Ser Thr Asn Tyr Asn Pro Ser Leu Lys
    50                  55                  60

Ser Arg Val Thr Ile Ser Val Asp Thr Ser Lys Asn Gln Phe Ser Leu
65                  70                  75                  80

Lys Leu Ser Ser Val Thr Ala Ala Asp Thr Ala Val Tyr Tyr Cys Ala
                85                  90                  95

Arg Met Trp Glu Ile Leu Gly Phe Asp Pro Trp Gly Gln Gly Thr Leu
            100                 105                 110

Val Thr Val Ser
        115

<210>   54
<211>   120
```

<212> PRT
<213> Artificial Sequence

<220>
<223> Description of Artificial Sequence: Synthetic peptide

<400> 54
```
Glu Val Gln Leu Gln Gln Ser Gly Pro Glu Leu Val Lys Pro Gly Ser
1               5                   10                  15

Ser Val Lys Ile Ser Cys Lys Ala Ser Gly Tyr Thr Phe Thr Asp Tyr
            20                  25                  30

Asn Ile Asn Trp Val Lys Gln Ser His Gly Lys Asn Leu Glu Trp Ile
        35                  40                  45

Gly Thr Ile Asn Pro Asn Asn Gly Val Thr Phe Tyr Asn Gln Arg Phe
    50                  55                  60

Lys Gly Gln Ala Thr Leu Thr Val Asp Lys Ser Ser Ser Thr Ala Tyr
65                  70                  75                  80

Met Glu Leu Arg Ser Leu Thr Ser Glu Asp Ser Ala Val Tyr Tyr Cys
                85                  90                  95

Ser Arg Asp Gly Tyr Asp Thr Tyr Tyr Ala Met Asp Tyr Trp Gly Gln
            100                 105                 110

Gly Thr Thr Leu Thr Val Ser Ser
        115                 120
```

<210> 55
<211> 120
<212> PRT
<213> Artificial Sequence

<220>
<223> Description of Artificial Sequence: Synthetic peptide

<400> 55
```
Glu Val Gln Leu Val Glu Ser Gly Gly Gly Leu Val Arg Pro Gly Gly
1               5                   10                  15

Ser Leu Lys Leu Ser Cys Ala Ala Ser Gly Phe Thr Phe Ser Asn Tyr
            20                  25                  30

Ala Met Ser Trp Val Arg Gln Thr Pro Glu Lys Arg Leu Glu Trp Val
        35                  40                  45

Ala Gly Ile Ser Asn Ser Gly Tyr Ser Leu Tyr Tyr Pro Asp Thr Leu
    50                  55                  60

Lys Gly Arg Phe Thr Ile Ser Arg Asp Asn Ala Arg Asn Thr Leu Tyr
65                  70                  75                  80
```

Leu Gln Met Ile Ser Leu Arg Ser Glu Asp Thr Ala Met Tyr Tyr Cys
                85                  90                  95

Ala Arg Asp Gly Gly Ser Tyr Pro Tyr Ala Met Asp Tyr Trp Gly His
            100                 105                 110

Gly Thr Ser Val Thr Val Ser Ser
        115                 120

<210> 56
<211> 117
<212> PRT
<213> Artificial Sequence

<220>
<223> Description of Artificial Sequence: Synthetic peptide

<400> 56
Gln Val Gln Leu Gln Glu Ser Gly Pro Gly Leu Val Lys Pro Ser Glu
1               5                   10                  15

Thr Leu Ser Leu Thr Cys Ser Val Ser Gly Ala Ser Ile Arg Asn Tyr
            20                  25                  30

Tyr Trp Ser Trp Ile Arg Gln Thr Ala Gly Lys Gly Leu Glu Trp Leu
        35                  40                  45

Gly His Ile Tyr Ser Thr Gly Asn Ile His Tyr Asn Pro Ser Leu Lys
    50                  55                  60

Ser Arg Val Thr Met Ser Val Asp Thr Ser Asn Asn Gln Phe Ser Leu
65                  70                  75                  80

Lys Leu Arg Ser Val Thr Ala Ala Asp Thr Ala Val Tyr Tyr Cys Ala
                85                  90                  95

Arg Asp Asn Gly Ala Ala Leu Phe Asp Tyr Trp Gly Gln Gly Thr Leu
            100                 105                 110

Val Thr Val Ser Ser
        115

<210> 57
<211> 116
<212> PRT
<213> Artificial Sequence

<220>
<223> Description of Artificial Sequence: Synthetic peptide

<400> 57
Glu Val Gln Leu Val Glu Ser Gly Gly Gly Leu Val Gln Pro Gly Gly
1               5                   10                  15

Ser Leu Arg Leu Ser Cys Thr Ala Ser Gly Phe Thr Phe Ser Ser Tyr
20 25 30

Trp Met Ser Trp Val Arg Gln Ala Pro Gly Lys Gly Leu Asp Trp Val
35 40 45

Ala Asn Ile Lys Arg Asp Gly Ser Glu Lys His Tyr Val Asp Ser Val
50 55 60

Lys Gly Arg Phe Thr Ile Ser Arg Asp Asn Ala Lys Asn Ser Leu Tyr
65 70 75 80

Leu Gln Met Asn Ser Leu Arg Ala Glu Asp Ser Ala Val Tyr Tyr Cys
85 90 95

Thr Arg Asp Tyr Gly Tyr Phe Asp Tyr Trp Gly Gln Gly Thr Leu Val
100 105 110

Thr Val Ser Ser
115

<210> 58
<211> 123
<212> PRT
<213> Artificial Sequence

<220>
<223> Description of Artificial Sequence: Synthetic peptide

<400> 58
Glu Val Gln Leu Val Glu Ser Gly Gly Gly Leu Val Gln Pro Gly Gly
1 5 10 15

Ser Leu Lys Val Ser Cys Glu Ala Ser Gly Phe Thr Phe Ser Val Ser
20 25 30

Ala Ile His Trp Val Arg Gln Ala Ser Gly Lys Gly Leu Glu Trp Ile
35 40 45

Gly Arg Ile Arg Ser Lys Gly Asn Ser Tyr Ala Thr Ala Tyr Asp Val
50 55 60

Ser Val Lys Gly Arg Phe Thr Ile Ser Arg Asp Asp Ser Lys Asn Thr
65 70 75 80

Ala Tyr Leu Gln Met Asp Ser Leu Lys Thr Glu Asp Thr Ala Val Tyr
85 90 95

Tyr Cys Thr Arg His Asn Asp Lys Trp Asn Tyr Tyr Gly Leu Asp Val
100 105 110

Trp Gly Gln Gly Thr Thr Val Thr Val Ser Ser
115 120

<210> 59
<211> 123
<212> PRT
<213> Artificial Sequence

<220>
<223> Description of Artificial Sequence: Synthetic peptide

<400> 59
Glu Val Gln Leu Val Glu Ser Gly Gly Gly Leu Val Gln Pro Gly Gly
1 5 10 15

Ser Leu Lys Val Ser Cys Glu Ala Ser Gly Phe Thr Phe Ser Val Ser
20 25 30

Ala Ile His Trp Val Arg Gln Ala Ser Gly Lys Gly Leu Glu Trp Ile
35 40 45

Gly Arg Ile Arg Ser Lys Gly Asn Ser Tyr Ala Thr Ala Tyr Ala Ala
50 55 60

Ser Val Lys Gly Arg Phe Thr Ile Ser Arg Asp Asp Ser Lys Asn Thr
65 70 75 80

Ala Tyr Leu Gln Met Asp Ser Leu Lys Thr Glu Asp Thr Ala Val Tyr
85 90 95

Tyr Cys Thr Arg His Asn Asp Lys Trp Asn Tyr Tyr Gly Leu Asp Val
100 105 110

Trp Gly Gln Gly Thr Thr Val Thr Val Ser Ser
115 120

<210> 60
<211> 123
<212> PRT
<213> Artificial Sequence

<220>
<223> Description of Artificial Sequence: Synthetic peptide

<400> 60
Glu Val Gln Leu Val Glu Ser Gly Gly Gly Leu Val Gln Pro Gly Gly
1 5 10 15

Ser Leu Lys Val Ser Cys Glu Ala Ser Gly Phe Thr Phe Ser Val Ser
20 25 30

Ala Ile His Trp Val Arg Gln Ala Ser Gly Lys Gly Leu Glu Trp Ile
35 40 45

Gly Arg Ile Arg Ser Lys Gly Asn Ser Tyr Ala Thr Ala Tyr Asn Val
50 55 60

```
Ser Val Lys Gly Arg Phe Thr Ile Ser Arg Asp Asp Ser Lys Asn Thr
65                  70                  75                  80

Ala Tyr Leu Gln Met Asp Ser Leu Lys Thr Glu Asp Thr Ala Val Tyr
                85                  90                  95

Tyr Cys Thr Arg His Asn Asp Lys Trp Asn Tyr Tyr Gly Leu Asp Val
            100                 105                 110

Trp Gly Gln Gly Thr Thr Val Thr Val Ser Ser
        115                 120

<210>   61
<211>   123
<212>   PRT
<213>   Artificial Sequence

<220>
<223>   Description of Artificial Sequence: Synthetic peptide

<400>   61
Glu Val Gln Leu Val Glu Ser Gly Gly Gly Leu Val Gln Pro Gly Gly
1               5                   10                  15

Ser Leu Lys Val Ser Cys Glu Ala Ser Gly Phe Thr Phe Ser Val Phe
            20                  25                  30

Ala Ile His Trp Val Arg Gln Ala Ser Gly Lys Gly Leu Glu Trp Ile
        35                  40                  45

Gly Arg Ile Arg Ser Lys Gly Asn Ser Tyr Ala Thr Ala Tyr Asp Val
    50                  55                  60

Ser Val Lys Gly Arg Phe Thr Ile Ser Arg Asp Asp Ser Lys Asn Thr
65                  70                  75                  80

Ala Tyr Leu Gln Met Asp Ser Leu Lys Thr Glu Asp Thr Ala Val Tyr
                85                  90                  95

Tyr Cys Thr Arg His Asn Asp Lys Trp Asn Tyr Tyr Gly Leu Asp Val
            100                 105                 110

Trp Gly Gln Gly Thr Thr Val Thr Val Ser Ser
        115                 120

<210>   62
<211>   11
<212>   PRT
<213>   Artificial Sequence

<220>
<223>   Description of Artificial Sequence: Synthetic peptide
```

<400> 62
Gln Ala Ser Gln Ala Ile Ser Asn Tyr Leu Asn
1 5 10

<210> 63
<211> 13
<212> PRT
<213> Artificial Sequence

<220>
<223> Description of Artificial Sequence: Synthetic peptide

<400> 63
Ser Gly Ser Ser Ser Asn Ile Gly Ser Asn Pro Val Asn
1 5 10

<210> 64
<211> 11
<212> PRT
<213> Artificial Sequence

<220>
<223> Description of Artificial Sequence: Synthetic peptide

<400> 64
Lys Ala Ser Gln Asp Val Arg Thr Ala Glu Ala
1 5 10

<210> 65
<211> 11
<212> PRT
<213> Artificial Sequence

<220>
<223> Description of Artificial Sequence: Synthetic peptide

<400> 65
Lys Ala Ser Gln Asn Val Gly Thr Tyr Val Ala
1 5 10

<210> 66
<211> 13
<212> PRT
<213> Artificial Sequence

<220>
<223> Description of Artificial Sequence: Synthetic peptide

<400> 66
Ser Gly Ser Ser Ser Asn Ile Gly Ser Asn Ile Val Asn
1 5 10

<210> 67
<211> 11

<212> PRT
<213> Artificial Sequence

<220>
<223> Description of Artificial Sequence: Synthetic peptide

<400> 67
Arg Ala Ser Gln Gly Ile Ser Ser Tyr Leu Ala
1 5 10

<210> 68
<211> 16
<212> PRT
<213> Artificial Sequence

<220>
<223> Description of Artificial Sequence: Synthetic peptide

<400> 68
Lys Ser Ser Gln Ser Leu Leu Phe Ser Asn Gly Tyr Lys Phe Leu Asp
1 5 10 15

<210> 69
<211> 7
<212> PRT
<213> Artificial Sequence

<220>
<223> Description of Artificial Sequence: Synthetic peptide

<400> 69
Asp Ala Ser Asn Leu Glu Thr
1 5

<210> 70
<211> 7
<212> PRT
<213> Artificial Sequence

<220>
<223> Description of Artificial Sequence: Synthetic peptide

<400> 70
Ser Asn Asn Gln Arg Pro Ser
1 5

<210> 71
<211> 7
<212> PRT
<213> Artificial Sequence

<220>
<223> Description of Artificial Sequence: Synthetic peptide

<400> 71
Ser Ala Ser Asn Arg Tyr Thr
1 5

<210> 72
<211> 7
<212> PRT
<213> Artificial Sequence

<220>
<223> Description of Artificial Sequence: Synthetic peptide

<400> 72
Ser Ala Ser Tyr Arg Tyr Ser
1 5

<210> 73
<211> 7
<212> PRT
<213> Artificial Sequence

<220>
<223> Description of Artificial Sequence: Synthetic peptide

<400> 73
Ala Ala Ser Thr Leu Gln Ser
1 5

<210> 74
<211> 7
<212> PRT
<213> Artificial Sequence

<220>
<223> Description of Artificial Sequence: Synthetic peptide

<400> 74
Leu Gly Ser Tyr Arg Ala Ser
1 5

<210> 75
<211> 9
<212> PRT
<213> Artificial Sequence

<220>
<223> Description of Artificial Sequence: Synthetic peptide

<400> 75
Gln His Tyr Asp Asn Leu Pro Tyr Thr
1 5

<210> 76
<211> 11

<212> PRT
<213> Artificial Sequence

<220>
<223> Description of Artificial Sequence: Synthetic peptide

<400> 76
Ala Ala Trp Asp Asp Ser Leu Asn Gly Pro Val
1               5                   10

<210> 77
<211> 9
<212> PRT
<213> Artificial Sequence

<220>
<223> Description of Artificial Sequence: Synthetic peptide

<400> 77
Gln Gln Tyr Tyr Thr Thr Pro Arg Thr
1               5

<210> 78
<211> 9
<212> PRT
<213> Artificial Sequence

<220>
<223> Description of Artificial Sequence: Synthetic peptide

<400> 78
Gln Gln Tyr Lys Ser Tyr Pro Leu Thr
1               5

<210> 79
<211> 9
<212> PRT
<213> Artificial Sequence

<220>
<223> Description of Artificial Sequence: Synthetic peptide

<400> 79
Gln Gln Leu Asn Ser Tyr Pro Val Thr
1               5

<210> 80
<211> 9
<212> PRT
<213> Artificial Sequence

<220>
<223> Description of Artificial Sequence: Synthetic peptide

<400> 80
Met Gln Ala Leu Gln Thr Pro Pro Thr
1 5

<210> 81
<211> 107
<212> PRT
<213> Artificial Sequence

<220>
<223> Description of Artificial Sequence: Synthetic peptide

<400> 81
Asp Ile Gln Met Thr Gln Ser Pro Ser Ser Leu Ser Ala Ser Val Gly
1 5 10 15

Asp Arg Val Thr Ile Thr Cys Gln Ala Ser Gln Ala Ile Ser Asn Tyr
20 25 30

Leu Asn Trp Tyr Gln Gln Lys Pro Gly Lys Ala Pro Lys Leu Leu Ile
35 40 45

Tyr Asp Ala Ser Asn Leu Glu Thr Gly Val Pro Ser Arg Phe Ser Gly
50 55 60

Ser Gly Ser Gly Thr Asp Phe Thr Phe Thr Ile Ser Ser Leu Gln Pro
65 70 75 80

Glu Asp Phe Ser Thr Tyr Phe Cys Gln His Tyr Asp Asn Leu Pro Tyr
85 90 95

Thr Phe Gly Gln Gly Thr Lys Leu Glu Ile Lys
100 105

<210> 82
<211> 110
<212> PRT
<213> Artificial Sequence

<220>
<223> Description of Artificial Sequence: Synthetic peptide

<400> 82
Gln Ser Val Leu Thr Gln Pro Pro Ser Ala Ser Gly Thr Pro Gly Gln
1 5 10 15

Arg Val Thr Ile Ser Cys Ser Gly Ser Ser Ser Asn Ile Gly Ser Asn
20 25 30

Pro Val Asn Trp Tyr Gln Gln Leu Pro Gly Thr Ala Pro Lys Leu Leu
35 40 45

Ile Tyr Ser Asn Asn Gln Arg Pro Ser Gly Val Pro Asp Arg Phe Ser
50 55 60

Gly Ser Lys Ser Gly Thr Ser Ala Ser Leu Ala Ile Ser Gly Leu Gln
65 70 75 80

Ser Glu Asp Glu Ala Asp Tyr Phe Cys Ala Ala Trp Asp Asp Ser Leu
85 90 95

Asn Gly Pro Val Phe Gly Gly Gly Thr Lys Leu Thr Val Leu
100 105 110

<210> 83
<211> 107
<212> PRT
<213> Artificial Sequence

<220>
<223> Description of Artificial Sequence: Synthetic peptide

<400> 83
Asp Ile Val Met Thr Gln Ser His Lys Phe Met Ser Thr Ser Val Gly
1 5 10 15

Asp Arg Val Ser Ile Thr Cys Lys Ala Ser Gln Asp Val Arg Thr Ala
20 25 30

Glu Ala Trp Tyr Gln Gln Lys Pro Gly Gln Ser Pro Lys Leu Leu Ile
35 40 45

Tyr Ser Ala Ser Asn Arg Tyr Thr Gly Val Pro Asp Arg Phe Thr Gly
50 55 60

Ser Gly Ser Gly Thr Asp Phe Thr Phe Thr Ile Ser Ser Val Gln Ala
65 70 75 80

Glu Asp Leu Ala Val Tyr Tyr Cys Gln Gln Tyr Tyr Thr Thr Pro Arg
85 90 95

Thr Phe Gly Gly Gly Thr Lys Leu Glu Ile Lys
100 105

<210> 84
<211> 107
<212> PRT
<213> Artificial Sequence

<220>
<223> Description of Artificial Sequence: Synthetic peptide

<400> 84
Asp Ile Val Met Thr Gln Ser Gln Lys Phe Met Ser Thr Ser Val Gly
1 5 10 15

Asp Arg Val Ser Val Thr Cys Lys Ala Ser Gln Asn Val Gly Thr Tyr
20 25 30

```
Val Ala Trp Tyr Gln Gln Lys Pro Gly His Ser Pro Lys Ala Leu Ile
        35                  40                  45

Tyr Ser Ala Ser Tyr Arg Tyr Ser Gly Val Pro Asp Arg Phe Thr Gly
    50                  55                  60

Ser Gly Ser Gly Thr Asp Phe Thr Leu Thr Ile Ser Asn Val Gln Ser
65                  70                  75                  80

Glu Asp Leu Ala Asp Tyr Phe Cys Gln Gln Tyr Lys Ser Tyr Pro Leu
                85                  90                  95

Thr Phe Gly Ala Gly Thr Lys Leu Glu Leu Lys
            100                 105

<210>   85
<211>   110
<212>   PRT
<213>   Artificial Sequence

<220>
<223>   Description of Artificial Sequence: Synthetic peptide

<400>   85
Gln Ser Val Leu Thr Gln Pro Pro Ser Ala Ser Gly Thr Pro Gly Gln
1               5                   10                  15

Arg Val Thr Ile Ser Cys Ser Gly Ser Ser Ser Asn Ile Gly Ser Asn
            20                  25                  30

Ile Val Asn Trp Tyr Gln Gln Phe Pro Gly Thr Ala Pro Lys Leu Leu
        35                  40                  45

Ile Tyr Ser Asn Asn Gln Arg Pro Ser Gly Val Pro Asp Arg Val Ser
    50                  55                  60

Gly Ser Lys Ser Gly Thr Ser Ala Ser Leu Ala Ile Ser Gly Leu Gln
65                  70                  75                  80

Ser Glu Asp Glu Ala Asp Tyr Tyr Cys Ala Ala Trp Asp Asp Ser Leu
                85                  90                  95

Asn Gly Pro Val Phe Gly Pro Gly Thr Lys Val Thr Val Leu
            100                 105                 110

<210>   86
<211>   107
<212>   PRT
<213>   Artificial Sequence

<220>
<223>   Description of Artificial Sequence: Synthetic peptide
```

<400> 86
Asp Ile Gln Leu Thr Gln Ser Pro Ser Phe Leu Ser Ala Ser Val Gly
1 5 10 15

Asp Arg Val Thr Ile Thr Cys Arg Ala Ser Gln Gly Ile Ser Ser Tyr
20 25 30

Leu Ala Trp Tyr Gln Gln Lys Pro Gly Lys Ala Pro Lys Val Leu Ile
35 40 45

Tyr Ala Ala Ser Thr Leu Gln Ser Gly Val Pro Ser Arg Phe Ser Gly
50 55 60

Ser Gly Ser Gly Thr Glu Phe Thr Leu Thr Ile Ser Ser Leu Gln Pro
65 70 75 80

Glu Asp Phe Ala Thr Tyr Phe Cys Gln Gln Leu Asn Ser Tyr Pro Val
85 90 95

Thr Phe Gly Gly Gly Thr Lys Val Glu Ile Lys
100 105

<210> 87
<211> 112
<212> PRT
<213> Artificial Sequence

<220>
<223> Description of Artificial Sequence: Synthetic peptide

<400> 87
Asp Ile Val Met Thr Gln Ser Pro Leu Ser Leu Pro Val Thr Pro Gly
1 5 10 15

Glu Pro Ala Ser Ile Ser Cys Lys Ser Ser Gln Ser Leu Leu Phe Ser
20 25 30

Asn Gly Tyr Lys Phe Leu Asp Trp Tyr Leu Gln Arg Pro Gly Gln Ser
35 40 45

Pro Gln Leu Leu Ile Tyr Leu Gly Ser Tyr Arg Ala Ser Gly Val Pro
50 55 60

Asp Arg Phe Ser Gly Ser Gly Ser Gly Thr Asp Phe Thr Leu Lys Ile
65 70 75 80

Ser Arg Val Glu Ala Glu Asp Val Gly Leu Tyr Tyr Cys Met Gln Ala
85 90 95

Leu Gln Thr Pro Pro Thr Phe Gly Gly Gly Thr Lys Val Glu Ile Lys
100 105 110

<210> 88
<211> 112

<212> PRT
<213> Artificial Sequence

<220>
<223> Description of Artificial Sequence: Synthetic peptide

<400> 88
Asp Ile Val Met Thr Gln Ser Pro Leu Ser Leu Pro Val Thr Pro Gly
1 5 10 15

Glu Pro Ala Ser Ile Ser Cys Lys Ser Ser Gln Ser Leu Leu Phe Ser
20 25 30

Asn Gly Tyr Lys Phe Leu Asp Trp Tyr Leu Gln Arg Pro Gly Gln Ser
35 40 45

Pro Gln Leu Leu Ile Tyr Leu Gly Ser Tyr Arg Ala Ser Gly Val Pro
50 55 60

Asp Arg Phe Ser Gly Ser Gly Ser Gly Thr Asp Phe Thr Leu Lys Ile
65 70 75 80

Ser Arg Val Glu Ala Glu Asp Val Gly Leu Tyr Tyr Cys Met Gln Ala
85 90 95

Leu Gln Thr Pro Pro Thr Phe Gly Gln Gly Thr Lys Val Glu Ile Lys
100 105 110

<210> 89
<211> 5
<212> PRT
<213> Artificial Sequence

<220>
<223> Description of Artificial Sequence: Synthetic peptide

<400> 89
Ser Tyr Gly Met His
1 5

<210> 90
<211> 17
<212> PRT
<213> Artificial Sequence

<220>
<223> Description of Artificial Sequence: Synthetic peptide

<400> 90
Val Ile Ser Tyr Asp Gly Ser Asn Lys Tyr Tyr Ala Asp Ser Val Lys
1 5 10 15

Gly

<210> 91
<211> 17
<212> PRT
<213> Artificial Sequence

<220>
<223> Description of Artificial Sequence: Synthetic peptide

<400> 91
Asn Ile Lys Gln His Gly Ser Glu Lys Tyr Tyr Val Asp Ser Val Lys
1 5 10 15

Gly

<210> 92
<211> 20
<212> PRT
<213> Artificial Sequence

<220>
<223> Description of Artificial Sequence: Synthetic peptide

<400> 92
Asp Phe Arg Ser Leu Asp Trp Leu Pro Pro Asp Ser Thr Ser Tyr Asp
1 5 10 15

Gly Met Asp Val
20

<210> 93
<211> 20
<212> PRT
<213> Artificial Sequence

<220>
<223> Description of Artificial Sequence: Synthetic peptide

<400> 93
Asp Phe Arg Ser Phe Asp Trp Leu Pro Pro Asp Ser Thr Ser Tyr Tyr
1 5 10 15

Gly Met Asp Val
20

<210> 94
<211> 9
<212> PRT
<213> Artificial Sequence

<220>
<223> Description of Artificial Sequence: Synthetic peptide

<400> 94
Asp Arg Asp Leu Gly Tyr Phe Asp Tyr
1 5

<210> 95
<211> 129
<212> PRT
<213> Artificial Sequence

<220>
<223> Description of Artificial Sequence: Synthetic peptide

<400> 95
Gln Val Gln Leu Val Glu Ser Gly Gly Gly Val Val Gln Pro Gly Arg
1 5 10 15

Ser Leu Arg Leu Ser Cys Ala Ala Ser Gly Phe Thr Phe Ser Ser Tyr
20 25 30

Gly Met His Trp Val Arg Gln Ser Pro Gly Lys Gly Leu Glu Trp Val
35 40 45

Ala Val Ile Ser Tyr Asp Gly Ser Asn Lys Tyr Tyr Ala Asp Ser Val
50 55 60

Lys Gly Arg Phe Thr Ile Ser Arg Asp Asn Ser Lys Ser Thr Leu Tyr
65 70 75 80

Leu Gln Met Asn Ser Leu Arg Ala Glu Asp Thr Ala Val Tyr Tyr Cys
85 90 95

Ala Lys Asp Phe Arg Ser Leu Asp Trp Leu Pro Pro Asp Ser Thr Ser
100 105 110

Tyr Asp Gly Met Asp Val Trp Gly Gln Gly Thr Thr Val Thr Val Ser
115 120 125

Ser

<210> 96
<211> 129
<212> PRT
<213> Artificial Sequence

<220>
<223> Description of Artificial Sequence: Synthetic peptide

<400> 96
Gln Val Gln Leu Val Glu Ser Gly Gly Gly Val Val Gln Pro Gly Arg
1 5 10 15

Ser Leu Arg Leu Ser Cys Ala Ala Ser Gly Phe Thr Phe Ser Ser Tyr
20 25 30

Gly Met His Trp Val Arg Gln Ala Pro Gly Lys Gly Leu Glu Trp Val
35 40 45

Ala Val Ile Ser Tyr Asp Gly Ser Asn Lys Tyr Tyr Ala Asp Ser Val
50 55 60

Lys Gly Arg Phe Thr Ile Ser Arg Asp Asn Ser Lys Asn Thr Leu Tyr
65 70 75 80

Leu Gln Met Asn Ser Leu Arg Ala Glu Asp Thr Ala Val Tyr Tyr Cys
85 90 95

Ala Lys Asp Phe Arg Ser Phe Asp Trp Leu Pro Pro Asp Ser Thr Ser
100 105 110

Tyr Tyr Gly Met Asp Val Trp Gly Gln Gly Thr Thr Val Thr Val Ser
115 120 125

Ser

<210> 97
<211> 118
<212> PRT
<213> Artificial Sequence

<220>
<223> Description of Artificial Sequence: Synthetic peptide

<400> 97
Glu Val Gln Leu Val Glu Ser Gly Gly Gly Phe Val Gln Pro Gly Gly
1 5 10 15

Ser Leu Arg Leu Ser Cys Ala Ala Ser Gly Phe Thr Phe Ser Ser Tyr
20 25 30

Trp Met Ser Trp Val Arg Gln Ala Pro Gly Lys Gly Leu Glu Trp Val
35 40 45

Ala Asn Ile Lys Gln His Gly Ser Glu Lys Tyr Tyr Val Asp Ser Val
50 55 60

Lys Gly Arg Phe Thr Ile Ser Arg Asp Asn Ala Lys Asn Ser Leu Tyr
65 70 75 80

Leu Gln Met Asn Ser Leu Arg Ala Glu Asp Thr Ala Val Tyr Tyr Cys
85 90 95

Ala Arg Asp Arg Asp Leu Gly Tyr Phe Asp Tyr Trp Gly Gln Gly Thr
100 105 110

Leu Val Thr Val Ser Ser
115

<210> 98
<211> 11
<212> PRT
<213> Artificial Sequence

<220>
<223> Description of Artificial Sequence: Synthetic peptide

<400> 98
Ser Gly His Lys Leu Gly Asp Lys Tyr Ala Ser
1 5 10

<210> 99
<211> 11
<212> PRT
<213> Artificial Sequence

<220>
<223> Description of Artificial Sequence: Synthetic peptide

<400> 99
Ser Gly Asp Lys Leu Gly Asp Lys Tyr Val Ser
1 5 10

<210> 100
<211> 11
<212> PRT
<213> Artificial Sequence

<220>
<223> Description of Artificial Sequence: Synthetic peptide

<400> 100
Ser Gly Asp Lys Leu Gly Ser Lys Phe Ala Ser
1 5 10

<210> 101
<211> 7
<212> PRT
<213> Artificial Sequence

<220>
<223> Description of Artificial Sequence: Synthetic peptide

<400> 101
Lys Asp Ser Lys Arg Pro Ser
1 5

<210> 102
<211> 7
<212> PRT
<213> Artificial Sequence

<220>
<223> Description of Artificial Sequence: Synthetic peptide

<400> 102
Gln Asp Arg Lys Arg Pro Ser
1 5

<210> 103
<211> 7
<212> PRT
<213> Artificial Sequence

<220>
<223> Description of Artificial Sequence: Synthetic peptide

<400> 103
Gln Asp Ser Lys Arg Pro Ser
1 5

<210> 104
<211> 9
<212> PRT
<213> Artificial Sequence

<220>
<223> Description of Artificial Sequence: Synthetic peptide

<400> 104
Gln Ala Trp Asp Ser Ser Thr Val Val
1 5

<210> 105
<211> 106
<212> PRT
<213> Artificial Sequence

<220>
<223> Description of Artificial Sequence: Synthetic peptide

<400> 105
Ser Tyr Glu Leu Thr Gln Pro Pro Ser Val Ser Val Ser Pro Gly Gln
1 5 10 15

Thr Ala Ser Ile Thr Cys Ser Gly His Lys Leu Gly Asp Lys Tyr Ala
20 25 30

Ser Trp Tyr Gln Gln Lys Pro Gly Gln Ser Pro Val Val Val Ile Tyr
35 40 45

Lys Asp Ser Lys Arg Pro Ser Gly Ile Pro Glu Arg Phe Ser Gly Ser
50 55 60

Asn Phe Gly Asn Thr Ala Thr Leu Thr Ile Ser Gly Thr Gln Ala Met
65 70 75 80

```
Asp Glu Ala Asp Tyr Tyr Cys Gln Ala Trp Asp Ser Ser Thr Val Val
                85                  90                  95

Phe Gly Gly Gly Thr Lys Leu Thr Val Leu
            100                 105

<210>   106
<211>   106
<212>   PRT
<213>   Artificial Sequence

<220>
<223>   Description of Artificial Sequence: Synthetic peptide

<400>   106
Ser Tyr Glu Leu Thr Gln Pro Pro Ser Val Ser Val Ser Pro Gly Gln
1               5                   10                  15

Thr Ala Ser Ile Thr Cys Ser Gly Asp Lys Leu Gly Asp Lys Tyr Val
            20                  25                  30

Ser Trp Tyr Gln Gln Lys Pro Gly Gln Ser Pro Val Val Val Ile His
        35                  40                  45

Gln Asp Arg Lys Arg Pro Ser Gly Ile Pro Glu Arg Phe Ser Gly Ser
    50                  55                  60

Asn Phe Gly Asn Thr Ala Thr Leu Thr Ile Ser Gly Thr Gln Ala Met
65                  70                  75                  80

Asp Glu Ala Asp Tyr Tyr Cys Gln Ala Trp Asp Ser Ser Thr Val Val
                85                  90                  95

Phe Gly Gly Gly Thr Lys Leu Thr Val Leu
            100                 105

<210>   107
<211>   106
<212>   PRT
<213>   Artificial Sequence

<220>
<223>   Description of Artificial Sequence: Synthetic peptide

<400>   107
Ser Tyr Glu Leu Thr Gln Pro Pro Ser Val Ser Val Ser Pro Gly Gln
1               5                   10                  15

Thr Ala Ser Ile Thr Cys Ser Gly Asp Lys Leu Gly Ser Lys Phe Ala
            20                  25                  30

Ser Trp Tyr Gln Gln Lys Pro Gly Gln Ser Pro Val Leu Val Ile Tyr
        35                  40                  45
```

```
Gln Asp Ser Lys Arg Pro Ser Gly Ile Pro Glu Arg Phe Ser Gly Ser
    50                  55                  60

Asn Ser Gly Asn Thr Ala Thr Leu Thr Ile Ser Gly Thr Gln Ala Met
65                  70                  75                  80

Asp Glu Ala Asp Tyr Tyr Cys Gln Ala Trp Asp Ser Ser Thr Val Val
                85                  90                  95

Phe Gly Gly Gly Thr Lys Leu Thr Val Leu
            100                 105

<210>   108
<211>   20
<212>   PRT
<213>   Artificial Sequence

<220>
<223>   Description of Artificial Sequence: Synthetic peptide

<400>   108
Gly Gly Ser Glu Gly Lys Ser Ser Gly Ser Gly Ser Glu Ser Lys Ser
1               5                   10                  15

Thr Gly Gly Ser
            20

<210>   109
<211>   8
<212>   PRT
<213>   Artificial Sequence

<220>
<223>   Description of Artificial Sequence: Synthetic peptide

<400>   109
Gly Gly Gly Ser Gly Gly Gly Ser
1               5

<210>   110
<211>   12
<212>   PRT
<213>   Artificial Sequence

<220>
<223>   Description of Artificial Sequence: Synthetic peptide

<400>   110
Gly Gly Gly Ser Gly Gly Gly Ser Gly Gly Gly Ser
1               5                   10

<210>   111
<211>   16
```

<212> PRT
<213> Artificial Sequence

<220>
<223> Description of Artificial Sequence: Synthetic peptide

<400> 111
Gly Gly Gly Ser Gly Gly Gly Ser Gly Gly Gly Ser Gly Gly Gly Ser
1 5 10 15

<210> 112
<211> 20
<212> PRT
<213> Artificial Sequence

<220>
<223> Description of Artificial Sequence: Synthetic peptide

<400> 112
Gly Gly Gly Ser Gly Gly Gly Ser Gly Gly Gly Ser Gly Gly Gly Ser
1 5 10 15

Gly Gly Gly Ser
20

<210> 113
<211> 15
<212> PRT
<213> Artificial Sequence

<220>
<223> Description of Artificial Sequence: Synthetic peptide

<400> 113
Gly Gly Gly Gly Ser Gly Gly Gly Gly Ser Gly Gly Gly Gly Ser
1 5 10 15

<210> 114
<211> 20
<212> PRT
<213> Artificial Sequence

<220>
<223> Description of Artificial Sequence: Synthetic peptide

<400> 114
Gly Gly Gly Gly Ser Gly Gly Gly Gly Ser Gly Gly Gly Gly Ser Gly
1 5 10 15

Gly Gly Gly Ser
20

<210> 115
<211> 25

<212> PRT
<213> Artificial Sequence

<220>
<223> Description of Artificial Sequence: Synthetic peptide

<400> 115
Gly Gly Gly Gly Ser Gly Gly Gly Gly Ser Gly Gly Gly Gly Ser Gly
1 5 10 15

Gly Gly Gly Ser Gly Gly Gly Gly Ser
20 25

<210> 116
<211> 18
<212> PRT
<213> Artificial Sequence

<220>
<223> Description of Artificial Sequence: Synthetic peptide

<400> 116
Gly Ser Thr Ser Gly Ser Gly Lys Pro Gly Ser Gly Glu Gly Ser Thr
1 5 10 15

Lys Gly

<210> 117
<211> 14
<212> PRT
<213> Artificial Sequence

<220>
<223> Description of Artificial Sequence: Synthetic peptide

<400> 117
Ile Arg Pro Arg Ala Ile Gly Gly Ser Lys Pro Arg Val Ala
1 5 10

<210> 118
<211> 15
<212> PRT
<213> Artificial Sequence

<220>
<223> Description of Artificial Sequence: Synthetic peptide

<400> 118
Gly Lys Gly Gly Ser Gly Lys Gly Gly Ser Gly Lys Gly Gly Ser
1 5 10 15

<210> 119
<211> 15

<212> PRT
<213> Artificial Sequence

<220>
<223> Description of Artificial Sequence: Synthetic peptide

<400> 119
Gly Gly Lys Gly Ser Gly Gly Lys Gly Ser Gly Gly Lys Gly Ser
1 5 10 15

<210> 120
<211> 15
<212> PRT
<213> Artificial Sequence

<220>
<223> Description of Artificial Sequence: Synthetic peptide

<400> 120
Gly Gly Gly Lys Ser Gly Gly Gly Lys Ser Gly Gly Gly Lys Ser
1 5 10 15

<210> 121
<211> 15
<212> PRT
<213> Artificial Sequence

<220>
<223> Description of Artificial Sequence: Synthetic peptide

<400> 121
Gly Lys Gly Lys Ser Gly Lys Gly Lys Ser Gly Lys Gly Lys Ser
1 5 10 15

<210> 122
<211> 15
<212> PRT
<213> Artificial Sequence

<220>
<223> Description of Artificial Sequence: Synthetic peptide

<400> 122
Gly Gly Gly Lys Ser Gly Gly Lys Gly Ser Gly Lys Gly Gly Ser
1 5 10 15

<210> 123
<211> 15
<212> PRT
<213> Artificial Sequence

<220>
<223> Description of Artificial Sequence: Synthetic peptide

<400> 123
Gly Lys Pro Gly Ser Gly Lys Pro Gly Ser Gly Lys Pro Gly Ser
1 5 10 15

<210> 124
<211> 20
<212> PRT
<213> Artificial Sequence

<220>
<223> Description of Artificial Sequence: Synthetic peptide

<400> 124
Gly Lys Pro Gly Ser Gly Lys Pro Gly Ser Gly Lys Pro Gly Ser Gly
1 5 10 15

Lys Pro Gly Ser
20

<210> 125
<211> 20
<212> PRT
<213> Artificial Sequence

<220>
<223> Description of Artificial Sequence: Synthetic peptide

<400> 125
Gly Lys Gly Lys Ser Gly Lys Gly Lys Ser Gly Lys Gly Lys Ser Gly
1 5 10 15

Lys Gly Lys Ser
20

<210> 126
<211> 14
<212> PRT
<213> Artificial Sequence

<220>
<223> Description of Artificial Sequence: Synthetic peptide

<400> 126
Ser Thr Ala Gly Asp Thr His Leu Gly Gly Glu Asp Phe Asp
1 5 10

<210> 127
<211> 15
<212> PRT
<213> Artificial Sequence

<220>
<223> Description of Artificial Sequence: Synthetic peptide

<400> 127
Gly Glu Gly Gly Ser Gly Glu Gly Gly Ser Gly Glu Gly Gly Ser
1 5 10 15

<210> 128
<211> 15
<212> PRT
<213> Artificial Sequence

<220>
<223> Description of Artificial Sequence: Synthetic peptide

<400> 128
Gly Gly Glu Gly Ser Gly Gly Glu Gly Ser Gly Gly Glu Gly Ser
1 5 10 15

<210> 129
<211> 15
<212> PRT
<213> Artificial Sequence

<220>
<223> Description of Artificial Sequence: Synthetic peptide

<400> 129
Gly Glu Gly Glu Ser Gly Glu Gly Glu Ser Gly Glu Gly Glu Ser
1 5 10 15

<210> 130
<211> 15
<212> PRT
<213> Artificial Sequence

<220>
<223> Description of Artificial Sequence: Synthetic peptide

<400> 130
Gly Gly Gly Glu Ser Gly Gly Glu Gly Ser Gly Glu Gly Gly Ser
1 5 10 15

<210> 131
<211> 20
<212> PRT
<213> Artificial Sequence

<220>
<223> Description of Artificial Sequence: Synthetic peptide

<400> 131
Gly Glu Gly Glu Ser Gly Glu Gly Glu Ser Gly Glu Gly Glu Ser Gly
1 5 10 15

Glu Gly Glu Ser
20

<210> 132
<211> 18
<212> PRT
<213> Artificial Sequence

<220>
<223> Description of Artificial Sequence: Synthetic peptide

<400> 132
Gly Ser Thr Ser Gly Ser Gly Lys Pro Gly Ser Gly Glu Gly Ser Thr
1 5 10 15

Lys Gly

<210> 133
<211> 19
<212> PRT
<213> Artificial Sequence

<220>
<223> Description of Artificial Sequence: Synthetic peptide

<400> 133
Pro Arg Gly Ala Ser Lys Ser Gly Ser Ala Ser Gln Thr Gly Ser Ala
1 5 10 15

Pro Gly Ser

<210> 134
<211> 19
<212> PRT
<213> Artificial Sequence

<220>
<223> Description of Artificial Sequence: Synthetic peptide

<400> 134
Gly Thr Ala Ala Ala Gly Ala Gly Ala Ala Gly Gly Ala Ala Ala Gly
1 5 10 15

Ala Ala Gly

<210> 135
<211> 19
<212> PRT
<213> Artificial Sequence

<220>
<223> Description of Artificial Sequence: Synthetic peptide

```
<400>   135
Gly Thr Ser Gly Ser Ser Gly Ser Gly Ser Gly Gly Ser Gly Ser Gly
1               5                   10                  15

Gly Gly Gly


<210>   136
<211>   20
<212>   PRT
<213>   Artificial Sequence

<220>
<223>   Description of Artificial Sequence: Synthetic peptide

<400>   136
Gly Lys Pro Gly Ser Gly Lys Pro Gly Ser Gly Lys Pro Gly Ser Gly
1               5                   10                  15

Lys Pro Gly Ser
            20

<210>   137
<211>   4
<212>   PRT
<213>   Artificial Sequence

<220>
<223>   Description of Artificial Sequence: Synthetic peptide

<400>   137
Gly Ser Gly Ser
1

<210>   138
<211>   10
<212>   PRT
<213>   Artificial Sequence

<220>
<223>   Description of Artificial Sequence: Synthetic peptide

<400>   138
Ala Pro Ala Pro Ala Pro Ala Pro Ala Pro
1               5                   10

<210>   139
<211>   20
<212>   PRT
<213>   Artificial Sequence

<220>
<223>   Description of Artificial Sequence: Synthetic peptide
```

<400> 139
Ala Pro Ala Pro Ala Pro Ala Pro Ala Pro Ala Pro Ala Pro Ala Pro
1 5 10 15

Ala Pro Ala Pro
20

<210> 140
<211> 32
<212> PRT
<213> Artificial Sequence

<220>
<223> Description of Artificial Sequence: Synthetic peptide

<400> 140
Ala Glu Ala Ala Ala Lys Glu Ala Ala Ala Lys Glu Ala Ala Ala Ala
1 5 10 15

Lys Glu Ala Ala Ala Ala Lys Glu Ala Ala Ala Ala Lys Ala Ala Ala
20 25 30

<210> 141
<211> 5
<212> PRT
<213> Artificial Sequence

<220>
<223> Description of Artificial Sequence: Synthetic peptide

<400> 141
Thr Tyr Ala Met Asn
1 5

<210> 142
<211> 19
<212> PRT
<213> Artificial Sequence

<220>
<223> Description of Artificial Sequence: Synthetic peptide

<400> 142
Arg Ile Arg Ser Lys Tyr Asn Asn Tyr Ala Thr Tyr Tyr Ala Ala Ser
1 5 10 15

Val Lys Gly

<210> 143
<211> 14
<212> PRT
<213> Artificial Sequence

<220>
<223> Description of Artificial Sequence: Synthetic peptide

<400> 143
His Gly Asn Phe Gly Asn Ser Tyr Val Ser Trp Phe Ala Tyr
1 5 10

<210> 144
<211> 14
<212> PRT
<213> Artificial Sequence

<220>
<223> Description of Artificial Sequence: Synthetic peptide

<400> 144
Arg Ser Ser Thr Gly Ala Val Thr Thr Ser Asn Tyr Ala Asn
1 5 10

<210> 145
<211> 7
<212> PRT
<213> Artificial Sequence

<220>
<223> Description of Artificial Sequence: Synthetic peptide

<400> 145
Gly Thr Asn Lys Arg Ala Pro
1 5

<210> 146
<211> 9
<212> PRT
<213> Artificial Sequence

<220>
<223> Description of Artificial Sequence: Synthetic peptide

<400> 146
Ala Leu Trp Tyr Ser Asn Leu Trp Val
1 5

<210> 147
<211> 125
<212> PRT
<213> Artificial Sequence

<220>
<223> Description of Artificial Sequence: Synthetic peptide

<400> 147
Glu Val Gln Leu Val Glu Ser Gly Gly Gly Leu Val Gln Pro Gly Gly
1 5 10 15

Ser Leu Arg Leu Ser Cys Ala Ala Ser Gly Phe Thr Phe Asn Thr Tyr
20 25 30

Ala Met Asn Trp Val Arg Gln Ala Pro Gly Lys Gly Leu Glu Trp Val
35 40 45

Ala Arg Ile Arg Ser Lys Tyr Asn Asn Tyr Ala Thr Tyr Tyr Ala Ala
50 55 60

Ser Val Lys Gly Arg Phe Thr Ile Ser Arg Asp Asp Ser Lys Asn Ser
65 70 75 80

Leu Tyr Leu Gln Met Asn Ser Leu Lys Thr Glu Asp Thr Ala Val Tyr
85 90 95

Tyr Cys Ala Arg His Gly Asn Phe Gly Asn Ser Tyr Val Ser Trp Phe
100 105 110

Ala Tyr Trp Gly Gln Gly Thr Leu Val Thr Val Ser Ser
115 120 125

<210> 148
<211> 109
<212> PRT
<213> Artificial Sequence

<220>
<223> Description of Artificial Sequence: Synthetic peptide

<400> 148
Gln Thr Val Val Thr Gln Glu Pro Ser Leu Thr Val Ser Pro Gly Gly
1 5 10 15

Thr Val Thr Leu Thr Cys Arg Ser Ser Thr Gly Ala Val Thr Thr Ser
20 25 30

Asn Tyr Ala Asn Trp Val Gln Gln Lys Pro Gly Gln Ala Pro Arg Gly
35 40 45

Leu Ile Gly Gly Thr Asn Lys Arg Ala Pro Gly Thr Pro Ala Arg Phe
50 55 60

Ser Gly Ser Leu Leu Gly Gly Lys Ala Ala Leu Thr Leu Ser Gly Val
65 70 75 80

Gln Pro Glu Asp Glu Ala Glu Tyr Tyr Cys Ala Leu Trp Tyr Ser Asn
85 90 95

Leu Trp Val Phe Gly Gly Gly Thr Lys Leu Thr Val Leu
100 105

<210> 149
<211> 7

<212> PRT
<213> Artificial Sequence

<220>
<223> Description of Artificial Sequence: Synthetic peptide

<400> 149
Asn Asn Asn Ala Ala Trp Ser
1 5

<210> 150
<211> 18
<212> PRT
<213> Artificial Sequence

<220>
<223> Description of Artificial Sequence: Synthetic peptide

<400> 150
Arg Thr Tyr Tyr Arg Ser Lys Trp Leu Tyr Asp Tyr Ala Val Ser Val
1 5 10 15

Lys Ser

<210> 151
<211> 8
<212> PRT
<213> Artificial Sequence

<220>
<223> Description of Artificial Sequence: Synthetic peptide

<400> 151
Gly Tyr Ser Ser Ser Phe Asp Tyr
1 5

<210> 152
<211> 14
<212> PRT
<213> Artificial Sequence

<220>
<223> Description of Artificial Sequence: Synthetic peptide

<400> 152
Thr Gly Thr Ser Ser Asn Ile Gly Thr Tyr Lys Phe Val Ser
1 5 10

<210> 153
<211> 7
<212> PRT
<213> Artificial Sequence

<220>
<223> Description of Artificial Sequence: Synthetic peptide

<400> 153
Glu Val Ser Lys Arg Pro Ser
1 5

<210> 154
<211> 10
<212> PRT
<213> Artificial Sequence

<220>
<223> Description of Artificial Sequence: Synthetic peptide

<400> 154
Val Ser Tyr Ala Gly Ser Gly Thr Leu Leu
1 5 10

<210> 155
<211> 120
<212> PRT
<213> Artificial Sequence

<220>
<223> Description of Artificial Sequence: Synthetic peptide

<400> 155
Gln Val Gln Leu Gln Gln Ser Gly Pro Arg Leu Val Arg Pro Ser Gln
1 5 10 15

Thr Leu Ser Leu Thr Cys Ala Ile Ser Gly Asp Ser Val Phe Asn Asn
20 25 30

Asn Ala Ala Trp Ser Trp Ile Arg Gln Ser Pro Ser Arg Gly Leu Glu
35 40 45

Trp Leu Gly Arg Thr Tyr Tyr Arg Ser Lys Trp Leu Tyr Asp Tyr Ala
50 55 60

Val Ser Val Lys Ser Arg Ile Thr Val Asn Pro Asp Thr Ser Arg Asn
65 70 75 80

Gln Phe Thr Leu Gln Leu Asn Ser Val Thr Pro Glu Asp Thr Ala Leu
85 90 95

Tyr Tyr Cys Ala Arg Gly Tyr Ser Ser Ser Phe Asp Tyr Trp Gly Gln
100 105 110

Gly Thr Leu Val Thr Val Ser Ser
115 120

<210> 156
<211> 110

<212> PRT
<213> Artificial Sequence

<220>
<223> Description of Artificial Sequence: Synthetic peptide

<400> 156
Gln Ser Ala Leu Thr Gln Pro Ala Ser Val Ser Gly Ser Pro Gly Gln
1 5 10 15

Ser Ile Thr Ile Ser Cys Thr Gly Thr Ser Ser Asn Ile Gly Thr Tyr
20 25 30

Lys Phe Val Ser Trp Tyr Gln Gln His Pro Asp Lys Ala Pro Lys Val
35 40 45

Leu Leu Tyr Glu Val Ser Lys Arg Pro Ser Gly Val Ser Ser Arg Phe
50 55 60

Ser Gly Ser Lys Ser Gly Asn Thr Ala Ser Leu Thr Ile Ser Gly Leu
65 70 75 80

Gln Ala Glu Asp Gln Ala Asp Tyr His Cys Val Ser Tyr Ala Gly Ser
85 90 95

Gly Thr Leu Leu Phe Gly Gly Gly Thr Lys Leu Thr Val Leu
100 105 110

<210> 157
<211> 45
<212> PRT
<213> Artificial Sequence

<220>
<223> Description of Artificial Sequence: Synthetic peptide

<400> 157
Thr Ser Thr Pro Ala Pro Arg Pro Pro Thr Pro Ala Pro Thr Ile Ala
1 5 10 15

Ser Gln Pro Leu Ser Leu Arg Pro Glu Ala Cys Arg Pro Ala Ala Gly
20 25 30

Gly Ala Val His Thr Arg Gly Leu Asp Phe Ala Cys Asp
35 40 45

<210> 158
<211> 15
<212> PRT
<213> Artificial Sequence

<220>
<223> Description of Artificial Sequence: Synthetic peptide

<400> 158
Glu Pro Lys Ser Cys Asp Lys Thr His Thr Cys Pro Pro Cys Pro
1 5 10 15

<210> 159
<211> 12
<212> PRT
<213> Artificial Sequence

<220>
<223> Description of Artificial Sequence: Synthetic peptide

<400> 159
Glu Arg Lys Cys Cys Val Glu Cys Pro Pro Cys Pro
1 5 10

<210> 160
<211> 32
<212> PRT
<213> Artificial Sequence

<220>
<223> Description of Artificial Sequence: Synthetic peptide

<400> 160
Glu Leu Lys Thr Pro Leu Gly Asp Thr Thr His Thr Cys Pro Arg Cys
1 5 10 15

Pro Glu Pro Lys Ser Cys Asp Thr Pro Pro Pro Cys Pro Arg Cys Pro
20 25 30

<210> 161
<211> 12
<212> PRT
<213> Artificial Sequence

<220>
<223> Description of Artificial Sequence: Synthetic peptide

<400> 161
Glu Ser Lys Tyr Gly Pro Pro Cys Pro Ser Cys Pro
1 5 10

<210> 162
<211> 24
<212> PRT
<213> Artificial Sequence

<220>
<223> Description of Artificial Sequence: Synthetic peptide

<400> 162
Ile Tyr Ile Trp Ala Pro Leu Ala Gly Thr Cys Gly Val Leu Leu Leu
1 5 10 15

Ser Leu Val Ile Thr Leu Tyr Cys
20

<210> 163
<211> 42
<212> PRT
<213> Artificial Sequence

<220>
<223> Description of Artificial Sequence: Synthetic peptide

<400> 163
Lys Arg Gly Arg Lys Lys Leu Leu Tyr Ile Phe Lys Gln Pro Phe Met
1 5 10 15

Arg Pro Val Gln Thr Thr Gln Glu Glu Asp Gly Cys Ser Cys Arg Phe
20 25 30

Pro Glu Glu Glu Glu Gly Gly Cys Glu Leu
35 40

<210> 164
<211> 112
<212> PRT
<213> Artificial Sequence

<220>
<223> Description of Artificial Sequence: Synthetic peptide

<400> 164
Arg Val Lys Phe Ser Arg Ser Ala Asp Ala Pro Ala Tyr Lys Gln Gly
1 5 10 15

Gln Asn Gln Leu Tyr Asn Glu Leu Asn Leu Gly Arg Arg Glu Glu Tyr
20 25 30

Asp Val Leu Asp Lys Arg Arg Gly Arg Asp Pro Glu Met Gly Gly Lys
35 40 45

Pro Arg Arg Lys Asn Pro Gln Glu Gly Leu Tyr Asn Glu Leu Gln Lys
50 55 60

Asp Lys Met Ala Glu Ala Tyr Ser Glu Ile Gly Met Lys Gly Glu Arg
65 70 75 80

Arg Arg Gly Lys Gly His Asp Gly Leu Tyr Gln Gly Leu Ser Thr Ala
85 90 95

Thr Lys Asp Thr Tyr Asp Ala Leu His Met Gln Ala Leu Pro Pro Arg
100 105 110

<210> 165
<211> 154

<212> PRT
<213> Artificial Sequence

<220>
<223> Description of Artificial Sequence: Synthetic peptide

<400> 165
```
Lys Arg Gly Arg Lys Lys Leu Leu Tyr Ile Phe Lys Gln Pro Phe Met
1               5                   10                  15

Arg Pro Val Gln Thr Thr Gln Glu Glu Asp Gly Cys Ser Cys Arg Phe
            20                  25                  30

Pro Glu Glu Glu Glu Gly Gly Cys Glu Leu Arg Val Lys Phe Ser Arg
        35                  40                  45

Ser Ala Asp Ala Pro Ala Tyr Lys Gln Gly Gln Asn Gln Leu Tyr Asn
    50                  55                  60

Glu Leu Asn Leu Gly Arg Arg Glu Glu Tyr Asp Val Leu Asp Lys Arg
65                  70                  75                  80

Arg Gly Arg Asp Pro Glu Met Gly Gly Lys Pro Arg Arg Lys Asn Pro
                85                  90                  95

Gln Glu Gly Leu Tyr Asn Glu Leu Gln Lys Asp Lys Met Ala Glu Ala
            100                 105                 110

Tyr Ser Glu Ile Gly Met Lys Gly Glu Arg Arg Arg Gly Lys Gly His
        115                 120                 125

Asp Gly Leu Tyr Gln Gly Leu Ser Thr Ala Thr Lys Asp Thr Tyr Asp
    130                 135                 140

Ala Leu His Met Gln Ala Leu Pro Pro Arg
145                 150
```

<210> 166
<211> 12
<212> PRT
<213> Artificial Sequence

<220>
<223> Description of Artificial Sequence: Synthetic peptide

<400> 166
```
Glu Ser Lys Tyr Gly Pro Pro Cys Pro Ser Cys Pro
1               5                   10
```

<210> 167
<211> 5
<212> PRT
<213> Artificial Sequence

<220>
<223>   Description of Artificial Sequence: Synthetic peptide

<400>   167
Asn Tyr Ala Met Asn
1               5

<210>   168
<211>   17
<212>   PRT
<213>   Artificial Sequence

<220>
<223>   Description of Artificial Sequence: Synthetic peptide

<400>   168
Trp Ile Asn Thr Asn Thr Gly Asn Pro Thr Tyr Ala Gln Gly Phe Thr
1               5                   10                  15

Gly

<210>   169
<211>   8
<212>   PRT
<213>   Artificial Sequence

<220>
<223>   Description of Artificial Sequence: Synthetic peptide

<400>   169
Asp Arg Asp Arg Gly Thr Asp Tyr
1               5

<210>   170
<211>   117
<212>   PRT
<213>   Artificial Sequence

<220>
<223>   Description of Artificial Sequence: Synthetic peptide

<400>   170
Gln Val Gln Leu Val Gln Ser Gly Ser Glu Leu Arg Lys Pro Gly Ala
1               5                   10                  15

Ser Val Lys Val Ser Cys Lys Ala Ser Gly Tyr Thr Phe Thr Asn Tyr
            20                  25                  30

Ala Met Asn Trp Val Arg Gln Ala Pro Gly Gln Gly Leu Glu Trp Met
        35                  40                  45

Gly Trp Ile Asn Thr Asn Thr Gly Asn Pro Thr Tyr Ala Gln Gly Phe
    50                  55                  60

```
Thr Gly Arg Phe Val Phe Ser Leu Asp Thr Ser Val Ser Ser Ala Tyr
65                  70                  75                  80

Leu Gln Ile Ser Ser Leu Lys Ala Glu Asp Thr Ala Met Tyr Tyr Cys
                85                  90                  95

Ala Thr Asp Arg Asp Arg Gly Thr Asp Tyr Trp Gly Gln Gly Thr Leu
            100                 105                 110

Val Thr Val Ser Ser
        115

<210>   171
<211>   118
<212>   PRT
<213>   Artificial Sequence

<220>
<223>   Description of Artificial Sequence: Synthetic peptide

<400>   171
Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Trp Met Ser Trp Val Arg Gln Ala Pro Gly Lys Gly Leu Glu Trp Val
        35                  40                  45

Ala Asn Ile Lys Gln His Gly Ser Glu Lys Tyr Tyr Val Asp Ser Val
    50                  55                  60

Lys Gly Arg Phe Thr Ile Ser Arg Asp Asn Ala Lys Asn Ser Leu Tyr
65                  70                  75                  80

Leu Gln Met Asn Ser Leu Arg Ala Glu Asp Thr Ala Val Tyr Tyr Cys
                85                  90                  95

Ala Arg Asp Arg Asp Leu Gly Tyr Phe Asp Tyr Trp Gly Gln Gly Thr
            100                 105                 110

Leu Val Thr Val Ser Ser
        115

<210>   172
<211>   7
<212>   PRT
<213>   Artificial Sequence

<220>
<223>   Description of Artificial Sequence: Synthetic peptide
```

<400> 172
Ser Ser Ser Tyr Tyr Trp Gly
1 5

<210> 173
<211> 16
<212> PRT
<213> Artificial Sequence

<220>
<223> Description of Artificial Sequence: Synthetic peptide

<400> 173
Ser Ile Asn Tyr Ser Gly Ser Thr Tyr Tyr Asn Pro Ser Leu Lys Ser
1 5 10 15

<210> 174
<211> 10
<212> PRT
<213> Artificial Sequence

<220>
<223> Description of Artificial Sequence: Synthetic peptide

<400> 174
Leu Asp Gly Tyr Glu Ser Pro Phe Asp Tyr
1 5 10

<210> 175
<211> 120
<212> PRT
<213> Artificial Sequence

<220>
<223> Description of Artificial Sequence: Synthetic peptide

<400> 175
Gln Leu Gln Leu Gln Glu Ser Gly Pro Gly Leu Val Lys Pro Ser Glu
1 5 10 15

Thr Leu Ser Leu Thr Cys Thr Val Ser Gly Gly Ser Ile Ser Ser Ser
20 25 30

Ser Tyr Tyr Trp Gly Trp Ile Arg Gln Pro Pro Gly Lys Gly Leu Asp
35 40 45

Trp Ile Gly Ser Ile Asn Tyr Ser Gly Ser Thr Tyr Tyr Asn Pro Ser
50 55 60

Leu Lys Ser Arg Val Thr Ile Ser Val Asp Thr Ser Lys Ile Gln Phe
65 70 75 80

Ser Leu Lys Leu Arg Ser Val Thr Ala Ala Asp Thr Ala Val Tyr Tyr
85 90 95

Cys Ala Arg Leu Asp Gly Tyr Glu Ser Pro Phe Asp Tyr Trp Gly Gln
            100                 105                 110

Gly Thr Leu Val Thr Val Ser Ser
        115                 120

<210> 176
<211> 5
<212> PRT
<213> Artificial Sequence

<220>
<223> Description of Artificial Sequence: Synthetic peptide

<400> 176
Asp Tyr Ala Met His
1               5

<210> 177
<211> 17
<212> PRT
<213> Artificial Sequence

<220>
<223> Description of Artificial Sequence: Synthetic peptide

<400> 177
Gly Ile Gly Trp Ser Gly Gly Ser Ile Val Tyr Ala Asp Ser Val Lys
1               5                   10                  15

Gly

<210> 178
<211> 10
<212> PRT
<213> Artificial Sequence

<220>
<223> Description of Artificial Sequence: Synthetic peptide

<400> 178
Asp Ser Pro Tyr Gly Asp Phe Phe Asp Tyr
1               5                   10

<210> 179
<211> 119
<212> PRT
<213> Artificial Sequence

<220>
<223> Description of Artificial Sequence: Synthetic peptide

<400> 179
Glu Val Gln Leu Val Glu Ser Gly Gly Gly Leu Val Gln Pro Gly Arg
1 5 10 15

Ser Leu Arg Leu Ser Cys Ala Ala Ser Gly Phe Thr Phe Asp Asp Tyr
20 25 30

Ala Met His Trp Val Arg Gln Ala Pro Gly Lys Gly Leu Glu Trp Val
35 40 45

Ser Gly Ile Gly Trp Ser Gly Gly Ser Ile Val Tyr Ala Asp Ser Val
50 55 60

Lys Gly Arg Phe Thr Ile Ser Arg Asp Asn Ala Lys Asn Ser Leu Tyr
65 70 75 80

Leu Gln Met Asn Ser Leu Arg Ala Glu Asp Thr Ala Leu Tyr Tyr Cys
85 90 95

Ala Lys Asp Ser Pro Tyr Gly Asp Phe Phe Asp Tyr Trp Gly Gln Gly
100 105 110

Thr Leu Val Thr Val Ser Ser
115

<210> 180
<211> 20
<212> PRT
<213> Artificial Sequence

<220>
<223> Description of Artificial Sequence: Synthetic peptide

<400> 180
Asp Phe Arg Ser Phe Asp Trp Leu Pro Pro Asp Ser Ala Ser Tyr His
1 5 10 15

Gly Met Asp Val
20

<210> 181
<211> 129
<212> PRT
<213> Artificial Sequence

<220>
<223> Description of Artificial Sequence: Synthetic peptide

<400> 181
Gln Val Gln Leu Val Glu Ser Gly Gly Gly Val Val Gln Pro Gly Arg
1 5 10 15

Ser Leu Arg Leu Ser Cys Val Ala Ser Gly Phe Thr Phe Ser Ser Tyr
20 25 30

Gly Met His Trp Val Arg Gln Ala Pro Gly Lys Gly Leu Glu Trp Val
35 40 45

Ala Val Ile Ser Tyr Asp Gly Ser Asn Lys Tyr Tyr Ala Asp Ser Val
50 55 60

Lys Gly Arg Phe Thr Ile Ser Arg Asp Asn Ser Lys Asn Thr Leu Tyr
65 70 75 80

Leu Gln Met Asn Ser Leu Arg Ala Glu Gly Thr Ala Val Tyr Tyr Cys
85 90 95

Ala Lys Asp Phe Arg Ser Phe Asp Trp Leu Pro Pro Asp Ser Ala Ser
100 105 110

Tyr His Gly Met Asp Val Trp Gly Gln Gly Thr Thr Val Thr Val Ser
115 120 125

Ser

<210> 182
<211> 14
<212> PRT
<213> Artificial Sequence

<220>
<223> Description of Artificial Sequence: Synthetic peptide

<400> 182
Thr Gly Thr Ser Ser Asp Val Gly Asp Tyr Asn Tyr Val Ser
1 5 10

<210> 183
<211> 7
<212> PRT
<213> Artificial Sequence

<220>
<223> Description of Artificial Sequence: Synthetic peptide

<400> 183
Asp Val Ser Asn Arg Pro Ser
1 5

<210> 184
<211> 11
<212> PRT
<213> Artificial Sequence

<220>
<223> Description of Artificial Sequence: Synthetic peptide

<400> 184
Ser Ser Tyr Ser Ser Ser Ser Ala Leu Glu Val
1 5 10

<210> 185
<211> 111
<212> PRT
<213> Artificial Sequence

<220>
<223> Description of Artificial Sequence: Synthetic peptide

<400> 185
Gln Ser Ala Leu Thr Gln Pro Ala Ser Val Ser Gly Ser Pro Gly Gln
1 5 10 15

Ser Ile Thr Ile Ser Cys Thr Gly Thr Ser Ser Asp Val Gly Asp Tyr
20 25 30

Asn Tyr Val Ser Trp Tyr Gln Gln His Pro Gly Lys Val Pro Lys Leu
35 40 45

Met Ile Tyr Asp Val Ser Asn Arg Pro Ser Gly Val Ser Asn Arg Phe
50 55 60

Ser Gly Ser Met Ser Gly Asn Thr Ala Ser Leu Thr Ile Ser Gly Leu
65 70 75 80

Gln Ala Glu Asp Glu Ala Asp Tyr Tyr Cys Ser Ser Tyr Ser Ser Ser
85 90 95

Ser Ala Leu Glu Val Phe Gly Gly Gly Thr Lys Leu Thr Val Leu
100 105 110

<210> 186
<211> 11
<212> PRT
<213> Artificial Sequence

<220>
<223> Description of Artificial Sequence: Synthetic peptide

<400> 186
Ser Gly Asn Lys Leu Gly Ala Lys Phe Ala Ser
1 5 10

<210> 187
<211> 7
<212> PRT
<213> Artificial Sequence

<220>
<223> Description of Artificial Sequence: Synthetic peptide

<400> 187
Gln Asp Asn Lys Arg Pro Ser
1 5

<210> 188
<211> 106
<212> PRT
<213> Artificial Sequence

<220>
<223> Description of Artificial Sequence: Synthetic peptide

<400> 188
Ser Tyr Glu Leu Thr Gln Pro Pro Ser Val Ser Val Ser Pro Gly Gln
1 5 10 15

Thr Ala Ser Ile Thr Cys Ser Gly Asn Lys Leu Gly Ala Lys Phe Ala
20 25 30

Ser Trp Tyr Gln Gln Lys Pro Gly Gln Ser Pro Val Leu Val Ile Tyr
35 40 45

Gln Asp Asn Lys Arg Pro Ser Gly Ile Pro Glu Arg Phe Ser Gly Ser
50 55 60

Asn Ser Gly Asn Thr Ala Thr Leu Thr Ile Ser Gly Thr Gln Ala Val
65 70 75 80

Asp Glu Ala Asp Tyr Tyr Cys Gln Ala Trp Asp Ser Ser Thr Val Val
85 90 95

Phe Gly Gly Gly Thr Lys Leu Thr Val Leu
100 105

<210> 189
<211> 11
<212> PRT
<213> Artificial Sequence

<220>
<223> Description of Artificial Sequence: Synthetic peptide

<400> 189
Arg Ala Ser Gln Gly Ile Ser Ser Trp Leu Ala
1 5 10

<210> 190
<211> 7
<212> PRT
<213> Artificial Sequence

<220>
<223> Description of Artificial Sequence: Synthetic peptide

<400> 190
Ala Ala Ser Ser Leu Gln Ser
1 5

<210> 191
<211> 9
<212> PRT
<213> Artificial Sequence

<220>
<223> Description of Artificial Sequence: Synthetic peptide

<400> 191
Gln Gln Ala Asn Ser Phe Pro Phe Thr
1 5

<210> 192
<211> 107
<212> PRT
<213> Artificial Sequence

<220>
<223> Description of Artificial Sequence: Synthetic peptide

<400> 192
Asp Ile Gln Met Thr Gln Ser Pro Ser Ser Val Ser Ala Ser Val Gly
1 5 10 15

Asp Arg Val Thr Ile Thr Cys Arg Ala Ser Gln Gly Ile Ser Ser Trp
20 25 30

Leu Ala Trp Tyr Gln Gln Lys Pro Gly Lys Ala Pro Lys Leu Leu Ile
35 40 45

Tyr Ala Ala Ser Ser Leu Gln Ser Gly Val Pro Ser Arg Phe Ser Gly
50 55 60

Ser Gly Ser Gly Thr Asp Phe Thr Leu Thr Ile Ser Ser Leu Gln Pro
65 70 75 80

Glu Asp Phe Ala Thr Tyr Tyr Cys Gln Gln Ala Asn Ser Phe Pro Phe
85 90 95

Thr Phe Gly Pro Gly Thr Lys Val Asp Ile Lys
100 105

<210> 193
<211> 17
<212> PRT
<213> Artificial Sequence

<220>
<223> Description of Artificial Sequence: Synthetic peptide

<400> 193
Lys Ser Ser Gln Thr Val Phe Tyr Ser Ser Asn Asn Lys Asn Tyr Leu
1 5 10 15

Ala

<210> 194
<211> 7
<212> PRT
<213> Artificial Sequence

<220>
<223> Description of Artificial Sequence: Synthetic peptide

<400> 194
Trp Ala Ser Thr Arg Lys Ser
1 5

<210> 195
<211> 9
<212> PRT
<213> Artificial Sequence

<220>
<223> Description of Artificial Sequence: Synthetic peptide

<400> 195
Gln His Tyr Tyr Ser Thr Pro Tyr Thr
1 5

<210> 196
<211> 113
<212> PRT
<213> Artificial Sequence

<220>
<223> Description of Artificial Sequence: Synthetic peptide

<400> 196
Asp Ile Val Met Thr Gln Ser Pro Asp Ser Leu Ala Val Ser Leu Gly
1 5 10 15

Glu Arg Ala Thr Ile Asn Cys Lys Ser Ser Gln Thr Val Phe Tyr Ser
20 25 30

Ser Asn Asn Lys Asn Tyr Leu Ala Trp Tyr Gln Gln Lys Pro Gly Gln
35 40 45

Pro Pro Lys Leu Leu Ile Ser Trp Ala Ser Thr Arg Lys Ser Gly Val
50 55 60

Pro Asp Arg Phe Ser Gly Ser Gly Ser Gly Thr Asp Phe Thr Leu Thr
65 70 75 80

```
Val Ser Ser Leu Gln Ala Glu Asp Val Ala Val Tyr Tyr Cys Gln His
                85                  90                  95

Tyr Tyr Ser Thr Pro Tyr Thr Phe Gly Gln Gly Thr Lys Leu Glu Ile
            100                 105                 110

Lys


<210>   197
<211>   11
<212>   PRT
<213>   Artificial Sequence

<220>
<223>   Description of Artificial Sequence: Synthetic peptide

<400>   197
Ser Gly Asp Lys Leu Gly Asp Lys Tyr Ala Ser
1               5                   10

<210>   198
<211>   7
<212>   PRT
<213>   Artificial Sequence

<220>
<223>   Description of Artificial Sequence: Synthetic peptide

<400>   198
Gln Asp Gly Lys Arg Pro Ser
1               5

<210>   199
<211>   9
<212>   PRT
<213>   Artificial Sequence

<220>
<223>   Description of Artificial Sequence: Synthetic peptide

<400>   199
Gln Ala Trp Asp Arg Asn Thr Val Val
1               5

<210>   200
<211>   106
<212>   PRT
<213>   Artificial Sequence

<220>
<223>   Description of Artificial Sequence: Synthetic peptide
```

<400> 200
Ser Tyr Glu Leu Thr Gln Pro Pro Ser Val Ser Val Ser Pro Gly Gln
1 5 10 15

Thr Ala Ser Ile Thr Cys Ser Gly Asp Lys Leu Gly Asp Lys Tyr Ala
20 25 30

Ser Trp Tyr Gln Gln Lys Pro Gly Gln Ser Pro Val Leu Val Ile Tyr
35 40 45

Gln Asp Gly Lys Arg Pro Ser Gly Ile Pro Glu Arg Phe Ser Gly Ser
50 55 60

Asn Phe Gly Asn Lys Ala Thr Leu Thr Ile Ser Gly Thr Gln Ala Met
65 70 75 80

Asp Glu Ala Asp Tyr Tyr Cys Gln Ala Trp Asp Arg Asn Thr Val Val
85 90 95

Phe Gly Gly Gly Thr Lys Leu Thr Val Leu
100 105

<210> 201
<211> 11
<212> PRT
<213> Artificial Sequence

<220>
<223> Description of Artificial Sequence: Synthetic peptide

<400> 201
Ser Gly Asp Lys Leu Gly Asp Lys Tyr Val Cys
1 5 10

<210> 202
<211> 106
<212> PRT
<213> Artificial Sequence

<220>
<223> Description of Artificial Sequence: Synthetic peptide

<400> 202
Ser Tyr Glu Leu Thr Gln Pro Pro Ser Val Ser Val Ser Pro Gly Gln
1 5 10 15

Thr Ala Ser Ile Thr Cys Ser Gly Asp Lys Leu Gly Asp Lys Tyr Val
20 25 30

Cys Trp Tyr Gln Gln Lys Pro Gly Gln Ser Pro Val Val Val Ile His
35 40 45

Gln Asp Arg Lys Arg Pro Ser Gly Ile Pro Glu Arg Phe Ser Gly Ser
50 55 60

```
Asn Phe Gly Asn Thr Ala Thr Leu Thr Ile Ser Gly Thr Gln Ala Met
65                  70                  75                  80

Asp Glu Ala Asp Tyr Tyr Cys Gln Ala Trp Asp Ser Ser Thr Val Val
                85                  90                  95

Phe Gly Gly Gly Thr Lys Leu Thr Val Leu
            100                 105

<210>   203
<211>   248
<212>   PRT
<213>   Artificial Sequence

<220>
<223>   Description of Artificial Sequence: Synthetic peptide

<400>   203
Gln Ser Ala Leu Thr Gln Pro Ala Ser Val Ser Gly Ser Pro Gly Gln
1               5                   10                  15

Ser Ile Thr Ile Ser Cys Thr Gly Thr Ser Ser Asp Val Gly Asp Tyr
            20                  25                  30

Asn Tyr Val Ser Trp Tyr Gln Gln His Pro Gly Lys Val Pro Lys Leu
        35                  40                  45

Met Ile Tyr Asp Val Ser Asn Arg Pro Ser Gly Val Ser Asn Arg Phe
    50                  55                  60

Ser Gly Ser Met Ser Gly Asn Thr Ala Ser Leu Thr Ile Ser Gly Leu
65                  70                  75                  80

Gln Ala Glu Asp Glu Ala Asp Tyr Tyr Cys Ser Ser Tyr Ser Ser Ser
                85                  90                  95

Ser Ala Leu Glu Val Phe Gly Gly Gly Thr Lys Leu Thr Val Leu Gly
            100                 105                 110

Gly Ser Glu Gly Lys Ser Ser Gly Ser Gly Ser Glu Ser Lys Ser Thr
        115                 120                 125

Gly Gly Ser Gln Val Gln Leu Val Gln Ser Gly Ser Glu Leu Arg Lys
    130                 135                 140

Pro Gly Ala Ser Val Lys Val Ser Cys Lys Ala Ser Gly Tyr Thr Phe
145                 150                 155                 160

Thr Asn Tyr Ala Met Asn Trp Val Arg Gln Ala Pro Gly Gln Gly Leu
                165                 170                 175

Glu Trp Met Gly Trp Ile Asn Thr Asn Thr Gly Asn Pro Thr Tyr Ala
            180                 185                 190
```

```
Gln Gly Phe Thr Gly Arg Phe Val Phe Ser Leu Asp Thr Ser Val Ser
        195                 200                 205

Ser Ala Tyr Leu Gln Ile Ser Ser Leu Lys Ala Glu Asp Thr Ala Met
    210                 215                 220

Tyr Tyr Cys Ala Thr Asp Arg Asp Arg Gly Thr Asp Tyr Trp Gly Gln
225                 230                 235                 240

Gly Thr Leu Val Thr Val Ser Ser
                245

<210>   204
<211>   244
<212>   PRT
<213>   Artificial Sequence

<220>
<223>   Description of Artificial Sequence: Synthetic peptide

<400>   204
Ser Tyr Glu Leu Thr Gln Pro Pro Ser Val Ser Val Ser Pro Gly Gln
1               5                   10                  15

Thr Ala Ser Ile Thr Cys Ser Gly Asn Lys Leu Gly Ala Lys Phe Ala
            20                  25                  30

Ser Trp Tyr Gln Gln Lys Pro Gly Gln Ser Pro Val Leu Val Ile Tyr
        35                  40                  45

Gln Asp Asn Lys Arg Pro Ser Gly Ile Pro Glu Arg Phe Ser Gly Ser
    50                  55                  60

Asn Ser Gly Asn Thr Ala Thr Leu Thr Ile Ser Gly Thr Gln Ala Val
65                  70                  75                  80

Asp Glu Ala Asp Tyr Tyr Cys Gln Ala Trp Asp Ser Ser Thr Val Val
                85                  90                  95

Phe Gly Gly Gly Thr Lys Leu Thr Val Leu Gly Gly Ser Glu Gly Lys
            100                 105                 110

Ser Ser Gly Ser Gly Ser Glu Ser Lys Ser Thr Gly Gly Ser Glu Val
        115                 120                 125

Gln Leu Val Glu Ser Gly Gly Gly Leu Val Gln Pro Gly Gly Ser Leu
    130                 135                 140

Arg Leu Ser Cys Ala Ala Ser Gly Phe Thr Phe Ser Ser Tyr Trp Met
145                 150                 155                 160

Ser Trp Val Arg Gln Ala Pro Gly Lys Gly Leu Glu Trp Val Ala Asn
                165                 170                 175
```

```
Ile Lys Gln His Gly Ser Glu Lys Tyr Tyr Val Asp Ser Val Lys Gly
            180                 185                 190

Arg Phe Thr Ile Ser Arg Asp Asn Ala Lys Asn Ser Leu Tyr Leu Gln
        195                 200                 205

Met Asn Ser Leu Arg Ala Glu Asp Thr Ala Val Tyr Tyr Cys Ala Arg
    210                 215                 220

Asp Arg Asp Leu Gly Tyr Phe Asp Tyr Trp Gly Gln Gly Thr Leu Val
225                 230                 235                 240

Thr Val Ser Ser


<210>   205
<211>   247
<212>   PRT
<213>   Artificial Sequence

<220>
<223>   Description of Artificial Sequence: Synthetic peptide

<400>   205
Asp Ile Gln Met Thr Gln Ser Pro Ser Ser Val Ser Ala Ser Val Gly
1               5                   10                  15

Asp Arg Val Thr Ile Thr Cys Arg Ala Ser Gln Gly Ile Ser Ser Trp
            20                  25                  30

Leu Ala Trp Tyr Gln Gln Lys Pro Gly Lys Ala Pro Lys Leu Leu Ile
        35                  40                  45

Tyr Ala Ala Ser Ser Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Ala Asn Ser Phe Pro Phe
                85                  90                  95

Thr Phe Gly Pro Gly Thr Lys Val Asp Ile Lys Gly Gly Ser Glu Gly
            100                 105                 110

Lys Ser Ser Gly Ser Gly Ser Glu Ser Lys Ser Thr Gly Gly Ser Gln
        115                 120                 125

Leu Gln Leu Gln Glu Ser Gly Pro Gly Leu Val Lys Pro Ser Glu Thr
    130                 135                 140

Leu Ser Leu Thr Cys Thr Val Ser Gly Gly Ser Ile Ser Ser Ser Ser
145                 150                 155                 160
```

Tyr Tyr Trp Gly Trp Ile Arg Gln Pro Pro Gly Lys Gly Leu Asp Trp
165 170 175

Ile Gly Ser Ile Asn Tyr Ser Gly Ser Thr Tyr Tyr Asn Pro Ser Leu
180 185 190

Lys Ser Arg Val Thr Ile Ser Val Asp Thr Ser Lys Ile Gln Phe Ser
195 200 205

Leu Lys Leu Arg Ser Val Thr Ala Ala Asp Thr Ala Val Tyr Tyr Cys
210 215 220

Ala Arg Leu Asp Gly Tyr Glu Ser Pro Phe Asp Tyr Trp Gly Gln Gly
225 230 235 240

Thr Leu Val Thr Val Ser Ser
245

<210> 206
<211> 252
<212> PRT
<213> Artificial Sequence

<220>
<223> Description of Artificial Sequence: Synthetic peptide

<400> 206
Asp Ile Val Met Thr Gln Ser Pro Asp Ser Leu Ala Val Ser Leu Gly
1 5 10 15

Glu Arg Ala Thr Ile Asn Cys Lys Ser Ser Gln Thr Val Phe Tyr Ser
20 25 30

Ser Asn Asn Lys Asn Tyr Leu Ala Trp Tyr Gln Gln Lys Pro Gly Gln
35 40 45

Pro Pro Lys Leu Leu Ile Ser Trp Ala Ser Thr Arg Lys Ser Gly Val
50 55 60

Pro Asp Arg Phe Ser Gly Ser Gly Ser Gly Thr Asp Phe Thr Leu Thr
65 70 75 80

Val Ser Ser Leu Gln Ala Glu Asp Val Ala Val Tyr Tyr Cys Gln His
85 90 95

Tyr Tyr Ser Thr Pro Tyr Thr Phe Gly Gln Gly Thr Lys Leu Glu Ile
100 105 110

Lys Gly Gly Ser Glu Gly Lys Ser Ser Gly Ser Gly Ser Glu Ser Lys
115 120 125

Ser Thr Gly Gly Ser Glu Val Gln Leu Val Glu Ser Gly Gly Gly Leu
130 135 140

Val Gln Pro Gly Arg Ser Leu Arg Leu Ser Cys Ala Ala Ser Gly Phe
145 150 155 160

Thr Phe Asp Asp Tyr Ala Met His Trp Val Arg Gln Ala Pro Gly Lys
165 170 175

Gly Leu Glu Trp Val Ser Gly Ile Gly Trp Ser Gly Gly Ser Ile Val
180 185 190

Tyr Ala Asp Ser Val Lys Gly Arg Phe Thr Ile Ser Arg Asp Asn Ala
195 200 205

Lys Asn Ser Leu Tyr Leu Gln Met Asn Ser Leu Arg Ala Glu Asp Thr
210 215 220

Ala Leu Tyr Tyr Cys Ala Lys Asp Ser Pro Tyr Gly Asp Phe Phe Asp
225 230 235 240

Tyr Trp Gly Gln Gly Thr Leu Val Thr Val Ser Ser
245 250

<210> 207
<211> 255
<212> PRT
<213> Artificial Sequence

<220>
<223> Description of Artificial Sequence: Synthetic peptide

<400> 207
Ser Tyr Glu Leu Thr Gln Pro Pro Ser Val Ser Val Ser Pro Gly Gln
1 5 10 15

Thr Ala Ser Ile Thr Cys Ser Gly His Lys Leu Gly Asp Lys Tyr Ala
20 25 30

Ser Trp Tyr Gln Gln Lys Pro Gly Gln Ser Pro Val Val Val Ile Tyr
35 40 45

Lys Asp Ser Lys Arg Pro Ser Gly Ile Pro Glu Arg Phe Ser Gly Ser
50 55 60

Asn Phe Gly Asn Thr Ala Thr Leu Thr Ile Ser Gly Thr Gln Ala Met
65 70 75 80

Asp Glu Ala Asp Tyr Tyr Cys Gln Ala Trp Asp Ser Ser Thr Val Val
85 90 95

Phe Gly Gly Gly Thr Lys Leu Thr Val Leu Gly Gly Ser Glu Gly Lys
100 105 110

Ser Ser Gly Ser Gly Ser Glu Ser Lys Ser Thr Gly Gly Ser Gln Val
115 120 125

```
Gln Leu Val Glu Ser Gly Gly Gly Val Val Gln Pro Gly Arg Ser Leu
    130                 135                 140

Arg Leu Ser Cys Ala Ala Ser Gly Phe Thr Phe Ser Ser Tyr Gly Met
145                 150                 155                 160

His Trp Val Arg Gln Ser Pro Gly Lys Gly Leu Glu Trp Val Ala Val
                165                 170                 175

Ile Ser Tyr Asp Gly Ser Asn Lys Tyr Tyr Ala Asp Ser Val Lys Gly
            180                 185                 190

Arg Phe Thr Ile Ser Arg Asp Asn Ser Lys Ser Thr Leu Tyr Leu Gln
        195                 200                 205

Met Asn Ser Leu Arg Ala Glu Asp Thr Ala Val Tyr Tyr Cys Ala Lys
    210                 215                 220

Asp Phe Arg Ser Leu Asp Trp Leu Pro Pro Asp Ser Thr Ser Tyr Asp
225                 230                 235                 240

Gly Met Asp Val Trp Gly Gln Gly Thr Thr Val Thr Val Ser Ser
                245                 250                 255

<210>   208
<211>   255
<212>   PRT
<213>   Artificial Sequence

<220>
<223>   Description of Artificial Sequence: Synthetic peptide

<400>   208
Ser Tyr Glu Leu Thr Gln Pro Pro Ser Val Ser Val Ser Pro Gly Gln
1               5                   10                  15

Thr Ala Ser Ile Thr Cys Ser Gly Asp Lys Leu Gly Asp Lys Tyr Ala
            20                  25                  30

Ser Trp Tyr Gln Gln Lys Pro Gly Gln Ser Pro Val Leu Val Ile Tyr
        35                  40                  45

Gln Asp Gly Lys Arg Pro Ser Gly Ile Pro Glu Arg Phe Ser Gly Ser
    50                  55                  60

Asn Phe Gly Asn Lys Ala Thr Leu Thr Ile Ser Gly Thr Gln Ala Met
65                  70                  75                  80

Asp Glu Ala Asp Tyr Tyr Cys Gln Ala Trp Asp Arg Asn Thr Val Val
                85                  90                  95

Phe Gly Gly Gly Thr Lys Leu Thr Val Leu Gly Gly Ser Glu Gly Lys
            100                 105                 110
```

```
Ser Ser Gly Ser Gly Ser Glu Ser Lys Ser Thr Gly Gly Ser Gln Val
        115                 120                 125

Gln Leu Val Glu Ser Gly Gly Gly Val Val Gln Pro Gly Arg Ser Leu
    130                 135                 140

Arg Leu Ser Cys Val Ala Ser Gly Phe Thr Phe Ser Ser Tyr Gly Met
145                 150                 155                 160

His Trp Val Arg Gln Ala Pro Gly Lys Gly Leu Glu Trp Val Ala Val
                165                 170                 175

Ile Ser Tyr Asp Gly Ser Asn Lys Tyr Tyr Ala Asp Ser Val Lys Gly
            180                 185                 190

Arg Phe Thr Ile Ser Arg Asp Asn Ser Lys Asn Thr Leu Tyr Leu Gln
        195                 200                 205

Met Asn Ser Leu Arg Ala Glu Gly Thr Ala Val Tyr Tyr Cys Ala Lys
    210                 215                 220

Asp Phe Arg Ser Phe Asp Trp Leu Pro Pro Asp Ser Ala Ser Tyr His
225                 230                 235                 240

Gly Met Asp Val Trp Gly Gln Gly Thr Thr Val Thr Val Ser Ser
                245                 250                 255

<210>   209
<211>   255
<212>   PRT
<213>   Artificial Sequence

<220>
<223>   Description of Artificial Sequence: Synthetic peptide

<400>   209
Ser Tyr Glu Leu Thr Gln Pro Pro Ser Val Ser Val Ser Pro Gly Gln
1               5                   10                  15

Thr Ala Ser Ile Thr Cys Ser Gly Asp Lys Leu Gly Asp Lys Tyr Val
            20                  25                  30

Ser Trp Tyr Gln Gln Lys Pro Gly Gln Ser Pro Val Val Val Ile His
        35                  40                  45

Gln Asp Arg Lys Arg Pro Ser Gly Ile Pro Glu Arg Phe Ser Gly Ser
    50                  55                  60

Asn Phe Gly Asn Thr Ala Thr Leu Thr Ile Ser Gly Thr Gln Ala Met
65                  70                  75                  80

Asp Glu Ala Asp Tyr Tyr Cys Gln Ala Trp Asp Ser Ser Thr Val Val
                85                  90                  95
```

```
Phe Gly Gly Gly Thr Lys Leu Thr Val Leu Gly Gly Ser Glu Gly Lys
            100                 105                 110

Ser Ser Gly Ser Gly Ser Glu Ser Lys Ser Thr Gly Gly Ser Gln Val
        115                 120                 125

Gln Leu Val Glu Ser Gly Gly Gly Val Val Gln Pro Gly Arg Ser Leu
    130                 135                 140

Arg Leu Ser Cys Ala Ala Ser Gly Phe Thr Phe Ser Ser Tyr Gly Met
145                 150                 155                 160

His Trp Val Arg Gln Ala Pro Gly Lys Gly Leu Glu Trp Val Ala Val
                165                 170                 175

Ile Ser Tyr Asp Gly Ser Asn Lys Tyr Tyr Ala Asp Ser Val Lys Gly
            180                 185                 190

Arg Phe Thr Ile Ser Arg Asp Asn Ser Lys Asn Thr Leu Tyr Leu Gln
        195                 200                 205

Met Asn Ser Leu Arg Ala Glu Asp Thr Ala Val Tyr Tyr Cys Ala Lys
    210                 215                 220

Asp Phe Arg Ser Phe Asp Trp Leu Pro Pro Asp Ser Thr Ser Tyr Tyr
225                 230                 235                 240

Gly Met Asp Val Trp Gly Gln Gly Thr Thr Val Thr Val Ser Ser
                245                 250                 255

<210>   210
<211>   255
<212>   PRT
<213>   Artificial Sequence

<220>
<223>   Description of Artificial Sequence: Synthetic peptide

<400>   210
Ser Tyr Glu Leu Thr Gln Pro Pro Ser Val Ser Val Ser Pro Gly Gln
1               5                   10                  15

Thr Ala Ser Ile Thr Cys Ser Gly Asp Lys Leu Gly Asp Lys Tyr Val
            20                  25                  30

Cys Trp Tyr Gln Gln Lys Pro Gly Gln Ser Pro Val Val Val Ile His
        35                  40                  45

Gln Asp Arg Lys Arg Pro Ser Gly Ile Pro Glu Arg Phe Ser Gly Ser
    50                  55                  60

Asn Phe Gly Asn Thr Ala Thr Leu Thr Ile Ser Gly Thr Gln Ala Met
65                  70                  75                  80
```

```
Asp Glu Ala Asp Tyr Tyr Cys Gln Ala Trp Asp Ser Ser Thr Val Val
                85                  90                  95

Phe Gly Gly Gly Thr Lys Leu Thr Val Leu Gly Gly Ser Glu Gly Lys
            100                 105                 110

Ser Ser Gly Ser Gly Ser Glu Ser Lys Ser Thr Gly Gly Ser Gln Val
        115                 120                 125

Gln Leu Val Glu Ser Gly Gly Gly Val Val Gln Pro Gly Arg Ser Leu
    130                 135                 140

Arg Leu Ser Cys Ala Ala Ser Gly Phe Thr Phe Ser Ser Tyr Gly Met
145                 150                 155                 160

His Trp Val Arg Gln Ala Pro Gly Lys Gly Leu Glu Trp Val Ala Val
                165                 170                 175

Ile Ser Tyr Asp Gly Ser Asn Lys Tyr Tyr Ala Asp Ser Val Lys Gly
            180                 185                 190

Arg Phe Thr Ile Ser Arg Asp Asn Ser Lys Asn Thr Leu Tyr Leu Gln
        195                 200                 205

Met Asn Ser Leu Arg Ala Glu Asp Thr Ala Val Tyr Tyr Cys Ala Lys
    210                 215                 220

Asp Phe Arg Ser Phe Asp Trp Leu Pro Pro Asp Ser Thr Ser Tyr Tyr
225                 230                 235                 240

Gly Met Asp Val Trp Gly Gln Gly Thr Thr Val Thr Val Ser Ser
                245                 250                 255

<210>   211
<211>   244
<212>   PRT
<213>   Artificial Sequence

<220>
<223>   Description of Artificial Sequence: Synthetic peptide

<400>   211
Ser Tyr Glu Leu Thr Gln Pro Pro Ser Val Ser Val Ser Pro Gly Gln
1               5                   10                  15

Thr Ala Ser Ile Thr Cys Ser Gly Asp Lys Leu Gly Ser Lys Phe Ala
            20                  25                  30

Ser Trp Tyr Gln Gln Lys Pro Gly Gln Ser Pro Val Leu Val Ile Tyr
        35                  40                  45

Gln Asp Ser Lys Arg Pro Ser Gly Ile Pro Glu Arg Phe Ser Gly Ser
    50                  55                  60
```

Asn Ser Gly Asn Thr Ala Thr Leu Thr Ile Ser Gly Thr Gln Ala Met
65                  70                  75                  80

Asp Glu Ala Asp Tyr Tyr Cys Gln Ala Trp Asp Ser Ser Thr Val Val
                85                  90                  95

Phe Gly Gly Gly Thr Lys Leu Thr Val Leu Gly Gly Ser Glu Gly Lys
            100                 105                 110

Ser Ser Gly Ser Gly Ser Glu Ser Lys Ser Thr Gly Gly Ser Glu Val
        115                 120                 125

Gln Leu Val Glu Ser Gly Gly Gly Phe Val Gln Pro Gly Gly Ser Leu
    130                 135                 140

Arg Leu Ser Cys Ala Ala Ser Gly Phe Thr Phe Ser Ser Tyr Trp Met
145                 150                 155                 160

Ser Trp Val Arg Gln Ala Pro Gly Lys Gly Leu Glu Trp Val Ala Asn
                165                 170                 175

Ile Lys Gln His Gly Ser Glu Lys Tyr Tyr Val Asp Ser Val Lys Gly
            180                 185                 190

Arg Phe Thr Ile Ser Arg Asp Asn Ala Lys Asn Ser Leu Tyr Leu Gln
        195                 200                 205

Met Asn Ser Leu Arg Ala Glu Asp Thr Ala Val Tyr Tyr Cys Ala Arg
    210                 215                 220

Asp Arg Asp Leu Gly Tyr Phe Asp Tyr Trp Gly Gln Gly Thr Leu Val
225                 230                 235                 240

Thr Val Ser Ser

<210> 212
<211> 243
<212> PRT
<213> Artificial Sequence

<220>
<223> Description of Artificial Sequence: Synthetic peptide

<400> 212
Asp Ile Gln Met Thr Gln Ser Pro Ser Ser Leu Ser Ala Ser Val Gly
1               5                   10                  15

Asp Arg Val Thr Ile Thr Cys Gln Ala Ser Gln Ala Ile Ser Asn Tyr
            20                  25                  30

Leu Asn Trp Tyr Gln Gln Lys Pro Gly Lys Ala Pro Lys Leu Leu Ile
        35                  40                  45

Tyr Asp Ala Ser Asn Leu Glu Thr Gly Val Pro Ser Arg Phe Ser Gly
    50                  55                  60

Ser Gly Ser Gly Thr Asp Phe Thr Phe Thr Ile Ser Ser Leu Gln Pro
65                  70                  75                  80

Glu Asp Phe Ser Thr Tyr Phe Cys Gln His Tyr Asp Asn Leu Pro Tyr
                85                  90                  95

Thr Phe Gly Gln Gly Thr Lys Leu Glu Ile Lys Gly Gly Ser Glu Gly
            100                 105                 110

Lys Ser Ser Gly Ser Gly Ser Glu Ser Lys Ser Thr Gly Gly Ser Gln
        115                 120                 125

Val Gln Leu Val Glu Ser Gly Gly Gly Leu Val Gln Pro Gly Gly Ser
    130                 135                 140

Leu Arg Leu Ser Cys Ala Ala Ser Gly Phe Thr Phe Ser Ser Phe Gly
145                 150                 155                 160

Met Ser Trp Val Arg Gln Ala Pro Gly Lys Gly Leu Glu Trp Val Ala
                165                 170                 175

Asn Ile Lys Arg Asp Gly Ser Glu Lys Tyr Tyr Val Asp Ser Val Lys
            180                 185                 190

Gly Arg Phe Thr Ile Ser Arg Asp Asn Ala Lys Asn Ser Leu Phe Leu
        195                 200                 205

Gln Met Ser Ser Leu Arg Ala Glu Asp Thr Ala Val Tyr Tyr Cys Val
    210                 215                 220

Arg Asp Tyr Gly Tyr Phe Asp Phe Trp Gly Gln Gly Thr Leu Val Thr
225                 230                 235                 240

Val Ser Ser

<210> 213
<211> 246
<212> PRT
<213> Artificial Sequence

<220>
<223> Description of Artificial Sequence: Synthetic peptide

<400> 213
Gln Ser Val Leu Thr Gln Pro Pro Ser Ala Ser Gly Thr Pro Gly Gln
1               5                   10                  15

Arg Val Thr Ile Ser Cys Ser Gly Ser Ser Ser Asn Ile Gly Ser Asn
            20                  25                  30

Pro Val Asn Trp Tyr Gln Gln Leu Pro Gly Thr Ala Pro Lys Leu Leu
35 40 45

Ile Tyr Ser Asn Asn Gln Arg Pro Ser Gly Val Pro Asp Arg Phe Ser
50 55 60

Gly Ser Lys Ser Gly Thr Ser Ala Ser Leu Ala Ile Ser Gly Leu Gln
65 70 75 80

Ser Glu Asp Glu Ala Asp Tyr Phe Cys Ala Ala Trp Asp Asp Ser Leu
85 90 95

Asn Gly Pro Val Phe Gly Gly Gly Thr Lys Leu Thr Val Leu Gly Gly
100 105 110

Ser Glu Gly Lys Ser Ser Gly Ser Gly Ser Glu Ser Lys Ser Thr Gly
115 120 125

Gly Ser Gln Val Gln Leu Gln Glu Ser Gly Pro Gly Leu Val Lys Pro
130 135 140

Ser Glu Thr Leu Ser Leu Thr Cys Thr Val Ser Gly Gly Ser Ile Ser
145 150 155 160

Ser Tyr Tyr Trp Gly Trp Ile Arg Gln Pro Pro Gly Lys Gly Leu Glu
165 170 175

Trp Ile Gly Tyr Ile Tyr Tyr Ser Gly Ser Thr Asn Tyr Asn Pro Ser
180 185 190

Leu Lys Ser Arg Val Thr Ile Ser Val Asp Thr Ser Lys Asn Gln Phe
195 200 205

Ser Leu Lys Leu Ser Ser Val Thr Ala Ala Asp Thr Ala Val Tyr Tyr
210 215 220

Cys Ala Arg Met Trp Glu Ile Leu Gly Phe Asp Pro Trp Gly Gln Gly
225 230 235 240

Thr Leu Val Thr Val Ser
245

<210> 214
<211> 247
<212> PRT
<213> Artificial Sequence

<220>
<223> Description of Artificial Sequence: Synthetic peptide

<400> 214
Asp Ile Val Met Thr Gln Ser His Lys Phe Met Ser Thr Ser Val Gly
1 5 10 15

```
Asp Arg Val Ser Ile Thr Cys Lys Ala Ser Gln Asp Val Arg Thr Ala
            20                  25                  30

Glu Ala Trp Tyr Gln Gln Lys Pro Gly Gln Ser Pro Lys Leu Leu Ile
        35                  40                  45

Tyr Ser Ala Ser Asn Arg Tyr Thr Gly Val Pro Asp Arg Phe Thr Gly
    50                  55                  60

Ser Gly Ser Gly Thr Asp Phe Thr Phe Thr Ile Ser Ser Val Gln Ala
65                  70                  75                  80

Glu Asp Leu Ala Val Tyr Tyr Cys Gln Gln Tyr Tyr Thr Thr Pro Arg
                85                  90                  95

Thr Phe Gly Gly Gly Thr Lys Leu Glu Ile Lys Gly Gly Ser Glu Gly
            100                 105                 110

Lys Ser Ser Gly Ser Gly Ser Glu Ser Lys Ser Thr Gly Gly Ser Glu
        115                 120                 125

Val Gln Leu Gln Gln Ser Gly Pro Glu Leu Val Lys Pro Gly Ser Ser
    130                 135                 140

Val Lys Ile Ser Cys Lys Ala Ser Gly Tyr Thr Phe Thr Asp Tyr Asn
145                 150                 155                 160

Ile Asn Trp Val Lys Gln Ser His Gly Lys Asn Leu Glu Trp Ile Gly
                165                 170                 175

Thr Ile Asn Pro Asn Asn Gly Val Thr Phe Tyr Asn Gln Arg Phe Lys
            180                 185                 190

Gly Gln Ala Thr Leu Thr Val Asp Lys Ser Ser Ser Thr Ala Tyr Met
        195                 200                 205

Glu Leu Arg Ser Leu Thr Ser Glu Asp Ser Ala Val Tyr Tyr Cys Ser
    210                 215                 220

Arg Asp Gly Tyr Asp Thr Tyr Tyr Ala Met Asp Tyr Trp Gly Gln Gly
225                 230                 235                 240

Thr Thr Leu Thr Val Ser Ser
                245
```

<210> 215
<211> 247
<212> PRT
<213> Artificial Sequence

<220>
<223> Description of Artificial Sequence: Synthetic peptide

<400> 215

```
Asp Ile Val Met Thr Gln Ser Gln Lys Phe Met Ser Thr Ser Val Gly
1               5                   10                  15

Asp Arg Val Ser Val Thr Cys Lys Ala Ser Gln Asn Val Gly Thr Tyr
            20                  25                  30

Val Ala Trp Tyr Gln Gln Lys Pro Gly His Ser Pro Lys Ala Leu Ile
        35                  40                  45

Tyr Ser Ala Ser Tyr Arg Tyr Ser Gly Val Pro Asp Arg Phe Thr Gly
    50                  55                  60

Ser Gly Ser Gly Thr Asp Phe Thr Leu Thr Ile Ser Asn Val Gln Ser
65                  70                  75                  80

Glu Asp Leu Ala Asp Tyr Phe Cys Gln Gln Tyr Lys Ser Tyr Pro Leu
                85                  90                  95

Thr Phe Gly Ala Gly Thr Lys Leu Glu Leu Lys Gly Gly Ser Glu Gly
            100                 105                 110

Lys Ser Ser Gly Ser Gly Ser Glu Ser Lys Ser Thr Gly Gly Ser Glu
        115                 120                 125

Val Gln Leu Val Glu Ser Gly Gly Gly Leu Val Arg Pro Gly Gly Ser
    130                 135                 140

Leu Lys Leu Ser Cys Ala Ala Ser Gly Phe Thr Phe Ser Asn Tyr Ala
145                 150                 155                 160

Met Ser Trp Val Arg Gln Thr Pro Glu Lys Arg Leu Glu Trp Val Ala
                165                 170                 175

Gly Ile Ser Asn Ser Gly Tyr Ser Leu Tyr Tyr Pro Asp Thr Leu Lys
            180                 185                 190

Gly Arg Phe Thr Ile Ser Arg Asp Asn Ala Arg Asn Thr Leu Tyr Leu
        195                 200                 205

Gln Met Ile Ser Leu Arg Ser Glu Asp Thr Ala Met Tyr Tyr Cys Ala
    210                 215                 220

Arg Asp Gly Gly Ser Tyr Pro Tyr Ala Met Asp Tyr Trp Gly His Gly
225                 230                 235                 240

Thr Ser Val Thr Val Ser Ser
                245
```

<210> 216
<211> 247
<212> PRT
<213> Artificial Sequence

<220>
<223> Description of Artificial Sequence: Synthetic peptide

<400> 216
Gln Ser Val Leu Thr Gln Pro Pro Ser Ala Ser Gly Thr Pro Gly Gln
1 5 10 15

Arg Val Thr Ile Ser Cys Ser Gly Ser Ser Ser Asn Ile Gly Ser Asn
20 25 30

Ile Val Asn Trp Tyr Gln Gln Phe Pro Gly Thr Ala Pro Lys Leu Leu
35 40 45

Ile Tyr Ser Asn Asn Gln Arg Pro Ser Gly Val Pro Asp Arg Val Ser
50 55 60

Gly Ser Lys Ser Gly Thr Ser Ala Ser Leu Ala Ile Ser Gly Leu Gln
65 70 75 80

Ser Glu Asp Glu Ala Asp Tyr Tyr Cys Ala Ala Trp Asp Asp Ser Leu
85 90 95

Asn Gly Pro Val Phe Gly Pro Gly Thr Lys Val Thr Val Leu Gly Gly
100 105 110

Ser Glu Gly Lys Ser Ser Gly Ser Gly Ser Glu Ser Lys Ser Thr Gly
115 120 125

Gly Ser Gln Val Gln Leu Gln Glu Ser Gly Pro Gly Leu Val Lys Pro
130 135 140

Ser Glu Thr Leu Ser Leu Thr Cys Ser Val Ser Gly Ala Ser Ile Arg
145 150 155 160

Asn Tyr Tyr Trp Ser Trp Ile Arg Gln Thr Ala Gly Lys Gly Leu Glu
165 170 175

Trp Leu Gly His Ile Tyr Ser Thr Gly Asn Ile His Tyr Asn Pro Ser
180 185 190

Leu Lys Ser Arg Val Thr Met Ser Val Asp Thr Ser Asn Asn Gln Phe
195 200 205

Ser Leu Lys Leu Arg Ser Val Thr Ala Ala Asp Thr Ala Val Tyr Tyr
210 215 220

Cys Ala Arg Asp Asn Gly Ala Ala Leu Phe Asp Tyr Trp Gly Gln Gly
225 230 235 240

Thr Leu Val Thr Val Ser Ser
245

<210> 217
<211> 243

<212> PRT
<213> Artificial Sequence

<220>
<223> Description of Artificial Sequence: Synthetic peptide

<400> 217
Asp Ile Gln Leu Thr Gln Ser Pro Ser Phe Leu Ser Ala Ser Val Gly
1 5 10 15

Asp Arg Val Thr Ile Thr Cys Arg Ala Ser Gln Gly Ile Ser Ser Tyr
20 25 30

Leu Ala Trp Tyr Gln Gln Lys Pro Gly Lys Ala Pro Lys Val Leu Ile
35 40 45

Tyr Ala Ala Ser Thr Leu Gln Ser Gly Val Pro Ser Arg Phe Ser Gly
50 55 60

Ser Gly Ser Gly Thr Glu Phe Thr Leu Thr Ile Ser Ser Leu Gln Pro
65 70 75 80

Glu Asp Phe Ala Thr Tyr Phe Cys Gln Gln Leu Asn Ser Tyr Pro Val
85 90 95

Thr Phe Gly Gly Gly Thr Lys Val Glu Ile Lys Gly Gly Ser Glu Gly
100 105 110

Lys Ser Ser Gly Ser Gly Ser Glu Ser Lys Ser Thr Gly Gly Ser Glu
115 120 125

Val Gln Leu Val Glu Ser Gly Gly Gly Leu Val Gln Pro Gly Gly Ser
130 135 140

Leu Arg Leu Ser Cys Thr Ala Ser Gly Phe Thr Phe Ser Ser Tyr Trp
145 150 155 160

Met Ser Trp Val Arg Gln Ala Pro Gly Lys Gly Leu Asp Trp Val Ala
165 170 175

Asn Ile Lys Arg Asp Gly Ser Glu Lys His Tyr Val Asp Ser Val Lys
180 185 190

Gly Arg Phe Thr Ile Ser Arg Asp Asn Ala Lys Asn Ser Leu Tyr Leu
195 200 205

Gln Met Asn Ser Leu Arg Ala Glu Asp Ser Ala Val Tyr Tyr Cys Thr
210 215 220

Arg Asp Tyr Gly Tyr Phe Asp Tyr Trp Gly Gln Gly Thr Leu Val Thr
225 230 235 240

Val Ser Ser

<210> 218
<211> 255
<212> PRT
<213> Artificial Sequence

<220>
<223> Description of Artificial Sequence: Synthetic peptide

<400> 218
```
Asp Ile Val Met Thr Gln Ser Pro Leu Ser Leu Pro Val Thr Pro Gly
1               5                   10                  15

Glu Pro Ala Ser Ile Ser Cys Lys Ser Ser Gln Ser Leu Leu Phe Ser
            20                  25                  30

Asn Gly Tyr Lys Phe Leu Asp Trp Tyr Leu Gln Arg Pro Gly Gln Ser
        35                  40                  45

Pro Gln Leu Leu Ile Tyr Leu Gly Ser Tyr Arg Ala Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Val Gly Leu Tyr Tyr Cys Met Gln Ala
                85                  90                  95

Leu Gln Thr Pro Pro Thr Phe Gly Gly Gly Thr Lys Val Glu Ile Lys
            100                 105                 110

Gly Gly Ser Glu Gly Lys Ser Ser Gly Ser Gly Ser Glu Ser Lys Ser
        115                 120                 125

Thr Gly Gly Ser Glu Val Gln Leu Val Glu Ser Gly Gly Gly Leu Val
    130                 135                 140

Gln Pro Gly Gly Ser Leu Lys Val Ser Cys Glu Ala Ser Gly Phe Thr
145                 150                 155                 160

Phe Ser Val Ser Ala Ile His Trp Val Arg Gln Ala Ser Gly Lys Gly
                165                 170                 175

Leu Glu Trp Ile Gly Arg Ile Arg Ser Lys Gly Asn Ser Tyr Ala Thr
            180                 185                 190

Ala Tyr Asp Val Ser Val Lys Gly Arg Phe Thr Ile Ser Arg Asp Asp
        195                 200                 205

Ser Lys Asn Thr Ala Tyr Leu Gln Met Asp Ser Leu Lys Thr Glu Asp
    210                 215                 220

Thr Ala Val Tyr Tyr Cys Thr Arg His Asn Asp Lys Trp Asn Tyr Tyr
225                 230                 235                 240
```

```
Gly Leu Asp Val Trp Gly Gln Gly Thr Thr Val Thr Val Ser Ser
                245                 250                 255

<210>   219
<211>   255
<212>   PRT
<213>   Artificial Sequence

<220>
<223>   Description of Artificial Sequence: Synthetic peptide

<400>   219
Asp Ile Val Met Thr Gln Ser Pro Leu Ser Leu Pro Val Thr Pro Gly
1               5                   10                  15

Glu Pro Ala Ser Ile Ser Cys Lys Ser Ser Gln Ser Leu Leu Phe Ser
            20                  25                  30

Asn Gly Tyr Lys Phe Leu Asp Trp Tyr Leu Gln Arg Pro Gly Gln Ser
        35                  40                  45

Pro Gln Leu Leu Ile Tyr Leu Gly Ser Tyr Arg Ala Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Val Gly Leu Tyr Tyr Cys Met Gln Ala
                85                  90                  95

Leu Gln Thr Pro Pro Thr Phe Gly Gly Gly Thr Lys Val Glu Ile Lys
            100                 105                 110

Gly Gly Ser Glu Gly Lys Ser Ser Gly Ser Gly Ser Glu Ser Lys Ser
        115                 120                 125

Thr Gly Gly Ser Glu Val Gln Leu Val Glu Ser Gly Gly Gly Leu Val
    130                 135                 140

Gln Pro Gly Gly Ser Leu Lys Val Ser Cys Glu Ala Ser Gly Phe Thr
145                 150                 155                 160

Phe Ser Val Ser Ala Ile His Trp Val Arg Gln Ala Ser Gly Lys Gly
                165                 170                 175

Leu Glu Trp Ile Gly Arg Ile Arg Ser Lys Gly Asn Ser Tyr Ala Thr
            180                 185                 190

Ala Tyr Ala Ala Ser Val Lys Gly Arg Phe Thr Ile Ser Arg Asp Asp
        195                 200                 205

Ser Lys Asn Thr Ala Tyr Leu Gln Met Asp Ser Leu Lys Thr Glu Asp
    210                 215                 220
```

```
Thr Ala Val Tyr Tyr Cys Thr Arg His Asn Asp Lys Trp Asn Tyr Tyr
225                 230                 235                 240

Gly Leu Asp Val Trp Gly Gln Gly Thr Thr Val Thr Val Ser Ser
                245                 250                 255

<210>   220
<211>   255
<212>   PRT
<213>   Artificial Sequence

<220>
<223>   Description of Artificial Sequence: Synthetic peptide

<400>   220
Asp Ile Val Met Thr Gln Ser Pro Leu Ser Leu Pro Val Thr Pro Gly
1               5                   10                  15

Glu Pro Ala Ser Ile Ser Cys Lys Ser Ser Gln Ser Leu Leu Phe Ser
            20                  25                  30

Asn Gly Tyr Lys Phe Leu Asp Trp Tyr Leu Gln Arg Pro Gly Gln Ser
        35                  40                  45

Pro Gln Leu Leu Ile Tyr Leu Gly Ser Tyr Arg Ala Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Val Gly Leu Tyr Tyr Cys Met Gln Ala
                85                  90                  95

Leu Gln Thr Pro Pro Thr Phe Gly Gly Gly Thr Lys Val Glu Ile Lys
            100                 105                 110

Gly Gly Ser Glu Gly Lys Ser Ser Gly Ser Gly Ser Glu Ser Lys Ser
        115                 120                 125

Thr Gly Gly Ser Glu Val Gln Leu Val Glu Ser Gly Gly Gly Leu Val
    130                 135                 140

Gln Pro Gly Gly Ser Leu Lys Val Ser Cys Glu Ala Ser Gly Phe Thr
145                 150                 155                 160

Phe Ser Val Ser Ala Ile His Trp Val Arg Gln Ala Ser Gly Lys Gly
                165                 170                 175

Leu Glu Trp Ile Gly Arg Ile Arg Ser Lys Gly Asn Ser Tyr Ala Thr
            180                 185                 190

Ala Tyr Asn Val Ser Val Lys Gly Arg Phe Thr Ile Ser Arg Asp Asp
        195                 200                 205
```

Ser Lys Asn Thr Ala Tyr Leu Gln Met Asp Ser Leu Lys Thr Glu Asp
    210                 215                 220

Thr Ala Val Tyr Tyr Cys Thr Arg His Asn Asp Lys Trp Asn Tyr Tyr
225                 230                 235                 240

Gly Leu Asp Val Trp Gly Gln Gly Thr Thr Val Thr Val Ser Ser
                245                 250                 255

<210>   221
<211>   255
<212>   PRT
<213>   Artificial Sequence

<220>
<223>   Description of Artificial Sequence: Synthetic peptide

<400>   221
Asp Ile Val Met Thr Gln Ser Pro Leu Ser Leu Pro Val Thr Pro Gly
1               5                   10                  15

Glu Pro Ala Ser Ile Ser Cys Lys Ser Ser Gln Ser Leu Leu Phe Ser
            20                  25                  30

Asn Gly Tyr Lys Phe Leu Asp Trp Tyr Leu Gln Arg Pro Gly Gln Ser
        35                  40                  45

Pro Gln Leu Leu Ile Tyr Leu Gly Ser Tyr Arg Ala Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Val Gly Leu Tyr Tyr Cys Met Gln Ala
                85                  90                  95

Leu Gln Thr Pro Pro Thr Phe Gly Gln Gly Thr Lys Val Glu Ile Lys
            100                 105                 110

Gly Gly Ser Glu Gly Lys Ser Ser Gly Ser Gly Ser Glu Ser Lys Ser
        115                 120                 125

Thr Gly Gly Ser Glu Val Gln Leu Val Glu Ser Gly Gly Gly Leu Val
    130                 135                 140

Gln Pro Gly Gly Ser Leu Lys Val Ser Cys Glu Ala Ser Gly Phe Thr
145                 150                 155                 160

Phe Ser Val Phe Ala Ile His Trp Val Arg Gln Ala Ser Gly Lys Gly
                165                 170                 175

Leu Glu Trp Ile Gly Arg Ile Arg Ser Lys Gly Asn Ser Tyr Ala Thr
            180                 185                 190

```
Ala Tyr Asp Val Ser Val Lys Gly Arg Phe Thr Ile Ser Arg Asp Asp
        195                 200                 205

Ser Lys Asn Thr Ala Tyr Leu Gln Met Asp Ser Leu Lys Thr Glu Asp
    210                 215                 220

Thr Ala Val Tyr Tyr Cys Thr Arg His Asn Asp Lys Trp Asn Tyr Tyr
225                 230                 235                 240

Gly Leu Asp Val Trp Gly Gln Gly Thr Thr Val Thr Val Ser Ser
                245                 250                 255
```

<210> 222
<211> 330
<212> PRT
<213> Homo sapiens

<400> 222

```
Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Lys Val Glu Pro Lys Ser Cys Asp Lys Thr His Thr Cys Pro Pro Cys
            100                 105                 110

Pro Ala Pro Glu Leu Leu Gly Gly Pro Ser Val Phe Leu Phe Pro Pro
        115                 120                 125

Lys Pro Lys Asp Thr Leu Met Ile Ser Arg Thr Pro Glu Val Thr Cys
    130                 135                 140

Val Val Val Asp Val Ser His Glu Asp Pro Glu Val Lys Phe Asn Trp
145                 150                 155                 160

Tyr Val Asp Gly Val Glu Val His Asn Ala Lys Thr Lys Pro Arg Glu
                165                 170                 175

Glu Gln Tyr Asn Ser Thr Tyr Arg Val Val Ser Val Leu Thr Val Leu
            180                 185                 190
```

```
His Gln Asp Trp Leu Asn Gly Lys Glu Tyr Lys Cys Lys Val Ser Asn
          195                 200                 205

Lys Ala Leu Pro Ala Pro Ile Glu Lys Thr Ile Ser Lys Ala Lys Gly
    210                 215                 220

Gln Pro Arg Glu Pro Gln Val Tyr Thr Leu Pro Pro Ser Arg Asp Glu
225                 230                 235                 240

Leu Thr Lys Asn Gln Val Ser Leu Thr Cys Leu Val Lys Gly Phe Tyr
                245                 250                 255

Pro Ser Asp Ile Ala Val Glu Trp Glu Ser Asn Gly Gln Pro Glu Asn
            260                 265                 270

Asn Tyr Lys Thr Thr Pro Pro Val Leu Asp Ser Asp Gly Ser Phe Phe
        275                 280                 285

Leu Tyr Ser Lys Leu Thr Val Asp Lys Ser Arg Trp Gln Gln Gly Asn
    290                 295                 300

Val Phe Ser Cys Ser Val Met His Glu Ala Leu His Asn His Tyr Thr
305                 310                 315                 320

Gln Lys Ser Leu Ser Leu Ser Pro Gly Lys
                325                 330

<210>   223
<211>   326
<212>   PRT
<213>   Homo sapiens

<400>   223
Ala Ser Thr Lys Gly Pro Ser Val Phe Pro Leu Ala Pro Cys Ser Arg
1               5                   10                  15

Ser Thr Ser Glu Ser Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Asn Phe Gly Thr Gln Thr
65                  70                  75                  80

Tyr Thr Cys Asn Val Asp His Lys Pro Ser Asn Thr Lys Val Asp Lys
                85                  90                  95

Thr Val Glu Arg Lys Cys Cys Val Glu Cys Pro Pro Cys Pro Ala Pro
            100                 105                 110
```

```
Pro Val Ala Gly Pro Ser Val Phe Leu Phe Pro Pro Lys Pro Lys Asp
        115                 120                 125

Thr Leu Met Ile Ser Arg Thr Pro Glu Val Thr Cys Val Val Val Asp
    130                 135                 140

Val Ser His Glu Asp Pro Glu Val Gln Phe Asn Trp Tyr Val Asp Gly
145                 150                 155                 160

Val Glu Val His Asn Ala Lys Thr Lys Pro Arg Glu Glu Gln Phe Asn
                165                 170                 175

Ser Thr Phe Arg Val Val Ser Val Leu Thr Val Val His Gln Asp Trp
            180                 185                 190

Leu Asn Gly Lys Glu Tyr Lys Cys Lys Val Ser Asn Lys Gly Leu Pro
        195                 200                 205

Ala Pro Ile Glu Lys Thr Ile Ser Lys Thr Lys Gly Gln Pro Arg Glu
    210                 215                 220

Pro Gln Val Tyr Thr Leu Pro Pro Ser Arg Glu Glu Met Thr Lys Asn
225                 230                 235                 240

Gln Val Ser Leu Thr Cys Leu Val Lys Gly Phe Tyr Pro Ser Asp Ile
                245                 250                 255

Ser Val Glu Trp Glu Ser Asn Gly Gln Pro Glu Asn Asn Tyr Lys Thr
            260                 265                 270

Thr Pro Pro Met Leu Asp Ser Asp Gly Ser Phe Phe Leu Tyr Ser Lys
        275                 280                 285

Leu Thr Val Asp Lys Ser Arg Trp Gln Gln Gly Asn Val Phe Ser Cys
    290                 295                 300

Ser Val Met His Glu Ala Leu His Asn His Tyr Thr Gln Lys Ser Leu
305                 310                 315                 320

Ser Leu Ser Pro Gly Lys
                325

<210>   224
<211>   327
<212>   PRT
<213>   Homo sapiens

<400>   224
Ala Ser Thr Lys Gly Pro Ser Val Phe Pro Leu Ala Pro Cys Ser Arg
1               5                   10                  15

Ser Thr Ser Glu Ser Thr Ala Ala Leu Gly Cys Leu Val Lys Asp Tyr
            20                  25                  30
```

```
Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Lys Thr
65                  70                  75                  80

Tyr Thr Cys Asn Val Asp His Lys Pro Ser Asn Thr Lys Val Asp Lys
                85                  90                  95

Arg Val Glu Ser Lys Tyr Gly Pro Pro Cys Pro Ser Cys Pro Ala Pro
            100                 105                 110

Glu Phe Leu Gly Gly Pro Ser Val Phe Leu Phe Pro Pro Lys Pro Lys
        115                 120                 125

Asp Thr Leu Met Ile Ser Arg Thr Pro Glu Val Thr Cys Val Val Val
    130                 135                 140

Asp Val Ser Gln Glu Asp Pro Glu Val Gln Phe Asn Trp Tyr Val Asp
145                 150                 155                 160

Gly Val Glu Val His Asn Ala Lys Thr Lys Pro Arg Glu Glu Gln Phe
                165                 170                 175

Asn Ser Thr Tyr Arg Val Val Ser Val Leu Thr Val Leu His Gln Asp
            180                 185                 190

Trp Leu Asn Gly Lys Glu Tyr Lys Cys Lys Val Ser Asn Lys Gly Leu
        195                 200                 205

Pro Ser Ser Ile Glu Lys Thr Ile Ser Lys Ala Lys Gly Gln Pro Arg
    210                 215                 220

Glu Pro Gln Val Tyr Thr Leu Pro Pro Ser Gln Glu Glu Met Thr Lys
225                 230                 235                 240

Asn Gln Val Ser Leu Thr Cys Leu Val Lys Gly Phe Tyr Pro Ser Asp
                245                 250                 255

Ile Ala Val Glu Trp Glu Ser Asn Gly Gln Pro Glu Asn Asn Tyr Lys
            260                 265                 270

Thr Thr Pro Pro Val Leu Asp Ser Asp Gly Ser Phe Phe Leu Tyr Ser
        275                 280                 285

Arg Leu Thr Val Asp Lys Ser Arg Trp Gln Glu Gly Asn Val Phe Ser
    290                 295                 300

Cys Ser Val Met His Glu Ala Leu His Asn His Tyr Thr Gln Lys Ser
305                 310                 315                 320
```

Leu Ser Leu Ser Leu Gly Lys
325

<210> 225
<211> 851
<212> PRT
<213> Macaca fascicularis

<400> 225
Asp Pro Arg Val Arg Leu Glu Val Gln Glu Ser Val Thr Val Gln Glu
1 5 10 15

Gly Leu Cys Val Leu Val Pro Cys Thr Phe Phe His Pro Val Pro Tyr
20 25 30

His Thr Arg Asn Ser Pro Val His Gly Tyr Trp Phe Arg Glu Gly Ala
35 40 45

Ile Val Ser Leu Asp Ser Pro Val Ala Thr Asn Lys Leu Asp Gln Glu
50 55 60

Val Gln Glu Glu Thr Gln Gly Arg Phe Arg Leu Leu Gly Asp Pro Ser
65 70 75 80

Arg Asn Asn Cys Ser Leu Ser Ile Val Asp Ala Arg Arg Arg Asp Asn
85 90 95

Gly Ser Tyr Phe Phe Arg Met Glu Lys Gly Ser Thr Lys Tyr Ser Tyr
100 105 110

Lys Ser Thr Gln Leu Ser Val His Val Thr Asp Leu Thr His Arg Pro
115 120 125

Gln Ile Leu Ile Pro Gly Ala Leu Asp Pro Asp His Ser Lys Asn Leu
130 135 140

Thr Cys Ser Val Pro Trp Ala Cys Glu Gln Gly Thr Pro Pro Ile Phe
145 150 155 160

Ser Trp Met Ser Ala Ala Pro Thr Ser Leu Gly Leu Arg Thr Thr His
165 170 175

Ser Ser Val Leu Ile Ile Thr Pro Arg Pro Gln Asp His Gly Thr Asn
180 185 190

Leu Thr Cys Gln Val Lys Phe Pro Gly Ala Gly Val Thr Thr Glu Arg
195 200 205

Thr Ile Gln Leu Asn Val Ser Tyr Ala Ser Gln Asn Pro Arg Thr Asp
210 215 220

Ile Phe Leu Gly Asp Gly Ser Gly Lys Gln Gly Val Val Gln Gly Ser
225 230 235 240

```
Gly Ser Gly Ser Glu Asn Leu Tyr Phe Gln Gly Val Arg Ser Ser Ser
                245                 250                 255

Asp Ala His Lys Ser Glu Val Ala His Arg Phe Lys Asp Leu Gly Glu
            260                 265                 270

Glu Asn Phe Lys Ala Leu Val Leu Ile Ala Phe Ala Gln Tyr Leu Gln
        275                 280                 285

Gln Ser Pro Phe Glu Asp His Val Lys Leu Val Asn Glu Val Thr Glu
    290                 295                 300

Phe Ala Lys Thr Cys Val Ala Asp Glu Ser Ala Glu Asn Cys Asp Lys
305                 310                 315                 320

Ser Leu His Thr Leu Phe Gly Asp Lys Leu Cys Thr Val Ala Thr Leu
                325                 330                 335

Arg Glu Thr Tyr Gly Glu Met Ala Asp Cys Cys Ala Lys Gln Glu Pro
            340                 345                 350

Glu Arg Asn Glu Cys Phe Leu Gln His Lys Asp Asp Asn Pro Asn Leu
        355                 360                 365

Pro Arg Leu Val Arg Pro Glu Val Asp Val Met Cys Thr Ala Phe His
    370                 375                 380

Asp Asn Glu Glu Thr Phe Leu Lys Lys Tyr Leu Tyr Glu Ile Ala Arg
385                 390                 395                 400

Arg His Pro Tyr Phe Tyr Ala Pro Glu Leu Leu Phe Phe Ala Lys Arg
                405                 410                 415

Tyr Lys Ala Ala Phe Thr Glu Cys Cys Gln Ala Ala Asp Lys Ala Ala
            420                 425                 430

Cys Leu Leu Pro Lys Leu Asp Glu Leu Arg Asp Glu Gly Lys Ala Ser
        435                 440                 445

Ser Ala Lys Gln Arg Leu Lys Cys Ala Ser Leu Gln Lys Phe Gly Glu
    450                 455                 460

Arg Ala Phe Lys Ala Trp Ala Val Ala Arg Leu Ser Gln Arg Phe Pro
465                 470                 475                 480

Lys Ala Glu Phe Ala Glu Val Ser Lys Leu Val Thr Asp Leu Thr Lys
                485                 490                 495

Val His Thr Glu Cys Cys His Gly Asp Leu Leu Glu Cys Ala Asp Asp
            500                 505                 510

Arg Ala Asp Leu Ala Lys Tyr Ile Cys Glu Asn Gln Asp Ser Ile Ser
        515                 520                 525
```

```
Ser Lys Leu Lys Glu Cys Cys Glu Lys Pro Leu Leu Glu Lys Ser His
    530                 535                 540

Cys Ile Ala Glu Val Glu Asn Asp Glu Met Pro Ala Asp Leu Pro Ser
545                 550                 555                 560

Leu Ala Ala Asp Phe Val Glu Ser Lys Asp Val Cys Lys Asn Tyr Ala
                565                 570                 575

Glu Ala Lys Asp Val Phe Leu Gly Met Phe Leu Tyr Glu Tyr Ala Arg
            580                 585                 590

Arg His Pro Asp Tyr Ser Val Val Leu Leu Leu Arg Leu Ala Lys Thr
        595                 600                 605

Tyr Glu Thr Thr Leu Glu Lys Cys Cys Ala Ala Ala Asp Pro His Glu
    610                 615                 620

Cys Tyr Ala Lys Val Phe Asp Glu Phe Lys Pro Leu Val Glu Glu Pro
625                 630                 635                 640

Gln Asn Leu Ile Lys Gln Asn Cys Glu Leu Phe Glu Gln Leu Gly Glu
                645                 650                 655

Tyr Lys Phe Gln Asn Ala Leu Leu Val Arg Tyr Thr Lys Lys Val Pro
            660                 665                 670

Gln Val Ser Thr Pro Thr Leu Val Glu Val Ser Arg Asn Leu Gly Lys
        675                 680                 685

Val Gly Ser Lys Cys Cys Lys His Pro Glu Ala Lys Arg Met Pro Cys
    690                 695                 700

Ala Glu Asp Tyr Leu Ser Val Val Leu Asn Gln Leu Cys Val Leu His
705                 710                 715                 720

Glu Lys Thr Pro Val Ser Asp Arg Val Thr Lys Cys Cys Thr Glu Ser
                725                 730                 735

Leu Val Asn Arg Arg Pro Cys Phe Ser Ala Leu Glu Val Asp Glu Thr
            740                 745                 750

Tyr Val Pro Lys Glu Phe Asn Ala Glu Thr Phe Thr Phe His Ala Asp
        755                 760                 765

Ile Cys Thr Leu Ser Glu Lys Glu Arg Gln Ile Lys Lys Gln Thr Ala
    770                 775                 780

Leu Val Glu Leu Val Lys His Lys Pro Lys Ala Thr Lys Glu Gln Leu
785                 790                 795                 800

Lys Ala Val Met Asp Asp Phe Ala Ala Phe Val Glu Lys Cys Cys Lys
                805                 810                 815
```

Ala Asp Asp Lys Glu Thr Cys Phe Ala Glu Glu Gly Lys Lys Leu Val
820 825 830

Ala Ala Ser Gln Ala Ala Leu Gly Leu Gly Gly Gly Ser His His His
835 840 845

His His His
850

<210> 226
<211> 855
<212> PRT
<213> Homo sapiens

<400> 226
Asp Pro Asn Phe Trp Leu Gln Val Gln Glu Ser Val Thr Val Gln Glu
1 5 10 15

Gly Leu Cys Val Leu Val Pro Cys Thr Phe Phe His Pro Ile Pro Tyr
20 25 30

Tyr Asp Lys Asn Ser Pro Val His Gly Tyr Trp Phe Arg Glu Gly Ala
35 40 45

Ile Ile Ser Arg Asp Ser Pro Val Ala Thr Asn Lys Leu Asp Gln Glu
50 55 60

Val Gln Glu Glu Thr Gln Gly Arg Phe Arg Leu Leu Gly Asp Pro Ser
65 70 75 80

Arg Asn Asn Cys Ser Leu Ser Ile Val Asp Ala Arg Arg Arg Asp Asn
85 90 95

Gly Ser Tyr Phe Phe Arg Met Glu Arg Gly Ser Thr Lys Tyr Ser Tyr
100 105 110

Lys Ser Pro Gln Leu Ser Val His Val Thr Asp Leu Thr His Arg Pro
115 120 125

Lys Ile Leu Ile Pro Gly Thr Leu Glu Pro Gly His Ser Lys Asn Leu
130 135 140

Thr Cys Ser Val Ser Trp Ala Cys Glu Gln Gly Thr Pro Pro Ile Phe
145 150 155 160

Ser Trp Leu Ser Ala Ala Pro Thr Ser Leu Gly Pro Arg Thr Thr His
165 170 175

Ser Ser Val Leu Ile Ile Thr Pro Arg Pro Gln Asp His Gly Thr Asn
180 185 190

Leu Thr Cys Gln Val Lys Phe Ala Gly Ala Gly Val Thr Thr Glu Arg
195 200 205

```
Thr Ile Gln Leu Asn Val Thr Tyr Val Pro Gln Asn Pro Thr Thr Gly
    210                 215                 220

Ile Phe Pro Gly Asp Gly Ser Gly Lys Gln Glu Thr Arg Ala Gly Val
225                 230                 235                 240

Val His Gly Ser Gly Ser Gly Ser Glu Asn Leu Tyr Phe Gln Gly Val
                245                 250                 255

Arg Ser Ser Ser Asp Ala His Lys Ser Glu Val Ala His Arg Phe Lys
            260                 265                 270

Asp Leu Gly Glu Glu Asn Phe Lys Ala Leu Val Leu Ile Ala Phe Ala
        275                 280                 285

Gln Tyr Leu Gln Gln Ser Pro Phe Glu Asp His Val Lys Leu Val Asn
    290                 295                 300

Glu Val Thr Glu Phe Ala Lys Thr Cys Val Ala Asp Glu Ser Ala Glu
305                 310                 315                 320

Asn Cys Asp Lys Ser Leu His Thr Leu Phe Gly Asp Lys Leu Cys Thr
                325                 330                 335

Val Ala Thr Leu Arg Glu Thr Tyr Gly Glu Met Ala Asp Cys Cys Ala
            340                 345                 350

Lys Gln Glu Pro Glu Arg Asn Glu Cys Phe Leu Gln His Lys Asp Asp
        355                 360                 365

Asn Pro Asn Leu Pro Arg Leu Val Arg Pro Glu Val Asp Val Met Cys
    370                 375                 380

Thr Ala Phe His Asp Asn Glu Glu Thr Phe Leu Lys Lys Tyr Leu Tyr
385                 390                 395                 400

Glu Ile Ala Arg Arg His Pro Tyr Phe Tyr Ala Pro Glu Leu Leu Phe
                405                 410                 415

Phe Ala Lys Arg Tyr Lys Ala Ala Phe Thr Glu Cys Cys Gln Ala Ala
            420                 425                 430

Asp Lys Ala Ala Cys Leu Leu Pro Lys Leu Asp Glu Leu Arg Asp Glu
        435                 440                 445

Gly Lys Ala Ser Ser Ala Lys Gln Arg Leu Lys Cys Ala Ser Leu Gln
    450                 455                 460

Lys Phe Gly Glu Arg Ala Phe Lys Ala Trp Ala Val Ala Arg Leu Ser
465                 470                 475                 480

Gln Arg Phe Pro Lys Ala Glu Phe Ala Glu Val Ser Lys Leu Val Thr
                485                 490                 495
```

```
Asp Leu Thr Lys Val His Thr Glu Cys Cys His Gly Asp Leu Leu Glu
            500                 505                 510

Cys Ala Asp Asp Arg Ala Asp Leu Ala Lys Tyr Ile Cys Glu Asn Gln
        515                 520                 525

Asp Ser Ile Ser Ser Lys Leu Lys Glu Cys Cys Glu Lys Pro Leu Leu
    530                 535                 540

Glu Lys Ser His Cys Ile Ala Glu Val Glu Asn Asp Glu Met Pro Ala
545                 550                 555                 560

Asp Leu Pro Ser Leu Ala Ala Asp Phe Val Glu Ser Lys Asp Val Cys
                565                 570                 575

Lys Asn Tyr Ala Glu Ala Lys Asp Val Phe Leu Gly Met Phe Leu Tyr
            580                 585                 590

Glu Tyr Ala Arg Arg His Pro Asp Tyr Ser Val Val Leu Leu Leu Arg
        595                 600                 605

Leu Ala Lys Thr Tyr Glu Thr Thr Leu Glu Lys Cys Cys Ala Ala Ala
    610                 615                 620

Asp Pro His Glu Cys Tyr Ala Lys Val Phe Asp Glu Phe Lys Pro Leu
625                 630                 635                 640

Val Glu Glu Pro Gln Asn Leu Ile Lys Gln Asn Cys Glu Leu Phe Glu
                645                 650                 655

Gln Leu Gly Glu Tyr Lys Phe Gln Asn Ala Leu Leu Val Arg Tyr Thr
            660                 665                 670

Lys Lys Val Pro Gln Val Ser Thr Pro Thr Leu Val Glu Val Ser Arg
        675                 680                 685

Asn Leu Gly Lys Val Gly Ser Lys Cys Cys Lys His Pro Glu Ala Lys
    690                 695                 700

Arg Met Pro Cys Ala Glu Asp Tyr Leu Ser Val Val Leu Asn Gln Leu
705                 710                 715                 720

Cys Val Leu His Glu Lys Thr Pro Val Ser Asp Arg Val Thr Lys Cys
                725                 730                 735

Cys Thr Glu Ser Leu Val Asn Arg Arg Pro Cys Phe Ser Ala Leu Glu
            740                 745                 750

Val Asp Glu Thr Tyr Val Pro Lys Glu Phe Asn Ala Glu Thr Phe Thr
        755                 760                 765

Phe His Ala Asp Ile Cys Thr Leu Ser Glu Lys Glu Arg Gln Ile Lys
    770                 775                 780
```

```
Lys Gln Thr Ala Leu Val Glu Leu Val Lys His Lys Pro Lys Ala Thr
785                 790                 795                 800

Lys Glu Gln Leu Lys Ala Val Met Asp Asp Phe Ala Ala Phe Val Glu
                805                 810                 815

Lys Cys Cys Lys Ala Asp Asp Lys Glu Thr Cys Phe Ala Glu Glu Gly
            820                 825                 830

Lys Lys Leu Val Ala Ala Ser Gln Ala Ala Leu Gly Leu Gly Gly Gly
        835                 840                 845

Ser His His His His His His
    850                 855

<210>   227
<211>   718
<212>   PRT
<213>   Homo sapiens

<400>   227
Asp Pro Asn Phe Trp Leu Gln Val Gln Glu Ser Val Thr Val Gln Glu
1               5                   10                  15

Gly Leu Cys Val Leu Val Pro Cys Thr Phe Phe His Pro Ile Pro Tyr
            20                  25                  30

Tyr Asp Lys Asn Ser Pro Val His Gly Tyr Trp Phe Arg Glu Gly Ala
        35                  40                  45

Ile Ile Ser Arg Asp Ser Pro Val Ala Thr Asn Lys Leu Asp Gln Glu
    50                  55                  60

Val Gln Glu Glu Thr Gln Gly Arg Phe Arg Leu Leu Gly Asp Pro Ser
65                  70                  75                  80

Arg Asn Asn Cys Ser Leu Ser Ile Val Asp Ala Arg Arg Arg Asp Asn
                85                  90                  95

Gly Ser Tyr Phe Phe Arg Met Glu Arg Gly Ser Thr Lys Tyr Ser Tyr
            100                 105                 110

Lys Ser Pro Gln Leu Ser Val His Val Thr Asp Leu Thr His Arg Asp
        115                 120                 125

Ala His Lys Ser Glu Val Ala His Arg Phe Lys Asp Leu Gly Glu Glu
    130                 135                 140

Asn Phe Lys Ala Leu Val Leu Ile Ala Phe Ala Gln Tyr Leu Gln Gln
145                 150                 155                 160

Ser Pro Phe Glu Asp His Val Lys Leu Val Asn Glu Val Thr Glu Phe
                165                 170                 175
```

```
Ala Lys Thr Cys Val Ala Asp Glu Ser Ala Glu Asn Cys Asp Lys Ser
            180                 185                 190

Leu His Thr Leu Phe Gly Asp Lys Leu Cys Thr Val Ala Thr Leu Arg
        195                 200                 205

Glu Thr Tyr Gly Glu Met Ala Asp Cys Cys Ala Lys Gln Glu Pro Glu
    210                 215                 220

Arg Asn Glu Cys Phe Leu Gln His Lys Asp Asp Asn Pro Asn Leu Pro
225                 230                 235                 240

Arg Leu Val Arg Pro Glu Val Asp Val Met Cys Thr Ala Phe His Asp
                245                 250                 255

Asn Glu Glu Thr Phe Leu Lys Lys Tyr Leu Tyr Glu Ile Ala Arg Arg
            260                 265                 270

His Pro Tyr Phe Tyr Ala Pro Glu Leu Leu Phe Phe Ala Lys Arg Tyr
        275                 280                 285

Lys Ala Ala Phe Thr Glu Cys Cys Gln Ala Ala Asp Lys Ala Ala Cys
    290                 295                 300

Leu Leu Pro Lys Leu Asp Glu Leu Arg Asp Glu Gly Lys Ala Ser Ser
305                 310                 315                 320

Ala Lys Gln Arg Leu Lys Cys Ala Ser Leu Gln Lys Phe Gly Glu Arg
                325                 330                 335

Ala Phe Lys Ala Trp Ala Val Ala Arg Leu Ser Gln Arg Phe Pro Lys
            340                 345                 350

Ala Glu Phe Ala Glu Val Ser Lys Leu Val Thr Asp Leu Thr Lys Val
        355                 360                 365

His Thr Glu Cys Cys His Gly Asp Leu Leu Glu Cys Ala Asp Asp Arg
    370                 375                 380

Ala Asp Leu Ala Lys Tyr Ile Cys Glu Asn Gln Asp Ser Ile Ser Ser
385                 390                 395                 400

Lys Leu Lys Glu Cys Cys Glu Lys Pro Leu Leu Glu Lys Ser His Cys
                405                 410                 415

Ile Ala Glu Val Glu Asn Asp Glu Met Pro Ala Asp Leu Pro Ser Leu
            420                 425                 430

Ala Ala Asp Phe Val Glu Ser Lys Asp Val Cys Lys Asn Tyr Ala Glu
        435                 440                 445

Ala Lys Asp Val Phe Leu Gly Met Phe Leu Tyr Glu Tyr Ala Arg Arg
    450                 455                 460
```

```
His Pro Asp Tyr Ser Val Val Leu Leu Leu Arg Leu Ala Lys Thr Tyr
465                 470                 475                 480

Glu Thr Thr Leu Glu Lys Cys Cys Ala Ala Ala Asp Pro His Glu Cys
                485                 490                 495

Tyr Ala Lys Val Phe Asp Glu Phe Lys Pro Leu Val Glu Glu Pro Gln
            500                 505                 510

Asn Leu Ile Lys Gln Asn Cys Glu Leu Phe Glu Gln Leu Gly Glu Tyr
        515                 520                 525

Lys Phe Gln Asn Ala Leu Leu Val Arg Tyr Thr Lys Lys Val Pro Gln
    530                 535                 540

Val Ser Thr Pro Thr Leu Val Glu Val Ser Arg Asn Leu Gly Lys Val
545                 550                 555                 560

Gly Ser Lys Cys Cys Lys His Pro Glu Ala Lys Arg Met Pro Cys Ala
                565                 570                 575

Glu Asp Tyr Leu Ser Val Val Leu Asn Gln Leu Cys Val Leu His Glu
            580                 585                 590

Lys Thr Pro Val Ser Asp Arg Val Thr Lys Cys Cys Thr Glu Ser Leu
        595                 600                 605

Val Asn Arg Arg Pro Cys Phe Ser Ala Leu Glu Val Asp Glu Thr Tyr
    610                 615                 620

Val Pro Lys Glu Phe Asn Ala Glu Thr Phe Thr Phe His Ala Asp Ile
625                 630                 635                 640

Cys Thr Leu Ser Glu Lys Glu Arg Gln Ile Lys Lys Gln Thr Ala Leu
                645                 650                 655

Val Glu Leu Val Lys His Lys Pro Lys Ala Thr Lys Glu Gln Leu Lys
            660                 665                 670

Ala Val Met Asp Asp Phe Ala Ala Phe Val Glu Lys Cys Cys Lys Ala
        675                 680                 685

Asp Asp Lys Glu Thr Cys Phe Ala Glu Glu Gly Lys Lys Leu Val Ala
    690                 695                 700

Ala Ser Gln Ala Ala Leu Gly Leu His His His His His His
705                 710                 715

<210>   228
<211>   715
<212>   PRT
<213>   Homo sapiens
```

<400> 228

```
Val His Val Thr Asp Leu Thr His Arg Pro Lys Ile Leu Ile Pro Gly
1               5                   10                  15

Thr Leu Glu Pro Gly His Ser Lys Asn Leu Thr Cys Ser Val Ser Trp
            20                  25                  30

Ala Cys Glu Gln Gly Thr Pro Pro Ile Phe Ser Trp Leu Ser Ala Ala
        35                  40                  45

Pro Thr Ser Leu Gly Pro Arg Thr Thr His Ser Ser Val Leu Ile Ile
    50                  55                  60

Thr Pro Arg Pro Gln Asp His Gly Thr Asn Leu Thr Cys Gln Val Lys
65                  70                  75                  80

Phe Ala Gly Ala Gly Val Thr Thr Glu Arg Thr Ile Gln Leu Asn Val
                85                  90                  95

Thr Tyr Val Pro Gln Asn Pro Thr Thr Gly Ile Phe Pro Gly Asp Gly
            100                 105                 110

Ser Gly Lys Gln Glu Thr Arg Ala Gly Val Val His Asp Ala His Lys
        115                 120                 125

Ser Glu Val Ala His Arg Phe Lys Asp Leu Gly Glu Glu Asn Phe Lys
    130                 135                 140

Ala Leu Val Leu Ile Ala Phe Ala Gln Tyr Leu Gln Gln Ser Pro Phe
145                 150                 155                 160

Glu Asp His Val Lys Leu Val Asn Glu Val Thr Glu Phe Ala Lys Thr
                165                 170                 175

Cys Val Ala Asp Glu Ser Ala Glu Asn Cys Asp Lys Ser Leu His Thr
            180                 185                 190

Leu Phe Gly Asp Lys Leu Cys Thr Val Ala Thr Leu Arg Glu Thr Tyr
        195                 200                 205

Gly Glu Met Ala Asp Cys Cys Ala Lys Gln Glu Pro Glu Arg Asn Glu
    210                 215                 220

Cys Phe Leu Gln His Lys Asp Asp Asn Pro Asn Leu Pro Arg Leu Val
225                 230                 235                 240

Arg Pro Glu Val Asp Val Met Cys Thr Ala Phe His Asp Asn Glu Glu
                245                 250                 255

Thr Phe Leu Lys Lys Tyr Leu Tyr Glu Ile Ala Arg Arg His Pro Tyr
            260                 265                 270

Phe Tyr Ala Pro Glu Leu Leu Phe Phe Ala Lys Arg Tyr Lys Ala Ala
        275                 280                 285
```

```
Phe Thr Glu Cys Cys Gln Ala Ala Asp Lys Ala Ala Cys Leu Leu Pro
    290                 295                 300

Lys Leu Asp Glu Leu Arg Asp Glu Gly Lys Ala Ser Ser Ala Lys Gln
305                 310                 315                 320

Arg Leu Lys Cys Ala Ser Leu Gln Lys Phe Gly Glu Arg Ala Phe Lys
                325                 330                 335

Ala Trp Ala Val Ala Arg Leu Ser Gln Arg Phe Pro Lys Ala Glu Phe
            340                 345                 350

Ala Glu Val Ser Lys Leu Val Thr Asp Leu Thr Lys Val His Thr Glu
        355                 360                 365

Cys Cys His Gly Asp Leu Leu Glu Cys Ala Asp Asp Arg Ala Asp Leu
    370                 375                 380

Ala Lys Tyr Ile Cys Glu Asn Gln Asp Ser Ile Ser Ser Lys Leu Lys
385                 390                 395                 400

Glu Cys Cys Glu Lys Pro Leu Leu Glu Lys Ser His Cys Ile Ala Glu
                405                 410                 415

Val Glu Asn Asp Glu Met Pro Ala Asp Leu Pro Ser Leu Ala Ala Asp
            420                 425                 430

Phe Val Glu Ser Lys Asp Val Cys Lys Asn Tyr Ala Glu Ala Lys Asp
        435                 440                 445

Val Phe Leu Gly Met Phe Leu Tyr Glu Tyr Ala Arg Arg His Pro Asp
    450                 455                 460

Tyr Ser Val Val Leu Leu Leu Arg Leu Ala Lys Thr Tyr Glu Thr Thr
465                 470                 475                 480

Leu Glu Lys Cys Cys Ala Ala Ala Asp Pro His Glu Cys Tyr Ala Lys
                485                 490                 495

Val Phe Asp Glu Phe Lys Pro Leu Val Glu Glu Pro Gln Asn Leu Ile
            500                 505                 510

Lys Gln Asn Cys Glu Leu Phe Glu Gln Leu Gly Glu Tyr Lys Phe Gln
        515                 520                 525

Asn Ala Leu Leu Val Arg Tyr Thr Lys Lys Val Pro Gln Val Ser Thr
    530                 535                 540

Pro Thr Leu Val Glu Val Ser Arg Asn Leu Gly Lys Val Gly Ser Lys
545                 550                 555                 560

Cys Cys Lys His Pro Glu Ala Lys Arg Met Pro Cys Ala Glu Asp Tyr
                565                 570                 575
```

```
Leu Ser Val Val Leu Asn Gln Leu Cys Val Leu His Glu Lys Thr Pro
                580                 585                 590

Val Ser Asp Arg Val Thr Lys Cys Cys Thr Glu Ser Leu Val Asn Arg
            595                 600                 605

Arg Pro Cys Phe Ser Ala Leu Glu Val Asp Glu Thr Tyr Val Pro Lys
    610                 615                 620

Glu Phe Asn Ala Glu Thr Phe Thr Phe His Ala Asp Ile Cys Thr Leu
625                 630                 635                 640

Ser Glu Lys Glu Arg Gln Ile Lys Lys Gln Thr Ala Leu Val Glu Leu
                    645                 650                 655

Val Lys His Lys Pro Lys Ala Thr Lys Glu Gln Leu Lys Ala Val Met
                660                 665                 670

Asp Asp Phe Ala Ala Phe Val Glu Lys Cys Cys Lys Ala Asp Asp Lys
            675                 680                 685

Glu Thr Cys Phe Ala Glu Glu Gly Lys Lys Leu Val Ala Ala Ser Gln
    690                 695                 700

Ala Ala Leu Gly Leu His His His His His His
705                 710                 715

<210>   229
<211>   135
<212>   PRT
<213>   Homo sapiens

<400>   229
Asp Pro Asn Phe Trp Leu Gln Val Gln Glu Ser Val Thr Val Gln Glu
1               5                   10                  15

Gly Leu Cys Val Leu Val Pro Cys Thr Phe Phe His Pro Ile Pro Tyr
                20                  25                  30

Tyr Asp Lys Asn Ser Pro Val His Gly Tyr Trp Phe Arg Glu Gly Ala
            35                  40                  45

Ile Ile Ser Arg Asp Ser Pro Val Ala Thr Asn Lys Leu Asp Gln Glu
    50                  55                  60

Val Gln Glu Glu Thr Gln Gly Arg Phe Arg Leu Leu Gly Asp Pro Ser
65                  70                  75                  80

Arg Asn Asn Cys Ser Leu Ser Ile Val Asp Ala Arg Arg Arg Asp Asn
                    85                  90                  95

Gly Ser Tyr Phe Phe Arg Met Glu Arg Gly Ser Thr Lys Tyr Ser Tyr
                100                 105                 110
```

```
Lys Ser Pro Gln Leu Ser Val His Val Thr Asp Leu Thr His Arg Gly
        115                 120                 125

Ser His His His His His His
    130                 135

<210>   230
<211>   132
<212>   PRT
<213>   Homo sapiens

<400>   230
Val His Val Thr Asp Leu Thr His Arg Pro Lys Ile Leu Ile Pro Gly
1               5                   10                  15

Thr Leu Glu Pro Gly His Ser Lys Asn Leu Thr Cys Ser Val Ser Trp
            20                  25                  30

Ala Cys Glu Gln Gly Thr Pro Pro Ile Phe Ser Trp Leu Ser Ala Ala
        35                  40                  45

Pro Thr Ser Leu Gly Pro Arg Thr Thr His Ser Ser Val Leu Ile Ile
    50                  55                  60

Thr Pro Arg Pro Gln Asp His Gly Thr Asn Leu Thr Cys Gln Val Lys
65                  70                  75                  80

Phe Ala Gly Ala Gly Val Thr Thr Glu Arg Thr Ile Gln Leu Asn Val
                85                  90                  95

Thr Tyr Val Pro Gln Asn Pro Thr Thr Gly Ile Phe Pro Gly Asp Gly
            100                 105                 110

Ser Gly Lys Gln Glu Thr Arg Ala Gly Val Val His Gly Ser His His
        115                 120                 125

His His His His
    130

<210>   231
<211>   251
<212>   PRT
<213>   Artificial Sequence

<220>
<223>   Description of Artificial Sequence: Synthetic peptide

<400>   231
Gln Asp Leu Glu Phe Gln Leu Val Ala Pro Glu Ser Val Thr Val Glu
1               5                   10                  15

Glu Gly Leu Cys Val His Val Pro Cys Ser Val Phe Tyr Pro Ser Ile
            20                  25                  30
```

```
Lys Leu Thr Leu Gly Pro Val Thr Gly Ser Trp Leu Arg Lys Gly Val
        35                  40                  45

Ser Leu His Glu Asp Ser Pro Val Ala Thr Ser Asp Pro Arg Gln Leu
    50                  55                  60

Val Gln Lys Ala Thr Gln Gly Arg Phe Gln Leu Leu Gly Asp Pro Gln
65                  70                  75                  80

Lys His Asp Cys Ser Leu Phe Ile Arg Asp Ala Gln Lys Asn Asp Thr
                85                  90                  95

Gly Met Tyr Phe Phe Arg Val Val Arg Glu Pro Phe Val Arg Tyr Ser
            100                 105                 110

Tyr Lys Lys Ser Gln Leu Ser Leu His Val Thr Ser Leu Ser Arg Thr
        115                 120                 125

Pro Lys Ile Leu Ile Pro Gly Thr Leu Glu Pro Gly His Ser Lys Asn
    130                 135                 140

Leu Thr Cys Ser Val Ser Trp Ala Cys Glu Gln Gly Thr Pro Pro Ile
145                 150                 155                 160

Phe Ser Trp Leu Ser Ala Ala Pro Thr Ser Leu Gly Pro Arg Thr Thr
                165                 170                 175

His Ser Ser Val Leu Ile Ile Thr Pro Arg Pro Gln Asp His Gly Thr
            180                 185                 190

Asn Leu Thr Cys Gln Val Lys Phe Ala Gly Ala Gly Val Thr Thr Glu
        195                 200                 205

Arg Thr Ile Gln Leu Asn Val Thr Tyr Val Pro Gln Asn Pro Thr Thr
    210                 215                 220

Gly Ile Phe Pro Gly Asp Gly Ser Gly Lys Gln Glu Thr Arg Ala Gly
225                 230                 235                 240

Val Val His Gly Ser His His His His His His
                245                 250

<210>   232
<211>   251
<212>   PRT
<213>   Artificial Sequence

<220>
<223>   Description of Artificial Sequence: Synthetic peptide

<400>   232
Gln Asp Leu Glu Phe Gln Leu Val Ala Pro Glu Ser Val Thr Val Glu
1               5                   10                  15
```

```
Glu Gly Leu Cys Val His Val Pro Cys Ser Val Phe Tyr Pro Ser Ile
            20                  25                  30

Lys Leu Thr Leu Gly Pro Val Thr Gly Ser Trp Leu Arg Lys Gly Val
        35                  40                  45

Ser Leu His Glu Asp Ser Pro Val Ala Thr Ser Asp Pro Arg Gln Leu
    50                  55                  60

Val Gln Lys Ala Thr Gln Gly Arg Phe Gln Leu Leu Gly Asp Pro Gln
65                  70                  75                  80

Lys His Asp Cys Ser Leu Phe Ile Arg Asp Ala Gln Lys Asn Asp Thr
                85                  90                  95

Gly Met Tyr Phe Phe Arg Val Val Arg Glu Pro Phe Val Arg Tyr Ser
            100                 105                 110

Tyr Lys Lys Ser Gln Leu Ser Leu His Val Thr Ser Leu Ser Arg Thr
        115                 120                 125

Pro Gln Ile Leu Ile Pro Gly Ala Leu Asp Pro Asp His Ser Lys Asn
    130                 135                 140

Leu Thr Cys Ser Val Pro Trp Ala Cys Glu Gln Gly Thr Pro Pro Ile
145                 150                 155                 160

Phe Ser Trp Met Ser Ala Ala Pro Thr Ser Leu Gly Leu Arg Thr Thr
                165                 170                 175

His Ser Ser Val Leu Ile Ile Thr Pro Arg Pro Gln Asp His Gly Thr
            180                 185                 190

Asn Leu Thr Cys Gln Val Lys Phe Pro Gly Ala Gly Val Thr Thr Glu
        195                 200                 205

Arg Thr Ile Gln Leu Asn Val Ser Tyr Ala Ser Gln Asn Pro Arg Thr
    210                 215                 220

Asp Ile Phe Leu Gly Asp Gly Ser Gly Arg Lys Ala Arg Lys Gln Gly
225                 230                 235                 240

Val Val Gln Gly Ser His His His His His His
                245                 250

<210>   233
<211>   250
<212>   PRT
<213>   Homo sapiens

<400>   233
Asp Pro Asn Phe Trp Leu Gln Val Gln Glu Ser Val Thr Val Gln Glu
1               5                   10                  15
```

```
Gly Leu Cys Val Leu Val Pro Cys Thr Phe Phe His Pro Ile Pro Tyr
            20                  25                  30

Tyr Asp Lys Asn Ser Pro Val His Gly Tyr Trp Phe Arg Glu Gly Ala
        35                  40                  45

Ile Ile Ser Arg Asp Ser Pro Val Ala Thr Asn Lys Leu Asp Gln Glu
    50                  55                  60

Val Gln Glu Glu Thr Gln Gly Arg Phe Arg Leu Leu Gly Asp Pro Ser
65                  70                  75                  80

Arg Asn Asn Cys Ser Leu Ser Ile Val Asp Ala Arg Arg Arg Asp Asn
                85                  90                  95

Gly Ser Tyr Phe Phe Arg Met Glu Arg Gly Ser Thr Lys Tyr Ser Tyr
            100                 105                 110

Lys Ser Pro Gln Leu Ser Val His Val Thr Asp Leu Thr His Arg Pro
        115                 120                 125

Lys Ile Leu Ile Pro Gly Thr Leu Glu Pro Gly His Ser Lys Asn Leu
    130                 135                 140

Thr Cys Ser Val Ser Trp Ala Cys Glu Gln Gly Thr Pro Pro Ile Phe
145                 150                 155                 160

Ser Trp Leu Ser Ala Ala Pro Thr Ser Leu Gly Pro Arg Thr Thr His
                165                 170                 175

Ser Ser Val Leu Ile Ile Thr Pro Arg Pro Gln Asp His Gly Thr Asn
            180                 185                 190

Leu Thr Cys Gln Val Lys Phe Ala Gly Ala Gly Val Thr Thr Glu Arg
        195                 200                 205

Thr Ile Gln Leu Asn Val Thr Tyr Val Pro Gln Asn Pro Thr Thr Gly
    210                 215                 220

Ile Phe Pro Gly Asp Gly Ser Gly Lys Gln Glu Thr Arg Ala Gly Val
225                 230                 235                 240

Val His Gly Ser His His His His His His
                245                 250

<210>   234
<211>   246
<212>   PRT
<213>   Macaca fascicularis

<400>   234
Asp Pro Arg Val Arg Leu Glu Val Gln Glu Ser Val Thr Val Gln Glu
1               5                   10                  15
```

Gly Leu Cys Val Leu Val Pro Cys Thr Phe Phe His Pro Val Pro Tyr
20 25 30

His Thr Arg Asn Ser Pro Val His Gly Tyr Trp Phe Arg Glu Gly Ala
35 40 45

Ile Val Ser Leu Asp Ser Pro Val Ala Thr Asn Lys Leu Asp Gln Glu
50 55 60

Val Gln Glu Glu Thr Gln Gly Arg Phe Arg Leu Leu Gly Asp Pro Ser
65 70 75 80

Arg Asn Asn Cys Ser Leu Ser Ile Val Asp Ala Arg Arg Arg Asp Asn
85 90 95

Gly Ser Tyr Phe Phe Arg Met Glu Lys Gly Ser Thr Lys Tyr Ser Tyr
100 105 110

Lys Ser Thr Gln Leu Ser Val His Val Thr Asp Leu Thr His Arg Pro
115 120 125

Gln Ile Leu Ile Pro Gly Ala Leu Asp Pro Asp His Ser Lys Asn Leu
130 135 140

Thr Cys Ser Val Pro Trp Ala Cys Glu Gln Gly Thr Pro Pro Ile Phe
145 150 155 160

Ser Trp Met Ser Ala Ala Pro Thr Ser Leu Gly Leu Arg Thr Thr His
165 170 175

Ser Ser Val Leu Ile Ile Thr Pro Arg Pro Gln Asp His Gly Thr Asn
180 185 190

Leu Thr Cys Gln Val Lys Phe Pro Gly Ala Gly Val Thr Thr Glu Arg
195 200 205

Thr Ile Gln Leu Asn Val Ser Tyr Ala Ser Gln Asn Pro Arg Thr Asp
210 215 220

Ile Phe Leu Gly Asp Gly Ser Gly Lys Gln Gly Val Val Gln Gly Ser
225 230 235 240

His His His His His His
245

<210> 235
<211> 230
<212> PRT
<213> Homo sapiens

<400> 235
Asp Pro Asn Phe Trp Leu Gln Val Gln Glu Ser Val Thr Val Gln Glu
1 5 10 15

```
Gly Leu Cys Val Leu Val Pro Cys Thr Phe Phe His Pro Ile Pro Tyr
            20                  25                  30

Tyr Asp Lys Asn Ser Pro Val His Gly Tyr Trp Phe Arg Glu Gly Ala
        35                  40                  45

Ile Ile Ser Arg Asp Ser Pro Val Ala Thr Asn Lys Leu Asp Gln Glu
    50                  55                  60

Val Gln Glu Glu Thr Gln Gly Arg Phe Arg Leu Leu Gly Asp Pro Ser
65                  70                  75                  80

Arg Asn Asn Cys Ser Leu Ser Ile Val Asp Ala Arg Arg Arg Asp Asn
                85                  90                  95

Gly Ser Tyr Phe Phe Arg Met Glu Arg Gly Ser Thr Lys Tyr Ser Tyr
            100                 105                 110

Lys Ser Pro Gln Leu Ser Val His Val Thr Asp Leu Thr His Arg Pro
        115                 120                 125

Lys Ile Leu Ile Pro Gly Thr Leu Glu Pro Gly His Ser Lys Asn Leu
    130                 135                 140

Thr Cys Ser Val Ser Trp Ala Cys Glu Gln Gly Thr Pro Pro Ile Phe
145                 150                 155                 160

Ser Trp Leu Ser Ala Ala Pro Thr Ser Leu Gly Pro Arg Thr Thr His
                165                 170                 175

Ser Ser Val Leu Ile Ile Thr Pro Arg Pro Gln Asp His Gly Thr Asn
            180                 185                 190

Leu Thr Cys Gln Val Lys Phe Ala Gly Ala Gly Val Thr Thr Glu Arg
        195                 200                 205

Thr Ile Gln Leu Asn Val Thr Tyr Val Pro Gln Asn Pro Thr Thr Gly
    210                 215                 220

His His His His His His
225                 230

<210>   236
<211>   5
<212>   PRT
<213>   Artificial Sequence

<220>
<223>   Description of Artificial Sequence: Synthetic peptide

<400>   236
Asp His Tyr Ile Asn
1               5
```

<210> 237
<211> 17
<212> PRT
<213> Artificial Sequence

<220>
<223> Description of Artificial Sequence: Synthetic peptide

<400> 237
Gln Ile Tyr Pro Gly Asp Gly Asn Thr Tyr Tyr Asn Gln Lys Phe Lys
1 5 10 15

Gly

<210> 238
<211> 9
<212> PRT
<213> Artificial Sequence

<220>
<223> Description of Artificial Sequence: Synthetic peptide

<400> 238
Asn Tyr Gly Asp Tyr Thr Ile Asp Phe
1 5

<210> 239
<211> 17
<212> PRT
<213> Artificial Sequence

<220>
<223> Description of Artificial Sequence: Synthetic peptide

<400> 239
Lys Ser Ser Gln Ser Leu Leu Tyr Ser Ser Asn Gln Lys Asn Tyr Leu
1 5 10 15

Ala

<210> 240
<211> 7
<212> PRT
<213> Artificial Sequence

<220>
<223> Description of Artificial Sequence: Synthetic peptide

<400> 240
Trp Ala Ser Thr Arg Glu Ser
1 5

<210> 241
<211> 8
<212> PRT
<213> Artificial Sequence

<220>
<223> Description of Artificial Sequence: Synthetic peptide

<400> 241
Gln Gln Tyr Tyr Arg Tyr His Thr
1 5

<210> 242
<211> 9
<212> PRT
<213> Artificial Sequence

<220>
<223> Description of Artificial Sequence: Synthetic peptide

<400> 242
Asn Met Gly Met Tyr Thr Ile Asp Phe
1 5

<210> 243
<211> 9
<212> PRT
<213> Artificial Sequence

<220>
<223> Description of Artificial Sequence: Synthetic peptide

<400> 243
Asn Met Gly Met Tyr Thr Leu Asp Phe
1 5

<210> 244
<211> 9
<212> PRT
<213> Artificial Sequence

<220>
<223> Description of Artificial Sequence: Synthetic peptide

<400> 244
Asn Tyr Gly Asp Tyr Thr Leu Asp Phe
1 5

<210> 245
<211> 118
<212> PRT
<213> Artificial Sequence

<220>
<223> Description of Artificial Sequence: Synthetic peptide

<400> 245
Glu Val Gln Leu Gln Gln Ser Gly Ala Glu Leu Ala Arg Pro Gly Ala
1 5 10 15

Ser Val Lys Leu Ser Cys Lys Ala Ser Gly Phe Thr Phe Thr Asp His
20 25 30

Tyr Ile Asn Trp Val Lys Gln Arg Thr Gly Gln Gly Leu Glu Trp Ile
35 40 45

Gly Gln Ile Tyr Pro Gly Asp Gly Asn Thr Tyr Tyr Asn Gln Lys Phe
50 55 60

Lys Gly Lys Ala Thr Leu Thr Ala Asp Lys Ser Ser Ser Thr Ala Tyr
65 70 75 80

Met Gln Leu Ser Ser Leu Thr Ser Glu Asp Ser Ala Val Tyr Phe Cys
85 90 95

Ala Pro Asn Tyr Gly Asp Tyr Thr Ile Asp Phe Trp Gly Gln Gly Thr
100 105 110

Ser Val Thr Val Ser Ser
115

<210> 246
<211> 118
<212> PRT
<213> Artificial Sequence

<220>
<223> Description of Artificial Sequence: Synthetic peptide

<400> 246
Glu Val Gln Leu Gln Gln Ser Gly Ala Glu Leu Ala Arg Pro Gly Ala
1 5 10 15

Ser Val Lys Leu Ser Cys Lys Ala Ser Gly Phe Thr Phe Thr Asp His
20 25 30

Tyr Ile Asn Trp Val Lys Gln Arg Thr Gly Gln Gly Leu Glu Trp Ile
35 40 45

Gly Gln Ile Tyr Pro Gly Asp Gly Asn Thr Tyr Tyr Asn Gln Lys Phe
50 55 60

Lys Gly Lys Ala Thr Leu Thr Ala Asp Lys Ser Ser Ser Thr Ala Tyr
65 70 75 80

Met Gln Leu Ser Ser Leu Thr Ser Glu Asp Ser Ala Val Tyr Phe Cys
85 90 95

Ala Pro Asn Met Gly Met Tyr Thr Ile Asp Phe Trp Gly Gln Gly Thr
100 105 110

Ser Val Thr Val Ser Ser
115

<210> 247
<211> 118
<212> PRT
<213> Artificial Sequence

<220>
<223> Description of Artificial Sequence: Synthetic peptide

<400> 247
Glu Val Gln Leu Gln Gln Ser Gly Ala Glu Leu Ala Arg Pro Gly Ala
1 5 10 15

Ser Val Lys Leu Ser Cys Lys Ala Ser Gly Phe Thr Phe Thr Asp His
20 25 30

Tyr Ile Asn Trp Val Lys Gln Arg Thr Gly Gln Gly Leu Glu Trp Ile
35 40 45

Gly Gln Ile Tyr Pro Gly Asp Gly Asn Thr Tyr Tyr Asn Gln Lys Phe
50 55 60

Lys Gly Lys Ala Thr Leu Thr Ala Asp Lys Ser Ser Ser Thr Ala Tyr
65 70 75 80

Met Gln Leu Ser Ser Leu Thr Ser Glu Asp Ser Ala Val Tyr Phe Cys
85 90 95

Ala Pro Asn Met Gly Met Tyr Thr Leu Asp Phe Trp Gly Gln Gly Thr
100 105 110

Ser Val Thr Val Ser Ser
115

<210> 248
<211> 118
<212> PRT
<213> Artificial Sequence

<220>
<223> Description of Artificial Sequence: Synthetic peptide

<400> 248
Glu Val Gln Leu Gln Gln Ser Gly Ala Glu Leu Ala Arg Pro Gly Ala
1 5 10 15

Ser Val Lys Leu Ser Cys Lys Ala Ser Gly Phe Thr Phe Thr Asp His
20 25 30

```
Tyr Ile Asn Trp Val Lys Gln Arg Thr Gly Gln Gly Leu Glu Trp Ile
        35                  40                  45

Gly Gln Ile Tyr Pro Gly Asp Gly Asn Thr Tyr Tyr Asn Gln Lys Phe
    50                  55                  60

Lys Gly Lys Ala Thr Leu Thr Ala Asp Lys Ser Ser Ser Thr Ala Tyr
65                  70                  75                  80

Met Gln Leu Ser Ser Leu Thr Ser Glu Asp Ser Ala Val Tyr Phe Cys
                85                  90                  95

Ala Pro Asn Met Gly Met Tyr Thr Leu Asp Phe Trp Gly Gln Gly Thr
            100                 105                 110

Ser Val Thr Val Ser Ser
        115

<210>   249
<211>   112
<212>   PRT
<213>   Artificial Sequence

<220>
<223>   Description of Artificial Sequence: Synthetic peptide

<400>   249
Asp Ile Val Met Ser Gln Ser Pro Ser Ser Leu Ala Val Ser Val Gly
1               5                   10                  15

Glu Lys Val Thr Met Ser Cys Lys Ser Ser Gln Ser Leu Leu Tyr Ser
            20                  25                  30

Ser Asn Gln Lys Asn Tyr Leu Ala Trp Tyr Gln Gln Lys Pro Gly Gln
        35                  40                  45

Ser Pro Lys Leu Leu Ile Tyr Trp Ala Ser Thr Arg Glu Ser Gly Val
    50                  55                  60

Pro Asp Arg Phe Thr Gly Ser Gly Ser Gly Thr Asp Phe Thr Leu Thr
65                  70                  75                  80

Ile Ser Ser Val Lys Ala Glu Asp Leu Ala Val Tyr Tyr Cys Gln Gln
                85                  90                  95

Tyr Tyr Arg Tyr His Thr Phe Gly Thr Gly Thr Lys Leu Glu Ile Lys
            100                 105                 110

<210>   250
<211>   717
<212>   PRT
<213>   Artificial Sequence
```

<220>
<223> Description of Artificial Sequence: Synthetic peptide

<400> 250

```
Met Ala Trp Val Trp Thr Leu Leu Phe Leu Met Ala Ala Ala Gln Ser
1               5                   10                  15

Ile Gln Ala Asp Ile Val Met Ser Gln Ser Pro Ser Ser Leu Ala Val
            20                  25                  30

Ser Val Gly Glu Lys Val Thr Met Ser Cys Lys Ser Ser Gln Ser Leu
        35                  40                  45

Leu Tyr Ser Ser Asn Gln Lys Asn Tyr Leu Ala Trp Tyr Gln Gln Lys
    50                  55                  60

Pro Gly Gln Ser Pro Lys Leu Leu Ile Tyr Trp Ala Ser Thr Arg Glu
65                  70                  75                  80

Ser Gly Val Pro Asp Arg Phe Thr Gly Ser Gly Ser Gly Thr Asp Phe
                85                  90                  95

Thr Leu Thr Ile Ser Ser Val Lys Ala Glu Asp Leu Ala Val Tyr Tyr
            100                 105                 110

Cys Gln Gln Tyr Tyr Arg Tyr His Thr Phe Gly Thr Gly Thr Lys Leu
        115                 120                 125

Glu Ile Lys Arg Thr Val Ala Ala Pro Ser Val Phe Ile Phe Pro Pro
    130                 135                 140

Ser Asp Glu Gln Leu Lys Ser Gly Thr Ala Ser Val Val Cys Leu Leu
145                 150                 155                 160

Asn Asn Phe Tyr Pro Arg Glu Ala Lys Val Gln Trp Lys Val Asp Asn
                165                 170                 175

Ala Leu Gln Ser Gly Asn Ser Gln Glu Ser Val Thr Glu Gln Asp Ser
            180                 185                 190

Lys Asp Ser Thr Tyr Ser Leu Ser Ser Thr Leu Thr Leu Ser Lys Ala
        195                 200                 205

Asp Tyr Glu Lys His Lys Val Tyr Ala Cys Glu Val Thr His Gln Gly
    210                 215                 220

Leu Ser Ser Pro Val Thr Lys Ser Phe Asn Arg Gly Glu Cys Gly Gly
225                 230                 235                 240

Ser Glu Gly Lys Ser Ser Gly Ser Gly Ser Glu Ser Lys Ser Thr Glu
                245                 250                 255

Gly Lys Ser Ser Gly Ser Gly Ser Glu Ser Lys Ser Thr Gly Gly Ser
            260                 265                 270
```

Glu Val Gln Leu Gln Gln Ser Gly Ala Glu Leu Ala Arg Pro Gly Ala
275 280 285

Ser Val Lys Leu Ser Cys Lys Ala Ser Gly Phe Thr Phe Thr Asp His
290 295 300

Tyr Ile Asn Trp Val Lys Gln Arg Thr Gly Gln Gly Leu Glu Trp Ile
305 310 315 320

Gly Gln Ile Tyr Pro Gly Asp Gly Asn Thr Tyr Tyr Asn Gln Lys Phe
325 330 335

Lys Gly Lys Ala Thr Leu Thr Ala Asp Lys Ser Ser Ser Thr Ala Tyr
340 345 350

Met Gln Leu Ser Ser Leu Thr Ser Glu Asp Ser Ala Val Tyr Phe Cys
355 360 365

Ala Pro Asn Tyr Gly Asp Tyr Thr Ile Asp Phe Trp Gly Gln Gly Thr
370 375 380

Ser Val Thr Val Ser Ser Ala Ser Thr Lys Gly Pro Ser Val Phe Pro
385 390 395 400

Leu Ala Pro Cys Ser Arg Ser Thr Ser Glu Ser Thr Ala Ala Leu Gly
405 410 415

Cys Leu Val Lys Asp Tyr Phe Pro Glu Pro Val Thr Val Ser Trp Asn
420 425 430

Ser Gly Ala Leu Thr Ser Gly Val His Thr Phe Pro Ala Val Leu Gln
435 440 445

Ser Ser Gly Leu Tyr Ser Leu Ser Ser Val Val Thr Val Pro Ser Ser
450 455 460

Ser Leu Gly Thr Lys Thr Tyr Thr Cys Asn Val Asp His Lys Pro Ser
465 470 475 480

Asn Thr Lys Val Asp Lys Arg Val Glu Ser Lys Tyr Gly Pro Pro Cys
485 490 495

Pro Pro Cys Pro Ala Pro Glu Ala Ala Gly Gly Pro Ser Val Phe Leu
500 505 510

Phe Pro Pro Lys Pro Lys Asp Thr Leu Met Ile Ser Arg Thr Pro Glu
515 520 525

Val Thr Cys Val Val Val Asp Val Ser Gln Glu Asp Pro Glu Val Gln
530 535 540

Phe Asn Trp Tyr Val Asp Gly Val Glu Val His Asn Ala Lys Thr Lys
545 550 555 560

```
Pro Arg Glu Glu Gln Phe Asn Ser Thr Tyr Arg Val Val Ser Val Leu
                565                 570                 575

Thr Val Leu His Gln Asp Trp Leu Asn Gly Lys Glu Tyr Lys Cys Lys
            580                 585                 590

Val Ser Asn Lys Gly Leu Pro Ser Ser Ile Glu Lys Thr Ile Ser Lys
        595                 600                 605

Ala Lys Gly Gln Pro Arg Glu Pro Gln Val Tyr Thr Leu Pro Pro Ser
    610                 615                 620

Gln Glu Glu Met Thr Lys Asn Gln Val Ser Leu Ser Cys Ala Val Lys
625                 630                 635                 640

Gly Phe Tyr Pro Ser Asp Ile Ala Val Glu Trp Glu Ser Asn Gly Gln
                645                 650                 655

Pro Glu Asn Asn Tyr Lys Thr Thr Pro Pro Val Leu Asp Ser Asp Gly
            660                 665                 670

Ser Phe Phe Leu Val Ser Arg Leu Thr Val Asp Lys Ser Arg Trp Gln
        675                 680                 685

Glu Gly Asn Val Phe Ser Cys Ser Val Met His Glu Ala Leu His Asn
    690                 695                 700

Arg Phe Thr Gln Lys Ser Leu Ser Leu Ser Leu Gly Lys
705                 710                 715

<210>   251
<211>   719
<212>   PRT
<213>   Artificial Sequence

<220>
<223>   Description of Artificial Sequence: Synthetic peptide

<400>   251
Met Ala Trp Val Trp Thr Leu Leu Phe Leu Met Ala Ala Ala Gln Ser
1               5                   10                  15

Ile Gln Ala Asp Ile Val Met Thr Gln Ser Pro Asp Ser Leu Ala Val
            20                  25                  30

Ser Leu Gly Glu Arg Ala Thr Ile Asn Cys Lys Ser Ser Gln Thr Val
        35                  40                  45

Phe Tyr Ser Ser Asn Asn Lys Asn Tyr Leu Ala Trp Tyr Gln Gln Lys
    50                  55                  60

Pro Gly Gln Pro Pro Lys Leu Leu Ile Ser Trp Ala Ser Thr Arg Lys
65                  70                  75                  80
```

```
Ser Gly Val Pro Asp Arg Phe Ser Gly Ser Gly Ser Gly Thr Asp Phe
                85                  90                  95

Thr Leu Thr Val Ser Ser Leu Gln Ala Glu Asp Val Ala Val Tyr Tyr
            100                 105                 110

Cys Gln His Tyr Tyr Ser Thr Pro Tyr Thr Phe Gly Gln Gly Thr Lys
        115                 120                 125

Leu Glu Ile Lys Arg Thr Val Ala Ala Pro Ser Val Phe Ile Phe Pro
    130                 135                 140

Pro Ser Asp Glu Gln Leu Lys Ser Gly Thr Ala Ser Val Val Cys Leu
145                 150                 155                 160

Leu Asn Asn Phe Tyr Pro Arg Glu Ala Lys Val Gln Trp Lys Val Asp
                165                 170                 175

Asn Ala Leu Gln Ser Gly Asn Ser Gln Glu Ser Val Thr Glu Gln Asp
            180                 185                 190

Ser Lys Asp Ser Thr Tyr Ser Leu Ser Ser Thr Leu Thr Leu Ser Lys
        195                 200                 205

Ala Asp Tyr Glu Lys His Lys Val Tyr Ala Cys Glu Val Thr His Gln
    210                 215                 220

Gly Leu Ser Ser Pro Val Thr Lys Ser Phe Asn Arg Gly Glu Cys Gly
225                 230                 235                 240

Gly Ser Glu Gly Lys Ser Ser Gly Ser Gly Ser Glu Ser Lys Ser Thr
                245                 250                 255

Glu Gly Lys Ser Ser Gly Ser Gly Ser Glu Ser Lys Ser Thr Gly Gly
            260                 265                 270

Ser Glu Val Gln Leu Val Glu Ser Gly Gly Gly Leu Val Gln Pro Gly
        275                 280                 285

Arg Ser Leu Arg Leu Ser Cys Ala Ala Ser Gly Phe Thr Phe Asp Asp
    290                 295                 300

Tyr Ala Met His Trp Val Arg Gln Ala Pro Gly Lys Gly Leu Glu Trp
305                 310                 315                 320

Val Ser Gly Ile Gly Trp Ser Gly Gly Ser Ile Val Tyr Ala Asp Ser
                325                 330                 335

Val Lys Gly Arg Phe Thr Ile Ser Arg Asp Asn Ala Lys Asn Ser Leu
            340                 345                 350

Tyr Leu Gln Met Asn Ser Leu Arg Ala Glu Asp Thr Ala Leu Tyr Tyr
        355                 360                 365
```

```
Cys Ala Lys Asp Ser Pro Tyr Gly Asp Phe Phe Asp Tyr Trp Gly Gln
    370                 375                 380

Gly Thr Leu Val Thr Val Ser Ser Ala Ser Thr Lys Gly Pro Ser Val
385                 390                 395                 400

Phe Pro Leu Ala Pro Cys Ser Arg Ser Thr Ser Glu Ser Thr Ala Ala
                405                 410                 415

Leu Gly Cys Leu Val Lys Asp Tyr Phe Pro Glu Pro Val Thr Val Ser
            420                 425                 430

Trp Asn Ser Gly Ala Leu Thr Ser Gly Val His Thr Phe Pro Ala Val
        435                 440                 445

Leu Gln Ser Ser Gly Leu Tyr Ser Leu Ser Ser Val Val Thr Val Pro
    450                 455                 460

Ser Ser Ser Leu Gly Thr Lys Thr Tyr Thr Cys Asn Val Asp His Lys
465                 470                 475                 480

Pro Ser Asn Thr Lys Val Asp Lys Arg Val Glu Ser Lys Tyr Gly Pro
                485                 490                 495

Pro Cys Pro Pro Cys Pro Ala Pro Glu Ala Ala Gly Gly Pro Ser Val
            500                 505                 510

Phe Leu Phe Pro Pro Lys Pro Lys Asp Thr Leu Met Ile Ser Arg Thr
        515                 520                 525

Pro Glu Val Thr Cys Val Val Val Asp Val Ser Gln Glu Asp Pro Glu
    530                 535                 540

Val Gln Phe Asn Trp Tyr Val Asp Gly Val Glu Val His Asn Ala Lys
545                 550                 555                 560

Thr Lys Pro Arg Glu Glu Gln Phe Asn Ser Thr Tyr Arg Val Val Ser
                565                 570                 575

Val Leu Thr Val Leu His Gln Asp Trp Leu Asn Gly Lys Glu Tyr Lys
            580                 585                 590

Cys Lys Val Ser Asn Lys Gly Leu Pro Ser Ser Ile Glu Lys Thr Ile
        595                 600                 605

Ser Lys Ala Lys Gly Gln Pro Arg Glu Pro Gln Val Tyr Thr Leu Pro
    610                 615                 620

Pro Ser Gln Glu Glu Met Thr Lys Asn Gln Val Ser Leu Trp Cys Leu
625                 630                 635                 640

Val Lys Gly Phe Tyr Pro Ser Asp Ile Ala Val Glu Trp Glu Ser Asn
                645                 650                 655
```

Gly Gln Pro Glu Asn Asn Tyr Lys Thr Thr Pro Pro Val Leu Asp Ser
660 665 670

Asp Gly Ser Phe Phe Leu Tyr Ser Arg Leu Thr Val Asp Lys Ser Arg
675 680 685

Trp Gln Glu Gly Asn Val Phe Ser Cys Ser Val Met His Glu Ala Leu
690 695 700

His Asn His Tyr Thr Gln Lys Ser Leu Ser Leu Ser Leu Gly Lys
705 710 715

<210> 252
<211> 713
<212> PRT
<213> Artificial Sequence

<220>
<223> Description of Artificial Sequence: Synthetic peptide

<400> 252
Met Ala Trp Val Trp Thr Leu Leu Phe Leu Met Ala Ala Ala Gln Ser
1 5 10 15

Ile Gln Ala Glu Ile Val Leu Thr Gln Ser Pro Gly Thr Leu Ser Leu
20 25 30

Ser Pro Gly Glu Arg Ala Thr Leu Ser Cys Arg Ala Ser Gln Ser Val
35 40 45

Ser Ser Ser Tyr Leu Ala Trp Tyr Gln Gln Lys Pro Gly Gln Ala Pro
50 55 60

Arg Leu Leu Ile Tyr Gly Ala Ser Ser Arg Ala Thr Gly Ile Pro Asp
65 70 75 80

Arg Phe Ser Gly Ser Gly Ser Gly Thr Asp Phe Thr Leu Thr Ile Ser
85 90 95

Arg Leu Glu Pro Glu Asp Phe Ala Val Tyr Tyr Cys Gln Gln Asp Tyr
100 105 110

Gly Phe Pro Trp Thr Phe Gly Gln Gly Thr Lys Val Glu Ile Lys Arg
115 120 125

Thr Val Ala Ala Pro Ser Val Phe Ile Phe Pro Pro Ser Asp Glu Gln
130 135 140

Leu Lys Ser Gly Thr Ala Ser Val Val Cys Leu Leu Asn Asn Phe Tyr
145 150 155 160

Pro Arg Glu Ala Lys Val Gln Trp Lys Val Asp Asn Ala Leu Gln Ser
165 170 175

```
Gly Asn Ser Gln Glu Ser Val Thr Glu Gln Asp Ser Lys Asp Ser Thr
            180                 185                 190

Tyr Ser Leu Ser Ser Thr Leu Thr Leu Ser Lys Ala Asp Tyr Glu Lys
        195                 200                 205

His Lys Val Tyr Ala Cys Glu Val Thr His Gln Gly Leu Ser Ser Pro
    210                 215                 220

Val Thr Lys Ser Phe Asn Arg Gly Glu Cys Gly Gly Ser Glu Gly Lys
225                 230                 235                 240

Ser Ser Gly Ser Gly Ser Glu Ser Lys Ser Thr Glu Gly Lys Ser Ser
                245                 250                 255

Gly Ser Gly Ser Glu Ser Lys Ser Thr Gly Gly Ser Glu Val Gln Leu
            260                 265                 270

Val Gln Ser Gly Ala Glu Val Lys Lys Pro Gly Glu Ser Leu Lys Ile
        275                 280                 285

Ser Cys Lys Gly Ser Gly Tyr Ser Phe Thr Ser Tyr Trp Ile Ser Trp
    290                 295                 300

Val Arg Gln Met Pro Gly Lys Gly Leu Glu Trp Met Gly Ile Ile Asp
305                 310                 315                 320

Pro Ser Asp Ser Asp Thr Arg Tyr Ser Pro Ser Phe Gln Gly Gln Val
                325                 330                 335

Thr Ile Ser Ala Asp Lys Ser Ile Ser Thr Ala Tyr Leu Gln Trp Ser
            340                 345                 350

Ser Leu Lys Ala Ser Asp Thr Ala Met Tyr Tyr Cys Ala Arg Gly Asp
        355                 360                 365

Gly Ser Thr Asp Leu Asp Tyr Trp Gly Gln Gly Thr Leu Val Thr Val
    370                 375                 380

Ser Ser Ala Ser Thr Lys Gly Pro Ser Val Phe Pro Leu Ala Pro Cys
385                 390                 395                 400

Ser Arg Ser Thr Ser Glu Ser Thr Ala Ala Leu Gly Cys Leu Val Lys
                405                 410                 415

Asp Tyr Phe Pro Glu Pro Val Thr Val Ser Trp Asn Ser Gly Ala Leu
            420                 425                 430

Thr Ser Gly Val His Thr Phe Pro Ala Val Leu Gln Ser Ser Gly Leu
        435                 440                 445

Tyr Ser Leu Ser Ser Val Val Thr Val Pro Ser Ser Ser Leu Gly Thr
    450                 455                 460
```

Lys Thr Tyr Thr Cys Asn Val Asp His Lys Pro Ser Asn Thr Lys Val
465 470 475 480

Asp Lys Arg Val Glu Ser Lys Tyr Gly Pro Pro Cys Pro Pro Cys Pro
485 490 495

Ala Pro Glu Ala Ala Gly Gly Pro Ser Val Phe Leu Phe Pro Pro Lys
500 505 510

Pro Lys Asp Thr Leu Met Ile Ser Arg Thr Pro Glu Val Thr Cys Val
515 520 525

Val Val Asp Val Ser Gln Glu Asp Pro Glu Val Gln Phe Asn Trp Tyr
530 535 540

Val Asp Gly Val Glu Val His Asn Ala Lys Thr Lys Pro Arg Glu Glu
545 550 555 560

Gln Phe Asn Ser Thr Tyr Arg Val Val Ser Val Leu Thr Val Leu His
565 570 575

Gln Asp Trp Leu Asn Gly Lys Glu Tyr Lys Cys Lys Val Ser Asn Lys
580 585 590

Gly Leu Pro Ser Ser Ile Glu Lys Thr Ile Ser Lys Ala Lys Gly Gln
595 600 605

Pro Arg Glu Pro Gln Val Tyr Thr Leu Pro Pro Ser Gln Glu Glu Met
610 615 620

Thr Lys Asn Gln Val Ser Leu Trp Cys Leu Val Lys Gly Phe Tyr Pro
625 630 635 640

Ser Asp Ile Ala Val Glu Trp Glu Ser Asn Gly Gln Pro Glu Asn Asn
645 650 655

Tyr Lys Thr Thr Pro Pro Val Leu Asp Ser Asp Gly Ser Phe Phe Leu
660 665 670

Tyr Ser Arg Leu Thr Val Asp Lys Ser Arg Trp Gln Glu Gly Asn Val
675 680 685

Phe Ser Cys Ser Val Met His Glu Ala Leu His Asn His Tyr Thr Gln
690 695 700

Lys Ser Leu Ser Leu Ser Leu Gly Lys
705 710

<210> 253
<211> 5
<212> PRT
<213> Artificial Sequence

<220>
<223> Description of Artificial Sequence: Synthetic peptide

<400> 253
Ser Tyr Trp Met Thr
1 5

<210> 254
<211> 5
<212> PRT
<213> Artificial Sequence

<220>
<223> Description of Artificial Sequence: Synthetic peptide

<400> 254
Asn Tyr Tyr Trp Ser
1 5

<210> 255
<211> 5
<212> PRT
<213> Artificial Sequence

<220>
<223> Description of Artificial Sequence: Synthetic peptide

<400> 255
Asn Tyr Trp Met Ser
1 5

<210> 256
<211> 17
<212> PRT
<213> Artificial Sequence

<220>
<223> Description of Artificial Sequence: Synthetic peptide

<400> 256
Asn Ile Lys Gln Asp Gly Ser Glu Arg Tyr Tyr Val Asp Ser Val Lys
1 5 10 15

Gly

<210> 257
<211> 16
<212> PRT
<213> Artificial Sequence

<220>
<223> Description of Artificial Sequence: Synthetic peptide

<400> 257
His Ile Phe Ser Thr Gly His Ile Asn Tyr Asp Ser Ser Leu Lys Ser
1 5 10 15

<210> 258
<211> 17
<212> PRT
<213> Artificial Sequence

<220>
<223> Description of Artificial Sequence: Synthetic peptide

<400> 258
Ser Ile Lys Arg Asp Gly Ser Asp Lys Tyr Tyr Val Asp Ser Val Lys
1 5 10 15

Gly

<210> 259
<211> 14
<212> PRT
<213> Artificial Sequence

<220>
<223> Description of Artificial Sequence: Synthetic peptide

<400> 259
Glu Val Gly Tyr Asn Trp Asn Gln Gly Gly Tyr Phe Asp Tyr
1 5 10

<210> 260
<211> 9
<212> PRT
<213> Artificial Sequence

<220>
<223> Description of Artificial Sequence: Synthetic peptide

<400> 260
Asp Asn Gly Ala Ala Leu Phe Asp Phe
1 5

<210> 261
<211> 5
<212> PRT
<213> Artificial Sequence

<220>
<223> Description of Artificial Sequence: Synthetic peptide

<400> 261
Gly Glu Phe Asp Tyr
1 5

<210> 262
<211> 123
<212> PRT
<213> Artificial Sequence

<220>
<223> Description of Artificial Sequence: Synthetic peptide

<400> 262
Glu Val Gln Leu Val Glu Ser Gly Gly Gly Leu Val Gln Pro Gly Gly
1 5 10 15

Ser Leu Arg Leu Ser Cys Val Val Ser Gly Phe Thr Phe Ser Ser Tyr
20 25 30

Trp Met Thr Trp Val Arg Gln Ala Pro Gly Lys Gly Leu Glu Trp Val
35 40 45

Ala Asn Ile Lys Gln Asp Gly Ser Glu Arg Tyr Tyr Val Asp Ser Val
50 55 60

Lys Gly Arg Phe Thr Ile Ser Arg Asp Ser Ala Lys Asn Ser Leu Tyr
65 70 75 80

Leu Gln Met Asn Ser Leu Arg Ala Glu Asp Thr Ala Val Tyr Tyr Cys
85 90 95

Ala Arg Glu Val Gly Tyr Asn Trp Asn Gln Gly Gly Tyr Phe Asp Tyr
100 105 110

Trp Gly Gln Gly Thr Leu Val Thr Val Ser Ser
115 120

<210> 263
<211> 117
<212> PRT
<213> Artificial Sequence

<220>
<223> Description of Artificial Sequence: Synthetic peptide

<400> 263
Gln Val Gln Leu Gln Glu Ser Gly Pro Gly Leu Val Lys Pro Ser Glu
1 5 10 15

Thr Leu Ser Leu Thr Cys Ser Val Ser Gly Gly Ser Ile Arg Asn Tyr
20 25 30

Tyr Trp Ser Trp Ile Arg Gln Ser Ala Gly Lys Glu Leu Glu Trp Phe
35 40 45

Gly His Ile Phe Ser Thr Gly His Ile Asn Tyr Asp Ser Ser Leu Lys
50 55 60

Ser Arg Val Thr Met Ser Val Asp Thr Ser Asn Asn Gln Phe Ser Leu
65 70 75 80

Lys Leu Arg Ser Val Thr Ala Ala Asp Thr Ala Val Tyr Tyr Cys Ala
85 90 95

Arg Asp Asn Gly Ala Ala Leu Phe Asp Phe Trp Gly Gln Gly Thr Leu
100 105 110

Val Thr Val Ser Ser
115

<210> 264
<211> 114
<212> PRT
<213> Artificial Sequence

<220>
<223> Description of Artificial Sequence: Synthetic peptide

<400> 264
Gln Val Gln Leu Val Glu Ser Gly Gly Gly Leu Val Gln Pro Gly Gly
1 5 10 15

Ser Leu Arg Leu Ser Cys Ala Ala Ser Gly Ile Thr Phe Ser Asn Tyr
20 25 30

Trp Met Ser Trp Val Arg Gln Ala Pro Gly Lys Gly Leu Glu Trp Val
35 40 45

Ala Ser Ile Lys Arg Asp Gly Ser Asp Lys Tyr Tyr Val Asp Ser Val
50 55 60

Lys Gly Arg Phe Thr Ile Ser Arg Asp Asn Ala Lys Asn Ser Leu Ser
65 70 75 80

Leu Gln Met His Ser Leu Arg Ala Glu Asp Thr Ala Val Tyr Tyr Cys
85 90 95

Ala Lys Gly Glu Phe Asp Tyr Trp Gly Gln Gly Thr Leu Val Thr Val
100 105 110

Ser Ser

<210> 265
<211> 16
<212> PRT
<213> Artificial Sequence

<220>
<223> Description of Artificial Sequence: Synthetic peptide

<400> 265
Arg Ser Ser Gln Ser Leu Leu His Ser Asp Gly Tyr Asn Tyr Leu Asp
1 5 10 15

<210> 266
<211> 16
<212> PRT
<213> Artificial Sequence

<220>
<223> Description of Artificial Sequence: Synthetic peptide

<400> 266
Arg Ser Ser Gln Ser Leu Val Tyr Ser Asp Gly Asn Thr Tyr Leu Asn
1 5 10 15

<210> 267
<211> 7
<212> PRT
<213> Artificial Sequence

<220>
<223> Description of Artificial Sequence: Synthetic peptide

<400> 267
Ser Asp Asn Gln Arg Pro Ser
1 5

<210> 268
<211> 7
<212> PRT
<213> Artificial Sequence

<220>
<223> Description of Artificial Sequence: Synthetic peptide

<400> 268
Lys Val Ser Thr Arg Asp Ser
1 5

<210> 269
<211> 9
<212> PRT
<213> Artificial Sequence

<220>
<223> Description of Artificial Sequence: Synthetic peptide

<400> 269
Met Gln Val Leu Gln Thr Pro Trp Thr
1 5

<210> 270
<211> 9

<212> PRT
<213> Artificial Sequence

<220>
<223> Description of Artificial Sequence: Synthetic peptide

<400> 270
Leu Gln Gly Thr His Trp Pro Trp Thr
1 5

<210> 271
<211> 112
<212> PRT
<213> Artificial Sequence

<220>
<223> Description of Artificial Sequence: Synthetic peptide

<400> 271
Asp Ile Val Met Thr Gln Ser Pro Leu Ser Leu Pro Val Thr Pro Gly
1 5 10 15

Glu Pro Ala Ser Ile Ser Cys Arg Ser Ser Gln Ser Leu Leu His Ser
20 25 30

Asp Gly Tyr Asn Tyr Leu Asp Trp Tyr Leu Gln Lys Ser Gly Gln Ser
35 40 45

Pro Gln Leu Leu Ile Tyr Leu Gly Ser Tyr Arg Ala Ser Gly Val Pro
50 55 60

Asp Arg Phe Ser Gly Ser Gly Ser Gly Thr Asp Phe Thr Leu Lys Ile
65 70 75 80

Ser Arg Val Glu Ala Glu Asp Val Gly Ile Tyr Tyr Cys Met Gln Val
85 90 95

Leu Gln Thr Pro Trp Thr Phe Gly Gln Gly Thr Lys Val Glu Ile Lys
100 105 110

<210> 272
<211> 110
<212> PRT
<213> Artificial Sequence

<220>
<223> Description of Artificial Sequence: Synthetic peptide

<400> 272
Gln Ser Val Leu Thr Gln Pro Pro Ser Ala Ser Gly Thr Pro Gly Gln
1 5 10 15

Arg Val Thr Ile Ser Cys Ser Gly Ser Ser Ser Asn Ile Gly Ser Asn
20 25 30

```
Ile Val Asn Trp Tyr Gln Gln Phe Pro Gly Thr Ala Pro Lys Leu Leu
        35                  40                  45

Leu Tyr Ser Asp Asn Gln Arg Pro Ser Gly Val Pro Asp Arg Phe Ser
    50                  55                  60

Gly Ser Lys Ser Gly Thr Ser Ala Ser Leu Ala Ile Ser Gly Leu Gln
65                  70                  75                  80

Ser Glu Asp Glu Ala Asp Tyr Tyr Cys Ala Ala Trp Asp Asp Ser Leu
                85                  90                  95

Asn Gly Pro Val Phe Gly Thr Gly Thr Lys Val Thr Val Leu
            100                 105                 110

<210>   273
<211>   112
<212>   PRT
<213>   Artificial Sequence

<220>
<223>   Description of Artificial Sequence: Synthetic peptide

<400>   273
Asp Val Val Met Thr Gln Ser Pro Leu Ser Leu Pro Val Thr Leu Gly
1               5                   10                  15

Gln Pro Ala Ser Ile Ser Cys Arg Ser Ser Gln Ser Leu Val Tyr Ser
            20                  25                  30

Asp Gly Asn Thr Tyr Leu Asn Trp Phe Gln Gln Arg Pro Gly Gln Ser
        35                  40                  45

Pro Arg Arg Leu Ile Tyr Lys Val Ser Thr Arg Asp Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Val Ala Val Tyr Tyr Cys Leu Gln Gly
                85                  90                  95

Thr His Trp Pro Trp Thr Phe Gly Gln Gly Thr Lys Val Glu Ile Lys
            100                 105                 110

<210>   274
<211>   255
<212>   PRT
<213>   Artificial Sequence

<220>
<223>   Description of Artificial Sequence: Synthetic peptide
```

<400> 274

```
Asp Ile Val Met Thr Gln Ser Pro Leu Ser Leu Pro Val Thr Pro Gly
1               5                   10                  15

Glu Pro Ala Ser Ile Ser Cys Arg Ser Ser Gln Ser Leu Leu His Ser
            20                  25                  30

Asp Gly Tyr Asn Tyr Leu Asp Trp Tyr Leu Gln Lys Ser Gly Gln Ser
        35                  40                  45

Pro Gln Leu Leu Ile Tyr Leu Gly Ser Tyr Arg Ala Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Val Gly Ile Tyr Tyr Cys Met Gln Val
                85                  90                  95

Leu Gln Thr Pro Trp Thr Phe Gly Gln Gly Thr Lys Val Glu Ile Lys
            100                 105                 110

Gly Gly Ser Glu Gly Lys Ser Ser Gly Ser Gly Ser Glu Ser Lys Ser
        115                 120                 125

Thr Gly Gly Ser Glu Val Gln Leu Val Glu Ser Gly Gly Gly Leu Val
    130                 135                 140

Gln Pro Gly Gly Ser Leu Arg Leu Ser Cys Val Val Ser Gly Phe Thr
145                 150                 155                 160

Phe Ser Ser Tyr Trp Met Thr Trp Val Arg Gln Ala Pro Gly Lys Gly
                165                 170                 175

Leu Glu Trp Val Ala Asn Ile Lys Gln Asp Gly Ser Glu Arg Tyr Tyr
            180                 185                 190

Val Asp Ser Val Lys Gly Arg Phe Thr Ile Ser Arg Asp Ser Ala Lys
        195                 200                 205

Asn Ser Leu Tyr Leu Gln Met Asn Ser Leu Arg Ala Glu Asp Thr Ala
    210                 215                 220

Val Tyr Tyr Cys Ala Arg Glu Val Gly Tyr Asn Trp Asn Gln Gly Gly
225                 230                 235                 240

Tyr Phe Asp Tyr Trp Gly Gln Gly Thr Leu Val Thr Val Ser Ser
                245                 250                 255
```

<210> 275
<211> 247
<212> PRT
<213> Artificial Sequence

<220>
<223> Description of Artificial Sequence: Synthetic peptide

<400> 275
```
Gln Ser Val Leu Thr Gln Pro Pro Ser Ala Ser Gly Thr Pro Gly Gln
1               5                   10                  15

Arg Val Thr Ile Ser Cys Ser Gly Ser Ser Ser Asn Ile Gly Ser Asn
            20                  25                  30

Ile Val Asn Trp Tyr Gln Gln Phe Pro Gly Thr Ala Pro Lys Leu Leu
        35                  40                  45

Leu Tyr Ser Asp Asn Gln Arg Pro Ser Gly Val Pro Asp Arg Phe Ser
    50                  55                  60

Gly Ser Lys Ser Gly Thr Ser Ala Ser Leu Ala Ile Ser Gly Leu Gln
65                  70                  75                  80

Ser Glu Asp Glu Ala Asp Tyr Tyr Cys Ala Ala Trp Asp Asp Ser Leu
                85                  90                  95

Asn Gly Pro Val Phe Gly Thr Gly Thr Lys Val Thr Val Leu Gly Gly
            100                 105                 110

Ser Glu Gly Lys Ser Ser Gly Ser Gly Ser Glu Ser Lys Ser Thr Gly
        115                 120                 125

Gly Ser Gln Val Gln Leu Gln Glu Ser Gly Pro Gly Leu Val Lys Pro
    130                 135                 140

Ser Glu Thr Leu Ser Leu Thr Cys Ser Val Ser Gly Gly Ser Ile Arg
145                 150                 155                 160

Asn Tyr Tyr Trp Ser Trp Ile Arg Gln Ser Ala Gly Lys Glu Leu Glu
                165                 170                 175

Trp Phe Gly His Ile Phe Ser Thr Gly His Ile Asn Tyr Asp Ser Ser
            180                 185                 190

Leu Lys Ser Arg Val Thr Met Ser Val Asp Thr Ser Asn Asn Gln Phe
        195                 200                 205

Ser Leu Lys Leu Arg Ser Val Thr Ala Ala Asp Thr Ala Val Tyr Tyr
    210                 215                 220

Cys Ala Arg Asp Asn Gly Ala Ala Leu Phe Asp Phe Trp Gly Gln Gly
225                 230                 235                 240

Thr Leu Val Thr Val Ser Ser
                245
```

<210> 276
<211> 246

<212> PRT
<213> Artificial Sequence

<220>
<223> Description of Artificial Sequence: Synthetic peptide

<400> 276
Asp Val Val Met Thr Gln Ser Pro Leu Ser Leu Pro Val Thr Leu Gly
1 5 10 15

Gln Pro Ala Ser Ile Ser Cys Arg Ser Ser Gln Ser Leu Val Tyr Ser
20 25 30

Asp Gly Asn Thr Tyr Leu Asn Trp Phe Gln Gln Arg Pro Gly Gln Ser
35 40 45

Pro Arg Arg Leu Ile Tyr Lys Val Ser Thr Arg Asp Ser Gly Val Pro
50 55 60

Asp Arg Phe Ser Gly Ser Gly Ser Gly Thr Asp Phe Thr Leu Lys Ile
65 70 75 80

Ser Arg Val Glu Ala Glu Asp Val Ala Val Tyr Tyr Cys Leu Gln Gly
85 90 95

Thr His Trp Pro Trp Thr Phe Gly Gln Gly Thr Lys Val Glu Ile Lys
100 105 110

Gly Gly Ser Glu Gly Lys Ser Ser Gly Ser Gly Ser Glu Ser Lys Ser
115 120 125

Thr Gly Gly Ser Gln Val Gln Leu Val Glu Ser Gly Gly Gly Leu Val
130 135 140

Gln Pro Gly Gly Ser Leu Arg Leu Ser Cys Ala Ala Ser Gly Ile Thr
145 150 155 160

Phe Ser Asn Tyr Trp Met Ser Trp Val Arg Gln Ala Pro Gly Lys Gly
165 170 175

Leu Glu Trp Val Ala Ser Ile Lys Arg Asp Gly Ser Asp Lys Tyr Tyr
180 185 190

Val Asp Ser Val Lys Gly Arg Phe Thr Ile Ser Arg Asp Asn Ala Lys
195 200 205

Asn Ser Leu Ser Leu Gln Met His Ser Leu Arg Ala Glu Asp Thr Ala
210 215 220

Val Tyr Tyr Cys Ala Lys Gly Glu Phe Asp Tyr Trp Gly Gln Gly Thr
225 230 235 240

Leu Val Thr Val Ser Ser
245

```
<210>   277
<211>   232
<212>   PRT
<213>   Homo sapiens

<400>   277
Glu Pro Lys Ser Ser Asp Lys Thr His Thr Cys Pro Pro Cys Pro Ala
1               5                   10                  15

Pro Glu Leu Leu Gly Gly Pro Ser Val Phe Leu Phe Pro Pro Lys Pro
            20                  25                  30

Lys Asp Thr Leu Met Ile Ser Arg Thr Pro Glu Val Thr Cys Val Val
        35                  40                  45

Val Asp Val Ser His Glu Asp Pro Glu Val Lys Phe Asn Trp Tyr Val
    50                  55                  60

Asp Gly Val Glu Val His Asn Ala Lys Thr Lys Pro Arg Glu Glu Gln
65                  70                  75                  80

Tyr Asn Ser Thr Tyr Arg Val Val Ser Val Leu Thr Val Leu His Gln
                85                  90                  95

Asp Trp Leu Asn Gly Lys Glu Tyr Lys Cys Lys Val Ser Asn Lys Ala
            100                 105                 110

Leu Pro Ala Pro Ile Glu Lys Thr Ile Ser Lys Ala Lys Gly Gln Pro
        115                 120                 125

Arg Glu Pro Gln Val Tyr Thr Leu Pro Pro Ser Arg Glu Glu Met Thr
    130                 135                 140

Lys Asn Gln Val Ser Leu Thr Cys Leu Val Lys Gly Phe Tyr Pro Ser
145                 150                 155                 160

Asp Ile Ala Val Glu Trp Glu Ser Asn Gly Gln Pro Glu Asn Asn Tyr
                165                 170                 175

Lys Thr Thr Pro Pro Val Leu Asp Ser Asp Gly Ser Phe Phe Leu Tyr
            180                 185                 190

Ser Lys Leu Thr Val Asp Lys Ser Arg Trp Gln Gln Gly Asn Val Phe
        195                 200                 205

Ser Cys Ser Val Met His Glu Ala Leu His Asn His Tyr Thr Gln Lys
    210                 215                 220

Ser Leu Ser Leu Ser Pro Gly Lys
225                 230

<210>   278
<211>   232
```

<212> PRT
<213> Homo sapiens

<400> 278

```
Glu Pro Lys Ser Cys Asp Lys Thr His Thr Cys Pro Pro Cys Pro Ala
1               5                   10                  15

Pro Glu Leu Leu Gly Gly Pro Ser Val Phe Leu Phe Pro Pro Lys Pro
            20                  25                  30

Lys Asp Thr Leu Met Ile Ser Arg Thr Pro Glu Val Thr Cys Val Val
        35                  40                  45

Val Asp Val Ser His Glu Asp Pro Glu Val Lys Phe Asn Trp Tyr Val
    50                  55                  60

Asp Gly Val Glu Val His Asn Ala Lys Thr Lys Pro Arg Glu Glu Gln
65                  70                  75                  80

Tyr Asn Ser Thr Tyr Arg Val Val Ser Val Leu Thr Val Leu His Gln
                85                  90                  95

Asp Trp Leu Asn Gly Lys Glu Tyr Lys Cys Lys Val Ser Asn Lys Ala
            100                 105                 110

Leu Pro Ala Pro Ile Glu Lys Thr Ile Ser Lys Ala Lys Gly Gln Pro
        115                 120                 125

Arg Glu Pro Gln Val Tyr Thr Leu Pro Pro Ser Arg Glu Glu Met Thr
    130                 135                 140

Lys Asn Gln Val Ser Leu Thr Cys Leu Val Lys Gly Phe Tyr Pro Ser
145                 150                 155                 160

Asp Ile Ala Val Glu Trp Glu Ser Asn Gly Gln Pro Glu Asn Asn Tyr
                165                 170                 175

Lys Thr Thr Pro Pro Val Leu Asp Ser Asp Gly Ser Phe Phe Leu Tyr
            180                 185                 190

Ser Lys Leu Thr Val Asp Lys Ser Arg Trp Gln Gln Gly Asn Val Phe
        195                 200                 205

Ser Cys Ser Val Met His Glu Ala Leu His Asn His Tyr Thr Gln Lys
    210                 215                 220

Ser Leu Ser Leu Ser Pro Gly Lys
225                 230
```

<210> 279
<211> 212
<212> PRT
<213> Artificial Sequence

<220>
<223> Description of Artificial Sequence: Synthetic peptide

<400> 279
```
Gln Val Gln Leu Val Glu Ser Gly Gly Gly Leu Val Gln Pro Gly Gly
1               5                   10                  15

Ser Leu Arg Leu Ser Cys Ala Ala Ser Gly Ile Thr Phe Ser Asn Tyr
            20                  25                  30

Trp Met Ser Trp Val Arg Gln Ala Pro Gly Lys Gly Leu Glu Trp Val
        35                  40                  45

Ala Ser Ile Lys Arg Asp Gly Ser Asp Lys Tyr Tyr Val Asp Ser Val
    50                  55                  60

Lys Gly Arg Phe Thr Ile Ser Arg Asp Asn Ala Lys Asn Ser Leu Ser
65                  70                  75                  80

Leu Gln Met His Ser Leu Arg Ala Glu Asp Thr Ala Val Tyr Tyr Cys
                85                  90                  95

Ala Lys Gly Glu Phe Asp Tyr Trp Gly Gln Gly Thr Leu Val Thr Val
            100                 105                 110

Ser Ser Ala Ser Thr Lys Gly Pro Ser Val Phe Pro Leu Ala Pro Ser
        115                 120                 125

Ser Lys Ser Thr Ser Gly Gly Thr Ala Ala Leu Gly Cys Leu Val Lys
    130                 135                 140

Asp Tyr Phe Pro Glu Pro Val Thr Val Ser Trp Asn Ser Gly Ala Leu
145                 150                 155                 160

Thr Ser Gly Val His Thr Phe Pro Ala Val Leu Gln Ser Ser Gly Leu
                165                 170                 175

Tyr Ser Leu Ser Ser Val Val Thr Val Pro Ser Ser Ser Leu Gly Thr
            180                 185                 190

Gln Thr Tyr Ile Cys Asn Val Asn His Lys Pro Ser Asn Thr Lys Val
        195                 200                 205

Asp Lys Lys Val
    210
```

<210> 280
<211> 215
<212> PRT
<213> Artificial Sequence

<220>
<223> Description of Artificial Sequence: Synthetic peptide

<400> 280
Gln Val Gln Leu Gln Glu Ser Gly Pro Gly Leu Val Lys Pro Ser Glu
1 5 10 15

Thr Leu Ser Leu Thr Cys Ser Val Ser Gly Gly Ser Ile Arg Asn Tyr
20 25 30

Tyr Trp Ser Trp Ile Arg Gln Ser Ala Gly Lys Glu Leu Glu Trp Phe
35 40 45

Gly His Ile Phe Ser Thr Gly His Ile Asn Tyr Asp Ser Ser Leu Lys
50 55 60

Ser Arg Val Thr Met Ser Val Asp Thr Ser Asn Asn Gln Phe Ser Leu
65 70 75 80

Lys Leu Arg Ser Val Thr Ala Ala Asp Thr Ala Val Tyr Tyr Cys Ala
85 90 95

Arg Asp Asn Gly Ala Ala Leu Phe Asp Phe Trp Gly Gln Gly Thr Leu
100 105 110

Val Thr Val Ser Ser Ala Ser Thr Lys Gly Pro Ser Val Phe Pro Leu
115 120 125

Ala Pro Ser Ser Lys Ser Thr Ser Gly Gly Thr Ala Ala Leu Gly Cys
130 135 140

Leu Val Lys Asp Tyr Phe Pro Glu Pro Val Thr Val Ser Trp Asn Ser
145 150 155 160

Gly Ala Leu Thr Ser Gly Val His Thr Phe Pro Ala Val Leu Gln Ser
165 170 175

Ser Gly Leu Tyr Ser Leu Ser Ser Val Val Thr Val Pro Ser Ser Ser
180 185 190

Leu Gly Thr Gln Thr Tyr Ile Cys Asn Val Asn His Lys Pro Ser Asn
195 200 205

Thr Lys Val Asp Lys Lys Val
210 215

<210> 281
<211> 212
<212> PRT
<213> Artificial Sequence

<220>
<223> Description of Artificial Sequence: Synthetic peptide

<400> 281
Gln Val Gln Leu Val Glu Ser Gly Gly Gly Leu Val Gln Pro Gly Gly
1 5 10 15

```
Ser Leu Arg Leu Ser Cys Ala Ala Ser Gly Ile Thr Phe Ser Asn Tyr
            20                  25                  30

Trp Met Ser Trp Val Arg Gln Ala Pro Gly Lys Gly Leu Glu Trp Val
        35                  40                  45

Ala Ser Ile Lys Arg Asp Gly Ser Asp Lys Tyr Tyr Val Asp Ser Val
    50                  55                  60

Lys Gly Arg Phe Thr Ile Ser Arg Asp Asn Ala Lys Asn Ser Leu Ser
65                  70                  75                  80

Leu Gln Met His Ser Leu Arg Ala Glu Asp Thr Ala Val Tyr Tyr Cys
                85                  90                  95

Ala Lys Gly Glu Phe Asp Tyr Trp Gly Gln Gly Thr Leu Val Thr Val
            100                 105                 110

Ser Ser Ala Ser Thr Lys Gly Pro Ser Val Phe Pro Leu Ala Pro Ser
        115                 120                 125

Ser Lys Ser Thr Ser Gly Gly Thr Ala Ala Leu Gly Cys Leu Val Lys
    130                 135                 140

Asp Tyr Phe Pro Glu Pro Val Thr Val Ser Trp Asn Ser Gly Ala Leu
145                 150                 155                 160

Thr Ser Gly Val His Thr Phe Pro Ala Val Leu Gln Ser Ser Gly Leu
                165                 170                 175

Tyr Ser Leu Ser Ser Val Val Thr Val Pro Ser Ser Ser Leu Gly Thr
            180                 185                 190

Gln Thr Tyr Ile Cys Asn Val Asn His Lys Pro Ser Asn Thr Lys Val
        195                 200                 205

Asp Lys Lys Val
    210

<210>   282
<211>   221
<212>   PRT
<213>   Artificial Sequence

<220>
<223>   Description of Artificial Sequence: Synthetic peptide

<400>   282
Glu Val Gln Leu Val Glu Ser Gly Gly Gly Leu Val Gln Pro Gly Gly
1               5                   10                  15

Ser Leu Lys Val Ser Cys Glu Ala Ser Gly Phe Thr Phe Ser Val Ser
            20                  25                  30
```

Ala Ile His Trp Val Arg Gln Ala Ser Gly Lys Gly Leu Glu Trp Ile
35 40 45

Gly Arg Ile Arg Ser Lys Gly Asn Ser Tyr Ala Thr Ala Tyr Ala Ala
50 55 60

Ser Val Lys Gly Arg Phe Thr Ile Ser Arg Asp Asp Ser Lys Asn Thr
65 70 75 80

Ala Tyr Leu Gln Met Asp Ser Leu Lys Thr Glu Asp Thr Ala Val Tyr
85 90 95

Tyr Cys Thr Arg His Asn Asp Lys Trp Asn Tyr Tyr Gly Leu Asp Val
100 105 110

Trp Gly Gln Gly Thr Thr Val Thr Val Ser Ser Ala Ser Thr Lys Gly
115 120 125

Pro Ser Val Phe Pro Leu Ala Pro Ser Ser Lys Ser Thr Ser Gly Gly
130 135 140

Thr Ala Ala Leu Gly Cys Leu Val Lys Asp Tyr Phe Pro Glu Pro Val
145 150 155 160

Thr Val Ser Trp Asn Ser Gly Ala Leu Thr Ser Gly Val His Thr Phe
165 170 175

Pro Ala Val Leu Gln Ser Ser Gly Leu Tyr Ser Leu Ser Ser Val Val
180 185 190

Thr Val Pro Ser Ser Ser Leu Gly Thr Gln Thr Tyr Ile Cys Asn Val
195 200 205

Asn His Lys Pro Ser Asn Thr Lys Val Asp Lys Lys Val
210 215 220

<210> 283
<211> 215
<212> PRT
<213> Artificial Sequence

<220>
<223> Description of Artificial Sequence: Synthetic peptide

<400> 283
Gln Val Gln Leu Gln Glu Ser Gly Pro Gly Leu Val Lys Pro Ser Glu
1 5 10 15

Thr Leu Ser Leu Thr Cys Ser Val Ser Gly Ala Ser Ile Arg Asn Tyr
20 25 30

Tyr Trp Ser Trp Ile Arg Gln Thr Ala Gly Lys Gly Leu Glu Trp Leu
35 40 45

```
Gly His Ile Tyr Ser Thr Gly Asn Ile His Tyr Asn Pro Ser Leu Lys
    50                  55                  60

Ser Arg Val Thr Met Ser Val Asp Thr Ser Asn Asn Gln Phe Ser Leu
65                  70                  75                  80

Lys Leu Arg Ser Val Thr Ala Ala Asp Thr Ala Val Tyr Tyr Cys Ala
                85                  90                  95

Arg Asp Asn Gly Ala Ala Leu Phe Asp Tyr Trp Gly Gln Gly Thr Leu
            100                 105                 110

Val Thr Val Ser Ser Ala Ser Thr Lys Gly Pro Ser Val Phe Pro Leu
        115                 120                 125

Ala Pro Ser Ser Lys Ser Thr Ser Gly Gly Thr Ala Ala Leu Gly Cys
    130                 135                 140

Leu Val Lys Asp Tyr Phe Pro Glu Pro Val Thr Val Ser Trp Asn Ser
145                 150                 155                 160

Gly Ala Leu Thr Ser Gly Val His Thr Phe Pro Ala Val Leu Gln Ser
                165                 170                 175

Ser Gly Leu Tyr Ser Leu Ser Ser Val Val Thr Val Pro Ser Ser Ser
            180                 185                 190

Leu Gly Thr Gln Thr Tyr Ile Cys Asn Val Asn His Lys Pro Ser Asn
        195                 200                 205

Thr Lys Val Asp Lys Lys Val
    210                 215

<210>   284
<211>   215
<212>   PRT
<213>   Artificial Sequence

<220>
<223>   Description of Artificial Sequence: Synthetic peptide

<400>   284
Gln Val Gln Leu Gln Glu Ser Gly Pro Gly Leu Val Lys Pro Ser Glu
1               5                   10                  15

Thr Leu Ser Leu Thr Cys Thr Val Ser Gly Gly Ser Ile Ser Ser Tyr
            20                  25                  30

Tyr Trp Gly Trp Ile Arg Gln Pro Pro Gly Lys Gly Leu Glu Trp Ile
        35                  40                  45

Gly Tyr Ile Tyr Tyr Ser Gly Ser Thr Asn Tyr Asn Pro Ser Leu Lys
    50                  55                  60
```

```
Ser Arg Val Thr Ile Ser Val Asp Thr Ser Lys Asn Gln Phe Ser Leu
65                  70                  75                  80

Lys Leu Ser Ser Val Thr Ala Ala Asp Thr Ala Val Tyr Tyr Cys Ala
                85                  90                  95

Arg Met Trp Glu Ile Leu Gly Phe Asp Pro Trp Gly Gln Gly Thr Leu
            100                 105                 110

Val Thr Val Ser Ser Ala Ser Thr Lys Gly Pro Ser Val Phe Pro Leu
        115                 120                 125

Ala Pro Ser Ser Lys Ser Thr Ser Gly Gly Thr Ala Ala Leu Gly Cys
    130                 135                 140

Leu Val Lys Asp Tyr Phe Pro Glu Pro Val Thr Val Ser Trp Asn Ser
145                 150                 155                 160

Gly Ala Leu Thr Ser Gly Val His Thr Phe Pro Ala Val Leu Gln Ser
                165                 170                 175

Ser Gly Leu Tyr Ser Leu Ser Ser Val Val Thr Val Pro Ser Ser Ser
            180                 185                 190

Leu Gly Thr Gln Thr Tyr Ile Cys Asn Val Asn His Lys Pro Ser Asn
        195                 200                 205

Thr Lys Val Asp Lys Lys Val
    210                 215

<210>   285
<211>   219
<212>   PRT
<213>   Artificial Sequence

<220>
<223>   Description of Artificial Sequence: Synthetic peptide

<400>   285
Asp Ile Val Met Thr Gln Ser Pro Leu Ser Leu Pro Val Thr Pro Gly
1               5                   10                  15

Glu Pro Ala Ser Ile Ser Cys Arg Ser Ser Gln Ser Leu Leu His Ser
            20                  25                  30

Asp Gly Tyr Asn Tyr Leu Asp Trp Tyr Leu Gln Lys Ser Gly Gln Ser
        35                  40                  45

Pro Gln Leu Leu Ile Tyr Leu Gly Ser Tyr Arg Ala Ser Gly Val Pro
    50                  55                  60

Asp Arg Phe Ser Gly Ser Gly Ser Gly Thr Asp Phe Thr Leu Lys Ile
65                  70                  75                  80
```

Ser Arg Val Glu Ala Glu Asp Val Gly Ile Tyr Tyr Cys Met Gln Val
85 90 95

Leu Gln Thr Pro Trp Thr Phe Gly Gln Gly Thr Lys Val Glu Ile Lys
100 105 110

Arg Thr Val Ala Ala Pro Ser Val Phe Ile Phe Pro Pro Ser Asp Glu
115 120 125

Gln Leu Lys Ser Gly Thr Ala Ser Val Val Cys Leu Leu Asn Asn Phe
130 135 140

Tyr Pro Arg Glu Ala Lys Val Gln Trp Lys Val Asp Asn Ala Leu Gln
145 150 155 160

Ser Gly Asn Ser Gln Glu Ser Val Thr Glu Gln Asp Ser Lys Asp Ser
165 170 175

Thr Tyr Ser Leu Ser Ser Thr Leu Thr Leu Ser Lys Ala Asp Tyr Glu
180 185 190

Lys His Lys Val Tyr Ala Cys Glu Val Thr His Gln Gly Leu Ser Ser
195 200 205

Pro Val Thr Lys Ser Phe Asn Arg Gly Glu Cys
210 215

<210> 286
<211> 216
<212> PRT
<213> Artificial Sequence

<220>
<223> Description of Artificial Sequence: Synthetic peptide

<400> 286
Gln Ser Val Leu Thr Gln Pro Pro Ser Ala Ser Gly Thr Pro Gly Gln
1 5 10 15

Arg Val Thr Ile Ser Cys Ser Gly Ser Ser Ser Asn Ile Gly Ser Asn
20 25 30

Ile Val Asn Trp Tyr Gln Gln Phe Pro Gly Thr Ala Pro Lys Leu Leu
35 40 45

Leu Tyr Ser Asp Asn Gln Arg Pro Ser Gly Val Pro Asp Arg Phe Ser
50 55 60

Gly Ser Lys Ser Gly Thr Ser Ala Ser Leu Ala Ile Ser Gly Leu Gln
65 70 75 80

Ser Glu Asp Glu Ala Asp Tyr Tyr Cys Ala Ala Trp Asp Asp Ser Leu
85 90 95

```
Asn Gly Pro Val Phe Gly Thr Gly Thr Lys Val Thr Val Leu Gly Gln
            100                 105                 110

Pro Lys Ala Ala Pro Ser Val Thr Leu Phe Pro Pro Ser Ser Glu Glu
        115                 120                 125

Leu Gln Ala Asn Lys Ala Thr Leu Val Cys Leu Ile Ser Asp Phe Tyr
    130                 135                 140

Pro Gly Ala Val Thr Val Ala Trp Lys Ala Asp Ser Ser Pro Val Lys
145                 150                 155                 160

Ala Gly Val Glu Thr Thr Thr Pro Ser Lys Gln Ser Asn Asn Lys Tyr
                165                 170                 175

Ala Ala Ser Ser Tyr Leu Ser Leu Thr Pro Glu Gln Trp Lys Ser His
            180                 185                 190

Arg Ser Tyr Ser Cys Gln Val Thr His Glu Gly Ser Thr Val Glu Lys
        195                 200                 205

Thr Val Ala Pro Thr Glu Cys Ser
    210                 215

<210>   287
<211>   219
<212>   PRT
<213>   Artificial Sequence

<220>
<223>   Description of Artificial Sequence: Synthetic peptide

<400>   287
Asp Val Val Met Thr Gln Ser Pro Leu Ser Leu Pro Val Thr Leu Gly
1               5                   10                  15

Gln Pro Ala Ser Ile Ser Cys Arg Ser Ser Gln Ser Leu Val Tyr Ser
            20                  25                  30

Asp Gly Asn Thr Tyr Leu Asn Trp Phe Gln Gln Arg Pro Gly Gln Ser
        35                  40                  45

Pro Arg Arg Leu Ile Tyr Lys Val Ser Thr Arg Asp Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Val Ala Val Tyr Tyr Cys Leu Gln Gly
                85                  90                  95

Thr His Trp Pro Trp Thr Phe Gly Gln Gly Thr Lys Val Glu Ile Lys
            100                 105                 110
```

```
Arg Thr Val Ala Ala Pro Ser Val Phe Ile Phe Pro Pro Ser Asp Glu
        115                 120                 125

Gln Leu Lys Ser Gly Thr Ala Ser Val Val Cys Leu Leu Asn Asn Phe
    130                 135                 140

Tyr Pro Arg Glu Ala Lys Val Gln Trp Lys Val Asp Asn Ala Leu Gln
145                 150                 155                 160

Ser Gly Asn Ser Gln Glu Ser Val Thr Glu Gln Asp Ser Lys Asp Ser
                165                 170                 175

Thr Tyr Ser Leu Ser Ser Thr Leu Thr Leu Ser Lys Ala Asp Tyr Glu
            180                 185                 190

Lys His Lys Val Tyr Ala Cys Glu Val Thr His Gln Gly Leu Ser Ser
        195                 200                 205

Pro Val Thr Lys Ser Phe Asn Arg Gly Glu Cys
    210                 215

<210>   288
<211>   219
<212>   PRT
<213>   Artificial Sequence

<220>
<223>   Description of Artificial Sequence: Synthetic peptide

<400>   288
Asp Ile Val Met Thr Gln Ser Pro Leu Ser Leu Pro Val Thr Pro Gly
1               5                   10                  15

Glu Pro Ala Ser Ile Ser Cys Lys Ser Ser Gln Ser Leu Leu Phe Ser
            20                  25                  30

Asn Gly Tyr Lys Phe Leu Asp Trp Tyr Leu Gln Arg Pro Gly Gln Ser
        35                  40                  45

Pro Gln Leu Leu Ile Tyr Leu Gly Ser Tyr Arg Ala Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Val Gly Leu Tyr Tyr Cys Met Gln Ala
                85                  90                  95

Leu Gln Thr Pro Pro Thr Phe Gly Gly Gly Thr Lys Val Glu Ile Lys
            100                 105                 110

Arg Thr Val Ala Ala Pro Ser Val Phe Ile Phe Pro Pro Ser Asp Glu
        115                 120                 125
```

```
Gln Leu Lys Ser Gly Thr Ala Ser Val Val Cys Leu Leu Asn Asn Phe
    130                 135                 140

Tyr Pro Arg Glu Ala Lys Val Gln Trp Lys Val Asp Asn Ala Leu Gln
145                 150                 155                 160

Ser Gly Asn Ser Gln Glu Ser Val Thr Glu Gln Asp Ser Lys Asp Ser
                165                 170                 175

Thr Tyr Ser Leu Ser Ser Thr Leu Thr Leu Ser Lys Ala Asp Tyr Glu
            180                 185                 190

Lys His Lys Val Tyr Ala Cys Glu Val Thr His Gln Gly Leu Ser Ser
        195                 200                 205

Pro Val Thr Lys Ser Phe Asn Arg Gly Glu Cys
    210                 215

<210>   289
<211>   216
<212>   PRT
<213>   Artificial Sequence

<220>
<223>   Description of Artificial Sequence: Synthetic peptide

<400>   289
Gln Ser Val Leu Thr Gln Pro Pro Ser Ala Ser Gly Thr Pro Gly Gln
1               5                   10                  15

Arg Val Thr Ile Ser Cys Ser Gly Ser Ser Ser Asn Ile Gly Ser Asn
            20                  25                  30

Ile Val Asn Trp Tyr Gln Gln Phe Pro Gly Thr Ala Pro Lys Leu Leu
        35                  40                  45

Ile Tyr Ser Asn Asn Gln Arg Pro Ser Gly Val Pro Asp Arg Val Ser
    50                  55                  60

Gly Ser Lys Ser Gly Thr Ser Ala Ser Leu Ala Ile Ser Gly Leu Gln
65                  70                  75                  80

Ser Glu Asp Glu Ala Asp Tyr Tyr Cys Ala Ala Trp Asp Asp Ser Leu
                85                  90                  95

Asn Gly Pro Val Phe Gly Pro Gly Thr Lys Val Thr Val Leu Gly Gln
            100                 105                 110

Pro Lys Ala Ala Pro Ser Val Thr Leu Phe Pro Pro Ser Ser Glu Glu
        115                 120                 125

Leu Gln Ala Asn Lys Ala Thr Leu Val Cys Leu Ile Ser Asp Phe Tyr
    130                 135                 140
```

```
Pro Gly Ala Val Thr Val Ala Trp Lys Ala Asp Ser Ser Pro Val Lys
145                 150                 155                 160

Ala Gly Val Glu Thr Thr Thr Pro Ser Lys Gln Ser Asn Asn Lys Tyr
                165                 170                 175

Ala Ala Ser Ser Tyr Leu Ser Leu Thr Pro Glu Gln Trp Lys Ser His
            180                 185                 190

Arg Ser Tyr Ser Cys Gln Val Thr His Glu Gly Ser Thr Val Glu Lys
        195                 200                 205

Thr Val Ala Pro Thr Glu Cys Ser
    210                 215

<210>   290
<211>   216
<212>   PRT
<213>   Artificial Sequence

<220>
<223>   Description of Artificial Sequence: Synthetic peptide

<400>   290
Gln Ser Val Leu Thr Gln Pro Pro Ser Ala Ser Gly Thr Pro Gly Gln
1               5                   10                  15

Arg Val Thr Ile Ser Cys Ser Gly Ser Ser Ser Asn Ile Gly Ser Asn
            20                  25                  30

Pro Val Asn Trp Tyr Gln Gln Leu Pro Gly Thr Ala Pro Lys Leu Leu
        35                  40                  45

Ile Tyr Ser Asn Asn Gln Arg Pro Ser Gly Val Pro Asp Arg Phe Ser
    50                  55                  60

Gly Ser Lys Ser Gly Thr Ser Ala Ser Leu Ala Ile Ser Gly Leu Gln
65                  70                  75                  80

Ser Glu Asp Glu Ala Asp Tyr Phe Cys Ala Ala Trp Asp Asp Ser Leu
                85                  90                  95

Asn Gly Pro Val Phe Gly Gly Gly Thr Lys Leu Thr Val Leu Gly Gln
            100                 105                 110

Pro Lys Ala Ala Pro Ser Val Thr Leu Phe Pro Pro Ser Ser Glu Glu
        115                 120                 125

Leu Gln Ala Asn Lys Ala Thr Leu Val Cys Leu Ile Ser Asp Phe Tyr
    130                 135                 140

Pro Gly Ala Val Thr Val Ala Trp Lys Ala Asp Ser Ser Pro Val Lys
145                 150                 155                 160
```

```
Ala Gly Val Glu Thr Thr Thr Pro Ser Lys Gln Ser Asn Asn Lys Tyr
                165                 170                 175

Ala Ala Ser Ser Tyr Leu Ser Leu Thr Pro Glu Gln Trp Lys Ser His
            180                 185                 190

Arg Ser Tyr Ser Cys Gln Val Thr His Glu Gly Ser Thr Val Glu Lys
        195                 200                 205

Thr Val Ala Pro Thr Glu Cys Ser
    210                 215

<210>   291
<211>   123
<212>   PRT
<213>   Artificial Sequence

<220>
<223>   Description of Artificial Sequence: Synthetic peptide

<400>   291
Glu Val Gln Leu Val Glu Ser Gly Gly Gly Leu Val Gln Pro Gly Gly
1               5                   10                  15

Ser Leu Arg Leu Ser Cys Val Val Ser Gly Phe Thr Phe Ser Ser Tyr
            20                  25                  30

Trp Met Thr Trp Val Arg Gln Ala Pro Gly Lys Gly Leu Glu Trp Val
        35                  40                  45

Ala Asn Ile Lys Gln Gly Gly Ser Glu Arg Tyr Tyr Val Asp Ser Val
    50                  55                  60

Lys Gly Arg Phe Thr Ile Ser Arg Asp Ser Ala Lys Asn Ser Leu Tyr
65                  70                  75                  80

Leu Gln Met Asn Ser Leu Arg Ala Glu Asp Thr Ala Val Tyr Tyr Cys
                85                  90                  95

Ala Arg Glu Val Gly Tyr Asn Trp Asn Gln Gly Gly Tyr Phe Asp Tyr
            100                 105                 110

Trp Gly Gln Gly Thr Leu Val Thr Val Ser Ser
        115                 120

<210>   292
<211>   123
<212>   PRT
<213>   Artificial Sequence

<220>
<223>   Description of Artificial Sequence: Synthetic peptide
```

<400> 292
Glu Val Gln Leu Val Glu Ser Gly Gly Gly Leu Val Gln Pro Gly Gly
1               5                   10                  15

Ser Leu Arg Leu Ser Cys Val Val Ser Gly Phe Thr Phe Ser Ser Tyr
            20                  25                  30

Trp Met Thr Trp Val Arg Gln Ala Pro Gly Lys Gly Leu Glu Trp Val
        35                  40                  45

Ala Asn Ile Lys Gln Ala Gly Ser Glu Arg Tyr Tyr Val Asp Ser Val
    50                  55                  60

Lys Gly Arg Phe Thr Ile Ser Arg Asp Ser Ala Lys Asn Ser Leu Tyr
65                  70                  75                  80

Leu Gln Met Asn Ser Leu Arg Ala Glu Asp Thr Ala Val Tyr Tyr Cys
                85                  90                  95

Ala Arg Glu Val Gly Tyr Asn Trp Asn Gln Gly Gly Tyr Phe Asp Tyr
            100                 105                 110

Trp Gly Gln Gly Thr Leu Val Thr Val Ser Ser
        115                 120

<210> 293
<211> 123
<212> PRT
<213> Artificial Sequence

<220>
<223> Description of Artificial Sequence: Synthetic peptide

<400> 293
Glu Val Gln Leu Val Glu Ser Gly Gly Gly Leu Val Gln Pro Gly Gly
1               5                   10                  15

Ser Leu Arg Leu Ser Cys Val Val Ser Gly Phe Thr Phe Ser Ser Tyr
            20                  25                  30

Trp Met Thr Trp Val Arg Gln Ala Pro Gly Lys Gly Leu Glu Trp Val
        35                  40                  45

Ala Asn Ile Lys Gln Tyr Gly Ser Glu Arg Tyr Tyr Val Asp Ser Val
    50                  55                  60

Lys Gly Arg Phe Thr Ile Ser Arg Asp Ser Ala Lys Asn Ser Leu Tyr
65                  70                  75                  80

Leu Gln Met Asn Ser Leu Arg Ala Glu Asp Thr Ala Val Tyr Tyr Cys
                85                  90                  95

Ala Arg Glu Val Gly Tyr Asn Trp Asn Gln Gly Gly Tyr Phe Asp Tyr
            100                 105                 110

Trp Gly Gln Gly Thr Leu Val Thr Val Ser Ser
115 120

<210> 294
<211> 123
<212> PRT
<213> Artificial Sequence

<220>
<223> Description of Artificial Sequence: Synthetic peptide

<400> 294
Glu Val Gln Leu Val Glu Ser Gly Gly Gly Leu Val Gln Pro Gly Gly
1 5 10 15

Ser Leu Arg Leu Ser Cys Val Val Ser Gly Phe Thr Phe Ser Ser Tyr
20 25 30

Trp Met Thr Trp Val Arg Gln Ala Pro Gly Lys Gly Leu Glu Trp Val
35 40 45

Ala Asn Ile Lys Gln Pro Gly Ser Glu Arg Tyr Tyr Val Asp Ser Val
50 55 60

Lys Gly Arg Phe Thr Ile Ser Arg Asp Ser Ala Lys Asn Ser Leu Tyr
65 70 75 80

Leu Gln Met Asn Ser Leu Arg Ala Glu Asp Thr Ala Val Tyr Tyr Cys
85 90 95

Ala Arg Glu Val Gly Tyr Asn Trp Asn Gln Gly Gly Tyr Phe Asp Tyr
100 105 110

Trp Gly Gln Gly Thr Leu Val Thr Val Ser Ser
115 120

<210> 295
<211> 123
<212> PRT
<213> Artificial Sequence

<220>
<223> Description of Artificial Sequence: Synthetic peptide

<400> 295
Glu Val Gln Leu Val Glu Ser Gly Gly Gly Leu Val Gln Pro Gly Gly
1 5 10 15

Ser Leu Arg Leu Ser Cys Val Val Ser Gly Phe Thr Phe Ser Ser Tyr
20 25 30

Trp Met Thr Trp Val Arg Gln Ala Pro Gly Lys Gly Leu Glu Trp Val
35 40 45

Ala Asn Ile Lys Gln Asn Gly Ser Glu Arg Tyr Tyr Val Asp Ser Val
    50                  55                  60

Lys Gly Arg Phe Thr Ile Ser Arg Asp Ser Ala Lys Asn Ser Leu Tyr
65                  70                  75                  80

Leu Gln Met Asn Ser Leu Arg Ala Glu Asp Thr Ala Val Tyr Tyr Cys
                85                  90                  95

Ala Arg Glu Val Gly Tyr Asn Trp Asn Gln Gly Gly Tyr Phe Asp Tyr
            100                 105                 110

Trp Gly Gln Gly Thr Leu Val Thr Val Ser Ser
        115                 120

<210> 296
<211> 123
<212> PRT
<213> Artificial Sequence

<220>
<223> Description of Artificial Sequence: Synthetic peptide

<400> 296
Glu Val Gln Leu Val Glu Ser Gly Gly Gly Leu Val Gln Pro Gly Gly
1               5                   10                  15

Ser Leu Arg Leu Ser Cys Val Val Ser Gly Phe Thr Phe Ser Ser Tyr
            20                  25                  30

Trp Met Thr Trp Val Arg Gln Ala Pro Gly Lys Gly Leu Glu Trp Val
        35                  40                  45

Ala Asn Ile Lys Gln Ser Gly Ser Glu Arg Tyr Tyr Val Asp Ser Val
    50                  55                  60

Lys Gly Arg Phe Thr Ile Ser Arg Asp Ser Ala Lys Asn Ser Leu Tyr
65                  70                  75                  80

Leu Gln Met Asn Ser Leu Arg Ala Glu Asp Thr Ala Val Tyr Tyr Cys
                85                  90                  95

Ala Arg Glu Val Gly Tyr Asn Trp Asn Gln Gly Gly Tyr Phe Asp Tyr
            100                 105                 110

Trp Gly Gln Gly Thr Leu Val Thr Val Ser Ser
        115                 120

<210> 297
<211> 123
<212> PRT
<213> Artificial Sequence

<220>
<223> Description of Artificial Sequence: Synthetic peptide

<400> 297
Glu Val Gln Leu Val Glu Ser Gly Gly Gly Leu Val Gln Pro Gly Gly
1 5 10 15

Ser Leu Arg Leu Ser Cys Val Val Ser Gly Phe Thr Phe Ser Ser Tyr
20 25 30

Trp Met Thr Trp Val Arg Gln Ala Pro Gly Lys Gly Leu Glu Trp Val
35 40 45

Ala Asn Ile Lys Gln Arg Gly Ser Glu Arg Tyr Tyr Val Asp Ser Val
50 55 60

Lys Gly Arg Phe Thr Ile Ser Arg Asp Ser Ala Lys Asn Ser Leu Tyr
65 70 75 80

Leu Gln Met Asn Ser Leu Arg Ala Glu Asp Thr Ala Val Tyr Tyr Cys
85 90 95

Ala Arg Glu Val Gly Tyr Asn Trp Asn Gln Gly Gly Tyr Phe Asp Tyr
100 105 110

Trp Gly Gln Gly Thr Leu Val Thr Val Ser Ser
115 120

<210> 298
<211> 123
<212> PRT
<213> Artificial Sequence

<220>
<223> Description of Artificial Sequence: Synthetic peptide

<400> 298
Glu Val Gln Leu Val Glu Ser Gly Gly Gly Leu Val Gln Pro Gly Gly
1 5 10 15

Ser Leu Arg Leu Ser Cys Val Val Ser Gly Phe Thr Phe Ser Ser Tyr
20 25 30

Trp Met Thr Trp Val Arg Gln Ala Pro Gly Lys Gly Leu Glu Trp Val
35 40 45

Ala Asn Ile Lys Gln Leu Gly Ser Glu Arg Tyr Tyr Val Asp Ser Val
50 55 60

Lys Gly Arg Phe Thr Ile Ser Arg Asp Ser Ala Lys Asn Ser Leu Tyr
65 70 75 80

Leu Gln Met Asn Ser Leu Arg Ala Glu Asp Thr Ala Val Tyr Tyr Cys
85 90 95

Ala Arg Glu Val Gly Tyr Asn Trp Asn Gln Gly Gly Tyr Phe Asp Tyr
100 105 110

Trp Gly Gln Gly Thr Leu Val Thr Val Ser Ser
115 120

<210> 299
<211> 123
<212> PRT
<213> Artificial Sequence

<220>
<223> Description of Artificial Sequence: Synthetic peptide

<400> 299
Glu Val Gln Leu Val Glu Ser Gly Gly Gly Leu Val Gln Pro Gly Gly
1 5 10 15

Ser Leu Arg Leu Ser Cys Val Val Ser Gly Phe Thr Phe Ser Ser Tyr
20 25 30

Trp Met Thr Trp Val Arg Gln Ala Pro Gly Lys Gly Leu Glu Trp Val
35 40 45

Ala Asn Ile Lys Gln Val Gly Ser Glu Arg Tyr Tyr Val Asp Ser Val
50 55 60

Lys Gly Arg Phe Thr Ile Ser Arg Asp Ser Ala Lys Asn Ser Leu Tyr
65 70 75 80

Leu Gln Met Asn Ser Leu Arg Ala Glu Asp Thr Ala Val Tyr Tyr Cys
85 90 95

Ala Arg Glu Val Gly Tyr Asn Trp Asn Gln Gly Gly Tyr Phe Asp Tyr
100 105 110

Trp Gly Gln Gly Thr Leu Val Thr Val Ser Ser
115 120

<210> 300
<211> 123
<212> PRT
<213> Artificial Sequence

<220>
<223> Description of Artificial Sequence: Synthetic peptide

<400> 300
Glu Val Gln Leu Val Glu Ser Gly Gly Gly Leu Val Gln Pro Gly Gly
1 5 10 15

Ser Leu Arg Leu Ser Cys Val Val Ser Gly Phe Thr Phe Ser Ser Tyr
20 25 30

Trp Met Thr Trp Val Arg Gln Ala Pro Gly Lys Gly Leu Glu Trp Val
35 40 45

Ala Asn Ile Lys Gln Thr Gly Ser Glu Arg Tyr Tyr Val Asp Ser Val
50 55 60

Lys Gly Arg Phe Thr Ile Ser Arg Asp Ser Ala Lys Asn Ser Leu Tyr
65 70 75 80

Leu Gln Met Asn Ser Leu Arg Ala Glu Asp Thr Ala Val Tyr Tyr Cys
85 90 95

Ala Arg Glu Val Gly Tyr Asn Trp Asn Gln Gly Gly Tyr Phe Asp Tyr
100 105 110

Trp Gly Gln Gly Thr Leu Val Thr Val Ser Ser
115 120

<210> 301
<211> 123
<212> PRT
<213> Artificial Sequence

<220>
<223> Description of Artificial Sequence: Synthetic peptide

<400> 301
Glu Val Gln Leu Val Glu Ser Gly Gly Gly Leu Val Gln Pro Gly Gly
1 5 10 15

Ser Leu Arg Leu Ser Cys Val Val Ser Gly Phe Thr Phe Ser Ser Tyr
20 25 30

Trp Met Thr Trp Val Arg Gln Ala Pro Gly Lys Gly Leu Glu Trp Val
35 40 45

Ala Asn Ile Lys Gln Phe Gly Ser Glu Arg Tyr Tyr Val Asp Ser Val
50 55 60

Lys Gly Arg Phe Thr Ile Ser Arg Asp Ser Ala Lys Asn Ser Leu Tyr
65 70 75 80

Leu Gln Met Asn Ser Leu Arg Ala Glu Asp Thr Ala Val Tyr Tyr Cys
85 90 95

Ala Arg Glu Val Gly Tyr Asn Trp Asn Gln Gly Gly Tyr Phe Asp Tyr
100 105 110

Trp Gly Gln Gly Thr Leu Val Thr Val Ser Ser
115 120

<210> 302
<211> 123

<212> PRT
<213> Artificial Sequence

<220>
<223> Description of Artificial Sequence: Synthetic peptide

<400> 302
Glu Val Gln Leu Val Glu Ser Gly Gly Gly Leu Val Gln Pro Gly Gly
1 5 10 15

Ser Leu Arg Leu Ser Cys Val Val Ser Gly Phe Thr Phe Ser Ser Tyr
20 25 30

Trp Met Thr Trp Val Arg Gln Ala Pro Gly Lys Gly Leu Glu Trp Val
35 40 45

Ala Asn Ile Lys Gln Trp Gly Ser Glu Arg Tyr Tyr Val Asp Ser Val
50 55 60

Lys Gly Arg Phe Thr Ile Ser Arg Asp Ser Ala Lys Asn Ser Leu Tyr
65 70 75 80

Leu Gln Met Asn Ser Leu Arg Ala Glu Asp Thr Ala Val Tyr Tyr Cys
85 90 95

Ala Arg Glu Val Gly Tyr Asn Trp Asn Gln Gly Gly Tyr Phe Asp Tyr
100 105 110

Trp Gly Gln Gly Thr Leu Val Thr Val Ser Ser
115 120

<210> 303
<211> 123
<212> PRT
<213> Artificial Sequence

<220>
<223> Description of Artificial Sequence: Synthetic peptide

<400> 303
Glu Val Gln Leu Val Glu Ser Gly Gly Gly Leu Val Gln Pro Gly Gly
1 5 10 15

Ser Leu Arg Leu Ser Cys Val Val Ser Gly Phe Thr Phe Ser Ser Tyr
20 25 30

Trp Met Thr Trp Val Arg Gln Ala Pro Gly Lys Gly Leu Glu Trp Val
35 40 45

Ala Asn Ile Lys Gln Asp Val Ser Glu Arg Tyr Tyr Val Asp Ser Val
50 55 60

Lys Gly Arg Phe Thr Ile Ser Arg Asp Ser Ala Lys Asn Ser Leu Tyr
65 70 75 80

```
Leu Gln Met Asn Ser Leu Arg Ala Glu Asp Thr Ala Val Tyr Tyr Cys
                85                  90                  95

Ala Arg Glu Val Gly Tyr Asn Trp Asn Gln Gly Gly Tyr Phe Asp Tyr
            100                 105                 110

Trp Gly Gln Gly Thr Leu Val Thr Val Ser Ser
        115                 120

<210>   304
<211>   123
<212>   PRT
<213>   Artificial Sequence

<220>
<223>   Description of Artificial Sequence: Synthetic peptide

<400>   304
Glu Val Gln Leu Val Glu Ser Gly Gly Gly Leu Val Gln Pro Gly Gly
1               5                   10                  15

Ser Leu Arg Leu Ser Cys Val Val Ser Gly Phe Thr Phe Ser Ser Tyr
            20                  25                  30

Trp Met Thr Trp Val Arg Gln Ala Pro Gly Lys Gly Leu Glu Trp Val
        35                  40                  45

Ala Asn Ile Lys Gln Asp Pro Ser Glu Arg Tyr Tyr Val Asp Ser Val
    50                  55                  60

Lys Gly Arg Phe Thr Ile Ser Arg Asp Ser Ala Lys Asn Ser Leu Tyr
65                  70                  75                  80

Leu Gln Met Asn Ser Leu Arg Ala Glu Asp Thr Ala Val Tyr Tyr Cys
                85                  90                  95

Ala Arg Glu Val Gly Tyr Asn Trp Asn Gln Gly Gly Tyr Phe Asp Tyr
            100                 105                 110

Trp Gly Gln Gly Thr Leu Val Thr Val Ser Ser
        115                 120

<210>   305
<211>   123
<212>   PRT
<213>   Artificial Sequence

<220>
<223>   Description of Artificial Sequence: Synthetic peptide

<400>   305
Glu Val Gln Leu Val Glu Ser Gly Gly Gly Leu Val Gln Pro Gly Gly
1               5                   10                  15
```

Ser Leu Arg Leu Ser Cys Val Val Ser Gly Phe Thr Phe Ser Ser Tyr
20 25 30

Trp Met Thr Trp Val Arg Gln Ala Pro Gly Lys Gly Leu Glu Trp Val
35 40 45

Ala Asn Ile Lys Gln Asp Arg Ser Glu Arg Tyr Tyr Val Asp Ser Val
50 55 60

Lys Gly Arg Phe Thr Ile Ser Arg Asp Ser Ala Lys Asn Ser Leu Tyr
65 70 75 80

Leu Gln Met Asn Ser Leu Arg Ala Glu Asp Thr Ala Val Tyr Tyr Cys
85 90 95

Ala Arg Glu Val Gly Tyr Asn Trp Asn Gln Gly Gly Tyr Phe Asp Tyr
100 105 110

Trp Gly Gln Gly Thr Leu Val Thr Val Ser Ser
115 120

<210> 306
<211> 123
<212> PRT
<213> Artificial Sequence

<220>
<223> Description of Artificial Sequence: Synthetic peptide

<400> 306
Glu Val Gln Leu Val Glu Ser Gly Gly Gly Leu Val Gln Pro Gly Gly
1 5 10 15

Ser Leu Arg Leu Ser Cys Val Val Ser Gly Phe Thr Phe Ser Ser Tyr
20 25 30

Trp Met Thr Trp Val Arg Gln Ala Pro Gly Lys Gly Leu Glu Trp Val
35 40 45

Ala Asn Ile Lys Gln Asp Ala Ser Glu Arg Tyr Tyr Val Asp Ser Val
50 55 60

Lys Gly Arg Phe Thr Ile Ser Arg Asp Ser Ala Lys Asn Ser Leu Tyr
65 70 75 80

Leu Gln Met Asn Ser Leu Arg Ala Glu Asp Thr Ala Val Tyr Tyr Cys
85 90 95

Ala Arg Glu Val Gly Tyr Asn Trp Asn Gln Gly Gly Tyr Phe Asp Tyr
100 105 110

Trp Gly Gln Gly Thr Leu Val Thr Val Ser Ser
115 120

<210> 307
<211> 123
<212> PRT
<213> Artificial Sequence

<220>
<223> Description of Artificial Sequence: Synthetic peptide

<400> 307

```
Glu Val Gln Leu Val Glu Ser Gly Gly Gly Leu Val Gln Pro Gly Gly
1               5                   10                  15

Ser Leu Arg Leu Ser Cys Val Val Ser Gly Phe Thr Phe Ser Ser Tyr
            20                  25                  30

Trp Met Thr Trp Val Arg Gln Ala Pro Gly Lys Gly Leu Glu Trp Val
        35                  40                  45

Ala Asn Ile Lys Gln Asp Leu Ser Glu Arg Tyr Tyr Val Asp Ser Val
    50                  55                  60

Lys Gly Arg Phe Thr Ile Ser Arg Asp Ser Ala Lys Asn Ser Leu Tyr
65                  70                  75                  80

Leu Gln Met Asn Ser Leu Arg Ala Glu Asp Thr Ala Val Tyr Tyr Cys
                85                  90                  95

Ala Arg Glu Val Gly Tyr Asn Trp Asn Gln Gly Gly Tyr Phe Asp Tyr
            100                 105                 110

Trp Gly Gln Gly Thr Leu Val Thr Val Ser Ser
        115                 120
```

<210> 308
<211> 123
<212> PRT
<213> Artificial Sequence

<220>
<223> Description of Artificial Sequence: Synthetic peptide

<400> 308

```
Glu Val Gln Leu Val Glu Ser Gly Gly Gly Leu Val Gln Pro Gly Gly
1               5                   10                  15

Ser Leu Arg Leu Ser Cys Val Val Ser Gly Phe Thr Phe Ser Ser Tyr
            20                  25                  30

Trp Met Thr Trp Val Arg Gln Ala Pro Gly Lys Gly Leu Glu Trp Val
        35                  40                  45

Ala Asn Ile Lys Gln Asp Gln Ser Glu Arg Tyr Tyr Val Asp Ser Val
    50                  55                  60
```

Lys Gly Arg Phe Thr Ile Ser Arg Asp Ser Ala Lys Asn Ser Leu Tyr
65 70 75 80

Leu Gln Met Asn Ser Leu Arg Ala Glu Asp Thr Ala Val Tyr Tyr Cys
85 90 95

Ala Arg Glu Val Gly Tyr Asn Trp Asn Gln Gly Gly Tyr Phe Asp Tyr
100 105 110

Trp Gly Gln Gly Thr Leu Val Thr Val Ser Ser
115 120

<210> 309
<211> 123
<212> PRT
<213> Artificial Sequence

<220>
<223> Description of Artificial Sequence: Synthetic peptide

<400> 309
Glu Val Gln Leu Val Glu Ser Gly Gly Gly Leu Val Gln Pro Gly Gly
1 5 10 15

Ser Leu Arg Leu Ser Cys Val Val Ser Gly Phe Thr Phe Ser Ser Tyr
20 25 30

Trp Met Thr Trp Val Arg Gln Ala Pro Gly Lys Gly Leu Glu Trp Val
35 40 45

Ala Asn Ile Lys Gln Asp Thr Ser Glu Arg Tyr Tyr Val Asp Ser Val
50 55 60

Lys Gly Arg Phe Thr Ile Ser Arg Asp Ser Ala Lys Asn Ser Leu Tyr
65 70 75 80

Leu Gln Met Asn Ser Leu Arg Ala Glu Asp Thr Ala Val Tyr Tyr Cys
85 90 95

Ala Arg Glu Val Gly Tyr Asn Trp Asn Gln Gly Gly Tyr Phe Asp Tyr
100 105 110

Trp Gly Gln Gly Thr Leu Val Thr Val Ser Ser
115 120

<210> 310
<211> 123
<212> PRT
<213> Artificial Sequence

<220>
<223> Description of Artificial Sequence: Synthetic peptide

<400> 310
Glu Val Gln Leu Val Glu Ser Gly Gly Gly Leu Val Gln Pro Gly Gly
1 5 10 15

Ser Leu Arg Leu Ser Cys Val Val Ser Gly Phe Thr Phe Ser Ser Tyr
20 25 30

Trp Met Thr Trp Val Arg Gln Ala Pro Gly Lys Gly Leu Glu Trp Val
35 40 45

Ala Asn Ile Lys Gln Asp His Ser Glu Arg Tyr Tyr Val Asp Ser Val
50 55 60

Lys Gly Arg Phe Thr Ile Ser Arg Asp Ser Ala Lys Asn Ser Leu Tyr
65 70 75 80

Leu Gln Met Asn Ser Leu Arg Ala Glu Asp Thr Ala Val Tyr Tyr Cys
85 90 95

Ala Arg Glu Val Gly Tyr Asn Trp Asn Gln Gly Gly Tyr Phe Asp Tyr
100 105 110

Trp Gly Gln Gly Thr Leu Val Thr Val Ser Ser
115 120

<210> 311
<211> 110
<212> PRT
<213> Artificial Sequence

<220>
<223> Description of Artificial Sequence: Synthetic peptide

<400> 311
Gln Ser Val Leu Thr Gln Pro Pro Ser Ala Ser Gly Thr Pro Gly Gln
1 5 10 15

Arg Val Thr Ile Ser Cys Ser Gly Ser Ser Ser Asn Ile Gly Ser Asn
20 25 30

Ile Val Asn Trp Tyr Gln Gln Phe Pro Gly Thr Ala Pro Lys Leu Leu
35 40 45

Leu Tyr Ser Asp Asn Gln Arg Pro Ser Gly Val Pro Asp Arg Phe Ser
50 55 60

Gly Ser Lys Ser Gly Thr Ser Ala Ser Leu Ala Ile Ser Gly Leu Gln
65 70 75 80

Ser Glu Asp Glu Ala Asp Tyr Tyr Cys Ala Ala Trp Asp Asp Ser Leu
85 90 95

Val Gly Pro Val Phe Gly Thr Gly Thr Lys Val Thr Val Leu
100 105 110

<210> 312
<211> 110
<212> PRT
<213> Artificial Sequence

<220>
<223> Description of Artificial Sequence: Synthetic peptide

<400> 312
Gln Ser Val Leu Thr Gln Pro Pro Ser Ala Ser Gly Thr Pro Gly Gln
1 5 10 15

Arg Val Thr Ile Ser Cys Ser Gly Ser Ser Ser Asn Ile Gly Ser Asn
20 25 30

Ile Val Asn Trp Tyr Gln Gln Phe Pro Gly Thr Ala Pro Lys Leu Leu
35 40 45

Leu Tyr Ser Asp Asn Gln Arg Pro Ser Gly Val Pro Asp Arg Phe Ser
50 55 60

Gly Ser Lys Ser Gly Thr Ser Ala Ser Leu Ala Ile Ser Gly Leu Gln
65 70 75 80

Ser Glu Asp Glu Ala Asp Tyr Tyr Cys Ala Ala Trp Asp Asp Ser Leu
85 90 95

Arg Gly Pro Val Phe Gly Thr Gly Thr Lys Val Thr Val Leu
100 105 110

<210> 313
<211> 110
<212> PRT
<213> Artificial Sequence

<220>
<223> Description of Artificial Sequence: Synthetic peptide

<400> 313
Gln Ser Val Leu Thr Gln Pro Pro Ser Ala Ser Gly Thr Pro Gly Gln
1 5 10 15

Arg Val Thr Ile Ser Cys Ser Gly Ser Ser Ser Asn Ile Gly Ser Asn
20 25 30

Ile Val Asn Trp Tyr Gln Gln Phe Pro Gly Thr Ala Pro Lys Leu Leu
35 40 45

Leu Tyr Ser Asp Asn Gln Arg Pro Ser Gly Val Pro Asp Arg Phe Ser
50 55 60

Gly Ser Lys Ser Gly Thr Ser Ala Ser Leu Ala Ile Ser Gly Leu Gln
65 70 75 80

Ser Glu Asp Glu Ala Asp Tyr Tyr Cys Ala Ala Trp Asp Asp Ser Leu
85 90 95

Pro Gly Pro Val Phe Gly Thr Gly Thr Lys Val Thr Val Leu
100 105 110

<210> 314
<211> 110
<212> PRT
<213> Artificial Sequence

<220>
<223> Description of Artificial Sequence: Synthetic peptide

<400> 314
Gln Ser Val Leu Thr Gln Pro Pro Ser Ala Ser Gly Thr Pro Gly Gln
1 5 10 15

Arg Val Thr Ile Ser Cys Ser Gly Ser Ser Ser Asn Ile Gly Ser Asn
20 25 30

Ile Val Asn Trp Tyr Gln Gln Phe Pro Gly Thr Ala Pro Lys Leu Leu
35 40 45

Leu Tyr Ser Asp Asn Gln Arg Pro Ser Gly Val Pro Asp Arg Phe Ser
50 55 60

Gly Ser Lys Ser Gly Thr Ser Ala Ser Leu Ala Ile Ser Gly Leu Gln
65 70 75 80

Ser Glu Asp Glu Ala Asp Tyr Tyr Cys Ala Ala Trp Asp Asp Ser Leu
85 90 95

Thr Gly Pro Val Phe Gly Thr Gly Thr Lys Val Thr Val Leu
100 105 110

<210> 315
<211> 110
<212> PRT
<213> Artificial Sequence

<220>
<223> Description of Artificial Sequence: Synthetic peptide

<400> 315
Gln Ser Val Leu Thr Gln Pro Pro Ser Ala Ser Gly Thr Pro Gly Gln
1 5 10 15

Arg Val Thr Ile Ser Cys Ser Gly Ser Ser Ser Asn Ile Gly Ser Asn
20 25 30

Ile Val Asn Trp Tyr Gln Gln Phe Pro Gly Thr Ala Pro Lys Leu Leu
35 40 45

```
Leu Tyr Ser Asp Asn Gln Arg Pro Ser Gly Val Pro Asp Arg Phe Ser
    50                  55                  60

Gly Ser Lys Ser Gly Thr Ser Ala Ser Leu Ala Ile Ser Gly Leu Gln
65                  70                  75                  80

Ser Glu Asp Glu Ala Asp Tyr Tyr Cys Ala Ala Trp Asp Asp Ser Leu
                85                  90                  95

Gly Gly Pro Val Phe Gly Thr Gly Thr Lys Val Thr Val Leu
            100                 105                 110

<210>   316
<211>   110
<212>   PRT
<213>   Artificial Sequence

<220>
<223>   Description of Artificial Sequence: Synthetic peptide

<400>   316
Gln Ser Val Leu Thr Gln Pro Pro Ser Ala Ser Gly Thr Pro Gly Gln
1               5                   10                  15

Arg Val Thr Ile Ser Cys Ser Gly Ser Ser Ser Asn Ile Gly Ser Asn
            20                  25                  30

Ile Val Asn Trp Tyr Gln Gln Phe Pro Gly Thr Ala Pro Lys Leu Leu
        35                  40                  45

Leu Tyr Ser Asp Asn Gln Arg Pro Ser Gly Val Pro Asp Arg Phe Ser
    50                  55                  60

Gly Ser Lys Ser Gly Thr Ser Ala Ser Leu Ala Ile Ser Gly Leu Gln
65                  70                  75                  80

Ser Glu Asp Glu Ala Asp Tyr Tyr Cys Ala Ala Trp Asp Asp Ser Leu
                85                  90                  95

Gln Gly Pro Val Phe Gly Thr Gly Thr Lys Val Thr Val Leu
            100                 105                 110

<210>   317
<211>   110
<212>   PRT
<213>   Artificial Sequence

<220>
<223>   Description of Artificial Sequence: Synthetic peptide

<400>   317
Gln Ser Val Leu Thr Gln Pro Pro Ser Ala Ser Gly Thr Pro Gly Gln
1               5                   10                  15
```

```
Arg Val Thr Ile Ser Cys Ser Gly Ser Ser Ser Asn Ile Gly Ser Asn
            20                  25                  30

Ile Val Asn Trp Tyr Gln Gln Phe Pro Gly Thr Ala Pro Lys Leu Leu
        35                  40                  45

Leu Tyr Ser Asp Asn Gln Arg Pro Ser Gly Val Pro Asp Arg Phe Ser
    50                  55                  60

Gly Ser Lys Ser Gly Thr Ser Ala Ser Leu Ala Ile Ser Gly Leu Gln
65                  70                  75                  80

Ser Glu Asp Glu Ala Asp Tyr Tyr Cys Ala Ala Trp Asp Asp Ser Leu
                85                  90                  95

Ser Gly Pro Val Phe Gly Thr Gly Thr Lys Val Thr Val Leu
            100                 105                 110

<210>  318
<211>  110
<212>  PRT
<213>  Artificial Sequence

<220>
<223>  Description of Artificial Sequence: Synthetic peptide

<400>  318
Gln Ser Val Leu Thr Gln Pro Pro Ser Ala Ser Gly Thr Pro Gly Gln
1               5                   10                  15

Arg Val Thr Ile Ser Cys Ser Gly Ser Ser Ser Asn Ile Gly Ser Asn
            20                  25                  30

Ile Val Asn Trp Tyr Gln Gln Phe Pro Gly Thr Ala Pro Lys Leu Leu
        35                  40                  45

Leu Tyr Ser Asp Asn Gln Arg Pro Ser Gly Val Pro Asp Arg Phe Ser
    50                  55                  60

Gly Ser Lys Ser Gly Thr Ser Ala Ser Leu Ala Ile Ser Gly Leu Gln
65                  70                  75                  80

Ser Glu Asp Glu Ala Asp Tyr Tyr Cys Ala Ala Trp Asp Asp Ser Leu
                85                  90                  95

Ala Gly Pro Val Phe Gly Thr Gly Thr Lys Val Thr Val Leu
            100                 105                 110

<210>  319
<211>  110
<212>  PRT
<213>  Artificial Sequence
```

```
<220>
<223>   Description of Artificial Sequence: Synthetic peptide

<400>   319
Gln Ser Val Leu Thr Gln Pro Pro Ser Ala Ser Gly Thr Pro Gly Gln
1               5                   10                  15

Arg Val Thr Ile Ser Cys Ser Gly Ser Ser Ser Asn Ile Gly Ser Asn
            20                  25                  30

Ile Val Asn Trp Tyr Gln Gln Phe Pro Gly Thr Ala Pro Lys Leu Leu
        35                  40                  45

Leu Tyr Ser Asp Asn Gln Arg Pro Ser Gly Val Pro Asp Arg Phe Ser
    50                  55                  60

Gly Ser Lys Ser Gly Thr Ser Ala Ser Leu Ala Ile Ser Gly Leu Gln
65                  70                  75                  80

Ser Glu Asp Glu Ala Asp Tyr Tyr Cys Ala Ala Trp Asp Asp Ser Leu
                85                  90                  95

Leu Gly Pro Val Phe Gly Thr Gly Thr Lys Val Thr Val Leu
            100                 105                 110

<210>   320
<211>   110
<212>   PRT
<213>   Artificial Sequence

<220>
<223>   Description of Artificial Sequence: Synthetic peptide

<400>   320
Gln Ser Val Leu Thr Gln Pro Pro Ser Ala Ser Gly Thr Pro Gly Gln
1               5                   10                  15

Arg Val Thr Ile Ser Cys Ser Gly Ser Ser Ser Asn Ile Gly Ser Asn
            20                  25                  30

Ile Val Asn Trp Tyr Gln Gln Phe Pro Gly Thr Ala Pro Lys Leu Leu
        35                  40                  45

Leu Tyr Ser Asp Asn Gln Arg Pro Ser Gly Val Pro Asp Arg Phe Ser
    50                  55                  60

Gly Ser Lys Ser Gly Thr Ser Ala Ser Leu Ala Ile Ser Gly Leu Gln
65                  70                  75                  80

Ser Glu Asp Glu Ala Asp Tyr Tyr Cys Ala Ala Trp Asp Asp Ser Leu
                85                  90                  95

Tyr Gly Pro Val Phe Gly Thr Gly Thr Lys Val Thr Val Leu
            100                 105                 110
```

<210> 321
<211> 110
<212> PRT
<213> Artificial Sequence

<220>
<223> Description of Artificial Sequence: Synthetic peptide

<400> 321
```
Gln Ser Val Leu Thr Gln Pro Pro Ser Ala Ser Gly Thr Pro Gly Gln
1               5                   10                  15

Arg Val Thr Ile Ser Cys Ser Gly Ser Ser Ser Asn Ile Gly Ser Asn
            20                  25                  30

Ile Val Asn Trp Tyr Gln Gln Phe Pro Gly Thr Ala Pro Lys Leu Leu
        35                  40                  45

Leu Tyr Ser Asp Asn Gln Arg Pro Ser Gly Val Pro Asp Arg Phe Ser
    50                  55                  60

Gly Ser Lys Ser Gly Thr Ser Ala Ser Leu Ala Ile Ser Gly Leu Gln
65                  70                  75                  80

Ser Glu Asp Glu Ala Asp Tyr Tyr Cys Ala Ala Trp Asp Asp Ser Leu
                85                  90                  95

Glu Gly Pro Val Phe Gly Thr Gly Thr Lys Val Thr Val Leu
            100                 105                 110
```

<210> 322
<211> 110
<212> PRT
<213> Artificial Sequence

<220>
<223> Description of Artificial Sequence: Synthetic peptide

<400> 322
```
Gln Ser Val Leu Thr Gln Pro Pro Ser Ala Ser Gly Thr Pro Gly Gln
1               5                   10                  15

Arg Val Thr Ile Ser Cys Ser Gly Ser Ser Ser Asn Ile Gly Ser Asn
            20                  25                  30

Ile Val Asn Trp Tyr Gln Gln Phe Pro Gly Thr Ala Pro Lys Leu Leu
        35                  40                  45

Leu Tyr Ser Asp Asn Gln Arg Pro Ser Gly Val Pro Asp Arg Phe Ser
    50                  55                  60

Gly Ser Lys Ser Gly Thr Ser Ala Ser Leu Ala Ile Ser Gly Leu Gln
65                  70                  75                  80
```

```
Ser Glu Asp Glu Ala Asp Tyr Tyr Cys Ala Ala Trp Asp Asp Ser Leu
                85                  90                  95

Phe Gly Pro Val Phe Gly Thr Gly Thr Lys Val Thr Val Leu
            100                 105                 110

<210>   323
<211>   110
<212>   PRT
<213>   Artificial Sequence

<220>
<223>   Description of Artificial Sequence: Synthetic peptide

<400>   323
Gln Ser Val Leu Thr Gln Pro Pro Ser Ala Ser Gly Thr Pro Gly Gln
1               5                   10                  15

Arg Val Thr Ile Ser Cys Ser Gly Ser Ser Ser Asn Ile Gly Ser Asn
            20                  25                  30

Ile Val Asn Trp Tyr Gln Gln Phe Pro Gly Thr Ala Pro Lys Leu Leu
        35                  40                  45

Leu Tyr Ser Asp Asn Gln Arg Pro Ser Gly Val Pro Asp Arg Phe Ser
    50                  55                  60

Gly Ser Lys Ser Gly Thr Ser Ala Ser Leu Ala Ile Ser Gly Leu Gln
65                  70                  75                  80

Ser Glu Asp Glu Ala Asp Tyr Tyr Cys Ala Ala Trp Asp Asp Ser Leu
                85                  90                  95

Asp Gly Pro Val Phe Gly Thr Gly Thr Lys Val Thr Val Leu
            100                 105                 110

<210>   324
<211>   110
<212>   PRT
<213>   Artificial Sequence

<220>
<223>   Description of Artificial Sequence: Synthetic peptide

<400>   324
Gln Ser Val Leu Thr Gln Pro Pro Ser Ala Ser Gly Thr Pro Gly Gln
1               5                   10                  15

Arg Val Thr Ile Ser Cys Ser Gly Ser Ser Ser Asn Ile Gly Ser Asn
            20                  25                  30

Ile Val Asn Trp Tyr Gln Gln Phe Pro Gly Thr Ala Pro Lys Leu Leu
        35                  40                  45
```

```
Leu Tyr Ser Asp Asn Gln Arg Pro Ser Gly Val Pro Asp Arg Phe Ser
    50                  55                  60

Gly Ser Lys Ser Gly Thr Ser Ala Ser Leu Ala Ile Ser Gly Leu Gln
65                  70                  75                  80

Ser Glu Asp Glu Ala Asp Tyr Tyr Cys Ala Ala Trp Asp Asp Ser Leu
                85                  90                  95

Ile Gly Pro Val Phe Gly Thr Gly Thr Lys Val Thr Val Leu
            100                 105                 110

<210>   325
<211>   110
<212>   PRT
<213>   Artificial Sequence

<220>
<223>   Description of Artificial Sequence: Synthetic peptide

<400>   325
Gln Ser Val Leu Thr Gln Pro Pro Ser Ala Ser Gly Thr Pro Gly Gln
1               5                   10                  15

Arg Val Thr Ile Ser Cys Ser Gly Ser Ser Ser Asn Ile Gly Ser Asn
            20                  25                  30

Ile Val Asn Trp Tyr Gln Gln Phe Pro Gly Thr Ala Pro Lys Leu Leu
        35                  40                  45

Leu Tyr Ser Asp Asn Gln Arg Pro Ser Gly Val Pro Asp Arg Phe Ser
    50                  55                  60

Gly Ser Lys Ser Gly Thr Ser Ala Ser Leu Ala Ile Ser Gly Leu Gln
65                  70                  75                  80

Ser Glu Asp Glu Ala Asp Tyr Tyr Cys Ala Ala Trp Asp Asp Ser Leu
                85                  90                  95

His Gly Pro Val Phe Gly Thr Gly Thr Lys Val Thr Val Leu
            100                 105                 110

<210>   326
<211>   110
<212>   PRT
<213>   Artificial Sequence

<220>
<223>   Description of Artificial Sequence: Synthetic peptide

<400>   326
Gln Ser Val Leu Thr Gln Pro Pro Ser Ala Ser Gly Thr Pro Gly Gln
1               5                   10                  15
```

Arg Val Thr Ile Ser Cys Ser Gly Ser Ser Ser Asn Ile Gly Ser Asn
20 25 30

Ile Val Asn Trp Tyr Gln Gln Phe Pro Gly Thr Ala Pro Lys Leu Leu
35 40 45

Leu Tyr Ser Asp Asn Gln Arg Pro Ser Gly Val Pro Asp Arg Phe Ser
50 55 60

Gly Ser Lys Ser Gly Thr Ser Ala Ser Leu Ala Ile Ser Gly Leu Gln
65 70 75 80

Ser Glu Asp Glu Ala Asp Tyr Tyr Cys Ala Ala Trp Asp Asp Ser Leu
85 90 95

Asn Val Pro Val Phe Gly Thr Gly Thr Lys Val Thr Val Leu
100 105 110

<210> 327
<211> 110
<212> PRT
<213> Artificial Sequence

<220>
<223> Description of Artificial Sequence: Synthetic peptide

<400> 327
Gln Ser Val Leu Thr Gln Pro Pro Ser Ala Ser Gly Thr Pro Gly Gln
1 5 10 15

Arg Val Thr Ile Ser Cys Ser Gly Ser Ser Ser Asn Ile Gly Ser Asn
20 25 30

Ile Val Asn Trp Tyr Gln Gln Phe Pro Gly Thr Ala Pro Lys Leu Leu
35 40 45

Leu Tyr Ser Asp Asn Gln Arg Pro Ser Gly Val Pro Asp Arg Phe Ser
50 55 60

Gly Ser Lys Ser Gly Thr Ser Ala Ser Leu Ala Ile Ser Gly Leu Gln
65 70 75 80

Ser Glu Asp Glu Ala Asp Tyr Tyr Cys Ala Ala Trp Asp Asp Ser Leu
85 90 95

Asn Pro Pro Val Phe Gly Thr Gly Thr Lys Val Thr Val Leu
100 105 110

<210> 328
<211> 110
<212> PRT
<213> Artificial Sequence

<220>
<223>   Description of Artificial Sequence: Synthetic peptide

<400>   328
Gln Ser Val Leu Thr Gln Pro Pro Ser Ala Ser Gly Thr Pro Gly Gln
1               5                   10                  15

Arg Val Thr Ile Ser Cys Ser Gly Ser Ser Ser Asn Ile Gly Ser Asn
            20                  25                  30

Ile Val Asn Trp Tyr Gln Gln Phe Pro Gly Thr Ala Pro Lys Leu Leu
        35                  40                  45

Leu Tyr Ser Asp Asn Gln Arg Pro Ser Gly Val Pro Asp Arg Phe Ser
    50                  55                  60

Gly Ser Lys Ser Gly Thr Ser Ala Ser Leu Ala Ile Ser Gly Leu Gln
65                  70                  75                  80

Ser Glu Asp Glu Ala Asp Tyr Tyr Cys Ala Ala Trp Asp Asp Ser Leu
                85                  90                  95

Asn Arg Pro Val Phe Gly Thr Gly Thr Lys Val Thr Val Leu
            100                 105                 110

<210>   329
<211>   110
<212>   PRT
<213>   Artificial Sequence

<220>
<223>   Description of Artificial Sequence: Synthetic peptide

<400>   329
Gln Ser Val Leu Thr Gln Pro Pro Ser Ala Ser Gly Thr Pro Gly Gln
1               5                   10                  15

Arg Val Thr Ile Ser Cys Ser Gly Ser Ser Ser Asn Ile Gly Ser Asn
            20                  25                  30

Ile Val Asn Trp Tyr Gln Gln Phe Pro Gly Thr Ala Pro Lys Leu Leu
        35                  40                  45

Leu Tyr Ser Asp Asn Gln Arg Pro Ser Gly Val Pro Asp Arg Phe Ser
    50                  55                  60

Gly Ser Lys Ser Gly Thr Ser Ala Ser Leu Ala Ile Ser Gly Leu Gln
65                  70                  75                  80

Ser Glu Asp Glu Ala Asp Tyr Tyr Cys Ala Ala Trp Asp Asp Ser Leu
                85                  90                  95

Asn Asp Pro Val Phe Gly Thr Gly Thr Lys Val Thr Val Leu
            100                 105                 110

```
<210>   330
<211>   110
<212>   PRT
<213>   Artificial Sequence

<220>
<223>   Description of Artificial Sequence: Synthetic peptide

<400>   330
Gln Ser Val Leu Thr Gln Pro Pro Ser Ala Ser Gly Thr Pro Gly Gln
1               5                   10                  15

Arg Val Thr Ile Ser Cys Ser Gly Ser Ser Ser Asn Ile Gly Ser Asn
            20                  25                  30

Ile Val Asn Trp Tyr Gln Gln Phe Pro Gly Thr Ala Pro Lys Leu Leu
        35                  40                  45

Leu Tyr Ser Asp Asn Gln Arg Pro Ser Gly Val Pro Asp Arg Phe Ser
    50                  55                  60

Gly Ser Lys Ser Gly Thr Ser Ala Ser Leu Ala Ile Ser Gly Leu Gln
65                  70                  75                  80

Ser Glu Asp Glu Ala Asp Tyr Tyr Cys Ala Ala Trp Asp Asp Ser Leu
                85                  90                  95

Asn Glu Pro Val Phe Gly Thr Gly Thr Lys Val Thr Val Leu
            100                 105                 110

<210>   331
<211>   110
<212>   PRT
<213>   Artificial Sequence

<220>
<223>   Description of Artificial Sequence: Synthetic peptide

<400>   331
Gln Ser Val Leu Thr Gln Pro Pro Ser Ala Ser Gly Thr Pro Gly Gln
1               5                   10                  15

Arg Val Thr Ile Ser Cys Ser Gly Ser Ser Ser Asn Ile Gly Ser Asn
            20                  25                  30

Ile Val Asn Trp Tyr Gln Gln Phe Pro Gly Thr Ala Pro Lys Leu Leu
        35                  40                  45

Leu Tyr Ser Asp Asn Gln Arg Pro Ser Gly Val Pro Asp Arg Phe Ser
    50                  55                  60

Gly Ser Lys Ser Gly Thr Ser Ala Ser Leu Ala Ile Ser Gly Leu Gln
65                  70                  75                  80
```

```
Ser Glu Asp Glu Ala Asp Tyr Tyr Cys Ala Ala Trp Asp Asp Ser Leu
                85                  90                  95

Asn Trp Pro Val Phe Gly Thr Gly Thr Lys Val Thr Val Leu
            100                 105                 110

<210>   332
<211>   110
<212>   PRT
<213>   Artificial Sequence

<220>
<223>   Description of Artificial Sequence: Synthetic peptide

<400>   332
Gln Ser Val Leu Thr Gln Pro Pro Ser Ala Ser Gly Thr Pro Gly Gln
1               5                   10                  15

Arg Val Thr Ile Ser Cys Ser Gly Ser Ser Ser Asn Ile Gly Ser Asn
            20                  25                  30

Ile Val Asn Trp Tyr Gln Gln Phe Pro Gly Thr Ala Pro Lys Leu Leu
        35                  40                  45

Leu Tyr Ser Asp Asn Gln Arg Pro Ser Gly Val Pro Asp Arg Phe Ser
    50                  55                  60

Gly Ser Lys Ser Gly Thr Ser Ala Ser Leu Ala Ile Ser Gly Leu Gln
65                  70                  75                  80

Ser Glu Asp Glu Ala Asp Tyr Tyr Cys Ala Ala Trp Asp Asp Ser Leu
                85                  90                  95

Asn Tyr Pro Val Phe Gly Thr Gly Thr Lys Val Thr Val Leu
            100                 105                 110

<210>   333
<211>   110
<212>   PRT
<213>   Artificial Sequence

<220>
<223>   Description of Artificial Sequence: Synthetic peptide

<400>   333
Gln Ser Val Leu Thr Gln Pro Pro Ser Ala Ser Gly Thr Pro Gly Gln
1               5                   10                  15

Arg Val Thr Ile Ser Cys Ser Gly Ser Ser Ser Asn Ile Gly Ser Asn
            20                  25                  30

Ile Val Asn Trp Tyr Gln Gln Phe Pro Gly Thr Ala Pro Lys Leu Leu
        35                  40                  45
```

```
Leu Tyr Ser Asp Asn Gln Arg Pro Ser Gly Val Pro Asp Arg Phe Ser
    50                  55                  60

Gly Ser Lys Ser Gly Thr Ser Ala Ser Leu Ala Ile Ser Gly Leu Gln
65                  70                  75                  80

Ser Glu Asp Glu Ala Asp Tyr Tyr Cys Ala Ala Trp Asp Asp Ser Leu
                85                  90                  95

Asn Ala Pro Val Phe Gly Thr Gly Thr Lys Val Thr Val Leu
            100                 105                 110

<210>   334
<211>   110
<212>   PRT
<213>   Artificial Sequence

<220>
<223>   Description of Artificial Sequence: Synthetic peptide

<400>   334
Gln Ser Val Leu Thr Gln Pro Pro Ser Ala Ser Gly Thr Pro Gly Gln
1               5                   10                  15

Arg Val Thr Ile Ser Cys Ser Gly Ser Ser Ser Asn Ile Gly Ser Asn
            20                  25                  30

Ile Val Asn Trp Tyr Gln Gln Phe Pro Gly Thr Ala Pro Lys Leu Leu
        35                  40                  45

Leu Tyr Ser Asp Asn Gln Arg Pro Ser Gly Val Pro Asp Arg Phe Ser
    50                  55                  60

Gly Ser Lys Ser Gly Thr Ser Ala Ser Leu Ala Ile Ser Gly Leu Gln
65                  70                  75                  80

Ser Glu Asp Glu Ala Asp Tyr Tyr Cys Ala Ala Trp Asp Asp Ser Leu
                85                  90                  95

Asn Asn Pro Val Phe Gly Thr Gly Thr Lys Val Thr Val Leu
            100                 105                 110

<210>   335
<211>   110
<212>   PRT
<213>   Artificial Sequence

<220>
<223>   Description of Artificial Sequence: Synthetic peptide

<400>   335
Gln Ser Val Leu Thr Gln Pro Pro Ser Ala Ser Gly Thr Pro Gly Gln
1               5                   10                  15
```

```
Arg Val Thr Ile Ser Cys Ser Gly Ser Ser Ser Asn Ile Gly Ser Asn
            20                  25                  30

Ile Val Asn Trp Tyr Gln Gln Phe Pro Gly Thr Ala Pro Lys Leu Leu
        35                  40                  45

Leu Tyr Ser Asp Asn Gln Arg Pro Ser Gly Val Pro Asp Arg Phe Ser
    50                  55                  60

Gly Ser Lys Ser Gly Thr Ser Ala Ser Leu Ala Ile Ser Gly Leu Gln
65                  70                  75                  80

Ser Glu Asp Glu Ala Asp Tyr Tyr Cys Ala Ala Trp Asp Asp Ser Leu
                85                  90                  95

Asn Lys Pro Val Phe Gly Thr Gly Thr Lys Val Thr Val Leu
            100                 105                 110

<210>   336
<211>   110
<212>   PRT
<213>   Artificial Sequence

<220>
<223>   Description of Artificial Sequence: Synthetic peptide

<400>   336
Gln Ser Val Leu Thr Gln Pro Pro Ser Ala Ser Gly Thr Pro Gly Gln
1               5                   10                  15

Arg Val Thr Ile Ser Cys Ser Gly Ser Ser Ser Asn Ile Gly Ser Asn
            20                  25                  30

Ile Val Asn Trp Tyr Gln Gln Phe Pro Gly Thr Ala Pro Lys Leu Leu
        35                  40                  45

Leu Tyr Ser Asp Asn Gln Arg Pro Ser Gly Val Pro Asp Arg Phe Ser
    50                  55                  60

Gly Ser Lys Ser Gly Thr Ser Ala Ser Leu Ala Ile Ser Gly Leu Gln
65                  70                  75                  80

Ser Glu Asp Glu Ala Asp Tyr Tyr Cys Ala Ala Trp Asp Asp Ser Leu
                85                  90                  95

Asn Leu Pro Val Phe Gly Thr Gly Thr Lys Val Thr Val Leu
            100                 105                 110

<210>   337
<211>   117
<212>   PRT
<213>   Artificial Sequence
```

<220>
<223> Description of Artificial Sequence: Synthetic peptide

<400> 337
```
Gln Val Gln Leu Gln Glu Ser Gly Pro Gly Leu Val Lys Pro Ser Glu
1               5                   10                  15

Thr Leu Ser Leu Thr Cys Ser Val Ser Gly Gly Ser Ile Arg Asn Tyr
            20                  25                  30

Tyr Trp Ser Trp Ile Arg Gln Ser Ala Gly Lys Glu Leu Glu Trp Phe
        35                  40                  45

Gly His Ile Phe Ser Thr Gly His Ile Asn Tyr Asp Ser Ser Leu Lys
    50                  55                  60

Ser Arg Val Thr Met Ser Val Asp Thr Ser Asn Asn Gln Phe Ser Leu
65                  70                  75                  80

Lys Leu Arg Ser Val Thr Ala Ala Asp Thr Ala Val Tyr Tyr Cys Ala
                85                  90                  95

Arg Asp Val Gly Ala Ala Leu Phe Asp Phe Trp Gly Gln Gly Thr Leu
            100                 105                 110

Val Thr Val Ser Ser
        115
```

<210> 338
<211> 117
<212> PRT
<213> Artificial Sequence

<220>
<223> Description of Artificial Sequence: Synthetic peptide

<400> 338
```
Gln Val Gln Leu Gln Glu Ser Gly Pro Gly Leu Val Lys Pro Ser Glu
1               5                   10                  15

Thr Leu Ser Leu Thr Cys Ser Val Ser Gly Gly Ser Ile Arg Asn Tyr
            20                  25                  30

Tyr Trp Ser Trp Ile Arg Gln Ser Ala Gly Lys Glu Leu Glu Trp Phe
        35                  40                  45

Gly His Ile Phe Ser Thr Gly His Ile Asn Tyr Asp Ser Ser Leu Lys
    50                  55                  60

Ser Arg Val Thr Met Ser Val Asp Thr Ser Asn Asn Gln Phe Ser Leu
65                  70                  75                  80

Lys Leu Arg Ser Val Thr Ala Ala Asp Thr Ala Val Tyr Tyr Cys Ala
                85                  90                  95
```

Arg Asp Ser Gly Ala Ala Leu Phe Asp Phe Trp Gly Gln Gly Thr Leu
100 105 110

Val Thr Val Ser Ser
115

<210> 339
<211> 117
<212> PRT
<213> Artificial Sequence

<220>
<223> Description of Artificial Sequence: Synthetic peptide

<400> 339
Gln Val Gln Leu Gln Glu Ser Gly Pro Gly Leu Val Lys Pro Ser Glu
1 5 10 15

Thr Leu Ser Leu Thr Cys Ser Val Ser Gly Gly Ser Ile Arg Asn Tyr
20 25 30

Tyr Trp Ser Trp Ile Arg Gln Ser Ala Gly Lys Glu Leu Glu Trp Phe
35 40 45

Gly His Ile Phe Ser Thr Gly His Ile Asn Tyr Asp Ser Ser Leu Lys
50 55 60

Ser Arg Val Thr Met Ser Val Asp Thr Ser Asn Asn Gln Phe Ser Leu
65 70 75 80

Lys Leu Arg Ser Val Thr Ala Ala Asp Thr Ala Val Tyr Tyr Cys Ala
85 90 95

Arg Asp Glu Gly Ala Ala Leu Phe Asp Phe Trp Gly Gln Gly Thr Leu
100 105 110

Val Thr Val Ser Ser
115

<210> 340
<211> 117
<212> PRT
<213> Artificial Sequence

<220>
<223> Description of Artificial Sequence: Synthetic peptide

<400> 340
Gln Val Gln Leu Gln Glu Ser Gly Pro Gly Leu Val Lys Pro Ser Glu
1 5 10 15

Thr Leu Ser Leu Thr Cys Ser Val Ser Gly Gly Ser Ile Arg Asn Tyr
20 25 30

```
Tyr Trp Ser Trp Ile Arg Gln Ser Ala Gly Lys Glu Leu Glu Trp Phe
        35                  40                  45

Gly His Ile Phe Ser Thr Gly His Ile Asn Tyr Asp Ser Ser Leu Lys
    50                  55                  60

Ser Arg Val Thr Met Ser Val Asp Thr Ser Asn Asn Gln Phe Ser Leu
65                  70                  75                  80

Lys Leu Arg Ser Val Thr Ala Ala Asp Thr Ala Val Tyr Tyr Cys Ala
                85                  90                  95

Arg Asp His Gly Ala Ala Leu Phe Asp Phe Trp Gly Gln Gly Thr Leu
            100                 105                 110

Val Thr Val Ser Ser
        115

<210>   341
<211>   117
<212>   PRT
<213>   Artificial Sequence

<220>
<223>   Description of Artificial Sequence: Synthetic peptide

<400>   341
Gln Val Gln Leu Gln Glu Ser Gly Pro Gly Leu Val Lys Pro Ser Glu
1               5                   10                  15

Thr Leu Ser Leu Thr Cys Ser Val Ser Gly Gly Ser Ile Arg Asn Tyr
            20                  25                  30

Tyr Trp Ser Trp Ile Arg Gln Ser Ala Gly Lys Glu Leu Glu Trp Phe
        35                  40                  45

Gly His Ile Phe Ser Thr Gly His Ile Asn Tyr Asp Ser Ser Leu Lys
    50                  55                  60

Ser Arg Val Thr Met Ser Val Asp Thr Ser Asn Asn Gln Phe Ser Leu
65                  70                  75                  80

Lys Leu Arg Ser Val Thr Ala Ala Asp Thr Ala Val Tyr Tyr Cys Ala
                85                  90                  95

Arg Asp Ala Gly Ala Ala Leu Phe Asp Phe Trp Gly Gln Gly Thr Leu
            100                 105                 110

Val Thr Val Ser Ser
        115

<210>   342
<211>   117
```

```
<212>   PRT
<213>   Artificial Sequence

<220>
<223>   Description of Artificial Sequence: Synthetic peptide

<400>   342
Gln Val Gln Leu Gln Glu Ser Gly Pro Gly Leu Val Lys Pro Ser Glu
1               5                   10                  15

Thr Leu Ser Leu Thr Cys Ser Val Ser Gly Gly Ser Ile Arg Asn Tyr
            20                  25                  30

Tyr Trp Ser Trp Ile Arg Gln Ser Ala Gly Lys Glu Leu Glu Trp Phe
        35                  40                  45

Gly His Ile Phe Ser Thr Gly His Ile Asn Tyr Asp Ser Ser Leu Lys
    50                  55                  60

Ser Arg Val Thr Met Ser Val Asp Thr Ser Asn Asn Gln Phe Ser Leu
65                  70                  75                  80

Lys Leu Arg Ser Val Thr Ala Ala Asp Thr Ala Val Tyr Tyr Cys Ala
                85                  90                  95

Arg Asp Gly Gly Ala Ala Leu Phe Asp Phe Trp Gly Gln Gly Thr Leu
            100                 105                 110

Val Thr Val Ser Ser
        115

<210>   343
<211>   117
<212>   PRT
<213>   Artificial Sequence

<220>
<223>   Description of Artificial Sequence: Synthetic peptide

<400>   343
Gln Val Gln Leu Gln Glu Ser Gly Pro Gly Leu Val Lys Pro Ser Glu
1               5                   10                  15

Thr Leu Ser Leu Thr Cys Ser Val Ser Gly Gly Ser Ile Arg Asn Tyr
            20                  25                  30

Tyr Trp Ser Trp Ile Arg Gln Ser Ala Gly Lys Glu Leu Glu Trp Phe
        35                  40                  45

Gly His Ile Phe Ser Thr Gly His Ile Asn Tyr Asp Ser Ser Leu Lys
    50                  55                  60

Ser Arg Val Thr Met Ser Val Asp Thr Ser Asn Asn Gln Phe Ser Leu
65                  70                  75                  80
```

```
Lys Leu Arg Ser Val Thr Ala Ala Asp Thr Ala Val Tyr Tyr Cys Ala
                85                  90                  95

Arg Asp Lys Gly Ala Ala Leu Phe Asp Phe Trp Gly Gln Gly Thr Leu
            100                 105                 110

Val Thr Val Ser Ser
        115

<210>   344
<211>   117
<212>   PRT
<213>   Artificial Sequence

<220>
<223>   Description of Artificial Sequence: Synthetic peptide

<400>   344
Gln Val Gln Leu Gln Glu Ser Gly Pro Gly Leu Val Lys Pro Ser Glu
1               5                   10                  15

Thr Leu Ser Leu Thr Cys Ser Val Ser Gly Gly Ser Ile Arg Asn Tyr
            20                  25                  30

Tyr Trp Ser Trp Ile Arg Gln Ser Ala Gly Lys Glu Leu Glu Trp Phe
        35                  40                  45

Gly His Ile Phe Ser Thr Gly His Ile Asn Tyr Asp Ser Ser Leu Lys
    50                  55                  60

Ser Arg Val Thr Met Ser Val Asp Thr Ser Asn Asn Gln Phe Ser Leu
65                  70                  75                  80

Lys Leu Arg Ser Val Thr Ala Ala Asp Thr Ala Val Tyr Tyr Cys Ala
                85                  90                  95

Arg Asp Thr Gly Ala Ala Leu Phe Asp Phe Trp Gly Gln Gly Thr Leu
            100                 105                 110

Val Thr Val Ser Ser
        115

<210>   345
<211>   117
<212>   PRT
<213>   Artificial Sequence

<220>
<223>   Description of Artificial Sequence: Synthetic peptide

<400>   345
Gln Val Gln Leu Gln Glu Ser Gly Pro Gly Leu Val Lys Pro Ser Glu
1               5                   10                  15
```

Thr Leu Ser Leu Thr Cys Ser Val Ser Gly Gly Ser Ile Arg Asn Tyr
20 25 30

Tyr Trp Ser Trp Ile Arg Gln Ser Ala Gly Lys Glu Leu Glu Trp Phe
35 40 45

Gly His Ile Phe Ser Thr Gly His Ile Asn Tyr Asp Ser Ser Leu Lys
50 55 60

Ser Arg Val Thr Met Ser Val Asp Thr Ser Asn Asn Gln Phe Ser Leu
65 70 75 80

Lys Leu Arg Ser Val Thr Ala Ala Asp Thr Ala Val Tyr Tyr Cys Ala
85 90 95

Arg Asp Phe Gly Ala Ala Leu Phe Asp Phe Trp Gly Gln Gly Thr Leu
100 105 110

Val Thr Val Ser Ser
115

<210> 346
<211> 117
<212> PRT
<213> Artificial Sequence

<220>
<223> Description of Artificial Sequence: Synthetic peptide

<400> 346
Gln Val Gln Leu Gln Glu Ser Gly Pro Gly Leu Val Lys Pro Ser Glu
1 5 10 15

Thr Leu Ser Leu Thr Cys Ser Val Ser Gly Gly Ser Ile Arg Asn Tyr
20 25 30

Tyr Trp Ser Trp Ile Arg Gln Ser Ala Gly Lys Glu Leu Glu Trp Phe
35 40 45

Gly His Ile Phe Ser Thr Gly His Ile Asn Tyr Asp Ser Ser Leu Lys
50 55 60

Ser Arg Val Thr Met Ser Val Asp Thr Ser Asn Asn Gln Phe Ser Leu
65 70 75 80

Lys Leu Arg Ser Val Thr Ala Ala Asp Thr Ala Val Tyr Tyr Cys Ala
85 90 95

Arg Asp Val Gly Ala Ala Leu Phe Asp Phe Trp Gly Gln Gly Thr Leu
100 105 110

Val Thr Val Ser Ser
115

<210> 347
<211> 117
<212> PRT
<213> Artificial Sequence

<220>
<223> Description of Artificial Sequence: Synthetic peptide

<400> 347
Gln Val Gln Leu Gln Glu Ser Gly Pro Gly Leu Val Lys Pro Ser Glu
1 5 10 15

Thr Leu Ser Leu Thr Cys Ser Val Ser Gly Gly Ser Ile Arg Asn Tyr
20 25 30

Tyr Trp Ser Trp Ile Arg Gln Ser Ala Gly Lys Glu Leu Glu Trp Phe
35 40 45

Gly His Ile Phe Ser Thr Gly His Ile Asn Tyr Asp Ser Ser Leu Lys
50 55 60

Ser Arg Val Thr Met Ser Val Asp Thr Ser Asn Asn Gln Phe Ser Leu
65 70 75 80

Lys Leu Arg Ser Val Thr Ala Ala Asp Thr Ala Val Tyr Tyr Cys Ala
85 90 95

Arg Asp Arg Gly Ala Ala Leu Phe Asp Phe Trp Gly Gln Gly Thr Leu
100 105 110

Val Thr Val Ser Ser
115

<210> 348
<211> 117
<212> PRT
<213> Artificial Sequence

<220>
<223> Description of Artificial Sequence: Synthetic peptide

<400> 348
Gln Val Gln Leu Gln Glu Ser Gly Pro Gly Leu Val Lys Pro Ser Glu
1 5 10 15

Thr Leu Ser Leu Thr Cys Ser Val Ser Gly Gly Ser Ile Arg Asn Tyr
20 25 30

Tyr Trp Ser Trp Ile Arg Gln Ser Ala Gly Lys Glu Leu Glu Trp Phe
35 40 45

Gly His Ile Phe Ser Thr Gly His Ile Asn Tyr Asp Ser Ser Leu Lys
50 55 60

Ser Arg Val Thr Met Ser Val Asp Thr Ser Asn Asn Gln Phe Ser Leu
65 70 75 80

Lys Leu Arg Ser Val Thr Ala Ala Asp Thr Ala Val Tyr Tyr Cys Ala
85 90 95

Arg Asp Leu Gly Ala Ala Leu Phe Asp Phe Trp Gly Gln Gly Thr Leu
100 105 110

Val Thr Val Ser Ser
115

<210> 349
<211> 117
<212> PRT
<213> Artificial Sequence

<220>
<223> Description of Artificial Sequence: Synthetic peptide

<400> 349
Gln Val Gln Leu Gln Glu Ser Gly Pro Gly Leu Val Lys Pro Ser Glu
1 5 10 15

Thr Leu Ser Leu Thr Cys Ser Val Ser Gly Gly Ser Ile Arg Asn Tyr
20 25 30

Tyr Trp Ser Trp Ile Arg Gln Ser Ala Gly Lys Glu Leu Glu Trp Phe
35 40 45

Gly His Ile Phe Ser Thr Gly His Ile Asn Tyr Asp Ser Ser Leu Lys
50 55 60

Ser Arg Val Thr Met Ser Val Asp Thr Ser Asn Asn Gln Phe Ser Leu
65 70 75 80

Lys Leu Arg Ser Val Thr Ala Ala Asp Thr Ala Val Tyr Tyr Cys Ala
85 90 95

Arg Asp His Gly Ala Ala Leu Phe Asp Phe Trp Gly Gln Gly Thr Leu
100 105 110

Val Thr Val Ser Ser
115

<210> 350
<211> 117
<212> PRT
<213> Artificial Sequence

<220>
<223> Description of Artificial Sequence: Synthetic peptide

```
<400>   350
Gln Val Gln Leu Gln Glu Ser Gly Pro Gly Leu Val Lys Pro Ser Glu
1               5                   10                  15

Thr Leu Ser Leu Thr Cys Ser Val Ser Gly Gly Ser Ile Arg Asn Tyr
            20                  25                  30

Tyr Trp Ser Trp Ile Arg Gln Ser Ala Gly Lys Glu Leu Glu Trp Phe
        35                  40                  45

Gly His Ile Phe Ser Thr Gly His Ile Asn Tyr Asp Ser Ser Leu Lys
    50                  55                  60

Ser Arg Val Thr Met Ser Val Asp Thr Ser Asn Asn Gln Phe Ser Leu
65                  70                  75                  80

Lys Leu Arg Ser Val Thr Ala Ala Asp Thr Ala Val Tyr Tyr Cys Ala
                85                  90                  95

Arg Asp Ile Gly Ala Ala Leu Phe Asp Phe Trp Gly Gln Gly Thr Leu
            100                 105                 110

Val Thr Val Ser Ser
        115

<210>   351
<211>   117
<212>   PRT
<213>   Artificial Sequence

<220>
<223>   Description of Artificial Sequence: Synthetic peptide

<400>   351
Gln Val Gln Leu Gln Glu Ser Gly Pro Gly Leu Val Lys Pro Ser Glu
1               5                   10                  15

Thr Leu Ser Leu Thr Cys Ser Val Ser Gly Gly Ser Ile Arg Asn Tyr
            20                  25                  30

Tyr Trp Ser Trp Ile Arg Gln Ser Ala Gly Lys Glu Leu Glu Trp Phe
        35                  40                  45

Gly His Ile Phe Ser Thr Gly His Ile Asn Tyr Asp Ser Ser Leu Lys
    50                  55                  60

Ser Arg Val Thr Met Ser Val Asp Thr Ser Asn Asn Gln Phe Ser Leu
65                  70                  75                  80

Lys Leu Arg Ser Val Thr Ala Ala Asp Thr Ala Val Tyr Tyr Cys Ala
                85                  90                  95

Arg Asp Asn Ser Ala Ala Leu Phe Asp Phe Trp Gly Gln Gly Thr Leu
            100                 105                 110
```

Val Thr Val Ser Ser
115

<210> 352
<211> 117
<212> PRT
<213> Artificial Sequence

<220>
<223> Description of Artificial Sequence: Synthetic peptide

<400> 352
Gln Val Gln Leu Gln Glu Ser Gly Pro Gly Leu Val Lys Pro Ser Glu
1 5 10 15

Thr Leu Ser Leu Thr Cys Ser Val Ser Gly Gly Ser Ile Arg Asn Tyr
20 25 30

Tyr Trp Ser Trp Ile Arg Gln Ser Ala Gly Lys Glu Leu Glu Trp Phe
35 40 45

Gly His Ile Phe Ser Thr Gly His Ile Asn Tyr Asp Ser Ser Leu Lys
50 55 60

Ser Arg Val Thr Met Ser Val Asp Thr Ser Asn Asn Gln Phe Ser Leu
65 70 75 80

Lys Leu Arg Ser Val Thr Ala Ala Asp Thr Ala Val Tyr Tyr Cys Ala
85 90 95

Arg Asp Asn Ala Ala Ala Leu Phe Asp Phe Trp Gly Gln Gly Thr Leu
100 105 110

Val Thr Val Ser Ser
115

<210> 353
<211> 110
<212> PRT
<213> Artificial Sequence

<220>
<223> Description of Artificial Sequence: Synthetic peptide

<400> 353
Gln Ser Val Leu Thr Gln Pro Pro Ser Ala Ser Gly Thr Pro Gly Gln
1 5 10 15

Arg Val Thr Ile Ser Cys Ser Gly Ser Ser Ser Asn Ile Gly Ser Asn
20 25 30

Ile Val Asn Trp Tyr Gln Gln Phe Pro Gly Thr Ala Pro Lys Leu Leu
35 40 45

```
Ile Tyr Ser Asn Asn Gln Arg Pro Ser Gly Val Pro Asp Arg Val Ser
    50                  55                  60

Gly Ser Lys Ser Gly Thr Ser Ala Ser Leu Ala Ile Ser Gly Leu Gln
65                  70                  75                  80

Ser Glu Asp Glu Ala Asp Tyr Tyr Cys Ala Ala Trp Asp Asp Ser Leu
                85                  90                  95

Gly Gly Pro Val Phe Gly Pro Gly Thr Lys Val Thr Val Leu
            100                 105                 110

<210>   354
<211>   110
<212>   PRT
<213>   Artificial Sequence

<220>
<223>   Description of Artificial Sequence: Synthetic peptide

<400>   354
Gln Ser Val Leu Thr Gln Pro Pro Ser Ala Ser Gly Thr Pro Gly Gln
1               5                   10                  15

Arg Val Thr Ile Ser Cys Ser Gly Ser Ser Ser Asn Ile Gly Ser Asn
            20                  25                  30

Ile Val Asn Trp Tyr Gln Gln Phe Pro Gly Thr Ala Pro Lys Leu Leu
        35                  40                  45

Ile Tyr Ser Asn Asn Gln Arg Pro Ser Gly Val Pro Asp Arg Val Ser
    50                  55                  60

Gly Ser Lys Ser Gly Thr Ser Ala Ser Leu Ala Ile Ser Gly Leu Gln
65                  70                  75                  80

Ser Glu Asp Glu Ala Asp Tyr Tyr Cys Ala Ala Trp Asp Asp Ser Leu
                85                  90                  95

Asp Gly Pro Val Phe Gly Pro Gly Thr Lys Val Thr Val Leu
            100                 105                 110

<210>   355
<211>   110
<212>   PRT
<213>   Artificial Sequence

<220>
<223>   Description of Artificial Sequence: Synthetic peptide

<400>   355
Gln Ser Val Leu Thr Gln Pro Pro Ser Ala Ser Gly Thr Pro Gly Gln
1               5                   10                  15
```

Arg Val Thr Ile Ser Cys Ser Gly Ser Ser Ser Asn Ile Gly Ser Asn
20 25 30

Ile Val Asn Trp Tyr Gln Gln Phe Pro Gly Thr Ala Pro Lys Leu Leu
35 40 45

Ile Tyr Ser Asn Asn Gln Arg Pro Ser Gly Val Pro Asp Arg Val Ser
50 55 60

Gly Ser Lys Ser Gly Thr Ser Ala Ser Leu Ala Ile Ser Gly Leu Gln
65 70 75 80

Ser Glu Asp Glu Ala Asp Tyr Tyr Cys Ala Ala Trp Asp Asp Ser Leu
85 90 95

Pro Gly Pro Val Phe Gly Pro Gly Thr Lys Val Thr Val Leu
100 105 110

<210> 356
<211> 110
<212> PRT
<213> Artificial Sequence

<220>
<223> Description of Artificial Sequence: Synthetic peptide

<400> 356
Gln Ser Val Leu Thr Gln Pro Pro Ser Ala Ser Gly Thr Pro Gly Gln
1 5 10 15

Arg Val Thr Ile Ser Cys Ser Gly Ser Ser Ser Asn Ile Gly Ser Asn
20 25 30

Ile Val Asn Trp Tyr Gln Gln Phe Pro Gly Thr Ala Pro Lys Leu Leu
35 40 45

Ile Tyr Ser Asn Asn Gln Arg Pro Ser Gly Val Pro Asp Arg Val Ser
50 55 60

Gly Ser Lys Ser Gly Thr Ser Ala Ser Leu Ala Ile Ser Gly Leu Gln
65 70 75 80

Ser Glu Asp Glu Ala Asp Tyr Tyr Cys Ala Ala Trp Asp Asp Ser Leu
85 90 95

Lys Gly Pro Val Phe Gly Pro Gly Thr Lys Val Thr Val Leu
100 105 110

<210> 357
<211> 110
<212> PRT
<213> Artificial Sequence

<220>
<223> Description of Artificial Sequence: Synthetic peptide

<400> 357
Gln Ser Val Leu Thr Gln Pro Pro Ser Ala Ser Gly Thr Pro Gly Gln
1 5 10 15

Arg Val Thr Ile Ser Cys Ser Gly Ser Ser Ser Asn Ile Gly Ser Asn
20 25 30

Ile Val Asn Trp Tyr Gln Gln Phe Pro Gly Thr Ala Pro Lys Leu Leu
35 40 45

Ile Tyr Ser Asn Asn Gln Arg Pro Ser Gly Val Pro Asp Arg Val Ser
50 55 60

Gly Ser Lys Ser Gly Thr Ser Ala Ser Leu Ala Ile Ser Gly Leu Gln
65 70 75 80

Ser Glu Asp Glu Ala Asp Tyr Tyr Cys Ala Ala Trp Asp Asp Ser Leu
85 90 95

Glu Gly Pro Val Phe Gly Pro Gly Thr Lys Val Thr Val Leu
100 105 110

<210> 358
<211> 110
<212> PRT
<213> Artificial Sequence

<220>
<223> Description of Artificial Sequence: Synthetic peptide

<400> 358
Gln Ser Val Leu Thr Gln Pro Pro Ser Ala Ser Gly Thr Pro Gly Gln
1 5 10 15

Arg Val Thr Ile Ser Cys Ser Gly Ser Ser Ser Asn Ile Gly Ser Asn
20 25 30

Ile Val Asn Trp Tyr Gln Gln Phe Pro Gly Thr Ala Pro Lys Leu Leu
35 40 45

Ile Tyr Ser Asn Asn Gln Arg Pro Ser Gly Val Pro Asp Arg Val Ser
50 55 60

Gly Ser Lys Ser Gly Thr Ser Ala Ser Leu Ala Ile Ser Gly Leu Gln
65 70 75 80

Ser Glu Asp Glu Ala Asp Tyr Tyr Cys Ala Ala Trp Asp Asp Ser Leu
85 90 95

Ala Gly Pro Val Phe Gly Pro Gly Thr Lys Val Thr Val Leu
100 105 110

```
<210>   359
<211>   110
<212>   PRT
<213>   Artificial Sequence

<220>
<223>   Description of Artificial Sequence: Synthetic peptide

<400>   359
Gln Ser Val Leu Thr Gln Pro Pro Ser Ala Ser Gly Thr Pro Gly Gln
1               5                   10                  15

Arg Val Thr Ile Ser Cys Ser Gly Ser Ser Ser Asn Ile Gly Ser Asn
            20                  25                  30

Ile Val Asn Trp Tyr Gln Gln Phe Pro Gly Thr Ala Pro Lys Leu Leu
        35                  40                  45

Ile Tyr Ser Asn Asn Gln Arg Pro Ser Gly Val Pro Asp Arg Val Ser
    50                  55                  60

Gly Ser Lys Ser Gly Thr Ser Ala Ser Leu Ala Ile Ser Gly Leu Gln
65                  70                  75                  80

Ser Glu Asp Glu Ala Asp Tyr Tyr Cys Ala Ala Trp Asp Asp Ser Leu
                85                  90                  95

Arg Gly Pro Val Phe Gly Pro Gly Thr Lys Val Thr Val Leu
            100                 105                 110

<210>   360
<211>   110
<212>   PRT
<213>   Artificial Sequence

<220>
<223>   Description of Artificial Sequence: Synthetic peptide

<400>   360
Gln Ser Val Leu Thr Gln Pro Pro Ser Ala Ser Gly Thr Pro Gly Gln
1               5                   10                  15

Arg Val Thr Ile Ser Cys Ser Gly Ser Ser Ser Asn Ile Gly Ser Asn
            20                  25                  30

Ile Val Asn Trp Tyr Gln Gln Phe Pro Gly Thr Ala Pro Lys Leu Leu
        35                  40                  45

Ile Tyr Ser Asn Asn Gln Arg Pro Ser Gly Val Pro Asp Arg Val Ser
    50                  55                  60

Gly Ser Lys Ser Gly Thr Ser Ala Ser Leu Ala Ile Ser Gly Leu Gln
65                  70                  75                  80
```

```
Ser Glu Asp Glu Ala Asp Tyr Tyr Cys Ala Ala Trp Asp Asp Ser Leu
                85                  90                  95

Gln Gly Pro Val Phe Gly Pro Gly Thr Lys Val Thr Val Leu
            100                 105                 110

<210>   361
<211>   110
<212>   PRT
<213>   Artificial Sequence

<220>
<223>   Description of Artificial Sequence: Synthetic peptide

<400>   361
Gln Ser Val Leu Thr Gln Pro Pro Ser Ala Ser Gly Thr Pro Gly Gln
1               5                   10                  15

Arg Val Thr Ile Ser Cys Ser Gly Ser Ser Ser Asn Ile Gly Ser Asn
            20                  25                  30

Ile Val Asn Trp Tyr Gln Gln Phe Pro Gly Thr Ala Pro Lys Leu Leu
        35                  40                  45

Ile Tyr Ser Asn Asn Gln Arg Pro Ser Gly Val Pro Asp Arg Val Ser
    50                  55                  60

Gly Ser Lys Ser Gly Thr Ser Ala Ser Leu Ala Ile Ser Gly Leu Gln
65                  70                  75                  80

Ser Glu Asp Glu Ala Asp Tyr Tyr Cys Ala Ala Trp Asp Asp Ser Leu
                85                  90                  95

Ile Gly Pro Val Phe Gly Pro Gly Thr Lys Val Thr Val Leu
            100                 105                 110

<210>   362
<211>   110
<212>   PRT
<213>   Artificial Sequence

<220>
<223>   Description of Artificial Sequence: Synthetic peptide

<400>   362
Gln Ser Val Leu Thr Gln Pro Pro Ser Ala Ser Gly Thr Pro Gly Gln
1               5                   10                  15

Arg Val Thr Ile Ser Cys Ser Gly Ser Ser Ser Asn Ile Gly Ser Asn
            20                  25                  30

Ile Val Asn Trp Tyr Gln Gln Phe Pro Gly Thr Ala Pro Lys Leu Leu
        35                  40                  45
```

```
Ile Tyr Ser Asn Asn Gln Arg Pro Ser Gly Val Pro Asp Arg Val Ser
    50                  55                  60

Gly Ser Lys Ser Gly Thr Ser Ala Ser Leu Ala Ile Ser Gly Leu Gln
65                  70                  75                  80

Ser Glu Asp Glu Ala Asp Tyr Tyr Cys Ala Ala Trp Asp Asp Ser Leu
                85                  90                  95

Val Gly Pro Val Phe Gly Pro Gly Thr Lys Val Thr Val Leu
            100                 105                 110

<210>   363
<211>   110
<212>   PRT
<213>   Artificial Sequence

<220>
<223>   Description of Artificial Sequence: Synthetic peptide

<400>   363
Gln Ser Val Leu Thr Gln Pro Pro Ser Ala Ser Gly Thr Pro Gly Gln
1               5                   10                  15

Arg Val Thr Ile Ser Cys Ser Gly Ser Ser Ser Asn Ile Gly Ser Asn
            20                  25                  30

Ile Val Asn Trp Tyr Gln Gln Phe Pro Gly Thr Ala Pro Lys Leu Leu
        35                  40                  45

Ile Tyr Ser Asn Asn Gln Arg Pro Ser Gly Val Pro Asp Arg Val Ser
    50                  55                  60

Gly Ser Lys Ser Gly Thr Ser Ala Ser Leu Ala Ile Ser Gly Leu Gln
65                  70                  75                  80

Ser Glu Asp Glu Ala Asp Tyr Tyr Cys Ala Ala Trp Asp Asp Ser Leu
                85                  90                  95

His Gly Pro Val Phe Gly Pro Gly Thr Lys Val Thr Val Leu
            100                 105                 110

<210>   364
<211>   110
<212>   PRT
<213>   Artificial Sequence

<220>
<223>   Description of Artificial Sequence: Synthetic peptide

<400>   364
Gln Ser Val Leu Thr Gln Pro Pro Ser Ala Ser Gly Thr Pro Gly Gln
1               5                   10                  15
```

Arg Val Thr Ile Ser Cys Ser Gly Ser Ser Ser Asn Ile Gly Ser Asn
20 25 30

Ile Val Asn Trp Tyr Gln Gln Phe Pro Gly Thr Ala Pro Lys Leu Leu
35 40 45

Ile Tyr Ser Asn Asn Gln Arg Pro Ser Gly Val Pro Asp Arg Val Ser
50 55 60

Gly Ser Lys Ser Gly Thr Ser Ala Ser Leu Ala Ile Ser Gly Leu Gln
65 70 75 80

Ser Glu Asp Glu Ala Asp Tyr Tyr Cys Ala Ala Trp Asp Asp Ser Leu
85 90 95

Lys Gly Pro Val Phe Gly Pro Gly Thr Lys Val Thr Val Leu
100 105 110

<210> 365
<211> 110
<212> PRT
<213> Artificial Sequence

<220>
<223> Description of Artificial Sequence: Synthetic peptide

<400> 365
Gln Ser Val Leu Thr Gln Pro Pro Ser Ala Ser Gly Thr Pro Gly Gln
1 5 10 15

Arg Val Thr Ile Ser Cys Ser Gly Ser Ser Ser Asn Ile Gly Ser Asn
20 25 30

Ile Val Asn Trp Tyr Gln Gln Phe Pro Gly Thr Ala Pro Lys Leu Leu
35 40 45

Ile Tyr Ser Asn Asn Gln Arg Pro Ser Gly Val Pro Asp Arg Val Ser
50 55 60

Gly Ser Lys Ser Gly Thr Ser Ala Ser Leu Ala Ile Ser Gly Leu Gln
65 70 75 80

Ser Glu Asp Glu Ala Asp Tyr Tyr Cys Ala Ala Trp Asp Asp Ser Leu
85 90 95

Trp Gly Pro Val Phe Gly Pro Gly Thr Lys Val Thr Val Leu
100 105 110

<210> 366
<211> 110
<212> PRT
<213> Artificial Sequence

<220>
<223> Description of Artificial Sequence: Synthetic peptide

<400> 366
```
Gln Ser Val Leu Thr Gln Pro Pro Ser Ala Ser Gly Thr Pro Gly Gln
1               5                   10                  15

Arg Val Thr Ile Ser Cys Ser Gly Ser Ser Ser Asn Ile Gly Ser Asn
            20                  25                  30

Ile Val Asn Trp Tyr Gln Gln Phe Pro Gly Thr Ala Pro Lys Leu Leu
        35                  40                  45

Ile Tyr Ser Asn Asn Gln Arg Pro Ser Gly Val Pro Asp Arg Val Ser
    50                  55                  60

Gly Ser Lys Ser Gly Thr Ser Ala Ser Leu Ala Ile Ser Gly Leu Gln
65                  70                  75                  80

Ser Glu Asp Glu Ala Asp Tyr Tyr Cys Ala Ala Trp Asp Asp Ser Leu
                85                  90                  95

Ser Gly Pro Val Phe Gly Pro Gly Thr Lys Val Thr Val Leu
            100                 105                 110
```

<210> 367
<211> 117
<212> PRT
<213> Artificial Sequence

<220>
<223> Description of Artificial Sequence: Synthetic peptide

<400> 367
```
Gln Val Gln Leu Gln Glu Ser Gly Pro Gly Leu Val Lys Pro Ser Glu
1               5                   10                  15

Thr Leu Ser Leu Thr Cys Ser Val Ser Gly Ala Ser Ile Arg Asn Tyr
            20                  25                  30

Tyr Trp Ser Trp Ile Arg Gln Thr Ala Gly Lys Gly Leu Glu Trp Leu
        35                  40                  45

Gly His Ile Tyr Ser Thr Gly Asn Ile His Tyr Asn Pro Ser Leu Lys
    50                  55                  60

Ser Arg Val Thr Met Ser Val Asp Thr Ser Asn Asn Gln Phe Ser Leu
65                  70                  75                  80

Lys Leu Arg Ser Val Thr Ala Ala Asp Thr Ala Val Tyr Tyr Cys Ala
                85                  90                  95

Arg Asp Arg Gly Ala Ala Leu Phe Asp Tyr Trp Gly Gln Gly Thr Leu
            100                 105                 110
```

```
Val Thr Val Ser Ser
        115

<210>   368
<211>   117
<212>   PRT
<213>   Artificial Sequence

<220>
<223>   Description of Artificial Sequence: Synthetic peptide

<400>   368
Gln Val Gln Leu Gln Glu Ser Gly Pro Gly Leu Val Lys Pro Ser Glu
1               5                   10                  15

Thr Leu Ser Leu Thr Cys Ser Val Ser Gly Ala Ser Ile Arg Asn Tyr
            20                  25                  30

Tyr Trp Ser Trp Ile Arg Gln Thr Ala Gly Lys Gly Leu Glu Trp Leu
        35                  40                  45

Gly His Ile Tyr Ser Thr Gly Asn Ile His Tyr Asn Pro Ser Leu Lys
    50                  55                  60

Ser Arg Val Thr Met Ser Val Asp Thr Ser Asn Asn Gln Phe Ser Leu
65                  70                  75                  80

Lys Leu Arg Ser Val Thr Ala Ala Asp Thr Ala Val Tyr Tyr Cys Ala
                85                  90                  95

Arg Asp Pro Gly Ala Ala Leu Phe Asp Tyr Trp Gly Gln Gly Thr Leu
            100                 105                 110

Val Thr Val Ser Ser
        115

<210>   369
<211>   117
<212>   PRT
<213>   Artificial Sequence

<220>
<223>   Description of Artificial Sequence: Synthetic peptide

<400>   369
Gln Val Gln Leu Gln Glu Ser Gly Pro Gly Leu Val Lys Pro Ser Glu
1               5                   10                  15

Thr Leu Ser Leu Thr Cys Ser Val Ser Gly Ala Ser Ile Arg Asn Tyr
            20                  25                  30

Tyr Trp Ser Trp Ile Arg Gln Thr Ala Gly Lys Gly Leu Glu Trp Leu
        35                  40                  45
```

```
Gly His Ile Tyr Ser Thr Gly Asn Ile His Tyr Asn Pro Ser Leu Lys
    50                  55                  60

Ser Arg Val Thr Met Ser Val Asp Thr Ser Asn Asn Gln Phe Ser Leu
65                  70                  75                  80

Lys Leu Arg Ser Val Thr Ala Ala Asp Thr Ala Val Tyr Tyr Cys Ala
                85                  90                  95

Arg Asp Gly Gly Ala Ala Leu Phe Asp Tyr Trp Gly Gln Gly Thr Leu
            100                 105                 110

Val Thr Val Ser Ser
        115

<210>   370
<211>   117
<212>   PRT
<213>   Artificial Sequence

<220>
<223>   Description of Artificial Sequence: Synthetic peptide

<400>   370
Gln Val Gln Leu Gln Glu Ser Gly Pro Gly Leu Val Lys Pro Ser Glu
1               5                   10                  15

Thr Leu Ser Leu Thr Cys Ser Val Ser Gly Ala Ser Ile Arg Asn Tyr
            20                  25                  30

Tyr Trp Ser Trp Ile Arg Gln Thr Ala Gly Lys Gly Leu Glu Trp Leu
        35                  40                  45

Gly His Ile Tyr Ser Thr Gly Asn Ile His Tyr Asn Pro Ser Leu Lys
    50                  55                  60

Ser Arg Val Thr Met Ser Val Asp Thr Ser Asn Asn Gln Phe Ser Leu
65                  70                  75                  80

Lys Leu Arg Ser Val Thr Ala Ala Asp Thr Ala Val Tyr Tyr Cys Ala
                85                  90                  95

Arg Asp Val Gly Ala Ala Leu Phe Asp Tyr Trp Gly Gln Gly Thr Leu
            100                 105                 110

Val Thr Val Ser Ser
        115

<210>   371
<211>   117
<212>   PRT
<213>   Artificial Sequence
```

<220>
<223> Description of Artificial Sequence: Synthetic peptide

<400> 371
```
Gln Val Gln Leu Gln Glu Ser Gly Pro Gly Leu Val Lys Pro Ser Glu
1               5                   10                  15

Thr Leu Ser Leu Thr Cys Ser Val Ser Gly Ala Ser Ile Arg Asn Tyr
            20                  25                  30

Tyr Trp Ser Trp Ile Arg Gln Thr Ala Gly Lys Gly Leu Glu Trp Leu
        35                  40                  45

Gly His Ile Tyr Ser Thr Gly Asn Ile His Tyr Asn Pro Ser Leu Lys
    50                  55                  60

Ser Arg Val Thr Met Ser Val Asp Thr Ser Asn Asn Gln Phe Ser Leu
65                  70                  75                  80

Lys Leu Arg Ser Val Thr Ala Ala Asp Thr Ala Val Tyr Tyr Cys Ala
                85                  90                  95

Arg Asp Leu Gly Ala Ala Leu Phe Asp Tyr Trp Gly Gln Gly Thr Leu
            100                 105                 110

Val Thr Val Ser Ser
        115
```

<210> 372
<211> 117
<212> PRT
<213> Artificial Sequence

<220>
<223> Description of Artificial Sequence: Synthetic peptide

<400> 372
```
Gln Val Gln Leu Gln Glu Ser Gly Pro Gly Leu Val Lys Pro Ser Glu
1               5                   10                  15

Thr Leu Ser Leu Thr Cys Ser Val Ser Gly Ala Ser Ile Arg Asn Tyr
            20                  25                  30

Tyr Trp Ser Trp Ile Arg Gln Thr Ala Gly Lys Gly Leu Glu Trp Leu
        35                  40                  45

Gly His Ile Tyr Ser Thr Gly Asn Ile His Tyr Asn Pro Ser Leu Lys
    50                  55                  60

Ser Arg Val Thr Met Ser Val Asp Thr Ser Asn Asn Gln Phe Ser Leu
65                  70                  75                  80

Lys Leu Arg Ser Val Thr Ala Ala Asp Thr Ala Val Tyr Tyr Cys Ala
                85                  90                  95
```

```
Arg Asp Asp Gly Ala Ala Leu Phe Asp Tyr Trp Gly Gln Gly Thr Leu
            100                 105                 110

Val Thr Val Ser Ser
        115

<210>   373
<211>   117
<212>   PRT
<213>   Artificial Sequence

<220>
<223>   Description of Artificial Sequence: Synthetic peptide

<400>   373
Gln Val Gln Leu Gln Glu Ser Gly Pro Gly Leu Val Lys Pro Ser Glu
1               5                   10                  15

Thr Leu Ser Leu Thr Cys Ser Val Ser Gly Ala Ser Ile Arg Asn Tyr
            20                  25                  30

Tyr Trp Ser Trp Ile Arg Gln Thr Ala Gly Lys Gly Leu Glu Trp Leu
        35                  40                  45

Gly His Ile Tyr Ser Thr Gly Asn Ile His Tyr Asn Pro Ser Leu Lys
    50                  55                  60

Ser Arg Val Thr Met Ser Val Asp Thr Ser Asn Asn Gln Phe Ser Leu
65                  70                  75                  80

Lys Leu Arg Ser Val Thr Ala Ala Asp Thr Ala Val Tyr Tyr Cys Ala
                85                  90                  95

Arg Asp Glu Gly Ala Ala Leu Phe Asp Tyr Trp Gly Gln Gly Thr Leu
            100                 105                 110

Val Thr Val Ser Ser
        115

<210>   374
<211>   117
<212>   PRT
<213>   Artificial Sequence

<220>
<223>   Description of Artificial Sequence: Synthetic peptide

<400>   374
Gln Val Gln Leu Gln Glu Ser Gly Pro Gly Leu Val Lys Pro Ser Glu
1               5                   10                  15

Thr Leu Ser Leu Thr Cys Ser Val Ser Gly Ala Ser Ile Arg Asn Tyr
            20                  25                  30
```

Tyr Trp Ser Trp Ile Arg Gln Thr Ala Gly Lys Gly Leu Glu Trp Leu
35 40 45

Gly His Ile Tyr Ser Thr Gly Asn Ile His Tyr Asn Pro Ser Leu Lys
50 55 60

Ser Arg Val Thr Met Ser Val Asp Thr Ser Asn Asn Gln Phe Ser Leu
65 70 75 80

Lys Leu Arg Ser Val Thr Ala Ala Asp Thr Ala Val Tyr Tyr Cys Ala
85 90 95

Arg Asp Ala Gly Ala Ala Leu Phe Asp Tyr Trp Gly Gln Gly Thr Leu
100 105 110

Val Thr Val Ser Ser
115

<210> 375
<211> 117
<212> PRT
<213> Artificial Sequence

<220>
<223> Description of Artificial Sequence: Synthetic peptide

<400> 375
Gln Val Gln Leu Gln Glu Ser Gly Pro Gly Leu Val Lys Pro Ser Glu
1 5 10 15

Thr Leu Ser Leu Thr Cys Ser Val Ser Gly Ala Ser Ile Arg Asn Tyr
20 25 30

Tyr Trp Ser Trp Ile Arg Gln Thr Ala Gly Lys Gly Leu Glu Trp Leu
35 40 45

Gly His Ile Tyr Ser Thr Gly Asn Ile His Tyr Asn Pro Ser Leu Lys
50 55 60

Ser Arg Val Thr Met Ser Val Asp Thr Ser Asn Asn Gln Phe Ser Leu
65 70 75 80

Lys Leu Arg Ser Val Thr Ala Ala Asp Thr Ala Val Tyr Tyr Cys Ala
85 90 95

Arg Asp Gln Gly Ala Ala Leu Phe Asp Tyr Trp Gly Gln Gly Thr Leu
100 105 110

Val Thr Val Ser Ser
115

<210> 376
<211> 117

<212> PRT
<213> Artificial Sequence

<220>
<223> Description of Artificial Sequence: Synthetic peptide

<400> 376
```
Gln Val Gln Leu Gln Glu Ser Gly Pro Gly Leu Val Lys Pro Ser Glu
1               5                   10                  15

Thr Leu Ser Leu Thr Cys Ser Val Ser Gly Ala Ser Ile Arg Asn Tyr
            20                  25                  30

Tyr Trp Ser Trp Ile Arg Gln Thr Ala Gly Lys Gly Leu Glu Trp Leu
        35                  40                  45

Gly His Ile Tyr Ser Thr Gly Asn Ile His Tyr Asn Pro Ser Leu Lys
    50                  55                  60

Ser Arg Val Thr Met Ser Val Asp Thr Ser Asn Asn Gln Phe Ser Leu
65                  70                  75                  80

Lys Leu Arg Ser Val Thr Ala Ala Asp Thr Ala Val Tyr Tyr Cys Ala
                85                  90                  95

Arg Asp Thr Gly Ala Ala Leu Phe Asp Tyr Trp Gly Gln Gly Thr Leu
            100                 105                 110

Val Thr Val Ser Ser
        115
```

<210> 377
<211> 117
<212> PRT
<213> Artificial Sequence

<220>
<223> Description of Artificial Sequence: Synthetic peptide

<400> 377
```
Gln Val Gln Leu Gln Glu Ser Gly Pro Gly Leu Val Lys Pro Ser Glu
1               5                   10                  15

Thr Leu Ser Leu Thr Cys Ser Val Ser Gly Ala Ser Ile Arg Asn Tyr
            20                  25                  30

Tyr Trp Ser Trp Ile Arg Gln Thr Ala Gly Lys Gly Leu Glu Trp Leu
        35                  40                  45

Gly His Ile Tyr Ser Thr Gly Asn Ile His Tyr Asn Pro Ser Leu Lys
    50                  55                  60

Ser Arg Val Thr Met Ser Val Asp Thr Ser Asn Asn Gln Phe Ser Leu
65                  70                  75                  80
```

```
Lys Leu Arg Ser Val Thr Ala Ala Asp Thr Ala Val Tyr Tyr Cys Ala
                85                  90                  95

Arg Asp Asn Ser Ala Ala Leu Phe Asp Tyr Trp Gly Gln Gly Thr Leu
            100                 105                 110

Val Thr Val Ser Ser
        115

<210>   378
<211>   117
<212>   PRT
<213>   Artificial Sequence

<220>
<223>   Description of Artificial Sequence: Synthetic peptide

<400>   378
Gln Val Gln Leu Gln Glu Ser Gly Pro Gly Leu Val Lys Pro Ser Glu
1               5                   10                  15

Thr Leu Ser Leu Thr Cys Ser Val Ser Gly Ala Ser Ile Arg Asn Tyr
            20                  25                  30

Tyr Trp Ser Trp Ile Arg Gln Thr Ala Gly Lys Gly Leu Glu Trp Leu
        35                  40                  45

Gly His Ile Tyr Ser Thr Gly Asn Ile His Tyr Asn Pro Ser Leu Lys
    50                  55                  60

Ser Arg Val Thr Met Ser Val Asp Thr Ser Asn Asn Gln Phe Ser Leu
65                  70                  75                  80

Lys Leu Arg Ser Val Thr Ala Ala Asp Thr Ala Val Tyr Tyr Cys Ala
                85                  90                  95

Arg Asp Asn Ala Ala Ala Leu Phe Asp Tyr Trp Gly Gln Gly Thr Leu
            100                 105                 110

Val Thr Val Ser Ser
        115

<210>   379
<211>   117
<212>   PRT
<213>   Artificial Sequence

<220>
<223>   Description of Artificial Sequence: Synthetic peptide

<400>   379
Gln Val Gln Leu Gln Glu Ser Gly Pro Gly Leu Val Lys Pro Ser Glu
1               5                   10                  15
```

```
Thr Leu Ser Leu Thr Cys Ser Val Ser Gly Ala Ser Ile Arg Asn Tyr
            20                  25                  30

Tyr Trp Ser Trp Ile Arg Gln Thr Ala Gly Lys Gly Leu Glu Trp Leu
        35                  40                  45

Gly His Ile Tyr Ser Thr Gly Asn Ile His Tyr Asn Pro Ser Leu Lys
    50                  55                  60

Ser Arg Val Thr Met Ser Val Asp Thr Ser Asn Asn Gln Phe Ser Leu
65                  70                  75                  80

Lys Leu Arg Ser Val Thr Ala Ala Asp Thr Ala Val Tyr Tyr Cys Ala
                85                  90                  95

Arg Asp Asn Leu Ala Ala Leu Phe Asp Tyr Trp Gly Gln Gly Thr Leu
            100                 105                 110

Val Thr Val Ser Ser
        115

<210>   380
<211>   117
<212>   PRT
<213>   Artificial Sequence

<220>
<223>   Description of Artificial Sequence: Synthetic peptide

<400>   380
Gln Val Gln Leu Gln Glu Ser Gly Pro Gly Leu Val Lys Pro Ser Glu
1               5                   10                  15

Thr Leu Ser Leu Thr Cys Ser Val Ser Gly Ala Ser Ile Arg Asn Tyr
            20                  25                  30

Tyr Trp Ser Trp Ile Arg Gln Thr Ala Gly Lys Gly Leu Glu Trp Leu
        35                  40                  45

Gly His Ile Tyr Ser Thr Gly Asn Ile His Tyr Asn Pro Ser Leu Lys
    50                  55                  60

Ser Arg Val Thr Met Ser Val Asp Thr Ser Asn Asn Gln Phe Ser Leu
65                  70                  75                  80

Lys Leu Arg Ser Val Thr Ala Ala Asp Thr Ala Val Tyr Tyr Cys Ala
                85                  90                  95

Arg Asp Asn Pro Ala Ala Leu Phe Asp Tyr Trp Gly Gln Gly Thr Leu
            100                 105                 110

Val Thr Val Ser Ser
        115
```

<210> 381
<211> 117
<212> PRT
<213> Artificial Sequence

<220>
<223> Description of Artificial Sequence: Synthetic peptide

<400> 381
Gln Val Gln Leu Gln Glu Ser Gly Pro Gly Leu Val Lys Pro Ser Glu
1 5 10 15

Thr Leu Ser Leu Thr Cys Ser Val Ser Gly Ala Ser Ile Arg Asn Tyr
20 25 30

Tyr Trp Ser Trp Ile Arg Gln Thr Ala Gly Lys Gly Leu Glu Trp Leu
35 40 45

Gly His Ile Tyr Ser Thr Gly Asn Ile His Tyr Asn Pro Ser Leu Lys
50 55 60

Ser Arg Val Thr Met Ser Val Asp Thr Ser Asn Asn Gln Phe Ser Leu
65 70 75 80

Lys Leu Arg Ser Val Thr Ala Ala Asp Thr Ala Val Tyr Tyr Cys Ala
85 90 95

Arg Asp Asn Thr Ala Ala Leu Phe Asp Tyr Trp Gly Gln Gly Thr Leu
100 105 110

Val Thr Val Ser Ser
115

<210> 382
<211> 117
<212> PRT
<213> Artificial Sequence

<220>
<223> Description of Artificial Sequence: Synthetic peptide

<400> 382
Gln Val Gln Leu Gln Glu Ser Gly Pro Gly Leu Val Lys Pro Ser Glu
1 5 10 15

Thr Leu Ser Leu Thr Cys Ser Val Ser Gly Ala Ser Ile Arg Asn Tyr
20 25 30

Tyr Trp Ser Trp Ile Arg Gln Thr Ala Gly Lys Gly Leu Glu Trp Leu
35 40 45

Gly His Ile Tyr Ser Thr Gly Asn Ile His Tyr Asn Pro Ser Leu Lys
50 55 60

```
Ser Arg Val Thr Met Ser Val Asp Thr Ser Asn Asn Gln Phe Ser Leu
65                  70                  75                  80

Lys Leu Arg Ser Val Thr Ala Ala Asp Thr Ala Val Tyr Tyr Cys Ala
                85                  90                  95

Arg Asp Asn Gln Ala Ala Leu Phe Asp Tyr Trp Gly Gln Gly Thr Leu
            100                 105                 110

Val Thr Val Ser Ser
        115

<210>   383
<211>   110
<212>   PRT
<213>   Artificial Sequence

<220>
<223>   Description of Artificial Sequence: Synthetic peptide

<400>   383
Gln Ser Val Leu Thr Gln Pro Pro Ser Ala Ser Gly Thr Pro Gly Gln
1               5                   10                  15

Arg Val Thr Ile Ser Cys Ser Gly Ser Ser Ser Asn Ile Gly Ser Asn
            20                  25                  30

Pro Val Asn Trp Tyr Gln Gln Leu Pro Gly Thr Ala Pro Lys Leu Leu
        35                  40                  45

Ile Tyr Ser Asn Asn Gln Arg Pro Ser Gly Val Pro Asp Arg Phe Ser
    50                  55                  60

Gly Ser Lys Ser Gly Thr Ser Ala Ser Leu Ala Ile Ser Gly Leu Gln
65                  70                  75                  80

Ser Glu Asp Glu Ala Asp Tyr Phe Cys Ala Ala Trp Asp Asp Ser Leu
                85                  90                  95

Ser Gly Pro Val Phe Gly Gly Gly Thr Lys Leu Thr Val Leu
            100                 105                 110

<210>   384
<211>   110
<212>   PRT
<213>   Artificial Sequence

<220>
<223>   Description of Artificial Sequence: Synthetic peptide

<400>   384
Gln Ser Val Leu Thr Gln Pro Pro Ser Ala Ser Gly Thr Pro Gly Gln
1               5                   10                  15
```

```
Arg Val Thr Ile Ser Cys Ser Gly Ser Ser Ser Asn Ile Gly Ser Asn
            20                  25                  30

Pro Val Asn Trp Tyr Gln Gln Leu Pro Gly Thr Ala Pro Lys Leu Leu
        35                  40                  45

Ile Tyr Ser Asn Asn Gln Arg Pro Ser Gly Val Pro Asp Arg Phe Ser
    50                  55                  60

Gly Ser Lys Ser Gly Thr Ser Ala Ser Leu Ala Ile Ser Gly Leu Gln
65                  70                  75                  80

Ser Glu Asp Glu Ala Asp Tyr Phe Cys Ala Ala Trp Asp Asp Ser Leu
                85                  90                  95

Gln Gly Pro Val Phe Gly Gly Gly Thr Lys Leu Thr Val Leu
            100                 105                 110

<210>   385
<211>   110
<212>   PRT
<213>   Artificial Sequence

<220>
<223>   Description of Artificial Sequence: Synthetic peptide

<400>   385
Gln Ser Val Leu Thr Gln Pro Pro Ser Ala Ser Gly Thr Pro Gly Gln
1               5                   10                  15

Arg Val Thr Ile Ser Cys Ser Gly Ser Ser Ser Asn Ile Gly Ser Asn
            20                  25                  30

Pro Val Asn Trp Tyr Gln Gln Leu Pro Gly Thr Ala Pro Lys Leu Leu
        35                  40                  45

Ile Tyr Ser Asn Asn Gln Arg Pro Ser Gly Val Pro Asp Arg Phe Ser
    50                  55                  60

Gly Ser Lys Ser Gly Thr Ser Ala Ser Leu Ala Ile Ser Gly Leu Gln
65                  70                  75                  80

Ser Glu Asp Glu Ala Asp Tyr Phe Cys Ala Ala Trp Asp Asp Ser Leu
                85                  90                  95

Val Gly Pro Val Phe Gly Gly Gly Thr Lys Leu Thr Val Leu
            100                 105                 110

<210>   386
<211>   110
<212>   PRT
<213>   Artificial Sequence
```

<220>
<223> Description of Artificial Sequence: Synthetic peptide

<400> 386
Gln Ser Val Leu Thr Gln Pro Pro Ser Ala Ser Gly Thr Pro Gly Gln
1 5 10 15

Arg Val Thr Ile Ser Cys Ser Gly Ser Ser Ser Asn Ile Gly Ser Asn
20 25 30

Pro Val Asn Trp Tyr Gln Gln Leu Pro Gly Thr Ala Pro Lys Leu Leu
35 40 45

Ile Tyr Ser Asn Asn Gln Arg Pro Ser Gly Val Pro Asp Arg Phe Ser
50 55 60

Gly Ser Lys Ser Gly Thr Ser Ala Ser Leu Ala Ile Ser Gly Leu Gln
65 70 75 80

Ser Glu Asp Glu Ala Asp Tyr Phe Cys Ala Ala Trp Asp Asp Ser Leu
85 90 95

Gly Gly Pro Val Phe Gly Gly Gly Thr Lys Leu Thr Val Leu
100 105 110

<210> 387
<211> 110
<212> PRT
<213> Artificial Sequence

<220>
<223> Description of Artificial Sequence: Synthetic peptide

<400> 387
Gln Ser Val Leu Thr Gln Pro Pro Ser Ala Ser Gly Thr Pro Gly Gln
1 5 10 15

Arg Val Thr Ile Ser Cys Ser Gly Ser Ser Ser Asn Ile Gly Ser Asn
20 25 30

Pro Val Asn Trp Tyr Gln Gln Leu Pro Gly Thr Ala Pro Lys Leu Leu
35 40 45

Ile Tyr Ser Asn Asn Gln Arg Pro Ser Gly Val Pro Asp Arg Phe Ser
50 55 60

Gly Ser Lys Ser Gly Thr Ser Ala Ser Leu Ala Ile Ser Gly Leu Gln
65 70 75 80

Ser Glu Asp Glu Ala Asp Tyr Phe Cys Ala Ala Trp Asp Asp Ser Leu
85 90 95

Trp Gly Pro Val Phe Gly Gly Gly Thr Lys Leu Thr Val Leu
100 105 110

```
<210>  388
<211>  110
<212>  PRT
<213>  Artificial Sequence

<220>
<223>  Description of Artificial Sequence: Synthetic peptide

<400>  388
Gln Ser Val Leu Thr Gln Pro Pro Ser Ala Ser Gly Thr Pro Gly Gln
1               5                   10                  15

Arg Val Thr Ile Ser Cys Ser Gly Ser Ser Ser Asn Ile Gly Ser Asn
            20                  25                  30

Pro Val Asn Trp Tyr Gln Gln Leu Pro Gly Thr Ala Pro Lys Leu Leu
        35                  40                  45

Ile Tyr Ser Asn Asn Gln Arg Pro Ser Gly Val Pro Asp Arg Phe Ser
    50                  55                  60

Gly Ser Lys Ser Gly Thr Ser Ala Ser Leu Ala Ile Ser Gly Leu Gln
65                  70                  75                  80

Ser Glu Asp Glu Ala Asp Tyr Phe Cys Ala Ala Trp Asp Asp Ser Leu
                85                  90                  95

Thr Gly Pro Val Phe Gly Gly Gly Thr Lys Leu Thr Val Leu
            100                 105                 110

<210>  389
<211>  110
<212>  PRT
<213>  Artificial Sequence

<220>
<223>  Description of Artificial Sequence: Synthetic peptide

<400>  389
Gln Ser Val Leu Thr Gln Pro Pro Ser Ala Ser Gly Thr Pro Gly Gln
1               5                   10                  15

Arg Val Thr Ile Ser Cys Ser Gly Ser Ser Ser Asn Ile Gly Ser Asn
            20                  25                  30

Pro Val Asn Trp Tyr Gln Gln Leu Pro Gly Thr Ala Pro Lys Leu Leu
        35                  40                  45

Ile Tyr Ser Asn Asn Gln Arg Pro Ser Gly Val Pro Asp Arg Phe Ser
    50                  55                  60

Gly Ser Lys Ser Gly Thr Ser Ala Ser Leu Ala Ile Ser Gly Leu Gln
65                  70                  75                  80
```

```
Ser Glu Asp Glu Ala Asp Tyr Phe Cys Ala Ala Trp Asp Asp Ser Leu
                85                  90                  95

Ile Gly Pro Val Phe Gly Gly Gly Thr Lys Leu Thr Val Leu
            100                 105                 110

<210>   390
<211>   110
<212>   PRT
<213>   Artificial Sequence

<220>
<223>   Description of Artificial Sequence: Synthetic peptide

<400>   390
Gln Ser Val Leu Thr Gln Pro Pro Ser Ala Ser Gly Thr Pro Gly Gln
1               5                   10                  15

Arg Val Thr Ile Ser Cys Ser Gly Ser Ser Ser Asn Ile Gly Ser Asn
            20                  25                  30

Pro Val Asn Trp Tyr Gln Gln Leu Pro Gly Thr Ala Pro Lys Leu Leu
        35                  40                  45

Ile Tyr Ser Asn Asn Gln Arg Pro Ser Gly Val Pro Asp Arg Phe Ser
    50                  55                  60

Gly Ser Lys Ser Gly Thr Ser Ala Ser Leu Ala Ile Ser Gly Leu Gln
65                  70                  75                  80

Ser Glu Asp Glu Ala Asp Tyr Phe Cys Ala Ala Trp Asp Asp Ser Leu
                85                  90                  95

Glu Gly Pro Val Phe Gly Gly Gly Thr Lys Leu Thr Val Leu
            100                 105                 110

<210>   391
<211>   110
<212>   PRT
<213>   Artificial Sequence

<220>
<223>   Description of Artificial Sequence: Synthetic peptide

<400>   391
Gln Ser Val Leu Thr Gln Pro Pro Ser Ala Ser Gly Thr Pro Gly Gln
1               5                   10                  15

Arg Val Thr Ile Ser Cys Ser Gly Ser Ser Ser Asn Ile Gly Ser Asn
            20                  25                  30

Pro Val Asn Trp Tyr Gln Gln Leu Pro Gly Thr Ala Pro Lys Leu Leu
        35                  40                  45
```

```
Ile Tyr Ser Asn Asn Gln Arg Pro Ser Gly Val Pro Asp Arg Phe Ser
    50                  55                  60

Gly Ser Lys Ser Gly Thr Ser Ala Ser Leu Ala Ile Ser Gly Leu Gln
65                  70                  75                  80

Ser Glu Asp Glu Ala Asp Tyr Phe Cys Ala Ala Trp Asp Asp Ser Leu
                85                  90                  95

Ala Gly Pro Val Phe Gly Gly Gly Thr Lys Leu Thr Val Leu
            100                 105                 110

<210>   392
<211>   110
<212>   PRT
<213>   Artificial Sequence

<220>
<223>   Description of Artificial Sequence: Synthetic peptide

<400>   392
Gln Ser Val Leu Thr Gln Pro Pro Ser Ala Ser Gly Thr Pro Gly Gln
1               5                   10                  15

Arg Val Thr Ile Ser Cys Ser Gly Ser Ser Ser Asn Ile Gly Ser Asn
            20                  25                  30

Pro Val Asn Trp Tyr Gln Gln Leu Pro Gly Thr Ala Pro Lys Leu Leu
        35                  40                  45

Ile Tyr Ser Asn Asn Gln Arg Pro Ser Gly Val Pro Asp Arg Phe Ser
    50                  55                  60

Gly Ser Lys Ser Gly Thr Ser Ala Ser Leu Ala Ile Ser Gly Leu Gln
65                  70                  75                  80

Ser Glu Asp Glu Ala Asp Tyr Phe Cys Ala Ala Trp Asp Asp Ser Leu
                85                  90                  95

Arg Gly Pro Val Phe Gly Gly Gly Thr Lys Leu Thr Val Leu
            100                 105                 110

<210>   393
<211>   110
<212>   PRT
<213>   Artificial Sequence

<220>
<223>   Description of Artificial Sequence: Synthetic peptide

<400>   393
Gln Ser Val Leu Thr Gln Pro Pro Ser Ala Ser Gly Thr Pro Gly Gln
1               5                   10                  15
```

```
Arg Val Thr Ile Ser Cys Ser Gly Ser Ser Ser Asn Ile Gly Ser Asn
            20                  25                  30

Pro Val Asn Trp Tyr Gln Gln Leu Pro Gly Thr Ala Pro Lys Leu Leu
        35                  40                  45

Ile Tyr Ser Asn Asn Gln Arg Pro Ser Gly Val Pro Asp Arg Phe Ser
    50                  55                  60

Gly Ser Lys Ser Gly Thr Ser Ala Ser Leu Ala Ile Ser Gly Leu Gln
65                  70                  75                  80

Ser Glu Asp Glu Ala Asp Tyr Phe Cys Ala Ala Trp Asp Asp Ser Leu
                85                  90                  95

Leu Gly Pro Val Phe Gly Gly Gly Thr Lys Leu Thr Val Leu
            100                 105                 110

<210>   394
<211>   110
<212>   PRT
<213>   Artificial Sequence

<220>
<223>   Description of Artificial Sequence: Synthetic peptide

<400>   394
Gln Ser Val Leu Thr Gln Pro Pro Ser Ala Ser Gly Thr Pro Gly Gln
1               5                   10                  15

Arg Val Thr Ile Ser Cys Ser Gly Ser Ser Ser Asn Ile Gly Ser Asn
            20                  25                  30

Pro Val Asn Trp Tyr Gln Gln Leu Pro Gly Thr Ala Pro Lys Leu Leu
        35                  40                  45

Ile Tyr Ser Asn Asn Gln Arg Pro Ser Gly Val Pro Asp Arg Phe Ser
    50                  55                  60

Gly Ser Lys Ser Gly Thr Ser Ala Ser Leu Ala Ile Ser Gly Leu Gln
65                  70                  75                  80

Ser Glu Asp Glu Ala Asp Tyr Phe Cys Ala Ala Trp Asp Asp Ser Leu
                85                  90                  95

Asn Val Pro Val Phe Gly Gly Gly Thr Lys Leu Thr Val Leu
            100                 105                 110

<210>   395
<211>   110
<212>   PRT
<213>   Artificial Sequence
```

<220>
<223> Description of Artificial Sequence: Synthetic peptide

<400> 395
Gln Ser Val Leu Thr Gln Pro Pro Ser Ala Ser Gly Thr Pro Gly Gln
1 5 10 15

Arg Val Thr Ile Ser Cys Ser Gly Ser Ser Ser Asn Ile Gly Ser Asn
20 25 30

Pro Val Asn Trp Tyr Gln Gln Leu Pro Gly Thr Ala Pro Lys Leu Leu
35 40 45

Ile Tyr Ser Asn Asn Gln Arg Pro Ser Gly Val Pro Asp Arg Phe Ser
50 55 60

Gly Ser Lys Ser Gly Thr Ser Ala Ser Leu Ala Ile Ser Gly Leu Gln
65 70 75 80

Ser Glu Asp Glu Ala Asp Tyr Phe Cys Ala Ala Trp Asp Asp Ser Leu
85 90 95

Asn Ala Pro Val Phe Gly Gly Gly Thr Lys Leu Thr Val Leu
100 105 110

<210> 396
<211> 110
<212> PRT
<213> Artificial Sequence

<220>
<223> Description of Artificial Sequence: Synthetic peptide

<400> 396
Gln Ser Val Leu Thr Gln Pro Pro Ser Ala Ser Gly Thr Pro Gly Gln
1 5 10 15

Arg Val Thr Ile Ser Cys Ser Gly Ser Ser Ser Asn Ile Gly Ser Asn
20 25 30

Pro Val Asn Trp Tyr Gln Gln Leu Pro Gly Thr Ala Pro Lys Leu Leu
35 40 45

Ile Tyr Ser Asn Asn Gln Arg Pro Ser Gly Val Pro Asp Arg Phe Ser
50 55 60

Gly Ser Lys Ser Gly Thr Ser Ala Ser Leu Ala Ile Ser Gly Leu Gln
65 70 75 80

Ser Glu Asp Glu Ala Asp Tyr Phe Cys Ala Ala Trp Asp Asp Ser Leu
85 90 95

Asn Pro Pro Val Phe Gly Gly Gly Thr Lys Leu Thr Val Leu
100 105 110

<210> 397
<211> 110
<212> PRT
<213> Artificial Sequence

<220>
<223> Description of Artificial Sequence: Synthetic peptide

<400> 397
```
Gln Ser Val Leu Thr Gln Pro Pro Ser Ala Ser Gly Thr Pro Gly Gln
1               5                   10                  15

Arg Val Thr Ile Ser Cys Ser Gly Ser Ser Ser Asn Ile Gly Ser Asn
            20                  25                  30

Pro Val Asn Trp Tyr Gln Gln Leu Pro Gly Thr Ala Pro Lys Leu Leu
        35                  40                  45

Ile Tyr Ser Asn Asn Gln Arg Pro Ser Gly Val Pro Asp Arg Phe Ser
    50                  55                  60

Gly Ser Lys Ser Gly Thr Ser Ala Ser Leu Ala Ile Ser Gly Leu Gln
65                  70                  75                  80

Ser Glu Asp Glu Ala Asp Tyr Phe Cys Ala Ala Trp Asp Asp Ser Leu
                85                  90                  95

Asn Leu Pro Val Phe Gly Gly Gly Thr Lys Leu Thr Val Leu
            100                 105                 110
```

<210> 398
<211> 110
<212> PRT
<213> Artificial Sequence

<220>
<223> Description of Artificial Sequence: Synthetic peptide

<400> 398
```
Gln Ser Val Leu Thr Gln Pro Pro Ser Ala Ser Gly Thr Pro Gly Gln
1               5                   10                  15

Arg Val Thr Ile Ser Cys Ser Gly Ser Ser Ser Asn Ile Gly Ser Asn
            20                  25                  30

Pro Val Asn Trp Tyr Gln Gln Leu Pro Gly Thr Ala Pro Lys Leu Leu
        35                  40                  45

Ile Tyr Ser Asn Asn Gln Arg Pro Ser Gly Val Pro Asp Arg Phe Ser
    50                  55                  60

Gly Ser Lys Ser Gly Thr Ser Ala Ser Leu Ala Ile Ser Gly Leu Gln
65                  70                  75                  80
```

Ser Glu Asp Glu Ala Asp Tyr Phe Cys Ala Ala Trp Asp Asp Ser Leu
85 90 95

Asn Thr Pro Val Phe Gly Gly Gly Thr Lys Leu Thr Val Leu
100 105 110

<210> 399
<211> 10
<212> PRT
<213> Artificial Sequence

<220>
<223> Description of Artificial Sequence: Synthetic peptide

<400> 399
Gly Gly Ser Ile Ser Ser Tyr Tyr Trp Gly
1 5 10

<210> 400
<211> 10
<212> PRT
<213> Artificial Sequence

<220>
<223> Description of Artificial Sequence: Synthetic peptide

<400> 400
Gly Phe Thr Phe Ser Val Ser Ala Ile His
1 5 10

<210> 401
<211> 10
<212> PRT
<213> Artificial Sequence

<220>
<223> Description of Artificial Sequence: Synthetic peptide

<400> 401
Gly Phe Thr Phe Ser Ser Tyr Trp Met Thr
1 5 10

<210> 402
<211> 10
<212> PRT
<213> Artificial Sequence

<220>
<223> Description of Artificial Sequence: Synthetic peptide

<400> 402
Gly Ala Ser Ile Arg Asn Tyr Tyr Trp Ser
1 5 10

<210> 403
<211> 10
<212> PRT
<213> Artificial Sequence

<220>
<223> Description of Artificial Sequence: Synthetic peptide

<400> 403
Gly Gly Ser Ile Arg Asn Tyr Tyr Trp Ser
1 5 10

<210> 404
<211> 10
<212> PRT
<213> Artificial Sequence

<220>
<223> Description of Artificial Sequence: Synthetic peptide

<400> 404
Gly Ile Thr Phe Ser Asn Tyr Trp Met Ser
1 5 10

<210> 405
<211> 9
<212> PRT
<213> Artificial Sequence

<220>
<223> Description of Artificial Sequence: Synthetic peptide

<400> 405
Tyr Ile Tyr Tyr Ser Gly Ser Thr Asn
1 5

<210> 406
<211> 12
<212> PRT
<213> Artificial Sequence

<220>
<223> Description of Artificial Sequence: Synthetic peptide

<400> 406
Arg Ile Arg Ser Lys Gly Asn Ser Tyr Ala Thr Ala
1 5 10

<210> 407
<211> 10
<212> PRT
<213> Artificial Sequence

<220>
<223> Description of Artificial Sequence: Synthetic peptide

<400> 407
Asn Ile Lys Gln Asp Gly Ser Glu Arg Tyr
1 5 10

<210> 408
<211> 9
<212> PRT
<213> Artificial Sequence

<220>
<223> Description of Artificial Sequence: Synthetic peptide

<400> 408
His Ile Tyr Ser Thr Gly Asn Ile His
1 5

<210> 409
<211> 9
<212> PRT
<213> Artificial Sequence

<220>
<223> Description of Artificial Sequence: Synthetic peptide

<400> 409
His Ile Phe Ser Thr Gly His Ile Asn
1 5

<210> 410
<211> 10
<212> PRT
<213> Artificial Sequence

<220>
<223> Description of Artificial Sequence: Synthetic peptide

<400> 410
Ser Ile Lys Arg Asp Gly Ser Asp Lys Tyr
1 5 10

<210> 411
<211> 9
<212> PRT
<213> Artificial Sequence

<220>
<223> Description of Artificial Sequence: Synthetic peptide

<400> 411
Met Trp Glu Ile Leu Gly Phe Asp Pro
1 5

<210> 412
<211> 12
<212> PRT
<213> Artificial Sequence

<220>
<223> Description of Artificial Sequence: Synthetic peptide

<400> 412
His Asn Asp Lys Trp Asn Tyr Tyr Gly Leu Asp Val
1               5                   10

<210> 413
<211> 14
<212> PRT
<213> Artificial Sequence

<220>
<223> Description of Artificial Sequence: Synthetic peptide

<400> 413
Glu Val Gly Tyr Asn Trp Asn Gln Gly Gly Tyr Phe Asp Tyr
1               5                   10

<210> 414
<211> 9
<212> PRT
<213> Artificial Sequence

<220>
<223> Description of Artificial Sequence: Synthetic peptide

<400> 414
Asp Asn Gly Ala Ala Leu Phe Asp Tyr
1               5

<210> 415
<211> 9
<212> PRT
<213> Artificial Sequence

<220>
<223> Description of Artificial Sequence: Synthetic peptide

<400> 415
Asp Asn Gly Ala Ala Leu Phe Asp Phe
1               5

<210> 416
<211> 5
<212> PRT
<213> Artificial Sequence

<220>
<223> Description of Artificial Sequence: Synthetic peptide

<400> 416
Gly Glu Phe Asp Tyr
1 5

<210> 417
<211> 13
<212> PRT
<213> Artificial Sequence

<220>
<223> Description of Artificial Sequence: Synthetic peptide

<400> 417
Ser Gly Ser Ser Ser Asn Ile Gly Ser Asn Pro Val Asn
1 5 10

<210> 418
<211> 16
<212> PRT
<213> Artificial Sequence

<220>
<223> Description of Artificial Sequence: Synthetic peptide

<400> 418
Lys Ser Ser Gln Ser Leu Leu Phe Ser Asn Gly Tyr Lys Phe Leu Asp
1 5 10 15

<210> 419
<211> 16
<212> PRT
<213> Artificial Sequence

<220>
<223> Description of Artificial Sequence: Synthetic peptide

<400> 419
Arg Ser Ser Gln Ser Leu Leu His Ser Asp Gly Tyr Asn Tyr Leu Asp
1 5 10 15

<210> 420
<211> 13
<212> PRT
<213> Artificial Sequence

<220>
<223> Description of Artificial Sequence: Synthetic peptide

<400> 420
Ser Gly Ser Ser Ser Asn Ile Gly Ser Asn Ile Val Asn
1 5 10

<210> 421
<211> 13
<212> PRT
<213> Artificial Sequence

<220>
<223> Description of Artificial Sequence: Synthetic peptide

<400> 421
Ser Gly Ser Ser Ser Asn Ile Gly Ser Asn Ile Val Asn
1               5                   10

<210> 422
<211> 16
<212> PRT
<213> Artificial Sequence

<220>
<223> Description of Artificial Sequence: Synthetic peptide

<400> 422
Arg Ser Ser Gln Ser Leu Val Tyr Ser Asp Gly Asn Thr Tyr Leu Asn
1               5                   10                  15

<210> 423
<211> 7
<212> PRT
<213> Artificial Sequence

<220>
<223> Description of Artificial Sequence: Synthetic peptide

<400> 423
Ser Asn Asn Gln Arg Pro Ser
1               5

<210> 424
<211> 7
<212> PRT
<213> Artificial Sequence

<220>
<223> Description of Artificial Sequence: Synthetic peptide

<400> 424
Leu Gly Ser Tyr Arg Ala Ser
1               5

<210> 425
<211> 7
<212> PRT
<213> Artificial Sequence

<220>
<223> Description of Artificial Sequence: Synthetic peptide

<400> 425
Leu Gly Ser Tyr Arg Ala Ser
1 5

<210> 426
<211> 7
<212> PRT
<213> Artificial Sequence

<220>
<223> Description of Artificial Sequence: Synthetic peptide

<400> 426
Ser Asn Asn Gln Arg Pro Ser
1 5

<210> 427
<211> 7
<212> PRT
<213> Artificial Sequence

<220>
<223> Description of Artificial Sequence: Synthetic peptide

<400> 427
Ser Asp Asn Gln Arg Pro Ser
1 5

<210> 428
<211> 7
<212> PRT
<213> Artificial Sequence

<220>
<223> Description of Artificial Sequence: Synthetic peptide

<400> 428
Lys Val Ser Thr Arg Asp Ser
1 5

<210> 429
<211> 11
<212> PRT
<213> Artificial Sequence

<220>
<223> Description of Artificial Sequence: Synthetic peptide

<400> 429
Ala Ala Trp Asp Asp Ser Leu Asn Gly Pro Val
1 5 10

<210> 430
<211> 9
<212> PRT
<213> Artificial Sequence

<220>
<223> Description of Artificial Sequence: Synthetic peptide

<400> 430
Met Gln Ala Leu Gln Thr Pro Pro Thr
1               5

<210> 431
<211> 9
<212> PRT
<213> Artificial Sequence

<220>
<223> Description of Artificial Sequence: Synthetic peptide

<400> 431
Met Gln Val Leu Gln Thr Pro Trp Thr
1               5

<210> 432
<211> 11
<212> PRT
<213> Artificial Sequence

<220>
<223> Description of Artificial Sequence: Synthetic peptide

<400> 432
Ala Ala Trp Asp Asp Ser Leu Asn Gly Pro Val
1               5                   10

<210> 433
<211> 11
<212> PRT
<213> Artificial Sequence

<220>
<223> Description of Artificial Sequence: Synthetic peptide

<400> 433
Ala Ala Trp Asp Asp Ser Leu Asn Gly Pro Val
1               5                   10

<210> 434
<211> 9
<212> PRT
<213> Artificial Sequence

<220>
<223>   Description of Artificial Sequence: Synthetic peptide

<400>   434
Leu Gln Gly Thr His Trp Pro Trp Thr
1               5

<210>   435
<211>   7
<212>   PRT
<213>   Artificial Sequence

<220>
<223>   Description of Artificial Sequence: Synthetic peptide

<400>   435
Gly Gly Ser Ile Ser Ser Tyr
1               5

<210>   436
<211>   7
<212>   PRT
<213>   Artificial Sequence

<220>
<223>   Description of Artificial Sequence: Synthetic peptide

<400>   436
Gly Phe Thr Phe Ser Val Ser
1               5

<210>   437
<211>   7
<212>   PRT
<213>   Artificial Sequence

<220>
<223>   Description of Artificial Sequence: Synthetic peptide

<400>   437
Gly Phe Thr Phe Ser Ser Tyr
1               5

<210>   438
<211>   7
<212>   PRT
<213>   Artificial Sequence

<220>
<223>   Description of Artificial Sequence: Synthetic peptide

<400>   438
Gly Ala Ser Ile Arg Asn Tyr
1               5

<210> 439
<211> 7
<212> PRT
<213> Artificial Sequence

<220>
<223> Description of Artificial Sequence: Synthetic peptide

<400> 439
Gly Gly Ser Ile Arg Asn Tyr
1 5

<210> 440
<211> 7
<212> PRT
<213> Artificial Sequence

<220>
<223> Description of Artificial Sequence: Synthetic peptide

<400> 440
Gly Ile Thr Phe Ser Asn Tyr
1 5

<210> 441
<211> 5
<212> PRT
<213> Artificial Sequence

<220>
<223> Description of Artificial Sequence: Synthetic peptide

<400> 441
Tyr Tyr Ser Gly Ser
1 5

<210> 442
<211> 8
<212> PRT
<213> Artificial Sequence

<220>
<223> Description of Artificial Sequence: Synthetic peptide

<400> 442
Arg Ser Lys Gly Asn Ser Tyr Ala
1 5

<210> 443
<211> 6
<212> PRT
<213> Artificial Sequence

<220>
<223> Description of Artificial Sequence: Synthetic peptide

<400> 443
Lys Gln Asp Gly Ser Glu
1 5

<210> 444
<211> 5
<212> PRT
<213> Artificial Sequence

<220>
<223> Description of Artificial Sequence: Synthetic peptide

<400> 444
Tyr Ser Thr Gly Asn
1 5

<210> 445
<211> 5
<212> PRT
<213> Artificial Sequence

<220>
<223> Description of Artificial Sequence: Synthetic peptide

<400> 445
Phe Ser Thr Gly His
1 5

<210> 446
<211> 6
<212> PRT
<213> Artificial Sequence

<220>
<223> Description of Artificial Sequence: Synthetic peptide

<400> 446
Lys Arg Asp Gly Ser Asp
1 5

<210> 447
<211> 8
<212> PRT
<213> Artificial Sequence

<220>
<223> Description of Artificial Sequence: Synthetic peptide

<400> 447
Met Trp Glu Ile Leu Gly Phe Asp
1 5

<210> 448
<211> 11
<212> PRT
<213> Artificial Sequence

<220>
<223> Description of Artificial Sequence: Synthetic peptide

<400> 448
His Asn Asp Lys Trp Asn Tyr Tyr Gly Leu Asp
1 5 10

<210> 449
<211> 13
<212> PRT
<213> Artificial Sequence

<220>
<223> Description of Artificial Sequence: Synthetic peptide

<400> 449
Glu Val Gly Tyr Asn Trp Asn Gln Gly Gly Tyr Phe Asp
1 5 10

<210> 450
<211> 8
<212> PRT
<213> Artificial Sequence

<220>
<223> Description of Artificial Sequence: Synthetic peptide

<400> 450
Asp Asn Gly Ala Ala Leu Phe Asp
1 5

<210> 451
<211> 8
<212> PRT
<213> Artificial Sequence

<220>
<223> Description of Artificial Sequence: Synthetic peptide

<400> 451
Asp Asn Gly Ala Ala Leu Phe Asp
1 5

<210> 452
<211> 4
<212> PRT
<213> Artificial Sequence

<220>
<223> Description of Artificial Sequence: Synthetic peptide

<400> 452
Gly Glu Phe Asp
1

<210> 453
<211> 9
<212> PRT
<213> Artificial Sequence

<220>
<223> Description of Artificial Sequence: Synthetic peptide

<400> 453
Ser Ser Ser Asn Ile Gly Ser Asn Pro
1 5

<210> 454
<211> 12
<212> PRT
<213> Artificial Sequence

<220>
<223> Description of Artificial Sequence: Synthetic peptide

<400> 454
Ser Gln Ser Leu Leu Phe Ser Asn Gly Tyr Lys Phe
1 5 10

<210> 455
<211> 12
<212> PRT
<213> Artificial Sequence

<220>
<223> Description of Artificial Sequence: Synthetic peptide

<400> 455
Ser Gln Ser Leu Leu His Ser Asp Gly Tyr Asn Tyr
1 5 10

<210> 456
<211> 9
<212> PRT
<213> Artificial Sequence

<220>
<223> Description of Artificial Sequence: Synthetic peptide

<400> 456
Ser Ser Ser Asn Ile Gly Ser Asn Ile
1 5

<210> 457
<211> 9
<212> PRT
<213> Artificial Sequence

<220>
<223> Description of Artificial Sequence: Synthetic peptide

<400> 457
Ser Ser Ser Asn Ile Gly Ser Asn Ile
1 5

<210> 458
<211> 12
<212> PRT
<213> Artificial Sequence

<220>
<223> Description of Artificial Sequence: Synthetic peptide

<400> 458
Ser Gln Ser Leu Val Tyr Ser Asp Gly Asn Thr Tyr
1 5 10

<210> 459
<211> 3
<212> PRT
<213> Artificial Sequence

<220>
<223> Description of Artificial Sequence: Synthetic peptide

<400> 459
Ser Asn Asn
1

<210> 460
<211> 3
<212> PRT
<213> Artificial Sequence

<220>
<223> Description of Artificial Sequence: Synthetic peptide

<400> 460
Leu Gly Ser
1

<210> 461
<211> 3
<212> PRT
<213> Artificial Sequence

<220>
<223> Description of Artificial Sequence: Synthetic peptide

<400> 461
Leu Gly Ser
1

<210> 462
<211> 3
<212> PRT
<213> Artificial Sequence

<220>
<223> Description of Artificial Sequence: Synthetic peptide

<400> 462
Ser Asn Asn
1

<210> 463
<211> 3
<212> PRT
<213> Artificial Sequence

<220>
<223> Description of Artificial Sequence: Synthetic peptide

<400> 463
Ser Asp Asn
1

<210> 464
<211> 3
<212> PRT
<213> Artificial Sequence

<220>
<223> Description of Artificial Sequence: Synthetic peptide

<400> 464
Lys Val Ser
1

<210> 465
<211> 8
<212> PRT
<213> Artificial Sequence

<220>
<223> Description of Artificial Sequence: Synthetic peptide

<400> 465
Trp Asp Asp Ser Leu Asn Gly Pro
1               5

<210> 466
<211> 6
<212> PRT
<213> Artificial Sequence

<220>
<223> Description of Artificial Sequence: Synthetic peptide

<400> 466
Ala Leu Gln Thr Pro Pro
1 5

<210> 467
<211> 6
<212> PRT
<213> Artificial Sequence

<220>
<223> Description of Artificial Sequence: Synthetic peptide

<400> 467
Val Leu Gln Thr Pro Trp
1 5

<210> 468
<211> 8
<212> PRT
<213> Artificial Sequence

<220>
<223> Description of Artificial Sequence: Synthetic peptide

<400> 468
Trp Asp Asp Ser Leu Asn Gly Pro
1 5

<210> 469
<211> 8
<212> PRT
<213> Artificial Sequence

<220>
<223> Description of Artificial Sequence: Synthetic peptide

<400> 469
Trp Asp Asp Ser Leu Asn Gly Pro
1 5

<210> 470
<211> 6
<212> PRT
<213> Artificial Sequence

<220>
<223> Description of Artificial Sequence: Synthetic peptide

<400> 470
Gly Thr His Trp Pro Trp
1 5

<210> 471
<211> 8
<212> PRT
<213> Artificial Sequence

<220>
<223> Description of Artificial Sequence: Synthetic peptide

<400> 471
Gly Gly Ser Ile Ser Ser Tyr Tyr
1 5

<210> 472
<211> 8
<212> PRT
<213> Artificial Sequence

<220>
<223> Description of Artificial Sequence: Synthetic peptide

<400> 472
Gly Phe Thr Phe Ser Val Ser Ala
1 5

<210> 473
<211> 8
<212> PRT
<213> Artificial Sequence

<220>
<223> Description of Artificial Sequence: Synthetic peptide

<400> 473
Gly Phe Thr Phe Ser Ser Tyr Trp
1 5

<210> 474
<211> 8
<212> PRT
<213> Artificial Sequence

<220>
<223> Description of Artificial Sequence: Synthetic peptide

<400> 474
Gly Ala Ser Ile Arg Asn Tyr Tyr
1 5

<210> 475
<211> 8
<212> PRT
<213> Artificial Sequence

<220>
<223> Description of Artificial Sequence: Synthetic peptide

<400> 475
Gly Gly Ser Ile Arg Asn Tyr Tyr
1 5

<210> 476
<211> 8
<212> PRT
<213> Artificial Sequence

<220>
<223> Description of Artificial Sequence: Synthetic peptide

<400> 476
Gly Ile Thr Phe Ser Asn Tyr Trp
1 5

<210> 477
<211> 7
<212> PRT
<213> Artificial Sequence

<220>
<223> Description of Artificial Sequence: Synthetic peptide

<400> 477
Ile Tyr Tyr Ser Gly Ser Thr
1 5

<210> 478
<211> 10
<212> PRT
<213> Artificial Sequence

<220>
<223> Description of Artificial Sequence: Synthetic peptide

<400> 478
Ile Arg Ser Lys Gly Asn Ser Tyr Ala Thr
1 5 10

<210> 479
<211> 8
<212> PRT
<213> Artificial Sequence

<220>
<223> Description of Artificial Sequence: Synthetic peptide

<400> 479
Ile Lys Gln Asp Gly Ser Glu Arg
1 5

<210> 480
<211> 7
<212> PRT
<213> Artificial Sequence

<220>
<223> Description of Artificial Sequence: Synthetic peptide

<400> 480
Ile Tyr Ser Thr Gly Asn Ile
1 5

<210> 481
<211> 7
<212> PRT
<213> Artificial Sequence

<220>
<223> Description of Artificial Sequence: Synthetic peptide

<400> 481
Ile Phe Ser Thr Gly His Ile
1 5

<210> 482
<211> 8
<212> PRT
<213> Artificial Sequence

<220>
<223> Description of Artificial Sequence: Synthetic peptide

<400> 482
Ile Lys Arg Asp Gly Ser Asp Lys
1 5

<210> 483
<211> 11
<212> PRT
<213> Artificial Sequence

<220>
<223> Description of Artificial Sequence: Synthetic peptide

<400> 483
Ala Arg Met Trp Glu Ile Leu Gly Phe Asp Pro
1 5 10

<210> 484
<211> 14
<212> PRT
<213> Artificial Sequence

<220>
<223> Description of Artificial Sequence: Synthetic peptide

<400> 484
Thr Arg His Asn Asp Lys Trp Asn Tyr Tyr Gly Leu Asp Val
1 5 10

<210> 485
<211> 16
<212> PRT
<213> Artificial Sequence

<220>
<223> Description of Artificial Sequence: Synthetic peptide

<400> 485
Ala Arg Glu Val Gly Tyr Asn Trp Asn Gln Gly Gly Tyr Phe Asp Tyr
1 5 10 15

<210> 486
<211> 11
<212> PRT
<213> Artificial Sequence

<220>
<223> Description of Artificial Sequence: Synthetic peptide

<400> 486
Ala Arg Asp Asn Gly Ala Ala Leu Phe Asp Tyr
1 5 10

<210> 487
<211> 11
<212> PRT
<213> Artificial Sequence

<220>
<223> Description of Artificial Sequence: Synthetic peptide

<400> 487
Ala Arg Asp Asn Gly Ala Ala Leu Phe Asp Phe
1 5 10

<210> 488
<211> 7
<212> PRT
<213> Artificial Sequence

<220>
<223> Description of Artificial Sequence: Synthetic peptide

<400> 488
Ala Lys Gly Glu Phe Asp Tyr
1 5

<210> 489
<211> 8
<212> PRT
<213> Artificial Sequence

<220>
<223> Description of Artificial Sequence: Synthetic peptide

<400> 489
Ser Ser Asn Ile Gly Ser Asn Pro
1 5

<210> 490
<211> 11
<212> PRT
<213> Artificial Sequence

<220>
<223> Description of Artificial Sequence: Synthetic peptide

<400> 490
Gln Ser Leu Leu Phe Ser Asn Gly Tyr Lys Phe
1 5 10

<210> 491
<211> 11
<212> PRT
<213> Artificial Sequence

<220>
<223> Description of Artificial Sequence: Synthetic peptide

<400> 491
Gln Ser Leu Leu His Ser Asp Gly Tyr Asn Tyr
1 5 10

<210> 492
<211> 8
<212> PRT
<213> Artificial Sequence

<220>
<223> Description of Artificial Sequence: Synthetic peptide

<400> 492
Ser Ser Asn Ile Gly Ser Asn Ile
1 5

<210> 493
<211> 8
<212> PRT
<213> Artificial Sequence

<220>
<223> Description of Artificial Sequence: Synthetic peptide

<400> 493
Ser Ser Asn Ile Gly Ser Asn Ile
1 5

<210> 494
<211> 11
<212> PRT
<213> Artificial Sequence

<220>
<223> Description of Artificial Sequence: Synthetic peptide

<400> 494
Gln Ser Leu Val Tyr Ser Asp Gly Asn Thr Tyr
1 5 10

<210> 495
<211> 3
<212> PRT
<213> Artificial Sequence

<220>
<223> Description of Artificial Sequence: Synthetic peptide

<400> 495
Ser Asn Asn
1

<210> 496
<211> 3
<212> PRT
<213> Artificial Sequence

<220>
<223> Description of Artificial Sequence: Synthetic peptide

<400> 496
Leu Gly Ser
1

<210> 497
<211> 3
<212> PRT
<213> Artificial Sequence

<220>
<223> Description of Artificial Sequence: Synthetic peptide

<400> 497
Leu Gly Ser
1

<210> 498
<211> 3
<212> PRT
<213> Artificial Sequence

<220>
<223> Description of Artificial Sequence: Synthetic peptide

<400> 498
Ser Asn Asn
1

<210> 499
<211> 3
<212> PRT
<213> Artificial Sequence

<220>
<223> Description of Artificial Sequence: Synthetic peptide

<400> 499
Ser Asp Asn
1

<210> 500
<211> 3
<212> PRT
<213> Artificial Sequence

<220>
<223> Description of Artificial Sequence: Synthetic peptide

<400> 500
Lys Val Ser
1

<210> 501
<211> 11
<212> PRT
<213> Artificial Sequence

<220>
<223> Description of Artificial Sequence: Synthetic peptide

<400> 501
Ala Ala Trp Asp Asp Ser Leu Asn Gly Pro Val
1               5                   10

<210> 502
<211> 9
<212> PRT
<213> Artificial Sequence

<220>
<223> Description of Artificial Sequence: Synthetic peptide

<400> 502
Met Gln Ala Leu Gln Thr Pro Pro Thr
1 5

<210> 503
<211> 9
<212> PRT
<213> Artificial Sequence

<220>
<223> Description of Artificial Sequence: Synthetic peptide

<400> 503
Met Gln Val Leu Gln Thr Pro Trp Thr
1 5

<210> 504
<211> 11
<212> PRT
<213> Artificial Sequence

<220>
<223> Description of Artificial Sequence: Synthetic peptide

<400> 504
Ala Ala Trp Asp Asp Ser Leu Asn Gly Pro Val
1 5 10

<210> 505
<211> 11
<212> PRT
<213> Artificial Sequence

<220>
<223> Description of Artificial Sequence: Synthetic peptide

<400> 505
Ala Ala Trp Asp Asp Ser Leu Asn Gly Pro Val
1 5 10

<210> 506
<211> 9
<212> PRT
<213> Artificial Sequence

<220>
<223> Description of Artificial Sequence: Synthetic peptide

<400> 506
Leu Gln Gly Thr His Trp Pro Trp Thr
1 5

<210> 507
<211> 6
<212> PRT
<213> Artificial Sequence

<220>
<223> Description of Artificial Sequence: Synthetic peptide

<400> 507
Ser Ser Tyr Tyr Trp Gly
1 5

<210> 508
<211> 6
<212> PRT
<213> Artificial Sequence

<220>
<223> Description of Artificial Sequence: Synthetic peptide

<400> 508
Ser Val Ser Ala Ile His
1 5

<210> 509
<211> 6
<212> PRT
<213> Artificial Sequence

<220>
<223> Description of Artificial Sequence: Synthetic peptide

<400> 509
Ser Ser Tyr Trp Met Thr
1 5

<210> 510
<211> 6
<212> PRT
<213> Artificial Sequence

<220>
<223> Description of Artificial Sequence: Synthetic peptide

<400> 510
Arg Asn Tyr Tyr Trp Ser
1 5

<210> 511
<211> 6
<212> PRT
<213> Artificial Sequence

<220>
<223> Description of Artificial Sequence: Synthetic peptide

<400> 511
Arg Asn Tyr Tyr Trp Ser
1 5

<210> 512
<211> 6
<212> PRT
<213> Artificial Sequence

<220>
<223> Description of Artificial Sequence: Synthetic peptide

<400> 512
Ser Asn Tyr Trp Met Ser
1 5

<210> 513
<211> 12
<212> PRT
<213> Artificial Sequence

<220>
<223> Description of Artificial Sequence: Synthetic peptide

<400> 513
Trp Ile Gly Tyr Ile Tyr Tyr Ser Gly Ser Thr Asn
1 5 10

<210> 514
<211> 15
<212> PRT
<213> Artificial Sequence

<220>
<223> Description of Artificial Sequence: Synthetic peptide

<400> 514
Trp Ile Gly Arg Ile Arg Ser Lys Gly Asn Ser Tyr Ala Thr Ala
1 5 10 15

<210> 515
<211> 13
<212> PRT
<213> Artificial Sequence

<220>
<223> Description of Artificial Sequence: Synthetic peptide

<400> 515
Trp Val Ala Asn Ile Lys Gln Asp Gly Ser Glu Arg Tyr
1 5 10

<210> 516
<211> 12
<212> PRT
<213> Artificial Sequence

<220>
<223> Description of Artificial Sequence: Synthetic peptide

<400> 516
Trp Leu Gly His Ile Tyr Ser Thr Gly Asn Ile His
1 5 10

<210> 517
<211> 12
<212> PRT
<213> Artificial Sequence

<220>
<223> Description of Artificial Sequence: Synthetic peptide

<400> 517
Trp Phe Gly His Ile Phe Ser Thr Gly His Ile Asn
1 5 10

<210> 518
<211> 13
<212> PRT
<213> Artificial Sequence

<220>
<223> Description of Artificial Sequence: Synthetic peptide

<400> 518
Trp Val Ala Ser Ile Lys Arg Asp Gly Ser Asp Lys Tyr
1 5 10

<210> 519
<211> 10
<212> PRT
<213> Artificial Sequence

<220>
<223> Description of Artificial Sequence: Synthetic peptide

<400> 519
Ala Arg Met Trp Glu Ile Leu Gly Phe Asp
1 5 10

<210> 520
<211> 13
<212> PRT
<213> Artificial Sequence

<220>
<223> Description of Artificial Sequence: Synthetic peptide

<400> 520
Thr Arg His Asn Asp Lys Trp Asn Tyr Tyr Gly Leu Asp
1 5 10

<210> 521
<211> 15
<212> PRT
<213> Artificial Sequence

<220>
<223> Description of Artificial Sequence: Synthetic peptide

<400> 521
Ala Arg Glu Val Gly Tyr Asn Trp Asn Gln Gly Gly Tyr Phe Asp
1 5 10 15

<210> 522
<211> 10
<212> PRT
<213> Artificial Sequence

<220>
<223> Description of Artificial Sequence: Synthetic peptide

<400> 522
Ala Arg Asp Asn Gly Ala Ala Leu Phe Asp
1 5 10

<210> 523
<211> 10
<212> PRT
<213> Artificial Sequence

<220>
<223> Description of Artificial Sequence: Synthetic peptide

<400> 523
Ala Arg Asp Asn Gly Ala Ala Leu Phe Asp
1 5 10

<210> 524
<211> 6
<212> PRT
<213> Artificial Sequence

<220>
<223> Description of Artificial Sequence: Synthetic peptide

<400> 524
Ala Lys Gly Glu Phe Asp
1 5

<210> 525
<211> 9
<212> PRT
<213> Artificial Sequence

<220>
<223> Description of Artificial Sequence: Synthetic peptide

<400> 525
Ile Gly Ser Asn Pro Val Asn Trp Tyr
1 5

<210> 526
<211> 12
<212> PRT
<213> Artificial Sequence

<220>
<223> Description of Artificial Sequence: Synthetic peptide

<400> 526
Leu Phe Ser Asn Gly Tyr Lys Phe Leu Asp Trp Tyr
1 5 10

<210> 527
<211> 12
<212> PRT
<213> Artificial Sequence

<220>
<223> Description of Artificial Sequence: Synthetic peptide

<400> 527
Leu His Ser Asp Gly Tyr Asn Tyr Leu Asp Trp Tyr
1 5 10

<210> 528
<211> 9
<212> PRT
<213> Artificial Sequence

<220>
<223> Description of Artificial Sequence: Synthetic peptide

<400> 528
Ile Gly Ser Asn Ile Val Asn Trp Tyr
1 5

<210> 529
<211> 9
<212> PRT
<213> Artificial Sequence

<220>
<223> Description of Artificial Sequence: Synthetic peptide

<400> 529
Ile Gly Ser Asn Ile Val Asn Trp Tyr
1               5

<210> 530
<211> 12
<212> PRT
<213> Artificial Sequence

<220>
<223> Description of Artificial Sequence: Synthetic peptide

<400> 530
Val Tyr Ser Asp Gly Asn Thr Tyr Leu Asn Trp Phe
1               5                   10

<210> 531
<211> 10
<212> PRT
<213> Artificial Sequence

<220>
<223> Description of Artificial Sequence: Synthetic peptide

<400> 531
Leu Leu Ile Tyr Ser Asn Asn Gln Arg Pro
1               5                   10

<210> 532
<211> 10
<212> PRT
<213> Artificial Sequence

<220>
<223> Description of Artificial Sequence: Synthetic peptide

<400> 532
Leu Leu Ile Tyr Leu Gly Ser Tyr Arg Ala
1               5                   10

<210> 533
<211> 10
<212> PRT
<213> Artificial Sequence

<220>
<223> Description of Artificial Sequence: Synthetic peptide

<400> 533
Leu Leu Ile Tyr Leu Gly Ser Tyr Arg Ala
1 5 10

<210> 534
<211> 10
<212> PRT
<213> Artificial Sequence

<220>
<223> Description of Artificial Sequence: Synthetic peptide

<400> 534
Leu Leu Ile Tyr Ser Asn Asn Gln Arg Pro
1 5 10

<210> 535
<211> 10
<212> PRT
<213> Artificial Sequence

<220>
<223> Description of Artificial Sequence: Synthetic peptide

<400> 535
Leu Leu Leu Tyr Ser Asp Asn Gln Arg Pro
1 5 10

<210> 536
<211> 10
<212> PRT
<213> Artificial Sequence

<220>
<223> Description of Artificial Sequence: Synthetic peptide

<400> 536
Arg Leu Ile Tyr Lys Val Ser Thr Arg Asp
1 5 10

<210> 537
<211> 10
<212> PRT
<213> Artificial Sequence

<220>
<223> Description of Artificial Sequence: Synthetic peptide

<400> 537
Ala Ala Trp Asp Asp Ser Leu Asn Gly Pro
1 5 10

<210> 538
<211> 8
<212> PRT
<213> Artificial Sequence

<220>
<223> Description of Artificial Sequence: Synthetic peptide

<400> 538
Met Gln Ala Leu Gln Thr Pro Pro
1 5

<210> 539
<211> 8
<212> PRT
<213> Artificial Sequence

<220>
<223> Description of Artificial Sequence: Synthetic peptide

<400> 539
Met Gln Val Leu Gln Thr Pro Trp
1 5

<210> 540
<211> 10
<212> PRT
<213> Artificial Sequence

<220>
<223> Description of Artificial Sequence: Synthetic peptide

<400> 540
Ala Ala Trp Asp Asp Ser Leu Asn Gly Pro
1 5 10

<210> 541
<211> 10
<212> PRT
<213> Artificial Sequence

<220>
<223> Description of Artificial Sequence: Synthetic peptide

<400> 541
Ala Ala Trp Asp Asp Ser Leu Asn Gly Pro
1 5 10

<210> 542
<211> 8
<212> PRT
<213> Description of Artificial Sequence: Synthetic peptide

<400> 542
Leu Gln Gly Thr His Trp Pro Trp
1 5